(19) Europäisches Patentamt
European Patent Office
Office européen des brevets

(11) EP 4 279 476 A1

(12) # EUROPEAN PATENT APPLICATION
published in accordance with Art. 153(4) EPC

(43) Date of publication:
**22.11.2023 Bulletin 2023/47**

(21) Application number: **22736786.9**

(22) Date of filing: **06.01.2022**

(51) International Patent Classification (IPC):
***C07C 41/16*** (2006.01) ***C07C 41/58*** (2006.01)
***C07C 43/23*** (2006.01) ***C07C 209/68*** (2006.01)
***C07C 211/27*** (2006.01) ***C07C 211/55*** (2006.01)
***C07C 211/56*** (2006.01) ***C07C 211/63*** (2006.01)
***C07D 237/24*** (2006.01) ***C07D 401/12*** (2006.01)
***C07D 401/14*** (2006.01) ***C40B 40/04*** (2006.01)
***C07D 213/65*** (2006.01) ***C07D 213/74*** (2006.01)
***C07D 213/82*** (2006.01) ***C07D 295/155*** (2006.01)
***C07D 295/192*** (2006.01) ***C07D 295/205*** (2006.01)
***C07D 239/42*** (2006.01) ***C07F 9/09*** (2006.01)

(52) Cooperative Patent Classification (CPC):
**C07C 41/16; C07C 41/58; C07C 43/23; C07C 209/68; C07C 211/27; C07C 211/55; C07C 211/56; C07C 211/63; C07D 213/65; C07D 213/74; C07D 213/82; C07D 237/24; C07D 239/42; C07D 295/155; C07D 295/192;**

(Cont.)

(86) International application number:
**PCT/JP2022/003468**

(87) International publication number:
**WO 2022/149618 (14.07.2022 Gazette 2022/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.01.2021 JP 2021001134**

(71) Applicant: **CHUGAI SEIYAKU KABUSHIKI KAISHA**
**Tokyo 115-8543 (JP)**

(72) Inventors:
- **HAYASE, Tadakatsu**
  **Gotemba-shi, Shizuoka 412-8513 (JP)**
- **TSUCHIYA, Satoshi**
  **Gotemba-shi, Shizuoka 412-8513 (JP)**
- **NOMURA, Kenichi**
  **Gotemba-shi, Shizuoka 412-8513 (JP)**
- **EMURA, Takashi**
  **Gotemba-shi, Shizuoka 412-8513 (JP)**

(74) Representative: **Vossius & Partner**
**Patentanwälte Rechtsanwälte mbB**
**Siebertstraße 3**
**81675 München (DE)**

(54) **METHOD FOR ALKYLATING ACIDIC FUNCTIONAL GROUP**

(57) The present invention provides a method for producing a compound having an alkylated acidic functional group, the method comprising reacting, in a mixture containing two or more compounds having an acidic functional group as substrates, the compounds with an alkylating agent selected from compounds represented by formula A, formula B, or formula C in the presence of a base to alkylate the acidic functional group.

EP 4 279 476 A1

Figure 1

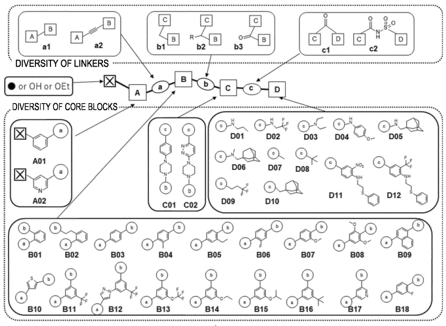


IN FIGURE 1, ● REPRESENTS 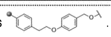 .

(52) Cooperative Patent Classification (CPC): (Cont.)
**C07D 295/205; C07D 401/12; C07D 401/14; C07F 9/09; C40B 40/04**

## Description

TECHNICAL FIELD

The present invention relates to a method for alkylating an acidic functional group. Furthermore, the present invention relates to a method for producing a compound, the method comprising a step of alkylating an acidic functional group.

BACKGROUND ART

Alkylation of acidic functional groups is a widely used reaction in the synthesis of compounds. In the case of alkylating a phenolic hydroxy group as the acidic functional group, for example, a method using iodomethane as an alkylating agent and potassium carbonate as a base is known (Non Patent Literature 1), but in addition to alkylation of the target phenolic hydroxy group, a tertiary amine is also alkylated as a side reaction (Non Patent Literature 2).

In order to selectively alkylate a phenolic hydroxy group, there are methods of using less reactive alkylating agents, for example, a method of using a trialkyl phosphate ester as an alkylating agent or a method of using a tetraalkyl ammonium salt as an alkylating agent. In these prior art, inorganic bases such as potassium carbonate (Patent Literature 1) and potassium hydroxide (Patent Literature 2) are used as a base.

In the case of alkylating (that is, esterifying) a carboxy group as the acidic functional group, for example, a method in which the carboxy group is heated to a high temperature in an alcohol with an acid catalyst and dehydration condensation is performed is known. As a method that is performed at a lower temperature, a method using iodomethane as an alkylating agent and potassium carbonate as a base is known (Non Patent Literature 3). However, similar to the alkylation of a phenolic hydroxy group, this is also known to be the reaction condition under which a tertiary amine is alkylated (Non Patent Literature 2).

As a less reactive alkylating agent, there is a method of using a trialkyl phosphate ester as an alkylating agent. In these prior art, the sodium salt of the carboxylic acid is prepared first and then the reaction is carried out (Non Patent Literatures 4 and 5).

CITATION LIST

PATENT LITERATURE

Patent Literature 1: U.S. Patent No. 4,453,017
Patent Literature 2: Japanese Patent Laid-Open No. 2006-213692

NON PATENT LITERATURE

Non Patent Literature 1: J. Am. Chem. Soc. 2007, 129, 15830.
Non Patent Literature 2: Bioorg. Med. Chem. Lett. 2004, 14, 3013.
Non Patent Literature 3: J. Med. Chem. 2011, 54, 1752.
Non Patent Literature 4: J. Am. Chem. Soc. 1990, 112, 297.
Non Patent Literature 5: J. Chem. Soc. PERKIN TRANS. I, 1989, 807.

SUMMARY OF INVENTION

TECHNICAL PROBLEM-

In the production of compound libraries, the structural transformation of acidic functional groups that occurs when they are cleaved from the solid phase not only controls the physical properties of a group of compounds in a compound library, but also leads to the expansion of structural diversity. Alkylation of acidic functional groups is one of the structural transformation methods, and especially when a mixture of compounds is used as a substrate for alkylation, an approach with extremely high selectivity is required in order to alkylate only the target acidic functional group while a wide variety of functional groups are present in the reaction system.

Although several methods of alkylating acidic functional groups are known, the following (1) to (3) need to be

considered in order to apply them to the production of compound libraries with high quality.

(1) In the post-treatment after alkylation, the reagent and impurities generated from the reagent can be removed from the target compound library by a convenient method (liquid-liquid extraction, distillation under reduced pressure, or a method using a solid phase reagent).
(2) In the case where the solubility of the reagent for alkylation is low, it can cause variation in reactivity when a mixture of compounds is used as a substrate.
(3) Compounds used as substrates may contain functional groups that can be alkylated other than the target acidic functional group, and when a compound having an alcoholic hydroxy group and a nitrogen-containing structure (amide, tertiary amine, pyridine, pyridazine, or the like) is used as a substrate, for example, there is a concern that alkylation to the alcoholic hydroxy group and nitrogen-containing structure (amide, tertiary amine, pyridine, pyridazine, or the like) may also proceed as a side reaction.

For example, in the synthesis of libraries, from the viewpoint of obtaining compound libraries with high quality in alkylation using a mixture containing a large number of compounds as a substrate, there is a need for an alkylation method with higher reactivity and selectivity. That is, when alkylation of a phenolic hydroxy group was performed based on the description in Patent Literatures 1 and 2, the yield was either low or unstable and the desired result could not be obtained. Stirring efficiency was found to be one factor responsible for the low reactivity and poor reproducibility, which was inferred to be due to low solubility of a reagent used for alkylation in the reaction mixture. An object of the present invention is to provide a novel alkylation method, and in particular, to provide an alkylation method with high yield and high regioselectivity. Furthermore, another object of the present invention is to provide a method for alkylating an acidic functional group, the method having a wide range of substrate adaptability, and in particular, being able to utilize a mixture containing multiple compounds as a substrate in the synthesis of a compound library.

SOLUTION TO PROBLEM

The present inventors have found an alkylation method with preferred reactivity and have reached the completion of the present invention. The present inventors have found that the method is applicable to a mixture containing multiple compounds as a substrate, and have confirmed that, for example, the method is applicable to the selective alkylation of an acidic functional group that occurs when it is cleaved from the solid phase in the production of a compound library.
In one aspect of the present invention, the following inventions are provided.

[1] A method for producing a compound having an alkylated acidic functional group, the method comprising

reacting, in a mixture containing two or more compounds having an acidic functional group as substrates, the compounds with an alkylating agent selected from compounds represented by formula A, formula B, or formula C in the presence of a base to alkylate the acidic functional group:

[Formula 1]

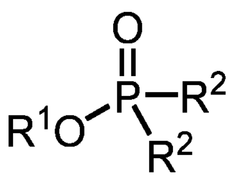

(A)

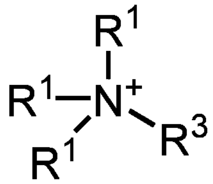

(B)

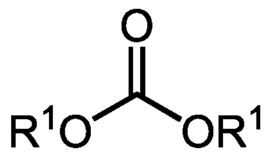

(C)

wherein $R^1$ is independently selected from a $C_{1-6}$ alkyl optionally substituted with one or more substituents selected from X;
X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;
$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and
$R^3$ is independently selected from a $C_{1-6}$ alkyl optionally substituted with one or more substituents selected from X, and an aryl, where the aryl is optionally substituted with one or more substituents independently selected

from a halogen atom, a $C_{1-4}$ alkyl, and a $C_{1-4}$ alkoxy,

wherein the base is a base selected from the group consisting of an organic base whose conjugate acid has a pKa of 23 to 34 in acetonitrile and an inorganic base whose conjugate acid has a pKa of 9 to 20 in water.
[2] The method according to [1], comprising removing an impurity after the alkylation.
[3] The method according to [1] or [2], wherein the base has a solubility in dimethylformamide (DMF) of 120 mg/mL or more at 25°C.
[4] The method according to any of [1] to [3], wherein the base is selected from the organic base.
[5] The method according to any of [1] to [4], wherein the base is selected from a phosphazene.
[6] A method for producing a compound having an alkoxy-substituted aromatic ring, the method comprising

reacting a compound containing a hydroxy group as a substituent on an aromatic ring with an alkylating agent selected from compounds represented by formula A, formula B, or formula C in the presence of a base to alkylate the hydroxy group as a substituent on an aromatic ring:

[Formula 2]

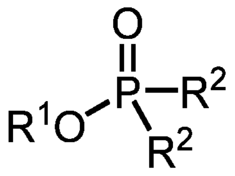
(A)

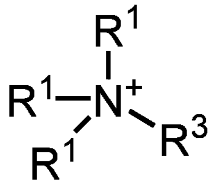
(B)

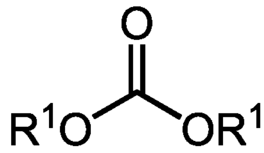
(C)

wherein $R^1$ is independently selected from a $C_{1-6}$ alkyl optionally substituted with one or more substituents selected from X;
X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;
$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and
$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

wherein the base is selected from a phosphazene.
[7] A method for producing an ester compound, the method comprising

reacting a compound containing a carboxy group with an alkylating agent selected from compounds represented by formula A, formula B, or formula C in the presence of a base to alkylate the carboxy group:

[Formula 3]

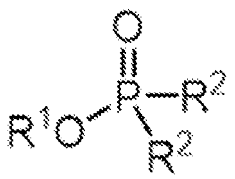
(A)

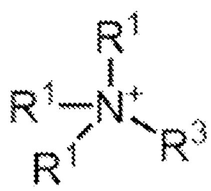
(B)

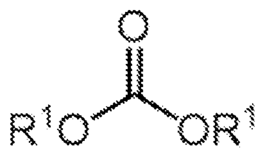
(C)

wherein $R^1$ is independently selected from a $C_{1-6}$ alkyl optionally substituted with one or more substituents selected from X;
X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$

alkyl, or a $C_{1-4}$ alkoxy;

$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and

$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

wherein the base is selected from a phosphazene.

[8] The method according to any of [1] to [7], wherein the alkylating agent is selected from the group consisting of alkylating agents represented by formulas A and B according to claim 1.

[9] The method according to any of [1] to [8], wherein the alkylating agent is selected from the group consisting of trimethyl phosphate, triethyl phosphate, and triallyl phosphate.

[10] The method according to any one of [1] to [9], wherein the base is at least one selected from the group consisting of 1-ethyl-2,2,4,4,4-pentakis(dimethylamino)-2λ5,4λ5-catenadi(phosphazene) (P2Et), 1-tert-butyl-2,2,4,4,4-pentakis(dimethylamino)-2λ5,4λ5-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphospholine (BEMP), and tert-butylimino-tri(pyrrolidino)phosphorane (BTPP).

[11] The method according to any of [1] to [10], wherein the acidic functional group is a hydroxy group as a substituent on an aromatic ring and/or a carboxy group.

[12] The method according to any of [1] to [11], wherein the acidic functional group is a hydroxy group as a substituent on an aromatic ring.

[13] The method according to any of [1] to [12], wherein the substrate contains one or more compounds containing a phenolic hydroxy group as the acidic functional group and a nitrogen atom that can be alkylated, and in the compound having a nitrogen atom in which alkylation proceeds most among the substrate, a production ratio of products by alkylation of the phenolic hydroxy group (alkylation A) and alkylation of the nitrogen atom (alkylation C), (alkylation A/alkylation C), is 1.5 or more.

[14] The method according to any of [1] to [10], wherein the acidic functional group is a carboxy group.

[15] The method according to any of [1] to [10] and [14], wherein the substrate contains one or more compounds containing a carboxy group as the acidic functional group and a nitrogen atom that can be alkylated, and in the compound having a nitrogen atom in which alkylation proceeds most among the substrate, a production ratio of products by alkylation of the carboxy group (alkylation D) and alkylation of the nitrogen atom (alkylation E), (alkylation D/alkylation E), is 1.5 or more.

[16] The method according to any of [1] to [15], wherein alkylation C or alkylation D is alkylation of a nitrogen atom contained in a nitrogen-containing structure selected from a diarylamine, an alkylarylamine, an aniline, a tertiary amine, an amide, a sulfonamide, an acylsulfonamide, and a pyridine.

[17] A method for producing a compound constituting a compound library, the method comprising producing a compound alkylated by the method according to any one of [1] to [16].

[18] The method according to any of [1] to [17], wherein a mixture containing 10 or more compounds is produced.

[19] An alkylating agent containing a compound represented by formula A, formula B, or formula C:

[Formula 4]

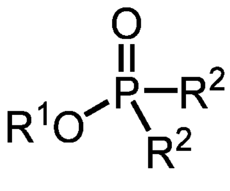
(A)

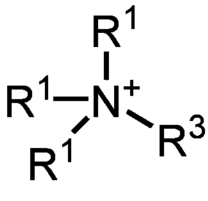
(B)

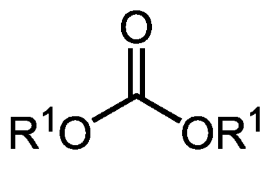
(C)

wherein $R^1$ is independently selected from a $C_{1-6}$ alkyl optionally substituted with one or more substituents selected from X;

X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;

$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and

$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

for use in a method for producing an alkylated compound, wherein the method is the method according to any of [1] to [18].

[A1] A method for producing a compound having an alkylated acidic functional group, the method comprising

reacting, in a mixture containing two or more compounds having an acidic functional group as substrates, the compounds with an alkylating agent selected from compounds represented by formula A, formula B, or formula C in the presence of a base to alkylate the acidic functional group:

[Formula 5]

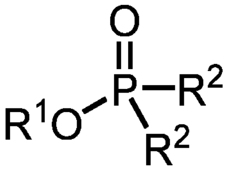

(A)

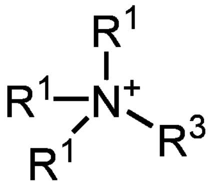

(B)

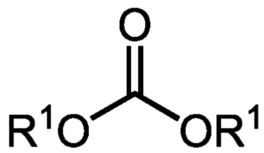

(C)

wherein $R^1$ is independently selected from a $C_{1-6}$ alkyl optionally substituted with one or more substituents selected from X;
X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;
$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and
$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

wherein the base is a base selected from the group consisting of an organic base whose conjugate acid has a pKa of 23 to 34 in acetonitrile and an inorganic base whose conjugate acid has a pKa of 9 to 20 in water.
[A2] The method according to [A1], comprising removing an impurity after the alkylation.
[A3] The method according to [A2], wherein the removal of an impurity is at least one selected from the group consisting of liquid-liquid separation, distillation under reduced pressure, and a method using a solid phase reagent.
[A4] The method according to [A2] or [A3], wherein the step of removing an impurity is liquid-liquid separation.
[A5] The method according to [A2] or [A3], wherein the step of removing an impurity is distillation under reduced pressure.
[A6] The method according to [A2] or [A3], wherein the step of removing an impurity is a method using a solid phase reagent.
[A7] The method according to any of [A2] to [A6], wherein the impurity is at least one selected from the group consisting of a remaining alkylating agent after the alkylation, the base, an alkylating agent-derived impurity, a base-derived impurity, and a solvent.
[A8] The method according to any of [A1] to [A7], wherein the base has a solubility in dimethylformamide (DMF) of 120 mg/mL or more at 25°C.
[A9] The method according to any of [A1] to [A8], wherein the base is selected from an organic base.
[A10] The method according to any of [A1] to [A9], wherein the base is a base selected from the group consisting of an organic base whose conjugated acid has a pKa of 24 to 34, 25 to 34, or 28 to 34 in acetonitrile.
[A11] The method according to any of [A1] to [A10], wherein the base is a base selected from the group consisting of an organic base whose conjugate acid has a pKa in acetonitrile that is equal to or more than a pKa value of 2-tert-butyl-1,1,3,3-tetramethylguanidine (BTMG), and/or is equal to or less than a pKa value of 1-tert-butyl-2,2,4,4,4-pentakis(dimethylamino)-2$\lambda^5$,4$\lambda^5$-catenadi(phosphazene) (P2tBu).
[A12] The method according to any of [A1] to [A9], wherein the base is selected from a phosphazene.

[A13] The method according to any of [A1] to [A12], wherein an alkoxy resulting from the alkylation has a structure of $-CH_2-O-$.
[A14] The method according to any of [A1] to [A13], wherein an alkoxy resulting from the alkylation is methoxy, ethoxy, or allyloxy.
[A15] The method according to any of [A1] to [A14], wherein the alkylation results in an ester having a structure of $-CH_2-O-(C=O)-$.
[A16] The method according to any of [A1] to [A15], wherein the alkylation results in a methyl ester, an ethyl ester, or an allyl ester.
[A17] The method according to any of [A1] to [A16], wherein the reaction is carried out in a solvent.
[A18] The method according to [A17], wherein the solvent contains at least one solvent selected from an ether solvent such as tetrahydrofuran (THF), 2-methyltetrahydrofuran, 4-methyltetrahydropyran, 1,4-dioxane, 1,2-dimethoxyethane (DME), t-butyl methyl ether (TBME), cyclopentyl methyl ether (CPME), and isosorbide dimethyl ether, an ester solvent such as ethyl acetate, propyl acetate, isopropyl acetate, butyl acetate, isobutyl acetate, and GVL ($\gamma$-valerolactone), a halogenated solvent such as dichloromethane (DCM), 1,2-dichloroethane (DCE), and chloroform, an amide solvent such as N,N-dimethylformamide (DMF), N-methyl-2-pyrrolidone (NMP), N,N-dimethylacetamide (DMA), N-ethyl-2-pyrrolidone (NEP), and N-butyl-2-pyrrolidone (NBP), a urea solvent such as N,N,N',N'-tetramethylurea (TMU), N,N'-dimethylpropyleneurea (DMPU), and 1,3-dimethyl-2-imidazolidinone (DMI), a sulfoxide solvent such as dimethyl sulfoxide (DMSO), and an aromatic solvent such as anisole, toluene, $\alpha,\alpha,\alpha$-trifluorotoluene, 1,2-dichlorobenzene, and benzene.
[A19] The method according to any of [A1] to [A18], wherein the reaction is carried out at 25 to 130°C, 40 to 120°C, 60 to 100°C, or 75 to 85°C.
[A20] The method according to any of [A1] to [A19], wherein the alkylating agent is used in an amount of 1.0 to 100.0 equivalents, 2.0 to 50.0 equivalents, 3.0 to 30.0 equivalents, 5.0 to 25.0 equivalents, 10.0 to 20.0 equivalents, or 12.5 to 17.5 equivalents relative to the acidic functional group.
[A21] The method according to any of [A1] to [A20], wherein the base is used in an amount of 1.0 to 100.0 equivalents, 1.5 to 50.0 equivalents, 1.5 to 30.0 equivalents, 3.0 to 25.0 equivalents, 8.0 to 20.0 equivalents, or 12.5 to 17.5 equivalents relative to the acidic functional group.
[A22] The method according to any of [A1] to [A21], wherein the alkylating agent is selected from the group consisting of compounds represented by formulas A and B according to [A1].
[A23] The method according to any of [A1] to [A22], wherein the alkylating agent is selected from the group consisting of a compound represented by formula A according to [A1].
[A24] The method according to any of [A1] to [A23], wherein the alkylating agent is selected from the group consisting of trimethyl phosphate, triethyl phosphate, and triallyl phosphate.
[A25] The method according to any of [A1] to [A24], wherein the base is at least one selected from the group consisting of 1-ethyl-2,2,4,4,4-pentakis(dimethylamino)-2$\lambda^5$,4$\lambda^5$-catenadi(phosphazene) (P2Et), 1-tert-butyl-2,2,4,4,4-pentakis(dimethylamino)-2$\lambda^5$,4$\lambda^5$-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphospholine (BEMP), tert-butylimino-tri(pyrrolidino)phosphorane (BTPP), 1,1,3,3-tetramethylguanidine (TMG), 2-tert-butyl-1,1,3,3-tetramethylguanidine (BTMG), 1,8-diazabicyclo[5.4.0]undec-7-ene (DBU), 7-methyl-1,5,7-triazabicyclo[4.4.0]dec-5-ene (MTBD), 1,5,7-triazabicyclo[4.4.0]dec-5-ene (TBD), and cesium carbonate ($Cs_2CO_3$).
[A26] The method according to any of [A1] to [A25], wherein the acidic functional group is a hydroxy group as a substituent on an aromatic ring.
[A27] The method according to any of [A1] to [A26], wherein the acidic functional group is a hydroxy group as a substituent on a benzene ring or a pyridine ring.
[A28] The method according to any of [A1] to [A27], wherein the substrate contains one or more compounds containing a phenolic hydroxy group as the acidic functional group and a nitrogen atom that can be alkylated, and in the compound having a nitrogen atom in which alkylation proceeds most among the substrate, a production ratio of products by alkylation of the phenolic hydroxy group (alkylation A) and alkylation of the nitrogen atom (alkylation C), (alkylation A/alkylation C), is 1.5 or more, 3 or more, 5 or more, 7 or more, 9 or more, 10 or more, 12 or more, 15 or more, 20 or more, 30 or more, or 50 or more.
[A29] The method according to any of [A1] to [A28], wherein alkylation C is alkylation of a nitrogen atom contained in a nitrogen-containing structure selected from a diarylamine, an alkylarylamine, an aniline, a tertiary amine, an amide, a sulfonamide, an acylsulfonamide, and a pyridine.
[A30] The method according to any of [A1] to [A25], wherein the acidic functional group is a carboxy group.
[A31] The method according to any of [A1] to [A25] and [A30], wherein the substrate contains one or more compounds containing a carboxy group as the acidic functional group and a nitrogen atom that can be alkylated, and in the compound having a nitrogen atom in which alkylation proceeds most among the substrate, a production ratio of products by alkylation of the carboxy group (alkylation D) and alkylation of the nitrogen atom (alkylation E), (alkylation

D/alkylation E), is 1.5 or more, 3 or more, 5 or more, 7 or more, 9 or more, 10 or more, 12 or more, 15 or more, 20 or more, 30 or more, or 50 or more.
[A32] The method according to [A31], wherein alkylation E is alkylation of a nitrogen atom contained in a nitrogen-containing structure selected from a diarylamine, an alkylarylamine, an aniline, a tertiary amine, an amide, a sulfonamide, an acylsulfonamide, and a pyridine.
[A33] The method according to any of [A1] to [A32], wherein the substrate contains one or more phenolic hydroxy groups and one or more carboxy groups as the acidic functional group, and any acidic functional group of the one or more phenolic hydroxy groups and one or more carboxy groups is alkylated.
[A34] The method according to any of [A1] to [A33], further comprising, in a substrate containing one or more phenolic hydroxy groups and one or more carboxy groups as the acidic functional group, alkylating the acidic functional group of both the one or more phenolic hydroxy groups and the one or more carboxy groups, and then converting one or more carboxy groups that have been alkylated to carboxy groups.
[A35] The method according to any of [A1] to [A34], wherein, in a substrate containing one or more phenolic hydroxy groups and one or more carboxy groups as the acidic functional group, the acidic functional group of both the one or more phenolic hydroxy groups and the one or more carboxy groups is alkylated, and then one or more carboxy groups that have been alkylated are converted to carboxy groups, thereby alkylating only the one or more phenolic hydroxy groups.
[A36] The method according to any of [A1] to [A35] for production of a compound constituting a compound library.
[A37] The method according to any of [A1] to [A36], wherein the mixture contains 10 or more compounds as the substrate.
[A38] The method according to any of [A1] to [A37], further comprising preparing a compound having an acidic functional group by cleavage from a solid phase support.

[B1] A method for producing a compound having an alkoxy-substituted aromatic ring, the method comprising

reacting a compound containing a hydroxy group as a substituent on an aromatic ring with an alkylating agent selected from compounds represented by formula A, formula B, or formula C in the presence of a base to alkylate the hydroxy group as a substituent on an aromatic ring:

[Formula 6]

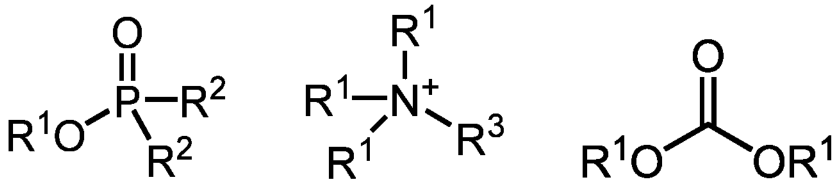

(A) (B) (C)

wherein $R^1$ is independently selected from a $C_{1-6}$ alkyl optionally substituted with one or more substituents selected from X;
X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;
$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and
$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

wherein the base is selected from a phosphazene.
[B2] The method according to [B1], comprising removing an impurity after the alkylation.
[B3] The method according to [B1], wherein the removal of an impurity is at least one selected from the group consisting of liquid-liquid separation, distillation under reduced pressure, and a method using a solid phase reagent.
[B4] The method according to [B2] or [B3], wherein the step of removing an impurity is liquid-liquid separation.
[B5] The method according to [B2] or [B3], wherein the step of removing an impurity is distillation under reduced pressure.

[B6] The method according to [B2] or [B3], wherein the step of removing an impurity is a method using a solid phase reagent.

[B7] The method according to any of [B1] to [B6], wherein the impurity is at least one selected from the group consisting of a remaining alkylating agent after the alkylation, the base, an alkylating agent-derived impurity, a base-derived impurity, and a solvent.

[B8] The method according to any of [B1] to [B7], wherein an alkoxy resulting from the alkylation has a structure of $-CH_2-O-$.

[B9] The method according to any of [B1] to [B8], wherein an alkoxy resulting from the alkylation is methoxy, ethoxy, or allyloxy.

[B10] The method according to any of [B1] to [B9], wherein the reaction is carried out in a solvent.

[B11] The method according to any of [B1] to [B10], wherein the solvent is at least one selected from the group consisting of an ether solvent such as tetrahydrofuran (THF), 2-methyltetrahydrofuran, 4-methyltetrahydropyran, 1,4-dioxane, 1,2-dimethoxyethane (DME), t-butyl methyl ether (TBME), cyclopentyl methyl ether (CPME), and isosorbide dimethyl ether, an ester solvent such as ethyl acetate, propyl acetate, isopropyl acetate, butyl acetate, isobutyl acetate, and GVL ($\gamma$-valerolactone), a halogenated solvent such as dichloromethane (DCM), 1,2-dichloroethane (DCE), and chloroform, an amide solvent such as N,N-dimethylformamide (DMF), N-methyl-2-pyrrolidone (NMP), N,N-dimethylacetamide (DMA), N-ethyl-2-pyrrolidone (NEP), and N-butyl-2-pyrrolidone (NBP), a urea solvent such as N,N,N',N'-tetramethylurea (TMU), N,N'-dimethylpropyleneurea (DMPU), and 1,3-dimethyl-2-imidazolidinone (DMI), a sulfoxide solvent such as dimethyl sulfoxide (DMSO), and an aromatic solvent such as anisole, toluene, $\alpha,\alpha,\alpha$-trifluorotoluene, 1,2-dichlorobenzene, and benzene.

[B12] The method according to any of [B1] to [B11], wherein the reaction is carried out at 25 to 130°C, 40 to 120°C, 60 to 100°C, or 75 to 85°C.

[B13] The method according to any of [B1] to [B12], wherein the alkylating agent is used in an amount of 1.0 to 100.0 equivalents, 2.0 to 50.0 equivalents, 3.0 to 30.0 equivalents, 5.0 to 25.0 equivalents, 10.0 to 20.0 equivalents, or 12.5 to 17.5 equivalents relative to the hydroxy group.

[B14] The method according to any of [B1] to [B13], wherein the base is used in an amount of 1.0 to 100.0 equivalents, 1.5 to 50.0 equivalents, 1.5 to 30.0 equivalents, 3.0 to 25.0 equivalents, 8.0 to 20.0 equivalents, or 12.5 to 17.5 equivalents relative to the hydroxy group.

[B15] The method according to any of [B1] to [B14], wherein the alkylating agent is selected from the group consisting of alkylating agents represented by formulas A and B according to [B1].

[B16] The method according to any of [B1] to [B15], wherein the alkylating agent is selected from the group consisting of trimethyl phosphate, triethyl phosphate, and triallyl phosphate.

[B17] The method according to any of [B1] to [B16], wherein the base is at least one selected from the group consisting of 1-ethyl-2,2,4,4,4-pentakis(dimethylamino)-2$\lambda^5$,4$\lambda^5$-catenadi(phosphazene) (P2Et), 1-tert-butyl-2,2,4,4,4-pentakis(dimethylamino)-2$\lambda^5$,4$\lambda^5$-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphospholine (BEMP), and tert-butylimino-tri(pyrrolidino)phosphorane (BTPP).

[B18] The method according to any of [B1] to [B17], wherein the hydroxy group as a substituent on an aromatic ring is a hydroxy group as a substituent on an aromatic ring selected from a benzene ring, a pyridine ring, a pyrimidine ring, a pyrazine ring, a triazine ring, a pyrazole ring, an imidazole ring, a furan ring, a thiophene ring, an oxazole ring, a thiazole ring, a naphthalene ring, a quinoline ring, an indole ring, an indazole ring, and an isoquinoline ring.

[B19] The method according to any of [B1] to [B18], wherein the hydroxy group as a substituent on an aromatic ring is a hydroxy group as a substituent on a benzene ring or a pyridine ring.

[B20] The method according to any of [B1] to [B19], wherein the reaction is carried out using two or more compounds as substrates, the substrate contains one or more compounds containing a phenolic hydroxy group as an acidic functional group and a nitrogen atom that can be alkylated, and in the compound having a nitrogen atom in which alkylation proceeds most among the substrate, a production ratio of products by alkylation of the phenolic hydroxy group (alkylation A) and alkylation of the nitrogen atom (alkylation C), (alkylation A/alkylation C), is 1.5 or more, 3 or more, 5 or more, 7 or more, 9 or more, 10 or more, 12 or more, 15 or more, 20 or more, 30 or more, or 50 or more.

[B21] The method according to any of [B1] to [B20] for production of a compound constituting a compound library.

[B22] The method according to any of [B1] to [B21], wherein the mixture contains 10 or more compounds as substrates.

[B23] The method according to any of [B1] to [B22], further comprising preparing a compound having a hydroxy group by cleavage from a solid phase support.

[C1] A method for producing a compound constituting a compound library, the method comprising producing a compound alkylated by the method according to any of [A1] to [A38], [B1] to [B23], and [E1] to [E21].

[C2] The method according to [C1], wherein a mixture containing 10 or more, 50 or more, 100 or more, 500 or more, or 1000 or more compounds is produced.

[D1] An alkylating agent containing a compound represented by formula A, formula B, or formula C:

[Formula 7]

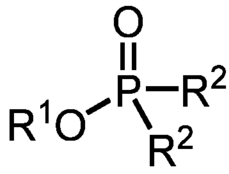

(A)

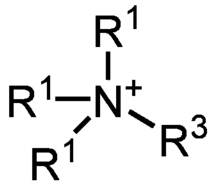

(B)

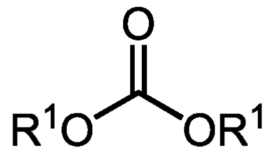

(C)

wherein $R^1$ is independently selected from a $C_{1-6}$ alkyl optionally substituted with one or more substituents selected from X;

X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;

$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and

$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

for use in a method for producing an alkylated compound, wherein the method is the method according to any of [A1] to [A38], [B1] to [B23], and [E1] to [E21].

[E1] A method for producing an ester compound, the method comprising

reacting a compound containing a carboxy group with an alkylating agent selected from compounds represented by formula A, formula B, or formula C in the presence of a base to alkylate the carboxy group:

[Formula 8]

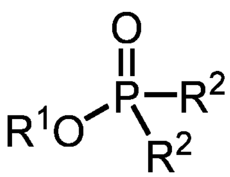

(A)

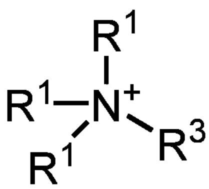

(B)

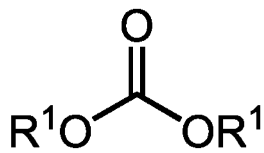

(C)

wherein $R^1$ is independently selected from a $C_{1-6}$ alkyl optionally substituted with one or more substituents selected from X;

X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;

$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and

$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

wherein the base is selected from a phosphazene.

[E2] The method according to [E1], comprising removing an impurity after the alkylation.

[E3] The method according to [E1], wherein the removal of an impurity is at least one selected from the group consisting of liquid-liquid separation, distillation under reduced pressure, and a method using a solid phase reagent.

[E4] The method according to [E2] or [E3], wherein the step of removing an impurity is liquid-liquid separation.

[E5] The method according to [E2] or [E3], wherein the step of removing an impurity is distillation under reduced pressure.

[E6] The method according to [E2] or [E3], wherein the step of removing an impurity is a method using a solid phase reagent.

[E7] The method according to any of [E1] to [E6], wherein the impurity is at least one selected from the group consisting of a remaining alkylating agent after the alkylation, the base, an alkylating agent-derived impurity, a base-derived impurity, and a solvent.

[E8] The method according to any of [E1] to [E7], wherein an ester resulting from the alkylation has a structure of $-CH_2-O-(C=O)-$.

[E9] The method according to any of [E1] to [E8], wherein an ester resulting from the alkylation is a methyl ester, an ethyl ester, or an allyl ester.

[E10] The method according to any of [E1] to [E9], wherein the reaction is carried out in a solvent.

[E11] The method according to any of [E1] to [E10], wherein the solvent is at least one selected from the group consisting of an ether solvent such as tetrahydrofuran (THF), 2-methyltetrahydrofuran, 4-methyltetrahydropyran, 1,4-dioxane, 1,2-dimethoxyethane (DME), t-butyl methyl ether (TBME), cyclopentyl methyl ether (CPME), and isosorbide dimethyl ether, an ester solvent such as ethyl acetate, propyl acetate, isopropyl acetate, butyl acetate, isobutyl acetate, and GVL ($\gamma$-valerolactone), a halogenated solvent such as dichloromethane (DCM), 1,2-dichloroethane (DCE), and chloroform, an amide solvent such as N,N-dimethylformamide (DMF), N-methyl-2-pyrrolidone (NMP), N,N-dimethylacetamide (DMA), N-ethyl-2-pyrrolidone (NEP), and N-butyl-2-pyrrolidone (NBP), a urea solvent such as N,N,N',N'-tetramethylurea (TMU), N,N'-dimethylpropyleneurea (DMPU), and 1,3-dimethyl-2-imidazolidinone (DMI), a sulfoxide solvent such as dimethyl sulfoxide (DMSO), and an aromatic solvent such as anisole, toluene, $\alpha,\alpha,\alpha$-trifluorotoluene, 1,2-dichlorobenzene, and benzene.

[E12] The method according to any of [E1] to [E11], wherein the reaction is carried out at 25 to 130°C, 40 to 120°C, 60 to 100°C, or 75 to 85°C.

[E13] The method according to any of [E1] to [E12], wherein the alkylating agent is used in an amount of 1.0 to 100.0 equivalents, 2.0 to 50.0 equivalents, 3.0 to 30.0 equivalents, 5.0 to 25.0 equivalents, 10.0 to 20.0 equivalents, or 12.5 to 17.5 equivalents relative to the hydroxy group.

[E14] The method according to any of [E1] to [E13], wherein the base is used in an amount of 1.0 to 100.0 equivalents, 1.5 to 50.0 equivalents, 1.5 to 30.0 equivalents, 3.0 to 25.0 equivalents, 8.0 to 20.0 equivalents, or 12.5 to 17.5 equivalents relative to the hydroxy group.

[E15] The method according to any of [E1] to [E14], wherein the alkylating agent is selected from the group consisting of alkylating agents represented by formulas A and B according to [B1].

[E16] The method according to any of [E1] to [E15], wherein the alkylating agent is selected from the group consisting of trimethyl phosphate, triethyl phosphate, and triallyl phosphate.

[E17] The method according to any of [E1] to [E16], wherein the base is at least one selected from the group consisting of 1-ethyl-2,2,4,4,4-pentakis(dimethylamino)-2$\lambda^5$,4$\lambda^5$-catenadi(phosphazene) (P2Et), 1-tert-butyl-2,2,4,4,4-pentakis(dimethylamino)-2$\lambda^5$,4$\lambda^5$-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphospholine (BEMP), and tert-butylimino-tri(pyrrolidino)phosphorane (BTPP).

[E18] The method according to any of [E1] to [E17], wherein the reaction is carried out using two or more compounds as substrates, the substrate contains one or more compounds containing a carboxy group as an acidic functional group and a nitrogen atom that can be alkylated, and in the compound having a nitrogen atom in which alkylation proceeds most among the substrate, a production ratio of products by alkylation of the carboxy group (alkylation D) and alkylation of the nitrogen atom (alkylation E), (alkylation D/alkylation E), is 1.5 or more, 3 or more, 5 or more, 7 or more, 9 or more, 10 or more, 12 or more, 15 or more, 20 or more, 30 or more, or 50 or more.

[E19] The method according to any of [E1] to [E18] for production of a compound constituting a compound library.

[E20] The method according to any of [E1] to [E19], wherein the mixture contains 10 or more compounds as substrates.

[E21] The method according to any of [E1] to [E20], further comprising preparing a compound having a carboxy group by cleavage from a solid phase support.

[F1] A method for producing a resin for solid phase synthesis having a side chain containing an alkylated acidic functional group, the method comprising,

using a resin containing two or more side chains having an acidic functional group as a substrate, reacting the resin with an alkylating agent selected from compounds represented by formula A, formula B, or formula C in the presence of a base to alkylate the acidic functional group:

[Formula 9]

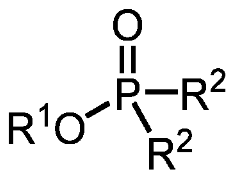

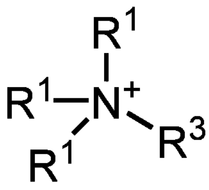

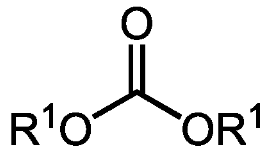

(A) (B) (C)

wherein $R^1$ is independently selected from a $C_{1-6}$ alkyl optionally substituted with one or more substituents selected from X;

X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;

$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and

$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

wherein the base is a base selected from the group consisting of an organic base whose conjugate acid has a pKa of 23 to 34 in acetonitrile and an inorganic base whose conjugate acid has a pKa of 9 to 20 in water.

[F2] The method according to [F1], comprising removing an impurity after the alkylation.

[F3] The method according to [F2], wherein the removal of an impurity is at least one selected from the group consisting of liquid-liquid separation, distillation under reduced pressure, and a method using a solid phase reagent.

[F4] The method according to [F2] or [F3], wherein the step of removing an impurity is liquid-liquid separation.

[F5] The method according to [F2] or [F3], wherein the step of removing an impurity is distillation under reduced pressure.

[F6] The method according to [F2] or [F3], wherein the step of removing an impurity is a method using a solid phase reagent.

[F7] The method according to any of [F2] to [F6], wherein the impurity is at least one selected from the group consisting of a remaining alkylating agent after the alkylation, the base, an alkylating agent-derived impurity, a base-derived impurity, and a solvent.

[F8] The method according to any of [F1] to [F7], wherein the base has a solubility in dimethylformamide (DMF) of 120 mg/mL or more at 25°C.

[F9] The method according to any of [F1] to [F8], wherein the base is selected from an organic base.

[F10] The method according to any of [F1] to [F9], wherein the base is a base selected from the group consisting of an organic base whose conjugated acid has a pKa of 24 to 34, 25 to 34, or 28 to 34 in acetonitrile.

[F11] The method according to any of [F1] to [F10], wherein the base is a base selected from the group consisting of an organic base whose conjugate acid has a pKa in acetonitrile that is equal to or more than a pKa value of 2-tert-butyl-1,1,3,3-tetramethylguanidine (BTMG), and/or is equal to or less than a pKa value of 1-tert-butyl-2,2,4,4,4-pentakis(dimethylamino)-2$\lambda^5$,4$\lambda^5$-catenadi(phosphazene) (P2tBu).

[F12] The method according to any of [F1] to [F9], wherein the base is selected from a phosphazene.

[F13] The method according to any of [F1] to [F12], wherein an alkoxy resulting from the alkylation has a structure of $-CH_2-O-$.

[F14] The method according to any of [F1] to [F13], wherein an alkoxy resulting from the alkylation is methoxy, ethoxy, or allyloxy.

[F15] The method according to any of [F1] to [F14], wherein the alkylation results in an ester having a structure of $-CH_2-O-(C=O)-$.

[F16] The method according to any of [F1] to [F15], wherein the alkylation results in a methyl ester, an ethyl ester, or an allyl ester.

[F17] The method according to any of [F1] to [F16], wherein the reaction is carried out in a solvent.

[F18] The method according to [F17], wherein the solvent contains at least one solvent selected from an ether solvent such as tetrahydrofuran (THF), 2-methyltetrahydrofuran, 4-methyltetrahydropyran, 1,4-dioxane, 1,2-dimethoxyethane (DME), t-butyl methyl ether (TBME), cyclopentyl methyl ether (CPME), and isosorbide dimethyl ether, an ester solvent such as ethyl acetate, propyl acetate, isopropyl acetate, butyl acetate, isobutyl acetate, and GVL

(γ-valerolactone), a halogenated solvent such as dichloromethane (DCM), 1,2-dichloroethane (DCE), and chloroform, an amide solvent such as N,N-dimethylformamide (DMF), N-methyl-2-pyrrolidone (NMP), N,N-dimethylacetamide (DMA), N-ethyl-2-pyrrolidone (NEP), and N-butyl-2-pyrrolidone (NBP), a urea solvent such as N,N,N',N'-tetramethylurea (TMU), N,N'-dimethylpropyleneurea (DMPU), and 1,3-dimethyl-2-imidazolidinone (DMI), a sulfoxide solvent such as dimethyl sulfoxide (DMSO), and an aromatic solvent such as anisole, toluene, α,α,α-trifluorotoluene, 1,2-dichlorobenzene, and benzene.

[F19] The method according to any of [F1] to [F18], wherein the reaction is carried out at 25 to 130°C, 40 to 120°C, 60 to 100°C, or 75 to 85°C.

[F20] The method according to any of [F1] to [F19], wherein the alkylating agent is used in an amount of 1.0 to 100.0 equivalents, 2.0 to 50.0 equivalents, 3.0 to 30.0 equivalents, 5.0 to 25.0 equivalents, 10.0 to 20.0 equivalents, or 12.5 to 17.5 equivalents relative to the acidic functional group.

[F21] The method according to any of [F1] to [F20], wherein the base is used in an amount of 1.0 to 100.0 equivalents, 1.5 to 50.0 equivalents, 1.5 to 30.0 equivalents, 3.0 to 25.0 equivalents, 8.0 to 20.0 equivalents, or 12.5 to 17.5 equivalents relative to the acidic functional group.

[F22] The method according to any of [F1] to [F21], wherein the alkylating agent is selected from the group consisting of compounds represented by formulas A and B according to [F1].

[F23] The method according to any of [F1] to [F22], wherein the alkylating agent is selected from the group consisting of a compound represented by formula A according to [F1].

[F24] The method according to any of [F1] to [F23], wherein the alkylating agent is selected from the group consisting of trimethyl phosphate, triethyl phosphate, and triallyl phosphate.

[F25] The method according to any of [F1] to [F24], wherein the base is at least one selected from the group consisting of 1-ethyl-2,2,4,4,4-pentakis(dimethylamino)-2$\lambda^5$,4$\lambda^5$-catenadi(phosphazene) (P2Et), 1-tert-butyl-2,2,4,4,4-pentakis(dimethylamino)-2$\lambda^5$,4$\lambda^5$-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphospholine (BEMP), tert-butyliminotri(pyrrolidino)phosphorane (BTPP), 1,1,3,3-tetramethylguanidine (TMG), 2-tert-butyl-1,1,3,3-tetramethylguanidine (BTMG), 1,8-diazabicyclo[5.4.0]undec-7-ene (DBU), 7-methyl-1,5,7-triazabicyclo[4.4.0]dec-5-ene (MTBD), 1,5,7-triazabicyclo[4.4.0]dec-5-ene (TBD), and cesium carbonate ($Cs_2CO_3$).

[F26] The method according to any of [F1] to [F25], wherein the acidic functional group is a hydroxy group as a substituent on an aromatic ring.

[F27] The method according to any of [F1] to [F26], wherein the acidic functional group is a hydroxy group as a substituent on an aromatic ring.

[F28] The method according to any of [F1] to [F27], wherein the resin for solid phase synthesis contains one or more side chains containing a phenolic hydroxy group as the acidic functional group and a nitrogen atom that can be alkylated, and in the compound having a nitrogen atom in which alkylation proceeds most among the resin, a production ratio of products by alkylation of the phenolic hydroxy group (alkylation A) and alkylation of the nitrogen atom (alkylation C), (alkylation A/alkylation C), is 1.5 or more, 3 or more, 5 or more, 7 or more, 9 or more, 10 or more, 12 or more, 15 or more, 20 or more, 30 or more, or 50 or more.

[F29] The method according to any of [F1] to [F28], wherein alkylation C is alkylation of a nitrogen atom contained in a nitrogen-containing structure selected from a diarylamine, an alkylarylamine, an aniline, a tertiary amine, an amide, a sulfonamide, an acylsulfonamide, and a pyridine.

[F30] The method according to any of [F1] to [F25], wherein the acidic functional group is a carboxy group.

[F31] The method according to any of [F1] to [F25] and [F30], wherein the resin for solid phase synthesis contains one or more side chains containing a carboxy group as the acidic functional group and a nitrogen atom that can be alkylated, and in the side chain having a nitrogen atom in which alkylation proceeds most among the resin, a production ratio of products by alkylation of the carboxy group (alkylation D) and alkylation of the nitrogen atom (alkylation E), (alkylation D/alkylation E), is 1.5 or more, 3 or more, 5 or more, 7 or more, 9 or more, 10 or more, 12 or more, 15 or more, 20 or more, 30 or more, or 50 or more.

[F32] The method according to [F31], wherein alkylation E is alkylation of a nitrogen atom contained in a nitrogen-containing structure selected from a diarylamine, an alkylarylamine, an aniline, a tertiary amine, an amide, a sulfonamide, an acylsulfonamide, and a pyridine.

[F33] The method according to any of [F1] to [F32], wherein the resin for solid phase synthesis contains one or more phenolic hydroxy groups and 1 or more carboxy groups in side chains as the acidic functional group, and any acidic functional group of the 1 or more phenolic hydroxy groups and 1 or more carboxy groups is alkylated.

[F34] The method according to any of [F1] to [F33], further comprising, in a resin for solid phase synthesis containing one or more phenolic hydroxy groups and one or more carboxy groups as the acidic functional group, alkylating the acidic functional group of both the one or more phenolic hydroxy groups and the one or more carboxy groups, and then converting one or more alkylated carboxy groups to carboxy groups.

[F35] The method according to any of [F1] to [F34], wherein, in a resin for solid phase synthesis containing one or

more phenolic hydroxy groups and one or more carboxy groups as the acidic functional group, the acidic functional group of both the one or more phenolic hydroxy groups and the one or more carboxy groups is alkylated, and then one or more alkylated carboxy groups are converted to carboxy groups, thereby alkylating only the one or more phenolic hydroxy groups.

[F36] The method according to any of [F1] to [F35] for production of a compound constituting a compound library.

[F37] The method according to any of [F1] to [F36], wherein the side chain has a chemical structure that binds a compound having an oxygen functional group to the resin via a linker, and the linker is degradable.

[F38] The method according to any of [F37], further comprising producing a compound containing an alkylated acidic functional group by degrading the linker by a reaction under acidic conditions, a reaction under alkaline conditions, a hydrogenation reaction, a reaction using an oxidizing agent, or a reaction with a Lewis acid.

[F39] The method according to [F37] or [F38], wherein the resin contains 10 or more compounds bonded to the resin via the linker.

[F40] The method according to any of [F1] to [F37], wherein one compound is bonded to one unit of the resin for solid phase synthesis via a linker, and a mixture of resins to which different compounds are bonded is used as the substrate.

[F41] The method according to any of [F1] to [F37], wherein a resin in which two or more different compounds are bonded to one unit of the resin for solid phase synthesis via a linker is used as the substrate.

## ADVANTAGEOUS EFFECT OF INVENTION

The present specification provides an alkylation method with high reactivity and high selectivity in which a mixture containing multiple compounds can be applied as a substrate.

## BRIEF DESCRIPTION OF DRAWINGS

Figure 1 shows the "core block diversity" and "linker diversity" for compounds constituting a library produced by the method of the present invention, and shows the compounds produced in Example 6.

Figure 2 shows the structure of 1000 compounds produced in Example 6-6.

Figure 3 shows the structure of 500 compounds produced in Examples 6-8.

## DESCRIPTION OF EMBODIMENTS

One aspect of the present invention relates to a method for producing a compound having an alkylated acidic functional group. Here, alkylation is not particularly limited, but examples thereof include alkylation that forms a $C_{1-6}$ alkoxy, specifically alkylation that forms methoxy, ethoxy, 1-propoxy, 2-propoxy, n-butoxy, i-butoxy, sec-butoxy, t-butoxy, 1-pentyloxy, 1-hexyloxy, and the like, and more specifically alkylation that forms methoxy, ethoxy, and the like.

The acidic functional group is not particularly limited as long as it is a group having an acidic proton, and examples thereof include a hydroxy group as a substituent on an aromatic ring, and a carboxylic acid. Here, the aromatic ring may be a carbocycle or a heterocycle containing a heteroatom, and may be a fused ring with an aromatic ring or a fused ring with a non-aromatic ring. As the aromatic ring, mention may be made of, for example, a 5- to 10-membered monocyclic or fused aromatic ring, and examples thereof include a pyrrole ring, a thiophene ring, a furan ring, a pyridine ring, a thiazole ring, an isothiazole ring, a pyrazole ring, an oxazole ring, an isoxazole ring, an imidazole ring, a triazole ring, a pyrimidine ring, a uridine ring, a pyrazine ring, a pyridazine ring, a quinoline ring, an isoquinoline ring, a 4H-quinolizine ring, a phthalazine ring, a naphthyridine ring, a quinoxaline ring, a quinazoline ring, a cinnoline ring, a pteridine ring, an indole ring, an indoline ring, a benzothiophene ring, a 1-methyl-1H-indole ring, a benzofuran ring, a benzisothiazole ring, a benzisoxazole ring, an indazole ring, a benzimidazole ring, a benzotriazole ring, an azaindole ring, and an imidazopyridine ring. In one aspect of the present invention, the acidic functional group is a hydroxy group as a substituent on an aromatic ring, such as a hydroxy group as a substituent on a benzene ring, a pyridine ring, an indole ring, a quinoline ring, or a pyrazole ring, and specifically, a hydroxy group as a substituent on a benzene ring or a pyridine ring.

As used herein, the "$C_{1-6}$ alkyl" is a monovalent group derived from a linear and branched saturated aliphatic hydrocarbon having 1 to 6 carbon atoms by removing one arbitrary hydrogen atom therefrom. Specific examples thereof include methyl, ethyl, n-propyl, isopropyl, n-butyl, isobutyl, sec-butyl, t-butyl, 1-methylpropyl, n-pentyl, isopentyl, 2-methylbutyl, 1,1-dimethylpropyl, 1-ethylpropyl, hexyl, 4-methylpentyl, and 2-ethylbutyl.

As used herein, the "$C_{1-4}$ alkyl" is a monovalent group derived from a linear and branched saturated aliphatic hydrocarbon having 1 to 4 carbon atoms by removing one arbitrary hydrogen atom therefrom. Specific examples thereof include methyl, ethyl, n-propyl, isopropyl, n-butyl, isobutyl, sec-butyl, t-butyl, and 1-methylpropyl.

As used herein, the "$C_{1-6}$ alkoxy" refers to a $C_{1-6}$ alkyl-O- group, where the $C_{1-6}$ alkyl is as already defined. Specific examples thereof include methoxy, ethoxy, 1-propoxy, 2-propoxy, n-butoxy, i-butoxy, sec-butoxy, t-butoxy, 1-pentyloxy, and 1-hexyloxy.

As used herein, the "$C_{1-4}$ alkoxy" refers to a $C_{1-4}$ alkyl-O- group, where the $C_{1-4}$ alkyl is as already defined. Specific examples thereof include methoxy, ethoxy, 1-propoxy, 2-propoxy, n-butoxy, i-butoxy, sec-butoxy, and t-butoxy.

As used herein, the "$C_{3-6}$ cycloalkyl" refers to a cyclic saturated aliphatic hydrocarbon group having 3 to 6 carbon atoms. Specific examples thereof include cyclopropyl, cyclobutyl, cyclopentyl, and cyclohexyl.

As used herein, the "$C_{2-7}$ alkenyl" refers to a linear or branched alkenyl group having 2 to 7 carbon atoms and includes, for example, ethenyl (vinyl), 1-propenyl, 2-propenyl (allyl), propen-2-yl, 3-butenyl (homoallyl), and the like.

As used herein, the "$C_{2-7}$ alkynyl" refers to a linear or branched alkynyl group having 2 to 7 carbon atoms and includes, for example, ethynyl, 1-propynyl, 2-propynyl, 1-butynyl, 2-butynyl, 3-butynyl, and the like.

In the present invention, the "$C_{6-10}$ aryl" refers to a monovalent aromatic hydrocarbon ring group. Examples of the $C_{6-10}$ aryl include phenyl, 1-naphthyl, and 2-naphthyl.

As used herein, the "halogen atom" refers to a fluorine atom, a chlorine atom, a bromine atom, an iodine atom, and the like. In the present invention, when a halogen atom is a substituent for an aryl, a heteroaryl, and the like, examples of the preferred halogen atom include a fluorine atom, a chlorine atom, and a bromine atom. In the present invention, when a halogen atom is a substituent for an alkyl or a group containing an alkyl as a part thereof (alkoxy, alkenyl, alkylthio, and the like), examples of the preferred halogen atom include a fluorine atom. Specific examples of the group having a halogen atom as a substituent include trifluoromethyl, pentafluoroethyl, trifluoromethoxy, pentafluoroethoxy, trifluoromethylthio, and pentafluoroethylthio.

In the definition of formula A, formula B, or formula C in the present specification, when a certain group is substituted with one or more substituents, the number of such groups may range from 1 to the number of substitutable locations and the number of substituents may be 1 to 5, 1 to 4, 1 to 3, 1 or 2, or 1, for example.

The heteroatom constituting an aromatic heterocycle in the present specification may be one or more, specifically 1 to 3, atoms selected from a nitrogen atom, an oxygen atom, and a sulfur atom, for example.

In the present invention, a compound selected from formula A, formula B, or formula C can be used as an alkylating agent. For example, a compound in which $R^2$ is - $OR^1$ in formula A can be used. Examples of $R^3$ in formula B include an aryl, specifically phenyl.

Examples of $R^1$ include a $C_{1-6}$ alkyl, specifically a $C_{1-5}$ alkyl, and more specifically a $C_{1-4}$ alkyl.

Examples of the alkylating agent represented by formula A include trimethyl phosphate, triethyl phosphate, dimethyl phenylphosphonate, diethyl phenylphosphonate, methyl diphenylphosphinate, and ethyl diphenylphosphinate, among which trimethyl phosphate, triethyl phosphate, tripropyl phosphate, triallyl phosphate, and the like are preferable.

Examples of the alkylating agent represented by formula B include trimethylphenylammonium chloride, trimethylphenylammonium bromide, trimethylphenylammonium iodide, triethylphenylammonium chloride, triethylphenylammonium bromide, triethylphenylammonium iodide, tetramethylammonium chloride, tetramethylammonium bromide, tetramethylammonium iodide, tetraethylammonium chloride, tetraethylammonium bromide, tetraethylammonium iodide, benzyltrimethylammonium chloride, benzyltrimethylammonium bromide, benzyltrimethylammonium iodide, benzyltriethylammonium chloride, benzyltriethylammonium bromide, and benzyltriethylammonium iodide, among which trimethylphenylammonium chloride, triethylphenylammonium iodide, and the like are preferable.

Examples of the alkylating agent represented by formula C include dimethyl carbonate, diethyl carbonate, di-n-propyl carbonate, diisopropyl carbonate, di-n-butyl carbonate, and di-tert-butyl carbonate, among which, dimethyl carbonate, diethyl carbonate, and the like are preferable.

In one aspect of the present invention, the production method includes removing an impurity after the alkylation. The impurity removal can be carried out by methods normally practiced in the art of the present invention. Here, examples of the impurity include an impurity derived from the reaction reagent consumed in the reaction, an unreacted reaction reagent, a decomposition product generated by the reaction, a product resulting from alkylation of the coexisting base by the alkylating agent, and a reaction solvent. In particular, specific examples of the impurity include triethyl phosphate, diethyl phosphate, tert-butylimino-tri(pyrrolidino)phosphorane (BTPP), an alkylated product of BTPP, and 1,3-dimethyl-2-imidazolidinone (DMI). Examples of the method for removing an impurity include liquid-liquid separation, distillation under reduced pressure, a method using a solid phase reagent, purification by normal phase or reversed phase silica gel column chromatography, and purification by GPC (molecular sieve).

The liquid-liquid separation for impurity removal can be carried out by methods normally practiced in the art of the present invention. The liquid-liquid separation is not particularly restricted as long as it is a combination of solvents that are separated into multiple layers for the purpose of separating the desired product group from the impurity (unwanted material) group. For example, it can be carried out by combining a solvent selected from an ester solvent such as ethyl acetate and isopropyl acetate, an ether solvent such as diethyl ether, diisopropyl ether, t-butyl methyl ether (TBME), cyclopentyl methyl ether (CPME), 2-methyltetrahydrofuran, and 4-methyltetrahydropyran, a halogenated solvent such as dichloromethane, chloroform, and 1,2-dichloroethane, an aromatic hydrocarbon solvent such as benzene and toluene,

and a hydrocarbon solvent such as hexane, cyclohexane, and heptane with a solvent selected from water, an acidic aqueous solution such as aqueous hydrochloric acid solution, and a basic aqueous solution such as aqueous sodium bicarbonate solution. A single solvent may be used, or a mixed solvent combining multiple solvents may be used. Also, the liquid-liquid separation can be carried out by combining organic solvents that will undergo layer separation, such as the combination of hexane and acetonitrile, if this meets the purpose of separating the desired product group from the impurity (unwanted material) group.

The distillation under reduced pressure for impurity removal can be carried out by methods normally practiced in the art of the present invention. The conditions for distillation under reduced pressure can be set as appropriate depending on the impurity to be removed, and for example, the pressure may be 100 to 400 mbar, 50 to 100 mbar, 5 to 50 mbar, 0.1 to 5 mbar, or the like, and the temperature may be 20 to 100°C, 25 to 50°C, 35 to 45°C, or the like.

The removal of an impurity using a solid phase reagent can be carried out by methods normally practiced in the art of the present invention. Here, examples of the solid phase reagent include macroporous triethylammonium methylpolystyrene carbonate, macroporous polystyrene sulfonic acid, amine-supported silica gel, and carboxylic acid-supported silica gel.

In one aspect of the present invention, as the base used in the alkylation reaction, a base selected from the group consisting of an organic base whose conjugate acid has a pKa of 23 to 34 in acetonitrile and an inorganic base whose conjugate acid has a pKa of 9 to 20 in water can be used. Here, the pKa of a conjugate acid of the base in acetonitrile can be measured at a measurement temperature of 25°C by the method described in the known literature J. Org. Chem. 1998, 63, 7868-7874. Also, as the pKa for a conjugate acid of an inorganic base in water, the value measured at 25°C by the method described in Experimental Chemistry Lecture, vol. 5, "Thermal Measurements and Equilibrium", pp. 460 (edited by the Chemical Society of Japan, published by Maruzen Publishing Co., Ltd.) can be used. In addition, as reference values, the calculated values using Advanced Chemistry Development (ACD/Labs) Software V11.02 ((C) 1994-2019 ACD/Labs), ADMET predictor (version 9.5), the values described in known literature Eur. J. Org. Chem. 2019, 6735-6748, Org. Biomol. Chem. 2017, 15, 4081-4085, Chem. Eur. J. 2020, Accepted Article (DOI: Chem. Eur. J 10.1002/chem.202003580, as of 2020/12/25), or the values listed in the Sigma-Aldrich catalog can be referenced as appropriate. As for the pKa values of conjugated acid in MeCN listed in the Sigma-Aldrich catalog, for example, for phosphazene bases, 1-ethyl-2,2,4,4,4-pentakis(dimethylamino)-2$\lambda^5$,4$\lambda^5$-catenadi(phosphazene) (P2Et) is 32.9, 1-tert-butyl-2,2,4,4,4-pentakis(dimethylamino)-2$\lambda^5$,4$\lambda^5$-catenadi(phosphazene) (P2tBu) is 33.5, tert-butylimino-tris(dimethylamino)phosphorane (P1tBu) is 26.9, 2-tert-butylimino-2-diethylamino-1,3-dimethylperhydro-1,3,2-diazaphospholine (BEMP) is 27.6, and tert-butylimino-tri(pyrrolidino)phosphorane (BTPP) is 28.4. Also, as for the pKa values of conjugate acid in MeCN listed in the known literature Eur. J. Org. Chem. 2019, 6735-6748, for amidine bases, 1,8-diazabicyclo[5.4.0]undec-7-ene (DBU) is 24.3, and for guanidine bases, 1,1,3,3- tetramethylguanidine (TMG) is 23.4, 7-methyl-1,5,7-triazabicyclo[4.4.0]dec-5-ene (MTBD) is 25.5, and 1,5,7-triazabicyclo[4.4.0]dec-5-ene (TBD) is 26.0. As for the pKa value for conjugate acid in MeCN listed in the known literature Chem. Eur. J. 2020, Accepted Article (DOI: Chem. Eur. J 10.1002/chem.202003580, as of 2020/12/25), 2-tert-butyl-1,1,3,3-tetramethylguanidine (BTMG) is 23.6. As for the pKa value of conjugate acid in water listed in the known literature Org. Biomol. Chem. 2017, 15, 4081-4085, $Cs_2CO_3$ is 10.3.

In one aspect of the present invention, it is preferable that the base used in the alkylation reaction have a solubility in dimethylformamide (DMF) of 120 mg/mL or more at 25°C. The solubility can be measured by methods normally practiced in the art of the present invention. Here, the base used can be, for example, an organic base. Also, the solubility of $Cs_2CO_3$ in dimethylformamide (DMF) listed in the known literature J. Org. Chem. 1984, 49, 1122-1125 is 119 mg/mL at 25°C.

In one aspect of the present invention, a phosphazene, an amidine, or a guanidine can be used as the base in the alkylation reaction. The phosphazene, amidine, or guanidine is not particularly limited as long as it can be used as the base. In one aspect of the present invention, the base used in the alkylation reaction is selected from the group consisting of 1-ethyl-2,2,4,4,4-pentakis(dimethylamino)-2$\lambda^5$,4$\lambda^5$-catenadi(phosphazene) (P2Et), 1-tert-butyl-2,2,4,4,4-pentakis(dimethylamino)-2$\lambda^5$,4$\lambda^5$-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphospholine (BEMP), and tert-butylimino-tri(pyrrolidino)phosphorane (BTPP) for the phosphazene, from 1,8-diazabicyclo[5.4.0]undec-7-ene (DBU) for the amidine, and from the group consisting of 1,1,3,3-tetramethylguanidine (TMG), 2-tert-butyl-1,1,3,3-tetramethylguanidine (BTMG), 7-methyl-1,5,7-triazabicyclo[4.4.0]dec-5-ene (MTBD), and 1,5,7-triazabicyclo[4.4.0]dec-5-ene (TBD) for the guanidine.

The alkylation reaction using the alkylating agent of formula A can be performed selecting conditions as appropriate based on the description in the present specification. In one aspect of the present invention, for example, the alkylation reaction using the alkylating agent of formula A can be carried out in a solvent (a single solvent may be used, or a mixed solvent combining multiple solvents may be used) selected from an ether solvent such as tetrahydrofuran (THF), 2-methyltetrahydrofuran, 4-methyltetrahydropyran, 1,4-dioxane, 1,2-dimethoxyethane (DME), t-butyl methyl ether (TBME), cyclopentyl methyl ether (CPME), and isosorbide dimethyl ether, an ester solvent such as ethyl acetate, propyl

acetate, isopropyl acetate, butyl acetate, isobutyl acetate, and GVL (γ-valerolactone), a halogenated solvent such as dichloromethane (DCM), 1,2-dichloroethane (DCE), and chloroform, an amide solvent such as N,N-dimethylformamide (DMF), N-methyl-2-pyrrolidone (NMP), N,N-dimethylacetamide (DMA), N-ethyl-2-pyrrolidone (NEP), and N-butyl-2-pyrrolidone (NBP), a urea solvent such as N,N,N',N'-tetramethylurea (TMU), N,N'-dimethylpropyleneurea (DMPU), and 1,3-dimethyl-2-imidazolidinone (DMI), a sulfoxide solvent such as dimethyl sulfoxide (DMSO), and an aromatic solvent such as anisole, toluene, α,α,α-trifluorotoluene, 1,2-dichlorobenzene, and benzene, at a temperature of 40 to 120°C, 60 to 100°C, or 75 to 85°C, and using a base selected from 1-ethyl-2,2,4,4,4-pentakis(dimethylamino)-2$\lambda^5$,4$\lambda^5$-catenadi(phosphazene) (P2Et), 1-tert-butyl-2,2,4,4,4-pentakis(dimethylamino)-2$\lambda^5$,4$\lambda^5$-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphospholine (BEMP), tert-butylimino-tri(pyrrolidino)phosphorane (BTPP), tert-butylimino-tri(pyrrolidino)phosphorane (BTPP), 1,1,3,3-tetramethylguanidine (TMG), 2-tert-butyl-1,1,3,3-tetramethylguanidine (BTMG), 1,8-diazabicyclo[5.4.0]undec-7-ene (DBU), 7-methyl-1,5,7-triazabicyclo[4.4.0]dec-5-ene (MTBD), 1,5,7-triazabicyclo[4.4.0]dec-5-ene (TBD), and cesium carbonate ($Cs_2CO_3$).

The alkylation reaction using the alkylating agent of formula B can be performed selecting conditions as appropriate based on the description in the present specification. In one aspect of the present invention, for example, the alkylation reaction using the alkylating agent of formula B can be carried out in a solvent (a single solvent may be used, or a mixed solvent combining multiple solvents may be used) selected from an ether solvent such as tetrahydrofuran (THF), 2-methyltetrahydrofuran, 4-methyltetrahydropyran, 1,4-dioxane, 1,2-dimethoxyethane (DME), t-butyl methyl ether (TBME), cyclopentyl methyl ether (CPME), and isosorbide dimethyl ether, an ester solvent such as ethyl acetate, propyl acetate, isopropyl acetate, butyl acetate, isobutyl acetate, and GVL (γ-valerolactone), a halogenated solvent such as dichloromethane (DCM), 1,2-dichloroethane (DCE), and chloroform, an amide solvent such as N,N-dimethylformamide (DMF), N-methyl-2-pyrrolidone (NMP), N,N-dimethylacetamide (DMA), N-ethyl-2-pyrrolidone (NEP), and N-butyl-2-pyrrolidone (NBP), a urea solvent such as N,N,N',N'-tetramethylurea (TMU), N,N'-dimethylpropyleneurea (DMPU), and 1,3-dimethyl-2-imidazolidinone (DMI), a sulfoxide solvent such as dimethyl sulfoxide (DMSO), and an aromatic solvent such as anisole, toluene, α,α,α-trifluorotoluene, 1,2-dichlorobenzene, and benzene, at a temperature of 40 to 120°C, 60 to 100°C, or 75 to 85°C, and using a base selected from 1-ethyl-2,2,4,4,4-pentakis(dimethylamino)-2$\lambda^5$,4$\lambda^5$-catenadi(phosphazene) (P2Et), 1-tert-butyl-2,2,4,4,4-pentakis(dimethylamino)-2$\lambda^5$,4$\lambda^5$-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphospholine (BEMP), tert-butylimino-tri(pyrrolidino)phosphorane (BTPP), tert-butylimino-tri(pyrrolidino)phosphorane (BTPP), 1,1,3,3-tetramethylguanidine (TMG), 2-tert-butyl-1,1,3,3-tetramethylguanidine (BTMG), 1,8-diazabicyclo[5.4.0]undec-7-ene (DBU), 7-methyl-1,5,7-triazabicyclo[4.4.0]dec-5-ene (MTBD), 1,5,7-triazabicyclo[4.4.0]dec-5-ene (TBD), and cesium carbonate ($Cs_2CO_3$).

The alkylation reaction using the alkylating agent of formula C can be performed selecting conditions as appropriate based on the description in the present specification. In one aspect of the present invention, for example, the alkylation reaction using the alkylating agent of formula C can be carried out in a solvent (a single solvent may be used, or a mixed solvent combining multiple solvents may be used) selected from an ether solvent such as tetrahydrofuran (THF), 2-methyltetrahydrofuran, 4-methyltetrahydropyran, 1,4-dioxane, 1,2-dimethoxyethane (DME), t-butyl methyl ether (TBME), cyclopentyl methyl ether (CPME), and isosorbide dimethyl ether, an ester solvent such as ethyl acetate, propyl acetate, isopropyl acetate, butyl acetate, isobutyl acetate, and GVL (γ-valerolactone), a halogenated solvent such as dichloromethane (DCM), 1,2-dichloroethane (DCE), and chloroform, an amide solvent such as N,N-dimethylformamide (DMF), N-methyl-2-pyrrolidone (NMP), N,N-dimethylacetamide (DMA), N-ethyl-2-pyrrolidone (NEP), and N-butyl-2-pyrrolidone (NBP), a urea solvent such as N,N,N',N'-tetramethylurea (TMU), N,N'-dimethylpropyleneurea (DMPU), and 1,3-dimethyl-2-imidazolidinone (DMI), a sulfoxide solvent such as dimethyl sulfoxide (DMSO), and an aromatic solvent such as anisole, toluene, α,α,α-trifluorotoluene, 1,2-dichlorobenzene, and benzene, at a temperature of 40 to 120°C, 60 to 100°C, or 75 to 85°C, and using a base selected from 1-ethyl-2,2,4,4,4-pentakis(dimethylamino)-2$\lambda^5$,4$\lambda^5$-catenadi(phosphazene) (P2Et), 1-tert-butyl-2,2,4,4,4-pentakis(dimethylamino)-2$\lambda^5$,4$\lambda^5$-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphospholine (BEMP), tert-butylimino-tri(pyrrolidino)phosphorane (BTPP), tert-butylimino-tri(pyrrolidino)phosphorane (BTPP), 1,1,3,3-tetramethylguanidine (TMG), 2-tert-butyl-1,1,3,3-tetramethylguanidine (BTMG), 1,8-diazabicyclo[5.4.0]undec-7-ene (DBU), 7-methyl-1,5,7-triazabicyclo[4.4.0]dec-5-ene (MTBD), 1,5,7-triazabicyclo[4.4.0]dec-5-ene (TBD), and cesium carbonate ($Cs_2CO_3$).

In one aspect of the present invention, it is possible to selectively alkylate a phenolic hydroxy group in a substrate in which a nitrogen atom that can be alkylated is present, obtaining the target compound at a high yield and suppressing the generation of byproducts. In one aspect, a substrate contains one or more compounds containing a phenolic hydroxy group as an acidic functional group and a nitrogen atom that can be alkylated, and in the compound having a nitrogen atom in which alkylation proceeds most among the substrate, the production ratio of products by alkylation of the phenolic hydroxy group (alkylation A) and alkylation of the nitrogen atom (alkylation C), (alkylation A/alkylation C), is 1.5 or more, 3 or more, 5 or more, 7 or more, 9 or more, 10 or more, 12 or more, 15 or more, 20 or more, 30 or more, or 50 or more.

Here, the production ratio can be determined by quantitative analysis of the respective production amounts of the product by alkylation A and the product by alkylation C using a normally practiced analysis method, such as HPLC. The nitrogen atom that can be alkylated is not particularly limited as long as it is a nitrogen atom on which alkylation proceeds under the alkylation reaction conditions of the present invention, and examples thereof include a ring nitrogen atom of a pyridine ring, a nitrogen atom of a tertiary amine, a nitrogen atom of an acylsulfonamide, a nitrogen atom of a diarylamine, a nitrogen atom of an alkylarylamine, a nitrogen atom of an aniline, a nitrogen atom of an amide, and a nitrogen atom of a sulfonamide. In particular, it has been confirmed that alkylation of the ring nitrogen atom of a pyridine ring generates byproducts, and in one aspect of the present invention, alkylation C is alkylation of the ring nitrogen atom of a pyridine ring. The production ratio of alkylation A and alkylation C can be confirmed at the completion of the reaction, and for example, the production ratio can be measured at the point when the raw material of alkylation A having a phenolic hydroxy group disappears.

Hereinafter, the present invention will be described in more detail using Reference Examples and Examples, but the present invention is not limited to these Examples. Note that, when described as "phenol" in the present specification, in addition to phenol itself, it may also mean a compound having an arbitrary substituent at an arbitrary location on the phenol skeleton. Furthermore, when described as "pyridinol" in the present specification, it means a compound having a hydroxy group on an arbitrary carbon atom of the pyridine ring, and the compound may have an arbitrary substituent at an arbitrary location.

## Examples

All starting materials, reagents, and solvents were obtained from commercial suppliers, or synthesized using known methods. The reagents and solvents were of reagent quality or better and were used as obtained from various commercial sources, unless otherwise noted.

HPLC purification of compounds was carried out using UV-Direcred AutoPurification System, MS-Direcred AutoPurification System (manufactured by Waters Corporation, 2545, 2545-SQD2 & 2545-SQD2-ELSD), Trilution (manufactured by Gilson, Inc.), or the like.

For the column chromatography silica gel, SHOKO Scientific Purif-Pack (R) SI (60 μm), SHOKO Purif-Pack (R)-EX SI (50 μm), Biotage (R) SNAP Cartridge KP-Sil, Biotage (R) SNAP Ultra, Biotage (R) Sfaer D (Duo) (60 μm), Biotage (R) Sfaer HC D (Duo) (20 μm), or the like was used as appropriate.

For the column chromatography amino silica gel, SHOKO Purif-Pack (R)-EX NH (50 μm), Biotage (R) SNAP Isolute NH2 (50 μm), Biotage (R) SNAP Cartridge KP-NH, or the like was used as appropriate.

For the column chromatography reversed phase silica gel, SHOKO Purif-Pack (R)-EX ODS (50 μm), Biotage (R) SNAP Ultra C18 (25 μm), Biotage (R) Sfaer C18 (30 μm), or the like was used as appropriate.

The $^{1}H$-NMR and $^{13}C$-NMR spectra were analyzed using or not using $Me_4Si$ as the internal standard substance, and using ECP-400 (manufactured by JEOL Ltd.), Agilent 400-MR (manufactured by Agilent Technologies), AVANCE3 Cryo-TCI, AVANCE3 400, AVANCE3 HD 400, AVANCE NEO 400, AVANCE3 HD 300, AVANCE3 300, AVANCE2 300, AVANCE NEO 300 (manufactured by Bruker), or the like as appropriate (s = singlet, brs = broad singlet, d = doublet, t = triplet, q = quartet, dd = double doublet, ddd = double double doublet, dt = double triplet, td = triple doublet, and m = multiplet).

The reaction tracking and purity measurement were carried out by performing retention time measurement and mass spectrometry using a mass spectrometer, SQD (manufactured by Waters Corporation) or 2020 (manufactured by Shimadzu Corporation), under the analysis conditions shown in the following tables, unless otherwise noted.

In Examples, the following abbreviations were used.

[Table 1]

| | |
|---|---|
| AC | Ammonium bicarbonate |
| AcOH | Acetic acid |
| area% | UV area% |
| AS-MS | Affinity selection mass spectrometry |
| BEMP | 2-tert-Butylimino-2-diethylamino-1,3-dimethylperhydro-1,3,2-diazaphospholine CAS: 98015-45-3 |
| BINAP | 2,2'-Bis(diphenylphosphino)-1,1'-binaphthyl CAS: 98327-87-8 |
| Boc | tert-Butoxycarbonyl group |
| brine | Saturated saline solution |

(continued)

| | |
|---|---|
| BTPP | tert-Butylimino-tri(pyrrolidino)phosphorane or Phosphazene base P1-t-Bu-tris(tetramethylene) CAS: 161118-67-8 |
| $CH_3CN$ | Acetonitrile |
| $Cs_2CO_3$ | Cesium carbonate |
| CsF | Cesium fluoride CAS: 13400-13-0 |
| CuI | Copper iodide |
| DCE | 1,2-Dichloroethane |
| DCM | Dichloromethane |
| DHP | 3,4-Dihydro-2H-pyran CAS: 110-87-2 |
| DIC | N,N'-Diisopropylcarbodiimide |
| Dioxane | 1,4-Dioxane |
| DIPEA | N,N-Diisopropylethylamine |
| DMAP | N,N-Dimethyl-4-aminopyridine |
| DME | 1,2-Dimethoxyethane |
| DMF | N,N-Dimethylformamide |
| DMI | 1,3-Dimethyl-2-imidazolidinone |
| DMSO | Dimethyl sulfoxide |
| EDCI·HCl | 1-Ethyl-3-(3-dimethylaminopropyl)carbodiimide hydrochloride |
| EtOAc | Ethyl acetate |
| EtOH | Ethanol |
| $Et_3PO_4$ | Triethyl phosphate |
| FA | Formic acid |
| HATU | O-(7-Aza-1H-benzotriazol-1-yl)-N,N,N',N'-tetramethyluronium hexafluorophosphate |
| HCl | Hydrochloric acid |
| $H_2O$ | Water |
| HOAt | 1-Hydroxybenzotriazole |
| $K_2CO_3$ | Potassium carbonate |
| KOtBu | Potassium tert-butoxide |
| $K_3PO_4$ | Tripotassium phosphate |
| $K_3PO_4$-$H_2O$ | Tripotassium phosphate monohydrate |
| LiOH | Lithium hydroxide |
| MeCN | Acetonitrile |
| MeOH | Methanol |
| 4-MeTHP | 4-Methyltetrahydropyran |
| $NaBH_3CN$ | Sodium cyanoborohydride |
| $NaBH(OAc)_3$ | Sodium triacetoxyborohydride |
| NAC | N-Acetyl-L-cysteine |
| $Na_2CO_3$ | Sodium carbonate |
| NaH | Sodium hydride |

(continued)

| | |
|---|---|
| $NaHCO_3$ | Sodium bicarbonate |
| NaOH | Sodium hydroxide |
| NaOtBu | Sodium tert-butoxide |
| $Na_2SO_4$ | Sodium sulfate |
| $NH_4Cl$ | Ammonium chloride |
| $NH_4HCO_3$ | Ammonium bicarbonate |
| NMP | N-Methyl-2-pyrrolidone |
| $P(Cy)_3Pd$ G3 | Tricyclohexylphosphine palladium 3rd generation precatalyst |
| $[PdCl(allyl)]_2$ | Allylpalladium(II) chloride dimer CAS: 12012-95-2 |
| $PdCl_2(PPh3)_2$ | Bis(triphenylphosphine)palladium(II) dichloride CAS: 13965-03-2 |
| $Pd_2(dba)_3$-$CHCl_3$ | Tris(dibenzylideneacetone)dipalladium(0) (chloroform adduct) CAS: 52522-40-4 |
| Pd(dppf)$Cl_2$·DCM | [1,1'-Bis(diphenylphosphino)ferrocene]dichloropalladium(II) dichloromethane complex (1:1) CAS: 95464-05-4 |
| Pd(dppf)$Cl_2$ | 1,1'-Bis(diphenylphosphino)ferrocene dichloropalladium(II) CAS:72287-26-4 |
| $Pd(PPh_3)_4$ | Tetraxtriphenylphosphine palladium(0) |
| pet ether | Petroleum ether |
| P2Et | Tetramethyl(tris(dimethylamino)phosphoranylidene)phosphoric triamide-ethyl-imine or 1-Ethyl-2,2,4,4,4-pentakis(dimethylamino)-2$\lambda^5$,4$\lambda^5$-catenadi(phosphazene) or Phosphazene base P2Et CAS: 165535-45-5 |
| $pKBH^+$ | pKa of conjugated acid of base |
| $(PMB)_2NH$ | Bis(4-methoxybenzyl)amine CAS: 17061-62-0 |
| $PPh_3$ | Triphenylphosphine |
| PPTS | Pyridinium p-toluenesulfonate |
| PltBu | tert-Butylimino-tris(dimethylamino)phosphorane CAS: 81675-81-2 |
| P2tBu | 1-tert-Butyl-2,2,4,4,4-pentakis(dimethylamino)-2$\lambda^5$,4$\lambda^5$-catenadi(phosphazene) or Phosphazene base P2-t-Bu CAS: 111324-03-9 |
| Rochelle salt | Potassium sodium tartrate tetrahydrate |
| $tBu_3P$ | Tri-tert-butylphosphine CAS: 13716-12-6 |
| TEA | Triethylamine |
| Temp. | Temperature |
| TFA | Trifluoroacetic acid |
| THF | Tetrahydrofuran |
| THP | Tetrahydropyranyl group |
| $Ti(OtBu)_4$ | Tetra-tert-butyl orthotitanate CAS:3087-39-6 |
| TMAF | Tetramethylammonium fluoride CAS: 373-68-2 |
| TMS | Trimethylsilyl group |
| XantphosPd G4 | Xantphos palladium 4th generation precatalyst CAS: 1621274-19-8 |
| Xphos Pd G4 | (2-Dicyclohexylphosphino-2',4',6'-triisopropyl-1,1'-biphenyl)[2-(2'-amino-1,1'-biphenyl) palladium 4th generation precatalyst CAS: 1599466-81-5 |

The LCMS analysis conditions are described in Table LC01, Table LC02, and Table LC03.

[Table 2]

[Table LC01]

| | Analysis method | Apparatus | Column | Mobile phase | | Gradient | note | | |
|---|---|---|---|---|---|---|---|---|---|
| | | | | A | B | | Wavelength | Temp (°C) | Flow rate (mL/min) |
| FA | PH-FA-01 | Shimadzu-2020 | HALO 2.0 um C18 90A 3.0 mm I.D.x30 mm L 2.0 um | 0.1% FA $H_2O$ | 0.1% FA MeCN | A/B = 95/5 to 5/95 (1.10 min) → 5/95 to 5/95 (0.5 min) → 5/95 to 95/5 (0.1 min) | (PDA total) 190 ~ 400 nm | 40 | 1.5 |
| | PH-FA-02 | Shimadzu-2020 | Ascentis Express C18 2.1 mm I.D. x 50 mm L 2.7 um | 0.1% FA $H_2O$ | 0.1% FA MeCN | A/B = 95/5 to 0/100 (1.0 min) → 0/100 to 0/100 (0.5 min) → 0/100 to 95/5 (0.1 min) | (PDA total) 190 ~ 400 nm | 40 | 1.0 |
| | PH-FA-03 | Shimadzu-2020 | Ascentis Express C18 2.1 mm I.D. x 50 mm L 2.7 um | 0.1% FA $H_2O$ | 0.1% FA MeCN | A/B = 95/5 to 5/95 (2.0 min) → 5/95 to 5/95 (0.7 min) → 5/95 to 95/5 (0.1 min) | (PDA total) 190 ~ 400 nm | 40 | 1.0 |
| | PH-FA-04 | Shimadzu-2020 | YMC-TriartC18 3.0 mm I.D. x 50 mm L 2.5 um | 0.1% FA $H_2O$ | 0.1% FA MeCN | A/B = 95/5 to 5/95 (1.1 min) → 5/95 to 5/95 (0.6 min) → 5/95 to 95/5 (0.05 min) | (PDA total) 190 ~ 400 nm | 40 | 1.2 |
| | PH-FA-05 | Shimadzu-2020 | Halo C18 2.1 mm I.D.x50 mm L 2.0 um | 0.1% FA $H_2O$ | 0.1% FA MeCN | A/B = 95/5 to 0/100 (0.7 min) → 0/100 to 0/100 (0.4 min) → 0/100 to 95/5 (0.1 min) | (PDA total) 190 ~ 400 nm | 40 | 1.0 |
| | PH-FA-06 | Shimadzu-2020 | Halo C18 2.1 mm I.D.x30 mm L 2.0 um | 0.1% FA $H_2O$ | 0.1% FA MeCN | A/B = 95/5 to 0/100 (1.2 min) → 0/100 to 0/100 (0.6 min) → 0/100 to 95/5 (0.1 min) | (PDA total) 190 ~ 400 nm | 40 | 1.0 |
| | PH-FA-07 | Shimadzu-2020 | HALO C18 90A 3.0 mm I.D.x30 mm L 2.0 um | 0.1% FA $H_2O$ | 0.1% FA MeCN | A/B = 95/5 to 0/100 (1.0 min) → 0/100 to 0/100 (0.5 min) → 0/100 to 95/5 (0.1 min) | (PDA total) 190 ~ 400 nm | 40 | 1.5 |
| TFA | PH-TFA-01 | Shimadzu-2020 | Shim-pack XR ODS-C18 3.0 mm I.D.x50 mm L 2.2 um | 0.05% TFA $H_2O$ | 0.05% TFA MeCN | A/B = 95/5 to 5/95 (1.1 min) → 5/95 to 5/95 (0.6 min) → 5/95 to 95/5 (0.05 min) | (PDA total) 190 ~ 400 nm | 40 | 1.2 |
| | PH-TFA-02 | Shimadzu-2020 | Shim-pack XR ODS-C18 3.0 mm I.D.x50 mm L 2.2 um | 0.05% TFA $H_2O$ | 0.05% TFA MeCN | A/B = 95/5 to 5/95 (1.0 min) → 5/95 to 5/95 (0.55 min) → 5/95 to 95/5 (0.10 min) | (PDA total) 190 ~ 400 nm | 40 | 1.2 |
| | PH-TFA-03 | Shimadzu-2020 | Shim-pack XR ODS-C18 3.0 mm I.D.x50 mm L 2.2 um | 0.05% TFA $H_2O$ | 0.05% TFA MeCN | A/B = 95/5 to 5/95 (2.0 min) → 5/95 to 5/95 (0.7 min) → 5/95 to 95/5 (0.05 min) | (PDA total) 190 ~ 400 nm | 40 | 1.2 |
| | PH-TFA-04 | Shimadzu-2020 | Shim-pack XR ODS-C18 3.0 mm I.D.x50 mm L 2.2 um | 0.05% TFA $H_2O$ | 0.05% TFA MeCN | A/B = 95/5 to 30/70 (3.0 min) → 30/70 to 5/95 (0.3 min) → 5/95 to 5/95 (0.45 min) → 5/95 to 95/5 (0.05min) | (PDA total) 190 ~ 400 nm | 40 | 1.2 |

[Table 3]

| | | | | | | | | | |
|---|---|---|---|---|---|---|---|---|---|
| TFA | PH-TFA-05 | Shimadzu-2020 | Shim-pack XR ODS-C18 3.0 mm I.D.x50 mm L 2.2 um | 0.05% TFA $H_2O$ | 0.05% TFA MeCN | A/B = 95/5 to 40/60 (4.0 min) → 40/60 to 5/95 (0.3 min) → 5/95 to 5/95 (0.45 min) → 5/95 to 95/5 (0.05min) | (PDA total) 190 ~ 400 nm | 40 | 1.2 |
| | PH-TFA-06 | Shimadzu-2020 | Shim-pack XR ODS-C18 3.0 mm I.D.x50 mm L 2.2 um | 0.05% TFA $H_2O$ | 0.05% TFA MeCN | A/B = 70/30 to 30/70 (3.6 min) → 30/70 to 5/95 (0.4 min) → 5/95 to 5/95 (0.6 min) → 5/95 to 95/5 (0.15min) | (PDA total) 190 ~ 400 nm | 40 | 1.2 |
| | PH-TFA-07 | Shimadzu-2020 | Shim-pack XR ODS-C18 3.0 mm I.D.x50 mm L 2.2 um | 0.05% TFA $H_2O$ | 0.05% TFA MeCN | A/B = 70/30 to 30/70 (3.8 min) → 30/70 to 0/100 (0.3 min) → 0/100 to 0/100 (0.5 min) → 0/100 to 95/5 (0.1 min) | (PDA total) 190 ~ 400 nm | 40 | 1.2 |
| | PH-TFA-08 | Shimadzu-2020 | Shim-pack XR ODS-C18 3.0 mm I.D.x50 mm L 2.2 um | 0.05% TFA $H_2O$ | 0.05% TFA MeCN | A/B = 70/30 to 5/95 (2.2 min) → 5/95 to 5/95 (0.5 min) → 5/95 to 95/5 (0.05 min) | (PDA total) 190 ~ 400 nm | 40 | 1.2 |
| | PH-TFA-09 | Shimadzu-2020 | Halo C18 2.1 mm I.D.x30 mm L 2.0 um | 0.05% TFA $H_2O$ | 0.05% TFA MeCN | A/B = 95/5 to 0/100 (0.7 min) → 0/100 to 0/100(0.25 min) → 0/100 to 95/5 (0.03 min) | (PDA total) 190 ~ 400 nm | 40 | 0.8 |
| | PH-TFA-10 | Shimadzu-2020 | Ascentis Express C18 3.0 mm I.D.x50 mm L 2.7 um | 0.05% TFA $H_2O$ | 0.05% TFA MeCN | A/B = 95/5 to 0/100 (1.1 min) → 0/100 to 0/100(0.5 min) → 0/100 to 95/5 (0.1 min) | (PDA total) 190 ~ 400 nm | 40 | 1.0 |
| | PH-TFA-11 | Shimadzu-2020 | Ascentis Express C18 3.0 mm I.D.x50 mm L 2.7 um | 0.05% TFA $H_2O$ | 0.05% TFA MeCN | A/B = 95/5 to 5/95 (1.1 min) → 5/95 to 5/95 (0.6 min) → 95/5 to 5/95 (0.05 min) | (PDA total) 190 ~ 400 nm | 40 | 1.0 |
| | PH-TFA-12 | Shimadzu-2020 | Ascentis Express C18 3.0 mm I.D.x50 mm L 2.7 um | 0.05% TFA $H_2O$ | 0.05% TFA MeCN | A/B = 95/5 to 5/95 (1.1 min) → 5/95 to 5/95 (0.5 min) → 95/5 to 5/95 (0.05 min) | (PDA total) 190 ~ 400 nm | 40 | 1.0 |
| | PH-TFA-13 | Shimadzu-2020 | HALO C18 90A 3.0 mm I.D.x30 mm L 2.0 um | 0.05% TFA $H_2O$ | 0.05% TFA MeCN | A/B = 95/5 to 0/100 (1.1 min) → 0/100 to 0/100(0.6 min) → 0/100 to 95/5 (0.1 min) | (PDA total) 190 ~ 400 nm | 40 | 1.3 |
| AC | PH-AC-01 | Shimadzu-2020 | Kinetex EVO C18 2.1 mm I.D.x 50 mm L 2.6 um | $H_2O$ + 6.5 mM $NH_4HCO_3$ pH10- | MeCN | A/B = 90/10 to 5/95 (2.0 min) → 5/95 to 5/95 (0.7 min) → 5/95 to 90/10 (0.1 min) | (PDA total) 190 ~ 400 nm | 35 | 1.0 |
| | PH-AC-02 | Shimadzu-2020 | Kinetex EVO C18 2.1 mm I.D. x 50 mm L 2.6 um | $H_2O$ + 6.5 mM $NH_4HCO_3$ pH10- | MeCN | A/B = 90/10 to 40/60 (3.0 min) → 40/60 to 5/95 (0.3 min) → 5/95 to 5/95 (0.45 min) → 5/95 to 90/10 (0.05 min) | (PDA total) 190 ~ 400 nm | 35 | 1.0 |
| | PH-AC-03 | Shimadzu-2020 | Kinetex EVO 3.0 mm I.D.x 50 mm L 2.6 um | $H_2O$ + 5 mM $NH_4HCO_3$ | MeCN | A/B = 90/10 to 5/95 (1.10 min) → 5/95 to 5/95 (0.7 min) → 5/95 to 90/10 (0.05 min) | (PDA total) 190 ~ 400 nm | 40 | 1.0 |

[Table 4]

[Table LC02]

| Analysis method name | Apparatus | Column | Mobile phase A | Mobile phase B | Gradient | Flow rate | Wavelength |
|---|---|---|---|---|---|---|---|
| SMD−FAO 5−1 | nexera/2020 | Ascentis Express C18 2.1mmI.D.x50mm,2.7um | 0.1% FA $H_2O$ | 0.1% FA MeCN | A/B = 95/5→0/100(1.5min) → 0/100(0.5min) | 1 mL/min | (PDA total) 210 ~ 400 nm |
| SMD−FA05−long25min | nexera/2020 | Ascentis Express C18 2.1mmI.D.x150mm,2.7um | 0.1% FA $H_2O$ | 0.1% FA MeCN | A/B = 95/5→95/5(1min)→ 0/100(15min)→0/100(4min) | 0.5mL/min | (PDA total) 210 ~ 400 nm |
| SMD−FA05−long25min | nexera/2020 | Ascentis Express C18 2.1mmI.D.x150mm,2.7um | 0.1% FA $H_2O$ | 0.1% FA MeCN | A/B = 95/5→0/100(15min)→ 0/100(5min) | 0.5mL/min | (PDA total) 210 ~ 400 nm |
| SMD−FAO 5−RP | nexera/2020 | Ascentis Express RP-Amide 2.1mmI.D.x50mm,2.7um | 0.1% FA $H_2O$ | 0.1% FA MeCN | A/B = 95/5→0/100(1.5min)→ 0/100(0.5min) | 1 mL/min | (PDA total) 210 ~ 400 nm |
| SMD−ACO 5−1 | nexera/2020 | Ascentis Express C18 2.1mmI.D.x50mm,2.7um | 10 mM $NH_4HCO_3$ $H_2O$ | MeCN | A/B = 95/5→5/95(1.5min)→ 5/95(0.5min) | 1 mL/min | (PDA total) 210 ~ 400 nm |
| SMD−TFA 05−long | nexera/2020 | Ascentis Express RP-Amide 2.1mmI.D.x50mm,2.7um | 0.05%TFA H2O | 0.05%TFA MeCN | A/B = 95/5→0/100(4.5min)→ 0/100(0.5min) | 1 mL/min | (PDA total) 210 ~ 400 nm |
| SQD−FAO 5−2 | Acquity UPLC/SQD 2 | Ascentis Express C18 2.1mmI.D.x50mm,2.7um | 0.1% FA $H_2O$ | 0.1% FA MeCN | A/B = 95/5→0/100(1.0min)→ 0/100(0.4min) | 1 mL/min | (PDA total) 210 ~ 400 nm |

[Table 5]

[Table LC03]

| | Analysis method | Apparatus | Column | Mobile phase A | Mobile phase B | Gradient | note Wavelength | note Temp (°C) | note Flow rate (mL/min) |
|---|---|---|---|---|---|---|---|---|---|
| FA | PH-FA-08 | Shimadzu-2020 | HALO C18 90A 3.0 mm I.D.x30 mm L 2.0 um | 0.1% FA $H_2O$ | 0.1% FA MeCN | A/B = 95/5 to 5/95 (1.1 min) → 5/95 to 5/95 (0.6 min) → 5/95 to 95/5 (0.05 min) | (PDA total) 190 ~ 400 nm | 40 | 1.5 |
| TFA | PH-TFA-14 | Shimadzu-2020 | Shim-pack XR ODS-C18 3.0 mm I.D.x 50 mm L 2.2 um | 0.05% TFA $H_2O$ | 0.05% TFA MeCN | A/B = 95/5 to 0/100 (1.1 min) → 0/100 to 0/100(0.6 min) → 0/100 to 95/5 (0.05 min) | (PDA total) 190 ~ 400 nm | 40 | 1.2 |
| TFA | PH-TFA-15 | Shimadzu-2020 | Shim-pack XR ODS-C18 3.0 mm I.D.x 50 mm L 2.2 um | 0.05% TFA $H_2O$ | 0.05% TFA MeCN | A/B = 95/5 to 0/100 (2.0 min) → 0/100 to 0/100(0.7 min) → 0/100 to 95/5 (0.05 min) | (PDA total) 190 ~ 400 nm | 40 | 1.2 |
| TFA | PH-TFA-16 | Shimadzu-2020 | HALO C18 90A 3.0 mm I.D.x30 mm L 2.0 um | 0.05% TFA $H_2O$ | 0.05% TFA MeCN | A/B = 70/30 to 30/70 (1.7 min) → 30/70 to 0/100 (0.8 min) → 0/100 to 0/100 (0.5 min) → 0/100 to 95/5 (0.03 min) | (PDA total) 190 ~ 400 nm | 40 | 1.2 |
| TFA | PH-TFA-17 | Shimadzu-2020 | HALO C18 90A 3.0 mm I.D.x30 mm L 2.0 um | 0.05% TFA $H_2O$ | 0.05% TFA MeCN | A/B = 80/20 to 40/60 (1.7 min) → 40/60 to 0/100 (0.8 min) → 0/100 to 0/100 (0.5 min) → 0/100 to 95/5 (0.03 min) | (PDA total) 190 ~ 400 nm | 40 | 1.2 |
| AC | PH-AC-04 | Shimadzu-2020 | Poroshell HPH-C18 3.0 mm I.D.x 50 mm L 2.7 um | $H_2O$ + 5 mM $NH_4HCO_3$ | MeCN | A/B = 90/10 to 5/95 (1.20 min) → 5/95 to 5/95 (0.5 min) → 5/95 to 90/10 (0.05 min) | (PDA total) 190 ~ 400 nm | 40 | 1.2 |

The descriptions of m/z $[M+H]^+$ and $(M+H)^+$ denoted in the LCMS analysis results in Examples all refer to values detected in positive mode, unless otherwise noted. Also, the UV area% in LCMS indicated values with PDA (190 to 400 nm or 210 to 400 nm), unless otherwise noted. When a specific wavelength (for example, 290 nm) is described, the UV area% at wavelengths up to +/- 4 nm centered on the described wavelength was described.

The expressions "concentration", "reduced pressure concentration", or "concentration under reduced pressure" refer to evaporation and removal of a solvent under reduced pressure with a rotary evaporator, a mechanical oil vacuum pump, or a mechanical oil-free vacuum pump.

For the expression "solvent is distilled off under reduced pressure with Genevac" or "distilled off", or for multiple samples at the same time, evaporation and removal of a solvent using a centrifugal evaporator system (manufactured by Genevac Ltd., EZ-2 Elite, HT-6, or HT-12) is indicated.

The expression "drying overnight under reduced pressure" refers to evaporation and removal of a solvent under reduced pressure with a rotary evaporator, a mechanical oil vacuum pump, or a mechanical oil-free vacuum pump.

The expressions "overnight" and "all night" refer to but are not limited to about 8 to 14 hours, unless otherwise noted.

The solid phase reaction can be performed in an arbitrary appropriate container, such as a glass vial that can be tightly sealed by a cap equipped with a Teflon packing or a column having a frit filter and an appropriate stopper. The container size is selected as appropriate such that there is sufficient space for the solvent and sufficient room for the resin to be effectively stirred, taking into account that certain resins may be significantly swollen when treated with organic solvents.

Stirring in the solid phase reaction was performed at 50 to 200 rpm using an appropriate shaker (for example, Tokyo Rikakikai Co., Ltd., EYELA, MMS-320, MMS-220H, or Asone, MyBL-100CS, or TAITEC, M·BR-104) as appropriate in order to ensure adequate mixing, which is a factor generally accepted as important for successful reaction on the resin.

When the substrate is a mixture in the solid phase reaction, the equivalent of reagents represents how many times (how many equivalents) the number of moles of reagent is used relative to the sum of the numbers of moles of functional groups used in the reaction that each compound in the mixture on the solid phase side has. For example, when described as 10 equivalents, it represents the use of 10 times (10 equivalents) the number of moles of reagent

relative to the sum of the numbers of moles of functional groups used in the reaction that each compound in the mixture on the solid phase side has.

Also, when the substrate is a mixture in the liquid phase reaction, the equivalent of reagents represents how many times (how many equivalents) the number of moles of reagent is used relative to the sum of the numbers of moles of functional groups used in the reaction that each compound in the mixture has. For example, when described as 10 equivalents, it represents the use of 10 times (10 equivalents) the number of moles of reagent relative to the sum of the numbers of moles of functional groups used in the reaction that each compound in the mixture has.

Note that, as for the number of moles of the mixture, the following cases may be considered, for example. Unless otherwise noted, any of the following values may be employed. In the case where individual compounds with known numbers of moles are mixed, the sum thereof may be considered as the number of moles of the mixture. Also, in the case where a mixture is synthesized in solid phase synthesis, the sum of values obtained by multiplying the loading rate (mmol/g) of the prepared solid phase supporting substrate by the amount of the solid phase supporting substrate used may be considered as the number of moles of the mixture. At that time, even if multiple steps are performed in the reaction on the solid phase, the conversion rate of each step may be considered as 100% (i.e., the theoretical amount), and when cleavage from the solid phase is performed, the cleavage efficiency may be considered as 100% (i.e., cleaved in the theoretical amount) and the subsequent reaction in the liquid phase may be carried out.

As for the amount of solvent used in the solid phase reaction, an arbitrary times volume (v/w) was utilized relative to the weight of the resin used in the reaction. For example, when 20 mg of resin was used and 20 v/w of solvent was used, 20 mg × 20 v/w = 400 μL of solvent was used.

As for the amount of solvent used when washing the resin after the reaction, an arbitrary times volume (v/w) was utilized relative to the weight of the resin used in the reaction. For example, when 20 mg of resin was used and 20 v/w of solvent was used, 20 mg × 20 v/w = 400 μL of solvent was used.

In order to monitor the progress of the reaction on the solid phase, it is necessary to collect the resin from the reaction container. At that time, using a micropipette fitted with a pipette tip cut at the appropriate length from the end, the resin was collected by sucking approximately 5 to 20 μL to ensure that the resin was included and was transferred onto the filter of a pipette tip equipped with a filter (for example, Thermo Scientific, tip with ART filter, ART20P, 2149P-05). Thereafter, the compound supported on the resin was cleaved from the resin by the following representative procedures for the resin on the filter. The resin was consecutively washed three times with NMP, three times with MeOH, and three times with DCM, a 10% TFA/DCM solution (50 μL) containing 0.05M trimethylbenzene was then added to the filter tip, and the resin was immersed for 3 to 10 minutes. The solution was filtered by applying air or nitrogen pressure over the filter tip and the filtrate was dissolved in NMP or DMI (250 μL). This solution (50 μL) was diluted with acetonitrile (250 μL) to prepare a LC sample, which was analyzed by UPLC-MS analysis to analyze the reaction progress.

The expression "cleavage" from the solid phase indicates desorption of the compound supported on the resin from the resin, such as treatment of the resin with a 10% TFA/DCM solution containing 0.05M trimethylbenzene to recover the supported compound into the solution.

The compound No. used in Examples was indicated by the combination of arbitrary alphabets, numbers, and symbols. For compounds in the state where they are supported on the solid phase, "R" was added at the end, for example, "ZZ001-01R". In contrast, the compound cleaved from the solid phase was indicated as "ZZ001" without "-01R".

The symbols

[Formula 10]

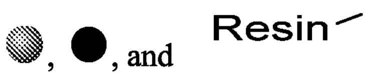
, , and Resin

used in the chemical structure representations in Examples refer to polystyrene resin and indicate the state where the compound is supported on the solid phase.

In "-01R" used in Examples to indicate that the compound is supported on the solid phase, the number indicates the type of resin used. For example, "01" in "-01R" indicates that the compound is supported on a brominated Wang resin, 4-(bromomethyl)phenoxymethyl polystyrene (for example, Merck KGaA, 4-(benzyloxy)benzyl bromide, polymer-supported). "02" in "-02R" indicates that the compound is supported on 4-(bromomethyl)phenoxyethyl polystyrene (for example, Merck KGaA, 2-(4-bromomethylphenoxy)ethyl polystyrene, sometimes denoted as modified Wang resin in Examples).

Notation example of "-01R"

[Formula 11]

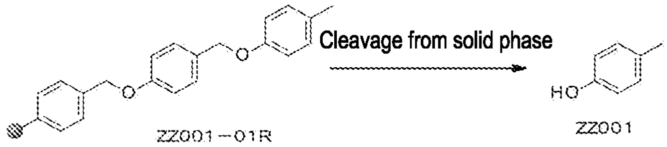


ZZ001-01R

ZZ001

Notation example of "-02R"

[Formula 12]

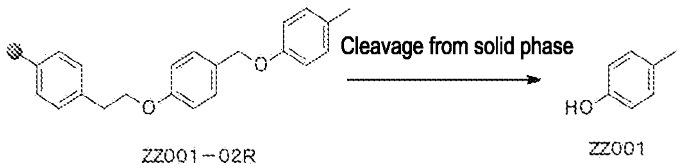


ZZ001-02R

ZZ001

For the solid phase-supported compound used in the solid phase synthesis, the loading rate (mmol/g) is shown, which refers to the loading rate calculated when the starting material is made to be supported on the solid phase (initial loading rate). Although the initial loading rate may be different from the loading rate after the reaction because of the change in molecular weight caused by the linkage reaction of building blocks, the initial loading rate was used throughout the synthesis steps.

Even when the solid phase-supported compound is the same, the loading rate may vary from lot to lot, but the same compound No. may be used for the compound No.

<u>Example 1: Synthesis of compounds used in the present specification</u>

Example 1-1: Synthesis methods for arenol compounds B-001 to B-015

The synthesis methods for substrates (arenol, model compounds) used in Example 2 and subsequent Examples will be shown beginning with the synthesis methods for building blocks BB-1 to BB-6 and intermediates (INT1 to INT7) used in the synthesis of arenol compounds (B-001 to B-015).

Example 1-1-1: Synthesis of building blocks BB-1 to BB-6

2-(5-Hydroxypyridin-3-yl)benzaldehyde: Synthesis of BB-1

[Formula 13]

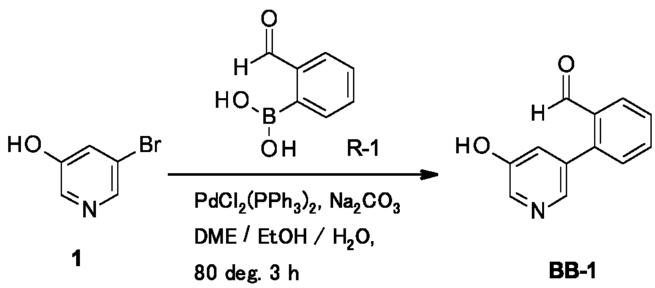


1

BB-1

To a 50 mL flask under a nitrogen atmosphere were added 3-hydroxy-5-bromopyridine (1) (0.5 g, 2.9 mmol, 1.0 eq), 2-formylbenzeneboronic acid (R-1) (861.7 mg, 5.7 mmol, 2.0 eq), $Na_2CO_3$ (456.8 mg, 4.3 mmol, 1.5 eq), and a mixed solution of DME/EtOH/$H_2O$ (4.0/4.0/1.7 mL). To this suspension was added $PdCl_2(PPh3)_2$ (100.8 mg, 140.0 μmol, 0.05 eq), and the obtained reaction mixture was stirred at 80°C for 3 hours under a nitrogen atmosphere. After cooling to room temperature, the reaction solution was filtered through Celite® and the Celite® was washed three times with ethyl acetate (25 mL). The filtrate was concentrated under reduced pressure and the residue was purified by silica gel column chromatography (0 to 80% ethyl acetate/petroleum ether) to afford 2-(5-hydroxypyridin-3-yl)benzaldehyde (BB-1) as a brown solid (300 mg, 51.4%).

LCMS (ESI, m/z): 200 $[M+H]^+$.

Retention time: 0.367 min.

Analysis condition: PH-FA-01.

5-Bromopyridine-3-carboxylic acid: Synthesis of BB-2

[Formula 14]

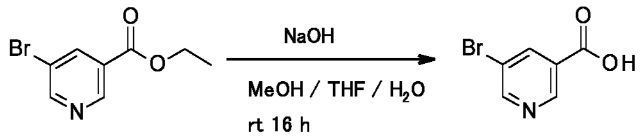


2 BB-2

To a 200 mL flask under a nitrogen atmosphere were added ethyl 5-bromopyridine-3-carboxylate (2) (5.0 g, 21.8 mmol, 1.0 eq) and a mixed solution of MeOH/THF/$H_2O$ (40 mL/40 mL/40 mL). To this solution was added NaOH (2.6 g, 65.5 mmol, 3.0 eq), and the obtained reaction mixture was stirred at room temperature for 16 hours and concentrated under reduced pressure.

The residue was dissolved in water (50 mL), and the pH was adjusted to an acidity of 5 to 6 using citric acid. The obtained solid was filtered, and the solid on the filter was washed three times with water (20 mL) and dried under reduced pressure at 30°C for 16 hours using a constant temperature dryer to afford 5-bromopyridine-3-carboxylic acid (BB-2) as an off-white solid (3.4 g). The obtained solid was used in the next step without carrying out further purification.

LCMS (ESI, m/z): 202, 204 $[M+H]^+$.

Retention time: 0.673 min.

Analysis condition: PH-FA-02.

4-Bromo-3-fluorobenzoic acid: Synthesis of BB-3

[Formula 15]

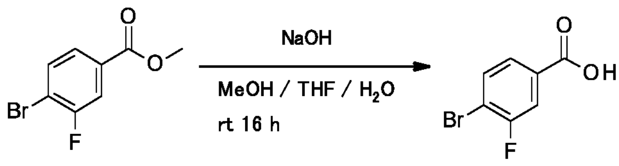


3

BB-3

To a 100 mL flask under a nitrogen atmosphere were added methyl 4- bromo-3-fluorobenzoate (3) (1.0 g, 4.3 mmol, 1.0 eq) and a mixed solution of MeOH/THF/$H_2O$ (7.0 mL/7.0 mL/7.0 mL). To this solution was added NaOH (0.5 g, 12.9 mmol, 3.0 eq), and the obtained reaction mixture was stirred at room temperature for 16 hours and concentrated under reduced pressure.

The residue was dissolved in water (20 mL), and the pH was adjusted to an acidity of 5 to 6 using 2M HCl. The obtained solid was filtered, and the solid on the filter was washed three times with water (10 mL) and dried under reduced pressure at 50°C for 16 hours using a constant temperature dryer to afford 4-bromo-3-fluorobenzoic acid (BB-3) as an off-white solid (0.9 g, 95.8%).

LCMS (ESI, m/z): No parent ions were detected.

Retention time: 0.874 min.

Analysis condition: PH-FA-02.

Trimethyl-[2-[5-(oxan-2-yloxy)pyridin-3-yl]ethynyl]silane: Synthesis of BB-4

[Formula 16]

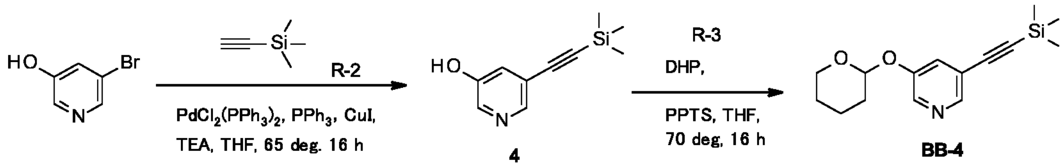


4

BB-4

First step: 5-(2-Trimethylsilylethynyl)pyridin-3-ol: Synthesis of compound 4

To a 50 mL flask under a nitrogen atmosphere were added 3-hydroxy-5-bromopyridine (1) (1.0 g, 5.7 mmol, 1.0 eq), TEA (8.0 mL), and THF (10 mL). To this solution were added trimethylsilylacetylene (R-2) (850 mg, 8.6 mmol, 1.5 eq), CuI (110 mg, 0.6 mmol, 0.1 eq), $PPh_3$ (150 mg, 0.6 mmol, 0.1 eq), and $PdCl_2(PPh_3)_2$ (400 mg, 0.6 mmol, 0.1 eq), and the obtained reaction mixture was stirred at 65°C for 16 hours under a nitrogen atmosphere. After cooling to room temperature, the reaction solution was filtered through Celite® and the Celite® was washed seven times with ethyl acetate (25 mL). The filtrate was concentrated under reduced pressure and the residue was purified by silica gel column chromatography (0 to 10% MeOH/DCM) to afford 5-(2-trimethylsilylethynyl)pyridin-3-ol (compound 4) as a brown solid (5.1 g, 66.0%).

LCMS (ESI, m/z): 192 $[M+H]^+$.

Retention time: 0.818 min.

Analysis condition: PH-FA-01.

Second step: Trimethyl-[2-[5-(oxan-2-yloxy)pyridin-3-yl]ethynyl]silane: Synthesis of compound BB-4

To a 200 mL flask under a nitrogen atmosphere were added 5-(2-trimethylsilylethynyl)pyridin-3-ol (compound 4) (2.6 g, 13.6 mmol, 1.0 eq), DHP (R-3) (8.0 g, 95.1 mmol, 7.0 eq), and THF (50 mL). To this solution was added PPTS (170 mg, 0.7 mmol, 0.05 eq), and the obtained reaction mixture was stirred at 70°C for 16 hours. After cooling to room temperature, the reaction solution was concentrated under reduced pressure. The residue was purified by silica gel column chromatography (0 to 80% ethyl acetate/petroleum ether) to afford trimethyl-[2-[5-(oxan-2-yloxy)pyridin-3-yl]ethynyl]silane (BB-4) as a yellow oil (2.7 g, 72.0%).

LCMS (ESI, m/z): 276 $(M+H)^+$.

Retention time: 1.214 min.

Analysis condition: PH-FA-02.

Trimethyl-[2-[4-(oxan-2-yloxy)phenyl]ethynyl]silane: Synthesis of BB-5

[Formula 17]

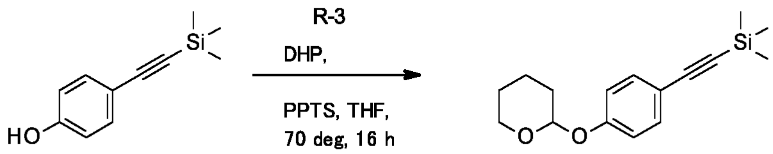


6

BB−5

To a 100 mL flask under a nitrogen atmosphere were added 4-(2-trimethylsilylethynyl)phenol (compound 6) (2.0 g, 10.5 mmol, 1.0 eq), DHP (R-3) (2.6 g, 7.0 eq), and THF (20 mL). To this solution was added PPTS (132.3 mg, 0.5 mmol, 0.05 eq), and the obtained reaction mixture was stirred at 70°C for 16 hours. After cooling to room temperature,

the reaction solution was concentrated under reduced pressure. The residue was purified by silica gel column chromatography (0 to 20% ethyl acetate/petroleum ether) to afford trimethyl-[2-[4-(oxan-2-yloxy)phenyl]ethynyl]silane (BB-5) as a brown oil (2.2 g, 76.0%).

LCMS (ESI, m/z): No parent ions were detected.

Retention time: 1.313 min.

Analysis condition: PH-FA-02.

3-Methyl-4-(2-phenylsulfanylethylamino)benzenesulfonamide: Synthesis of BB-6

[Formula 18]

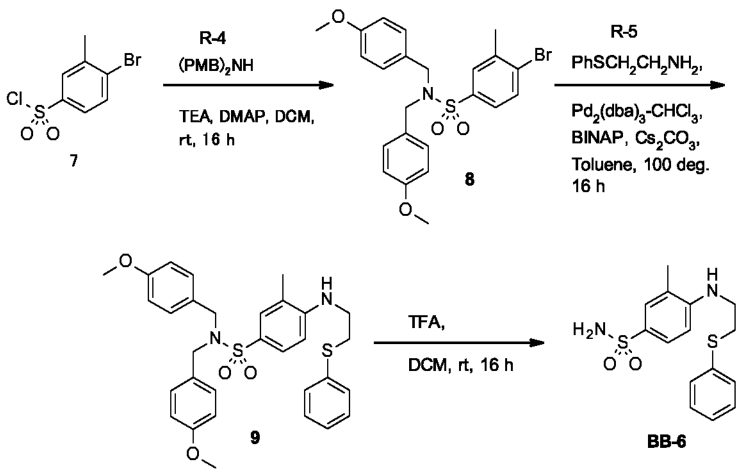


First step: Synthesis of 4-bromo-N,N-bis[(4-methoxyphenyl)methyl]-3-methylbenzenesulfonamide (compound 8)

To a 500 mL flask under a nitrogen atmosphere were added 4-bromo-3-methylbenzenesulfonic acid chloride (7) (10.0 g, 37.1 mmol, 1.0 eq) and DCM (220 mL). To this solution were added $(PMB)_2NH$ (R-4) (1.4 g, 55.6 mmol, 1.5 eq), TEA (7.5 g, 74.2 mmol, 2.0 eq), and DMAP (453.2 mg, 3.7 mmol, 0.1 eq), and the obtained reaction mixture was stirred at room temperature for 16 hours. Water (100 mL) was added to the reaction solution and the mixture was extracted three times with DCM (150 mL).

The organic layers were mixed, dried over $Na_2SO_4$, then filtered, and the filtrate was concentrated under reduced pressure.

The residue was purified by silica gel column chromatography (0 to 40% ethyl acetate/petroleum ether) to afford 4-bromo-N,N-bis[(4-methoxyphenyl)methyl]-3-methylbenzenesulfonamide (compound 8) as an off-white solid (9.0 g, 89.9%).

LCMS (ESI, m/z): No parent ions were detected.

Retention time: 1.235 min.

Analysis condition: PH-FA-02.

Second step: Synthesis of N,N-bis[(4-methoxyphenyl)methyl]-3-methyl-4-(2-phenylsulfanylethylamino)benzenesulfonamide (compound 9)

To a 100 mL flask under a nitrogen atmosphere were added 4-bromo-N,N-bis[(4-methoxyphenyl)methyl]-3-methylbenzenesulfonamide (compound 8) (9.0 g, 18.3 mmol, 1.0 eq), 2-(benzenesulfanyl)ethanamine (R-5) (3.5 g, 22.9 mmol, 1.25 eq), $Cs_2CO_3$ (17.9 g, 55.0 mmol, 3.0 eq), and toluene (30 mL). To this suspension were added $Pd_2(dba)_3$-$CHCl_3$ (1.9 g, 1.8 mmol, 0.2 eq) and BINAP (2.3 g, 3.7 mmol, 0.2 eq), and the obtained reaction mixture was stirred at 100°C for 16 hours under a nitrogen atmosphere. After cooling to room temperature, the reaction solution was filtered through Celite® and the Celite® was washed three times with ethyl acetate (25 mL). The filtrate was concentrated under reduced pressure and the residue was purified by silica gel column chromatography (0 to 80% ethyl acetate/petroleum ether) to afford N,N-bis[(4-methoxyphenyl)methyl]-3-methyl-4-(2-phenylsulfanylethylamino)benzenesulfonamide (compound 9) as a brown oil (10.0 g, 67.8%).

LCMS (ESI, m/z): 563.6 $[M+H]^+$.

Retention time: 1.630 min.

Analysis condition: PH-TFA-01.

Third step: 3-Methyl-4-(2-phenylsulfanylethylamino)benzenesulfonamide: Synthesis of BB-6

To a 500 mL flask under a nitrogen atmosphere were added N,N-bis[(4-methoxyphenyl)methyl]-3-methyl-4-(2-phenylsulfanylethylamino)benzenesulfonamide (compound 9) (10.0 g, 17.8 mmol, 1.0 eq) and DCM (50 mL). To this solution was added TFA (180 mL), the obtained reaction mixture was stirred at room temperature for 16 hours, and the reaction solution was concentrated under reduced pressure. The residue was purified by reversed phase silica gel column chromatography (C18) (0 to 80% $CH_3CN$/water) to afford 3-methyl-4-(2-phenylsulfanylethylamino)benzenesulfonamide (BB-6) as a light yellow solid (2.5 g, 43.6%).

LCMS (ESI, m/z): 323 $[M+H]^+$.

Retention time: 1.179 min.

Analysis condition: PH-AC-01.

$^1$H-NMR (300 MHz, DMSO-$d_6$) $\delta$ 7.53-7.30 (m, 6H), 7.29-7.16 (m, 1H), 6.19 (d, J = 2.4 Hz, 2H), 6.54 (d, J = 8.4 Hz, 1H), 5.78 (m, 1H), 3.40 (m, 2H), 3.18 (m, 2H), 2.10 (s, 3H).

Example 1-1-2: Synthesis of intermediates (INT1 to INT7)

4-[4-[(2-Bromophenyl)methyl]piperazin-1-yl]benzoic acid: Synthesis of INT1

[Formula 19]

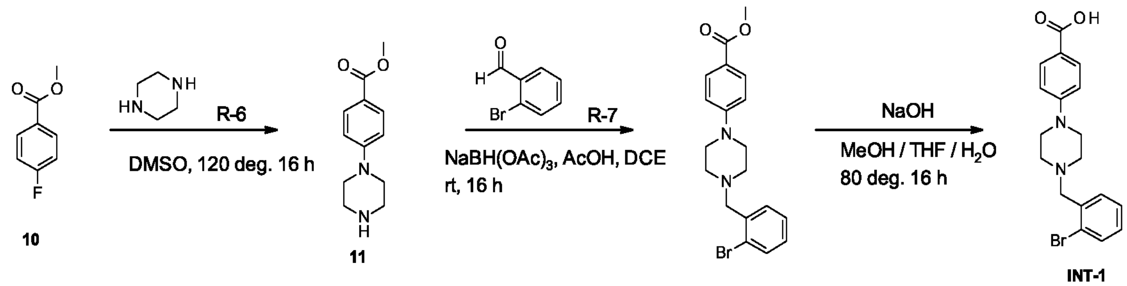


10 11 12 INT-1

First step: Synthesis of methyl 4-piperazin-1-ylbenzoate (compound 11)

To a 50 mL flask under a nitrogen atmosphere were added methyl 4-fluorobenzoate (10) (1.0 g, 6.5 mmol, 1.0 eq) and DMSO (10 mL). To this solution was added piperazine (R-6) (1.7 g, 19.5 mmol, 3.0 eq), and the obtained reaction mixture was stirred at 120°C for 16 hours. Water (20 mL) was added to the reaction solution and the precipitated solid was recovered by filtration to afford methyl 4-piperazin-1-ylbenzoate (compound 11) as an off-white solid (1.3 g). The obtained solid was used in the next step without carrying out further purification.

LCMS (ESI, m/z): 221 $[M+H]^+$.

Retention time: 0.521 min.

Analysis condition: PH-TFA-09.

Second step: Synthesis of methyl 4-[4-[(2-bromophenyl)methyl]piperazin-1-yl]benzoate (compound 12)

To a 100 mL flask under a nitrogen atmosphere were added methyl 4-piperazin-1-ylbenzoate (compound 11) (1.1 g, 5.0 mmol, 1.0 eq) and DCE (25 mL). To this solution were added 2-bromobenzaldehyde (R-7) (1.8 g, 9.9 mmol, 2.0 eq) and AcOH (0.6 mL), and the obtained reaction mixture was stirred at room temperature for 1 hour. To this reaction solution was added $NaBH(OAc)_3$ (4.2 g, 20.0 mmol, 4.0 eq), and the mixture was stirred at room temperature for 16 hours. A saturated aqueous $NH_4Cl$ solution (20 mL) was added to the reaction solution and the mixture was extracted three times with ethyl acetate (50 mL).

The organic layers were mixed, washed with a saturated aqueous $NaHCO_3$ solution (100 mL) and brine (100 mL), dried over $Na_2SO_4$, then filtered, and the filtrate was concentrated under reduced pressure.

The residue was purified by silica gel column chromatography (0 to 50% ethyl acetate/petroleum ether) to afford

methyl 4-[4-[(2-bromophenyl)methyl]piperazin-1-yl]benzoate (compound 12) as an off-white solid (0.9 g, 46.3%).

LCMS (ESI, m/z): 389, 391 $[M+H]^+$.

Retention time: 0.623 min.

Analysis condition: PH-TFA-09.

Third step: 4-[4-[(2-Bromophenyl)methyl]piperazin-1-yl]benzoic acid: Synthesis of INT1

To a 100 mL flask under a nitrogen atmosphere were added methyl 4-[4-[(2-bromophenyl)methyl]piperazin-1-yl]benzoate (compound 12) (1.1 g, 2.7 mmol, 1.0 eq) and a mixed solution of MeOH/THF/$H_2O$ (6.0 mL/6.0 mL/6.0 mL). To this solution was added NaOH (0.3 g, 8.2 mmol, 3.0 eq), the obtained reaction mixture was stirred at 80°C for 16 hours, and the reaction solution was cooled to room temperature and then concentrated under reduced pressure.

The residue was dissolved in water (20 mL), and the pH was adjusted to an acidity of 2 to 3 using concentrated hydrochloric acid. The obtained solid was filtered and recovered. The recovered solid was purified by crushing and washing using petroleum ether to afford 4-[4-[(2-bromophenyl)methyl]piperazin-1-yl]benzoic acid (INT1) as an off-white solid (0.9 g, 93.0%).

LCMS (ESI, m/z): 375, 377 $[M+H]^+$.

Retention time: 0.599 min.

Analysis condition: PH-TFA-09.

N-Benzylsulfonyl-4-[4-[(2-bromophenyl)methyl]piperazin-1-yl]benzamide: Synthesis of INT2

[Formula 20]

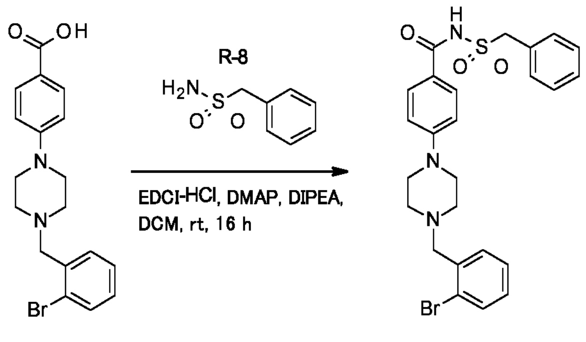


INT-1 INT-2

To a 200 mL flask under a nitrogen atmosphere were added 4-[4-[(2-bromophenyl)methyl]piperazin-1-yl]benzoic acid (INT1) (6.0 g, 16.0 mmol, 1.0 eq), benzenemethanesulfonamide (R-8) (2.0 g, 11.7 mmol, 0.7 eq), DMAP (1.5 g, 12.7 mmol, 0.7 eq), and DCM (50 mL). To this solution were added EDCI·HCl (4.8 g, 25.1 mmol, 2.0 eq) and DIPEA (6.4 g, 49.6 mmol, 3.0 eq), and the obtained reaction mixture was stirred at room temperature for 16 hours. A saturated aqueous $NH_4Cl$ solution (50 mL) was added to the reaction solution and the mixture was extracted three times with DCM (50 mL). The organic layers were mixed, dried over $Na_2SO_4$, then filtered, and the filtrate was concentrated under reduced pressure.

The residue was purified by silica gel column chromatography (0 to 80% ethyl acetate/petroleum ether) to afford N-benzylsulfonyl-4-[4-[(2-bromophenyl)methyl]piperazin-1-yl]benzamide (INT2) as an off-white solid (4.2 g, 68.1%).

LCMS (ESI, m/z): 528, 530 $[M+H]^+$.

Retention time: 1.052 min.

Analysis condition: PH-TFA-02.

N-Benzylsulfonyl-4-piperazin-1-ylbenzamide: Synthesis of INT3

[Formula 21]

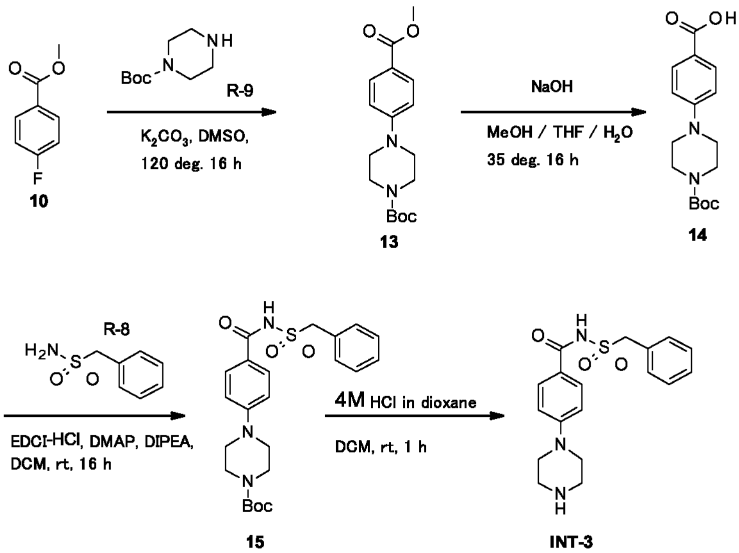


First step: tert-Butyl 4-(4-methoxycarbonylphenyl)piperazine-1-carboxylate: Synthesis of compound 13

To a 200 mL flask under a nitrogen atmosphere were added methyl 4-fluorobenzoate (10) (5.0 g, 32.4 mmol, 1.0 eq), $K_2CO_3$ (6.7 g, 48.6 mmol, 1.5 eq), and DMSO (50 mL). To this suspension was added Boc-piperazine (R-9) (6.0 g, 32.4 mmol, 1.0 eq), and the obtained reaction mixture was stirred at 120°C for 16 hours. Water (50 mL) was added to the reaction solution and the precipitated solid was filtered. The filtered solid was washed three times with water (20 mL) and dried under reduced pressure at 30°C for 16 hours using a constant temperature dryer to afford tert-butyl 4-(4-methoxycarbonylphenyl)piperazine-1-carboxylate (compound 13) as a light yellow solid (3.7 g). The obtained solid was used in the next step without carrying out further purification.

LCMS (ESI, m/z): 321 $[M+H]^+$.

Retention time: 1.090 min.

Analysis condition: PH-AC-03.

Second step: 4-[4-[(2-Methylpropan-2-yl)oxycarbonyl]piperazin-1-yl]benzoic acid: Synthesis of compound 14

To a 200 mL flask under a nitrogen atmosphere were added tert-butyl 4-(4-methoxycarbonylphenyl)piperazine-1-carboxylate (compound 13) (3.7 g, 11.5 mmol, 1.0 eq) and a mixed solution of MeOH/THF/$H_2O$ (25 mL/25 mL/25 mL). To this solution was added NaOH (1.4 g, 34.6 mmol, 3.0 eq) at 35°C, and the obtained reaction mixture was stirred at 35°C for 16 hours. The reaction solution was cooled to room temperature and then concentrated under reduced pressure.

The residue was dissolved in water (50 mL), and the pH was adjusted to an acidity of 5 to 6 using citric acid. The precipitated solid was filtered and the solid on the filter was washed three times with water (25 mL). The obtained solid was dried under reduced pressure at 50°C for 16 hours using a constant temperature dryer to afford 4-[4-[(2-methylpropan-2-yl)oxycarbonyl]piperazin-1-yl]benzoic acid (compound 14) as an off-white solid (3.3 g). The obtained solid was used in the next step without carrying out further purification.

LCMS (ESI, m/z): 307 $[M+H]^+$.

Retention time: 0.601 min.

Analysis condition: PH-AC-03.

Third step: tert-Butyl 4-[4-(benzylsulfonylcarbamoyl)phenyl]piperazine-1-carboxylate: Synthesis of compound 15

To a 100 mL flask under a nitrogen atmosphere were added 4-[4-[(2-methylpropan-2-yl)oxycarbonyl]piperazin-1-yl]benzoic acid (compound 14) (1.5 g, 4.9 mmol, 1.0 eq), benzenemethanesulfonamide (R-8) (0.8 g, 4.9 mmol, 1.0 eq), DMAP (0.6 g, 4.9 mmol, 1.0 eq), and DCM (30 mL). To this solution were added EDCI·HCl (1.9 g, 9.8 mmol, 2.0 eq) and DIPEA (2.5 g, 19.6 mmol, 4.0 eq), and the obtained reaction mixture was stirred at room temperature for 16 hours. A saturated aqueous $NH_4Cl$ solution (30 mL) was added to the reaction solution and the mixture was extracted three times with DCM (40 mL). The organic layers were mixed, dried over $Na_2SO_4$, then filtered, and the filtrate was concentrated under reduced pressure.

The residue was purified by silica gel column chromatography (0 to 80% ethyl acetate/petroleum ether) to afford tert-butyl 4-[4-(benzylsulfonylcarbamoyl)phenyl]piperazine-1-carboxylate (compound 15) as a light yellow solid (870 mg, 38.7%).

LCMS (ESI, m/z): 460 $[M+H]^+$.

Retention time: 0.749 min.

Analysis condition: PH-AC-03.

Fourth step: N-Benzylsulfonyl-4-piperazin-1-ylbenzamide: Synthesis of INT3

To a 50 mL flask under a nitrogen atmosphere were added tert-butyl 4-[4-(benzylsulfonylcarbamoyl)phenyl]piperazine-1-carboxylate (compound 15) (0.9 g, 1.9 mmol, 1.0 eq) and DCM (2.0 mL). To this solution was added a 4M solution of HCl in 1,4-dioxane (15 mL), and the obtained reaction mixture was stirred at room temperature for 1 hour. The reaction solution was concentrated under reduced pressure, the obtained residue was dissolved in water (15 mL), and the pH was adjusted to a basicity of 8 to 9 using ammonia water. The precipitated solid was filtered and the obtained solid was purified by reversed phase silica gel column chromatography (C18) (0 to 80% $CH_3CN$/0.5 g/L aqueous ammonium bicarbonate solution) to afford N-benzylsulfonyl-4-piperazin-1-ylbenzamide (INT3) as an off-white solid (0.3 g, 45.6%).

LCMS (ESI, m/z): 360 $[M+H]^+$.

Retention time: 0.639 min.

Analysis condition: PH-FA-02.

N-Benzylsulfonyl-4-[4-(5-bromopyridine-3-carbonyl)piperazin-1-yl]benzamide: Synthesis of INT4

[Formula 22]

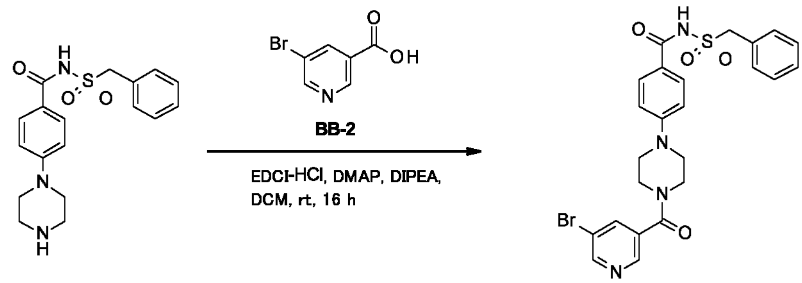


INT-3

INT-4

To a 100 mL flask under a nitrogen atmosphere were added N-benzylsulfonyl-4-piperazin-1-ylbenzamide (INT3) (2.0 g, 5.6 mmol, 1.0 eq), 5-bromopyridine-3-carboxylic acid (BB-2) (1.4 g, 6.7 mmol, 1.2 eq), DMAP (0.7 g, 5.6 mmol, 1.0 eq), and DCM (30 mL). To this solution were added EDCI·HCl (2.1 g, 11.1 mmol, 2.0 eq) and DIPEA (2.9 g, 22.3 mmol, 4.0 eq), and the obtained reaction mixture was stirred at room temperature for 16 hours. A saturated aqueous $NH_4Cl$ solution (30 mL) was added to the reaction solution and the mixture was extracted three times with DCM (50 mL). The organic layers were mixed, dried over $Na_2SO_4$, then filtered, and the filtrate was concentrated under reduced pressure.

The residue was purified by reversed phase silica gel column chromatography (C18) (30 to 80% $CH_3CN$/water) to afford N-benzylsulfonyl-4-[4-(5-bromopyridine-3-carbonyl)piperazin-1-yl]benzamide (INT4) as a light yellow solid (1.1 g, 34.6%).

LCMS (ESI, m/z): 543, 545 $[M+H]^+$.

Retention time: 0.923 min.

Analysis condition: PH-FA-02.

N-Benzylsulfonyl-4-[4-(4-bromo-3-fluorobenzoyl)piperazin-1-yl]benzamide: Synthesis of INT5

[Formula 23]

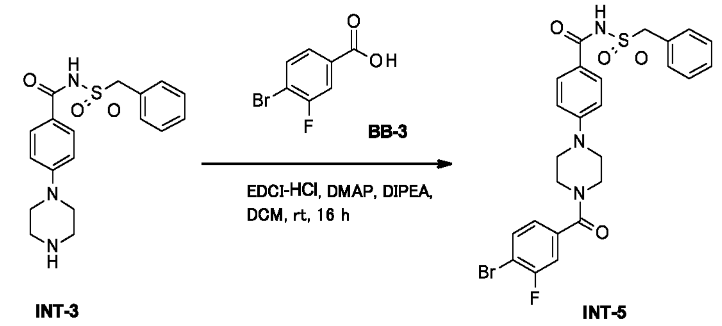


To a 50 mL flask under a nitrogen atmosphere were added N-benzylsulfonyl-4-piperazin-1-ylbenzamide (INT3) (0.9 g, 2.4 mmol, 1.0 eq), N-benzylsulfonyl-4-piperazin-1-ylbenzamide (BB-3) (0.7 g, 3.2 mmol, 1.3 eq), DMAP (0.3 g, 2.4 mmol, 1.0 eq), and DCM (20 mL). To this solution were added EDCI·HCl (0.9 g, 4.9 mmol, 2.0 eq) and DIPEA (1.3 g, 9.8 mmol, 4.0 eq), and the obtained reaction mixture was stirred at room temperature for 16 hours. A saturated aqueous $NH_4Cl$ solution (30 mL) was added to the reaction solution and the mixture was extracted three times with DCM (50 mL). The organic layers were mixed, dried over $Na_2SO_4$, then filtered, and the filtrate was concentrated under reduced pressure.

The residue was purified by silica gel column chromatography (0 to 100% ethyl acetate/petroleum ether, then 0 to 10% MeOH/DCM) to afford N-benzylsulfonyl-4-[4-(4-bromo-3-fluorobenzoyl)piperazin-1-yl]benzamide (INT5) as a light yellow solid (1.2 g, 71.7%).

LCMS (ESI, m/z): 560, 562 $[M+H]^+$.

Retention time: 1.002 min.

Analysis condition: PH-FA-02.

N-Benzylsulfonyl-6-piperazin-1-ylpyridazine-3-carboxamide: Synthesis of INT6

[Formula 24]

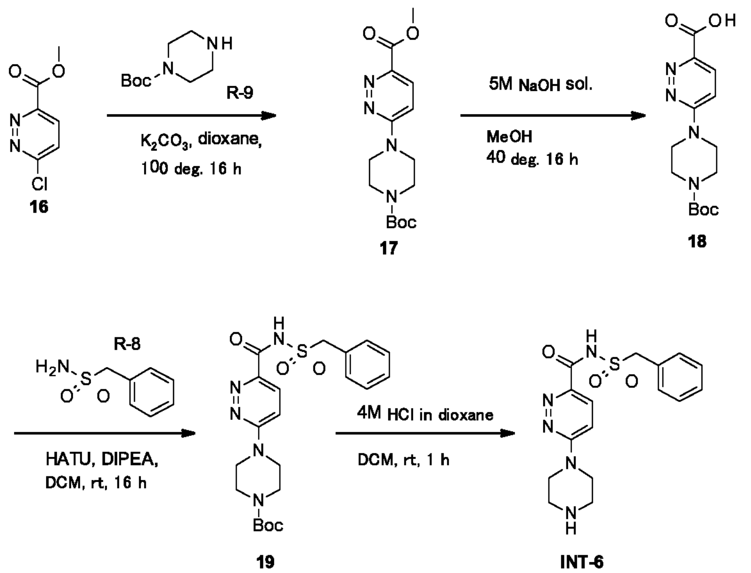


First step: Methyl 6-[4-[(2-methylpropan-2-yl)oxycarbonyl]piperazin-1-yl]pyridazine-3-carboxylate: Synthesis of compound 17

To a 1 L flask under a nitrogen atmosphere were added methyl 6-chloropyridazinecarboxylate (16) (20 g, 116.3 mmol, 1.0 eq), $K_2CO_3$ (40 g, 290.7 mmol, 2.5 eq), and 1,4-dioxane (400 mL). To this suspension was added Boc-piperazine (R-9) (32.4 g, 174.4 mmol, 1.5 eq), and the obtained reaction mixture was stirred at 100°C for 16 hours. The

reaction mixture was filtered and the filtered solid was washed three times with DCM (100 mL). The filtrate was washed three times with water (250 mL), then dried over $Na_2SO_4$, then filtered, and the filtrate was concentrated under reduced pressure. The residue was purified by silica gel column chromatography (0 to 80% ethyl acetate/petroleum ether) to afford methyl 6-[4-[(2-methylpropan-2-yl)oxycarbonyl]piperazin-1-yl]pyridazine-3-carboxylate (compound 17) as a light yellow solid (11.4 g, 30.5%).

LCMS (ESI, m/z): 323 $[M+H]^+$.

Retention time: 0.852 min.

Analysis condition: PH-FA-02.

Second step: 6-[4-[(2-Methylpropan-2-yl)oxycarbonyl]piperazin-1-yl]pyridazine-3-carboxylic acid: Synthesis of compound 18

To a 500 mL flask under a nitrogen atmosphere were added methyl 6-[4-[(2-methylpropan-2-yl)oxycarbonyl]piperazin-1-yl]pyridazine-3-carboxylate (compound 17) (11.4 g, 35.4 mmol, 1.0 eq) and MeOH (200 mL). To this solution was added a 5M aqueous NaOH solution (35.4 mL, 177.1 mmol, 5.0 eq), and the obtained reaction mixture was stirred at 40°C for 16 hours. The reaction solution was cooled to room temperature and then concentrated under reduced pressure.

The residue was dissolved in water (200 mL), and the pH was adjusted to an acidity of 5 to 6 using a 2M aqueous HCl solution. The precipitated solid was filtered and the solid on the filter was washed three times with water (100 mL). The recovered solid was dried under reduced pressure at 50°C for 16 hours using a constant temperature dryer to afford 6-[4-[(2-methylpropan-2-yl)oxycarbonyl]piperazin-1-yl]pyridazine-3-carboxylic acid (compound 18) as a light yellow solid (5.4 g). The obtained solid was used in the next step without carrying out further purification.

LCMS (ESI, m/z): 309 $[M+H]^+$.

Retention time: 0.704 min.

Analysis condition: PH-FA-02.

Compound 18, whose synthetic method is illustrated in Example 1-1, Synthesis of INT6, may also be denoted as compound AC-012 in the present specification.

Third step: 6-[4-[(2-Methylpropan-2-yl)oxycarbonyl]piperazin-1-yl]pyridazine-3-carboxylic acid: Synthesis of compound 19

To a 300 mL flask under a nitrogen atmosphere were added 6-[4-[(2-methylpropan-2-yl)oxycarbonyl]piperazin-1-yl]pyridazine-3-carboxylic acid (compound 18) (5.4 g, 17.5 mmol, 1.0 eq), benzenemethanesulfonamide (R-8) (4.5 g, 26.3 mmol, 1.5 eq), and DCM (120 mL). To this solution were added HATU (10.0 g, 26.3 mmol, 1.5 eq) and DIPEA (9.1 g, 70.1 mmol, 4.0 eq), and the obtained reaction mixture was stirred at room temperature for 16 hours. A saturated aqueous $NH_4Cl$ solution (75 mL) was added to the reaction solution and the mixture was extracted three times with DCM (100 mL). The organic layers were mixed, dried over $Na_2SO_4$, then filtered, and the filtrate was concentrated under reduced pressure.

The residue was purified by silica gel column chromatography (0 to 100% ethyl acetate/petroleum ether, then 0 to 10% MeOH/DCM) to afford 6-[4-[(2-methylpropan-2-yl)oxycarbonyl]piperazin-1-yl]pyridazine-3-carboxylic acid (compound 19) as a light yellow solid (5.1 g, 63.1%).

LCMS (ESI, m/z): 462 $[M+H]^+$.

Retention time: 1.220 min.

Analysis condition: PH-TFA-10.

Fourth step: N-Benzylsulfonyl-6-piperazin-1-ylpyridazine-3-carboxamide: Synthesis of INT6

To a 300 mL flask under a nitrogen atmosphere were added 6-[4-[(2-methylpropan-2-yl)oxycarbonyl]piperazin-1-yl]pyridazine-3-carboxylic acid (compound 19) (5.1 g, 11.1 mmol, 1.0 eq) and DCM (30 mL). To this solution was added a 4M solution of HCl in 1,4-dioxane (60 mL), and the obtained reaction mixture was stirred at room temperature for 1 hour. The reaction solution was concentrated under reduced pressure, the obtained residue was dissolved in water (50 mL), and the pH was adjusted to a basicity of 8 to 9 using ammonia water. The precipitated solid was filtered and the obtained solid was washed three times with water (30 mL). The recovered solid was dried under reduced pressure at 50°C for 16 hours using a constant temperature dryer to afford N-benzylsulfonyl-6-piperazin-1-ylpyridazine-3-carboxamide (INT6) as an off-white solid (3.7 g). The obtained solid was used in the next step without carrying out further purification.

LCMS (ESI, m/z): 362 $[M+H]^+$.

Retention time: 0.594 min.

Analysis condition: PH-FA-02.

N-Benzylsulfonyl-6-[4-(5-bromopyridine-3-carbonyl)piperazin-1-yl]pyridazine-3-carboxamide: Synthesis of INT7

[Formula 25]

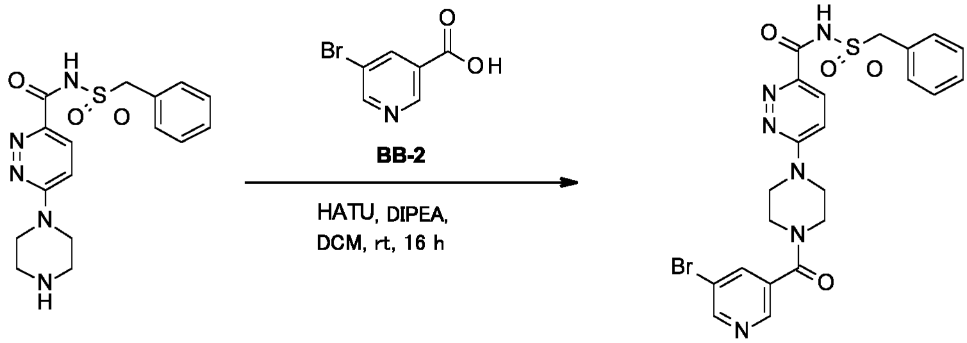


INT-6 INT-7

To a 100 mL flask under a nitrogen atmosphere were added N-benzylsulfonyl-6-piperazin-1-ylpyridazine-3-carboxamide (INT6) (2.0 g, 5.5 mmol, 1.0 eq), 5-bromopyridine-3-carboxylic acid (BB-2) (1.4 g, 6.6 mmol, 1.2 eq), and DCM (40 mL). To this solution were added HATU (3.2 g, 8.3 mmol, 1.5 eq) and DIPEA (2.9 g, 22.1 mmol, 4.0 eq), and the obtained reaction mixture was stirred at room temperature for 16 hours. A saturated aqueous $NH_4Cl$ solution (30 mL) was added to the reaction solution and the mixture was extracted three times with DCM (50 mL). The organic layers were mixed, dried over $Na_2SO_4$, then filtered, and the filtrate was concentrated under reduced pressure. The residue was purified by silica gel column chromatography (0 to 10% MeOH/DCM) to afford N-benzylsulfonyl-6-[4-(5-bromopyridine-3-carbonyl)piperazin-1-yl]pyridazine-3-carboxamide (INT7) as a light yellow solid (1.6 g, 50.0%).

LCMS (ESI, m/z): 545, 547 $[M+H]^+$.

Retention time: 1.075 min.

Analysis condition: PH-TFA-11.

N-Benzylsulfonyl-6-[4-(4-bromo-3-fluorobenzoyl)piperazin-1-yl]pyridazine-3-carboxamide: Synthesis of INT8

[Formula 26]

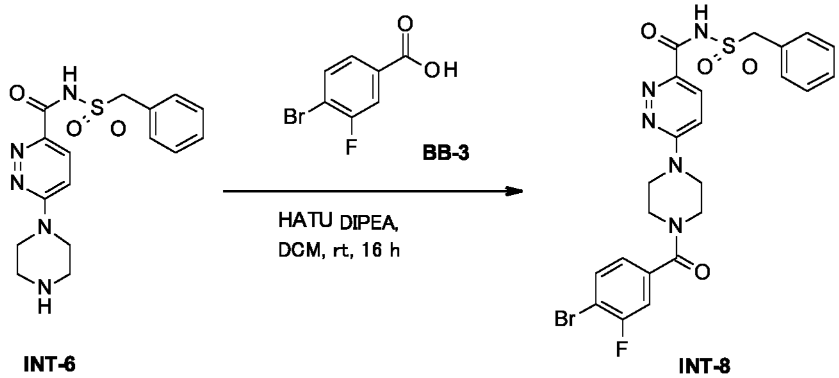


INT-6 INT-8

To a 50 mL flask under a nitrogen atmosphere were added N-benzylsulfonyl-6-piperazin-1-ylpyridazine-3-carboxamide (INT6) (1.1 g, 3.0 mmol, 1.0 eq), 4-bromo-3-fluorobenzoic acid (BB-3) (0.9 g, 4.0 mmol, 1.3 eq) and DCM (20 mL). To this solution were added HATU (1.7 g, 4.6 mmol, 1.5 eq) and DIPEA (1.6 g, 12.2 mmol, 4.0 eq), and the obtained reaction mixture was stirred at room temperature for 16 hours. A saturated aqueous $NH_4Cl$ solution (20 mL) was added to the reaction solution and the mixture was extracted three times with DCM (40 mL). The organic layers were mixed, dried over $Na_2SO_4$, then filtered, and the filtrate was concentrated under reduced pressure.

The residue was purified by silica gel column chromatography (0 to 100% ethyl acetate/petroleum ether, then 0 to 10% MeOH/DCM) to afford N-benzylsulfonyl-6-[4-(4-bromo-3-fluorobenzoyl)piperazin-1-yl]pyridazine-3-carboxamide (INT 8) as a light yellow solid (1.3 g, 74.8%).

LCMS (ESI, m/z): 562, 564 [M+H]+.

Retention time: 0.887 min.

Analysis condition: PH-TFA-10.

Example 1-1-3: Synthesis of arenol compounds B-001 to B-015

N-Benzylsulfonyl-4-[4-[[2-(4-hydroxyphenyl)phenyl]methyl]piperazin-1-yl]benzamide: Synthesis of B-001

[Formula 27]

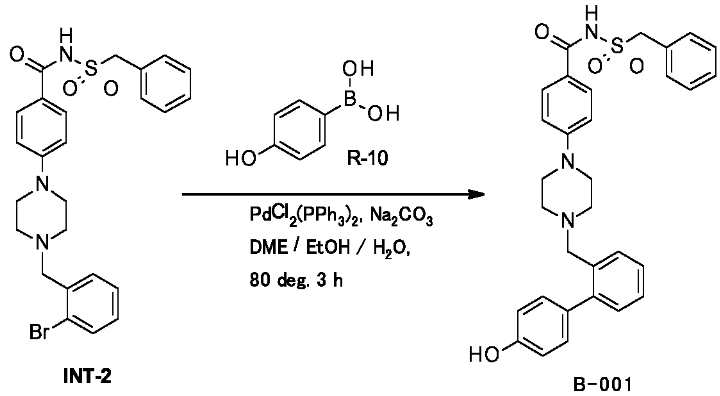


INT-2

B-001

To a 50 mL flask under a nitrogen atmosphere were added 4-[4-[(2-bromophenyl)methyl]piperazin-1-yl]benzoic acid (INT-2) (0.5 g, 0.9 mmol, 1.0 eq), 4-hydroxybenzeneboronic acid (R-10) (261 mg, 1.9 mmol, 2.0 eq), $Na_2CO_3$ (130 mg, 1.2 mmol, 1.3 eq), and a mixed solution of DME/EtOH/$H_2O$ (4.0 mL/4.0 mL/4.0 mL). To this suspension was added $PdCl_2(PPh_3)_2$ (66.4 mg, 0.09 mmol, 0.10 eq), and the obtained reaction mixture was stirred at 80°C for 3 hours under a nitrogen atmosphere. After cooling to room temperature, the reaction solution was filtered through Celite® and the Celite® was washed three times with ethyl acetate (50 mL). The filtrate was concentrated under reduced pressure and the residue was purified by silica gel column chromatography (0 to 100% ethyl acetate/petroleum ether, then 0 to 10% MeOH/DCM). The obtained mixture was further purified by reversed phase preparative HPLC (XBridge Prep OBD C18 column) (34 to 54% $CH_3CN$/10 mmol/L aqueous ammonium bicarbonate solution) to afford N-benzylsulfonyl-4-[4-[[2-(4-hydroxyphenyl)phenyl]methyl]piperazin-1-yl]benzamide (B-001) as a colorless solid (263 mg, 51.2%).

LCMS (ESI, m/z): 542 [M+H]+.

Retention time: 1.457 min.

Analysis condition: PH-TFA-03.

$^1$H-NMR (300 MHz, DMSO-$d_6$) δ 11.54 (s, 1H), 9.45 (s, 1H), 7.79 (d, J = 6.0 Hz, 2H), 7.54-7.50 (m, 1H), 7.37-7.27 (m, 7H), 7.26-7.19 (m, 3H), 6.91 (d, J = 6.0 Hz, 2H), 6.81 (d, J = 6.0 Hz, 2H), 4.70 (s, 2H), 3.43 (s, 2H), 3.30-3.23 (m, 4H), 2.46-2.38 (m, 4H).

N-Benzylsulfonyl-4-[4-[[2-(5-hydroxypyridin-3-yl)phenyl]methyl]piperazin-1-yl]benzamide: Synthesis of B-002

[Formula 28]

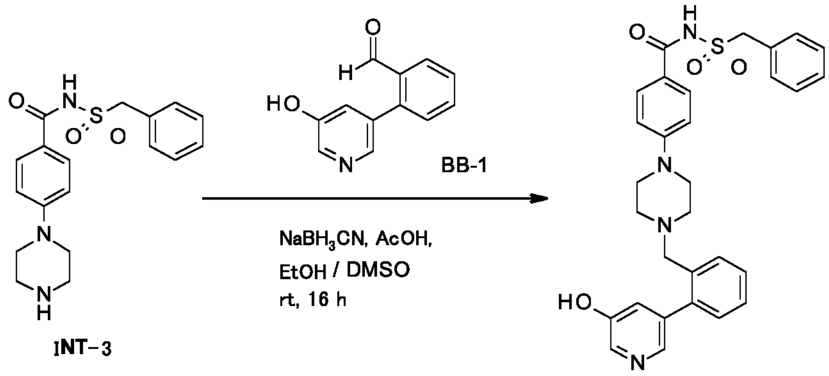


INT-3

B-002

To a 50 mL flask under a nitrogen atmosphere were added N-benzylsulfonyl-4-piperazin-1-ylbenzamide (INT-3) (0.3 g, 0.9 mmol, 1.0 eq) and a mixed solution of EtOH/DMSO (5.0 mL/5.0 mL). To this solution were added 2-(5-hydroxypyridin-3-yl)benzaldehyde (BB-1) (0.3 g, 1.7 mmol, 2.0 eq) and AcOH (0.8 mL), and the obtained reaction mixture was stirred at room temperature for 1 hour. To this reaction solution was added $NaBH_3CN$ (0.1 g, 1.7 mmol, 2.0 eq), and the mixture was stirred at room temperature for 16 hours. The reaction solution was concentrated under reduced pressure, and the obtained residue was purified by reversed phase silica gel column chromatography (C18) (0 to 80% $CH_3CN$/water) and further purified by reversed phase preparative HPLC (XBridge Prep OBD C18 column) (19 to 39% $CH_3CN$/10 mmol/L aqueous ammonium bicarbonate solution) to afford N-benzylsulfonyl-4-[4-[[2-(5-hydroxypyridin-3-yl)phenyl]methyl]piperazin-1-yl]benzamide (B-002) as an off-white solid (146.5 mg, 30.5%).

LCMS (ESI, m/z): 543 $[M+H]^+$.

Retention time: 0.752 min.

Analysis condition: PH-AC-01.

$^1$H-NMR (300 MHz, DMSO-$d_6$) $\delta$ 11.52 (s, 1H), 9.94 (s, 1H), 8.07 (dd, J = 24.9, 2.1 Hz, 2H), 7.77 (d, J = 8.7 Hz, 2H), 7.57-7.19 (m, 10H), 6.87 (d, J = 8.7 Hz, 2H), 4.65 (s, 2H), 3.39 (s, 2H), 3.26-3.15 (m, 4H), 2.45-2.33 (m, 4H).

N-Benzylsulfonyl-4-[4-[5-[2-(5-hydroxypyridin-3-yl)ethynyl]pyridine-3-carbonyl]piperazin-1-yl]benzamide: Synthesis of B-003

[Formula 29]

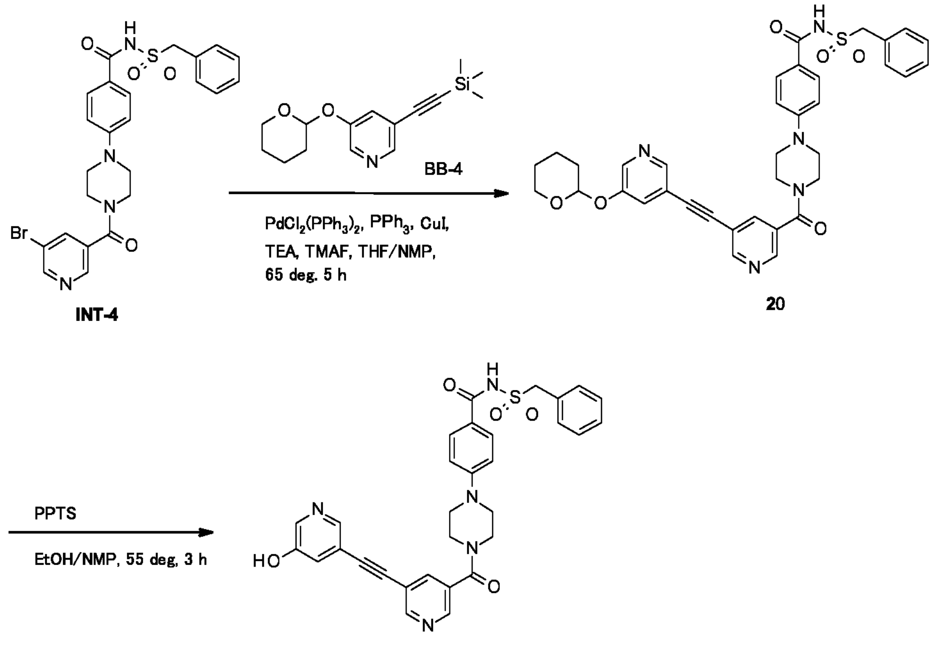


B-003

N-Benzylsulfonyl-4-[4-[5-[2-[5-(oxan-2-yloxy)pyridin-3-yl]ethynyl]pyridine-3-carbonyl]piperazin-1-yl]benzamide: Synthesis of compound 20

To a 50 mL flask under a nitrogen atmosphere were added N-benzylsulfonyl-4-[4-(5-bromopyridine-3-carbonyl)piperazin-1-yl]benzamide (INT-4) (0.5 g, 0.9 mmol, 1.0 eq), trimethyl-[2-[5-(oxan-2-yloxy)pyridin-3-yl]ethynyl]silane (BB-4) (380.1 mg, 1.4 mmol, 1.5 eq), and a mixed solution of TEA/THF/NMP (4.0 mL/5.0 mL/3.0 mL). To this solution were added CuI (17.5 mg, 0.09 mmol, 0.1 eq), $PPh_3$ (24.1 mg, 0.09 mmol, 0.1 eq), $PdCl_2(PPh_3)_2$ (64.6 mg, 0.09 mmol, 0.1 eq), and TMAF (182.2 mg, 1.1 mmol, 1.2 eq), and the obtained reaction mixture was stirred at 65°C for 5 hours under a nitrogen atmosphere. After cooling to room temperature, the reaction solution was filtered through Celite® and the Celite® was washed three times with ethyl acetate (25 mL). The filtrate was concentrated under reduced pressure and the residue was purified by silica gel column chromatography (0 to 10% MeOH/DCM) to afford N-benzylsulfonyl-4-[4-[5-[2-[5-(oxan-2-yloxy)pyridin-3-yl]ethynyl]pyridine-3-carbonyl]piperazin-1-yl]benzamide (compound 20) as a brown oil (0.6 g, 92.6%).

LCMS (ESI, m/z): 666 $[M+H]^+$.

Retention time: 1.020 min.

Analysis condition: PH-FA-02.

N-Benzylsulfonyl-4-[4-[5-[2-(5-hydroxypyridin-3-yl)ethynyl]pyridine-3-carbonyl]piperazin-1-yl]benzamide: Synthesis of B-003

To a 30 mL flask under a nitrogen atmosphere were added N-benzylsulfonyl-4-[4-[5-[2-[5-(oxan-2-yloxy)pyridin-3-yl]ethynyl]pyridine-3-carbonyl]piperazin-1-yl]benzamide (compound 20) (0.6 g, 0.9 mmol, 1.0 eq) and a mixed solution of EtOH/NMP (5.0 mL/3.0 mL). To this solution was added PPTS (0.5 g, 1.9 mmol, 2.0 eq), and the obtained reaction mixture was stirred at 55°C for 3 hours. The reaction solution was concentrated under reduced pressure and the obtained residue was purified by reversed phase preparative HPLC (Xselect CSH OBD column) (23 to 53% $CH_3CN$/0.1% aqueous formic acid solution) to afford N-benzylsulfonyl-4-[4-[5-[2-(5-hydroxypyridin-3-yl)ethynyl]pyridine-3-carbonyl]piperazin-1-yl]benzamide (B-003) as a light yellow solid (171.2 mg, 30.5%).

LCMS (ESI, m/z): 582 $[M+H]^+$.

Retention time: 1.426 min.

Analysis condition: PH-TFA-03.

$^1H$-NMR (300 MHz, DMSO-$d_6$) $\delta$ 11.61 (s, 1H), 10.35 (s, 1H), 8.87 (d, J = 2.1 Hz, 1H), 8.69 (d, J = 2.1 Hz, 1H), 8.26 (d, J = 1.8 Hz, 1H), 8.23-8.13 (m, 1H), 8.12 (d, J = 2.1 Hz, 1H), 7.83 (d, J = 9.0 Hz, 2H), 7.34 (m, J = 8.8, 5.6, 2.7 Hz, 6H), 6.98 (d, J = 9.0 Hz, 2H), 4.83 (s, 2H), 3.77 (s, 2H), 3.46 (d, J = 20.7 Hz, 6H).

N-Benzylsulfonyl-4-[4-[3-fluoro-4-(5-hydroxypyridin-3-yl)benzoyl]piperazin-1 - yl]benzamide: Synthesis of B-004

[Formula 30]

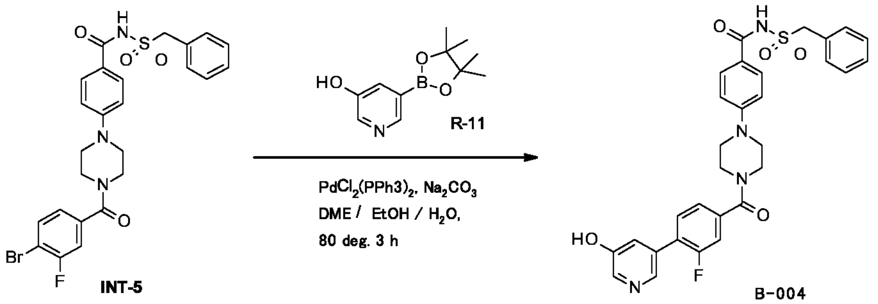


INT-5

B-004

To a 50 mL flask under a nitrogen atmosphere were added N-benzylsulfonyl-4-[4-(4-bromo-3-fluorobenzoyl)piperazin-1-yl]benzamide (INT-5) (0.3 g, 0.6 mmol, 1.0 eq), 3-hydroxy-5-pyridineboronic acid pinacol ester (R-11) (276.1 mg, 1.2 mmol, 2.0 eq), $Na_2CO_3$ (86.0 mg, 0.8 mmol, 1.3 eq), and a mixed solution of DME/EtOH/$H_2O$ (6.0 mL/6.0 mL/6.0 mL). To this suspension was added $PdCl_2(PPh_3)_2$ (43.8 mg, 0.06 mmol, 0.1 eq), and the obtained reaction mixture was stirred at 80°C for 3 hours under a nitrogen atmosphere. After cooling to room temperature, the reaction solution was filtered through Celite® and the Celite® was washed three times with ethyl acetate (25 mL). The filtrate was concentrated under reduced pressure, and the obtained residue was purified by reversed phase silica gel column chromatography (C18) (0 to 80% $CH_3CN$/water) and further purified by reversed phase preparative HPLC (YMC-Actus Triart C18) (20 to 50% $CH_3CN$/10 mmol/L aqueous ammonium bicarbonate solution) to afford N-benzylsulfonyl-4-[4-[3-fluoro-4-(5-hydroxypyridin-3-yl)benzoyl]piperazin-1-yl]benzamide (B-004) as an off-white solid (168.4 mg, 46.5%).

LCMS (ESI, m/z): 575 $[M+H]^+$.

Retention time: 2.138 min.

Analysis condition: PH-TFA-04.

$^1$H-NMR (300 MHz, DMSO-$d_6$) $\delta$ 11.61 (s, 1H), 10.18 (s, 1H), 8.25-8.20 (m, 2H), 7.82 (d, J = 8.7 Hz, 2H), 7.65 (t, J = 8.1 Hz, 1H), 7.49-7.30 (m, 8H), 6.93 (d, J = 8.7 Hz, 2H), 4.64 (s, 2H), 3.76-3.32 (m, 8H).

N-Benzylsulfonyl-6-[4-[5-[2-(5-hydroxypyridin-3-yl)ethynyl]pyridine-3-carbonyl]piperazin-1-yl]pyridazine-3-carboxamide: Synthesis of B-005

[Formula 31]

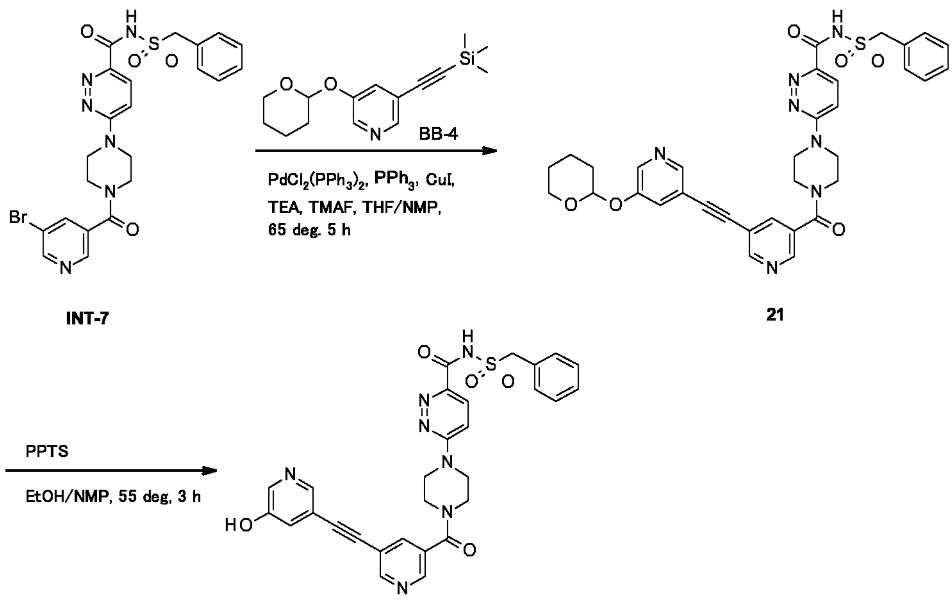


INT-7

21

B-005

N-Benzylsulfonyl-6-[4-[5-[2-[5-(oxan-2-yloxy)pyridin-3-yl]ethynyl]pyridine-3-carbonyl]piperazin-1-yl]pyridazine-3-carboxamide: Synthesis of compound 21

To a 50 mL flask under a nitrogen atmosphere were added N-benzylsulfonyl-6-[4-(5-bromopyridine-3-carbonyl)piperazin-1-yl]pyridazine-3-carboxamide (INT-7) (0.5 g, 0.9 mmol, 1.0 eq), trimethyl-[2-[5-(oxan-2-yloxy)pyridin-3-yl]ethynyl]silane (BB-4) (387 mg, 1.4 mmol, 1.5 eq), and a mixed solution of TEA/THF/NMP (4.0 mL/5.0 mL/3.0 mL). To this solution were added CuI (17.8 mg, 0.09 mmol, 0.1 eq), $PPh_3$ (24.5 mg, 0.09 mmol, 0.1 eq), $PdCh(PPh_3)_2$ (66.0 mg, 0.09 mmol, 0.1 eq), and TMAF (185.2 mg, 1.1 mmol, 1.2 eq), and the obtained reaction mixture was stirred at 65°C for 5 hours under a nitrogen atmosphere. After cooling to room temperature, the reaction solution was filtered through Celite® and the Celite® was washed three times with ethyl acetate (25 mL). The filtrate was concentrated under reduced pressure and the residue was purified by reversed phase silica gel column chromatography (C18) (30 to 95% $CH_3CN$/water) to afford N-benzylsulfonyl-6-[4-[5-[2-[5-(oxan-2-yloxy)pyridin-3-yl]ethynyl]pyridine-3-carbonyl]piperazin-1-yl]pyridazine-3-carboxamide (compound 21) as a yellow solid (0.4 g, 57.6%).

LCMS (ESI, m/z): 668 $[M+H]^+$.

Retention time: 0.983 min.

Analysis condition: PH-FA-02.

N-Benzylsulfonyl-6-[4-[5-[2-(5-hydroxypyridin-3-yl)ethynyl]pyridine-3-carbonyl]piperazin-1-yl]pyridazine-3-carboxamide: Synthesis of B-005

To a 30 mL flask under a nitrogen atmosphere were added N-benzylsulfonyl-6-[4-[5-[2-[5-(oxan-2-yloxy)pyridin-3-yl]ethynyl]pyridine-3-carbonyl]piperazin-1-yl]pyridazine-3-carboxamide (compound 21) (0.4 g, 0.6 mmol, 1.0 eq) and a mixed solution of EtOH/NMP (5.0 mL/3.0 mL). To this solution was added PPTS (0.3 g, 1.2 mmol, 2.0 eq), and the obtained reaction mixture was stirred at 55°C for 3 hours. The reaction solution was concentrated under reduced pressure and the obtained residue was purified by reversed phase preparative HPLC (XBridge C18 OBD Prep Column) (33 to 50% $CH_3CN$/0.1% aqueous formic acid solution) to afford N-benzylsulfonyl-6-[4-[5-[2-(5-hydroxypyridin-3-yl)ethynyl]pyridine-3-carbonyl]piperazin-1-yl]pyridazine-3-carboxamide (B-005) as a light yellow solid (88.9 mg, 25.3%).

LCMS (ESI, m/z): 584 $[M+H]^+$.

Retention time: 1.350 min.

Analysis condition: PH-TFA-03.

$^1$H-NMR (400 MHz, DMSO-$d_6$) $\delta$ 11.95 (br, 1H), 10.36 (s, 1H), 8.88-8.70 (m, 2H), 8.48-8.05 (m, 2H), 7.95 (d, J = 9.6 Hz, 1H), 7.65-7.20 (m, 6H), 4.83 (s, 2H), 4.18-3.67 (m, 5H), 3.46-3.01 (m, 3H).

N-Benzylsulfonyl-6-[4-[3-fluoro-4-(5-hydroxypyridin-3-yl)benzoyl]piperazin-1 - yl]pyridazine-3-carboxamide: Synthesis of B-006

[Formula 32]

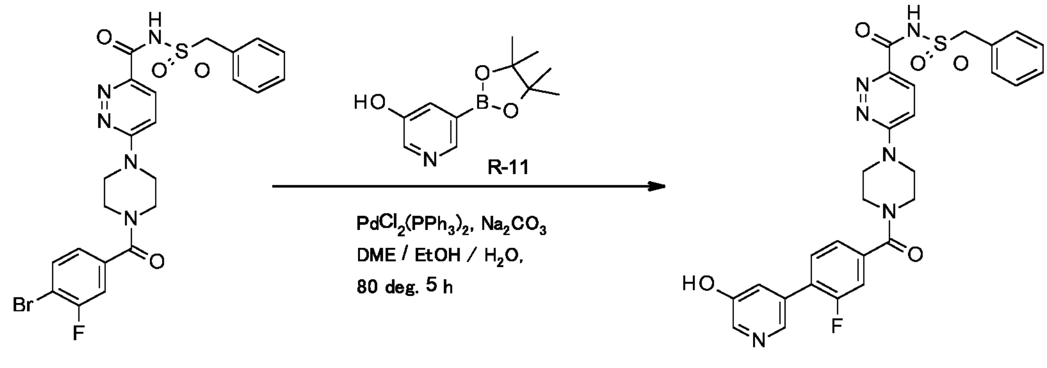


INT-8 B-006

To a 50 mL flask under a nitrogen atmosphere were added N-benzylsulfonyl-6-[4-(4-bromo-3-fluorobenzoyl)piperazin-1-yl]pyridazine-3-carboxamide (INT-8) (0.3 g, 0.6 mmol, 1.0 eq), 3-hydroxy-5-pyridineboronic acid pinacol ester (R-11) (275.0 mg, 1.2 mmol, 2.0 eq), $Na_2CO_3$ (85.8 mg, 0.8 mmol, 1.3 eq), and a mixed solution of DME/EtOH/$H_2O$ (5.0 mL/5.0 mL/5.0 mL). To this suspension was added $PdCl_2(PPh_3)_2$ (43.9 mg, 0.06 mmol, 0.1 eq), and the obtained reaction mixture was stirred at 80°C for 5 hours under a nitrogen atmosphere. After cooling to room temperature, the reaction solution was filtered through Celite® and the Celite® was washed three times with ethyl acetate (25 mL). The filtrate was concentrated under reduced pressure, and the obtained residue was purified by reversed phase silica gel column chromatography (C18) (0 to 80% $CH_3CN$/water) and further purified by reversed phase preparative HPLC (XBridge Prep OBD C18 column) (10 to 41% $CH_3CN$/10 mmol/L aqueous ammonium bicarbonate solution) to afford N-benzylsulfonyl-6- [4- [3 -fluoro-4-(5 -hydroxypyridin-3 -yl)benzoyl]piperazin-1 - yl]pyridazine-3-carboxamide (B-006) as a light brown solid (66.9 mg, 18.6%).

LCMS (ESI, m/z): 577 $[M+H]^+$.

Retention time: 2.573 min.

Analysis condition: PH-TFA-05.

$^1$H-NMR (300 MHz, DMSO-$d_6$) $\delta$ 10.18 (s, 1H), 8.26-8.19 (m, 2H), 7.89 (d, J = 9.6 Hz, 1H), 7.68 (t, J = 8.1 Hz, 1H), 7.53-7.21 (m, 10H), 4.66 (s, 2H), 3.90-3.44 (m, 8H).

N-Benzylsulfonyl-6-[4-[3-fluoro-4-(4-hydroxyphenyl)benzoyl]piperazin-1 - yl]pyridazine-3-carboxamide: Synthesis of B-007

[Formula 33]

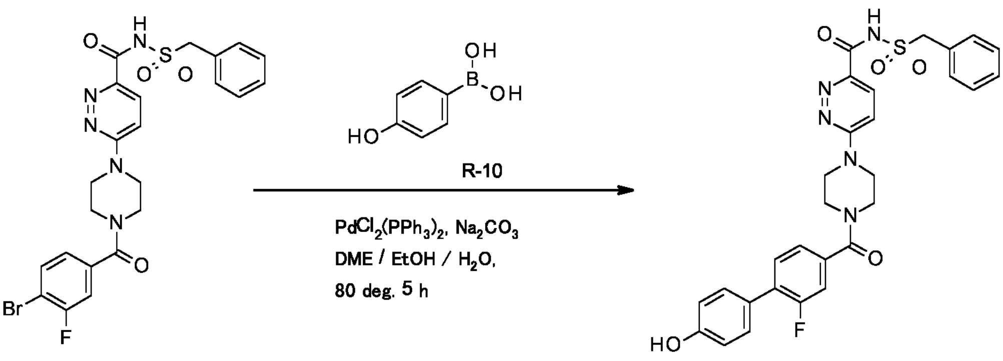


INT-8 B-007

To a 100 mL flask under a nitrogen atmosphere were added N-benzylsulfonyl-6-[4-(4-bromo-3-fluorobenzoyl)piperazin-1-yl]pyridazine-3-carboxamide (INT-8) (0.5 g, 1.0 mmol, 1.0 eq), 4-hydroxybenzeneboronic acid (R-10) (269.8 mg, 2.0 mmol, 2.0 eq), $Na_2CO_3$ (134.7 mg, 1.3 mmol, 1.3 eq), and a mixed solution of DME/EtOH/$H_2O$ (7.0

mL/7.0 mL/7.0 mL). To this suspension was added $PdCl_2(PPh_3)_2$ (68.6 mg, 0.1 mmol, 0.1 eq), and the obtained reaction mixture was stirred at 80°C for 5 hours under a nitrogen atmosphere. After cooling to room temperature, the reaction solution was filtered through Celite® and the Celite® was washed three times with ethyl acetate (50 mL). The filtrate was concentrated under reduced pressure and the residue was purified by reversed phase silica gel column chromatography (C18) (0 to 80% $CH_3CN$/water). The obtained mixture was further purified by reversed phase preparative HPLC (XBridge Prep Phenyl OBD column) (44 to 60% $CH_3CN$/10 mmol/L aqueous ammonium bicarbonate solution) to afford N-benzylsulfonyl-6-[4-[3-fluoro-4-(4-hydroxyphenyl)benzoyl]piperazin-1-yl]pyridazine-3-carboxamide (B-007) as an off-white solid (59.8 mg, 10.6%).

LCMS (ESI, m/z): 576 $[M+H]^+$.

Retention time: 2.018 min.

Analysis condition: PH-TFA-06.

$^1$H-NMR (300 MHz, DMSO-$d_6$) $\delta$ 9.73 (s, 1H), 7.92 (d, J = 6.6 Hz, 1H), 7.60-7.54 (m, 1H), 7.44-7.34 (m, 10H), 6.90-6.87 (m, 2H), 4.78 (s, 2H), 3.92-3.51 (m, 8H).

N-Benzylsulfonyl-4-[4-[5-[2-(4-hydroxyphenyl)ethynyl]pyridine-3-carbonyl]piperazin-1-yl]benzamide: Synthesis of B-008

[Formula 34]

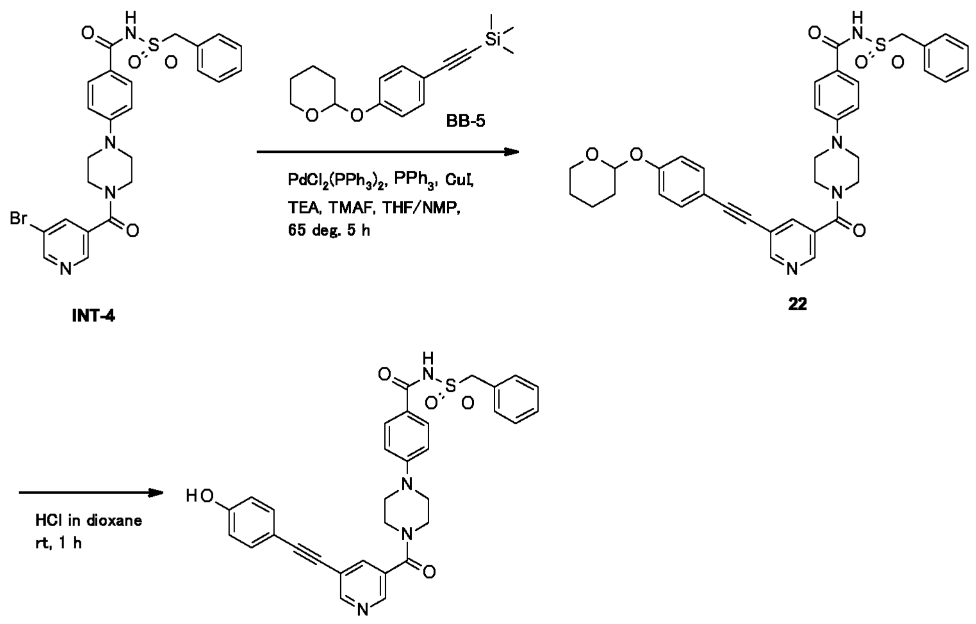


INT-4

22

B-008

N-Benzylsulfonyl-4-[4-[5-[2-[4-(oxan-2-yloxy)phenyl]ethynyl]pyridine-3-carbonyl]piperazin-1-yl]benzamide: Synthesis of compound 22

To a 30 mL flask under a nitrogen atmosphere were added N-benzylsulfonyl-4-[4-(5-bromopyridine-3-carbonyl)piperazin-1-yl]benzamide (INT-4) (0.5 g, 0.9 mmol, 1.0 eq), trimethyl-[2-[4-(oxan-2-yloxy)phenyl]ethynyl]silane (BB-5) (378.8 mg, 1.4 mmol, 1.5 eq), and a mixed solution of TEA/THF/NMP (4.0 mL/5.0 mL/3.0 mL). To this solution were added CuI (17.5 mg, 0.09 mmol, 0.1 eq), $PPh_3$ (24.1 mg, 0.09 mmol, 0.1 eq), $PdCl_2(PPh_3)_2$ (64.6 mg, 0.09 mmol, 0.1 eq), and TMAF (182.0 mg, 1.4 mmol, 1.2 eq), and the obtained reaction mixture was stirred at 65°C for 5 hours under a nitrogen atmosphere. After cooling to room temperature, the reaction solution was filtered through Celite® and the Celite® was washed three times with ethyl acetate (25 mL). The filtrate was concentrated under reduced pressure and the residue was purified by silica gel column chromatography (0 to 10% MeOH/DCM) to afford N-benzylsulfonyl-4-[4-[5-[2-[4-(oxan-2-yloxy)phenyl]ethynyl]pyridine-3-carbonyl]piperazin-1-yl]benzamide (compound 22) as a brown oil (0.7 g, 95.0%).

LCMS (ESI, m/z): 665 $[M+H]^+$.

Retention time: 1.744 min.

Analysis condition: PH-FA-03.

N-Benzylsulfonyl-4-[4-[5-[2-(4-hydroxyphenyl)ethynyl]pyridine-3-carbonyl]piperazin-1-yl]benzamide: Synthesis of B-008

To a 30 mL flask under a nitrogen atmosphere were added N-benzylsulfonyl-4-[4-[5-[2-[4-(oxan-2-yloxy)phenyl]ethynyl]pyridine-3-carbonyl]piperazin-1-yl]benzamide (compound 22) (0.7 g, 1.0 mmol, 1.0 eq) and a 4M solution of HCl in 1,4-dioxane (5 mL). This solution was stirred at room temperature for 1 hour. The reaction solution was concentrated under reduced pressure, and the obtained residue was purified by silica gel column chromatography (0 to 10% MeOH/DCM) and further purified by reversed phase preparative HPLC (XBridge Prep OBD C18 column) (46 to 63% $CH_3CN$/10 mmol/L aqueous ammonium bicarbonate solution) to afford N-benzylsulfonyl-4-[4-[5-[2-(4-hydroxyphenyl)ethynyl]pyridine-3-carbonyl]piperazin-1-yl]benzamide (B-008) as a light yellow solid (94.7 mg, 15.5%).
LCMS (ESI, m/z): 581 $[M+H]^+$.
Retention time: 0.816 min.
Analysis condition: PH-AC-01.
$^1$H-NMR (400 MHz, DMSO-$d_6$) $\delta$ 11.61 (s, 1H), 10.05 (s, 1H), 8.79 (d, J = 2.0 Hz, 1H), 8.62 (d, J = 2.1 Hz, 1H), 8.02 (t, J = 2.1 Hz, 1H), 7.83 (d, J = 8.5 Hz, 2H), 7.44 (d, J = 8.2 Hz, 2H), 7.32 (s, 5H), 7.30 (d, J = 6.3 Hz, 1H), 6.96 (d, J = 8.6 Hz, 2H), 6.83 (d, J = 8.3 Hz, 2H), 4.70 (s, 2H), 3.77 (s, 2H), 3.42 (m, 5H).

N-Benzylsulfonyl-4-[4-[[2-[2-(5-hydroxypyridin-3-yl)ethynyl]phenyl]methyl]piperazin-1-yl]benzamide: Synthesis of B-009

[Formula 35]

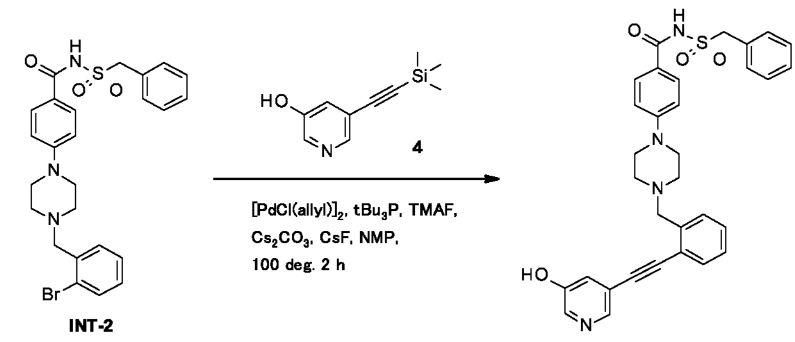


To a 100 mL flask under a nitrogen atmosphere were added N-benzylsulfonyl-4-[4-[(2-bromophenyl)methyl]piperazin-1-yl]benzamide (INT-2) (2.0 g, 3.8 mmol, 1.0 eq), 5-(2-trimethylsilylethynyl)pyridin-3-ol (compound 4) (2.9 g, 15.1 mmol, 4.0 eq), $Cs_2CO_3$ (3.7 g, 11.2 mmol, 3.0 eq), and NMP (20 mL). To this solution were added $[PdCl(allyl)]_2$ (69.2 mg, 0.2 mmol, 0.05 eq), $tBu_3P$ (153 mg, 0.8 mmol, 0.2 eq), CsF (1.1 g, 7.6 mmol, 2.0 eq), and TMAF (749 mg, 4.5 mmol, 1.2 eq), and the obtained reaction mixture was stirred at 100°C for 2 hours under a nitrogen atmosphere. After cooling to room temperature, the reaction solution was filtered through Celite® and the Celite® was washed three times with ethyl acetate (25 mL). The filtrate was concentrated under reduced pressure, and purification by reversed phase silica gel column chromatography (C18) (5 to 95% $CH_3CN$/water), purification by reversed phase preparative HPLC (Xselect CSH OBD column) (14 to 39% $CH_3CN$/0.1% aqueous formic acid solution), and further purification by silica gel column chromatography (0 to 10% EtOH/DCM) afforded N-benzylsulfonyl-4-[4-[[2-[2-(5-hydroxypyridin-3-yl)ethynyl]phenyl]methyl]piperazin-1-yl]benzamide (B-009) as an off-white solid (91 mg, 4.2%).
LCMS (ESI, m/z): 567 $[M+H]^+$.
Retention time: 0.772 min.
Analysis condition: PH-AC-01.
$^1$H-NMR (400 MHz, DMSO-$d_6$) $\delta$ 11.48 (s, 1H), 10.24 (s, 1H), 8.23 (d, J = 1.6 Hz, 1H), 8.16 (d, J = 2.8 Hz, 1H), 7.84-7.76 (m, 2H), 7.56 (m, 2H), 7.45 (m, 1H), 7.41-7.27 (m, 7H), 6.99-6.92 (m, 2H), 4.81 (s, 2H), 3.79 (s, 2H), 3.45-3.30 (m, 4H), 2.65-2.60 (m, 4H).

N-Benzylsulfonyl-4-[4-[[2-[2-(4-hydroxyphenyl)ethynyl]phenyl]methyl]piperazin-1-yl]benzamide: Synthesis of B-010

[Formula 36]

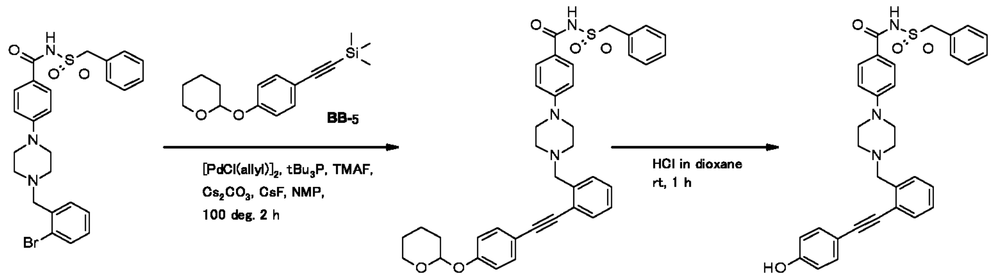


INT-2 23 B-010

N-Benzylsulfonyl-4-[4-[[2-[2-[4-(oxan-2-yloxy)phenyl]ethynyl]phenyl]methyl]piperazin-1-yl]benzamide: Synthesis of compound 23

To a 30 mL flask under a nitrogen atmosphere were added INT-2 (0.7 g, 1.3 mmol, 1.0 eq), BB-5 (1.4 g, 5.3 mmol, 4.0 eq), $Cs_2CO_3$ (1.3 g, 4.0 mmol, 3.0 eq), and NMP (7.0 mL). To this solution were added $[PdCl(allyl)]_2$ (24.2 mg, 0.06 mmol, 0.05 eq), $tBu_3P$ (53.5 mg, 0.3 mmol, 0.2 eq), CsF (402 mg, 2.6 mmol, 2.0 eq), and TMAF (262 mg, 1.6 mmol, 1.2 eq), and the obtained reaction mixture was stirred at 100°C for 2 hours under a nitrogen atmosphere. After cooling to room temperature, the reaction solution was filtered through Celite® and the Celite® was washed three times with ethyl acetate (25 mL). The filtrate was concentrated under reduced pressure and the residue was purified by silica gel column chromatography (0 to 100% ethyl acetate/petroleum ether) to afford N-benzylsulfonyl-4-[4-[[2-[2-[4-(oxan-2-yloxy)phenyl]ethynyl]phenyl]methyl]piperazin-1-yl]benzamide (compound 23) as a brown solid (680 mg, 63.2%).
LCMS (ESI, m/z): 650 $[M+H]^+$.
Retention time: 0.926 min.
Analysis condition: PH-FA-02.

N-Benzylsulfonyl-4-[4-[[2-[2-(4-hydroxyphenyl)ethynyl]phenyl]methyl]piperazin-1-yl]benzamide: Synthesis of B-010

To a 30 mL flask under a nitrogen atmosphere were added N-benzylsulfonyl-4-[4-[[2-[2-[4-(oxan-2-yloxy)phenyl]ethynyl]phenyl]methyl]piperazin-1-yl]benzamide (compound 23) (0.4 g, 0.6 mmol, 1.0 eq) and a 4M solution of HCl in 1,4-dioxane (5.0 mL). This solution was stirred at room temperature for 1 hour. The reaction solution was concentrated under reduced pressure, and the obtained residue was purified by reversed phase preparative HPLC (C18) (25 to 55% $CH_3CN$/10 mmol/L aqueous ammonium bicarbonate solution) and purified by reversed phase silica gel column chromatography (XBridge Prep OBD C18 column) (5 to 95% $CH_3CN$/water) to afford N-benzylsulfonyl-4-[4-[[2-[2-(4-hydroxyphenyl)ethynyl]phenyl]methyl]piperazin-1-yl]benzamide (B-010) as a light yellow solid (92.5 mg, 23.5%).
LCMS (ESI, m/z): 566 $[M+H]^+$.
Retention time: 1.010 min.
Analysis condition: PH-AC-01.
$^1$H-NMR (300 MHz, DMSO-$d_6$) $\delta$ 11.50 (s, 1H), 9.93 (s, 1H), 7.81 (d, J = 8.7 Hz, 2H), 7.51 (d, J = 7.2 Hz, 2H), 7.43-7.27 (m, 9H), 6.96 (d, J = 9.0 Hz, 2H), 6.82 (d, J = 8.7 Hz, 2H), 4.81 (s, 2H), 3.79 (s, 2H), 3.33 (s, 4H), 2.63 (s, 4H).

4-[4-[[2-(4-Hydroxyphenyl)phenyl]methyl]piperazin-1-yl]-N-[3-methyl-4-(2-phenylsulfanylethylamino)phenyl]sulfonylbenzamide: Synthesis of B-011

[Formula 37]

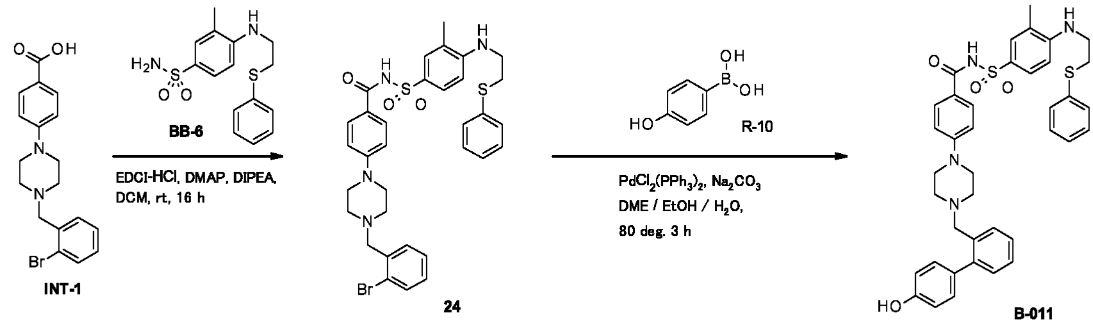


INT-1 24 B-011

4-[4-[(2-Bromophenyl)methyl]piperazin-1-yl]-N-[3-methyl-4-(2-phenylsulfanylethylamino)phenyl]sulfonylbenzamide: Synthesis of compound 24

To a 100 mL flask under a nitrogen atmosphere were added 4-[4-[(2-bromophenyl)methyl]piperazin-1-yl]benzoic acid (INT1) (1.7 g, 4.6 mmol, 1.5 eq), 3-methyl-4-(2-phenylsulfanylethylamino)benzenesulfonamide (BB-6) (1.0 g, 3.1 mmol, 1.0 eq), DMAP (0.4 g, 3.1 mmol, 1.0 eq), and DCM (34 mL). To this solution were added EDCI·HCl (1.2 g, 6.2 mmol, 2.0 eq) and DIPEA (1.6 g, 12.4 mmol, 4.0 eq), and the obtained reaction mixture was stirred at room temperature for 16 hours. A saturated aqueous $NH_4Cl$ solution (30 mL) was added to the reaction solution and the mixture was extracted three times with DCM (40 mL). The organic layers were mixed, dried over $Na_2SO_4$, then filtered, and the filtrate was concentrated under reduced pressure.

The residue was purified by silica gel column chromatography (20 to 100% ethyl acetate/petroleum ether) to afford 4-[4-[(2-bromophenyl)methyl]piperazin-1-yl]-N-[3-methyl-4-(2-phenylsulfanylethylamino)phenyl]sulfonylbenzamide (compound 24) as a light yellow solid (1.1 g, 49.6%).

LCMS (ESI, m/z): 679, 681 $[M+H]^+$.

Retention time: 1.242 min.

Analysis condition: PH-TFA-01.

4-[4-[[2-(4-Hydroxyphenyl)phenyl]methyl]piperazin-1-yl]-N-[3-methyl-4-(2-phenylsulfanylethylamino)phenyl]sulfonylbenzamide: Synthesis of B-011

To a 50 mL flask under a nitrogen atmosphere were added 4-[4-[(2-bromophenyl)methyl]piperazin-1-yl]-N-[3-methyl-4-(2-phenylsulfanylethylamino)phenyl]sulfonylbenzamide (compound 24) (0.3 g, 0.4 mmol, 1.0 eq), 4-hydroxybenzeneboronic acid (R-10) (121.8 mg, 0.9 mmol, 2.0 eq), $Na_2CO_3$ (60.8 mg, 0.6 mmol, 1.3 eq), and a mixed solution of DME/EtOH/$H_2O$ (4.0 mL/4.0 mL/4.0 mL). To this suspension was added $PdCl_2(PPh_3)_2$ (31.0 mg, 0.04 mmol, 0.1 eq), and the obtained reaction mixture was stirred at 80°C for 3 hours under a nitrogen atmosphere. After cooling to room temperature, the reaction solution was filtered through Celite® and the Celite® was washed three times with ethyl acetate (50 mL). The filtrate was concentrated under reduced pressure and the residue was purified by silica gel column chromatography (0 to 100% ethyl acetate/petroleum ether, then 0 to 10% MeOH/DCM). The obtained crude product was further purified by reversed phase preparative HPLC (XBridge Shield RP18 OBD column) (37 to 59% $CH_3CN$/10 mmol/L aqueous ammonium bicarbonate solution) to afford 4-[4-[[2-(4-hydroxyphenyl)phenyl]methyl]piperazin-1-yl]-N-[3-methyl-4-(2-phenylsulfanylethylamino)phenyl]sulfonylbenzamide (B-011) as an off-white solid (37.9 mg, 12.3%).

LCMS (ESI, m/z): 693 $[M+H]^+$.

Retention time: 1.196 min.

Analysis condition: PH-AC-01.

$^1$H-NMR (300 MHz, DMSO-$d_6$) δ 11.66 (s, 1H), 9.46 (s, 1H), 7.72 (d, J = 8.7 Hz, 2H), 7.61-7.56 (m, 1H), 7.52-7.46 (m, 2H), 7.40-7.28 (m, 6H), 7.26-7.14 (m, 4H), 6.89 (d, J = 9.0 Hz, 2H), 6.86-6.75 (m, 2H), 6.57 (d, J = 8.7 Hz, 1H), 5.99 (bs, 1H), 3.46-3.36 (m, 4H), 3.28-3.22 (m, 4H), 3.21-3.14 (m, 2H), 2.43-2.37 (m, 4H), 2.09 (s, 3H).

4-[4-[[2-(4-Hydroxyphenyl)phenyl]methyl]piperazin-1-yl]-N-[3-nitro-4-(2-phenylsulfanylethylamino)phenyl]sulfonylbenzamide: Synthesis of B-012

[Formula 38]

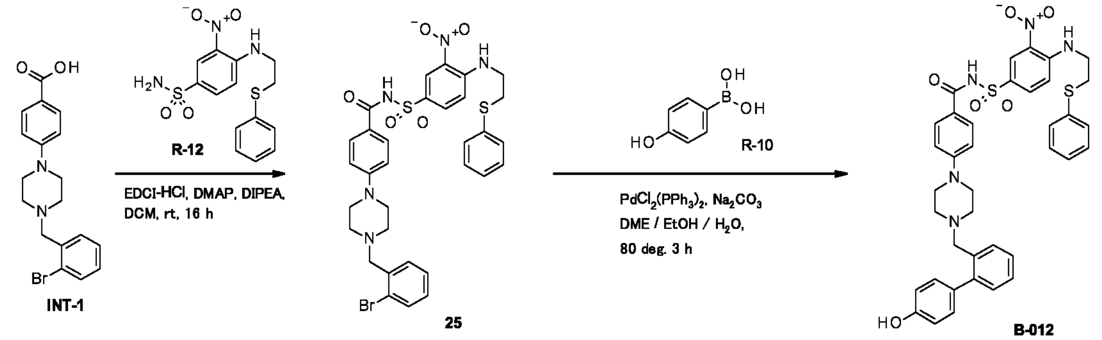


4-[4-[(2-Bromophenyl)methyl]piperazin-1-yl]-N-[3-nitro-4-(2-phenylsulfanylethylamino)phenyl]sulfonylbenzamide: Synthesis of compound 25

To a 100 mL flask under a nitrogen atmosphere were added 4-[4-[(2-bromophenyl)methyl]piperazin-1-yl]benzoic acid (INT1) (1.1 g, 2.8 mmol, 1.4 eq), 3-nitro-4-(2-phenylsulfanylethylamino)benzenesulfonamide (R-12) (0.7 g, 2.1 mmol, 1.0 eq), DMAP (0.3 g, 2.1 mmol, 1.0 eq), and DCM (20 mL). To this solution were added EDCI·HCl (0.8 g, 4.2 mmol, 2.0 eq) and DIPEA (1.1 g, 8.5 mmol, 4.0 eq), and the obtained reaction mixture was stirred at room temperature for 16 hours. A saturated aqueous $NH_4Cl$ solution (20 mL) was added to the reaction solution and the mixture was extracted three times with DCM (30 mL). The organic layers were mixed, dried over $Na_2SO_4$, then filtered, and the filtrate was concentrated under reduced pressure.

The residue was purified by reversed phase silica gel column chromatography (C18) (0 to 80% $CH_3CN$/0.5 g/L aqueous ammonium bicarbonate solution) to afford 4-[4-[(2-bromophenyl)methyl]piperazin-1-yl]-N-[3-nitro-4-(2-phenylsulfanylethylamino)phenyl]sulfonylbenzamide (compound 25) as a yellow solid (554.7 mg, 36.8%).

LCMS (ESI, m/z): 710, 712 $[M+H]^+$.

Retention time: 1.166 min.

Analysis condition: PH-TFA-02.

Note that compound R-12 used in Example 1-1-3, synthesis method for compound 25, may also be denoted as compound BB41 in the present specification.

4-[4-[[2-(4-Hydroxyphenyl)phenyl]methyl]piperazin-1-yl]-N-[3-nitro-4-(2-phenylsulfanylethylamino)phenyl]sulfonylbenzamide: Synthesis of B-012

To a 50 mL flask under a nitrogen atmosphere were added 4-[4-[(2-bromophenyl)methyl]piperazin-1-yl]-N-[3-nitro-4-(2-phenylsulfanylethylamino)phenyl]sulfonylbenzamide (compound 25) (0.3 g, 0.4 mmol, 1.0 eq), 4-hydroxybenzeneboronic acid (R-10) (72.8 mg, 0.5 mmol, 1.5 eq), $Na_2CO_3$ (48.5 mg, 0.5 mmol, 1.3 eq), and a mixed solution of DME/EtOH/$H_2O$ (5.0 mL/5.0 mL/5.0 mL). To this suspension was added $PdCl_2(PPh_3)_2$ (24.7 mg, 0.03 mmol, 0.1 eq), and the obtained reaction mixture was stirred at 80°C for 3 hours under a nitrogen atmosphere. After cooling to room temperature, the reaction solution was filtered through Celite® and the Celite® was washed three times with ethyl acetate (50 mL). The filtrate was concentrated under reduced pressure and the residue was purified by reversed phase silica gel column chromatography (C18) (0 to 80% $CH_3CN$/water). The obtained crude product was further purified by reversed phase preparative HPLC (C18) (34 to 54% $CH_3CN$/10 mmol/L aqueous ammonium bicarbonate solution) to afford 4-[4-[[2-(4-hydroxyphenyl)phenyl]methyl]piperazin-1 -yl]-N-[3-nitro-4-(2-phenylsulfanylethylamino)phenyl]sulfonylbenzamide (B-012) as a yellow solid (35.4 mg, 13.8%).

LCMS (ESI, m/z): 724 $[M+H]^+$.

Retention time: 2.248 min.

Analysis condition: PH-AC-02.

$^1$H-NMR (300 MHz, DMSO-$d_6$) $\delta$ 11.90 (s, 1H), 9.44 (s, 1H), 8.66 (s, 1H), 8.55 (d, J = 2.1 Hz, 1H), 7.88 (dd, J = 9.0, 2.4 Hz, 1H), 7.72 (d, J = 8.7 Hz, 2H), 7.55-7.46 (m, 1H), 7.41-7.34 (m, 2H), 7.30 (m, 3H), 7.26-7.07 (m, 6H), 6.91-6.76 (m, 4H), 3.70-3.57 (m, 2H), 3.43 (s, 2H), 3.28-3.19 (m, 6H), 2.42 (s, 4H).

4-[4-[[2-(3-Hydroxyphenyl)phenyl]methyl]piperazin-1-yl]-N-[3-nitro-4-(2-phenylsulfanylethylamino)phenyl]sulfonylbenzamide: Synthesis of B-013

[Formula 39]

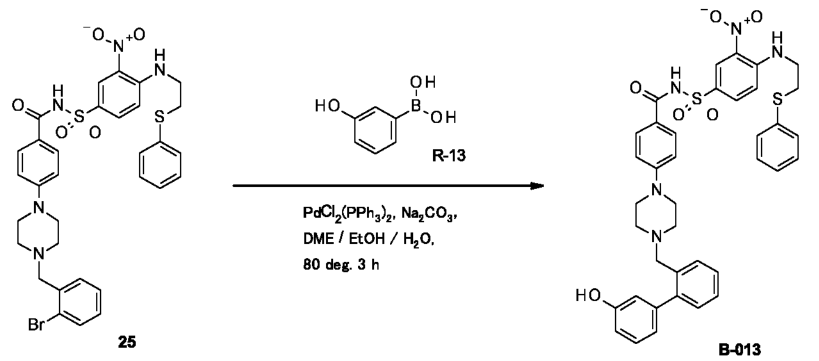


To a 50 mL flask under a nitrogen atmosphere were added 4-[4-[(2-bromophenyl)methyl]piperazin-1-yl]-N-[3-nitro-4-(2-phenylsulfanylethylamino)phenyl]sulfonylbenzamide (compound 25) (0.2 g, 0.3 mmol, 1.0 eq), 3-hydroxybenzeneboronic acid (R-13) (89.3 mg, 0.6 mmol, 2.0 eq), $Na_2CO_3$ (44.6 mg, 0.4 mmol, 1.3 eq), and a mixed solution of DME/EtOH/$H_2O$ (1/1/1, 5.0 mL). To this suspension was added $PdCl_2(PPh_3)_2$ (22.7 mg, 0.03 mmol, 0.1 eq), and the obtained reaction mixture was stirred at 80°C for 3 hours under a nitrogen atmosphere. After cooling to room temperature, the reaction solution was diluted with EtOAc (100 mL) and washed three times with water (50 mL). The organic layer was dried over $Na_2SO_4$, filtered, and the filtrate was then concentrated under reduced pressure. The residue was purified by reversed phase silica gel column chromatography (C18) (5 to 40% $CH_3CN$/water). The obtained crude product was further purified by reversed phase preparative HPLC (YMC-Actus Triart C18) (40 to 70% $CH_3CN$/10 mmol/L aqueous ammonium bicarbonate solution) to afford 4-[4-[[2-(3-hydroxyphenyl)phenyl]methyl]piperazin-1-yl]-N-[3-nitro-4-(2-phenylsulfanylethylamino)phenyl]sulfonylbenzamide (B-013) as a yellow solid (28.6 mg, 12.2%).

LCMS (ESI, m/z): 724 $[M+H]^+$.

Retention time: 2.382 min.

Analysis condition: PH-TFA-07.

$^1$H-NMR (300 MHz, DMSO-$d_6$) $\delta$ 11.92 (s, 1H), 9.44 (s, 1H), 8.70 (d, J = 6.0 Hz, 1H), 8.55 (d, J = 2.1 Hz, 1H), 7.91-7.84 (m, 1H), 7.71 (d, J = 9.0 Hz, 2H), 7.58-7.48 (m, 1H), 7.40-7.07 (m, 10H), 6.87 (d, J = 9.0 Hz, 2H), 6.80-6.69 (m, 3H), 3.66-3.64 (m, 2H), 3.46 (s, 2H), 3.30-3.25 (m, 6H), 2.41 (s, 4H).

N-(Benzenesulfonyl)-4-[4-[[2-(5-hydroxypyridin-3-yl)phenyl]methyl]piperazin-1-yl]benzamide: Synthesis of B-014

[Formula 40]

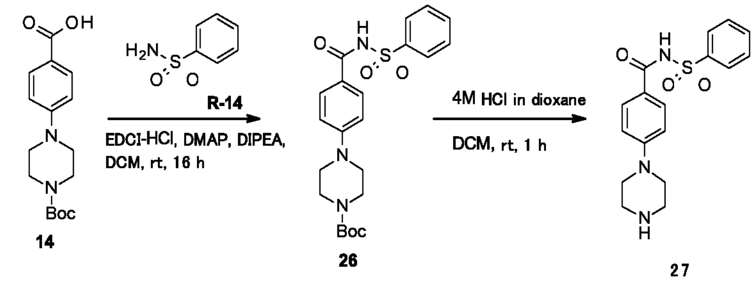


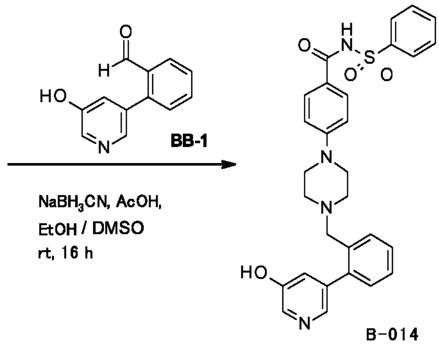


First step: tert-Butyl 4-[4-(benzenesulfonylcarbamoyl)phenyl]piperazine-1-carboxylate: Synthesis of compound 26

To a 200 mL flask under a nitrogen atmosphere were added 4-[4-[(2-methylpropan-2-yl)oxycarbonyl]piperazin-1-yl]benzoic acid (compound 14) (3.0 g, 9.8 mmol, 1.0 eq), benzenesulfonamide (R-14) (1.5 g, 9.8 mmol, 1.0 eq), DMAP (1.2 g, 9.8 mmol, 1.0 eq), and DCM (60 mL). To this solution were added EDCI·HCl (3.7 g, 19.6 mmol, 2.0 eq) and DIPEA (5.1 g, 39.2 mmol, 4.0 eq), and the obtained reaction mixture was stirred at room temperature for 16 hours. A saturated aqueous $NH_4Cl$ solution (60 mL) was added to the reaction solution and the mixture was extracted three times with DCM (60 mL). The organic layers were mixed, dried over $Na_2SO_4$, then filtered, and the filtrate was concentrated under reduced pressure.

The residue was purified by silica gel column chromatography (0 to 10% MeOH/DCM) to afford tert-butyl 4-[4-(benzenesulfonylcarbamoyl)phenyl]piperazine-1-carboxylate (compound 26) as a light yellow solid (2.2 g, 50.4%).

LCMS (ESI, m/z): 446 $[M+H]^+$.

Retention time: 1.023 min.

Analysis condition: PH-FA-02.

Second step: N-(Benzenesulfonyl)-4-piperazin-1-ylbenzamide: Synthesis of compound 27

To a 100 mL flask under a nitrogen atmosphere were added tert-butyl 4-[4-(benzenesulfonylcarbamoyl)phenyl]piperazine-1-carboxylate (compound 26) (2.2 g, 4.9 mmol, 1.0 eq) and DCM (8.0 mL). To this solution was added a 4M solution of HCl in 1,4-dioxane (40 mL), and the obtained reaction mixture was stirred at room temperature for 1 hour. The reaction solution was concentrated under reduced pressure, the obtained residue was dissolved in water (40 mL), and the pH was adjusted to a basicity of 8 to 9 using ammonia water. The precipitated solid was filtered and the obtained solid was purified by reversed phase silica gel column chromatography (C18) (0 to 80% $CH_3CN$/0.5 g/L aqueous ammonium bicarbonate solution) to afford N-(benzenesulfonyl)-4-piperazin-1-ylbenzamide (compound 27) as a light yellow solid (1.4 g, 79.2%).

LCMS (ESI, m/z): 346 $[M+H]^+$.

Retention time: 0.612 min.

Analysis condition: PH-FA-02.

Third step: N-(Benzenesulfonyl)-4-[4-[[2-(5-hydroxypyridin-3-yl)phenyl]methyl]piperazin-1-yl]benzamide: Synthesis of B-014

To a 50 mL flask under a nitrogen atmosphere were added N-(benzenesulfonyl)-4-piperazin-1-ylbenzamide (compound 27) (0.4 g, 1.0 mmol, 1.0 eq) and a mixed solution of EtOH/DMSO (4.0 mL/6.0 mL). To this solution were

added 2-(5-hydroxypyridin-3-yl)benzaldehyde (BB-1) (0.4 g, 2.0 mmol, 2.0 eq) and AcOH (1.0 mL), and the obtained reaction mixture was stirred at room temperature for 1 hour. To this reaction solution was added $NaBH_3CN$ (0.1 g, 2.0 mmol, 2.0 eq), the mixture was stirred at room temperature for 16 hours, and the reaction solution was concentrated under reduced pressure.

The residue was purified by reversed phase silica gel column chromatography (C18) (0 to 80% $CH_3CN$/water) and further purified by reversed phase preparative HPLC (XBridge Shield RP18 OBD column) (19 to 33% $CH_3CN$/10 mmol/L aqueous ammonium bicarbonate solution) to afford N-(benzenesulfonyl)-4-[4-[[2-(5-hydroxypyridin-3-yl)phenyl]methyl]piperazin-1-yl]benzamide (B-014) as an off-white solid (248.3 mg, 45.4%).

LCMS (ESI, m/z): 529 $[M+H]^+$.

Retention time: 0.666 min.

Analysis condition: PH-AC-01.

$^1$H-NMR (300 MHz, DMSO-$d_6$) $\delta$ 12.04 (s, 1H), 9.93 (s, 1H), 8.11 (d, J = 2.7 Hz, 1H), 8.02 (d, J = 1.8 Hz, 1H), 7.96-7.87 (m, 2H), 7.71 (d, J = 8.7 Hz, 2H), 7.65-7.46 (m, 4H), 7.44-7.30 (m, 2H), 7.29-7.19 (m, 2H), 6.86 (d, J = 8.7 Hz, 2H), 3.39 (s, 2H), 3.26-3.12 (m, 4H), 2.44-2.30 (m, 4H).

N-Benzylsulfonyl-4-[4-[3-fluoro-4-(4-hydroxyphenyl)benzoyl]piperazin-1 - yl]benzamide: Synthesis of B-015

[Formula 41]

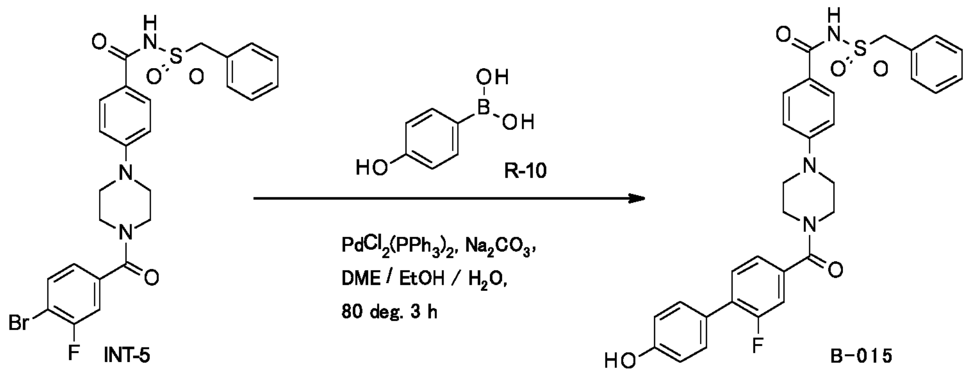


To a 100 mL flask under a nitrogen atmosphere were added N-benzylsulfonyl-4-[4-(4-bromo-3-fluorobenzoyl)piperazin-1-yl]benzamide (INT-5) (0.5 g, 0.8 mmol, 1.0 eq), 3-hydroxybenzeneboronic acid (R-10) (221.5 mg, 1.6 mmol, 2.0 eq), $Na_2CO_3$ (110.6 mg, 1.0 mmol, 1.3 eq), and a mixed solution of DME/EtOH/$H_2O$ (7.0 mL/7.0 mL/7.0 mL). To this suspension was added $PdCl_2(PPh_3)_2$ (56.4 mg, 0.08 mmol, 0.1 eq), and the obtained reaction mixture was stirred at 80°C for 3 hours under a nitrogen atmosphere. After cooling to room temperature, the reaction solution was filtered through Celite® and the Celite® was washed three times with ethyl acetate (50 mL). The filtrate was concentrated under reduced pressure and the residue was purified by reversed phase silica gel column chromatography (C18) (0 to 80% $CH_3CN$/water). The obtained crude product was further purified by reversed phase preparative HPLC (XBridge Prep OBD C18 column) (19 to 39% $CH_3CN$/10 mmol/L aqueous ammonium bicarbonate solution) to afford N-benzylsulfonyl-4-[4-[3-fluoro-4-(4-hydroxyphenyl)benzoyl]piperazin-1-yl]benzamide (B-015) as an off-white solid (187.7 mg, 40.8%).

LCMS (ESI, m/z): 574 $[M+H]^+$.

Retention time: 1.483 min.

Analysis condition: PH-TFA-08.

$^1$H-NMR (300 MHz, DMSO-$d_6$) $\delta$ 11.60 (s, 1H), 9.72 (s, 1H), 7.81 (d, J = 8.7 Hz, 2H), 7.55 (t, J = 8.1 Hz, 1H), 7.46-7.37 (m, 2H), 7.36-7.23 (m, 6H), 7.16-7.00 (m, 1H), 6.96-6.83 (m, 4H), 4.59 (s, 2H), 3.91-3.48 (m, 4H), 3.45-3.32 (m, 4H).

Example 1-2: Model substrate synthesis

Example 1-2-1: Synthesis of compound B-016 (tert-butyl 4-[4-[(4-fluorophenyl)methylcarbamoyl]phenyl]piperazine-1 -carboxylate)

[Formula 42]

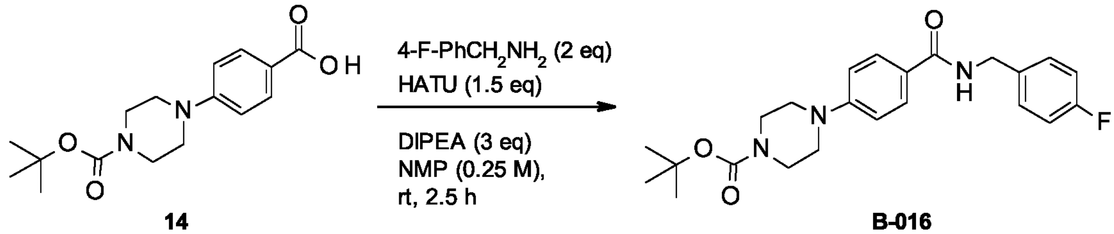


14
(CAS: 162046-66-4)

B-016

Under a nitrogen atmosphere, 4-[4-[(2-methylpropan-2-yl)oxycarbonyl]piperazin-1-yl]benzoic acid (compound 14, CAS: 162046-66-4) (0.5 g, 1.5 mmol) and DIPEA (784.0 µL, 4.5 mmol, 3.0 eq) were suspended in NMP (6.0 mL). To this suspension, HATU (856.0 mg, 2.3 mmol, 1.5 eq) was gradually added at room temperature. After stirring for 5 minutes, 4-fluorophenylmethylamine (343.0 µL, 3.0 mmol, 2.0 eq) was added dropwise to the reaction solution. The obtained reaction solution was stirred at room temperature for 2.5 hours. After the reaction was completed, water (40 mL) was added dropwise to the reaction solution at room temperature, and the reaction solution was stirred at 80°C for 5 minutes using a heating block. The resulting suspended solution was removed from the heating block and stirred at room temperature for 10 minutes. The obtained precipitate was filtered using a Kiriyama funnel, and the obtained solid was washed five times with water (20 mL) and twice with a mixed solvent ($CH_3CN$-$H_2O$, 1:1, 20 mL). The obtained solid was dried under reduced pressure to afford the title compound (tert-butyl 4-[4-[(4-fluorophenyl)methylcarbamoyl]phenyl]piperazine-1-carboxylate) (compound B-016) as a pale red solid (543.0 mg, 88.0%).

LCMS (ESI, m/z): 414.1 $[M+H]^+$.

Retention time: 1.173 min (analysis condition SMD-FA05-1).

$^1$H-NMR (400 MHz, DMSO-$d_6$) δ 8.770 (t, J = 5.7 Hz, 1H), 7.782 (d, J = 9.0 Hz, 2H), 7.782 (dd, J = 5.8, 8.8 Hz, 2H), 7.133 (t, J = 8.8 Hz, 2H), 6.969 (d, J = 9.0 Hz, 2H), 4.421 (d, J = 5.7 Hz, 2H), 3.454 (t, J = 5.1 Hz, 4H), 3.237 (t, J = 5.1 Hz, 4H), 1.422 (s, 9H).

Example 1-2-2: tert-Butyl 4-[4-[(4-fluorophenyl)methylcarbamoyl]phenyl]piperazine-1-carboxylate: Synthesis of compound B-044

[Formula 43]

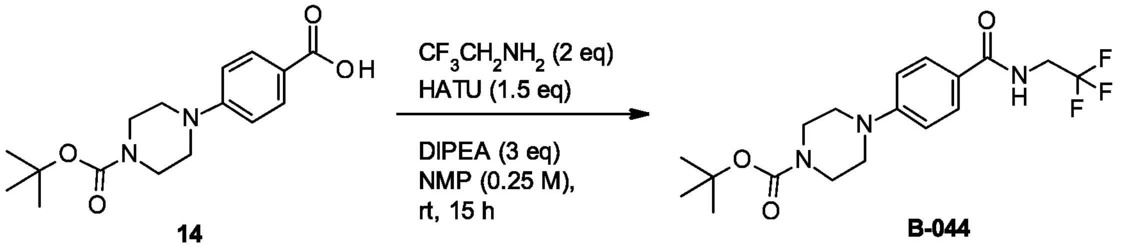


14
(CAS: 162046-66-4)

B-044

Under a nitrogen atmosphere, 4-[4-[(2-methylpropan-2-yl)oxycarbonyl]piperazin-1-yl]benzoic acid (compound 14, CAS: 162046-66-4) (0.4 g, 1.3 mmol) and DIPEA (653.0 µL, 3.8 mmol, 3.0 eq) were suspended in NMP (5.0 mL). To this suspension, HATU (713.0 mg, 1.9 mmol, 1.5 eq) was gradually added at room temperature. After stirring for 5 minutes, 2,2,2-trifluoroethylamine (196.0 µL, 2.5 mmol, 2.0 eq) was added dropwise to the reaction solution. The obtained reaction solution was stirred at room temperature for 15 hours.

After the reaction was completed, water (40 mL) was added dropwise to the reaction solution at room temperature, and the reaction solution was stirred at 80°C for 5 minutes using a heating block. The resulting suspended solution was removed from the heating block and stirred at room temperature for 10 minutes. The obtained precipitate was filtered using a Kiriyama funnel. The obtained solid was washed five times with water (20 mL) and twice with a mixed solvent ($CH_3CN$-$H_2O$, 1:1, 20 mL). The obtained solid was dried under reduced pressure to afford the title compound (tert-butyl 4-[4-[(4-fluorophenyl)methylcarbamoyl]phenyl]piperazine-1-carboxylate) (compound B-044) as a colorless solid (406.7 mg, 84.0%).

LCMS (ESI, m/z): 388.1 $[M+H]^+$.

Retention time: 1.104 min (analysis condition SMD-FA05-1).

$^1$H-NMR (400 MHz, DMSO-$d_6$) δ 8.761 (t, J = 6.4 Hz, 1H), 7.788 (d, J = 9.2 Hz, 2H), 6.984 (d, J = 9.2 Hz, 2H), 4.084-3.996 (m, 2H), 3.455 (t, J = 5.2 Hz, 4H), 3.265 (t, J = 5.2 Hz, 4H), 1.423 (s, 9H).

Example 1-2-3: 6-[4-[(2-methylpropan-2-yl)oxycarbonyl]piperazin-1-yl]pyridazine-3-carboxylic acid: Synthesis of compound AC-012

[Formula 44]

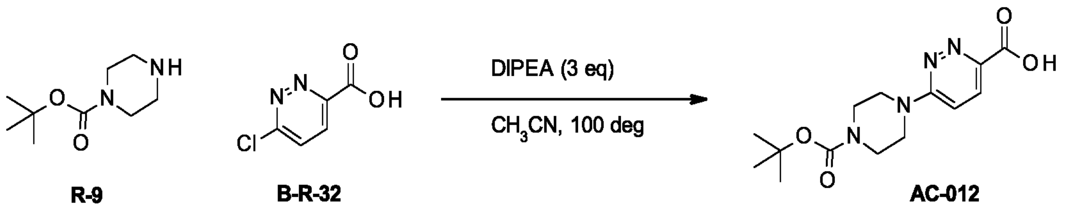


R-9 B-R-32 AC-012

To a 50 mL flask were added 6-chloropyridazine-3-carboxylic acid (B-R-32, CAS: 5096-73-1) (1.0 g, 6.3 mmol), 1-Boc piperazine (R-9, CAS: 57260-71-6) (1.8 g, 9.5 mmol, 1.5 eq), DIPEA (3.3 mL, 18.9 mmol, 3.0 eq), and $CH_3CN$ (15 mL) were added, the flask was degassed under reduced pressure, and the inside of the reaction mixture was then converted to a nitrogen atmosphere by the introduction of nitrogen. The reaction solution was heated at 100°C for 16 hours. After cooling the reaction solution to room temperature, the reaction solvent was concentrated under reduced pressure. The obtained residue was purified by reversed phase silica gel column chromatography (C18) (15 to 50%, 0.1% formic acid $CH_3CN$/0.1% aqueous formic acid solution) to afford the title compound (6-[4-[(2-methylpropan-2-yl)oxycarbonyl]piperazin-1-yl]pyridazine-3-carboxylic acid) (AC-012) as a light yellow amorphous (1.3 g, 66.3%).

LCMS (ESI, m/z): 309.2 $[M+H]^+$.

Retention time: 0.702 min (analysis condition SMD-FA05-1).

$^1$H-NMR (400 MHz, $CDCl_3$) δ 8.011 (d, J = 9.8 Hz, 1H), 6.987 (d, J = 9.0 Hz, 1H), 3.814 (bs, 4H), 3.616 (bs, 4H), 1.489 (s, 9H).

Example 1-2-4: tert-Butyl 4-[6-(2,2,2-trifluoroethylcarbamoyl)pyridazin-3-yl]piperazine-1-carboxylate: Synthesis of compound B-045

[Formula 45]

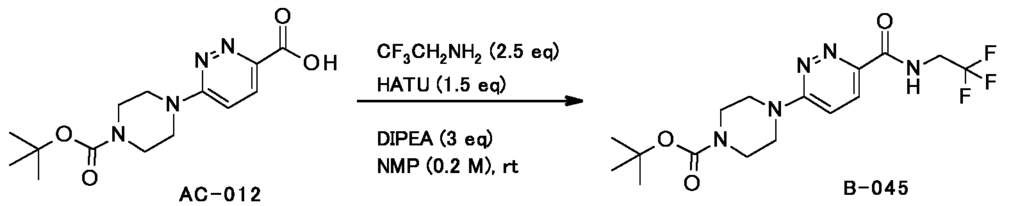


AC-012 B-045

Under a nitrogen atmosphere, 6-[4-[(2-methylpropan-2-yl)oxycarbonyl]piperazin-1-yl]pyridazine-3-carboxylic acid (compound AC-012) (0.3 g, 1.0 mmol) and DIPEA (523.0 μL, 3.0 mmol, 3.0 eq) were suspended in NMP (5.0 mL). To this suspension, HATU (570.0 mg, 1.5 mmol, 1.5 eq) was gradually added at room temperature. After stirring for 5 minutes, 2,2,2-trifluoroethylamine (196.0 μL, 2.5 mmol, 2.5 eq) was added dropwise to the reaction solution. The obtained reaction solution was stirred at room temperature for 15 hours.

After the reaction was completed, water (40 mL) was added dropwise to the reaction solution at room temperature, and the reaction solution was stirred at 80°C for 5 minutes using a heating block. The resulting suspended solution was removed from the heating block and stirred at room temperature for 10 minutes. The obtained precipitate was filtered using a Kiriyama funnel. The obtained solid was washed five times with water (20 mL) and twice with a mixed solvent ($CH_3CN$-$H_2O$, 1:1, 20 mL). The obtained solid was dried under reduced pressure to afford the title compound (tert-butyl 4-[6-(2,2,2-trifluoroethylcarbamoyl)pyridazin-3-yl]piperazine-1-carboxylate) (compound B-045) as a colorless solid (259.0 mg, 66.5%).

LCMS (ESI, m/z): 390 $[M+H]^+$.

Retention time: 1.029 min (analysis condition SMD-FA05-1).

$^1$H-NMR (400 MHz, DMSO-$d_6$) δ 9.399 (t, J = 6.4 Hz, 1H), 7.895 (d, J = 9.6 Hz, 1H), 7.366 (d, J = 9.6 Hz, 1H), 4.118-4.029 (m, 2H), 3.744 (t, J = 5.4 Hz, 4H), 3.480 (t, J = 5.4 Hz, 4H), 1.432 (s, 9H).

Example 1-2-5: 2-Fluoro-N-(2-fluorophenyl)aniline: Synthesis of B-046

[Formula 46]

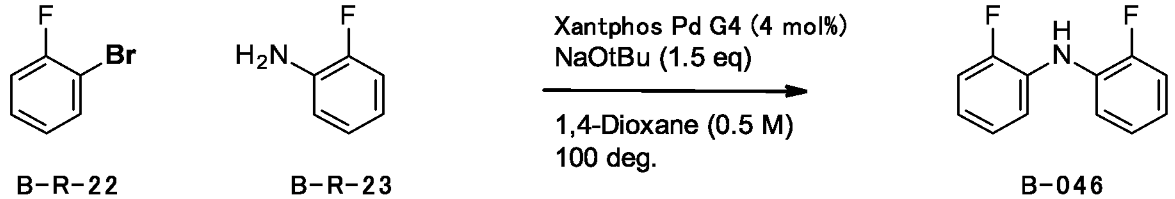


B-R-22 B-R-23 B-046

To a 10 mL vial under a nitrogen atmosphere were added 2-fluorobromobenzene (B-R-22) (273.0 μL, 2.5 mmol), 2-fluoroaniline (B-R-23) (290.0 μL,3.0 mmol, 1.2 eq), Xantphos Pd G4 (96.0 mg, 0.1 mmol, 4 mol%), NaOtBu (360.0 mg, 3.7 mmol, 1.5 eq), and 1,4-dioxane (5.0 mL), and the reaction mixture was stirred at 100°C for 21 hours. The reaction solution was filtered through Celite®, washed four times with ethyl acetate (20 mL), and the filtrate was concentrated under reduced pressure. The residue was purified by silica gel column chromatography (0 to 20% ethyl acetate/hexane) to afford the title compound (2-fluoro-N-(2-fluorophenyl)aniline) (compound B-046) as a colorless oil (375.3 mg, 73.2%).

LCMS (ESI, m/z): 205.9 $[M+H]^+$.

Retention time: 1.236 min (analysis condition SMD-FA05-1).

$^1$H-NMR (400 MHz, DMSO-$d_6$) δ 7.719 (bs, 1H), 7.207 (dd, J = 8.2, 1.4 Hz, 1H), 7.177 (dd, J = 8.2, 1.4 Hz, 1H), 7.071 (ddd, J = 7.4, 7.4, 1.4 Hz, 2H), 7.015-6.917 (m, 4H).

Example 1-2-6: N-Phenylpyridin-3-amine: Synthesis of B-047

[Formula 47]

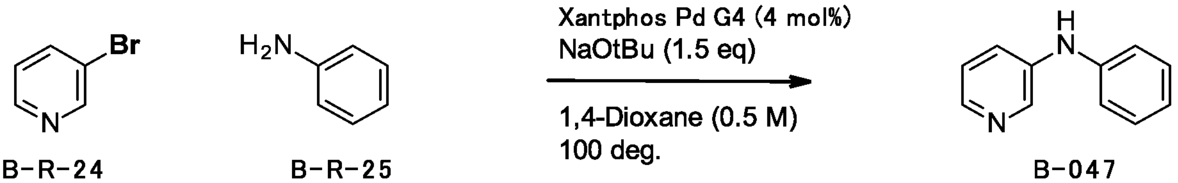


B-R-24 B-R-25 B-047

Using 3-bromopyridine (B-R-24) (241.0 μL, 2.5 mmol) and aniline (B-R-25) (273.0 μL, 3.0 mmol, 1.2 eq), the title compound (N-phenylpyridin-3-amine) (compound B-047) was synthesized by the same method as in Example 1-2-5 and obtained as an off-white solid (358.0 mg, 84.0%).

LCMS (ESI, m/z): 170.9 $[M+H]^+$.

Retention time: 0.471 min (analysis condition SMD-FA05-1).

$^1$H-NMR (400 MHz, DMSO-$d_6$) δ 8.335 (bs, 2H), 8.019 (dd, J = 4.6, 1.4 Hz, 1H), 7.457 (ddd, J = 8.0, 1.4, 1.4 Hz, 1H), 7.290-7.205 (m, 3H), 7.103-7.082 (m, 2H), 6.905-6.866 (m, 1H).

Example 1-2-7: N-Phenylpyrimidin-5-amine: Synthesis of B-048

[Formula 48]

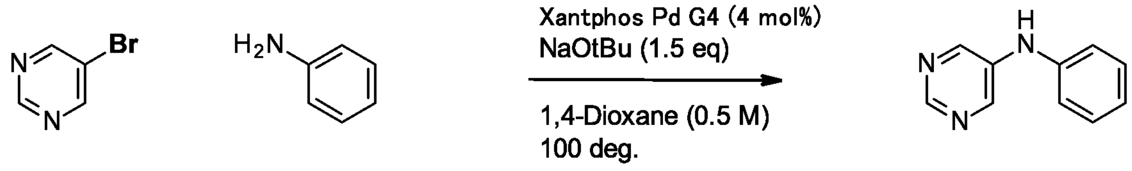


B-R-26 B-R-25 B-048

Using 5-bromopyrimidine (B-R-26) (397.0 mg, 2.5 mmol) and aniline (B-R-25) (273.0 μL, 3.0 mmol, 1.2 eq), the title compound (N-phenylpyrimidin-5-amine) (compound B-048) was synthesized by the same method as in Example 1-2-5 and obtained as a brown solid (73.9 mg, 17.0%).

LCMS (ESI, m/z): 171.9 $[M+H]^+$.

Retention time: 0.725 min (analysis condition SMD-FA05-1).

$^1$H-NMR (400 MHz, DMSO-$d_6$) δ 8.630 (s, 1H), 8.54.0 (s, 2H), 8.522 (s, 1H), 7.304 (dd, J = 8.0, 7.2 Hz, 2H), 7.148 (d, J = 8.0 Hz, 2H), 6.954 (t, J = 7.2 Hz, 1H).

Example 1-2-8: N-Pyridin-3-ylpyridin-3-amine: Synthesis of B-049

[Formula 49]

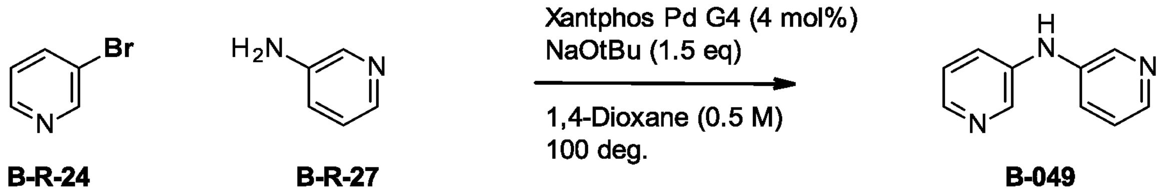


**B-R-24** **B-R-27** **B-049**

Using 3-bromopyridine (B-R-24) (241.0 μL, 2.5 mmol) and 3-aminopyridine (B-R-27) (282.0 mg, 3.0 mmol, 1.2 eq), the title compound (N-pyridin-3-ylpyridin-3-amine) (compound B-049) was synthesized by the same method as in Example 1-2-5 and obtained as a brown solid (354.8 mg, 83.0%).

LCMS (ESI, m/z): 172 $[M+H]^+$.

Retention time: 0.136 min (analysis condition SMD-FA05-1).

$^1$H-NMR (400 MHz, DMSO-$d_6$) δ 8.536 (s, 1H), 8.370 (d, J = 2.4 Hz, 2H), 8.090 (dd, J = 4.7, 1.4 Hz, 2H), 7.504 (ddd, J = 8.2, 1.4, 1.4 Hz, 2H), 7.266 (dd, J = 8.2, 4.7 Hz, 2H).

Example 1-2-9: N-Phenylpyridin-2-amine: Synthesis of B-050

[Formula 50]

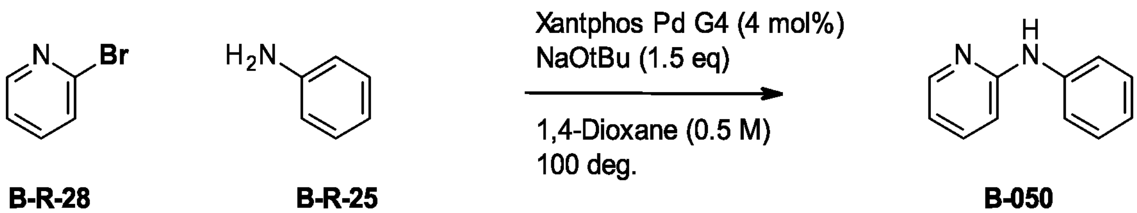


**B-R-28** **B-R-25** **B-050**

Using 2-bromopyridine (B-R-28) (238.0 μL, 2.5 mmol) and aniline (B-R-25) (273.0 μL, 3.0 mmol, 1.2 eq), the title compound (N-phenylpyridin-2-amine) (compound B-050) was synthesized by the same method as in Example 1-2-5 and obtained as an off-white solid (57.5 mg, 13.5%).

LCMS (ESI, m/z): 170.9 $[M+H]^+$.

Retention time: 0.434 min (analysis condition SMD-FA05-1).

$^1H$-NMR (400 MHz, DMSO-$d_6$) δ 8.980 (s, 1H), 8.144 (dd, J = 5.0, 1.0 Hz, 1H), 7.675 (d, J = 9.6 Hz, 2H), 7.569-7.526 (m, 1H), 7.252 (dd, J = 7.6, 7.6 Hz, 2H), 6.892-6.853 (m, 1H), 6.830 (d, 8.0 Hz, 1H), 6.742-6.711 (m, 1H).

Example 1-2-10: 5-(4-Methylphenyl)pyridin-2-amine: Synthesis of B-051

[Formula 51]

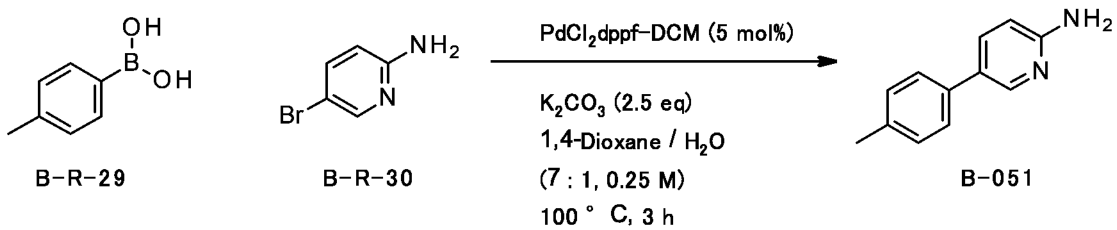


B-R-29 B-R-30 B-051

To a 12 mL vial under a nitrogen atmosphere were added 2-amino-5-bromopyridine (B-R-30) (346.0 mg, 2.0 mmol), p-tolueneboronic acid (B-R-29) (326.0 mg, 2.4 mmol, 1.2 eq), $PdCl_2$(dppf)-DCM (82.0 mg, 0.1 mmol, 5 mol%), $K_2CO_3$ (691.0 mg, 5.0 mmol, 2.5 eq), 1,4-dioxane (7.0 mL), and $H_2O$ (1.0 mL), and the reaction mixture was stirred at 100°C for 3 hours. The reaction solution was filtered through Celite®, washed four times with ethyl acetate (20 mL), and the filtrate was concentrated under reduced pressure. The residue was purified by amino-modified silica gel column chromatography (0 to 100% ethyl acetate/hexane) to afford the title compound (5-(4-methylphenyl)pyridin-2-amine) (compound B-051) as an off-white solid (282.3 mg, 77.0%).

LCMS (ESI, m/z): 184.9 $[M+H]^+$.

Retention time: 0.580 min (analysis condition SMD-FA05-1).

$^1H$-NMR (400 MHz, DMSO-$d_6$) δ 8.205 (d, J = 2.5 Hz, 1H), 7.656 (dd, J = 8.6, 2.5 Hz, 1H), 7.441 (d, J = 8.0 Hz, 2H), 7.203 (d, J = 8.0 Hz, 2H), 6.511 (d, J = 8.6 Hz, 1H), 5.993 (s, 2H), 2.308 (s, 3H).

Example 1-2-11: 6-(4-Methylphenyl)pyridin-3-amine: Synthesis of B-052

[Formula 52]

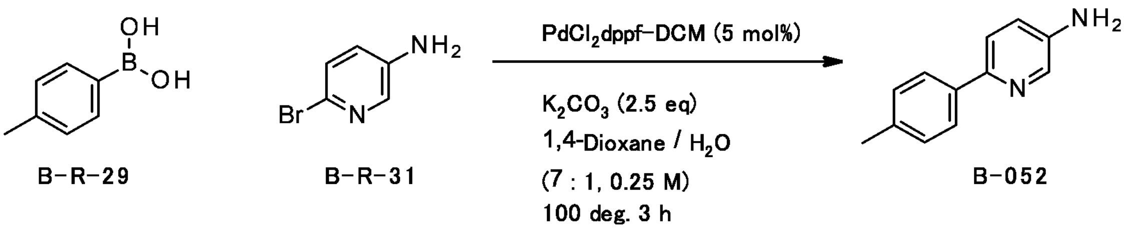


B-R-29 B-R-31 B-052

Using 3-amino-6-bromopyridine (B-R-31) (346.0 mg, 2.0 mmol), the title compound (6-(4-methylphenyl)pyridin-3-amine) (compound B-052) was synthesized by the same method as in Example 1-2-10 and obtained as an off-white solid (246.7 mg, 66.9%).

LCMS (ESI, m/z): 185.0 $[M+H]^+$.

Retention time: 0.527 min (analysis condition SMD-FA05-1).

$^1H$-NMR (400 MHz, DMSO-$d_6$) δ 8.013 (d, J = 2.6 Hz, 1H), 7.804 (d, J = 8.2 Hz, 2H), 7.576 (d, J = 8.4 Hz, 1H), 7.190 (d, J = 8.2 Hz, 2H), 6.985 (dd, J = 8.4, 2.6 Hz, 1H), 5.399 (s, 2H), 2.310 (s, 3H).

Example 1-2-12: Prop-2-enyl 4-piperazin-1-ylbenzoate: Synthesis of BB27

[Formula 53]

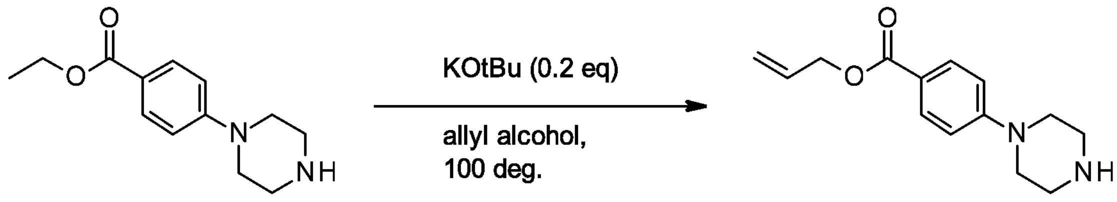


B-R-32 BB-27

To a 50 mL flask were added allyl alcohol (10 mL) and KOtBu (96.0 mg, 0.8 mmol, 0.2 eq), and the mixture was stirred for 10 minutes under a nitrogen atmosphere. To this mixture was added ethyl 4-piperazin-1-ylbenzoate compound (B-R-32, CAS: 80518-57-6) (1.0 g, 4.3 mmol, 1.0 eq) at once, and the obtained mixture was heated at 100°C for 1 hour. After cooling the reaction solution to room temperature, the reaction solvent was concentrated under reduced pressure. To the obtained residue was added allyl alcohol (12 mL), and the mixture was further heated at 100°C for 16.5 hours. After cooling the reaction solution to room temperature, the reaction solvent was concentrated under reduced pressure. The obtained residue was purified by amino-modified silica gel column chromatography (50 to 100% ethyl acetate/hexane) to afford the title compound (prop-2-enyl 4-piperazin-1-ylbenzoate) (BB27) (720 mg, 69.0%) as a colorless waxy solid.

LCMS (ESI, m/z): 247.1 $[M+H]^+$.

Retention time: 0.595 min (analysis condition SMD-FA05-1).

$^1$H-NMR (400 MHz, DMSO-$d_6$) $\delta$ 7.795 (d, J = 9.0 Hz, 2H), 6.956 (d, J = 9.0 Hz, 2H), 6.072-5.977 (m, 1H), 5.387-5.332 (m, 1H), 5.262-5.225 (m, 1H), 4.737-4.716 (m, 2H), 3.229-3.204 (m, 4H), 2.816-2.790 (m, 4H), 2.323 (bs, 1H).

Example 1-3: Ethylated product samples of model substrates: Synthesis of B-053 to B075

Ethylated product samples of model substrates were synthesized by four different methods as shown in Examples 1-3-1 to 1-3-4, and measurement of the retention time and mass spectrometry were carried out by LCMS.

Example 1-3-1: Ethylation reaction using NaH as base: Synthesis method A

A reaction to a model substrate (tert-butyl 4-[4-(2,2,2-trifluoroethylcarbamoyl)phenyl]piperazine-1-carboxylate: B-044) is shown as a representative example of an ethylation reaction using NaH as a base.

[Formula 54]

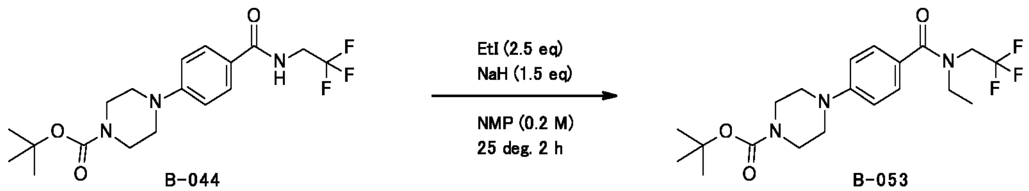


B-044 B-053

Under a nitrogen atmosphere, to a 1.5 mL glass vial were added tert-butyl 4-[4-(2,2,2-trifluoroethylcarbamoyl)phenyl]piperazine-1-carboxylate (B-044) (39.0 mg, 0.1 mmol) and NMP (0.5 mL). To this solution was added NaH (6.0 mg, 0.15 mmol, 1.5 eq) at room temperature. After stirring for 5 minutes, iodoethane (20.0 μL, 0.25 mmol, 2.5 eq) was added to the reaction solution. The obtained reaction solution was stirred at room temperature for 2 hours.

Reaction tracking

At 2 hours after the start of the reaction, 5 μL of the reaction solution was sampled in a glove bag and diluted with $CH_3CN$ (1000 μL) to prepare a LC sample. Measurement of the retention time and mass spectrometry were carried out by LCMS.

Following synthesis method A, the reaction was carried out using the substrate amounts and solvents shown in Table 1-3, and measurement of the retention time and mass spectrometry were carried out by LCMS.

Example 1-3-2: Ethylation reaction using $K_2CO_3$ as base: Synthesis method B

A reaction to a reagent (N-butylaniline: B-R-11) is shown as a representative example of an ethylation reaction using $K_2CO_3$ as a base.

[Formula 55]

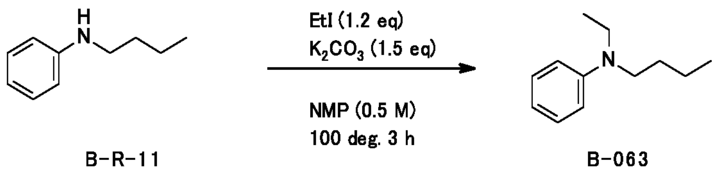


B-R-11 B-063

Under a nitrogen atmosphere, to a 2.0 mL glass vial were added N-butylaniline (B-R-11) (75.0 mg, 0.5 mmol), $K_2CO_3$ (104.0 mg, 0.75 mmol, 1.5 eq), and NMP (1.3 mL). To this suspension, iodoethane (48.0 μL, 0.6 mmol, 1.2 eq) was added. The resulting suspended reaction solution was stirred at 100°C for 3 hours.

Reaction tracking

At 3 hours after the start of the reaction, 5 μL of the reaction solution was sampled in a glove bag and diluted with $CH_3CN$ (1000 μL) to prepare a LC sample. Measurement of the retention time and mass spectrometry were carried out by LCMS.

Following synthesis method B, the reaction was carried out with the substrate amounts, solvents, and reaction temperatures shown in Table 1-3, and measurement of the retention time and mass spectrometry were carried out by LCMS.

Example 1-3-3: Ethylation reaction without using base: Synthesis method C

[Formula 56]

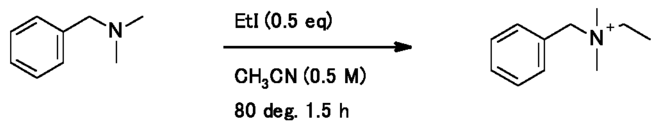


B-R-15 B-070

Under a nitrogen atmosphere, to a 2.0 mL glass vial were added N,N-dimethylbenzylamine (B-R-15) (68.0 mg, 0.5 mmol) and $CH_3CN$ (1.0 mL). To this suspension, iodoethane (20.0 μL, 0.25 mmol, 0.5 eq) was added. The obtained reaction solution was stirred at 80°C for 1.5 hours.

Reaction tracking

At 1.5 hours after the start of the reaction, 5 μL of the reaction solution was sampled in a glove bag and diluted with $CH_3CN$ (1000 μL) to prepare a LC sample. Measurement of the retention time and mass spectrometry were carried out by LCMS.

Example 1-3-4: Ethylation reaction using KtOBu as base: Synthesis method D

A reaction to a reagent (benzyl alcohol: B-R-16) is shown as a representative example of an ethylation reaction using KtOBu as a base.

[Formula 57]

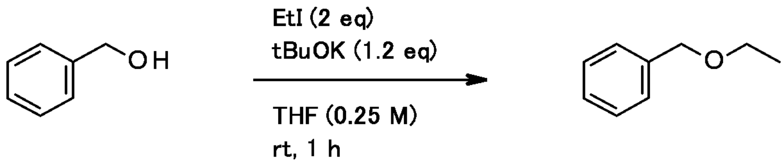


B-R-16 B-071

Under a nitrogen atmosphere, to a solution of benzyl alcohol (B-R-16) (27.0 mg, 0.25 mmol) and THF (1.0 mL) in a 3.0 mL glass vial was added KOtBu (34.0 mg, 0.3 mmol, 1.2 eq), and the mixture was stirred for 5 minutes. To the obtained reaction solution was added iodoethane (40.0 μL, 0.5 mmol, 2.0 eq), and the reaction solution was stirred at room temperature for 1 hour.

Reaction tracking

At 1 hour after the start of the reaction, 5 μL of the reaction solution was sampled in a glove bag and diluted with $CH_3CN$ (1000 μL) to prepare a LC sample. Measurement of the retention time was carried out by LCMS.
Following synthesis method D, the reaction was carried out with the substrate amounts shown in Table 1-3, and measurement of the retention time was carried out by LCMS.
The results are shown in Table 1-3.

[Table 6]

[Table 1-3]

| Run | Substr ate | Substrate (structure) | Substrat e (mg, mmol) | Meth od | Solven t(M) | Temp (°C) | Time (h) | Ethylated compound | Ethylated compound (structure) | m/z [M+H] + | Retention time | Analysis method |
|---|---|---|---|---|---|---|---|---|---|---|---|---|
| 1 | B-044 | 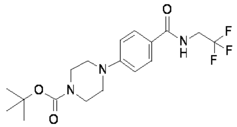 | 39, 0.1 | A | NMP (0.2) | rt | 2.0 | B-053 | 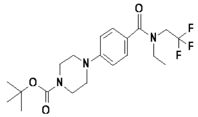 | 416 | 1.240 | SMD-FA05-1 |
| 2 | B-045 | 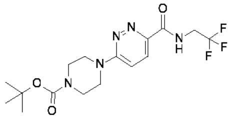 | 39, 0.1 | A | NMP (0.2) | rt | 2.0 | B-054 | 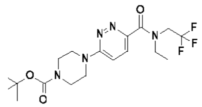 | 418 | 1.087 | SMD-FA05-1 |
| 3 | B-R-06 | 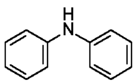 | 42, 0.25 | A | NMP (0.25) | rt | 1.5 | B-055 | 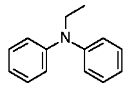 | 198 | 1.432 | SMD-FA05-1 |
| 4 | B-046 | 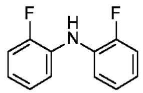 | 51, 0.25 | A | NMP (0.25) | rt | 2.0 | B-056 | 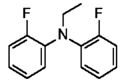 | 234 | 1.372 | SMD-FA05-1 |
| 5 | B-047 | 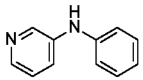 | 43, 0.25 | A | DMF (0.25) | rt | 1.5 | B-057 | 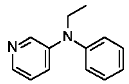 | 199 | 1.047, 0.667 | SMD-AC05-1 |
| 6 | B-R-09 | 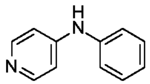 | 43, 0.25 | A | DMF (0.25) | rt | 1.5 | B-058 | 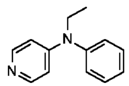 | 199 | 0.935, 0.667 | SMD-AC05-1 |
| 7 | B-048 | 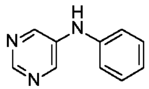 | 21, 0.125 | A | DMF (0.25) | rt | 1.5 | B-059 | 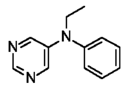 | 200 | 0.893, 0.927 | SMD-AC05-1 |
| 8 | B-049 | 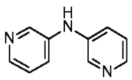 | 43, 0.25 | A | DMF (0.25) | rt | 1.5 | B-060 | 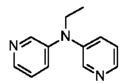 | 200 | 0.737, 0.397 | SMD-AC05-1 |

(continued)

| Run | Substr ate | Substrate (structure) | Substrat e (mg, mmol) | Meth od | Solven t(M) | Temp (°C) | Time (h) | Ethylated compound | Ethylated compound (structure) | m/z [M+H] + | Retention time | Analysis method |
|---|---|---|---|---|---|---|---|---|---|---|---|---|
| 9 | B-050 | 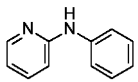 | 43, 0.25 | A | NMP (0.25) | rt | 2.0 | B-061 | 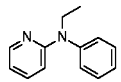 | 199 | 0.548 | SMD-FA05-1 |
| 10 | B-R-10 | 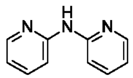 | 43, 0.25 | A | NMP (0.25) | rt | 1.5 | B-062 | 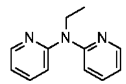 | 200 | 0.463 | SMD-FA05-1 |
| 11 | B-R-11 | 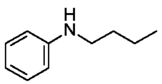 | 75, 0.5 | B | NMP (0.5) | 100 | 3.0 | B-063 | 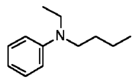 | 178 | 0.612 | SMD-FA05-1 |

[Table 7]

| | | | | | | | | | | | | |
|---|---|---|---|---|---|---|---|---|---|---|---|---|
| 12 | B-R-12 | 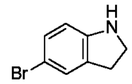 | 50, 0.25 | A | NMP (0.25) | rt | 2.0 | B-064 | 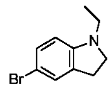 | 226 | 1.301 | SMD-FA05-1 |
| 13 | B-R-13 | 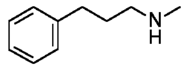 | 37, 0.25 | B | CH3C N (0.25) | 80 | 1.5 | B-065 | 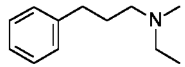 | 178 | 0.516 | SMD-FA05-1 |
| 14 | BB27 | 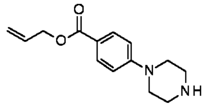 | 62, 0.25 | B | CH3C N (0.25) | 80 | 2.0 | B-066 | 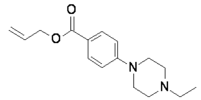 | 275 | 1.120 | SMD-AC05-1 |
| 15 | B-051 | 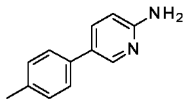 | 46, 0.25 | A | THF (0.25) | rt | 5.0 | B-067 | 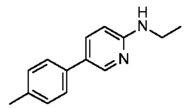 | 213 | 0.641 | SMD-FA05-1 |
| 16 | B-052 | 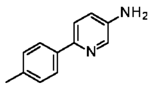 | 46, 0.25 | A | THF (0.25) | rt | 5.0 | B-068 | 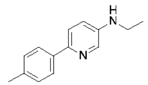 | 213 | 0.640 | SMD-FA05-1 |
| 17 | B-R-14 | 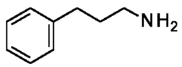 | 68, 0.5 | B | Dioxa ne (0.25) | 80 | 1.5 | B-069 | 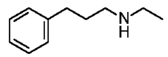 | 164 | 0.506 | SMD-FA05-1 |
| 18 | B-R-15 | 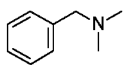 | 68, 0.075 | C | CH3C N (0.5) | 80 | 1.5 | B-070 | 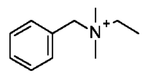 | 164[a] | 0.416 | SMD-AC05-1 |
| 19 | B-R-16 | 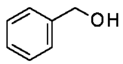 | 27, 0.25 | D | THF (0.25) | rt | 1.0 | B-071 | 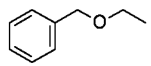 | ND | 1.089 | SMD-FA05-1 |
| 20 | B-R-17 | 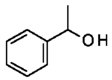 | 31, 0.25 | D | THF (0.25) | rt | 1.0 | B-072 | 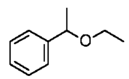 | ND | 1.181 | SMD-FA05-1 |
| 21 | B-R-18 | 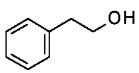 | 31, 0.25 | D | THF (0.25) | rt | 1.0 | B-073 | 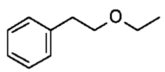 | ND | 1.157 | SMD-FA05-1 |
| 22 | B-R-19 | 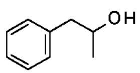 | 34, 0.25 | D | THF (0.25) | rt | 1.0 | B-074 | 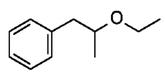 | ND | 1.241 | SMD-FA05-1 |

(continued)

| | | | | | | | | | | | | |
|---|---|---|---|---|---|---|---|---|---|---|---|---|
| 23 | B-R-20 | 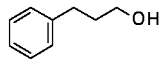 | 34, 0.25 | D | THF (0.25) | rt | 0.5 | B-075 | 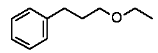 | ND | 1.256 | SMD-FA05-1 |

[a]: $[M]^+$

Note that the ethylated products of Runs 1 and 2 in Table 1-3 are denoted as N-ethyl forms, but there is a possibility that they are O-ethyl forms. Also, Runs 5 to 10 are denoted as diarylamine-N-ethyl forms, but since there are two or more reaction points, there is a possibility that they are other than N-ethyl forms. In particular, since two monoethyl forms are observed in Runs 5 to 8, two retention times are shown.

Example 1-4: Synthesis method of arenol compound B-080 (6-[4-[[2-(3-hydroxyphenyl)phenyl]methyl]piperazin-1-yl]-N-(4-methoxyphenyl)pyridazine-3-carboxamide)

[Formula 58]

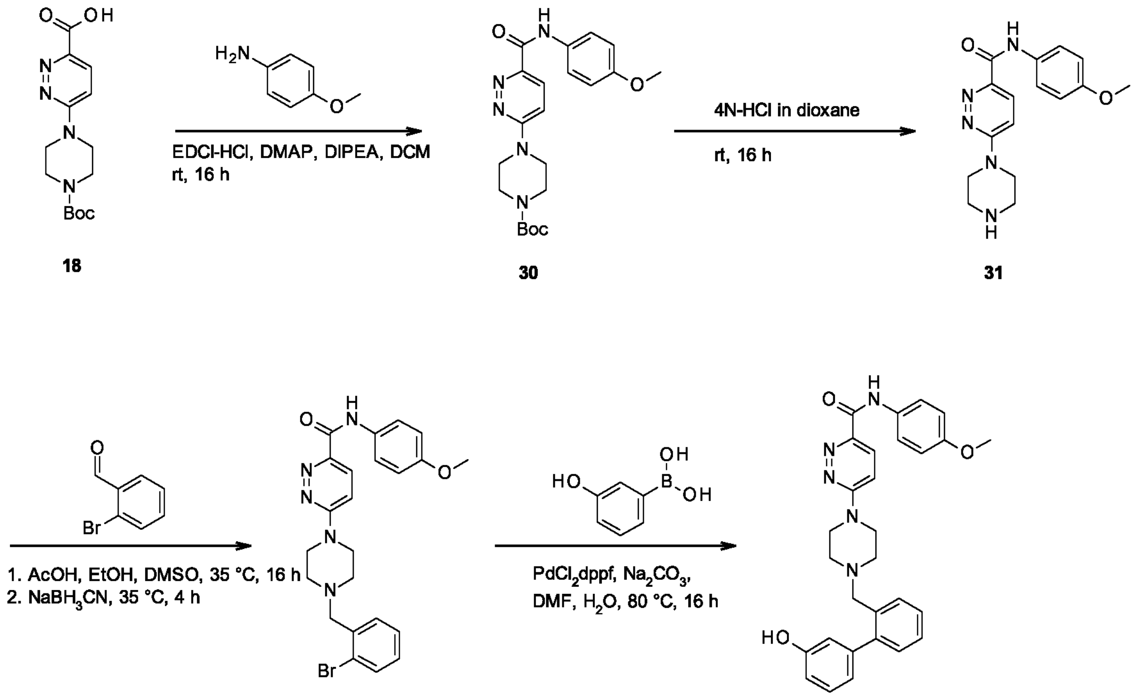


18 30 31

32 B-080

First step: tert-Butyl 4-[6-[(4-methoxyphenyl)carbamoyl]pyridazin-3-yl]piperazine-1-carboxylate: Synthesis of compound 30

To a 200 mL flask under a nitrogen atmosphere were added 6-[4-[(2-methylpropan-2-yl)oxycarbonyl]piperazin-1-yl]pyridazine-3-carboxylic acid (compound 18) (6.0 g, 19.5 mmol, 1.0 eq), 4-methoxyaniline (2.4 g, 19.5 mmol, 1.0 eq), and DCM (60 mL). To this solution were added EDCI·HCl (7.5 g, 38.9 mmol, 2.0 eq), DMAP (2.4 g, 19.5 mmol, 1.0 eq), and DIPEA (1.0 g, 77.8 mmol, 4.0 eq), and the obtained reaction mixture was stirred at room temperature for 16 hours. A saturated aqueous $NH_4Cl$ solution (50 mL) was added to the reaction solution and the mixture was extracted three times with DCM (100 mL). The organic layers were mixed, dried over $Na_2SO_4$, then filtered, and the filtrate was concentrated under reduced pressure.

The residue was purified by silica gel column chromatography (0 to 60% ethyl acetate/petroleum ether) to afford tert-butyl 4-[6-[(4-methoxyphenyl)carbamoyl]pyridazin-3-yl]piperazine-1-carboxylate (compound 30) as a yellow solid (1.4 g, 16.5%).

LCMS (ESI, m/z): 414 $[M+H]^+$.

Retention time: 1.283 min.

Analysis condition: PH-TFA-14.

Second step: N-(4-Methoxyphenyl)-6-piperazin-1-ylpyridazine-3-carboxamide: Synthesis of compound 31

To a 100 mL flask under a nitrogen atmosphere were added tert-butyl 4-[6-[(4-methoxyphenyl)carbamoyl]pyridazin-3-yl]piperazine-1-carboxylate (compound 30) (1.4 g, 3.4 mmol, 1.0 eq) and DCM (30 mL). To this solution was added a 4M solution of HCl in 1,4-dioxane (20 mL), and the obtained reaction mixture was stirred at room temperature

for 16 hours. The reaction solution was concentrated under reduced pressure, the obtained residue was dissolved in water (20 mL), and the pH was adjusted to a basicity of 8 to 9 using ammonia water. The precipitated solid was filtered and the obtained solid was washed three times with water (20 mL). The recovered solid was dried under reduced pressure at 50°C for 16 hours using a constant temperature dryer to afford N-(4-methoxyphenyl)-6-piperazin-1-ylpyridazine-3-carboxamide (compound 31) as a yellow solid (890 mg). The obtained solid was used in the next step without carrying out further purification.

LCMS (ESI, m/z): 314 $[M+H]^+$.

Retention time: 0.915 min.

Analysis condition: PH-TFA-14.

Third step: 6-[4-[(2-Bromophenyl)methyl]piperazin-1-yl]-N-(4-methoxyphenyl)pyridazine-3-carboxamide: Synthesis of compound 32

To a 50 mL flask under a nitrogen atmosphere were added N-(4-methoxyphenyl)-6-piperazin-1-ylpyridazine-3-carboxamide (compound 31) (0.9 g, 2.9 mmol, 1.0 eq) and a mixed solution of EtOH/DMSO (9.0 mL/9.0 mL). To this solution were added 2-bromobenzaldehyde (1.1 g, 5.7 mmol, 2.0 eq) and AcOH (0.5 mL, 7.5 mmol, 2.7 eq), and the obtained reaction mixture was stirred at 35°C for 16 hours. To this reaction solution was added $NaBH_3CN$ (361 mg, 5.7 mmol, 2.0 eq), and the mixture was stirred at 35°C for 16 hours. The reaction solution was concentrated under reduced pressure and the obtained residue was purified by silica gel column chromatography (0 to 60% ethyl acetate/petroleum ether) to afford 6-[4-[(2-bromophenyl)methyl]piperazin-1-yl]-N-(4-methoxyphenyl)pyridazine-3-carboxamide (compound 32) as an off-white solid (0.9 g, 59.8%).

LCMS (ESI, m/z): 482, 484 $[M+H]^+$.

Retention time: 1.429 min.

Analysis condition: PH-AC-04.

Fourth step: 6-[4-[[2-(3-Hydroxyphenyl)phenyl]methyl]piperazin-1-yl]-N-(4-methoxyphenyl)pyridazine-3-carboxamide: Synthesis of arenol compound B-080

To a 50 mL flask under a nitrogen atmosphere were added 6-[4-[(2-bromophenyl)methyl]piperazin-1-yl]-N-(4-methoxyphenyl)pyridazine-3-carboxamide (compound 32) (1.4 g, 2.9 mmol, 1.0 eq), 3-hydroxybenzeneboronic acid (1.6 g, 11.6 mmol, 4.0 eq), $Na_2CO_3$ (922.8 mg, 8.7 mmol, 3.0 eq), and a mixed solution of DMF/$H_2O$ (14.0 mL/1.4 mL). To this suspension was added $PdCl_2$(dppf) (127.4 mg, 0.2 mmol, 0.06 eq), and the obtained reaction mixture was stirred at 80°C for 16 hours under a nitrogen atmosphere. After cooling to room temperature, the reaction solution was filtered through Celite® and the Celite® was washed three times with ethyl acetate (50 mL). The filtrate was washed three times with water (50 mL) and dried over $Na_2SO_4$. After filtering the solid, the filtrate was concentrated under reduced pressure. The residue was purified by silica gel column chromatography (0 to 10% MeOH/DCM) to afford 6-[4-[[2-(3-hydroxyphenyl)phenyl]methyl]piperazin-1 -yl] -N-(4-methoxyphenyl)pyridazine-3 - carboxamide (B-080) as a yellow solid (564.9 mg, 38.5%).

LCMS (ESI, m/z): 496 $[M+H]^+$.

Retention time: 1.381 min.

Analysis condition: PH-TFA-15.

$^1$H-NMR (300 MHz, DMSO-$d_6$) $\delta$ 10.57 (s, 1H), 9.45 (s, 1H), 7.92 (d, J = 9.6 Hz, 1H), 7.87-7.76 (m, 2H), 7.57 (d, J = 7.2 Hz, 1H), 7.39-7.31 (m, 3H), 7.24-7.20 (m, 2H), 6.97-6.87 (m, 2H), 6.84-6.72 (m, 3H), 3.74-3.70 (m, 6H), 3.45 (s, 2H), 2.48-2.43 (m, 4H).

Example 1-5: Synthesis method of carboxylic acid compound B-082 (3-[2-[[4-[4-[[3-nitro-4-(2-phenylsulfanylethylamino)phenyl]sulfonylcarbamoyl]phenyl]piperazin-1-yl]methyl]phenyl]benzoic acid)

[Formula 59]

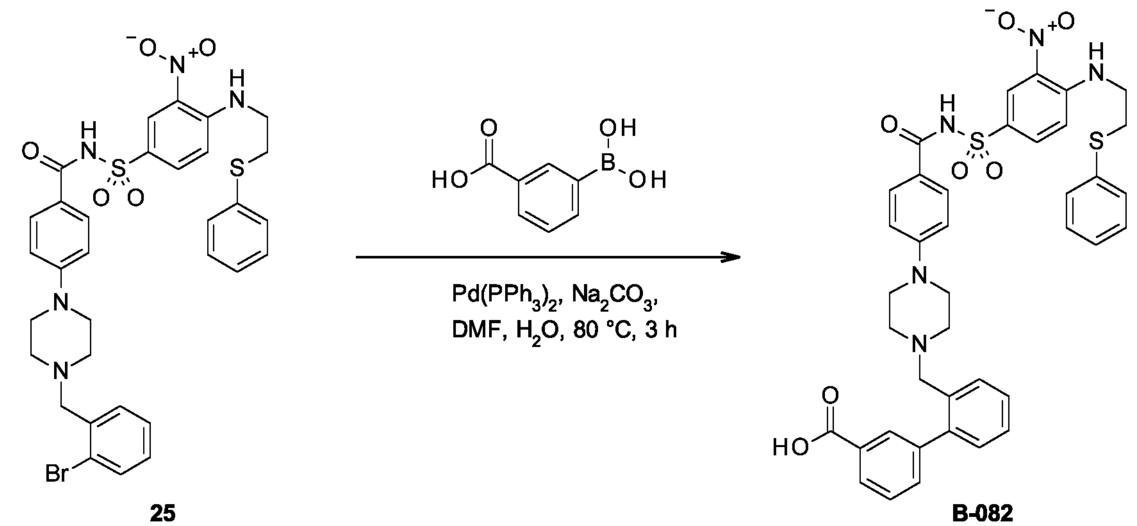


25

B-082

3-[2-[[4-[4-[[3-nitro-4-(2-phenylsulfanylethylamino)phenyl] sulfonylcarbamoyl]phenyl]piperazin-1-yl]methyl]phenyl]benzoic acid: Synthesis of carboxylic acid compound B-082

To a 50 mL flask under a nitrogen atmosphere were added 4-[4-[(2-bromophenyl)methyl]piperazin-1-yl]-N-[3-nitro-4-(2-phenylsulfanylethylamino)phenyl]sulfonylbenzamide (compound 25) (0.4 g, 0.6 mmol, 1.0 eq), 3-boronobenzoic acid (186.8 mg, 1.1 mmol, 2.0 eq), $Na_2CO_3$ (179.0 mg, 1.7 mmol, 3.0 eq), and a mixed solution of DMF/$H_2O$ (8.0 mL, 10:1). To this suspension was added $Pd(PPh_3)_2Cl_2$ (39.5 mg, 0.06 mmol, 0.1 eq), and the obtained reaction mixture was stirred at 80°C for 3 hours under a nitrogen atmosphere. After cooling to room temperature, the reaction solution was filtered through Celite® and the Celite® was washed three times with ethyl acetate (50 mL). The filtrate was washed three times with water (50 mL) and dried over $Na_2SO_4$. After filtering the solid, the filtrate was concentrated under reduced pressure. The residue was purified by reversed phase silica gel column chromatography (C18) (0 to 45% $CH_3CN$/0.1% aqueous $NH_4HCO_3$ solution) and further purified by reversed phase silica gel column chromatography (C18) (0 to 30% $CH_3CN$/0.1% aqueous formic acid solution) to afford 3-[2-[[4-[4-[[3-nitro-4-(2-phenylsulfanylethylamino)phenyl] sulfonylcarbamoyl]phenyl]piperazin-1-yl]methyl]phenyl]benzoic acid (B-082) as a yellow solid (138.3 mg, 32.3%).

LCMS (ESI, m/z): 752 $[M+H]^+$.

Retention time: 1.171 min.

Analysis condition: PH-TFA-16.

$^1$H-NMR (300 MHz, DMSO-$d_6$) δ 12.93 (s, 1H), 11.92 (s, 1H), 8.73 (t, J = 6.0 Hz, 1H), 8.56 (d, J = 2.3 Hz, 1H), 8.12 (t, J = 1.8 Hz, 1H), 7.94-7.86 (m, 2H), 7.73-7.61 (m, 3H), 7.59-7.47 (m, 2H), 7.42-7.14 (m, 9H), 6.89-6.86 (m, 2H), 3.67-3.60 (m, 2H), 3.36-3.24 (m, 8H), 2.48-2.41 (m, 4H).

Example 1-6: Synthesis method of carboxylic acid compound B-084 (5-[2-[[4-[4-[[3-nitro-4-(2-phenylsulfanylethylamino)phenyl]sulfonylcarbamoyl]phenyl]piperazin-1-yl]methyl]phenyl]pyridine-3-carboxylic acid)

[Formula 60]

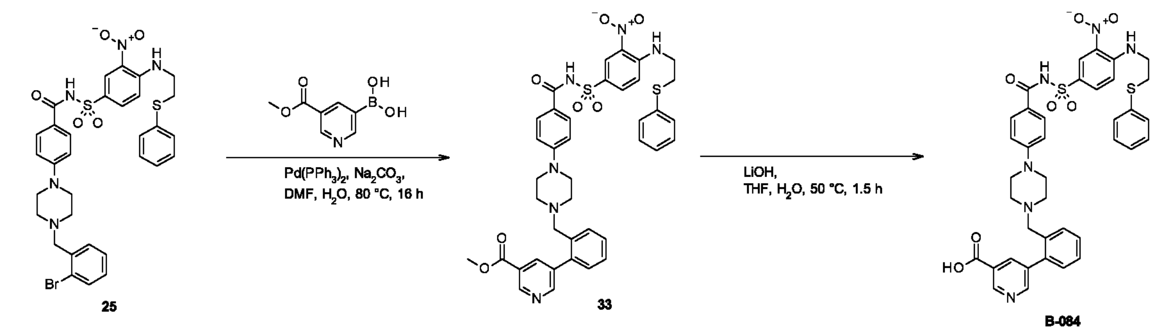


25

33

B-084

First step: Methyl 5-[2-[[4-[4-[[3-nitro-4-(2-phenylsulfanylethylamino)phenyl] sulfonylcarbamoyl]phenyl]piperazin-1-yl]methyl]phenyl]pyridine-3-carboxylate: Synthesis of compound 33

To a 50 mL flask under a nitrogen atmosphere were added 4-[4-[(2-bromophenyl)methyl]piperazin-1-yl]-N-[3-nitro-4-(2-phenylsulfanylethylamino)phenyl]sulfonylbenzamide (compound 25) (0.2 g, 0.3 mmol, 1.0 eq), (5-methoxycarbonylpyridin-3-yl)boronic acid (0.3 g, 1.4 mmol, 5.0 eq), $Na_2CO_3$ (89.5 mg, 0.8 mmol, 3.0 eq), and a mixed solution of DMF/$H_2O$ (4.0 mL, 10:1). To this suspension was added $Pd(PPh_3)_2Cl_2$ (19.8 mg, 0.03 mmol, 0.1 eq), and the obtained reaction mixture was stirred at 80°C for 16 hours under a nitrogen atmosphere. After cooling to room temperature, the reaction solution was filtered through Celite® and the Celite® was washed three times with ethyl acetate (25 mL). The filtrate was washed three times with water (25 mL) and dried over $Na_2SO_4$. After filtering the solid, the filtrate was concentrated under reduced pressure. The residue was purified by reversed phase silica gel column chromatography (C18) (5 to 70% $CH_3CN$/0.1% aqueous formic acid solution) to afford methyl 5-[2-[[4-[4-[[3-nitro-4-(2-phenylsulfanylethylamino)phenyl] sulfonylcarbamoyl]phenyl]piperazin-1-yl]methyl]phenyl]pyridine-3-carboxylate (compound 33) as a yellow solid (193.0 mg, 89.6%).

LCMS (ESI, m/z): 767 $[M+H]^+$.

Retention time: 0.823 min.

Analysis condition: PH-FA-08.

Second step: 5-[2-[[4-[4-[[3-Nitro-4-(2-phenylsulfanylethylamino)phenyl] sulfonylcarbamoyl]phenyl]piperazin-1-yl]methyl]phenyl]pyridine-3-carboxylic acid: Synthesis of carboxylic acid compound B-084

To a 30 mL flask under a nitrogen atmosphere were added methyl 5-[2-[[4-[4-[[3-nitro-4-(2-phenylsulfanylethylamino)phenyl] sulfonylcarbamoyl]phenyl]piperazin-1-yl]methyl]phenyl]pyridine-3-carboxylate (compound 33) (0.4 g, 0.5 mmol, 1.0 eq) and THF (8.0 mL). To this reaction solution was added an aqueous LiOH solution (2 mL, 62.5 mg, 2.6 mmol, 5 eq), and the obtained reaction mixture was stirred at 50°C for 1.5 hours under a nitrogen atmosphere. After cooling to room temperature, the reaction solution was concentrated under reduced pressure. The residue was purified by reversed phase silica gel column chromatography (C18) (0 to 45% $CH_3CN$/0.1% aqueous $NH_4HCO_3$ solution) and further purified by reversed phase silica gel column chromatography (C18) (0 to 30% $CH_3CN$/0.1% aqueous formic acid solution) to afford 5-[2-[[4-[4-[[3-nitro-4-(2-phenylsulfanylethylamino)phenyl] sulfonylcarbamoyl]phenyl]piperazin-1-yl]methyl]phenyl]pyridine-3-carboxylic acid (B-084) as a yellow solid (100.0 mg, 25.2%).

LCMS (ESI, m/z): 753 $[M+H]^+$.

Retention time: 1.428 min.

Analysis condition: PH-TFA-17.

$^1$H-NMR (300 MHz, DMSO-$d_6$) $\delta$ 13.37 (s, 1H), 11.98 (s, 1H), 9.04 (d, J = 2.0 Hz, 1H), 8.81-8.78 (m, 2H), 8.57-8.54 (m, 2H), 7, 88 (dd, J = 9.2, 2.3 Hz, 1H), 7.71 (d, J = 8.8 Hz, 2H), 7.45-7.33 (m, 6H), 7.27-7.13 (m, 4H), 6.89-6.86 (m, 2H), 3.67-3.61 (m, 2H), 3.36-3.22 (m, 8H), 2.48-2.40 (m, 4H).

Example 2: Investigation on O-selective methylation of arenol to substrates having various functional groups in molecule

Example 2-1: Investigation on various methylating agents using compound B-001: N-benzylsulfonyl-4-[4-[[2-(4-hydroxyphenyl)phenyl]methyl]piperazin-1-yl]benzamide (phenol) as substrate

When carrying out O-selective methylation of arenol to substrates having various functional groups, selection of the methylating agent (electrophile) is important. At first, confirmation of the range of applicable methylating agents was carried out. Note that, when described as "phenol" in the present specification, in addition to phenol itself, those in which an arbitrary substituent is added to an arbitrary location on the phenol skeleton may also be described as "phenol".

Example 2-1-1: Methylation of phenol using trimethylphenylammonium chloride B-R-01

[Formula 61]

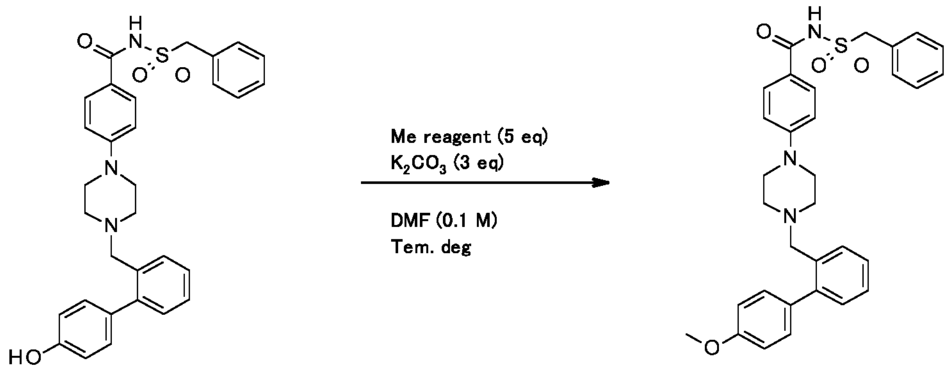


B-001 B-018

In a glove bag that had been purged with nitrogen, to a 0.6 mL screw cap vial were added N-benzylsulfonyl-4-[4-[[2-(4-hydroxyphenyl)phenyl]methyl]piperazin-1-yl]benzamide (B-001) (2.7 mg, 5.0 μmol, 1.0 eq) and DMF (50.0 μL). To the obtained solution was added $K_2CO_3$ (2.1 mg, 15.0 μmol, 3.0 eq), and after mixing for 15 to 20 seconds, trimethylphenylammonium chloride (B-R-01) (4.3 mg, 25.0 μmol, 5.0 eq) was added. The obtained reaction mixture was stirred at 80°C at 1400 rpm. Note that the rpm stands for "rotations per minute" and refers to the number of rotations in one minute. "Stirring at 1400 rpm" means stirring by 1400 rotations in one minute.

Reaction tracking

During the process, at 3 hours after the start of the reaction, 5 μL of the reaction solution was sampled and diluted with $CH_3CN$ (1000 μL) under a nitrogen atmosphere to prepare a LC sample. The reaction progress was analyzed by carrying out LC-MS analysis. The results are as shown in Table 2-1, Run 1.
N-Benzylsulfonyl-4-[4-[[2-(4-methoxyphenyl)phenyl]methyl]piperazin-1-yl]benzamide: Compound B-018.
LCMS (ESI, m/z): 556 $[M+H]^+$.
Retention time: 0.62 min (analysis condition SQD-FA05-2).
(The retention time of the raw material ArOH is 0.55 min (analysis condition SQD-FA05-2).

Example 2-1-2: Methylation of phenol using dimethyl carbonate B-R-02, trimethyl phosphate B-R-03, and methyl para-toluenesulfonate B-R-04

By the same method as in Example 2-1-1, the reaction was carried out using dimethyl carbonate (B-R-02) (2.3 mg, 25.0 μmol, 5.0 eq), trimethyl phosphate (B-R-03) (2.9 μL, 25.0 μmol, 5.0 eq), and methyl para-toluenesulfonate (B-R-04) (4.7 mg, 25.0 μmol, 5.0 eq), respectively, instead of trimethylphenylammonium chloride (B-R-01).

Reaction tracking

The reaction progress was analyzed by the same method as in Example 2-1-1.
The results of the reaction progress are shown in Table 2-1.

[Table 8]

[Table 2-1]

| Run | Me reagent (25.0 μmol) | Temp. (°C) | Time (h) | SM (B-001) (area%) | TM (B-018) (area%) | Di-Me (area%) |
|---|---|---|---|---|---|---|
| 1 | $Me_3PhN$-Cl (B-R-01) (4.3 mg) | 80 | 3 | ND | 100 | ND |
| 2 | $Me_2CO_3$ (B-R-02) (2.3 mg) | 80 | 18 | 89.3 | 10.7 | ND |
| 3 | $Me_3PO_4$ (B-R-03) (2.9 μL) | 80 | 18 | ND | 92.7 | ND |

(continued)

| Run | Me reagent (25.0 μmol) | Temp. (°C) | Time (h) | SM (B-001) (area%) | TM (B-018) (area%) | Di-Me (area%) |
|---|---|---|---|---|---|---|
| 4 | MeOTs (B-R-04) (4.7 mg) | rt | 18 | ND | 12.1 | 70.0 |

From the above results, it was confirmed that methyl para-toluenesulfonate (B-R-04), which is used as a common methylating agent, has low functional group selectivity due to its high reactivity, resulting in methylation at two locations in the substrate (the positions in the molecule at which the methylation at two locations occurred were not identified). Therefore, the use of methyl para-toluenesulfonate (B-R-04) for the present purpose was confirmed to be inappropriate (Table 2-1, Run 4). On the other hand, for the methylammonium salt, trimethyl phosphate, and dimethyl carbonate, it was confirmed that highly selective O-methylation of arenol can be achieved even in substrates having a tertiary amine or an acylsulfonamide in the molecule (Table 2-1, Runs 1 to 3).

Example 2-2: Investigation on combinations of various methylating agents and bases using compound B-002: N-benzylsulfonyl-4-[4-[[2-(5-hydroxypyridin-3-yl)phenyl]methyl]piperazin-1-yl]benzamide (pyridinol) as substrate

In Example 2-1, the range of appropriate methylating agents was specified. In the present Example, using substrate B-002 (pyridinol), which has an even greater variety of other functional groups compared to substrate B-001 (phenol), confirmation for the methylating agents in the identified range and bases was carried out.

Note that, when described as "pyridinol" in the present specification, it refers to a compound having a hydroxy group on an arbitrary carbon atom of the pyridine ring. At this time, it also refers to those in which an arbitrary substituent is attached to any other carbon atom that constitutes the pyridine ring.

Example 2-2-1: Methylation of pyridinol B-002: N-benzylsulfonyl-4-[4-[[2-(4-methoxyphenyl)phenyl]methyl]piperazin-1-yl]benzamide using trimethylphenylammonium chloride (B-R-01) (correlation of conversion rates for different stirrer sizes and numbers of rotations) (Table 2-2, Run 1 (350 rpm))

[Formula 62]

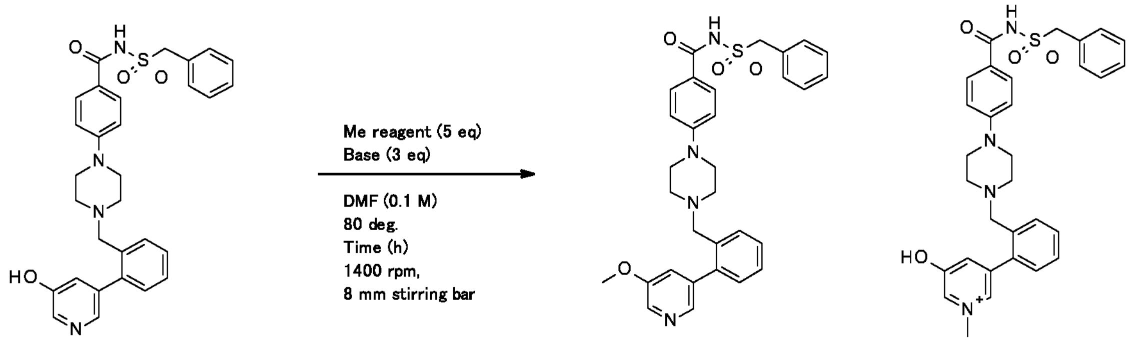


B-002 B-019 B-020

In a glove bag that had been purged with nitrogen, to a 0.6 mL screw cap vial were added N-benzylsulfonyl-4-[4-[[2-(4-methoxyphenyl)phenyl]methyl]piperazin-1-yl]benzamide (B-002) (3.8 mg, 7.0 μmol, 1.0 eq) and DMF (70.0 μL). To the obtained solution was added $Cs_2CO_3$ (6.8 mg, 21.0 μmol, 3.0 eq), and after confirming the mixing, trimethylphenylammonium chloride (B-R-01) (6.0 mg, 35.0 μmol, 5.0 eq) was added. The obtained reaction mixture was stirred at 80°C at a number of rotations of 350 rpm.

Reaction tracking

During the process, at 3 hours after the start of the reaction, 5 μL of the reaction solution was sampled in a glove bag and diluted with $CH_3CN$ (1000 μL) to prepare a LC sample. The reaction progress was analyzed by carrying out LC-MS analysis.

N-Benzylsulfonyl-4-[4-[[2-(5-methoxypyridin-3-yl)phenyl]methyl]piperazin-1-yl]benzamide: Compound B-019.

LCMS (ESI, m/z): 557.2 $[M+H]^+$.

Retention time: 0.745 min (analysis condition SMD-FA05-1).

(The retention time of the raw material ArOH is 0.648 min (analysis condition SMD-FA05-1).

N-Benzylsulfonyl-4-[4-[[2-(5-hydroxy-1-methylpyridin-1-ium-3-yl)phenyl]methyl]piperazin-1-yl]benzamide: Compound B-020.

LCMS (ESI, m/z): 557.2 $[M+H]^+$.

Retention time: 0.600 min (analysis condition SMD-FA05-1).

Example 2-2-2: Methylation of pyridinol B-002: N-benzylsulfonyl-4-[4-[[2-(5-hydroxypyridin-3-yl)phenyl]methyl]piperazin-1-yl]benzamide using trimethylphenylammonium chloride B-R-01 (correlation of conversion rates for different stirrer sizes and numbers of rotations) (Table 2-2, Run 2 (1400 rpm))

In a glove bag that had been purged with nitrogen, to a 0.6 mL screw cap vial were added N-benzylsulfonyl-4-[4-[[2-(5-hydroxypyridin-3-yl)phenyl]methyl]piperazin-1-yl]benzamide (B-002) (3.8 mg, 7.0 μmol, 1.0 eq) and DMF (70.0 μL). To the obtained solution was added $Cs_2CO_3$ (6.8 mg, 21.0 μmol, 3.0 eq), and after confirming the mixing, trimethylphenylammonium chloride (B-R-01) (6.0 mg, 35.0 μmol, 5.0 eq) was added. The obtained reaction mixture was stirred at 80°C for 3 hours at a number of rotations of 1400 rpm.

Reaction tracking

The reaction progress was analyzed by the same method as in Example 2-2-1.

Example 2-2-3: Methylation of pyridinol B-002: N-benzylsulfonyl-4-[4-[[2-(5-hydroxypyridin-3-yl)phenyl]methyl]piperazin-1-yl]benzamide using trimethylphenylammonium chloride B-R-01 (investigation on phosphazene bases) (Table 2-2, Runs 3 to 5)

In a glove bag that had been purged with nitrogen, to a 0.6 mL screw cap vial were added N-benzylsulfonyl-4-[4-[[2-(5-hydroxypyridin-3-yl)phenyl]methyl]piperazin-1-yl]benzamide (B-002) (3.8 mg, 7.0 μmol, 1.0 eq) and DMF (70.0 μL). To the obtained solution was added a phosphazene base P1tBu (5.3 μL, 21.0 μmol, 3.0 eq, Run 3), and after confirming the mixing, trimethylphenylammonium chloride (B-R-01) (6.0 mg, 35.0 μmol, 5.0 eq) was added. The obtained reaction mixture was stirred at 80°C for 3 hours at a number of rotations of 1400 rpm.

Note that the same reaction was also performed using BEMP (6.1 μL, 21.0 μmol, 3.0 eq, Run 4) or P2Et (7.0 μL, 21.0 μmol, 3.0 eq, Run 5) instead of the above phosphazene base P1tBu.

Reaction tracking

The reaction progress was analyzed by the same method as in Example 2-2-1.

Example 2-2-4: Methylation of pyridinol B-002: N-benzylsulfonyl-4-[4-[[2-(5-hydroxypyridin-3-yl)phenyl]methyl]piperazin-1-yl]benzamide using P2Et as base (investigation on methylating agents) Table 2-2, Runs 6 to 7)

In a glove bag that had been purged with nitrogen, to a 0.6 mL screw cap vial were added N-benzylsulfonyl-4-[4-[[2-(5-hydroxypyridin-3-yl)phenyl]methyl]piperazin-1-yl]benzamide (B-002) (3.8 mg, 7.0 μmol, 1.0 eq) and DMF (70.0 μL). To the obtained solution was added P2Et (7.0 μL, 21.0 μmol, 3.0 eq), and after confirming the mixing, trimethyl phosphate (B-R-03) (4.1 μL, 35.0 μmol, 5.0 eq, Run 6) was added to the vial as a methylating agent. The obtained reaction mixture was stirred at 80°C for the reaction time shown in Table 2-2 at a number of rotations of 1400 rpm.

Note that the same reaction was also performed using dimethyl carbonate (B-R-02) (3.0 μL, 35.0 μmol, 5.0 eq, Run 7) instead of the above trimethyl phosphate (B-R-03).

Reaction tracking

The reaction progress was analyzed by the same method as in Example 2-2-1.

The results of the reaction progress are shown in Table 2-2. Note that the $pKBH^+$ values in $CH_3CN$ shown in Table 2-2 were transcribed from the Aldrich web site for phosphazene bases (https://www.sigmaaldrich.com/chemistry/chemical-synthesis/technology-spotlights/phosphazenes.html) (viewed on December 2, 2020).

[Table 9]

[Table 2-2]

| Run | Me reagent | Base | Base / pKBH+ ($CH_3CN$) | Time (h) | SM (B-002) (area%) | TM (B-019) (area%) | N-Me(B020) (area%) | Byproducts (area%) |
|---|---|---|---|---|---|---|---|---|
| 1[a] | $Me_3PhN$-Cl (B-R-01) | $Cs_2CO_3$ | | 3 | 86.5 | 2.6 | 10.5 | ND |
| 2 | $Me_3PhN$-Cl (B-R-01) | $Cs_2CO_3$ | | 3 | ND | 86.6 | 12.1 | 1.3 |
| 3 | $Me_3PhN$-Cl (B-R-01) | P1tBu | 26.9 | 3 | 40.8 | 38.2 | 21.0 | ND |
| 4 | $Me_3PhN$-Cl (B-R-01) | BEMP | 27.6 | 3 | 9.7 | 54.9 | 35.4 | ND |
| 5 | $Me_3PhN$-Cl (B-R-01) | P2Et | 32.9 | 3 | ND | 88.4 | 11.6 | ND |
| 6 | $Me_3PO_4$ (B-R-03) | P2Et | 32.9 | 1 | ND | 85.0 | 13.2 | 1.8 |
| 7 | $Me_2CO_3$ (B-R-02) | P2Et | 32.9 | 23 | 55.8 | 32.9 | 11.2 | ND |
| [a]: 5 mm stirring bar, 350 rpm | | | | | | | | |

From the present results, it was shown that, when cesium carbonate was used as a base, reproducibility of the reaction rate can be improved by controlling the number of stirring, although the added base was not completely dissolved (Table 2-2, Runs 1 & 2). On the other hand, concerns about reproducibility of the reaction can be eliminated by using organic bases that are completely soluble (Table 2-2, Runs 3 to 7).

It was also shown that various combinations between the methylammonium salt, trimethyl phosphate, and dimethyl carbonate as the methylating agent and the carbonate and phosphazene bases as the base provided good results (Table 2-2, Runs 5 to 7).

In particular, when using the trimethylphenylammonium salt or trimethyl phosphate as the methylating agent and $Cs_2CO_3$ or P2Et as the base, the reaction was completed within 3 hours, and the selectivity between the target product (compound B-019) and the N-methylated product (compound B-020) was confirmed to be good (Table 2-2, Runs 2, 5, and 6).

<u>Example 2-3: Investigation on methylation using compound B-003: N-benzylsulfonyl-4-[4-[5-[2-(5-hydroxypyridin-3-yl)ethynyl]pyridine-3-carbonyl]piperazin-1-yl]benzamide (pyridinol) as substrate</u>

With respect to the reaction conditions for methylation confirmed in Example 2-2, the functional group selectivity of the reaction was further confirmed using substrate B-003: N-benzylsulfbnyl-4-[4-[5-[2-(5-hydroxypyridin-3-yl)ethynyl]pyridine-3-carbonyl]piperazin-1-yl]benzamide.

[Formula 63]

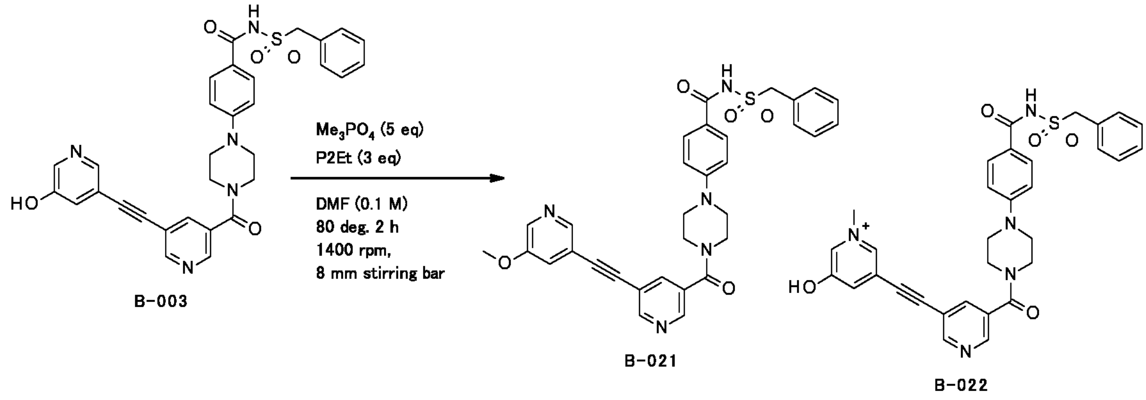


In a glove bag that had been purged with nitrogen, to a 0.6 mL screw cap vial were added N-benzylsulfonyl-4-[4-[5-[2-(5-hydroxypyridin-3-yl)ethynyl]pyridine-3-carbonyl]piperazin-1-yl]benzamide (B-003) (4.1 mg, 7.0 μmol, 1.0 eq) and DMF (70.0 μL). To the obtained solution was added P2Et (7.0 μL, 21.0 μmol, 3.0 eq), and after confirming the mixing, trimethyl phosphate (B-R-03) (4.1 μL, 35.0 μmol, 5.0 eq) was added. The obtained reaction mixture was stirred at 80°C at a number of rotations of 1400 rpm.

Reaction tracking

During the process, at 2 hours after the start of the reaction, 5 μL of the reaction solution was sampled in a glove bag and diluted with $CH_3CN$ (1000 μL) to prepare a LC sample. The reaction progress was analyzed by carrying out LC-MS analysis (analysis condition SMD-FA05-1). As a result, production of the target product (B-021) was confirmed to be 89.1 area% and production of the N-methylated product (B-022) was confirmed to be 8.3 area%.

N-Benzylsulfonyl-4-[4-[5-[2-(5-methoxypyridin-3-yl)ethynyl]pyridine-3-carbonyl]piperazin-1-yl]benzamide: Compound B-021.

LCMS (ESI, m/z): 596.1 $[M+H]^+$.

Retention time: 1.011 min (analysis condition SMD-FA05-1).

(The retention time of the raw material ArOH is 0.888 min (analysis condition SMD-FA05-1).

N-Benzylsulfonyl-4-[4-[5-[2-(5-hydroxy-1-methylpyridin-1-ium-3-yl)ethynyl]pyridine-3-carbonyl]piperazin-1-yl]benzamide: Compound B-022.

LCMS (ESI, m/z): 596.1 $[M+H]^+$.

Retention time: 0.714 min (analysis condition SMD-FA05-1).

From the above results, it was shown that the present reaction conditions are applicable as a highly selective O-methylation method for arenol even when using substrate B-003 (pyridinol: N-benzylsulfonyl-4-[4-[5-[2-(5-hydroxypyridin-3-yl)ethynyl]pyridine-3-carbonyl]piperazin-1-yl]benzamide), which contains an acetylene group and a pyridine ring.

As described above, in Example 2, it was shown that O-selective methylation of arenol can be achieved to substrates having many functional groups that can be methylated (specifically, a pyridine ring, a tertiary amine, and an acylsulfonamide). The results show that highly selective O-selective methylation is not only applicable to a single substrate having various functional groups in the molecule, but also possible to a compound library containing multiple substrates containing these groups of functional groups.

Example 3: Investigation on O-selective ethylation of arenol to substrates having various functional groups in molecule

As shown in Example 2, O-selective methylation of arenol was achieved by using organic bases and the trimethylammonium salt or trimethyl phosphate as a methylating agent. Based on this finding, investigation on O-selective ethylation was carried out using an ammonium salt and triethyl phosphate.

Example 3-1: O-Selective ethylation of pyridinol using compound B-002: N-benzylsulfonyl-4-[4-[[2-(5-hydroxypyridin-3-yl)phenyllmethyllpiperazin-1-yllbenzamide (pyridinol) as substrate

[Formula 64]

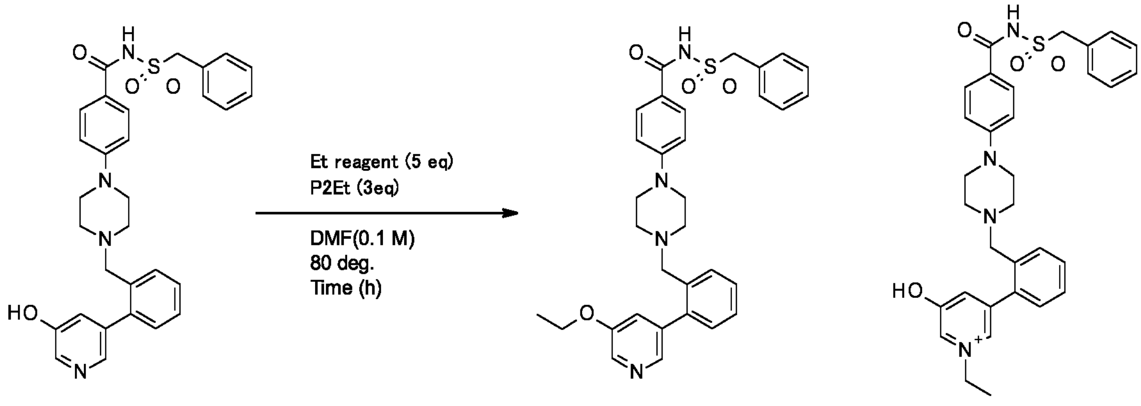


B-002 B-023 B-024

Example 3-1-1: O-Selective ethylation of compound B-002: N-benzylsulfonyl-4-[4-[[2-(5-hydroxypyridin-3-yl)phenyl]methyl]piperazin-1-yl]benzamide (pyridinol) using ammonium salt as ethylating agent

In a glove bag that had been purged with nitrogen, to a 0.6 mL screw cap vial were added N-benzylsulfonyl-4-[4-[[2-(5-hydroxypyridin-3-yl)phenyl]methyl]piperazin-1-yl]benzamide (B-002) (3.8 mg, 7.0 μmol, 1.0 eq) and DMF (70.0 μL). To the obtained solution was added P2Et (7.0 μL, 21.0 μmol, 3.0 eq), and after confirming the mixing, triethylphenylammonium iodide (B-R-07) (10.7 mg, 35.0 μmol, 5.0 eq) was added. The obtained reaction mixture was stirred at 80°C at a number of rotations of 800 rpm.

Reaction tracking

At 9.5 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-1, Run 1.

N-Benzylsulfonyl-4-[4-[[2-(5-ethoxypyridin-3-yl)phenyl]methyl]piperazin-1-yl]benzamide: Compound B-023.

LCMS (ESI, m/z): 571.2 $[M+H]^+$.

Retention time: 0.789 min (analysis condition SMD-FA05-1).

(The retention time of the raw material ArOH is 0.658 min (analysis condition SMD-FA05-1).

N-Benzylsulfonyl-4-[4-[[2-(1-ethyl-5-hydroxypyridin-1-ium-3-yl)phenyl]methyl]piperazin-1-yl]benzamide: Compound B-024.

LCMS (ESI, m/z): 571.2 $[M+H]^+$.

Retention time: 0.626 min (analysis condition SMD-FA05-1).

Example 3-1-2: O-Selective ethylation of substrate B-002: N-benzylsulfonyl-4-[4-[[2-(5-hydroxypyridin-3-yl)phenyl]methyl]piperazin-1-yl]benzamide: N-benzylsulfonyl-4-[4-[[2-(5-hydroxypyridin-3-yl)phenyl]methyl]piperazin-1-yl]benzamide (pyridinol) using triethyl phosphate as ethylating agent

In a glove bag that had been purged with nitrogen, to a 0.6 mL screw cap vial were added N-benzylsulfonyl-4-[4-[[2-(5-hydroxypyridin-3-yl)phenyl]methyl]piperazin-1-yl]benzamide (B-002) (3.8 mg, 7.0 μmol, 1.0 eq) and DMF (70.0 μL). To the obtained solution was added P2Et (7.0 μL, 21.0 μmol, 3.0 eq), and after confirming the mixing, triethyl phosphate (B-R-05) (6.0 μL, 35.0 μmol, 5.0 eq) was added. The obtained reaction mixture was stirred at 80°C at a number of rotations of 800 rpm.

Reaction tracking

During the process, at 1, 2.5, 5, and 19 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-1, Run 2.

Example 3-1-3: O-Selective ethylation of substrate B-002: N-benzylsulfonyl-4-[4-[[2-(5-hydroxypyridin-3-yl)phenyl]methyl]piperazin-1-yl]benzamide (pyridinol) using P2tBu, bulky phosphazene base, and using triethyl phosphate as ethylating agent

In a glove bag that had been purged with nitrogen, to a 0.6 mL screw cap vial were added N-benzylsulfonyl-4-[4-[[2-(5-hydroxypyridin-3-yl)phenyl]methyl]piperazin-1-yl]benzamide (B-002) (3.8 mg, 7.0 μmol, 1.0 eq) and DMF (70.0 μL). To the obtained solution was added P2tBu (10.5 μL, 2.0 mol/L, 21.0 μmol, 3.0 eq), and after confirming the mixing, triethyl phosphate (B-R-05) (6.0 μL, 35.0 μmol, 5.0 eq) was added. The obtained reaction mixture was stirred at 80°C at a number of rotations of 800 rpm.

Reaction tracking

During the process, at 1, 2.5, 5, and 21 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-1, Run 3.

Note that the $pKBH^+$ values in $CH_3CN$ shown in Table 3-1 were transcribed from the Aldrich web site for phosphazene bases (https://www.sigmaaldrich.com/chemistry/chemical-synthesis/technology-spotlights/phosphazenes.html) (viewed on December 2, 2020).

Note that the Ratio (O-/N-) shown in Table 3-1 was calculated as (area% of O-ethylated product (B-023))/(area% of N-ethylated product (B-024)).

The results of Example 3-1 are shown in Table 3-1.

[Table 10]

[Table 3-1]

| Run | Et reagent | Base / $pKBH^+$ ($CH_3CN$) | Time (h) | SM (B-002) (area%) | O-Et (B-023) (area%) | N-Et (B-024) (area%) | Ratio (O-/N-) | Byproducts |
|---|---|---|---|---|---|---|---|---|
| 1 | $Et_3PhN$-I (B-R-07) | P2Et (32.9) | 9.5 | 28.5 | 53.3 | 5.2 | 10.2 | 13.0 |
| 2 | $Et_3PO_4$ (B-R-05) | P2Et (32.9) | 1 | 34.5 | 62.5 | 3.0 | 20.8 | ND |
| | | | 2.5 | 4.8 | 91.0 | 4.2 | 21.7 | ND |
| | | | 5 | 0.1 | 96.1 | 3.8 | 25.3 | ND |
| | | | 19 | ND | 96.7 | 3.3 | 29.3 | ND |
| 3 | $Et_3PO_4$ (B-R-05) | P2tBu (33.5) | 1 | 14.1 | 82.3 | 3.6 | 22.8 | ND |
| | | | 2.5 | 1.4 | 94.9 | 3.7 | 25.6 | ND |
| | | | 5 | ND | 96.6 | 3.1 | 31.2 | ND |
| | | | 21 | ND | 97.9 | 1.7 | 57.6 | ND |

As shown in Table 3-1, O-selective ethylation of substrate B-002 was achieved under the reaction conditions using triethyl phosphate/P2Et (Table 3-1, Run 2). O-selective ethylation was also achieved by using P2tBu as a base, which is more bulky than P2Et with similar basicity ($pKBH^+$ value) (Table 3-1, Run 3).

Note that, under the above conditions, the O-selectivity was also confirmed to be improved over time. While not limited to any specific theory, this improvement in selectivity is the result of the progress of the elimination reaction of N-ethylated product B-024 by the action of the base in the reaction system, resulting in its conversion to the raw material B-002, which again undergoes O-ethylation.

Example 3-2: O-Selective ethylation of pyridinol using compound B-004: N-benzylsulfonyl-4-[4-[3-fluoro-4-(5-hydroxypyridin-3-yl)benzoyl]piperazin-1-yl]benzamide as substrate

[Formula 65]

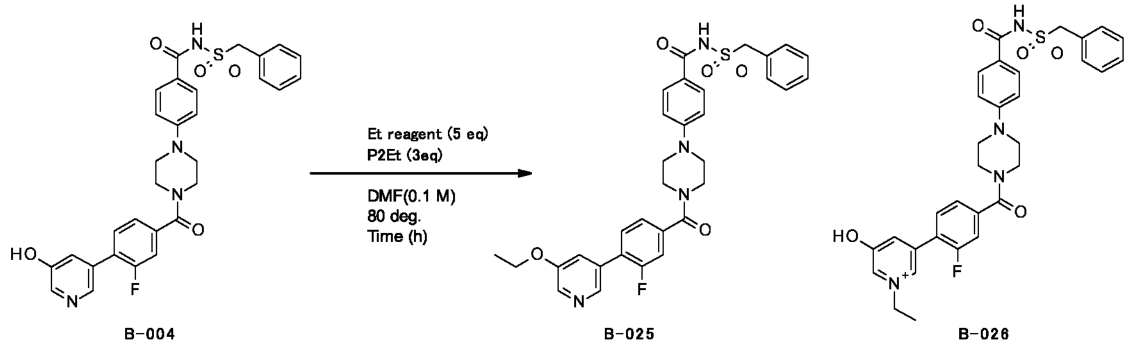


B-004 B-025 B-026

Example 3-2-1: O-Selective ethylation of substrate B-004: N-benzylsulfonyl-4-[4-[3-fluoro-4-(5-hydroxypyridin-3-yl)benzoyl]piperazin-1-yl]benzamide) (pyridinol) using ammonium salt as ethylating agent

In a glove bag that had been purged with nitrogen, using N-benzylsulfonyl-4-[4-[3-fluoro-4-(5-hydroxypyridin-3-yl)benzoyl]piperazin-1-yl]benzamide) (B-004) (4.0 mg, 7.0 μmol, 1.0 eq) in a 0.6 mL screw cap vial, the reaction was performed by the same method as in Example 3-1-1 and the reaction progress was analyzed. The results were as shown in Table 3-2, Run 1.

N-Benzylsulfonyl-4-[4-[4-(5-ethoxypyridin-3-yl)-3-fluorobenzoyl]piperazin-1-yl]benzamide: Compound B-025.

LCMS (ESI, m/z): 603.2 $[M+H]^+$.

Retention time: 1.052 min (analysis condition SMD-FA05-1).

(The retention time of the raw material ArOH is 0.831 min (analysis condition SMD-FA05-1).

N-Benzylsulfonyl-4-[4-[4-(1-ethyl-5-hydroxypyridin-1-ium-3-yl)-3-fluorobenzoyl]piperazin-1-yl]benzamide: Compound B-026.

LCMS (ESI, m/z): 603.2 $[M+H]^+$.

Retention time: 0.756 min (analysis condition SMD-FA05-1).

Example 3-2-2: O-Selective ethylation of substrate B-004: pyridinol, N-benzylsulfonyl-4-[4-[3-fluoro-4-(5-hydroxypyridin-3-yl)benzoyl]piperazin-1-yl]benzamide using triethyl phosphate as ethylating agent

In a glove bag that had been purged with nitrogen, using pyridinol, N-benzylsulfonyl-4-[4-[3-fluoro-4-(5-hydroxypyridin-3-yl)benzoyl]piperazin-1-yl]benzamide (B-004) (4.0 mg, 7.0 μmol, 1.0 eq) in a 0.6 mL screw cap vial, the reaction was performed by the same method as in Example 3-1-2 and the reaction progress was analyzed. The results were as shown in Table 3-2, Run 2.

The results of Example 3-2 are shown in Table 3-2. Note that the Ratio (O-/N-) shown in Table 3-2 was calculated as (area% of O-ethylated product (B-025))/(area% of N-ethylated product (B-026)).

[Table 11]

[Table 3-2]

| Run | Et reagent | Time (h) | SM (B-004) (area%) | O-Et (B-025) (area%) | N-Et (B-026) (area%) | Ratio (O-/N-) | Byproducts (area%) |
|---|---|---|---|---|---|---|---|
| 1 | $Et_3PhN$-I (B-R-07) | 9.5 | 24.1 | 66.6 | 2.6 | 25.6 | 6.6 |
| 2 | $Et_3PO_4$ (B-R-05) | 1 | 42.4 | 55.9 | 1.7 | 32.9 | NO |

(continued)

| Run | Et reagent | Time (h) | SM (B-004) (area%) | O-Et (B-025) (area%) | N-Et (B-026) (area%) | Ratio (O-/N-) | Byproducts (area%) |
|---|---|---|---|---|---|---|---|
| | | 2.5 | 9.7 | 88.0 | 1.9 | 46.3 | NO |
| | | 5 | 2.2 | 96.0 | 1.2 | 80.0 | ND |
| | | 19 | NO | 99.0 | 0.6 | 165.0 | NO |

As shown in Table 3-2, it was shown that substrate B-004 can also be O-selectively ethylated under the reaction conditions using triethyl phosphate/P2Et (Table 3-2, Run 2). While not limited to any specific theory, the pyridine of substrate B-004 is more electron deficient compared to substrate B-002 due to the influence of the fluoro group and amide group (carbonyl group) on the adjacent benzene ring, and because of their influence, N-ethylation was suppressed. As a result, O-selectivity was improved over the case where B-002 was used as the substrate.

Example 3-3: O-Selective ethylation of pyridinol using substrates B-003: N-benzylsulfonyl-4-[4-[5-[2-(5-hydroxypyridin-3-yl)ethynyl]pyridine-3-carbonyl]piperazin-1-yl]benzamide and B-005: N-benzylsulfonyl-6-[4-[5-[2-(5-hydroxypyridin-3-yl)ethynyl]pyridine-3-carbonyl]piperazin-1-yl]pyridazine-3-carboxamide as compounds having an acetylene functional group

[Formula 66]

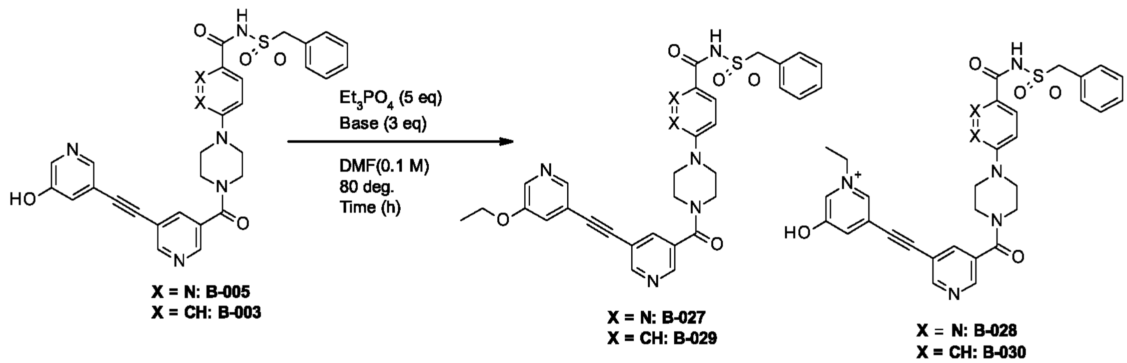


X = N: B-005
X = CH: B-003

X = N: B-027
X = CH: B-029

X = N: B-028
X = CH: B-030

Example 3-3-1: O-Selective ethylation of substrate B-005: N-benzylsulfonyl-6-[4-[5-[2-(5-hydroxypyridin-3-yl)ethynyl]pyridine-3-carbonyl]piperazin-1-yl]pyridazine-3-carboxamide (pyridinol) using triethyl phosphate as ethylating agent

In a glove bag that had been purged with nitrogen, to a 0.6 mL screw cap vial were added N-benzylsulfonyl-6-[4-[5-[2-(5-hydroxypyridin-3-yl)ethynyl]pyridine-3-carbonyl]piperazin-1-yl]pyridazine-3-carboxamide (B-005) (4.1 mg, 7.0 μmol, 1.0 eq) and DMF (70.0 μL). To the obtained solution was added P2Et (7.0 μL, 21.0 μmol, 3.0 eq), and after confirming the mixing, triethyl phosphate (B-R-05) (6.0 μL, 35.0 μmol, 5.0 eq) was added. The obtained reaction mixture was stirred at 80°C at a number of rotations of 800 rpm.

Reaction tracking

During the process, at 3, 5, and 20 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-3, Run 1.

N-Benzylsulfonyl-6-[4-[5-[2-(5-ethoxypyridin-3-yl)ethynyl]pyridine-3-carbonyl]piperazin-1-yl]pyridazine-3-carboxamide: Compound B-027.

LCMS (ESI, m/z): 612.1 $[M+H]^+$.

Retention time: 1.014 min (analysis condition SMD-FA05-1).

(The retention time of the raw material ArOH is 0.831 min (analysis condition SMD-FA05-1).

N-Benzylsulfonyl-6-[4-[5-[2-(1-ethyl-5-hydroxypyridin-1-ium-3-yl)ethynyl]pyridine-3-carbonyl]piperazin-1-yl]pyridazine-3-carboxamide: Compound B-028.

LCMS (ESI, m/z): 612.2 $[M+H]^+$.

Retention time: 0.693 min (analysis condition SMD-FA05-1).

Compound B-031 (B-027-P2Et adduct: compound showing MS (Exact MS: 951.44) with P2Et added to B-027, structure not determined).

LCMS (ESI, m/z): 951.4 $[M]^+$.

Retention time: 0.909 min (analysis condition SMD-FA05-1).

Example 3-3-2: O-Selective ethylation of substrate B-003: N-benzylsulfonyl-4-[4-[5-[2-(5-hydroxypyridin-3-yl)ethynyl]pyridine-3-carbonyl]piperazin-1-yl]benzamide (pyridinol) by triethyl phosphate using P2Et as base

In a glove bag that had been purged with nitrogen, to a 0.6 mL screw cap vial were added N-benzylsulfonyl-4-[4-[5-[2-(5-hydroxypyridin-3-yl)ethynyl]pyridine-3-carbonyl]piperazin-1-yl]benzamide (B-003) (4.1 mg, 7.0 μmol, 1.0 eq) and DMF (70.0 μL). To the obtained solution was added P2Et (7.0 μL, 21.0 μmol, 3.0 eq), and after confirming the mixing, triethyl phosphate (B-R-05) (6.0 μL, 35.0 μmol, 5.0 eq) was added. The obtained reaction mixture was stirred at 80°C at a number of rotations of 800 rpm.

Reaction tracking

During the process, at 2.5, 5, and 21 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-3, Run 2.

N-Benzylsulfonyl-4-[4-[5-[2-(5-ethoxypyridin-3-yl)ethynyl]pyridine-3-carbonyl]piperazin-1-yl]benzamide: Compound B-029.

LCMS (ESI, m/z): 610.2 $[M+H]^+$.

Retention time: 1.069 min (analysis condition SMD-FA05-1).

(The retention time of the raw material ArOH is 0.889 min (analysis condition SMD-FA05-1).

N-Benzylsulfonyl-4-[4-[5-[2-(1-ethyl-5-hydroxypyridin-1-ium-3-yl)ethynyl]pyridine-3-carbonyl]piperazin-1-yl]benzamide: Compound B-030.

LCMS (ESI, m/z): 610 $[M+H]^+$.

Retention time: 0.737 min (analysis condition SMD-FA05-1).

Compound B-032 (B-029-P2Et adduct: compound showing MS (Exact MS: 949.45) with P2Et added to B-029, structure not determined).

LCMS (ESI, m/z): 949.4 $[M]^+$.

Retention time: 0.929 min (analysis condition SMD-FA05-1).

From the results of Example 3-3-1 and Example 3-3-2, when ethylation of these substrates having acetylene (B-005 and B-003) was carried out using P2Et as the base, the respective byproducts (B-031 and B-032) were observed, whose structures were not determined but were presumed to be P2Et added to the target compounds, O-ethylated products B-027 and B-029. In order to avoid these side reactions, ethylation was investigated using P2tBu as the base whose conjugated acid has a pKa ($pKBH^+$) similar to that of P2Et and which is more bulky.

Example 3-3-3: O-Selective ethylation of substrate B-003: N-benzylsulfonyl-4-[4-[5-[2-(5-hydroxypyridin-3-yl)ethynyl]pyridine-3-carbonyl]piperazin-1-yl]benzamide (pyridinol) using P2tBu, bulky phosphazene base, and using triethyl phosphate as ethylating agent (avoiding production of base adduct)

In a glove bag that had been purged with nitrogen, to a 0.6 mL screw cap vial were added N-benzylsulfonyl-4-[4-[5-[2-(5-hydroxypyridin-3-yl)ethynyl]pyridine-3-carbonyl]piperazin-1-yl]benzamide (B-003) (4.1 mg, 7.0 μmol, 1.0 eq) and DMF (70.0 μL). To the obtained solution was added P2tBu (10.5 μL, 2 mol/L, 21.0 μmol, 3.0 eq), and after confirming the mixing, triethyl phosphate (B-R-05) (6.0 μL, 35.0 μmol, 5.0 eq) was added. The obtained reaction mixture was stirred at 80°C at a number of rotations of 800 rpm.

Reaction tracking

During the process, at 2.5, 5, and 21 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-3, Run 3.

The results of Example 3-3 are shown in Table 3-3. Note that the $pKBH^+$ values in $CH_3CN$ shown in Table 3-3 were transcribed from the Aldrich web site for phosphazene bases (https://www.sigmaaldrich.com/chemistry/chemical-synthesis/technology-spotlights/phosphazenes.html) (viewed on December 2, 2020).

Note that the Ratio (O-/N-) shown in Table 3-3 was calculated as (area% of O-ethylated product)/(area% of N-ethylated product).

[Table 12]

[Table 3-3]

| Run | Substrate | X | Base / pKBH+ ($CH_3CN$) | Time (h) | SM (area%) | O-Et (area%) | N-Et (area%) | Ratio (O-/N-) | Byproduct (base-adduct) (area%) |
|---|---|---|---|---|---|---|---|---|---|
| 1 | B-005 | N | P2Et (32.9) | 3 | 26.0 | 70.1 | 0.8 | 87.6 | 3.0 |
| | | | | 5 | 11.0 | 84.1 | 0.2 | 420.5 | 4.2 |
| | | | | 20 | ND | 92.5 | 0.04 | 2312.5 | 4.4 |
| 2 | B-003 | CH | P2Et (32.9) | 2.5 | 36.1 | 59.1 | 0.3 | 197.0 | 4.4 |
| | | | | 5 | 12.6 | 79.2 | <0.1 | >100 | 8.0 |
| | | | | 21 | ND | 90.3 | ND | >100 | 9.2 |
| 3 | B-003 | CH | P2tBu (33.5) | 2.5 | 17.8 | 82.2 | ND | >100 | ND |
| | | | | 5 | 3.4 | 96.3 | ND | >100 | ND |
| | | | | 21 | ND | 98.3 | ND | >100 | ND |

As shown in Table 3-3, Run 3, it was shown that the use of the bulky base can avoid the production of an impurity (B-032), which may be due to the addition reaction of the phosphazene base to acetylene, and can achieve O-selective ethylation.

Example 3-4: Experiment to confirm selectivity for amide group during ethylation

Whether the O-selective ethylation conditions for arenol by the combination of triethyl phosphate/phosphazene base shown so far can be performed in the presence of an amide group was confirmed. Specifically, instead of evaluating the selectivity using a substrate containing both arenol and other functional groups in a single molecule as confirmed in Examples 3-1 to 3-3, an amide substrate (tert-butyl 4-[4-[(4-fluorophenyl)methylcarbamoyl]phenyl]piperazine-1-carboxylate) (B-016) was used as a model substrate here and It was decided to confirm the selectivity by showing that the amide substrate B-016 was not ethylated under the conditions found in Example 3-3, which allows for selective ethylation of arenol.

[Formula 67]

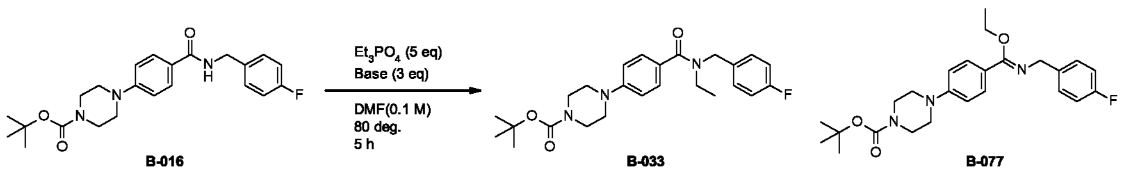


B-016 B-033 B-077

Example 3-4-1: Ethylation of tert-butyl 4-[4-[(4-fluorophenyl)methylcarbamoyl]phenyl]piperazine-1-carboxylate (B-016) (amide model substrate) using P2tBu as base and triethyl phosphate as ethylating agent

In a glove bag that had been purged with nitrogen, to a 0.6 mL screw cap vial were added tert-butyl 4-[4-[(4-fluorophenyl)methylcarbamoyl]phenyl]piperazine-1-carboxylate (B-016) (4.1 mg, 10.0 μmol, 1.0 eq) and DMF (100.0 μL). To the obtained solution was added P2tBu (15.0 μL, 2.0 mol/L, 30.0 μmol, 3.0 eq), and after confirming the mixing, triethyl phosphate (B-R-05) (8.5 μL, 50.0 μmol, 5.0 eq) was added. The obtained reaction mixture was stirred at 80°C at a number of rotations of 800 rpm.

Reaction tracking

During the process, at 1.5, 3, and 5 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-4, Run 1.

tert-Butyl 4- [4-[ethyl- [(4-fluorophenyl)methyl] carbamoyl]phenyl]piperazine-1 - carboxylate: Compound B-033.

LCMS (ESI, m/z): 442 $[M+H]^+$.

Retention time: 1.308 min (analysis condition SMD-FA05-1).

(The retention time of the raw material amide is 1.172 min (analysis condition SMD-FA05-1).

Example 3-4-2: Ethylation of tert-butyl 4-[4-[(4-fhiorophenyl)methylcarbamoyl]phenyl]piperazine-1-carboxylate (B-016) (amide model substrate) using P2Et as base and triethyl phosphate as ethylating agent

In a glove bag that had been purged with nitrogen, to a 0.6 mL screw cap vial were added tert-butyl 4-[4-[(4-fluorophenyl)methylcarbamoyl]phenyl]piperazine-1-carboxylate (B-016) (4.1 mg, 10.0 μmol, 1.0 eq) and DMF (100.0 μL). To the obtained solution was added P2Et (10.0 μL, 30.0 μmol, 3.0 eq), and after confirming the mixing, triethyl phosphate (B-R-05) (8.5 μL, 50.0 μmol, 5.0 eq) was added. The obtained reaction mixture was stirred at 80°C at a number of rotations of 800 rpm.

Reaction tracking

During the process, at 1.5, 3, and 5 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-4, Run 2.

Example 3-4-3: Ethylation of tert-butyl 4-[4-[(4-fluorophenyl)methylcarbamoyl]phenyl]piperazine-1-carboxylate (B-016) (amide model substrate) using phosphazenes (BEMP and P1tBu) with $pKBH^+$ (pKa of conjugated acid) of 30 or less

The reaction was carried out by the same method as in Example 3-4-2, using BEMP (8.7 μL, 30.0 μmol, 3.0 eq, Run 3) and P1tBu (7.6 μL, 30.0 μmol, 3.0 eq, Run 4) instead of P2Et, respectively.

Reaction tracking

The reaction progress was analyzed by the same method as in Example 3-4-2. The results were as shown in Table 3-4, Runs 3 and 4.

The results of Example 3-4 are shown in Table 3-4. Note that the $pKBH^+$ values in $CH_3CN$ shown in Table 3-4 were transcribed from the Aldrich web site for phosphazene bases (https://www.sigmaaldrich.com/chemistry/chemical-synthesis/technology-spotlights/phosphazenes.html) (viewed on December 2, 2020).

[Table 13]

[Table 3-4]

| Run | Base / $pKBH^+$ ($CH_3CN$) | Time (h) | SM (B-016) (area%) | TM (B-033) or (B-077) (area%) |
|---|---|---|---|---|
| 1 | P2tBu (33.5) | 5 | 89.4 | 10.6 |
| 2 | P2tEt (32.9) | 5 | 91.1 | 8.9 |
| 3 | BEMP (27.6) | 5 | 100.0 | ND |
| 4 | P1tBu (26.9) | 5 | 100.0 | ND |

As shown in Table 3-4, Runs 1 & 2, when a highly basic base with a $pKBH^+$ (pKa of conjugated acid) of 30 or more was present, the amide reacted with triethyl phosphate to give a N- or O-ethylated product. This indicates that the amide functional group cannot tolerate ethylation conditions under basic conditions with $pKBH^+$ of 30 or more.

On the other hand, when bases with a $pKBH^+$ of 30 or less were present in the reaction, as shown in Runs 3 & 4, ethylation of the amide did not proceed.

The results of the present experiment indicate that, when the substrate has an amide bond, the use of a base with a pKBH+ (pKa of conjugated acid) of 30 or less allows for highly selective O-selective ethylation of arenol even on a substrate with a coexisting amide group.

Example 3-5: Confirmation of optimal conditions for ethylation reaction assuming presence of amide bond in substrate

Using a base with a pKBH+ (pKa of conjugated acid) of 30 or less as shown in Example 3-4, the O-selective ethylation of arenol was investigated using N-benzylsulfonyl-4-[4-[3-fluoro-4-(5-hydroxypyridin-3-yl)benzoyl]piperazin-1-yl]benzamide (B-004) and N-benzylsulfonyl-4-[4-[5-[2-(5-hydroxypyridin-3-yl)ethynyl]pyridine-3-carbonyl]piperazin-1-yl]benzamide (B-003) as substrates.

Example 3-5-1: Investigation on optimal base in O-selective ethylation using N-benzylsulifonyl-4-[4-[3-fluoro-4-(5-hydroxypyridin-3-yl)benzoyl]piperazin-1-yl]benzamide (B-004) (pyridinol) as substrate

[Formula 68]

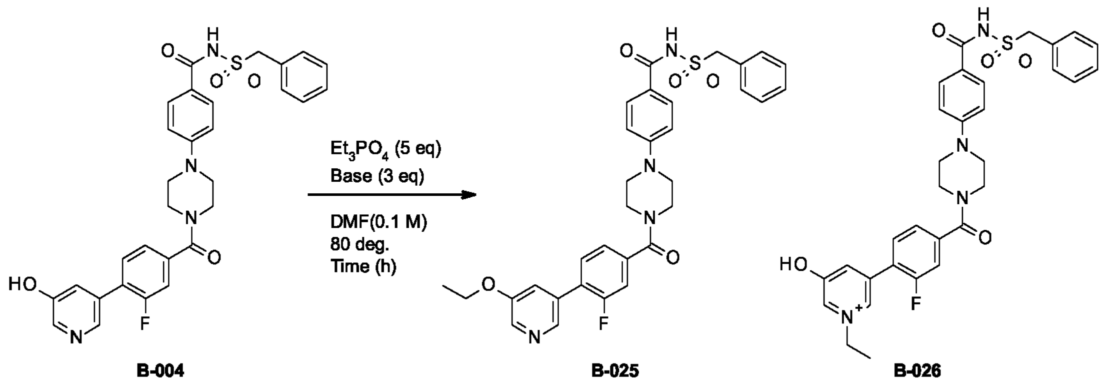


B-004 B-025 B-026

In a glove bag that had been purged with nitrogen, to a 0.6 mL screw cap vial were added N-benzylsulfonyl-4-[4-[3-fluoro-4-(5-hydroxypyridin-3-yl)benzoyl]piperazin-1-yl]benzamide (B-004) (4.0 mg, 7.0 μmol, 1.0 eq) and DMF (70.0 μL). To that solution was added P1tBu (5.3 μL, 21.0 μmol, 3.0 eq, Run 1), and after confirming the mixing, triethyl phosphate (B-R-05) (6.0 μL, 35.0 μmol, 5.0 eq) was added. The obtained reaction mixture was stirred at 80°C at a number of rotations of 800 rpm. Note that the same reaction was performed using BEMP (6.1 μL, 21.0 μmol, 3.0 eq, Run 2) or BTPP (6.4 μL, 21.0 μmol, 3.0 eq, Run 3) instead of P1tBu.

Reaction tracking

During the process, at 2.5, 5, 23, and 29 hours after the start of the reaction, 5 μL of the reaction solution was sampled in a glove bag and diluted with $CH_3CN$ (1000 μL) to prepare a LC sample. The reaction progress was analyzed by carrying out LC-MS analysis.

The results of Example 3-5-1 are shown in Table 3-5-1. Note that the pKBH+ values in $CH_3CN$ shown in Table 3-5-1 were transcribed from the Aldrich web site for phosphazene bases (https://www.sigmaaldrich.com/chemistry/chemical-synthesis/technology-spotlights/phosphazenes.html) (viewed on December 2, 2020).

Also, the Ratio (O-/N-) shown in Table 3-5-1 was calculated as (area% of O-ethylated product (B-025))/(area% of N-ethylated product (B-026)).

[Table 14]

[Table 3-5-1]

| Run | Base / pKBH+ ($CH_3CN$) | Time (h) | SM (B-004) (area%) | O-Et (B-025) (area%) | N-Et (B-026) (area%) | Ratio (O-/N-) | Byproducts (area%) |
|---|---|---|---|---|---|---|---|
| 1 | P1tBu (26.9) | 2.5 | 87.2 | 11.1 | 1.6 | 6.9 | ND |

(continued)

| Run | Base / pKBH+ ($CH_3CN$) | Time (h) | SM (B-004) (area%) | O-Et (B-025) (area%) | N-Et (B-026) (area%) | Ratio (O-/N-) | Byproducts (area%) |
|---|---|---|---|---|---|---|---|
| | | 5<br>23 | 74.5<br>25.1 | 22.4<br>66.3 | 3.1<br>8.0 | 7.2<br>8.3 | ND<br>ND |
| | | 29 | 19.3 | 72.1 | 8.0 | 9.0 | ND |
| 2 | BEMP (27.6) | 2.5<br>5 | 82.2<br>67.6 | 16.1<br>29.4 | 1.7<br>2.9 | 9.5<br>10.1 | ND<br>ND |
| | | 23 | 13.3 | 79.3 | 6.8 | 11.7 | ND |
| | | 29 | 7.8 | 84.6 | 6.9 | 12.3 | ND |
| 3 | BTPP (28.4) | 2.5<br>5 | 71.1<br>46.2 | 26.9<br>50.3 | 1.9<br>3.5 | 14.1<br>14.4 | ND<br>ND |
| | | 23 | 0.8 | 93.5 | 4.8 | 19.5 | ND |
| | | 29 | 0.2 | 94.3 | 4.5 | 20.9 | ND |

Example 3-5-2: Investigation on optimal base in O-selective ethylation using N-benzylsulfonyl-4-[4-[5-[2-(5-hydroxypyridin-3-yl)ethynyl]pyridine-3-carbonyl]piperazin-1-yl]benzamide (B-003) (pyridinol) having acetylene bond as substrate

[Formula 69]

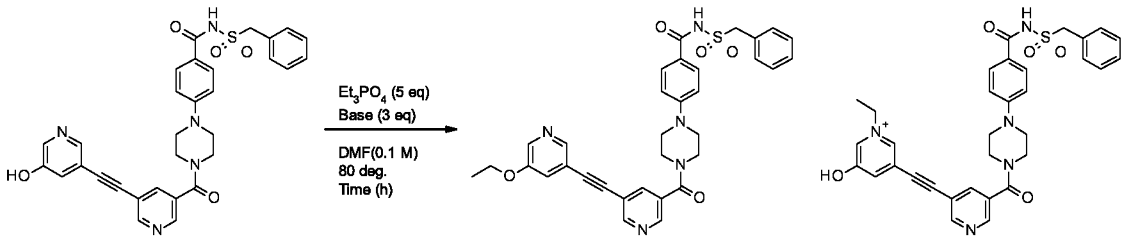


B-003 B-029 B-030

In a glove bag that had been purged with nitrogen, to a 0.6 mL screw cap vial were added N-benzylsulfonyl-4-[4-[5-[2-(5-hydroxypyridin-3-yl)ethynyl]pyridine-3-carbonyl]piperazin-1-yl]benzamide (B-003) (4.1 mg, 7.0 μmol, 1.0 eq) and DMF (70.0 μL). To this solution was added P1tBu (5.3 μL, 21.0 μmol, 3.0 eq, Run 1), and after confirming the mixing, triethyl phosphate (B-R-05) (6.0 μL, 35.0 μmol, 5.0 eq) was added. The obtained reaction mixture was stirred at 80°C at a number of rotations of 800 rpm. Note that the same reaction was performed using BEMP (6.1 μL, 21.0 μmol, 3.0 eq, Run 2) or BTPP (6.4 μL, 21.0 μmol, 3.0 eq, Run 3) instead of P1tBu.

Reaction tracking

During the process, at 1, 5, 22, and 30 hours after the start of the reaction, 5 μL of the reaction solution was sampled in a glove bag and diluted with $CH_3CN$ (1000 μL) to prepare a LC sample. The reaction progress was analyzed by carrying out LC-MS analysis.

The results of Example 3-5-2 are shown in Table 3-5-2. Note that the pKBH+ values in $CH_3CN$ shown in Table 3-5-2 were transcribed from the Aldrich web site for phosphazene bases (https://www.sigmaaldrich.com/chemistry/chemical-synthesis/technology-spotlights/phosphazenes.html) (viewed on December 2, 2020).

Also, the Ratio (O-/N-) shown in Table 3-5-2 was calculated as (area% of O-ethylated product (B-029))/(area% of N-ethylated product (B-030)).

[Table 15]

[Table 3-5-2]

| Run | Base / pKBH+ ($CH_3CN$) | Time (h) | SM (B-003) (area%) | O-Et (B-029) (area%) | N-Et (B-030) (area%) | Ratio (O-/N-) | Byproducts (area%) |
|---|---|---|---|---|---|---|---|
| 1 | P1tBu (26.9) | 1 | 93.1 | 6.5 | 0.3 | 21.7 | ND |
| | | 5 | 62.5 | 35.5 | 1.7 | 20.9 | ND |
| | | 22 | 7.1 | 89.7 | 2.7 | 33.2 | ND |
| | | 30 | 2.7 | 94.3 | 2.3 | 41.0 | ND |
| 2 | BEMP (27.6) | 1 | 91.2 | 8.5 | 0.3 | 28.3 | ND |
| | | 5 | 52.4 | 45.8 | 1.7 | 26.9 | ND |
| | | 22 | 1.5 | 96.0 | 1.8 | 53.3 | ND |
| | | 30 | 0.3 | 97.6 | 1.3 | 75.0 | ND |
| 3 | BTPP (28.4) | 1 | 83.4 | 18.0 | 0.6 | 26.7 | ND |
| | | 5 | 27.1 | 70.9 | 1.7 | 41.7 | ND |
| | | 22 | ND | 98.8 | 0.2 | 494.0 | ND |

From the above results (Tables 3-5-1 and 3-5-2), it was shown that a high conversion rate of the reaction can be achieved while securing a wide range of functional group selectivity using a base with a $pKBH^+$ (pKa of conjugated acid) of 30 or less. In the case where an amide bond that can be ethylated is present, the use of a base with a $pKBH^+$ (pKa of conjugated acid) (in acetonitrile) of 30 or less as the base used in the reaction is as shown in Example 3-4. The results of Example 3-5 show that a reaction rate sufficient for synthesis and high O-selectivity can be achieved when, among bases with a $pKBH^+$ of 30 or less, a bulky base, especially BTPP, is selected as the base used in the reaction (Table 3-5-1, Run 3 and Table 3-5-2, Run 3).

Example 3-5-3: Investigation on optimal base in O-selective ethylation using N-benzylsulfonyl-6-[4-[3-fluoro-4-(5-hydroxypyridin-3-yl)benzoyl]piperazin-1-yl]pyridazine-3-carboxamide (B-006) as substrate having pyridazine

[Formula 70]

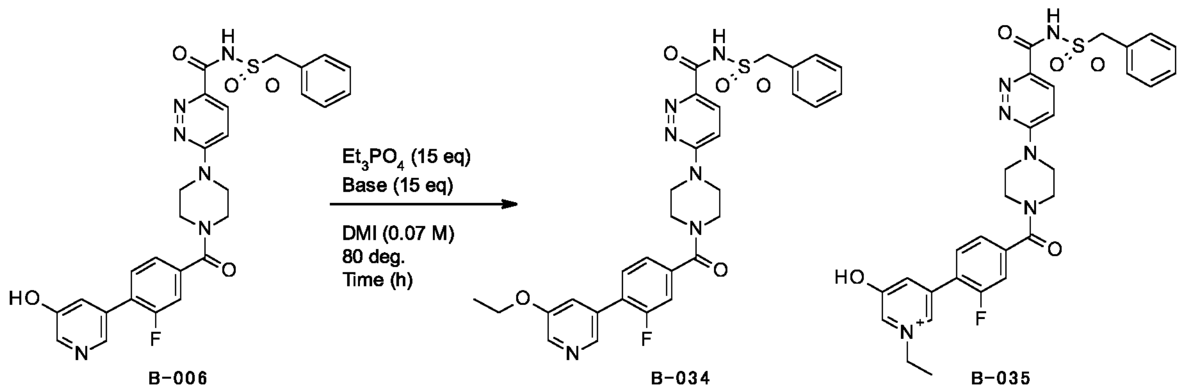


B-006 B-034 B-035

In a glove bag that had been purged with nitrogen, to a 0.6 mL screw cap vial were added N-benzylsulfonyl-6-[4-[3-fluoro-4-(5-hydroxypyridin-3-yl)benzoyl]piperazin-1-yl]pyridazine-3-carboxamide (B-006) (4.0 mg, 7.0 μmol, 1.0 eq) and DMI (101.0 μL). To this solution was added P2Et (34.9 μL, 105.0 μmol, 15.0 eq), and after confirming the mixing, triethyl phosphate (B-R-05) (17.9 μL, 105.0 μmol, 15.0 eq) was added. The obtained reaction mixture was stirred at 80°C at a number of rotations of 800 rpm.

Also, instead of P2Et, the reaction was performed in the same manner, using BTPP (32.1 μL, 105.0 μmol, 15.0 eq, Run 2), P1tBu (26.7 μL, 105.0 μmol, 15.0 eq, Run 3), TBD (1,5,7-triazabicyclo[4.4.0]dec-5-ene) (14.6 mg, 105.0 μmol, 15.0 eq, Run 4), MTBD (7-methyl-1,5,7-triazabicyclo[4.4.0]dec-5-ene) (15.1 μL, 105.0 μmol, 15.0 eq, Run 5),

DBU (1,8-diazabicyclo[5.4.0]undec-7-ene) (15.7 μL, 105.0 μmol, 15.0 eq, Run 6), BTMG (2-tert-butyl-1,1,3,3-tetramethylguanidine) (20.9 μL, 105.0 μmol, 15.0 eq, Run 7), and DIPEA (diisopropylethylamine) (18.3 μL, 105.0 μmol, 15.0 eq, Run 8).

Reaction tracking

During the process, at 1.5 and 21 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-5-3, Run 1.

N-Benzylsulfonyl-6-[4-[4-(5-ethoxypyridin-3-yl)-3-fluorobenzoyl]piperazin-1-yl]pyridazine-3-carboxamide: Compound B-034.

LCMS (ESI, m/z): 605.1 $[M+H]^+$.

Retention time: 0.984 min (analysis condition SMD-FA05-1).

(The retention time of the raw material ArOH is 0.764 min (analysis condition SMD-FA05-1).

N-Benzylsulfonyl-6-[4-[4-(1-ethyl-5-hydroxypyridin-1-ium-3-yl)-3-fluorobenzoyl]piperazin-1-yl]pyridazine-3-carboxamide: Compound B-035.

LCMS (ESI, m/z): 605.1 $[M+H]^+$.

Retention time: 0.707 min (analysis condition SMD-FA05-1).

The results of Example 3-5-3 are shown in Table 3-5-3. Note that the Ratio (O-/N-) shown in Table 3-5-3 was calculated as (area% of O-ethylated product (B-034))/(area% of N-ethylated product (B-035)). Also, the $pKBH^+$ values in $CH_3CN$ shown in Table 3-5-3 were transcribed from the Aldrich web site for phosphazene bases (https://www.sigmaaldrich.com/chemistry/chemical-synthesis/technology-spotlights/phosphazenes.html) (viewed on December 2, 2020) and non patent literature (Chem. Eur. J. 2021, 27, 4216-4229).

[Table 16]

[Table 3-5-3]

| Run | Base / $pKBH^+$ ($CH_3CN$) | Time (h) | SM (B-006) (area%) | O-Et(B-034) (area%) | N-Et(B-035) (area%) | Ratio (O-/N-) | Byproducts (area%) |
|---|---|---|---|---|---|---|---|
| 1 | P2Et (32.9) | 1.5 | 5.9 | 91.1 | 1.1 | 82.8 | 1.9 |
| | | 21 | ND | 90.8 | ND | >100 | 9.4 |
| 2 | BTPP (28.4) | 1.5 | 58.7 | 38.0 | 2.0 | 19.0 | 1.3 |
| | | 21 | ND | 93.5 | 3.4 | 27.5 | 3.1 |
| 3 | P1tBu (26.9) | 1.5<br>21 | 77.0<br>2.9 | 20.8<br>91.3 | 2.0<br>5.3 | 10.4<br>17.2 | 0.2<br>0.5 |
| 4 | TBD (26.0) | 1.5 | 81.2 | 17.3 | 1.3 | 13.3 | 0.2 |
| | | 21 | 8.0 | 87.7 | 3.9 | 22.5 | 0.4 |
| 5 | MTBD (25.5) | 1.5 | 81.3 | 17.0 | 1.5 | 11.3 | 0.2 |
| | | 21 | 4.3 | 89.7 | 5.5 | 16.3 | 0.5 |
| 6 | DBU (24.3) | 1.5<br>21 | 92.2<br>37.4 | 6.1<br>56.3 | 0.7<br>4.0 | 8.7<br>14.0 | 1.0<br>2.3 |
| 7 | BTMG (23.6) | 1.5 | 81.2 | 16.4 | 1.7 | 9.6 | 0.7 |
| | | 21 | 3.6 | 89.7 | 5.6 | 16.1 | 1.1 |
| 8 | DIPEA (18.8) | 1.5 | 99.3 | ND | 0.4 | ND | 0.3 |
| | | 21 | 94.6 | ND | 4.4 | ND | 1.0 |

As shown in Table 3-5-3, it was confirmed that, when using bases with a $pKBH^+$ value of greater than 28 in

$CH_3CN$ (P2Et and BTPP), the reaction rate is observed at which the raw materials disappear in 21 h, and in the case of bases with a $pKBH^+$ value of 23 to 28 in $CH_3CN$ (BTMG, MTBD, TBD, and P1tBu), the reaction rate is decreased compared to BTPP, but O-selective ethylation proceeds. It was also confirmed that the reaction does not proceed in the case of DIPEA with a $pKBH^+$ value of 18.8 in $CH_3CN$. In the reaction using DBU with a $pKBH^+$ value in the range of 23 to 28 in $CH_3CN$, the reaction progress was slower than the reactions of other bases with a $pKBH^+$ value in the range. While not limited to any specific theory, this is a result of the reaction of DBU with triethyl phosphate consuming the ethylating agent, thereby inhibiting the progress of the reaction.

Example 3-6: Confirmation of influence when using large excess of reagent to substrate, assuming compound library that is mixture of multiple substrates

When multiple substrates are mixed, there will be a large excess of reagent per substrate. In the following, the influence of the presence of a large excess of reagent is confirmed using N-benzylsulfonyl-4-[4-[3-fluoro-4-(5-hydroxypyridin-3-yl)benzoyl]piperazin-1-yl]benzamide (B-004) as a substrate.

Example 3-6-1: Investigation on O-selective ethylation using N-benzylsulfonyl-4-[4-[3-fluoro-4-(5-hydroxypyridin-3-yl)benzoyl]piperazin-1-yl]benzamide (B-004) (pyridinol) as substrate under excess reagent conditions

[Formula 71]

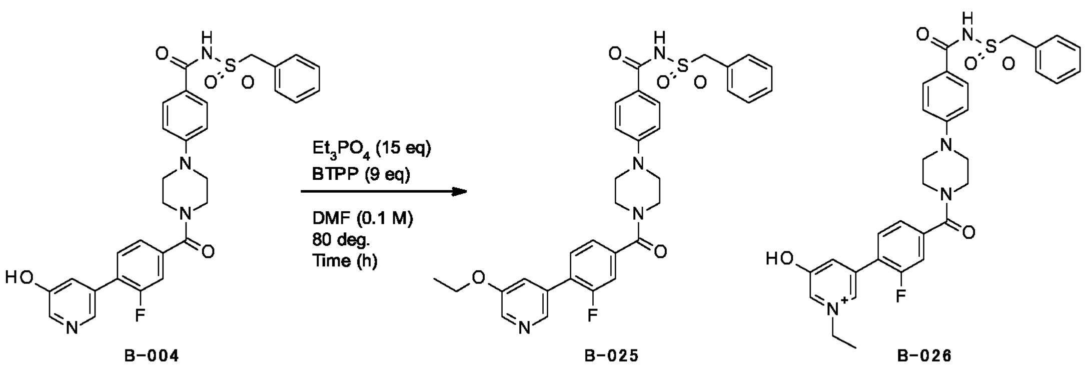


B-004 B-025 B-026

In a glove bag that had been purged with nitrogen, to a 0.6 mL screw cap vial were added N-benzylsulfonyl-4-[4-[3-fluoro-4-(5-hydroxypyridin-3-yl)benzoyl]piperazin-1-yl]benzamide (B-004) (4.0 mg, 7.0 μmol, 1.0 eq) and DMF (70.0 μL). To this solution was added BTPP (19.3 μL, 63.0 μmol, 9.0 eq), and after confirming the mixing, triethyl phosphate (B-R-05) (17.9 μL, 105.0 μmol, 15.0 eq) was added. The obtained reaction mixture was stirred at 80°C at a number of rotations of 800 rpm. The results were as shown in Table 3-6, Run 1.

Reaction tracking

During the process, at 2.5, 7, and 23 hours after the start of the reaction, 5 μL of the reaction solution was sampled in a glove bag and diluted with $CH_3CN$ (1000 μL) to prepare a LC sample. The reaction progress was analyzed by carrying out LC-MS analysis.

Example 3-6-2: Investigation on O-selective ethylation using N-benzylsulfonyl-4-[4-[3-fluoro-4-(5-hydroxypyridin-3-yl)benzoyl]piperazin-1-yl]benzamide (B-004) (pyridinol) as substrate and using DMI as reaction solvent

[Formula 72]

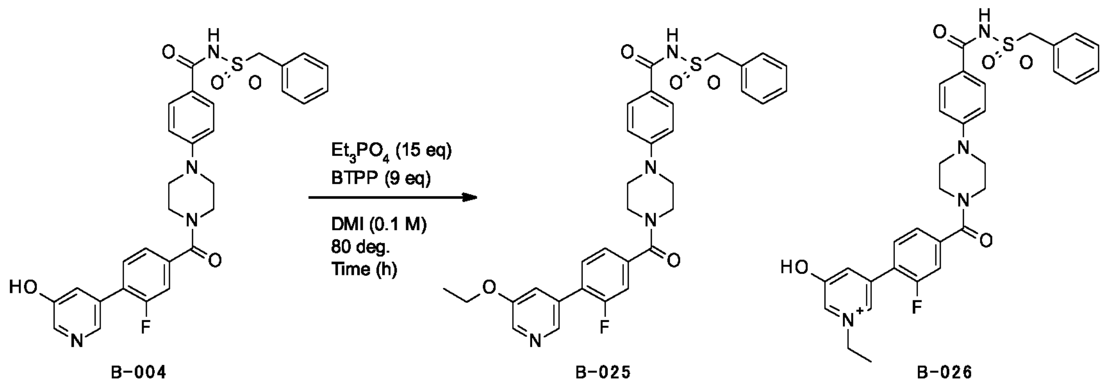


B-004 B-025 B-026

Using DMI (70.0 μL) as the reaction solvent, the reaction was performed by the same method as in Example 3-6-1.

Reaction tracking

During the process, at 2.5, 5, and 23 hours after the start of the reaction, the reaction progress was analyzed by the same method as in Example 3-6-1. The results were as shown in Table 3-6, Run 2.

Example 3-6-3: Investigation on O-selective ethylation using N-benzylsulfonyl-4-[4-[3-fluoro-4-(5-hydroxypyridin-3-yl)benzoyl]piperazin-1-yl]benzamide (B-004) (pyridinol) as substrate and using DMI as reaction solvent under excess reagent conditions

[Formula 73]

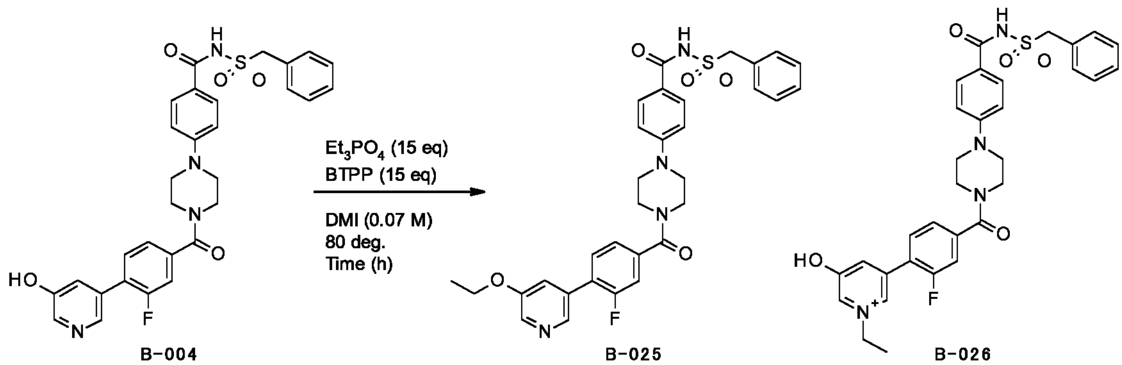


B-004 B-025 B-026

In a glove bag that had been purged with nitrogen, to a 0.6 mL screw cap vial were added N-benzylsulfonyl-4-[4-[3-fluoro-4-(5-hydroxypyridin-3-yl)benzoyl]piperazin-1-yl]benzamide (B-004) (4.0 mg, 7.0 μmol, 1.0 eq) and DMI (101.0 μL). To this solution was added BTPP (32.1 μL, 105.0 μmol, 15.0 eq), and after confirming the mixing, triethyl phosphate (B-R-05) (17.9 μL, 105.0 μmol, 15.0 eq) was added. The obtained reaction mixture was stirred at 80°C at a number of rotations of 800 rpm.

Reaction tracking

During the process, at 2.5, 5, and 23 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-6, Run 3.

The results of Example 3-6 are shown in Table 3-6. Note that the Ratio (O-/N-) shown in Table 3-6 was calculated as (area% of O-ethylated product (B-025))/(area% of N-ethylated product (B-026)).

[Table 17]

[Table 3-6]

| Run | Solvent (M) | $Et_3PO_4$ (eq) | BTPP (eq) | Time (h) | SM (B-004) (area%) | O-Et (B-025) (area%) | N-Et (B-026) (area%) | Ratio (O-/N-) |
|---|---|---|---|---|---|---|---|---|
| 1 | DMF (0.1 M) | 15 | 9 | 2.5 | 26.0 | 89.3 | 4.0 | 17.3 |
| | | | | 7<br>23 | 1.4<br>ND | 93.2<br>95.9 | 4.4<br>2.6 | 21.2<br>36.9 |
| 2 | DMI (0.1 M) | 15 | 9 | 2.5 | 20.6 | 75.2 | 3.3 | 22.8 |
| | | | | 5 | 2.5 | 92.7 | 3.6 | 25.7 |
| | | | | 23 | ND | 96.9 | 1.7 | 57.0 |
| 3 | DMI (0.07 M) | 15 | 15 | 2.5 | 28.6 | 67.8 | 2.7 | 25.1 |
| | | | | 5 | 11.0 | 85.0 | 3.0 | 28.3 |
| | | | | 23 | ND | 97.0 | 1.7 | 57.0 |

From the above results, it was confirmed that even when a large excess of reagent is present for one substrate, there is no reduction in functional group selectivity, and application to a compound library that is a mixture of multiple substrates can be performed without problems (Table 3-6, Runs 1 to 3).

Note that it was confirmed that, even when DMI is used in addition to DMF as a solvent, both the selectivity of the reaction and the conversion rate of the reaction can be performed with no inferiority (Table 3-6, Runs 2 to 3).

<u>Example 3-7: Confirmation of substrate generality under reaction conditions set in Example 3-6-3</u>

The reaction conditions set in Example 3-6-3 were adapted to substrates having various functional groups to confirm the substrate generality.

<u>Example 3-7-1: Confirmation of O-selective ethylation using N-benzylsulfonyl-6-[4-[3-fluoro-4-(5-hydroxypyridin-3-yl)benzoyl]piperazin-1-yl]pyridazine-3-carboxamide (B-006) as substrate having pyridazine</u>

[Formula 74]

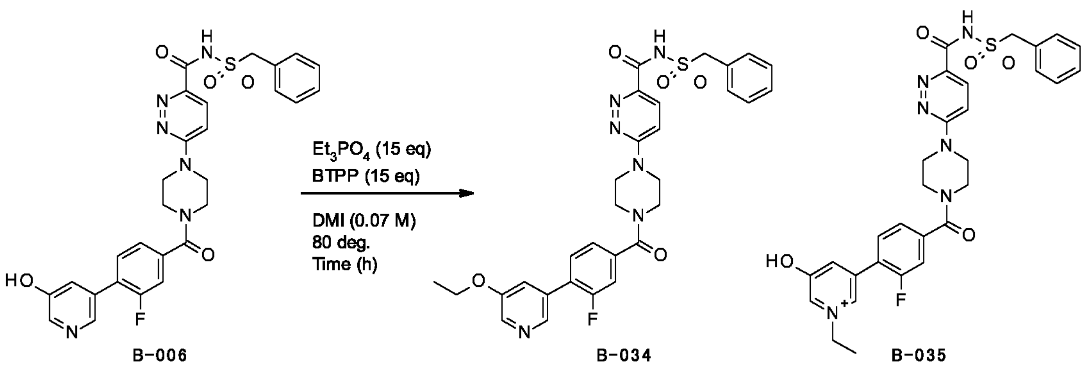


B-006 B-034 B-035

In a glove bag that had been purged with nitrogen, to a 0.6 mL screw cap vial were added N-benzylsulfonyl-6-[4-[3-fluoro-4-(5-hydroxypyridin-3-yl)benzoyl]piperazin-1-yl]pyridazine-3-carboxamide (B-006) (4.0 mg, 7.0 μmol, 1.0

eq) and DMI (101.0 μL). To this solution was added BTPP (32.1 μL, 105.0 μmol, 15.0 eq), and after confirming the mixing, triethyl phosphate (B-R-05) (17.9 μL, 105.0 μmol, 15.0 eq) was added. The obtained reaction mixture was stirred at 80°C at a number of rotations of 800 rpm.

Reaction tracking

During the process, at 2.5, 5, and 21 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-7-1, Run 1.
N-Benzylsulfonyl-6-[4-[4-(5-ethoxypyridin-3-yl)-3-fluorobenzoyl]piperazin-1-yl]pyridazine-3-carboxamide: Compound B-034.
LCMS (ESI, m/z): 605.2 $[M+H]^+$.
Retention time: 0.986 min (analysis condition SMD-FA05-1).
(The retention time of the raw material ArOH is 0.764 min (analysis condition SMD-FA05-1).
N-Benzylsulfonyl-6-[4-[4-(1-ethyl-5-hydroxypyridin-1-ium-3-yl)-3-fluorobenzoyl]piperazin-1-yl]pyridazine-3-carboxamide: Compound B-035.
LCMS (ESI, m/z): 605.1 $[M+H]^+$.
Retention time: 0.707 min (analysis condition SMD-FA05-1).
The results of Example 3-7-1 are shown in Table 3-7-1. Note that the Ratio (O-/N-) shown in Table 3-7-1 was calculated as (area% of O-ethylated product (B-034))/(area% of N-ethylated product (B-035)).

[Table 18]

[Table 3-7-1]

| Run | Time (h) | SM (B-006) (area%) | O-Et (B-034) (area%) | N-Et (B-035) (area%) | Ratio (O-/N-) | Byproducts (area%) (not identified) |
|---|---|---|---|---|---|---|
| 1 | 2.5 | 34.1 | 62.4 | 3.1 | 20.1 | ND |
| | 5 | 9.2 | 86.5 | 3.7 | 23.4 | 0.5 |
| | 21 | ND | 94.8 | 2.7 | 35.1 | 0.7 |

Example 3-7-2: Confirmation of O-selective ethylation using N-benzylsulfonyl-6-[4-[3-fluoro-4-(4-hydroxyphenyl)benzoyl]piperazin-1-yl]pyridazine-3--carboxamide (B-007) as substrate having pyridazine

[Formula 75]

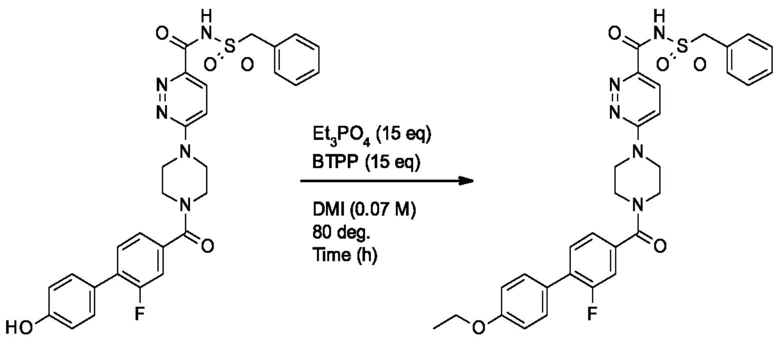


B-007 B-036

Using N-benzylsulfonyl-6-[4-[3-fluoro-4-(4-hydroxyphenyl)benzoyl]piperazin-1 - yl]pyridazine-3-carboxamide (B-007) (4.0 mg, 7.0 μmol, 1.0 eq), the reaction was performed as in Example 3-7-1 and the reaction progress was analyzed. The results were as shown in Table 3-7-2, Run 1.
N-Benzylsulfonyl-6-[4-[4-(4-ethoxyphenyl)-3-fluorobenzoyl]piperazin-1-yl]pyridazine-3-carboxamide: B-036.

LCMS (ESI, m/z): 604.2 [M+H]+.

Retention time: 1.225 min (analysis condition SMD-FA05-1).

(The retention time of the raw material ArOH is 1.000 min (analysis condition SMD-FA05-1).

The results of Example 3-7-2 are shown in Table 3-7-2.

[Table 19]

[Table 3-7-2]

| Run | Time (h) | SM (B-007) (area%) | O-Et (B-036) (area%) | Byproducts (area%) (not identified) |
|---|---|---|---|---|
| 1 | 2.5 | 43.5 | 55.7 | ND |
| | 5 | 19.3 | 79.6 | 0.5 |
| | 21 | ND | 96.9 | 1.8 |

Example 3-7-3: O-Selective ethylation of pyridinol using N-benzylsulfonyl-6-[4-[5-[2-(5-hydroxypyridin-3-yl)ethynyl]pyridine-3-carbonyl]piperazin-1-yl]pyridazine-3-carboxamide (B-005) and N-benzylsulfonyl-4-[4-[5-[2-(5-hydroxypyridin-3-yl)ethynyl]pyridine-3-carbonyl]piperazin-1-yl]benzamide (B-003) as substrates having acetylene functional group

[Formula 76]

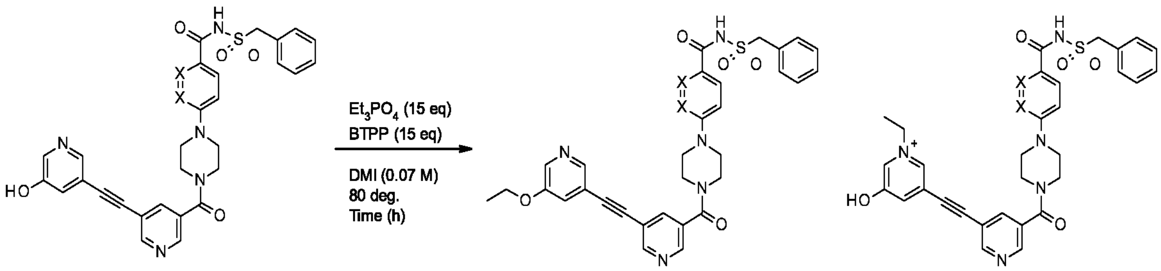


X = N: B-005
X = CH: B-003

X = N: B-027
X = CH: B-029

X = N: B-029
X = CH: B-030

Using N-benzylsulfonyl-6-[4-[5-[2-(5-hydroxypyridin-3-yl)ethynyl]pyridine-3-carbonyl]piperazin-1-yl]pyridazine-3-carboxamide (B-005) (4.1 mg, 7.0 μmol, 1.0 eq, Table 3-7-3, Run 1) or N-benzylsulfonyl-4-[4-[5-[2-(5-hydroxypyridin-3-yl)ethynyl]pyridine-3-carbonyl]piperazin-1-yl]benzamide (B-003) (4.1 mg, 7.0 μmol, 1.0 eq, Table 3-7-3, Run 2), the reaction was performed as in Example 3-7-1 and the reaction progress was analyzed. The results were as shown in Table 3-7-3, Run 1 and Run 2.

The results of Example 3-7-3 are shown in Table 3-7-3. Note that the Ratio (O-/N-) shown in Table 3-7-3 was calculated as (area% of O-ethylated product)/(area% of N-ethylated product).

[Table 20]

[Table 3-7-3]

| Run | Substrate | X | Time (h) | SM (OH) (area%) | O-Et (area%) | N-Et (area%) | Ratio (O-/N-) | Byproducts (area%) (not identified) |
|---|---|---|---|---|---|---|---|---|
| 1 | B-005 | N | 2.5 | 30.0 | 67.0 | 1.5 | 44.7 | ND |
| | | | 5 | 8.2 | 88.5 | 1.5 | 58.9 | ND |
| | | | 21 | ND | 95.9 | ND | >100 | 1.8 |
| 2 | B-003 | CH | 2.5 | 30.0 | 68.2 | 1.2 | 56.8 | 0.4 |

(continued)

| Run | Substrate | X | Time (h) | SM (OH) (area%) | O-Et (area%) | N-Et (area%) | Ratio (O-/N-) | Byproducts (area%) (not identified) |
|---|---|---|---|---|---|---|---|---|
| | | | 5 | 8.7 | 90.0 | 0.8 | 112.5 | 0.5 |
| | | | 21 | ND | 99.2 | ND | >100 | 0.6 |

Example 3-7-4: O-Selective ethylation of phenol using N-benzylsulfonyl-4-[4-[5-[2-(4-hydroxyphenyl)ethynyl]pyridine-3-carbonyl]piperazin-1-yl]benzamide (B-008) as substrate having acetylene functional group

[Formula 77]

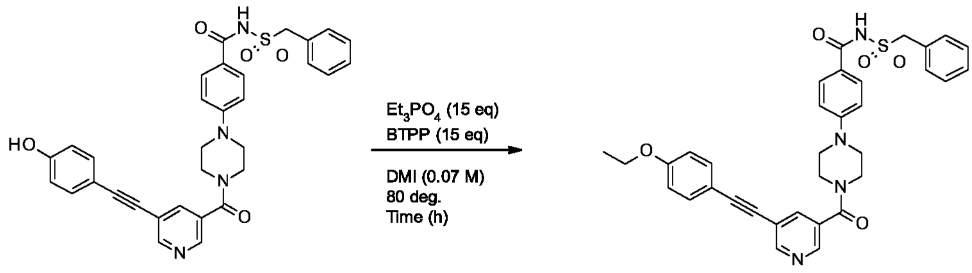


B-008 B-037

Using N-benzylsulfonyl-4-[4-[5-[2-(4-hydroxyphenyl)ethynyl]pyridine-3-carbonyl]piperazin-1-yl]benzamide (B-008) (4.1 mg, 7.0 μmol, 1.0 eq), the reaction was performed as in Example 3-7-1 and the reaction progress was analyzed. The results were as shown in Table 3-7-4, Run 1.

N-Benzylsulfonyl-4-[4-[5-[2-(4-ethoxyphenyl)ethynyl]pyridine-3-carbonyl]piperazin-1-yl]benzamide: Compound B-037.

LCMS (ESI, m/z): 609.2 $[M+H]^+$.

Retention time: 1.229 min (analysis condition SMD-FA05-1).

(The retention time of the raw material ArOH is 1.011 min (analysis condition SMD-FA05-1).

The results of Example 3-7-4 are shown in Table 3-7-4.

[Table 21]

[Table 3-7-4]

| Run | Time (h) | SM (B-008) (area%) | O-Et (B-037) (area%) | Byproducts (area%) (not identified) |
|---|---|---|---|---|
| 1 | 2.5 | 34.2 | 64.4 | 0.7 |
| | 5 | 6.8 | 91.4 | 0.8 |
| | 21 | ND | 98.0 | 0.7 |

Example 3-7-5: O-Selective ethylation of pyridinol using N-benzylsulfonyl-4-[4-[[2-[2-(5-hydroxypyridin-3-yl)ethynyl]phenyl]methyl]piperazin-1-yl]benzamide (B-009) as substrate having acetylene functional group

[Formula 78]

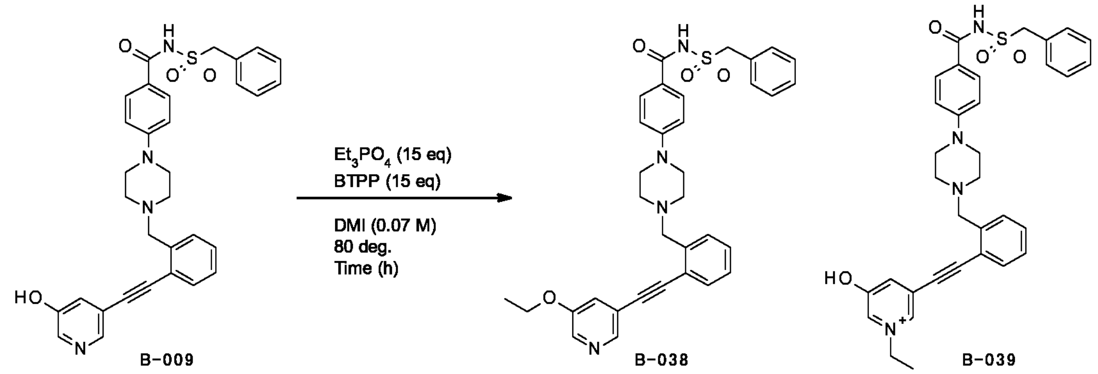


B-009 B-038 B-039

Using N-benzylsulfonyl-4-[4-[[2-[2-(5-hydroxypyridin-3-yl)ethynyl]phenyl]methyl]piperazin-1-yl]benzamide (B-009) (4.0 mg, 7.0 μmol, 1.0 eq), the reaction was performed as in Example 3-7-1 and the reaction progress was analyzed. The results were as shown in Table 3-7-5, Run 1.

N-Benzylsulfonyl-4-[4-[[2-[2-(5-ethoxypyridin-3-yl)ethynyl]phenyl]methyl]piperazin-1-yl]benzamide: Compound B-038.

LCMS (ESI, m/z): 595.2 $[M+H]^+$.

Retention time: 0.879 min (analysis condition SMD-FA05-1).

(The retention time of the raw material ArOH is 0.749 min (analysis condition SMD-FA05-1).

N-Benzylsulfonyl-4-[4-[[2-[2-(5-ethoxypyridin-3-yl)ethynyl]phenyl]methyl]piperazin-1-yl]benzamide: Compound B-039.

LCMS (ESI, m/z): 595.2 $[M+H]^+$.

Retention time: 0.636 min (analysis condition SMD-FA05-1).

The results of Example 3-7-5 are shown in Table 3-7-5. Note that the Ratio (O-/N-) shown in Table 3-7-5 was calculated as (area% of O-ethylated product (B-038))/(area% of N-ethylated product (B-039)).

[Table 22]

[Table 3-7-5]

| Run | Time (h) | SM (B-009) (area%) | O-Et (B-038) (area%) | N-Et (B-039) (area%) | Ratio (O-/N-) | Byproducts (area%) (not identified) |
|---|---|---|---|---|---|---|
| 1 | 2.5 | 21.3 | 76.5 | 1.7 | 45.0 | ND |
| | 5 | 1.5 | 96.8 | 1.0 | 96.8 | ND |
| | 21 | ND | 98.4 | ND | >100 | ND |

<u>Example 3-7-6: O-Selective ethylation of phenol using N-benzylsulfonyl-4-[4-[[2-[2-(4-hydroxyphenyl)ethynyl]phenyl]methyl]piperazin-1-yl]benzamide (B-010) as substrate having acetylene functional group</u>

[Formula 79]

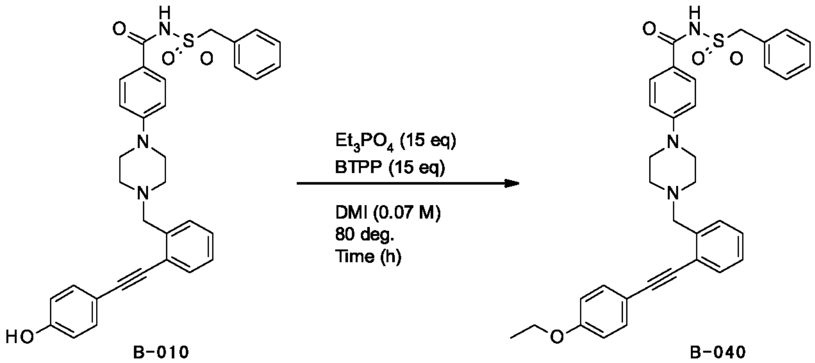


B-010 B-040

Using N-benzylsulfonyl-4-[4-[[2-[2-(4-hydroxyphenyl)ethynyl]phenyl]methyl]piperazin-1-yl]benzamide (B-010) (4.0 mg, 7.0 μmol, 1.0 eq), the reaction was performed as in Example 3-7-1 and the reaction progress was analyzed. The results were as shown in Table 3-7-6, Run 1.

N-Benzylsulfonyl-4-[4-[[2-[2-(4-ethoxyphenyl)ethynyl]phenyl]methyl]piperazin-1-yl]benzamide: Compound B-040.

LCMS (ESI, m/z): 594.2 $[M+H]^+$.

Retention time: 0.976 min (analysis condition SMD-FA05-1).

(The retention time of the raw material ArOH is 0.846 min (analysis condition SMD-FA05-1).

The results of Example 3-7-6 are shown in Table 3-7-6.

[Table 23]

[Table 3-7-6]

| Run | Time (h) | SM (B-010) (area%) | O-Et (8-040) (area%) | Byproducts (area%) (not identified) |
|---|---|---|---|---|
| 1 | 2.5 | 23.6 | 75.8 | ND |
| | 5 | 3.6 | 95.8 | ND |
| | 21 | ND | 99.4 | ND |

Example 3-7-7: O-Selective ethylation of phenol using 4-[4-[[2-(4-hydroxyphenyl)phenyl]methyl]piperazin-1-yl]-N-[3-methyl-4-(2-phenylsulfanylethylamino)phenyl]sulfonylbenzamide(B-011),4-[4-[[2-(4-hydroxyphenyl)phenyl]methyl]piperazin-1-yl]-N-[3-nitro-4-(2-phenylsulfanylethylamino)phenyl]sulfonylbenzamide (B-012), and 4-[4-[[2-(3-hydroxyphenyl)phenyl]methyl]piperazin-1-yl]-N-[3-nitro-4-(2-phenylsulfanylethylamino)phenyl]sulfonylbenzamide (B-013) as substrates having aniline NH group

[Formula 80]

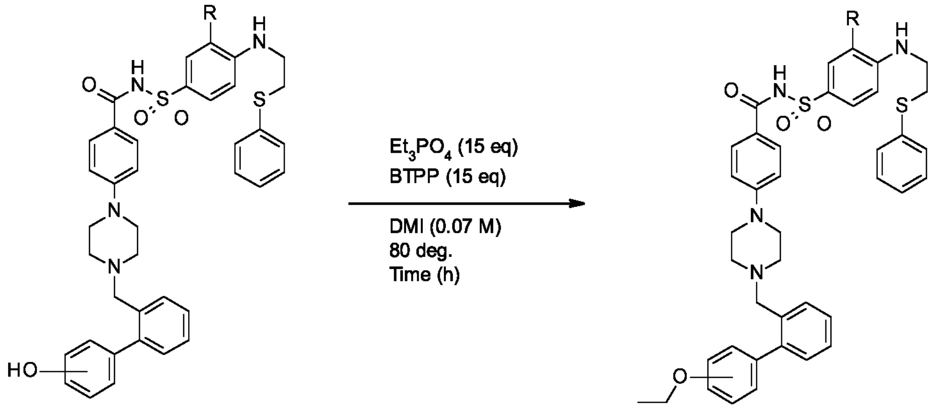


R = Me, p-OH: B-011
R = NO2, p-OH: B-012
R = NO2, m-OH: B-013

R = Me, p-OEt: B-041
R = NO2, p-OEt: B-042
R = NO2, m-OEt: B-043

Using 4-[4-[[2-(4-hydroxyphenyl)phenyl]methyl]piperazin-1-yl]-N-[3-methyl-4-(2-phenylsulfanylethylamino)phenyl]sulfonylbenzamide (B-011) (4.8 mg, 7.0 μmol, 1.0 eq, Table 3-7-7, Run 1), 4-[4-[[2-(4-hydroxyphenyl)phenyl]methyl]piperazin-1-yl]-N-[3-nitro-4-(2-phenylsulfanylethylamino)phenyl]sulfonylbenzamide (B-012) (5.1 mg, 7.0 μmol, 1.0 eq, Table 3-7-7, Run 2), or 4-[4-[[2-(3-hydroxyphenyl)phenyl]methyl]piperazin-1-yl]-N-[3-nitro-4-(2-phenylsulfanylethylamino)phenyl]sulfonylbenzamide (B-013) (5.1 mg, 7.0 μmol, 1.0 eq, Table 3-7-7, Run 3), the reaction was performed as in Example 3-7-1 and the reaction progress was analyzed. The results were as shown in Table 3-7-7, Run 1 to Run 3.

4-[4-[[2-(4-Ethoxyphenyl)phenyl]methyl]piperazin-1-yl]-N-[3-methyl-4-(2-phenylsulfanylethylamino)phenyl]sulfonylbenzamide: Compound B-041.

LCMS (ESI, m/z): 721.2 $[M+H]^+$.

Retention time: 1.068 min (analysis condition SMD-FA05-1).

(The retention time of the raw material ArOH is 0.954 min (analysis condition SMD-FA05-1).

4-[4-[[2-(4-Ethoxyphenyl)phenyl]methyl]piperazin-1-yl]-N-[3-nitro-4-(2-phenylsulfanylethylamino)phenyl]sulfonylbenzamide: Compound B-042.

LCMS (ESI, m/z): 752.2 $[M+H]^+$.

Retention time: 1.070 min (analysis condition SMD-FA05-1).

(The retention time of the raw material ArOH is 0.953 min (analysis condition SMD-FA05-1).

4-[4-[[2-(3-Ethoxyphenyl)phenyl]methyl]piperazin-1-yl]-N-[3-nitro-4-(2-phenylsulfanylethylamino)phenyl]sulfonylbenzamide: B-043.

LCMS (ESI, m/z): 752.2 $[M+H]^+$.

Retention time: 1.077 min (analysis condition SMD-FA05-1).

(The retention time of the raw material ArOH is 0.960 min (analysis condition SMD-FA05-1).

B-043 produced by the reaction illustrated in Example 3-7-7 may also be denoted as compound B2Q045-01 or AG005 in the present specification.

The results of Example 3-7-7 are shown in Table 3-7-7.

[Table 24]

[Table 3-7-7]

| Run | Substrate | R | OH (position) | Time (h) | SM (OH) (area%) | O-Et (area%) | Di-Et (area%) | Byproducts (area%) (not identified) |
|---|---|---|---|---|---|---|---|---|
| 1 | B-011 | Me | *p-* | 2.5 | 53.8 | 42.8 | 0.4 | ND |
| | | | | 5<br>23 | 34.6<br>1.8 | 62.3<br>94.3 | 0.3<br>0.4 | ND<br>ND |
| 2 | B-012 | NO2 | *p-* | 2.5 | 76.3 | 23.4 | ND | ND |

(continued)

| Run | Substrate | R | OH (position) | Time (h) | SM (OH) (area%) | O-Et (area%) | Di-Et (area%) | Byproducts (area%) (not identified) |
|---|---|---|---|---|---|---|---|---|
| | | | | 5 | 43.5 | 55.0 | ND | ND |
| | | | | 30 | 1.0 | 97.9 | ND | ND |
| 3 | B-013 | NO2 | *m-* | 2.5 | 76.8 | 22.1 | ND | ND |
| | | | | 5 | 47.5 | 51.2 | ND | ND |
| | | | | 30 | 1.4 | 96.8 | ND | ND |

As shown in Tables 3-7-1 to 3-7-7 above, it was confirmed that the reaction conditions set in Example 3-6-3 can carry out O-selective ethylation to arenol having various functional groups. Specifically, O-selective ethylation conditions for arenol in the presence of functional groups such as a tertiary amine, an acylsulfonamide, an aniline, an alkyne, and a pyridine ring were found. Note that the present approach of the present invention is a widely applicable approach, not limited to the functional groups exemplified in the present Example.

Example 3-7-8: Effect of addition of ethanol in O-selective ethylation of phenol using 6-[4-[[2-(3-hydroxyphenyl)phenyl]methyllpiperazin-1-yl]-N-(4-methoxyphenyl)pyridazine-3-carboxamide (B-080) as substrate having pyridazine amide NH group

[Formula 81]

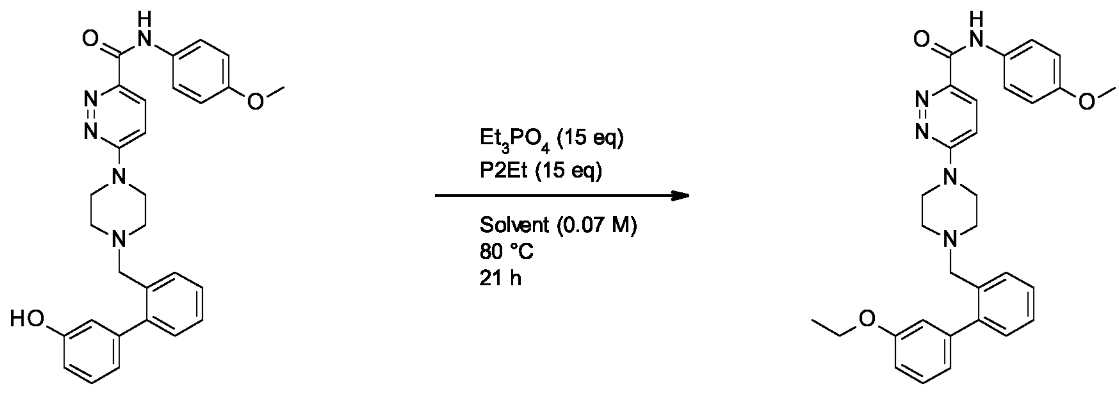


B-080 B-081

Run 1: DMF solvent reaction

In a glove bag that had been purged with nitrogen, to a 0.6 mL screw cap vial were added 6-[4-[[2-(3-hydroxyphenyl)phenyl]methyl]piperazin-1-yl]-N-(4-methoxyphenyl)pyridazine-3-carboxamide (B-080) (3.5 mg, 7.0 μmol, 1.0 eq) and DMF (100.0 μL). To this solution was added BTPP (32.1 μL, 105.0 μmol, 15.0 eq), and after confirming the mixing, triethyl phosphate (B-R-05) (17.9 μL, 105.0 μmol, 15.0 eq) was added. The obtained reaction mixture was stirred at 80°C at a number of rotations of 800 rpm.

Reaction tracking

During the process, at 2 and 21 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-7-8, Run 1.

6-[4-[[2-(3-Ethoxyphenyl)phenyl]methyl]piperazin-1-yl]-N-(4-methoxyphenyl)pyridazine-3-carboxamide: Compound B-081.

LCMS (ESI, m/z): 524.2 $[M+H]^+$.

Retention time: 0.901 min (analysis condition SMD-FA05-1).

(The retention time of the raw material ArOH is 0.751 min (analysis condition SMD-FA05-1).

Run 2: DMF-EtOH mixed solvent reaction

In a glove bag that had been purged with nitrogen, to a 0.6 mL screw cap vial were added 6-[4-[[2-(3-hydroxyphenyl)phenyl]methyl]piperazin-1-yl]-N-(4-methoxyphenyl)pyridazine-3-carboxamide (B-080) (3.5 mg, 7.0 μmol, 1.0 eq), DMF

(84.0 μL), and EtOH (17.0 μL) (DMF:EtOH = 5:1). The reaction was performed as in Example 3-7-8, Run 1, and the reaction progress was analyzed. The results were as shown in Table 3-7-8, Run 1 to Run 2.

The results of Example 3-7-8 are shown in Table 3-7-8.

[Table 25]

[Table 3-7-8]

| Run | Solvent | Time (h) | SM (B-080) (area%) | O-Et (B-081) (area%) | Byproducts (area%) (not identified) |
|---|---|---|---|---|---|
| 1 | DMF | 2 | 45.6 | 52.6 | 1.8 |
| | | 21 | ND | 97.8 | 2.2 |
| 2 | DMF-EtOH | 2 | 26.1 | 71.8 | 2.1 |
| | 5:1 | 21 | ND | 97.6 | 2.4 |

As shown in Table 3-7-8, it was confirmed that O-selective ethylation of arenol can be performed even when EtOH is added as a solvent.

Example 3-8: Confirmation of further functional group selectivity under reaction conditions set in Example 3-6-3

In Example 3-7, it was confirmed that the reaction conditions set in Example 3-6-3 allow for selective O-ethylation of arenol even when many functional groups coexist. Under the present reaction conditions, confirmation of further functional group selectivity was carried out. The method of confirmation was to add a compound having a functional group to the ethylation reaction of a phenol model substrate (4-hydroxy-4'-methoxybiphenyl (B-R-21) (pKa[a] = 9.94)) and to investigate whether the ethylation of the compound having a functional group proceeds. In other words, if the hydroxy group of 4-hydroxy-4'-methoxybiphenyl (B-R-21) is selectively ethylated compared to the added compound having a functional group, then the functional group selectivity will be confirmed.

[a]: calculated by ADMET predictor (version 9.5)

Example 3-8-1: Confirmation of ethylation reaction in the presence of amide with acidity comparable to phenol

One example of a functional group that exhibits an acidity comparable to that of phenol is the NH group of a secondary amide functional group. Under the reaction conditions set in Example 3-6-3, the possibility of ethylation of the NH group of a secondary amide functional group was investigated.

[Formula 82]

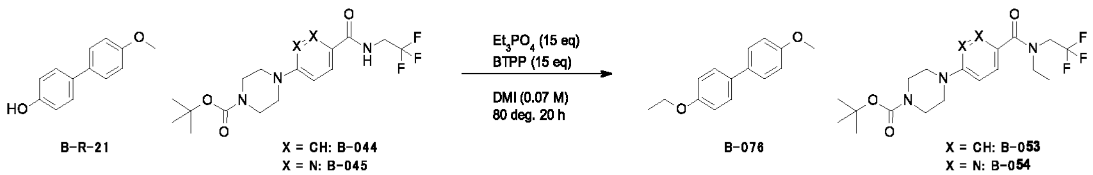

B-R-21
X = CH: B-044
X = N: B-045
B-076
X = CH: B-053
X = N: B-054

Table 3-8-1, Run 1: In a glove bag that had been purged with nitrogen, to a 1.5 mL screw cap vial were added 4-hydroxy-4'-methoxybiphenyl (B-R-21) (7.0 mg, 35.0 μmol, 1.0 eq), a compound having an amide group, tert-butyl 4-[4-(2,2,2-trifluoroethylcarbamoyl)phenyl]piperazine-1-carboxylate (B-044) (6.8 mg, 18.0 μmol, 0.5 eq), and DMI (0.5 mL). To this solution was added BTPP (161.0 μL, 525.0 μmol, 15.0 eq), and after confirming the mixing, triethyl phosphate

(B-R-05) (89.0 μL, 525.0 μmol, 15.0 eq) was added. The obtained reaction mixture was stirred at 80°C at a number of rotations of 800 rpm.

Reaction tracking

At 20 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-8-1, Run 1.

1-Ethoxy-4-(4-methoxyphenyl)benzene: Compound B-076.

Retention time: 1.340 min (analysis condition SMD-FA05-1).

(The retention time of the raw material ArOH is 0.993 min (analysis condition SMD-FA05-1).

In Table 3-8-1, Run 2, using tert-butyl 4-[6-(2,2,2-trifluoroethylcarbamoyl)pyridazin-3-yl]piperazine-1-carboxylate (B-045) (6.8 mg, 18.0 μmol, 0.5 eq) instead of tert-butyl 4-[4-(2,2,2-trifluoroethylcarbamoyl)phenyl]piperazine-1-carboxylate (B-044), the reaction was performed as in Table 3-8-1, Run 1 and the reaction progress was analyzed. The results were as shown in Table 3-8-1, Run 2.

The results of Example 3-8-1 are shown in Table 3-8-1. Note that the conversion rate (%) (B-R-21B-076) shown in Table 3-8-1 was calculated as (B-R-21, area%)/[(B-R-21, area%) + (B-076, area%)] × 100. Also, the conversion rate (%) (additive) was calculated as (ethylated product of additive, area%)/[(ethylated product of additive, area%) + (additive, area%)] × 100. The sample when the additive was ethylated was synthesized as shown in Example 1-3 and confirmed with reference to the retention time and detected MS in Table 1-3.

[Table 26]

[Table 3-8-1]

| Run | Additive | | pKa[a] | Conversion rate (%) B-R-21 / B-076 | Conversion rate (%) (Additive) |
|---|---|---|---|---|---|
| 1 | B-044 | 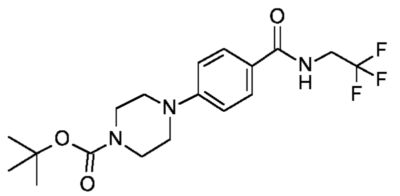 | 11.03 | 96.1 | 0 |
| 2 | B-045 | 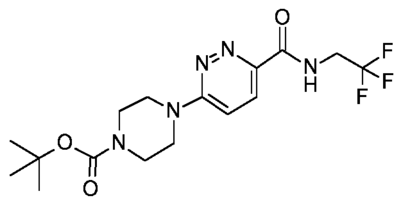 | 10.92 | 99.4 | 0 |
| [a]: calculated by ADMET predictor (version 9.5) | | | | | |

As shown in Table 3-8-1, ethylation of the phenol model substrate (B-R-21) proceeded under the present conditions, but the reaction did not proceed for any of the added amide entities (B-044 and B-045). From this, it was confirmed that, in one aspect, the application of the present conditions allows for O-selective alkylation of arenol relative to an amide functional group having an acidity comparable to that of phenol.

Example 3-8-2: Confirmation of ethylation reaction in the presence of diarylamine

The possibility of N-ethylation of a diarylamine in the case where the NH group of a secondary diarylamine is present in the reaction was investigated. In the following, the method of identifying the applicable range of a diarylamine functional group is illustrated as an example.

[Formula 83]

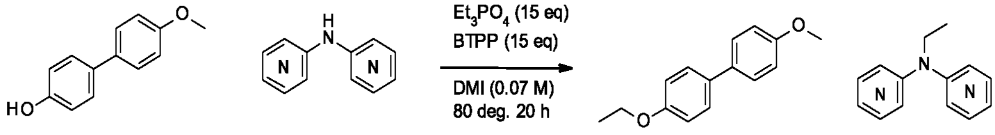


B-R-21 B-R-08 ~ B-R-10 B-076 B-055 ~ 062

In a glove bag that had been purged with nitrogen, to a 1.5 mL screw cap vial were added 4-hydroxy-4'-methoxybiphenyl (B-R-21) (7.0 mg, 35.0 μmol, 1.0 eq), diphenylamine (B-R-08) (5.9 mg, 35.0 μmol, 1.0 eq, Run 1), and DMI (0.5 mL). To this solution was added BTPP (161.0 μL, 525.0 μmol, 15.0 eq), and after confirming the mixing, triethyl phosphate (B-R-05) (89.0 μL, 525.0 μmol, 15.0 eq) was added. The obtained reaction mixture was stirred at 80°C at a number of rotations of 800 rpm.

Also, instead of diphenylamine (B-R-08), the reaction was performed in the same manner, using 2-fluoro-N-(2-fluorophenyl)aniline (B-46) (7.2 mg, 35.0 μmol, 1.0 eq, Run 2), N-phenylpyridin-3-amine (B-47) (6.0 mg, 35.0 μmol, 1.0 eq, Run 3), 4-anilinopyridine (B-R-09) (6.0 mg, 35.0 μmol, 1.0 eq, Run 4), N-phenylpyrimidin-5-amine (B-048) (6.0 mg, 35.0 μmol, 1.0 eq, Run 5), N-pyridin-3-ylpyridin-3-amine (B-049) (6.0 mg, 35.0 μmol, 1.0 eq, Run 6), N-phenylpyridin-2-amine (B-050) (6.0 mg, 35.0 μmol, 1.0 eq, Run 7), and 2,2'-dipyridylamine (B-R-10) (6.0 mg, 35.0 μmol, 1.0 eq, Run 8).

Reaction tracking

At 20 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-8-2.

The results of Example 3-8-2 are shown in Table 3-8-2. Note that the conversion rate (%) (B-R-21B-076) shown in Table 3-8-2 was calculated as (B-R-21, area%)/[(B-R-21, area%) + (B-076, area%)] × 100. Also, the conversion rate (%) (additive) was calculated as (ethylated product of additive, area%)/[(ethylated product of additive, area%) + (additive, area%)] × 100. The sample when the additive was ethylated was synthesized as shown in Example 1-3 and confirmed with reference to the retention time and detected MS in Table 1-3.

[Table 27]

[Table 3-8-2]

| Run | Additive | | pKa[a] | Conversion rate (%) B-R-21 / B-076 | Conversion rate (%) (Additive) |
|---|---|---|---|---|---|
| 1 | B-R-08 | 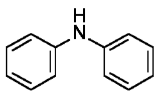 | 2.58 | 99.4 | < 0.1 |
| 2 | B-046 | 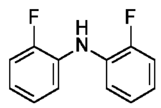 | 1.22 | 99.1 | < 0.1 |
| 3 | B-047 | 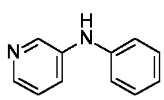 | 0.52 | 99.6 | < 0.1 |
| 4 | B-R-09 | 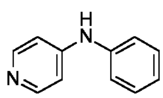 | 0.07 | 99.2 | 5.1 |
| 5 | B-048 | 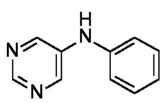 | -0.25 | 99.6 | 0.7 |

(continued)

| Run | Additive | | pKa[a] | Conversion rate (%) B-R-21 / B-076 | Conversion rate (%) (Additive) |
|---|---|---|---|---|---|
| 6 | B-049 | 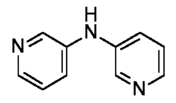 | -1.04 | 99.7 | 1.1 |
| 7 | B-050 | 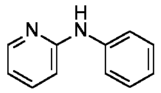 | -1.99 | 99.7 | < 0.1 |
| 8 | B-R-10 | 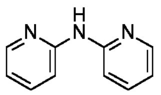 | -4.7 | 99.8 | < 0.1 |
| [a]: calculated by ADMET predictor (version 9.5) | | | | | |

As shown in Table 3-8-2, it was confirmed that ethylation of 4-hydroxy-4'-methoxybiphenyl (B-R-21) proceeded under the present conditions, but the reaction conversion rate of the added diarylamine entities was 5% or less. From these results, it is shown that the present approach of the present invention allows for O-selective alkylation of arenol even when a diarylamine entity is present in the reaction system, not limited to the diarylamines exemplified in the present Example.

Example 3-8-3: Confirmation of ethylation reaction in the presence of various amines

The possibility of N-ethylation of amines in the case where various amines are present in the reaction was investigated. In the following, the method of identifying the applicable range of various amine functional groups is illustrated as an example.

[Formula 84]

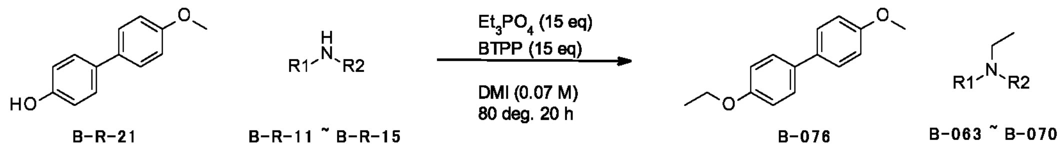


B-R-21 B-R-11 ~ B-R-15 B-076 B-063 ~ B-070

In a glove bag that had been purged with nitrogen, to a 1.5 mL screw cap vial were added 4-hydroxy-4'-methoxybiphenyl (B-R-21) (7.0 mg, 35.0 μmol, 1.0 eq), N-butylaniline (B-R-11) (11.0 μL, 70.0 μmol, 2.0 eq), and DMI (0.5 mL). To this solution was added BTPP (161.0 μL, 525.0 μmol, 15.0 eq), and after confirming the mixing, triethyl phosphate (B-R-05) (89.0 μL, 525.0 μmol, 15.0 eq) was added. The obtained reaction mixture was stirred at 80°C at a number of rotations of 800 rpm.

Also, instead of N-butylaniline (B-R-11), the reaction was performed in the same manner, using 5-bromoindoline (B-R-12) (10.4 mg, 53.0 μmol, 1.5 eq, Run 2), (3-phenylpropyl)methylamine (B-R-13) (14.0 μL, 88.0 μmol, 2.5 eq, Run 3), prop-2-enyl 4-piperazin-1-ylbenzoate (BB-27) (8.6 mg, 35.0 μmol, 1.0 eq, Run 4), 5-(4-methylphenyl)pyridin-2-amine (B-051) (6.5 mg, 35.0 μmol, 1.0 eq, Run 5), 6-(4-methylphenyl)pyridin-3-amine (B-052) (6.5 mg, 35.0 μmol, 1.0 eq, Run 6), 3-phenyl-1-propylamine (B-R-14) (12.0 μL, 88.0 μmol, 2.5 eq, Run 7), and N,N-dimethylbenzylamine (B-R-15) (13.0 μL, 88.0 μmol, 2.5 eq, Run 8).

Reaction tracking

At 20 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. The results were as shown in Table 3-8-3.

The results of Example 3-8-3 are shown in Table 3-8-3. Note that the conversion rate (%) (B-R-21B-076) shown in Table 3-8-3 was calculated as (B-R-21, area%)/[(B-R-21, area%) + (B-076, area%)] × 100. Also, the conversion rate (%) (additive) was calculated as (ethylated product of additive, area%)/[(ethylated product of additive, area%) + (additive,

area%)] × 100. The sample when the additive was ethylated was synthesized as shown in Example 1-3 and confirmed with reference to the retention time and detected MS in Table 1-3.

[Table 28]

[Table 3-8-3]

| Run | Additive | | | Additive (equivalent) | Conversion rate (%) B-R-21 / B-076 | Conversion rate (%) (Additive) |
|---|---|---|---|---|---|---|
| 1 | sec-acyclic ArNH | B-R-11 | 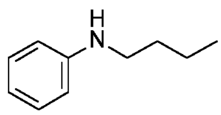 | 2.0 | 99.6 | < 0.1 |
| 2 | see-cyclic ArNH | B-R-12 | 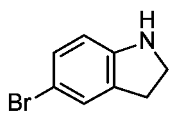 | 1.5 | 99.6 | 2.3 |
| 3 | sec-acyclic amine | B-R-13 | 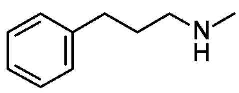 | 2.5 | 99.0 | 13.9 |
| 4 | see-cyclic amine | BB27 | 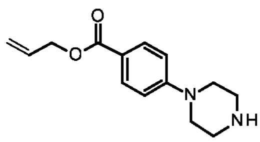 | 1.0 | 99.6 | 32.6 |
| 5 | pri-ArNH2 | B-051 | 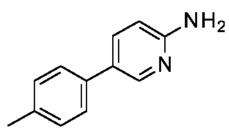 | 1.0 | 99.6 | < 0.1 |
| 6 | pri-ArNH2 | B-052 | 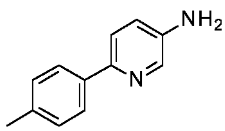 | 1.0 | 99.5 | 0.15 |
| 7 | pri-amine | B-R-14 | 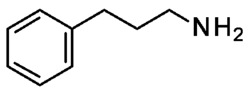 | 2.5 | 98.7 | 11.4 |
| 8 | tert-amine | B-R-15 | 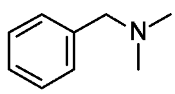 | 2.5 | 98.2 | < 0.1 |

While not limited to any specific theory, in the case where the amine functional group themselves are highly reactive (for example, a secondary acyclic amine, a secondary cyclic amine, and a primary amine), alkylation of the amine functional groups was observed under the present conditions (Table 3-8-3, Run 3, Run 4, and Run 7). It is shown that these amine functional groups need to be protected if alkylation of arenol is to be done.

In addition, the alkylation progress of amine functional groups other than the above (a secondary aniline, a primary aniline, and a tertiary amine) was 5% or less under the present conditions, and it was thus confirmed that selective alkylation of arenol is possible even when an amine functional group is present, not limited to the amine functional groups exemplified in the present Example.

Example 3-8-4: Confirmation of ethylation reaction in the presence of various alcohols

The possibility of O-ethylation of alcohols in the case where various alcohols are present in the reaction was investigated. In the following, the method of identifying the applicable range of various alcohol functional groups is illustrated as an example.

[Formula 85]

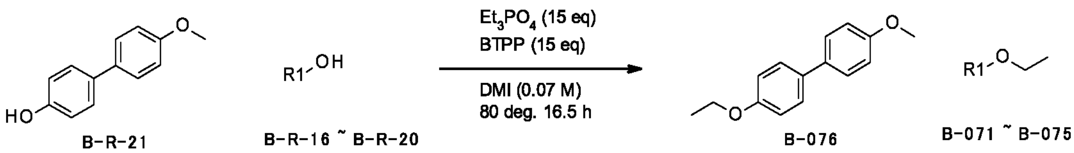


B-R-21 B-R-16 ~ B-R-20 B-076 B-071 ~ B-075

In a glove bag that had been purged with nitrogen, to a 1.5 mL screw cap vial were added 4-hydroxy-4'-methoxybiphenyl (B-021) (7.0 mg, 35.0 μmol, 1.0 eq), benzyl alcohol (B-R-16) (3.6 μL, 35.0 μmol, 1.0 eq), and DMI (0.5 mL). To this solution was added BTPP (161.0 μL, 525.0 μmol, 15.0 eq), and after confirming the mixing, triethyl phosphate (B-R-05) (89.0 μL, 525.0 μmol, 15.0 eq) was added. The obtained reaction mixture was stirred at 80°C for 16.5 hours at a number of rotations of 800 rpm.

Also, instead of benzyl alcohol (B-R-16), the reaction was performed in the same manner, using 1-phenylethanol (B-R-17) (4.2 μL, 35.0 μmol, 1.0 eq, Run 2), 2-phenylethanol (B-R-18) (4.2 μL, 35.0 μmol, 1.0 eq, Run 3), 1-phenyl-2-propanol (B-R-19) (4.9 μL, 35.0 μmol, 1.0 eq, Run 4), and 3-phenyl-1-propanol (B-R-20) (4.8 μL, 35.0 μmol, 1.0 eq, Run 5).

Reaction tracking

At 16.5 hours after the start of the reaction, 5 μL of the reaction solution was sampled in a glove bag and diluted with $CH_3CN$ (1000 μL) to prepare a LC sample. The reaction progress was analyzed by carrying out LC-MS analysis.

The results of Example 3-8-4 are shown in Table 3-8-4. Note that the conversion rate (%) (B-R-21B-076) shown in Table 3-8-4 was calculated as (B-R-21, area%)/[(B-R-21, area%) + (B-076, area%)] × 100. Also, the conversion rate (%) (additive) was calculated as (ethylated product of additive, area%)/[(ethylated product of additive, area%) + (additive, area%)] × 100. The sample when the additive was ethylated was synthesized as shown in Example 1-3 and confirmed with reference to the retention time in Table 1-3.

[Table 29]

[Table 3-8-4]

| Run | Additive | | Conversion rate (%) B-R-21 / B-076 | Conversion rate (%) (Additive) |
|---|---|---|---|---|
| 1 | B-R-16 | 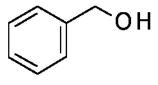 | 99.7 | 3.4 |
| 2 | B-R-17 | 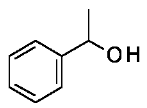 | 99.2 | < 0.1 |
| 3 | B-R-18 | 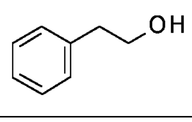 | 97.9 | < 0.1 |
| 4 | B-R-19 | 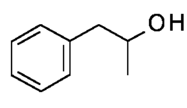 | 98.3 | < 0.1 |

(continued)

| Run | Additive | | Conversion rate (%) B-R-21 / B-076 | Conversion rate (%) (Additive) |
|---|---|---|---|---|
| 5 | B-R-20 | 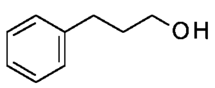 | 98.5 | < 0.1 |

As shown in Table 3-8-4, the alkylation progress of alcohol functional groups was 5% or less under the present conditions, and it was thus confirmed that selective alkylation of arenol is possible even when an alcohol functional group is present, not limited to the alcohol functional groups exemplified in the present Example.

As described above, from the results of Example 3-8, it was confirmed that, without being limited to the functional groups, such as an amide, a diarylamine, a secondary aniline, a primary aniline, a tertiary amine, and an alcohol, exemplified in the present Example, the O-ethylation of arenol proceeds in a functional group-selective manner even when these functional groups coexist.

Example 3-9: O-Selective ethylation of arenol in mixture of three substrates

Using the finding in Example 3, O-selective ethylation of arenol was actually attempted on a mixture of substrates, N-benzylsulfonyl-4-[4-[3-fluoro-4-(5-hydroxypyridin-3-yl)benzoyl]piperazin-1-yl]benzamide (B-004), N-benzylsulfonyl-6-[4-[3-fluoro-4-(5-hydroxypyridin-3-yl)benzoyl]piperazin-1-yl]pyridazine-3-carboxamide (B-006), and N-benzylsulfonyl-6-[4-[3-fluoro-4-(4-hydroxyphenyl)benzoyl]piperazin-1-yl]pyridazine-3-carboxamide (B-007).

[Formula 86]

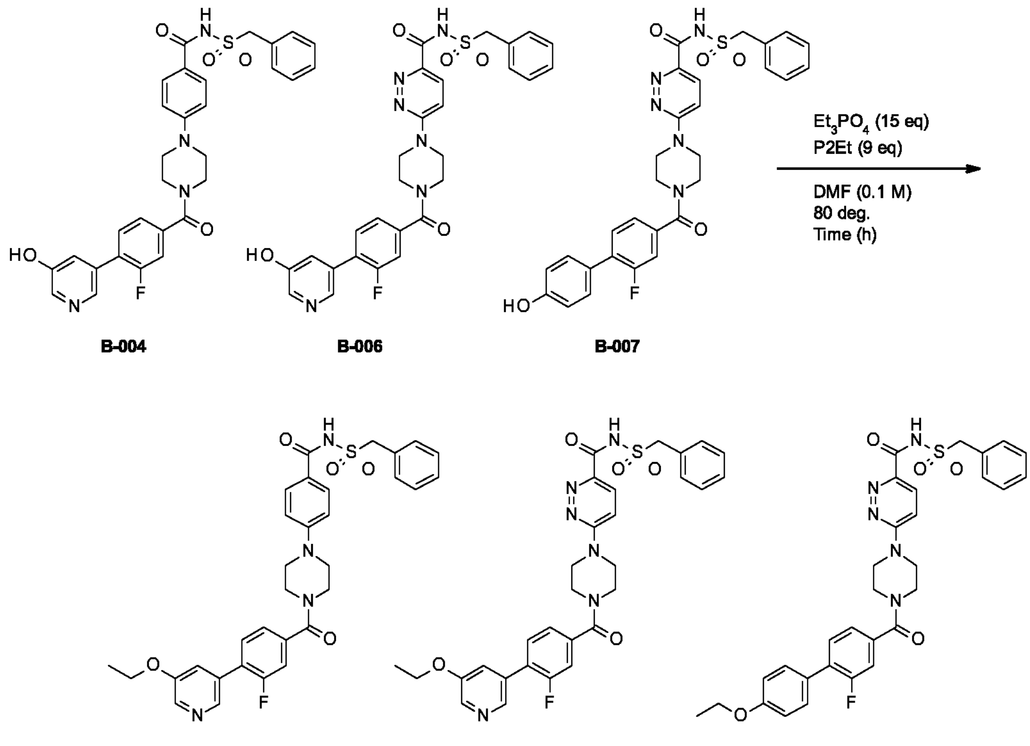


B-004 B-006 B-007

B-025 B-034 B-036

In a glove bag that had been purged with nitrogen, to a 1.5 mL screw cap vial were added (N-benzylsulfonyl-4-[4-[3-fluoro-4-(5-hydroxypyridin-3-yl)benzoyl]piperazin-1-yl]benzamide (B-004) (4.0 mg, 7.0 μmol, 1.0 eq), N-benzylsulfonyl-6-[4-[3-fluoro-4-(5-hydroxypyridin-3-yl)benzoyl]piperazin-1-yl]pyridazine-3-carboxamide (B-006) (4.0 mg, 7.0 μmol, 1.0 eq), N-benzylsulfonyl-6-[4-[3-fluoro-4-(4-hydroxyphenyl)benzoyl]piperazin-1-yl]pyridazine-3-carboxamide (B-007) (4.0 mg, 7.0 μmol, 1.0 eq), and DMF (210.0 μL). To this solution was added P2Et (21.0 μL, 63.0 μmol, 9.0 eq), and after confirming the mixing, triethyl phosphate (B-R-05) (17.9 μL, 105.0 μmol, 15.0 eq) was added. The obtained reaction mixture was stirred at 80°C at a number of rotations of 800 rpm.

Reaction tracking

During the process, at 1, 2.5, 5, and 21 hours after the start of the reaction, 5 μL of the reaction solution was sampled in a glove bag and diluted with $CH_3CN$ (1000 μL) to prepare a LC sample. The reaction progress was analyzed by carrying out LC-MS analysis.

The results of Example 3-9 are shown in Table 3-9. Note that the Ratio (O-/N-) shown in Table 3-9 was calculated as (area% of O-ethylated product)/(area% of N-ethylated product).

[Table 30]

[Table 3-9]

| Time (h) | B-004 (area%) | O-Et (B-025) (area%) | N-Et (B-026) (area%) | B-006 (area%) | OEt (B-034) (area%) | N-Et (B-035) (area%) | B-007 (area%) | O-Et (B-036) (area%) |
|---|---|---|---|---|---|---|---|---|
| 0 | 33.1 | ND | ND | 34.0 | ND | ND | 32.8 | ND |
| 1 | 16.3 | 15.5 | 0.3 | 16.9 | 16.8 | 0.6 | 8.6 | 25.0 |
| 2.5 | 6.9 | 24.5 | 0.4 | 6.6 | 25.9 | 0.7 | 2.4 | 32.5 |
| 5 | 2.1 | 29.4 | 0.3 | 1.2 | 32.3 | 0.4 | ND | 34.1 |
| 21 | ND | 31.5 | 0.2 | ND | 33.5 | 0.2 | ND | 34.1 |
| | | Ratio (O- / N-): 131 | | | Ratio (O- / N-): 139 | | | |

From the present results, it was shown that O-selective ethylation of arenol is possible in the coexistence of many functional groups, even when the substrate is actually a mixture, as is assumed from the investigation results when using a single substrate. Under the present reaction conditions, no problems arising from the mixing of multiple substrates are observed.

Example 4: Investigation on removal of residual reagent and reagent-derived impurity when carrying out ethylation to mixture of multiple substrates

When carrying out a reaction to a mixture of multiple substrates, it is necessary to appropriately remove the residual reagent and reagent-derived impurity in addition to setting the functional group-selective reaction conditions. For example, in one aspect, purification by normal phase and reversed phase columns, which is commonly utilized in laboratories, may not always be applicable as the optimal separation method. In the following, investigation on removal of unwanted reagent and the like was carried out.

Example 4-1: Investigation on removal of unwanted reagent using distillation under reduced pressure and solid phase reagent when carrying out ethylation on mixture of multiple substrates

After carrying out the ethylation reaction using a mixture of the multiple substrates shown in Table 4-1, N-benzylsulfonyl-4-[4-[5-[2-(5-hydroxypyridin-3-yl)ethynyl]pyridine-3-carbonyl]piperazin-1-yl]benzamide (B-003), 4-[4-[[2-(3-hydroxyphenyl)phenyl]methyl]piperazin-1-yl]-N-[3-nitro-4-(2-phenylsulfanylethylamino)phenyl]sulfonylbenzamide (B-013), N-(benzenesulfonyl)-4-[4-[[2-(5-hydroxypyridin-3-yl)phenyl]methyl]piperazin-1-yl]benzamide (B-014), and N-benzylsulfonyl-4-[4-[3-fluoro-4-(4-hydroxyphenyl)benzoyl]piperazin-1-yl]benzamide (B-015), investigation on the method of removing the reagent was performed. For the substrates used, compounds exhibiting a wide range of acidity (cpKa) and acidity of conjugate acid ($cpKBH^+$) were selected from among the compounds included in the library described in Example 6. Note that, for the cpKa and $cpKBH^+$, the calculated value of pKa and the calculated value of $pKBH^+$ were shown, and the values calculated using ADMET predictor (version 9.5) were utilized. Also, when performing ethylation, assuming utilization in library synthesis, ethylation was performed with the coexistence of an acid component that may be mixed into the substrates used for ethylation (here, as an example, assuming that the arenol compounds are supported on Wang resin, TFA, which may be commonly used in the cleavage step from the solid phase). One example of operations is shown below.

[Table 31]

[Table 4-1]

| Compound No. | B-003 | 8-013 |
|---|---|---|
| Structural formula | 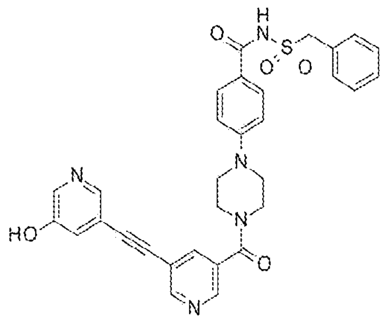 | 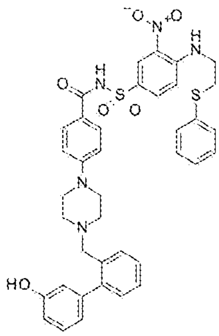 |
| Compound name | N-Benzylsulfonyl-4-[4-[5-[2-(5-hydroxypyridin-3-yl)ethynyl]pyridine-3-carbonyl]piperazin-1-yl]benzamide | 4-[4-[[2-(3-Hydroxyphenyl) phenyl]methyl]piperazin-1-yl]-N-[3-nitro-4-(2-phenylsulfanylethylamino)phenyl] sulfonylbenzamide |
| exact MS | 581.17 | 723.22 |
| lowest cpKa | 5.95 | 5. 08 |
| higest cpKBH+ | 4.01 | 6.96 |
| | | |
| Compound No. | 8-014 | B-015 |
| Structural formula | 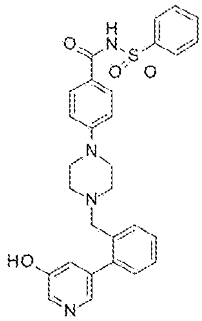 | 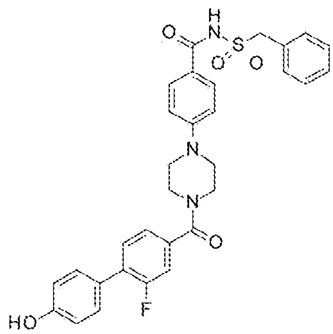 |
| Compound name | N-(Benzenesulfonyl)-4-[4-[[2-(5-hydroxypyridin-3-yl)phenyl] methyl]piperazin-1-yl]benzamide | N-Benzylsulfonyl-4-[4-[3-fluoro-4-(4-hydroxyphenyl)benzoyl] piperazin-1-yl]benzamide |
| exact MS | 528. 18 | 673. 17 |
| lowest cpKa | 5.53 | 6.09 |
| higest cpKBH+ | 6,92 | 4.08 |

In a glove bag that had been purged with nitrogen, to a 4.0 mL screw cap vial were added N-benzylsulfonyl-4-[4-[5-[2-(5-hydroxypyridin-3-yl)ethynyl]pyridine-3-carbonyl]piperazin-1-yl]benzamide (B-003) (4.8 mg, 83.0 μmol),

4-[4-[[2-(3-hydroxyphenyl)phenyl] methyl]piperazin-1-yl]-N-[3-nitro-4-(2-phenylsulfanylethylamino)phenyl]sulfonylbenzamide (B-013) (6.0 mg, 83.0 μmol), N-(benzenesulfonyl)-4-[4-[[2-(5-hydroxypyridin-3-yl)phenyl]methyl]piperazin-1-yl]benzamide (B-014) (4.4 mg, 83.0 μmol), N-benzylsulfonyl-4-[4-[3-fluoro-4-(4-hydroxyphenyl)benzoyl]piperazin-1-yl]benzamide (B-015) (4.7 mg, 83.0 μmol), and DMI (550.0 μL). To this solution was added BTPP (151.0 μL, 495.0 μmol), and after confirming the mixing, triethyl phosphate (B-R-05) (84.0 μL, 495.0 μmol) was added. The obtained reaction solution was stirred at 80°C for 30 hours. After 30 hours, 3 μL of the reaction solution was sampled and diluted with MeCN (1000 μL) to prepare a LC sample, on which LC-MS analysis was carried out. This solution was used as the initial solution at the time of reagent removal operation. Thereafter, mesitylene (151.0 μL, 33.0 μmol) was added to the reaction solution, and 10 μL of the reaction solution was sampled and diluted with DMSO-$d_6$ (580.0 μL), which was used as the initial solution for confirmation of reagent residual amount by $^1$H-NMR.

While washing the reaction solution with MeCN (250.0 μL), the base (BTPP) used in the reaction was adsorbed onto carboxylic acid-supported silica gel (ISOLUTE (R) CBA manufactured by Biotage AB, 1 g/6 mL, 0.7 meq/g) for the purpose of its adsorption and removal, and allowed to stand for 10 minutes. MeCN (1.0 mL) was added, pressurized, and the filtrate was recovered in a test tube. The carboxylic acid-supported silica gel was washed and pressure filtered twice with MeCN (1.0 mL) and all filtrates were collected together to make solution A. Also, for the purpose of evaluating the degree of loss due to adsorption of the substrate group (B-003, B-013, B-014, and B-015) or the product group (N-benzylsulfonyl-4-[4-[5-[2-(5-ethoxypyridin-3-yl)ethynyl]pyridine-3-carbonyl]piperazin-1-yl]benzamide (B-029), 4-[4-[[2-(3-ethoxyphenyl)phenyl]methyl]piperazin-1-yl]-N-[3-nitro-4-(2-phenylsulfanylethylamino)phenyl]sulfonylbenzamide (B-043), N-(benzenesulfonyl)-4-[4-[[2-(5-ethoxypyridin-3-yl)phenyl]methyl]piperazin-1-yl]benzamide (B-077), N-benzylsulfonyl-4-[4-[4-(4-ethoxyphenyl)-3-fluorobenzoyl]piperazin-1-yl]benzamide (B-078)) onto the carboxylic acid-supported silica gel, the carboxylic acid-supported silica gel was washed and pressure filtered three times with a 2M solution of TEA in MeCN (1.0 mL), and all filtrates were collected together to make solution B. After diluting solutions A and B with MeCN until their volumes were almost equal, 25 μL were sampled and diluted with MeCN (1000 μL). By preparing LC samples and carrying out LC-MS analysis, the proportion of compounds contained in solutions A and B was calculated and the recovery ratio of each compound that was recovered in solution A was determined. After blowing nitrogen into the test tube to distill off some of the solvent, the solvent was distilled off under reduced pressure in a centrifugal evaporator (Genevac Ltd.) (40°C, after reducing pressure at 50 to 200 mbar until it was detected that the low boiling point solvent had been completely distilled off, 40°C, < 0.5 mbar for 16 hours), and a mixture of the compounds (B-029, B-043, B-077, and B-078) shown in Table 4-2 was obtained as an oily substance. The obtained substance was dissolved in DMSO-$d_6$, and by performing $^1$H-NMR analysis, the residual reagent was compared.

[Table 32]

[Table 4-2] Structures of compounds obtained by ethylation

| compound No. | Structural formula | Compound name | retention time (min) | exact MS |
|---|---|---|---|---|
| B-029 | 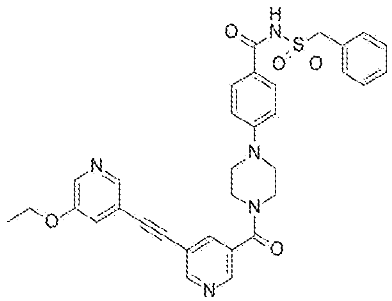 | N-Benzylsulfonyl-4-[4-[5-[2-(5-ethoxypyridin-3-yl)ethynyl]pyridine-3-carbonyl]piperazin-1-yl]benzamide | 1.05 | 609.20 |
| B-043 | 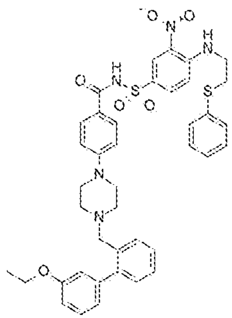 | 4-[4-[[2-(3-Ethoxyphenyl)phenyl] methyl]piperazin-1-yl]-N-[3-nitro-4-(2-phenylsulfanylethylamino) phenyl]sulfonylbenzamide | 1.00 | 751. 25 |
| B-077 | 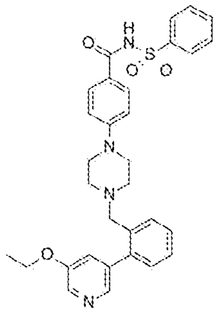 | N-(Benzenesulfonyl)-4-[4-[[2-(5-ethoxypyridin-3-yl)phenyl]methyl] piperazin-1-yl]benzamide | 0. 72 | 556.21 |

(continued)

| compound No. | Structural formula | Compound name | retent ion time (min) | exact MS |
|---|---|---|---|---|
| B-078 | 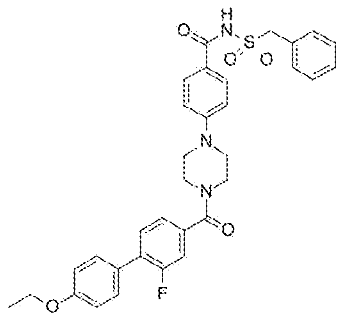 | N-Benzylsulfonyl-4-[4-[[2-(4-prop-2-enoxyphenyl)phenyl] methyl]piperazin-1-yl]benzamide | 1.22 | 601. 20 |

The results of LCMS analysis upon carboxylic acid-supported silica gel treatment are shown in Table 4-3.

[Table 33]

[Table 4-3]

| Compound No. | B-029 | B-043 |
|---|---|---|
| Structural formula | 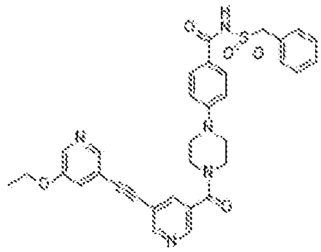 | 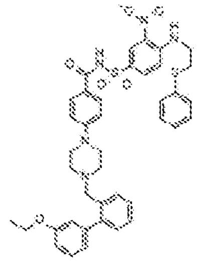 |
| UV area% (310 nm) in initial solution | 28.5 | 14.0 |
| UV area% (310 nm) in solution A | 30.4 | 14.2 |
| a; UV area (310 nm) in solution A | 99384 | 46570 |
| b; UV area (310 nm) in solution B | nd | 367 |
| Recovery ratio % in solution A (a/(a + b) × 100) | 100 | 99.2 |
| Analysis condition | SMD-FA05-RP | |
| Compound No. | B-077 | B-078 |
| Structural formula | 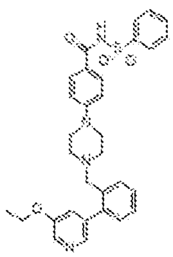 | 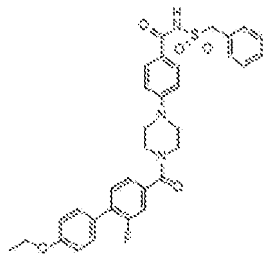 |
| UV area% (310 nm) in initial solution | 21.6 | 21.1 |
| UV area% (310 nm) in solution A | 21.5 | 22.1 |
| a; UV area (310 nm) in solution A | 70390 | 72281 |
| b; UV area (310 nm) in solution B | 1351 | nd |
| Recovery ratio % in solution A (a/(a + b) × 100) | 98.1 | 100 |
| Analysis condition | SMD-FA05-RP | |

The UV area% in solution A obtained after the carboxylic acid-supported silica gel treatment was confirmed to have no significant change compared to the UV area% of each compound in the initial solution. This shows that no certain compound is specifically adsorbed onto the silica gel. Also, the UV areas of each compound in solutions A and B were compared to calculate the recovery ratio in solution A = UV area (310 nm) in solution A/{UV area (310 nm) in solution A + UV area (310 nm) in solution B}. The results revealed that 98.1% or more of each compound was recovered in solution A. From the above results, it was revealed that, although various acidities and basicities are present among the compounds, they can be recovered without being adsorbed by the carboxylic acid-supported silica gel treatment.

The results of $^{1}$H-NMR analysis upon carboxylic acid-supported silica gel treatment are shown in Table 4-4.

[Table 34]

[Table 4-4]

| Analysis target compound | BTPP | BTPP-TFA | $Et_3P0_4$ | 8-043 |
|---|---|---|---|---|
| Structural formula of analysis target compound | 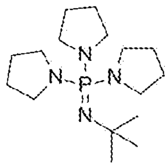 | 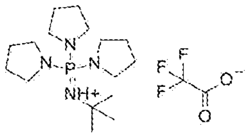 | 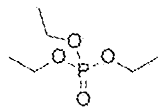 | 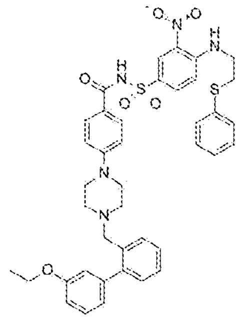 |
| Analysis target peak (ppm) | 1.729 | | 3. 998 | 8. 863 |
| Shape of analysis target peak, number of corresponding protons | br-s, 12H | br-m, 12H | m, 8H | d, 1H |
| Integral value in initial solution | 1051.2 | nd | 505. 2 | 1.00 |
| Molar ratio in initial solution | 87. 6 | nd | 84.2 | 1.0 |
| Integral value after concentration treatment | nd | 11.3 | 5.01 | 1.00 |
| Molar ratio after concentration treatment | nd | 0.94 | 0.835 | 1.0 |

From the analysis of $^1$H-NMR data in the initial solution prepared after the completion of the reaction, it was confirmed that a large excess of BTPP (about 87.6 times the number of moles) and $Et_3PO_4$ (about 84.2 times the number of moles) were present relative to 4-[4-[[2-(3-ethoxyphenyl)phenyl]methyl]piperazin-1-yl]-N-[3-nitro-4-(2-phenylsulfanylethylamino)phenyl]sulfonylbenzamide (B-043). On the other hand, from the analysis of $^1$H-NMR data in the solution prepared from the oily substance on which the carboxylic acid-supported silica gel treatment and concentration operation had been performed, it was confirmed that the free form of BTPP was not detected and was completely removed. It was also confirmed that the salt of BTPP with TFA was reduced to about 0.94 times the number of moles of B-043, and that $Et_3PO_4$ was significantly removed to the extent that it was confirmed at about 0.835 times the number of moles of B-043. Since BTPP formed a salt with TFA that had been coexisted in the substrate beforehand, it was observed in a certain amount, but its effect is very minor since the buffer is used at the time of evaluation in biological experiments of compound groups supplied by the present approach. Furthermore, in the absence of TFA, it does not pose any problem. Note that, while not limited to any specific theory, $Et_3PO_4$ (boiling point: 216°C, the website of Tokyo Chemical Industry Co., Ltd. (https://www.tcichemicals.com/JP/ja/p/P0270), viewed on November 18, 2020) is a neutral compound and was therefore not removed by the carboxylic acid-supported silica gel, but was removed by concentration under reduced pressure.

As described above, it was demonstrated that, even when ethylation is carried out on a mixture of multiple substrates, it is possible to remove the unwanted reagent by utilizing the solid phase reagent and performing distillation

under reduced pressure. It was identified that the method of ethylation in the present invention can appropriately remove the residual reagent and reagent-derived impurity, and is a method applicable to synthesis of mixture libraries.

Example 4-2: Investigation on removal of unwanted reagent by liquid-liquid separation and distillation under reduced pressure when carrying out ethylation on mixture of multiple substrates

Example 4-2-1: Removal of unwanted reagent by liquid-liquid separation and distillation under reduced pressure using ethyl acetate as organic solvent and using saturated aqueous ammonium chloride solution as acidic solution

[Formula 87]

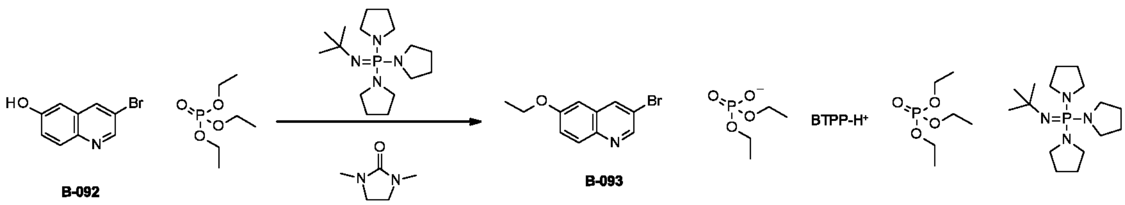


B-092

B-093

In a glove bag that had been purged with nitrogen, to a 2.0 mL screw cap vial were added 3-bromoquinolin-6-ol (B-092) (15.7 mg, 70.0 μmol) and DMI (1.0 mL). To this solution was added BTPP (64.2 μL, 210 μmol, 3.0 eq), and after confirming the mixing, triethyl phosphate (B-R-05) (59.5 μL, 350 μmol, 5.0 eq) was added. The obtained reaction solution was stirred at 80°C for 22 hours.

Reaction tracking

At 22 hours after the start of the reaction, 5 μL of the reaction solution was sampled in a glove bag and diluted with $CH_3CN$ (1000 μL) to prepare a LC sample. As a result of carrying out LC-MS analysis to analyze the reaction progress, it was confirmed that the raw material 3-bromoquinolin-6-ol (B-092) disappeared and 97.2 area% of 3-bromo-6-ethoxyquinoline (B-093) was produced.
3-Bromo-6-ethoxyquinoline: Compound B-093.
LCMS (ESI, m/z): 251.9, 253.9 $[M+H]^+$.
Retention time: 1.176 min (analysis condition SMD-FA05-1).
(The retention time of the raw material ArOH is 0.789 min (analysis condition SMD-FA05-1).

Liquid-liquid separation

While diluting with ethyl acetate (10 mL, 10 times the volume of DMI), the reaction solution was transferred to a 50 mL separatory funnel, and a saturated aqueous ammonium chloride solution (3 mL, 3 times the volume of DMI) was added for the purpose of washing off the base (BTPP) used in the reaction with an acidic aqueous solution to carry out liquid-liquid separation. After draining the aqueous solution, a saturated aqueous ammonium chloride solution (3 mL, 3 times the volume of DMI) was added to the ethyl acetate solution, and liquid-liquid separation by washing was repeated eight times (nine washings in total).
From the ethyl acetate solution after nine washings, 100 μL was sampled and diluted with $CH_3CN$ (750 μL) to prepare a LC sample. From the ninth washing aqueous solution, 200 μL was sampled and diluted with water (800 μL) to prepare a LC sample. LC-MS analysis was carried out to analyze the remaining status of BTPP in the ethyl acetate solution and the removal degree of BTPP in the aqueous solution, and as a result, no m/z of BTPP was observed in the organic solution. Also, the m/z of BTPP was observed in the aqueous solution.
After washing the ethyl acetate solution with water (5 mL), it was dried over sodium sulfate, and the filtrate after filtration was concentrated under reduced pressure. From the obtained 75.8 mg of liquid residue, 10 mg was sampled and diluted with DMSO-$d_6$ (750.0 μL), and $^1$H-NMR analysis was carried out to confirm the amount of reagent remaining. The results are shown in Table 4-5. As shown in the molar ratio column of Table 4-5 for the liquid-liquid separation concentrate, it was confirmed that washing the ethyl acetate solution of the reaction solution with a saturated aqueous ammonium chloride solution reduced the amount of BTPP remaining to a molar ratio of about 0.02 times relative to B-093. On the other hand, neutral compounds triethyl phosphate and DMI were observed to remain at molar ratios of about

3.4 times and about 1.6 times, respectively, relative to B-093.

The sample on which the $^1$H-NMR analysis after the liquid-liquid separation had been performed was recovered, and for the purpose of removing the neutral compounds (triethyl phosphate and DMI) remaining in the liquid-liquid separation concentrate, the solvent was distilled off under reduced pressure in a centrifugal evaporator (Genevac Ltd.) (40°C, after reducing pressure at 50 to 200 mbar until it was detected that the low boiling point solvent had been completely distilled off, 40°C, < 0.5 mbar for 16 hours) to obtain B-093 as a waxy solid substance. The obtained substance was dissolved in DMSO-$d_6$, and by performing $^1$H-NMR analysis, the residual reagent was confirmed.

The results of $^1$H-NMR data analysis prepared from the waxy solid substance after the concentration treatment are shown in Table 4-5. The analysis results confirmed that triethyl phosphate was not detected and was completely removed. It was confirmed that BTPP was removed to the extent that it was confirmed at a molar ratio of about 0.06 times relative to 3-bromo-6-ethoxyquinoline (B-093), and DMI at a molar ratio of about 0.085 times.

[Table 35]

[Table 4-5]

| Analysis target compound | BTPP | $Et_3PO_4$ | DMI | B-093 |
|---|---|---|---|---|
| Structural formula of analysis target compound | 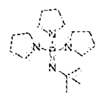 | 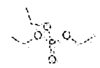 | 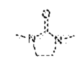 | 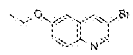 |
| Analysis target peak (ppm) | 1.722 | | 3.199 | 7.353 |
| Shape of analysis target peak, number of corresponding protons | br-ε. 12H | 1.239 t.9H | s. 4H | d. 1H |
| Integral value in concentrate **after** liquid-liquid separation | 0.27 | 30.53 | 6.39 | 1.00 |
| Molar ratio in concentrate after liquid-liquid separation | 0.02 | 3.4 | 1.6 | 1.0 |
| Integral value after concentration treatment | 0.67 | NO | 0.34 | 1.00 |
| Molar ratio after concentration treatment | 0.06 | NO | 0.085 | 1.0 |

Example 4-2-2: Removal of unwanted reagent by liquid-liquid separation and distillation under reduced pressure using ethyl acetate as organic solvent and using 5% aqueous potassium hydrogen sulfate as acidic solution

[Formula 88]

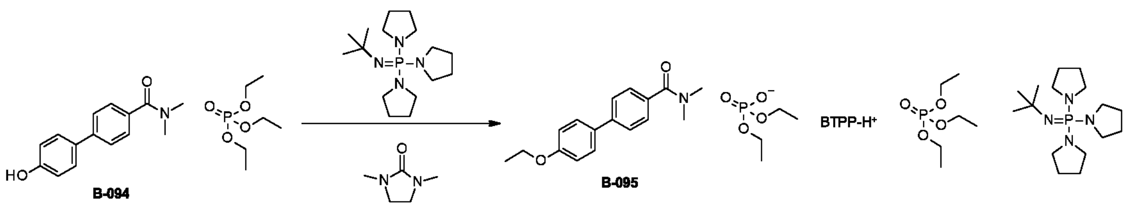


B-094 B-095

In a glove bag that had been purged with nitrogen, to a 2.0 mL screw cap vial were added 4-(4-hydroxyphenyl)-N,N-dimethylbenzamide (B-094) (16.9 mg, 70.0 μmol) and DMI (1.0 mL). To this solution was added BTPP (64.2 μL, 210 μmol, 3.0 eq), and after confirming the mixing, triethyl phosphate (B-R-05) (59.5 μL, 350 μmol, 5.0 eq) was added. The obtained reaction solution was stirred at 80°C for 22 hours. After confirming the reaction progress, to the reaction solution were added BTPP (257.0 μL, 840 μmol, 12.0 eq) and triethyl phosphate (B-R-05) (119.0 μL, 700 μmol, 10.0 eq), and the mixture was stirred at 80°C for 21 hours.

Reaction tracking

At 22 hours after the start of the reaction and 21 hours after the addition of the reagent, 5 μL of the reaction solution was sampled in a glove bag and diluted with $CH_3CN$ (1000 μL) to prepare a LC sample. As a result of analyzing

the reaction progress by carrying out LC-MS analysis, at 22 hours from the start of the reaction, 15.5 area% of the raw material 4-(4-hydroxyphenyl)-N,N-dimethylbenzamide (B-094) remained and 83.3 area% of 4-(4-ethoxyphenyl)-N,N-dimethylbenzamide (B-095) was produced. At 21 hours after the addition of the reagent, it was confirmed that the raw material 4-(4-hydroxyphenyl)-N,N-dimethylbenzamide (B-094) disappeared and 98.3 area% of 4-(4-ethoxyphenyl)-N,N-dimethylbenzamide (B-095) was produced.

4-(4-Ethoxyphenyl)-N,N-dimethylbenzamide: Compound B-095.

LCMS (ESI, m/z): 270.1 $[M+H]^+$.

Retention time: 1.063 min (analysis condition SMD-FA05-1).

(The retention time of the raw material ArOH is 0.760 min (analysis condition SMD-FA05-1).

Liquid-liquid separation

While diluting with ethyl acetate (15 mL, 15 times the volume of DMI), the reaction solution was transferred to a 50 mL separatory funnel, and a 5% aqueous potassium hydrogen sulfate solution (4 mL, 4 times the volume of DMI) was added for the purpose of washing off the base (BTPP) used in the reaction with an acidic aqueous solution to carry out liquid-liquid separation. After draining the aqueous solution, a 5% aqueous potassium hydrogen sulfate solution (4 mL, 4 times the volume of DMI) was added to the ethyl acetate solution, and liquid-liquid separation by washing was repeated five times (six washings in total).

From the ethyl acetate solution after six washings, 100 $\mu$L was sampled and diluted with $CH_3CN$ (750 $\mu$L) to prepare a LC sample. From the sixth washing aqueous solution, 200 $\mu$L was sampled and diluted with water (800 $\mu$L) to prepare a LC sample. LC-MS analysis was carried out to analyze the remaining status of BTPP in the ethyl acetate solution and the removal degree of BTPP in the aqueous solution, and as a result, no m/z of BTPP was observed in the organic solution. Also, the m/z of BTPP was observed in the aqueous solution.

After washing the ethyl acetate solution with water (5 mL), it was dried over sodium sulfate, and the filtrate after filtration was concentrated under reduced pressure. From the obtained 132.5 mg of waxy residue, 10 mg was sampled and diluted with DMSO-d6 (750.0 $\mu$L), and $^1$H-NMR analysis was carried out to confirm the amount of reagent remaining. The results are shown in Table 4-6.

As shown in the molar ratio column of Table 4-6 for the liquid-liquid separation concentrate, it was confirmed that washing the ethyl acetate solution of the reaction solution with a 5% aqueous potassium hydrogen sulfate solution reduced the amount of BTPP remaining to a molar ratio of about 0.03 times relative to B-095. On the other hand, neutral compounds triethyl phosphate and DMI were observed to remain at molar ratios of about 3.5 times and about 0.2 times, respectively, relative to B-095.

The sample on which the $^1$H-NMR analysis after the liquid-liquid separation had been performed was recovered, and for the purpose of removing the neutral compounds (triethyl phosphate and DMI) remaining in the liquid-liquid separation concentrate, the solvent was distilled off under reduced pressure in a centrifugal evaporator (Genevac Ltd.) (40°C, after reducing pressure at 50 to 200 mbar until it was detected that the low boiling point solvent had been completely distilled off, 40°C, < 0.5 mbar for 16 hours) to obtain B-095 as a waxy solid substance. The obtained substance was dissolved in DMSO-$d_6$, and by performing $^1$H-NMR analysis, the residual reagent was confirmed.

The results of $^1$H-NMR data analysis prepared from the waxy solid substance after the concentration treatment are shown in Table 4-6. The analysis results confirmed that triethyl phosphate was not detected and was completely removed. It was confirmed that BTPP was removed to the extent that it was confirmed at a molar ratio of about 0.05 times relative to 4-(4-ethoxyphenyl)-N,N-dimethylbenzamide (B-095), and DMI at a molar ratio of about 0.15 times.

[Table 36]

[Table 4-6]

| Analysis target compound | BTPP | $Et_3PO_4$ | DM | B-095 |
|---|---|---|---|---|
| Structural formula of analysis target compound | 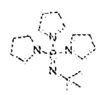 | 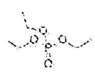 | 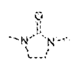 | 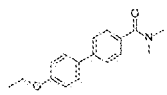 |
| Analysis target peak (ppm) | 1.722 | | 3.199 | 7.454 |
| Shape of analysis target peek, number of corresponding protons | br-s. 12H | quintet , 6H | s. 4H | d. 2H |

(continued)

| Analysis target compound | BTPP | $Et_3PO_4$ | DM | B-095 |
|---|---|---|---|---|
| Integral value in concentrate after liquid-liquid separation | 0.32 | 21.21 | 0.83 | 2.00 |
| Molar ratio in concentrate after liquid-liquid separation | 0.03 | 3.5 | 0.2 | 1.0 |
| Integral value after concentration treatment | 0.64 | NO | 0.62 | 2.00 |
| Molar ratio after concentration treatment | 0.05 | NO | 0.15 | 1.0 |

Example 4-2-3: Removal of unwanted reagent by liquid-liquid separation and distillation under reduced pressure when carrying out ethylation on mixture of multiple substrates

[Formula 89]

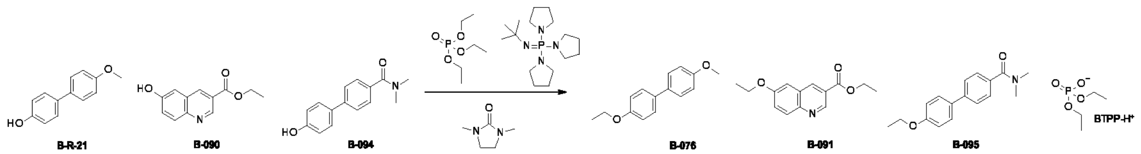


In a glove bag that had been purged with nitrogen, to a 3.0 mL screw cap vial were added 4-hydroxy-4'-methoxybiphenyl (B-R-21) (14.0 mg, 70.0 μmol, 1.0 eq), ethyl 6-hydroxyquinoline-3-carboxylate (B-090) (15.0 mg, 70.0 μmol, 1.0 eq), 4-(4-hydroxyphenyl)-N,N-dimethylbenzamide (B-094) (17.0 mg, 70.0 μmol, 1.0 eq), and DMI (1.0 mL). To this solution was added BTPP (321.0 μL, 1.05 mmol, 15.0 eq), and after confirming the mixing, triethyl phosphate (B-R-05) (179.0 μL, 1.05 μmol, 15.0 eq) was added. The obtained reaction solution was stirred at 80°C for 24 hours.

Reaction tracking

At 2 and 24 hours after the start of the reaction, 5 μL of the reaction solution was sampled in a glove bag and diluted with $CH_3CN$ (1000 μL) to prepare a LC sample. As a result of analyzing the reaction progress by carrying out LC-MS analysis, after 24 hours, it was confirmed that 1.3 area% of the raw material 4-hydroxy-4'-methoxybiphenyl (B-R-21) remained and ethyl 6-hydroxyquinoline-3-carboxylate (B-090) and 4-(4-hydroxyphenyl)-N,N-dimethylbenzamide (B-094) disappeared. It was also confirmed that 24.1 area% of 1-ethoxy-4-(4-methoxyphenyl)benzene (B-076), 49.5 area% of ethyl 6-ethoxyquinoline-3-carboxylate (B-091), and 23.1 area% of 4-(4-ethoxyphenyl)-N,N-dimethylbenzamide (B-095) were produced.

Ethyl 6-ethoxyquinoline-3-carboxylate: Compound B-091.

LCMS (ESI, m/z): 246.1 $[M+H]^+$.

Retention time: 1.076 min (analysis condition SMD-FA05-1).

(The retention time of the raw material ArOH is 0.708 min (analysis condition SMD-FA05-1).

Liquid-liquid separation

While diluting with ethyl acetate (15 mL, 15 times the volume of DMI), the reaction solution was transferred to a 50 mL separatory funnel, and a 5% aqueous potassium hydrogen sulfate solution (4 mL, 4 times the volume of DMI) was added for the purpose of washing off the base (BTPP) used in the reaction with an acidic aqueous solution to carry out liquid-liquid separation. After draining the aqueous solution, a 5% aqueous potassium hydrogen sulfate solution (4 mL, 4 times the volume of DMI) was added to the ethyl acetate solution, and liquid-liquid separation by washing was repeated five times (six washings in total).

From the ethyl acetate solution after six washings, 100 μL was sampled and diluted with $CH_3CN$ (750 μL) to prepare a LC sample. From the sixth washing aqueous solution, 200 μL was sampled and diluted with water (800 μL) to prepare a LC sample. LC-MS analysis was carried out to analyze the remaining status of BTPP in the ethyl acetate solution and the removal degree of BTPP in the aqueous solution, and as a result, no m/z of BTPP was observed in the organic solution. Also, the m/z of BTPP was observed in the aqueous solution.

After washing the ethyl acetate solution with water (5 mL), it was dried over sodium sulfate, and the filtrate after

filtration was concentrated under reduced pressure. From the obtained 193.0 mg of waxy solid residue, 10 mg was sampled and diluted with DMSO-$d_6$ (750.0 μL), and $^1$H-NMR analysis was carried out to confirm the amount of reagent remaining. The results are shown in Table 4-7.

As shown in the molar ratio column of Table 4-7 for the liquid-liquid separation concentrate, it was confirmed that washing the ethyl acetate solution of the reaction solution with a 5% aqueous potassium hydrogen sulfate solution reduced the amount of BTPP remaining to a molar ratio of about 0.05 times relative to B-091. On the other hand, neutral compounds triethyl phosphate and DMI were observed to remain at molar ratios of about 8.7 times and about 1.3 times, respectively, relative to B-091.

The sample on which the $^1$H-NMR analysis after the liquid-liquid separation had been performed was recovered, and for the purpose of removing the neutral compounds (triethyl phosphate and DMI) remaining in the liquid-liquid separation concentrate, the solvent was distilled off under reduced pressure in a centrifugal evaporator (Genevac Ltd.) (40°C, after reducing pressure at 50 to 200 mbar until it was detected that the low boiling point solvent had been completely distilled off, 40°C, < 0.5 mbar for 16 hours) to obtain a mixture of B-076, B-091, and B-095 as a waxy solid substance. The obtained substance was dissolved in DMSO-$d_6$, and by performing $^1$H-NMR analysis, the residual reagent was confirmed.

The results of $^1$H-NMR data analysis prepared from the waxy solid substance after the concentration treatment are shown in Table 4-7. The analysis results confirmed that triethyl phosphate was not detected and was completely removed. It was confirmed that BTPP was removed to the extent that it was confirmed at a molar ratio of about 0.05 times relative to ethyl 6-ethoxyquinoline-3-carboxylate (B-091), and DMI at a molar ratio of about 1.0 times.

[Table 37]

[Table 4-7]

| Analysis target compound | BTPP | $Et_3PO_4$ | DMI | B-091 |
|---|---|---|---|---|
| Structural formula of analysis target compound | 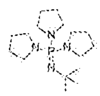 | 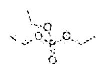 | 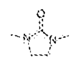 | 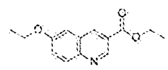 |
| Analysis target peak (ppm) | 1. 722 | 4.000 | 3.199 | B.873 |
| Shape of analysis target peak, number of corresponding protons | br-s, 12H | quintet, 6H | s, 4H | d, 1H |
| Integral value in concentrate **after** liquid-liquid separation | 0.58 | 52.35 | 5.03 | 1.00 |
| Molar ratio in concentrate after liquid-liquid separation | 0.05 | 8.7 | 1.3 | 1.0 |
| Integral value after concentration treatment | 0.56 | NO | 4.21 | 1.00 |
| Molar ratio after concentration treatment | 0.05 | NO | 1.05 | 1.0 |

As described above, it was demonstrated, even when ethylation is carried out on a mixture of multiple substrates, it is possible to remove the unwanted reagent by utilizing liquid-liquid separation with an acidic aqueous solution after dilution with an organic solvent and by performing distillation under reduced pressure. It was identified that the method of ethylation in the present invention can appropriately remove the residual reagent and reagent-derived impurity, and is a method applicable to synthesis of mixture libraries.

Example 5: Investigation on O-selective allylation of arenol to substrates having multiple reactive functional groups in molecule

[Formula 90]

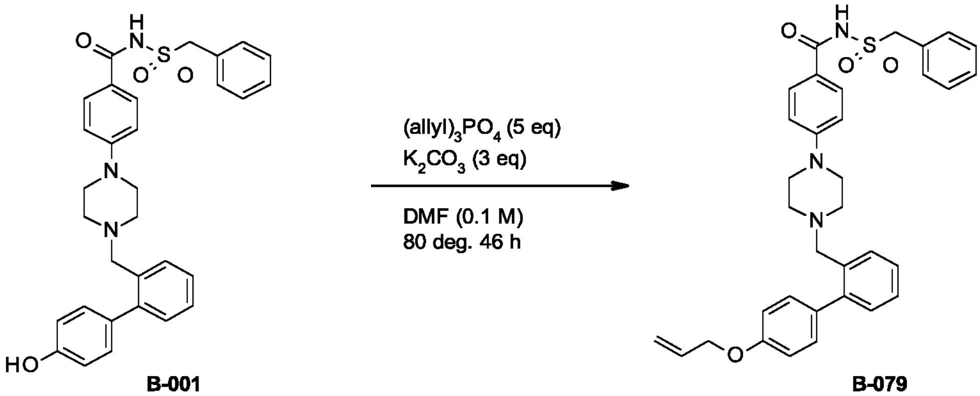


B-001 B-079

In a glove bag that had been purged with nitrogen, to a 0.6 mL screw cap vial were added N-benzylsulfonyl-4-[4-[[2-(4-hydroxyphenyl)phenyl]methyl]piperazin-1-yl]benzamide (B-001) (2.7 mg, 5.0 μmol, 1.0 eq) and DMF (50.0 μL). To the obtained solution was added $K_2CO_3$ (2.1 mg, 15.0 μmol, 3.0 eq), and after mixing for 15 to 20 seconds, triallyl phosphate (5.1 μL, 25.0 μmol, 5.0 eq) was added. The obtained reaction mixture was stirred at 80°C for 46 hours at 1400 rpm.

Reaction tracking

During the process, at 46 hours after the start of the reaction, 5 μL of the reaction solution was sampled in a glove bag and diluted with $CH_3CN$ (1000 μL) to prepare a LC sample. The reaction progress was analyzed by carrying out LC-MS analysis. As a result, production of 100 area% of N-benzylsulfonyl-4-[4-[[2-(4-prop-2-enoxyphenyl)phenyl]methyl]piperazin-1-yl]benzamide (compound B-079) was observed.

N-Benzylsulfonyl-4-[4-[[2-(4-prop-2-enoxyphenyl)phenyl]methyl]piperazin-1-yl]benzamide: Compound B-079.

LCMS (ESI, m/z): 582 $[M+H]^+$.

Retention time: 0.66 min (analysis condition SQD-FA05-2).

(The retention time of the raw material ArOH is 0.55 min (analysis condition SQD-FA05-2).

From the above results, it was shown that allylation is also applicable as O-selective alkylation of arenol to a substrates having multiple reactive functional groups.

Example 6: Production example of compound library having diversity in functional groups using O-selective ethylation method for arenol

In Example 6, utilizing the findings obtained in Examples 2 to 5, one example of their application to synthesis of a compound library having diversity in functional groups will be shown, in which highly selective ethylation to arenol having various reactive functional groups is carried out and post-treatment that does not require purification operation with a column is performed. For the purpose of illustrating the practicality of a compound library prepared using the method of the present invention and demonstrating that the desired compound group is obtained, binding evaluation of the synthesized mixture to Bcl-2 using AS-MS (Example 7), individual synthesis of Bcl-2 binding compounds identified by AS-MS (Example 8), and binding evaluation of the individually synthesized compounds to Bcl-2 by surface plasmon resonance (SPR) (Example 9) were performed. The core blocks and linkers that constitute the compounds included in the library are shown in Figure 1.

In Figure 1,

[Formula 91]

● represents 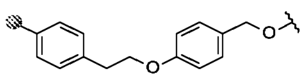 .

As an example, 1,200 compounds were set as library compounds from among various compounds resulting from combinations of core blocks and linkers shown in Figure 1, and synthesis of a mixture library was performed. Note that, for the purpose of confirming that the mixture library provided by the present approach appropriately functions at

the time of AS-MS evaluation, the library compounds were set to include four compounds that had been previously synthesized by the method shown in Example 8 and evaluated for binding by SPR as shown in Example 9: B2Q045-01 ($K_D$ = 1.2 μM), B2Q046-01 ($K_D$ = 8.1 μM), B2R045-01 ($K_D$ = > 20 μM), and B2R046-01 ($K_D$ = > 20 μM). The compounds whose binding ability had been previously evaluated, B2Q045-01, B2Q046-01, B2R045-01, and B2R046-01, are compounds that were presumed to maintain their binding ability to Bcl-2 according to known literature (Expert Opinion on Drug Discovery 2008, 3(9), 1123-1143 and J. Med. Chem. 2008, 51, 6902-6915), and the binding was actually confirmed.

The library synthesis was carried out by a method in which, after introducing each building block, that is, after the linker formation reaction, a mixture was prepared for each type of reactive functional group present on the building block, and the subsequent linker formation reaction was performed.

The method is shown below as an example of a mixture library synthesis method having diversity in linkers.

In the library synthesis shown below, the building blocks shown in [Table LBB02] were used.

[Table 38]

[Table LBB02]

| Compound No. | Structural formula | Compound name |
|---|---|---|
| BB01 | 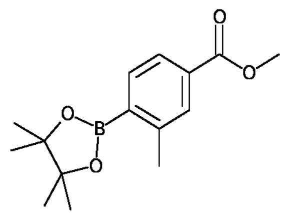 | Methyl 3-methyl-4-(4,4,5,5-tetramethyl-1,3,2-dioxaborolan-2-yl)benzoate |
| BB02 | 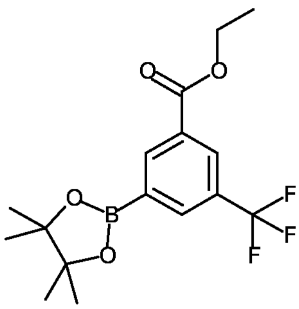 | Ethyl 3-(4,4,5,5-tetramethyl-1,3,2-dioxaborolan-2-yl)-5-(trifluoromethyl)benzoate |
| BB03 | 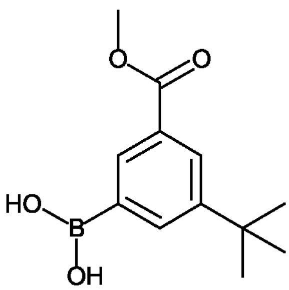 | (3-tert-Butyl-5-methoxycarbonylphenyl) boronic acid |

[Table 38-1]

| | | |
|---|---|---|
| BB04 | 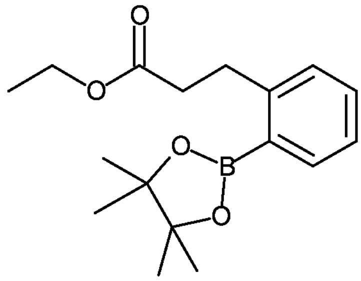 | Ethyl 3-[2-(4,4,5,5-tetramethyl-1,3,2-dioxaborolan-2-yl)phenyl]propanoate |
| BB05 | 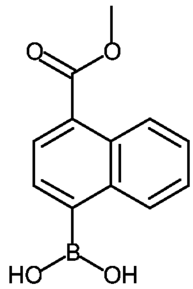 | (4-Methoxycarbonylnaphthalen-1-yl)boronic acid |
| BB06 | 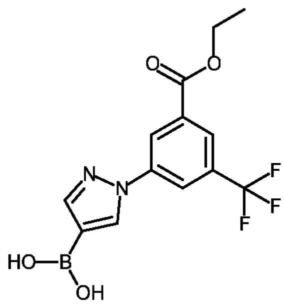 | {1-[3-Ethoxycarbonyl-5- (trifluoromethyl)phenyl] pyrazol-4-yl}boronic acid |
| BB07 | 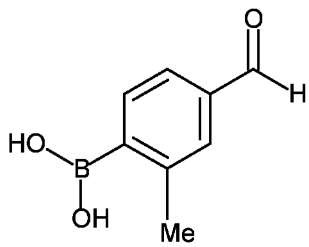 | (4-Formyl-2-methylphenyl)boronic acid |
| BB08 | 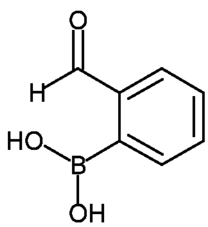 | (2-Formylphenyl)boronic acid |
| BB09 | 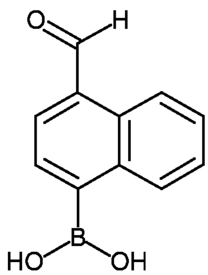 | (4-Formylnaphthalen-1-yl)boronic acid |

(continued)

| | | |
|---|---|---|
| BB10 | 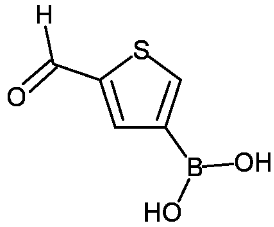 | (5-Formylthiophen-3-yl)boronic acid |
| BB11 | 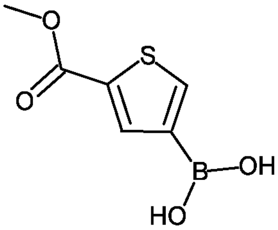 | (5-(Methoxycarbonyl)thiophen-3-yl)boronic acid |
| BB12 | 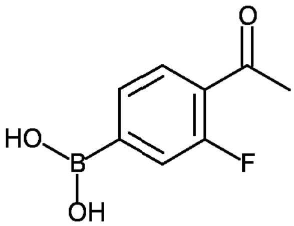 | (4-Acetyl-3-fluorophenyl)boronic acid |
| BB13 | 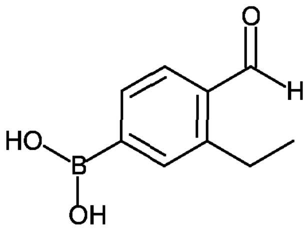 | (3-Ethyl-4-formylphenyl)boronic acid |
| BB14 | 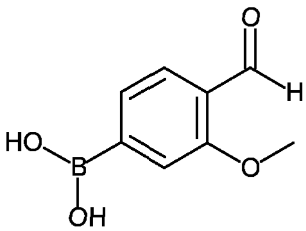 | (4-Formyl-3-methoxyphenyl)boronic acid |
| BB15 | 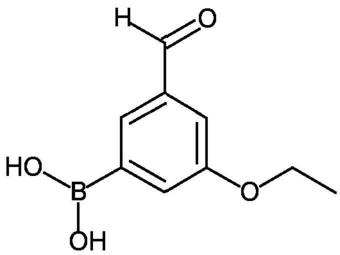 | (3-Ethoxy-5-formylphenyl)boronic acid |

(continued)

| | | |
|---|---|---|
| BB16 | 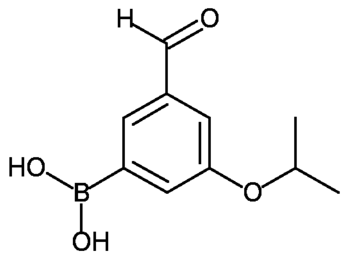 | (3-Formyl-5-propan-2-yloxyphenyl)boronic acid |
| BB17 | 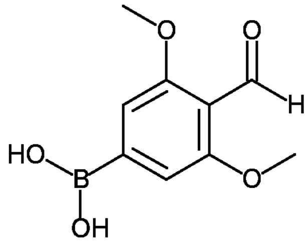 | (4-Formyl-3,5-dimethoxyphenyl)boronic acid |
| BB18 | 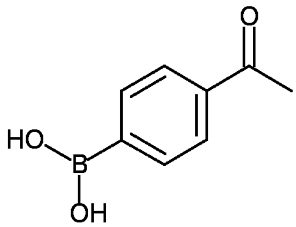 | (4-Acetylphenyl)boronic acid |
| BB19 | 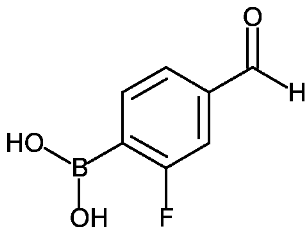 | (2-Fluoro-4-formylphenyl)boronic acid |
| BB20 | 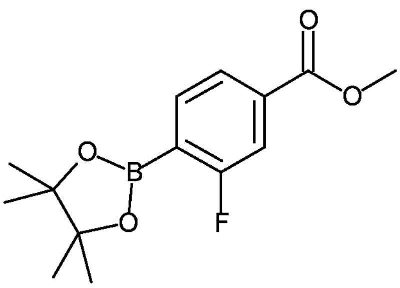 | Methyl 3-fluoro-4-(4,4,5,5-tetramethyl-1,3,2-dioxaborolan-2-yl)benzoate |
| BB21 | 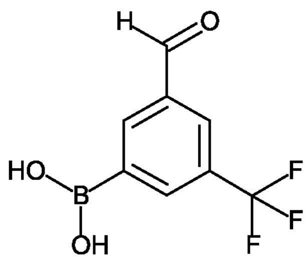 | [3-Formyl-5-(trifluoromethyl)phenyl] boronic acid |

(continued)

| | | |
|---|---|---|
| BB22 | 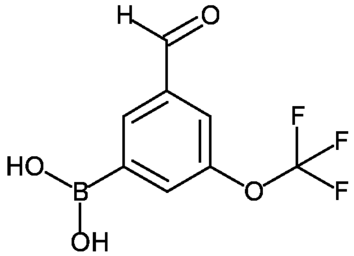 | [3-Formyl-5-(trifluoromethoxy)phenyl] boronic acid |
| BB23 | 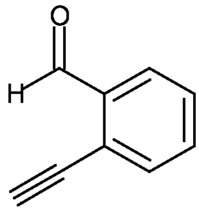 | 2-Ethynylbenzaldehyde |
| BB24 | 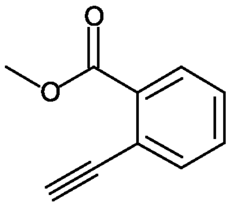 | Methyl 2-ethynylbenzoate |
| BB25 | 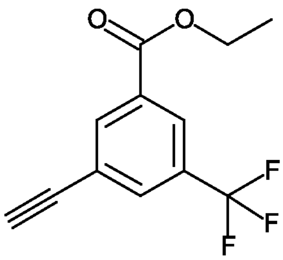 | Ethyl 3-ethynyl-5-(trifluoromethyl)benzoate |
| BB26 | 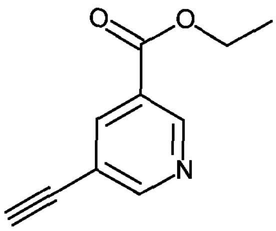 | Ethyl 5-ethynylpyridine-3-carboxylate |
| BB27 | 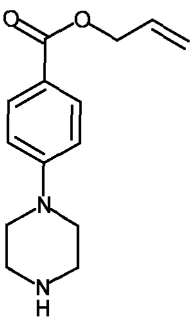 | Allyl 4-(piperazin-1-yl)benzoate |

(continued)

| | | |
|---|---|---|
| BB28 | 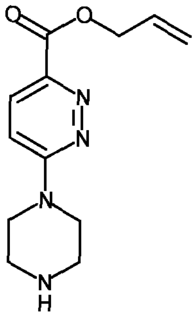 | Allyl 6-(piperazin-1-yl)pyridazine-3-carboxylate |
| BB29 | 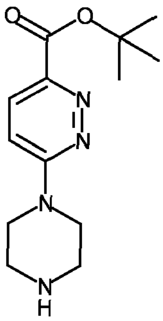 | tert-Butyl 6-(piperazin-1-yl)pyridazine-3-carboxylate |
| BB30 | 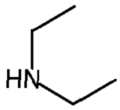 | N-Ethylethanamine |
| BB31 | 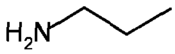 | Propan-1-amine |
| BB32 | 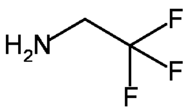 | 2,2,2-Trifluoroethanamine |
| BB33 | 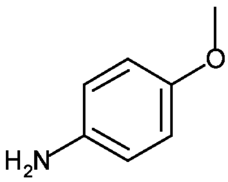 | 4-Methoxyaniline |
| BB34 | 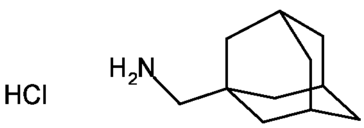 | 1-Adamantylmethanamine hydrochloride |
| BB35 | 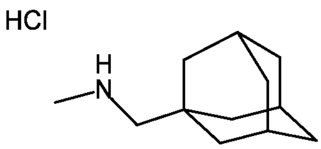 | 1-(1-Adamantyl)-N-methylmethanamine hydrochloride |
| BB36 | 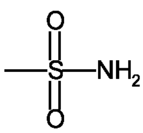 | Methanesulfonamide |

(continued)

| | | |
|---|---|---|
| BB37 | 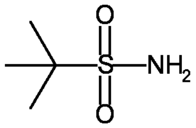 | 2-Methylpropane-2-sulfonamide |
| BB38 | 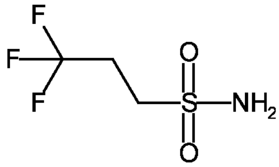 | 3,3,3-Trifluoropropane-1- sulfonamide |
| BB40 | 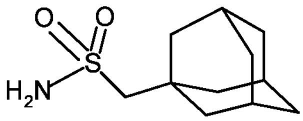 | 1-Adamantylmethanesulfonamide |
| BB41 | 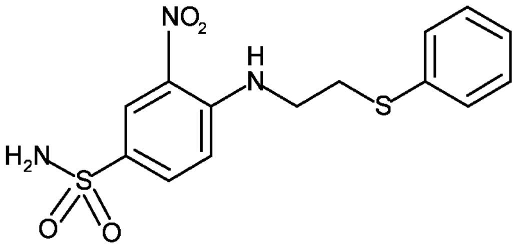 | 3-Nitro-4-{[2-(phenylthio)ethyl]amino} benzenesulfonamide |
| BB42 | 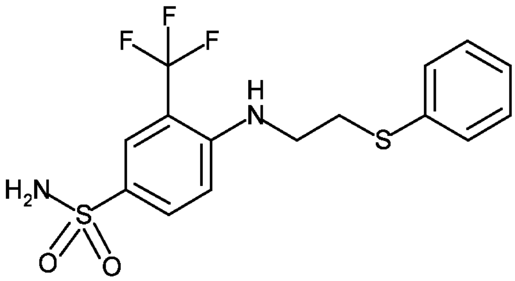 | 4-(2-Phenylsulfanylethylamino)-3-(trifluoromethyl) benzenesulfonamide |

In the present Example 6, compounds supported on a brominated modified Wang resin: 4-(bromomethyl)phenoxyethyl polystyrene, for example, Merck KGaA, 2-(4-bromomethyl)phenoxyethyl polystyrene, were used, and "-02R" is added to the end of the compound No. of the compound supported on the solid phase. In the tables in Example 6, only the compound name after cleavage may be given in the conversion reaction for compounds supported on the solid phase, and the compound No. without "-02R" may also be given.

Example 6-1-1-A: Synthesis of mixtures mixEG01-02R to mixEG04-02R and mixEG18-02R

[Formula 92]

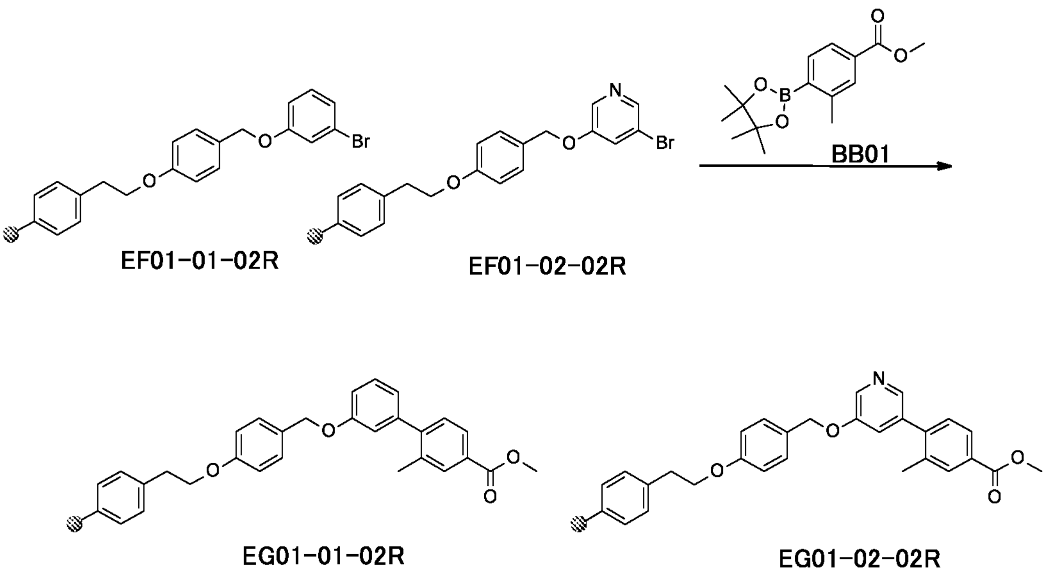


EF01-01-02R EF01-02-02R

EG01-01-02R EG01-02-02R

Under a nitrogen atmosphere, to a 4 mL glass vial were added compound EF01-01-02R (57 mg, loading rate 0.556 mmol/g, 0.032 mmol), compound EF01-02-02R (43 mg, loading rate 0.733 mmol/g, 0.032 mmol), and NMP (1.95 mL), and the mixture was shaken at room temperature for 1 hour. Under a nitrogen atmosphere, methyl 3-methyl-4-(4,4,5,5-tetramethyl-1,3,2-dioxaborolan-2-yl)benzoate (BB01) (107 mg, 0.387 mmol), an aqueous $K_3PO_4$ solution (1.7M, 228 μL, 0.387 mmol), and a solution of $P(Cy)_3Pd$ G3 in NMP (0.04M, 323 μL, 13 μmol) were added, and the mixture was shaken at 90°C for 20 hours. The suspension of the reaction solution and the resins was transferred to a syringe equipped with a filter using NMP, washed repeatedly three times with NMP (2 mL), three times with a solution (2 mL) of NMP containing NAC (10 mg/mL):water (1:1), three times with water (2 mL), three times with MeOH (2 mL), three times with DCM (2 mL), and three times with heptane (2 mL), and the resins were then dried under reduced pressure to obtain the title compounds shown in [Table EG01]. The building blocks used and the compounds contained in the obtained mixture are shown in [Table EG01].

The 1.7M aqueous $K_3PO_4$ solution was prepared by using a 5 mL measuring flask under a nitrogen atmosphere and diluting $K_3PO_4$ (1.8 g, 8.48 mmol) with distilled water.

The 0.04M solution of $P(Cy)_3Pd$ G3 in NMP was prepared by adding NMP (1 mL) to $P(Cy)_3Pd$ G3 (26 mg, 0.04 mmol) in a 2 mL glass vial under a nitrogen atmosphere and shaking to dissolve the mixture.

Example 6-1-1-B: Synthesis of mixtures mixEG02-02R to mixEG04-02R and mixEG18-02R

Using compound EF01-01-02R (57 mg, loading rate 0.556 mmol/g, 0.032 mmol), compound EF01-02-02R (43 mg, loading rate 0.733 mmol/g, 0.032 mmol), and the boronic acid or boronate ester reagents (BB02 to 04 and 18) shown in [Table LBB02], the title compounds shown in [Table EG01] were synthesized by the same method as in Example 6-1-1-A. The building blocks used, the compounds contained in the obtained mixture, and the reaction time are shown in [Table EG01].

[Table 39]

[Table EG01]

| BB No. used | Synthesized mixture No. | Resin compound No. | Post-cleavage mixture No. | Post-cleavage compound No. | Compound name | Reaction time (hr) |
|---|---|---|---|---|---|---|
| BB01 | mixEG01-02R | EG01-01-02R | mixEG01 | EG01-01 | Methyl 4-(3-hydroxyphenyl)-3-methylbenzoate | 20 |
| | | EG01-02-02R | | EG01-02 | Methyl 4-(5-hydroxypyridin-3-yl)-3-methylbenzoate | |
| BB02 | mixEG02-02R | EG01-03-02R | mixEG02 | EG01-03 | Ethyl 3-(3-hydroxyphenyl)-5-(trifluoromethyl)benzoate | 20 |
| | | EG01-04-02R | | EG01-04 | Ethyl 3-(5-hydroxypyridin-3-yl)-5-(trifluoromethyl)benzoate | |
| BB03 | mixEG03-02R | EG01-05-02R | mixEG03 | EG01-05 | Methyl 3-tert-butyl-5-(3-hydroxyphenyl)benzoate | 20 |
| | | EG01-06-02R | | EG01-06 | Methyl 3-tert-butyl-5-(5-hydroxypyridin-3-yl)benzoate | |
| BB04 | mixEG04-02R | EG01-07-02R | mixEG04 | EG01-07 | Ethyl 3-[2-(3-hydroxyphenyl)phenyl]propanoate | 20 |
| | | EG01-08-02R | | EG01-08 | Ethyl 3-[2-(5-hydroxypyridin-3-yl)phenyl]propanoate | |
| BB18 | mixEG18-02R | EG01-09-02R | mixEG18 | EG01-09 | 1-[4-(3-Hydroxvphenyl)phenyl]ethanone | 20 |
| | | EG01-10-02R | | EG01-10 | 1-[4-(5-Hydroxypyridin-3-yl)phenyl]ethanone | |

Example 6-1-2-A: Synthesis of mixture mixEG05-02R

[Formula 93]

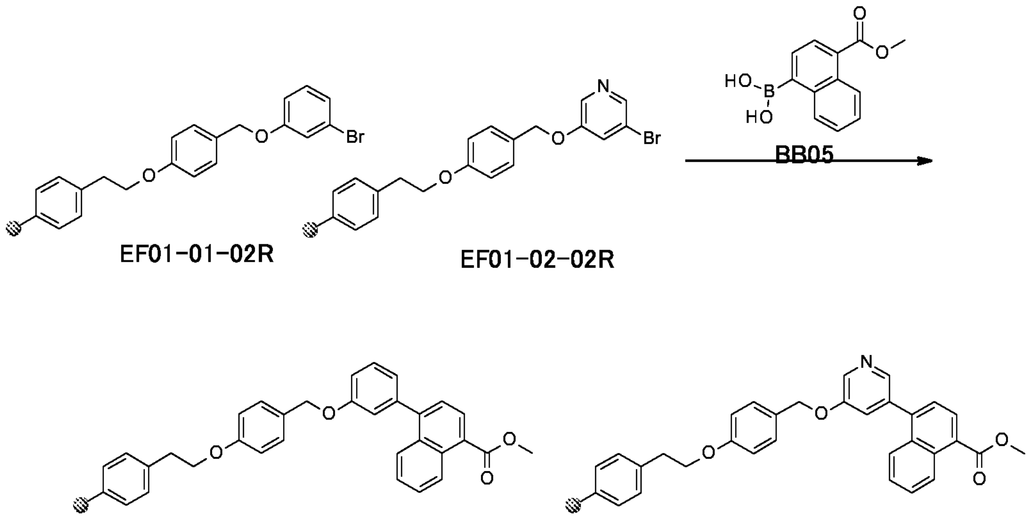

BB05

EF01-01-02R EF01-02-02R

EG05-01-02R EG05-02-02R

Under a nitrogen atmosphere, to a 4 mL glass vial were added compound EF01-01-02R (57 mg, loading rate 0.556 mmol/g, 0.032 mmol), compound EF01-02-02R (43 mg, loading rate 0.733 mmol/g, 0.032 mmol), and NMP (1.95 mL), and the mixture was shaken at room temperature for 1 hour. Under a nitrogen atmosphere, (4-methoxycarbonylnaphthalen-1-yl)boronic acid (BB05) (178 mg, 0.774 mmol), an aqueous $K_3PO_4$ solution (1.7M, 228 μL, 0.387 mmol), a solution of $P(Cy)_3Pd$ G3 in NMP (0.04M, 323 μL, 13 μmol) were added, and the mixture was shaken at 90°C for 20 hours. The suspension of the reaction solution and the resins was transferred to a syringe equipped with a filter using NMP, washed repeatedly three times with NMP (2 mL), three times with a solution (2 mL) of NMP containing NAC (10 mg/mL):water (1:1), three times with water (2 mL), three times with MeOH (2 mL), three times with DCM (2 mL), and three times with heptane (2 mL), and the resins were then dried under reduced pressure to obtain the title compounds shown in [Table EG05]. The building blocks used, the compounds contained in the obtained mixture, and the reaction time are shown in [Table EG05].

Example 6-1-2-B: Synthesis of mixture mixEG06-02R

Using compound EF01-01-02R (57 mg, loading rate 0.556 mmol/g, 0.032 mmol), compound EF01-02-02R (43 mg, loading rate 0.733 mmol/g, 0.032 mmol), and [1-[3-ethoxycarbonyl-5-(trifluoromethyl)phenyl]pyrazol-4-yl]boronic acid (BB06) shown in [Table LBB02], the title compounds shown in [Table EG05] were synthesized by the same method as in Example 6-1-2-A. The building blocks used, the compounds contained in the obtained mixture, and the reaction time are shown in [Table EG05].

[Table 40]

[Table EG05]

| BB No. used | Synthesized mixture No. | Resin compound No. | Post-cleavage mixture No. | Post-cleavage compound No. | Compound name | Reaction time (hr) |
|---|---|---|---|---|---|---|
| BB05 | mixEG05-02R | EG05-01-02R | mixEG05 | EG05-01 | Methyl 4-(3-hydroxyphenyl)naphthalene-1-carboxylate | 20 |
| | | EG05-02-02R | | EG05-02 | Methyl 4-(5-hydroxypyridin-3-yl)naphthalene-1-carboxylate | |
| BB06 | mixEG06-02R | EG05-03-02R | mixEG06 | EG05-03 | Ethyl 3-[4-(3-hydroxyphenyl)pyrazol-1-yl]-5-(trifluoromethyl)benzoate | 20 |
| | | EG05-04-02R | | EG05-04 | Ethyl 3-[4-(5-hydroxypyridin-3-yl)pyrazol-1-yl]-5-(trifluoromethyl)benzoate | |

Example 6-1-3-A: Synthesis of mixture mixEG07-02R

[Formula 94]

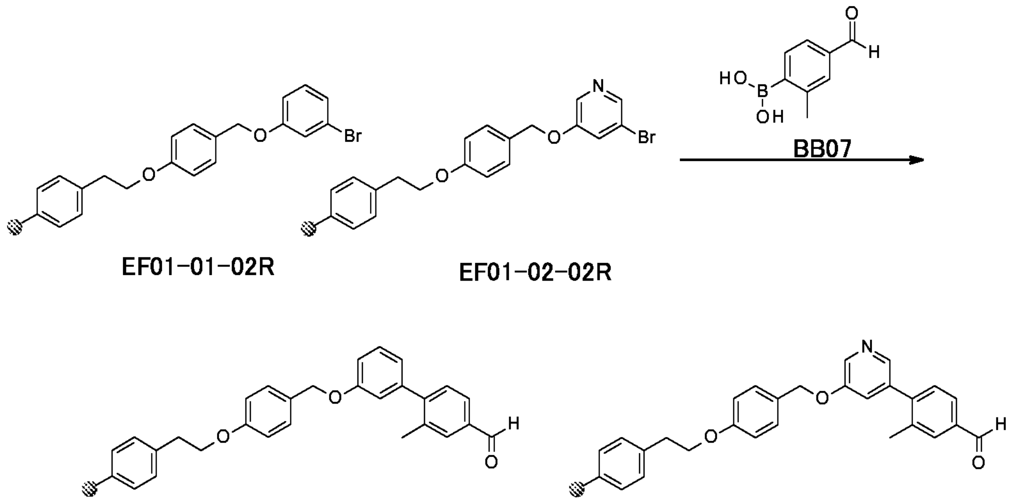


EF01-01-02R EF01-02-02R

EG07-01-02R EG07-02-02R

Under a nitrogen atmosphere, to a 3 mL glass vial were added compound EF01-01-02R (57 mg, loading rate 0.556 mmol/g, 0.032 mmol), compound EF01-02-02R (43 mg, loading rate 0.733 mmol/g, 0.032 mmol), NMP (1.4 mL), and water (72 μL), and the mixture was shaken at room temperature for 1 hour. Under a nitrogen atmosphere, (4-formyl-2-methylphenyl)boronic acid (BB07) (63.5 mg, 0.387 mmol), $Na_2CO_3$ (41 mg, 0.387 mmol), a solution of $P(Cy)_3Pd$ G3 in NMP (0.04M, 323 μL, 13 μmol) were added, and the mixture was shaken at 90°C for 21 hours. The suspension of the reaction solution and the resins was transferred to a syringe equipped with a filter using NMP, washed repeatedly three times with NMP (2 mL), three times with a solution (2 mL) of NMP containing NAC (10 mg/mL):water (1:1), three times with water (2 mL), three times with MeOH (2 mL), three times with DCM (2 mL), and three times with heptane (2 mL), and the resins were then dried under reduced pressure to obtain the title compounds shown in [Table EG07]. The building blocks used, the compounds contained in the obtained mixture, and the reaction time are shown in [Table EG07] .

Example 6-1-3-B: Synthesis of mixtures mixEG08-02R to mixEG17-02R

Using compound EF01-01-02R (57 mg, loading rate 0.556 mmol/g, 0.032 mmol), compound EF01-02-02R (43 mg, loading rate 0.733 mmol/g, 0.032 mmol), and the boronic acid or boronate ester reagents (BB08 to 17) shown in [Table LBB02], the title mixtures shown in [Table EG07] were synthesized by the same method as in Example 6-1-3-A. The building blocks used, the compounds contained in the obtained mixture, and the reaction time are shown in [Table EG07].

[Table 41]

[Table EG07]

| BB No. used | Synthesized mixture No. | Resin compound No. | Post-cleavage mixture No. | Post-cleavage compound No. | Compound name | Reaction time (hr) |
|---|---|---|---|---|---|---|
| BB07 | mixEG07-02R | EG07-0102R | mixEG07 | EG07-01 | 4-(3-Hydroxyphenyl)-3-methylbenzaldehyde | 21 |
| | | EG07-0202R | | EG07-02 | 4-(5-Hydroxypyridin-3-yl)-3- methylbenzaldehyde | |
| BB08 | mixEG08-02R | EG07-0302R | mixEG08 | EG07-03 | 2-(3-Hydroxyphenyl)benzaldehyde | 21 |
| | | EG07-0402R | | EG07-04 | 2-(5-Hydroxypyridin-3-vl)benzaldehvde | |
| BB09 | mixEG09-02R | EG07-0502R | mixEG09 | EG07-05 | 4-(3-Hydroxyphenyl)naphthalene-1-carbaldehyde | 21 |
| | | EG07-0602R | | EG07-06 | 4-(5-Hydroxypyridin-3-yl)naphthalene-1-carbaldehyde | |
| BB10 | mixEG 10-02R | EG07-0702R | mixEG10 | EG07-07 | 4-(3-Hydroxyphenyl)thiophene-2-carbaldehyde | 21 |
| | | EG07-0802R | | EG07-08 | 4-(5-Hydroxypyridin-3-yl)thiophene-2-carbaldehyde | |
| BB11 | mixEG11-02R | EG07-0902R | mixEG11 | EG07-09 | Methyl 4-(3-hydroxyphenyl)thiophene-2-carboxylate | 21 |
| | | EG07-1002R | | EG07-10 | Methyl 4-(5-hydroxypyridin-3-yl)thiophene-2-carboxylate | |
| BB12 | mixEG12-02R | EG07-1102R | mixEG12 | EG07-11 | 1-[2-Fluoro-4-(3-hydroxyphenyl)phenyl]ethanone | 27 |
| | | EG07-1202R | | EG07-12 | 1 -[2-Fluoro-4-(5-hydroxypyridin-3-yl)phenyl]ethanone | |
| BB13 | mixEG13-02R | EG07-1302R | mixEG13 | EG07-13 | 2-Ethyl-4-(3-hydroxyphenyl)benzaldehyde | 66 |
| | | EG07-1402R | | EG07-14 | 2-Ethyl-4-(5-hydroxypyridin-3-yl)benzaldehyde | |
| BB14 | mixEG14-02R | EG07-1502R | mixEG14 | EG07-15 | 4-(3-Hydroxyphenyl)-2-methoxybenzaldehyde | 66 |
| | | EG07-1602R | | EG07-16 | 4-(5-Hydroxypyridin-3-yl)-2-methoxybenzaldehyde | |
| BB15 | mixEG15-02R | EG07-1702R | mixEG15 | EG07-17 | 3-Ethoxy-5-(3-hydroxyphenyl)benzaldehyde | 66 |
| | | EG07-1802R | | EG07-18 | 3-Ethoxy-5-(5-hydroxypyridin-3-yl)benzaldehyde | |

(continued)

| BB No. used | Synthesized mixture No. | Resin compound No. | Post-cleavage mixture No. | Post-cleavage compound No. | Compound name | Reaction time (hr) |
|---|---|---|---|---|---|---|
| BB16 | mixEG16-02R | EG07-1902R | mixEG16 | EG07-19 | 3-(3-Hydroxyphenyl)-5-propan-2-yloxybenzaldehyde | 66 |
| | | EG07-2002R | | EG07-20 | 3-(5-Hydroxypyridin-3-yl)-5-propan-2-yloxybenzaldehyde | |
| BB17 | mixEGI7-02R | EG07-2102R | mixEG17 | EG07-21 | 4-(3-Hydroxyphenyl)-2,6-dimethoxybenzaldehyde | 66 |
| | | EG07-2202R | | EG07-22 | 4-(5-Hydroxypyridin-3-yl)-2,6-dimethoxybenzaldehyde | |

Example 6-1-4-A: Synthesis of mixture mixEG19-02R

[Formula 95]

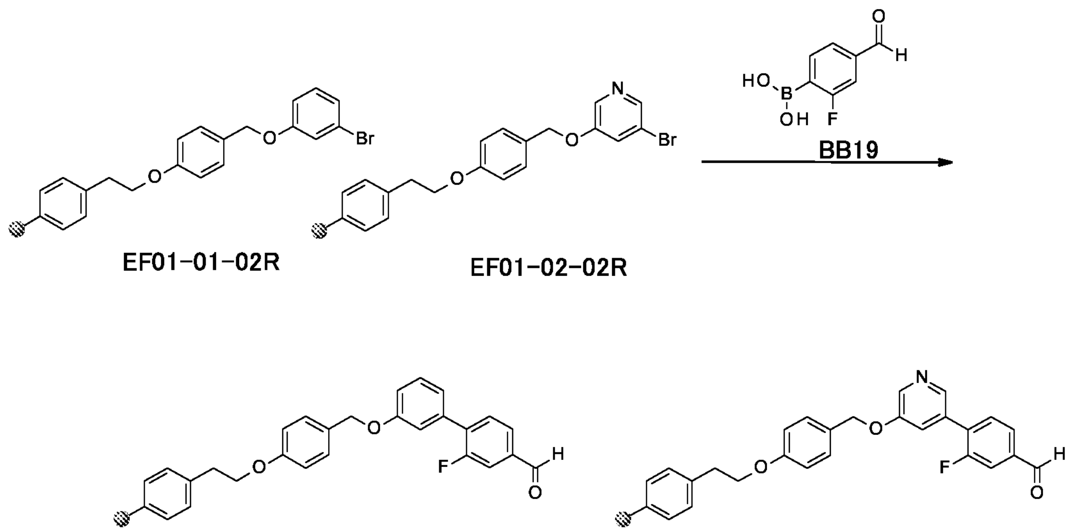

BB19

EF01-01-02R EF01-02-02R

EG19-01-02R EG19-02-02R

Under a nitrogen atmosphere, to a 4 mL glass vial were added compound EF01-01-02R (57 mg, loading rate 0.556 mmol/g, 0.032 mmol), compound EF01-02-02R (43 mg, loading rate 0.733 mmol/g, 0.032 mmol), and 1,4-dioxane (1.8 mL), and the mixture was shaken at room temperature for 1 hour. Under a nitrogen atmosphere, (2-fluoro-4-formylphenyl)boronic acid (BB19) (65 mg, 0.387 mmol), $K_3PO_4$-$H_2O$ (89 mg, 0.387 mmol), pinacol (45.7 mg, 0.387 mmol), and a solution of $P(Cy)_3Pd$ G3 in 1,4-dioxane (0.04M, 323 μL, 13 μmol) were added, and the mixture was shaken at 90°C for 22 hours. The suspension of the reaction solution and the resins was transferred to a syringe equipped with a filter using NMP, washed repeatedly three times with NMP (2 mL), three times with a solution (2 mL) of NMP containing NAC (10 mg/mL):water (1:1), three times with water (2 mL), three times with MeOH (2 mL), three times with DCM (2 mL), and three times with heptane (2 mL), and the resins were then dried under reduced pressure to obtain the title compounds shown in [Table EG19]. The building blocks used, the compounds contained in the obtained mixture, and the reaction time are shown in [Table EG19].

The 0.04M solution of $P(Cy)_3Pd$ G3 in 1,4-dioxane was prepared by adding 1,4-dioxane (1 mL) to $P(Cy)_3Pd$ G3 (26 mg, 0.04 mmol) in a 2 mL glass vial under a nitrogen atmosphere and shaking to dissolve the mixture.

Example 6-1-4-B: Synthesis of mixture mixEG20-02R

Using compound EF01-01-02R (57 mg, loading rate 0.556 mmol/g, 0.032 mmol), compound EF01-02-02R (43 mg, loading rate 0.733 mmol/g, 0.032 mmol), and methyl 3-fluoro-4-(4,4,5,5-tetramethyl-1,3,2-dioxaborolan-2-yl)benzoate (BB20) shown in [Table LBB02], the title compounds shown in [Table EG19] were synthesized by the same method as in Example 6-1-4-A. The building blocks used, the compounds contained in the obtained mixture, and the reaction time are shown in [Table EG19].

[Table 42]

[Table EG19]

| BB No. used | Synthesized mixture No. | Resin compound No. | Post-cleavage mixture No. | Post-cleavage compound No. | Compound name | Reaction time (hr) |
|---|---|---|---|---|---|---|
| BB19 | mixEG19-02R | EG19-01-02R | mixEG19 | EG19-01 | 3-Fluoro-4-(3-hydroxyphenyl)benzaldehyde | 22 |
| | | EG19-02-02R | | EG19-02 | 3-Fluoro-4-(5-hydroxypyridin-3-yl)benzaldehvde | |
| BB20 | mixEG20-02R | EG19-03-02R | mixEG20 | EG19-03 | Methyl 3-fluoro-4-(3-hydroxyphenyl)benzoate | 22 |
| | | EG19-04-02R | | EG19-04 | Methyl 3-fluoro-4-(5-hydroxypyridin-3-yl)benzoate | |

Example 6-1-5: Synthesis of mixture mixEG21-02R

[Formula 96]

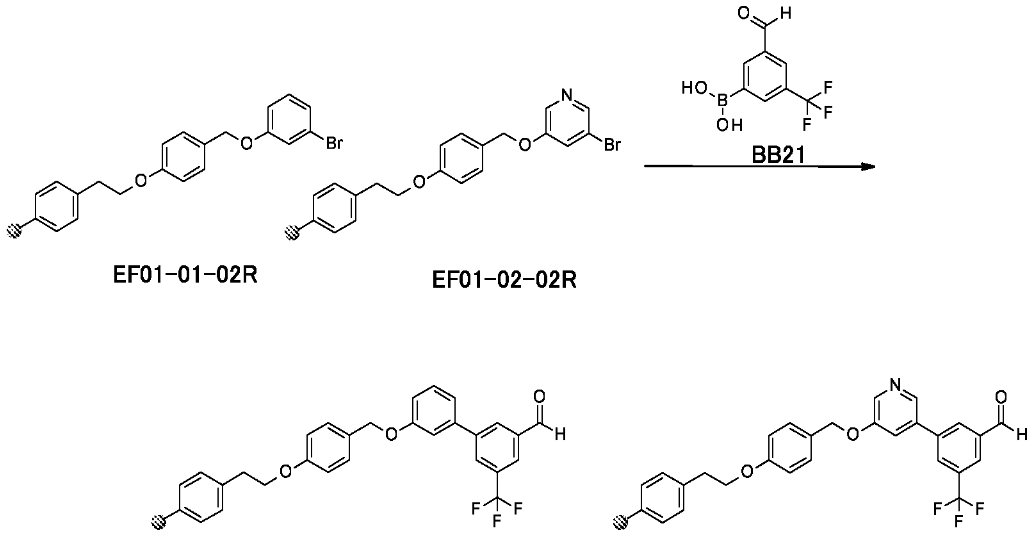


EF01-01-02R EF01-02-02R

EG21-01-02R EG21-02-02R

Under a nitrogen atmosphere, to a 4 mL glass vial were added compound EF01-01-02R (57 mg, loading rate 0.556 mmol/g, 0.032 mmol), compound EF01-02-02R (43 mg, loading rate 0.733 mmol/g, 0.032 mmol), and 4-MeTHP (1.8 mL), and the mixture was shaken at room temperature for 1 hour. Under a nitrogen atmosphere, [3-formyl-5-(trifluoromethyl)phenyl]boronic acid (BB21) (84 mg, 0.387 mmol), $Na_2CO_3$ (41 mg, 0.387 mmol), a 0.04M solution of $P(Cy)_3Pd$ G3 in 4-MeTHP (323 μL, 13 μmol), and water (75 μL, 4.16 mmol) were added, and the mixture was shaken at 90°C for 22 hours. The suspension of the reaction solution and the resins was transferred to a syringe equipped with a filter using NMP, washed repeatedly three times with NMP (2 mL), three times with a solution (2 mL) of NMP containing NAC (10 mg/mL):water (1:1), three times with water (2 mL), three times with MeOH (2 mL), three times with DCM (2 mL), and three times with heptane (2 mL), and the resins were then dried under reduced pressure to obtain the title mixture mixEG21-02R shown in [Table EG21]. The building blocks used, the compounds contained in the obtained mixture, and the reaction time are shown in [Table EG21].

The 0.04M solution of $P(Cy)_3Pd$ G3 in 4-MeTHP was prepared by adding 4-MeTHP (1 mL) to $P(Cy)_3Pd$ G3 (26 mg, 0.04 mmol) in a 2 mL glass vial under a nitrogen atmosphere and shaking to dissolve the mixture.

[Table 43]

[Table EG21]

| BB No. used | Synthesized mixture No. | Resin compound No. | Post-cleavage mixture No. | Post-cleavage compound No. | Compound name | Reaction time (hr) |
|---|---|---|---|---|---|---|
| BB21 | mixEG21-02R | EG21-01-02R | mixEG21 | EG21-01 | 3-(3-Hydroxyphenyl)-5-(trifluoromethyl)benzaldehyde | 22 |
| | | EG21-02-02R | | EG21-02 | 3-(5-Hydroxypyridin-3-yl)-5-(trifluoromethyl)benzaldehyde | |

Example 6-1-6: Synthesis of mixture mixEG22-02R

Using compound EF01-01-02R (57 mg, loading rate 0.556 mmol/g, 0.032 mmol), compound EF01-02-02R (43 mg, loading rate 0.733 mmol/g, 0.032 mmol), and the boronic acid reagent (BB22) shown in [Table LBB02], the title mixture mixEG22-02R shown in [Table EG22] was synthesized by the same method as in Example 6-1-5 and obtained. The building blocks used, the compounds contained in the obtained mixture, and the reaction time are shown in [Table EG22].

[Table 44]

[Table EG22]

| BB No. used | Synthesized mixture No. | Resin compound No. | Post-cleavage mixture No. | Post-cleavage compound No. | Compound name | Reaction time (hr) |
|---|---|---|---|---|---|---|
| BB22 | mixEG22-02R | EG21-03-02R | mixEG22 | EG21-03 | 3-(3-Hydroxyphenyl)-5-(trifluoromethoxy)benzaldehyde | 22 |
| | | EG21-04-02R | | EG21-04 | 3-(5-Hydroxypyridin-3-yl)-5-(trifluoromethoxy)benzaldehyde | |

Example 6-1-7-A: Synthesis of mixture mixEG23-02R

[Formula 97]

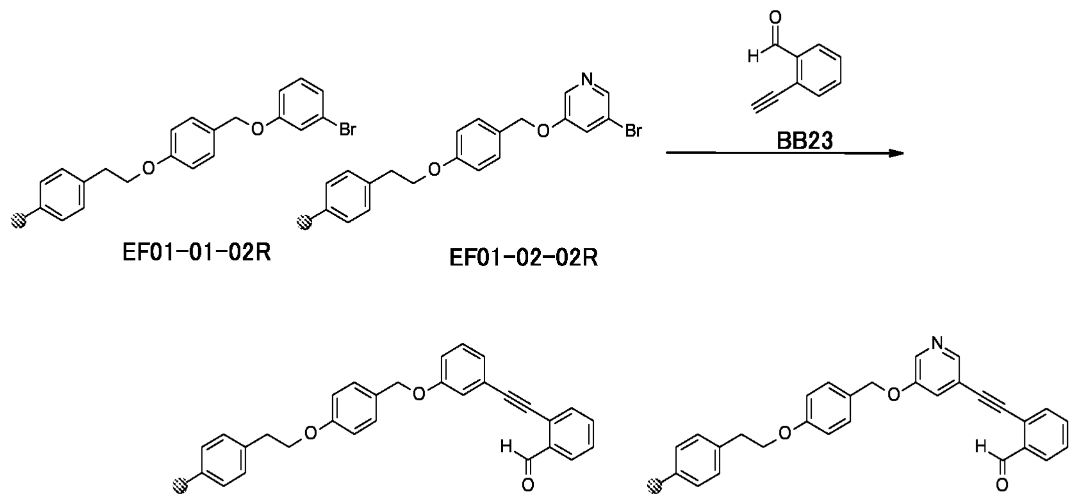


EF01-01-02R EF01-02-02R

EG23-01-02R EG23-02-02R

Under a nitrogen atmosphere, to a 3 mL glass vial were added compound EF01-01-02R (57 mg, loading rate 0.556 mmol/g, 0.032 mmol), compound EF01-02-02R (43 mg, loading rate 0.733 mmol/g, 0.032 mmol), NMP (1.11 mL), and diisopropylamine (188 μL), and the mixture was shaken at room temperature for 1 hour. Under a nitrogen atmosphere, 2-ethynylbenzaldehyde (BB23) (174 mg, 1.34 mmol) and a solution of XPhos Pd G4 (0.1 M, 200 μL, 20 μmol) in NMP were added, and the mixture was shaken at 80°C for 39 hours. The suspension of the reaction solution and the resins was transferred to a syringe equipped with a filter using NMP, washed repeatedly three times with NMP (2 mL), three times with a solution (2 mL) of NMP containing NAC (10 mg/mL):water (1:1), three times with water (2 mL), three times with MeOH (2 mL), three times with DCM (2 mL), and three times with heptane (2 mL), and the resins were then dried under reduced pressure to obtain the title compounds shown in [Table EG23]. The building blocks used and the compounds contained in the obtained mixture are shown in [Table EG23].

The 0.1M solution of XPhos Pd G4 in NMP was prepared by adding NMP (1 mL) to XPhos Pd G4 (86 mg, 0.1 mmol) in a 3 mL glass vial under a nitrogen atmosphere and shaking to dissolve the mixture.

Example 6-1-7-B: Synthesis of mixtures mixEG24-02R to mixEG26-02R

Using compound EF01-01-02R (57 mg, loading rate 0.556 mmol/g, 0.032 mmol), compound EF01-02-02R (43 mg, loading rate 0.733 mmol/g, 0.032 mmol), and the acetylene reagents (BB23 to 26) shown in [Table LBB02], the title mixtures shown in [Table EG23] were synthesized by the same method as in Example 6-1-7-A. The building blocks used, the compounds contained in the obtained mixture, and the reaction time are shown in [Table EG23].

[Table 45]

[Table EG23]

| BB No. used | Synthesized mixture No. | Resin compound No. | Post-cleavage mixture No. | Post-cleavage compound No. | Compound name | Reaction time (hr) |
|---|---|---|---|---|---|---|
| BB23 | mixEG23-02R | EG23-01-02R | mixEG23 | EG23-01 | 2-[2-(3-Hydroxyphenyl)ethynyl]benzaldehyde | 39 |
| | | EG23-02-02R | | EG23-02 | 2-[2-(5-Hydroxypyridin-3-yl)ethynyllbenzaldehyde | |
| BB24 | mixEG24-02R | EG23-03-02R | mixEG24 | EG23-03 | Methyl 2-((3-hydroxyphenyl)ethynyl)benzoate | 22 |
| | | EG23-04-02R | | EG23-04 | Methyl 2-((5-hydroxypyridin-3-yl)ethynyl)benzoate | |
| BB25 | mixEG25-02R | EG23-05-02R | mixEG25 | EG23-05 | Ethyl 3-[2-(3-hydroxyphenyl)ethynyl]-5-(trifluoromethyl)benzoate | 39 |
| | | EG23-06-02R | | EG23-06 | Ethyl 3-[2-(5-hydroxypyridin-3-yl)ethynyl]-5-(trifluoromethyl)benzoate | |
| BB26 | mixEG26-02R | EG23-07-02R | mixEG26 | EG23-07 | Ethyl 5-[2-(3-hydroxyphenyl)ethynyl]pyridine-3-carboxylate | 39 |
| | | EG23-08-02R | | EG23-08 | Ethyl 5-[2-(5-hydroxypyridin-3-yl)ethynyl]pyridine-3-carboxylate | |

Example 6-2-1: Synthesis of mixture mixEH01-02R

[Formula 98]

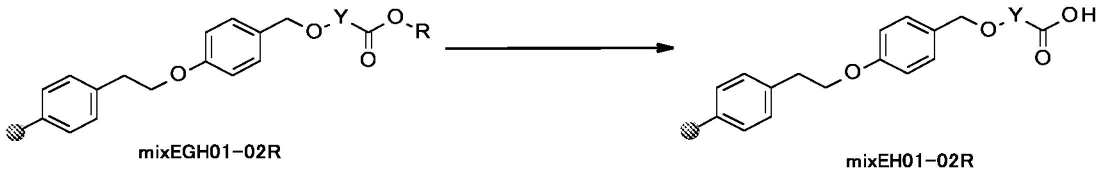

Under a nitrogen atmosphere, to a 10 mL glass vial were added the mixture mixEGH01-02R (220 mg, loading rate 0.651 mmol/g, 0.143 mmol) shown in [Table EGH01], NMP (5.3 mL), and methanol (1.3 mL), and the mixture was shaken at room temperature for 1 hour. A 5M aqueous sodium hydroxide solution (198 μL, 0.992 mmol) was added and the mixture was shaken at room temperature for 17 hours. The suspension of the reaction solution and the resins was transferred to a syringe equipped with a filter using NMP, washed repeatedly three times with NMP (4 mL), three times with water (4 mL), three times with NMP (4 mL), three times with a HOAt/NMP solution (0.2M, 4 mL), three times with NMP (4 mL), three times with MeOH (4 mL), three times with DCM (3 mL), and three times with heptane (4 mL), and the resins were then dried under reduced pressure to obtain the title mixture (mixEH01-02R) shown in [Table EH01]. The compounds contained in the obtained mixture are shown in [Table EH01].

[Table 46]

[Table EGH01]: Mixture mixEGH01-02R

| Mixture No. used | Amount of mixture used (mg) | Resin compound No. | Post-cleavage compound No. | Compound name |
|---|---|---|---|---|
| mixEG01-02R | 20.7 | EG01-01-02R | EG01-01 | Methyl 4-(3-hydroxyphenyl)-3-methylbenzoate |
| | | EG01-02-02R | EG01-02 | Methyl 4-(5-hydroxypyridin-3-yl)-3-methylbenzoate |
| mixEG01-03R | 20.0 | EG01-03-02R | EG01-03 | Ethyl 3-(3-hydroxyphenyl)-5-(trifluoromethyl)benzoate |
| | | EG01-04-02R | EG01-04 | Ethyl 3-(5-hydroxypyridin-3-yl)-5-(trifluoromethyl)benzoate |
| mixEG01-04R | 20.4 | EG01-05-02R | EG01-05 | Methyl 3-tert-butyl-5-(3-hydroxyphenyl)benzoate |
| | | EG01-06-02R | EG01-06 | Methyl 3-tert-butyl-5-(5-hydroxypyridin-3-yl)benzoate |
| mixEG01-05R | 20.3 | EG01-07-02R | EG01-07 | Ethyl 3-[2-(3-hydroxyphenyl)phenyl]propanoate |
| | | EG01-08-02R | EG01-08 | Ethyl 3-[2-(5-hydroxypyridin-3-yl)phenyl]propanoate |
| mixEG01-06R | 19.9 | EG01-09-02R | EG01-09 | Methyl 4-(3-hydroxyphenyl)naphthalene-1-carboxylate |
| | | EG01-10-02R | EG01-10 | Methyl 4-(5-hydroxypyridin-3-yl)naphthalene-1-carboxylate |

(continued)

| Mixture No. used | Amount of mixture used (mg) | Resin compound No. | Post-cleavage compound No. | Compound name |
|---|---|---|---|---|
| mixEG01-07R | 20.3 | EG01-11-02R | EG01-11 | Ethyl 3-[4-(3-hydroxyphenyl)pyrazol-1-yl]-5-(trifluoromethyl)benzoate |
| | | EG01-12-02R | EG01-12 | Ethyl 3-[4-(5-hydroxypyridin-3-yl)pyrazol-1-yl]-5-(trifluoromethyl)benzoate |
| mixEG12-02R | 20.0 | EG08-09-02R | EG08-09 | Methyl 4-(3-hydroxyphenyl)thiophene-2-carboxylate |
| | | EG08-10-02R | EG08-10 | Methyl 4-(5-hydroxypyridin-3-yl)thiophene-2-carboxylate |
| mixEG20-02R | 20.4 | EG19-03-02R | EG 19-03 | Methyl 3-fluoro-4-(3-hydroxyphenyl)benzoate |
| | | EG19-04-02R | EG 19-04 | Methyl 3-fluoro-4-(5-hydroxypyridin-3-yl)benzoate |
| mixEG24-02R | 19.5 | EG23-03-02R | EG23-03 | Methyl 2-((3-hydroxyphenyl)ethynyl)benzoate |
| | | EG23-04-02R | EG23-04 | Methyl 2-((5-hydroxypyridin-3-yl)ethynyl)benzoate |
| mixEG25-02R | 19.5 | EG23-05-02R | EG23-05 | Ethyl 3-[2-(3-hydroxyphenyl)ethynyl]-5-(trifluoromethyl)benzoate |
| | | EG23-06-02R | EG23-06 | Ethyl 3-[2-(5-hydroxypyridin-3-yl)ethynyl]-5-(trifluoromethyl)benzoate |
| mixEG26-02R | 19.4 | EG23-07-02R | EG23-07 | Ethyl 5-[2-(3-hydroxyphenyl)ethynyl]pyridine-3-carboxylate |
| | | EG23-08-02R | EG23-08 | Ethyl 5-[2-(5-hydroxypyridin-3-yl)ethynyl]pyridine-3-carboxylate |

[Table 47]

[Table EH01] Mixture No.: mixEH01-02R, post-cleavage mixture No.: mixEH01

| Resin compound No. | Post-cleavage compound No. | Compound name |
|---|---|---|
| EH01-01-02R | EH01-01 | 4-(3-Hydroxyphenyl)thiophene-2-carboxylic acid |
| EH01-02-02R | EH01-02 | 4-(5-Hydroxypyridin-3-yl)thiophene-2-carboxylic acid |
| EH01-03-02R | EH01-03 | 4-(3-Hydroxyphenyl)-3-methylbenzoic acid |
| EH01-04-02R | EH01-04 | 4-(5-Hydroxypyridin-3-yl)-3-methylbenzoic acid |
| EH01-05-02R | EH01-05 | 3-Fluoro-4-(3-hydroxyphenyl)benzoic acid |
| EH01-06-02R | EH01-06 | 3 -Fluoro-4-(5-hydroxypyridin-3 -yl)benzoic acid |
| EH01-07-02R | EH01-07 | 2-[2-(3-Hydroxyphenyl)ethynyl]benzoic acid |
| EH01-08-02R | EH01-08 | 2-[2-(5-Hydroxypyridin-3-yl)ethynyl]benzoic acid |
| EH01-09-02R | EH01-09 | 5-[2-(3-Hydroxyphenyl)ethynyl]pyridine-3-carboxylic acid |

(continued)

| Resin compound No. | Post-cleavage compound No. | Compound name |
|---|---|---|
| EH01-10-02R | EH01-10 | 5-[2-(5-Hydroxypyridin-3-yl)ethynyl]pyridine-3-carboxylic acid |
| EH01-11-02R | EH01-11 | 3-[2-(3-Hydroxyphenyl)phenyl]propanoic acid |
| EH01-12-02R | EH01-12 | 3-[2-(5-Hydroxypyridin-3-yl)phenyl]propanoic acid |
| EH01-13-02R | EH01-13 | 4-(3-Hydroxyphenyl)naphthalene-1-carboxylic acid |
| EH01-14-02R | EH01-14 | 4-(5-Hydroxypyridin-3-yl)naphthalene-1-carboxylic acid |
| EH01-15-02R | EH01-15 | 3-tert-Butyl-5-(3-hydroxyphenyl)benzoic acid |
| EH01-16-02R | EH01-16 | 3-tert-Butyl-5-(5-hydroxypyridin-3-yl)benzoic acid |
| EH01-17-02R | EH01-17 | 3-(3-Hydroxyphenyl)-5-(trifluoromethyl)benzoic acid |
| EH01-18-02R | EH01-18 | 3-(5-Hydroxypyridin-3-yl)-5-(trifluoromethyl)benzoic acid |
| EH01-19-02R | EH01-19 | 3 -[2-(3-Hydroxyphenyl)ethynyl]-5-(trifluoromethyl)benzoic acid |
| EH01-20-02R | EH01-20 | 3 -[2-(5-Hydroxypyridin-3-yl)ethynyl]-5-(trifluoromethyl)benzoic acid |
| EH01-21-02R | EH01-21 | 3-[4-(3-Hydroxyphenyl)pyrazol-1-yl]-5-(trifluoromethyl)benzoic acid |
| EH01-22-02R | EH01-22 | 3-[4-(5-Hydroxypyridin-3-yl)pyrazol-1-yl]-5-(trifluoromethyl)benzoic acid |

Example 6-2-2: Synthesis of mixture mixEH02-02R

[Formula 99]

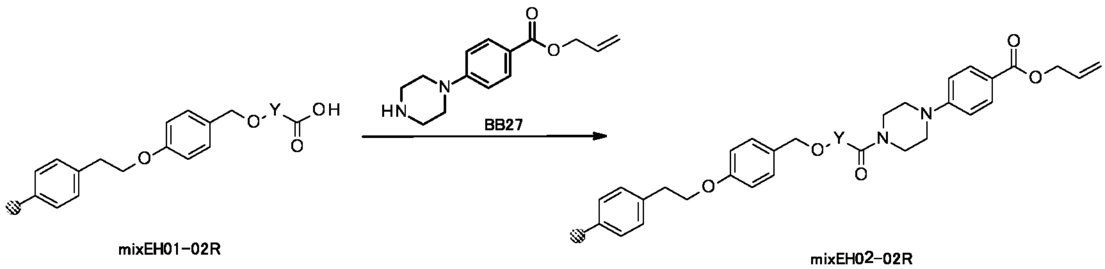


mixEH01-02R

mixEH02-02R

Under a nitrogen atmosphere, to an 8 mL glass vial were added the mixture mixEH01-02R (173 mg, loading rate 0.651 mmol/g, 0.113 mmol) shown in [Table EH01] and NMP (4.4 mL), and the mixture was shaken at room temperature for 1 hour. Allyl 4-(piperazin-1-yl)benzoate (BB27, 83 mg, 0.339 mmol), DIC (106 μL, 0.677 mmol), and HOAt (92 mg, 0.677 mmol) were added, and the mixture was shaken at 40 degrees for 20 hours. The suspension of the reaction solution and the resins was transferred to a syringe equipped with a filter using NMP, washed repeatedly three times with NMP (4 mL), three times with MeOH (4 mL), three times with DCM (4 mL), and three times with heptane (4 mL), and the resins were then dried under reduced pressure to obtain the title mixture shown in [Table EH02]. The compounds contained in the obtained mixture are shown in [Table EH02].

In this amidation reaction, two compounds (EH02-07-02R and EH02-08-02R) among the compounds listed in [Table EH02] may be missing.

[Table 48]

[Table EH02] Mixture No.: mixEH02-02R, post-cleavage mixture No.: mixEH02

| Resin compound No. | Post-cleavage compound No. | Compound name |
|---|---|---|
| EH02-01-02R | EH02-01 | Prop-2-enyl 4-[4-[4-(3-hydroxyphenyl)thiophene-2-carbonyl]piperazin-1-yl]benzoate |
| EH02-02-02R | EH02-02 | Prop-2-enyl 4-[4-[4-(5-hydroxypyridin-3-yl)thiophene-2-carbonyl]piperazin-1 -yl]benzoate |
| EH02-03-02R | EH02-03 | Prop-2-enyl 4-[4-[4-(3-hydroxyphenyl)-3-methylbenzoyl]piperazin-1-yl]benzoate |
| EH02-04-02R | EH02-04 | Prop-2-enyl 4-[4-[4-(5-hydroxypyridin-3-yl)-3-methylbenzoyl]piperazin-1-yl]benzoate |
| EH02-05-02R | EH02-05 | Prop-2-enyl 4-[4-[3-fluoro-4-(3-hydroxyphenyl)benzoyl]piperazin-1-yl]benzoate |
| EH02-06-02R | EH02-06 | Prop-2-enyl 4-[4-[3-fluoro-4-(5-hydroxypyridin-3-yl)benzoyl]piperazin-1-yl]benzoate |
| EH02-07-02R | EH02-07 | Prop-2-enyl 4-[4-[2-[2-(3-hydroxyphenyl)ethynyl]benzoyl]piperazin-1-yl]benzoate |
| EH02-08-02R | EH02-08 | Prop-2-enyl 4-[4-[2-[2-(5-hydroxypyridin-3-yl)ethynyl]benzoyl]piperazin-1-yl]benzoate |
| EH02-09-02R | EH02-09 | Prop-2-enyl 4-[4-[5-[2-(3-hydroxyphenyl)ethynyl]pyridine-3-carbonyl]piperazin-1-yl]benzoate |
| EH02-10-02R | EH02-10 | Prop-2-enyl 4-[4-[5-[2-(5-hydroxypyridin-3-yl)ethynyl]pyridine-3-carbonyl]piperazin-1 -yl]benzoate |
| EH02-11-02R | EH02-11 | Prop-2-enyl 4-[4-[3-[2-(3-hydroxyphenyl)phenyl]propanoyl]piperazin-1-yl]benzoate |
| EH02-12-02R | EH02-12 | Prop-2-enyl 4-[4-[3-[2-(5-hydroxypyridin-3-yl)phenyl]propanoyl]piperazin-1-yl]benzoate |
| EH02-13-02R | EH02-13 | Prop-2-enyl 4-[4-[4-(3-hydroxyphenyl)naphthalene-1-carbonyl]piperazin-1-yl]benzoate |
| EH02-14-02R | EH02-14 | Prop-2-enyl 4-[4-[4-(5-hydroxypyridin-3-yl)naphthalene-1-carbonyl]piperazin-1-yl]benzoate |
| EH02-15-02R | EH02-15 | Prop-2-enyl 4-[4-[3-tert-butyl-5-(3-hydroxyphenyl)benzoyl]piperazin-1-yl]benzoate |
| EH02-16-02R | EH02-16 | Prop-2-enyl 4-[4-[3-tert-butyl-5-(5-hydroxypyridin-3-yl)benzoyl]piperazin-1-yl]benzoate |
| EH02-17-02R | EH02-17 | Prop-2-enyl 4-[4-[3-(3-hydroxyphenyl)-5-(trifluoromethyl)benzoyl]piperazin-1-yl]benzoate |
| EH02-18-02R | EH02-18 | Prop-2-enyl 4-[4-[3-(5-hydroxypyridin-3-yl)-5-(trifluoromethyl)benzoyl]piperazin-1-yl]benzoate |
| EH02-19-02R | EH02-19 | Prop-2-enyl 4-[4-[3-[2-(3-hydroxyphenyl)ethynyl]-5-(trifluoromethyl)benzoyl]piperazin-1-yl]benzoate |
| EH02-20-02R | EH02-20 | Prop-2-enyl 4-[4-[3-[2-(5-hydroxypyridin-3-yl)ethynyl]-5-(trifluoromethyl)benzoyl]piperazin-1-yl]benzoate |
| EH02-21-02R | EH02-21 | Prop-2-enyl 4-[4-[3-[4-(3-hydroxyphenyl)pyrazol-1-yl]-5-(trifluoromethyl)benzoyl]piperazin-1-yl]benzoate |

(continued)

| Resin compound No. | Post-cleavage compound No. | Compound name |
|---|---|---|
| EH02-22-02R | EH02-22 | Prop-2-enyl 4-[4-[3-[4-(5-hydroxypyridin-3-yl)pyrazol-1-yl]-5-(trifluoromethyl)benzoyl]piperazin-1-yl]benzoate |

Example 6-2-3: Synthesis of mixture mixEH03-02R

[Formula 100]

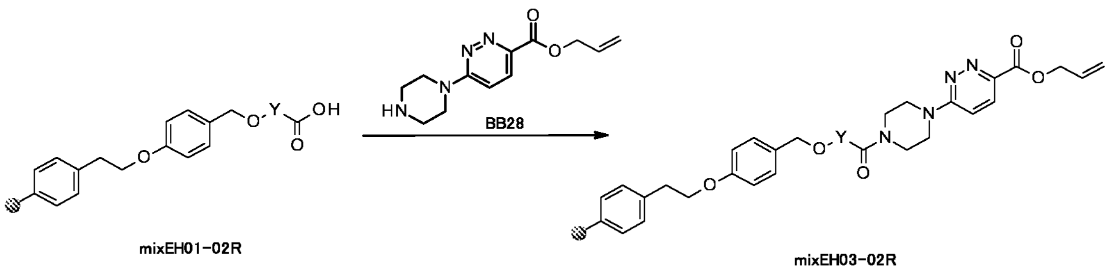


mixEH01-02R

mixEH03-02R

By the same method as in Example 6-2-2, the title mixture shown in [Table EH03] was obtained using allyl 6-(piperazin-1-yl)pyridazine-3-carboxylate (BB28) and the mixture mixEH01-02R (175 mg, loading rate 0.651 mmol/g, 0.114 mmol) shown in [Table EH01]. The compounds contained in the obtained mixture are shown in [Table EH03].

In this amidation reaction, two compounds (EH03-07-02R and EH03-08-02R) among the compounds listed in [Table EH03] may be missing.

[Table 49]

[Table EH03] Mixture No.: mixEH03-02R, post-cleavage mixture No.: mixEH03

| Resin compound No. | Post-cleavage compound No. | Compound name |
|---|---|---|
| EH03-01-02R | EH03-01 | Prop-2-enyl 6-[4-[4-(3-hydroxyphenyl)thiophene-2-carbonyl]piperazin-1-yl]pyridazine-3-carboxylate |
| EH03-02-02R | EH03-02 | Prop-2-enyl 6-[4-[4-(5-hydroxypyridin-3-yl)thiophene-2-carbonyl]piperazin-1-yl]pyridazine-3-carboxylate |
| EH03-03-02R | EH03-03 | Prop-2-enyl 6-[4-[4-(3-hydroxyphenyl)-3-methylbenzoyl]piperazin-1-yl]pyridazine-3 -carboxylate |
| EH03-04-02R | EH03-04 | Prop-2-enyl 6-[4-[4-(5-hydroxypyridin-3-yl)-3-methylbenzoyl]piperazin-1-yl]pyridazine-3-carboxylate |
| EH03-05-02R | EH03-05 | Prop-2-enyl 6-[4-[3-fluoro-4-(3-hydroxyphenyl)benzoyl]piperazin-l-yl]pyridazine-3 -carboxylate |
| EH03-06-02R | EH03-06 | Prop-2-enyl 6-[4-[3-fluoro-4-(5-hydroxypyridin-3-yl)benzoyl]piperazin-1-yl]pyridazine-3-carboxylate |
| EH03-07-02R | EH03-07 | Prop-2-enyl 6-[4-[2-[2-(3-hydroxyphenyl)ethynyl]benzoyl]piperazin-1-yl]pyridazine-3 -carboxylate |
| EH03-08-02R | EH03-08 | Prop-2-enyl 6-[4-[2-[2-(5-hydroxypyridin-3-yl)ethynyl]benzoyl]piperazin-1-yl]pyridazine-3-carboxylate |

(continued)

| Resin compound No. | Post-cleavage compound No. | Compound name |
|---|---|---|
| EH03-09-02R | EH03-09 | Prop-2-enyl 6-[4-[5-[2-(3-hydroxyphenyl)ethynyl]pyridine-3-carbonyl]piperazin-1-yl]pyridazine-3-carboxylate |
| EH03-10-02R | EH03-10 | Prop-2-enyl 6-[4-[5-[2-(5-hydroxypyridin-3-yl)ethynyl]pyridine-3-carbonyl]piperazin-1-yl]pyridazine-3-carboxylate |
| EH03-11-02R | EH03-11 | Prop-2-enyl 6-[4-[3-[2-(3-hydroxyphenyl)phenyl]propanoyl]piperazin-1-yl]pyridazine-3-carboxylate |
| EH03-12-02R | EH03-12 | Prop-2-enyl 6-[4-[3-[2-(5-hydroxypyridin-3-yl)phenyl]propanoyl]piperazin-1-yl]pyridazine-3-carboxylate |
| EH03-13-02R | EH03-13 | Prop-2-enyl 6-[4-[4-(3-hydroxyphenyl)naphthalene-1-carbonyl]piperazin-1-yl]pyridazine-3-carboxylate |
| EH03-14-02R | EH03-14 | Prop-2-enyl 6-[4-[4-(5-hydroxypyridin-3-yl)naphthalene-1-carbonyl]piperazin-1-yl]pyridazine-3-carboxylate |
| EH03-15-02R | EH03-15 | Prop-2-enyl 6-[4-[3-tert-butyl-5-(3-hydroxyphenyl)benzoyl]piperazin-1-yl]pyridazine-3-carboxylate |
| EH03-16-02R | EH03-16 | Prop-2-enyl 6-[4-[3-tert-butyl-5-(5-hydroxypyridin-3-yl)benzoyl]piperazin-1-yl]pyridazine-3-carboxylate |
| EH03-17-02R | EH03-17 | Prop-2-enyl 6-[4-[3-(3-hydroxyphenyl)-5-(trifluoromethyl)benzoyl]piperazin-1-yl]pyridazine-3-carboxylate |
| EH03-18-02R | EH03-18 | Prop-2-enyl 6-[4-[3-(5-hydroxypyridin-3-yl)-5-(trifluoromethyl)benzoyl]piperazin-1-yl]pyridazine-3-carboxylate |
| EH03-19-02R | EH03-19 | Prop-2-enyl 6-[4-[3-[2-(3-hydroxyphenyl)ethynyl]-5-(trifluoromethyl)benzoyl]piperazin-1-yl]pyridazine-3-carboxylate |
| EH03-20-02R | EH03-20 | Prop-2-enyl 6-[4-[3-[2-(5-hydroxypyridin-3-yl)ethynyl]-5-(trifluoromethyl)benzoyl]piperazin-1-yl]pyridazine-3-carboxylate |
| EH03-21-02R | EH03-21 | Prop-2-enyl 6-[4-[3-[4-(3-hydroxyphenyl)pyrazol-1-yl]-5-(trifluoromethyl)benzoyl]piperazin-1-yl]pyridazine-3-carboxylate |
| EH03-22-02R | EH03-22 | Prop-2-enyl 6-[4-[3-[4-(5-hydroxypyridin-3-yl)pyrazol-1-yl]-5-(trifluoromethyl)benzoyl]piperazin-1-yl]pyridazine-3-carboxylate |

Example 6-2-4: Synthesis of mixture mixEH04-02R

[Formula 101]

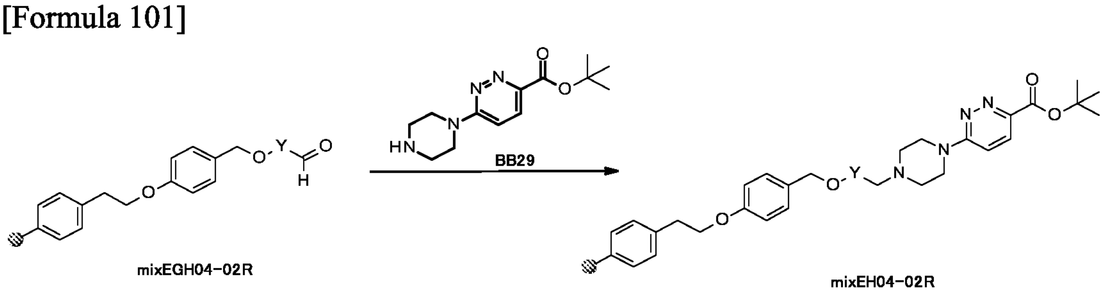


mixEGH04-02R

mixEH04-02R

Under a nitrogen atmosphere, mixEG07-02R (20.3 mg), mixEG08-02R (21.0 mg), mixEG09-02R (21.6 mg), mixEG10-02R (19.8 mg), mixEG13-02R (20.6 mg), mixEG14-02R (21.1 mg), mixEG 15-02R (20.5 mg), mixEG 16-02R (20.5 mg), mixEG17-02R (20.5 mg), mixEG19-02R (20.1 mg), mixEG21-02R (21.9 mg), mixEG22-02R (20.4 mg), and

mixEG23-02R (20.6 mg) shown in [Table EGH04] were transferred to a 10 mL screw cap vial to make a mixture of solid phase-supported aromatic aldehydes used in the present Example: mixEGH04-02R (265 mg, loading rate 0.651 mmol/g, 0.172 mmol). DCE (5.57 mL, 21 v/w) was added, and the mixture was shaken at room temperature for 1 hour. Under a nitrogen atmosphere, tert-butyl 6-piperazin-1-ylpyridazine-3-carboxylate (BB29) (137 mg) and $Ti(OtBu)_4$ (333 μL) were added, and the mixture was shaken at 60°C for 1 hour. Thereafter, under a nitrogen atmosphere, sodium triacetoxyborohydride (439 mg) and a 2 v/v% solution of AcOH in DCE (2.39 mL) were added, and the mixture was shaken at room temperature for 20 hours. After adding MeOH (1 mL) to the reaction solution, the suspension of resins was transferred to a syringe equipped with a filter using MeOH (1 mL) (repeated six times in total), washed repeatedly three times with MeOH (5.3 mL), three times with NMP (5.3 mL), three times with a $NaHCO_3$ solution (0.15M, NMP:$H_2O$ = 1:5 solution, 5.3 mL), three times with water (5.3 mL), three times with MeOH (5.3 mL), three times with DCM (5.3 mL), and three times with heptane (5.3 mL), and then dried under reduced pressure to obtain the title mixture mixEH04-02R. The compounds contained in the mixture are shown in [Table EH04].

[Table 50]

[Table EGH04] Mixture No.: mixEGH04-02R, post-cleavage mixture No.: mixEGH04

| Mixture No. used | Resin compound No. | Post-cleavage compound No. | Compound name |
|---|---|---|---|
| mixEG07-02R | EG07-0102R | EG07-01 | 4-(3-Hydroxyphenyl)-3-methylbenzaldehyde |
| | EG07-0202R | EG07-02 | 4-(5-Hydroxypyridin-3-yl)-3-methylbenzaldehyde |
| mixEG08-02R | EG07-0302R | EG07-03 | 2-(3-Hydroxyphenyl)benzaldehyde |
| | EG07-0402R | EG07-04 | 2-(5-Hydroxypyridin-3-yl)benzaldehyde |
| mixEG09-02R | EG07-0502R | EG07-05 | 4-(3-Hydroxyphenyl)naphthalene-1-carbaldehyde |
| | EG07-0602R | EG07-06 | 4-(5-Hydroxypyridin-3-yl)naphthalene-1-carbaldehyde |
| mixEG 10-02R | EG07-0702R | EG07-07 | 4-(3-Hydroxyphenyl)thiophene-2-carbaldehyde |
| | EG07-0802R | EG07-08 | 4-(5-Hydroxypyridin-3-yl)thiophene-2-carbaldehyde |
| mixEG13-02R | EG07-1302R | EG07-13 | 2-Ethyl-4-(3-hydroxyphenyl)benzaldehyde |
| | EG07-1402R | EG07-14 | 2-Ethyl-4-(5-hydroxypyridin-3-yl)benzaldehyde |
| mixEG14-02R | EG07-1502R | EG07-15 | 4-(3-Hydroxyphenyl)-2-methoxybenzaldehyde |
| | EG07-1602R | EG07-16 | 4-(5-Hydroxypyridin-3-yl)-2-methoxybenzaldehyde |
| mixEG15-02R | EG07-1702R | EG07-17 | 3-Ethoxy-5-(3-hydroxyphenyl)benzaldehyde |
| | EG07-1802R | EG07-18 | 3-Ethoxy-5-(5 -hydroxypyridin-3 -yl)benzaldehyde |
| mixEG16-02R | EG07-1902R | EG07-19 | 3-(3-Hydroxyphenyl)-5-propan-2-yloxybenzaldehyde |
| | EG07-2002R | EG07-20 | 3-(5-Hydroxypyridin-3-yl)-5-propan-2-yloxybenzaldehyde |
| mixEG17-02R | EG07-2102R | EG07-21 | 4-(3-Hydroxyphenyl)-2,6-dimethoxybenzaldehyde |
| | EG07-2202R | EG07-22 | 4-(5-Hydroxypyridin-3-yl)-2,6-dimethoxybenzaldehyde |
| mixEG19-02R | EG19-01-02R | EG19-01 | 3-Fluoro-4-(3-hydroxyphenyl)benzaldehyde |
| | EG19-02-02R | EG19-02 | 3-Fluoro-4-(5-hydroxypyridin-3-yl)benzaldehyde |
| mixEG21-02R | EG21-01-02R | EG21-01 | 3-(3-Hydroxyphenyl)-5-(trifluoromethyl)benzaldehyde |
| | EG21-02-02R | EG21-02 | 3-(5-Hydroxypyridin-3-yl)-5-(trifluoromethyl)benzaldehyde |
| mixEG22-02R | EG21-03-02R | EG21-03 | 3-(3-Hydroxyphenyl)-5-(trifluoromethoxy)benzaldehyde |

(continued)

| Mixture No. used | Resin compound No. | Post-cleavage compound No. | Compound name |
|---|---|---|---|
| | EG21-04-02R | EG21-04 | 3-(5-Hydroxypyridin-3-yl)-5-(trifluoromethoxy)benzaldehyde |
| mixEG23-02R | EG23-01-02R | EG23-01 | 2-[2-(3-Hydroxyphenyl)ethynyl]benzaldehyde |
| | EG23-02-02R | EG23-02 | 2-[2-(5-Hydroxypyridin-3-yl)ethynyl]benzaldehyde |

[Table 51]

[Table EH04] Mixture No.: mixEH04-02R, post-cleavage mixture No.: mixEH04

| Resin compound No. | Post-cleavage compound No. | Compound name |
|---|---|---|
| EH04-01-02R | EH04-01 | tert-Butyl 6-[4-[[2-(3-hydroxyphenyl)phenyl]methyl]piperazin-1-yl]pyridazine-3 -carboxylate |
| EH04-02-02R | EH04-02 | tert-Butyl 6-[4-[[2-(5-hydroxypyridin-3-yl)phenyl]methyl]piperazin-1-yl]pyridazine-3-carboxylate |
| EH04-03-02R | EH04-03 | tert-Butyl 6-[4-[[4-(3-hydroxyphenyl)-3-methylphenyl]methyl]piperazin-1-yl]pyridazine-3-carboxylate |
| EH04-04-02R | EH04-04 | tert-Butyl6-[4-[[4-(5-hydroxypyridin-3-yl)-3-methylphenyl]methyl]piperazin-1-yl]pyridazine-3-carboxylate |
| EH04-05-02R | EH04-05 | tert-Butyl6-[4-[[2-ethyl-4-(3-hydroxyphenyl)phenyl]methyl]piperazin-1-yl]pyridazine-3-carboxylate |
| EH04-06-02R | EH04-06 | tert-Butyl 6-[4-[[2-ethyl-4-(5-hydroxypyridin-3-yl)phenyl]methyl]piperazin-1-yl]pyridazine-3-carboxylate |
| EH04-07-02R | EH04-07 | tert-Butyl 6-[4-[[3-fluoro-4-(3-hydroxyphenyl)phenyl]methyl]piperazin-1-yl]pyridazine-3-carboxylate |
| EH04-08-02R | EH04-08 | tert-Butyl 6-[4-[[3-fluoro-4-(5-hydroxypyridin-3-yl)phenyl]methyl]piperazin-1-yl]pyridazine-3-carboxylate |
| EH04-09-02R | EH04-09 | tert-Butyl6-[4-[[4-(3-hydroxyphenyl)-2-methoxyphenyl]methyl]piperazin-1-yl]pyridazine-3-carboxylate |
| EH04-10-02R | EH04-10 | tert-Butyl 6-[4-[[4-(5-hydroxypyridin-3-yl)-2-methoxyphenyl]methyl]piperazin-1-yl]pyridazine-3-carboxylate |
| EH04-11-02R | EH04-11 | tert-Butyl 6-[4-[[3-ethoxy-5-(3-hydroxyphenyl)phenyl]methyl]piperazin-1-yl]pyridazine-3-carboxylate |
| EH04-12-02R | EH04-12 | tert-Butyl 6-[4-[[3-ethoxy-5-(5-hydroxypyridin-3-yl)phenyl]methyl]piperazin-1-yl]pyridazine-3-carboxylate |
| EH04-13-02R | EH04-13 | tert-Butyl 6-[4-[[3-(3-hydroxyphenyl)-5-propan-2-yloxyphenyl]methyl]piperazin-1-yl]pyridazine-3-carboxylate |
| EH04-14-02R | EH04-14 | tert-Butyl 6-[4-[[3-(5-hydroxypyridin-3-yl)-5-propan-2-yloxyphenyl]methyl]piperazin-1-yl]pyridazine-3-carboxylate |
| EH04-15-02R | EH04-15 | tert-Butyl 6-[4-[[4-(3-hydroxyphenyl)-2,6-dimethoxyphenyl]methyl]piperazin-1-yl]pyridazine-3-carboxylate |
| EH04-16-02R | EH04-16 | tert-Butyl 6-[4-[[4-(5-hydroxypyridin-3-yl)-2,6-dimethoxyphenyl]methyl]piperazin-1-yl]pyridazine-3-carboxylate |

(continued)

| Resin compound No. | Post-cleavage compound No. | Compound name |
|---|---|---|
| EH04-17-02R | EH04-17 | tert-Butyl 6-[4-[[3-(3-hydroxyphenyl)-5-(trifluoromethyl)phenyl]methyl]piperazin-1-yl]pyridazine-3-carboxylate |
| EH04-18-02R | EH04-18 | tert-Butyl6-[4-[[3-(5-hydroxypyridin-3-yl)-5-(trifluoromethyl)phenyl]methyl]piperazin-1-yl]pyridazine-3-carboxylate |
| EH04-19-02R | EH04-19 | tert-Butyl 6-[4-[[3-(3-hydroxyphenyl)-5-(trifluoromethoxy)phenyl]methyl]piperazin-1-yl]pyridazine-3- carboxylate |
| EH04-20-02R | EH04-20 | tert-Butyl 6-[4-[[3-(5-hydroxypyridin-3-yl)-5-(trifluoromethoxy)phenyl]methyl]piperazin-1-yl]pyridazine-3 - carboxylate |
| EH04-21-02R | EH04-21 | tert-Butyl 6-[4-[[4-(3-hydroxyphenyl)naphthalen-1-yl]methyl]piperazin-1-yl]pyridazine-3-carboxylate |
| EH04-22-02R | EH04-22 | tert-Butyl 6-[4-[[4-(5-hydroxypyridin-3-yl)naphthalen-1-yl]methyl]piperazin-1-yl]pyridazine-3-carboxylate |
| EH04-23-02R | EH04-23 | tert-Butyl 6-[4-[[4-(3-hydroxyphenyl)thiophen-2-yl]methyl]piperazin-1-yl]pyridazine-3-carboxylate |
| EH04-24-02R | EH04-24 | tert-Butyl 6-[4-[[4-(5-hydroxypyridin-3-yl)thiophen-2-yl]methyl]piperazin-1-yl]pyridazine-3-carboxylate |
| EH04-25-02R | EH04-25 | tert-Butyl 6-[4-[[2-[2-(3-hydroxyphenyl)ethynyl]phenyl]methyl]piperazin-1-yl]pyridazine-3-carboxylate |
| EH04-26-02R | EH04-26 | tert-Butyl 6-[4-[[2-[2-(5-hydroxypyridin-3-yl)ethynyl]phenyl]methyl]piperazin-1-yl]pyridazine-3-carboxylate |

Example 6-2-5: Synthesis of mixture mixEH05-02R

[Formula 102]

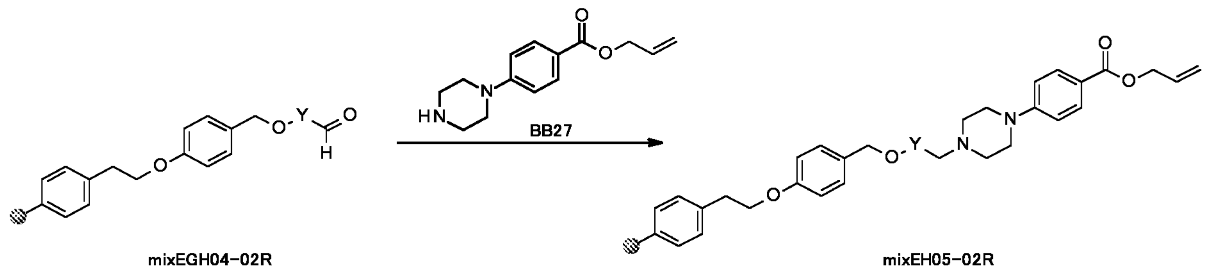


mixEGH04−02R mixEH05−02R

Under a nitrogen atmosphere, mixEG07-02R (20.1 mg), mixEG08-02R (20.3 mg), mixEG09-02R (20.4 mg), mixEG10-02R (20.3 mg), mixEG13-02R (20.8 mg), mixEGl4-02R (20.6 mg), mixEG15-02R (20.1 mg), mixEG16-02R (20.0 mg), mixEG17-02R (20.3 mg), mixEG19-02R (20.8 mg), mixEG21-02R (20.5 mg), mixEG22-02R (20.8 mg), and mixEG23-02R (20.6 mg) shown in [Table EGH04] were transferred to a 10 mL screw cap vial to make a mixture of solid phase-supported aromatic aldehydes used in the present Example: mixEGH04-02R (260 mg, loading rate 0.651 mmol/g). DCE (5.46 mL, 21 v/w) was added, and the mixture was shaken at room temperature for 1 hour. Under a nitrogen atmosphere, allyl 6-piperazin-1-ylpyridazine-3-carboxylate (BB27) (125 mg) and $Ti(OtBu)_4$ (317 μL) were added, and the mixture was shaken at 60°C for 1 hour. Thereafter, under a nitrogen atmosphere, sodium triacetoxyborohydride (430 mg) and a 2 v/v% solution of AcOH in DCE (2.34 mL) were added, and the mixture was shaken at 50°C for 3 hours. After cooling to room temperature, the suspension of resins was transferred to a column equipped with a filter using MeOH (1 mL) (repeated six times in total), washed repeatedly three times with MeOH (5.3 mL), three times with NMP (5.3 mL), three times with a 0.15M $NaHCO_3$ solution (NMP:$H_2O$ = 1:5 solution, 5.3 mL), three times with water (5.3 mL), three times with MeOH (5.3 mL), three times with DCM (5.3 mL), and three times with heptane (5.3 mL), and then dried

under reduced pressure to obtain the title mixture mixEH05-02R shown in [Table EH05]. The compounds contained in the mixture are shown in [Table EH05].

[Table 52]

[Table EH05] Mixture No.: mixEH05-02R, post-cleavage mixture No.: mixEH05

| Resin compound No. | Post-cleavage compound No. | Compound name |
|---|---|---|
| EH05-01-02R | EH05-01 | Prop-2-enyl 4-[4-[[2-(3-hydroxyphenyl)phenyl]methyl]piperazin-1-yl]benzoate |
| EH05-02-02R | EH05-02 | Prop-2-enyl 4-[4-[[2-(5-hydroxypyridin-3-yl)phenyl]methyl]piperazin-1-yl]benzoate |
| EH05-03-02R | EH05-03 | Prop-2-enyl 4-[4-[[4-(3-hydroxyphenyl)-3-methylphenyl]methyl]piperazin-1-yl]benzoate |
| EH05-04-02R | EH05-04 | Prop-2-enyl 4-[4-[[4-(5-hydroxypyridin-3-yl)-3-methylphenyl]methyl]piperazin-1-yl]benzoate |
| EH05-05-02R | EH05-05 | Prop-2-enyl 4-[4-[[2-ethyl-4-(3-hydroxyphenyl)phenyl]methyl]piperazin-1-yl]benzoate |
| EH05-06-02R | EH05-06 | Prop-2-enyl 4-[4-[[2-ethyl-4-(5-hydroxypyridin-3-yl)phenyl]methyl]piperazin-1-yl]benzoate |
| EH05-07-02R | EH05-07 | Prop-2-enyl 4-[4-[[3-fluoro-4-(3-hydroxyphenyl)phenyl]methyl]piperazin-1-yl]benzoate |
| EH05-08-02R | EH05-08 | Prop-2-enyl 4-[4-[[3-fluoro-4-(5-hydroxypyridin-3-yl)phenyl]methyl]piperazin-1-yl]benzoate |
| EH05-09-02R | EH05-09 | Prop-2-enyl 4-[4-[[4-(3-hydroxyphenyl)-2-methoxyphenyl]methyl]piperazin-1-yl]benzoate |
| EH05-10-02R | EH05-10 | Prop-2-enyl 4-[4-[[4-(5-hydroxypyridin-3-yl)-2-methoxyphenyl]methyl]piperazin-1-yl]benzoate |
| EH05-11-02R | EH05-11 | Prop-2-enyl 4-[4-[[3-ethoxy-5-(3-hydroxyphenyl)phenyl]methyl]piperazin-1-yl]benzoate |
| EH05-12-02R | EH05-12 | Prop-2-enyl 4-[4-[[3-ethoxy-5-(5-hydroxypyridin-3-yl)phenyl]methyl]piperazin-1-yl]benzoate |
| EH05-13-02R | EH05-13 | Prop-2-enyl 4-[4-[[3-(3-hydroxyphenyl)-5-propan-2-yloxyphenyl]methyl]piperazin-1-yl]benzoate |
| EH05-14-02R | EH05-14 | Prop-2-enyl 4-[4-[[3-(5-hydroxypyridin-3-yl)-5-propan-2-yloxyphenyl]methyl]piperazin-1-yl]benzoate |
| EH05-15-02R | EH05-15 | Prop-2-enyl 4-[4-[[4-(3-hydroxyphenyl)-2,6-dimethoxyphenyl]methyl]piperazin-1-yl]benzoate |
| EH05-16-02R | EH05-16 | Prop-2-enyl 4-[4-[[4-(5-hydroxypyridin-3-yl)-2,6-dimethoxyphenyl]methyl]piperazin-1-yl]benzoate |
| EH05-17-02R | EH05-17 | Prop-2-enyl 4-[4-[[3-(3-hydroxyphenyl)-5-(trifluoromethyl)phenyl]methyl]piperazin-1-yl]benzoate |
| EH05-18-02R | EH05-18 | Prop-2-enyl 4-[4-[[3-(5-hydroxypyridin-3-yl)-5-(trifluoromethyl)phenyl]methyl]piperazin-1-yl]benzoate |
| EH05-19-02R | EH05-19 | Prop-2-enyl 4-[4-[[3-(3-hydroxyphenyl)-5-(trifluoromethoxy)phenyl]methyl]piperazin-1-yl]benzoate |

(continued)

| Resin compound No. | Post-cleavage compound No. | Compound name |
|---|---|---|
| EH05-20-02R | EH05-20 | Prop-2-enyl 4-[4-[[3-(5-hydroxypyridin-3-yl)-5-(trifluoromethoxy)phenyl]methyl]piperazin-1-yl]benzoate |
| EH05-21-02R | EH05-21 | Prop-2-enyl 4-[4-[[4-(3-hydroxyphenyl)naphthalen-1-yl]methyl]piperazin-1-yl]benzoate |
| EH05-22-02R | EH05-22 | Prop-2-enyl 4-[4-[[4-(5-hydroxypyridin-3-yl)naphthalen-1-yl]methyl]piperazin-1-yl]benzoate |
| EH05-23-02R | EH05-23 | Prop-2-enyl 4-[4-[[4-(3-hydroxyphenyl)thiophen-2-yl]methyl]piperazin-1-yl]benzoate |
| EH05-24-02R | EH05-24 | Prop-2-enyl 4-[4-[[4-(5-hydroxypyridin-3-yl)thiophen-2-yl]methyl]piperazin-1-yl]benzoate |
| EH05-25-02R | EH05-25 | Prop-2-enyl 4-[4-[[2-[2-(3-hydroxyphenyl)ethynyl]phenyl]methyl]piperazin-1-yl]benzoate |
| EH05-26-02R | EH05-26 | Prop-2-enyl 4-[4-[[2-[2-(5-hydroxypyridin-3-yl)ethynyl]phenyl]methyl]piperazin-1-yl]benzoate |

Example 6-2-6: Synthesis of mixture mixEH06-02R

[Formula 103]

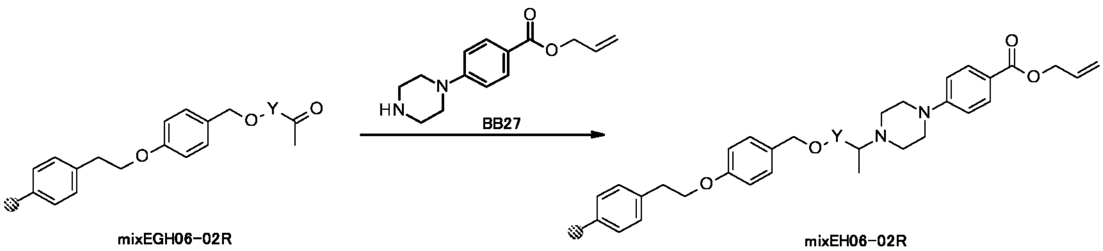


mixEGH06-02R

mixEH06-02R

Under a nitrogen atmosphere, to a 5 mL glass vial were added the mixture mixEGH06-02R (60 mg, loading rate 0.645 mmol/g, 0.039 mmol) shown in [Table EGH06] and anisole (2.25 mL), and the mixture was shaken at room temperature for 1 hour. Under a nitrogen atmosphere, allyl 4-(piperazin-1-yl)benzoate (BB27, 114 mg, 0.464 mmol) and $Ti(OtBu)_4$ (299 μL, 0.774 mmol) were added, and the mixture was shaken at 140°C for 20 hours. After cooling the reaction solution to room temperature, sodium triacetoxyborohydride (246 mg, 1.16 mmol) was added under a nitrogen atmosphere, and the mixture was shaken at 140°C for 20 hours. The reaction container was cooled to room temperature and MeOH (1.2 mL) was added to the reaction solution. The obtained suspension of reaction solution and resins were transferred to a syringe equipped with a filter using MeOH, washed repeatedly three times with NMP (1.2 mL), three times with a Rochelle salt solution (0.05M, NMP:water = 1:1 solution, 1.2 mL), three times with water (1.2 mL), three times with a semisaturated $NaHCO_3$ solution (1.2 mL), three times with water (1.2 mL), 69 times with NMP (1.2 mL), three times with MeOH (1.2 mL), three times with DCM (1.2 mL), and three times with heptane (1.2 mL), and then dried under reduced pressure to obtain the title compound shown in [Table EH06].

The compounds contained in the obtained mixture are shown in [Table EH06].

[Table 53]

[Table EGH06] Mixture No.: mixEGH06-02R, post-cleavage mixture No.: mixEGH06

| Mixture No. used | Amount of mixture used (mg) | Resin compound No. | Post-cleavage compound No. | Compound name |
|---|---|---|---|---|
| mixEG13-02R | 30 | EG07-13-02R | EG07-13 | 4-[4-(4-Acetyl-3-fluorophenyl)phenyl]-1-hydroxy-N,N-dimethylnaphthalene-2-carboxamide |
| | | EG07-14-02R | EG07-14 | 4-[5-(4-Acetyl-3-fluorophenyl)pyridin-2-yl]-1-hydroxy-N,N-dimethylnaphthalene-2-carboxamide |
| mixEG18-02R | 30 | EG01-13-02R | EG01-13 | 4-[4-(4-Acetylphenyl)phenyl]-1-hydroxy-N,N-dimethylnaphthalene-2-carboxamide |
| | | EG01-14-02R | EG01-14 | 4-[5-(4-Acetylphenyl)pyridin-2-yl]-1-hydroxy-N,N-dimethylnaphthalene-2-carboxamide |

[Table 54]

[Table EH06] Mixture No.: mixEH06-02R, post-cleavage mixture No.: mixEH06

| Resin compound No. | Post-cleavage compound No. | Compound name |
|---|---|---|
| EH06-01-02R | EH06-01 | Prop-2-enyl 4-[4-[1-[2-fluoro-4-(3-hydroxyphenyl)phenyl]ethyl]piperazin-1-yl]benzoate |
| EH06-02-02R | EH06-02 | Prop-2-enyl 4-[4-[1-[2-fluoro-4-(5-hydroxypyridin-3-yl)phenyl]ethyl]piperazin-1-yl]benzoate |
| EH06-03-02R | EH06-03 | Prop-2-enyl 4-[4-[1-[4-(3-hydroxyphenyl)phenyl]ethyl]piperazin-1-yl]benzoate |
| EH06-04-02R | EH06-04 | Prop-2-enyl 4-[4-[1-[4-(5-hydroxypyridin-3-yl)phenyl]ethyl]piperazin-1-yl]benzoate |

Example 6-3-1: Synthesis of mixture mixEI01-02R

[Formula 104]

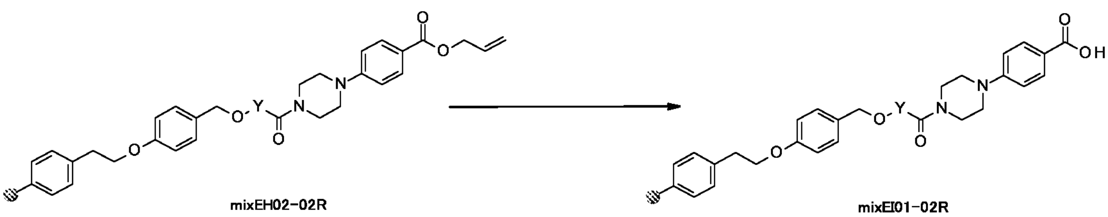

mixEH02-02R

mixEI01-02R

Under a nitrogen atmosphere, to an 8 mL screw cap vial were added the mixture mixEH02-02R (174 mg, loading rate 0.651 mmol/g, 0.113 mmol) shown in [Table EH02] and THF (5.2 mL), and the mixture was shaken at room temperature for 1 hour. Under a nitrogen atmosphere, tetraxtriphenylphosphine palladium(0) (39.3 mg, 34 μmol) and morpholine (49.4 μL, 0.566 mmol) were added, and the mixture was shaken at room temperature for 2 hours and 45 minutes. The suspension of resins was transferred to a column equipped with a filter using NMP, washed repeatedly three times with NMP (4 mL), three times with MeOH (4 mL), three times with a NAC solution (0.3M, NMP:water = 5:1

solution, 4 mL), three times with a solution of malonic acid in NMP (0.05M, 4 mL), three times with a solution of HOAt in NMP (0.2M, 4 mL), three times with NMP (4 mL), three times with MeOH (4 mL), three times with DCM (4 mL), and three times with heptane (4 mL), and then dried under reduced pressure to obtain the title mixture. The compounds contained in the obtained mixture are shown in [Table EI01].

[Table 55]

[Table EI01] Mixture No.: mixEI01-02R, post-cleavage mixture No.: mixEI01

| Resin compound No. | Post-cleavage compound No. | Compound name |
|---|---|---|
| EI01-01-02R | EI01-01 | 4-[4-[4-(3-Hydroxyphenyl)thiophene-2-carbonyl]piperazin-1-yl]benzoic acid |
| EI01-02-02R | EI01-02 | 4-[4-[4-(5-Hydroxypyridin-3-yl)thiophene-2-carbonyl]piperazin-1-yl]benzoic acid |
| EI01-03-02R | EI01-03 | 4-[4-[4-(3-Hydroxyphenyl)-3-methylbenzoyl]piperazin-1-yl]benzoic acid |
| EI01-04-02R | EI01-04 | 4-[4-[4-(5-Hydroxypyridin-3-yl)-3-methylbenzoyl]piperazin-1-yl]benzoic acid |
| EI01-05-02R | EI01-05 | 4-[4-[3-Fluoro-4-(3-hydroxyphenyl)benzoyl]piperazin-1-yl]benzoic acid |
| EI01-06-02R | EI01-06 | 4-[4-[3-Fluoro-4-(5-hydroxypyridin-3 -yl)benzoyl]piperazin-1- yl]benzoic acid |
| EI01-07-02R | EI01-07 | 4-[4-[2-[2-(3-Hydroxyphenyl)ethynyl]benzoyl]piperazin-1-yl]benzoic acid |
| EI01-08-02R | EI01-08 | 4-[4-[2-[2-(5-Hydroxypyridin-3-yl)ethynyl]benzoyl]piperazin-1-yl]benzoic acid |
| EI01-09-02R | EI01-09 | 4-[4-[5-[2-(3-Hydroxyphenyl)ethynyl]pyridine-3-carbonyl]piperazin-1-yl]benzoic acid |
| EI01-10-02R | EI01-10 | 4-[4-[5-[2-(5-Hydroxypyridin-3-yl)ethynyl]pyridine-3-carbonyl]piperazin-1-yl]benzoic acid |
| EI01-11-02R | EI01-11 | 4-[4-[3-[2-(3 -Hydroxyphenyl)phenyl]propanoyl]piperazin-1 -yl]benzoic acid |
| EI01-12-02R | EI01-12 | 4-[4-[3-[2-(5-Hydroxypyridin-3-yl)phenyl]propanoyl]piperazin-1-yl]benzoic acid |
| EI01-13-02R | EI01-13 | 4-[4-[4-(3-Hydroxyphenyl)naphthalene-1-carbonyl]piperazin-1-yl]benzoic acid |
| EI01-14-02R | EI01-14 | 4-[4-[4-(5-Hydroxypyridin-3-yl)naphthalene-1-carbonyl]piperazin-1-yl]benzoic acid |
| EI01-15-02R | EI01-15 | 4-[4-[3-tert-Butyl-5-(3-hydroxyphenyl)benzoyl]piperazin-1-yl]benzoic acid |
| EI01-16-02R | EI01-16 | 4-[4-[3-tert-Butyl-5-(5-hydroxypyridin-3-yl)benzoyl]piperazin-1-yl]benzoic acid |
| EI01-17-02R | EI01-17 | 4-[4-[3-(3-Hydroxyphenyl)-5-(trifluoromethyl)benzoyl]piperazin-1-yl]benzoic acid |
| EI01-18-02R | EI01-18 | 4-[4-[3-(5-Hydroxypyridin-3-yl)-5-(trifluoromethyl)benzoyl]piperazin-1-yl]benzoic acid |
| EI01-19-02R | EI01-19 | 4-[4-[3-[2-(3-Hydroxyphenyl)ethynyl]-5-(trifluoromethyl)benzoyl]piperazin-1-yl]benzoic acid |

(continued)

| Resin compound No. | Post-cleavage compound No. | Compound name |
|---|---|---|
| EI01-20-02R | EI01-20 | 4-[4-[3-[2-(5-Hydroxypyridin-3-yl)ethynyl]-5-(trifluoromethyl)benzoyl]piperazin-1-yl]benzoic acid |
| EI01-21-02R | EI01-21 | 4-[4-[3 -[4-(3-Hydroxyphenyl)pyrazol-1-yl]-5-(trifluoromethyl)benzoyl]piperazin-1-yl]benzoic acid |
| EI01-22-02R | EI01-22 | 4-[4-[3-[4-(5-Hydroxypyridin-3-yl)pyrazol-1-yl]-5-(trifluoromethyl)benzoyl]piperazin-1-yl]benzoic acid |

Example 6-3-2: Synthesis of mixture mixEI02-02R

[Formula 105]

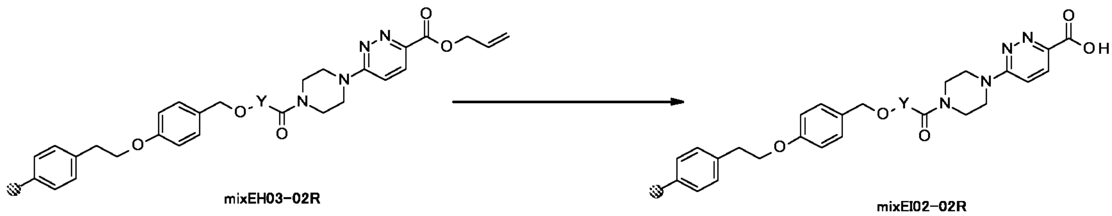

Under a nitrogen atmosphere, to an 8 mL glass vial were added the mixture mixEH03-02R (174 mg, loading rate 0.651 mmol/g, 0.113 mmol) shown in [Table EH03] and THF (5.2 mL), and the mixture was shaken at room temperature for 1 hour. Under a nitrogen atmosphere, $Pd(PPh_3)_4$ (6.6 mg, 5.66 μmol) and morpholine (34.5 μL, 0.397 mmol) were added, and the mixture was shaken at room temperature for 2 hours and 45 minutes. The suspension of resins was transferred to a column equipped with a filter using NMP, washed repeatedly three times with NMP (4 mL), three times with MeOH (4 mL), three times with a NAC solution (0.3M, NMP:water = 5:1 solution, 4 mL), three times with a solution of malonic acid in NMP (0.05M, 4 mL), three times with a solution of HOAt in NMP (0.2M, 4 mL), three times with NMP (4 mL), three times with MeOH (4 mL), three times with DCM (4 mL), and three times with heptane (4 mL), and then dried under reduced pressure to obtain the title mixture shown in [Table EI02]. The compounds contained in the obtained mixture are shown in [Table EI02].

[Table 56]

[Table EI02] Mixture No. mixEI02-02R, post-cleavage mixture No. mixEI02

| Resin compound No. | Post-cleavage compound No. | Compound name |
|---|---|---|
| EI02-01-02R | EI02-01 | 6-[4-[4-(3-Hydroxyphenyl)thiophene-2-carbonyl]piperazin-1-yl]pyridazine-3-carboxylic acid |
| EI02-02-02R | EI02-02 | 6-[4-[4-(5-Hydroxypyridin-3-yl)thiophene-2-carbonyl]piperazin-1-yl]pyridazine-3-carboxylic acid |
| EI02-03-02R | EI02-03 | 6-[4-[4-(3-Hydroxyphenyl)-3-methylbenzoyl]piperazin-1-yl]pyridazine-3-carboxylic acid |
| EI02-04-02R | EI02-04 | 6-[4-[4-(5-Hydroxypyridin-3-yl)-3-methylbenzoyl]piperazin-1-yl]pyridazine-3-carboxylic acid |

(continued)

| Resin compound No. | Post-cleavage compound No. | Compound name |
|---|---|---|
| EI02-05-02R | EI02-05 | 6-[4-[3-Fluoro-4-(3-hydroxyphenyl)benzoyl]piperazin-1-yl]pyridazine-3-carboxylic acid |
| EI02-06-02R | EI02-06 | 6-[4-[3-Fluoro-4-(5-hydroxypyridin-3 -yl)benzoyl]piperazin-1- yl]pyridazine-3-carboxylic acid |
| EI02-07-02R | EI02-07 | 6-[4-[2-[2-(3-Hydroxyphenyl)ethynyl]benzoyl]piperazin-1-yl]pyridazine-3-carboxylic acid |
| EI02-08-02R | EI02-08 | 6-[4-[2-[2-(5-Hydroxypyridin-3-yl)ethynyl]benzoyl]piperazin-1-yl]pyridazine-3-carboxylic acid |
| EI02-09-02R | EI02-09 | 6-[4-[5-[2-(3-Hydroxyphenyl)ethynyl]pyridine-3-carbonyl]piperazin-1-yl]pyridazine-3-carboxylic acid |
| EI02-10-02R | EI02-10 | 6-[4-[5-[2-(5-Hydroxypyridin-3-yl)ethynyl]pyridine-3-carbonyl]piperazin-1-yl]pyridazine-3-carboxylic acid |
| EI02-11-02R | EI02-11 | 6-[4-[3 -[2-(3 -Hydroxyphenyl)phenyl]propanoyl]piperazin-1- yl]pyridazine-3-carboxylic acid |
| EI02-12-02R | EI02-12 | 6-[4-[3-[2-(5-Hydroxypyridin-3-yl)phenyl]propanoyl]piperazin-1-yl]pyridazine-3-carboxylic acid |
| EI02-13-02R | EI02-13 | 6-[4-[4-(3-Hydroxyphenyl)naphthalene-1-carbonyl]piperazin-1-yl]pyridazine-3-carboxylic acid |
| EI02-14-02R | EI02-14 | 6-[4-[4-(5-Hydroxypyridin-3-yl)naphthalene-1-carbonyl]piperazin-1-yl]pyridazine-3-carboxylic acid |
| EI02-15-02R | EI02-15 | 6-[4-[3-tert-Butyl-5-(3-hydroxyphenyl)benzoyl]piperazin-1-yl]pyridazine-3-carboxylic acid |
| EI02-16-02R | EI02-16 | 6-[4-[3-tert-Butyl-5-(5-hydroxypyridin-3-yl)benzoyl]piperazin-1-yl]pyridazine-3-carboxylic acid |
| EI02-17-02R | EI02-17 | 6-[4-[3-(3-Hydroxyphenyl)-5-(trifluoromethyl)benzoyl]piperazin-1-yl]pyridazine-3-carboxylic acid |
| EI02-18-02R | EI02-18 | 6-[4-[3-(5-Hydroxypyridin-3-yl)-5-(trifluoromethyl)benzoyl]piperazin-1-yl]pyridazine-3-carboxylic acid |
| EI02-19-02R | EI02-19 | 6-[4-[3-[2-(3-Hydroxyphenyl)ethynyl]-5-(trifluoromethyl)benzoyl]piperazin-1-yl]pyridazine-3-carboxylic acid |
| EI02-20-02R | EI02-20 | 6-[4-[3-[2-(5-Hydroxypyridin-3-yl)ethynyl]-5-(trifluoromethyl)benzoyl]piperazin-1-yl]pyridazine-3-carboxylic acid |
| EI02-21-02R | EI02-21 | 6-[4-[3-[4-(3-Hydroxyphenyl)pyrazol-1-yl]-5-(trifluoromethyl)benzoyl]piperazin-1-yl]pyridazine-3-carboxylic acid |
| EI02-22-02R | EI02-22 | 6-[4-[3-[4-(5-Hydroxypyridin-3-yl)pyrazol-1-yl]-5-(trifluoromethyl)benzoyl]piperazin-1-yl]pyridazine-3-carboxylic acid |

Example 6-3-3: Synthesis of mixture mixEI03-02R

[Formula 106]

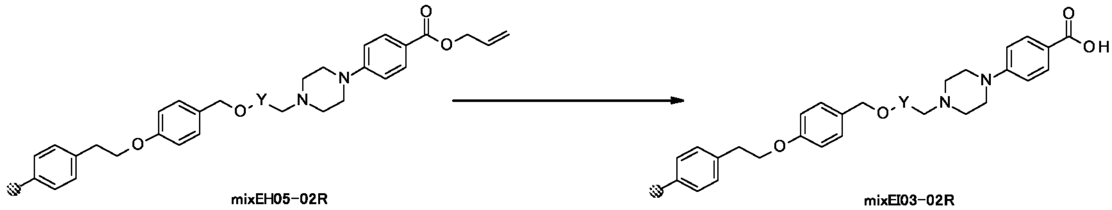

mixEH05-02R mixEI03-02R

Under a nitrogen atmosphere, to a 10 mL screw cap vial were added the mixture mixEH05-02R (260 mg, loading rate 0.651 mmol/g, 0.169 mmol) shown in [Table EH05] and THF (7.8 mL), and the mixture was shaken at room temperature for 1 hour. Under a nitrogen atmosphere, tetraxtriphenylphosphine palladium(0) (58.7 mg, 51 μmol) and morpholine (73.7 μL, 0.846 mmol) were added, and the mixture was shaken at room temperature for 2 hours and 45 minutes. The suspension of resins was transferred to a column equipped with a filter using NMP, washed repeatedly three times with NMP (6 mL), three times with MeOH (6 mL), three times with a NAC solution (0.3M, NMP:water = 5:1 solution, 6 mL), three times with a solution of malonic acid in NMP (0.05M, 6 mL), three times with a solution of HOAt in NMP (0.2M, 6 mL), three times with NMP (6 mL), three times with MeOH (6 mL), three times with DCM (6 mL), and three times with heptane (6 mL), and then dried under reduced pressure to obtain the title mixture. The compounds contained in the obtained mixture are shown in [Table EI03].

[Table 57]

[Table EI03] Mixture No.: mixEI03-02R, post-cleavage mixture No.: mixEI02

| Resin compound No. | Post-cleavage compound No. | Compound name |
|---|---|---|
| EI03-01-02R | E103-01 | 4-[4-[[2-(3-Hydroxyphenyl)phenyl]methyl]piperazin-1-yl]benzoic acid |
| EI03-02-02R | EI03-02 | 4-[4-[[2-(5-Hydroxypyridin-3-yl)phenyl]methyl]piperazin-1-yl]benzoic acid |
| EI03-03-02R | EI03-03 | 4-[4-[[4-(3 -Hydroxyphenyl)-3 -methylphenyl]methyl]piperazin-1 - yl]benzoic acid |
| EI03-04-02R | EI03-04 | 4-[4-[[4-(5-Hydroxypyridin-3-yl)-3-methylphenyl]methyl]piperazin-1-yl]benzoic acid |
| EI03-05-02R | EI03-05 | 4-[4-[[2-Ethyl-4-(3-hydroxyphenyl)phenyl]methyl]piperazin-1-yl]benzoic acid |
| EI03-06-02R | EI03-06 | 4-[4-[[2-Ethyl-4-(5-hydroxypyridin-3-yl)phenyl]methyl]piperazin-1-yl]benzoic acid |
| EI03-07-02R | EI03-07 | 4-[4-[[3-Fluoro-4-(3-hydroxyphenyl)phenyl]methyl]piperazin-1-yl]benzoic acid |
| EI03-08-02R | EI03-08 | 4-[4-[[3-Fluoro-4-(5-hydroxypyridin-3-yl)phenyl]methyl]piperazin-1-yl]benzoic acid |
| EI03-09-02R | EI03-09 | 4-[4-[[4-(3-Hydroxyphenyl)-2-methoxyphenyl]methyl]piperazin-1-yl]benzoic acid |
| EI03-10-02R | EI03-10 | 4-[4-[[4-(5-Hydroxypyridin-3-yl)-2-methoxyphenyl]methyl]piperazin-1-yl]benzoic acid |
| EI03-11-02R | EI03-11 | 4-[4-[[3 -Ethoxy-5 -(3 -hydroxyphenyl)phenyl]methyl]piperazin-1 - yl]benzoic acid |
| EI03-12-02R | EI03-12 | 4-[4-[[3-Ethoxy-5-(5-hydroxypyridin-3-yl)phenyl]methyl]piperazin-1-yl]benzoic acid |
| EI03-13-02R | EI03-13 | 4-[4-[[3-(3-Hydroxyphenyl)-5-propan-2-yloxyphenyl]methyl]piperazin-1-yl]benzoic acid |

(continued)

| Resin compound No. | Post-cleavage compound No. | Compound name |
|---|---|---|
| EI03-14-02R | EI03-14 | 4-[4-[[3-(5-Hydroxypyridin-3-yl)-5-propan-2-yloxyphenyl]methyl]piperazin-1-yl]benzoic acid |
| EI03-15-02R | EI03-15 | 4-[4-[[4-(3-Hydroxyphenyl)-2,6-dimethoxyphenyl]methyl]piperazin-1-yl]benzoic acid |
| EI03-16-02R | EI03-16 | 4-[4-[[4-(5-Hydroxypyridin-3-yl)-2,6-dimethoxyphenyl]methyl]piperazin-1-yl]benzoic acid |
| EI03-17-02R | EI03-17 | 4-[4-[[3-(3-Hydroxyphenyl)-5-(trifluoromethyl)phenyl]methyl]piperazin-1-yl]benzoic acid |
| EI03-18-02R | EI03-18 | 4-[4-[[3-(5-Hydroxypyridin-3-yl)-5-(trifluoromethyl)phenyl]methyl]piperazin-1-yl]benzoic acid |
| EI03-19-02R | EI03-19 | 4-[4-[[3-(3-Hydroxyphenyl)-5-(trifluoromethoxy)phenyl]methyl]piperazin-1-yl]benzoic acid |
| EI03-20-02R | EI03-20 | 4-[4-[[3-(5-Hydroxypyridin-3-yl)-5-(trifluoromethoxy)phenyl]methyl]piperazin-1-yl]benzoic acid |
| EI03-21-02R | EI03-21 | 4-[4-[[4-(3-Hydroxyphenyl)naphthalen-1-yl]methyl]piperazin-1-yl]benzoic acid |
| EI03-22-02R | EI03-22 | 4-[4-[[4-(5-Hydroxypyridin-3-yl)naphthalen-1-yl]methyl]piperazin-1-yl]benzoic acid |
| EI03-23-02R | EI03-23 | 4-[4-[[4-(3-Hydroxyphenyl)thiophen-2-yl]methyl]piperazin-1-yl]benzoic acid |
| EI03-24-02R | EI03-24 | 4-[4-[[4-(5-Hydroxypyridin-3-yl)thiophen-2-yl]methyl]piperazin-1-yl]benzoic acid |
| EI03-25-02R | EI03-25 | 4-[4-[[2-[2-(3-Hydroxyphenyl)ethynyl]phenyl]methyl]piperazin-1-yl]benzoic acid |
| EI03-26-02R | EI03-26 | 4-[4-[[2-[2-(5-Hydroxypyridin-3-yl)ethynyl]phenyl]methyl]piperazin-1-yl]benzoic acid |

Example 6-3-4: Synthesis of mixture mixEI04-02R

[Formula 107]

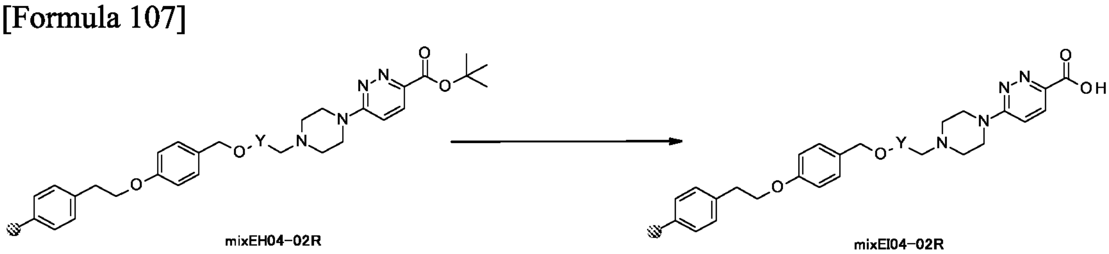

mixEH04-02R

mixEI04-02R

Under a nitrogen atmosphere, to an 8.0 mL screw cap vial were added the mixture mixEH04-02R (300 mg, loading rate 0.651 mmol/g) shown in [Table EH04], THF (8.07 mL, 27 v/w), and MeOH (807 μL, 2.7 v/w), and the mixture was shaken at room temperature for 1 hour. Under a nitrogen atmosphere, an aqueous LiOH solution (2M, 781 μL) was added, and the mixture was shaken at room temperature for 8 hours. The suspension of resins was transferred to a column equipped with a filter using MeOH, washed repeatedly three times with NMP (6.0 mL), three times with $H_2O$ (6.0 mL), three times with NMP (6.0 mL), three times with a solution of HOAt in NMP (0.2M, 6.0 mL), three times with NMP (6.0 mL), three times with MeOH (6.0 mL), three times with DCM (6.0 mL), and three times with heptane (6.0 mL), and then dried under reduced pressure to obtain the title mixture mixEI04-02R. The compounds contained in the obtained mixture are shown in [Table EI04].

[Table 58]

[Table EI04] Mixture No.: mixEI04-02R, post-cleavage mixture No.: mixEI04

| Resin compound No. | Post-cleavage compound No. | Compound name |
|---|---|---|
| EI04-01-02R | EI04-01 | 6-[4-[[2-(3-Hydroxyphenyl)phenyl]methyl]piperazin-1-yl]pyridazine-3-carboxylic acid |
| EI04-02-02R | EI04-02 | 6-[4-[[2-(5-Hydroxypyridin-3-yl)phenyl]methyl]piperazin-1-yl]pyridazine-3-carboxylic acid |
| EI04-03-02R | EI04-03 | 6-[4-[[4-(3 -Hydroxyphenyl)-3 -methylphenyl]methyl]piperazin-1 -yl]pyridazine-3-carboxylic acid |
| EI04-04-02R | EI04-04 | 6-[4-[[4-(5-Hydroxypyridin-3-yl)-3-methylphenyl]methyl]piperazin-1-yl]pyridazine-3-carboxylic acid |
| EI04-05-02R | EI04-05 | 6-[4-[[2-Ethyl-4-(3-hydroxyphenyl)phenyl]methyl]piperazin-1-yl]pyridazine-3-carboxylic acid |
| EI04-06-02R | EI04-06 | 6-[4-[[2-Ethyl-4-(5-hydroxypyridin-3-yl)phenyl]methyl]piperazin-1-yl]pyridazine-3-carboxylic acid |
| EI04-07-02R | EI04-07 | 6-[4-[[3-Fluoro-4-(3-hydroxyphenyl)phenyl]methyl]piperazin-1-yl]pyridazine-3-carboxylic acid |
| EI04-08-02R | EI04-08 | 6-[4-[[3-Fluoro-4-(5-hydroxypyridin-3-yl)phenyl]methyl]piperazin-1-yl]pyridazine-3-carboxylic acid |
| EI04-09-02R | EI04-09 | 6-[4-[[4-(3-Hydroxyphenyl)-2-methoxyphenyl]methyl]piperazin-1-yl]pyridazine-3-carboxylic acid |
| EI04-10-02R | EI04-10 | 6-[4-[[4-(5-Hydroxypyridin-3-yl)-2-methoxyphenyl]methyl]piperazin-1-yl]pyridazine-3-carboxylic acid |
| EI04-11-02R | EI04-11 | 6-[4-[[3 -Ethoxy-5 -(3 -hydroxyphenyl)phenyl]methyl]piperazin-1 -yl]pyridazine-3-carboxylic acid |
| EI04-12-02R | EI04-12 | 6-[4-[[3-Ethoxy-5-(5-hydroxypyridin-3-yl)phenyl]methyl]piperazin-1-yl]pyridazine-3-carboxylic acid |
| EI04-13-02R | EI04-13 | 6-[4-[[3-(3-Hydroxyphenyl)-5-propan-2-yloxyphenyl]methyl]piperazin-1-yl]pyridazine-3-carboxylic acid |
| EI04-14-02R | EI04-14 | tert-Butyl 6-[4-[[3-(5-hydroxypyridin-3-yl)-5-propan-2-yloxyphenyl]methyl]piperazin-1-yl]pyridazine-3-carboxylate |
| EI04-15-02R | EI04-15 | 6-[4-[[4-(3-Hydroxyphenyl)-2,6-dimethoxyphenyl]methyl]piperazin-1-yl]pyridazine-3-carboxylic acid |
| EI04-16-02R | EI04-16 | 6-[4-[[4-(5-Hydroxypyridin-3-yl)-2,6-dimethoxyphenyl]methyl]piperazin-1-yl]pyridazine-3-carboxylic acid |
| EI04-17-02R | EI04-17 | 6-[4-[[3-(3-Hydroxyphenyl)-5-(trifluoromethyl)phenyl]methyl]piperazin-1-yl]pyridazine-3-carboxylic acid |
| EI04-18-02R | EI04-18 | 6-[4-[[3-(5-Hydroxypyridin-3-yl)-5-(trifluoromethyl)phenyl]methyl]piperazin-1-yl]pyridazine-3 -carboxylic acid |
| EI04-19-02R | EI04-19 | 6-[4-[[3-(3-Hydroxyphenyl)-5-(trifluoromethoxy)phenyl]methyl]piperazin-1-yl]pyridazine-3 - carboxylic acid |
| EI04-20-02R | EI04-20 | 6-[4-[[3-(5-Hydroxypyridin-3-yl)-5-(trifluoromethoxy)phenyl]methyl]piperazin-1-yl]pyridazine-3 - carboxylic acid |

(continued)

| Resin compound No. | Post-cleavage compound No. | Compound name |
|---|---|---|
| EI04-21-02R | EI04-21 | 6-[4-[[4-(3-Hydroxyphenyl)naphthalen-1-yl]methyl]piperazin-1-yl]pyridazine-3-carboxylic acid |
| EI04-22-02R | EI04-22 | 6-[4-[[4-(5-Hydroxypyridin-3-yl)naphthalen-1-yl]methyl]piperazin-1-yl]pyridazine-3-carboxylic acid |
| EI04-23-02R | EI04-23 | 6-[4-[[4-(3-Hydroxyphenyl)thiophen-2-yl]methyl]piperazin-1-yl]pyridazine-3-carboxylic acid |
| EI04-24-02R | EI04-24 | 6-[4-[[4-(5-Hydroxypyridin-3-yl)thiophen-2-yl]methyl]piperazin-1-yl]pyridazine-3-carboxylic acid |
| EI04-25-02R | EI04-25 | 6-[4-[[2-[2-(3-Hydroxyphenyl)ethynyl]phenyl]methyl]piperazin-1-yl]pyridazine-3-carboxylic acid |
| EI04-26-02R | EI04-26 | 6-[4-[[2-[2-(5-Hydroxypyridin-3-yl)ethynyl]phenyl]methyl]piperazin-1-yl]pyridazine-3-carboxylic acid |

Example 6-3-5: Synthesis of mixture mixEI05-02R

[Formula 108]

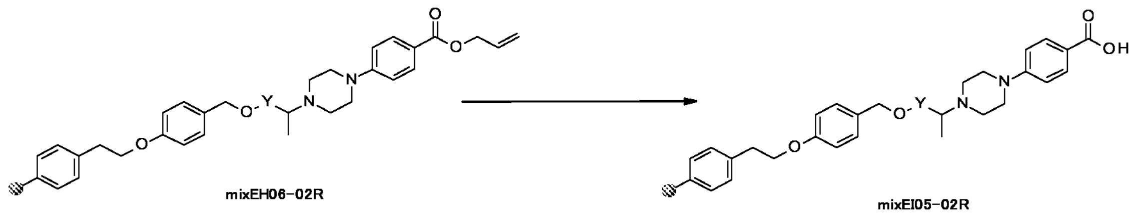

Under a nitrogen atmosphere, to a 2 mL screw cap vial were added the mixture mixEH06-02R (50 mg, loading rate 0.645 mmol/g, 32 μmol) shown in [Table EH06] and THF (1.5 mL), and the mixture was shaken at room temperature for 1 hour. Under a nitrogen atmosphere, tetraxtriphenylphosphine palladium(0) (11.2 mg, 9.68 μmol) and morpholine (14.1 μL, 0.161 mmol) were added, and the mixture was shaken at room temperature for 3 hours. The suspension of resins was transferred to a column equipped with a filter using NMP, washed repeatedly three times with NMP (1 mL), three times with MeOH (1 mL), three times with a NAC solution (0.3M, NMP:water = 5:1 solution, 1 mL), three times with a solution of malonic acid in NMP (0.05M, 1 mL), three times with a solution of HOAt in NMP (0.2M, 1 mL), three times with NMP (1 mL), three times with MeOH (1 mL), three times with DCM (1 mL), and three times with heptane (1 mL), and then dried under reduced pressure to obtain the title mixture. The names of compounds contained in the obtained mixture are shown in [Table EI05].

[Table 59]

[Table EI05] Mixture No.: mixEI05-02R, post-cleavage mixture No.: mixEI05

| Resin compound No. | Post-cleavage compound No. | Compound name |
|---|---|---|
| EI05-01-02R | EI05-01 | 4-[4-[1-[2-Fluoro-4-(3-hydroxyphenyl)phenyl]ethyl]piperazin-1-yl]benzoic acid |

(continued)

| Resin compound No. | Post-cleavage compound No. | Compound name |
|---|---|---|
| EI05-02-02R | EI05-02 | 4-[4-[1-[2-Fluoro-4-(5-hydroxypyridin-3-yl)phenyl]ethyl]piperazin-1-yl]benzoic acid |
| EI05-03-02R | EI05-03 | 4-[4-[1-[4-(3-Hydroxyphenyl)phenyl]ethyl]piperazin-1-yl]benzoic acid |
| EI05-04-02R | EI05-04 | 4-[4-[1-[4-(5-Hydroxypyridin-3-yl)phenyl]ethyl]piperazin-1 -yl]benzoic acid |

Example 6-3-6: Synthesis of mixture mixEI06-02R

[Formula 109]

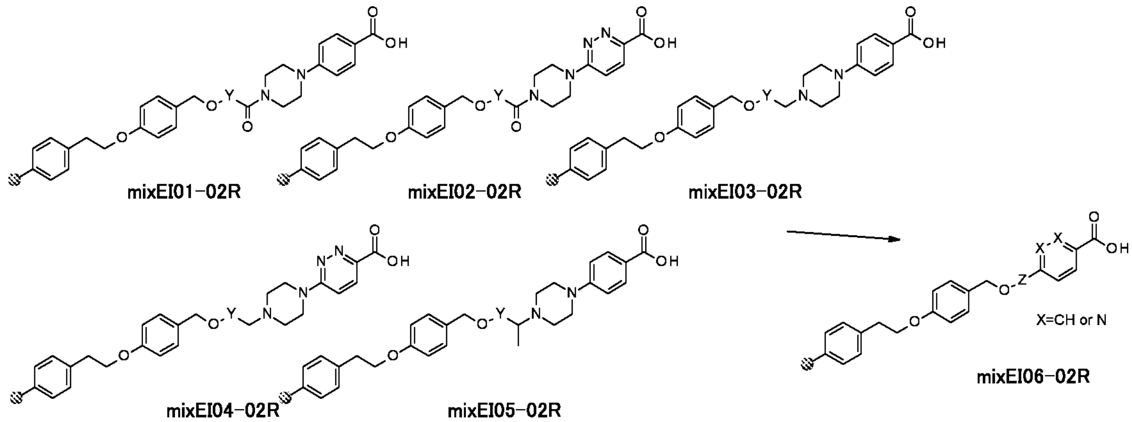


mixEI01-02R mixEI02-02R mixEI03-02R mixEI04-02R mixEI05-02R mixEI06-02R

The mixtures mixEI01-02R (155.9 mg, loading rate 0.651 mmol/g, 0.101 mmol), mixEI02-02R (153.5 mg, loading rate 0.651 mmol/g, 99.9 μmol), mixEI03-02R (182.5 mg, loading rate 0.651 mmol/g, 0.119 mmol), mixEI04-02R (182.9 mg, loading rate 0.651 mmol/g, 0.119 mmol), mixEI05-02R (28.6 mg, loading rate 0.645 mmol/g, 18.4 μmol) shown in [Table EI01], [Table EI02], [Table EI03], [Table EI04], and [Table EI05] were transferred to a column equipped with a filter, washed repeatedly three times with NMP (14 mL), three times with MeOH (14 mL), three times with DCM (14 mL), and three times with heptane (14 mL), and then dried under reduced pressure to obtain the title mixture mixEI06-02R shown in [Table EI06] (703 mg, loading rate 0.648 mmol/g, 0.456 mmol).

[Table 60]

[Table EI06] Mixture No.: mixEI06-02R, post-cleavage mixture No.: mixEI06

| Resin compound No. | Post-cleavage compound No. | Compound name |
|---|---|---|
| EI01-01-02R | EI01-01 | 4-[4-[4-(3-Hydroxyphenyl)thiophene-2-carbonyl]piperazin-1-yl]benzoic acid |
| EI01-02-02R | EI01-02 | 4-[4-[4-(5-Hydroxypyridin-3-yl)thiophene-2-carbonyl]piperazin-1-yl]benzoic acid |
| EI01-03-02R | EI01-03 | 4-[4-[4-(3-Hydroxyphenyl)-3-methylbenzoyl]piperazin-1-yl]benzoic acid |
| EI01-04-02R | EI01-04 | 4-[4-[4-(5-Hydroxypyridin-3-yl)-3-methylbenzoyl]piperazin-1-yl]benzoic acid |

(continued)

| Resin compound No. | Post-cleavage compound No. | Compound name |
|---|---|---|
| EI01-05-02R | EI01-05 | 4-[4-[3-Fluoro-4-(3-hydroxyphenyl)benzoyl]piperazin-1-yl]benzoic acid |
| EI01-06-02R | EI01-06 | 4-[4-[3-Fluoro-4-(5-hydroxypyridin-3-yl)benzoyl]piperazin-1- yl]benzoic acid |
| EI01-07-02R | EI01-07 | 4-[4-[2-[2-(3-Hydroxyphenyl)ethynyl]benzoyl]piperazin-1-yl]benzoic acid |
| EI01-08-02R | EI01-08 | 4-[4-[2-[2-(5-Hydroxypyridin-3-yl)ethynyl]benzoyl]piperazin-1-yl]benzoic acid |
| EI01-09-02R | EI01-09 | 4-[4-[5-[2-(3-Hydroxyphenyl)ethynyl]pyridine-3-carbonyl]piperazin-1-yl]benzoic acid |
| EI01-10-02R | EI01-10 | 4-[4-[5-[2-(5-Hydroxypyridin-3-yl)ethynyl]pyridine-3-carbonyl]piperazin-1-yl]benzoic acid |
| EI01-11-02R | EI01-11 | 4-[4-[3-[2-(3-Hydroxyphenyl)phenyl]propanoyl]piperazin-1-yl]benzoic acid |
| EI01-12-02R | EI01-12 | 4-[4-[3-[2-(5-Hydroxypyridin-3-yl)phenyl]propanoyl]piperazin-1-yl]benzoic acid |
| EI01-13-02R | EI01-13 | 4-[4-[4-(3-Hydroxyphenyl)naphthalene-1-carbonyl]piperazin-1-yl]benzoic acid |
| EI01-14-02R | EI01-14 | 4-[4-[4-(5-Hydroxypyridin-3-yl)naphthalene-1-carbonyl]piperazin-1-yl]benzoic acid |
| EI01-15-02R | EI01-15 | 4-[4-[3-tert-Butyl-5-(3-hydroxyphenyl)benzoyl]piperazin-1-yl]benzoic acid |
| EI01-16-02R | EI01-16 | 4-[4-[3-tert-Butyl-5-(5-hydroxypyridin-3-yl)benzoyl]piperazin-1-yl]benzoic acid |
| EI01-17-02R | EI01-17 | 4-[4-[3-(3-Hydroxyphenyl)-5-(trifluoromethyl)benzoyl]piperazin-1-yl]benzoic acid |
| EI01-18-02R | EI01-18 | 4-[4-[3-(5-Hydroxypyridin-3-yl)-5-(trifluoromethyl)benzoyl]piperazin-1-yl]benzoic acid |
| EI01-19-02R | EI01-19 | 4-[4-[3-[2-(3-Hydroxyphenyl)ethynyl]-5-(trifluoromethyl)benzoyl]piperazin-1-yl]benzoic acid |
| EI01-20-02R | EI01-20 | 4-[4-[3-[2-(5-Hydroxypyridin-3-yl)ethynyl]-5-(trifluoromethyl)benzoyl]piperazin-1-yl]benzoic acid |
| EI01-21-02R | EI01-21 | 4-[4-[3-[4-(3-Hydroxyphenyl)pyrazol-1-yl]-5-(trifluoromethyl)benzoyl]piperazin-1-yl]benzoic acid |
| EI01-22-02R | EI01-22 | 4-[4-[3-[4-(5-Hydroxypyridin-3-yl)pyrazol-1-yl]-5-(trifluoromethyl)benzoyl]piperazin-1-yl]benzoic acid |
| EI02-01-02R | EI02-01 | 6-[4-[4-(3-Hydroxyphenyl)thiophene-2-carbonyl]piperazin-1-yl]pyridazine-3-carboxylic acid |
| EI02-02-02R | EI02-02 | 6-[4-[4-(5-Hydroxypyridin-3-yl)thiophene-2-carbonyl]piperazin-1-yl]pyridazine-3-carboxylic acid |
| EI02-03-02R | EI02-03 | 6-[4-[4-(3-Hydroxyphenyl)-3-methylbenzoyl]piperazin-1-yl]pyridazine-3-carboxylic acid |
| EI02-04-02R | EI02-04 | 6-[4-[4-(5-Hydroxypyridin-3-yl)-3-methylbenzoyl]piperazin-1-yl]pyridazine-3-carboxylic acid |
| EI02-05-02R | EI02-05 | 6-[4-[3-Fluoro-4-(3-hydroxyphenyl)benzoyl]piperazin-1-yl]pyridazine-3-carboxylic acid |
| EI02-06-02R | EI02-06 | 6-[4-[3-Fluoro-4-(5-hydroxypyridin-3-yl)benzoyl]piperazin-1- yl]pyridazine-3-carboxylic acid |

(continued)

| Resin compound No. | Post-cleavage compound No. | Compound name |
|---|---|---|
| EI02-07-02R | EI02-07 | 6-[4-[2-[2-(3-Hydroxyphenyl)ethynyl]benzoyl]piperazin-1-yl]pyridazine-3-carboxylic acid |
| EI02-08-02R | EI02-08 | 6-[4-[2-[2-(5-Hydroxypyridin-3-yl)ethynyl]benzoyl]piperazin-1-yl]pyridazine-3-carboxylic acid |
| EI02-09-02R | EI02-09 | 6-[4-[5-[2-(3-Hydroxyphenyl)ethynyl]pyridine-3-carbonyl]piperazin-1-yl]pyridazine-3-carboxylic acid |
| EI02-10-02R | EI02-10 | 6-[4-[5-[2-(5-Hydroxypyridin-3-yl)ethynyl]pyridine-3-carbonyl]piperazin-1-yl]pyridazine-3-carboxylic acid |
| EI02-11-02R | EI02-11 | 6-[4-[3-[2-(3-Hydroxyphenyl)phenyl]propanoyl]piperazin-1- yl]pyridazine-3-carboxylic acid |
| EI02-12-02R | EI02-12 | 6-[4-[3-[2-(5-Hydroxypyridin-3-yl)phenyl]propanoyl]piperazin-1-yl]pyridazine-3-carboxylic acid |
| EI02-13-02R | EI02-13 | 6-[4-[4-(3-Hydroxyphenyl)naphthalene-1-carbonyl]piperazin-1-yl]pyridazine-3-carboxylic acid |
| EI02-14-02R | EI02-14 | 6-[4-[4-(5-Hydroxypyridin-3-yl)naphthalene-1-carbonyl]piperazin-1-yl]pyridazine-3-carboxylic acid |
| EI02-15-02R | EI02-15 | 6-[4-[3-tert-Butyl-5-(3-hydroxyphenyl)benzoyl]piperazin-1-yl]pyridazine-3-carboxylic acid |
| EI02-16-02R | EI02-16 | 6-[4-[3-tert-Butyl-5-(5-hydroxypyridin-3-yl)benzoyl]piperazin-1-yl]pyridazine-3-carboxylic acid |
| EI02-17-02R | EI02-17 | 6-[4-[3-(3-Hydroxyphenyl)-5-(trifluoromethyl)benzoyl]piperazin-1-yl]pyridazine-3-carboxylic acid |
| EI02-18-02R | EI02-18 | 6-[4-[3-(5-Hydroxypyridin-3-yl)-5-(trifluoromethyl)benzoyl]piperazin-1-yl]pyridazine-3-carboxylic acid |
| EI02-19-02R | EI02-19 | 6-[4-[3-[2-(3-Hydroxyphenyl)ethynyl]-5-(trifluoromethyl)benzoyl]piperazin-1-yl]pyridazine-3-carboxylic acid |
| EI02-20-02R | EI02-20 | 6-[4-[3-[2-(5-Hydroxypyridin-3-yl)ethynyl]-5-(trifluoromethyl)benzoyl]piperazin-1-yl]pyridazine-3-carboxylic acid |
| EI02-21-02R | EI02-21 | 6-[4-[3-[4-(3-Hydroxyphenyl)pyrazol-1-yl]-5-(trifluoromethyl)benzoyl]piperazin-1-yl]pyridazine-3-carboxylic acid |
| EI02-22-02R | EI02-22 | 6-[4-[3-[4-(5-Hydroxypyridin-3-yl)pyrazol-1-yl]-5-(trifluoromethyl)benzoyl]piperazin-1-yl]pyridazine-3-carboxylic acid |
| EI03-01-02R | E103-01 | 4-[4-[[2-(3-Hydroxyphenyl)phenyl]methyl]piperazin-1-yl]benzoic acid |
| EI03-02-02R | EI03-02 | 4-[4-[[2-(5-Hydroxypyridin-3-yl)phenyl]methyl]piperazin-1-yl]benzoic acid |
| EI03-03-02R | EI03-03 | 4-[4-[[4-(3-Hydroxyphenyl)-3-methylphenyl]methyl]piperazin-1 - yl]benzoic acid |
| EI03-04-02R | EI03-04 | 4-[4-[[4-(5-Hydroxypyridin-3-yl)-3-methylphenyl]methyl]piperazin-1-yl]benzoic acid |
| EI03-05-02R | EI03-05 | 4-[4-[[2-Ethyl-4-(3-hydroxyphenyl)phenyl]methyl]piperazin-1-yl]benzoic acid |
| EI03-06-02R | EI03-06 | 4-[4-[[2-Ethyl-4-(5-hydroxypyridin-3-yl)phenyl]methyl]piperazin-1-yl]benzoic acid |

(continued)

| Resin compound No. | Post-cleavage compound No. | Compound name |
|---|---|---|
| EI03-07-02R | EI03-07 | 4-[4-[[3-Fluoro-4-(3-hydroxyphenyl)phenyl]methyl]piperazin-1-yl]benzoic acid |
| EI03-08-02R | EI03-08 | 4-[4-[[3-Fluoro-4-(5-hydroxypyridin-3-yl)phenyl]methyl]piperazin-1-yl]benzoic acid |
| EI03-09-02R | EI03-09 | 4-[4-[[4-(3-Hydroxyphenyl)-2-methoxyphenyl]methyl]piperazin-1-yl]benzoic acid |
| EI03-10-02R | EI03-10 | 4-[4-[[4-(5-Hydroxypyridin-3-yl)-2-methoxyphenyl]methyl]piperazin-1-yl]benzoic acid |
| EI03-11-02R | EI03-11 | 4-[4-[[3-Ethoxy-5-(3-hydroxyphenyl)phenyl]methyl]piperazin-1- yl]benzoic acid |
| EI03-12-02R | EI03-12 | 4-[4-[[3-Ethoxy-5-(5-hydroxypyridin-3-yl)phenyl]methyl]piperazin-1-yl]benzoic acid |
| EI03-13-02R | EI03-13 | 4-[4-[[3-(3-Hydroxyphenyl)-5-propan-2-yloxyphenyl]methyl]piperazin-1-yl]benzoic acid |
| EI03-14-02R | EI03-14 | 4-[4-[[3-(5-Hydroxypyridin-3-yl)-5-propan-2-yloxyphenyl]methyl]piperazin-1-yl]benzoic acid |
| EI03-15-02R | EI03-15 | 4-[4-[[4-(3-Hydroxyphenyl)-2,6-dimethoxyphenyl]methyl]piperazin-1-yl]benzoic acid |
| EI03-16-02R | EI03-16 | 4-[4-[[4-(5-Hydroxypyridin-3-yl)-2,6-dimethoxyphenyl]methyl]piperazin-1-yl]benzoic acid |
| EI03-17-02R | EI03-17 | 4-[4-[[3-(3-Hydroxyphenyl)-5-(trifluoromethyl)phenyl]methyl]piperazin-1-yl]benzoic acid |
| EI03-18-02R | EI03-18 | 4-[4-[[3-(5-Hydroxypyridin-3-yl)-5-(trifluoromethyl)phenyl]methyl]piperazin-1-yl]benzoic acid |
| EI03-19-02R | EI03-19 | 4-[4-[[3-(3-Hydroxyphenyl)-5-(trifluoromethoxy)phenyl]methyl]piperazin-1-yl]benzoic acid |
| EI03-20-02R | EI03-20 | 4-[4-[[3-(5-Hydroxypyridin-3-yl)-5-(trifluoromethoxy)phenyl]methyl]piperazin-1-yl]benzoic acid |
| EI03-21-02R | EI03-21 | 4-[4-[[4-(3-Hydroxyphenyl)naphthalen-1-yl]methyl]piperazin-1-yl]benzoic acid |
| EI03-22-02R | EI03-22 | 4-[4-[[4-(5-Hydroxypyridin-3-yl)naphthalen-1-yl]methyl]piperazin-1-yl]benzoic acid |
| EI03-23-02R | EI03-23 | 4-[4-[[4-(3-Hydroxyphenyl)thiophen-2-yl]methyl]piperazin-1-yl]benzoic acid |
| EI03-24-02R | EI03-24 | 4-[4-[[4-(5-Hydroxypyridin-3-yl)thiophen-2-yl]methyl]piperazin-1-yl]benzoic acid |
| EI03-25-02R | EI03-25 | 4-[4-[[2-[2-(3-Hydroxyphenyl)ethynyl]phenyl]methyl]piperazin-1-yl]benzoic acid |
| EI03-26-02R | EI03-26 | 4-[4-[[2-[2-(5-Hydroxypyridin-3-yl)ethynyl]phenyl]methyl]piperazin-1-yl]benzoic acid |
| EI04-01-02R | EI04-01 | 6-[4-[[2-(3-Hydroxyphenyl)phenyl]methyl]piperazin-1-yl]pyridazine-3-carboxylic acid |
| EI04-02-02R | EI04-02 | 6-[4-[[2-(5-Hydroxypyridin-3-yl)phenyl]methyl]piperazin-1-yl]pyridazine-3-carboxylic acid |

(continued)

| Resin compound No. | Post-cleavage compound No. | Compound name |
|---|---|---|
| EI04-03-02R | EI04-03 | 6-[4-[[4-(3-Hydroxyphenyl)-3-methylphenyl]methyl]piperazin-1-yl]pyridazine-3-carboxylic acid |
| EI04-04-02R | EI04-04 | 6-[4-[[4-(5-Hydroxypyridin-3-yl)-3-methylphenyl]methyl]piperazin-1-yl]pyridazine-3-carboxylic acid |
| EI04-05-02R | EI04-05 | 6-[4-[[2-Ethyl-4-(3-hydroxyphenyl)phenyl]methyl]piperazin-1-yl]pyridazine-3-carboxylic acid |
| EI04-06-02R | EI04-06 | 6-[4-[[2-Ethyl-4-(5-hydroxypyridin-3-yl)phenyl]methyl]piperazin-1-yl]pyridazine-3-carboxylic acid |
| EI04-07-02R | EI04-07 | 6-[4-[[3-Fluoro-4-(3-hydroxyphenyl)phenyl]methyl]piperazin-1-yl]pyridazine-3-carboxylic acid |
| EI04-08-02R | EI04-08 | 6-[4-[[3-Fluoro-4-(5-hydroxypyridin-3-yl)phenyl]methyl]piperazin-1-yl]pyridazine-3-carboxylic acid |
| EI04-09-02R | EI04-09 | 6-[4-[[4-(3-Hydroxyphenyl)-2-methoxyphenyl]methyl]piperazin-1-yl]pyridazine-3-carboxylic acid |
| EI04-10-02R | EI04-10 | 6-[4-[[4-(5-Hydroxypyridin-3-yl)-2-methoxyphenyl]methyl]piperazin-1-yl]pyridazine-3-carboxylic acid |
| EI04-11-02R | EI04-11 | 6-[4-[[3-Ethoxy-5-(3-hydroxyphenyl)phenyl]methyl]piperazin-1- yl]pyridazine-3-carboxylic acid |
| EI04-12-02R | EI04-12 | 6-[4-[[3-Ethoxy-5-(5-hydroxypyridin-3-yl)phenyl]methyl]piperazin-1-yl]pyridazine-3-carboxylic acid |
| EI04-13-02R | EI04-13 | 6-[4-[[3-(3-Hydroxyphenyl)-5-propan-2-yloxyphenyl]methyl]piperazin-1-yl]pyridazine-3-carboxylic acid |
| EI04-14-02R | EI04-14 | 6-[4-[[3-(5-Hydroxypyridin-3-yl)-5-propan-2-yloxyphenyl]methyl]piperazin-1-yl]pyridazine-3-carboxylic acid |
| EI04-15-02R | EI04-15 | 6-[4-[[4-(3-Hydroxyphenyl)-2,6-dimethoxyphenyl]methyl]piperazin-1-yl]pyridazine-3-carboxylic acid |
| EI04-16-02R | EI04-16 | 6-[4-[[4-(5-Hydroxypyridin-3-yl)-2,6-dimethoxyphenyl]methyl]piperazin-1-yl]pyridazine-3-carboxylic acid |
| EI04-17-02R | EI04-17 | 6-[4-[[3-(3-Hydroxyphenyl)-5-(trifluoromethyl)phenyl]methyl]piperazin-1-yl]pyridazine-3-carboxylic acid |
| EI04-18-02R | EI04-18 | 6-[4-[[3-(5-Hydroxypyridin-3-yl)-5-(trifluoromethyl)phenyl]methyl]piperazin-1-yl]pyridazine-3-carboxylic acid |
| EI04-19-02R | EI04-19 | 6-[4-[[3-(3-Hydroxyphenyl)-5-(trifluoromethoxy)phenyl]methyl]piperazin-1-yl]pyridazine-3- carboxylic acid |
| EI04-20-02R | EI04-20 | 6-[4-[[3-(5-Hydroxypyridin-3-yl)-5-(trifluoromethoxy)phenyl]methyl]piperazin-1-yl]pyridazine-3- carboxylic acid |
| EI04-21-02R | EI04-21 | 6-[4-[[4-(3-Hydroxyphenyl)naphthalen-1-yl]methyl]piperazin-1-yl]pyridazine-3-carboxylic acid |
| EI04-22-02R | EI04-22 | 6-[4-[[4-(5-Hydroxypyridin-3-yl)naphthalen-1-yl]methyl]piperazin-1-yl]pyridazine-3-carboxylic acid |
| EI04-23-02R | EI04-23 | 6-[4-[[4-(3-Hydroxyphenyl)thiophen-2-yl]methyl]piperazin-1-yl]pyridazine-3-carboxylic acid |

(continued)

| Resin compound No. | Post-cleavage compound No. | Compound name |
|---|---|---|
| EI04-24-02R | EI04-24 | 6-[4-[[4-(5-Hydroxypyridin-3-yl)thiophen-2-yl]methyl]piperazin-1-yl]pyridazine-3-carboxylic acid |
| EI04-25-02R | EI04-25 | 6-[4-[[2-[2-(3-Hydroxyphenyl)ethynyl]phenyl]methyl]piperazin-1-yl]pyridazine-3-carboxylic acid |
| EI04-26-02R | EI04-26 | 6-[4-[[2-[2-(5-Hydroxypyridin-3-yl)ethynyl]phenyl]methyl]piperazin-1-yl]pyridazine-3-carboxylic acid |
| EI05-01-02R | EI05-01 | 4-[4-[1-[2-Fluoro-4-(3-hydroxyphenyl)phenyl]ethyl]piperazin-1- yl]benzoic acid |
| EI05-02-02R | EI05-02 | 4-[4-[1-[2-Fluoro-4-(5-hydroxypyridin-3-yl)phenyl]ethyl]piperazin-1-yl]benzoic acid |
| EI05-03-02R | EI05-03 | 4-[4-[1-[4-(3-Hydroxyphenyl)phenyl]ethyl]piperazin-1-yl]benzoic acid |
| EI05-04-02R | EI05-04 | 4-[4-[1-[4-(5-Hydroxypyridin-3-yl)phenyl]ethyl]piperazin-1-yl]benzoic acid |

Example 6-3-7-A: Synthesis of mixture mixEI07-02R

[Formula 110]

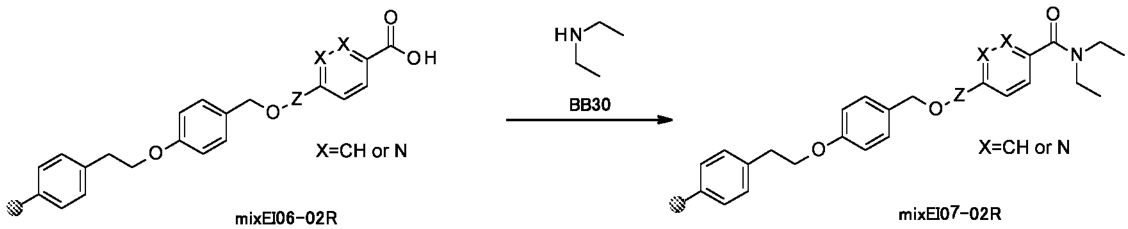


mixEI06-02R

mixEI07-02R

To a 2 mL glass vial were added the mixture mixEI06-02R (50 mg, loading rate 0.648 mmol/g, 0.0324 mmol) shown in [Table EI06] and NMP (1.28 mL), and the mixture was shaken at room temperature for 1 hour. Diethylamine (BB30, 10.1 μL, 0.097 mmol), DIC (30.3 μL, 0.194 mmol), and HOAt (26.5 mg, 0.194 mmol) were added, and the mixture was shaken at 40°C for 22 hours. The suspension of the reaction solution and the resins was transferred to a syringe equipped with a filter using NMP, washed repeatedly three times with NMP (1.2 mL), three times with MeOH (1.2 mL), three times with DCM (1.2 mL), and three times with heptane (1.2 mL), and the resins were then dried under reduced pressure to obtain the title mixture shown in [Table EI07]. The names of compounds contained in the obtained mixture are shown in [Table EI07].

[Table 61]

[Table EI07] Mixture No.: mixEI07-02R, amine reagent used: BB30, post-cleavage mixture No.: mixEI07

| Resin compound No. | Post-cleavage compound No. | Another name of post-cleavage compound in Table EJ01 | Compound name |
|---|---|---|---|
| EI07-01-02R | EI07-01 | 4J01 | N,N-Diethyl-6-[4-[4-(3-hydroxyphenyl)thiophene-2-carbonyl]piperazin-1-yl]pyridazine-3-carboxamide |

(continued)

| Resin compound No. | Post-cleavage compound No. | Another name of post-cleavage compound in Table EJ01 | Compound name |
|---|---|---|---|
| EI07-02-02R | EI07-02 | 4J02 | N,N-Diethyl-6-[4-[4-(5-hydroxypyridin-3-yl)thiophene-2-carbonyl]piperazin-1-yl]pyridazine-3-carboxamide |
| EI07-03-02R | EI07-03 | 4J03 | N,N-Diethyl-6-[4-[4-(3-hydroxyphenyl)-3-methylbenzoyl]piperazin-1-yl]pyridazine-3-carboxamide |
| EI07-04-02R | EI07-04 | 4J04 | N,N-Diethyl-6-[4-[4-(5-hydroxypyridin-3-yl)-3-methylbenzoyl]piperazin-1-yl]pyridazine-3-carboxamide |
| EI07-05-02R | EI07-05 | 4J05 | N,N-Diethyl-6-[4-[3-fluoro-4-(3-hydroxyphenyl)benzoyl]piperazin-1-yl]pyridazine-3-carboxamide |
| EI07-06-02R | EI07-06 | 4J06 | N,N-Diethyl-6-[4-[3-fluoro-4-(5-hydroxypyridin-3-yl)benzoyl]piperazin-1-yl]pyridazine-3-carboxamide |
| EI07-07-02R | EI07-07 | 4J07 | N,N-Diethyl-6-[4-[2-[2-(3-hydroxyphenyl)ethynyl]benzoyl]piperazin-1-yl]pyridazine-3-carboxamide |
| EI07-08-02R | EI07-08 | 4J08 | N,N-Diethyl-6-[4-[2-[2-(5-hydroxypyridin-3-yl)ethynyl]benzoyl]piperazin-1-yl]pyridazine-3-carboxamide |
| EI07-09-02R | EI07-09 | 4J09 | N,N-Diethyl-6-[4-[5-[2-(3-hydroxyphenyl)ethynyl]pyridine-3-carbonyl]piperazin-1-yl]pyridazine-3-carboxamide |
| EI07-10-02R | EI07-10 | 4J10 | N,N-Diethyl-6-[4-[5-[2-(5-hydroxypyridin-3-yl)ethynyl]pyridine-3-carbonyl]piperazin-1-yl]pyridazine-3- carboxamide |
| EI07-11-02R | EI07-11 | 4J11 | N,N-Diethyl-6-[4-[3-[2-(3-hydroxyphenyl)phenyl]propanoyl]piperazin-1-yl]pyridazine-3-carboxamide |
| EI07-12-02R | EI07-12 | 4J12 | N,N-Diethyl-6-[4-[3-[2-(5-hydroxypyridin-3-yl)phenyl]propanoyl]piperazin-1-yl]pyridazine-3-carboxamide |
| EI07-13-02R | EI07-13 | 4J13 | N,N-Diethyl-6-[4-[4-(3-hydroxyphenyl)naphthalene-1-carbonyl]piperazin-1-yl]pyridazine-3-carboxamide |
| EI07-14-02R | EI07-14 | 4J14 | N,N-Diethyl-6-[4-[4-(5-hydroxypyridin-3-yl)naphthalene-1-carbonyl]piperazin-1-yl]pyridazine-3-carboxamide |
| EI07-15-02R | EI07-15 | 4J15 | 6-[4-[3-tert-Butyl-5-(3-hydroxyphenyl)benzoyl]piperazin-1-yl]-N,N-diethylpyridazine-3-carboxamide |
| EI07-16-02R | EI07-16 | 4J16 | 6-[4-[3-tert-Butyl-5-(5-hydroxypyridin-3-yl)benzoyl]piperazin-1-yl]-N,N-diethylpyridazine-3-carboxamide |
| EI07-17-02R | EI07-17 | 4J17 | N,N-Diethyl-6-[4-[3-(3-hydroxyphenyl)-5-(trifluoromethyl)benzoyl]piperazin-1-yl]pyridazine-3-carboxamide |
| EI07-18-02R | EI07-18 | 4J18 | N,N-Diethyl-6-[4-[3-(5-hydroxypyridin-3-yl)-5-(trifluoromethyl)benzoyl]piperazin-1-yl]pyridazine-3-carboxamide |

(continued)

| Resin compound No. | Post-cleavage compound No. | Another name of post-cleavage compound in Table EJ01 | Compound name |
|---|---|---|---|
| EI07-19-02R | EI07-19 | 4J19 | N,N-Diethyl-6-[4-[3-[2-(3-hydroxyphenyl)ethynyl]-5-(trifluoromethyl)benzoyl]piperazin-1-yl]pyridazine-3-carboxamide |
| EI07-20-02R | EI07-20 | 4J20 | N,N-Diethyl-6-[4-[3-[2-(5-hydroxypyridin-3-yl)ethynyl]-5-(trifluoromethyl)benzoyl]piperazin-1-yl]pyridazine-3-carboxamide |
| EI07-21-02R | EI07-21 | 4J21 | N,N-Diethyl-6-[4-[3-[4-(3-hydroxyphenyl)pyrazol-1-yl]-5-(trifluoromethyl)benzoyl]piperazin-1-yl]pyridazine-3-carboxamide |
| EI07-22-02R | EI07-22 | 4J22 | N,N-Diethyl-6-[4-[3-[4-(5-hydroxypyridin-3-yl)pyrazol-1-yl]-5-(trifluoromethyl)benzoyl]piperazin-1-yl]pyridazine-3-carboxamide |
| EI07-23-02R | EI07-23 | 4J23 | N,N-Diethyl-4-[4-[4-(3-hydroxyphenyl)thiophene-2-carbonyl1piperazin-1-yl]benzamide |
| EI07-24-02R | EI07-24 | 4J24 | N,N-Diethyl-4-[4-[4-(5-hydroxypyridin-3-yl)thiophene-2-carbonyl]piperazin-1-yl]benzamide |
| EI07-25-02R | EI07-25 | 4J25 | N,N-Diethyl-4-[4-[4-(3-hydroxyphenyl)-3-methylbenzoyl]piperazin-1-yl]benzamide |
| EI07-26-02R | EI07-26 | 4J26 | N,N-Diethyl-4-[4-[4-(5-hydroxypyridin-3-yl)-3-methylbenzoyl]piperazin-1-yl]benzamide |
| EI07-27-02R | EI07-27 | 4J27 | N,N-Diethyl-4-[4-[3-fluoro-4-(3-hydroxyphenyl)benzoyl]piperazin-1-yl]benzamide |
| EI07-28-02R | EI07-28 | 4J28 | N,N-Diethyl-4-[4-[3-fluoro-4-(5-hydroxypyridin-3-yl)benzoyl]piperazin-1-yl]benzamide |
| EI07-29-02R | EI07-29 | 4J29 | N,N-Diethyl-4-[4-[2-[2-(3-hydroxyphenyl)ethynyl]benzoyl]piperazin-1-yl]benzamide |
| EI07-30-02R | EI07-30 | 4J30 | N,N-Diethyl-4-[4-[2-[2-(5-hydroxypyridin-3-yl)ethynyl]benzoyl]piperazin-1-yl]benzamide |
| EI07-31-02R | EI07-31 | 4J31 | N,N-Diethyl-4-[4-[5-[2-(3-hydroxyphenyl)ethynyl]pyridine-3-carbonyl]piperazin-1-yl]benzamide |
| EI07-32-02R | EI07-32 | 4J32 | N,N-Diethyl-4-[4-[5-[2-(5-hydroxypyridin-3-yl)ethynyl]pyridine-3-carbonyl]piperazin-1-yl]benzamide |
| EI07-33-02R | EI07-33 | 4J33 | N,N-Diethyl-4-[4-[3-[2-(3-hydroxyphenyl)phenyl]propanoyl]piperazin-1-yl]benzamide |
| EI07-34-02R | EI07-34 | 4J34 | N,N-Diethyl-4-[4-[3-[2-(5-hydroxypyridin-3-yl)phenyl]propanoyl]piperazin-1-yl]benzamide |
| EI07-35-02R | EI07-35 | 4J35 | N,N-Diethyl-4-[4-[4-(3-hydroxyphenyl)naphthalene-1-carbonyl]piperazin-1-yl]benzamide |
| EI07-36-02R | EI07-36 | 4J36 | N,N-Diethyl-4-[4-[4-(5-hydroxypyridin-3-yl)naphthalene-1-carbonyl1piperazin-1-yl]benzamide |
| EI07-37-02R | EI07-37 | 4J37 | 4-[4-[3-tert-Butyl-5-(3-hydroxyphenyl)benzoyl]piperazin-1-yl]-N,N-diethylbenzamide |

(continued)

| Resin compound No. | Post-cleavage compound No. | Another name of post-cleavage compound in Table EJ01 | Compound name |
|---|---|---|---|
| EI07-38-02R | EI07-38 | 4J38 | 4-[4-[3-tert-Butyl-5-(5-hydroxypyridin-3-yl)benzoyl1piperazin-1-yl]-N,N-diethylbenzamide |
| EI07-39-02R | EI07-39 | 4J39 | N,N-Diethyl-4-[4-[3-(3 -hydroxyphenyl)-5-(trifluoromethyl)benzoyl]piperazin-1yl]benzamide |
| EI07-40-02R | EI07-40 | 4J40 | N,N-Diethyl-4-[4-[3-(5-hydroxypyridin-3-yl)-5-(trifluoromethyl)benzoyl]piperazin-1-yl]benzamide |
| EI07-41-02R | EI07-41 | 4J41 | N,N-Diethyl-4-[4-[3-[2-(3-hydroxyphenyl)ethynyl]-5-(trifluoromethyl)benzoyl]piperazin-1yl]benzamide |
| EI07-42-02R | EI07-42 | 4J42 | N,N-Diethyl-4-[4-[3-[2-(5-hydroxypyridin-3-yl)ethynyl]-5-(trifluoromethyl)benzoyl]piperazin-1-yl]benzamide |
| EI07-43-02R | EI07-43 | 4J43 | N,N-Diethyl-4-[4-[3-[4-(3-hydroxyphenyl)pyrazol-1-yl]-5-(trifluoromethyl)benzoyl]piperazin-1yl]benzamide |
| EI07-44-02R | EI07-44 | 4J44 | N,N-Diethyl-4-[4-[3-[4-(5-hydroxypyridin-3-yl)pyrazol-1-yl]-5-(trifluoromethyl)benzoyl]piperazin-1-yl]benzamide |
| EI07-45-02R | EI07-45 | 4J45 | N,N-Diethyl-4-[4-[[2-(3-hydroxyphenyl)phenyl]methyl]piperazin-1-yl]benzamide |
| EI07-46-02R | EI07-46 | 4J46 | N,N-Diethyl-4-[4-[[2-(5-hydroxypyridin-3-yl)phenyl]methyl]piperazin-1-yl]benzamide |
| EI07-47-02R | EI07-47 | 4J47 | N,N-Diethyl-4-[4-[[4-(3-hydroxyphenyl)-3-methylphenyl]methyl]piperazin-1-yl]benzamide |
| EI07-48-02R | EI07-48 | 4J48 | N,N-Diethyl-4-[4-[[4-(5-hydroxypyridin-3-yl)-3-methylphenyl]methyl]piperazin-1-yl]benzamide |
| EI07-49-02R | EI07-49 | 4J49 | N,N-Diethyl-4-[4-[[2-ethyl-4-(3-hydroxyphenyl)phenyl]methyl]piperazin-1-yl]benzamide |
| EI07-50-02R | EI07-50 | 4J50 | N,N-Diethyl-4-[4-[[2-ethyl-4-(5-hydroxypyridin-3-yl)phenyl]methyl]piperazin-1-yl]benzamide |
| EI07-51-02R | EI07-51 | 4J51 | N,N-Diethyl-4-[4-[[3-fluoro-4-(3-hydroxyphenyl)phenyl]methyl1piperazin-1-yl]benzamide |
| EI07-52-02R | EI07-52 | 4J52 | N,N-Diethyl-4-[4-[[3-fluoro-4-(5-hydroxypyridin-3-yl)phenyl]methyl]piperazin-1-yl]benzamide |
| EI07-53-02R | EI07-53 | 4J53 | N,N-Diethyl-4-[4-[[4-(3-hydroxyphenyl)-2-methoxyphenyl]methyl]piperazin-1-yl]benzamide |
| EI07-54-02R | EI07-54 | 4J54 | N,N-Diethyl-4-[4-[[4-(5-hydroxypyridin-3-yl)-2-methoxyphenyl]methyl]piperazin-1-yl]benzamide |
| EI07-55-02R | EI07-55 | 4J55 | 4-[4-[[3-Ethoxy-5-(3-hydroxyphenyl)phenyl]methyl]piperazin-1-yl]-N,N-diethylbenzamide |
| EI07-56-02R | EI07-56 | 4J56 | 4-[4-[3-Ethoxy-5-(5-hydroxypyridin-3-yl)phenyl]methyl]piperazin-1-yl]-N,N-diethylbenzamide |
| EI07-57-02R | EI07-57 | 4J57 | N,N-Diethyl-4-[4-[[3-(3-hydroxyphenyl)-5-propan-2-yloxyphenyl]methyl]piperazin-1-yl]benzamide |

(continued)

| Resin compound No. | Post-cleavage compound No. | Another name of post-cleavage compound in Table EJ01 | Compound name |
|---|---|---|---|
| EI07-58-02R | EI07-58 | 4J58 | N,N-Diethyl-4-[4-[[3-(5-hydroxypyridin-3-yl)-5-propan-2-yloxyphenyl]methyl]piperazin-1-yl]benzamide |
| EI07-59-02R | EI07-59 | 4J59 | N,N-Diethyl-4-[4-[[4-(3-hydroxyphenyl)-2,6-dimethoxyphenyl]methyllpiperazin-1-yl]benzamide |
| EI07-60-02R | EI07-60 | 4J60 | N,N-Diethyl-4-[4-[[4-(5-hydroxypyridin-3-yl)-2,6-dimethoxyphenyl]methyl]piperazin-1-yl]benzamide |
| EI07-61-02R | EI07-61 | 4J61 | N,N-Diethyl-4-[4-[[3-(3-hydroxyphenyl)-5-(trifluoromethyl)phenyl]methyl]piperazin-1-yl]benzamide |
| EI07-62-02R | EI07-62 | 4J62 | N,N-Diethyl-4-[4-[[3-(5-hydroxypyridin-3-yl)-5-(trifluoromethyl)phenyl]methyl]piperazin-1-yl]benzamide |
| EI07-63-02R | EI07-63 | 4J63 | N,N-Diethyl-4-[4-[[3-(3-hydroxyphenyl)-5-(trifluoromethoxy)phenyl]methyl]piperazin-1-yl]benzamide |
| EI07-64-02R | EI07-64 | 4J64 | N,N-Diethyl-4-[4-[[3-(5-hydroxypyridin-3-yl)-5-(trifluoromethoxy)phenyl]methyl]piperazin-1-yl]benzamide |
| EI07-65-02R | EI07-65 | 4J65 | N,N-Diethyl-4-[4-[[4-(3-hydroxyphenyl)naphthalen-1-yl]methyl]piperazin-1-yl]benzamide |
| EI07-66-02R | EI07-66 | 4J66 | N,N-Diethyl-4-[4-[[4-(5-hydroxypyridin-3-yl)naphthalen-1-yl]methyl]piperazin-1-yl]benzamide |
| EI07-67-02R | EI07-67 | 4J67 | N,N-Diethyl-4-[4-[[4-(3-hydroxyphenyl)thiophen-2-yl]methyl]piperazin-1-yl]benzamide |
| EI07-68-02R | EI07-68 | 4J68 | N,N-Diethyl-4-[4-[[4-(5-hydroxypyridin-3-yl)thiophen-2-yl]methyl]piperazin-1-yl]benzamide |
| EI07-69-02R | EI07-69 | 4J69 | N,N-Diethyl-4-[4-[[2-[2-(3-hydroxyphenyl)ethynyl]phenyl]methyl]piperazin-1-yl]benzamide |
| EI07-70-02R | EI07-70 | 4J70 | N,N-Diethyl-4-[4-[[2-[2-(5-hydroxypyridin-3-yl)ethynyl]phenyl]methyl]piperazin-1-yl]benzamide |
| EI07-71-02R | EI07-71 | 4J71 | N,N-Diethyl-6-[4-[[2-(3-hydroxyphenyl)phenyl]methyl]piperazin-1-yl]pyridazine-3-carboxamide |
| EI07-72-02R | EI07-72 | 4J72 | N,N-Diethyl-6-[4-[[2-(5-hydroxypyridin-3-yl)phenyl]methyl]piperazin-1-yl]pyridazine-3-carboxamide |
| EI07-73-02R | EI07-73 | 4J73 | N,N-Diethyl-6-[4-[[4-(3-hydroxyphenyl)-3-methylphenyl]methyl]piperazin-1-yl]pyridazine-3-carboxamide |
| EI07-74-02R | EI07-74 | 4J74 | N,N-Diethyl-6-[4-[[4-(5-hydroxypyridin-3-yl)-3-methylphenyl]methyl]piperazin-1-yl]pyridazine-3-carboxamide |

(continued)

| Resin compound No. | Post-cleavage compound No. | Another name of post-cleavage compound in Table EJ01 | Compound name |
|---|---|---|---|
| EI07-75-02R | EI07-75 | 4J75 | N,N-Diethyl-6-[4-[[2-ethyl-4-(3-hydroxyphenyl)phenyl]methyl]piperazin-1-yl]pyridazine-3-carboxamide |
| EI07-76-02R | EI07-76 | 4J76 | N,N-Diethyl-6-[4-[[2-ethyl-4-(5-hydroxypyridin-3-yl)phenyl]methyl]piperazin-1-yl]pyridazine-3-carboxamide |
| EI07-77-02R | EI07-77 | 4J77 | N,N-Diethyl-6-[4-[[3-fluoro-4-(3-hydroxyphenyl)phenyl]methyl]piperazin-1-yl]pyridazine-3-carboxamide |
| EI07-78-02R | EI07-78 | 4J78 | N,N-Diethyl-6-[4-[[3-fluoro-4-(5-hydroxypyridin-3-yl)phenyl]methyl]piperazin-1-yl]pyridazine-3-carboxamide |
| EI07-79-02R | EI07-79 | 4J79 | N,N-Diethyl-6-[4-[[4-(3-hydroxyphenyl)-2-methoxyphenyl]methyl]piperazin-1-yl]pyridazine-3-carboxamide |
| EI07-80-02R | EI07-80 | 4J80 | N,N-Diethyl-6-[4-[[4-(5-hydroxypyridin-3-yl)-2-methoxyphenyl]methyl]piperazin-1-yl]pyridazine-3-carboxamide |
| EI07-81-02R | EI07-81 | 4J81 | 6-[4-[[3-Ethoxy-5-(3-hydroxyphenyl)phenyl]methyl]piperazin-1-yl]-N,N-diethylpyridazine-3-carboxamide |
| EI07-82-02R | EI07-82 | 4J82 | 6-[4-[[3-Ethoxy-5-(5-hydroxypyridin-3-yl)phenyl]methyl]piperazin-1-yl]-N,N-diethylpyridazine-3-carboxamide |
| EI07-83-02R | EI07-83 | 4J83 | N,N-Diethyl-6-[4-[[3-(3-hydroxyphenyl)-5-propan-2-yloxyphenyl]methyl]piperazin-1-yl]pyridazine-3-carboxamide |
| EI07-84-02R | EI07-84 | 4J84 | N,N-Diethyl-6-[4-[[3-(5-hydroxypyridin-3-yl)-5-propan-2-yloxyphenyl]methyl]piperazin-1-yl]pyridazine-3-carboxamide |
| EI07-85-02R | EI07-85 | 4J85 | N,N-Diethyl-6-[4-[[4-(3-hydroxyphenyl)-2,6-dimethoxyphenyl]methyl]piperazin-1-yl]pyridazine-3-carboxamide |
| EI07-86-02R | EI07-86 | 4J86 | N,N-Diethyl-6-[4-[[4-(5-hydroxypyridin-3-yl)-2,6-dimethoxyphenyl]methyl]piperazin-1-yl]pyridazine-3-carboxamide |
| EI07-87-02R | EI07-87 | 4J87 | N,N-Diethyl-6-[4-[[3-(3-hydroxyphenyl)-5-(trifluoromethyl)phenyl]methyl]piperazin-1-yl]pyridazine-3-carboxamide |
| EI07-88-02R | EI07-88 | 4J88 | N,N-Diethyl-6-[4-[[3-(5-hydroxypyridin-3-yl)-5-(trifluoromethyl)phenyl]methyl]piperazin-1-yl]pyridazine-3-carboxamide |
| EI07-89-02R | EI07-89 | 4J89 | N,N-Diethyl-6-[4-[[3-(3-hydroxyphenyl)-5-(trifluoromethoxy)phenyl]methyl]piperazin-1-yl]pyridazine-3- carboxamide |

(continued)

| Resin compound No. | Post-cleavage compound No. | Another name of post-cleavage compound in Table EJ01 | Compound name |
|---|---|---|---|
| EI07-90-02R | EI07-90 | 4J90 | N,N-Diethyl-6-[4-[[3-(5-hydroxypyridin-3-yl)-5-(trifluoromethoxy)phenyl]methyl]piperazin-1-yl]pyridazine-3 - carboxamide |
| EI07-91-02R | EI07-91 | 4J91 | N,N-Diethyl-6-[4-[[4-(3-hydroxyphenyl)naphthalen-1-yl]methyl1piperazin-1-yl1pyridazine-3-carboxamide |
| EI07-92-02R | EI07-92 | 4J92 | N,N-Diethyl-6-[4-[[4-(5-hydroxypyridin-3-yl)naphthalen-1-yl]methyl]piperazin-1-yl]pyridazine-3-carboxamide |
| EI07-93-02R | EI07-93 | 4J93 | N,N-Diethyl-6-[4-[[4-(3-hydroxyphenyl)thiophen-2-yl]methyl]piperazin-1-yl]pyridazine-3-carboxamide |
| EI07-94-02R | EI07-94 | 4J94 | N,N-Diethyl-6-[4-[[4-(5-hydroxypyridin-3-yl)thiophen-2-yl]methyl]piperazin-1-yl]pyridazine-3-carboxamide |
| EI07-95-02R | EI07-95 | 4J95 | N,N-Diethyl-6-[4-[[2-[2-(3-hydroxyphenyl)ethynyl]phenyl]methyl]piperazin-1-yl]pyridazine-3 -carboxamide |
| EI07-96-02R | EI07-96 | 4J96 | N,N-Diethyl-6-[4-[[2-[2-(5-hydroxypyridin-3-yl)ethynyl]phenyl]methyl]piperazin-1-yl]pyridazine-3-carboxamide |
| EI07-97-02R | EI07-97 | 4J97 | N,N-Diethyl-4-[4-[1-[4-(3-hydroxyphenyl)phenyl]ethyl]piperazin-1-yl]benzamide |
| EI07-98-02R | EI07-98 | 4J98 | N,N-Diethyl-4-[4-[1-[4-(5-hydroxypyridin-3-yl)phenyl]ethyl]piperazin-1-yl]benzamide |
| EI07-99-02R | EI07-99 | 4J99 | N,N-Diethyl-4-[4-[1-[2-fluoro-4-(3-hydroxyphenyl)phenyl]ethyl]piperazin-1-yl]benzamide |
| EI07-100-02R | EI07-100 | 4J100 | N,N-Diethyl-4-[4-[1-[2-fluoro-4-(5-hydroxypyridin-3-yl)phenyl]ethyl]piperazin-1-yl]benzamide |

Example 6-3-7-B: Synthesis of mixtures mixEI08-02R to mixEI10-02R

Using the mixture mixEI06-02R shown in [Table EI06] (50 mg, loading rate 0.648 mmol/g, 0.0324 mmol) and the amine reagents (BB31 to BB33), the title mixtures shown in [Table EI08] to [Table EI10] were synthesized by the same method as in Example 6-3-7-A and obtained. The names of compounds contained in the obtained mixtures are shown in [Table EI08] to [Table EI10].

[Table 62]

[Table EI08] Mixture No.: mixEI08-02R, amine reagent used: BB31, post-cleavage mixture No.: mixEI08

| Resin compound No. | Post-cleavage compound No. | Another name of post-cleavage compound in Table EJ02 | Compound name |
|---|---|---|---|
| EI08-01-02R | EI08-01 | 1M01 | 6-[4-[4-(3-Hydroxyphenyl)thiophene-2-carbonyl]piperazin-1-yl]-N-propylpyridazine-3-carboxamide |

(continued)

| Resin compound No. | Post-cleavage compound No. | Another name of post-cleavage compound in Table EJ02 | Compound name |
|---|---|---|---|
| EI08-02-02R | EI08-02 | 1M02 | 6-[4-[4-(5-Hydroxypyridin-3-yl)thiophene-2-carbonyl]piperazin-1-yl]-N-propylpyridazine-3-carboxamide |
| EI08-03-02R | EI08-03 | 1M03 | 6-[4-[4-(3-Hydroxyphenyl)-3-methylbenzoyl]piperazin-1-yl]-N-propylpyridazine-3-carboxamide |
| EI08-04-02R | EI08-04 | 1M04 | 6-[4-[4-(5-Hydroxypyridin-3-yl)-3-methylbenzoyl]piperazin-1-yl]-N-propylpyridazine-3-carboxamide |
| EI08-05-02R | EI08-05 | 1M05 | 6-[4-[3-Fluoro-4-(3-hydroxyphenyl)benzoyl]piperazin-1-yl]-N-propylpyridazine-3-carboxamide |
| EI08-06-02R | EI08-06 | 1M06 | 6-[4-[3-Fluoro-4-(5-hydroxypyridin-3 -yl)benzoyl]piperazin-1 - yl]-N-propylpyridazine-3 -carboxamide |
| EI08-07-02R | EI08-07 | 1M07 | 6-[4-[2-[2-(3-Hydroxyphenyl)ethynyl]benzoyl]piperazin-1-yl]-N-propylpyridazine-3 -carboxamide |
| EI08-08-02R | EI08-08 | 1M08 | 6-[4-[2-[2-(5-Hydroxypyridin-3-yl)ethynyl]benzoyl]piperazin-1-yl]-N-propylpyridazine-3-carboxamide |
| EI08-09-02R | EI08-09 | 1M09 | 6-[4-[5-[2-(3-Hydroxyphenyl)ethynyl]pyridine-3-carbonyl]piperazin-1-yl]-N-propylpyridazine-3-carboxamide |
| EI08-10-02R | EI08-10 | 1M10 | 6-[4-[5-[2-(5-Hydroxypyridin-3-yl)ethynyl]pyridine-3-carbonyl]piperazin-1 -yl] -N-propylpyridazine-3 -carboxamide |
| EI08-11-02R | EI08-11 | 1M11 | 6-[4-[3-[2-(3 -Hydroxyphenyl)phenyl]propanoyl]piperazin-1 - yl]-N-propylpyridazine-3-carboxamide |
| EI08-12-02R | EI08-12 | 1M12 | 6-[4-[3-[2-(5-Hydroxypyridin-3-yl)phenyl]propanoyl]piperazin-1 -yl] -N-propylpyridazine-3 - carboxamide |
| EI08-13-02R | EI08-13 | 1M13 | 6-[4-[4-(3-Hydroxyphenyl)naphthalene-1 -carbonyl]piperazin-1-yl]-N-propylpyridazine-3-carboxamide |
| EI08-14-02R | EI08-14 | 1M14 | 6-[4-[4-(5-Hydroxypyridin-3-yl)naphthalene-1-carbonyl]piperazin-1-yl]-N-propylpyridazine-3-carboxamide |
| EI08-15-02R | EI08-15 | 1M15 | 6-[4-[3-tert-Butyl-5-(3-hydroxyphenyl)benzoyl]piperazin-1-yl]-N-propylpyridazine-3-carboxamide |
| EI08-16-02R | EI08-16 | 1M16 | 6-[4-[3-tert-Butyl-5-(5-hydroxypyridin-3-yl)benzoyl]piperazin-1-yl]-N-propylpyridazine-3-carboxamide |
| EI08-17-02R | EI08-17 | 1M17 | 6-[4-[3-(3-Hydroxyphenyl)-5-(trifluoromethyl)benzoyl]piperazin-1 -yl] -N-propylpyridazine-3-carboxamide |

(continued)

| Resin compound No. | Post-cleavage compound No. | Another name of post-cleavage compound in Table EJ02 | Compound name |
|---|---|---|---|
| EI08-18-02R | EI08-18 | 1M18 | 6-[4-[3-(5-Hydroxypyridin-3-yl)-5-(trifluoromethyl)benzoyl]piperazin-1 -yl] -N-propylpyridazine-3-carboxamide |
| EI08-19-02R | EI08-19 | 1M19 | 6-[4-[3-[2-(3-Hydroxyphenyl)ethynyl]-5-(trifluoromethyl)benzoyl]piperazin-1 -yl] -N-propylpyridazine-3-carboxamide |
| EI08-20-02R | EI08-20 | 1M20 | 6-[4-[3-[2-(5-Hydroxypyridin-3-yl)ethynyl]-5-(trifluoromethyl)benzoyl]piperazin-1 -yl] -N-propylpyridazine-3-carboxamide |
| EI08-21-02R | EI08-21 | 1M21 | 6-[4-[3 -[4-(3 -Hydroxyphenyl)pyrazol-1 -yl] -5-(trifluoromethyl)benzoyl]piperazin-1 -yl] -N-propylpyridazine-3-carboxamide |
| EI08-22-02R | EI08-22 | 1M22 | 6-[4-[3 -[4-(5 -Hydroxypyridin-3 -yl)pyrazol-1 -yl] -5 -(trifluoromethyl)benzoyl]piperazin-1 -yl] -N-propylpyridazine-3-carboxamide |
| EI08-23-02R | EI08-23 | 1M23 | 4-[4-[4-(3-Hydroxyphenyl)thiophene-2-carbonyl]piperazin-1-yl]-N-propylbenzamide |
| EI08-24-02R | EI08-24 | 1M24 | 4-[4-[4-(5-Hydroxypyridin-3-yl)thiophene-2-carbonyl]piperazin-1-yl]-N-propylbenzamide |
| EI08-25-02R | EI08-25 | 1M25 | 4-[4-[4-(3-Hydroxyphenyl)-3-methylbenzoyl]piperazin-1-yl]-N-propylbenzamide |
| EI08-26-02R | EI08-26 | 1M26 | 4-[4-[4-(5-Hydroxypyridin-3-yl)-3-methylbenzoyl]piperazin-1-yl]-N-propylbenzamide |
| EI08-27-02R | EI08-27 | 1M27 | 4-[4-[3-luoro-4-(3-hydroxyphenyl)benzoyl]piperazin-1-yl]-N-propylbenzamide |
| EI08-28-02R | EI08-28 | 1M28 | 4-[4-[3-Fluoro-4-(5-hydroxypyridin-3 -yl)benzoyl]piperazin-1 - yl]-N-propylbenzamide |
| EI08-29-02R | EI08-29 | 1M29 | 4-[4-[2-[2-(3-Hydroxyphenyl)ethynyl]benzoyl]piperazin-1-yl]-N-propylbenzamide |
| EI08-30-02R | EI08-30 | 1M30 | 4-[4-[2-[2-(5-Hydroxypyridin-3-yl)ethynyl]benzoyl]piperazin-1-yl]-N-propylbenzamide |
| EI08-31-02R | EI08-31 | 1M31 | 4-[4-[5-[2-(3-Hydroxyphenyl)ethynyl]pyridine-3-carbonyl]piperazin-1-yl]-N-propylbenzamide |
| EI08-32-02R | EI08-32 | 1M32 | 4-[4-[5-[2-(5-Hydroxypyridin-3-yl)ethynyl]pyridine-3-carbonyl]piperazin-1-yl]-N-propylbenzamide |
| EI08-33-02R | EI08-33 | 1M33 | 4-[4-[3-[2-(3 -Hydroxyphenyl)phenyl]propanoyl]piperazin-1 - yl]-N-propylbenzamide |
| EI08-34-02R | EI08-34 | 1M34 | 4-[4-[3-[2-(5-Hydroxypyridin-3-yl)phenyl]propanoyl]piperazin-1-yl]-N-propylbenzamide |
| EI08-35-02R | EI08-35 | 1M35 | 4-[4-[4-(3-Hydroxyphenyl)naphthalene-1-carbonyl]piperazin-1-yl1-N-propylbenzamide |
| EI08-36-02R | EI08-36 | 1M36 | 4-[4-[4-(5-Hydroxypyridin-3-yl)naphthalene-1-carbonyl]piperazin-1-yl]-N-propylbenzamide |

(continued)

| Resin compound No. | Post-cleavage compound No. | Another name of post-cleavage compound in Table EJ02 | Compound name |
|---|---|---|---|
| EI08-37-02R | EI08-37 | 1M37 | 4-[4-[3-tert-Butyl-5-(3-hydroxyphenyl)benzoyl]piperazin-1-yl]-N-propylbenzamide |
| EI08-38-02R | EI08-38 | 1M38 | 4-[4-[3-tert-Butyl-5-(5-hydroxypyridin-3-yl)benzoyl]piperazin-1-yl]-N-propylbenzamide |
| EI08-39-02R | EI08-39 | 1M39 | 4-[4-[3-(3-Hydroxyphenyl)-5-(trifluoromethyl)benzoyl]piperazin-1-yl]-N-propylbenzamide |
| EI08-40-02R | EI08-40 | 1M40 | 4-[4-[3-(5-Hydroxypyridin-3-yl)-5-(trifluoromethyl)benzoyl]piperazin-1-yl]-N-propylbenzamide |
| EI08-41-02R | EI08-41 | 1M41 | 4-[4-[3-[2-(3-Hydroxyphenyl)ethynyl]-5-(trifluoromethyl)benzoyl]piperazin-1-yl]-N-propylbenzamide |
| EI08-42-02R | EI08-42 | 1M42 | 4-[4-[3-[2-(5-Hydroxypyridin-3-yl)ethynyl]-5-(trifluoromethyl)benzoyl]piperazin-1-yl]-N-propylbenzamide |
| EI08-43-02R | EI08-43 | 1M43 | 4-[4-[3-[4-(3-Hydroxyphenyl)pyrazol-1-yl]-5-(trifluoromethyl)benzoyl]piperazin-1 -yl] -N-propylbenzamide |
| EI08-44-02R | EI08-44 | 1M44 | 4-[4-[3-[4-(5-Hydroxypyridin-3-yl)pyrazol-1-yl]-5-(trifluoromethyl)benzoyl]piperazin-1-yl]-N-propylbenzamide |
| EI08-45-02R | EI08-45 | 1M45 | 4-[4-[[2-(3-Hydroxyphenyl)phenyl]methyl]piperazin-1-yl]-N-propylbenzamide |
| EI08-46-02R | EI08-46 | 1M46 | 4-[4-[[2-(5-Hydroxypyridin-3-yl)phenyl]methyl]piperazin-1-yl]-N-propylbenzamide |
| EI08-47-02R | EI08-47 | 1M47 | 4-[4-[[4-(3-Hydroxyphenyl)-3-methylphenyl]methyl]piperazin-1-yl]-N-propylbenzamide |
| EI08-48-02R | EI08-48 | 1M48 | 4-[4-[[4-(5-Hydroxypyridin-3-yl)-3-methylphenyl]methyl]piperazin-1-yl]-N-propylbenzamide |
| EI08-49-02R | EI08-49 | 1M49 | 4-[4-[[2-Ethyl-4-(3-hydroxyphenyl)phenyl]methyl]piperazin-1-yl]-N-propylbenzamide |
| EI08-50-02R | EI08-50 | 1M50 | 4-[4-[[2-Ethyl-4-(5-hydroxypyridin-3-yl)phenyl]methyl]piperazin-1-yl]-N-propylbenzamide |
| EI08-51-02R | EI08-51 | 1M51 | 4-[4-[[3-Fluoro-4-(3-hydroxyphenyl)phenyl]methyl]piperazin-1-yl]-N-propylbenzamide |
| EI08-52-02R | EI08-52 | 1M52 | 4-[4-[[3-Fluoro-4-(5-hydroxypyridin-3-yl)phenyl]methyl]piperazin-1-yl]-N-propylbenzamide |
| EI08-53-02R | EI08-53 | 1M53 | 4-[4-[[4-(3-Hydroxyphenyl)-2-methoxyphenyl]methyl]piperazin-1-yl]-N-propylbenzamide |

(continued)

| Resin compound No. | Post-cleavage compound No. | Another name of post-cleavage compound in Table EJ02 | Compound name |
|---|---|---|---|
| EI08-54-02R | EI08-54 | 1M54 | 4-[4-[[4-(5-Hydroxypyridin-3-yl)-2-methoxyphenyl]methyl]piperazin-1-yl]-N-propylbenzamide |
| EI08-55-02R | EI08-55 | 1M55 | 4-[4-[[3-Ethoxy-5-(3-hydroxyphenyl)phenyl]methyl]piperazin-1-yl]-N-propylbenzamide |
| EI08-56-02R | EI08-56 | 1M56 | 4-[4-[[3-Ethoxy-5-(5-hydroxypyridin-3-yl)phenyl]methyl]piperazin-1-yl]-N-propylbenzamide |
| EI08-57-02R | EI08-57 | 1M57 | 4-[4-[[3-(3 -Hydroxyphenyl)-5-propan-2-yloxyphenyl]methyl]piperazin-1-yl]-N-propylbenzamide |
| EI08-58-02R | EI08-58 | 1M58 | 4-[4-[[3-(5-Hydroxypyridin-3-yl)-5-propan-2-yloxyphenyl]methyl]piperazin-1-yl]-N-propylbenzamide |
| EI08-59-02R | EI08-59 | 1M59 | 4-[4-[[4-(3-Hydroxyphenyl)-2,6-dimethoxyphenyl]methyl]piperazin-1-yl]-N-propylbenzamide |
| EI08-60-02R | EI08-60 | 1M60 | 4-[4-[[4-(5-Hydroxypyridin-3-yl)-2,6-dimethoxyphenyl]methyl]piperazin-1-yl]-N-propylbenzamide |
| EI08-61-02R | EI08-61 | 1M61 | 4-[4-[[3-(3-Hydroxyphenyl)-5-(trifluoromethyl)phenyl]methyl]piperazin-1 -yl]-N-propylbenzamide |
| EI08-62-02R | EI08-62 | 1M62 | 4-[4-[[3-(5-Hydroxypyridin-3-yl)-5-(trifluoromethyl)phenyl]methyl]piperazin-1 -yl]-N-propylbenzamide |
| EI08-63-02R | EI08-63 | 1M63 | 4-[4-[[3-(3-Hydroxyphenyl)-5-(trifluoromethoxy)phenyl]methyl]piperazin-1 -yl]-N-propylbenzamide |
| EI08-64-02R | EI08-64 | 1M64 | 4-[4-[[3-(5-Hydroxypyridin-3-yl)-5-(trifluoromethoxy)phenyl]methyl]piperazin-1 -yl]-N-propylbenzamide |
| EI08-65-02R | EI08-65 | 1M65 | 4-[4-[[4-(3-Hydroxyphenyl)naphthalen-1-yl]methyl]piperazin-1-yl]-N-propylbenzamide |
| EI08-66-02R | EI08-66 | 1M66 | 4-[4-[[4-(5-Hydroxypyridin-3-yl)naphthalen-1-yl]methyl]piperazin-1-yl]-N-propylbenzamide |
| EI08-67-02R | EI08-67 | 1M67 | 4-[4-[[4-(3-Hydroxyphenyl)thiophen-2-yl]methyl]piperazin-1-yl]-N-propylbenzamide |
| EI08-68-02R | EI08-68 | 1M68 | 4-[4-[[4-(5-Hydroxypyridin-3-yl)thiophen-2-yl]methyl]piperazin-1-yl]-N-propylbenzamide |
| EI08-69-02R | EI08-69 | 1M69 | 4-[4-[[2-[2-(3-Hydroxyphenyl)ethynyl]phenyl]methyl]piperazin-1-yl] -N-propylbenzamide |
| EI08-70-02R | EI08-70 | 1M70 | 4-[4-[[2-[2-(5-Hydroxypyridin-3-yl)ethynyl]phenyl]methyl]piperazin-1-yl]-N-propylbenzamide |

(continued)

| Resin compound No. | Post-cleavage compound No. | Another name of post-cleavage compound in Table EJ02 | Compound name |
|---|---|---|---|
| EI08-71-02R | EI08-71 | 1M71 | 6-[4-[[2-(3-Hydroxyphenyl)phenyl]methyl]piperazin-1-yl]-N-propylpyridazine-3-carboxamide |
| EI08-72-02R | EI08-72 | 1M72 | 6-[4-[[2-(5-Hydroxypyridin-3-yl)phenyl]methyl]piperazin-1-yl]-N-propylpyridazine-3 -carboxamide |
| EI08-73-02R | EI08-73 | 1M73 | 6-[4-[[4-(3-Hydroxyphenyl)-3-methylphenyl]methyl]piperazin-1 -yl] -N-propylpyridazine-3 - carboxamide |
| EI08-74-02R | EI08-74 | 1M74 | 6-[4-[[4-(5-Hydroxypyridin-3-yl)-3-methylphenyl]methyl]piperazin-1 -yl] -N-propylpyridazine-3 - carboxamide |
| EI08-75-02R | EI08-75 | 1M75 | 6-[4-[[2-Ethyl-4-(3-hydroxyphenyl)phenyl]methyl]piperazin-1-yl]-N-propylpyridazine-3-carboxamide |
| EI08-76-02R | EI08-76 | 1M76 | 6-4 [[2-Ethyl-4-(5-hydroxypyridin-3-yl)phenyl]methyl]piperazin-1-yl]-N-propylpyridazine-3-carboxamide |
| EI08-77-02R | EI08-77 | 1M77 | 6-[4-[[3-Fluoro-4-(3-hydroxyphenyl)phenyl]methyl]piperazin-1 -yl] -N-propylpyridazine-3 -carboxamide |
| EI08-78-02R | EI08-78 | 1M78 | 6-[4-[[3-Fluoro-4-(5-hydroxypyridin-3-yl)phenyl]methyl]piperazin-1-yl]-N-propylpyridazine-3-carboxamide |
| EI08-79-02R | EI08-79 | 1M79 | 6-[4-[[4-(3-Hydroxyphenyl)-2-methoxyphenyl]methyl]piperazin-1-yl]-N-propylpyridazine-3-carboxamide |
| EI08-80-02R | EI08-80 | 1M80 | 6-[4-[[4-(5-Hydroxypyridin-3-yl)-2-methoxyphenyl]methyl]piperazin-1-yl]-N-propylpyridazine-3-carboxamide |
| EI08-81-02R | EI08-81 | 1M81 | 6-[4-[[3-Ethoxy-5-(3-hydroxyphenyl)phenyl]methyl]piperazin-1-yl]-N-propylpyridazine-3-carboxamide |
| EI08-82-02R | EI08-82 | 1M82 | 6-[4-[[3-Ethoxy-5-(5-hydroxypyridin-3-yl)phenyl]methyl]piperazin-1-yl]-N-propylpyridazine-3-carboxamide |
| EI08-83-02R | EI08-83 | 1M83 | 6-[4-[[3-(3-Hydroxyphenyl)-5-propan-2-yloxyphenyl]methyl]piperazin-1-yl]-N-propylpyridazine-3-carboxamide |
| EI08-84-02R | EI08-84 | 1M84 | 6-[4-[[3-(5-Hydroxypyridin-3-yl)-5-propan-2-yloxyphenyl]methyl]piperazin-1-yl]-N-propylpyridazine-3-carboxamide |
| EI08-85-02R | EI08-85 | 1M85 | 6-[4-[[4-(3-Hydroxyphenyl)-2,6-dimethoxyphenyl]methyl]piperazin-1-yl]-N-propylpyridazine-3-carboxamide |

(continued)

| Resin compound No. | Post-cleavage compound No. | Another name of post-cleavage compound in Table EJ02 | Compound name |
|---|---|---|---|
| EI08-86-02R | EI08-86 | 1M86 | 6-[4-[[4-(5-Hydroxypyridin-3-yl)-2,6-dimethoxyphenyl]methyl]piperazin-1-yl]-N-propylpyridazine-3-carboxamide |
| EI08-87-02R | EI08-87 | 1M87 | 6-[4-[[3-(3-Hydroxyphenyl)-5-(trifluoromethyl)phenyl]methyl]piperazin-1 -yl]-N-propylpyridazine-3-carboxamide |
| EI08-88-02R | EI08-88 | 1M88 | 6-[4-[[3-(5-Hydroxypyridin-3-yl)-5-(trifluoromethyl)phenyl]methyl]piperazin-1 -yl]-N-propylpyridazine-3-carboxamide |
| EI08-89-02R | EI08-89 | 1M89 | 6-[4-[[3-(3-Hydroxyphenyl)-5-(trifluoromethoxy)phenyl]methyl]piperazin-1 -yl]-N-propylpyridazine-3-carboxamide |
| EI08-90-02R | EI08-90 | 1M90 | 6-[4-[[3-(5-Hydroxypyridin-3-yl)-5-(trifluoromethoxy)phenyl]methyl]piperazin-1 -yl]-N-propylpyridazine-3-carboxamide |
| EI08-91-02R | EI08-91 | 1M91 | 6-[4-[[4-(3 -Hydroxyphenyl)naphthalen-1 -yl]methyl]piperazin-1-yl]-N-propylpyridazine-3-carboxamide |
| EI08-92-02R | EI08-92 | 1M92 | 6-[4-[[4-(5-Hydroxypyridin-3-yl)naphthalen-1-yl]methyl]piperazin-1-yl]-N-propylpyridazine-3-carboxamide |
| EI08-93-02R | EI08-93 | 1M93 | 6-[4-[[4-(3-Hydroxyphenyl)thiophen-2-yl]methyl]piperazin-1-yl]-N-propylpyridazine-3-carboxamide |
| EI08-94-02R | EI08-94 | 1M94 | 6-[4-[[4-(5-Hydroxypyridin-3-yl)thiophen-2-yl]methyl]piperazin-1-yl]-N-propylpyridazine-3-carboxamide |
| EI08-95-02R | EI08-95 | 1M95 | 6-[4-[[2-[2-(3-Hydroxyphenyl)ethynyl]phenyl]methyl]piperazin-1-yl] -N-propylpyridazine-3-carboxamide |
| EI08-96-02R | EI08-96 | 1M96 | 6-[4-[[2-[2-(5-Hydroxypyridin-3-yl)ethynyl]phenyl]methyl]piperazin-1-yl]-N-propylpyridazine-3-carboxamide |
| EI08-97-02R | EI08-97 | 1M97 | 4-[4-[1-[4-(3-Hydroxyphenyl)phenyl]ethyl]piperazin-1-yl] -N-propylbenzamide |
| EI08-98-02R | EI08-98 | 1M98 | 4-[4-[1-[4-(5-Hydroxypyridin-3-yl)phenyl]ethyl]piperazin-1-yl]-N-propylbenzamide |
| EI08-99-02R | EI08-99 | 1M99 | 4-[4-[1-[2-Fluoro-4-(3-hydroxyphenyl)phenyl]ethyl]piperazin-1-yl1-N-propylbenzamide |
| EI08-100-02R | EI08-100 | 1M100 | 4-[4-[1-[2-Fluoro-4-(5-hydroxypyridin-3-yl)phenyl]ethyl]piperazin-1-yl]-N-propylbenzamide |

[Table 63]

[Table EI09] Mixture No.: mixEI09-02R, amine reagent used: BB32, post-cleavage mixture No.: mixEI09

| Resin compound No. | Post-cleavage compound No. | Another name of post-cleavage compound in Table EJ03 | Compound name |
|---|---|---|---|
| EI09-01-02R | EI09-01 | 1K01 | 6-[4-[4-(3-Hydroxyphenyl)thiophene-2-carbonyl]piperazin-1-yl]-N-propylpyridazine-3-carboxamide |
| EI09-02-02R | EI09-02 | 1K02 | 6-[4-[4-(5-Hydroxypyridin-3-yl)thiophene-2-carbonyl]piperazin-1-yl]-N-propylpyridazine-3-carboxamide |
| EI09-03-02R | EI09-03 | 1K03 | 6-[4-[4-(3-Hydroxyphenyl)-3-methylbenzoyl]piperazin-1-yl]-N-propylpyridazine-3-carboxamide |
| EI09-04-02R | EI09-04 | 1K04 | 6-[4-[4-(5-Hydroxypyridin-3-yl)-3-methylbenzoyl]piperazin-1-yl]-N-propylpyridazine-3-carboxamide |
| EI09-05-02R | EI09-05 | 1K05 | 6-[4-[3-Fluoro-4-(3-hydroxyphenyl)benzoyl]piperazin-1-yl]-N-propylpyridazine-3-carboxamide |
| EI09-06-02R | EI09-06 | 1K06 | 6-[4-[3-Fluoro-4-(5-hydroxypyridin-3-yl)benzoyl]piperazin-1- yl]-N-propylpyridazine-3-carboxamide |
| EI09-07-02R | EI09-07 | 1K07 | 6-[4-[2-[2-(3-Hydroxyphenyl)ethynyl]benzoyl]piperazin-1-yl]-N-propylpyridazine-3-carboxamide |
| EI09-08-02R | EI09-08 | 1K08 | 6-[4-[2-[2-(5-Hydroxypyridin-3-yl)ethynyl]benzoyl]piperazin-1-yl]-N-propylpyridazine-3-carboxamide |
| EI09-09-02R | EI09-09 | 1K09 | 6-[4-[5-[2-(3-Hydroxyphenyl)ethynyl]pyridine-3-carbonyl]piperazin-1-yl]-N-propylpyridazine-3-carboxamide |
| EI09-10-02R | EI09-10 | 1K10 | 6-[4-[5-[2-(5-Hydroxypyridin-3-yl)ethynyl]pyridine-3-carbonyl]piperazin-1-yl]-N-propylpyridazine-3 -carboxamide |
| EI09-11-02R | EI09-11 | 1K11 | 6-[4-[3-[2-(3-Hydroxyphenyl)phenyl]propanoyl]piperazin-1- yl]-N-propylpyridazine-3-carboxamide |
| EI09-12-02R | EI09-12 | 1K12 | 6-[4-[3-[2-(5-Hydroxypyridin-3-yl)phenyl]propanoyl]piperazin-1-yl]-N-propylpyridazine-3-carboxamide |
| EI09-13-02R | EI09-13 | 1K13 | 6-[4-[4-(3-Hydroxyphenyl)naphthalene-1-carbonyl]piperazin-1-yl]-N-propylpyridazine-3-carboxamide |
| EI09-14-02R | EI09-14 | 1K14 | 6-[4-[4-(5-Hydroxypyridin-3-yl)naphthalene-1-carbonyl]piperazin-1-yl]-N-propylpyridazine-3-carboxamide |
| EI09-15-02R | EI09-15 | 1K15 | 6-[4-[3-tert-Butyl-5-(3-hydroxyphenyl)benzoyl]piperazin-1-yl]-N-propylpyridazine-3-carboxamide |

(continued)

| Resin compound No. | Post-cleavage compound No. | Another name of post-cleavage compound in Table EJ03 | Compound name |
|---|---|---|---|
| EI09-16-02R | EI09-16 | 1K16 | 6-[4-[3-tert-Butyl-5-(5-hydroxypyridin-3-yl)benzoyl]piperazin-1-yl]-N-propylpyridazine-3-carboxamide |
| EI09-17-02R | EI09-17 | 1K17 | 6-[4-[3-(3-Hydroxyphenyl)-5-(trifluoromethyl)benzoyl]piperazin-1-yl]-N-propylpyridazine-3-carboxamide |
| EI09-18-02R | EI09-18 | 1K18 | 6-[4-[3-(5-Hydroxypyridin-3-yl)-5-(trifluoromethyl)benzoyl]piperazin-1-yl]-N-propylpyridazine-3-carboxamide |
| EI09-19-02R | EI09-19 | 1K19 | 6-[4-[3-[2-(3-Hydroxyphenyl)ethynyl]-5-(trifluoromethyl)benzoyl]piperazin-1-yl]-N-propylpyridazine-3-carboxamide |
| EI09-20-02R | EI09-20 | 1K20 | 6-[4-[3-[2-(5-Hydroxypyridin-3-yl)ethynyl]-5-(trifluoromethyl)benzoyl]piperazin-1-yl]-N-propylpyridazine-3-carboxamide |
| EI09-21-02R | EI09-21 | 1K21 | 6-[4-[3-[4-(3-Hydroxyphenyl)pyrazol-1-yl]-5-(trifluoromethyl)benzoyl]piperazin-1-yl]-N-propylpyridazine-3-carboxamide |
| EI09-22-02R | EI09-22 | 1K22 | 6-[4-[3-[4-(5-Hydroxypyridin-3-yl)pyrazol-1-yl]-5-(trifluoromethyl)benzoyl]piperazin-1-yl]-N-propylpyridazine-3-carboxamide |
| EI09-23-02R | EI09-23 | 1K23 | 4-[4-[4-(3-Hydroxyphenyl)thiophene-2-carbonyl]piperazin-1-yl]-N-propylbenzamide |
| EI09-24-02R | EI09-24 | 1K24 | 4-[4-[4-(5-Hydroxypyridin-3-yl)thiophene-2-carbonyl]piperazin-1-yl]-N-propylbenzamide |
| EI09-25-02R | EI09-25 | 1K25 | 4-[4-[4-(3-Hydroxyphenyl)-3-methylbenzoyl]piperazin-1-yl]-N-propylbenzamide |
| EI09-26-02R | EI09-26 | 1K26 | 4-[4-[4-(5-Hydroxypyridin-3-yl)-3-methylbenzoyl]piperazin-1-yl]-N-propylbenzamide |
| EI09-27-02R | EI09-27 | 1K27 | 4-[4-[3-Fluoro-4-(3-hydroxyphenyl)benzoyl]piperazin-1-yl]-N-propylbenzamide |
| EI09-28-02R | EI09-28 | 1K28 | 4-[4-[3-Fluoro-4-(5-hydroxypyridin-3-yl)benzoyl]piperazin-1 - yl]-N-propylbenzamide |
| EI09-29-02R | EI09-29 | 1K29 | 4-[4-[2-[2-(3-Hydroxyphenyl)ethynyl]benzoyl]piperazin-1-yl]-N-propylbenzamide |
| EI09-30-02R | EI09-30 | 1K30 | 4-[4-[2-[2-(5-Hydroxypyridin-3-yl)ethynyl]benzoyl]piperazin-1-yl]-N-propylbenzamide |
| EI09-31-02R | EI09-31 | 1K31 | 4-[4-[5-[2-(3-Hydroxyphenyl)ethynyl]pyridine-3-carbonyl]piperazin-1-yl]-N-propylbenzamide |
| EI09-32-02R | EI09-32 | 1K32 | 4-[4-[5-[2-(5-Hydroxypyridin-3-yl)ethynyl]pyridine-3-carbonyl]piperazin-1-yl]-N-propylbenzamide |
| EI09-33-02R | EI09-33 | 1K33 | 4-[4-[3-[2-(3 -Hydroxyphenyl)phenyl]propanoyl]piperazin-1 - yl]-N-propylbenzamide |

(continued)

| Resin compound No. | Post-cleavage compound No. | Another name of post-cleavage compound in Table EJ03 | Compound name |
|---|---|---|---|
| EI09-34-02R | EI09-34 | 1K34 | 4-[4-[3-[2-(5-Hydroxypyridin-3-yl)phenyl]propanoyl]piperazin-1-yl]-N-propylbenzamide |
| EI09-35-02R | EI09-35 | 1K35 | 4-[4-[4-(3-Hydroxyphenyl)naphthalene-1-carbonyl]piperazin-1-yl]-N-propylbenzamide |
| EI09-36-02R | EI09-36 | 1K36 | 4-[4-[4-(5-Hydroxypyridin-3-yl)naphthalene-1-carbonyl]piperazin-1-yl]-N-propylbenzamide |
| EI09-37-02R | EI09-37 | 1K37 | 4-[4-[3-tert-Butyl-5-(3-hydroxyphenyl)benzoyl]piperazin-1-yl]-N-propylbenzamide |
| EI09-38-02R | EI09-38 | 1K38 | 4-[4-[3-tert-Butyl-5-(5-hydroxypyridin-3-yl)benzoyl]piperazin-1-yl]-N-propylbenzamide |
| EI09-39-02R | EI09-39 | 1K39 | 4-[4-[3-(3-Hydroxyphenyl)-5-(trifluoromethyl)benzoyl]piperazin-1-yl]-N-propylbenzamide |
| EI09-40-02R | EI09-40 | 1K40 | 4-[4-[3-(5-Hydroxypyridin-3-yl)-5-(trifluoromethyl)benzoyl]piperazin-1-yl]-N-propylbenzamide |
| EI09-41-02R | EI09-41 | 1K41 | 4-[4-[3-[2-(3-Hydroxyphenyl)ethynyl]-5-(trifluoromethyl)benzoyl]piperazin-1-yl]-N-propylbenzamide |
| EI09-42-02R | EI09-42 | 1K42 | 4-[4-[3-[2-(5-Hydroxypyridin-3-yl)ethynyl]-5-(trifluoromethyl)benzoyl]piperazin-1-yl]-N-propylbenzamide |
| EI09-43-02R | EI09-43 | 1K43 | 4-[4-[3-[4-(3-Hydroxyphenyl)pyrazol-1-yl] -5-(trifluoromethyl)benzoyl]piperazin-1-yl]-N-propylbenzamide |
| EI09-44-02R | EI09-44 | 1K44 | 4-[4-[3-[4-(5-Hydroxypyridin-3-yl)pyrazol-1-yl]-5-(trifluoromethyl)benzoyl]piperazin-1-yl]-N-propylbenzamide |
| EI09-45-02R | EI09-45 | 1K45 | 4-[4-[[2-(3-Hydroxyphenyl)phenyl]methyl]piperazin-1-yl]-N-propylbenzamide |
| EI09-46-02R | EI09-46 | 1K46 | 4-[4-[[2-(5-Hydroxypyridin-3-yl)phenyl]methyl]piperazin-1-yl]-N-propylbenzamide |
| EI09-47-02R | EI09-47 | 1K47 | 4-[4-[[4-(3-Hydroxyphenyl)-3-methylphenyl]methyl]piperazin-1-yl]-N-propylbenzamide |
| EI09-48-02R | EI09-48 | 1K48 | 4-[4-[[4-(5-Hydroxypyridin-3-yl)-3-methylphenyl]methyl]piperazin-1-yl]-N-propylbenzamide |
| EI09-49-02R | EI09-49 | 1K49 | 4-[4-[[2-Ethyl-4-(3-hydroxyphenyl)phenyl]methyl]piperazin-1-yl]-N-propylbenzamide |
| EI09-50-02R | EI09-50 | 1K50 | 4-4 [[2-Ethyl-4-(5-hydroxypyridin-3-yl)phenyl]methyl]piperazin-1-yl]-N-propylbenzamide |

(continued)

| Resin compound No. | Post-cleavage compound No. | Another name of post-cleavage compound in Table EJ03 | Compound name |
|---|---|---|---|
| EI09-51-02R | EI09-51 | 1K51 | 4-[4-[[3-Fluoro-4-(3-hydroxyphenyl)phenyl]methyl]piperazin-1-yl]-N-propylbenzamide |
| EI09-52-02R | EI09-52 | 1K52 | 4-[4-[[3-Fluoro-4-(5-hydroxypyridin-3-yl)phenyl]methyl]piperazin-1-yl]-N-propylbenzamide |
| EI09-53-02R | EI09-53 | 1K53 | 4-[4-[[4-(3-Hydroxyphenyl)-2-methoxyphenyl]methyl]piperazin-1-yl]-N-propylbenzamide |
| EI09-54-02R | EI09-54 | 1K54 | 4-[4-[[4-(5-Hydroxypyridin-3-yl)-2-methoxyphenyl]methyl]piperazin-1-yl]-N-propylbenzamide |
| EI09-55-02R | EI09-55 | 1K55 | 4-[4-[[3-Ethoxy-5-(3-hydroxyphenyl)phenyl]methyl]piperazin-1-yl]-N-propylbenzamide |
| EI09-56-02R | EI09-56 | 1K56 | 4-[4-[[3-Ethoxy-5-(5-hydroxypyridin-3-yl)phenyl]methyl]piperazin-1-yl]-N-propylbenzamide |
| EI09-57-02R | EI09-57 | 1K57 | 4-[4-[[3-(3-Hydroxyphenyl)-5-propan-2-yloxyphenyl]methyl]piperazin-1-yl]-N-propylbenzamide |
| EI09-58-02R | EI09-58 | 1K58 | 4-[4-[[3-(5-Hydroxypyridin-3-yl)-5-propan-2-yloxyphenyl]methyl]piperazin-1-yl]-N-propylbenzamide |
| EI09-59-02R | EI09-59 | 1K59 | 4-[4-[[4-(3-Hydroxyphenyl)-2,6-dimethoxyphenyl]methyl]piperazin-1-yl]-N-propylbenzamide |
| EI09-60-02R | EI09-60 | 1K60 | 4-[4-[[4-(5-Hydroxypyridin-3-yl)-2,6-dimethoxyphenyl]methyl]piperazin-1-yl]-N-propylbenzamide |
| EI09-61-02R | EI09-61 | 1K61 | 4-[4-[[3-(3-Hydroxyphenyl)-5-(trifluoromethyl)phenyl]methyl]piperazin-1-yl]-N-propylbenzamide |
| EI09-62-02R | EI09-62 | 1K62 | 4-[4-[[3-(5-Hydroxypyridin-3-yl)-5-(trifluoromethyl)phenyl]methyl]piperazin-1-yl]-N-propylbenzamide |
| EI09-63-02R | EI09-63 | 1K63 | 4-[4-[[3-(3-Hydroxyphenyl)-5-(trifluoromethoxy)phenyl]methyl]piperazin-1-yl] -N-propylbenzamide |
| EI09-64-02R | EI09-64 | 1K64 | 4-[4-[[3-(5-Hydroxypyridin-3-yl)-5-(trifluoromethoxy)phenyl]methyl]piperazin-1-yl]-N-propylbenzamide |
| EI09-65-02R | EI09-65 | 1K65 | 4-[4-[[4-(3-Hydroxyphenyl)naphthalen-1-yl]methyl]piperazin-1-yl]-N-propylbenzamide |
| EI09-66-02R | EI09-66 | 1K66 | 4-[4-[[4-(5-Hydroxypyridin-3-yl)naphthalen-1-yl]methyl]piperazin-1-yl]-N-propylbenzamide |
| EI09-67-02R | EI09-67 | 1K67 | 4-[4-[[4-(3-Hydroxyphenyl)thiophen-2-yl]methyl]piperazin-1-yl]-N-propylbenzamide |

(continued)

| Resin compound No. | Post-cleavage compound No. | Another name of post-cleavage compound in Table EJ03 | Compound name |
|---|---|---|---|
| EI09-68-02R | EI09-68 | 1K68 | 4-[4-[[4-(5-Hydroxypyridin-3-yl)thiophen-2-yl]methyl]piperazin-1-yl]-N-propylbenzamide |
| EI09-69-02R | EI09-69 | 1K69 | 4-[4-[[2-[2-(3-Hydroxyphenyl)ethynyl]phenyl]methyl]piperazin-1-yl] -N-propylbenzamide |
| EI09-70-02R | EI09-70 | 1K70 | 4-[4-[[2-[2-(5-Hydroxypyridin-3-yl)ethynyl]phenyl]methyl]piperazin-1-yl]-N-propylbenzamide |
| EI09-71-02R | EI09-71 | 1K71 | 6-[4-[[2-(3-Hydroxyphenyl)phenyl]methyl]piperazin-1-yl]-N-propylpyridazine-3-carboxamide |
| EI09-72-02R | EI09-72 | 1K72 | 6-[4-[[2-(5-Hydroxypyridin-3-yl)phenyl]methyl]piperazin-1-yl]-N-propylpyridazine-3-carboxamide |
| EI09-73-02R | EI09-73 | 1K73 | 6-[4-[[4-(3-Hydroxyphenyl)-3-methylphenyl]methyl]piperazin-1-yl]-N-propylpyridazine-3- carboxamide |
| EI09-74-02R | EI09-74 | 1K74 | 6-[4-[[4-(5-Hydroxypyridin-3-yl)-3-methylphenyl]methyl]piperazin-1-yl]-N-propylpyridazine-3- carboxamide |
| EI09-75-02R | EI09-75 | 1K75 | 6-[4-[[2-Ethyl-4-(3-hydroxyphenyl)phenyl]methyl]piperazin-1-yl]-N-propylpyridazine-3-carboxamide |
| EI09-76-02R | EI09-76 | 1K76 | 6-[4-[[2-Ethyl-4-(5-hydroxypyridin-3-yl)phenyl]methyl]piperazin-1-yl]-N-propylpyridazine-3-carboxamide |
| EI09-77-02R | EI09-77 | 1K77 | 6-[4-[[3-Fluoro-4-(3-hydroxyphenyl)phenyl]methyl]piperazin-1-yl]-N-propylpyridazine-3-carboxamide |
| EI09-78-02R | EI09-78 | 1K78 | 6-4-[[3-Fluoro-4-(5-hydroxypyridin-3-yl)phenyl]methyl]piperazin-1-yl]-N-propylpyridazine-3-carboxamide |
| EI09-79-02R | EI09-79 | 1K79 | 6-[4-[[4-(3-Hydroxyphenyl)-2-methoxyphenyl]methyl]piperazin-1-yl]-N-propylpyridazine-3-carboxamide |
| EI09-80-02R | EI09-80 | 1K80 | 6-[4-[[4-(5-Hydroxypyridin-3-yl)-2-methoxyphenyl]methyl]piperazin-1-yl]-N-propylpyridazine-3-carboxamide |
| EI09-81-02R | EI09-81 | 1K81 | 6-[4-[[3-Ethoxy-5-(3-hydroxyphenyl)phenyl]methyl]piperazin-1-yl]-N-propylpyridazine-3-carboxamide |
| EI09-82-02R | EI09-82 | 1K82 | 6-[4-[[3-Ethoxy-5-(5-hydroxypyridin-3-yl)phenyl]methyl]piperazin-1-yl]-N-propylpyridazine-3-carboxamide |

(continued)

| Resin compound No. | Post-cleavage compound No. | Another name of post-cleavage compound in Table EJ03 | Compound name |
|---|---|---|---|
| EI09-83-02R | EI09-83 | 1K83 | 6-[4-[[3-(3-Hydroxyphenyl)-5-propan-2-yloxyphenyl]methyl]piperazin-1-yl]-N-propylpyridazine-3-carboxamide |
| EI09-84-02R | EI09-84 | 1K84 | 6-[4-[[3-(5-Hydroxypyridin-3-yl)-5-propan-2-yloxyphenyl]methyl]piperazin-1-yl]-N-propylpyridazine-3-carboxamide |
| EI09-85-02R | EI09-85 | 1K85 | 6-[4-[[4-(3-Hydroxyphenyl)-2,6-dimethoxyphenyl]methyl]piperazin-1-yl]-N-propylpyridazine-3-carboxamide |
| EI09-86-02R | EI09-86 | 1K86 | 6-[4-[[4-(5-Hydroxypyridin-3-yl)-2,6-dimethoxyphenyl]methyl]piperazin-1-yl]-N-propylpyridazine-3-carboxamide |
| EI09-87-02R | EI09-87 | 1K87 | 6-[4-[[3-(3-Hydroxyphenyl)-5-(trifluoromethyl)phenyl]methyl]piperazin-1-yl]-N-propylpyridazine-3-carboxamide |
| EI09-88-02R | EI09-88 | 1K88 | 6-[4-[[3-(5-Hydroxypyridin-3-yl)-5-(trifluoromethyl)phenyl]methyl]piperazin-1-yl]-N-propylpyridazine-3-carboxamide |
| EI09-89-02R | EI09-89 | 1K89 | 6-[4-[[3-(3-Hydroxyphenyl)-5-(trifluoromethoxy)phenyl]methyl]piperazin-1-yl]-N-propylpyridazine-3-carboxamide |
| EI09-90-02R | EI09-90 | 1K90 | 6-[4-[[3-(5-Hydroxypyridin-3-yl)-5-(trifluoromethoxy)phenyl]methyl]piperazin-1-yl]-N-propylpyridazine-3-carboxamide |
| EI09-91-02R | EI09-91 | 1K91 | 6-[4-[[4-(3-Hydroxyphenyl)naphthalen-1-yl]methyl]piperazin-1-yl]-N-propylpyridazine-3-carboxamide |
| EI09-92-02R | EI09-92 | 1K92 | 6-[4-[[4-(5-Hydroxypyridin-3-yl)naphthalen-1-yl]methyl]piperazin-1-yl]-N-propylpyridazine-3-carboxamide |
| EI09-93-02R | EI09-93 | 1K93 | 6-[4-[[4-(3-Hydroxyphenyl)thiophen-2-yl]methyl]piperazin-1-yl]-N-propylpyridazine-3-carboxamide |
| EI09-94-02R | EI09-94 | 1K94 | 6-[4-[[4-(5-Hydroxypyridin-3-yl)thiophen-2-yl]methyl]piperazin-1-yl]-N-propylpyridazine-3-carboxamide |
| EI09-95-02R | EI09-95 | 1K95 | 6-[4-[[2-[2-(3-Hydroxyphenyl)ethynyl]phenyl]methyl]piperazin-1-yl]-N-propylpyridazine-3-carboxamide |
| EI09-96-02R | EI09-96 | 1K96 | 6-[4-[[2-[2-(5-Hydroxypyridin-3-yl)ethynyl]phenyl]methyl]piperazin-1-yl]-N-propylpyridazine-3-carboxamide |
| EI09-97-02R | EI09-97 | 1K97 | 4-[4-[1-[4-(3-Hydroxyphenyl)phenyl]ethyl]piperazin-1-yl]-N-propylbenzamide |

(continued)

| Resin compound No. | Post-cleavage compound No. | Another name of post-cleavage compound in Table EJ03 | Compound name |
|---|---|---|---|
| EI09-98-02R | EI09-98 | 1K98 | 4-[4-[1-[4-(5-Hydroxypyridin-3-yl)phenyl]ethyl]piperazin-1- yl]-N-propylbenzamide |
| EI09-99-02R | EI09-99 | 1K99 | 4-[4-[1-[2-Fluoro-4-(3-hydroxyphenyl)phenyl]ethyl]piperazin-1-yl]-N-propylbenzamide |
| EI09-100-02R | EI09-100 | 1K100 | 4-[4-[1-[2-Fluoro-4-(5-hydroxypyridin-3-yl)phenyl]ethyl]piperazin-1-yl]-N-propylbenzamide |

[Table 64]

[Table EI10] Mixture No.: mixEI10-02R, amine reagent used: BB33, post-cleavage mixture No.: mixEI10

| Resin compound No. | Post-cleavage compound No. | Another name of post-cleavage compound in Table EJ01 | Compound name |
|---|---|---|---|
| EI10-01-02R | EI10-01 | 1O01 | 6-[4-[4-(3-Hydroxyphenyl)thiophene-2-carbonyl]piperazin-1-yl]-N-(4-methoxyphenyl)pyridazine-3-carboxamide |
| EI10-02-02R | EI10-02 | 1O02 | 6-[4-[4-(5-Hydroxypyridin-3-yl)thiophene-2-carbonyl]piperazin-1-yl]-N-(4-methoxyphenyl)pyridazine-3-carboxamide |
| EI10-03-02R | EI10-03 | 1O03 | 6-[4-[4-(3-Hydroxyphenyl)-3-methylbenzoyl]piperazin-1-yl]-N-(4-methoxyphenyl)pyridazine-3-carboxamide |
| EI10-04-02R | EI10-04 | 1O04 | 6-[4-[4-(5-Hydroxypyridin-3-yl)-3-methylbenzoyl]piperazin-1-yl]-N-(4-methoxyphenyl)pyridazine-3-carboxamide |
| EI10-05-02R | EI10-05 | 1O05 | 6-[4-[3-Fluoro-4-(3-hydroxyphenyl)benzoyl]piperazin-1-yl]-N-(4-methoxyphenyl)pyridazine-3-carboxamide |
| EI10-06-02R | EI10-06 | 1O06 | 6-[4-[3-Fluoro-4-(5-hydroxypyridin-3-yl)benzoyl]piperazin-1- yl]-N-(4-methoxyphenyl)pyridazine-3-carboxamide |
| EI10-07-02R | EI10-07 | 1O07 | 6-[4-[2-[2-(3-Hydroxyphenyl)ethynyl]benzoyl]piperazin-1-yl]-N-(4-methoxyphenyl)pyridazine-3-carboxamide |
| EI10-08-02R | EI10-08 | 1O08 | 6-[4-[2-[2-(5-Hydroxypyridin-3-yl)ethynyl]benzoyl]piperazin-1-yl]-N-(4-methoxyphenyl)pyridazine-3-carboxamide |
| EI10-09-02R | EI10-09 | 1O09 | 6-[4-[5-[2-(3-Hydroxyphenyl)ethynyl]pyridine-3-carbonyl]piperazin-1-yl]-N-(4-methoxyphenyl)pyridazine-3-carboxamide |
| EI10-10-02R | EI10-10 | 1O10 | 6-[4-[5-[2-(5-Hydroxypyridin-3-yl)ethynyl]pyridine-3-carbonyl]piperazin-1-yl]-N-(4-methoxyphenyl)pyridazine-3-carboxamide |
| EI10-11-02R | EI10-11 | 1O11 | 6-[4-[3-[2-(3-Hydroxyphenyl)phenyl]propanoyl]piperazin-1-yl]-N-(4-methoxyphenyl)pyridazine-3-carboxamide |

(continued)

| Resin compound No. | Post-cleavage compound No. | Another name of post-cleavage compound in Table EJ01 | Compound name |
|---|---|---|---|
| EI10-12-02R | EI10-12 | 1O12 | 6-[4-[3-[2-(5-Hydroxypyridin-3-yl)phenyl]propanoyl]piperazin-1-yl]-N-(4-methoxyphenyl)pyridazine-3-carboxamide |
| EI10-13-02R | EI10-13 | 1O13 | 6-[4-[4-(3-Hydroxyphenyl)naphthalene-1-carbonyl]piperazin-1-yl]-N-(4-methoxyphenyl)pyridazine-3-carboxamide |
| EI10-14-02R | EI10-14 | 1O14 | 6-[4-[4-(5-Hydroxypyridin-3-yl)naphthalene-1-carbonyl]piperazin-1-yl]-N-(4-methoxyphenyl)pyridazine-3-carboxamide |
| EI10-15-02R | EI10-15 | 1O15 | 6-[4-[3-tert-Butyl-5-(3-hydroxyphenyl)benzoyl]piperazin-1-yl]-N-(4-methoxyphenyl)pyridazine-3-carboxamide |
| EI10-16-02R | EI10-16 | 1O16 | 6-[4-[3-tert-Butyl-5-(5-hydroxypyridin-3-yl)benzoyl]piperazin-1-yl]-N-(4-methoxyphenyl)pyridazine-3-carboxamide |
| EI10-17-02R | EI10-17 | 1O17 | 6-[4-[3-(3-Hydroxyphenyl)-5-(trifluoromethyl)benzoyl]piperazin-1-yl]-N-(4-methoxyphenyl)pyridazine-3-carboxamide |
| EI10-18-02R | EI10-18 | 1O18 | 6-[4-[3-(5-Hydroxypyridin-3-yl)-5-(trifluoromethyl)benzoyl]piperazin-1-yl]-N-(4-methoxyphenyl)pyridazine-3-carboxamide |
| EI10-19-02R | EI10-19 | 1O19 | 6-[4-[3-[2-(3-Hydroxyphenyl)ethynyl]-5-(trifluoromethyl)benzoyl]piperazin-1-yl]-N-(4-methoxyphenyl)pyridazine-3-carboxamide |
| EI10-20-02R | EI10-20 | 1O20 | 6-[4-[3-[2-(5-Hydroxypyridin-3-yl)ethynyl]-5-(trifluoromethyl)benzoyl]piperazin-1-yl]-N-(4-methoxyphenyl)pyridazine-3-carboxamide |
| EI10-21-02R | EI10-21 | 1O21 | 6-[4-[3-[4-(3-Hydroxyphenyl)pyrazol-1-yl]-5-(trifluoromethyl)benzoyl]piperazin-1-yl]-N-(4-methoxyphenyl)pyridazine-3-carboxamide |
| EI10-22-02R | EI10-22 | 1O22 | 6-[4-[3-[4-(5-Hydroxypyridin-3-yl)pyrazol-1-yl]-5-(trifluoromethyl)benzoyl]piperazin-1-yl]-N-(4-methoxyphenyl)pyridazine-3-carboxamide |
| EI10-23-02R | EI10-23 | 1O23 | 4-[4-[4-(3-Hydroxyphenyl)thiophene-2-carbonyl]piperazin-1-yl]-N-(4-methoxyphenyl)benzamide |
| EI10-24-02R | EI10-24 | 1O24 | 4-[4-[4-(5-Hydroxypyridin-3-yl)thiophene-2-carbonyl]piperazin-1-yl]-N-(4-methoxyphenyl)benzamide |
| EI10-25-02R | EI10-25 | 1O25 | 4-[4-[4-(3-Hydroxyphenyl)-3-methylbenzoyl]piperazin-1-yl]-N-(4-methoxyphenyl)benzamide |
| EI10-26-02R | EI10-26 | 1O26 | 4-[4-[4-(5-Hydroxypyridin-3-yl)-3-methylbenzoyl]piperazin-1-yl]-N-(4-methoxyphenyl)benzamide |
| EI10-27-02R | EI10-27 | 1O27 | 4-[4-[3-Fluoro-4-(3-hydroxyphenyl)benzoyl]piperazin-1-yl]-N-(4-methoxyphenyl)benzamide |
| EI10-28-02R | EI10-28 | 1O28 | 4-[4-[3-Fluoro-4-(5-hydroxypyridin-3-yl)benzoyl]piperazin-1- yl]-N-(4-methoxyphenyl)benzamide |

(continued)

| Resin compound No. | Post-cleavage compound No. | Another name of post-cleavage compound in Table EJ01 | Compound name |
|---|---|---|---|
| EI10-29-02R | EI10-29 | 1O29 | 4-[4-[2-[2-(3-Hydroxyphenyl)ethynyl]benzoyl]piperazin-1-yl]-N-(4-methoxyphenyl)benzamide |
| EI10-30-02R | EI10-30 | 1O30 | 4-[4-[2-[2-(5-Hydroxypyridin-3-yl)ethynyl]benzoyl]piperazin-1-yl]-N-(4-methoxyphenyl)benzamide |
| EI10-31-02R | EI10-31 | 1O31 | 4-[4-[5-[2-(3-Hydroxyphenyl)ethynyl]pyridine-3-carbonyl]piperazin-1-yl]-N-(4-methoxyphenyl)benzamide |
| EI10-32-02R | EI10-32 | 1O32 | 4-[4-[5-[2-(5-Hydroxypyridin-3-yl)ethynyl]pyridine-3-carbonyl]piperazin-1-yl]-N-(4-methoxyphenyl)benzamide |
| EI10-33-02R | EI10-33 | 1O33 | 4-[4-[3-[2-(3-Hydroxyphenyl)phenyl]propanoyl]piperazin-1-yl]-N-(4-methoxyphenyl)benzamide |
| EI10-34-02R | EI10-34 | 1O34 | 4-[4-[3-[2-(5-Hydroxypyridin-3-yl)phenyl]propanoyl]piperazin-1-yl]-N-(4-methoxyphenyl)benzamide |
| EI10-35-02R | EI10-35 | 1O35 | 4-[4-[4-(3-Hydroxyphenyl)naphthalene-1-carbonyl]piperazin-1-yl]-N-(4-methoxyphenyl)benzamide |
| EI10-36-02R | EI10-36 | 1O36 | 4-[4-[4-(5-Hydroxypyridin-3-yl)naphthalene-1-carbonyl]piperazin-1-yl]-N-(4-methoxyphenyl)benzamide |
| EI10-37-02R | EI10-37 | 1O37 | 4-[4-[3-tert-Butyl-5-(3-hydroxyphenyl)benzoyl]piperazin-1-yl]-N-(4-methoxyphenyl)benzamide |
| EI10-38-02R | EI10-38 | 1O38 | 4-[4-[3-tert-Butyl-5-(5-hydroxypyridin-3-yl)benzoyl]piperazin-1-yl]-N-(4-methoxyphenyl)benzamide |
| EI10-39-02R | EI10-39 | 1O39 | 4-[4-[3-(3-Hydroxyphenyl)-5-(trifluoromethyl)benzoyl]piperazin-1-yl]-N-(4-methoxyphenyl)benzamide |
| EI10-40-02R | EI10-40 | 1O40 | 4-[4-[3-(5-Hydroxypyridin-3-yl)-5-(trifluoromethyl)benzoyl]piperazin-1-yl]-N-(4-methoxyphenyl)benzamide |
| EI10-41-02R | EI10-41 | 1O41 | 4-[4-[3-[2-(3-Hydroxyphenyl)ethynyl]-5-(trifluoromethyl)benzoyl]piperazin-1-yl]-N-(4-methoxyphenyl)benzamide |
| EI10-42-02R | EI10-42 | 1O42 | 4-[4-[3-[2-(5-Hydroxypyridin-3-yl)ethynyl]-5-(trifluoromethyl)benzoyl]piperazin-1-yl]-N-(4-methoxyphenyl)benzamide |
| EI10-43-02R | EI10-43 | 1O43 | 4-[4-[3-[4-(3-Hydroxyphenyl)pyrazol-1-yl]-5-(trifluoromethyl)benzoyl]piperazin-1-yl]-N-(4-methoxyphenyl)benzamide |
| EI10-44-02R | EI10-44 | 1O44 | 4-[4-[3-[4-(5-Hydroxypyridin-3-yl)pyrazol-1-yl]-5-(trifluoromethyl)benzoyl]piperazin-1-yl]-N-(4-methoxyphenyl)benzamide |
| EI10-45-02R | EI10-45 | 1O45 | 4-[4-[[2-(3-Hydroxyphenyl)phenyl]methyl]piperazin-1-yl]-N-(4-methoxyphenyl)benzamide |

(continued)

| Resin compound No. | Post-cleavage compound No. | Another name of post-cleavage compound in Table EJ01 | Compound name |
|---|---|---|---|
| EI10-46-02R | EI10-46 | 1O46 | 4-[4-[[2-(5-Hydroxypyridin-3-yl)phenyl]methyl]piperazin-1-yl]-N-(4-methoxyphenyl)benzamide |
| EI10-47-02R | EI10-47 | 1O47 | 4-[4-[[4-(3-Hydroxyphenyl)-3-methylphenyl]methyl]piperazin-1-yl]-N-(4-methoxyphenyl)benzamide |
| EI10-48-02R | EI10-48 | 1O48 | 4-[4-[[4-(5-Hydroxypyridin-3-yl)-3-methylphenyl]methyl]piperazin-1-yl]-N-(4-methoxyphenyl)benzamide |
| EI10-49-02R | EI10-49 | 1O49 | 4-[4-[[2-Ethyl-4-(3-hydroxyphenyl)phenyl]methyl]piperazin-1-yl]-N-(4-methoxyphenyl)benzamide |
| EI10-50-02R | EI10-50 | 1O50 | 4-[4-[[2-Ethyl-4-(5-hydroxypyridin-3-yl)phenyl]methyl]piperazin-1-yl]-N-(4-methoxyphenyl)benzamide |
| EI10-51-02R | EI10-51 | 1O51 | 4-[4-[[3-Fluoro-4-(3-hydroxyphenyl)phenyl]methyl]piperazin-1-yl]-N-(4-methoxyphenyl)benzamide |
| EI10-52-02R | EI10-52 | 1O52 | 4-[4-[[3-Fluoro-4-(5-hydroxypyridin-3-yl)phenyl]methyl]piperazin-1-yl]-N-(4-methoxyphenyl)benzamide |
| EI10-53-02R | EI10-53 | 1O53 | 4-[4-[[4-(3-Hydroxyphenyl)-2-methoxyphenyl]methyl]piperazin-1-yl]-N-(4-methoxyphenyl)benzamide |
| EI10-54-02R | EI10-54 | 1O54 | 4-[4-[[4-(5-Hydroxypyridin-3-yl)-2-methoxyphenyl]methyl]piperazin-1-yl]-N-(4-methoxyphenyl)benzamide |
| EI10-55-02R | EI10-55 | 1O55 | 4-[4-[[3-Ethoxy-5-(3-hydroxyphenyl)phenyl]methyl]piperazin-1-yl]-N-(4-methoxyphenyl)benzamide |
| EI10-56-02R | EI10-56 | 1O56 | 4-[4-[[3-Ethoxy-5-(5-hydroxypyridin-3-yl)phenyl]methyl]piperazin-1-yl]-N-(4-methoxyphenyl)benzamide |
| EI10-57-02R | EI10-57 | 1O57 | 4-[4-[[3-(3-Hydroxyphenyl)-5-propan-2-yloxyphenyl]methyl]piperazin-1-yl]-N-(4-methoxyphenyl)benzamide |
| EI10-58-02R | EI10-58 | 1O58 | 4-[4-[[3-(5-Hydroxypyridin-3-yl)-5-propan-2-yloxyphenyl]methyl]piperazin-1-yl]-N-(4-methoxyphenyl)benzamide |
| EI10-59-02R | EI10-59 | 1O59 | 4-[4-[[4-(3-Hydroxyphenyl)-2,6-dimethoxyphenyl]methyl]piperazin-1-yl]-N-(4-methoxyphenyl)benzamide |
| EI10-60-02R | EI10-60 | 1O60 | 4-[4-[[4-(5-Hydroxypyridin-3-yl)-2,6-dimethoxyphenyl]methyl]piperazin-1-yl]-N-(4-methoxyphenyl)benzamide |

(continued)

| Resin compound No. | Post-cleavage compound No. | Another name of post-cleavage compound in Table EJ01 | Compound name |
|---|---|---|---|
| EI10-61-02R | EI10-61 | 1O61 | 4-[4-[[3-(3-Hydroxyphenyl)-5-(trifluoromethyl)phenyl]methyl]piperazin-1-yl]-N-(4-methoxyphenyl)benzamide |
| EI10-62-02R | EI10-62 | 1O62 | 4-[4-[[3-(5-Hydroxypyridin-3-yl)-5-(trifluoromethyl)phenyl]methyl]piperazin-1-yl]-N-(4-methoxyphenyl)benzamide |
| EI10-63-02R | EI10-63 | 1O63 | 4-[4-[[3-(3-Hydroxyphenyl)-5-(trifluoromethoxy)phenyl]methyl]piperazin-1-yl]-N-(4-methoxyphenyl)benzamide |
| EI10-64-02R | EI10-64 | 1O64 | 4-[4-[[3-(5-Hydroxypyridin-3-yl)-5-(trifluoromethoxy)phenyl]methyl]piperazin-1-yl]-N-(4-methoxyphenyl)benzamide |
| EI10-65-02R | EI10-65 | 1O65 | 4-[4-[[4-(3-Hydroxyphenyl)naphthalen-1-yl]methyl]piperazin-1-yl]-N-(4-methoxyphenyl)benzamide |
| EI10-66-02R | EI10-66 | 1O66 | 4-[4-[[4-(5-Hydroxypyridin-3-yl)naphthalen-1-yl]methyl]piperazin-1-yl]-N-(4-methoxyphenyl)benzamide |
| EI10-67-02R | EI10-67 | 1O67 | 4-[4-[[4-(3-Hydroxyphenyl)thiophen-2-yl]methyl]piperazin-1-yl]-N-(4-methoxyphenyl)benzamide |
| EI10-68-02R | EI10-68 | 1O68 | 4-[4-[[4-(5-Hydroxypyridin-3-yl)thiophen-2-yl]methyl]piperazin-1-yl]-N-(4-methoxyphenyl)benzamide |
| EI10-69-02R | EI10-69 | 1O69 | 4-[4-[[2-[2-(3-Hydroxyphenyl)ethynyl]phenyl]methyl]piperazin-1-yl]-N-(4-methoxyphenyl)benzamide |
| EI10-70-02R | EI10-70 | 1O70 | 4-[4-[[2-[2-(5-Hydroxypyridin-3-yl)ethynyl]phenyl]methyl]piperazin-1-yl]-N-(4-methoxyphenyl)benzamide |
| EI10-71-02R | EI10-71 | 1O71 | 6-[4-[[2-(3-Hydroxyphenyl)phenyl]methyl]piperazin-1-yl]-N-(4-methoxyphenyl)pyridazine-3-carboxamide |
| EI10-72-02R | EI10-72 | 1O72 | 6-[4-[[2-(5-Hydroxypyridin-3-yl)phenyl]methyl]piperazin-1-yl]-N-(4-methoxyphenyl)pyridazine-3-carboxamide |
| EI10-73-02R | EI10-73 | 1O73 | 6-[4-[[4-(3-Hydroxyphenyl)-3-methylphenyl]methyl]piperazin-1-yl]-N-(4-methoxyphenyl)pyridazine-3-carboxamide |
| EI10-74-02R | EI10-74 | 1O74 | 6-[4-[[4-(5-Hydroxypyridin-3-yl)-3-methylphenyl]methyl]piperazin-1-yl]-N-(4-methoxyphenyl)pyridazine-3-carboxamide |
| EI10-75-02R | EI10-75 | 1O75 | 6-[4-[[2-Ethyl-4-(3-hydroxyphenyl)phenyl]methyl]piperazin-1-yl]-N-(4-methoxyphenyl)pyridazine-3-carboxamide |
| EI10-76-02R | EI10-76 | 1O76 | 6-[4-[[2-Ethyl-4-(5-hydroxypyridin-3-yl)phenyl]methyl]piperazin-1-yl]-N-(4-methoxyphenyl)pyridazine-3-carboxamide |
| EI10-77-02R | EI10-77 | 1O77 | 6-[4-[[3-Fluoro-4-(3-hydroxyphenyl)phenyl]methyl]piperazin-1-yl]-N-(4-methoxyphenyl)pyridazine-3-carboxamide |

(continued)

| Resin compound No. | Post-cleavage compound No. | Another name of post-cleavage compound in Table EJ01 | Compound name |
|---|---|---|---|
| EI10-78-02R | EI10-78 | 1O78 | 6-[4-[[3-Fluoro-4-(5-hydroxypyridin-3-yl)phenyl]methyl]piperazin-1-yl]-N-(4-methoxyphenyl)pyridazine-3-carboxamide |
| EI10-79-02R | EI10-79 | 1O79 | 6-[4-[[4-(3-Hydroxyphenyl)-2-methoxyphenyl]methyl]piperazin-1-yl]-N-(4-methoxyphenyl)pyridazine-3-carboxamide |
| EI10-80-02R | EI10-80 | 1O80 | 6-[4-[[4-(5-Hydroxypyridin-3-yl)-2-methoxyphenyl]methyl]piperazin-1-yl]-N-(4-methoxyphenyl)pyridazine-3-carboxamide |
| EI10-81-02R | EI10-81 | 1O81 | 6-[4-[[3-Ethoxy-5-(3-hydroxyphenyl)phenyl]methyl]piperazin-1-yl]-N-(4-methoxyphenyl)pyridazine-3-carboxamide |
| EI10-82-02R | EI10-82 | 1O82 | 6-[4-[[3-Ethoxy-5-(5-hydroxypyridin-3-yl)phenyl]methyl]piperazin-1-yl]-N-(4-methoxyphenyl)pyridazine-3-carboxamide |
| EI10-83-02R | EI10-83 | 1O83 | 6-[4-[[3-(3-Hydroxyphenyl)-5-propan-2-yloxyphenyl]methyl]piperazin-1-yl]-N-(4-methoxyphenyl)pyridazine-3-carboxamide |
| EI10-84-02R | EI10-84 | 1O84 | 6-[4-[[3-(5-Hydroxypyridin-3-yl)-5-propan-2-yloxyphenyl]methyl]piperazin-1-yl]-N-(4-methoxyphenyl)pyridazine-3-carboxamide |
| EI10-85-02R | EI10-85 | 1O85 | 6-[4-[[4-(3-Hydroxyphenyl)-2,6-dimethoxyphenyl]methyl]piperazin-1-yl]-N-(4-methoxyphenyl)pyridazine-3-carboxamide |
| EI10-86-02R | EI10-86 | 1O86 | 6-[4-[[4-(5-Hydroxypyridin-3-yl)-2,6-dimethoxyphenyl]methyl]piperazin-1-yl]-N-(4-methoxyphenyl)pyridazine-3-carboxamide |
| EI10-87-02R | EI10-87 | 1O87 | 6-[4-[[3-(3-Hydroxyphenyl)-5-(trifluoromethyl)phenyl]methyl]piperazin-1-yl]-N-(4-methoxyphenyl)pyridazine-3-carboxamide |
| EI10-88-02R | EI10-88 | 1O88 | 6-[4-[[3-(5-Hydroxypyridin-3-yl)-5-(trifluoromethyl)phenyl]methyl]piperazin-1-yl]-N-(4-methoxyphenyl)pyridazine-3-carboxamide |
| EI10-89-02R | EI10-89 | 1O89 | 6-[4-[[3-(3-Hydroxyphenyl)-5-(trifluoromethoxy)phenyl]methyl]piperazin-1-yl]-N-(4-methoxyphenyl)pyridazine-3-carboxamide |
| EI10-90-02R | EI10-90 | 1O90 | 6-[4-[[3-(5-Hydroxypyridin-3-yl)-5-(trifluoromethoxy)phenyl]methyl]piperazin-1-yl]-N-(4-methoxyphenyl)pyridazine-3-carboxamide |
| EI10-91-02R | EI10-91 | 1O91 | 6-[4-[[4-(3-Hydroxyphenyl)naphthalen-1-yl]methyl]piperazin-1-yl]-N-(4-methoxyphenyl)pyridazine-3-carboxamide |
| EI10-92-02R | EI10-92 | 1O92 | 6-[4-[[4-(5-Hydroxypyridin-3-yl)naphthalen-1-yl]methyl]piperazin-1-yl]-N-(4-methoxyphenyl)pyridazine-3- carboxamide |

(continued)

| Resin compound No. | Post-cleavage compound No. | Another name of post-cleavage compound in Table EJ01 | Compound name |
|---|---|---|---|
| EI10-93-02R | EI10-93 | 1O93 | 6-[4-[[4-(3-Hydroxyphenyl)thiophen-2-yl]methyl]piperazin-1-yl]-N-(4-methoxyphenyl)pyridazine-3-carboxamide |
| EI10-94-02R | EI10-94 | 1O94 | 6-[4-[[4-(5-Hydroxypyridin-3-yl)thiophen-2-yl]methyl]piperazin-1-yl]-N-(4-methoxyphenyl)pyridazine-3-carboxamide |
| EI10-95-02R | EI10-95 | 1O95 | 6-[4-[[2-[2-(3-Hydroxyphenyl)ethynyl]phenyl]methyl]piperazin-1-yl]-N-(4-methoxyphenyl)pyridazine-3-carboxamide |
| EI10-96-02R | EI10-96 | 1O96 | 6-[4-[[2-[2-(5-Hydroxypyridin-3-yl)ethynyl]phenyl]methyl]piperazin-1-yl]-N-(4-methoxyphenyl)pyridazine-3-carboxamide |
| EI10-97-02R | EI10-97 | 1O97 | 4-[4-[1-[4-(3-Hydroxyphenyl)phenyl]ethyl]piperazin-1-yl]-N-(4-methoxyphenyl)benzamide |
| EI10-98-02R | EI10-98 | 1O98 | 4-[4-[1-[4-(5-Hydroxypyridin-3-yl)phenyl]ethyl]piperazin-1-yl]-N-(4-methoxyphenyl)benzamide |
| EI10-99-02R | EI10-99 | 1O99 | 4-[4-[1-[2-Fluoro-4-(3-hydroxyphenyl)phenyl]ethyl]piperazin-1-yl]-N-(4-methoxyphenyl)benzamide |
| EI10-100-02R | EI10-100 | 1O100 | 4-[4-[1-[2-Fluoro-4-(5-hydroxypyridin-3-yl)phenyl]ethyl]piperazin-1-yl]-N-(4-methoxyphenyl)benzamide |

Example 6-3-8: Synthesis of mixture mixEI11-02R

[Formula 111]

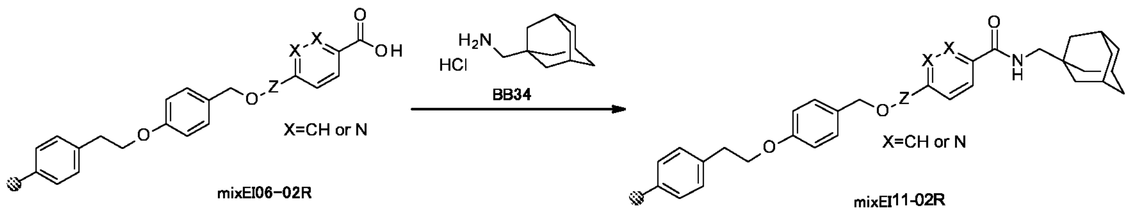


To a 2 mL glass vial were added the mixture mixEI06-02R (50 mg, loading rate 0.648 mmol/g, 0.0324 mmol) shown in [Table EI06] and NMP (1.28 mL) and the mixture was shaken at room temperature for 1 hour. 1-Adamantylmethanamine hydrochloride (BB34, 19.6 mg, 97 μmol), DIC (30.3 μL, 0.194 mmol), HOAt (26.5 mg, 0.194 mmol), and DIPEA (17 μL, 97 μmol) were added, and the mixture was shaken at 40°C for 22 hours. The suspension of the reaction solution and the resins was transferred to a syringe equipped with a filter using a NMP solution. After washing repeatedly three times with NMP (1.2 mL), three times with MeOH (1.2 mL), three times with DCM (1.2 mL), and three times with heptane (1.2 mL), the resins were dried under reduced pressure to obtain the title mixture shown in [Table EI11]. The names of compounds contained in the obtained mixture are shown in [Table EI11].

[Table 64-1]

[Table EI11] Mixture No.: mixEI11-02R, amine reagent used: BB34, post-cleavage mixture No.: mixEI11

| Resin compound No. | Post-cleavage compound No. | Another name of post-cleavage compound in Table EJ05 | Compound name |
|---|---|---|---|
| EI11-01-02R | EI11-01 | 1A01 | N-(1 -Adamantylmethyl)-6-[4-[4-(3 -hydroxyphenyl)thiophene-2-carbonyl]piperazin-1-yl]pyridazine-3-carboxamide |
| EI11-02-02R | EI11-02 | 1A02 | N-(1-Adamantylmethyl)-6-[4-[4-(5-hydroxypyridin-3-yl)thiophene-2-carbonyl]piperazin-1-yl]pyridazine-3-carboxamide |
| EI11-03-02R | EI11-03 | 1A03 | N-(1-Adamantylmethyl)-6-[4-[4-(3-hydroxyphenyl)-3-methylbenzoyl]piperazin-1-yl]pyridazine-3-carboxamide |
| EI11-04-02R | EI11-04 | 1A04 | N-(1-Adamantylmethyl)-6-[4-[4-(5-hydroxypyridin-3-yl)-3-methylbenzoyl]piperazin-1-yl]pyridazine-3-carboxamide |
| EI11-05-02R | EI11-05 | 1A05 | N-(1-Adamantylmethyl)-6-[4-[3-fluoro-4-(3-hydroxyphenyl)benzoyl]piperazin-1-yl]pyridazine-3-carboxamide |
| EI11-06-02R | EI11-06 | 1A06 | N-(1-Adamantylmethyl)-6-[4-[3-fluoro-4-(5-hydroxypyridin-3-yl)benzoyl]piperazin-1-yl]pyridazine-3-carboxamide |
| EI11-07-02R | EI11-07 | 1A07 | N-(1-Adamantylmethyl)-6-[4-[2-[2-(3-hydroxyphenyl)ethynyl]benzoyl]piperazin-1-yl]pyridazine-3-carboxamide |
| EI11-08-02R | EI11-08 | 1A08 | N-(1-Adamantylmethyl)-6-[4-[2-[2-(5-hydroxypyridin-3-yl)ethynyl]benzoyl]piperazin-1-yl]pyridazine-3-carboxamide |
| EI11-09-02R | EI11-09 | 1A09 | N-(1-Adamantylmethyl)-6-[4-[5-[2-(3-hydroxyphenyl)ethynyl]pyridine-3-carbonyl]piperazin-1-yl]pyridazine-3 -carboxamide |
| EI11-10-02R | EI11-10 | 1A10 | N-(1-Adamantylmethyl)-6-[4-[5-[2-(5-hydroxypyridin-3-yl)ethynyl]pyridine-3-carbonyl]piperazin-1-yl]pyridazine-3- carboxamide |
| EI11-11-02R | EI11-11 | 1A11 | N-(1-Adamantylmethyl)-6-[4-[3-[2-(3-hydroxyphenyl)phenyl]propanoyl]piperazin-1-yl]pyridazine-3-carboxamide |
| EI11-12-02R | EI11-12 | 1A12 | N-(1-Adamantylmethyl)-6-[4-[3-[2-(5-hydroxypyridin-3-yl)phenyl]propanoyl]piperazin-1-yl]pyridazine-3-carboxamide |
| EI11-13-02R | EI11-13 | 1A13 | N-(1-Adamantylmethyl)-6-[4-[4-(3-hydroxyphenyl)naphthalene-1 -carbonyl]piperazin-1 - yl]pyridazine-3 -carboxamide |

(continued)

| Resin compound No. | Post-cleavage compound No. | Another name of post-cleavage compound in Table EJ05 | Compound name |
|---|---|---|---|
| EI11-14-02R | EI11-14 | 1A14 | N-(1-Adamantylmethyl)-6-[4-[4-(5-hydroxypyridin-3-yl)naphthalene-1-carbonyl]piperazin-1-yl]pyridazine-3-carboxamide |
| EI11-15-02R | EI11-15 | 1A15 | N-(1-Adamantylmethyl)-6-[4-[3-tert-butyl-5-(3-hydroxyphenyl)benzoyl]piperazin-1-yl]pyridazine-3-carboxamide |
| EI11-16-02R | EI11-16 | 1A16 | N-(1-Adamantylmethyl)-6-[4-[3-tert-butyl-5-(5-hydroxypyridin-3-yl)benzoyl]piperazin-1-yl]pyridazine-3-carboxamide |
| EI11-17-02R | EI11-17 | 1A17 | N-(1-Adamantylmethyl)-6-[4-[3-(3-hydroxyphenyl)-5-(trifluoromethyl)benzoyl]piperazin-1-yl]pyridazine-3-carboxamide |
| EI11-18-02R | EI11-18 | 1A18 | N-(1-Adamantylmethyl)-6-[4-[3-(5-hydroxypyridin-3-yl)-5-(trifluoromethyl)benzoyl]piperazin-1 -yl]pyridazine-3-carboxamide |
| EI11-19-02R | EI11-19 | 1A19 | N-(1-Adamantylmethyl)-6-[4-[3-[2-(3-hydroxyphenyl)ethynyl]-5-(trifluoromethyl)benzoyl]piperazin-1 -yl]pyridazine-3 -carboxamide |
| EI11-20-02R | EI11-20 | 1A20 | N-(1-Adamantylmethyl)-6-[4-[3-[2-(5-hydroxypyridin-3-yl)ethynyl]-5-(trifluoromethyl)benzoyl]piperazin-1 - yl]pyridazine-3-carboxamide |
| EI11-21-02R | EI11-21 | 1A21 | N-(1-Adamantylmethyl)-6-[4-[3-[4-(3-hydroxyphenyl)pyrazol-1 -yl]-5-(trifluoromethyl)benzoyl]piperazin-1-yl]pyridazine-3-carboxamide |
| EI11-22-02R | EI11-22 | 1A22 | N-(1-Adamantylmethyl)-6-[4-[3-[4-(5-hydroxypyridin-3-yl)pyrazol-1-yl]-5-(trifluoromethyl)benzoyl]piperazin-1 - yl]pyridazine-3 -carboxamide |
| EI11-23-02R | EI11-23 | 1A23 | N-(1-Adamantylmethyl)-4-[4-[4-(3-hydroxyphenyl)thiophene-2-carbonyl]piperazin-1-yl]benzamide |
| EI11-24-02R | EI11-24 | 1A24 | N-(1-Adamantylmethyl)-4-[4-[4-(5-hydroxypyridin-3-yl)thiophene-2-carbonyl]piperazin-1-yl]benzamide |
| EI11-25-02R | EI11-25 | 1A25 | N-(1-Adamantylmethyl)-4-[4-[4-(3-hydroxyphenyl)-3-methylbenzoyl]piperazin-1-yl]benzamide |
| EI11-26-02R | EI11-26 | 1A26 | N-(1-Adamantylmethyl)-4-[4-[4-(5-hydroxypyridin-3-yl)-3-methylbenzoyl]piperazin-1-yl]benzamide |
| EI11-27-02R | EI11-27 | 1A27 | N-(1-Adamantylmethyl)-4-[4-[3-fluoro-4-(3-hydroxyphenyl)benzoyl]piperazin-1-yl]benzamide |
| EI11-28-02R | EI11-28 | 1A28 | N-(1-Adamantylmethyl)-4-[4-[3-fluoro-4-(5-hydroxypyridin-3-yl)benzoyl]piperazin-1-yl]benzamide |
| EI11-29-02R | EI11-29 | 1A29 | N-(1-Adamantylmethyl)-4-[4-[2-[2-(3-hydroxyphenyl)ethynyl]benzoyl]piperazin-1-yl]benzamide |
| EI11-30-02R | EI11-30 | 1A30 | N-(1-Adamantylmethyl)-4-[4-[2-[2-(5-hydroxypyridin-3-yl)ethynyl]benzoyl]piperazin-1-yl]benzamide |

(continued)

| Resin compound No. | Post-cleavage compound No. | Another name of post-cleavage compound in Table EJ05 | Compound name |
|---|---|---|---|
| EI11-31-02R | EI11-31 | 1A31 | N-(1-Adamantylmethyl)-4-[4-[5-[2-(3-hydroxyphenyl)ethynyl]pyridine-3 -carbonyl]piperazin-1 -yl]benzamide |
| EI11-32-02R | EI11-32 | 1A32 | N-(1-Adamantylmethyl)-4-[4-[5-[2-(5-hydroxypyridin-3-yl)ethynyl]pyridine-3-carbonyl]piperazin-1-yl]benzamide |
| EI11-33-02R | EI11-33 | 1A33 | N-(1-Adamantylmethyl)-4-[4-[3-[2-(3-hydroxyphenyl)phenyl]propanoyl]piperazin-1-yl]benzamide |
| EI11-34-02R | EI11-34 | 1A34 | N-(1-Adamantylmethyl)-4-[4-[3-[2-(5-hydroxypyridin-3-yl)phenyl]propanoyl]piperazin-1-yl]benzamide |
| EI11-35-02R | EI11-35 | 1A35 | N-(1-Adamantylmethyl)-4-[4-[4-(3-hydroxyphenyl)naphthalene-1 -carbonyl]piperazin-1 - yl]benzamide |
| EI11-36-02R | EI11-36 | 1A36 | N-(1-Adamantylmethyl)-4-[4-[4-(5-hydroxypyridin-3-yl)naphthalene-1-carbonyl]piperazin-1-yl]benzamide |
| EI11-37-02R | EI11-37 | 1A37 | N-(1-Adamantylmethyl)-4-[4-[3-tert-butyl-5-(3-hydroxyphenyl)benzoyl]piperazin-1-yl]benzamide |
| EI11-38-02R | EI11-38 | 1A38 | N-(1-Adamantylmethyl)-4-[4-[3-tert-butyl-5-(5-hydroxypyridin-3-yl)benzoyl]piperazin-1-yl]benzamide |
| EI11-39-02R | EI11-39 | 1A39 | N-(1-Adamantylmethyl)-4-[4-[3-(3-hydroxyphenyl)-5-(trifluoromethyl)benzoyl]piperazin-1 -yl]benzamide |
| EI11-40-02R | EI11-40 | 1A40 | N-(1-Adamantylmethyl)-4-[4-[3-(5-hydroxypyridin-3-yl)-5-(trifluoromethyl)benzoyl]piperazin-1yl]benzamide |
| EI11-41-02R | EI11-41 | 1A41 | N-(1-Adamantylmethyl)-4-[4-[3-[2-(3-hydroxyphenyl)ethynyl]-5-(trifluoromethyl)benzoyl]piperazin-1-yl]benzamide |
| EI11-42-02R | EI11-42 | 1A42 | N-(1-Adamantylmethyl)-4-[4-[3-[2-(5-hydroxypyridin-3-yl)ethynyl]-5-(trifluoromethyl)benzoyl]piperazin-1 - yl]benzamide |
| EI11-43-02R | EI11-43 | 1A43 | N-(1-Adamantylmethyl)-4-[4-[3-[4-(3-hydroxyphenyl)pyrazol-1-yl]-5-(trifluoromethyl)benzoyl]piperazin-1-yl]benzamide |
| EI11-44-02R | EI11-44 | 1A44 | N-(1-Adamantylmethyl)-4-[4-[3-[4-(5-hydroxypyridin-3-yl)pyrazol-1-yl]-5-(trifluoromethyl)benzoyl]piperazin-1 - yl]benzamide |
| EI11-45-02R | EI11-45 | 1A45 | N-(1-Adamantylmethyl)-4-[4-[[2-(3-hydroxyphenyl)phenyl]methyl]piperazin-1-yl]benzamide |
| EI11-46-02R | EI11-46 | 1A46 | N-(1-Adamantylmethyl)-4-[4-[[2-(5-hydroxypyridin-3-yl)phenyl]methyl]piperazin-1-yl]benzamide |
| EI11-47-02R | EI11-47 | 1A47 | N-(1-Adamantylmethyl)-4-[4-[[4-(3-hydroxyphenyl)-3-methylphenyl]methyl]piperazin-1-yl]benzamide |

(continued)

| Resin compound No. | Post-cleavage compound No. | Another name of post-cleavage compound in Table EJ05 | Compound name |
|---|---|---|---|
| EI11-48-02R | EI11-48 | 1A48 | N-(1-Adamantylmethyl)-4-[4-[[4-(5-hydroxypyridin-3-yl)-3-methylpkenyllmethyl]piperazin-1-yl]benzamide |
| EI11-49-02R | EI11-49 | 1A49 | N-(1-Adamantylmethyl)-4-[4-[[2-ethyl-4-(3- hydroxyphenyl)phenyl]methyl]piperazin-1-yl]benzamide |
| EI11-50-02R | EI11-50 | 1A50 | N-(1-Adamantylmethyl)-4-[4-[[2-ethyl-4-(5-hydroxypyridin-3-yl)phenyl]methyl]piperazin-1-yl]benzamide |
| EI11-51-02R | EI11-51 | 1A51 | N-(1-Adamantylmethyl)-4-[4-[[3-fluoro-4-(3-hydroxyphenyl)phenyl]methyl]piperazin-1-yl]benzamide |
| EI11-52-02R | EI11-52 | 1A52 | N-(1-Adamantylmethyl)-4-[4-[[3-fluoro-4-(5-hydroxypyridin-3-yl)phenyl]methyl]piperazin-1-yl]benzamide |
| EI11-53-02R | EI11-53 | 1A53 | N-(1-Adamantylmethyl)-4-[4-[[4-(3-hydroxyphenyl)-2-methoxyphenyl]methyl]piperazin-1-yl]benzamide |
| EI11-54-02R | EI11-54 | 1A54 | N-(1-Adamantylmethyl)-4-[4-[[4-(5-hydroxypyridin-3-yl)-2-methoxyphenyl]methyl]piperazin-1-yl]benzamide |
| EI11-55-02R | EI11-55 | 1A55 | N-(1-Adamantylmethyl)-4-[4-[[3-ethoxy-5-(3- hydroxyphenyl)phenyl]methyl]piperazin-1-yl]benzamide |
| EI11-56-02R | EI11-56 | 1A56 | N-(1-Adamantylmethyl)-4-[4-[[3-ethoxy-5-(5-hydroxypyridin-3-yl)phenyl]methyl]piperazin-1-yl]benzamide |
| EI11-57-02R | EI11-57 | 1A57 | N-(1-Adamantylmethyl)-4-[4-[[3-(3-hydroxyphenyl)-5-propan-2-yloxyphenyl]methyl]piperazin-1-yl]benzamide |
| EI11-58-02R | EI11-58 | 1A58 | N-(1-Adamantylmethyl)-4-[4-[[3-(5-hydroxypyridin-3-yl)-5-propan-2-yloxyphenyl]methyl]piperazin-1-yl]benzamide |
| EI11-59-02R | EI11-59 | 1A59 | N-(1-Adamantylmethyl)-4-[4-[[4-(3-hydroxyphenyl)-2,6-dimethoxyphenyl]methyl]piperazin-1-yl]benzamide |
| EI11-60-02R | EI11-60 | 1A60 | N-(1-Adamantylmethyl)-4-[4-[[4-(5-hydroxypyridin-3-yl)-2,6-dimethoxyphenyllmethyllpiperazin-1-yl]benzamide |
| EI11-61-02R | EI11-61 | 1A61 | N-(1-Adamtlmethyl)-4-[4-[[3-(3-hydroxyphenyl)-5-(trifluoromethyl)phenyl]methyl]piperazin-1-yl]benzamide |
| EI11-62-02R | EI11-62 | 1A62 | N-(1-Adamantylmethyl)-4-[4-[[3-(5-hydroxypyridin-3-yl)-5-(trifluoromethyl)phenyl]methyl]piperazin-1-yl]benzamide |

(continued)

| Resin compound No. | Post-cleavage compound No. | Another name of post-cleavage compound in Table EJ05 | Compound name |
|---|---|---|---|
| EI11-63-02R | EI11-63 | 1A63 | N-(1-Adamantylmethyl)-4-[4-[[3-(3-hydroxyphenyl)-5-(trifluoromethoxy)phenyl]methyl]piperazin-1-yl]benzamide |
| EI11-64-02R | EI11-64 | 1A64 | N-(1-Adamantylmethyl)-4-[4-[[3-(5-hydroxypyridin-3-yl)-5-(trifluoromethoxy)phenyl]methyl]piperazin-1-yl]benzamide |
| EI11-65-02R | EI11-65 | 1A65 | N-(1-Adamantylmethyl)-4-[4-[[4-(3-hydroxyphenyl)naphthalen-1-yl]methyl]piperazin-1-yl]benzamide |
| EI11-66-02R | EI11-66 | 1A66 | N-(1-Adamantylmethyl)-4-[4-[[4-(5-hydroxypyridin-3-yl)naphthalen-1-yl]methyl]piperazin-1-yl]benzamide |
| EI11-67-02R | EI11-67 | 1A67 | N-(1-Adamantylmethyl)-4-[4-[[4-(3-hydroxyphenyl)thiophen-2-yl]methyl]piperazin-1-yl]benzamide |
| EI11-68-02R | EI11-68 | 1A68 | N-(1-Adamantylmethyl)-4-[4-[[4-(5-hydroxypyridin-3-yl)thiophen-2-yl]methyl]piperazin-1-yl]benzamide |
| EI11-69-02R | EI11-69 | 1A69 | N-(1-Adamantylmethyl)-4-[4-[[2-[2-(3-hydroxyphenyl)ethynyl]phenyl]methyl]piperazin-1-yl]benzamide |
| EI11-70-02R | EI11-70 | 1A70 | N-(1-Adamantylmethyl)-4-[4-[[2-[2-(5-hydroxypyridin-3-yl)ethynyl]phenyl]methyl]piperazin-1-yl]benzamide |
| EI11-71-02R | EI11-71 | 1A71 | N-(1-Adamantylmethyl)-6-[4-[[2-(3-hydroxyphenyl)phenyl]methyl]piperazin-1-yl]pyridazine-3-carboxamide |
| EI11-72-02R | EI11-72 | 1A72 | N-(1-Adamantylmethyl)-6-[4-[[2-(5-hydroxypyridin-3-yl)phenyl]methyl]piperazin-1-yl]pyridazine-3-carboxamide |
| EI11-73-02R | EI11-73 | 1A73 | N-(1-Adamantylmethyl)-6-[4-[[4-(3-hydroxyphenyl)-3-methylphenyl]methyl]piperazin-1-yl]pyridazine-3-carboxamide |
| EI11-74-02R | EI11-74 | 1A74 | N-(1-Adamantylmethyl)-6-[4-[[4-(5-hydroxypyridin-3-yl)-3-methylphenyl]methyl]piperazin-1-yl]pyridazine-3-carboxamide |
| EI11-75-02R | EI11-75 | 1A75 | N-(1-Adamantylmethyl)-6-[4-[[2-ethyl-4-(3-hydroxyphenyl)phenyl]methyl]piperazin-1-yl]pyridazine-3-carboxamide |
| EI11-76-02R | EI11-76 | 1A76 | N-(1-Adamantylmethyl)-6-[4-[[2-ethyl-4-(5-hydroxypyridin-3-yl)phenyl]methyl]piperazin-1-yl]pyridazine-3-carboxamide |
| EI11-77-02R | EI11-77 | 1A77 | N-(1-Adamantylmethyl)-6-[4-[[3-fluoro-4-(3-hydroxyphenyl)phenyl]methyl]piperazin-1-yl]pyridazine-3-carboxamide |

(continued)

| Resin compound No. | Post-cleavage compound No. | Another name of post-cleavage compound in Table EJ05 | Compound name |
|---|---|---|---|
| EI11-78-02R | EI11-78 | 1A78 | N-(1-Adamantylmethyl)-6-[4-[[3-fluoro-4-(5-hydroxypyridin-3-yl)phenyl]methyl]piperazin-1-yl]pyridazine-3-carboxamide |
| EI11-79-02R | EI11-79 | 1A79 | N-(1-Adamantylmethyl)-6-[4-[[4-(3-hydroxyphenyl)-2-methoxyphenyl]methyl]piperazin-1-yl]pyridazine-3-carboxamide |
| EI11-80-02R | EI11-80 | 1A80 | N-(1-Adamantylmethyl)-6-[4-[[4-(5-hydroxypyridin-3-yl)-2-methoxyphenyl]methyl]piperazin-1-yl]pyridazine-3-carboxamide |
| EI11-81-02R | EI11-81 | 1A81 | N-(1-Adamantylmethyl)-6-[4-[[3-ethoxy-5-(3-hydroxyphenyl)phenyl]methyl]piperazin-1-yl]pyridazine-3- carboxamide |
| EI11-82-02R | EI11-82 | 1A82 | N-(1-Adamantylmethyl)-6-[4-[[3-ethoxy-5-(5-hydroxypyridin-3-yl)phenyl]methyl]piperazin-1-yl]pyridazine-3-carboxamide |
| EI11-83-02R | EI11-83 | 1A83 | N-(1-Adamantylmethyl)-6-[4-[[3-(3-hydroxyphenyl)-5-propan-2-yloxyphenyl]methyl]piperazin-1-yl]pyridazine-3-carboxamide |
| EI11-84-02R | EI11-84 | 1A84 | N-(1-Adamantylmethyl)-6-[4-[[3-(5-hydroxypyridin-3-yl)-5-propan-2-yloxyphenyl]methyl]piperazin-1-yl]pyridazine-3-carboxamide |
| EI11-85-02R | EI11-85 | 1A85 | N-(1-Adamantylmethyl)-6-[4-[[4-(3-hydroxyphenyl)-2,6-dimethoxyphenyl]methyl]piperazin-1-yl]pyridazine-3-carboxamide |
| EI11-86-02R | EI11-86 | 1A86 | N-(1-Adamantylmethyl)-6-[4-[[4-(5-hydroxypyridm-3-yl)-2,6-dimethoxyphenyl]methyl]piperazin-1-yl]pyridazine-3-carboxamide |
| EI11-87-02R | EI11-87 | 1A87 | N-(1-Adamantylmethyl)-6-[4-[[3-(3-hydroxyphenyl)-5-(trifluoromethyl)phenyl]methyl]piperazin-1-yl]pyridazine-3-carboxamide |
| EI11-88-02R | EI11-88 | 1A88 | N-(1-Adamantylmethyl)-6-[4-[[3-(5-hydroxypyridin-3-yl)-5-(trifluoromethyl)phenyl]methyl]piperazin-1-yl]pyridazine-3-carboxamide |
| EI11-89-02R | EI11-89 | 1A89 | N-(1-Adamantylmethyl)-6-[4-[[3-(3-hydroxyphenyl)-5-(trifluoromethoxy)phenyl]methyl]piperazin-1-yl]pyridazine-3- carboxamide |
| EI11-90-02R | EI11-90 | 1A90 | N-(1-Adamantylmethyl)-6-[4-[[3-(5-hydroxypyridin-3-yl)-5-(trifluoromethoxy)phenyl]methyl]piperazin-1-yl]pyridazine-3- carboxamide |
| EI11-91-02R | EI11-91 | 1A91 | N-(1-Adamantylmethyl)-6-[4-[[4-(3-hydroxyphenyl)naphthalen-1-yl]methyl]piperazin-1-yl]pyridazine-3-carboxamide |

(continued)

| Resin compound No. | Post-cleavage compound No. | Another name of post-cleavage compound in Table EJ05 | Compound name |
|---|---|---|---|
| EI11-92-02R | EI11-92 | 1A92 | N-(1-Adamantylmethyl)-6-[4-[[4-(5-hydroxypyridin-3-yl)naphthalen-1-yl]methyl]piperazin-1-yl]pyridazine-3-carboxamide |
| EI11-93-02R | EI11-93 | 1A93 | N-(1-Adamantylmethyl)-6-[4-[[4-(3-hydroxyphenyl)thiophen-2-yl]methyl]piperazin-1-yl]pyridazine-3-carboxamide |
| EI11-94-02R | EI11-94 | 1A94 | N-(1-Adamantylmethyl)-6-[4-[[4-(5-hydroxypyridin-3-yl)thiophen-2-yl]methyl]piperazin-1-yl]pyridazine-3 - carboxamide |
| EI11-95-02R | EI11-95 | 1A95 | N-(1-Adamantylmethyl)-6-[4-[[2-[2-(3-hydroxyphenyl)ethynyl]phenyl]methyl]piperazin-1-yl]pyridazine-3-carboxamide |
| EI11-96-02R | EI11-96 | 1A96 | N-(1-Adamantylmethyl)-6-[4-[[2-[2-(5-hydroxypyridin-3-yl)ethynyl]phenyl]methyl]piperazin-1 -yl]pyridazine-3 - carboxamide |
| EI11-97-02R | EI11-97 | 1A97 | N-(1-Adamantylmethyl)-4-[4-[1-[4-(3-hydroxyphenyl)phenyl]ethyl]piperazin-1-yl]benzamide |
| EI11-98-02R | EI11-98 | 1A98 | N-(1-Adamantylmethyl)-4-[4-[1-[4-(5-hydroxypyridin-3-yl)phenyl]ethyl]piperazin-1-yl]benzamide |
| EI11-99-02R | EI11-99 | 1A99 | N-(1-Adamantylmethyl)-4-[4-[1-[2-fluoro-4-(3-hydroxyphenyl)phenyl]ethyl]piperazin-1-yl]benzamide |
| EI11-100-02R | EI11-100 | 1A100 | N-(1-Adamantylmethyl)-4-[4-[1-[2-fluoro-4-(5-hydroxypyridin-3-yl)phenyl]ethyl]piperazin-1-yl]benzamide |

Example 6-3-9: Synthesis of mixture mixEI12-02R

[Formula 112]

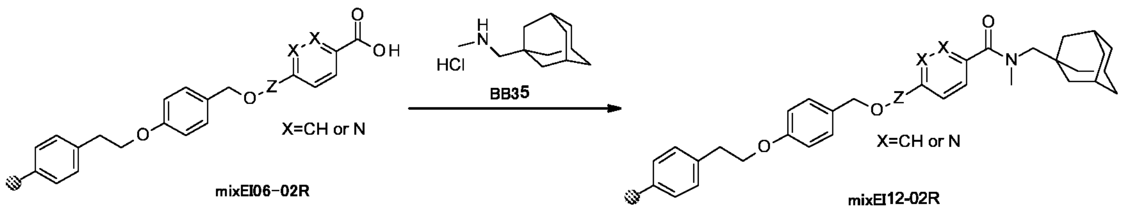


Using the mixture mixEI06-02R (50 mg, loading rate 0.648 mmol/g, 0.0324 mmol) shown in [Table EI06] and 1-(1-adamantyl)-N-methylmethanamine (BB35), the title mixture shown in [Table EI12] was synthesized by the same method as in Example 6-3-8 and obtained. The names of compounds contained in the obtained mixture are shown in [Table EI12].

[Table 64-2]

[Table EI12] Mixture No.: mixEI12-02R, amine reagent used: BB35, post-cleavage mixture No.: mixEI12

| Resin compound No. | Post-cleavage compound No. | Another name of post-cleavage compound in Table EJ06 | Compound name |
|---|---|---|---|
| EI12-01-02R | EI12-01 | 3A01 | N-(1-Adamantylmethyl)-6-[4-[4-(3-hydroxyphenyl)thiophene-2-carbonyl]piperazin-1-yl]-N-methylpyridazine-3-carboxamide |
| EI12-02-02R | EI12-02 | 3A02 | N-(1-Adamantylmethyl)-6-[4-[4-(5-hydroxypyridin-3-yl)thiophene-2-carbonyl]piperazin-1-yl]-N-methylpyridazine-3-carboxamide |
| EI12-03-02R | EI12-03 | 3A03 | N-(1-Adamantylmethyl)-6-[4-[4-(3-hydroxyphenyl)-3-methylbenzoyl]piperazin-1-yl] -N-methylpyridazine-3- carboxamide |
| EI12-04-02R | EI12-04 | 3A04 | N-(1-Adamantylmethyl)-6-[4-[4-(5-hydroxypyridin-3-yl)-3-methylbenzoyl]piperazin-1-yl]-N-methylpyridazine-3-carboxamide |
| EI12-05-02R | EI12-05 | 3A05 | N-(1-Adamantylmethyl)-6-[4-[3-fluoro-4-(3-hydroxyphenyl)benzoyl]piperazin-1-yl]-N-methylpyridazine-3-carboxamide |
| EI12-06-02R | EI12-06 | 3A06 | N-(1-Adamantylmethyl)-6-[4-[3-fluoro-4-(5-hydroxypyridin-3-yl)benzoyl]piperazin-1-yl]-N-methylpyridazine-3-carboxamide |
| EI12-07-02R | EI12-07 | 3A07 | N-(1-Adamantylmethyl)-6-[4-[2-[2-(3-hydroxyphenyl)ethynyl]benzoyl]piperazin-1-yl]-N-methylpyridazine-3-carboxamide |
| EI12-08-02R | EI12-08 | 3A08 | N-(1-Adamantylmethyl)-6-[4-[2-[2-(5-hydroxypyridin-3-yl)ethynyl]benzoyl]piperazin-1-yl]-N-methylpyridazine-3- carboxamide |
| EI12-09-02R | EI12-09 | 3A09 | N-(1-Adamantylmethyl)-6-[4-[5-[2-(3-hydroxyphenyl)ethynyl]pyridine-3-carbonyl]piperazin-1-yl]- N-methylpyridazine-3-carboxamide |
| EI12-10-02R | EI12-10 | 3A10 | N-(1-Adamantylmethyl)-6-[4-[5-[2-(5-hydroxypyridin-3-yl)ethynyl]pyridine-3-carbonyl]piperazin-1-yl]-N-methylpyridazine-3-carboxamide |
| EI12-11-02R | EI12-11 | 3A11 | N-(1-Adamantylmethyl)-6-[4-[3-[2-(3-hydroxyphenyl)phenyl]propanoyl]piperazin-1-yl]-N-methylpyridazine-3-carboxamide |
| EI12-12-02R | EI12-12 | 3A12 | N-(1-Adamantylmethyl)-6-[4-[3-[2-(5-hydroxypyridin-3-yl)phenyl]propanoyl]piperazin-1-yl]-N-methylpyridazine-3-carboxamide |
| EI12-13-02R | EI12-13 | 3A13 | N-(1-Adamantylmethyl)-6-[4-[4-(3-hydroxyphenyl)naphthalene-1-carbonyl]piperazin-1-yl]-N-methylpyridazine-3-carboxamide |

(continued)

| Resin compound No. | Post-cleavage compound No. | Another name of post-cleavage compound in Table EJ06 | Compound name |
|---|---|---|---|
| EI12-14-02R | EI12-14 | 3A14 | N-(1-Adamantylmethyl)-6-[4-[4-(5-hydroxypyridin-3-yl)naphthalene-1-carbonyl]piperazin-1-yl]-N-methylpyridazine-3-carboxamide |
| EI12-15-02R | EI12-15 | 3A15 | N-(1-Adamantylmethyl)-6-[4-[3-tert-butyl-5-(3-hydroxyphenyl)benzoyl]piperazin-1-yl]-N-methylpyridazine-3-carboxamide |
| EI12-16-02R | EI12-16 | 3A16 | N-(1-Adamantylmethyl)-6-[4-[3-tert-butyl-5-(5-hydroxypyridin-3-yl)benzoyl]piperazin-1-yl]-N-methylpyridazine-3-carboxamide |
| EI12-17-02R | EI12-17 | 3A17 | N-(1-Adamantylmethyl)-6-[4-[3-(3-hydroxyphenyl)-5-(trifluoromethyl)benzoyl]piperazin-1-yl]-N-methylpyridazine-3-carboxamide |
| EI12-18-02R | EI12-18 | 3A18 | N-(1-Adamantylmethyl)-6-[4-[3-(5-hydroxypyridin-3-yl)-5-(trifluoromethyl)benzoyl]piperazin-1-yl]-N-methylpyridazine-3-carboxamide |
| EI12-19-02R | EI12-19 | 3A19 | N-(1-Adamantylmethyl)-6-[4-[3-[2-(3-hydroxyphenyl)ethynyl]-5-(trifluoromethyl)benzoyl]piperazin-1-yl]-N-methylpyridazine-3-carboxamide |
| EI12-20-02R | EI12-20 | 3A20 | N-(1-Adamantylmethyl)-6-[4-[3-[2-(5-hydroxypyridin-3-yl)ethynyl]-5-(trifluoromethyl)benzoyl]piperazin-1-yl]-N-methylpyridazine-3-carboxamide |
| EI12-21-02R | EI12-21 | 3A21 | N-(1-Adamantylmethyl)-6-[4-[3-[4-(3-hydroxyphenyl)pyrazol-1-yl]-5- (trifluoromethyl)benzoyl]piperazin-1-yl]-N-methylpyridazine-3-carboxamide |
| EI12-22-02R | EI12-22 | 3A22 | N-(1-Adamantylmethyl)-6-[4-[3-[4-(5-hydroxypyridin-3-yl)pyrazol-1-yl]-5-(trifluoromethyl)benzoyl]piperazin-1-yl]-N-methylpyridazine-3-carboxamide |
| EI12-23-02R | EI12-23 | 3A23 | N-(1-Adamantylmethyl)-4-[4-[4-(3-hydroxyphenyl)thiophene-2-carbonyl]piperazin-1-yl] -N-methylbenzamide |
| EI12-24-02R | EI12-24 | 3A24 | N-(1-Adamantylmethyl)-4-[4-[4-(5-hydroxypyridin-3-yl)thiophene-2-carbonyl]piperazin-1-yl]-N-methylbenzamide |
| EI12-25-02R | EI12-25 | 3A25 | N-(1-Adamantylmethyl)-4-[4-[4-(3-hydroxyphenyl)-3-methylbenzoyl]piperazin-1 -yl]-N-methylbenzamide |
| EI12-26-02R | EI12-26 | 3A26 | N-(1-Adamantylmethyl)-4-[4-[4-(5-hydroxypyridin-3-yl)-3-methylbenzoyl]piperazin-1-yl]-N-methylbenzamide |
| EI12-27-02R | EI12-27 | 3A27 | N-(1-Adamantylmethyl)-4-[4-[3-fluoro-4-(3-hydroxyphenyl)benzoyl]piperazin-1-yl]-N-methylbenzamide |

(continued)

| Resin compound No. | Post-cleavage compound No. | Another name of post-cleavage compound in Table EJ06 | Compound name |
|---|---|---|---|
| EI12-28-02R | EI12-28 | 3A28 | N-(1-Adamantylmethyl)-4-[4-[3-fluoro-4-(5-hydroxypyridin-3-yl)benzoyl]piperazin-1-yl]-N-methylbenzamide |
| EI12-29-02R | EI12-29 | 3A29 | N-(1-Adamantylmethyl)-4-[4-[2-[2-(3-hydroxyphenyl)ethynyl]benzoyl]piperazin-1-yl]-N-methylbenzamide |
| EI12-30-02R | EI12-30 | 3A30 | N-(1-Adamantylmethyl)-4-[4-[2-[2-(5-hydroxypyridin-3-yl)ethynyl]benzoyl]piperazin-1-yl]-N-methylbenzamide |
| EI12-31-02R | EI12-31 | 3A31 | N-(1-Adamantylmethyl)-4-[4-[5-[2-(3-hydroxyphenyl)ethynyl]pyridine-3-carbonyl]piperazin-1-yl]-N-methylbenzamide |
| EI12-32-02R | EI12-32 | 3A32 | N-(1-Adamantylmethyl)-4-[4-[5-[2-(5-hydroxypyridin-3-yl)ethynyl]pyridine-3-carbonyl]piperazin-1-yl]-N-methylbenzamide |
| EI12-33-02R | EI12-33 | 3A33 | N-(1-Adamantylmethyl)-4-[4-[3-[2-(3-hydroxyphenyl)phenyl]propanoyl]piperazin-1-yl]-N-methylbenzamide |
| EI12-34-02R | EI12-34 | 3A34 | N-(1-Adamantylmethyl)-4-[4-[3-[2-(5-hydroxypyridin-3-yl)phenyl]propanoyl]piperazin-1-yl]-N-methylbenzamide |
| EI12-35-02R | EI12-35 | 3A35 | N-(1-Adamantylmethyl)-4-[4-[4-(3-hydroxyphenyl)naphthalene-1-carbonyl]piperazin-1-yl]-N-methylbenzamide |
| EI12-36-02R | EI12-36 | 3A36 | N-(1-Adamantylmethyl)-4-[4-[4-(5-hydroxypyridin-3-yl)naphthalene-1-carbonyl]piperazin-1-yl]-N-methylbenzamide |
| EI12-37-02R | EI12-37 | 3A37 | N-(1-Adamantylmethyl)-4-[4-[3-tert-butyl-5-(3-hydroxyphenyl)benzoyl]piperazin-1-yl]-N-methylbenzamide |
| EI12-38-02R | EI12-38 | 3A38 | N-(1-Adamantylmethyl)-4-[4-[3-tert-butyl-5-(5-hydroxypyridin-3-yl)benzoyl]piperazin-1-yl]-N-methylbenzamide |
| EI12-39-02R | EI12-39 | 3A39 | N-(1-Adamantylmethyl)-4-[4-[3-(3-hydroxyphenyl)-5-(trifluoromethyl)benzoyl]piperazin-1-yl]-N-methylbenzamide |
| EI12-40-02R | EI12-40 | 3A40 | N-(1-Adamantylmethyl)-4-[4-[3-(5-hydroxypyridin-3-yl)-5-(trifluoromethyl)benzoyl]piperazin-1-yl]-N-methylbenzamide |
| EI12-41-02R | EI12-41 | 3A41 | N-(1-Adamantylmethyl)-4-[4-[3-[2-(3-hydroxyphenyl)ethynyl]-5-(trifluoromethyl)benzoyl]piperazin-1-yl]-N-methylbenzamide |

(continued)

| Resin compound No. | Post-cleavage compound No. | Another name of post-cleavage compound in Table EJ06 | Compound name |
|---|---|---|---|
| EI12-42-02R | EI12-42 | 3A42 | N-(1-Adamantylmethyl)-4-[4-[3-[2-(5-hydroxypyridin-3-yl)ethynyl]-5-(trifluoromethyl)benzoyl]piperazin-1-yl]-N-methylbenzamide |
| EI12-43-02R | EI12-43 | 3A43 | N-(1-Adamantylmethyl)-4-[4-[3-[4-(3-hydroxyphenyl)pyrazol-1-yl]-5-(trifluoromethyl)benzoyl]piperazin-1-yl]-N-methylbenzamide |
| EI12-44-02R | EI12-44 | 3A44 | N-(1-Adamantylmethyl)-4-[4-[3-[4-(5-hydroxypyridin-3-yl)pyrazol-1-yl]-5-(trifluoromethyl)benzoyl]piperazin-1-yl]-N-methylbenzamide |
| EI12-45-02R | EI12-45 | 3A45 | N-(1-Adamantylmethyl)-4-[4-[[2-(3-hydroxyphenyl)phenyl]methyl]piperazin-1-yl]-N-methylbenzamide |
| EI12-46-02R | EI12-46 | 3A46 | N-(1-Adamantylmethyl)-4-[4-[[2-(5-hydroxypyridin-3-yl)phenyl]methyl]piperazin-1-yl]-N-methylbenzamide |
| EI12-47-02R | EI12-47 | 3A47 | N-(1-Adamantylmethyl)-4-[4-[[4-(3-hydroxyphenyl)-3-methylphenyl]methyllpiperazin-1-yl]-N-methylbenzamide |
| EI12-48-02R | EI12-48 | 3A48 | N-(1-Adamantylmethyl)-4-[4-[[4-(5-hydroxypyridin-3-yl)-3-methylphenyl]methyl]piperazin-1-yl]-N-methylbenzamide |
| EI12-49-02R | EI12-49 | 3A49 | N-(1-Adamantylmethyl)-4-[4-[[2-ethyl-4-(3-hydroxyphenyl)phenyl]methyl]piperazin-1-yl]-N-methylbenzamide |
| EI12-50-02R | EI12-50 | 3A50 | N-(1-Adamantylmethyl)-4-[4-[[2-ethyl-4-(5-hydroxypyridin-3-yl)phenyl]methyl]piperazin-1-yl]-N-methylbenzamide |
| EI12-51-02R | EI12-51 | 3A51 | N-(1-Adamantylmethyl)-4-[4-[[3-fluoro-4-(3-hydroxyphenyl)phenyl]methyl]piperazin-1-yl]-N-methylbenzamide |
| EI12-52-02R | EI12-52 | 3A52 | N-(1-Adamantylmethyl)-4-[4-[[3-fluoro-4-(5-hydroxypyridin-3-yl)phenyl]methyl]piperazin-1-yl]-N-methylbenzamide |
| EI12-53-02R | EI12-53 | 3A53 | N-(1-Adamantylmethyl)-4-[4-[[4-(3-hydroxyphenyl)-2-methoxyphenyl]methyl]piperazin-1-yl]-N-methylbenzamide |
| EI12-54-02R | EI12-54 | 3A54 | N-(1-Adamantylmethyl)-4-[4-[[4-(5-hydroxypyridin-3-yl)-2-methoxyphenyl]methyl]piperazin-1-yl]-N-methylbenzamide |
| EI12-55-02R | EI12-55 | 3A55 | N-(1-Adamantylmethyl)-4-[4-[[3-ethoxy-5-(3-hydroxyphenyl)phenyl]methyl]piperazin-1-yl]-N-methylbenzamide |

(continued)

| Resin compound No. | Post-cleavage compound No. | Another name of post-cleavage compound in Table EJ06 | Compound name |
|---|---|---|---|
| EI12-56-02R | EI12-56 | 3A56 | N-(1-Adamantylmethyl)-4-[4-[[3-ethoxy-5-(5-hydroxypyridin-3-yl)phenyl]methyl]piperazin-1-yl]-N-methylbenzamide |
| EI12-57-02R | EI12-57 | 3A57 | N-(1-Adamantylmethyl)-4-[4-[[3-(3-hydroxyphenyl)-5-propan-2-yloxyphenyl]methyl]piperazin-1-yl]-N-methylbenzamide |
| EI12-58-02R | EI12-58 | 3A58 | N-(1-Adamantylmethyl)-4-[4-[[3-(5-hydroxypyridin-3-yl)-5-propan-2-yloxyphenyl]methyl]piperazin-1-yl]-N-methylbenzamide |
| EI12-59-02R | EI12-59 | 3A59 | N-(1-Adamantylmethyl)-4-[4-[[4-(3-hydroxyphenyl)-2,6-dimethoxyphenyl]methyl]piperazin-1-yl]-N-methylbenzamide |
| EI12-60-02R | EI12-60 | 3A60 | N-(1-Adamantylmethyl)-4-[4-[[4-(5-hydroxypyridin-3-yl)-2,6-dimethoxyphenyl]methyl]piperazin-1-yl]-N-methylbenzamide |
| EI12-61-02R | EI12-61 | 3A61 | N-(1-Adamantylmethyl)-4-[4-[[3-(3-hydroxyphenyl)-5-(trifluoromethyl)phenyl]methyl]piperazin-1-yl]-N-methylbenzamide |
| EI12-62-02R | EI12-62 | 3A62 | N-(1-Adamantylmethyl)-4-[4-[[3-(5-hydroxypyridin-3-yl)-5-(trifluoromethyl)phenyl]methyl]piperazin-1-yl]-N-methylbenzamide |
| EI12-63-02R | EI12-63 | 3A63 | N-(1-Adamantylmethyl)-4-[4-[[3-(3-hydroxyphenyl)-5-(trifluoromethoxy)phenyl]methyl]piperazin-1-yl]-N-methylbenzamide |
| EI12-64-02R | EI12-64 | 3A64 | N-(1-Adamantylmethyl)-4-[4-[[3-(5-hydroxypyridin-3-yl)-5-(trifluoromethoxy)phenyl]methyl]piperazin-1-yl]-N-methylbenzamide |
| EI12-65-02R | EI12-65 | 3A65 | N-(1-Adamantylmethyl)-4-[4-[[4-(3-hydroxyphenyl)naphthalen-1-yl]methyl]piperazin-1-yl]-N-methylbenzamide |
| EI12-66-02R | EI12-66 | 3A66 | N-(1-Adamantylmethyl)-4-[4-[[4-(5-hydroxypyridin-3-yl)naphthalen-1-yl]methyl]piperazin-1-yl]-N-methylbenzamide |
| EI12-67-02R | EI12-67 | 3A67 | N-(1-Adamantylmethyl)-4-[4-[[4-(3-hydroxyphenyl)thiophen-2-yl]methyl]piperazin-1-yl]-N-methylbenzamide |
| EI12-68-02R | EI12-68 | 3A68 | N-(1-Adamantylmethyl)-4-[4-[[4-(5-hydroxypyridin-3-yl)thiophen-2-yl]methyl]piperazin-1-yl]-N-methylbenzamide |
| EI12-69-02R | EI12-69 | 3A69 | N-(1-Adamantylmethyl)-4-[4-[[2-[2-(3-hydroxyphenyl)ethynyl]phenyl]methyl]piperazin-1-yl]-N-methylbenzamide |

(continued)

| Resin compound No. | Post-cleavage compound No. | Another name of post-cleavage compound in Table EJ06 | Compound name |
|---|---|---|---|
| EI12-70-02R | EI12-70 | 3A70 | N-(1-Adamantylmethyl)-4-[4-[[2-[2-(5-hydroxypyridin-3 - yl)ethynyl]phenyl]methyl]piperazin-1-yl]-N-methylbenzamide |
| EI12-71-02R | EI12-71 | 3A71 | N-(1-Adamantylmethyl)-6-[4-[[2-(3-hydroxyphenyl)phenyl]methyl]piperazin-1-yl]-N-methylpyridazine-3-carboxamide |
| EI12-72-02R | EI12-72 | 3A72 | N-(1-Adamantylmethyl)-6-[4-[[2-(5-hydroxypyridin-3-yl)phenyl]methyl]piperazin-1-yl]-N-methylpyridazine-3-carboxamide |
| EI12-73-02R | EI12-73 | 3A73 | N-(1-Adamantylmethyl)-6-[4-[[4-(3-hydroxyphenyl)-3-methylphenyl]methyl]piperazin-1-yl]-N-methylpyridazine-3-carboxamide |
| EI12-74-02R | EI12-74 | 3A74 | N-(1-Adamantylmethyl)-6-[4-[[4-(5-hydroxypyridin-3-yl)-3-methylphenyl]methyl]piperazin-1-yl]-N-methylpyridazine-3-carboxamide |
| EI12-75-02R | EI12-75 | 3A75 | N-(1-Adamantylmethyl)-6-[4-[[2-ethyl-4-(3-hydroxyphenyl)phenyl]methyl]piperazin-1-yl]-N-methylpyridazine-3-carboxamide |
| EI12-76-02R | EI12-76 | 3A76 | N-(1-Adamantylmethyl)-6-[4-[[2-ethyl-4-(5-hydroxypyridin-3-yl)phenyl]methyl]piperazin-1-yl]-N-methylpyridazine-3- carboxamide |
| EI12-77-02R | EI12-77 | 3A77 | N-(1-Adamantylmethyl)-6-[4-[[3-fluoro-4-(3-hydroxyphenyl)phenyl]methyl]piperazin-1-yl]-N-methylpyridazine-3-carboxamide |
| EI12-78-02R | EI12-78 | 3A78 | N-(1-Adamantylmethyl)-6-[4-[[3-fluoro-4-(5-hydroxypyridin-3-yl)phenyl]methyl]piperazin-1-yl]-N-methylpyridazine-3- carboxamide |
| EI12-79-02R | EI12-79 | 3A79 | N-(1-Adamantylmethyl)-6-[4-[[4-(3-hydroxyphenyl)-2-methoxyphenyl]methyl]piperazin-1-yl]-N-methylpyridazine-3-carboxamide |
| EI12-80-02R | EI12-80 | 3A80 | N-(1-Adamantylmethyl)-6-[4-[[4-(5-hydroxypyridin-3-yl)-2-methoxyphenyl]methyl]piperazin-1-yl]-N-methylpyridazine-3-carboxamide |
| EI12-81-02R | EI12-81 | 3A81 | N-(1-Adamantylmethyl)-6-[4-[[3-ethoxy-5-(3-hydroxyphenyl)phenyl]methyl]piperazin-1-yl]-N-methylpyridazine-3 -carboxamide |
| EI12-82-02R | EI12-82 | 3A82 | N-(1-Adamantylmethyl)-6-[4-[[3-ethoxy-5-(5-hydroxypyridin-3-yl)phenyl]methyl]piperazin-1-yl]-N-methylpyridazine-3- carboxamide |
| EI12-83-02R | EI12-83 | 3A83 | N-(1-Adamantylmethyl)-6-[4-[[3-(3-hydroxyphenyl)-5-propan-2-yloxyphenyl]methyl]piperazin-1-yl]-N-methylpyridazine-3-carboxamide |

(continued)

| Resin compound No. | Post-cleavage compound No. | Another name of post-cleavage compound in Table EJ06 | Compound name |
|---|---|---|---|
| EI12-84-02R | EI12-84 | 3A84 | N-(1-Adamantylmethyl)-6-[4-[[3-(5-hydroxypyridin-3-yl)-5-propan-2-yloxyphenyl]methyl]piperazin-1-yl]-N-methylpyridazine-3-carboxamide |
| EI12-85-02R | EI12-85 | 3A85 | N-(1-Adamantylmethyl)-6-[4-[[4-(3-hydroxyphenyl)-2,6-dimethoxyphenyl]methyl]piperazin-1-yl]-N-methylpyridazine-3-carboxamide |
| EI12-86-02R | EI12-86 | 3A86 | N-(1-Adamantylmethyl)-6-[4-[[4-(5-hydroxypyridin-3-yl)-2,6-dimethoxyphenyl]methyl]piperazin-1-yl]-N-methylpyridazine-3-carboxamide |
| EI12-87-02R | EI12-87 | 3A87 | N-(1-Adamantylmethyl)-6-[4-[[3-(3-hydroxyphenyl)-5-(trifluoromethyl)phenyl]methyl]piperazin-1-yl]-N-methylpyridazine-3 -carboxamide |
| EI12-88-02R | EI12-88 | 3A88 | N-(1-Adamantylmethyl)-6-[4-[[3-(5-hydroxypyridin-3-yl)-5-(trifluoromethyl)phenyl]methyl]piperazin-1-yl]-N-methylpyridazine-3-carboxamide |
| EI12-89-02R | EI12-89 | 3A89 | N-(1-Adamantylmethyl)-6-[4-[[3-(3-hydroxyphenyl)-5-(trifluoromethoxy)phenyl]methyl]piperazin-1-yl]-N-methylpyridazine-3-carboxamide |
| EI12-90-02R | EI12-90 | 3A90 | N-(1-Adamantylmethyl)-6-[4-[[3-(5-hydroxypyridin-3-yl)-5-(trifluoromethoxy)phenyl]methyl]piperazin-1 -yl]-N-methylpyridazine-3-carboxamide |
| EI12-91-02R | EI12-91 | 3A91 | N-(1-Adamantylmethyl)-6-[4-[[4-(3-hydroxyphenyl)naphthalen-1-yl]methyl]piperazin-1-yl]-N-methylpyridazine-3-carboxamide |
| EI12-92-02R | EI12-92 | 3A92 | N-(1-Adamantylmethyl)-6-[4-[[4-(5-hydroxypyridin-3-yl)naphthalen-1-yl]methyl]piperazin-1-yl]-N-methylpyridazine-3-carboxamide |
| EI12-93-02R | EI12-93 | 3A93 | N-(1-Adamantylmethyl)-6-[4-[[4-(3-hydroxyphenyl)thiophen-2-yl]methyl]piperazin-1-yl]-N-methylpyridazine-3-carboxamide |
| EI12-94-02R | EI12-94 | 3A94 | N-(1-Adamantylmethyl)-6-[4-[[4-(5-hydroxypyridin-3-yl)thiophen-2-yl]methyl]piperazin-1-yl]-N-methylpyridazine-3-carboxamide |
| EI12-95-02R | EI12-95 | 3A95 | N-(1-Adamantylmethyl)-6-[4-[[2-[2-(3-hydroxyphenyl)ethynyl]phenyl]methyl]piperazin-1-yl]-N-methylpyridazine-3-carboxamide |
| EI12-96-02R | EI12-96 | 3A96 | N-(1-Adamantylmethyl)-6-[4-[[2-[2-(5-hydroxypyridin-3-yl)ethynyl]phenyl]methyl]piperazin-1-yl]-N-methylpyridazine-3-carboxamide |
| EI12-97-02R | EI12-97 | 3A97 | N-(1-Adamantylmethyl)-4-[4-[1-[4-(3-hydroxyphenyl)phenyl]ethyl]piperazin-1-yl]-N-methylbenzamide |

(continued)

| Resin compound No. | Post-cleavage compound No. | Another name of post-cleavage compound in Table EJ06 | Compound name |
|---|---|---|---|
| EI12-98-02R | EI12-98 | 3A98 | N-(1-Adamantylmethyl)-4-[4-[1-[4-(5-hydroxypyridin-3-yl)phenyl]ethyl]piperazin-1-yl]-N-methylbenzamide |
| EI12-99-02R | EI12-99 | 3A99 | N-(1-Adamantylmethyl)-4-[4-[1-[2-fluoro-4-(3- hydroxyphenyl)phenyl]ethyl]piperazin-1-yl]-N-methylbenzamide |
| EI12-100-02R | EI12-100 | 3A100 | N-(1-Adamantylmethyl)-4-[4-[1-[2-fluoro-4-(5-hydroxypyridin-3-yl)phenyl]ethyl]piperazin-1-yl]-N-methylbenzamide |

Example 6-3-10-A: Synthesis of mixture mixEIl3-02R

[Formula 113]

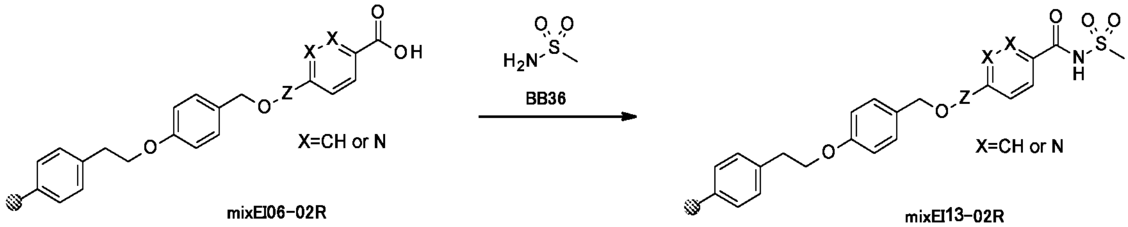


mixEI06-02R

mixEI13-02R

To a 2 mL glass vial were added the mixture mixEI06-02R (50 mg, loading rate 0.648 mmol/g, 0.0324 mmol) shown in [Table EI06] and NMP (1.13 mL), and the mixture was shaken at room temperature for 1 hour. HATU (73.9 mg, 0.194 mmol) and 2,6-lutidine (22.6 μL, 0.194 mmol) were added at room temperature, and the mixture was shaken at 40°C for 3 hours. Methanesulfonamide (BB36, 21.6 mg, 0.227 mmol) and the phosphazene base PltBu (124 μL, 0.486 mmol) were added at room temperature, and the mixture was shaken at 40°C for 18 hours. N-propylamine (27 μL, 0.324 mmol) was added at room temperature, and the mixture was shaken for 1 hour. The suspension of the reaction solution and the resins was transferred to a syringe equipped with a filter using NMP, washed repeatedly three times with NMP (1.2 mL), three times with HOAt/NMP (0.2M, 1.2 mL), three times with NMP (1.2 mL), three times with MeOH (1.2 mL), three times with DCM (1.2 mL), and three times with heptane (1.2 mL), and then dried under reduced pressure to obtain the title mixture shown in [Table EI13]. The names of compounds contained in the obtained mixture are shown in [Table EI13].

[Table 65]

[Table EI13] Mixture No.: mixEI13-02R, amine reagent used: BB36, post-cleavage mixture No.: mixEI13

| Resin compound No. | Post-cleavage compound No. | Another name of post-cleavage compound in Table EJ07 | Compound name |
|---|---|---|---|
| EI13-01-02R | EI13-01 | 2I01 | 6-[4-[4-(3-Hydroxyphenyl)thiophene-2-carbonyl]piperazin-1-yl]-N-methylsulfonylpyridazine-3-carboxamide |
| EI13-02-02R | EI13-02 | 2I02 | 6-[4-[4-(5-Hydroxypyridin-3-yl)thiophene-2-carbonyl]piperazin-1-yl]-N-methylsulfonylpyridazine-3-carboxamide |
| EI13-03-02R | EI13-03 | 2I03 | 6-[4-[4-(3-Hydroxyphenyl)-3-methylbenzoyl]piperazin-1-yl]-N-methylsulfonylpyridazine-3-carboxamide |
| EI13-04-02R | EI13-04 | 2I04 | 6-[4-[4-(5-Hydroxypyridin-3-yl)-3-methylbenzoyl]piperazin-1-yl]-N-methylsulfonylpyridazine-3-carboxamide |
| EI13-05-02R | EI13-05 | 2I05 | 6-[4-[3-Fluoro-4-(3-hydroxyphenyl)benzoyl]piperazin-1-yl]-N-methylsulfonylpyridazine-3-carboxamide |
| EI13-06-02R | EI13-06 | 2I06 | 6-[4-[3-Fluoro-4-(5-hydroxypyridin-3-yl)benzoyl]piperazin-1-yl]-N-methylsulfonylpyridazine-3-carboxamide |
| EI13-07-02R | EI13-07 | 2I07 | 6-[4-[2-[2-(3-Hydroxyphenyl)ethynyl]benzoyl]piperazin-1-yl]-N-methylsulfonylpyridazine-3-carboxamide |
| EI13-08-02R | EI13-08 | 2I08 | 6-[4-[2-[2-(5-Hydroxypyridin-3-yl)ethynyl]benzoyl]piperazin-1-yl]-N-methylsulfonylpyridazine-3-carboxamide |

(continued)

| Resin compound No. | Post-cleavage compound No. | Another name of post-cleavage compound in Table EJ07 | Compound name |
|---|---|---|---|
| EI13-09-02R | EI13-09 | 2I09 | 6-[4-[5-[2-(3-Hydroxyphenyl)ethynyl]pyridine-3-carbonyl]piperazin-1-yl]-N-methylsulfonylpyridazine-3-carboxamide |
| EI13-10-02R | EI13-10 | 2I10 | 6-[4-[5-[2-(5-Hydroxypyridin-3-yl)ethynyl]pyridine-3-carbonyl]piperazin-1-yl]-N-methylsulfonylpyridazine-3-carboxamide |
| EI13-11-02R | EI13-11 | 2I11 | 6-[4-[3-[2-(3-Hydroxyphenyl)phenyl]propanoyl]piperazin-1-yl]-N-methylsulfonylpyridazine-3-carboxamide |
| EI13-12-02R | EI13-12 | 2I12 | 6-[4-[3-[2-(5-Hydroxypyridin-3-yl)phenyl]propanoyl]piperazin-1-yl]-N-methylsulfonylpyridazine-3-carboxamide |
| EI13-13-02R | EI13-13 | 2I13 | 6-[4-[4-(3-Hydroxyphenyl)naphthalene-1-carbonyl]piperazin-1-yl]-N-methylsulfonylpyridazine-3-carboxamide |
| EI13-14-02R | EI13-14 | 2I14 | 6-[4-[4-(5-Hydroxypyridin-3-yl)naphthalene-1-carbonyl]piperazin-1-yl]-N-methylsulfonylpyridazine-3-carboxamide |
| EI13-15-02R | EI13-15 | 2I15 | 6-[4-[3-tert-Butyl-5-(3-hydroxyphenyl)benzoyl]piperazin-1-yl]-N-methylsulfonylpyridazine-3-carboxamide |
| EI13-16-02R | EI13-16 | 2I16 | 6-[4-[3-tert-Butyl-5-(5-hydroxypyridin-3-yl)benzoyl]piperazin-1-yl]-N-methylsulfonylpyridazine-3-carboxamide |
| EI13-17-02R | EI13-17 | 2I17 | 6-[4-[3-(3-Hydroxyphenyl)-5-(trifluoromethyl)benzoyl]piperazin-1-yl]-N-methylsulfonylpyridazine-3-carboxamide |
| EI13-18-02R | EI13-18 | 2I18 | 6-[4-[3-(5-Hydroxypyridin-3-yl)-5-(trifluoromethyl)benzoyl]piperazin-1-yl]-N-methylsulfonylpyridazine-3-carboxamide |
| EI13-19-02R | EI13-19 | 2I19 | 6-[4-[3-[2-(3-Hydroxyphenyl)ethynyl]-5-(trifluoromethyl)benzoyl]piperazin-1-yl]-N-methylsulfonylpyridazine-3-carboxamide |
| EI13-20-02R | EI13-20 | 2120 | 6-[4-[3-[2-(5-Hydroxypyridin-3-yl)ethynyl]-5-(trifluoromethyl)benzoyl]piperazin-1-yl]-N-methylsulfonylpyridazine-3-carboxamide |
| EI13-21-02R | EI13-21 | 2121 | 6-[4-[3-[4-(3-Hydroxyphenyl)pyrazol-1-yl]-5-(trifluoromethyl)benzoyl]piperazin-1-yl]-N-methylsulfonylpyridazine-3-carboxamide |
| EI13-22-02R | EI13-22 | 2122 | 6-[4-[3-[4-(5-Hydroxypyridin-3-yl)pyrazol-1-yl]-5-(trifluoromethyl)benzoyl]piperazin-1-yl]-N-methylsulfonylpyridazine-3-carboxamide |
| EI13-23-02R | EI13-23 | 2123 | 4-[4-[4-(3-Hydroxyphenyl)thiophene-2-carbonyl]piperazin-1-yl]-N-methylsulfonylbenzamide |
| EI13-24-02R | EI13-24 | 2124 | 4-[4-[4-(5-Hydroxypyridin-3-yl)thiophene-2-carbonyl]piperazin-1-yl]-N-methylsulfonylbenzamide |

(continued)

| Resin compound No. | Post-cleavage compound No. | Another name of post-cleavage compound in Table EJ07 | Compound name |
|---|---|---|---|
| EI13-25-02R | EI13-25 | 2125 | 4-[4-[4-(3-Hydroxyphenyl)-3-methylbenzoyl]piperazin-1-yl]-N-methylsulfonylbenzamide |
| EI13-26-02R | EI13-26 | 2126 | 4-[4-[4-(5-Hydroxypyridin-3-yl)-3-methylbenzoyl]piperazin-1-yl]-N-methylsulfonylbenzamide |
| EI13-27-02R | EI13-27 | 2127 | 4-4[3-Fluoro-4-(3-hydroxyphenyl)benzoyl]piperazin-1-yl]-N-methylsulfonylbenzamide |
| EI13-28-02R | EI13-28 | 2128 | 4-[4-[3-Fluoro-4-(5-hydroxypyridin-3-yl)benzoyl]piperazin-1- yl]-N-methylsulfonylbenzamide |
| EI13-29-02R | EI13-29 | 2129 | 4-[4-[2-[2-(3-Hydroxyphenyl)ethynyl]benzoyl]piperazin-1-yl]-N-methylsulfonylbenzamide |
| EI13-30-02R | EI13-30 | 2I30 | 4-[4-[2-[2-(5-Hydroxypyridin-3-yl)ethynyl]benzoyl]piperazin-1-yl]-N-methylsulfonylbenzamide |
| EI13-31-02R | EI13-31 | 2I31 | 4-[4-[5-[2-(3-Hydroxyphenyl)ethynyl]pyridine-3-carbonyl]piperazin-1-yl]-N-methylsulfonylbenzamide |
| EI13-32-02R | EI13-32 | 2I32 | 4-[4-[5-[2-(5-Hydroxypyridin-3-yl)ethynyl]pyridine-3-carbonyl]piperazin-1-yl]-N-methylsulfonylbenzamide |
| EI13-33-02R | EI13-33 | 2I33 | 4-[4-[3-[2-(3-Hydroxyphenyl)phenyl]propanoyl]piperazin-1-yl]-N-methylsulfonylbenzamide |
| EI13-34-02R | EI13-34 | 2I34 | 4-[4-[3-[2-(5-Hydroxypyridin-3-yl)phenyl]propanoyl]piperazin-1-yl]-N-methylsulfonylbenzamide |
| EI13-35-02R | EI13-35 | 2I35 | 4-[4-[4-(3-Hydroxyphenyl)naphthalene-1-carbonyl]piperazin-1-yl]-N-methylsulfonylbenzamide |
| EI13-36-02R | EI13-36 | 2I36 | 4-[4-[4-(5-Hydroxypyridin-3-yl)naphthalene-1-carbonyl]piperazin-1-yl]-N-methylsulfonylbenzamide |
| EI13-37-02R | EI13-37 | 2I37 | 4-[4-[-tert-Butyl-5-(3-hydroxyphenyl)benzoyl]piperazin-1-yl]-N-methylsulfonylbenzamide |
| EI13-38-02R | EI13-38 | 2I38 | 4-[4-[3-tert-Butyl-5-(5-hydroxypyridin-3-yl)benzoyl]piperazin-1-yl]-N-methylsulfonylbenzamide |
| EI13-39-02R | EI13-39 | 2I39 | 4-[4-[3-(3-Hydroxyphenyl)-5-(trifluoromethyl)benzoyl]piperazin-1-yl]-N-methylsulfonylbenzamide |
| EI13-40-02R | EI13-40 | 2I40 | 4-[4-[3-(5-Hydroxypyridin-3-yl)-5-(trifluoromethyl)benzoyl]piperazin-1-yl]-N-methylsulfonylbenzamide |
| EI13-41-02R | EI13-41 | 2I41 | 4-[4-[3-[2-(3-Hydroxyphenyl)ethynyl]-5-(trifluoromethyl)benzoyl]piperazin-1-yl]-N-methylsulfonylbenzamide |
| EI13-42-02R | EI13-42 | 2I42 | 4-[4-3-[2-(5-Hydroxypyridin-3-yl)ethynyl]-5-(trifluoromethyl)benzoyl]piperazin-1-yl]-N-methylsulfonylbenzamide |

(continued)

| Resin compound No. | Post-cleavage compound No. | Another name of post-cleavage compound in Table EJ07 | Compound name |
|---|---|---|---|
| EI13-43-02R | EI13-43 | 2143 | 4-[4-[3-[4-(3-Hydroxyphenyl)pyrazol-1-yl]-5-(trifluoromethyl)benzoyl]piperazin-1-yl]-N-methylsulfonylbenzamide |
| EI13-44-02R | EI13-44 | 2I44 | 4-[4-[3-[4-(5-Hydroxypyridin-3-yl)pyrazol-1-yl]-5-(trifluoromethyl)benzoyl]piperazin-1-yl]-N-methylsulfonylbenzamide |
| EI13-45-02R | EI13-45 | 2145 | 4-[4-[[2-(3-Hydroxyphenyl)phenyl]methyl]piperazin-1-yl]-N-methylsulfonylbenzamide |
| EI13-46-02R | EI13-46 | 2I46 | 4-[4-[[2-(5-Hydroxypyridin-3-yl)phenyl]methyl]piperazin-1-yl]-N-methylsulfonylbenzamide |
| EI13-47-02R | EI13-47 | 2I47 | 4-[4-[[4-(3-Hydroxyphenyl)-3-methylphenyl]methyl]piperazin-1-yl]-N-methylsulfonylbenzamide |
| EI13-48-02R | EI13-48 | 2148 | 4-[4-[[4-(5-Hydroxypyridin-3-yl)-3-methylphenyl]methyl]piperazin-1-yl]-N-methylsulfonylbenzamide |
| EI13-49-02R | EI13-49 | 2I49 | 4-[4-[[2-Ethyl-4-(3-hydroxyphenyl)phenyl]methyl]piperazin-1-yl]-N-methylsulfonylbenzamide |
| EI13-50-02R | EI13-50 | 2150 | 4-[4-[[2-Ethyl-4-(5-hydroxypyridin-3-yl)phenyl]methyl]piperazin-1-yl]-N-methylsulfonylbenzamide |
| EI13-51-02R | EI13-51 | 2I51 | 4-[4-[[3-Fluoro-4-(3-hydroxyphenyl)phenyl]methyl]piperazin-1-yl]-N-methylsulfonylbenzamide |
| EI13-52-02R | EI13-52 | 2I52 | 4-[4-[[3-Fluoro-4-(5-hydroxypyridin-3-yl)phenyllmethyl]piperazin-1-yl]-N-methylsulfonylbenzamide |
| EI13-53-02R | EI13-53 | 2I53 | 4-[4-[[4-(3-Hydroxyphenyl)-2-methoxyphenyl]methyl]piperazin-1-yl]-N-methylsulfonylbenzamide |
| EI13-54-02R | EI13-54 | 2I54 | 4-[4-[[4-(5-Hydroxypyridin-3-yl)-2-methoxyphenyl]methyl]piperazin-1-yl]-N-methylsulfonylbenzamide |
| EI13-55-02R | EI13-55 | 2I55 | 4-[4-[[3-Ethoxy-5-(3-hydroxyphenyl)phenyl]methyl]piperazin-1-yl]-N-methylsulfonylbenzamide |
| EI13-56-02R | EI13-56 | 2I56 | 4-[4-[[3-Ethoxy-5-(5-hydroxypyridin-3-yl)phenyl]methyl]piperazin-1-yl]-N-methylsulfonylbenzamide |
| EI13-57-02R | EI13-57 | 2I57 | 4-[4-[[3-(3-Hydroxyphenyl)-5-propan-2-yloxyphenyl]methyl]piperazin-1-yl]-N-methylsulfonylbenzamide |

(continued)

| Resin compound No. | Post-cleavage compound No. | Another name of post-cleavage compound in Table EJ07 | Compound name |
|---|---|---|---|
| EI13-58-02R | EI13-58 | 2I58 | 4-[4-[[3-(5-Hydroxypyridin-3-yl)-5-propan-2-yloxyphenyl]methyl]piperazin-1-yl]-N-methylsulfonylbenzamide |
| EI13-59-02R | EI13-59 | 2I59 | 4-[4-[[4-(3-Hydroxyphenyl)-2,6-dimethoxyphenyl]methyl]piperazin-1-yl]-N-methylsulfonylbenzamide |
| EI13-60-02R | EI13-60 | 2I60 | 4-[4-[[4-(5-Hydroxypyridin-3-yl)-2,6-dimethoxyphenyl]methyl]piperazin-1-yl]-N-methylsulfonylbenzamide |
| EI13-61-02R | EI13-61 | 2I61 | 4-[4-[[3-(3-Hydroxyphenyl)-5-(trifluoromethyl)phenyl]methyl]piperazin-1-yl]-N-methylsulfonylbenzamide |
| EI13-62-02R | EI13-62 | 2I62 | 4-[4-[3-(5-Hydroxypyridin-3-yl)-5-(trifluoromethyl)phenyl]methyl]piperazin-1-yl]-N-methylsulfonylbenzamide |
| EI13-63-02R | EI13-63 | 2I63 | 4-[4-[[3-(3-Hydroxyphenyl)-5-(trifluoromethoxy)phenyl]methyl]piperazin-1-yl]-N-methylsulfonylbenzamide |
| EI13-64-02R | EI13-64 | 2I64 | 4-[4-[[3-(5-Hydroxypyridin-3-yl)-5-(trifluoromethoxy)phenyl]methyl]piperazin-1-yl]-N-methylsulfonylbenzamide |
| EI13-65-02R | EI13-65 | 2I65 | 4-[4-[[4-(3-Hydroxyphenyl)naphthalen-1-yl]methyl]piperazin-1-yl]-N-methylsulfonylbenzamide |
| EI13-66-02R | EI13-66 | 2166 | 4-[4-[[4-(5-Hydroxypyridin-3-yl)naphthalen-1-yl]methyl]piperazin-1-yl]-N-methylsulfonylbenzamide |
| EI13-67-02R | EI13-67 | 2I67 | 4-[4-[[4-(3-Hydroxyphenyl)thiophen-2-yl]methyl]piperazin-1-yl]-N-methylsulfonylbenzamide |
| EI13-68-02R | EI13-68 | 2168 | 4-[4-[[4-(5-Hydroxypyridin-3-yl)thiophen-2-yl]methyl]piperazin-1-yl]-N-methylsulfonylbenzamide |
| EI13-69-02R | EI13-69 | 2169 | 4-[4-[[2-[2-(3-Hydroxyphenyl)ethynyl]phenyl]methyl]piperazin-1-yl]-N-methylsulfonylbenzamide |
| EI13-70-02R | EI13-70 | 2170 | 4-[4-[[2-[2-(5-Hydroxypyridin-3-yl)ethynyl]phenyl]methyl]piperazin-1-yl]-N-methylsulfonylbenzamide |
| EI13-71-02R | EI13-71 | 2I71 | 6-[4-[[2-(3-Hydroxyphenyl)phenyl]methyl]piperazin-1-yl]-N-methylsulfonylpyridazine-3-carboxamide |
| EI13-72-02R | EI13-72 | 2I72 | 6-[4-[[2-(5-Hydroxypyridin-3-yl)phenyl]methyl]piperazin-1-yl]-N-methylsulfonylpyridazine-3-carboxamide |
| EI13-73-02R | EI13-73 | 2I73 | 6-[4-[[4-(3-Hydroxyphenyl)-3-methylphenyl]methyl]piperazin-1-yl]-N-methylsulfonylpyridazine-3-carboxamide |

(continued)

| Resin compound No. | Post-cleavage compound No. | Another name of post-cleavage compound in Table EJ07 | Compound name |
|---|---|---|---|
| EI13-74-02R | EI13-74 | 2I74 | 6-[4-[4-(5-Hydroxypyridin-3-yl)-3-methylphenyl]methyl]piperazin-1-yl]-N-methylsulfonylpyridazine-3-carboxamide |
| EI13-75-02R | EI13-75 | 2I75 | 6-[4-[[2-Ethyl-4-(3-hydroxyphenyl)phenyl]methyl]piperazin-1-yl]-N-methylsulfonylpyridazine-3-carboxamide |
| EI13-76-02R | EI13-76 | 2I76 | 6-[4-[[2-Ethyl-4-(5-hydroxypyridin-3-yl)phenyl]methyl]piperazin-1-yl]-N-methylsulfonylpyridazine-3-carboxamide |
| EI13-77-02R | EI13-77 | 2I77 | 6-[4-[3-Fluoro-4-(3-hydroxyphenyl)phenyl]methyl]piperazin-1-yl]-N-methylsulfonylpyridazine-3-carboxamide |
| EI13-78-02R | EI13-78 | 2I78 | 6-[4-[[3-Fluoro-4-(5-hydroxypyridin-3-yl)phenyl]methyl]piperazin-1-yl]-N-methylsulfonylpyridazine-3-carboxamide |
| EI13-79-02R | EI13-79 | 2I79 | 6-[4-[[4-(3-Hydroxyphenyl)-2-methoxyphenyl]methyl]piperazin-1-yl]-N-methylsulfonylpyridazine-3-carboxamide |
| EI13-80-02R | EI13-80 | 2I80 | 6-[4-[[4-(5-Hydroxypyridin-3-yl)-2-methoxyphenyl]methyl]piperazin-1-yl]-N-methylsulfonylpyridazine-3-carboxamide |
| EI13-81-02R | EI13-81 | 2I81 | 6-[4-[[3-Ethoxy-5-(3-hydroxyphenyl)phenyl]methyl]piperazin-1-yl]-N-methylsulfonylpyridazine-3-carboxamide |
| EI13-82-02R | EI13-82 | 2182 | 6-[4-[[3-Ethoxy-5-(5-hydroxypyridin-3-yl)phenyl]methyl]piperazin-1-yl]-N-methylsulfonylpyridazine-3-carboxamide |
| EI13-83-02R | EI13-83 | 2I83 | 6-[4-[[3-(3-Hydroxyphenyl)-5-propan-2-yloxyphenyl]methyl]piperazin-1-yl]-N-methylsulfonylpyridazine-3-carboxamide |
| EI13-84-02R | EI13-84 | 2I84 | 6-[4-[[3-(5-Hydroxypyridin-3-yl)-5-propan-2-yloxyphenyl]methyl]piperazin-1-yl]-N-methylsulfonylpyridazine-3-carboxamide |
| EI13-85-02R | EI13-85 | 2I85 | 6-[4-[[4-(3-Hydroxyphenyl)-2,6-dimethoxyphenyl]methyl]piperazin-1-yl]-N-methylsulfonylpyridazine-3-carboxamide |
| EI13-86-02R | EI13-86 | 2186 | 6-[4-[[4-(5-Hydroxypyridin-3-yl)-2,6-dimethoxyphenyl]methyl]piperazin-1-yl]-N-methylsulfonylpyridazine-3-carboxamide |
| EI13-87-02R | EI13-87 | 2187 | 6-[4-[[3-(3-Hydroxyphenyl)-5-(trifluoromethyl)phenyl]methyl]piperazin-1-yl]-N-methylsulfonylpyridazine-3-carboxamide |
| EI13-88-02R | EI13-88 | 2I88 | 6-[4-[[3-(5-Hydroxypyridin-3-yl)-5-(trifluoromethyl)phenyl]methyl]piperazin-1-yl]-N-methylsulfonylpyridazine-3-carboxamide |

(continued)

| Resin compound No. | Post-cleavage compound No. | Another name of post-cleavage compound in Table EJ07 | Compound name |
|---|---|---|---|
| EI13-89-02R | EI13-89 | 2I89 | 6-[4-[[3-(3-Hydroxyphenyl)-5-(trifluoromethoxy)phenyl]methyl]piperazin-1-yl]-N-methylsulfonylpyridazine-3-carboxamide |
| EI13-90-02R | EI13-90 | 2I90 | 6-[4-[3-(5-Hydroxypyridin-3-yl)-5-(trifluoromethoxy)phenyl]methyl]piperazin-1-yl]-N-methylsulfonylpyridazine-3-carboxamide |
| EI13-91-02R | EI13-91 | 2I91 | 6-[4-[[4-(3-Hydroxyphenyl)naphthalen-1-yl]methyl]piperazin-1-yl]-N-methylsulfonylpyridazine-3-carboxamide |
| EI13-92-02R | EI13-92 | 2I92 | 6-[4-[[4-(5-Hydroxypyridin-3-yl)naphthalen-1-yl]methyl]piperazin-1-yl]-N-methylsulfonylpyridazine-3 -carboxamide |
| EI13-93-02R | EI13-93 | 2I93 | 6-[4-[[4-(3-Hydroxyphenyl)thiophen-2-yl]methyl]piperazin-1-yl]-N-methylsulfonylpyridazine-3-carboxamide |
| EI13-94-02R | EI13-94 | 2I94 | 6-[4-[[4-(5-Hydroxypyridin-3-yl)thiophen-2-yl]methyl]piperazin-1-yl]-N-methylsulfonylpyridazine- 3-carboxamide |
| EI13-95-02R | EI13-95 | 2I95 | 6-[4-[[2-[2-(3-Hydroxyphenyl)ethynyl]phenyl]methyl]piperazin-1-yl]-N-methylsulfonylpyridazine-3-carboxamide |
| EI13-96-02R | EI13-96 | 2I96 | 6-[4-[[2-[2-(5-Hydroxypyridin-3-yl)ethynyl]phenyl]methyl]piperazin-1-yl]-N-methylsulfonylpyridazine-3-carboxamide |
| EI13-97-02R | EI13-97 | 2I97 | 4-[4-[1-[4-(3-Hydroxyphenyl)phenyl]ethyl]piperazin-1-yl]-N-methylsulfonylbenzamide |
| EI13-98-02R | EI13-98 | 2I98 | 4-[4-[1-[4-(5-Hydroxypyridin-3-yl)phenyl]ethyl]piperazin-1-yl]-N-methylsulfonylbenzamide |
| EI13-99-02R | EI13-99 | 2I99 | 4-[4-[1-[2-Fluoro-4-(3-hydroxyphenyl)phenyl]ethyl]piperazin-1-yl]-N-methylsulfonylbenzamide |
| EI13-100-02R | EI13-100 | 2I100 | 4-[4-[1-[2-Fluoro-4-(5-hydroxypyridin-3-yl)phenyl]ethyl]piperazin-1-yl]-N-methylsulfonylbenzamide |

Example 6-3-10-B: Synthesis of mixtures mixEI14-02R to mixEI18-02R

Using the mixture mixEI06-02R shown in [Table EI06] (50 mg, loading rate 0.648 mmol/g, 0.0324 mmol) and the amine reagents (BB37 to 38, 40 to 42), the title mixtures shown in [Table EI14] to [Table EI18] were synthesized by the same method as in Example 6-3-10-A and obtained. The names of compounds contained in the obtained mixtures are shown in [Table EI14] to [Table EI18].

[Table 66]

[Table EI14] mixEI14-02R, amine reagent used: BB37, post-cleavage mixture No.: mixEI14

| Resin compound No. | Post-cleavage compound No. | Another name of post-cleavage compound in Table EJ08 | Compound name |
|---|---|---|---|
| EI14-01-02R | EI14-01 | 2L01 | N-tert-Butylsulfonyl-6-[4-[4-(3-hydroxyphenyl)thiophene-2-carbonyl]piperazin-1-yl]pyridazine-3-carboxamide |
| EI14-02-02R | EI14-02 | 2L02 | N-tert-Butylsulfonyl-6-[4-[4-(5-hydroxypyridin-3-yl)thiophene-2-carbonyl]piperazin-1-yl]pyridazine-3-carboxamide |
| EI14-03-02R | EI14-03 | 2L03 | N-tert-Butylsulfonyl-6-[4-[4-(3-hydroxyphenyl)-3-methylbenzoyl]piperazin-1-yl]pyridazine-3-carboxamide |
| EI14-04-02R | EI14-04 | 2L04 | N-tert-Butylsulfonyl-6-[4-[4-(5-hydroxypyridin-3-yl)-3-methylbenzoyl]piperazin-1-yl]pyridazine-3-carboxamide |
| EI14-05-02R | EI14-05 | 2L05 | N-tert-Butylsulfonyl-6-[4-[3-fluoro-4-(3-hydroxyphenyl)benzoyl]piperazin-1-yl]pyridazine-3-carboxamide |
| EI14-06-02R | EI14-06 | 2L06 | N-tert-Butylsulfonyl-6-[4-[3-fluoro-4-(5-hydroxypyridin-3-yl)benzoyl]piperazin-1-yl]pyridazine-3-carboxamide |
| EI14-07-02R | EI14-07 | 2L07 | N-tert-Butylsulfonyl-6-[4-[2-[2-(3-hydroxyphenyl)ethynyl]benzoyl]piperazin-1-yl]pyridazine-3-carboxamide |
| EI14-08-02R | EI14-08 | 2L08 | N-tert-Butylsulfonyl-6-[4-[2-[2-(5-hydroxypyridin-3-yl)ethynyl]benzoyl]piperazin-1-yl]pyridazine-3-carboxamide |
| EI14-09-02R | EI14-09 | 2L09 | N-tert-Butylsulfonyl-6-[4-[5-[2-(3-hydroxyphenyl)ethynyl]pyridine-3-carbonyl]piperazin-1-yl]pyridazine-3-carboxamide |
| EI14-10-02R | EI14-10 | 2L10 | N-tert-Butylsulfonyl-6-[4-[5-[2-(5-hydroxypyridin-3-yl)ethynyl]pyridine-3-carbonyl]piperazin-1-yl]pyridazine-3- carboxamide |
| EI14-11-02R | EI14-11 | 2L11 | N-tert-Butylsulfonyl-6-[4-[3-[2-(3-hydroxyphenyl)phenyl]propanoyl]piperazin-1-yl]pyridazine-3-carboxamide |
| EI14-12-02R | EI14-12 | 2L12 | N-tert-Butylsulfonyl-6-[4-[3-[2-(5-hydroxypyridin-3-yl)phenyl]propanoyl]piperazin-1-yl]pyridazine-3-carboxamide |
| EI14-13-02R | EI14-13 | 2L13 | N-tert-Butylsulfonyl-6-[4-[4-(3-hydroxyphenyl)naphthalene-1-carbonyl]piperazin-1-yl]pyridazine-3-carboxamide |

(continued)

| Resin compound No. | Post-cleavage compound No. | Another name of post-cleavage compound in Table EJ08 | Compound name |
|---|---|---|---|
| EI14-14-02R | EI14-14 | 2L14 | N-tert-Butylsulfonyl-6-[4-[4-(5-hydroxypyridin-3-yl)naphthalene-1-carbonyl]piperazin-1-yl]pyridazine-3-carboxamide |
| EI14-15-02R | EI14-15 | 2L15 | 6-[4-[3-tert-Butyl-5-(3-hydroxyphenyl)benzoyl]piperazin-1-yl]-N-tert-butylsulfonylpyridazine-3-carboxamide |
| EI14-16-02R | EI14-16 | 2L16 | 6-[4-[3-tert-Butyl-5-(5-hydroxypyridin-3-yl)benzoyl]piperazin-1-yl]-N-tert-butylsulfonylpyridazine-3-carboxamide |
| EI14-17-02R | EI14-17 | 2L17 | N-tert-Butylsulfonyl-6-[4-[3-(3-hydroxyphenyl)-5-(trifluoromethyl)benzoyl]piperazin-1-yl]pyridazine-3-carboxamide |
| EI14-18-02R | EI14-18 | 2L18 | N-tert-Butylsulfonyl-6-[4-[3-(5-hydroxypyridin-3-yl)-5-(trifluoromethyl)benzoyl]piperazin-1-yl]pyridazine-3-carboxamide |
| EI14-19-02R | EI14-19 | 2L19 | N-tert-Butylsulfonyl-6-[4-[3-[2-(3-hydroxyphenyl)ethynyl]-5-(trifluoromethyl)benzoyl]piperazin-1-yl]pyridazine-3-carboxamide |
| EI14-20-02R | EI14-20 | 2L20 | N-tert-Butylsulfonyl-6-[4-[3-[2-(5-hydroxypyridin-3-yl)ethynyl]-5-(trifluoromethyl)benzoyl]piperazin-1-yl]pyridazine-3-carboxamide |
| EI14-21-02R | EI14-21 | 2L21 | N-tert-Butylsulfonyl-6-[4-[3-[4-(3-hydroxyphenyl)pyrazol-1-yl]-5-(trifluoromethyl)benzoyl]piperazin-1-yl]pyridazine-3- carboxamide |
| EI14-22-02R | EI14-22 | 2L22 | N-tert-Butylsulfonyl-6-[4-[3-[4-(5-hydroxypyridin-3-yl)pyrazol-1-yl]-5-(trifluoromethyl)benzoyl]piperazin-1- yl]pyridazine-3-carboxamide |
| EI14-23-02R | EI14-23 | 2L23 | N-tert-Butylsulfonyl-4-[4-[4-(3-hydroxyphenyl)thiophene-2-carbonyl]piperazin-1-yl]benzamide |
| EI14-24-02R | EI14-24 | 2L24 | N-tert-Butylsulfonyl-4-[4-[4-(5-hydroxypyridin-3-yl)thiophene-2-carbonyl]piperazin-1-yl]benzamide |
| EI14-25-02R | EI14-25 | 2L25 | N-tert-Butylsulfonyl-4-[4-[4-(3-hydroxyphenyl)-3-methylbenzoyl]piperazin-1 -yl]benzamide |
| EI14-26-02R | EI14-26 | 2L26 | N-tert-Butylsulfonyl-4-[4-[4-(5-hydroxypyridin-3-yl)-3-methylbenzoyl]piperazin-1-yl]benzamide |
| EI14-27-02R | EI14-27 | 2L27 | N-tert-Butylsulfonyl-4-[4-[3-fluoro-4-(3-hydroxyphenyl)benzoyl]piperazin-1-yl]benzamide |
| EI14-28-02R | EI14-28 | 2L28 | N-tert-Butylsulfonyl-4-[4-[3-fluoro-4-(5-hydroxypyridin-3-yl)benzoyl]piperazin-1-yl]benzamide |
| EI14-29-02R | EI14-29 | 2L29 | N-tert-Butylsulfonyl-4-[4-[2-[2-(3-hydroxyphenyl)ethynyl]benzoyl]piperazin-1-yl]benzamide |
| EI14-30-02R | EI14-30 | 2L30 | N-tert-Butylsulfonyl-4-[4-[2-[2-(5-hydroxypyridin-3-yl)ethynyl]benzoyl]piperazin-1-yl]benzamide |

(continued)

| Resin compound No. | Post-cleavage compound No. | Another name of post-cleavage compound in Table EJ08 | Compound name |
|---|---|---|---|
| EI14-31-02R | EI14-31 | 2L31 | N-tert-Butylsulfonyl-4-[4-[5-[2-(3-hydroxyphenyl)ethynyl]pyridine-3-carbonyl]piperazin-1-yl]benzamide |
| EI14-32-02R | EI14-32 | 2L32 | N-tert-Butylsulfonyl-4-[4-[5-[2-(5-hydroxypyridin-3-yl)ethynyl]pyridine-3-carbonyl]piperazin-1-yl]benzamide |
| EI14-33-02R | EI14-33 | 2L33 | N-tert-Butylsulfonyl-4-[4-[3-[2-(3-hydroxyphenyl)phenyl]propanoyl]piperazin-1-yl]benzamide |
| EI14-34-02R | EI14-34 | 2L34 | N-tert-Butylsulfonyl-4-[4-[3-[2-(5-hydroxypyridin-3-yl)phenyl]propanoyl]piperazin-1-yl]benzamide |
| EI14-35-02R | EI14-35 | 2L35 | N-tert-Butylsulfonyl-4-[4-[4-(3-hydroxyphenyl)naphthalene-1-carbonyl]piperazin-1-yl]benzamide |
| EI14-36-02R | EI14-36 | 2L36 | N-tert-Butylsulfonyl-4-[4-[4-(5-hydroxypyridin-3-yl)naphthalene-1-carbonyl]piperazin-1-yl]benzamide |
| EI14-37-02R | EI14-37 | 2L37 | 4-[4-[3-tert-Butyl-5-(3-hydroxyphenyl)benzoyl]piperazin-1-yl]-N-tert-butylsulfonylbenzamide |
| EI14-38-02R | EI14-38 | 2L38 | 4-[4-[3-tert-Butyl-5-(5-hydroxypyridin-3-yl)benzoyl]piperazin-1-yl]-N-tert-butylsulfonylbenzamide |
| EI14-39-02R | EI14-39 | 2L39 | N-tert-Butylsulfonyl-4-[4-[3-(3-hydroxyphenyl)-5-(trifluoromethyl)benzoyl]piperazin-1-yl]benzamide |
| EI14-40-02R | EI14-40 | 2L40 | N-tert-Butylsulfonyl-4-[4-[3-(5-hydroxypyridin-3-yl)-5-(trifluoromethyl)benzoyl]piperazin-1-yl]benzamide |
| EI14-41-02R | EI14-41 | 2L41 | N-tert-Butylsulfonyl-4-[4-[3-[2-(3-hydroxyphenyl)ethynyl]-5-(trifluoromethyl)benzoyl]piperazin-1-yl]benzamide |
| EI14-42-02R | EI14-42 | 2L42 | N-tert-Butylsulfonyl-4-[4-[3-[2-(5-hydroxypyridin-3-yl)ethynyl]-5-(trifluoromethyl)benzoyl]piperazin-1-yl]benzamide |
| EI14-43-02R | EI14-43 | 2L43 | N-tert-Butylsulfonyl-4-[4-[3-[4-(3-hydroxyphenyl)pyrazol-1-yl]-5-(trifluoromethyl)benzoyl]piperazin-1-yl]benzamide |
| EI14-44-02R | EI14-44 | 2L44 | N-tert-Butylsulfonyl-4-[4-[3-[4-(5-hydroxypyridin-3-yl)pyrazol-1-yl]-5-(trifluoromethyl)benzoyl]piperazin-1-yl]benzamide |
| EI14-45-02R | EI14-45 | 2L45 | N-tert-Butylsulfonyl-4-[4-[[2-(3- hydroxyphenyl)phenyl]methyl]piperazin-1-yl]benzamide |
| EI14-46-02R | EI14-46 | 2L46 | N-tert-Butylsulfonyl-4-[4-[[2-(5-hydroxypyridin-3-yl)phenyl]methyl]piperazin-1-yl]benzamide |
| EI14-47-02R | EI14-47 | 2L47 | N-tert-Butylsulfonyl-4-[4-[[4-(3-hydroxyphenyl)-3-methylpkenyllmethyl]piperazin-1-yl]benzamide |
| EI14-48-02R | EI14-48 | 2L48 | N-tert-Butylsulfonyl-4-[4-[[4-(5-hydroxypyridin-3-yl)-3-methylpkenyllmethyl]piperazin-1-yl]benzamide |
| EI14-49-02R | EI14-49 | 2L49 | N-tert-Butylsulfonyl-4-[4-[[2-ethyl-4-(3-hydroxyphenyl)phenyl]methyl]piperazin-1-yl]benzamide |

(continued)

| Resin compound No. | Post-cleavage compound No. | Another name of post-cleavage compound in Table EJ08 | Compound name |
|---|---|---|---|
| EI14-50-02R | EI14-50 | 2L50 | N-tert-Butylsulfonyl-4-[4-[[2-ethyl-4-(5-hydroxypyridin-3-yl)phenyl]methyl]piperazin-1-yl]benzamide |
| EI14-51-02R | EI14-51 | 2L51 | N-tert-Butylsulfonyl-4-[4-[[3-fluoro-4-(3-hydroxyphenyl)phenyl]methyl]piperazin-1-yl]benzamide |
| EI14-52-02R | EI14-52 | 2L52 | N-tert-Butylsulfonyl-4-[4-[[3-fluoro-4-(5-hydroxypyridin-3-yl)phenyl]methyl]piperazin-1-yl]benzamide |
| EI14-53-02R | EI14-53 | 2L53 | N-tert-Butylsulfonyl-4-[4-[[4-(3-hydroxyphenyl)-2-methoxyphenyl]methyl]piperazin-1-yl]benzamide |
| EI14-54-02R | EI14-54 | 2L54 | N-tert-Butylsulfonyl-4-[4-[[4-(5-hydroxypyridin-3-yl)-2-methoxyphenyl]methyl]piperazin-1-yl]benzamide |
| EI14-55-02R | EI14-55 | 2L55 | N-tert-Butylsulfonyl-4-[4-[[3-ethoxy-5-(3-hydroxyphenyl)phenyl]methyl]piperazin-1-yl]benzamide |
| EI14-56-02R | EI14-56 | 2L56 | N-tert-Butylsulfonyl-4-[4-[[3-ethoxy-5-(5-hydroxypyridin-3-yl)phenyl]methyl]piperazin-1-yl]benzamide |
| EI14-57-02R | EI14-57 | 2L57 | N-tert-Butylsulfonyl-4-[4-[[3-(3-hydroxyphenyl)-5-propan-2-yloxyphenyl]methyl]piperazin-1-yl]benzamide |
| EI14-58-02R | EI14-58 | 2L58 | N-tert-Butylsulfonyl-4-[4-[[3-(5-hydroxypyridin-3-yl)-5-propan-2-yloxyphenyl]methyl]piperazin-1-yl]benzamide |
| EI14-59-02R | EI14-59 | 2L59 | N-tert-Butylsulfonyl-4-[4-[[4-(3-hydroxyphenyl)-2,6-dimethoxyphenyl]methyl]piperazin-1-yl]benzamide |
| EI14-60-02R | EI14-60 | 2L60 | N-tert-Butylsulfonyl-4-[4-[[4-(5-hydroxypyridin-3-yl)-2,6-dimethoxyphenyl]methyl]piperazin-1-yl]benzamide |
| EI14-61-02R | EI14-61 | 2L61 | N-tert-Butylsulfonyl-4-[4-[[3-(3-hydroxyphenyl)-5-(trifluoromethyl)phenyl]methyl]piperazin-1-yl]benzamide |
| EI14-62-02R | EI14-62 | 2L62 | N-tert-Butylsulfonyl-4-[4-[[3-(5-hydroxypyridin-3-yl)-5-(trifluoromethyl)phenyl]methyl]piperazin-1-yl]benzamide |
| EI14-63-02R | EI14-63 | 2L63 | N-tert-Butylsulfonyl-4-[4-[[3-(3-hydroxyphenyl)-5-(trifluoromethoxy)phenyl]methyl]piperazin-1-yl]benzamide |
| EI14-64-02R | EI14-64 | 2L64 | N-tert-Butylsulfonyl-4-[4-[[3-(5-hydroxypyridin-3-yl)-5-(trifluoromethoxy)phenyl]methyl]piperazin-1-yl]benzamide |
| EI14-65-02R | EI14-65 | 2L65 | N-tert-Butylsulfonyl-4-[4-[[4-(3-hydroxyphenyl)naphthalen-1-yl]methyl]piperazin-1-yl]benzamide |
| EI14-66-02R | EI14-66 | 2L66 | N-tert-Butylsulfonyl-4-[4-[[4-(5-hydroxypyridin-3-yl)naphthalen-1-yl]methyl]piperazin-1-yl]benzamide |
| EI14-67-02R | EI14-67 | 2L67 | N-tert-Butylsulfonyl-4-[4-[[4-(3-hydroxyphenyl)thiophen-2-yl]methyl]piperazin-1-yl]benzamide |

(continued)

| Resin compound No. | Post-cleavage compound No. | Another name of post-cleavage compound in Table EJ08 | Compound name |
|---|---|---|---|
| EI14-68-02R | EI14-68 | 2L68 | N-tert-Butylsulfonyl-4-[4-[[4-(5-hydroxypyridin-3-yl)thiophen-2-yl]methyl]piperazin-1-yl]benzamide |
| EI14-69-02R | EI14-69 | 2L69 | N-tert-Butylsulfonyl-4-[4-[[2-[2-(3-hydroxyphenyl)ethynyl]phenyl]methyl]piperazin-1-yl]benzamide |
| EI14-70-02R | EI14-70 | 2L70 | N-tert-Butylsulfonyl-4-[4-[[2-[2-(5-hydroxypyridin-3-yl)ethynyl]phenyl]methyl]piperazin-1-yl]benzamide |
| EI14-71-02R | EI14-71 | 2L71 | N-tert-Butylsulfonyl-6-[4-[[2-(- hydroxyphenyl)phenyl]methyl]piperazin-1 -yl]pyridazine-3- carboxamide |
| EI14-72-02R | EI14-72 | 2L72 | N-tert-Butylsulfonyl-6-[4-[[2-(5-hydroxypyridin-3-yl)phenyl]methyl]piperazin-1-yl]pyridazine-3-carboxamide |
| EI14-73-02R | EI14-73 | 2L73 | N-tert-Butylsulfonyl-6-[4-[[4-(3-hydroxyphenyl)-3-methylphenyl]methyl]piperazin-1-yl]pyridazine-3-carboxamide |
| EI14-74-02R | EI14-74 | 2L74 | N-tert-Butylsulfonyl-6-[4-[[4-(5-hydroxypyridin-3-yl)-3-methylphenyl]methyl]piperazin-1-yl]pyridazine-3-carboxamide |
| EI14-75-02R | EI14-75 | 2L75 | N-tert-Butylsulfonyl-6-[4-[[2-ethyl-4-(3-hydroxyphenyl)phenyl]methyl]piperazin-1-yl]pyridazine-3-carboxamide |
| EI14-76-02R | EI14-76 | 2L76 | N-tert-Butylsulfonyl-6-[4-[[2-ethyl-4-(5-hydroxypyridin-3-yl)phenyl]methyl]piperazin-1-yl]pyridazine-3-carboxamide |
| EI14-77-02R | EI14-77 | 2L77 | N-tert-Butylsulfonyl-6-[4-[[3-fluoro-4-(3-hydroxyphenyl)phenyl]methyl]piperazin-1-yl]pyridazine-3-carboxamide |
| EI14-78-02R | EI14-78 | 2L78 | N-tert-Butylsulfonyl-6-[4-[[3-fluoro-4-(5-hydroxypyridin-3-yl)phenyl]methyl]piperazin-1-yl]pyridazine-3-carboxamide |
| EI14-79-02R | EI14-79 | 2L79 | N-tert-Butylsulfonyl-6-[4-[[4-(3-hydroxyphenyl)-2-methoxyphenyl]methyl]piperazin-1-yl]pyridazine-3-carboxamide |
| EI14-80-02R | EI14-80 | 2L80 | N-tert-Butylsulfonyl-6-[4-[[4-(5-hydroxypyridin-3-yl)-2-methoxyphenyl]methyl]piperazin-1-yl]pyridazine-3-carboxamide |
| EI14-81-02R | EI14-81 | 2L81 | N-tert-Butylsulfonyl-6-[4-[[3-ethoxy-5-(3-hydroxyphenyl)phenyl]methyl]piperazin-1-yl]pyridazine-3-carboxamide |
| EI14-82-02R | EI14-82 | 2L82 | N-tert-Butylsulfonyl-6-[4-[[3-ethoxy-5-(5-hydroxypyridin-3-yl)phenyl]methyl]piperazin-1-yl]pyridazine-3-carboxamide |

(continued)

| Resin compound No. | Post-cleavage compound No. | Another name of post-cleavage compound in Table EJ08 | Compound name |
|---|---|---|---|
| EI14-83-02R | EI14-83 | 2L83 | N-tert-Butylsulfonyl-6-[4-[[3-(3-hydroxyphenyl)-5-propan-2-yloxyphenyl]methyl]piperazin-1-yl]pyridazine-3-carboxamide |
| EI14-84-02R | EI14-84 | 2L84 | N-tert-Butylsulfonyl-6-[4-[[3-(5-hydroxypyridin-3-yl)-5-propan-2-yloxyphenyl]methyl]piperazin-1-yl]pyridazine-3-carboxamide |
| EI14-85-02R | EI14-85 | 2L85 | N-tert-Butylsulfonyl-6-[4-[[4-(3-hydroxyphenyl)-2,6-dimethoxyphenyl]methyl]piperazin-1-yl]pyridazine-3-carboxamide |
| EI14-86-02R | EI14-86 | 2L86 | N-tert-Butylsulfonyl-6-[4-[[4-(5-hydroxypyridin-3-yl)-2,6-dimethoxyphenyl]methyl]piperazin-1-yl]pyridazine-3-carboxamide |
| EI14-87-02R | EI14-87 | 2L87 | N-tert-Butylsulfonyl-6-[4-[[3-(3-hydroxyphenyl)-5-(trifluoromethyl)phenyl]methyl]piperazin-1-yl]pyridazine-3-carboxamide |
| EI14-88-02R | EI14-88 | 2L88 | N-tert-Butylsulfonyl-6-[4-[[3-(5-hydroxypyridin-3-yl)-5-(trifluoromethyl)phenyl]methyl]piperazin-1-yl]pyridazine-3-carboxamide |
| EI14-89-02R | EI14-89 | 2L89 | N-tert-Butylsulfonyl-6-[4-[[3-(3-hydroxyphenyl)-5-(trifluoromethoxy)phenyl]methyl]piperazin-1-yl]pyridazine-3- carboxamide |
| EI14-90-02R | EI14-90 | 2L90 | N-tert-Butylsulfonyl-6-[4-[[3-(5-hydroxypyridin-3-yl)-5-(trifluoromethoxy)phenyl]methyl]piperazin-1-yl]pyridazine-3-carboxamide |
| EI14-91-02R | EI14-91 | 2L91 | N-tert-Butylsulfonyl-6-[4-[[4-(3-hydroxyphenyl)naphthalen-1-yl]methyl]piperazin-1-yl]pyridazine-3-carboxamide |
| EI14-92-02R | EI14-92 | 2L92 | N-tert-Butylsulfonyl-6-[4-[[4-(5-hydroxypyridin-3-yl)naphthalen-1-yl]methyl]piperazin-1-yl]pyridazine-3-carboxamide |
| EI14-93-02R | EI14-93 | 2L93 | N-tert-Butylsulfonyl-6-[4-[[4-(3-hydroxyphenyl)thiophen-2-yl]methyl]piperazin-1-yl]pyridazine-3-carboxamide |
| EI14-94-02R | EI14-94 | 2L94 | N-tert-Butylsulfonyl-6-[4-[[4-(5-hydroxypyridin-3-yl)thiophen-2-yl]methyl]piperazin-1-yl]pyridazine-3-carboxamide |
| EI14-95-02R | EI14-95 | 2L95 | N-tert-Butylsulfonyl-6-[4-[[2-[2-(3-hydroxyphenyl)ethynyl]phenyl]methyl]piperazin-1-yl]pyridazine-3-carboxamide |
| EI14-96-02R | EI14-96 | 2L96 | N-tert-Butylsulfonyl-6-[4-[[2-[2-(5-hydroxypyridin-3-yl)ethynyl]phenyl]methyl]piperazin-1-yl]pyridazine-3-carboxamide |

(continued)

| Resin compound No. | Post-cleavage compound No. | Another name of post-cleavage compound in Table EJ08 | Compound name |
|---|---|---|---|
| EI14-97-02R | EI14-97 | 2L97 | N-tert-Butylsulfonyl-4-[4-[1-[4-(3-hydroxyphenyl)phenyl]ethyl]piperazin-1-yl]benzamide |
| EI14-98-02R | EI14-98 | 2L98 | N-tert-Butylsulfonyl-4-[4-[1-[4-(5-hydroxypyridin-3-yl)phenyl]ethyl]piperazin-1-yl]benzamide |
| EI14-99-02R | EI14-99 | 2L99 | N-tert-Butylsulfonyl-4-[4-[1-[2-fluoro-4-(3-hydroxyphenyl)phenyl]ethyl]piperazin-1-yl]benzamide |
| EI14-100-02R | EI14-100 | 2L100 | N-tert-Butylsulfonyl-4-[4-[1-[2-fluoro-4-(5-hydroxypyridin-3-yl)phenyl]ethyl]piperazin-1-yl]benzamide |

[Table 67]

[Table EI15] mixEI15-02R, amine reagent used: BB38, post-cleavage mixture No.: mixEI15

| Resin compound No. | Post-cleavage compound No. | Another name of post-cleavage compound in Table EJ09 | Compound name |
|---|---|---|---|
| EI15-01-02R | EI15-01 | 2K01 | 6-[4-[4-(3-Hydroxyphenyl)thiophene-2-carbonyl]piperazin-1-yl]-N-(3,3,3-trifluoropropylsulfonyl)pyridazine-3-carboxamide |
| EI15-02-02R | EI15-02 | 2K02 | 6-[4-[4-(5-Hydroxypyridin-3-yl)thiophene-2-carbonyl]piperazin-1-yl]-N-(3,3,3-trifluoropropylsulfonyl)pyridazine-3-carboxamide |
| EI15-03-02R | EI15-03 | 2K03 | 6-[4-[4-(3-Hydroxyphenyl)-3-methylbenzoyl]piperazin-1-yl]-N-(3,3,3-trifluoropropylsulfonyl)pyridazine-3-carboxamide |
| EI15-04-02R | EI15-04 | 2K04 | 6-[4-[4-(5-Hydroxypyridin-3-yl)-3-methylbenzoyl]piperazin-1-yl]-N-(3,3,3-trifluoropropylsulfonyl)pyridazine-3- carboxamide |
| EI15-05-02R | EI15-05 | 2K05 | 6-[4-[3-Fluoro-4-(3-hydroxyphenyl)benzoyl]piperazin-1-yl]-N-(3,3,3-trifluoropropylsulfonyl)pyridazine-3-carboxamide |
| EI15-06-02R | EI15-06 | 2K06 | 6-[4-[3-Fluoro-4-(5-hydroxypyridin-3-yl)benzoyl]piperazin-1-yl]-N-(3,3,3-trifluoropropylsulfonyl)pyridazine-3- carboxamide |
| EI15-07-02R | EI15-07 | 2K07 | 6-[4-[2-[2-(3-Hydroxyphenyl)ethynyl]benzoyl]piperazin-1-yl]-N-(3,3,3-trifluoropropylsulfonyl)pyridazine-3-carboxamide |
| EI15-08-02R | EI15-08 | 2K08 | 6-[4-[2-[2-(5-Hydroxypyridin-3-yl)ethynyl]benzoyl]piperazin-1-yl]-N-(3,3,3-trifluoropropylsulfonyl)pyridazine-3-carboxamide |
| EI15-09-02R | EI15-09 | 2K09 | 6-[4-[5-[2-(3-Hydroxyphenyl)ethynyl]pyridine-3-carbonyl]piperazin-1-yl]-N-(3,3,3-trifluoropropylsulfonyl)pyridazine-3-carboxamide |
| EI15-10-02R | EI15-10 | 2K10 | 6-[4-[5-[2-(5-Hydroxypyridin-3-yl)ethynyl]pyridine-3-carbonyl]piperazin-1-yl]-N-(3,3,3-trifluoropropylsulfonyl)pyridazine-3-carboxamide |
| EI15-11-02R | EI15-11 | 2K11 | 6-[4-[3-[2-(3-Hydroxyphenyl)phenyl]propanoyl]piperazin-1-yl]-N-(3,3,3-trifluoropropylsulfonyl)pyridazine-3-carboxamide |
| EI15-12-02R | EI15-12 | 2K12 | 6-[4-[3-[2-(5-Hydroxypyridin-3-yl)phenyl]propanoyl]piperazin-1-yl]-N-(3,3,3-trifluoropropylsulfonyl)pyridazine-3-carboxamide |
| EI15-13-02R | EI15-13 | 2K13 | 6-[4-[4-(3-Hydroxyphenyl)naphthalene-1-carbonyl]piperazin-1-yl]-N-(3,3,3-trifluoropropylsulfonyl)pyridazine-3- carboxamide |

(continued)

| Resin compound No. | Post-cleavage compound No. | Another name of post-cleavage compound in Table EJ09 | Compound name |
|---|---|---|---|
| EI15-14-02R | EI15-14 | 2K14 | 6-[4-[4-(5-Hydroxypyridin-3-yl)naphthalene-1-carbonyl]piperazin-1-yl]-N-(3,3,3-trifluoropropylsulfonyl)pyridazine-3-carboxamide |
| EI15-15-02R | EI15-15 | 2K15 | 6-[4-[3-tert-Butyl-5-(3-hydroxyphenyl)benzoyl]piperazin-1-yl]-N-(3,3,3-trifluoropropylsulfonyl)pyridazine-3- carboxamide |
| EI15-16-02R | EI15-16 | 2K16 | 6-[4-[3-tert-Butyl-5-(5-hydroxypyridin-3-yl)benzoyl]piperazin-1-yl]-N-(3,3,3-trifluoropropylsulfonyl)pyridazine-3-carboxamide |
| EI15-17-02R | EI15-17 | 2K17 | 6-[4-[3-(3-Hydroxyphenyl)-5-(trifluoromethyl)benzoyl]piperazin-1-yl]-N-(3,3,3-trifluoropropylsulfonyl)pyridazine-3-carboxamide |
| EI15-18-02R | EI15-18 | 2K18 | 6-[4-[3-(5-Hydroxypyridin-3-yl)-5-(trifluoromethyl)benzoyl]piperazin-1-yl]-N-(3,3,3-trifluoropropylsulfonyl)pyridazine-3-carboxamide |
| EI15-19-02R | EI15-19 | 2K19 | 6-[4-[3-[2-(3-Hydroxyphenyl)ethynyl]-5-(trifluoromethyl)benzoyl]piperazin-1-yl]-N-(3,3,3-trifluoropropylsulfonyl)pyridazine-3-carboxamide |
| EI15-20-02R | EI15-20 | 2K20 | 6-[4-[3-[2-(5-Hydroxypyridin-3-yl)ethynyl]-5-(trifluoromethyl)benzoyl]piperazin-1-yl]-N-(3,3,3-trifluoropropylsulfonyl)pyridazine-3-carboxamide |
| EI15-21-02R | EI15-21 | 2K21 | 6-[4-[3-[4-(3-Hydroxyphenyl)pyrazol-1-yl]-5-(trifluoromethyl)benzoyl]piperazin-1-yl]-N-(3,3,3-trifluoropropylsulfonyl)pyridazine-3-carboxamide |
| EI15-22-02R | EI15-22 | 2K22 | 6-[4-[3-[4-(5-Hydroxypyridin-3-yl)pyrazol-1-yl]-5-(trifluoromethyl)benzoyl]piperazin-1-yl]-N-(3,3,3-trifluoropropylsulfonyl)pyridazine-3-carboxamide |
| EI15-23-02R | EI15-23 | 2K23 | 4-[4-[4-(3-Hydroxyphenyl)thiophene-2-carbonyl]piperazin-1-yl]-N-(3,3,3-trifluoropropylsulfonyl)benzamide |
| EI15-24-02R | EI15-24 | 2K24 | 4-[4-[4-(5-Hydroxypyridin-3-yl)thiophene-2-carbonyl]piperazin-1-yl]-N-(3,3,3-trifluoropropylsulfonyl)benzamide |
| EI15-25-02R | EI15-25 | 2K25 | 4-[4-[4-(3-Hydroxyphenyl)-3-methylbenzoyl]piperazin-1-yl]-N-(3,3,3-trifluoropropylsulfonyl)benzamide |
| EI15-26-02R | EI15-26 | 2K26 | 4-[4-[4-(5-Hydroxypyridin-3-yl)-3-methylbenzoyl]piperazin-1-yl]-N-(3,3,3-trifluoropropylsulfonyl)benzamide |
| EI15-27-02R | EI15-27 | 2K27 | 4-[4-[3-Fluoro-4-(3-hydroxyphenyl)benzoyl]piperazin-1-yl]-N-(3,3,3-trifluoropropylsulfonyl)benzamide |
| EI15-28-02R | EI15-28 | 2K28 | 4-[4-[3-Fluoro-4-(5-hydroxypyridin-3-yl)benzoyl]piperazin-1-yl]-N-(3,3,3-trifluoropropylsulfonyl)benzamide |

(continued)

| Resin compound No. | Post-cleavage compound No. | Another name of post-cleavage compound in Table EJ09 | Compound name |
|---|---|---|---|
| EI15-29-02R | EI15-29 | 2K29 | 4-[4-[2-[2-(3-Hydroxyphenyl)ethynyl]benzoyl]piperazin-1-yl]-N-(3,3,3-trifluoropropylsulfonyl)benzamide |
| EI15-30-02R | EI15-30 | 2K30 | 4-[4-[2-[2-(5-Hydroxypyridin-3-yl)ethynyl]benzoyl]piperazin-1-yl]-N-(3,3,3-trifluoropropylsulfonyl)benzamide |
| EI15-31-02R | EI15-31 | 2K31 | 4-[4-[5-[2-(3-Hydroxyphenyl)ethynyl]pyridine-3-carbonyl]piperazin-1-yl]-N-(3,3,3-trifluoropropylsulfonyl)benzamide |
| EI15-32-02R | EI15-32 | 2K32 | 4-[4-[5-[2-(5-Hydroxypyridin-3-yl)ethynyl]pyridine-3-carbonyl]piperazin-1-yl]-N-(3,3,3-trifluoropropylsulfonyl)benzamide |
| EI15-33-02R | EI15-33 | 2K33 | 4-[4-[3-[2-(3-Hydroxyphenyl)phenyl]propanoyl]piperazin-1-yl]-N-(3,3,3-trifluoropropylsulfonyl)benzamide |
| EI15-34-02R | EI15-34 | 2K34 | 4-[4-[3-[2-(5-Hydroxypyridin-3-yl)phenyl]propanoyl]piperazin-1-yl]-N-(3,3,3-trifluoropropylsulfonyl)benzamide |
| EI15-35-02R | EI15-35 | 2K35 | 4-[4-[4-(3-Hydroxyphenyl)naphthalene-1-carbonyl]piperazin-1-yl]-N-(3,3,3-trifluoropropylsulfonyl)benzamide |
| EI15-36-02R | EI15-36 | 2K36 | 4-[4-[4-(5-Hydroxypyridin-3-yl)naphthalene-1-carbonyl]piperazin-1-yl]-N-(3,3,3-trifluoropropylsulfonyl)benzamide |
| EI15-37-02R | EI15-37 | 2K37 | 4-[4-[3-tert-Butyl-5-(3-hydroxyphenyl)benzoyl]piperazin-1-yl]-N-(3,3,3-trifluoropropylsulfonyl)benzamide |
| EI15-38-02R | EI15-38 | 2K38 | 4-[4-[3-tert-Butyl-5-(5-hydroxypyridin-3-yl)benzoyl]piperazin-1-yl]-N-(3,3,3-trifluoropropylsulfonyl)benzamide |
| EI15-39-02R | EI15-39 | 2K39 | 4-[4-[3-(3-Hydroxyphenyl)-5-(trifluoromethyl)benzoyl]piperazin-1-yl]-N-(3,3,3-trifluoropropylsulfonyl)benzamide |
| EI15-40-02R | EI15-40 | 2K40 | 4-[4-[3-(5-Hydroxypyridin-3-yl)-5-(trifluoromethyl)benzoyl]piperazin-1-yl]-N-(3,3,3-trifluoropropylsulfonyl)benzamide |
| EI15-41-02R | EI15-41 | 2K41 | 4-[4-[3-[2-(3-Hydroxyphenyl)ethynyl]-5-(trifluoromethyl)benzoyl]piperazin-1-yl]-N-(3,3,3-trifluoropropylsulfonyl)benzamide |
| EI15-42-02R | EI15-42 | 2K42 | 4-[4-[3-[2-(5-Hydroxypyridin-3-yl)ethynyl]-5-(trifluoromethyl)benzoyl]piperazin-1-yl]-N-(3,3,3-trifluoropropylsulfonyl)benzamide |
| EI15-43-02R | EI15-43 | 2K43 | 4-[4-[3-[4-(3-Hydroxyphenyl)pyrazol-1-yl]-5-(trifluoromethyl)benzoyl]piperazin-1-yl] -N-(3,3,3-trifluoropropylsulfonyl)benzamide |

(continued)

| Resin compound No. | Post-cleavage compound No. | Another name of post-cleavage compound in Table EJ09 | Compound name |
|---|---|---|---|
| EI15-44-02R | EI15-44 | 2K44 | 4-[4-[3-[4-(5-Hydroxypyridin-3-yl)pyrazol-1-yl]-5-(trifluoromethyl)benzoyl]piperazin-1-yl]-N-(3,3,3-trifluoropropylsulfonyl)benzamide |
| EI15-45-02R | EI15-45 | 2K45 | 4-[4-[[2-(3-Hydroxyphenyl)phenyl]methyl]piperazin-1-yl]-N-(3,3,3-trifluoropropylsulfonyl)benzamide |
| EI15-46-02R | EI15-46 | 2K46 | 4-[4-[[2-(5-Hydroxypyridin-3-yl)phenyl]methyl]piperazin-1-yl]-N-(3,3,3-trifluoropropylsulfonyl)benzamide |
| EI15-47-02R | EI15-47 | 2K47 | 4-[4-[[4-(3-Hydroxyphenyl)-3-methylphenyl]methyl]piperazin-1-yl]-N-(3,3,3-trifluoropropylsulfonyl)benzamide |
| EI15-48-02R | EI15-48 | 2K48 | 4-[4-[[4-(5-Hydroxypyridin-3-yl)-3-methylphenyl]methyl]piperazin-1-yl]-N-(3,3,3-trifluoropropylsulfonyl)benzamide |
| EI15-49-02R | EI15-49 | 2K49 | 4-[4-[[2-Ethyl-4-(3-hydroxyphenyl)phenyl]methyl]piperazin-1-yl]-N-(3,3,3-trifluoropropylsulfonyl)benzamide |
| EI15-50-02R | EI15-50 | 2K50 | 4-[4-[[2-Ethyl-4-(5-hydroxypyridin-3-yl)phenyl]methyl]piperazin-1-yl]-N-(3,3,3-trifluoropropylsulfonyl)benzamide |
| EI15-51-02R | EI15-51 | 2K51 | 4-[4-[[3-Fluoro-4-(3-hydroxyphenyl)phenyl]methyl]piperazin-1-yl]-N-(3,3,3-trifluoropropylsulfonyl)benzamide |
| EI15-52-02R | EI15-52 | 2K52 | 4-[4-[[3-Fluoro-4-(5-hydroxypyridin-3-yl)phenyl]methyl]piperazin-1-yl]-N-(3,3,3-trifluoropropylsulfonyl)benzamide |
| EI15-53-02R | EI15-53 | 2K53 | 4-[4-[[4-(3-Hydroxyphenyl)-2-methoxyphenyl]methyl]piperazin-1-yl]-N-(3,3,3-trifluoropropylsulfonyl)benzamide |
| EI15-54-02R | EI15-54 | 2K54 | 4-[4-[[4-(5-Hydroxypyridin-3-yl)-2-methoxyphenyl]methyl]piperazin-1-yl]-N-(3,3,3-trifluoropropylsulfonyl)benzamide |
| EI15-55-02R | EI15-55 | 2K55 | 4-[4-[[3-Ethoxy-5-(3-hydroxyphenyl)phenyl]methyl]piperazin-1-yl]-N-(3,3,3-trifluoropropylsulfonyl)benzamide |
| EI15-56-02R | EI15-56 | 2K56 | 4-[4-[[3-Ethoxy-5-(5-hydroxypyridin-3-yl)phenyl]methyl]piperazin-1-yl]-N-(3,3,3-trifluoropropylsulfonyl)benzamide |
| EI15-57-02R | EI15-57 | 2K57 | 4-[4-[[3-(3-Hydroxyphenyl)-5-propan-2-yloxyphenyl]methyl]piperazin-1-yl]-N-(3,3,3-trifluoropropylsulfonyl)benzamide |
| EI15-58-02R | EI15-58 | 2K58 | 4-[4-[[3-(5-Hydroxypyridin-3-yl)-5-propan-2-yloxyphenyl]methyl]piperazin-1-yl]-N-(3,3,3-trifluoropropylsulfonyl)benzamide |

(continued)

| Resin compound No. | Post-cleavage compound No. | Another name of post-cleavage compound in Table EJ09 | Compound name |
|---|---|---|---|
| EI15-59-02R | EI15-59 | 2K59 | 4-[4-[[4-(3-Hydroxyphenyl)-2,6-dimethoxyphenyl]methyl]piperazin-1-yl]-N-(3,3,3-trifluoropropylsulfonyl)benzamide |
| EI15-60-02R | EI15-60 | 2K60 | 4-[4-[[4-(5-Hydroxypyridin-3-yl)-2,6-dimethoxyphenyl]methyl]piperazin-1-yl]-N-(3,3,3-trifluoropropylsulfonyl)benzamide |
| EI15-61-02R | EI15-61 | 2K61 | 4-[4-[[3-(3-Hydroxyphenyl)-5-(trifluoromethyl)phenyl]methyl]piperazin-1-yl]-N-(3,3,3-trifluoropropylsulfonyl)benzamide |
| EI15-62-02R | EI15-62 | 2K62 | 4-[4-[[3-(5-Hydroxypyridin-3-yl)-5-(trifluoromethyl)phenyl]methyl]piperazin-1-yl]-N-(3,3,3-trifluoropropylsulfonyl)benzamide |
| EI15-63-02R | EI15-63 | 2K63 | 4-[4-[[3-(3-Hydroxyphenyl)-5-(trifluoromethoxy)phenyl]methyl]piperazin-1-yl]-N-(3,3,3-trifluoropropylsulfonyl)benzamide |
| EI15-64-02R | EI15-64 | 2K64 | 4-[4-[[3-(5-Hydroxypyridin-3-yl)-5-(trifluoromethoxy)phenyl]methyl]piperazin-1-yl]-N-(3,3,3-trifluoropropylsulfonyl)benzamide |
| EI15-65-02R | EI15-65 | 2K65 | 4-[4-[[4-(3-Hydroxyphenyl)naphthalen-1-yl]methyl]piperazin-1-yl]-N-(3,3,3-trifluoropropylsulfonyl)benzamide |
| EI15-66-02R | EI15-66 | 2K66 | 4-[4-[[4-(5-Hydroxypyridin-3-yl)naphthalen-1-yl]methyl]piperazin-1-yl]-N-(3,3,3-trifluoropropylsulfonyl)benzamide |
| EI15-67-02R | EI15-67 | 2K67 | 4-[4-[[4-(3-Hydroxyphenyl)thiophen-2-yl]methyl]piperazin-1-yl]-N-(3,3,3-trifluoropropylsulfonyl)benzamide |
| EI15-68-02R | EI15-68 | 2K68 | 4-[4-[[4-(5-Hydroxypyridin-3-yl)thiophen-2-yl]methyl]piperazin-1-yl]-N-(3,3,3-trifluoropropylsulfonyl)benzamide |
| EI15-69-02R | EI15-69 | 2K69 | 4-[4-[[2-[2-(3-Hydroxyphenyl)ethynyl]phenyl]methyl]piperazin-1-yl]-N-(3,3,3-trifluoropropylsulfonyl)benzamide |
| EI15-70-02R | EI15-70 | 2K70 | 4-[4-[[2-[2-(5-Hydroxypyridin-3-yl)ethynyl]phenyl]methyl]piperazin-1-yl]-N-(3,3,3-trifluoropropylsulfonyl)benzamide |
| EI15-71-02R | EI15-71 | 2K71 | 6-[4-[[2-(3-Hydroxyphenyl)phenyl]methyl]piperazin-1-yl]-N-(3,3,3-trifluoropropylsulfonyl)pyridazine-3-carboxamide |
| EI15-72-02R | EI15-72 | 2K72 | 6-[4-[[2-(5-Hydroxypyridin-3-yl)phenyl]methyl]piperazin-1-yl]-N-(3,3,3-trifluoropropylsulfonyl)pyridazine-3- carboxamide |
| EI15-73-02R | EI15-73 | 2K73 | 6-[4-[[4-(3-Hydroxyphenyl)-3-methylphenyl]methyl]piperazin-1-yl]-N-(3,3,3-trifluoropropylsulfonyl)pyridazine-3-carboxamide |

(continued)

| Resin compound No. | Post-cleavage compound No. | Another name of post-cleavage compound in Table EJ09 | Compound name |
|---|---|---|---|
| EI15-74-02R | EI15-74 | 2K74 | 6-[4-[[4-(5-Hydroxypyridin-3-yl)-3-methylphenyl]methyl]piperazin-1-yl]-N-(3,3,3-trifluoropropylsulfonyl)pyridazine-3-carboxamide |
| EI15-75-02R | EI15-75 | 2K75 | 6-[4-[[2-Ethyl-4-(3-hydroxyphenyl)phenyl]methyl]piperazin-1-yl]-N-(3,3,3-trifluoropropylsulfonyl)pyridazine-3- carboxamide |
| EI15-76-02R | EI15-76 | 2K76 | 6-[4-[[2-Ethyl-4-(5-hydroxypyridin-3-yl)phenyl]methyl]piperazin-1-yl]-N-(3,3,3-trifluoropropylsulfonyl)pyridazine-3-carboxamide |
| EI15-77-02R | EI15-77 | 2K77 | 6-[4-[[3-Fluoro-4-(3-hydroxyphenyl)phenyl]methyl]piperazin-1-yl]-N-(3,3,3-trifluoropropylsulfonyl)pyridazine-3- carboxamide |
| EI15-78-02R | EI15-78 | 2K78 | 6-[4-[[3-Fluoro-4-(5-hydroxypyridin-3-yl)phenyl]methyl]piperazin-1-yl]-N-(3,3,3-trifluoropropylsulfonyl)pyridazine-3-carboxamide |
| EI15-79-02R | EI15-79 | 2K79 | 6-[4-[[4-(3-Hydroxyphenyl)-2-methoxyphenyl]methyl]piperazin-1-yl]-N-(3,3,3-trifluoropropylsulfonyl)pyridazine-3-carboxamide |
| EI15-80-02R | EI15-80 | 2K80 | 6-[4-[[4-(5-Hydroxypyridin-3-yl)-2-methoxyphenyl]methyl]piperazin-1-yl]-N-(3,3,3-trifluoropropylsulfonyl)pyridazine-3-carboxamide |
| EI15-81-02R | EI15-81 | 2K81 | 6-[4-[[3-Ethoxy-5-(3-hydroxyphenyl)phenyl]methyl]piperazin-1-yl]-N-(3,3,3-trifluoropropylsulfonyl)pyridazine-3-carboxamide |
| EI15-82-02R | EI15-82 | 2K82 | 6-[4-[[3-Ethoxy-5-(5-hydroxypyridin-3-yl)phenyl]methyl]piperazin-1-yl]-N-(3,3,3-trifluoropropylsulfonyl)pyridazine-3-carboxamide |
| EI15-83-02R | EI15-83 | 2K83 | 6-[4-[[3-(3-Hydroxyphenyl)-5-propan-2-yloxyphenyl]methyl]piperazin-1-yl]-N-(3,3,3-trifluoropropylsulfonyl)pyridazine-3-carboxamide |
| EI15-84-02R | EI15-84 | 2K84 | 6-[4-[[3-(5-Hydroxypyridin-3-yl)-5-propan-2-yloxyphenyl]methyl]piperazin-1-yl]-N-(3,3,3-trifluoropropylsulfonyl)pyridazine-3-carboxamide |
| EI15-85-02R | EI15-85 | 2K85 | 6-[4-[[4-(3-Hydroxyphenyl)-2,6-dimethoxyphenyl]methyl]piperazin-1-yl]-N-(3,3,3-trifluoropropylsulfonyl)pyridazine-3-carboxamide |
| EI15-86-02R | EI15-86 | 2K86 | 6-[4-[[4-(5-Hydroxypyridin-3-yl)-2,6-dimethoxyphenyl]methyl]piperazin-1-yl]-N-(3,3,3-trifluoropropylsulfonyl)pyridazine-3-carboxamide |
| EI15-87-02R | EI15-87 | 2K87 | 6-[4-[[3-(3-Hydroxyphenyl)-5-(trifluoromethyl)phenyl]methyl]piperazin-1-yl]-N-(3,3,3-trifluoropropylsulfonyl)pyridazine-3-carboxamide |

(continued)

| Resin compound No. | Post-cleavage compound No. | Another name of post-cleavage compound in Table EJ09 | Compound name |
|---|---|---|---|
| EI15-88-02R | EI15-88 | 2K88 | 6-[4-[[3-(5-Hydroxypyridin-3-yl)-5-(trifluoromethyl)phenyl]methyl]piperazin-1-yl]-N-(3,3,3-trifluoropropylsulfonyl)pyridazine-3-carboxamide |
| EI15-89-02R | EI15-89 | 2K89 | 6-[4-[[3-(3-Hydroxyphenyl)-5-(trifluoromethoxy)phenyl]methyl]piperazin-1-yl]-N-(3,3,3-trifluoropropylsulfonyl)pyridazine-3-carboxamide |
| EI15-90-02R | EI15-90 | 2K90 | 6-[4-[[3-(5-Hydroxypyridin-3-yl)-5-(trifluoromethoxy)phenyl]methyl]piperazin-1-yl]-N-(3,3,3-trifluoropropylsulfonyl)pyridazine-3-carboxamide |
| EI15-91-02R | EI15-91 | 2K91 | 6-[4-[[4-(3-Hydroxyphenyl)naphthalen-1-yl]methyl]piperazin-1-yl]-N-(3,3,3-trifluoropropylsulfonyl)pyridazine-3- carboxamide |
| EI15-92-02R | EI15-92 | 2K92 | 6-[4-[[4-(5-Hydroxypyridin-3-yl)naphthalen-1-yl]methyl]piperazin-1-yl]-N-(3,3,3-trifluoropropylsulfonyl)pyridazine-3-carboxamide |
| EI15-93-02R | EI15-93 | 2K93 | 6-[4-[[4-(3-Hydroxyphenyl)thiophen-2-yl]methyl]piperazin-1-yl]-N-(3,3,3-trifluoropropylsulfonyl)pyridazine-3- carboxamide |
| EI15-94-02R | EI15-94 | 2K94 | 6-[4-[[4-(5-Hydroxypyridin-3-yl)thiophen-2-yl]methyl]piperazin-1-yl]-N-(3,3,3-trifluoropropylsulfonyl)pyridazine-3-carboxamide |
| EI15-95-02R | EI15-95 | 2K95 | 6-[4-[[2-[2-(3-Hydroxyphenyl)ethynyl]phenyl]methyl]piperazin-1-yl]-N-(3,3,3-trifluoropropylsulfonyl)pyridazine-3-carboxamide |
| EI15-96-02R | EI15-96 | 2K96 | 6-[4-[[2-[2-(5-Hydroxypyridin-3-yl)ethynyl]phenyl]methyl]piperazin-1-yl]-N-(3,3,3-trifluoropropylsulfonyl)pyridazine-3-carboxamide |
| EI15-97-02R | EI15-97 | 2K97 | 4-[4-[1-[4-(3-Hydroxyphenyl)phenyl]ethyl]piperazin-1-yl]-N-(3,3,3-trifluoropropylsulfonyl)benzamide |
| EI15-98-02R | EI15-98 | 2K98 | 4-[4-[1-[4-(5-Hydroxypyridin-3-yl)phenyl]ethyl]piperazin-1- yl]-N-(3,3,3-trifluoropropylsulfonyl)benzamide |
| EI15-99-02R | EI15-99 | 2K99 | 4-[4-[1-[2-Fluoro-4-(3-hydroxyphenyl)phenyl]ethyl]piperazin-1-yl]-N-(3,3,3-trifluoropropylsulfonyl)benzamide |
| EI15-100-02R | EI15-100 | 2K100 | 4-[4-[1-[2-Fluoro-4-(5-hydroxypyridin-3-yl)phenyl]ethyl]piperazin-1-yl]-N-(3,3,3-trifluoropropylsulfonyl)benzamide |

[Table 68]

[Table EI16] mixEI16-02R, amine reagent used: BB40, post-cleavage mixture No.: mixEI16

| Resin compound No. | Post-cleavage compound No. | Another name of post-cleavage compound in Table EJ10 | Compound name |
|---|---|---|---|
| EI16-01-02R | EI16-01 | 2A01 | N-(1-Adamantylmethylsulfonyl)-6-[4-[4-(3-hydroxyphenyl)thiophene-2-carbonyl]piperazin-1-yl]pyridazine-3-carboxamide |
| EI16-02-02R | EI16-02 | 2A02 | N-(1-Adamantylmethylsulfonyl)-6-[4-[4-(5-hydroxypyridin-3-yl)thiophene-2-carbonyl]piperazin-1-yl]pyridazine-3-carboxamide |
| EI16-03-02R | EI16-03 | 2A03 | N-(1-Adamantylmethylsulfonyl)-6-[4-[4-(3-hydroxyphenyl)-3-methylbenzoyl]piperazin-1-yl]pyridazine-3-carboxamide |
| EI16-04-02R | EI16-04 | 2A04 | N-(1-Adamantylmethylsulfonyl)-6-[4-[4-(5-hydroxypyridin-3-yl)-3-methylbenzoyl]piperazin-1-yl]pyridazine-3-carboxamide |
| EI16-05-02R | EI16-05 | 2A05 | N-(1-Adamantylmethylsulfonyl)-6-[4-[3-fluoro-4-(3-hydroxyphenyl)benzoyl]piperazin-1-yl]pyridazine-3-carboxamide |
| EI16-06-02R | EI16-06 | 2A06 | N-(1-Adamantylmethylsulfonyl)-6-[4-[3-fluoro-4-(5-hydroxypyridin-3 -yl)benzoyl]piperazin-1-yl]pyridazine-3-carboxamide |
| EI16-07-02R | EI16-07 | 2A07 | N-(1-Adamantylmethylsulfonyl)-6-[4-[2-[2-(3-hydroxyphenyl)ethynyl]benzoyl]piperazin-1-yl]pyridazine-3-carboxamide |
| EI16-08-02R | EI16-08 | 2A08 | N-(1-Adamantylmethylsulfonyl)-6-[4-[2-[2-(5-hydroxypyridin-3-yl)ethynyl]benzoyl]piperazin-1-yl]pyridazine-3-carboxamide |
| EI16-09-02R | EI16-09 | 2A09 | N-(1-Adamantylmethylsulfonyl)-6-[4-[5-[2-(3-hydroxyphenyl)ethynyl]pyridine-3-carbonyl]piperazin-1-yl]pyridazine-3-carboxamide |
| EI16-10-02R | EI16-10 | 2A10 | N-(1-Adamantylmethylsulfonyl)-6-[4-[5-[2-(5-hydroxypyridin-3-yl)ethynyl]pyridine-3-carbonyl]piperazin-1- yl]pyridazine-3-carboxamide |
| EI16-11-02R | EI16-11 | 2A11 | N-(1-Adamantylmethylsulfonyl)-6-[4-[3-[2-(3-hydroxyphenyl)phenyl]propanoyl]piperazin-1-yl]pyridazine-3-carboxamide |
| EI16-12-02R | EI16-12 | 2A12 | N-(1-Adamantylmethylsulfonyl)-6-[4-[3-[2-(5-hydroxypyridin-3-yl)phenyl]propanoyl]piperazin-1-yl]pyridazine-3-carboxamide |
| EI16-13-02R | EI16-13 | 2A13 | N-(1-Adamantylmethylsulfonyl)-6-[4-[4-(3-hydroxyphenyl)naphthalene-1-carbonyl]piperazin-1-yl]pyridazine-3-carboxamide |

(continued)

| Resin compound No. | Post-cleavage compound No. | Another name of post-cleavage compound in Table EJ10 | Compound name |
|---|---|---|---|
| EI16-14-02R | EI16-14 | 2A14 | N-(1-Adamantylmethylsulfonyl)-6-[4-[4-(5-hydroxypyridin-3-yl)naphthalene-1-carbonyl]piperazin-1-yl]pyridazine-3-carboxamide |
| EI16-15-02R | EI16-15 | 2A15 | N-(1-Adamantylmethylsulfonyl)-6-[4-[3-tert-butyl-5-(3-hydroxyphenyl)benzoyl]piperazin-1-yl]pyridazine-3-carboxamide |
| EI16-16-02R | EI16-16 | 2A16 | N-(1-Adamantylmethylsulfonyl)-6-[4-[3-tert-butyl-5-(5-hydroxypyridin-3-yl)benzoyl]piperazin-1-yl]pyridazine-3-carboxamide |
| EI16-17-02R | EI16-17 | 2A17 | N-(1-Adamantylmethylsulfonyl)-6-[4-[3-(3-hydroxyphenyl)-5-(trifluoromethyl)benzoyl]piperazin-1-yl]pyridazine-3-carboxamide |
| EI16-18-02R | EI16-18 | 2A18 | N-(1-Adamantylmethylsulfonyl)-6-[4-[3-(5-hydroxypyridin-3-yl)-5-(trifluoromethyl)benzoyl]piperazin-1-yl]pyridazine-3- carboxamide |
| EI16-19-02R | EI16-19 | 2A19 | N-(1-Adamantylmethylsulfonyl)-6-[4-[3-[2-(3-hydroxyphenyl)ethynyl]-5-(trifluoromethyl)benzoyl]piperazin-1-yl]pyridazine-3-carboxamide |
| EI16-20-02R | EI16-20 | 2A20 | N-(1-Adamantylmethylsulfonyl)-6-[4-[3-[2-(5-hydroxypyridin-3-yl)ethynyl]-5-(trifluoromethyl)benzoyl]piperazin-1-yl]pyridazine-3-carboxamide |
| EI16-21-02R | EI16-21 | 2A21 | N-(1-Adamantylmethylsulfonyl)-6-[4-[3-[4-(3-hydroxyphenyl)pyrazol-1-yl]-5-(trifluoromethyl)benzoyl]piperazin-1-yl]pyridazine-3-carboxamide |
| EI16-22-02R | EI16-22 | 2A22 | N-(1-Adamantylmethylsulfonyl)-6-[4-[3-[4-(5-hydroxypyridin-3-yl)pyrazol-1-yl]-5-(trifluoromethyl)benzoyl]piperazin-1-yl]pyridazine-3- carboxamide |
| EI16-23-02R | EI16-23 | 2A23 | N-(1-Adamantylmethylsulfonyl)-4-[4-[4-(3-hydroxyphenyl)thiophene-2-carbonyl]piperazin-1-yl]benzamide |
| EI16-24-02R | EI16-24 | 2A24 | N-(1-Adamantylmethylsulfonyl)-4-[4-[4-(5-hydroxypyridin-3-yl)thiophene-2-carbonyl]piperazin-1-yl]benzamide |
| EI16-25-02R | EI16-25 | 2A25 | N-(1-Adamantylmethylsulfonyl)-4-[4-[4-(3-hydroxyphenyl)-3-methylbenzoyl]piperazin-1-yl]benzamide |
| EI16-26-02R | EI16-26 | 2A26 | N-(1-Adamantylmethylsulfonyl)-4-[4-[4-(5-hydroxypyridin-3-yl)-3-methylbenzoyl]piperazin-1-yl]benzamide |
| EI16-27-02R | EI16-27 | 2A27 | N-(1-Adamantylmethylsulfonyl)-4-[4-[3-fluoro-4-(3-hydroxyphenyl)benzoyl]piperazin-1-yl]benzamide |
| EI16-28-02R | EI16-28 | 2A28 | N-(1-Adamantylmethylsulfonyl)-4-[4-[3-fluoro-4-(5-hydroxypyridin-3-yl)benzoyl]piperazin-1-yl]benzamide |

(continued)

| Resin compound No. | Post-cleavage compound No. | Another name of post-cleavage compound in Table EJ10 | Compound name |
|---|---|---|---|
| EI16-29-02R | EI16-29 | 2A29 | N-(1-Adamantylmethylsulfonyl)-4-[4-[2-[2-(3-hydroxyphenyl)ethynyl]benzoyl]piperazin-1-yl]benzamide |
| EI16-30-02R | EI16-30 | 2A30 | N-(1-Adamantylmethylsulfonyl)-4-[4-[2-[2-(5-hydroxypyridin-3-yl)ethynyl]benzoyl]piperazin-1-yl]benzamide |
| EI16-31-02R | EI16-31 | 2A31 | N-(1-Adamantylmethylsulfonyl)-4-[4-[5-[2-(3-hydroxyphenyl)ethynyl]pyridine-3-carbonyl]piperazin-1-yl]benzamide |
| EI16-32-02R | EI16-32 | 2A32 | N-(1-Adamantylmethylsulfonyl)-4-[4-[5-[2-(5-hydroxypyridin-3-yl)ethynyl]pyridine-3-carbonyl]piperazin-1- yl]benzamide |
| EI16-33-02R | EI16-33 | 2A33 | N-(1-Adamantylmethylsulfonyl)-4-[4-[3-[2-(3-hydroxyphenyl)phenyl]propanoyl]piperazin-1-yl]benzamide |
| EI16-34-02R | EI16-34 | 2A34 | N-(1-Adamantylmethylsulfonyl)-4-[4-[3-[2-(5-hydroxypyridin-3-yl)phenyl]propanoyl]piperazin-1-yl]benzamide |
| EI16-35-02R | EI16-35 | 2A35 | N-(1-Adamantylmethylsulfonyl)-4-[4-[4-(3-hydroxyphenyl)naphthalene-1-carbonyl]piperazin-1-yl]benzamide |
| EI16-36-02R | EI16-36 | 2A36 | N-(1-Adamantylmethylsulfonyl)-4-[4-[4-(5-hydroxypyridin-3-yl)naphthalene-1-carbonyl]piperazin-1-yl]benzamide |
| EI16-37-02R | EI16-37 | 2A37 | N-(1-Adamantylmethylsulfonyl)-4-[4-[3-tert-butyl-5-(3-hydroxyphenyl)benzoyl]piperazin-1-yl]benzamide |
| EI16-38-02R | EI16-38 | 2A38 | N-(1-Adamantylmethylsulfonyl)-4-[4-[3-tert-butyl-5-(5-hydroxypyridin-3-yl)benzoyl]piperazin-1-yl]benzamide |
| EI16-39-02R | EI16-39 | 2A39 | N-(1-Adamantylmethylsulfonyl)-4-[4-[3-(3-hydroxyphenyl)-5-(trifluoromethyl)benzoyl]piperazin-1-yl]benzamide |
| EI16-40-02R | EI16-40 | 2A40 | N-(1-Adamantylmethylsulfonyl)-4-[4-[3-(5-hydroxypyridin-3-yl)-5-(trifluoromethyl)benzoyl]piperazin-1 -yl]benzamide |
| EI16-41-02R | EI16-41 | 2A41 | N-(1-Adamantylmethylsulfonyl)-4-[4-[3-[2-(3-hydroxyphenyl)ethynyl]-5-(trifluoromethyl)benzoyl]piperazin-1-yl]benzamide |
| EI16-42-02R | EI16-42 | 2A42 | N-(1-Adamantylmethylsulfonyl)-4-[4-[3-[2-(5-hydroxypyridin-3-yl)ethynyl]-5-(trifluoromethyl)benzoyl]piperazin-1-yl]benzamide |
| EI16-43-02R | EI16-43 | 2A43 | N-(1-Adamantylmethylsulfonyl)-4-[4-[3-[4-(3-hydroxyphenyl)pyrazol-1-yl]-5-(trifluoromethyl)benzoyl]piperazin-1-yl]benzamide |

(continued)

| Resin compound No. | Post-cleavage compound No. | Another name of post-cleavage compound in Table EJ10 | Compound name |
|---|---|---|---|
| EI16-44-02R | EI16-44 | 2A44 | N-(1-Adamantylmethylsulfonyl)-4-[4-[3-[4-(5-hydroxypyridin-3-yl)pyrazol-1-yl]-5-(trifluoromethyl)benzoyl]piperazin-1-yl]benzamide |
| EI16-45-02R | EI16-45 | 2A45 | N-(1-Adamantylmethylsulfonyl)-4-[4-[[2-(3-hydroxyphenyl)phenyl]methyl]piperazin-1-yl]benzamide |
| EI16-46-02R | EI16-46 | 2A46 | N-(1-Adamantylmethylsulfonyl)-4-[4-[[2-(5-hydroxypyridin-3-yl)phenyl]methyl]piperazin-1-yl]benzamide |
| EI16-47-02R | EI16-47 | 2A47 | N-(1-Adamantylmethylsulfonyl)-4-[4-[[4-(3-hydroxyphenyl)-3-methylphenyl]methyl]piperazin-1-yl]benzamide |
| EI16-48-02R | EI16-48 | 2A48 | N-(1-Adamantylmethylsulfonyl)-4-[4-[[4-(5-hydroxypyridin-3-yl)-3-methylphenyl]methyl]piperazin-1-yl]benzamide |
| EI16-49-02R | EI16-49 | 2A49 | N-(1-Adamantylmethylsulfonyl)-4-[4-[[2-ethyl-4-(3-hydroxyphenyl)phenyl]methyl]piperazin-1-yl]benzamide |
| EI16-50-02R | EI16-50 | 2A50 | N-(1-Adamantylmethylsulfonyl)-4-[4-[[2-ethyl-4-(5-hydroxypyridin-3-yl)phenyl]methyl]piperazin-1-yl]benzamide |
| EI16-51-02R | EI16-51 | 2A51 | N-(1-Adamantylmethylsulfonyl)-4-[4-[[3-fluoro-4-(3-hydroxyphenyl)phenyl]methyl]piperazin-1-yl]benzamide |
| EI16-52-02R | EI16-52 | 2A52 | N-(1-Adamantylmethylsulfonyl)-4-[4-[[3-fluoro-4-(5-hydroxypyridin-3-yl)phenyl]methyl]piperazin-1-yl]benzamide |
| EI16-53-02R | EI16-53 | 2A53 | N-(1-Adamantylmethylsulfonyl)-4-[4-[[4-(3-hydroxyphenyl)-2-methoxyphenyl]methyl]piperazin-1-yl]benzamide |
| EI16-54-02R | EI16-54 | 2A54 | N-(1-Adamantylmethylsulfonyl)-4-[4-[[4-(5-hydroxypyridin-3-yl)-2-methoxyphenyl]methyl]piperazin-1-yl]benzamide |
| EI16-55-02R | EI16-55 | 2A55 | N-(1-Adamantylmethylsulfonyl)-4-[4-[[3-ethoxy-5-(3-hydroxyphenyl)phenyl]methyl]piperazin-1-yl]benzamide |
| EI16-56-02R | EI16-56 | 2A56 | N-(1-Adamantylmethylsulfonyl)-4-[4-[[3-ethoxy-5-(5-hydroxypyridin-3-yl)phenyl]methyl]piperazin-1-yl]benzamide |
| EI16-57-02R | EI16-57 | 2A57 | N-(1-Adamantylmethylsulfonyl)-4-[4-[[3-(3-hydroxyphenyl)-5-propan-2-yloxyphenyl]methyl]piperazin-1-yl]benzamide |
| EI16-58-02R | EI16-58 | 2A58 | N-(1-Adamantylmethylsulfonyl)-4-[4-[[3-(5-hydroxypyridin-3-yl)-5-propan-2-yloxyphenyl]methyl]piperazin-1-yl]benzamide |

(continued)

| Resin compound No. | Post-cleavage compound No. | Another name of post-cleavage compound in Table EJ10 | Compound name |
|---|---|---|---|
| EI16-59-02R | EI16-59 | 2A59 | N-(1-Adamantylmethylsulfonyl)-4-[4-[[4-(3-hydroxyphenyl)-2,6-dimethoxyphenyl]methyl]piperazin-1-yl]benzamide |
| EI16-60-02R | EI16-60 | 2A60 | N-(1-Adamantylmethylsulfonyl)-4-[4-[[4-(5-hydroxypyndin-3-yl)-2,6-dimethoxyphenyl]methyl]piperazin-1-yl]benzamide |
| EI16-61-02R | EI16-61 | 2A61 | N-(1-Adamantylmethylsulfonyl)-4-[4-[[3-(3-hydroxyphenyl)-5-(trifluoromethyl)phenyl]methyl]piperazin-1-yl]benzamide |
| EI16-62-02R | EI16-62 | 2A62 | N-(1-Adamantylmethylsulfonyl)-4-[4-[[3-(5-hydroxypyridin-3-yl)-5-(trifluoromethyl)phenyl]methyl]piperazin-1-yl]benzamide |
| EI16-63-02R | EI16-63 | 2A63 | N-(1-Adamantylmethylsulfonyl)-4-[4-[[3-(3-hydroxyphenyl)-5-(trifluoromethoxy)phenyl]methyl]piperazin-1-yl]benzamide |
| EI16-64-02R | EI16-64 | 2A64 | N-(1-Adamantylmethylsulfonyl)-4-[4-[[3-(5-hydroxypyridin-3-yl)-5-(trifluoromethoxy)phenyl]methyl]piperazin-1- yl]benzamide |
| EI16-65-02R | EI16-65 | 2A65 | N-(1-Adamantylmethylsulfonyl)-4-[4-[[4-(3-hydroxyphenyl)naphthalen-1-yl]methyl]piperazin-1-yl]benzamide |
| EI16-66-02R | EI16-66 | 2A66 | N-(1-Adamantylmethylsulfonyl)-4-[4-[[4-(5-hydroxypyridin-3-yl)naphthalen-1-yl]methyl]piperazin-1-yl]benzamide |
| EI16-67-02R | EI16-67 | 2A67 | N-(1-Adamantylmethylsulfonyl)-4-[4-[[4-(3-hydroxyphenyl)thiophen-2-yl]methyl]piperazin-1-yl]benzamide |
| EI16-68-02R | EI16-68 | 2A68 | N-(1-Adamantylmethylsulfonyl)-4-[4-[[4-(5-hydroxypyridin-3-yl)thiophen-2-yl]methyl]piperazin-1-yl]benzamide |
| EI16-69-02R | EI16-69 | 2A69 | N-(1-Adamantylmethylsulfonyl)-4-[4-[[2-[2-(3-hydroxyphenyl)ethynyl]phenyl]methyl]piperazin-1-yl]benzamide |
| EI16-70-02R | EI16-70 | 2A70 | N-(1-Adamantylmethylsulfonyl)-4-[4-[[2-[2-(5-hydroxypyridin-3-yl)ethynyl]phenyl]methyl]piperazin-1-yl]benzamide |
| EI16-71-02R | EI16-71 | 2A71 | N-(1-Adamantylmethylsulfonyl)-6-[4-[[2-(3-hydroxyphenyl)phenyl]methyl]piperazin-1-yl]pyridazine-3-carboxamide |
| EI16-72-02R | EI16-72 | 2A72 | N-(1-Adamantylmethylsulfonyl)-6-[4-[[2-(5-hydroxypyridin-3-yl)phenyl]methyl]piperazin-1-yl]pyridazine-3-carboxamide |

(continued)

| Resin compound No. | Post-cleavage compound No. | Another name of post-cleavage compound in Table EJ10 | Compound name |
|---|---|---|---|
| EI16-73-02R | EI16-73 | 2A73 | N-(1-Adamantylmethylsulfonyl)-6-[4-[[4-(3-hydroxyphenyl)-3-methylphenyl]methyl]piperazin-1-yl]pyridazine-3-carboxamide |
| EI16-74-02R | EI16-74 | 2A74 | N-(1-Adamantylmethylsulfonyl)-6-[4-[[4-(5-hydroxypyridin-3-yl)-3-methylphenyl]methyl]piperazin-1-yl]pyridazine-3 - carboxamide |
| EI16-75-02R | EI16-75 | 2A75 | N-(1-Adamantylmethylsulfonyl)-6-[4-[[2-ethyl-4-(3-hydroxyphenyl)phenyl]methyl]piperazin-1-yl]pyridazine-3- carboxamide |
| EI16-76-02R | EI16-76 | 2A76 | N-(1-Adamantylmethylsulfonyl)-6-[4-[[2-ethyl-4-(5-hydroxypyridin-3-yl)phenyl]methyl]piperazin-1-yl]pyridazine-3-carboxamide |
| EI16-77-02R | EI16-77 | 2A77 | N-(1-Adamantylmethylsulfonyl)-6-[4-[[3-fluoro-4-(3- hydroxyphenyl)phenyl]methyl]piperazin-1-yl]pyridazine-3-carboxamide |
| EI16-78-02R | EI16-78 | 2A78 | N-(1-Adamantylmethylsulfonyl)-6-[4-[[3-fluoro-4-(5-hydroxypyridin-3-yl)phenyl]methyl]piperazin-1-yl]pyridazine-3-carboxamide |
| EI16-79-02R | EI16-79 | 2A79 | N-(1-Adamantylmethylsulfonyl)-6-[4-[[4-(3-hydroxyphenyl)-2-methoxyphenyl]methyl]piperazin-1-yl]pyridazine-3- carboxamide |
| EI16-80-02R | EI16-80 | 2A80 | N-(1-Adamantylmethylsulfonyl)-6-[4-[[4-(5-hydroxypyridin-3-yl)-2-methoxyphenyl]methyl]piperazin-1-yl]pyridazine-3- carboxamide |
| EI16-81-02R | EI16-81 | 2A81 | N-(1-Adamantylmethylsulfonyl)-6-[4-[[3-ethoxy-5-(3-hydroxyphenyl)phenyl]methyl]piperazin-1-yl]pyridazine-3- carboxamide |
| EI16-82-02R | EI16-82 | 2A82 | N-(1-Adamantylmethylsulfonyl)-6-[4-[[3-ethoxy-5-(5-hydroxypyridin-3 -yl)phenyl]methyl]piperazin-1-yl]pyridazine-3-carboxamide |
| EI16-83-02R | EI16-83 | 2A83 | N-(1-Adamantylmethylsulfonyl)-6-[4-[[3-(3-hydroxyphenyl)-5-propan-2-yloxyphenyl]methyl]piperazin-1-yl]pyridazine-3-carboxamide |
| EI16-84-02R | EI16-84 | 2A84 | N-(1-Adamantylmethylsulfonyl)-6-[4-[[3-(5-hydroxypyridin-3-yl)-5-propan-2-yloxyphenyl]methyl]piperazin-1-yl]pyridazine-3-carboxamide |
| EI16-85-02R | EI16-85 | 2A85 | N-(1-Adamantylmethylsulfonyl)-6-[4-[[4-(3-hydroxyphenyl)-2,6-dimethoxyphenyl]methyl]piperazin-1-yl]pyridazine-3- carboxamide |
| EI16-86-02R | EI16-86 | 2A86 | N-(1-Adamantylmethylsulfonyl)-6-[4-[[4-(5-hydroxypyridin-3-yl)-2,6-dimethoxyphenyl]methyl]piperazin-1-yl]pyridazine-3-carboxamide |

(continued)

| Resin compound No. | Post-cleavage compound No. | Another name of post-cleavage compound in Table EJ10 | Compound name |
|---|---|---|---|
| EI16-87-02R | EI16-87 | 2A87 | N-(1-Adamantylmethylsulfonyl)-6-[4-[[3-(3-hydroxyphenyl)-5-(trifluoromethyl)phenyl]methyl]piperazin-1-yl]pyridazine-3- carboxamide |
| EI16-88-02R | EI16-88 | 2A88 | N-(1-Adamantylmethylsulfonyl)-6-[4-[[3-(5-hydroxypyridin-3-yl)-5-(trifluoromethyl)phenyl]methyl]piperazin-1-yl]pyridazine-3-carboxamide |
| EI16-89-02R | EI16-89 | 2A89 | N-(1-Adamantylmethylsulfonyl)-6-[4-[[3-(3-hydroxyphenyl)-5-(trifluoromethoxy)phenyl]methyl]piperazin-1-yl]pyridazine-3-carboxamide |
| EI16-90-02R | EI16-90 | 2A90 | N-(1-Adamantylmethylsulfonyl)-6-[4-[[3-(5-hydroxypyridin-3-yl)-5-(trifluoromethoxy)phenyl]methyl]piperazin-1-yl]pyridazine-3-carboxamide |
| EI16-91-02R | EI16-91 | 2A91 | N-(1-Adamantylmethylsulfonyl)-6-[4-[[4-(3-hydroxyphenyl)naphthalen-1-yl]methyl]piperazin-1-yl]pyridazine-3-carboxamide |
| EI16-92-02R | EI16-92 | 2A92 | N-(1-Adamantylmethylsulfonyl)-6-[4-[[4-(5-hydroxypyridin-3-yl)naphthalen-1-yl]methyl]piperazin-1-yl]pyridazine-3-carboxamide |
| EI16-93-02R | EI16-93 | 2A93 | N-(1-Adamantylmethylsulfonyl)-6-[4-[[4-(3-hydroxyphenyl)thiophen-2-yl]methyl]piperazin-1-yl]pyridazine-3-carboxamide |
| EI16-94-02R | EI16-94 | 2A94 | N-(1-Adamantylmethylsulfonyl)-6-[4-[[4-(5-hydroxypyridin-3-yl)thiophen-2-yl]methyl]piperazin-1-yl]pyridazine-3-carboxamide |
| EI16-95-02R | EI16-95 | 2A95 | N-(1-Adamantylmethylsulfonyl)-6-[4-[[2-[2-(3-hydroxyphenyl)ethynyl]phenyl]methyl]piperazin-1-yl]pyridazine-3-carboxamide |
| EI16-96-02R | EI16-96 | 2A96 | N-(1-Adamantylmethylsulfonyl)-6-[4-[[2-[2-(5-hydroxypyridin-3-yl)ethynyl]phenyl]methyl]piperazin-1-yl]pyridazine-3-carboxamide |
| EI16-97-02R | EI16-97 | 2A97 | N-(1-Adamantylmethylsulfonyl)-4-[4-[1-[4-(3-hydroxyphenyl)phenyl]ethyl]piperazin-1-yl]benzamide |
| EI16-98-02R | EI16-98 | 2A98 | N-(1-Adamantylmethylsulfonyl)-4-[4-[1-[4-(5-hydroxypyridin-3-yl)phenyl]ethyl]piperazin-1-yl]benzamide |
| EI16-99-02R | EI16-99 | 2A99 | N-(1-Adamantylmethylsulfonyl)-4-[4-[1-[2-fluoro-4-(3-hydroxyphenyl)phenyl]ethyl]piperazin-1-yl]benzamide |
| EI16-100-02R | EI16-100 | 2A100 | N-(1-Adamantylmethylsulfonyl)-4-[4-[1-[2-fluoro-4-(5-hydroxypyridin-3 -yl)phenyl] ethyl]piperazin-1-yl]benzamide |

[Table 69]

Table EI17] mixEI17-02R, amine reagent used: BB41, post-cleavage mixture No.: mixEI17

| Resin compound No. | Post-cleavage compound No. | Another name of post-cleavage compound in Table EJ11 | Compound name |
|---|---|---|---|
| EI17-01-02R | EI17-01 | 2Q01 | 6-[4-[4-(3-Hydroxyphenyl)thiophene-2-carbonyl]piperazin-1-yl]-N-[3-nitro-4-(2-phenylsulfanylethylamino)phenyl] sulfonylpyridazine-3-carboxamide |
| EI17-02-02R | EI17-02 | 2Q02 | 6-[4-[4-(5-Hydroxypyridin-3-yl)thiophene-2-carbonyl]piperazin-1-yl]-N-[3-nitro-4-(2-phenylsulfanylethylamino)phenyl] sulfonylpyridazine-3-carboxamide |
| EI17-03-02R | EI17-03 | 2Q03 | 6-[4-[4-(3-Hydroxyphenyl)-3-methylbenzoyl]piperazin-1-yl]-N-[3-nitro-4-(2-phenylsulfanylethylamino)phenyl]sulfonylpyridazine-3-carboxamide |
| EI17-04-02R | EI17-04 | 2Q04 | 6-[4-[4-(5-Hydroxypyridin-3-yl)-3-methylbenzoyl]piperazin-1-yl]-N-[3-nitro-4-(2-phenylsulfanylethylamino)phenyl]sulfonylpyridazine-3-carboxamide |
| EI17-05-02R | EI17-05 | 2Q05 | 6-[4-[3-Fluoro-4-(3-hydroxyphenyl)benzoyl]piperazin-1-yl]-N-[3-nitro-4-(2-phenylsulfanylethylamino)phenyl]sulfonylpyridazine-3-carboxamide |
| EI17-06-02R | EI17-06 | 2Q06 | 6-[4-[3-Fluoro-4-(5-hydroxypyridin-3-yl)benzoyl]piperazin-1- yl]-N-[3-nitro-4-(2-phenylsulfanylethylamino)phenyl] sulfonylpyridazine-3-carboxamide |
| EI17-07-02R | EI17-07 | 2Q07 | 6-[4-[2-[2-(3-Hydroxyphenyl)ethynyl]benzoyl]piperazin-1-yl]-N-[3-nitro-4-(2-phenylsulfanylethylamino)phenyl] sulfonylpyridazine-3-carboxamide |
| EI17-08-02R | EI17-08 | 2Q08 | 6-[4-[2-[2-(5-Hydroxypyridin-3-yl)ethynyl]benzoyl]piperazin-1-yl]-N-[3-nitro-4-(2-phenylsulfanylethylamino)phenyl] sulfonylpyridazine-3-carboxamide |
| EI17-09-02R | EI17-09 | 2Q09 | 6-[4-[5-[2-(3-Hydroxyphenyl)ethynyl]pyridine-3-carbonyl]piperazin-1-yl]-N-[3-nitro-4-(2-phenylsulfanylethylamino)phenyl]sulfonylpyridazine-3-carboxamide |
| EI17-10-02R | EI17-10 | 2Q10 | 6-[4-[5-[2-(5-Hydroxypyridin-3-yl)ethynyl]pyridine-3-carbonyl]piperazin-1-yl]-N-[3-nitro-4-(2-phenylsulfanylethylamino)phenyl] sulfonylpyridazine-3-carboxamide |
| EI17-11-02R | EI17-11 | 2Q11 | 6-[4-[3-[2-(3-Hydroxyphenyl)phenyl]propanoyl]piperazin-1- yl]-N-[3-nitro-4-(2-phenylsulfanylethylamino)phenyl] sulfonylpyridazine-3-carboxamide |

(continued)

| Resin compound No. | Post-cleavage compound No. | Another name of post-cleavage compound in Table EJ11 | Compound name |
|---|---|---|---|
| EI17-12-02R | EI17-12 | 2Q12 | 6-[4-[3-[2-(5-Hydroxypyridin-3-yl)phenyl]propanoyl]piperazin-1-yl]-N-[3-nitro-4-(2-phenylsulfanylethylamino)phenyl]sulfonylpyridazine-3-carboxamide |
| EI17-13-02R | EI17-13 | 2Q13 | 6-[4-[4-(3-Hydroxyphenyl)naphthalene-1-carbonyl]piperazin-1-yl]-N-[3-nitro-4-(2-phenylsulfanylethylamino)phenyl] sulfonylpyridazine-3-carboxamide |
| EI17-14-02R | EI17-14 | 2Q14 | 6-[4-[4-(5-Hydroxypyridin-3-yl)naphthalene-1-carbonyl]piperazin-1-yl]-N-[3-nitro-4-(2-phenylsulfanylethylamino)phenyl]sulfonylpyridazine-3-carboxamide |
| EI17-15-02R | EI17-15 | 2Q15 | 6-[4-[3-tert-Butyl-5-(3-hydroxyphenyl)benzoyl]piperazin-1-yl]-N-[3-nitro-4-(2-phenylsulfanylethylamino)phenyl]sulfonylpyridazine-3-carboxamide |
| EI17-16-02R | EI17-16 | 2Q16 | 6-[4-[3-tert-Butyl-5-(5-hydroxypyridin-3-yl)benzoyl]piperazin-1-yl]-N-[3-nitro-4-(2-phenylsulfanylethylamino)phenyl] sulfonylpyridazine-3-carboxamide |
| EI17-17-02R | EI17-17 | 2Q17 | 6-[4-[3-(3-Hydroxyphenyl)-5-(trifluoromethyl)benzoyl]piperazin-1-yl]-N-[3-nitro-4-(2-phenylsulfanylethylamino)phenyl]sulfonylpyridazine-3-carboxamide |
| EI17-18-02R | EI17-18 | 2Q18 | 6-[4-[3-(5-Hydroxypyridin-3-yl)-5-(trifluoromethyl)benzoyl]piperazin-1-yl]-N-[3-nitro-4-(2-phenylsulfanylethylamino)phenyl]sulfonylpyridazine-3-carboxamide |
| EI17-19-02R | EI17-19 | 2Q19 | 6-[4-[3-[2-(3-Hydroxyphenyl)ethynyl]-5-(trifluoromethyl)benzoyl]piperazin-1-yl]-N-[3-nitro-4-(2-phenylsulfanylethylamino)phenyl] sulfonylpyridazine-3-carboxamide |
| EI17-20-02R | EI17-20 | 2Q20 | 6-[4-[3-[2-(5-Hydroxypyridin-3-yl)ethynyl]-5-(trifluoromethyl)benzoyl]piperazin-1-yl]-N-[3-nitro-4-(2-phenylsulfanylethylamino)phenyl]sulfonylpyridazine-3-carboxamide |
| EI17-21-02R | EI17-21 | 2Q21 | 6-[4-[3-[4-(3-Hydroxyphenyl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| EI17-22-02R | EI17-22 | 2Q22 | 6-[4-[3-[4-(5-Hydroxypyridin-3-yl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |

(continued)

| Resin compound No. | Post-cleavage compound No. | Another name of post-cleavage compound in Table EJ11 | Compound name |
|---|---|---|---|
| EI17-23-02R | EI17-23 | 2Q23 | 4-[4-[4-(3-Hydroxyphenyl)thiophene-2-carbonyl]piperazin-1-yl]-N-[3-nitro-4-(2-phenylsulfanylethylamino)phenyl]sulfonylbenzamide |
| EI17-24-02R | EI17-24 | 2Q24 | 4-[4-[4-(5-Hydroxypyridin-3-yl)thiophene-2-carbonyl]piperazin-1-yl]-N-[3-nitro-4-(2-phenylsulfanylethylamino)phenyl]sulfonylbenzamide |
| EI17-25-02R | EI17-25 | 2Q25 | 4-[4-[4-(3-Hydroxyphenyl)-3-methylbenzoyl]piperazin-1-yl]-N-[3-nitro-4-(2-phenylsulfanylethylamino)phenyl]sulfonylbenzamide |
| EI17-26-02R | EI17-26 | 2Q26 | 4-[4-[4-(5-Hydroxypyridin-3-yl)-3-methylbenzoyl]piperazin-1-yl]-N-[3-nitro-4-(2-phenylsulfanylethylamino)phenyl]sulfonylbenzamide |
| EI17-27-02R | EI17-27 | 2Q27 | [4-[3-Fluoro-4-3-hydroxyphenyl)benzoyl]piperazin-1-yl]-N-[3-nitro-4-(2-phenylsulfanylethylamino)phenyl]sulfonylbenzamide |
| EI17-28-02R | EI17-28 | 2Q28 | 4-[4-[3-Fluoro-4-(5-hydroxypyridin-3-yl)benzoyl]piperazin-1- yl]-N-[3-nitro-4-(2-phenylsulfanylethylamino)phenyl]sulfonylbenzamide |
| EI17-29-02R | EI17-29 | 2Q29 | 4-[4-[2-[2-(3-Hydroxyphenyl)ethynyl]benzoyl]piperazin-1-yl]-N-[3-nitro-4-(2-phenylsulfanylethylamino)phenyl]sulfonylbenzamide |
| EI17-30-02R | EI17-30 | 2Q30 | 4-[4-[2-[2-(5-Hydroxypyridin-3-yl)ethynyl]benzoyl]piperazin-1-yl]-N-[3-nitro-4-(2-phenylsulfanylethylamino)phenyl]sulfonylbenzamide |
| EI17-31-02R | EI17-31 | 2Q31 | 4-[4-[5-[2-(3-Hydroxyphenyl)ethynyl]pyridine-3-carbonyl]piperazin-1-yl]-N-[3-nitro-4-(2-phenylsulfanylethylamino)phenyl]sulfonylbenzamide |
| EI17-32-02R | EI17-32 | 2Q32 | 4-[4-[5-[2-(5-Hydroxypyridin-3-yl)ethynyl]pyridine-3-carbonyl]piperazin-1-yl]-N-[3-nitro-4-(2-phenylsulfanylethylamino)phenyl]sulfonylbenzamide |
| EI17-33-02R | EI17-33 | 2Q33 | 4-[4-[3-[2-(3-Hydroxyphenyl)phenyl]propanoyl]piperazin-1- yl]-N-[3-nitro-4-(2-phenylsulfanylethylamino)phenyl]sulfonylbenzamide |
| EI17-34-02R | EI17-34 | 2Q34 | 4-[4-[3-[2-(5-Hydroxypyridin-3-yl)phenyl]propanoyl]piperazin-1-yl]-N-[3-nitro-4-(2-phenylsulfanylethylamino)phenyl]sulfonylbenzamide |
| EI17-35-02R | EI17-35 | 2Q35 | 4-[4-[4-(3-Hydroxyphenyl)naphthalene-1-carbonyl]piperazin-1-yl]-N-[3-nitro-4-(2-phenylsulfanylethylamino)phenyl]sulfonylbenzamide |
| EI17-36-02R | EI17-36 | 2Q36 | 4-[4-[4-(5-Hydroxypyridin-3-yl)naphthalene-1-carbonyl]piperazin-1-yl]-N-[3-nitro-4-(2-phenylsulfanylethylamino)phenyl]sulfonylbenzamide |
| EI17-37-02R | EI17-37 | 2Q37 | 4-[4-[3-tert-Butyl-5-(3-hydroxyphenyl)benzoyl]piperazin-1-yl]-N-[3-nitro-4-(2-phenylsulfanylethylamino)phenyl]sulfonylbenzamide |

(continued)

| Resin compound No. | Post-cleavage compound No. | Another name of post-cleavage compound in Table EJ11 | Compound name |
|---|---|---|---|
| EI17-38-02R | EI17-38 | 2Q38 | 4-[4-[3-tert-Butyl-5-(5-hydroxypyridin-3-yl)benzoyl]piperazin-1-yl]-N-[3-nitro-4-(2-phenylsulfanylethylamino)phenyl]sulfonylbenzamide |
| EI17-39-02R | EI17-39 | 2Q39 | 4-[4-[3-(3-Hydroxyphenyl)-5-(trifluoromethyl)benzoyl]piperazin-1-yl]-N-[3-nitro-4-(2-phenylsulfanylethylamino)phenyl]sulfonylbenzamide |
| EI17-40-02R | EI17-40 | 2Q40 | 4-[4-[3-(5-Hydroxypyridin-3-yl)-5-(trifluoromethyl)benzoyl]piperazin-1-yl]-N-[3-nitro-4-(2-phenylsulfanylethylamino)phenyl]sulfonylbenzamide |
| EI17-41-02R | EI17-41 | 2Q41 | 4-[4-[3-[2-(3-Hydroxyphenyl)ethynyl]-5-(trifluoromethyl)benzoyl]piperazin-1-yl]-N-[3-nitro-4-(2-phenylsulfanylethylamino)phenyl]sulfonylbenzamide |
| EI17-42-02R | EI17-42 | 2Q42 | 4-[4-[3-[2-(5-Hydroxypyridin-3-yl)ethynyl]-5-(trifluoromethyl)benzoyl]piperazin-1-yl]-N-[3-nitro-4-(2-phenylsulfanylethylamino)phenyl]sulfonylbenzamide |
| EI17-43-02R | EI17-43 | 2Q43 | 4-[4-[3-[4-(3-Hydroxyphenyl)pyrazol-1-yl]-5-(trifluoromethyl)benzoyl]piperazin-1-yl]-N-[3-nitro-4-(2-phenylsulfanylethylamino)phenyl]sulfonylbenzamide |
| EI17-44-02R | EI17-44 | 2Q44 | 4-[4-[3-[4-(5-Hydroxypyridin-3-yl)pyrazol-1-yl]-5-(trifluoromethyl)benzoyl]piperazin-1-yl]-N-[3-nitro-4-(2-phenylsulfanylethylamino)phenyl]sulfonylbenzamide |
| EI17-45-02R | EI17-45 | 2Q45 | 4-[4-[[2-(3-Hydroxyphenyl)phenyl]methyl]piperazin-1-yl]-N-[3-nitro-4-(2-phenylsulfanylethylamino)phenyl]sulfonylbenzamide |
| EI17-46-02R | EI17-46 | 2Q46 | 4-[4-[[2-(5-Hydroxypyridin-3-yl)phenyl]methyl]piperazin-1-yl]-N-[3-nitro-4-(2-phenylsulfanylethylamino)phenyl]sulfonylbenzamide |
| EI17-47-02R | EI17-47 | 2Q47 | 4-[4-[[4-(3-Hydroxyphenyl)-3-methylphenyl]methyl]piperazin-1-yl]-N-[3-nitro-4-(2-phenylsulfanylethylamino)phenyl]sulfonylbenzamide |
| EI17-48-02R | EI17-48 | 2Q48 | 4-[4-[[4-(5-Hydroxypyridin-3-yl)-3-methylphenyl]methyl]piperazin-1-yl]-N-[3-nitro-4-(2-phenylsulfanylethylamino)phenyl]sulfonylbenzamide |
| EI17-49-02R | EI17-49 | 2Q49 | 4-[4-[[2-Ethyl-4-(3-hydroxyphenyl)phenyl]methyl]piperazin-1-yl]-N-[3-nitro-4-(2-phenylsulfanylethylamino)phenyl]sulfonylbenzamide |
| EI17-50-02R | EI17-50 | 2Q50 | 4-[4-[[2-Ethyl-4-(5-hydroxypyridin-3-yl)phenyl]methyl]piperazin-1-yl]-N-[3-nitro-4-(2-phenylsulfanylethylamino)phenyl]sulfonylbenzamide |
| EI17-51-02R | EI17-51 | 2Q51 | 4-[4-[[3-Fluoro-4-(3-hydroxyphenyl)phenyl]methyl]piperazin-1-yl]-N-[3-nitro-4-(2-phenylsulfanylethylamino)phenyl]sulfonylbenzamide |
| EI17-52-02R | EI17-52 | 2Q52 | 4-[4-[[3-Fluoro-4-(5-hydroxypyridin-3-yl)phenyl]methyl]piperazin-1-yl]-N-[3-nitro-4-(2-phenylsulfanylethylamino)phenyl]sulfonylbenzamide |

(continued)

| Resin compound No. | Post-cleavage compound No. | Another name of post-cleavage compound in Table EJ11 | Compound name |
|---|---|---|---|
| EI17-53-02R | EI17-53 | 2Q53 | 4-[4-[[4-(3-Hydroxyphenyl)-2-methoxyphenyl]methyl]piperazin-1-yl]-N-[3-nitro-4-(2-phenylsulfanylethylamino)phenyl]sulfonylbenzamide |
| EI17-54-02R | EI17-54 | 2Q54 | 4-[4-[[4-(5-Hydroxypyridin-3-yl)-2-methoxyphenyl]methyl]piperazin-1-yl]-N-[3-nitro-4-(2-phenylsulfanylethylamino)phenyl]sulfonylbenzamide |
| EI17-55-02R | EI17-55 | 2Q55 | 4-[4-[[3-Ethoxy-5-(3-hydroxyphenyl)phenyl]methyl]piperazin-1-yl]-N-[3-nitro-4-(2-phenylsulfanylethylamino)phenyl]sulfonylbenzamide |
| EI17-56-02R | EI17-56 | 2Q56 | 4-[4-[[3-Ethoxy-5-(5-hydroxypyridin-3-yl)phenyl]methyl]piperazin-1-yl]-N-[3-nitro-4-(2-phenylsulfanylethylamino)phenyl]sulfonylbenzamide |
| EI17-57-02R | EI17-57 | 2Q57 | 4-[4-[[3-(3-Hydroxyphenyl)-5-propan-2-yloxyphenyl]methyl]piperazin-1-yl]-N-[3-nitro-4-(2-phenylsulfanylethylamino)phenyl]sulfonylbenzamide |
| EI17-58-02R | EI17-58 | 2Q58 | 4-[4-[[3-(5-Hydroxypyridin-3-yl)-5-propan-2-yloxyphenyl]methyl]piperazin-1-yl]-N-[3-nitro-4-(2-phenylsulfanylethylamino)phenyl]sulfonylbenzamide |
| EI17-59-02R | EI17-59 | 2Q59 | 4-[4-[[4-(3-Hydroxyphenyl)-2,6-dimethoxyphenyl]methyl]piperazin-1-yl]-N-[3-nitro-4-(2-phenylsulfanylethylamino)phenyl]sulfonylbenzamide |
| EI17-60-02R | EI17-60 | 2Q60 | 4-[4-[[4-(5-Hydroxypyridin-3-yl)-2,6-dimethoxyphenyl]methyl]piperazin-1-yl]-N-[3-nitro-4-(2-phenylsulfanylethylamino)phenyl]sulfonylbenzamide |
| EI17-61-02R | EI17-61 | 2Q61 | 4-[4-[[3-(3-Hydroxyphenyl)-5-(trifluoromethyl)phenyl]methyl]piperazin-1-yl]-N-[3-nitro-4-(2-phenylsulfanylethylamino)phenyl]sulfonylbenzamide |
| EI17-62-02R | EI17-62 | 2Q62 | 4-[4-[[3-(5-Hydroxypyridin-3-yl)-5-(trifluoromethyl)phenyl]methyl]piperazin-1-yl]-N-[3-nitro-4-(2-phenylsulfanylethylamino)phenyl]sulfonylbenzamide |
| EI17-63-02R | EI17-63 | 2Q63 | 4-[4-[[3-(3-Hydroxyphenyl)-5-(trifluoromethoxy)phenyl]methyl]piperazin-1-yl]-N-[3-nitro-4-(2-phenylsulfanylethylamino)phenyl]sulfonylbenzamide |
| EI17-64-02R | EI17-64 | 2Q64 | 4-[4-[[3-(5-Hydroxypyridin-3-yl)-5-(trifluoromethoxy)phenyl]methyl]piperazin-1-yl]-N-[3-nitro-4-(2-phenylsulfanylethylamino)phenyl]sulfonylbenzamide |
| EI17-65-02R | EI17-65 | 2Q65 | 4-[4-[[4-(3-Hydroxyphenyl)naphthalen-1-yl]methyl]piperazin-1-yl]-N-[3-nitro-4-(2-phenylsulfanylethylamino)phenyl]sulfonylbenzamide |
| EI17-66-02R | EI17-66 | 2Q66 | 4-[4-[[4-(5-Hydroxypyridin-3-yl)naphthalen-1-yl]methyl]piperazin-1-yl]-N-[3-nitro-4-(2-phenylsulfanylethylamino)phenyl]sulfonylbenzamide |

(continued)

| Resin compound No. | Post-cleavage compound No. | Another name of post-cleavage compound in Table EJ11 | Compound name |
|---|---|---|---|
| EI17-67-02R | EI17-67 | 2Q67 | 4-[4-[[4-(3-Hydroxyphenyl)thiophen-2-yl]methyl]piperazin-1-yl]-N-[3-nitro-4-(2-phenylsulfanylethylamino)phenyl]sulfonylbenzamide |
| EI17-68-02R | EI17-68 | 2Q68 | 4-[4-[[4-(5-Hydroxypyridin-3-yl)thiophen-2-yl]methyl]piperazin-1-yl]-N-[3-nitro-4-(2-phenylsulfanylethylamino)phenyl]sulfonylbenzamide |
| EI17-69-02R | EI17-69 | 2Q69 | 4-[4-[[2-[2-(3-Hydroxyphenyl)ethynyl]phenyl]methyl]piperazin-1-yl]-N-[3-nitro-4-(2-phenylsulfanylethylamino)phenyl]sulfonylbenzamide |
| EI17-70-02R | EI17-70 | 2Q70 | 4-[4-[[2-[2-(5-Hydroxypyridin-3-yl)ethynyl]phenyl]methyl]piperazin-1-yl]-N-[3-nitro-4-(2-phenylsulfanylethylamino)phenyl]sulfonylbenzamide |
| EI17-71-02R | EI17-71 | 2Q71 | 6-[4-[[2-(3-Hydroxyphenyl)phenyl]methyl]piperazin-1-yl]-N-[3-nitro-4-(2-phenylsulfanylethylamino)phenyl] sulfonylpyridazine-3-carboxamide |
| EI17-72-02R | EI17-72 | 2Q72 | 6-[4-[[2-(5-Hydroxypyridin-3-yl)phenyl]methyl]piperazin-1-yl]-N-[3-nitro-4-(2-phenylsulfanylethylamino)phenyl] sulfonylpyridazine-3-carboxamide |
| EI17-73-02R | EI17-73 | 2Q73 | 6-[4-[[4-(3-Hydroxyphenyl)-3-methylphenyl]methyl]piperazin-1-yl]-N-[3-nitro-4-(2-phenylsulfanylethylamino)phenyl]sulfonylpyridazine-3-carboxamide |
| EI17-74-02R | EI17-74 | 2Q74 | 6-[4-[[4-(5-Hydroxypyridin-3-yl)-3-methylphenyl]methyl]piperazin-1-yl]-N-[3-nitro-4-(2-phenylsulfanylethylamino)phenyl]sulfonylpyridazine-3-carboxamide |
| EI17-75-02R | EI17-75 | 2Q75 | 6-[4-[[2-Ethyl-4-(3-hydroxyphenyl)phenyl]methyl]piperazin-1-yl]-N-[3-nitro-4-(2-phenylsulfanylethylamino)phenyl]sulfonylpyridazine-3-carboxamide |
| EI17-76-02R | EI17-76 | 2Q76 | 6-[4-[[2-Ethyl-4-(5-hydroxypyridin-3-yl)phenyl]methyl]piperazin-1-yl]-N-[3-nitro-4-(2-phenylsulfanylethylamino)phenyl] sulfonylpyridazine-3-carboxamide |
| EI17-77-02R | EI17-77 | 2Q77 | 6-[4-[[3-Fluoro-4-(3-hydroxyphenyl)phenyl]methyl]piperazin-1-yl]-N-[3-nitro-4-(2-phenylsulfanylethylamino)phenyl] sulfonylpyridazine-3-carboxamide |
| EI17-78-02R | EI17-78 | 2Q78 | 6-[4-[[3-Fluoro-4-(5-hydroxypyridin-3-yl)phenyl]methyl]piperazin-1-yl]-N-[3-nitro-4-(2-phenylsulfanylethylamino)phenyl] sulfonylpyridazine-3-carboxamide |

(continued)

| Resin compound No. | Post-cleavage compound No. | Another name of post-cleavage compound in Table EJ11 | Compound name |
|---|---|---|---|
| EI17-79-02R | EI17-79 | 2Q79 | 6-[4-[[4-(3-Hydroxyphenyl)-2-methoxyphenyl]methyl]piperazin-1-yl]-N-[3-nitro-4-(2-phenylsulfanylethylamino)phenyl] sulfonylpyridazine-3-carboxamide |
| EI17-80-02R | EI17-80 | 2Q80 | 6-[4-[[4-(5-Hydroxypyridin-3-yl)-2-methoxyphenyl]methyl]piperazin-1-yl]-N-[3-nitro-4-(2-phenylsulfanylethylamino)phenyl] sulfonylpyridazine-3-carboxamide |
| EI17-81-02R | EI17-81 | 2Q81 | 6-[4-[[3-Ethoxy-5-(3-hydroxyphenyl)phenyl]methyl]piperazin-1-yl]-N-[3-nitro-4-(2-phenylsulfanylethylamino)phenyl]sulfonylpyridazine-3- carboxamide |
| EI17-82-02R | EI17-82 | 2Q82 | 6-[4-[[3-Ethoxy-5-(5-hydroxypyridin-3-yl)phenyl]methyl]piperazin-1-yl]-N-[3-nitro-4-(2-phenylsulfanylethylamino)phenyl] sulfonylpyridazine-3-carboxamide |
| EI17-83-02R | EI17-83 | 2Q83 | 6-[4-[[3-(3-Hydroxyphenyl)-5-propan-2-yloxyphenyl]methyl]piperazin-1-yl]-N-[3-nitro-4-(2-phenylsulfanylethylamino)phenyl]sulfonylpyridazine-3-carboxamide |
| EI17-84-02R | EI17-84 | 2Q84 | 6-[4-[[3-(5-Hydroxypyridin-3-yl)-5-propan-2-yloxyphenyl]methyl]piperazin-1-yl]-N-[3-nitro-4-(2-phenylsulfanylethylamino)phenyl] sulfonylpyridazine-3-carboxamide |
| EI17-85-02R | EI17-85 | 2Q85 | 6-[4-[[4-(3-Hydroxyphenyl)-2,6-dimethoxyphenyl]methyl]piperazin-1-yl]-N-[3-nitro-4-(2-phenylsulfanylethylamino)phenyl]sulfonylpyridazine-3-carboxamide |
| EI17-86-02R | EI17-86 | 2Q86 | 6-[4-[[4-(5-Hydroxypyridin-3-yl)-2,6-dimethoxyphenyl]methyl]piperazin-1-yl]-N-[3-nitro-4-(2-phenylsulfanylethylamino)phenyl]sulfonylpyridazine-3-carboxamide |
| EI17-87-02R | EI17-87 | 2Q87 | 6-[4-[[3-(3-Hydroxyphenyl)-5-(trifluoromethyl)phenyl]methyl]piperazin-1-yl]-N-[3-nitro-4-(2-phenylsulfanylethylamino)phenyl]sulfonylpyridazine-3- carboxamide |
| EI17-88-02R | EI17-88 | 2Q88 | 6-[4-[[3-(5-Hydroxypyridin-3-yl)-5-(trifluoromethyl)phenyl]methyl]piperazin-1-yl]-N-[3-nitro-4-(2-phenylsulfanylethylamino)phenyl]sulfonylpyridazine-3- carboxamide |
| EI17-89-02R | EI17-89 | 2Q89 | 6-[4-[[3-(3-Hydroxyphenyl)-5-(trifluoromethoxy)phenyl]methyl]piperazin-1-yl]-N-[3-nitro-4-(2-phenylsulfanylethylamino)phenyl]sulfonylpyridazine-3-carboxamide |

(continued)

| Resin compound No. | Post-cleavage compound No. | Another name of post-cleavage compound in Table EJ11 | Compound name |
|---|---|---|---|
| EI17-90-02R | EI17-90 | 2Q90 | 6-[4-[[3-(5-Hydroxypyridin-3-yl)-5-(trifluoromethoxy)phenyl]methyl]piperazin-1-yl]-N-[3-nitro-4-(2-phenylsulfanylethylamino)phenyl] sulfonylpyridazine-3-carboxamide |
| EI17-91-02R | EI17-91 | 2Q91 | 6-[4-[[4-(3-Hydroxyphenyl)naphthalen-1-yl]methyl]piperazin-1-yl]-N-[3-nitro-4-(2-phenylsulfanylethylamino)phenyl]sulfonylpyridazine-3-carboxamide |
| EI17-92-02R | EI17-92 | 2Q92 | 6-[4-[[4-(5-Hydroxypyridin-3-yl)naphthalen-1-yl]methyl]piperazin-1-yl]-N-[3-nitro-4-(2-phenylsulfanylethylamino)phenyl]sulfonylpyridazine-3-carboxamide |
| EI17-93-02R | EI17-93 | 2Q93 | 6-[4-[[4-(3-Hydroxyphenyl)thiophen-2-yl]methyl]piperazin-1-yl]-N-[3-nitro-4-(2-phenylsulfanylethylamino)phenyl]sulfonylpyridazine-3-carboxamide |
| EI17-94-02R | EI17-94 | 2Q94 | 6-[4-[[4-(5-Hydroxypyridin-3-yl)thiophen-2-yl]methyl]piperazin-1-yl]-N-[3-nitro-4-(2-phenylsulfanylethylamino)phenyl]sulfonylpyridazine-3-carboxamide |
| EI17-95-02R | EI17-95 | 2Q95 | 6-[4-[[2-[2-(3-Hydroxyphenyl)ethynyl]phenyl]methyl]piperazin-1-yl]-N-[3-nitro-4-(2-phenylsulfanylethylamino)phenyl]sulfonylpyridazine-3-carboxamide |
| EI17-96-02R | EI17-96 | 2Q96 | 6-[4-[[2-[2-(5-Hydroxypyridin-3-yl)ethynyl]phenyl]methyl]piperazin-1-yl]-N-[3-nitro-4-(2-phenylsulfanylethylamino)phenyl]sulfonylpyridazine-3-carboxamide |
| EI17-97-02R | EI17-97 | 2Q97 | 4-[4-[1-[4-(3-Hydroxyphenyl)phenyl]ethyl]piperazin-1-yl]-N-[3-nitro-4-(2-phenylsulfanylethylamino)phenyl]sulfonylbenzamide |
| EI17-98-02R | EI17-98 | 2Q98 | 4-[4-[1-[4-(5-Hydroxypyridin-3-yl)phenyl]ethyl]piperazin-1-yl]-N-[3-nitro-4-(2-phenylsulfanylethylamino)phenyl]sulfonylbenzamide |
| EI17-99-02R | EI17-99 | 2Q99 | 4-[4-[1-[2-Fluoro-4-(3-hydroxyphenyl)phenyl]ethyl]piperazin-1-yl]-N-[3-nitro-4-(2-phenylsulfanylethylamino)phenyl]sulfonylbenzamide |
| EI17-100-02R | EI17-100 | 2Q 100 | 4-[4-[1-[2-Fluoro-4-(5-hydroxypyridin-3-yl)phenyl]ethyl]piperazin-1-yl]-N-[3-nitro-4-(2-phenylsulfanylethylamino)phenyl]sulfonylbenzamide |

[Table 70]

[Table EI18] mixEII8-02R, amine reagent used: BB42, post-cleavage mixture No.: mixEI18

| Resin compound No. | Post-cleavage compound No. | Another name of post-cleavage compound in Table EJ12 | Compound name |
|---|---|---|---|
| EI18-01-02R | EI18-01 | 2R01 | 6-[4-[4-(3-Hydroxyphenyl)thiophene-2-carbonyl]piperazin-1-yl]-N-[4-(2-phenylsulfanylethylamino)-3-(trifluoromethyl)phenyl]sulfonylpyridazine-3-carboxamide |
| EI18-02-02R | EI18-02 | 2R02 | 6-[4-[4-(5-Hydroxypyridin-3-yl)thiophene-2-carbonyl]piperazin-1-yl]-N-[4-(2-phenylsulfanylethylamino)-3-(trifluoromethyl)phenyl]sulfonylpyridazine-3-carboxamide |
| EI18-03-02R | EI18-03 | 2R03 | 6-[4-[4-(3-Hydroxyphenyl)-3-methylbenzoyl]piperazin-1-yl]-N-[4-(2-phenylsulfanylethylamino)-3-(trifluoromethyl)phenyl]sulfonylpyridazine-3-carboxamide |
| EI18-04-02R | EI18-04 | 2R04 | 6-[4-[4-(5-Hydroxypyridin-3-yl)-3-methylbenzoyl]piperazin-1-yl]-N-[4-(2-phenylsulfanylethylamino)-3-(trifluoromethyl)phenyl] sulfonylpyridazine-3-carboxamide |
| EI18-05-02R | EI18-05 | 2R05 | 6-[4-[3-Fluoro-4-(3-hydroxyphenyl)benzoyl]piperazin-1-yl]-N-[4-(2-phenylsulfanylethylamino)-3-(trifluoromethyl)phenyl]sulfonylpyridazine-3-carboxamide |
| EI18-06-02R | EI18-06 | 2R06 | 6-[4-[3-Fluoro-4-(5-hydroxypyridin-3-yl)benzoyl]piperazin-1- yl]-N-[4-(2-phenylsulfanylethylamino)-3-(trifluoromethyl)phenyl]sulfonylpyridazine-3-carboxamide |
| EI18-07-02R | EI18-07 | 2R07 | 6-[4-[2-[2-(3-Hydroxyphenyl)ethynyl]benzoyl]piperazin-1-yl]-N-[4-(2-phenylsulfanylethylamino)-3-(trifluoromethyl)phenyl]sulfonylpyridazine-3-carboxamide |
| EI18-08-02R | EI18-08 | 2R08 | 6-[4-[2-[2-(5-Hydroxypyridin-3-yl)ethynyl]benzoyl]piperazin-1-yl]-N-[4-(2-phenylsulfanylethylamino)-3-(trifluoromethyl)phenyl]sulfonylpyridazine-3-carboxamide |
| EI18-09-02R | EI18-09 | 2R09 | 6-[4-[5-[2-(3-Hydroxyphenyl)ethynyl]pyridine-3-carbonyl]piperazin-1-yl]-N-[4-(2-phenylsulfanylethylamino)-3-(trifluoromethyl)phenyl]sulfonylpyridazine-3-carboxamide |
| EI18-10-02R | EI18-10 | 2R10 | 6-[4-[5-[2-(5-Hydroxypyridin-3-yl)ethynyl]pyridine-3-carbonyl]piperazin-1-yl]-N-[4-(2-phenylsulfanylethylamino)-3-(trifluoromethyl)phenyl]sulfonylpyridazine-3-carboxamide |

(continued)

| Resin compound No. | Post-cleavage compound No. | Another name of post-cleavage compound in Table EJ12 | Compound name |
|---|---|---|---|
| EI18-11-02R | EI18-11 | 2R11 | 6-[4-[3-[2-(3-Hydroxyphenyl)phenyl]propanoyl]piperazin-1- yl]-N-[4-(2-phenylsulfanylethylamino)-3-(trifluoromethyl)phenyl]sulfonylpyridazine-3-carboxamide |
| EI18-12-02R | EI18-12 | 2R12 | 6-[4-[3-[2-(5-Hydroxypyridin-3-yl)phenyl]propanoyl]piperazin-1-yl]-N-[4-(2-phenylsulfanylethylamino)-3-(trifluoromethyl)phenyl]sulfonylpyridazine-3-carboxamide |
| EI18-13-02R | EI18-13 | 2R13 | 6-[4-[4-(3-Hydroxyphenyl)naphthalene-1-carbonyl]piperazin-1-yl]-N-[4-(2-phenylsulfanylethylamino)-3-(trifluoromethyl)phenyl]sulfonylpyridazine-3-carboxamide |
| EI18-14-02R | EI18-14 | 2R14 | 6-[4-[4-(5-Hydroxypyridin-3-yl)naphthalene-1-carbonyl]piperazin-1-yl]-N-[4-(2-phenylsulfanylethylamino)-3-(trifluoromethyl)phenyl]sulfonylpyridazine-3-carboxamide |
| EI18-15-02R | EI18-15 | 2R15 | 6-[4-[3-tert-Butyl-5-(3-hydroxyphenyl)benzoyl]piperazin-1-yl]-N-[4-(2-phenylsulfanylethylamino)-3-(trifluoromethyl)phenyl]sulfonylpyridazine-3-carboxamide |
| EI18-16-02R | EI18-16 | 2R16 | 6-[4-[3-tert-Butyl-5-(5-hydroxypyridin-3-yl)benzoyl]piperazin-1-yl]-N-[4-(2-phenylsulfanylethylamino)-3-(trifluoromethyl)phenyl]sulfonylpyridazine-3-carboxamide |
| EI18-17-02R | EI18-17 | 2R17 | 6-[4-[3-(3-Hydroxyphenyl)-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| EI18-18-02R | EI18-18 | 2R18 | 6-[4-[3-(5-Hydroxypyridin-3-yl)-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| EI18-19-02R | EI18-19 | 2R19 | 6-[4-[3-[2-(3-Hydroxyphenyl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| EI18-20-02R | EI18-20 | 2R20 | 6-[4-[3-[2-(5-Hydroxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |

(continued)

| Resin compound No. | Post-cleavage compound No. | Another name of post-cleavage compound in Table EJ12 | Compound name |
|---|---|---|---|
| EI18-21-02R | EI18-21 | 2R21 | 6-[4-[3-[4-(3-Hydroxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| EI18-22-02R | EI18-22 | 2R22 | 6-[4-[3-[4-(5-Hydroxypyridin-3-yl)pyrazol-1-yl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| EI18-23-02R | EI18-23 | 2R23 | 4-[4-[4-(3-Hydroxyphenyl)thiophene-2-carbonyl]piperazin-1-yl]-N-[4-(2-phenylsulfanylethylamino)-3-(trifluoromethyl)phenyl]sulfonylbenzamide |
| EI18-24-02R | EI18-24 | 2R24 | 4-[4-[4-(5-Hydroxypyridin-3-yl)thiophene-2-carbonyl]piperazin-1-yl]-N-[4-(2-phenylsulfanylethylamino)-3-(trifluoromethyl)phenyl] sulfonylbenzamide |
| EI18-25-02R | EI18-25 | 2R25 | 4-[4-[4-(3-Hydroxyphenyl)-3-methylbenzoyl]piperazin-1-yl]-N-[4-(2-phenylsulfanylethylamino)-3-(trifluoromethyl)phenyl]sulfonylbenzamide |
| EI18-26-02R | EI18-26 | 2R26 | 4-[4-[4-(5-Hydroxypyridin-3-yl)-3-methylbenzoyl]piperazin-1-yl]-N-[4-(2-phenylsulfanylethylamino)-3-(trifluoromethyl)phenyl]sulfonylbenzamide |
| EI18-27-02R | EI18-27 | 2R27 | 4-[4-[3-Fluoro-4-(3-hydroxyphenyl)benzoyl]piperazin-1-yl]-N-[4-(2-phenylsulfanylethylamino)-3-(trifluoromethyl)phenyl]sulfonylbenzamide |
| EI18-28-02R | EI18-28 | 2R28 | 4-[4-[3-Fluoro-4-(5-hydroxypyridin-3-yl)benzoyl]piperazin-1- yl]-N-[4-(2-phenylsulfanylethylamino)-3-(trifluoromethyl)phenyl]sulfonylbenzamide |
| EI18-29-02R | EI18-29 | 2R29 | 4-[4-[2-[2-(3-Hydroxyphenyl)ethynyl]benzoyl]piperazin-1-yl]-N-[4-(2-phenylsulfanylethylamino)-3-(trifluoromethyl)phenyl]sulfonylbenzamide |
| EI18-30-02R | EI18-30 | 2R30 | 4-[4-[2-[2-(5-Hydroxypyridin-3-yl)ethynyl]benzoyl]piperazin-1-yl]-N-[4-(2-phenylsulfanylethylamino)-3-(trifluoromethyl)phenyl]sulfonylbenzamide |
| EI18-31-02R | EI18-31 | 2R31 | 4-[4-[5-[2-(3-Hydroxyphenyl)ethynyl]pyridine-3-carbonyl]piperazin-1-yl]-N-[4-(2-phenylsulfanylethylamino)-3-(trifluoromethyl)phenyl]sulfonylbenzamide |
| EI18-32-02R | EI18-32 | 2R32 | 4-[4-[5-[2-(5-Hydroxypyridin-3-yl)ethynyl]pyridine-3-carbonyl]piperazin-1-yl]-N-[4-(2-phenylsulfanylethylamino)-3-(trifluoromethyl)phenyl]sulfonylbenzamide |

(continued)

| Resin compound No. | Post-cleavage compound No. | Another name of post-cleavage compound in Table EJ12 | Compound name |
|---|---|---|---|
| EI18-33-02R | EI18-33 | 2R33 | 4-[4-[3-[2-(3-Hydroxyphenyl)phenyl]propanoyl]piperazin-1- yl]-N-[4-(2-phenylsulfanylethylamino)-3-(trifluoromethyl)phenyl]sulfonylbenzamide |
| EI18-34-02R | EI18-34 | 2R34 | 4-[4-[3-[2-(5-Hydroxypyridin-3-yl)phenyl]propanoyl]piperazin-1-yl]-N-[4-(2-phenylsulfanylethylamino)-3-(trifluoromethyl)phenyl]sulfonylbenzamide |
| EI18-35-02R | EI18-35 | 2R35 | 4-[4-[4-(3-Hydroxyphenyl)naphthalene-1-carbonyl]piperazin-1-yl]-N-[4-(2-phenylsulfanylethylamino)-3-(trifluoromethyl)phenyl]sulfonylbenzamide |
| EI18-36-02R | EI18-36 | 2R36 | 4-[4-[4-(5-Hydroxypyridin-3-yl)naphthalene-1-carbonyl]piperazin-1-yl]-N-[4-(2-phenylsulfanylethylamino)-3-(trifluoromethyl)phenyl]sulfonylbenzamide |
| EI18-37-02R | EI18-37 | 2R37 | 4-[4-[3-tert-Butyl-5-(3-hydroxyphenyl)benzoyl]piperazin-1-yl]-N-[4-(2-phenylsulfanylethylamino)-3-(trifluoromethyl)phenyl]sulfonylbenzamide |
| EI18-38-02R | EI18-38 | 2R38 | 4-[4-[3-tert-Butyl-5-(5-hydroxypyridin-3-yl)benzoyl]piperazin-1-yl]-N-[4-(2-phenylsulfanylethylamino)-3-(trifluoromethyl)phenyl]sulfonylbenzamide |
| EI18-39-02R | EI18-39 | 2R39 | 4-[4-[3-(3-Hydroxyphenyl)-5-(trifluoromethyl)benzoyl]piperazin-1-yl]-N-[4-(2-phenylsulfanylethylamino)-3-(trifluoromethyl)phenyl]sulfonylbenzamide |
| EI18-40-02R | EI18-40 | 2R40 | 4-[4-[3-(5-Hydroxypyridin-3-yl)-5-(trifluoromethyl)benzoyl]piperazin-1-yl]-N-[4-(2-phenylsulfanylethylamino)-3-(trifluoromethyl)phenyl]sulfonylbenzamide |
| EI18-41-02R | EI18-41 | 2R41 | 4-[4-[3-[2-(3-Hydroxyphenyl)ethynyl]-5-(trifluoromethyl)benzoyl]piperazin-1-yl]-N-[4-(2-phenylsulfanylethylamino)-3-(trifluoromethyl)phenyl]sulfonylbenzamide |
| EI18-42-02R | EI18-42 | 2R42 | 4-[4-[3-[2-(5-Hydroxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylbenzamide |
| EI18-43-02R | EI18-43 | 2R43 | 4-[4-[3-[4-(3-Hydroxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylbenzamide |
| EI18-44-02R | EI18-44 | 2R44 | 4-[4-[3-[4-(5-Hydroxypyridin-3-yl)pyrazol-1-yl]-5-(trifluoromethyl)benzoyl]piperazin-1-yl]-N-[4-(2-phenylsulfanylethylamino)-3-(trifluoromethyl)phenyl]sulfonylbenzamide |

(continued)

| Resin compound No. | Post-cleavage compound No. | Another name of post-cleavage compound in Table EJ12 | Compound name |
|---|---|---|---|
| EI18-45-02R | EI18-45 | 2R45 | 4-[4-[[2-(3-Hydroxyphenyl)phenyl]methyl]piperazin-1-yl]-N-[4-(2-phenylsulfanylethylamino)-3-(trifluoromethyl)phenyl]sulfonylbenzamide |
| EI18-46-02R | EI18-46 | 2R46 | 4-[4-[[2-(5-Hydroxypyridin-3-yl)phenyl]methyl]piperazin-1-yl]-N-[4-(2-phenylsulfanylethylamino)-3-(trifluoromethyl)phenyl]sulfonylbenzamide |
| EI18-47-02R | EI18-47 | 2R47 | 4-[4-[[4-(3-Hydroxyphenyl)-3-methylphenyl]methyl]piperazin-1-yl]-N-[4-(2-phenylsulfanylethylamino)-3-(trifluoromethyl)phenyl]sulfonylbenzamide |
| EI18-48-02R | EI18-48 | 2R48 | 4-[4-[[4-(5-Hydroxypyridin-3-yl)-3-methylphenyl]methyl]piperazin-1-yl]-N-[4-(2-phenylsulfanylethylamino)-3-(trifluoromethyl)phenyl]sulfonylbenzamide |
| EI18-49-02R | EI18-49 | 2R49 | 4-[4-[[2-Ethyl-4-(3-hydroxyphenyl)phenyl]methyl]piperazin-1-yl]-N-[4-(2-phenylsulfanylethylamino)-3-(trifluoromethyl)phenyl]sulfonylbenzamide |
| EI18-50-02R | EI18-50 | 2R50 | 4-[4-[[2-Ethyl-4-(5-hydroxypyridin-3-yl)phenyl]methyl]piperazin-1-yl]-N-[4-(2-phenylsulfanylethylamino)-3-(trifluoromethyl)phenyl]sulfonylbenzamide |
| EI18-51-02R | EI18-51 | 2R51 | 4-[4-[[3-Fluoro-4-(3-hydroxyphenyl)phenyl]methyl]piperazin-1-yl]-N-[4-(2-phenylsulfanylethylamino)-3-(trifluoromethyl)phenyl]sulfonylbenzamide |
| EI18-52-02R | EI18-52 | 2R52 | 4-[4-[[3-Fluoro-4-(5-hydroxypyridin-3-yl)phenyl]methyl]piperazin-1-yl]-N-[4-(2-phenylsulfanylethylamino)-3-(trifluoromethyl)phenyl]sulfonylbenzamide |
| EI18-53-02R | EI18-53 | 2R53 | 4-[4-[[4-(3-Hydroxyphenyl)-2-methoxyphenyl]methyl]piperazin-1-yl]-N-[4-(2-phenylsulfanylethylamino)-3-(trifluoromethyl)phenyl]sulfonylbenzamide |
| EI18-54-02R | EI18-54 | 2R54 | 4-[4-[[4-(5-Hydroxypyridin-3-yl)-2-methoxyphenyl]methyl]piperazin-1-yl]-N-[4-(2-phenylsulfanylethylamino)-3-(trifluoromethyl)phenyl]sulfonylbenzamide |
| EI18-55-02R | EI18-55 | 2R55 | 4-[4-[[3-Ethoxy-5-(3-hydroxyphenyl)phenyl]methyl]piperazin-1-yl]-N-[4-(2-phenylsulfanylethylamino)-3-(trifluoromethyl)phenyl]sulfonylbenzamide |
| EI18-56-02R | EI18-56 | 2R56 | 4-[4-[[3-Ethoxy-5-(5-hydroxypyridin-3-yl)phenyl]methyl]piperazin-1-yl]-N-[4-(2-phenylsulfanylethylamino)-3-(trifluoromethyl)phenyl]sulfonylbenzamide |

(continued)

| Resin compound No. | Post-cleavage compound No. | Another name of post-cleavage compound in Table EJ12 | Compound name |
|---|---|---|---|
| EI18-57-02R | EI18-57 | 2R57 | 4-[4-[[3-(3-Hydroxyphenyl)-5-propan-2-yloxyphenyl]methyl]piperazin-1-yl]-N-[4-(2-phenylsulfanylethylamino)-3-(trifluoromethyl)phenyl]sulfonylbenzamide |
| EI18-58-02R | EI18-58 | 2R58 | 4-[4-[[3-(5-Hydroxypyridin-3-yl)-5-propan-2-yloxyphenyl]methyl]piperazin-1-yl]-N-[4-(2-phenylsulfanylethylamino)-3-(trifluoromethyl)phenyl]sulfonylbenzamide |
| EI18-59-02R | EI18-59 | 2R59 | 4-[4-[[4-(3-Hydroxyphenyl)-2,6-dimethoxyphenyl]methyl]piperazin-1-yl]-N-[4-(2-phenylsulfanylethylamino)-3-(trifluoromethyl)phenyl]sulfonylbenzamide |
| EI18-60-02R | EI18-60 | 2R60 | 4-[4-[[4-(5-Hydroxypyridin-3-yl)-2,6-dimethoxyphenyl]methyl]piperazin-1-yl]-N-[4-(2-phenylsulfanylethylamino)-3-(trifluoromethyl)phenyl]sulfonylbenzamide |
| EI18-61-02R | EI18-61 | 2R61 | 4-[4-[[3-(3-Hydroxyphenyl)-5-(trifluoromethyl)phenyl]methyl]piperazin-1-yl]-N-[4-(2-phenylsulfanylethylamino)-3-(trifluoromethyl)phenyl]sulfonylbenzamide |
| EI18-62-02R | EI18-62 | 2R62 | 4-[4-[[3-(5-Hydroxypyridin-3-yl)-5-(trifluoromethyl)phenyl]methyl]piperazin-1-yl]-N-[4-(2-phenylsulfanylethylamino)-3-(trifluoromethyl)phenyl]sulfonylbenzamide |
| EI18-63-02R | EI18-63 | 2R63 | 4-[4-[[3-(3-Hydroxyphenyl)-5-(trifluoromethoxy)phenyl]methyl]piperazin-1-yl]-N-[4-(2-phenylsulfanylethylamino)-3-(trifluoromethyl)phenyl]sulfonylbenzamide |
| EI18-64-02R | EI18-64 | 2R64 | 4-[4-[[3-(5-Hydroxypyridin-3-yl)-5-(trifluoromethoxy)phenyl]methyl]piperazin-1-yl]-N-[4-(2-phenylsulfanylethylamino)-3-(trifluoromethyl)phenyl]sulfonylbenzamide |
| EI18-65-02R | EI18-65 | 2R65 | 4-[4-[[4-(3-Hydroxyphenyl)naphthalen-1-yl]methyl]piperazin-1-yl]-N-[4-(2-phenylsulfanylethylamino)-3-(trifluoromethyl)phenyl]sulfonylbenzamide |
| EI18-66-02R | EI18-66 | 2R66 | 4-[4-[[4-(5-Hydroxypyridin-3-yl)naphthalen-1-yl]methyl]piperazin-1-yl]-N-[4-(2-phenylsulfanylethylamino)-3-(trifluoromethyl)phenyl] sulfonylbenzamide |
| EI18-67-02R | EI18-67 | 2R67 | 4-[4-[[4-(3-Hydroxyphenyl)thiophen-2-yl]methyl]piperazin-1-yl]-N-[4-(2-phenylsulfanylethylamino)-3-(trifluoromethyl)phenyl]sulfonylbenzamide |

(continued)

| Resin compound No. | Post-cleavage compound No. | Another name of post-cleavage compound in Table EJ12 | Compound name |
|---|---|---|---|
| EI18-68-02R | EI18-68 | 2R68 | 4-[4-[[4-(5-Hydroxypyridin-3-yl)thiophen-2-yl]methyl]piperazin-1-yl]-N-[4-(2-phenylsulfanylethylamino)-3-(trifluoromethyl)phenyl]sulfonylbenzamide |
| EI18-69-02R | EI18-69 | 2R69 | 4-[4-[[2-[2-(3-Hydroxyphenyl)ethynyl]phenyl]methyl]piperazin-1-yl]-N-[4-(2-phenylsulfanylethylamino)-3-(trifluoromethyl)phenyl]sulfonylbenzamide |
| EI18-70-02R | EI18-70 | 2R70 | 4-[4-[[2-[2-(5-Hydroxypyridin-3-yl)ethynyl]phenyl]methyl]piperazin-1-yl]-N-[4-(2-phenylsulfanylethylamino)-3-(trifluoromethyl)phenyl]sulfonylbenzamide |
| EI18-71-02R | EI18-71 | 2R71 | 6-[4-[[2-(3-Hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| EI18-72-02R | EI18-72 | 2R72 | 6-[4-[[2-(5-Hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| EI18-73-02R | EI18-73 | 2R73 | 6-[4-[[4-(3-Hydroxyphenyl)-3-methylphenyl]methyl]piperazin-1-yl]-N-[4-(2-phenylsulfanylethylamino)-3-(trifluoromethyl)phenyl] sulfonylpyridazine-3-carboxamide |
| EI18-74-02R | EI18-74 | 2R74 | 6-[4-[[4-(5-Hydroxypyridin-3-yl)-3-methylphenyl]methyl]piperazin-1-yl]-N-[4-(2-phenylsulfanylethylamino)-3-(trifluoromethyl)phenyl] sulfonylpyridazine-3-carboxamide |
| EI18-75-02R | EI18-75 | 2R75 | 6-[4-[[2-Ethyl-4-(3-hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| EI18-76-02R | EI18-76 | 2R76 | 6-[4-[[2-Ethyl-4-(5-hydroxypyridin-3-yl)phenyl]methyl]piperazin-1-yl]-N-[4-(2-phenylsulfanylethylamino)-3-(trifluoromethyl)phenyl] sulfonylpyridazine-3-carboxamide |
| EI18-77-02R | EI18-77 | 2R77 | 6-[4-[[3-Fluoro-4-(3-hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| EI18-78-02R | EI18-78 | 2R78 | 6-[4-[[3-Fluoro-4-(5-hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |

(continued)

| Resin compound No. | Post-cleavage compound No. | Another name of post-cleavage compound in Table EJ12 | Compound name |
|---|---|---|---|
| EI18-79-02R | EI18-79 | 2R79 | 6-[4-[[4-(3-Hydroxyphenyl)-2-methoxyphenyl]methyl]piperazin-1-yl]-N-[4-(2-phenylsulfanylethylamino)-3-(trifluoromethyl)phenyl] sulfonylpyridazine-3-carboxamide |
| EI18-80-02R | EI18-80 | 2R80 | 6-[4-[[4-(5-Hydroxypyridin-3-yl)-2-methoxyphenyl]methyl]piperazin-1-yl]-N-[4-(2-phenylsulfanylethylamino)-3-(trifluoromethyl)phenyl] sulfonylpyridazine-3-carboxamide |
| EI18-81-02R | EI18-81 | 2R81 | 6-[4-[[3-Ethoxy-5-(3-hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| EI18-82-02R | EI18-82 | 2R82 | 6-[4-[[3-Ethoxy-5-(5-hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| EI18-83-02R | EI18-83 | 2R83 | 6-[4-[[3-(3-Hydroxyphenyl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| EI18-84-02R | EI18-84 | 2R84 | 6-[4-[[3-(5-Hydroxypyridin-3-yl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| EI18-85-02R | EI18-85 | 2R85 | 6-[4-[[4-(3-Hydroxyphenyl)-2,6-dimethoxyphenyl]methyl]piperazin-1-yl]-N-[4-(2-phenylsulfanylethylamino)-3-(trifluoromethyl)phenyl]sulfonylpyridazine-3-carboxamide |
| EI18-86-02R | EI18-86 | 2R86 | 6-[4-[[4-(5-Hydroxypyridin-3-yl)-2,6-dimethoxyphenyl]methyl]piperazin-1-yl]-N-[4-(2-phenylsulfanylethylamino)-3-(trifluoromethyl)phenyl] sulfonylpyridazine-3-carboxamide |
| EI18-87-02R | EI18-87 | 2R87 | 6-[4-[[3-(3-Hydroxyphenyl)-5-(trifluoromethyl)phenyl]methyl]piperazin-1-yl]-N-[4-(2-phenylsulfanylethylamino)-3-(trifluoromethyl)phenyl] sulfonylpyridazine-3-carboxamide |
| EI18-88-02R | EI18-88 | 2R88 | 6-[4-[[3-(5-Hydroxypyridin-3-yl)-5-(trifluoromethyl)phenyl]methyl]piperazin-1-yl]-N-[4-(2-phenylsulfanylethylamino)-3-(trifluoromethyl)phenyl]sulfonylpyridazine-3-carboxamide |
| EI18-89-02R | EI18-89 | 2R89 | 6-[4-[[3-(3-Hydroxyphenyl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |

(continued)

| Resin compound No. | Post-cleavage compound No. | Another name of post-cleavage compound in Table EJ12 | Compound name |
|---|---|---|---|
| EI18-90-02R | EI18-90 | 2R90 | 6-[4-[[3-(5-Hydroxypyridin-3-yl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |
| EI18-91-02R | EI18-91 | 2R91 | 6-[4-[[4-(3-Hydroxyphenyl)naphthalen-1-yl]methyl]piperazin-1-yl]-N-[4-(2-phenylsulfanylethylamino)-3-(trifluoromethyl)phenyl]sulfonylpyridazine-3-carboxamide |
| EI18-92-02R | EI18-92 | 2R92 | 6-[4-[[4-(5-Hydroxypyridin-3-yl)naphthalen-1-yl]methyl]piperazin-1-yl]-N-[4-(2-phenylsulfanylethylamino)-3-(trifluoromethyl)phenyl]sulfonylpyridazine-3-carboxamide |
| EI18-93-02R | EI18-93 | 2R93 | 6-[4-[[4-(3-Hydroxyphenyl)thiophen-2-yl]methyl]piperazin-1-yl]-N-[4-(2-phenylsulfanylethylamino)-3-(trifluoromethyl)phenyl]sulfonylpyridazine-3-carboxamide |
| EI18-94-02R | EI18-94 | 2R94 | 6-[4-[[4-(5-Hydroxypyridin-3-yl)thiophen-2-yl]methyl]piperazin-1-yl]-N-[4-(2-phenylsulfanylethylamino)-3-(trifluoromethyl)phenyl]sulfonylpyridazine-3-carboxamide |
| EI18-95-02R | EI18-95 | 2R95 | 6-[4-[[2-[2-(3-Hydroxyphenyl)ethynyl]phenyl]methyl]piperazin-1-yl]-N-[4-(2-phenylsulfanylethylamino)-3-(trifluoromethyl)phenyl]sulfonylpyridazine-3-carboxamide |
| EI18-96-02R | EI18-96 | 2R96 | 6-[4-[[2-[2-(5-Hydroxypyridin-3-yl)ethynyl]phenyl]methyl]piperazin-1-yl]-N-[4-(2-phenylsulfanylethylamino)-3-(trifluoromethyl)phenyl]sulfonylpyridazine-3-carboxamide |
| EI18-97-02R | EI18-97 | 2R97 | 4-[4-[1-[4-(3-Hydroxyphenyl)phenyl]ethyl]piperazin-1-yl]-N-[4-(2-phenylsulfanylethylamino)-3-(trifluoromethyl)phenyl]sulfonylbenzamide |
| EI18-98-02R | EI18-98 | 2R98 | 4-[4-[1-[4-(5-Hydroxypyridin-3-yl)phenyl]ethyl]piperazin-1-yl]-N-[4-(2-phenylsulfanylethylamino)-3-(trifluoromethyl)phenyl]sulfonylbenzamide |
| EI18-99-02R | EI18-99 | 2R99 | 4-[4-[1-[2-Fluoro-4-(3-hydroxyphenyl)phenyl]ethyl]piperazin-1-yl]-N-[4-(2-phenylsulfanylethylamino)-3-(trifluoromethyl)phenyl]sulfonylbenzamide |
| EI18-100-02R | EI18-100 | 2R100 | 4-[4-[1-[2-Fluoro-4-(5-hydroxypyridin-3-yl)phenyl]ethyl]piperazin-1-yl]-N-[4-(2-phenylsulfanylethylamino)-3-(trifluoromethyl)phenyl]sulfonylbenzamide |

Example 6-4-1: Synthesis of mixture mixEJ01

[Formula 114]

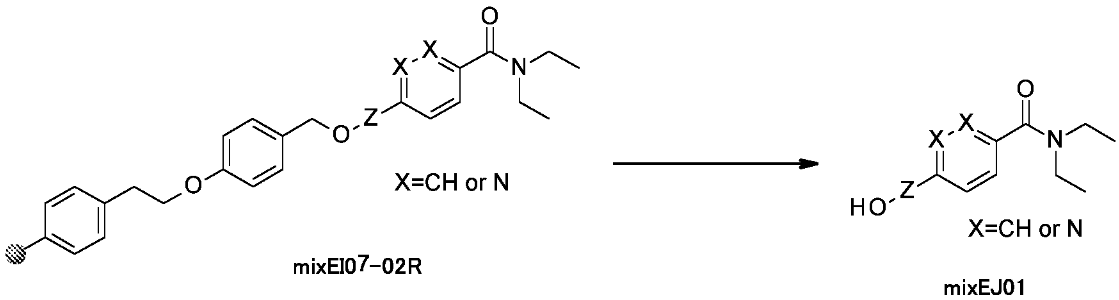


mixEI07-02R

mixEJ01

Under a nitrogen atmosphere, to a 1.6 mL glass vial were added the mixture mixEI07-02R shown in [Table EI07] (a mixture that may contain 100 compounds, 49.2 mg, loading rate 0.648 mmol/g, 31.9 μmol) and 1,2,3-trimethylbenzene/DCM (0.23M, 700 μL), and the mixture was shaken at room temperature for 1 hour. TFA (300 μL, 3.89 mmol) was added and the mixture was shaken at room temperature for 3 hours. The suspension of the reaction solution and the resins was transferred to a syringe equipped with a filter using a DCM solution and filtered under nitrogen pressure into a test tube in which DMI (0.5 mL) had been placed. Washing twice with DCM (0.5 mL), twice with DMI (0.5 mL), twice with DCM (0.5 mL), and twice with DMI (0.5 mL) was repeated. For the filtrate, the solvent was distilled off under reduced pressure in Genevac (40°C, after reducing pressure at 50 to 200 mbar until it was detected that the low boiling point solvent had been completely distilled off, 40°C, < 0.5 mbar for 16 hours), and the title mixture mixEJ01 shown in [Table EIJ01] was obtained. Analysis was performed under the analysis condition SMD-FA05-long-25min, and the UV area was confirmed at a UV wavelength of 290 nm. Note that the analysis results of the mixture are shown in [Table EJ01].

[Table 71]

[Table EIJ01]

| | |
|---|---|
| Mixture No. used | mixEI07-02R |
| Post-cleavage mixture No. | mixEJ01 |
| Compound No. in Table EJ01 | Compound name |
| 4J01 | N,N-Diethyl-6-[4-[4-(3-hydroxyphenyl)thiophene-2-carbonyl]piperazin-1-yl]pyridazine-3-carboxamide |
| 4J02 | N,N-Diethyl-6-[4-[4-(5-hydroxypyridin-3-yl)thiophene-2-carbonyl]piperazin-1-yl]pyridazine-3-carboxamide |
| 4J03 | N,N-Diethyl-6-[4-[4-(3-hydroxyphenyl)-3-methylbenzoyl]piperazin-1-yl]pyridazine-3-carboxamide |
| 4J04 | N,N-Diethyl-6-[4-[4-(5-hydroxypyridin-3-yl)-3-methylbenzoyl]piperazin-1-yl]pyridazine-3-carboxamide |
| 4J05 | N,N-Diethyl-6-[4-[3-fluoro-4-(3-hydroxyphenyl)benzoyl]piperazin-1-yl]pyridazine-3-carboxamide |
| 4J06 | N,N-Diethyl-6-[4-[3-fluoro-4-(5-hydroxypyridin-3-yl)benzoyl]piperazin-1-yl]pyridazine-3-carboxamide |
| 4J07 | N,N-Diethyl-6-[4-[2-[2-(3-hydroxyphenyl)ethynyl]benzoyl]piperazin-1-yl]pyridazine-3-carboxamide |

(continued)

| Mixture No. used | mixEI07-02R |
|---|---|
| Post-cleavage mixture No. | mixEJ01 |
| Compound No. in Table EJ01 | Compound name |
| 4J08 | N,N-Diethyl-6-[4-[2-[2-(5-hydroxypyridin-3-yl)ethynyl]benzoyl]piperazin-1-yl]pyridazine-3-carboxamide |
| 4J09 | N,N-Diethyl-6-[4-[5-[2-(3-hydroxyphenyl)ethynyl]pyridine-3-carbonyl]piperazin-1-yl]pyridazine-3-carboxamide |
| 4J10 | N,N-Diethyl-6-[4-[5-[2-(5-hydroxypyridin-3-yl)ethynyl]pyridine-3-carbonyl]piperazin-1-yl]pyridazine-3-carboxamide |
| 4J11 | N,N-Diethyl-6-[4-[3-[2-(3-hydroxyphenyl)phenyl]propanoyl]piperazin-1- yl]pyridazine-3-carboxamide |
| 4J12 | N,N-Diethyl-6-[4-[3-[2-(5-hydroxypyridin-3-yl)phenyl]propanoyl]piperazin-1-yl]pyridazine-3-carboxamide |
| 4J13 | N,N-Diethyl-6-[4-[4-(3-hydroxyphenyl)naphthalene-1-carbonyl]piperazin-1-yl]pyridazine-3-carboxamide |
| 4J14 | N,N-Diethyl-6-[4-[4-(5-hydroxypyridin-3-yl)naphthalene-1-carbonyl]piperazin-1-yl]pyridazine-3-carboxamide |
| 4J15 | 6-[4-[3-tert-Butyl-5-(3-hydroxyphenyl)benzoyl]piperazin-1-yl]-N,N-diethylpyridazine-3-carboxamide |
| 4J16 | 6-[4-[3-tert-Butyl-5-(5-hydroxypyridin-3-yl)benzoyl]piperazin-1-yl]-N,N-diethylpyridazine-3-carboxamide |
| 4J17 | N,N-Diethyl-6-[4-[3-(3-hydroxyphenyl)-5-(trifluoromethyl)benzoyl]piperazin-1-yl]pyridazine-3-carboxamide |
| 4J18 | N,N-Diethyl-6-[4-[3-(5-hydroxypyridin-3-yl)-5-(trifluoromethyl)benzoyl]piperazin-1-yl]pyridazine-3-carboxamide |
| 4J19 | N,N-Diethyl-6-[4-[3-[2-(3-hydroxyphenyl)ethynyl]-5-(trifluoromethyl)benzoyl]piperazin-1-yl]pyridazine-3-carboxamide |
| 4J20 | N,N-Diethyl-6-[4-[3-[2-(5-hydroxypyridin-3-yl)ethynyl]-5-(trifluoromethyl)benzoyl]piperazin-1-yl]pyridazine-3-carboxamide |
| 4J21 | N,N-Diethyl-6-[4-[3-[4-(3-hydroxyphenyl)pyrazol-1-yl]-5-(trifluoromethyl)benzoyl]piperazin-1-yl]pyridazine-3-carboxamide |
| 4J22 | N,N-Diethyl-6-[4-[3-[4-(5-hydroxypyridin-3-yl)pyrazol-1-yl]-5-(trifluoromethyl)benzoyl]piperazin-1-yl]pyridazine-3-carboxamide |
| 4J23 | N,N-Diethyl-4-[4-[4-(3-hydroxyphenyl)thiophene-2-carbonyl]piperazin-1-yl]benzamide |
| 4J24 | N,N-Diethyl-4-[4-[4-(5-hydroxypyridin-3-yl)thiophene-2-carbonyl]piperazin-1-yl]benzamide |
| 4J25 | N,N-Diethyl-4-[4-[4-(3-hydroxyphenyl)-3-methylbenzoyl]piperazin-1-yl]benzamide |
| 4J26 | N,N-Diethyl-4-[4-[4-(5-hydroxypyridin-3-yl)-3-methylbenzoyl]piperazin-1-yl]benzamide |
| 4J27 | N,N-Diethyl-4-[4-[3-fluoro-4-(3-hydroxyphenyl)benzoyl]piperazin-1-yl]benzamide |
| 4J28 | N,N-Diethyl-4-[4-[3-fluoro-4-(5-hydroxypyridin-3-yl)benzoyl]piperazin-1-yl]benzamide |
| 4J29 | N,N-Diethyl-4-[4-[2-[2-(3-hydroxyphenyl)ethynyl]benzoyl]piperazin-1-yl]benzamide |
| 4J30 | N,N-Diethyl-4-[4-[2-[2-(5-hydroxypyridin-3-yl)ethynyl]benzoyl]piperazin-1-yl]benzamide |

(continued)

| Mixture No. used | mixEI07-02R |
|---|---|
| Post-cleavage mixture No. | mixEJ01 |
| Compound No. in Table EJ01 | Compound name |
| 4J31 | N,N-Diethyl-4-[4-[5-[2-(3-hydroxyphenyl)ethynyl]pyridine-3-carbonyl]piperazin-1-yl]benzamide |
| 4J32 | N,N-Diethyl-4-[4-[5-[2-(5-hydroxypyridin-3-yl)ethynyl]pyridine-3-carbonyl]piperazin-1-yl]benzamide |
| 4J33 | N,N-Diethyl-4-[4-[3-[2-(3-hydroxyphenyl)phenyl]propanoyl]piperazin-1- yl]benzamide |
| 4J34 | N,N-Diethyl-4-[4-[3-[2-(5-hydroxypyridin-3-yl)phenyl]propanoyl]piperazin-1-yl]benzamide |
| 4J35 | N,N-Diethyl-4-[4-[4-(3-hydroxyphenyl)naphthalene-1-carbonyl]piperazin-1-yl]benzamide |
| 4J36 | N,N-Diethyl-4-[4-[4-(5-hydroxypyridin-3-yl)naphthalene-1-carbonyl]piperazin-1-yl]benzamide |
| 4J37 | 4-[4-[3-tert-Butyl-5-(3-hydroxyphenyl)benzoyl]piperazin-1-yl]-N,N-diethylbenzamide |
| 4J38 | 4-[4-[3-tert-Butyl-5-(5-hydroxypyridin-3-yl)benzoyl]piperazin-1-yl]-N,N-diethylbenzamide |
| 4J39 | N,N-Diethyl-4-[4-[3-(3-hydroxyphenyl)-5-(trifluoromethyl)benzoyl]piperazin-1-yl]benzamide |
| 4J40 | N,N-Diethyl-4-[4-[3-(5-hydroxypyridin-3-yl)-5-(trifluoromethyl)benzoyl]piperazin-1-yl]benzamide |
| 4J41 | N,N-Diethyl-4-[4-[3-[2-(3-hydroxyphenyl)ethynyl]-5-(trifluoromethyl)benzoyl]piperazin-1-yl]benzamide |
| 4J42 | N,N-Diethyl-4-[4-[3-[2-(5-hydroxypyridin-3-yl)ethynyl]-5-(trifluoromethyl)benzoyl]piperazin-1-yl]benzamide |
| 4J43 | N,N-Diethyl-4-[4-[3-[4-(3-hydroxyphenyl)pyrazol-1-yl]-5-(trifluoromethyl)benzoyl]piperazin-1-yl]benzamide |
| 4J44 | N,N-Diethyl-4-[4-[3-[4-(5-hydroxypyridin-3-yl)pyrazol-1-yl]-5-(trifluoromethyl)benzoyl]piperazin-1-yl]benzamide |
| 4J45 | N,N-Diethyl-4-[4-[[2-(3-hydroxyphenyl)phenyl]methyl]piperazin-1-yl]benzamide |
| 4J46 | N,N-Diethyl-4-[4-[[2-(5-hydroxypyridin-3-yl)phenyl]methyl]piperazin-1-yl]benzamide |
| 4J47 | N,N-Diethyl-4-[4-[[4-(3-hydroxyphenyl)-3-methylphenyl]methyl]piperazin-1-yl]benzamide |
| 4J48 | N,N-Diethyl-4-[4-[[4-(5-hydroxypyridin-3-yl)-3-methylphenyl]methyl]piperazin-1-yl]benzamide |
| 4J49 | N,N-Diethyl-4-[4-[[2-ethyl-4-(3-hydroxyphenyl)phenyl]methyl]piperazin-1-yl]benzamide |
| 4J50 | N,N-Diethyl-4-[4-[[2-ethyl-4-(5-hydroxypyridin-3-yl)phenyl]methyl]piperazin-1-yl]benzamide |
| 4J51 | N,N-Diethyl-4-[4-[[3-fluoro-4-(3-hydroxyphenyl)phenyl]methyl]piperazin-1-yl]benzamide |
| 4J52 | N,N-Diethyl-4-[4-[[3-fluoro-4-(5-hydroxypyridin-3-yl)phenyl]methyl]piperazin-1-yl]benzamide |
| 4J53 | N,N-Diethyl-4-[4-[[4-(3-hydroxyphenyl)-2-methoxyphenyl]methyl]piperazin-1-yl]benzamide |
| 4J54 | N,N-Diethyl-4-[4-[[4-(5-hydroxypyridin-3-yl)-2-methoxyphenyl]methyl]piperazin-1-yl]benzamide |
| 4J55 | 4-[4-[[3-Ethoxy-5-(3-hydroxyphenyl)phenyl]methyl]piperazin-1-yl]-N,N-diethylbenzamide |
| 4J56 | 4-[4-[[3-Ethoxy-5-(5-hydroxypyridin-3-yl)phenyl]methyl]piperazin-1-yl]-N,N-diethylbenzamide |
| 4J57 | N,N-Diethyl-4-[4-[[3-(3-hydroxyphenyl)-5-propan-2-yloxyphenyl]methyl]piperazin-1-yl]benzamide |
| 4J58 | N,N-Diethyl-4-[4-[[3-(5-hydroxypyridin-3-yl)-5-propan-2-yloxyphenyl]methyl]piperazin-1-yl]benzamide |
| 4J59 | N,N-Diethyl-4-[4-[[4-(3-hydroxyphenyl)-2,6-dimethoxyphenyl]methyl]piperazin-1-yl]benzamide |

(continued)

| Mixture No. used | mixEI07-02R |
|---|---|
| Post-cleavage mixture No. | mixEJ01 |
| Compound No. in Table EJ01 | Compound name |
| 4J60 | N,N-Diethyl-4-[4-[[4-(5-hydroxypyridin-3-yl)-2,6-dimethoxyphenyl]methyl]piperazin-1-yl]benzamide |
| 4J61 | N,N-Diethyl-4-[4-[[3-(3-hydroxyphenyl)-5-(trifluoromethyl)phenyl]methyl]piperazin-1-yl]benzamide |
| 4J62 | N,N-Diethyl-4-[4-[[3-(5-hydroxypyridin-3-yl)-5-(trifluoromethyl)phenyl]methyl]piperazin-1-yl]benzamide |
| 4J63 | N,N-Diethyl-4-[4-[[3-(3-hydroxyphenyl)-5-(trifluoromethoxy)phenyl]methyllpiperazin-1-yl]benzamide |
| 4J64 | N,N-Diethyl-4-[4-[[3-(5-hydroxypyridin-3-yl)-5-(trifluoromethoxy)phenyl]methyl]piperazin-1-yl]benzamide |
| 4J65 | N,N-Diethyl-4-[4-[[4-(3-hydroxyphenyl)naphthalen-1-yl]methyl]piperazin-1-yl]benzamide |
| 4J66 | N,N-Diethyl-4-[4-[[4-(5-hydroxypyridin-3-yl)naphthalen-1-yl]methyl]piperazin-1-yl]benzamide |
| 4J67 | N,N-Diethyl-4-[4-[[4-(3-hydroxyphenyl)thiophen-2-yl]methyl]piperazin-1-yl]benzamide |
| 4J68 | N,N-Diethyl-4-[4-[[4-(5-hydroxypyridin-3-yl)thiophen-2-yl]methyl]piperazin-1-yl]benzamide |
| 4J69 | N,N-Diethyl-4-[4-[[2-[2-(3-hydroxyphenyl)ethynyl]phenyl]methyl]piperazin-1-yl]benzamide |
| 4J70 | N,N-Diethyl-4-[4-[[2-[2-(5-hydroxypyridin-3-yl)ethynyl]phenyl]methyl]piperazin-1-yl]benzamide |
| 4171 | N,N-Diethyl-6-[4-[[2-(3-hydroxyphenyl)phenyl]methyl]piperazin-1-yl]pyridazine-3-carboxamide |
| 4J72 | N,N-Diethyl-6-[4-[[2-(5-hydroxypyridin-3-yl)phenyl]methyl]piperazin-1-yl]pyridazine-3-carboxamide |
| 4J73 | N,N-Diethyl-6-[4-[[4-(3-hydroxyphenyl)-3-methylphenyl]methyl]piperazin-1-yl]pyridazine-3-carboxamide |
| 4J74 | N,N-Diethyl-6-[4-[[4-(5-hydroxypyridin-3-yl)-3-methylphenyl]methyl]piperazin-1-yl]pyridazine-3-carboxamide |
| 4J75 | N,N-Diethyl-6-[4-[[2-ethyl-4-(3-hydroxyphenyl)phenyl]methyl]piperazin-1-yl]pyridazine-3-carboxamide |
| 4J76 | N,N-Diethyl-6-[4-[[2-ethyl-4-(5-hydroxypyridin-3-yl)phenyl]methyl]piperazin-1-yl]pyridazine-3-carboxamide |
| 4J77 | N,N-Diethyl-6-[4-[[3-fluoro-4-(3-hydroxyphenyl)phenyl]methyl]piperazin-1-yl]pyridazine-3-carboxamide |
| 4J78 | N,N-Diethyl-6-[4-[[3-fluoro-4-(5-hydroxypyridin-3-yl)phenyl]methyl]piperazin-1-yl]pyridazine-3-carboxamide |
| 4J79 | N,N-Diethyl-6-[4-[[4-(3-hydroxyphenyl)-2-methoxyphenyl]methyl]piperazin-1-yl]pyridazine-3-carboxamide |
| 4J80 | N,N-Diethyl-6-[4-[[4-(5-hydroxypyridin-3-yl)-2-methoxyphenyl]methyl]piperazin-1-yl]pyridazine-3-carboxamide |
| 4J81 | 6-[4-[[3-Ethoxy-5-(3-hydroxyphenyl)phenyl]methyl]piperazin-1-yl]-N,N-diethylpyridazine-3-carboxamide |

(continued)

| Mixture No. used | mixEI07-02R |
|---|---|
| Post-cleavage mixture No. | mixEJ01 |
| Compound No. in Table EJ01 | Compound name |
| 4J82 | 6-[4-[[3-Ethoxy-5-(5-hydroxypyridin-3-yl)phenyl]methyl]piperazin-1-yl]-N,N-diethylpyridazine-3-carboxamide |
| 4J83 | N,N-Diethyl-6-[4-[[3-(3-hydroxyphenyl)-5-propan-2-yloxyphenyl]methyl]piperazin-1-yl]pyridazine-3-carboxamide |
| 4J84 | N,N-Diethyl-6-[4-[[3-(5-hydroxypyridin-3-yl)-5-propan-2-yloxyphenyl]methyl]piperazin-1-yl]pyridazine-3-carboxamide |
| 4J85 | N,N-Diethyl-6-[4-[[4-(3-hydroxyphenyl)-2,6-dimethoxyphenyl]methyl]piperazin-1-yl]pyridazine-3-carboxamide |
| 4J86 | N,N-Diethyl-6-[4-[[4-(5-hydroxypyridin-3-yl)-2,6-dimethoxyphenyl]methyl]piperazin-1-yl]pyridazine-3-carboxamide |
| 4J87 | N,N-Diethyl-6-[4-[[3-(3-hydroxyphenyl)-5-(trifluoromethyl)phenyl]methyl]piperazin-1-yl]pyridazine-3-carboxamide |
| 4J88 | N,N-Diethyl-6-[4-[[3-(5-hydroxypyridin-3-yl)-5-(trifluoromethyl)phenyl]methyl]piperazin-1-yl]pyridazine-3 -carboxamide |
| 4J89 | N,N-Diethyl-6-[4-[[3-(3-hydroxyphenyl)-5-(trifluoromethoxy)phenyl]methyl]piperazin-1-yl]pyridazine-3-carboxamide |
| 4J90 | N,N-Diethyl-6-[4-[[3-(5-hydroxypyridin-3-yl)-5-(trifluoromethoxy)phenyl]methyl]piperazin-1-yl]pyridazine-3-carboxamide |
| 4J91 | N,N-Diethyl-6-[4-[[4-(3-hydroxyphenyl)naphthalen-1-yl]methyl]piperazin-1-yl]pyridazine-3-carboxamide |
| 4J92 | N,N-Diethyl-6-[4-[[4-(5-hydroxypyridin-3-yl)naphthalen-1-yl]methyl]piperazin-1 -yl]pyridazine-3-carboxamide |
| 4J93 | N,N-Diethyl-6-[4-[[4-(3-hydroxyphenyl)thiophen-2-yl]methyl]piperazin-1-yl]pyridazine-3-carboxamide |
| 4J94 | N,N-Diethyl-6-[4-[[4-(5-hydroxypyridin-3-yl)thiophen-2-yl]methyl]piperazin-1-yl]pyridazine-3-carboxamide |
| 4J95 | N,N-Diethyl-6-[4-[[2-[2-(3-hydroxyphenyl)ethynyl]phenyl]methyl]piperazin-1-yl]pyridazine-3-carboxamide |
| 4J96 | N,N-Diethyl-6-[4-[[2-[2-(5-hydroxypyridin-3-yl)ethynyl]phenyl]methyl]piperazin-1-yl]pyridazine-3-carboxamide |
| 4J97 | N,N-Diethyl-4-[4-[1-[4-(3-hydroxyphenyl)phenyl]ethyl]piperazin-1-yl]benzamide |
| 4J98 | N,N-Diethyl-4-[4-[1 -[4-(5-hydroxypyridin-3-yl)phenyl]ethyl]piperazin-1- yl]benzamide |
| 4J99 | N,N-Diethyl-4-[4-[1-[2-fluoro-4-(3-hydroxyphenyl)phenyl]ethyl]piperazin-1-yl]benzamide |
| 4J100 | N,N-Diethyl-4-[4-[1-[2-fluoro-4-(5-hydroxypyridin-3-yl)phenyl]ethyl]piperazin-1-yl]benzamide |

[Table EJ01] describes the retention time (min) of the observed UV peak, the $(M+H)^+$ observed in that UV peak, the retention time of the MS peak in the MS chromatogram, and the number of the compound attributed to the UV peak and the MS peak. Since two compounds may be missing in Example 6-2-2 and two compounds may be missing in Example 6-2-3, four compounds may be missing among the 100 compounds shown in Table [EJ01].

[Table 72]

[Table EJ01]

| Mixture No. | mixEJ01 | | | | | | | |
|---|---|---|---|---|---|---|---|---|
| m/z [M+H]+ | Retention time of MS peak (min) | Retention time of UV peak (min) | Attributed compound No. | | m/z [M+H]+ | Retention time of MS peak (min) | Retention time of UV peak (min) | Attributed compound No. |
| 453.207 | 3.457 | 3.41 | 4J94 | | 458.28 | 6.217 | 6.17 | 4J47, 4J97 |
| 461.266 | 3.58 | 3.54 | 4J74 | | 529.217 | 6.29 | 6.25 | 4J18, 4J64 |
| 465.195 | 3.667 | 3.63 | 4J24, 4J78 | | 504.286 | 6.297 | 6.25 | 4J59, 4J83 |
| 477.23 | 3.763 | 3.73 | 4J100, 4J28, 4J80 | | 487.27 | 6.37 | 6.34 | 4J34 |
| 447.25 | 3.883 | 3.85 | 4J72 | | 514.306 | 6.383 | 6.34 | 4J37, 4J87 |
| 475.245 | 4.037 | 3.99 | 4J04, 4J54, 4J76 | | 517.292 | 6.5 | 6.5 | 4J16 |
| 451.216 | 4.113 | 4.07 | 4J68 | | 509.255 | 6.517 | 6.5 | 4J36 |
| 507.271 | 4.17 | 4.13 | 4J86 | | 504.286 | 6.533 | 6.5 | 4J59, 4J83 |
| 497.266 | 4.22 | 4.13 | 4J92 | | 595.239 | 6.543 | 6.5 | 4J22 |
| 459.275 | 4.257 | 4.22 | 4J48, 4J98 | | 472.259 | 6.55 | 6.5 | 4J25, 4J49 |
| 491.277 | 4.27 | 4.22 | 4J82 | | 468.265 | 6.563 | 6.5 | 4J69 |
| 463.25 | 4.36 | 4.32 | 4J52 | | 530.237 | 6.573 | 6.5 | 4J89 |
| 445.26 | 4.527 | 4.48 | 4J46 | | 488.266 | 6.6 | 6.5 | 4J11, 4J55 |
| 505.281 | 4.617 | 4.56 | 4J60, 4J84 | | 484.234 | 6.703 | 6.67 | 4J07, 4J32 |
| 473.255 | 4.683 | 4.65 | 4J26, 4J50 | | 494.28 | 6.71 | 6.67 | 4J65 |
| 467.186 | 4.8 | 4.77 | 4J02 | | 502.306 | 6.897 | 6.86 | 4J57 |
| 515.302 | 4.81 | 4.77 | 4J38, 4J88 | | 512.252 | 6.977 | 6.94 | 4J61 |
| 475.245 | 4.843 | 4.81 | 4J04, 4J54, 4J76 | | 485.23 | 7.05 | 7.02 | 4J08, 4J09 |
| 505.281 | 4.85 | 4.81 | 4J60, 4J84 | | 486.225 | 7.07 | 7.02 | 4J10, 4J33 |
| 471.25 | 4.883 | 4.81 | 4J96 | | 528.222 | 7.15 | 7.11 | 4J17, 4J63 |
| 495.275 | 4.89 | 4.86 | 4J66 | | 466.191 | 7.217 | 7.18 | 4J01 |
| 489.261 | 4.953 | 4.93 | 4J12, 4J56 | | 478.225 | 7.42 | 7.38 | 4J05 |
| 479.22 | 4.957 | 4.93 | 4J06 | | 527.226 | 7.48 | 7.44 | 4J40 |
| 531.233 | 4.997 | 4.96 | 4J90 | | 515.302 | 7.547 | 7.52 | 4J38, 4J88 |
| 452.211 | 5.15 | 5.11 | 4J93 | | 474.25 | 7.57 | 7.52 | 4J03, 4J53, 4J75 |
| 446.255 | 5.257 | 5.23 | 4J71 | | 593.248 | 7.6 | 7.57 | 4J44 |
| 503.302 | 5.263 | 5.23 | 4J58 | | 484.234 | 7.82 | 7.78 | 4J07, 4J32 |
| 489.261 | 5.367 | 5.34 | 4J12, 4J56 | | 553.217 | 7.82 | 7.78 | 4J20 |
| 464.2 | 5.38 | 5.34 | 4J23, 4J77 | | 488.266 | 8.027 | 7.99 | 4J11, 4J55 |
| 511.245 | 5.423 | 5.39 | 4J14 | | 510.25 | 8.133 | 8.1 | 4J13 |
| 513.247 | 5.493 | 5.47 | 4J62 | | 483.239 | 8.26 | 8.25 | 4J30, 4J31 |
| 469.26 | 5.497 | 5.47 | 4J70 | | 483.239 | 8.293 | 8.25 | 4J30, 4J31 |
| 477.23 | 5.513 | 5.47 | 4J100, 4J28, 4J80 | | 464.2 | 8.61 | 8.57 | 4J23, 4J77 |
| 476.234 | 5.517 | 5.47 | 4J27, 4J79, 4J99 | | 528.222 | 8.657 | 8.61 | 4J17, 4J63 |
| 460.271 | 5.527 | 5.47 | 4J73 | | 476.234 | 8.727 | 8.69 | 4J27, 4J79, 4J99 |
| 459.275 | 5.567 | 5.47 | 4J48, 4J98 | | 476.234 | 8.76 | 8.69 | 4J27, 4J79, 4J99 |
| 529.217 | 5.667 | 5.64 | 4J18, 4J64 | | 472.259 | 8.893 | 8.86 | 4J25, 4J49 |
| 506.276 | 5.83 | 5.8 | 4J85 | | 474.25 | 8.9 | 8.86 | 4J03, 4J53, 4J75 |
| 450.221 | 5.857 | 5.8 | 4J67 | | 482.244 | 9.05 | 9.02 | 4J29 |
| 475.245 | 5.903 | 5.87 | 4J04, 4J54, 4J76 | | 551.226 | 9.06 | 9.02 | 4J42 |
| 473.255 | 5.907 | 5.87 | 4J26, 4J50 | | 594.243 | 9.093 | 9.05 | 4J21 |
| 465.195 | 5.913 | 5.87 | 4J24, 4J78 | | 485.23 | 9.24 | 9.23 | 4J08, 4J09 |
| 444.265 | 5.947 | 5.87 | 4J45 | | 486.225 | 9.257 | 9.23 | 4J10, 4J33 |
| 490.281 | 5.977 | 5.97 | 4J81 | | 516.297 | 9.277 | 9.23 | 4J15 |
| 470.255 | 6.01 | 5.97 | 4J95 | | 508.259 | 9.407 | 9.37 | 4J35 |
| 462.255 | 6.057 | 6.04 | 4J51 | | 552.222 | 9.457 | 9.41 | 4J19 |
| 458.28 | 6.07 | 6.04 | 4J47, 4J97 | | 526.231 | 9.87 | 9.83 | 4J39 |
| 496.271 | 6.077 | 6.04 | 4J91 | | 592.253 | 10.25 | 10.21 | 4J43 |
| 477.23 | 6.15 | 6.1 | 4J100, 4J28, 4J80 | | 514.306 | 10.503 | 10.47 | 4J37, 4J87 |
| 474.25 | 6.207 | 6.17 | 4J03, 4J53, 4J75 | | 550.231 | 10.623 | 10.58 | 4J41 |

Example 6-4-2: Synthesis of mixtures mixEJ02 to mixEJ12

Using the mixtures shown in [Table EI08] to [Table EI18] (mixEI08-02R to mixEI18-02R), the title mixtures shown in [Table EU02] to [Table EU12], mixEJ02 to mixEJ12, were synthesized by the same method as in Example

6-4-1 and obtained. Analysis was performed under the analysis condition SMD-FA05-long-25min, and the UV area was confirmed at a UV wavelength of 290 nm. Note that the analysis results of the mixtures. Note that the analysis results of the mixtures are shown in [Table EJ02] to [Table EJ12].

[Table 73]

[Table EU02]

| Mixture No. used | mixEI08-02R |
|---|---|
| Post-cleavage mixture No. | mixEJ02 |
| Compound No. in Table EJ02 | Compound name |
| 1M01 | 6-[4-[4-(3-Hydroxyphenyl)thiophene-2-carbonyl]piperazin-1-yl]-N-propylpyridazine-3-carboxamide |
| 1M02 | 6-[4-[4-(5-Hydroxypyridin-3-yl)thiophene-2-carbonyl]piperazin-1-yl]-N-propylpyridazine-3-carboxamide |
| 1M03 | 6-[4-[4-(3-Hydroxyphenyl)-3-methylbenzoyl]piperazin-1-yl]-N-propylpyridazine-3-carboxamide |
| 1M04 | 6-[4-[4-(5-Hydroxypyridin-3-yl)-3-methylbenzoyl]piperazin-1-yl]-N-propylpyridazine-3-carboxamide |
| 1M05 | 6-[4-[3-Fluoro-4-(3-hydroxyphenyl)benzoyl]piperazin-1-yl]-N-propylpyridazine-3-carboxamide |
| 1M06 | 6-[4-[3-Fluoro-4-(5-hydroxypyridin-3-yl)benzoyl]piperazin-1-yl]-N-propylpyridazine-3-carboxamide |
| 1M07 | 6-[4-[2-[2-(3-Hydroxyphenyl)ethynyl]benzoyl]piperazin-1-yl]-N-propylpyridazine-3-carboxamide |
| 1M08 | 6-[4-[2-[2-(5-Hydroxypyridin-3-yl)ethynyl]benzoyl]piperazin-1-yl]-N-propylpyridazine-3-carboxamide |
| 1M09 | 6-[4-[5-[2-(3-Hydroxyphenyl)ethynyl]pyridine-3-carbonyl]piperazin-1-yl]-N-propylpyridazine-3-carboxamide |
| 1M10 | 6-[4-[5-[2-(5-Hydroxypyridin-3-yl)ethynyl]pyridine-3-carbonyl]piperazin-1-yl]-N-propylpyridazine-3-carboxamide |
| 1M11 | 6-[4-[3-[2-(3-Hydroxyphenyl)phenyl]propanoyl]piperazin-1-yl]-N-propylpyridazine-3-carboxamide |
| 1M12 | 6-[4-[3-[2-(5-Hydroxypyridin-3-yl)phenyl]propanoyl]piperazin-1-yl]-N-propylpyridazine-3-carboxamide |
| 1M13 | 6-[4-[4-(3-Hydroxyphenyl)naphthalene-1-carbonyl]piperazin-1-yl]-N-propylpyridazine-3-carboxamide |
| 1M14 | 6-[4-[4-(5-Hydroxypyridin-3-yl)naphthalene-1-carbonyl]piperazin-1-yl]-N-propylpyridazine-3-carboxamide |
| 1M15 | 6-[4-[3-tert-Butyl-5-(3-hydroxyphenyl)benzoyl]piperazin-1-yl]-N-propylpyridazine-3-carboxamide |
| 1M16 | 6-[4-[3-tert-Butyl-5-(5-hydroxypyridin-3-yl)benzoyl]piperazin-1-yl]-N-propylpyridazine-3-carboxamide |
| 1M17 | 6-[4-[3-(3-Hydroxyphenyl)-5-(trifluoromethyl)benzoyl]piperazin-1-yl]-N-propylpyridazine-3-carboxamide |
| 1M18 | 6-[4-[3-(5-Hydroxypyridin-3-yl)-5-(trifluoromethyl)benzoyl]piperazin-1-yl]-N-propylpyridazine-3-carboxamide |
| 1M19 | 6-[4-[3-[2-(3-Hydroxyphenyl)ethynyl]-5-(trifluoromethyl)benzoyl]piperazin-1-yl]-N-propylpyridazine-3-carboxamide |

(continued)

| Mixture No. used | mixEI08-02R |
|---|---|
| Post-cleavage mixture No. | mixEJ02 |
| Compound No. in Table EJ02 | Compound name |
| 1M20 | 6-[4-[3-[2-(5-Hydroxypyridin-3-yl)ethynyl]-5-(trifluoromethyl)benzoyl]piperazin-1-yl]-N-propylpyridazine-3-carboxamide |
| 1M21 | 6-[4-[3-[4-(3-Hydroxyphenyl)pyrazol-1-yl]-5-(trifluoromethyl)benzoyl]piperazin-1-yl]-N-propylpyridazine-3-carboxamide |
| 1M22 | 6-[4-[3-[4-(5-Hydroxypyridin-3-yl)pyrazol-1-yl]-5-(trifluoromethyl)benzoyl]piperazin-1-yl]-N-propylpyridazine-3-carboxamide |
| 1M23 | 4-[4-[4-(3-Hydroxyphenyl)thiophene-2-carbonyl]piperazin-1-yl]-N-propylbenzamide |
| 1M24 | 4-[4-[4-(5-Hydroxypyridin-3-yl)thiophene-2-carbonyl]piperazin-1-yl]-N-propylbenzamide |
| 1M25 | 4-[4-[4-(3-Hydroxyphenyl)-3-methylbenzoyl]piperazin-1-yl]-N-propylbenzamide |
| 1M26 | 4-[4-[4-(5-Hydroxypyridin-3-yl)-3-methylbenzoyl]piperazin-1-yl]-N-propylbenzamide |
| 1M27 | 4-[4-[3-Fluoro-4-(3-hydroxyphenyl)benzoyl]piperazin-1-yl]-N-propylbenzamide |
| 1M28 | 4-[4-[3-Fluoro-4-(5-hydroxypyridin-3-yl)benzoyl]piperazin-1-yl]-N-propylbenzamide |
| 1M29 | 4-[4-[2-[2-(3-Hydroxyphenyl)ethynyl]benzoyl]piperazin-1-yl]-N-propylbenzamide |
| 1M30 | 4-[4-[2-[2-(5-Hydroxypyridin-3-yl)ethynyl]benzoyl]piperazin-1-yl]-N-propylbenzamide |
| 1M31 | 4-[4-[5-[2-(3-Hydroxyphenyl)ethynyl]pyridine-3-carbonyl]piperazin-1-yl]-N-propylbenzamide |
| 1M32 | 4-[4-[5-[2-(5-Hydroxypyridin-3-yl)ethynyl]pyridine-3-carbonyl]piperazin-1-yl]-N-propylbenzamide |
| 1M33 | 4-[4-[3-[2-(3-Hydroxyphenyl)phenyl]propanoyl]piperazin-1-yl]-N-propylbenzamide |
| 1M34 | 4-[4-[3-[2-(5-Hydroxypyridin-3-yl)phenyl]propanoyl]piperazin-1-yl]-N-propylbenzamide |
| 1M35 | 4-[4-[4-(3-Hydroxyphenyl)naphthalene-1-carbonyl]piperazin-1-yl]-N-propylbenzamide |
| 1M36 | 4-[4-[4-(5-Hydroxypyridin-3-yl)naphthalene-1-carbonyl]piperazin-1-yl]-N-propylbenzamide |
| 1M37 | 4-[4-[3-tert-Butyl-5-(3-hydroxyphenyl)benzoyl]piperazin-1-yl]-N-propylbenzamide |
| 1M38 | 4-[4-[3-tert-Butyl-5-(5-hydroxypyridin-3-yl)benzoyl]piperazin-1-yl]-N-propylbenzamide |
| 1M39 | 4-[4-[3-(3-Hydroxyphenyl)-5-(trifluoromethyl)benzoyl]piperazin-1-yl]-N-propylbenzamide |
| 1M40 | 4-[4-[3-(5-Hydroxypyridin-3-yl)-5-(trifluoromethyl)benzoyl]piperazin-1-yl]-N-propylbenzamide |
| 1M41 | 4-[4-[3-[2-(3-Hydroxyphenyl)ethynyl]-5-(trifluoromethyl)benzoyl]piperazin-1-yl]-N-propylbenzamide |
| 1M42 | 4-[4-[3-[2-(5-Hydroxypyridin-3-yl)ethynyl]-5-(trifluoromethyl)benzoyl]piperazin-1-yl]-N-propylbenzamide |
| 1M43 | 4-[4-[3-[4-(3-Hydroxyphenyl)pyrazol-1-yl]-5-(trifluoromethyl)benzoyl]piperazin-1-yl]-N-propylbenzamide |
| 1M44 | 4-[4-[3-[4-(5-Hydroxypyridin-3-yl)pyrazol-1-yl]-5-(trifluoromethyl)benzoyl]piperazin-1-yl]-N-propylbenzamide |
| 1M45 | 4-[4-[[2-(3-Hydroxyphenyl)phenyl]methyl]piperazin-1-yl]-N-propylbenzamide |
| 1M46 | 4-[4-[[2-(5-Hydroxypyridin-3-yl)phenyl]methyl]piperazin-1-yl]-N-propylbenzamide |
| 1M47 | 4-[4-[[4-(3-Hydroxyphenyl)-3-methylphenyl]methyl]piperazin-1-yl]-N-propylbenzamide |
| 1M48 | 4-[4-[[4-(5-Hydroxypyridin-3-yl)-3-methylphenyl]methyl]piperazin-1-yl]-N-propylbenzamide |

(continued)

| Mixture No. used | mixEI08-02R |
|---|---|
| Post-cleavage mixture No. | mixEJ02 |
| Compound No. in Table EJ02 | Compound name |
| 1M49 | 4-[4-[[2-Ethyl-4-(3-hydroxyphenyl)phenyl]methyl]piperazin-1-yl]-N-propylbenzamide |
| 1M50 | 4-[4-[[2-Ethyl-4-(5-hydroxypyridin-3-yl)phenyl]methyl]piperazin-1-yl]-N-propylbenzamide |
| 1M51 | 4-[4-[[3-Fluoro-4-(3-hydroxyphenyl)phenyl]methyl]piperazin-1-yl]-N-propylbenzamide |
| 1M52 | 4-[4-[[3-Fluoro-4-(5-hydroxypyridin-3-yl)phenyl]methyl]piperazin-1-yl]-N-propylbenzamide |
| 1M53 | 4-[4-[[4-(3-Hydroxyphenyl)-2-methoxyphenyl]methyl]piperazin-1-yl]-N-propylbenzamide |
| 1M54 | 4-[4-[[4-(5-Hydroxypyridin-3-yl)-2-methoxyphenyl]methyl]piperazin-1-yl]-N-propylbenzamide |
| 1M55 | 4-[4-[[3-Ethoxy-5-(3-hydroxyphenyl)phenyl]methyl]piperazin-1-yl]-N-propylbenzamide |
| 1M56 | 4-[4-[[3-Ethoxy-5-(5-hydroxypyridin-3-yl)phenyl]methyl]piperazin-1-yl]-N-propylbenzamide |
| 1M57 | 4-[4-[[3-(3-Hydroxyphenyl)-5-propan-2-yloxyphenyl]methyl]piperazin-1-yl]-N-propylbenzamide |
| 1M58 | 4-[4-[[3-(5-Hydroxypyridin-3-yl)-5-propan-2-yloxyphenyl]methyl]piperazin-1-yl]-N-propylbenzamide |
| 1M59 | 4-[4-[[4-(3-Hydroxyphenyl)-2,6-dimethoxyphenyl]methyl]piperazin-1-yl]-N-propylbenzamide |
| 1M60 | 4-[4-[[4-(5-Hydroxypyridin-3-yl)-2,6-dimethoxyphenyl]methyl]piperazin-1-yl]-N-propylbenzamide |
| 1M61 | 4-[4-[[3-(3-Hydroxyphenyl)-5-(trifluoromethyl)phenyl]methyl]piperazin-1-yl]-N-propylbenzamide |
| 1M62 | 4-[4-[[3-(5-Hydroxypyridin-3-yl)-5-(trifluoromethyl)phenyl]methyl]piperazin-1-yl]-N-propylbenzamide |
| 1M63 | 4-[4-[[3-(3-Hydroxyphenyl)-5-(trifluoromethoxy)phenyl]methyl]piperazin-1-yl]-N-propylbenzamide |
| 1M64 | 4-[4-[[3-(5-Hydroxypyridin-3-yl)-5-(trifluoromethoxy)phenyl]methyl]piperazin-1-yl]-N-propylbenzamide |
| 1M65 | 4-[4-[[4-(3-Hydroxyphenyl)naphthalen-1-yl]methyl]piperazin-1-yl]-N-propylbenzamide |
| 1M66 | 4-[4-[[4-(5-Hydroxypyridin-3-yl)naphthalen-1-yl]methyl]piperazin-1-yl]-N-propylbenzamide |
| 1M67 | 4-[4-[[4-(3-Hydroxyphenyl)thiophen-2-yl]methyl]piperazin-1-yl]-N-propylbenzamide |
| 1M68 | 4-[4-[[4-(5-Hydroxypyridin-3-yl)thiophen-2-yl]methyl]piperazin-1-yl]-N-propylbenzamide |
| 1M69 | 4-[4-[[2-[2-(3-Hydroxyphenyl)ethynyl]phenyl]methyl]piperazin-1-yl]-N-propylbenzamide |
| 1M70 | 4-[4-[[2-[2-(5-Hydroxypyridin-3-yl)ethynyl]phenyl]methyl]piperazin-1-yl]-N-propylbenzamide |
| 1M71 | 6-[4-[[2-(3-Hydroxyphenyl)phenyl]methyl]piperazin-1-yl]-N-propylpyridazine-3-carboxamide |
| 1M72 | 6-[4-[[2-(5-Hydroxypyridin-3-yl)phenyl]methyl]piperazin-1-yl]-N-propylpyridazine-3-carboxamide |
| 1M73 | 6-[4-[[4-(3-Hydroxyphenyl)-3-methylphenyl]methyl]piperazin-1-yl]-N-propylpyridazine-3-carboxamide |
| 1M74 | 6-[4-[[4-(5-Hydroxypyridin-3-yl)-3-methylphenyl]methyl]piperazin-1-yl]-N-propylpyridazine-3-carboxamide |
| 1M75 | 6-[-[[2-Ethyl-4-(3-hydroxyphenyl)phenyl]methyl]piperazin-1-yl]-N-propylpyridazine-3-carboxamide |
| 1M76 | 6-[4-[[2-Ethyl-4-(5-hydroxypyridin-3-yl)phenyl]methyl]piperazin-1-yl]-N-propylpyridazine-3-carboxamide |

(continued)

| Mixture No. used | mixEI08-02R |
|---|---|
| Post-cleavage mixture No. | mixEJ02 |
| Compound No. in Table EJ02 | Compound name |
| 1M77 | 6-[4-[[3-Fluoro-4-(3-hydroxyphenyl)phenyl]methyl]piperazin-1-yl]-N-propylpyridazine-3-carboxamide |
| 1M78 | 6-[4-[[3-Fluoro-4-(5-hydroxypyridin-3-yl)phenyl]methyl]piperazin-1-yl]-N-propylpyridazine-3-carboxamide |
| 1M79 | 6-[4-[[4-(3-Hydroxyphenyl)-2-methoxyphenyl]methyl]piperazin-1-yl]-N-propylpyridazine-3-carboxamide |
| 1M80 | 6-[4-[[4-(5-Hydroxypyridin-3-yl)-2-methoxyphenyl]methyl]piperazin-1-yl]-N-propylpyridazine-3-carboxamide |
| 1M81 | 6-[4-[[3-Ethoxy-5-(3-hydroxyphenyl)phenyl]methyl]piperazin-1-yl]-N-propylpyridazine-3-carboxamide |
| 1M82 | 6-[4-[[3-Ethoxy-5-(5-hydroxypyridin-3-yl)phenyl]methyl]piperazin-1-yl]-N-propylpyridazine-3-carboxamide |
| 1M83 | 6-[4-[[3-(3-Hydroxyphenyl)-5-propan-2-yloxyphenyl]methyl]piperazin-1-yl]-N-propylpyridazine-3-carboxamide |
| 1M84 | 6-[4-[[3-(5-Hydroxypyridin-3-yl)-5-propan-2-yloxyphenyl]methyl]piperazin-1-yl]-N-propylpyridazine-3-carboxamide |
| 1M85 | 6-[4-[[4-(3-Hydroxyphenyl)-2,6-dimethoxyphenyl]methyl]piperazin-1-yl]-N-propylpyridazine-3-carboxamide |
| 1M86 | 6-[4-[[4-(5-Hydroxypyridin-3-yl)-2,6-dimethoxyphenyl]methyl]piperazin-1-yl]-N-propylpyridazine-3-carboxamide |
| 1M87 | 6-[4-[[3-(3-Hydroxyphenyl)-5-(trifluoromethyl)phenyl]methyl]piperazin-1-yl]-N-propylpyridazine-3-carboxamide |
| 1M88 | 6-[4-[[3-(5-Hydroxypyridin-3-yl)-5-(trifluoromethyl)phenyl]methyl]piperazin-1-yl]-N-propylpyridazine-3-carboxamide |
| 1M89 | 6-[4-[[3-(3-Hydroxyphenyl)-5-(trifluoromethoxy)phenyl]methyl]piperazin-1-yl]-N-propylpyridazine-3-carboxamide |
| 1M90 | 6-[4-[[3-(5-Hydroxypyridin-3-yl)-5-(trifluoromethoxy)phenyl]methyl]piperazin-1-yl]-N-propylpyridazine-3- carboxamide |
| 1M91 | 6-[4-[[4-(3-Hydroxyphenyl)naphthalen-1-yl]methyl]piperazin-1-yl]-N-propylpyridazine-3-carboxamide |
| 1M92 | 6-[4-[[4-(5-Hydroxypyridin-3-yl)naphthalen-1-yl]methyl]piperazin-1-yl]-N-propylpyridazine-3-carboxamide |
| 1M93 | 6-[4-[[4-(3-Hydroxyphenyl)thiophen-2-yl]methyl]piperazin-1-yl]-N-propylpyridazine-3-carboxamide |
| 1M94 | 6-[4-[[4-(5-Hydroxypyridin-3-yl)thiophen-2-yl]methyl]piperazin-1-yl]-N-propylpyridazine-3-carboxamide |
| 1M95 | 6-[4-[[2-[2-(3-Hydroxyphenyl)ethynyl]phenyl]methyl]piperazin-1-yl]-N-propylpyridazine-3-carboxamide |
| 1M96 | 6-[4-[[2-[2-(5-Hydroxypyridin-3-yl)ethynyl]phenyl]methyl]piperazin-1-yl]-N-propylpyridazine-3-carboxamide |

(continued)

| Mixture No. used | mixEI08-02R |
|---|---|
| Post-cleavage mixture No. | mixEJ02 |
| Compound No. in Table EJ02 | Compound name |
| 1M97 | 4-[4-[1-[4-(3-Hydroxyphenyl)phenyl]ethyl]piperazin-1-yl]-N-propylbenzamide |
| 1M98 | 4-[4-[1-[4-(5-Hydroxypyridin-3-yl)phenyl]ethyl]piperazin-1-yl]-N-propylbenzamide |
| 1M99 | 4-[4-[1-[2-Fluoro-4-(3 -hydroxyphenyl)phenyl]ethyl]piperazin-1 -yl] -N-propylbenzamide |
| 1M100 | 4-[4-[1-[2-Fluoro-4-(5-hydroxypyridin-3-yl)phenyl]ethyl]piperazin-1-yl]-N-propylbenzamide |

[Table 74]

[Table EU03]

| Mixture No. used | mixEI09-02R |
|---|---|
| Post-cleavage mixture No. | mixEJ03 |
| Compound No. in Table EJ03 | Compound name |
| 1K01 | 6-[4-[4-(3-Hydroxyphenyl)thiophene-2-carbonyl]piperazin-1-yl]-N-propylpyridazine-3-carboxamide |
| 1K02 | 6-[4-[4-(5-Hydroxypyridin-3-yl)thiophene-2-carbonyl]piperazin-1-yl]-N-propylpyridazine-3-carboxamide |
| 1K03 | 6-[4-[4-(3-Hydroxyphenyl)-3-methylbenzoyl]piperazin-1-yl]-N-propylpyridazine-3-carboxamide |
| 1K04 | 6-[4-[4-(5-Hydroxypyridin-3-yl)-3-methylbenzoyl]piperazin-1-yl]-N-propylpyridazine-3-carboxamide |
| 1K05 | 6-[4-[3-Fluoro-4-(3-hydroxyphenyl)benzoyl]piperazin-1-yl]-N-propylpyridazine-3-carboxamide |
| 1K06 | 6-[4-[3-Fluoro-4-(5-hydroxypyridin-3-yl)benzoyl]piperazin-1-yl]-N-propylpyridazine-3-carboxamide |
| 1K07 | 6-[4-[2-[2-(3-Hydroxyphenyl)ethynyl]benzoyl]piperazin-1-yl]-N-propylpyridazine-3-carboxamide |
| 1K08 | 6-[4-[2-[2-(5-Hydroxypyridin-3-yl)ethynyl]benzoyl]piperazin-1-yl]-N-propylpyridazine-3-carboxamide |
| 1K09 | 6-[4-[5-[2-(3-Hydroxyphenyl)ethynyl]pyridine-3-carbonyl]piperazin-1-yl]-N-propylpyridazine-3-carboxamide |
| 1K10 | 6-[-[5-[2-(5-Hydroxypyridin-3-yl)ethynyl]pyridine-3-carbonyl]piperazin-1-yl]-N-propylpyridazine-3-carboxamide |
| 1K11 | 6-[4-[3-[2-(3-Hydroxyphenyl)phenyl]propanoyl]piperazin-1-yl]-N-propylpyridazine-3-carboxamide |
| 1K12 | 6-[4-[3-[2-(5-Hydroxypyridin-3-yl)phenyl]propanoyl]piperazin-1-yl]-N-propylpyridazine-3-carboxamide |
| 1K13 | 6-[4-[4-(3-Hydroxyphenyl)naphthalene-1-carbonyl]piperazin-1-yl]-N-propylpyridazine-3-carboxamide |

(continued)

| Mixture No. used | mixEI09-02R |
|---|---|
| Post-cleavage mixture No. | mixEJ03 |
| Compound No. in Table EJ03 | Compound name |
| 1K14 | 6-[4-[4-(5-Hydroxypyridin-3-yl)naphthalene-1-carbonyl]piperazin-1-yl]-N-propylpyridazine-3-carboxamide |
| 1K15 | 6-[4-[3-tert-Butyl-5-(3-hydroxyphenyl)benzoyl]piperazin-1-yl]-N- propylpyridazine-3-carboxamide |
| 1K16 | 6-[4-[3-tert-Butyl-5-(5-hydroxypyridin-3-yl)benzoyl]piperazin-1-yl]-N-propylpyridazine-3-carboxamide |
| 1K17 | 6-[4-[3-(3-Hydroxyphenyl)-5-(trifluoromethyl)benzoyl]piperazin-1-yl]-N-propylpyridazine-3-carboxamide |
| 1K18 | 6-[4-[3-(5-Hydroxypyridin-3-yl)-5-(trifluoromethyl)benzoyl]piperazin-1-yl]-N-propylpyridazine-3-carboxamide |
| 1K19 | 6-[4-[3-[2-(3-Hydroxyphenyl)ethynyl]-5-(trifluoromethyl)benzoyl]piperazin-1-yl]-N-propylpyridazine-3-carboxamide |
| 1K20 | 6-[4-[3-[2-(5-Hydroxypyridin-3-yl)ethynyl]-5-(trifluoromethyl)benzoyl]piperazin-1-yl]-N-propylpyridazine-3-carboxamide |
| 1K21 | 6-[4-[3-[4-(3-Hydroxyphenyl)pyrazol-1-yl]-5-(trifluoromethyl)benzoyl]piperazin-1-yl]-N-propylpyridazine-3-carboxamide |
| 1K22 | 6-[4-[3-[4-(5-Hydroxypyridin-3-yl)pyrazol-1-yl]-5-(trifluoromethyl)benzoyl]piperazin-1-yl]-N-propylpyridazine-3-carboxamide |
| 1K23 | 4-[4-[4-(3-Hydroxyphenyl)thiophene-2-carbonyl]piperazin-1-yl]-N-propylbenzamide |
| 1K24 | 4-[4-[4-(5-Hydroxypyridin-3-yl)thiophene-2-carbonyl]piperazin-1-yl]-N-propylbenzamide |
| 1K25 | 4-[4-[4-(3-Hydroxyphenyl)-3-methylbenzoyl]piperazin-1-yl]-N-propylbenzamide |
| 1K26 | 4-[4-[4-(5-Hydroxypyridin-3-yl)-3-methylbenzoyl]piperazin-1-yl]-N-propylbenzamide |
| 1K27 | 4-[4-[3-Fluoro-4-(3-hydroxyphenyl)benzoyl]piperazin-1-yl]-N-propylbenzamide |
| 1K28 | 4-[4-[3-Fluoro-4-(5-hydroxypyridin-3-yl)benzoyl]piperazin-1-yl]-N-propylbenzamide |
| 1K29 | 4-[4-[2-[2-(3-Hydroxyphenyl)ethynyl]benzoyl]piperazin-1-yl]-N-propylbenzamide |
| 1K30 | 4-[4-[2-[2-(5-Hydroxypyridin-3-yl)ethynyl]benzoyl]piperazin-1-yl]-N-propylbenzamide |
| 1K31 | 4-[4-[5-[2-(3-Hydroxyphenyl)ethynyl]pyridine-3-carbonyl]piperazin-1-yl]-N-propylbenzamide |
| 1K32 | 4-[4-[5-[2-(5-Hydroxypyridin-3-yl)ethynyl]pyridine-3-carbonyl]piperazin-1-yl]-N-propylbenzamide |
| 1K33 | 4-[4-[3-[2-(3-Hydroxyphenyl)phenyl]propanoyl]piperazin-1-yl]-N-propylbenzamide |
| 1K34 | 4-[4-[3-[2-(5-Hydroxypyridin-3-yl)phenyl]propanoyl]piperazin-1-yl]-N-propylbenzamide |
| 1K35 | 4-[4-[4-(3-Hydroxyphenyl)naphthalene-1-carbonyl]piperazin-1-yl]-N-propylbenzamide |
| 1K36 | 4-[4-[4-(5-Hydroxypyridin-3-yl)naphthalene-1-carbonyl]piperazin-1-yl]-N-propylbenzamide |
| 1K37 | 4-[4-[3-tert-Butyl-5-(3-hydroxyphenyl)benzoyl]piperazin-1-yl]-N-propylbenzamide |
| 1K38 | 4-[4-[3-tert-Butyl-5-(5-hydroxypyridin-3-yl)benzoyl]piperazin-1-yl]-N-propylbenzamide |
| 1K39 | 4-[4-[3-(3-Hydroxyphenyl)-5-(trifluoromethyl)benzoyl]piperazin-1-yl]-N-propylbenzamide |
| 1K40 | 4-[4-[3-(5-Hydroxypyridin-3-yl)-5-(trifluoromethyl)benzoyl]piperazin-1-yl]-N-propylbenzamide |

(continued)

| Mixture No. used | mixEI09-02R |
|---|---|
| Post-cleavage mixture No. | mixEJ03 |
| Compound No. in Table EJ03 | Compound name |
| 1K41 | 4-[4-[3-[2-(3-Hydroxyphenyl)ethynyl]-5-(trifluoromethyl)benzoyl]piperazin-1-yl]-N-propylbenzamide |
| 1K42 | 4-[4-[3-[2-(5-Hydroxypyridin-3-yl)ethynyl]-5-(trifluoromethyl)benzoyl]piperazin-1-yl]-N-propylbenzamide |
| 1K43 | 4-[4-[3-[4-(3-Hydroxyphenyl)pyrazol-1-yl]-5-(trifluoromethyl)benzoyl]piperazin-1-yl]-N-propylbenzamide |
| 1K44 | 4-[4-[3-[4-(5-Hydroxypyridin-3-yl)pyrazol-1-yl]-5-(trifluoromethyl)benzoyl]piperazin-1-yl]-N-propylbenzamide |
| 1K45 | 4-[4-[[2-(3-Hydroxyphenyl)phenyl]methyl]piperazin-1-yl]-N-propylbenzamide |
| 1K46 | 4-[4-[[2-(5-Hydroxypyridin-3-yl)phenyl]methyl]piperazin-1-yl]-N-propylbenzamide |
| 1K47 | 4-[4-[[4-(3-Hydroxyphenyl)-3-methylphenyl]methyl]piperazin-1-yl]-N-propylbenzamide |
| 1K48 | 4-[4-[[4-(5-Hydroxypyridin-3-yl)-3-methylphenyl]methyl]piperazin-1-yl]-N-propylbenzamide |
| 1K49 | 4-[-[[2-Ethyl-4-(3-hydroxyphenyl)phenyl]methyl]piperazin-1-yl]-N-propylbenzamide |
| 1K50 | 4-[4-[[2-Ethyl-4-(5-hydroxypyridin-3-yl)phenyl]methyl]piperazin-1-yl]-N-propylbenzamide |
| 1K51 | 4-[4-[[3-Fluoro-4-(3-hydroxyphenyl)phenyl]methyl]piperazin-1-yl]-N-propylbenzamide |
| 1K52 | 4-[4-[[3-Fluoro-4-(5-hydroxypyridin-3-yl)phenyl]methyl]piperazin-1-yl]-N-propylbenzamide |
| 1K53 | 4-[4-[[4-(3-Hydroxyphenyl)-2-methoxyphenyl]methyl]piperazin-1-yl]-N-propylbenzamide |
| 1K54 | 4-[4-[[4-(5-Hydroxypyridin-3-yl)-2-methoxyphenyl]methyl]piperazin-1-yl]-N-propylbenzamide |
| 1K55 | 4-[4-[[3-Ethoxy-5-(3-hydroxyphenyl)phenyl]methyl]piperazin-1-yl]-N-propylbenzamide |
| 1K56 | 4-[4-[[3-Ethoxy-5-(5-hydroxypyridin-3-yl)phenyl]methyl]piperazin-1-yl]-N-propylbenzamide |
| 1K57 | 4-[4-[[3-(3-Hydroxyphenyl)-5-propan-2-yloxyphenyl]methyl]piperazin-1-yl]-N-propylbenzamide |
| 1K58 | 4-[4-[[3-(5-Hydroxypyridin-3-yl)-5-propan-2-yloxyphenyl]methyl]piperazin-1-yl]-N-propylbenzamide |
| 1K59 | 4-[4-[[4-(3-Hydroxyphenyl)-2,6-dimethoxyphenyl]methyl]piperazin-1-yl]-N-propylbenzamide |
| 1K60 | 4-[4-[[4-(5-Hydroxypyridin-3-yl)-2,6-dimethoxyphenyl]methyl]piperazin-1-yl]-N-propylbenzamide |
| 1K61 | 4-[4-[[3-(3-Hydroxyphenyl)-5-(trifluoromethyl)phenyl]methyl]piperazin-1-yl]-N-propylbenzamide |
| 1K62 | 4-[4-[[3-(5-Hydroxypyridin-3-yl)-5-(trifluoromethyl)phenyl]methyl]piperazin-1-yl]-N-propylbenzamide |
| 1K63 | 4-[4-[[3-(3-Hydroxyphenyl)-5-(trifluoromethoxy)phenyl]methyl]piperazin-1-yl]-N-propylbenzamide |
| 1K64 | 4-[4-[[3-(5-Hydroxypyridin-3-yl)-5-(trifluoromethoxy)phenyl]methyl]piperazin-1-yl]-N-propylbenzamide |
| 1K65 | 4-[4-[[4-(3-Hydroxyphenyl)naphthalen-1-yl]methyl]piperazin-1-yl]-N-propylbenzamide |
| 1K66 | 4-[4-[[4-(5-Hydroxypyridin-3-yl)naphthalen-1-yl]methyl]piperazin-1-yl]-N-propylbenzamide |
| 1K67 | 4-[4-[[4-(3-Hydroxyphenyl)thiophen-2-yl]methyl]piperazin-1-yl]-N-propylbenzamide |
| 1K68 | 4-[4-[[4-(5-Hydroxypyridin-3-yl)thiophen-2-yl]methyl]piperazin-1-yl]-N-propylbenzamide |
| 1K69 | 4-[4-[[2-[2-(3-Hydroxyphenyl)ethynyl]phenyl]methyl]piperazin-1-yl]-N-propylbenzamide |

(continued)

| Mixture No. used | mixEI09-02R |
|---|---|
| Post-cleavage mixture No. | mixEJ03 |
| Compound No. in Table EJ03 | Compound name |
| 1K70 | 4-[4-[[2-[2-(5-Hydroxypyridin-3-yl)ethynyl]phenyl]methyl]piperazin-1-yl]-N-propylbenzamide |
| 1K71 | 6-[4-[[2-(3-Hydroxyphenyl)phenyl]methyl]piperazin-1-yl]-N-propylpyridazine-3-carboxamide |
| 1K72 | 6-[4-[[2-(5-Hydroxypyridin-3-yl)phenyl]methyl]piperazin-1-yl]-N-propylpyridazine-3-carboxamide |
| 1K73 | 6-[4-[[4-(3-Hydroxyphenyl)-3-methylphenyl]methyl]piperazin-1-yl]-N-propylpyridazine-3-carboxamide |
| 1K74 | 6-[4-[[4-(5-Hydroxypyridin-3-yl)-3-methylphenyl]methyl]piperazin-1-yl]-N-propylpyridazine-3-carboxamide |
| 1K75 | 6-[-[[2-Ethyl-4-(3-hydroxyphenyl)phenyl]methyl]piperazin-1-yl]-N-propylpyridazine-3-carboxamide |
| 1K76 | 6-[4-[[2-Ethyl-4-(5-hydroxypyridin-3-yl)phenyl]methyl]piperazin-1-yl]-N-propylpyridazine-3-carboxamide |
| 1K77 | 6-[4-[[3-Fluoro-4-(3-hydroxyphenyl)phenyl]methyl]piperazin-1-yl]-N-propylpyridazine-3-carboxamide |
| 1K78 | 6-[4-[[3-Fluoro-4-(5-hydroxypyridin-3-yl)phenyl]methyl]piperazin-1-yl]-N-propylpyridazine-3-carboxamide |
| 1K79 | 6-[4-[[4-(3-Hydroxyphenyl)-2-methoxyphenyl]methyl]piperazin-1-yl]-N-propylpyridazine-3-carboxamide |
| 1K80 | 6-[4-[[4-(5-Hydroxypyridin-3-yl)-2-methoxyphenyl]methyl]piperazin-1-yl]-N-propylpyridazine-3-carboxamide |
| 1K81 | 6-[4-[[3-Ethoxy-5-(3-hydroxyphenyl)phenyl]methyl]piperazin-1-yl]-N-propylpyridazine-3-carboxamide |
| 1K82 | 6-[4-[[3-Ethoxy-5-(5-hydroxypyridin-3-yl)phenyl]methyl]piperazin-1-yl]-N-propylpyridazine-3-carboxamide |
| 1K83 | 6-[4-[[3-(3-Hydroxyphenyl)-5-propan-2-yloxyphenyl]methyl]piperazin-1-yl]-N-propylpyridazine-3-carboxamide |
| 1K84 | 6-[4-[[3-(5-Hydroxypyridin-3-yl)-5-propan-2-yloxyphenyl]methyl]piperazin-1-yl]-N-propylpyridazine-3-carboxamide |
| 1K85 | 6-[4-[[4-(3-Hydroxyphenyl)-2,6-dimethoxyphenyl]methyl]piperazin-1-yl]-N-propylpyridazine-3-carboxamide |
| 1K86 | 6-[4-[[4-(5-Hydroxypyridin-3-yl)-2,6-dimethoxyphenyl]methyl]piperazin-1-yl]-N-propylpyridazine-3-carboxamide |
| 1K87 | 6-[4-[[3-(3-Hydroxyphenyl)-5-(trifluoromethyl)phenyl]methyl]piperazin-1-yl]-N-propylpyridazine-3-carboxamide |
| 1K88 | 6-[4-[[3-(5-Hydroxypyridin-3-yl)-5-(trifluoromethyl)phenyl]methyl]piperazin-1-yl]-N-propylpyridazine-3-carboxamide |
| 1K89 | 6-[4-[[3-(3-Hydroxyphenyl)-5-(trifluoromethoxy)phenyl]methyl]piperazin-1-yl]-N-propylpyridazine-3-carboxamide |
| 1K90 | 6-[4-[[3-(5-Hydroxypyridin-3-yl)-5-(trifluoromethoxy)phenyl]methyl]piperazin-1-yl]-N-propylpyridazine-3-carboxamide |

(continued)

| Mixture No. used | mixEI09-02R |
|---|---|
| Post-cleavage mixture No. | mixEJ03 |
| Compound No. in Table EJ03 | Compound name |
| 1K91 | 6-[4-[[4-(3-Hydroxyphenyl)naphthalen-1-yl]methyl]piperazin-1-yl]-N-propylpyridazine-3-carboxamide |
| 1K92 | 6-[4-[[4-(5-Hydroxypyridin-3-yl)naphthalen-1-yl]methyl]piperazin-1-yl]-N-propylpyridazine-3-carboxamide |
| 1K93 | 6-[4-[[4-(3-Hydroxyphenyl)thiophen-2-yl]methyl]piperazin-1-yl]-N-propylpyridazine-3-carboxamide |
| 1K94 | 6-[4-[[4-(5-Hydroxypyridin-3-yl)thiophen-2-yl]methyl]piperazin-1-yl]-N-propylpyridazine-3-carboxamide |
| 1K95 | 6-[4-[[2-[2-(3-Hydroxyphenyl)ethynyl]phenyl]methyl]piperazin-1-yl]-N-propylpyridazine-3-carboxamide |
| 1K96 | 6-[4-[[2-[2-(5-Hydroxypyridin-3-yl)ethynyl]phenyl]methyl]piperazin-1-yl]-N-propylpyridazine-3-carboxamide |
| 1K97 | 4-[4-[1-[4-(3-Hydroxyphenyl)phenyl]ethyl]piperazin-1-yl]-N-propylbenzamide |
| 1K98 | 4-[4-[1-[4-(5-Hydroxypyridin-3-yl)phenyl]ethyl]piperazin-1-yl]-N-propylbenzamide |
| 1K99 | 4-[4-[1-[2-Fluoro-4-(3-hydroxyphenyl)phenyl]ethyl]piperazin-1-yl]-N-propylbenzamide |
| 1K100 | 4-[4-[1-[2-Fluoro-4-(5-hydroxypyridin-3-yl)phenyl]ethyl]piperazin-1-yl]-N-propylbenzamide |

[Table 75]

[Table EU04]

| Mixture No. used | mixEI10-02R |
|---|---|
| Post-cleavage mixture No. | mixEJ04 |
| Compound No. in Table EJ04 | Compound name |
| 1O01 | 6-[4-[4-(3-Hydroxyphenyl)thiophene-2-carbonyl]piperazin-1-yl]-N-(4-methoxyphenyl)pyridazine-3-carboxamide |
| 1O02 | 6-[4-[4-(5-Hydroxypyridin-3-yl)thiophene-2-carbonyl]piperazin-1-yl]-N-(4-methoxyphenyl)pyridazine-3-carboxamide |
| 1O03 | 6-[4-[4-(3-Hydroxyphenyl)-3-methylbenzoyl]piperazin-1-yl]-N-(4-methoxyphenyl)pyridazine-3-carboxamide |
| 1O04 | 6-[4-[4-(5-Hydroxypyridin-3-yl)-3-methylbenzoyl]piperazin-1-yl]-N-(4-methoxyphenyl)pyridazine-3-carboxamide |
| 1O05 | 6-[4-[3-Fluoro-4-(3-hydroxyphenyl)benzoyl]piperazin-1-yl]-N-(4-methoxyphenyl)pyridazine-3-carboxamide |
| 1O06 | 6-[4-[3-Fluoro-4-(5-hydroxypyridin-3-yl)benzoyl]piperazin-1-yl]-N-(4-methoxyphenyl)pyridazine-3-carboxamide |

(continued)

| Mixture No. used | mixEI10-02R |
|---|---|
| Post-cleavage mixture No. | mixEJ04 |
| Compound No. in Table EJ04 | Compound name |
| 1O07 | 6-[4-[2-[2-(3-Hydroxyphenyl)ethynyl]benzoyl]piperazin-1-yl]-N-(4-methoxyphenyl)pyridazine-3-carboxamide |
| 1O08 | 6-[4-[2-[2-(5-Hydroxypyridin-3-yl)ethynyl]benzoyl]piperazin-1-yl]-N-(4-methoxyphenyl)pyridazine-3-carboxamide |
| 1O09 | 6-[4-[5-[2-(3-Hydroxyphenyl)ethynyl]pyridine-3-carbonyl]piperazin-1-yl]-N-(4-methoxyphenyl)pyridazine-3-carboxamide |
| 1O10 | -[4-[5-[2-(5-Hydroxypyridin-3-yl)ethynyl]pyridine-3-carbonyl]piperazin-1-yl]-N-(4-methoxyphenyl)pyridazine-3-carboxamide |
| 1O11 | 6-[4-[3-[2-(3-Hydroxyphenyl)phenyl]propanoyl]piperazin-1-yl]-N-(4-methoxyphenyl)pyridazine-3-carboxamide |
| 1O12 | 6-[4-[3-[2-(5-Hydroxypyridin-3-yl)phenyl]propanoyl]piperazin-1-yl]-N-(4-methoxyphenyl)pyridazine-3-carboxamide |
| 1O13 | 6-[4-[4-(3-Hydroxyphenyl)naphthalene-1-carbonyl]piperazin-1-yl]-N-(4-methoxyphenyl)pyridazine-3-carboxamide |
| 1O14 | 6-[4-[4-(5-Hydroxypyridin-3-yl)naphthalene-1-carbonyl]piperazin-1-yl]-N-(4-methoxyphenyl)pyridazine-3-carboxamide |
| 1O15 | 6-[4-[3-tert-Butyl-5-(3-hydroxyphenyl)benzoyl]piperazin-1-yl]-N-(4-methoxyphenyl)pyridazine-3-carboxamide |
| 1O16 | 6-[4-[3-tert-Butyl-5-(5-hydroxypyridin-3-yl)benzoyl]piperazin-1-yl]-N-(4-methoxyphenyl)pyridazine-3-carboxamide |
| 1O17 | 6-[4-[3-(3-Hydroxyphenyl)-5-(trifluoromethyl)benzoyl]piperazin-1-yl]-N-(4-methoxyphenyl)pyridazine-3-carboxamide |
| 1O18 | 6-[4-[3-(5-Hydroxypyridin-3-yl)-5-(trifluoromethyl)benzoyl]piperazin-1-yl]-N-(4-methoxyphenyl)pyridazine-3-carboxamide |
| 1O19 | 6-[4-[3-[2-(3-Hydroxyphenyl)ethynyl]-5-(trifluoromethyl)benzoyl]piperazin-1-yl]-N-(4-methoxyphenyl)pyridazine-3-carboxamide |
| 1O20 | 6-[4-[3-[2-(5-Hydroxypyridin-3-yl)ethynyl]-5-(trifluoromethyl)benzoyl]piperazin-1-yl]-N-(4-methoxyphenyl)pyridazine-3- carboxamide |
| 1O21 | 6-[4-[3-[4-(3-Hydroxyphenyl)pyrazol-1-yl]-5-(trifluoromethyl)benzoyl]piperazin-1-yl]-N-(4-methoxyphenyl)pyridazine-3- carboxamide |
| 1O22 | 6-[4-[3-[4-(5-Hydroxypyridin-3-yl)pyrazol-1-yl]-5-(trifluoromethyl)benzoyl]piperazin-1-yl]-N-(4-methoxyphenyl)pyridazine-3- carboxamide |
| 1O23 | 4-[4-[4-(3-Hydroxyphenyl)thiophene-2-carbonyl]piperazin-1-yl]-N-(4-methoxyphenyl)benzamide |
| 1O24 | 4-[4-[4-(5-Hydroxypyridin-3-yl)thiophene-2-carbonyl]piperazin-1-yl]-N-(4-methoxyphenyl)benzamide |
| 1O25 | 4-[4-[4-(3-Hydroxyphenyl)-3-methylbenzoyl]piperazin-1-yl]-N-(4-methoxyphenyl)benzamide |
| 1O26 | 4-[4-[4-(5-Hydroxypyridin-3-yl)-3-methylbenzoyl]piperazin-1-yl]-N-(4-methoxyphenyl)benzamide |
| 1O27 | 4-[4-[3-Fluoro-4-(3-hydroxyphenyl)benzoyl]piperazin-1-yl]-N-(4-methoxyphenyl)benzamide |
| 1O28 | 4-[4-[3-Fluoro-4-(5-hydroxypyridin-3-yl)benzoyl]piperazin-1-yl]-N-(4-methoxyphenyl)benzamide |

(continued)

| Mixture No. used | mixEI10-02R |
|---|---|
| Post-cleavage mixture No. | mixEJ04 |
| Compound No. in Table EJ04 | Compound name |
| 1O29 | 4-[4-[2-[2-(3-Hydroxyphenyl)ethynyl]benzoyl]piperazin-1-yl]-N-(4-methoxyphenyl)benzamide |
| 1O30 | 4-[4-[2-[2-(5-Hydroxypyridin-3-yl)ethynyl]benzoyl]piperazin-1-yl]-N-(4-methoxyphenyl)benzamide |
| 1O31 | 4-[4-[5-[2-(3-Hydroxyphenyl)ethynyl]pyridine-3-carbonyl]piperazin-1-yl]-N-(4-methoxyphenyl)benzamide |
| 1O32 | -[4-[5-[2-(-Hydroxypyridin-3-yl)ethynyl]pyridine-3-carbonyl]piperazin-1-yl]-N-(4-methoxyphenyl)benzamide |
| 1O33 | 4-[4-[3-[2-(3-Hydroxyphenyl)phenyl]propanoyl]piperazin-1-yl]-N-(4-methoxyphenyl)benzamide |
| 1O34 | 4-[4-[3-[2-(5-Hydroxypyridin-3-yl)phenyl]propanoyl]piperazin-1-yl]-N-(4-methoxyphenyl)benzamide |
| 1O35 | 4-[4-[4-(3-Hydroxyphenyl)naphthalene-1-carbonyl]piperazin-1-yl]-N-(4-methoxyphenyl)benzamide |
| 1O36 | 4-[4-[4-(5-Hydroxypyridin-3-yl)naphthalene-1-carbonyl]piperazin-1-yl]-N-(4-methoxyphenyl)benzamide |
| 1O37 | 4-[4-[3-tert-Butyl-5-(3-hydroxyphenyl)benzoyl]piperazin-1-yl]-N-(4-methoxyphenyl)benzamide |
| 1O38 | 4-[4-[3-tert-Butyl-5-(5-hydroxypyridin-3-yl)benzoyl]piperazin-1-yl]-N-(4-methoxyphenyl)benzamide |
| 1O39 | 4-[4-[3-(3-Hydroxyphenyl)-5-(trifluoromethyl)benzoyl]piperazin-1-yl]-N-(4-methoxyphenyl)benzamide |
| 1O40 | 4-[4-[3-(5-Hydroxypyridin-3-yl)-5-(trifluoromethyl)benzoyl]piperazin-1-yl]-N-(4-methoxyphenyl)benzamide |
| 1O41 | 4-[4-[3-[2-(3-Hydroxyphenyl)ethynyl]-5-(trifluoromethyl)benzoyl]piperazin-1-yl]-N-(4-methoxyphenyl)benzamide |
| 1O42 | 4-[4-[3-[2-(5-Hydroxypyridin-3-yl)ethynyl]-5-(trifluoromethyl)benzoyl]piperazin-1-yl]-N-(4-methoxyphenyl)benzamide |
| 1O43 | 4-[4-[3-[4-(3-Hydroxyphenyl)pyrazol-1-yl]-5-(trifluoromethyl)benzoyl]piperazin-1-yl]-N-(4-methoxyphenyl)benzamide |
| 1O44 | 4-[4-[3-[4-(5-Hydroxypyridin-3-yl)pyrazol-1-yl]-5-(trifluoromethyl)benzoyl]piperazin-1-yl]-N-(4-methoxyphenyl)benzamide |
| 1O45 | 4-[4-[[2-(3-Hydroxyphenyl)phenyl]methyl]piperazin-1-yl]-N-(4-methoxyphenyl)benzamide |
| 1O46 | 4-[4-[[2-(5-Hydroxypyridin-3-yl)phenyl]methyl]piperazin-1-yl]-N-(4-methoxyphenyl)benzamide |
| 1O47 | 4-[4-[[4-(3-Hydroxyphenyl)-3-methylphenyl]methyl]piperazin-1-yl]-N-(4-methoxyphenyl)benzamide |
| 1O48 | 4-[4-[[4-(5-Hydroxypyridin-3-yl)-3-methylphenyl]methyl]piperazin-1-yl]-N-(4-methoxyphenyl)benzamide |
| 1O49 | 4-[4-[[2-Ethyl-4-(3-hydroxyphenyl)phenyl]methyl]piperazin-1-yl]-N-(4-methoxyphenyl)benzamide |
| 1O50 | 4-[4-[[2-Ethyl-4-(5-hydroxypyridin-3-yl)phenyl]methyl]piperazin-1-yl]-N-(4-methoxyphenyl)benzamide |

(continued)

| Mixture No. used | mixEI10-02R |
|---|---|
| Post-cleavage mixture No. | mixEJ04 |
| Compound No. in Table EJ04 | Compound name |
| 1O51 | 4-[4-[[3-Fluoro-4-(3-hydroxyphenyl)phenyl]methyl]piperazin-1-yl]-N-(4-methoxyphenyl)benzamide |
| 1O52 | 4-[4-[[3-Fluoro-4-(5-hydroxypyridin-3-yl)phenyl]methyl]piperazin-1-yl]-N-(4-methoxyphenyl)benzamide |
| 1O53 | 4-[4-[[4-(3-Hydroxyphenyl)-2-methoxyphenyl]methyl]piperazin-1-yl]-N-(4-methoxyphenyl)benzamide |
| 1O54 | 4-[4-[[4-(5-Hydroxypyridin-3-yl)-2-methoxyphenyl]methyl]piperazin-1-yl]-N-(4-methoxyphenyl)benzamide |
| 1O55 | 4-[4-[[3-Ethoxy-5-(3-hydroxyphenyl)phenyl]methyl]piperazin-1-yl]-N-(4-methoxyphenyl)benzamide |
| 1O56 | 4-[4-[[3-Ethoxy-5-(5-hydroxypyridin-3-yl)phenyl]methyl]piperazin-1-yl]-N-(4-methoxyphenyl)benzamide |
| 1O57 | 4-[4-[[3-(3-Hydroxyphenyl)-5-propan-2-yloxyphenyl]methyl]piperazin-1-yl]-N-(4-methoxyphenyl)benzamide |
| 1O58 | 4-[4-[[3-(5-Hydroxypyridin-3-yl)-5-propan-2-yloxyphenyl]methyl]piperazin-1-yl]-N-(4-methoxyphenyl)benzamide |
| 1O59 | 4-[4-[[4-(3-Hydroxyphenyl)-2,6-dimethoxyphenyl]methyl]piperazin-1-yl]-N-(4-methoxyphenyl)benzamide |
| 1O60 | 4-[4-[[4-(5-Hydroxypyridin-3-yl)-2,6-dimethoxyphenyl]methyl]piperazin-1-yl]-N-(4-methoxyphenyl)benzamide |
| 1O61 | 4-[4-[[3-(3-Hydroxyphenyl)-5-(trifluoromethyl)phenyl]methyl]piperazin-1-yl]-N-(4-methoxyphenyl)benzamide |
| 1O62 | 4-[4-[[3-(5-Hydroxypyridin-3-yl)-5-(trifluoromethyl)phenyl]methyl]piperazin-1-yl]-N-(4-methoxyphenyl)benzamide |
| 1O63 | 4-[4-[[3-(3-Hydroxyphenyl)-5-(trifluoromethoxy)phenyl]methyl]piperazin-1-yl]-N-(4-methoxyphenyl)benzamide |
| 1O64 | 4-[4-[[3-(5-Hydroxypyridin-3-yl)-5-(trifluoromethoxy)phenyl]methyl]piperazin-1-yl]-N-(4-methoxyphenyl)benzamide |
| 1O65 | 4-[4-[[4-(3-Hydroxyphenyl)naphthalen-1-yl]methyl]piperazin-1-yl]-N-(4-methoxyphenyl)benzamide |
| 1O66 | 4-[4-[[4-(5-Hydroxypyridin-3-yl)naphthalen-1-yl]methyl]piperazin-1-yl]-N-(4-methoxyphenyl)benzamide |
| 1O67 | 4-[4-[[4-(3-Hydroxyphenyl)thiophen-2-yl]methyl]piperazin-1-yl]-N-(4-methoxyphenyl)benzamide |
| 1O68 | 4-[4-[[4-(5-Hydroxypyridin-3-yl)thiophen-2-yl]methyl]piperazin-1-yl]-N-(4-methoxyphenyl)benzamide |
| 1O69 | 4-[4-[[2-[2-(3-Hydroxyphenyl)ethynyl]phenyl]methyl]piperazin-1-yl]-N-(4-methoxyphenyl)benzamide |
| 1O70 | 4-[4-[[2-[2-(5-Hydroxypyridin-3-yl)ethynyl]phenyl]methyl]piperazin-1-yl]-N-(4-methoxyphenyl)benzamide |

(continued)

| Mixture No. used | mixEI10-02R |
|---|---|
| Post-cleavage mixture No. | mixEJ04 |
| Compound No. in Table EJ04 | Compound name |
| 1O71 | 6-[4-[[2-(3-Hydroxyphenyl)phenyl]methyl]piperazin-1-yl]-N-(4-methoxyphenyl)pyridazine-3-carboxamide |
| 1O72 | 6-[4-[[2-(5-Hydroxypyridin-3-yl)phenyl]methyl]piperazin-1-yl]-N-(4-methoxyphenyl)pyridazine-3-carboxamide |
| 1O73 | 6-[4-[[4-(3-Hydroxyphenyl)-3-methylphenyl]methyl]piperazin-1-yl]-N-(4-methoxyphenyl)pyridazine-3-carboxamide |
| 1O74 | 6-[4-[[4-(5-Hydroxypyridin-3-yl)-3-methylphenyl]methyl]piperazin-1-yl]-N-(4-methoxyphenyl)pyridazine-3-carboxamide |
| 1O75 | 6-[4-[[2-Ethyl-4-(3-hydroxyphenyl)phenyl]methyl]piperazin-1-yl]-N-(4-methoxyphenyl)pyridazine-3-carboxamide |
| 1O76 | 6-[4-[[2-Ethyl-4-(5-hydroxypyridin-3-yl)phenyl]methyl]piperazin-1-yl]-N-(4-methoxyphenyl)pyridazine-3-carboxamide |
| 1O77 | 6-[4-[[3-Fluoro-4-(3-hydroxyphenyl)phenyl]methyl]piperazin-1-yl]-N-(4-methoxyphenyl)pyridazine-3-carboxamide |
| 1O78 | 6-[4-[[3-Fluoro-4-(5-hydroxypyridin-3-yl)phenyl]methyl]piperazin-1-yl]-N-(4-methoxyphenyl)pyridazine-3-carboxamide |
| 1O79 | 6-[4-[[4-(3-Hydroxyphenyl)-2-methoxyphenyl]methyl]piperazin-1-yl]-N-(4-methoxyphenyl)pyridazine-3-carboxamide |
| 1O80 | 6-[4-[[4-(5-Hydroxypyridin-3-yl)-2-methoxyphenyl]methyl]piperazin-1-yl]-N-(4-methoxyphenyl)pyridazine-3-carboxamide |
| 1O81 | 6-[4-[[3-Ethoxy-5-(3-hydroxyphenyl)phenyl]methyl]piperazin-1-yl]-N-(4-methoxyphenyl)pyridazine-3-carboxamide |
| 1O82 | 6-[4-[[3-Ethoxy-5-(5-hydroxypyridin-3-yl)phenyl]methyl]piperazin-1-yl]-N-(4-methoxyphenyl)pyridazine-3-carboxamide |
| 1O83 | 6-[4-[[3-(3-Hydroxyphenyl)-5-propan-2-yloxyphenyl]methyl]piperazin-1-yl]-N-(4-methoxyphenyl)pyridazine-3-carboxamide |
| 1O84 | 6-[4-[[3-(5-Hydroxypyridin-3-yl)-5-propan-2-yloxyphenyl]methyl]piperazin-1-yl]-N-(4-methoxyphenyl)pyridazine-3-carboxamide |
| 1O85 | 6-[4-[[4-(3-Hydroxyphenyl)-2,6-dimethoxyphenyl]methyl]piperazin-1-yl]-N-(4-methoxyphenyl)pyridazine-3-carboxamide |
| 1O86 | 6-[4-[[4-(5-Hydroxypyridin-3-yl)-2,6-dimethoxyphenyl]methyl]piperazin-1-yl]-N-(4-methoxyphenyl)pyridazine-3-carboxamide |
| 1O87 | 6-[4-[[3-(3-Hydroxyphenyl)-5-(trifluoromethyl)phenyl]methyl]piperazin-1-yl]-N-(4-methoxyphenyl)pyridazine-3-carboxamide |
| 1O88 | 6-[4-[[3-(5-Hydroxypyridin-3-yl)-5-(trifluoromethyl)phenyl]methyl]piperazin-1-yl]-N-(4-methoxyphenyl)pyridazine-3-carboxamide |
| 1O89 | 6-[4-[[3-(3-Hydroxyphenyl)-5-(trifluoromethoxy)phenyl]methyl]piperazin-1-yl]-N-(4-methoxyphenyl)pyridazine-3-carboxamide |
| 1O90 | 6-[4-[[3-(5-Hydroxypyridin-3-yl)-5-(trifluoromethoxy)phenyl]methyl]piperazin-1-yl]-N-(4-methoxyphenyl)pyridazine-3-carboxamide |

(continued)

| Mixture No. used | mixEI10-02R |
|---|---|
| Post-cleavage mixture No. | mixEJ04 |
| Compound No. in Table EJ04 | Compound name |
| 1O91 | 6-[4-[[4-(3-Hydroxyphenyl)naphthalen-1-yl]methyl]piperazin-1-yl]-N-(4-methoxyphenyl)pyridazine-3-carboxamide |
| 1O92 | 6-[4-[[4-(5-Hydroxypyridin-3-yl)naphthalen-1-yl]methyl]piperazin-1-yl]-N-(4-methoxyphenyl)pyridazine-3-carboxamide |
| 1O93 | 6-[4-[[4-(3-Hydroxyphenyl)thiophen-2-yl]methyl]piperazin-1-yl]-N-(4-methoxyphenyl)pyridazine-3-carboxamide |
| 1O94 | 6-[4-[[4-(5-Hydroxypyridin-3-yl)thiophen-2-yl]methyl]piperazin-1-yl]-N-(4-methoxyphenyl)pyridazine-3-carboxamide |
| 1O95 | 6-[4-[[2-[2-(3-Hydroxyphenyl)ethynyl]phenyl]methyl]piperazin-1-yl]-N-(4-methoxyphenyl)pyridazine-3-carboxamide |
| 1O96 | 6-[4-[[2-[2-(5-Hydroxypyridin-3-yl)ethynyl]phenyl]methyl]piperazin-1-yl]-N-(4-methoxyphenyl)pyridazine-3-carboxamide |
| 1O97 | 4-[4-[1-[4-(3-Hydroxyphenyl)phenyl]ethyl]piperazin-1-yl]-N-(4-methoxyphenyl)benzamide |
| 1O98 | 4-[4-[1-[4-(5-Hydroxypyridin-3-yl)phenyl]ethyl]piperazin-1-yl]-N-(4-methoxyphenyl)benzamide |
| 1O99 | 4-[4-[1-[2-Fluoro-4-(3-hydroxyphenyl)phenyl]ethyl]piperazin-1-yl]-N-(4-methoxyphenyl)benzamide |
| 1O100 | 4-[4-[1-[2-Fluoro-4-(5-hydroxypyridin-3-yl)phenyl]ethyl]piperazin-1-yl]-N-(4-methoxyphenyl)benzamide |

[Table 76]

[Table EU05]

| Mixture No. used | mixEI11-02R |
|---|---|
| Post-cleavage mixture No. | mixEJ05 |
| Compound No. in Table EJ05 | Compound name |
| 1A01 | N-(1-Adamantylmethyl)-6-[4-[4-(3-hydroxyphenyl)thiophene-2-carbonyl]piperazin-1-yl]pyridazine-3-carboxamide |
| 1A02 | N-(1-Adamantylmethyl)-6-[4-[4-(5-hydroxypyridin-3-yl)thiophene-2-carbonyl]piperazin-1-yl]pyridazine-3-carboxamide |
| 1A03 | N-(1-Adamantylmethyl)-6-[4-[4-(3-hydroxyphenyl)-3-methylbenzoyl]piperazin-1-yl]pyridazine-3-carboxamide |
| 1A04 | N-(1-Adamantylmethyl)-6-[4-[4-(5-hydroxypyridin-3-yl)-3-methylbenzoyl]piperazin-1-yl]pyridazine-3-carboxamide |
| 1A05 | N-(1-Adamantylmethyl)-6-[4-[3-fluoro-4-(3-hydroxyphenyl)benzoyl]piperazin-1-yl]pyridazine-3-carboxamide |

(continued)

| Mixture No. used | mixEI11-02R |
|---|---|
| Post-cleavage mixture No. | mixEJ05 |
| Compound No. in Table EJ05 | Compound name |
| 1A06 | N-(1-Adamantylmethyl)-6-[4-[3-fluoro-4-(5-hydroxypyridin-3-yl)benzoyl]piperazin-1-yl]pyridazine-3-carboxamide |
| 1A07 | N-(1-Adamantylmethyl)-6-[4-[2-[2-(3-hydroxyphenyl)ethynyl]benzoyl]piperazin-1-yl]pyridazine-3-carboxamide |
| 1A08 | N-(1-Adamantylmethyl)-6-[4-[2-[2-(5-hydroxypyridin-3-yl)ethynyl]benzoyl]piperazin-1-yl]pyridazine-3-carboxamide |
| 1A09 | N-(1-Adamantylmethyl)-6-[4-[5-[2-(3-hydroxyphenyl)ethynyl]pyridine-3-carbonyl]piperazin-1-yl]pyridazine-3-carboxamide |
| 1A10 | N-(1-Adamantylmethyl)-6-[4-[5-[2-(5-hydroxypyridin-3-yl)ethynyl]pyridine-3-carbonyl]piperazin-1-yl]pyridazine-3-carboxamide |
| 1A11 | N-(1-Adamantylmethyl)-6-[4-[3-[2-(3-hydroxyphenyl)phenyl]propanoyl]piperazin-1-yl]pyridazine-3-carboxamide |
| 1A12 | N-(1-Adamantylmethyl)-6-[4-[3-[2-(5-hydroxypyridin-3-yl)phenyl]propanoyl]piperazin-1-yl]pyridazine-3-carboxamide |
| 1A13 | N-(1-Adamantylmethyl)-6-[4-[4-(3-hydroxyphenyl)naphthalene-1-carbonyl]piperazin-1-yl]pyridazine-3-carboxamide |
| 1A14 | N-(1-Adamantylmethyl)-6-[4-[4-(5-hydroxypyridin-3-yl)naphthalene-1-carbonyl]piperazin-1-yl]pyridazine-3-carboxamide |
| 1A15 | N-(1-Adamantylmethyl)-6-[4-[3-tert-butyl-5-(3-hydroxyphenyl)benzoyl]piperazin-1-yl]pyridazine-3-carboxamide |
| 1A16 | N-(1-Adamantylmethyl)-6-[4-[3-tert-butyl-5-(5-hydroxypyridin-3-yl)benzoyl]piperazin-1-yl]pyridazine-3-carboxamide |
| 1A17 | N-(1-Adamantylmethyl)-6-[4-[3-(3-hydroxyphenyl)-5-(trifluoromethyl)benzoyl]piperazin-1-yl]pyridazine-3-carboxamide |
| 1A18 | N-(1-Adamantylmethyl)-6-[4-[3-(5-hydroxypyridin-3-yl)-5-(trifluoromethyl)benzoyl]piperazin-1-yl]pyridazine-3-carboxamide |
| 1A19 | N-(1-Adamantylmethyl)-6-[4-[3-[2-(3-hydroxyphenyl)ethynyl]-5-(trifluoromethyl)benzoyl]piperazin-1-yl]pyridazine-3-carboxamide |
| 1A20 | N-(1-Adamantylmethyl)-6-[4-[3-[2-(5-hydroxypyridin-3-yl)ethynyl]-5-(trifluoromethyl)benzoyl]piperazin-1-yl]pyridazine-3-carboxamide |
| 1A21 | N-(1-Adamantylmethyl)-6-[4-[3-[4-(3-hydroxyphenyl)pyrazol-1-yl]-5-(trifluoromethyl)benzoyl]piperazin-1-yl]pyridazine-3-carboxamide |
| 1A22 | N-(1-Adamantylmethyl)-6-[4-[3-[4-(5-hydroxypyridin-3-yl)pyrazol-1-yl]-5-(trifluoromethyl)benzoyl]piperazin-1-yl]pyridazine-3-carboxamide |
| 1A23 | N-(1-Adamantylmethyl)-4-[4-[4-(3-hydroxyphenyl)thiophene-2-carbonyl]piperazin-1-yl]benzamide |
| 1A24 | N-(1-Adamantylmethyl)-4-[4-[4-(5-hydroxypyridin-3-yl)thiophene-2-carbonyl]piperazin-1-yl]benzamide |
| 1A25 | N-(1-Adamantylmethyl)-4-[4-[4-(3-hydroxyphenyl)-3-methylbenzoyl]piperazin-1-yl]benzamide |

(continued)

| Mixture No. used | mixEI11-02R |
|---|---|
| Post-cleavage mixture No. | mixEJ05 |
| Compound No. in Table EJ05 | Compound name |
| 1A26 | N-(1-Adamantylmethyl)-4-[4-[4-(5-hydroxypyridin-3-yl)-3-methylbenzoyl]piperazin-1-yl]benzamide |
| 1A27 | N-(1-Adamantylmethyl)-4-[4-[3-fluoro-4-(3-hydroxyphenyl)benzoyl]piperazin-1-yl]benzamide |
| 1A28 | N-(1-Adamantylmethyl)-4-[4-[3-fluoro-4-(5-hydroxypyridin-3-yl)benzoyl]piperazin-1-yl]benzamide |
| 1A29 | N-(1-Adamantylmethyl)-4-[4-[2-[2-(3-hydroxyphenyl)ethynyl]benzoyl]piperazin-1-yl]benzamide |
| 1A30 | N-(1-Adamantylmethyl)-4-[4-[2-[2-(5-hydroxypyridin-3-yl)ethynyl]benzoyl]piperazin-1-yl]benzamide |
| 1A31 | N-(1-Adamantylmethyl)-4-[4-[5-[2-(3-hydroxyphenyl)ethynyl]pyridine-3-carbonyl]piperazin-1-yl]benzamide |
| 1A32 | N-(1-Adamantylmethyl)-4-[4-[5-[2-(5-hydroxypyridin-3-yl)ethynyl]pyridine-3-carbonyl]piperazin-1-yl]benzamide |
| 1A33 | N-(1-Adamantylmethyl)-4-[4-[3-[2-(3-hydroxyphenyl)phenyl]propanoyl]piperazin-1-yl]benzamide |
| 1A34 | N-(1-Adamantylmethyl)-4-[4-[3-[2-(5-hydroxypyridin-3-yl)phenyl]propanoyl]piperazin-1-yl]benzamide |
| 1A35 | N-(1-Adamantylmethyl)-4-[4-[4-(3-hydroxyphenyl)naphthalene-1-carbonyl]piperazin-1-yl]benzamide |
| 1A36 | N-(1-Adamantylmethyl)-4-[4-[4-(5-hydroxypyridin-3-yl)naphthalene-1-carbonyl]piperazin-1-yl]benzamide |
| 1A37 | N-(1-Adamantylmethyl)-4-[4-[3-tert-butyl-5-(3-hydroxyphenyl)benzoyl]piperazin-1-yl]benzamide |
| 1A38 | N-(1-Adamantylmethyl)-4-[4-[3-tert-butyl-5-(5-hydroxypyridin-3-yl)benzoyl]piperazin-1-yl]benzamide |
| 1A39 | N-(1-Adamantylmethyl)-4-[4-[3-(3-hydroxyphenyl)-5-(trifluoromethyl)benzoyl]piperazin-1 -yl]benzamide |
| 1A40 | N-(1-Adamantylmethyl)-4-[4-[3-(5-hydroxypyridin-3-yl)-5-(trifluoromethyl)benzoyl]piperazin-1-yl]benzamide |
| 1A41 | N-(1-Adamantylmethyl)-4-[4-[3-[2-(3-hydroxyphenyl)ethynyl]-5-(trifluoromethyl)benzoyl]piperazin-1 -yl]benzamide |
| 1A42 | N-(1-Adamantylmethyl)-4-[4-[3-[2-(5-hydroxypyridin-3-yl)ethynyl]-5-(trifluoromethyl)benzoyl]piperazin-1-yl]benzamide |
| 1A43 | N-(1-Adamantylmethyl)-4-[4-[3-[4-(3-hydroxyphenyl)pyrazol-1-yl]-5-(trifluoromethyl)benzoyl]piperazin-1 -yl]benzamide |
| 1A44 | N-(1-Adamantylmethyl)-4-[4-[3-[4-(5-hydroxypyridin-3-yl)pyrazol-1-yl]-5-(trifluoromethyl)benzoyl]piperazin-1-yl]benzamide |
| 1A45 | N-(1-Adamantylmethyl)-4-[4-[[2-(3-hydroxyphenyl)phenyl]methyl]piperazin-1-yl]benzamide |
| 1A46 | N-(1-Adamantylmethyl)-4-[4-[[2-(5-hydroxypyridin-3-yl)phenyl]methyl]piperazin-1-yl]benzamide |
| 1A47 | N-(1-Adamantylmethyl)-4-[4-[[4-(3-hydroxyphenyl)-3-methylphenyl]methyl]piperazin-1-yl]benzamide |

(continued)

| Mixture No. used | mixEI11-02R |
|---|---|
| Post-cleavage mixture No. | mixEJ05 |
| Compound No. in Table EJ05 | Compound name |
| 1A48 | N-(1-Adamantylmethyl)-4-[4-[[4-(5-hydroxypyridin-3-yl)-3-methylphenyl]methyl]piperazin-1-yl]benzamide |
| 1A49 | N-(1-Adamantylmethyl)-4-[4-[[2-ethyl-4-(3-hydroxyphenyl)phenyl]methyl]piperazin-1-yl]benzamide |
| 1A50 | N-(1-Adamantylmethyl)-4-[4-[[2-ethyl-4-(5-hydroxypyridin-3-yl)phenyl]methyl]piperazin-1-yl]benzamide |
| 1A51 | N-(1-Adamantylmethyl)-4-[4-[[3-fluoro-4-(3-hydroxyphenyl)phenyl]methyl]piperazin-1-yl]benzamide |
| 1A52 | N-(1-Adamantylmethyl)-4-[4-[[3-fluoro-4-(5-hydroxypyridin-3-yl)phenyl]methyl]piperazin-1-yl]benzamide |
| 1A53 | N-(1-Adamantylmethyl)-4-[4-[[4-(3-hydroxyphenyl)-2-methoxyphenyl]methyl]piperazin-1-yl]benzamide |
| 1A54 | N-(1-Adamantylmethyl)-4-[4-[[4-(5-hydroxypyridin-3-yl)-2-methoxyphenyl]methyl]piperazin-1-yl]benzamide |
| 1A55 | N-(1-Adamantylmethyl)-4-[4-[[3-ethoxy-5-(3-hydroxyphenyl)phenyl]methyl]piperazin-1-yl]benzamide |
| 1A56 | N-(1-Adamantylmethyl)-4-[4-[[3-ethoxy-5-(5-hydroxypyridin-3-yl)phenyl]methyl]piperazin-1-yl]benzamide |
| 1A57 | N-(1-Adamantylmethyl)-4-[4-[[3-(3-hydroxyphenyl)-5-propan-2-yloxyphenyl]methyl]piperazin-1-yl]benzamide |
| 1A58 | N-(1-Adamantylmethyl)-4-[4-[[3-(5-hydroxypyridin-3-yl)-5-propan-2-yloxyphenyl]methyl]piperazin-1-yl]benzamide |
| 1A59 | N-(1-Adamantylmethyl)-4-[4-[[4-(3-hydroxyphenyl)-2,6-dimethoxyphenyl]methyl]piperazin-1-yl]benzamide |
| 1A60 | N-(1-Adamantylmethyl)-4-[4-[[4-(5-hydroxypyridin-3-yl)-2,6-dimethoxyphenyl]methyl]piperazin-1-yl]benzamide |
| 1A61 | N-(1-Adamantylmethyl)-4-[4-[[3-(3-hydroxyphenyl)-5-(trifluoromethyl)phenyl]methyl]piperazin-1-yl]benzamide |
| 1A62 | N-(1-Adamantylmethyl)-4-[4-[[3-(5-hydroxypyridin-3-yl)-5-(trifluoromethyl)phenyl]methyl]piperazin-1-yl]benzamide |
| 1A63 | N-(1-Adamantylmethyl)-4-[4-[[3-(3-hydroxyphenyl)-5-(trifluoromethoxy)phenyl]methyl]piperazin-1-yl]benzamide |
| 1A64 | N-(1-Adamantylmethyl)-4-[4-[[3-(5-hydroxypyridin-3-yl)-5-(trifluoromethoxy)phenyl]methyl]piperazin-1-yl]benzamide |
| 1A65 | N-(1-Adamantylmethyl)-4-[4-[[4-(3-hydroxyphenyl)naphthalen-1-yl]methyl]piperazin-1-yl]benzamide |
| 1A66 | N-(1-Adamantylmethyl)-4-[4-[[4-(5-hydroxypyridin-3-yl)naphthalen-1-yl]methyl]piperazin-1-yl]benzamide |
| 1A67 | N-(1-Adamantylmethyl)-4-[4-[[4-(3-hydroxyphenyl)thiophen-2-yl]methyl]piperazin-1-yl]benzamide |

(continued)

| Mixture No. used | mixEI11-02R |
|---|---|
| Post-cleavage mixture No. | mixEJ05 |
| Compound No. in Table EJ05 | Compound name |
| 1A68 | N-(1-Adamantylmethyl)-4-[4-[[4-(5-hydroxypyridin-3-yl)thiophen-2-yl]methyl]piperazin-1-yl]benzamide |
| 1A69 | N-(1-Adamantylmethyl)-4-[4-[[2-[2-(3-hydroxyphenyl)ethynyl]phenyl]methyl]piperazin-1-yl]benzamide |
| 1A70 | N-(1-Adamantylmethyl)-4-[4-[[2-[2-(5-hydroxypyridin-3-yl)ethynyl]phenyl]methyl]piperazin-1-yl]benzamide |
| 1A71 | N-(1-Adamantylmethyl)-6-[4-[[2-(3-hydroxyphenyl)phenyl]methyl]piperazin-1-yl]pyridazine-3-carboxamide |
| 1A72 | N-(1-Adamantylmethyl)-6-[4-[[2-(5-hydroxypyridin-3-yl)phenyl]methyl]piperazin-1-yl]pyridazine-3-carboxamide |
| 1A73 | N-(1-Adamantylmethyl)-6-[4-[[4-(3-hydroxyphenyl)-3-methylphenyl]methyl]piperazin-1-yl]pyridazine-3-carboxamide |
| 1A74 | N-(1-Adamantylmethyl)-6-[4-[[4-(5-hydroxypyridin-3-yl)-3-methylphenyl]methyl]piperazin-1-yl]pyridazine-3-carboxamide |
| 1A75 | N-(1-Adamantylmethyl)-6-[4-[[2-ethyl-4-(3-hydroxyphenyl)phenyl]methyl]piperazin-1-yl]pyridazine-3-carboxamide |
| 1A76 | N-(1-Adamantylmethyl)-6-[4-[[2-ethyl-4-(5-hydroxypyridin-3-yl)phenyl]methyl]piperazin-1-yl]pyridazine-3-carboxamide |
| 1A77 | N-(1-Adamantylmethyl)-6-[4-[[3-fluoro-4-(3-hydroxyphenyl)phenyl]methyl]piperazin-1-yl]pyridazine-3-carboxamide |
| 1A78 | N-(1-Adamantylmethyl)-6-[4-[[3-fluoro-4-(5-hydroxypyridin-3-yl)phenyl]methyl]piperazin-1-yl]pyridazine-3-carboxamide |
| 1A79 | N-(1-Adamantylmethyl)-6-[4-[[4-(3-hydroxyphenyl)-2-methoxyphenyl]methyl]piperazin-1-yl]pyridazine-3-carboxamide |
| 1A80 | N-(1-Adamantylmethyl)-6-[4-[[4-(5-hydroxypyridin-3-yl)-2-methoxyphenyl]methyl]piperazin-1-yl]pyridazine-3-carboxamide |
| 1A81 | N-(1-Adamantylmethyl)-6-[4-[[3-ethoxy-5-(3-hydroxyphenyl)phenyl]methyl]piperazin-1-yl]pyridazine-3-carboxamide |
| 1A82 | N-(1-Adamantylmethyl)-6-[4-[[3-ethoxy-5-(5-hydroxypyridin-3-yl)phenyl]methyl]piperazin-1-yl]pyridazine-3-carboxamide |
| 1A83 | N-(1-Adamantylmethyl)-6-[4-[[3-(3-hydroxyphenyl)-5-propan-2-yloxyphenyl]methyl]piperazin-1-yl]pyridazine-3-carboxamide |
| 1A84 | N-(1-Adamantylmethyl)-6-[4-[[3-(5-hydroxypyridin-3-yl)-5-propan-2-yloxyphenyl]methyl]piperazin-1-yl]pyridazine-3-carboxamide |
| 1A85 | N-(1-Adamantylmethyl)-6-[4-[[4-(3-hydroxyphenyl)-2,6-dimethoxyphenyl]methyl]piperazin-1-yl]pyridazine-3-carboxamide |
| 1A86 | N-(1-Adamantylmethyl)-6-[4-[[4-(5-hydroxypyridin-3-yl)-2,6-dimethoxyphenyl]methyl]piperazin-1-yl]pyridazine-3-carboxamide |
| 1A87 | N-(1-Adamantylmethyl)-6-[4-[[3-(3-hydroxyphenyl)-5-(trifluoromethyl)phenyl]methyl]piperazin-1-yl]pyridazine-3-carboxamide |

(continued)

| Mixture No. used | mixEI11-02R |
|---|---|
| Post-cleavage mixture No. | mixEJ05 |
| Compound No. in Table EJ05 | Compound name |
| 1A88 | N-(1-Adamantylmethyl)-6-[4-[[3-(5-hydroxypyridin-3-yl)-5-(trifluoromethyl)phenyl]methyl]piperazin-1-yl]pyridazine-3-carboxamide |
| 1A89 | N-(1-Adamantylmethyl)-6-[4-[[3-(3-hydroxyphenyl)-5-(trifluoromethoxy)phenyl]methyl]piperazin-1-yl]pyridazine-3-carboxamide |
| 1A90 | N-(1-Adamantylmethyl)-6-[4-[[3-(5-hydroxypyridin-3-yl)-5-(trifluoromethoxy)phenyl]methyl]piperazin-1-yl]pyridazine-3-carboxamide |
| 1A91 | N-(1-Adamantylmethyl)-6-[4-[[4-(3-hydroxyphenyl)naphthalen-1-yl]methyl]piperazin-1-yl]pyridazine-3-carboxamide |
| 1A92 | N-(1-Adamantylmethyl)-6-[4-[[4-(5-hydroxypyridin-3-yl)naphthalen-1-yl]methyl]piperazin-1-yl]pyridazine-3-carboxamide |
| 1A93 | N-(1-Adamantylmethyl)-6-[4-[[4-(3-hydroxyphenyl)thiophen-2-yl]methyl]piperazin-1-yl]pyridazine-3-carboxamide |
| 1A94 | N-(1-Adamantylmethyl)-6-[4-[[4-(5-hydroxypyridin-3-yl)thiophen-2-yl]methyl]piperazin-1-yl]pyridazine-3-carboxamide |
| 1A95 | N-(1-Adamantylmethyl)-6-[4-[[2-[2-(3-hydroxyphenyl)ethynyl]phenyl]methyl]piperazin-1-yl]pyridazine-3-carboxamide |
| 1A96 | N-(1-Adamantylmethyl)-6-[4-[[2-[2-(5-hydroxypyridin-3-yl)ethynyl]phenyl]methyl]piperazin-1-yl]pyridazine-3-carboxamide |
| 1A97 | N-(1-Adamantylmethyl)-4-[4-[1-[4-(3-hydroxyphenyl)phenyl]ethyl]piperazin-1-yl]benzamide |
| 1A98 | N-(1-Adamantylmethyl)-4-[4-[1-[4-(5-hydroxypyridin-3-yl)phenyl]ethyl]piperazin-1-yl]benzamide |
| 1A99 | N-(1-Adamantylmethyl)-4-[4-[1-[2-fluoro-4-(3-hydroxyphenyl)phenyl]ethyl]piperazin-1-yl]benzamide |
| 1A100 | N-(1-Adamantylmethyl)-4-[4-[1-[2-fluoro-4-(5-hydroxypyridin-3-yl)phenyl]ethyl]piperazin-1-yl]benzamide |

[Table 77]

[Table EU06]

| Mixture No. used | mixEI12-02R |
|---|---|
| Post-cleavage mixture No. | mixEJ06 |
| Compound No. in Table EJ06 | Compound name |
| 3A01 | N-(1-Adamantylmethyl)-6-[4-[4-(3-hydroxyphenyl)thiophene-2-carbonyl]piperazin-1-yl]-N-methylpyridazine-3-carboxamide |
| 3A02 | N-(1-Adamantylmethyl)-6-[4-[4-(5-hydroxypyridin-3-yl)thiophene-2-carbonyl]piperazin-1-yl]-N-methylpyridazine-3-carboxamide |

(continued)

| Mixture No. used | mixEI12-02R |
|---|---|
| Post-cleavage mixture No. | mixEJ06 |
| Compound No. in Table EJ06 | Compound name |
| 3A03 | N-(1-Adamantylmethyl)-6-[4-[4-(3-hydroxyphenyl)-3-methylbenzoyl]piperazin-1-yl]-N-methylpyridazine-3-carboxamide |
| 3A04 | N-(1-Adamantylmethyl)-6-[4-[4-(5-hydroxypyridin-3-yl)-3-methylbenzoyl]piperazin-1-yl]-N-methylpyridazine-3-carboxamide |
| 3A05 | N-(1-Adamantylmethyl)-6-[4-[3-fluoro-4-(3-hydroxyphenyl)benzoyl]piperazin-1-yl]-N-methylpyridazine-3-carboxamide |
| 3A06 | N-(1-Adamantylmethyl)-6-[4-[3-fluoro-4-(5-hydroxypyridin-3-yl)benzoyl]piperazin-1-yl]-N-methylpyridazine-3-carboxamide |
| 3A07 | N-(1-Adamantylmethyl)-6-[4-[2-[2-(3-hydroxyphenyl)ethynyl]benzoyl]piperazin-1-yl]-N-methylpyridazine-3-carboxamide |
| 3A08 | N-(1-Adamantylmethyl)-6-[4-[2-[2-(5-hydroxypyridin-3-yl)ethynyl]benzoyl]piperazin-1-yl]-N-methylpyridazine-3-carboxamide |
| 3A09 | N-(1-Adamantylmethyl)-6-[4-[5-[2-(3-hydroxyphenyl)ethynyl]pyridine-3-carbonyl]piperazin-1-yl]-N-methylpyridazine-3-carboxamide |
| 3A10 | N-(1-Adamantylmethyl)-6-[4-[5-[2-(5-hydroxypyridin-3-yl)ethynyl]pyridine-3-carbonyl]piperazin-1-yl]-N-methylpyridazine-3-carboxamide |
| 3A11 | N-(1-Adamantylmethyl)-6-[4-[3-[2-(3-hydroxyphenyl)phenyl]propanoyl]piperazin-1-yl]-N-methylpyridazine-3- carboxamide |
| 3A12 | N-(1-Adamantylmethyl)-6-[4-[3-[2-(5-hydroxypyridin-3-yl)phenyl]propanoyl]piperazin-1-yl]-N-methylpyridazine-3-carboxamide |
| 3A13 | N-(1-Adamantylmethyl)-6-[4-[4-(3-hydroxyphenyl)naphthalene-1-carbonyl]piperazin-1-yl]-N-methylpyridazine-3-carboxamide |
| 3A14 | N-(1-Adamantylmethyl)-6-[4-[4-(5-hydroxypyridin-3-yl)naphthalene-1-carbonyl]piperazin-1-yl]-N-methylpyridazine-3-carboxamide |
| 3A15 | N-(1-Adamantylmethyl)-6-[4-[3-tert-butyl-5-(3-hydroxyphenyl)benzoyl]piperazin-1-yl]-N-methylpyridazine-3-carboxamide |
| 3A16 | N-(1-Adamantylmethyl)-6-[4-[3-tert-butyl-5-(5-hydroxypyridin-3-yl)benzoyl]piperazin-1-yl]-N-methylpyridazine-3-carboxamide |
| 3A17 | N-(1-Adamantylmethyl)-6-[4-[3-(3-hydroxyphenyl)-5-(trifluoromethyl)benzoyl]piperazin-1-yl]-N-methylpyridazine-3-carboxamide |
| 3A18 | N-(1-Adamantylmethyl)-6-[4-[3-(5-hydroxypyridin-3-yl)-5-(trifluoromethyl)benzoyl]piperazin-1-yl]-N-methylpyridazine-3-carboxamide |
| 3A19 | N-(1-Adamantylmethyl)-6-[4-[3-[2-(3-hydroxyphenyl)ethynyl]-5-(trifluoromethyl)benzoyl]piperazin-1-yl]-N-methylpyridazine-3-carboxamide |
| 3A20 | N-(1-Adamantylmethyl)-6-[4-[3-[2-(5-hydroxypyridin-3-yl)ethynyl]-5-(trifluoromethyl)benzoyl]piperazin-1-yl]-N-methylpyridazine-3-carboxamide |
| 3A21 | N-(1-Adamantylmethyl)-6-[4-[3-[4-(3-hydroxyphenyl)pyrazol-1-yl]-5-(trifluoromethyl)benzoyl]piperazin-1-yl]-N-methylpyridazine-3-carboxamide |
| 3A22 | N-(1-Adamantylmethyl)-6-[4-[3-[4-(5-hydroxypyridin-3-yl)pyrazol-1-yl]-5-(trifluoromethyl)benzoyl]piperazin-1-yl]-N-methylpyridazine-3-carboxamide |

(continued)

| Mixture No. used | mixEI12-02R |
|---|---|
| Post-cleavage mixture No. | mixEJ06 |
| Compound No. in Table EJ06 | Compound name |
| 3A23 | N-(1-Adamantylmethyl)-4-[4-[4-(3-hydroxyphenyl)thiophene-2-carbonyl]piperazin-1-yl]-N-methylbenzamide |
| 3A24 | N-(1-Adamantylmethyl)-4-[4-[4-(5-hydroxypyridin-3-yl)thiophene-2-carbonyl]piperazin-1-yl]-N-methylbenzamide |
| 3A25 | N-(1-Adamantylmethyl)-4-[4-[4-(3-hydroxyphenyl)-3-methylbenzoyl]piperazin-1-yl]-N-methylbenzamide |
| 3A26 | N-(1-Adamantylmethyl)-4-[4-[4-(5-hydroxypyridin-3-yl)-3-methylbenzoyl]piperazin-1-yl]-N-methylbenzamide |
| 3A27 | N-(1-Adamantylmethyl)-4-[4-[3-fluoro-4-(3-hydroxyphenyl)benzoyl]piperazin-1-yl]-N-methylbenzamide |
| 3A28 | N-(1-Adamantylmethyl)-4-[4-[3-fluoro-4-(5-hydroxypyridin-3-yl)benzoyl]piperazin-1-yl]-N-methylbenzamide |
| 3A29 | N-(1-Adamantylmethyl)-4-[4-[2-[2-(3-hydroxyphenyl)ethynyl]benzoyl]piperazin-1-yl]-N-methylbenzamide |
| 3A30 | N-(1-Adamantylmethyl)-4-[4-[2-[2-(5-hydroxypyridin-3-yl)ethynyl]benzoyl]piperazin-1-yl]-N-methylbenzamide |
| 3A31 | N-(1-Adamantylmethyl)-4-[4-[5-[2-(3-hydroxyphenyl)ethynyl]pyridine-3-carbonyl]piperazin-1-yl]-N-methylbenzamide |
| 3A32 | N-(1-Adamantylmethyl)-4-[4-[5-[2-(5-hydroxypyridin-3-yl)ethynyl]pyridine-3-carbonyl]piperazin-1-yl]-N-methylbenzamide |
| 3A33 | N-(1-Adamantylmethyl)-4-[4-[3-[2-(3-hydroxyphenyl)phenyl]propanoyl]piperazin-1-yl]-N-methylbenzamide |
| 3A34 | N-(1-Adamantylmethyl)-4-[4-[3-[2-(5-hydroxypyridin-3-yl)phenyl]propanoyl]piperazin-1-yl]-N-methylbenzamide |
| 3A35 | N-(1-Adamantylmethyl)-4-[4-[4-(3-hydroxyphenyl)naphthalene-1-carbonyl]piperazin-1-yl]-N-methylbenzamide |
| 3A36 | N-(1-Adamantylmethyl)-4-[4-[4-(5-hydroxypyridin-3-yl)naphthalene-1-carbonyl]piperazin-1-yl]-N-methylbenzamide |
| 3A37 | N-(1-Adamantylmethyl)-4-[4-[3-tert-butyl-5-(3-hydroxyphenyl)benzoyl]piperazin-1-yl]-N-methylbenzamide |
| 3A38 | N-(1-Adamantylmethyl)-4-[4-[3-tert-butyl-5-(5-hydroxypyridin-3-yl)benzoyl]piperazin-1-yl]-N-methylbenzamide |
| 3A39 | N-(1-Adamantylmethyl)-4-[4-[3-(3-hydroxyphenyl)-5-(trifluoromethyl)benzoyl]piperazin-1-yl]-N-methylbenzamide |
| 3A40 | N-(1-Adamantylmethyl)-4-[4-[3-(5-hydroxypyridin-3-yl)-5-(trifluoromethyl)benzoyl]piperazin-1-yl]-N-methylbenzamide |
| 3A41 | N-(1-Adamantylmethyl)-4-[4-[3-[2-(3-hydroxyphenyl)ethynyl]-5-(trifluoromethyl)benzoyl]piperazin-1-yl]-N-methylbenzamide |
| 3A42 | N-(1-Adamantylmethyl)-4-[4-[3-[2-(5-hydroxypyridin-3-yl)ethynyl]-5-(trifluoromethyl)benzoyl]piperazin-1-yl]-N-methylbenzamide |

(continued)

| Mixture No. used | mixEI12-02R |
|---|---|
| Post-cleavage mixture No. | mixEJ06 |
| Compound No. in Table EJ06 | Compound name |
| 3A43 | N-(1-Adamantylmethyl)-4-[4-[3-[4-(3-hydroxyphenyl)pyrazol-1-yl]-5-(trifluoromethyl)benzoyl]piperazin-1-yl]-N-methylbenzamide |
| 3A44 | N-(1-Adamantylmethyl)-4-[4-[3-[4-(5-hydroxypyridin-3-yl)pyrazol-1-yl]-5-(trifluoromethyl)benzoyl]piperazin-1-yl]-N-methylbenzamide |
| 3A45 | N-(1-Adamantylmethyl)-4-[4-[[2-(3-hydroxyphenyl)phenyl]methyl]piperazin-1-yl]-N-methylbenzamide |
| 3A46 | N-(1-Adamantylmethyl)-4-[4-[[2-(5-hydroxypyridin-3-yl)phenyl]methyl]piperazin-1-yl]-N-methylbenzamide |
| 3A47 | N-(1-Adamantylmethyl)-4-[4-[[4-(3-hydroxyphenyl)-3-methylphenyl]methyl]piperazin-1-yl]-N-methylbenzamide |
| 3A48 | N-(1-Adamantylmethyl)-4-[4-[[4-(5-hydroxypyridin-3-yl)-3-methylphenyl]methyl]piperazin-1-yl]-N-methylbenzamide |
| 3A49 | N-(1-Adamantylmethyl)-4-[4-[[2-ethyl-4-(3-hydroxyphenyl)phenyl]methyl]piperazin-1-yl]-N-methylbenzamide |
| 3A50 | N-(1-Adamantylmethyl)-4-[4-[[2-ethyl-4-(5-hydroxypyridin-3-yl)phenyl]methyl]piperazin-1-yl]-N-methylbenzamide |
| 3A51 | N-(1-Adamantylmethyl)-4-[4-[[3-fluoro-4-(3-hydroxyphenyl)phenyl]methyl]piperazin-1-yl]-N-methylbenzamide |
| 3A52 | N-(1-Adamantylmethyl)-4-[4-[[3-fluoro-4-(5-hydroxypyridin-3-yl)phenyl]methyl]piperazin-1-yl]-N-methylbenzamide |
| 3A53 | N-(1-Adamantylmethyl)-4-[4-[[4-(3-hydroxyphenyl)-2-methoxyphenyl]methyl]piperazin-1-yl]-N-methylbenzamide |
| 3A54 | N-(1-Adamantylmethyl)-4-[4-[[4-(5-hydroxypyridin-3-yl)-2-methoxyphenyl]methyl]piperazin-1-yl]-N-methylbenzamide |
| 3A55 | N-(1-Adamantylmethyl)-4-[4-[[3-ethoxy-5-(3-hydroxyphenyl)phenyl]methyl]piperazin-1-yl]-N-methylbenzamide |
| 3A56 | N-(1-Adamantylmethyl)-4-[4-[[3-ethoxy-5-(5-hydroxypyridin-3-yl)phenyl]methyl]piperazin-1-yl]-N-methylbenzamide |
| 3A57 | N-(1-Adamantylmethyl)-4-[4-[[3-(3-hydroxyphenyl)-5-propan-2-yloxyphenyl]methyl]piperazin-1-yl]-N-methylbenzamide |
| 3A58 | N-(1-Adamantylmethyl)-4-[4-[[3-(5-hydroxypyridin-3-yl)-5-propan-2-yloxyphenyl]methyl]piperazin-1-yl]-N-methylbenzamide |
| 3A59 | N-(1-Adamantylmethyl)-4-[4-[[4-(3-hydroxyphenyl)-2,6-dimethoxyphenyl]methyl]piperazin-1-yl]-N-methylbenzamide |
| 3A60 | N-(1-Adamantylmethyl)-4-[4-[[4-(5-hydroxypyridin-3-yl)-2,6-dimethoxyphenyl]methyl]piperazin-1-yl]-N-methylbenzamide |
| 3A61 | N-(1-Adamantylmethyl)-4-[4-[[3-(3-hydroxyphenyl)-5-(trifluoromethyl)phenyl]methyl]piperazin-1-yl]-N-methylbenzamide |
| 3A62 | N-(1-Adamantylmethyl)-4-[4-[[3-(5-hydroxypyridin-3-yl)-5-(trifluoromethyl)phenyl]methyl]piperazin-1-yl]-N-methylbenzamide |

(continued)

| Mixture No. used | mixEI12-02R |
|---|---|
| Post-cleavage mixture No. | mixEJ06 |
| Compound No. in Table EJ06 | Compound name |
| 3A63 | N-(1-Adamantylmethyl)-4-[4-[[3-(3-hydroxyphenyl)-5-(trifluoromethoxy)phenyl]methyl]piperazin-1-yl]-N-methylbenzamide |
| 3A64 | N-(1-Adamantylmethyl)-4-[4-[[3-(5-hydroxypyridin-3-yl)-5-(trifluoromethoxy)phenyl]methyl]piperazin-1-yl]-N-methylbenzamide |
| 3A65 | N-(1-Adamantylmethyl)-4-[4-[[4-(3-hydroxyphenyl)naphthalen-1-yl]methyl]piperazin-1-yl]-N-methylbenzamide |
| 3A66 | N-(1-Adamantylmethyl)-4-[4-[[4-(5-hydroxypyridin-3-yl)naphthalen-1-yl]methyl]piperazin-1-yl]-N-methylbenzamide |
| 3A67 | N-(1-Adamantylmethyl)-4-[4-[[4-(3-hydroxyphenyl)thiophen-2-yl]methyl]piperazin-1-yl]-N-methylbenzamide |
| 3A68 | N-(1-Adamantylmethyl)-4-[4-[[4-(5-hydroxypyridin-3-yl)thiophen-2-yl]methyl]piperazin-1-yl]-N-methylbenzamide |
| 3A69 | N-(1-Adamantylmethyl)-4-[4-[[2-[2-(3-hydroxyphenyl)ethynyl]phenyl]methyl]piperazin-1-yl]-N-methylbenzamide |
| 3A70 | N-(1-Adamantylmethyl)-4-[4-[[2-[2-(5-hydroxypyridin-3-yl)ethynyl]phenyl]methyl]piperazin-1-yl]-N-methylbenzamide |
| 3A71 | N-(1-Adamantylmethyl)-6-[4-[[2-(3-hydroxyphenyl)phenyl]methyl]piperazin-1-yl]-N-methylpyridazine-3- carboxamide |
| 3A72 | N-(1-Adamantylmethyl)-6-[4-[[2-(5-hydroxypyridin-3-yl)phenyl]methyl]piperazin-1-yl]-N-methylpyridazine-3-carboxamide |
| 3A73 | N-(1-Adamantylmethyl)-6-[4-[[4-(3-hydroxyphenyl)-3-methylphenyl]methyl]piperazin-1-yl]-N-methylpyridazine-3-carboxamide |
| 3A74 | N-(1-Adamantylmethyl)-6-[4-[[4-(5-hydroxypyridin-3-yl)-3-methylphenyl]methyl]piperazin-1-yl]-N-methylpyridazine-3-carboxamide |
| 3A75 | N-(1-Adamantylmethyl)-6-[4-[[2-ethyl-4-(3-hydroxyphenyl)phenyl]methyl]piperazin-1-yl]-N-methylpyridazine-3-carboxamide |
| 3A76 | N-(1-Adamantylmethyl)-6-[4-[[2-ethyl-4-(5-hydroxypyridin-3-yl)phenyl]methyl]piperazin-1-yl]-N-methylpyridazine-3-carboxamide |
| 3A77 | N-(1-Adamantylmethyl)-6-[4-[[3-fluoro-4-(3-hydroxyphenyl)phenyl]methyl]piperazin-1-yl]-N-methylpyridazine-3-carboxamide |
| 3A78 | N-(1-Adamantylmethyl)-6-[4-[[3-fluoro-4-(5-hydroxypyridin-3-yl)phenyl]methyl]piperazin-1-yl]-N-methylpyridazine-3-carboxamide |
| 3A79 | N-(1-Adamantylmethyl)-6-[4-[[4-(3-hydroxyphenyl)-2-methoxyphenyl]methyl]piperazin-1-yl]-N-methylpyridazine-3-carboxamide |
| 3A80 | N-(1-Adamantylmethyl)-6-[4-[[4-(5-hydroxypyridin-3-yl)-2-methoxyphenyl]methyl]piperazin-1-yl]-N-methylpyridazine-3-carboxamide |
| 3A81 | N-(1-Adamantylmethyl)-6-[4-[[3-ethoxy-5-(3-hydroxyphenyl)phenyl]methyl]piperazin-1-yl]-N-methylpyridazine-3- carboxamide |
| 3A82 | N-(1-Adamantylmethyl)-6-[4-[[3-ethoxy-5-(5-hydroxypyridin-3-yl)phenyl]methyl]piperazin-1-yl]-N-methylpyridazine-3-carboxamide |

(continued)

| Mixture No. used | mixEI12-02R |
|---|---|
| Post-cleavage mixture No. | mixEJ06 |
| Compound No. in Table EJ06 | Compound name |
| 3A83 | N-(1-Adamantylmethyl)-6-[4-[[3-(3-hydroxyphenyl)-5-propan-2-yloxyphenyl]methyl]piperazin-1-yl]-N-methylpyridazine-3-carboxamide |
| 3A84 | N-(1-Adamantylmethyl)-6-[4-[[3-(5-hydroxypyridin-3-yl)-5-propan-2-yloxyphenyl]methyl]piperazin-1-yl]-N-methylpyridazine-3-carboxamide |
| 3A85 | N-(1-Adamantylmethyl)-6-[4-[[4-(3-hydroxyphenyl)-2,6-dimethoxyphenyl]methyl]piperazin-1-yl]-N-methylpyridazine-3-carboxamide |
| 3A86 | N-(1-Adamantylmethyl)-6-[4-[[4-(5-hydroxypyridin-3-yl)-2,6-dimethoxyphenyl]methyl]piperazin-1-yl]-N-methylpyridazine-3-carboxamide |
| 3A87 | N-(1-Adamantylmethyl)-6-[4-[[3-(3-hydroxyphenyl)-5-(trifluoromethyl)phenyl]methyl]piperazin-1-yl]-N-methylpyridazine-3-carboxamide |
| 3A88 | N-(1-Adamantylmethyl)-6-[4-[[3-(5-hydroxypyridin-3-yl)-5-(trifluoromethyl)phenyl]methyl]piperazin-1-yl]-N-methylpyridazine-3-carboxamide |
| 3A89 | N-(1-Adamantylmethyl)-6-[4-[[3-(3-hydroxyphenyl)-5-(trifluoromethoxy)phenyl]methyl]piperazin-1-yl]-N-methylpyridazine-3-carboxamide |
| 3A90 | N-(1-Adamantylmethyl)-6-[4-[[3-(5-hydroxypyridin-3-yl)-5-(trifluoromethoxy)phenyl]methyl]piperazin-1-yl]-N-methylpyridazine-3-carboxamide |
| 3A91 | N-(1-Adamantylmethyl)-6-[4-[[4-(3-hydroxyphenyl)naphthalen-1-yl]methyl]piperazin-1-yl]-N-methylpyridazine-3-carboxamide |
| 3A92 | N-(1-Adamantylmethyl)-6-[4-[[4-(5-hydroxypyridin-3-yl)naphthalen-1-yl]methyl]piperazin-1-yl]-N-methylpyridazine-3-carboxamide |
| 3A93 | N-(1-Adamantylmethyl)-6-[4-[[4-(3-hydroxyphenyl)thiophen-2-yl]methyl]piperazin-1-yl]-N-methylpyridazine-3-carboxamide |
| 3A94 | N-(1-Adamantylmethyl)-6-[4-[[4-(5-hydroxypyridin-3-yl)thiophen-2-yl]methyl]piperazin-1-yl]-N-methylpyridazine-3-carboxamide |
| 3A95 | N-(1-Adamantylmethyl)-6-[4-[[2-[2-(3-hydroxyphenyl)ethynyl]phenyl]methyl]piperazin-1-yl]-N-methylpyridazine-3-carboxamide |
| 3A96 | N-(1-Adamantylmethyl)-6-[4-[[2-[2-(5-hydroxypyridin-3-yl)ethynyl]phenyl]methyl]piperazin-1-yl]-N-methylpyridazine-3-carboxamide |
| 3A97 | N-(1-Adamantylmethyl)-4-[4-[1-[4-(3-hydroxyphenyl)phenyl]ethyl]piperazin-1-yl]-N-methylbenzamide |
| 3A98 | N-(1-Adamantylmethyl)-4-[4-[1-[4-(5-hydroxypyridin-3-yl)phenyl]ethyl]piperazin-1-yl]-N-methylbenzamide |
| 3A99 | N-(1-Adamantylmethyl)-4-[4-[1-[2-fluoro-4-(3-hydroxyphenyl)phenyl]ethyl]piperazin-1-yl]-N-methylbenzamide |
| 3A100 | N-(1-Adamantylmethyl)-4-[4-[1-[2-fluoro-4-(5-hydroxypyridin-3-yl)phenyl]ethyl]piperazin-1-yl]-N-methylbenzamide |

[Table 78]

[Table EU07]

| Mixture No. used | mixEI13-02R |
|---|---|
| Post-cleavage mixture No. | mixEJ07 |
| Compound No. in Table EJ07 | Compound name |
| 2I01 | 6-[4-[4-(3-Hydroxyphenyl)thiophene-2-carbonyl]piperazin-1-yl]-N-methylsulfonylpyridazine-3-carboxamide |
| 2I02 | 6-[4-[4-(5-Hydroxypyridin-3-yl)thiophene-2-carbonyl]piperazin-1-yl]-N-methylsulfonylpyridazine-3-carboxamide |
| 2I03 | 6-[4-[4-(3-Hydroxyphenyl)-3-methylbenzoyl]piperazin-1-yl]-N-methylsulfonylpyridazine-3-carboxamide |
| 2I04 | 6-[4-[4-(5-Hydroxypyridin-3-yl)-3-methylbenzoyl]piperazin-1-yl]-N-methylsulfonylpyridazine-3-carboxamide |
| 2I05 | 6-[4-[3-Fluoro-4-(3-hydroxyphenyl)benzoyl]piperazin-1-yl]-N-methylsulfonylpyridazine-3-carboxamide |
| 2I06 | 6-[4-[3-Fluoro-4-(5-hydroxypyridin-3-yl)benzoyl]piperazin-1-yl]-N-methylsulfonylpyridazine-3-carboxamide |
| 2I07 | 6-[4-[2-[2-(3-Hydroxyphenyl)ethynyl]benzoyl]piperazin-1-yl]-N-methylsulfonylpyridazine-3-carboxamide |
| 2I08 | 6-[4-[2-[2-(5-Hydroxypyridin-3-yl)ethynyl]benzoyl]piperazin-1-yl]-N-methylsulfonylpyridazine-3-carboxamide |
| 2I09 | 6-[4-[5-[2-(3-Hydroxyphenyl)ethynyl]pyridine-3-carbonyl]piperazin-1-yl]-N-methylsulfonylpyridazine-3-carboxamide |
| 2I10 | 6-[4-[5-[2-(5-Hydroxypyridin-3-yl)ethynyl]pyridine-3-carbonyl]piperazin-1-yl]-N-methylsulfonylpyridazine-3-carboxamide |
| 2I11 | 6-[4-[3-[2-(3-Hydroxyphenyl)phenyl]propanoyl]piperazin-1-yl]-N-methylsulfonylpyridazine-3-carboxamide |
| 2I12 | 6-[4-[3-[2-(5-Hydroxypyridin-3-yl)phenyl]propanoyl]piperazin-1-yl]-N-methylsulfonylpyridazine-3-carboxamide |
| 2I13 | 6-[4-[4-(3-Hydroxyphenyl)naphthalene-1-carbonyl]piperazin-1-yl]-N-methylsulfonylpyridazine-3-carboxamide |
| 2I14 | 6-[4-[4-(5-Hydroxypyridin-3-yl)naphthalene-1-carbonyl]piperazin-1-yl]-N-methylsulfonylpyridazine-3-carboxamide |
| 2I15 | 6-[4-[3-tert-Butyl-5-(3-hydroxyphenyl)benzoyl]piperazin-1-yl]-N-methylsulfonylpyridazine-3-carboxamide |
| 2I16 | 6-[4-[3-tert-Butyl-5-(5-hydroxypyridin-3-yl)benzoyl]piperazin-1-yl]-N-methylsulfonylpyridazine-3-carboxamide |
| 2I17 | 6-[4-[3-(3-Hydroxyphenyl)-5-(trifluoromethyl)benzoyl]piperazin-1-yl]-N-methylsulfonylpyridazine-3-carboxamide |
| 2I18 | 6-[4-[3-(5-Hydroxypyridin-3-yl)-5-(trifluoromethyl)benzoyl]piperazin-1-yl]-N-methylsulfonylpyridazine-3-carboxamide |
| 2I19 | 6-[4-[3-[2-(3-Hydroxyphenyl)ethynyl]-5-(trifluoromethyl)benzoyl]piperazin-1-yl]-N-methylsulfonylpyridazine-3-carboxamide |

(continued)

| Mixture No. used | mixEI13-02R |
|---|---|
| Post-cleavage mixture No. | mixEJ07 |
| Compound No. in Table EJ07 | Compound name |
| 2I20 | 6-[4-[3-[2-(5-Hydroxypyridin-3-yl)ethynyl]-5-(trifluoromethyl)benzoyl]piperazin-1-yl]-N-methylsulfonylpyridazine-3- carboxamide |
| 2I21 | 6-[4-[3-[4-(3-Hydroxyphenyl)pyrazol-1-yl]-5-(trifluoromethyl)benzoyl]piperazin-1-yl]-N-methylsulfonylpyridazine-3-carboxamide |
| 2I22 | 6-[4-[3-[4-(5-Hydroxypyridin-3-yl)pyrazol-1-yl]-5- (trifluoromethyl)benzoyl]piperazin-1-yl]-N-methylsulfonylpyridazine-3- carboxamide |
| 2I23 | 4-[4-[4-(3-Hydroxyphenyl)thiophene-2-carbonyl]piperazin-1-yl]-N-methylsulfonylbenzamide |
| 2I24 | 4-[4-[4-(5-Hydroxypyridin-3-yl)thiophene-2-carbonyl]piperazin-1-yl]-N-methylsulfonylbenzamide |
| 2I25 | 4-[4-[4-(3-Hydroxyphenyl)-3-methylbenzoyl]piperazin-1-yl]-N-methylsulfonylbenzamide |
| 2I26 | 4-[4-[4-(5-Hydroxypyridin-3-yl)-3-methylbenzoyl]piperazin-1-yl]-N-methylsulfonylbenzamide |
| 2I27 | 4-[4-[3-Fluoro-4-(3-hydroxyphenyl)benzoyl]piperazin-1-yl]-N-methylsulfonylbenzamide |
| 2I28 | 4-[4-[3-Fluoro-4-(5-hydroxypyridin-3-yl)benzoyl]piperazin-1-yl]-N-methylsulfonylbenzamide |
| 2I29 | 4-[4-[2-[2-(3-Hydroxyphenyl)ethynyl]benzoyl]piperazin-1-yl]-N-methylsulfonylbenzamide |
| 2I30 | 4-[4-[2-[2-(5-Hydroxypyridin-3-yl)ethynyl]benzoyl]piperazin-1-yl]-N-methylsulfonylbenzamide |
| 2I31 | 4-[4-[5-[2-(3-Hydroxyphenyl)ethynyl]pyridine-3-carbonyl]piperazin-1-yl]-N-methylsulfonylbenzamide |
| 2I32 | 4-[4-[5-[2-(5-Hydroxypyridin-3-yl)ethynyl]pyridine-3-carbonyl]piperazin-1-yl]-N-methylsulfonylbenzamide |
| 2I33 | 4-[4-[3-[2-(3-Hydroxyphenyl)phenyl]propanoyl]piperazin-1-yl]-N-methylsulfonylbenzamide |
| 2I34 | 4-[4-[3-[2-(5-Hydroxypyridin-3-yl)phenyl]propanoyl]piperazin-1-yl]-N-methylsulfonylbenzamide |
| 2I35 | 4-[4-[4-(3-Hydroxyphenyl)naphthalene-1-carbonyl]piperazin-1-yl]-N-methylsulfonylbenzamide |
| 2I36 | 4-[4-[4-(5-Hydroxypyridin-3-yl)naphthalene-1-carbonyl]piperazin-1-yl]-N-methylsulfonylbenzamide |
| 2I37 | 4-[4-[3-tert-Butyl-5-(3-hydroxyphenyl)benzoyl]piperazin-1-yl]-N-methylsulfonylbenzamide |
| 2I38 | 4-[4-[3-tert-Butyl-5-(5-hydroxypyridin-3-yl)benzoyl]piperazin-1-yl]-N-methylsulfonylbenzamide |
| 2I39 | 4-[4-[3-(3-Hydroxyphenyl)-5-(trifluoromethyl)benzoyl]piperazin-1-yl]-N-methylsulfonylbenzamide |
| 2I40 | 4-[4-[3-(5-Hydroxypyridin-3-yl)-5-(trifluoromethyl)benzoyl]piperazin-1-yl]-N-methylsulfonylbenzamide |
| 2I41 | 4-[4-[3-[2-(3-Hydroxyphenyl)ethynyl]-5-(trifluoromethyl)benzoyl]piperazin-1-yl]-N-methylsulfonylbenzamide |
| 2I42 | 4-[4-[3-[2-(5-Hydroxypyridin-3-yl)ethynyl]-5-(trifluoromethyl)benzoyl]piperazin-1-yl]-N-methylsulfonylbenzamide |
| 2I43 | 4-[4-[3-[4-(3-Hydroxyphenyl)pyrazol-1-yl]-5-(trifluoromethyl)benzoyl]piperazin-1-yl]-N-methylsulfonylbenzamide |
| 2I44 | 4-[4-[3-[4-(5-Hydroxypyridin-3-yl)pyrazol-1-yl]-5- (trifluoromethyl)benzoyl]piperazin-1-yl]-N-methylsulfonylbenzamide |
| 2I45 | 4-[4-[[2-(3-Hydroxyphenyl)phenyl]methyl]piperazin-1-yl]-N-methylsulfonylbenzamide |

(continued)

| Mixture No. used | mixEI13-02R |
|---|---|
| Post-cleavage mixture No. | mixEJ07 |
| Compound No. in Table EJ07 | Compound name |
| 2I46 | 4-[4-[[2-(5-Hydroxypyridin-3-yl)phenyl]methyl]piperazin-1-yl]-N-methylsulfonylbenzamide |
| 2I47 | 4-[4-[[4-(3-Hydroxyphenyl)-3-methylphenyl]methyl]piperazin-1-yl]-N-methylsulfonylbenzamide |
| 2I48 | 4-[4-[[4-(5-Hydroxypyridin-3-yl)-3-methylphenyl]methyl]piperazin-1-yl]-N-methylsulfonylbenzamide |
| 2I49 | 4-[4-[[2-Ethyl-4-(3-hydroxyphenyl)phenyl]methyl]piperazin-1-yl]-N-methylsulfonylbenzamide |
| 2I50 | 4-[4-[[2-Ethyl-4-(5-hydroxypyridin-3-yl)phenyl]methyl]piperazin-1-yl]-N-methylsulfonylbenzamide |
| 2I51 | 4-[4-[[3-Fluoro-4-(3-hydroxyphenyl)phenyl]methyl]piperazin-1-yl]-N-methylsulfonylbenzamide |
| 2I52 | 4-[4-[[3-Fluoro-4-(5-hydroxypyridin-3-yl)phenyl]methyl]piperazin-1-yl]-N-methylsulfonylbenzamide |
| 2I53 | 4-[4-[[4-(3-Hydroxyphenyl)-2-methoxyphenyl]methyl]piperazin-1-yl]-N-methylsulfonylbenzamide |
| 2I54 | 4-[4-[[4-(5-Hydroxypyridin-3-yl)-2-methoxyphenyl]methyl]piperazin-1-yl]-N-methylsulfonylbenzamide |
| 2I55 | 4-[4-[[3-Ethoxy-5-(3-hydroxyphenyl)phenyl]methyl]piperazin-1-yl]-N-methylsulfonylbenzamide |
| 2I56 | 4-[4-[[3-Ethoxy-5-(5-hydroxypyridin-3-yl)phenyl]methyl]piperazin-1-yl]-N-methylsulfonylbenzamide |
| 2I57 | 4-[4-[[3-(3-Hydroxyphenyl)-5-propan-2-yloxyphenyl]methyl]piperazin-1-yl]-N-methylsulfonylbenzamide |
| 2I58 | 4-[4-[[3-(5-Hydroxypyridin-3-yl)-5-propan-2-yloxyphenyl]methyl]piperazin-1-yl]-N-methylsulfonylbenzamide |
| 2I59 | 4-[4-[[4-(3-Hydroxyphenyl)-2,6-dimethoxyphenyl]methyl]piperazin-1-yl]-N-methylsulfonylbenzamide |
| 2I60 | 4-[4-[[4-(5-Hydroxypyridin-3-yl)-2,6-dimethoxyphenyl]methyl]piperazin-1-yl]-N-methylsulfonylbenzamide |
| 2I61 | 4-[4-[[3-(3-Hydroxyphenyl)-5-(trifluoromethyl)phenyl]methyl]piperazin-1-yl]-N-methylsulfonylbenzamide |
| 2I62 | 4-[4-[[3-(5-Hydroxypyridin-3-yl)-5-(trifluoromethyl)phenyl]methyl]piperazin-1-yl]-N-methylsulfonylbenzamide |
| 2I63 | 4-[4-[[3-(3-Hydroxyphenyl)-5-(trifluoromethoxy)phenyl]methyl]piperazin-1-yl]-N-methylsulfonylbenzamide |
| 2I64 | 4-[4-[[3-(5-Hydroxypyridin-3-yl)-5-(trifluoromethoxy)phenyl]methyl]piperazin-1-yl]-N-methylsulfonylbenzamide |
| 2I65 | 4-[4-[[4-(3-Hydroxyphenyl)naphthalen-1-yl]methyl]piperazin-1-yl]-N-methylsulfonylbenzamide |
| 2I66 | 4-[4-[[4-(5-Hydroxypyridin-3-yl)naphthalen-1-yl]methyl]piperazin-1-yl]-N-methylsulfonylbenzamide |
| 2I67 | 4-[4-[[4-(3-Hydroxyphenyl)thiophen-2-yl]methyl]piperazin-1-yl]-N-methylsulfonylbenzamide |
| 2I68 | 4-[4-[[4-(5-Hydroxypyridin-3-yl)thiophen-2-yl]methyl]piperazin-1-yl]-N-methylsulfonylbenzamide |
| 2I69 | 4-[4-[[2-[2-(3-Hydroxyphenyl)ethynyl]phenyl]methyl]piperazin-1-yl]-N-methylsulfonylbenzamide |

(continued)

| Mixture No. used | mixEI13-02R |
|---|---|
| Post-cleavage mixture No. | mixEJ07 |
| Compound No. in Table EJ07 | Compound name |
| 2I70 | 4-[4-[[2-[2-(5-Hydroxypyridin-3-yl)ethynyl]phenyl]methyl]piperazin-1-yl]-N-methylsulfonylbenzamide |
| 2I71 | 6-[4-[[2-(3-Hydroxyphenyl)phenyl]methyl]piperazin-1-yl]-N-methylsulfonylpyridazine-3-carboxamide |
| 2I72 | 6-[4-[[2-(5-Hydroxypyridin-3-yl)phenyl]methyl]piperazin-1-yl]-N-methylsulfonylpyridazine-3-carboxamide |
| 2I73 | 6-[4-[[4-(3-Hydroxyphenyl)-3-methylphenyl]methyl]piperazin-1-yl]-N-methylsulfonylpyridazine-3-carboxamide |
| 2I74 | 6-[4-[[4-(5-Hydroxypyridin-3-yl)-3-methylphenyl]methyl]piperazin-1-yl]-N-methylsulfonylpyridazine-3-carboxamide |
| 2I75 | 6-[4-[[2-Ethyl-4-(3-hydroxyphenyl)phenyl]methyl]piperazin-1-yl]-N-methylsulfonylpyridazine-3-carboxamide |
| 2I76 | 6-[4-[[2-Ethyl-4-(5-hydroxypyridin-3-yl)phenyl]methyl]piperazin-1-yl]-N-methylsulfonylpyridazine-3-carboxamide |
| 2I77 | 6-[4-[[3-Fluoro-4-(3-hydroxyphenyl)phenyl]methyl]piperazin-1-yl]-N-methylsulfonylpyridazine-3-carboxamide |
| 2I78 | 6-[4-[[3-Fluoro-4-(5-hydroxypyridin-3-yl)phenyl]methyl]piperazin-1-yl]-N-methylsulfonylpyridazine-3-carboxamide |
| 2I79 | 6-[4-[[4-(3-Hydroxyphenyl)-2-methoxyphenyl] methyl]piperazin-1-yl]-N-methylsulfonylpyridazine-3-carboxamide |
| 2I80 | 6-[4-[[4-(5-Hydroxypyridin-3-yl)-2-methoxyphenyl]methyl]piperazin-1-yl]-N-methylsulfonylpyridazine-3-carboxamide |
| 2I81 | 6-[4-[[3-Ethoxy-5-(3-hydroxyphenyl)phenyl]methyl]piperazin-1-yl]-N-methylsulfonylpyridazine-3-carboxamide |
| 2I82 | 6-[4-[[3-Ethoxy-5-(5-hydroxypyridin-3-yl)phenyl]methyl]piperazin-1-yl]-N-methylsulfonylpyridazine-3-carboxamide |
| 2I83 | 6-[4-[[3-(3-Hydroxyphenyl)-5-propan-2-yloxyphenyl]methyl]piperazin-1-yl]-N-methylsulfonylpyridazine-3-carboxamide |
| 2I84 | 6-[4-[[3-(5-Hydroxypyridin-3-yl)-5-propan-2-yloxyphenyl]methyl]piperazin-1-yl]-N-methylsulfonylpyridazine-3-carboxamide |
| 2I85 | 6-[4-[[4-(3-Hydroxyphenyl)-2,6-dimethoxyphenyl]methyl]piperazin-1-yl]-N-methylsulfonylpyridazine-3-carboxamide |
| 2I86 | 6-[4-[[4-(5-Hydroxypyridin-3-yl)-2,6-dimethoxyphenyl]methyl]piperazin-1-yl]-N-methylsulfonylpyridazine-3-carboxamide |
| 2I87 | 6-[4-[[3-(3-Hydroxyphenyl)-5-(trifluoromethyl)phenyl]methyl]piperazin-1-yl]-N-methylsulfonylpyridazine-3-carboxamide |
| 2I88 | 6-[4-[[3-(5-Hydroxypyridin-3-yl)-5-(trifluoromethyl)phenyl]methyl]piperazin-1-yl]-N-methylsulfonylpyridazine-3-carboxamide |
| 2I89 | 6-[4-[[3-(3-Hydroxyphenyl)-5-(trifluoromethoxy)phenyl]methyl]piperazin-1-yl]-N-methylsulfonylpyridazine-3-carboxamide |

(continued)

| Mixture No. used | mixEI13-02R |
|---|---|
| Post-cleavage mixture No. | mixEJ07 |
| Compound No. in Table EJ07 | Compound name |
| 2I90 | 6-[4-[[3-(5-Hydroxypyridin-3-yl)-5-(trifluoromethoxy)phenyl]methyl]piperazin-1-yl]-N-methylsulfonylpyridazine-3-carboxamide |
| 2I91 | 6-[4-[[4-(3-Hydroxyphenyl)naphthalen-1-yl]methyl]piperazin-1-yl]-N-methylsulfonylpyridazine-3-carboxamide |
| 2I92 | 6-[4-[[4-(5-Hydroxypyridin-3-yl)naphthalen-1-yl]methyl]piperazin-1-yl]-N-methylsulfonylpyridazine-3-carboxamide |
| 2I93 | 6-[4-[[4-(3-Hydroxyphenyl)thiophen-2-yl]methyl]piperazin-1-yl]-N-methylsulfonylpyridazine-3-carboxamide |
| 2I94 | 6-[4-[[4-(5-Hydroxypyridin-3-yl)thiophen-2-yl]methyl]piperazin-1-yl]-N-methylsulfonylpyridazine-3-carboxamide |
| 2I95 | 6-[4-[[2-[2-(3-Hydroxyphenyl)ethynyl]phenyl]methyl]piperazin-1-yl]-N-methylsulfonylpyridazine-3-carboxamide |
| 2I96 | 6-[4-[[2-[2-(5-Hydroxypyridin-3-yl)ethynyl]phenyl]methyl]piperazin-1-yl]-N-methylsulfonylpyridazine-3-carboxamide |
| 2I97 | 4-[4-[1-[4-(3-Hydroxyphenyl)phenyl]ethyl]piperazin-1-yl]-N-methylsulfonylbenzamide |
| 2I98 | 4-[4-[1-[4-(5-Hydroxypyridin-3-yl)phenyl]ethyl]piperazin-1-yl]-N-methylsulfonylbenzamide |
| 2I99 | 4-[4-[1-[2-Fluoro-4-(3-hydroxyphenyl)phenyl]ethyl]piperazin-1-yl]-N-methylsulfonylbenzamide |
| 2I100 | 4-[4-[1-[2-Fluoro-4-(5-hydroxypyridin-3-yl)phenyl]ethyl]piperazin-1-yl]-N-methylsulfonylbenzamide |

[Table 79]

[Table EIJ08]

| Mixture No. used | mixEI14-02R |
|---|---|
| Post-cleavage mixture No. | mixEJ08 |
| Compound No. in Table EJ08 | Compound name |
| 2L01 | N-tert-Butylsulfonyl-6-[4-[4-(3-hydroxyphenyl)thiophene-2-carbonyl]piperazin-1-yl]pyridazine-3-carboxamide |
| 2L02 | N-tert-Butylsulfonyl-6-[4-[4-(5-hydroxypyridin-3-yl)thiophene-2-carbonyl]piperazin-1-yl]pyridazine-3-carboxamide |
| 2L03 | N-tert-Butylsulfonyl-6-[4-[4-(3-hydroxyphenyl)-3-methylbenzoyl]piperazin-1- yl]pyridazine-3-carboxamide |
| 2L04 | N-tert-Butylsulfonyl-6-[4-[4-(5-hydroxypyridin-3-yl)-3-methylbenzoyl]piperazin-1-yl]pyridazine-3-carboxamide |

(continued)

| Mixture No. used | mixEI14-02R |
|---|---|
| Post-cleavage mixture No. | mixEJ08 |
| Compound No. in Table EJ08 | Compound name |
| 2L05 | N-tert-Butylsulfonyl-6-[4-[3-fluoro-4-(3-hydroxyphenyl)benzoyl]piperazin-1-yl]pyridazine-3-carboxamide |
| 2L06 | N-tert-Butylsulfonyl-6-[4-[3-fluoro-4-(5-hydroxypyridin-3-yl)benzoyl]piperazin-1-yl]pyridazine-3-carboxamide |
| 2L07 | N-tert-Butylsulfonyl-6-[4-[2-[2-(3-hydroxyphenyl)ethynyl]benzoyl]piperazin-1-yl]pyridazine-3-carboxamide |
| 2L08 | N-tert-Butylsulfonyl-6-[4-[2-[2-(5-hydroxypyridin-3-yl)ethynyl]benzoyl]piperazin-1-yl]pyridazine-3-carboxamide |
| 2L09 | N-tert-Butylsulfonyl-6-[4-[5-[2-(3-hydroxyphenyl)ethynyl]pyridine-3-carbonyl]piperazin-1-yl]pyridazine-3-carboxamide |
| 2L10 | N-tert-Butylsulfonyl-6-[4-[5-[2-(5-hydroxypyridin-3-yl)ethynyl]pyridine-3-carbonyl]piperazin-1-yl]pyridazine-3-carboxamide |
| 2L11 | N-tert-Butylsulfonyl-6-[4-[3-[2-(3-hydroxyphenyl)phenyl]propanoyl]piperazin-1-yl]pyridazine-3-carboxamide |
| 2L12 | N-tert-Butylsulfonyl-6-[4-[3-[2-(5-hydroxypyridin-3-yl)phenyl]propanoyl]piperazin-1-yl]pyridazine-3-carboxamide |
| 2L13 | N-tert-Butylsulfonyl-6-[4-[4-(3-hydroxyphenyl)naphthalene-1-carbonyl]piperazin-1-yl]pyridazine-3-carboxamide |
| 2L14 | N-tert-Butylsulfonyl-6-[4-[4-(5-hydroxypyridin-3-yl)naphthalene-1-carbonyl]piperazin-1-yl]pyridazine-3-carboxamide |
| 2L15 | 6-[4-[3-tert-Butyl-5-(3-hydroxyphenyl)benzoyl]piperazin-1-yl]-N-tert-butylsulfonylpyridazine-3-carboxamide |
| 2L16 | 6-[4-[3-tert-Butyl-5-(5-hydroxypyridin-3-yl)benzoyl]piperazin-1-yl]-N-tert-butylsulfonylpyridazine-3-carboxamide |
| 2L17 | N-tert-Butylsulfonyl-6-[4-[3-(3-hydroxyphenyl)-5-(trifluoromethyl)benzoyl]piperazin-1-yl]pyridazine-3-carboxamide |
| 2L18 | N-tert-Butylsulfonyl-6-[4-[3-(5-hydroxypyridin-3-yl)-5-(trifluoromethyl)benzoyl]piperazin-1-yl]pyridazine-3-carboxamide |
| 2L19 | N-tert-Butylsulfonyl-6-[4-[3-[2-(3-hydroxyphenyl)ethynyl]-5-(trifluoromethyl)benzoyl]piperazin-1-yl]pyridazine-3-carboxamide |
| 2L20 | N-tert-Butylsulfonyl-6-[4-[3-[2-(5-hydroxypyridin-3-yl)ethynyl]-5-(trifluoromethyl)benzoyl]piperazin-1-yl]pyridazine-3-carboxamide |
| 2L21 | N-tert-Butylsulfonyl-6-[4-[3-[4-(3-hydroxyphenyl)pyrazol-1-yl]-5-(trifluoromethyl)benzoyl]piperazin-1-yl]pyridazine-3-carboxamide |
| 2L22 | N-tert-Butylsulfonyl-6-[4-[3-[4-(5-hydroxypyridin-3-yl)pyrazol-1-yl]-5-(trifluoromethyl)benzoyl]piperazin-1-yl]pyridazine-3-carboxamide |
| 2L23 | N-tert-Butylsulfonyl-4-[4-[4-(3-hydroxyphenyl)thiophene-2-carbonyl]piperazin-1-yl]benzamide |
| 2L24 | N-tert-Butylsulfonyl-4-[4-[4-(5-hydroxypyridin-3-yl)thiophene-2-carbonyl]piperazin-1-yl]benzamide |

(continued)

| Mixture No. used | mixEI14-02R |
|---|---|
| Post-cleavage mixture No. | mixEJ08 |
| Compound No. in Table EJ08 | Compound name |
| 2L25 | N-tert-Butylsulfonyl-4-[4-[4-(3-hydroxyphenyl)-3-methylbenzoyl]piperazin-1- yl]benzamide |
| 2L26 | N-tert-Butylsulfonyl-4-[4-[4-(5-hydroxypyridin-3-yl)-3-methylbenzoyl]piperazin-1-yl]benzamide |
| 2L27 | N-tert-Butylsulfonyl-4-[4-[3-fluoro-4-(3-hydroxyphenyl)benzoyl]piperazin-1\ yl]benzamide |
| 2L28 | N-tert-Butylsulfonyl-4-[4-[3-fluoro-4-(5-hydroxypyridin-3-yl)benzoyl]piperazin-1-yl]benzamide |
| 2L29 | N-tert-Butylsulfonyl-4-[4-[2-[2-(3-hydroxyphenyl)ethynyl]benzoyl]piperazin-1-yl]benzamide |
| 2L30 | N-tert-Butylsulfonyl-4-[4-[2-[2-(5-hydroxypyridin-3-yl)ethynyl]benzoyl]piperazin-1-yl]benzamide |
| 2L31 | N-tert-Butylsulfonyl-4-[4-[5-[2-(3-hydroxyphenyl)ethynyl]pyridine-3-carbonyl]piperazin-1-yl]benzamide |
| 2L32 | N-tert-Butylsulfonyl-4-[4-[5-[2-(5-hydroxypyridin-3-yl)ethynyl]pyridine-3-carbonyl]piperazin-1-yl]benzamide |
| 2L33 | N-tert-Butylsulfonyl-4-[4-[3-[2-(3-hydroxyphenyl)phenyl]propanoyl]piperazin-1-yl]benzamide |
| 2L34 | N-tert-Butylsulfonyl-4-[4-[3-[2-(5-hydroxypyridin-3-yl)phenyl]propanoyl]piperazin-1-yl]benzamide |
| 2L35 | N-tert-Butylsulfonyl-4-[4-[4-(3-hydroxyphenyl)naphthalene-1-carbonyl]piperazin-1-yl]benzamide |
| 2L36 | N-tert-Butylsulfonyl-4-[4-[4-(5-hydroxypyridin-3-yl)naphthalene-1- carbonyl]piperazin-1-yl]benzamide |
| 2L37 | 4-[4-[3-tert-Butyl-5-(3-hydroxyphenyl)benzoyl]piperazin-1-yl]-N-tert-butylsulfonylbenzamide |
| 2L38 | 4-[4-[3-tert-Butyl-5-(5-hydroxypyridin-3-yl)benzoyl]piperazin-1-yl]-N-tert-butylsulfonylbenzamide |
| 2L39 | N-tert-Butylsulfonyl-4-[4-[3-(3-hydroxyphenyl)-5-(trifluoromethyl)benzoyl]piperazin-1-yl]benzamide |
| 2L40 | N-tert-Butylsulfonyl-4-[4-[3-(5-hydroxypyridin-3-yl)-5-(trifluoromethyl)benzoyl]piperazin-1-yl]benzamide |
| 2L41 | N-tert-Butylsulfonyl-4-[4-[3-[2-(3-hydroxyphenyl)ethynyl]-5-(trifluoromethyl)benzoyl]piperazin-1-yl]benzamide |
| 2L42 | N-tert-Butylsulfonyl-4-[4-[3-[2-(5-hydroxypyridin-3-yl)ethynyl]-5-(trifluoromethyl)benzoyl]piperazin-1-yl]benzamide |
| 2L43 | N-tert-Butylsulfonyl-4-[4-[3-[4-(3-hydroxyphenyl)pyrazol-1-yl]-5-(trifluoromethyl)benzoyl]piperazin-1-yl]benzamide |
| 2L44 | N-tert-Butylsulfonyl-4-[4-[3-[4-(5-hydroxypyridin-3-yl)pyrazol-1-yl]-5-(trifluoromethyl)benzoyl]piperazin-1-yl]benzamide |
| 2L45 | N-tert-Butylsulfonyl-4-[4-[[2-(3-hydroxyphenyl)phenyl]methyl]piperazin-1-yl]benzamide |
| 2L46 | N-tert-Butylsulfonyl-4-[4-[[2-(5-hydroxypyridin-3-yl)phenyl]methyl]piperazin-1-yl]benzamide |
| 2L47 | N-tert-Butylsulfonyl-4-[4-[[4-(3-hydroxyphenyl)-3-methylphenyl]methyl]piperazin-1-yl]benzamide |
| 2L48 | N-tert-Butylsulfonyl-4-[4-[[4-(5-hydroxypyridin-3-yl)-3-methylphenyl]methyl]piperazin-1-yl]benzamide |
| 2L49 | N-tert-Butylsulfonyl-4-[4-[[2-ethyl-4-(3-hydroxyphenyl)phenyl]methyl]piperazin-1-yl]benzamide |
| 2L50 | N-tert-Butylsulfonyl-4-[4-[[2-ethyl-4-(5-hydroxypyridin-3-yl)phenyl]methyl]piperazin-1-yl]benzamide |

(continued)

| Mixture No. used | mixEI14-02R |
|---|---|
| Post-cleavage mixture No. | mixEJ08 |
| Compound No. in Table EJ08 | Compound name |
| 2L51 | N-tert-Butylsulfonyl-4-[4-[[3-fluoro-4-(3-hydroxyphenyl)phenyl]methyl]piperazin-1-yl]benzamide |
| 2L52 | N-tert-Butylsulfonyl-4-[4-[[3-fluoro-4-(5-hydroxypyridin-3-yl)phenyl]methyl]piperazin-1-yl]benzamide |
| 2L53 | N-tert-Butylsulfonyl-4-[4-[[4-(3-hydroxyphenyl)-2-methoxyphenyl]methyl]piperazin-1-yl]benzamide |
| 2L54 | N-tert-Butylsulfonyl-4-[4-[[4-(5-hydroxypyridin-3-yl)-2-methoxyphenyl]methyl]piperazin-1-yl]benzamide |
| 2L55 | N-tert-Butylsulfonyl-4-[4-[[3-ethoxy-5-(3-hydroxyphenyl)phenyl]methyl]piperazin-1-yl]benzamide |
| 2L56 | N-tert-Butylsulfonyl-4-[4-[[3-ethoxy-5-(5-hydroxypyridin-3-yl)phenyl]methyl]piperazin-1-yl]benzamide |
| 2L57 | N-tert-Butylsulfonyl-4-[4-[[3-(3-hydroxyphenyl)-5-propan-2-yloxyphenyl]methyl]piperazin-1-yl]benzamide |
| 2L58 | N-tert-Butylsulfonyl-4-[4-[[3-(5-hydroxypyridin-3-yl)-5-propan-2-yloxyphenyl]methyl]piperazin-1-yl]benzamide |
| 2L59 | N-tert-Butylsulfonyl-4-[4-[[4-(3-hydroxyphenyl)-2,6-dimethoxyphenyl]methyl]piperazin-1-yl]benzamide |
| 2L60 | N-tert-Butylsulfonyl-4-[4-[[4-(5-hydroxypyridin-3-yl)-2,6-dimethoxyphenyl]methyl]piperazin-1-yl]benzamide |
| 2L61 | N-tert-Butylsulfonyl-4-[4-[[3-(3-hydroxyphenyl)-5-(trifluoromethyl)phenyl]methyl]piperazin-1-yl]benzamide |
| 2L62 | N-tert-Butylsulfonyl-4-[4-[[3-(5-hydroxypyridin-3-yl)-5-(trifluoromethyl)phenyl]methyl]piperazin-1-yl]benzamide |
| 2L63 | N-tert-Butylsulfonyl-4-[4-[[3-(3-hydroxyphenyl)-5-(trifluoromethoxy)phenyl]methyl]piperazin-1-yl]benzamide |
| 2L64 | N-tert-Butylsulfonyl-4-[4-[[3-(5-hydroxypyridin-3-yl)-5-(trifluoromethoxy)phenyl]methyl]piperazin-1-yl]benzamide |
| 2L65 | N-tert-Butylsulfonyl-4-[4-[[4-(3-hydroxyphenyl)naphthalen-1-yl]methyl]piperazin-1-yl]benzamide |
| 2L66 | N-tert-Butylsulfonyl-4-[4-[[4-(5-hydroxypyridin-3-yl)naphthalen-1-yl]methyl]piperazin-1-yl]benzamide |
| 2L67 | N-tert-Butylsulfonyl-4-[4-[[4-(3-hydroxyphenyl)thiophen-2-yl]methyl]piperazin-1-yl]benzamide |
| 2L68 | N-tert-Butylsulfonyl-4-[4-[[4-(5-hydroxypyridin-3-yl)thiophen-2-yl]methyl]piperazin-1-yl]benzamide |
| 2L69 | N-tert-Butylsulfonyl-4-[4-[[2-[2-(3-hydroxyphenyl)ethynyl]phenyl]methyl]piperazin-1-yl]benzamide |
| 2L70 | N-tert-Butylsulfonyl-4-[4-[[2-[2-(5-hydroxypyridin-3-yl)ethynyl]phenyl]methyl]piperazin-1-yl]benzamide |
| 2L71 | N-tert-Butylsulfonyl-6-[4-[[2-(3-hydroxyphenyl)phenyl]methyl]piperazin-1-yl]pyridazine-3-carboxamide |
| 2L72 | N-tert-Butylsulfonyl-6-[4-[[2-(5-hydroxypyridin-3-yl)phenyl]methyl]piperazin-1-yl]pyridazine-3-carboxamide |

(continued)

| Mixture No. used | mixEI14-02R |
|---|---|
| Post-cleavage mixture No. | mixEJ08 |
| Compound No. in Table EJ08 | Compound name |
| 2L73 | N-tert-Butylsulfonyl-6-[4-[[4-(3-hydroxyphenyl)-3-methylphenyl]methyl]piperazin-1-yl]pyridazine-3-carboxamide |
| 2L74 | N-tert-Butylsulfonyl-6-[4-[[4-(5-hydroxypyridin-3-yl)-3-methylphenyl]methyl]piperazin-1-yl]pyridazine-3-carboxamide |
| 2L75 | N-tert-Butylsulfonyl-6-[4-[[2-ethyl-4-(3-hydroxyphenyl)phenyl]methyl]piperazin-1-yl]pyridazine-3-carboxamide |
| 2L76 | N-tert-Butylsulfonyl-6-[4-[[2-ethyl-4-(5-hydroxypyridin-3-yl)phenyl]methyl]piperazin-1-yl]pyridazine-3-carboxamide |
| 2L77 | N-tert-Butylsulfonyl-6-[4-[[3-fluoro-4-(3-hydroxyphenyl)phenyl]methyl]piperazin-1-yl]pyridazine-3-carboxamide |
| 2L78 | N-tert-Butylsulfonyl-6-[4-[[3-fluoro-4-(5-hydroxypyridin-3-yl)phenyl]methyl]piperazin-1-yl]pyridazine-3-carboxamide |
| 2L79 | N-tert-Butylsulfonyl-6-[4-[[4-(3-hydroxyphenyl)-2-methoxyphenyl]methyl]piperazin-1-yl]pyridazine-3-carboxamide |
| 2L80 | N-tert-Butylsulfonyl-6-[4-[[4-(5-hydroxypyridin-3-yl)-2-methoxyphenyl]methyl]piperazin-1-yl]pyridazine-3-carboxamide |
| 2L81 | N-tert-Butylsulfonyl-6-[4-[[3-ethoxy-5-(3-hydroxyphenyl)phenyl]methyl]piperazin-1-yl]pyridazine-3-carboxamide |
| 2L82 | N-tert-Butylsulfonyl-6-[4-[[3-ethoxy-5-(5-hydroxypyridin-3-yl)phenyl]methyl]piperazin-1-yl]pyridazine-3-carboxamide |
| 2L83 | N-tert-Butylsulfonyl-6-[4-[[3-(3-hydroxyphenyl)-5-propan-2-yloxyphenyl]methyl]piperazin-1-yl]pyridazine-3-carboxamide |
| 2L84 | N-tert-Butylsulfonyl-6-[4-[[3-(5-hydroxypyridin-3-yl)-5-propan-2-yloxyphenyl]methyl]piperazin-1-yl]pyridazine-3-carboxamide |
| 2L85 | N-tert-Butylsulfonyl-6-[4-[[4-(3-hydroxyphenyl)-2,6-dimethoxyphenyl]methyl]piperazin-1-yl]pyridazine-3-carboxamide |
| 2L86 | N-tert-Butylsulfonyl-6-[4-[[4-(5-hydroxypyridin-3-yl)-2,6-dimethoxyphenyl]methyl]piperazin-1-yl]pyridazine-3-carboxamide |
| 2L87 | N-tert-Butylsulfonyl-6-[4-[[3-(3-hydroxyphenyl)-5-(trifluoromethyl)phenyl]methyl]piperazin-1-yl]pyridazine-3-carboxamide |
| 2L88 | N-tert-Butylsulfonyl-6-[4-[[3-(5-hydroxypyridin-3-yl)-5-(trifluoromethyl)phenyl]methyl]piperazin-1-yl]pyridazine-3-carboxamide |
| 2L89 | N-tert-Butylsulfonyl-6-[4-[[3-(3-hydroxyphenyl)-5-(trifluoromethoxy)phenyl]methyl]piperazin-1-yl]pyridazine-3-carboxamide |
| 2L90 | N-tert-Butylsulfonyl-6-[4-[[3-(5-hydroxypyridin-3-yl)-5-(trifluoromethoxy)phenyl]methyl]piperazin-1-yl]pyridazine-3-carboxamide |
| 2L91 | N-tert-Butylsulfonyl-6-[4-[[4-(3-hydroxyphenyl)naphthalen-1-yl]methyl]piperazin-1-yl]pyridazine-3-carboxamide |
| 2L92 | N-tert-Butylsulfonyl-6-[4-[[4-(5-hydroxypyridin-3-yl)naphthalen-1-yl]methyl]piperazin-1-yl]pyridazine-3-carboxamide |

(continued)

| Mixture No. used | mixEI14-02R |
|---|---|
| Post-cleavage mixture No. | mixEJ08 |
| Compound No. in Table EJ08 | Compound name |
| 2L93 | N-tert-Butylsulfonyl-6-[4-[[4-(3-hydroxyphenyl)thiophen-2-yl]methyl]piperazin-1-yl]pyridazine-3-carboxamide |
| 2L94 | N-tert-Butylsulfonyl-6-[4-[[4-(5-hydroxypyridin-3-yl)thiophen-2-yl]methyl]piperazin-1-yl]pyridazine-3-carboxamide |
| 2L95 | N-tert-Butylsulfonyl-6-[4-[[2-[2-(3-hydroxyphenyl)ethynyl]phenyl]methyl]piperazin-1-yl]pyridazine-3- carboxamide |
| 2L96 | N-tert-Butylsulfonyl-6-[4-[[2-[2-(5-hydroxypyridin-3-yl)ethynyl]phenyl]methyl]piperazin-1-yl]pyridazine-3-carboxamide |
| 2L97 | N-tert-Butylsulfonyl-4-[4-[1-[4-(3-hydroxyphenyl)phenyl]ethyl]piperazin-1-yl]benzamide |
| 2L98 | N-tert-Butylsulfonyl-4-[4-[1-[4-(5-hydroxypyridin-3-yl)phenyl]ethyl]piperazin-1-yl]benzamide |
| 2L99 | N-tert-Butylsulfonyl-4-[4-[1-[2-fluoro-4-(3-hydroxyphenyl)phenyl]ethyl]piperazin-1-yl]benzamide |
| 2L100 | N-tert-Butylsulfonyl-4-[4-[1-[2-fluoro-4-(5-hydroxypyridin-3-yl)phenyl]ethyl]piperazin-1-yl]benzamide |

[Table 80]

[Table EIJ09]

| Mixture No. used | mixEI15-02R |
|---|---|
| Post-cleavage mixture No. | mixEJ09 |
| Compound No. in Table EJ09 | Compound name |
| 2K01 | 6-[4-[4-(3-Hydroxyphenyl)thiophene-2-carbonyl]piperazin-1-yl]-N-(3,3,3-trifluoropropylsulfonyl)pyridazine-3-carboxamide |
| 2K02 | 6-[4-[4-(5-Hydroxypyridin-3-yl)thiophene-2-carbonyl]piperazin-1-yl]-N-(3,3,3-trifluoropropylsulfonyl)pyridazine-3-carboxamide |
| 2K03 | 6-[4-[4-(3-Hydroxyphenyl)-3-methylbenzoyl]piperazin-1-yl]-N-(3,3,3-trifluoropropylsulfonyl)pyridazine-3-carboxamide |
| 2K04 | 6-[4-[4-(5-Hydroxypyridin-3-yl)-3-methylbenzoyl]piperazin-1-yl]-N-(3,3,3-trifluoropropylsulfonyl)pyridazine-3-carboxamide |
| 2K05 | 6-[4-[3-Fluoro-4-(3-hydroxyphenyl)benzoyl]piperazin-1-yl]-N-(3,3,3-trifluoropropylsulfonyl)pyridazine-3-carboxamide |
| 2K06 | 6-[4-[3-Fluoro-4-(5-hydroxypyridin-3-yl)benzoyl]piperazin-1-yl]-N-(3,3,3-trifluoropropylsulfonyl)pyridazine-3-carboxamide |
| 2K07 | 6-[4-[2-[2-(3-Hydroxyphenyl)ethynyl]benzoyl]piperazin-1-yl]-N-(3,3,3-trifluoropropylsulfonyl)pyridazine-3-carboxamide |

(continued)

| Mixture No. used | mixEI15-02R |
|---|---|
| Post-cleavage mixture No. | mixEJ09 |
| Compound No. in Table EJ09 | Compound name |
| 2K08 | 6-[4-[2-[2-(5-Hydroxypyridin-3-yl)ethynyl]benzoyl]piperazin-1-yl]-N-(3,3,3-trifluoropropylsulfonyl)pyridazine-3-carboxamide |
| 2K09 | 6-[4-[5-[2-(3-Hydroxyphenyl)ethynyl]pyridine-3-carbonyl]piperazin-1-yl]-N-(3,3,3-trifluoropropylsulfonyl)pyridazine-3-carboxamide |
| 2K10 | 6-[4-[5-[2-(5-Hydroxypyridin-3-yl)ethynyl]pyridine-3-carbonyl]piperazin-1-yl]-N -(3,3,3-trifluoropropylsulfonyl)pyridazine-3 -carboxamide |
| 2K11 | 6-[4-[3-[2-(3-Hydroxyphenyl)phenyl]propanoyl]piperazin-1-yl]-N-(3,3,3-trifluoropropylsulfonyl)pyridazine-3-carboxamide |
| 2K12 | 6-[4-[3-[2-(5-Hydroxypyridin-3-yl)phenyl]propanoyl]piperazin-1-yl]-N-(3,3,3-trifluoropropylsulfonyl)pyridazine-3-carboxamide |
| 2K13 | 6-[4-[4-(3-Hydroxyphenyl)naphthalene-1-carbonyl]piperazin-1-yl]-N-(3,3,3-trifluoropropylsulfonyl)pyridazine-3-carboxamide |
| 2K14 | 6-[4-[4-(5-Hydroxypyridin-3-yl)naphthalene-1-carbonyl]piperazin-1-yl]-N-(3,3,3-trifluoropropylsulfonyl)pyridazine-3-carboxamide |
| 2K15 | 6-[4-[3-tert-Butyl-5-(3-hydroxyphenyl)benzoyl]piperazin-1-yl]-N-(3,3,3-trifluoropropylsulfonyl)pyridazine-3-carboxamide |
| 2K16 | 6-[4-[3-tert-Butyl-5-(5-hydroxypyridin-3-yl)benzoyl]piperazin-1-yl]-N-(3,3,3-trifluoropropylsulfonyl)pyridazine-3-carboxamide |
| 2K17 | 6-[4-[3-(3-Hydroxyphenyl)-5-(trifluoromethyl)benzoyl]piperazin-1-yl]-N-(3,3,3-trifluoropropylsulfonyl)pyridazine-3-carboxamide |
| 2K18 | 6-[4-[3-(5-Hydroxypyridin-3-yl)-5-(trifluoromethyl)benzoyl]piperazin-1-yl]-N-(3,3,3-trifluoropropylsulfonyl)pyridazine-3-carboxamide |
| 2K19 | 6-[4-[3-[2-(3-Hydroxyphenyl)ethynyl]-5-(trifluoromethyl)benzoyl]piperazin-1- yl]-N-(3,3,3-trifluoropropylsulfonyl)pyridazine-3-carboxamide |
| 2K20 | 6-[4-[3-[2-(5-Hydroxypyridin-3-yl)ethynyl]-5-(trifluoromethyl)benzoyl]piperazin-1-yl]-N-(3,3,3-trifluoropropylsulfonyl)pyridazine-3-carboxamide |
| 2K21 | 6-[4-[3-[4-(3-Hydroxyphenyl)pyrazol-1-yl]-5-(trifluoromethyl)benzoyl]piperazin-1-yl]-N-(3,3,3-trifluoropropylsulfonyl)pyridazine-3-carboxamide |
| 2K22 | 6-[4-[3-[4-(5-Hydroxypyridin-3-yl)pyrazol-1-yl]-5-(trifluoromethyl)benzoyl]piperazin-1-yl]-N-(3,3,3-trifluoropropylsulfonyl)pyridazine-3-carboxamide |
| 2K23 | 4-[4-[4-(3-Hydroxyphenyl)thiophene-2-carbonyl]piperazin-1-yl]-N-(3,3,3-trifluoropropylsulfonyl)benzamide |
| 2K24 | 4-[4-[4-(5-Hydroxypyridin-3-yl)thiophene-2-carbonyl]piperazin-1-yl]-N-(3,3,3-trifluoropropylsulfonyl)benzamide |
| 2K25 | 4-[4-[4-(3-Hydroxyphenyl)-3-methylbenzoyl]piperazin-1-yl]-N-(3,3,3-trifluoropropylsulfonyl)benzamide |
| 2K26 | 4-[4-[4-(5-Hydroxypyridin-3-yl)-3-methylbenzoyl]piperazin-1-yl]-N-(3,3,3-trifluoropropylsulfonyl)benzamide |
| 2K27 | 4-[4-[3-Fluoro-4-(3-hydroxyphenyl)benzoyl]piperazin-1-yl]-N-(3,3,3-trifluoropropylsulfonyl)benzamide |

(continued)

| Mixture No. used | mixEI15-02R |
|---|---|
| Post-cleavage mixture No. | mixEJ09 |
| Compound No. in Table EJ09 | Compound name |
| 2K28 | 4-[4-[3-Fluoro-4-(5-hydroxypyridin-3-yl)benzoyl]piperazin-1-yl]-N-(3,3,3-trifluoropropylsulfonyl)benzamide |
| 2K29 | 4-[4-[2-[2-(3-Hydroxyphenyl)ethynyl]benzoyl]piperazin-1-yl]-N-(3,3,3-trifluoropropylsulfonyl)benzamide |
| 2K30 | 4-[4-[2-[2-(5-Hydroxypyridin-3-yl)ethynyl]benzoyl]piperazin-1-yl]-N-(3,3,3-trifluoropropylsulfonyl)benzamide |
| 2K31 | 4-[4-[5-[2-(3-Hydroxyphenyl)ethynyl]pyridine-3-carbonyl]piperazin-1-yl]-N-(3,3,3-trifluoropropylsulfonyl)benzamide |
| 2K32 | 4-[4-[5-[2-(5-Hydroxypyridin-3-yl)ethynyl]pyridine-3-carbonyl]piperazin-1-yl]-N-(3,3,3-trifluoropropylsulfonyl)benzamide |
| 2K33 | 4-[4-[3-[2-(3-Hydroxyphenyl)phenyl]propanoyl]piperazin-1-yl]-N-(3,3,3-trifluoropropylsulfonyl)benzamide |
| 2K34 | 4-[4-[3-[2-(5-Hydroxypyridin-3-yl)phenyl]propanoyl]piperazin-1-yl]-N-(3,3,3-trifluoropropylsulfonyl)benzamide |
| 2K35 | 4-[4-[4-(3-Hydroxyphenyl)naphthalene-1-carbonyl]piperazin-1-yl]-N-(3,3,3-trifluoropropylsulfonyl)benzamide |
| 2K36 | 4-[4-[4-(5-Hydroxypyridin-3-yl)naphthalene-1-carbonyl]piperazin-1-yl]-N-(3,3,3-trifluoropropylsulfonyl)benzamide |
| 2K37 | 4-[4-[3-tert-Butyl-5-(3-hydroxyphenyl)benzoyl]piperazin-1-yl]-N-(3,3,3-trifluoropropylsulfonyl)benzamide |
| 2K38 | 4-[4-[3-tert-Butyl-5-(5-hydroxypyridin-3-yl)benzoyl]piperazin-1-yl]-N-(3,3,3-trifluoropropylsulfonyl)benzamide |
| 2K39 | 4-[4-[3-(3-Hydroxyphenyl)-5-(trifluoromethyl)benzoyl]piperazin-1-yl]-N-(3,3,3-trifluoropropylsulfonyl)benzamide |
| 2K40 | 4-[4-[3-(5-Hydroxypyridin-3-yl)-5-(trifluoromethyl)benzoyl]piperazin-1-yl]-N-(3,3,3-trifluoropropylsulfonyl)benzamide |
| 2K41 | 4-[4-[3-[2-(3-Hydroxyphenyl)ethynyl]-5-(trifluoromethyl)benzoyl]piperazin-1- yl]-N-(3,3,3-trifluoropropylsulfonyl)benzamide |
| 2K42 | 4-[4-[3-[2-(5-Hydroxypyridin-3-yl)ethynyl]-5-(trifluoromethyl)benzoyl]piperazin-1-yl]-N-(3,3,3 -trifluoropropylsulfonyl)benzamide |
| 2K43 | 4-[4-[3-[4-(3-Hydroxyphenyl)pyrazol-1-yl]-5-(trifluoromethyl)benzoyl]piperazin-1-yl]-N-(3,3,3-trifluoropropylsulfonyl)benzamide |
| 2K44 | 4-[4-[3-[4-(5-Hydroxypyridin-3-yl)pyrazol-1-yl]-5- (trifluoromethyl)benzoyl]piperazin-1-yl]-N-(3,3,3- trifluoropropylsulfonyl)benzamide |
| 2K45 | 4-[4-[[2-(3-Hydroxyphenyl)phenyl]methyl]piperazin-1-yl]-N-(3,3,3-trifluoropropylsulfonyl)benzamide |
| 2K46 | 4-[4-[[2-(5-Hydroxypyridin-3-yl)phenyl]methyl]piperazin-1-yl]-N-(3,3,3-trifluoropropylsulfonyl)benzamide |
| 2K47 | 4-[4-[[4-(3-Hydroxyphenyl)-3-methylphenyl]methyl]piperazin-1-yl]-N-(3,3,3-trifluoropropylsulfonyl)benzamide |

(continued)

| Mixture No. used | mixEI15-02R |
|---|---|
| Post-cleavage mixture No. | mixEJ09 |
| Compound No. in Table EJ09 | Compound name |
| 2K48 | 4-[4-[[4-(5-Hydroxypyridin-3-yl)-3-methylphenyl]methyl]piperazin-1-yl]-N-(3,3,3-trifluoropropylsulfonyl)benzamide |
| 2K49 | 4-[4-[[2-Ethyl-4-(3-hydroxyphenyl)phenyl]methyl]piperazin-1-yl]-N-(3,3,3-trifluoropropylsulfonyl)benzamide |
| 2K50 | 4-[4-[[2-Ethyl-4-(5-hydroxypyridin-3-yl)phenyl]methyl]piperazin-1-yl]-N-(3,3,3-trifluoropropylsulfonyl)benzamide |
| 2K51 | 4-[4-[[3-Fluoro-4-(3-hydroxyphenyl)phenyl]methyl]piperazin-1-yl]-N-(3,3,3-trifluoropropylsulfonyl)benzamide |
| 2K52 | 4-[4-[[3 -Fluoro-4-(5-hydroxypyridin-3-yl)phenyl]methyl]piperazin-1-yl]-N-(3,3,3-trifluoropropylsulfonyl)benzamide |
| 2K53 | 4-[4-[[4-(3-Hydroxyphenyl)-2-methoxyphenyl]methyl]piperazin-1-yl]-N-(3,3,3-trifluoropropylsulfonyl)benzamide |
| 2K54 | 4-[4-[[4-(5-Hydroxypyridin-3-yl)-2-methoxyphenyl]methyl]piperazin-1-yl]-N-(3,3,3-trifluoropropylsulfonyl)benzamide |
| 2K55 | 4-[4-[[3-Ethoxy-5-(3-hydroxyphenyl)phenyl]methyl]piperazin-1-yl]-N-(3,3,3-trifluoropropylsulfonyl)benzamide |
| 2K56 | 4-[4-[[3-Ethoxy-5-(5-hydroxypyridin-3-yl)phenyl]methyl]piperazin-1-yl]-N-(3,3,3-trifluoropropylsulfonyl)benzamide |
| 2K57 | 4-[4-[[3-(3-Hydroxyphenyl)-5-propan-2-yloxyphenyl]methyl]piperazin-1-yl]-N-(3,3,3-trifluoropropylsulfonyl)benzamide |
| 2K58 | 4-[4-[[3-(5-Hydroxypyridin-3-yl)-5-propan-2-yloxyphenyl]methyl]piperazin-1-yl]-N-(3,3,3-trifluoropropylsulfonyl)benzamide |
| 2K59 | 4-[4-[[4-(3-Hydroxyphenyl)-2,6-dimethoxyphenyl]methyl]piperazin-1-yl]-N-(3,3,3-trifluoropropylsulfonyl)benzamide |
| 2K60 | 4-[4-[[4-(5-Hydroxypyridin-3-yl)-2,6-dimethoxyphenyl]methyl]piperazin-1-yl]-N-(3,3,3-trifluoropropylsulfonyl)benzamide |
| 2K61 | 4-[4-[[3-(3-Hydroxyphenyl)-5-(trifluoromethyl)phenyl]methyl]piperazin-1-yl]-N-(3,3,3-trifluoropropylsulfonyl)benzamide |
| 2K62 | 4-[4-[[3-(5-Hydroxypyridin-3-yl)-5-(trifluoromethyl)phenyl]methyl]piperazin-1-yl]-N-(3,3,3-trifluoropropylsulfonyl)benzamide |
| 2K63 | 4-[4-[[3-(3-Hydroxyphenyl)-5-(trifluoromethoxy)phenyl]methyl]piperazin-1-yl]-N-(3,3,3-trifluoropropylsulfonyl)benzamide |
| 2K64 | 4-[4-[[3-(5-Hydroxypyridin-3-yl)-5-(trifluoromethoxy)phenyl]methyl]piperazin-1-yl]-N-(3,3,3-trifluoropropylsulfonyl)benzamide |
| 2K65 | 4-[4-[[4-(3-Hydroxyphenyl)naphthalen-1-yl]methyl]piperazin-1-yl]-N-(3,3,3-trifluoropropylsulfonyl)benzamide |
| 2K66 | 4-[4-[[4-(5-Hydroxypyridin-3-yl)naphthalen-1-yl]methyl]piperazin-1-yl]-N-(3,3,3-trifluoropropylsulfonyl)benzamide |
| 2K67 | 4-[4-[[4-(3-Hydroxyphenyl)thiophen-2-yl]methyl]piperazin-1-yl]-N-(3,3,3-trifluoropropylsulfonyl)benzamide |

(continued)

| Mixture No. used | mixEI15-02R |
|---|---|
| Post-cleavage mixture No. | mixEJ09 |
| Compound No. in Table EJ09 | Compound name |
| 2K68 | 4-[4-[[4-(5-Hydroxypyridin-3-yl)thiophen-2-yl]methyl]piperazin-1-yl]-N-(3,3,3-trifluoropropylsulfonyl)benzamide |
| 2K69 | 4-[4-[[2-[2-(3-Hydroxyphenyl)ethynyl]phenyl]methyl]piperazin-1-yl]-N-(3,3,3-trifluoropropylsulfonyl)benzamide |
| 2K70 | 4-[4-[[2-[2-(5-Hydroxypyridin-3-yl)ethynyl]phenyl]methyl]piperazin-1-yl]-N-(3,3,3-trifluoropropylsulfonyl)benzamide |
| 2K71 | 6-[4-[[2-(3-Hydroxyphenyl)phenyl]methyl]piperazin-1-yl]-N-(3,3,3-trifluoropropylsulfonyl)pyridazine-3-carboxamide |
| 2K72 | 6-[4-[[2-(5-Hydroxypyridin-3-yl)phenyl]methyl]piperazin-1-yl]-N-(3,3,3-trifluoropropylsulfonyl)pyridazine-3-carboxamide |
| 2K73 | 6-[4-[[4-(3-Hydroxyphenyl)-3-methylphenyl]methyl]piperazin-1-yl]-N-(3,3,3-trifluoropropylsulfonyl)pyridazine-3-carboxamide |
| 2K74 | 6-[4-[[4-(5-Hydroxypyridin-3-yl)-3-methylphenyl]methyl]piperazin-1-yl]-N-(3,3,3-trifluoropropylsulfonyl)pyridazine-3-carboxamide |
| 2K75 | 6-[4-[[2-Ethyl-4-(3-hydroxyphenyl)phenyl]methyl]piperazin-1-yl]-N-(3,3,3-trifluoropropylsulfonyl)pyridazine-3-carboxamide |
| 2K76 | 6-[4-[[2-Ethyl-4-(5-hydroxypyridin-3-yl)phenyl]methyl]piperazin-1-yl]-N-(3,3,3-trifluoropropylsulfonyl)pyridazine-3-carboxamide |
| 2K77 | 6-[4-[[3-Fluoro-4-(3-hydroxyphenyl)phenyl]methyl]piperazin-1-yl]-N-(3,3,3-trifluoropropylsulfonyl)pyridazine-3-carboxamide |
| 2K78 | 6-[4-[[3-Fluoro-4-(5-hydroxypyridin-3-yl)phenyl]methyl]piperazin-1-yl]-N-(3,3,3-trifluoropropylsulfonyl)pyridazine-3-carboxamide |
| 2K79 | 6-[4-[[4-(3-Hydroxyphenyl)-2-methoxyphenyl]methyl]piperazin-1-yl]-N-(3,3,3-trifluoropropylsulfonyl)pyridazine-3-carboxamide |
| 2K80 | 6-[4-[[4-(5-Hydroxypyridin-3-yl)-2-methoxyphenyl]methyl]piperazin-1-yl]-N-(3,3,3-trifluoropropylsulfonyl)pyridazine-3-carboxamide |
| 2K81 | 6-[4-[[3-Ethoxy-5-(3-hydroxyphenyl)phenyl]methyl]piperazin-1-yl]-N-(3,3,3-trifluoropropylsulfonyl)pyridazine-3-carboxamide |
| 2K82 | 6-[4-[[3-Ethoxy-5-(5-hydroxypyridin-3-yl)phenyl]methyl]piperazin-1-yl]-N-(3,3,3-trifluoropropylsulfonyl)pyridazine-3-carboxamide |
| 2K83 | 6-[4-[[3-(3-Hydroxyphenyl)-5-propan-2-yloxyphenyl]methyl]piperazin-1-yl]-N-(3,3,3-trifluoropropylsulfonyl)pyridazine-3-carboxamide |
| 2K84 | 6-[4-[[3-(5-Hydroxypyridin-3-yl)-5-propan-2-yloxyphenyl]methyl]piperazin-1-yl]-N -(3,3,3-trifluoropropylsulfonyl)pyridazine-3 -carboxamide |
| 2K85 | 6-[4-[[4-(3-Hydroxyphenyl)-2,6-dimethoxyphenyl]methyl]piperazin-1-yl]-N-(3,3,3-trifluoropropylsulfonyl)pyridazine-3-carboxamide |
| 2K86 | 6-[4-[[4-(5-Hydroxypyridin-3-yl)-2,6-dimethoxyphenyl]methyl]piperazin-1- yl]-N-(3,3,3-trifluoropropylsulfonyl)pyridazine-3-carboxamide |
| 2K87 | 6-[4-[[3-(3-Hydroxyphenyl)-5-(trifluoromethyl)phenyl]methyl]piperazin-1-yl]-N-(3,3,3-trifluoropropylsulfonyl)pyridazine-3-carboxamide |

(continued)

| Mixture No. used | mixEI15-02R |
|---|---|
| Post-cleavage mixture No. | mixEJ09 |
| Compound No. in Table EJ09 | Compound name |
| 2K88 | 6-[4-[[3-(5-Hydroxypyridin-3-yl)-5-(trifluoromethyl)phenyl]methyl]piperazin-1-yl]-N-(3,3,3-trifluoropropylsulfonyl)pyridazine-3-carboxamide |
| 2K89 | 6-[4-[[3-(3-Hydroxyphenyl)-5-(trifluoromethoxy)phenyl]methyl]piperazin-1-yl]-N-(3,3,3-trifluoropropylsulfonyl)pyridazine-3-carboxamide |
| 2K90 | 6-[4-[[3-(5-Hydroxypyridin-3-yl)-5-(trifluoromethoxy)phenyl]methyl]piperazin-1-yl]-N-(3,3,3-trifluoropropylsulfonyl)pyridazine-3-carboxamide |
| 2K91 | 6-[4-[[4-(3-Hydroxyphenyl)naphthalen-1-yl]methyl]piperazin-1-yl]-N-(3,3,3-trifluoropropylsulfonyl)pyridazine-3-carboxamide |
| 2K92 | 6-[4-[[4-(5-Hydroxypyridin-3-yl)naphthalen-1-yl]methyl]piperazin-1-yl]-N-(3,3,3-trifluoropropylsulfonyl)pyridazine-3-carboxamide |
| 2K93 | 6-[4-[[4-(3-Hydroxyphenyl)thiophen-2-yl]methyl]piperazin-1-yl]-N-(3,3,3-trifluoropropylsulfonyl)pyridazine-3-carboxamide |
| 2K94 | 6-[4-[[4-(5-Hydroxypyridin-3-yl)thiophen-2-yl]methyl]piperazin-1-yl]-N-(3,3,3-trifluoropropylsulfonyl)pyridazine-3-carboxamide |
| 2K95 | 6-[4-[[2-[2-(3-Hydroxyphenyl)ethynyl]phenyl]methyl]piperazin-1-yl]-N-(3,3,3-trifluoropropylsulfonyl)pyridazine-3-carboxamide |
| 2K96 | 6-[4-[[2-[2-(5-Hydroxypyridin-3-yl)ethynyl]phenyl]methyl]piperazin-1-yl]-N-(3,3,3-trifluoropropylsulfonyl)pyridazine-3-carboxamide |
| 2K97 | 4-[4-[1-[4-(3-Hydroxyphenyl)phenyl]ethyl]piperazin-1-yl]-N-(3,3,3-trifluoropropylsulfonyl)benzamide |
| 2K98 | 4-[4-[1-[4-(5-Hydroxypyridin-3-yl)phenyl]ethyl]piperazin-1-yl]-N-(3,3,3-trifluoropropylsulfonyl)benzamide |
| 2K99 | 4-[4-[1-[2-Fluoro-4-(3-hydroxyphenyl)phenyl]ethyl]piperazin-1-yl]-N-(3,3,3-trifluoropropylsulfonyl)benzamide |
| 2K100 | 4-[4-[1-[2-Fluoro-4-(5-hydroxypyridin-3-yl)phenyl]ethyl]piperazin-1-yl]-N-(3,3,3-trifluoropropylsulfonyl)benzamide |

[Table 81]

[Table EIJ10]

| Mixture No. used | mixEI16-02R |
|---|---|
| Post-cleavage mixture No. | mixEJ10 |
| Compound No. in Table EJ10 | Compound name |
| 2A01 | N-(1-Adamantylmethylsulfonyl)-6-[4-[4-(3-hydroxyphenyl)thiophene-2-carbonyl]piperazin-1-yl]pyridazine-3-carboxamide |

(continued)

| Mixture No. used | mixEI16-02R |
|---|---|
| Post-cleavage mixture No. | mixEJ10 |
| Compound No. in Table EJ10 | Compound name |
| 2A02 | N-(1-Adamantylmethylsulfonyl)-6-[4-[4-(5-hydroxypyridin-3-yl)thiophene-2-carbonyl]piperazin-1-yl]pyridazine-3-carboxamide |
| 2A03 | N-(1-Adamantylmethylsulfonyl)-6-[4-[4-(3-hydroxyphenyl)-3-methylbenzoyl]piperazin-1-yl]pyridazine-3-carboxamide |
| 2A04 | N-(1-Adamantylmethylsulfonyl)-6-[4-[4-(5-hydroxypyridin-3-yl)-3-methylbenzoyl]piperazin-1-yl]pyridazine-3-carboxamide |
| 2A05 | N-(1-Adamantylmethylsulfonyl)-6-[4-[3-fluoro-4-(3-hydroxyphenyl)benzoyl]piperazin-1-yl]pyridazine-3-carboxamide |
| 2A06 | N-(1-Adamantylmethylsulfonyl)-6-[4-[3-fluoro-4-(5-hydroxypyridin-3-yl)benzoyl]piperazin-1-yl]pyridazine-3-carboxamide |
| 2A07 | N-(1-Adamantylmethylsulfonyl)-6-[4-[2-[2-(3-hydroxyphenyl)ethynyl]benzoyl]piperazin-1-yl]pyridazine-3-carboxamide |
| 2A08 | N-(1-Adamantylmethylsulfonyl)-6-[4-[2-[2-(5-hydroxypyridin-3-yl)ethynyl]benzoyl]piperazin-1-yl]pyridazine-3-carboxamide |
| 2A09 | N-(1-Adamantylmethylsulfonyl)-6-[4-[5-[2-(3-hydroxyphenyl)ethynyl]pyridine-3 -carbonyl]piperazin-1-yl]pyridazine- 3-carboxamide |
| 2A10 | N-(1-Adamantylmethylsulfonyl)-6-[4-[5-[2-(5-hydroxypyridin-3-yl)ethynyl]pyridine-3-carbonyl]piperazin-1-yl]pyridazine-3-carboxamide |
| 2A11 | N-(1-Adamantylmethylsulfonyl)-6-[4-[3-[2-(3-hydroxyphenyl)phenyl]propanoyl]piperazin-1-yl]pyridazine-3-carboxamide |
| 2A12 | N-(1-Adamantylmethylsulfonyl)-6-[4-[3-[2-(5-hydroxypyridin-3-yl)phenyl]propanoyl]piperazin-1-yl]pyridazine-3-carboxamide |
| 2A13 | N-(1-Adamantylmethylsulfonyl)-6-[4-[4-(3-hydroxyphenyl)naphthalene-1-carbonyl]piperazin-1-yl]pyridazine-3-carboxamide |
| 2A14 | N-(1-Adamantylmethylsulfonyl)-6-[4-[4-(5-hydroxypyridin-3-yl)naphthalene-1-carbonyl]piperazin-1-yl]pyridazine-3-carboxamide |
| 2A15 | N-(1-Adamantylmethylsulfonyl)-6-[4-[3-tert-butyl-5-(3-hydroxyphenyl)benzoyl]piperazin-1-yl]pyridazine-3-carboxamide |
| 2A16 | N-(1-Adamantylmethylsulfonyl)-6-[4-[3-tert-butyl-5-(5-hydroxypyridin-3-yl)benzoyl]piperazin-1-yl]pyridazine-3-carboxamide |
| 2A17 | N-(1-Adamantylmethylsulfonyl)-6-[4-[3-(3-hydroxyphenyl)-5-(trifluoromethyl)benzoyl]piperazin-1-yl]pyridazine-3-carboxamide |
| 2A18 | N-(1-Adamantylmethylsulfonyl)-6-[4-[3-(5-hydroxypyridin-3-yl)-5-(trifluoromethyl)benzoyl]piperazin-1-yl]pyridazine-3-carboxamide |
| 2A19 | N-(1-Adamantylmethylsulfonyl)-6-[4-[3-[2-(3-hydroxyphenyl)ethynyl]-5-(trifluoromethyl)benzoyl]piperazin-1-yl]pyridazine-3-carboxamide |
| 2A20 | N-(l-Adamantylmethylsulfonyl)-6-[4-[3-[2-(5-hydroxypyridin-3-yl)ethynyl]-5-(trifluoromethyl)benzoyl]piperazin-1-yl]pyridazine-3-carboxamide |
| 2A21 | N-(1-Adamantylmethylsulfonyl)-6-[4-[3-[4-(3-hydroxyphenyl)pyrazol-1-yl]-5-(trifluoromethyl)benzoyl]piperazin-1-yl]pyridazine-3-carboxamide |

(continued)

| Mixture No. used | mixEI16-02R |
|---|---|
| Post-cleavage mixture No. | mixEJ10 |
| Compound No. in Table EJ10 | Compound name |
| 2A22 | N-(1-Adamantylmethylsulfonyl)-6-[4-[3-[4-(5-hydroxypyridin-3-yl)pyrazol-1-yl]-5-(trifluoromethyl)benzoyl]piperazin-1-yl]pyridazine-3-carboxamide |
| 2A23 | N-(1-Adamantylmethylsulfonyl)-4-[4-[4-(3-hydroxyphenyl)thiophene-2-carbonyl]piperazin-1-yl]benzamide |
| 2A24 | N-(1-Adamantylmethylsulfonyl)-4-[4-[4-(5-hydroxypyridin-3-yl)thiophene-2-carbonyl]piperazin-1-yl]benzamide |
| 2A25 | N-(1-Adamantylmethylsulfonyl)-4-[4-[4-(3-hydroxyphenyl)-3-methylbenzoyl]piperazin-1-yl]benzamide |
| 2A26 | N-(1-Adamantylmethylsulfonyl)-4-[4-[4-(5-hydroxypyridin-3-yl)-3-methylbenzoyl]piperazin-1-yl]benzamide |
| 2A27 | N-(1-Adamantylmethylsulfonyl)-4-[4-[3-fluoro-4-(3-hydroxyphenyl)benzoyl]piperazin-1-yl]benzamide |
| 2A28 | N-(1-Adamantylmethylsulfonyl)-4-[4-[3-fluoro-4-(5-hydroxypyridin-3-yl)benzoyl]piperazin-1-yl]benzamide |
| 2A29 | N-(1-Adamantylmethylsulfonyl)-4-[4-[2-[2-(3-hydroxyphenyl)ethynyl]benzoyl]piperazin-1-yl]benzamide |
| 2A30 | N-(1-Adamantylmethylsulfonyl)-4-[4-[2-[2-(5-hydroxypyridin-3-yl)ethynyl]benzoyl]piperazin-1-yl]benzamide |
| 2A31 | N-(1-Adamantylmethylsulfonyl)-4-[4-[5-[2-(3-hydroxyphenyl)ethynyl]pyridine-3-carbonyl]piperazin-1-yl]benzamide |
| 2A32 | N-(1-Adamantylmethylsulfonyl)-4-[4-[5-[2-(5-hydroxypyridin-3-yl)ethynyl]pyridine-3-carbonyl]piperazin-1-yl]benzamide |
| 2A33 | N-(1-Adamantylmethylsulfonyl)-4-[4-[3-[2-(3-hydroxyphenyl)phenyl]propanoyl]piperazin-1-yl]benzamide |
| 2A34 | N-(1-Adamantylmethylsulfonyl)-4-[4-[3-[2-(5-hydroxypyridin-3-yl)phenyl]propanoyl]piperazin-1-yl]benzamide |
| 2A35 | N-(1-Adamantylmethylsulfonyl)-4-[4-[4-(3-hydroxyphenyl)naphthalene-1-carbonyl]piperazin-1-yl]benzamide |
| 2A36 | N-(1-Adamantylmethylsulfonyl)-4-[4-[4-(5-hydroxypyridin-3-yl)naphthalene-1-carbonyl]piperazin-1-yl]benzamide |
| 2A37 | N-(1-Adamantylmethylsulfonyl)-4-[4-[3 -tert-butyl-5-(3-hydroxyphenyl)benzoyl]piperazin-1-yl]benzamide |
| 2A38 | N-(1-Adamantylmethylsulfonyl)-4-[4-[3-tert-butyl-5-(5-hydroxypyridin-3-yl)benzoyl]piperazin-1-yl]benzamide |
| 2A39 | N-(1-Adamantylmethylsulfonyl)-4-[4-[3-(3-hydroxyphenyl)-5-(trifluoromethyl)benzoyl]piperazin-1-yl]benzamide |
| 2A40 | N-(1-Adamantylmethylsulfonyl)-4-[4-[3-(5-hydroxypyridin-3-yl)-5-(trifluoromethyl)benzoyl]piperazin-1-yl]benzamide |
| 2A41 | N-(1-Adamantylmethylsulfonyl)-4-[4-[3-[2-(3-hydroxyphenyl)ethynyl]-5-(trifluoromethyl)benzoyl]piperazin-1 -yl]benzamide |

(continued)

| Mixture No. used | mixEI16-02R |
|---|---|
| Post-cleavage mixture No. | mixEJ10 |
| Compound No. in Table EJ10 | Compound name |
| 2A42 | N-(1-Adamantylmethylsulfonyl)-4-[4-[3-[2-(5-hydroxypyridin-3-yl)ethynyl]-5-(trifluoromethyl)benzoyl]piperazin-1-yl]benzamide |
| 2A43 | N-(1-Adamantylmethylsulfonyl)-4-[4-[3-[4-(3-hydroxyphenyl)pyrazol-1-yl]-5-(trifluoromethyl)benzoyl]piperazin-1 -yl]benzamide |
| 2A44 | N-(1-Adamantylmethylsulfonyl)-4-[4-[3-[4-(5-hydroxypyridin-3-yl)pyrazol-1-yl]-5-(trifluoromethyl)benzoyl]piperazin-1-yl]benzamide |
| 2A45 | N-(1-Adamantylmethylsulfonyl)-4-[4-[[2-(3-hydroxyphenyl)phenyl]methyl]piperazin-1-yl]benzamide |
| 2A46 | N-(1-Adamantylmethylsulfonyl)-4-[4-[[2-(5-hydroxypyridin-3-yl)phenyl]methyl]piperazin-1-yl]benzamide |
| 2A47 | N-(1-Adamantylmethylsulfonyl)-4-[4-[[4-(3-hydroxyphenyl)-3-methylphenyl]methyl]piperazin-1-yl]benzamide |
| 2A48 | N-(1-Adamantylmethylsulfonyl)-4-[4-[[4-(5-hydroxypyridin-3-yl)-3-methylphenyl]methyl]piperazin-1-yl]benzamide |
| 2A49 | N-(1-Adamantylmethylsulfonyl)-4-[4-[[2-ethyl-4-(3-hydroxyphenyl)phenyl]methyl]piperazin-1-yl]benzamide |
| 2A50 | N-(1-Adamantylmethylsulfonyl)-4-[4-[[2-ethyl-4-(5-hydroxypyridin-3-yl)phenyl]methyl]piperazin-1-yl]benzamide |
| 2A51 | N-(1-Adamantylmethylsulfonyl)-4-[4-[[3-fluoro-4-(3-hydroxyphenyl)phenyl]methyl]piperazin-1-yl]benzamide |
| 2A52 | N-(1-Adamantylmethylsulfonyl)-4-[4-[[3-fluoro-4-(5-hydroxypyridin-3-yl)phenyl]methyl]piperazin-1-yl]benzamide |
| 2A53 | N-(1-Adamantylmethylsulfonyl)-4-[4-[[4-(3-hydroxyphenyl)-2-methoxyphenyl]methyl]piperazin-1-yl]benzamide |
| 2A54 | N-(1-Adamantylmethylsulfonyl)-4-[4-[[4-(5-hydroxypyridin-3-yl)-2-methoxyphenyl]methyl]piperazin-1-yl]benzamide |
| 2A55 | N-(1-Adamantylmethylsulfonyl)-4-[4-[[3-ethoxy-5-(3-hydroxyphenyl)phenyl]methyl]piperazin-1-yl]benzamide |
| 2A56 | N-(1-Adamantylmethylsulfonyl)-4-[4-[[3-ethoxy-5-(5-hydroxypyridin-3-yl)phenyl]methyl]piperazin-1-yl]benzamide |
| 2A57 | N-(1-Adamantylmethylsulfonyl)-4-[4-[[3-(3-hydroxyphenyl)-5-propan-2-yloxyphenyl]methyl]piperazin-1-yl]benzamide |
| 2A58 | N-(1-Adamantylmethylsulfonyl)-4-[4-[[3-(5-hydroxypyridin-3-yl)-5-propan-2-yloxyphenyl]methyl]piperazin-1-yl]benzamide |
| 2A59 | N-(1-Adamantylmethylsulfonyl)-4-[4-[[4-(3-hydroxyphenyl)-2,6-dimethoxyphenyl]methyl]piperazin-1-yl]benzamide |
| 2A60 | N-(1-Adamantylmethylsulfonyl)-4-[4-[[4-(5-hydroxypyridin-3-yl)-2,6-dimethoxyphenyl]methyl]piperazin-1-yl]benzamide |
| 2A61 | N-(1-Adamantylmethylsulfonyl)-4-[4-[[3-(3-hydroxyphenyl)-5-(trifluoromethyl)phenyl]methyl]piperazin-1-yl]benzamide |

(continued)

| Mixture No. used | mixEI16-02R |
|---|---|
| Post-cleavage mixture No. | mixEJ10 |
| Compound No. in Table EJ10 | Compound name |
| 2A62 | N-(1-Adamantylmethylsulfonyl)-4-[4-[[3-(5-hydroxypyridin-3-yl)-5-(trifluoromethyl)phenyl]methyl]piperazin-1-yl]benzamide |
| 2A63 | N-(1-Adamantylmethylsulfonyl)-4-[4-[[3-(3-hydroxyphenyl)-5-(trifluoromethoxy)phenyl]methyl]piperazin-1-yl]benzamide |
| 2A64 | N-(1-Adamantylmethylsulfonyl)-4-[4-[[3-(5-hydroxypyridin-3-yl)-5-(trifluoromethoxy)phenyl]methyl]piperazin-1-yl]benzamide |
| 2A65 | N-(1-Adamantylmethylsulfonyl)-4-[4-[[4-(3-hydroxyphenyl)naphthalen-1-yl]methyl]piperazin-1-yl]benzamide |
| 2A66 | N-(1-Adamantylmethylsulfonyl)-4-[4-[[4-(5-hydroxypyridin-3-yl)naphthalen-1-yl]methyl]piperazin-1-yl]benzamide |
| 2A67 | N-(1-Adamantylmethylsulfonyl)-4-[4-[[4-(3-hydroxyphenyl)thiophen-2-yl]methyl]piperazin-1-yl]benzamide |
| 2A68 | N-(1-Adamantylmethylsulfonyl)-4-[4-[[4-(5-hydroxypyridin-3-yl)thiophen-2-yl]methyl]piperazin-1-yl]benzamide |
| 2A69 | N-(1-Adamantylmethylsulfonyl)-4-[4-[[2-[2-(3-hydroxyphenyl)ethynyl]phenyl]methyl]piperazin-1-yl]benzamide |
| 2A70 | N-(1-Adamantylmethylsulfonyl)-4-[4-[[2-[2-(5-hydroxypyridin-3-yl)ethynyl]phenyl]methyl]piperazin-1-yl]benzamide |
| 2A71 | N-(1-Adamantylmethylsulfonyl)-6-[4-[[2-(3-hydroxyphenyl)phenyl]methyl]piperazin-1-yl]pyridazine-3-carboxamide |
| 2A72 | N-(1-Adamantylmethylsulfonyl)-6-[4-[[2-(5-hydroxypyridin-3-yl)phenyl]methyl]piperazin-1-yl]pyridazine-3-carboxamide |
| 2A73 | N-(1-Adamantylmethylsulfonyl)-6-[4-[[4-(3-hydroxyphenyl)-3-methylphenyl]methyl]piperazin-1-yl]pyridazine-3-carboxamide |
| 2A74 | N-(1-Adamantylmethylsulfonyl)-6-[4-[[4-(5-hydroxypyridin-3-yl)-3-methylphenyl]methyl]piperazin-1-yl]pyridazine-3-carboxamide |
| 2A75 | N-(1-Adamantylmethylsulfonyl)-6-[4-[[2-ethyl-4-(3-hydroxyphenyl)phenyl]methyl]piperazin-1-yl]pyridazine-3-carboxamide |
| 2A76 | N-(1-Adamantylmethylsulfonyl)-6-[4-[[2-ethyl-4-(5-hydroxypyridin-3-yl)phenyl]methyl]piperazin-1-yl]pyridazine-3-carboxamide |
| 2A77 | N-(1-Adamantylmethylsulfonyl)-6-[4-[[3-fluoro-4-(3-hydroxyphenyl)phenyl]methyl]piperazin-1-yl]pyridazine-3-carboxamide |
| 2A78 | N-(1-Adamantylmethylsulfonyl)-6-[4-[[3-fluoro-4-(5-hydroxypyridin-3-yl)phenyl]methyl]piperazin-1-yl]pyridazine-3-carboxamide |
| 2A79 | N-(1-Adamantylmethylsulfonyl)-6-[4-[[4-(3-hydroxyphenyl)-2-methoxyphenyl]methyl]piperazin-1-yl]pyridazine-3-carboxamide |
| 2A80 | N-(1-Adamantylmethylsulfonyl)-6-[4-[[4-(5-hydroxypyridin-3-yl)-2-methoxyphenyl]methyl]piperazin-1-yl]pyridazine-3-carboxamide |
| 2A81 | N-(1-Adamantylmethylsulfonyl)-6-[4-[[3-ethoxy-5-(3-hydroxyphenyl)phenyl]methyl]piperazin-1-yl]pyridazine-3-carboxamide |

(continued)

| Mixture No. used | mixEI16-02R |
|---|---|
| Post-cleavage mixture No. | mixEJ10 |
| Compound No. in Table EJ10 | Compound name |
| 2A82 | N-(1-Adamantylmethylsulfonyl)-6-[4-[[3-ethoxy-5-(5-hydroxypyridin-3-yl)phenyl]methyl]piperazin-1-yl]pyridazine-3-carboxamide |
| 2A83 | N-(1-Adamantylmethylsulfonyl)-6-[4-[[3-(3-hydroxyphenyl)-5-propan-2-yloxyphenyl]methyl]piperazin-1-yl]pyridazine-3-carboxamide |
| 2A84 | N-(1-Adamantylmethylsulfonyl)-6-[4-[[3-(5-hydroxypyridin-3-yl)-5-propan-2-yloxyphenyl]methyl]piperazin-1-yl]pyridazine-3-carboxamide |
| 2A85 | N-(1-Adamantylmethylsulfonyl)-6-[4-[[4-(3-hydroxyphenyl)-2,6-dimethoxyphenyl]methyl]piperazin-1-yl]pyridazine-3-carboxamide |
| 2A86 | N-(1-Adamantylmethylsulfonyl)-6-[4-[[4-(5-hydroxypyridin-3-yl)-2,6-dimethoxyphenyl]methyl]piperazin-1-yl]pyridazine-3-carboxamide |
| 2A87 | N-(1-Adamantylmethylsulfonyl)-6-[4-[[3-(3-hydroxyphenyl)-5-(trifluoromethyl)phenyl]methyl]piperazin-1-yl]pyridazine-3-carboxamide |
| 2A88 | N-(1-Adamantylmethylsulfonyl)-6-[4-[[3-(5-hydroxypyridin-3-yl)-5-(trifluoromethyl)phenyl]methyl]piperazin-1-yl]pyridazine-3-carboxamide |
| 2A89 | N-(1-Adamantylmethylsulfonyl)-6-[4-[[3-(3-hydroxyphenyl)-5-(trifluoromethoxy)phenyl]methyl]piperazin-1-yl]pyridazine-3-carboxamide |
| 2A90 | N-(1-Adamantylmethylsulfonyl)-6-[4-[[3-(5-hydroxypyridin-3-yl)-5-(trifluoromethoxy)phenyl]methyl]piperazin-1-yl]pyridazine-3-carboxamide |
| 2A91 | N-(1-Adamantylmethylsulfonyl)-6-[4-[[4-(3-hydroxyphenyl)naphthalen-1-yl]methyl]piperazin-1-yl]pyridazine-3-carboxamide |
| 2A92 | N-(1-Adamantylmethylsulfonyl)-6-[4-[[4-(5-hydroxypyridin-3-yl)naphthalen-1-yl]methyl]piperazin-1-yl]pyridazine-3-carboxamide |
| 2A93 | N-(1-Adamantylmethylsulfonyl)-6-[4-[[4-(3-hydroxyphenyl)thiophen-2-yl]methyl]piperazin-1-yl]pyridazine-3-carboxamide |
| 2A94 | N-(1-Adamantylmethylsulfonyl)-6-[4-[[4-(5-hydroxypyridin-3-yl)thiophen-2-yl]methyl]piperazin-1-yl]pyridazine-3-carboxamide |
| 2A95 | N-(1-Adamantylmethylsulfonyl)-6-[4-[[2-[2-(3-hydroxyphenyl)ethynyl]phenyl]methyl]piperazin-1-yl]pyridazine-3-carboxamide |
| 2A96 | N-(1-Adamantylmethylsulfonyl)-6-[4-[[2-[2-(5-hydroxypyridin-3-yl)ethynyl]phenyl]methyl]piperazin-1-yl]pyridazine-3-carboxamide |
| 2A97 | N-(1-Adamantylmethylsulfonyl)-4-[4-[1-[4-(3- hydroxyphenyl)phenyl]ethyl]piperazin-1-yl]benzamide |
| 2A98 | N-(1-Adamantylmethylsulfonyl)-4-[4-[1-[4-(5-hydroxypyridin-3-yl)phenyl]ethyl]piperazin-1-yl]benzamide |
| 2A99 | N-(1-Adamantylmethylsulfonyl)-4-[4-[1-[2-fluoro-4-(3-hydroxyphenyl)phenyl]ethyl]piperazin-1-yl]benzamide |
| 2A100 | N-(1-Adamantylmethylsulfonyl)-4-[4-[1-[2-fluoro-4-(5-hydroxypyridin-3-yl)phenyl]ethyl]piperazin-1-yl]benzamide |

[Table 82]

Table EU11]

| Mixture No. used | mixEI17-02R |
|---|---|
| Post-cleavage mixture No. | mixEJ11 |
| Compound No. in Table EJ11 | Compound name |
| 2Q01 | 6-[4-[4-(3-Hydroxyphenyl)thiophene-2-carbonyl]piperazin-1-yl]-N-[3-nitro-4-(2-phenylsulfanylethylamino)phenyl]sulfonylpyridazine-3-carboxamide |
| 2Q02 | 6-[4-[4-(5-Hydroxypyridin-3-yl)thiophene-2-carbonyl]piperazin-1-yl]-N-[3-nitro-4-(2-phenylsulfanylethylamino)phenyl]sulfonylpyridazine-3-carboxamide |
| 2Q03 | 6-[4-[4-(3-Hydroxyphenyl)-3-methylbenzoyl]piperazin-1-yl]-N-[3-nitro-4-(2-phenylsulfanylethylamino)phenyl] sulfonylpyridazine-3-carboxamide |
| 2Q04 | 6-[4-[4-(5-Hydroxypyridin-3-yl)-3-methylbenzoyl]piperazin-1-yl]-N-[3-nitro-4-(2-phenylsulfanylethylamino)phenyl]sulfonylpyridazine-3-carboxamide |
| 2Q05 | 6-[4-[3-Fluoro-4-(3-hydroxyphenyl)benzoyl]piperazin-1-yl]-N-[3-nitro-4-(2-phenylsulfanylethylamino)phenyl] sulfonylpyridazine-3-carboxamide |
| 2Q06 | 6-[4-[3-Fluoro-4-(5-hydroxypyridin-3-yl)benzoyl]piperazin-1-yl]-N-[3-nitro-4-(2-phenylsulfanylethylamino)phenyl]sulfonylpyridazine-3-carboxamide |
| 2Q07 | 6-[4-[2-[2-(3-Hydroxyphenyl)ethynyl]benzoyl]piperazin-1-yl]-N-[3-nitro-4-(2-phenylsulfanylethylamino)phenyl] sulfonylpyridazine-3-carboxamide |
| 2Q08 | 6-[4-[2-[2-(5-Hydroxypyridin-3-yl)ethynyl]benzoyl]piperazin-1-yl]-N-[3-nitro-4-(2-phenylsulfanylethylamino)phenyl]sulfonylpyridazine-3-carboxamide |
| 2Q09 | 6-[4-[5-[2-(3-Hydroxyphenyl)ethynyl]pyridine-3-carbonyl]piperazin-1-yl]-N-[3-nitro-4-(2-phenylsulfanylethylamino)phenyl]sulfonylpyridazine-3- carboxamide |
| 2Q10 | 6-[4-[5-[2-(5-Hydroxypyridin-3-yl)ethynyl]pyridine-3-carbonyl]piperazin-1-yl]-N-[3-nitro-4-(2-phenylsulfanylethylamino)phenyl]sulfonylpyridazine-3-carboxamide |
| 2Q11 | 6-[4-[3-[2-(3-Hydroxyphenyl)phenyl]propanoyl]piperazin-1-yl]-N-[3-nitro-4-(2-phenylsulfanylethylamino)phenyl]sulfonylpyridazine-3-carboxamide |
| 2Q12 | 6-[4-[3-[2-(5-Hydroxypyridin-3-yl)phenyl]propanoyl]piperazin-1-yl]-N-[3-nitro-4-(2-phenylsulfanylethylamino)phenyl]sulfonylpyridazine-3- carboxamide |
| 2Q13 | 6-[4-[4-(3-Hydroxyphenyl)naphthalene-1-carbonyl]piperazin-1-yl]-N-[3-nitro-4-(2-phenylsulfanylethylamino)phenyl]sulfonylpyridazine-3-carboxamide |
| 2Q14 | 6-[4-[4-(5-Hydroxypyridin-3-yl)naphthalene-1-carbonyl]piperazin-1-yl]-N-[3-nitro-4-(2-phenylsulfanylethylamino)phenyl]sulfonylpyridazine-3- carboxamide |
| 2Q15 | 6-[4-[3-tert-Butyl-5-(3-hydroxyphenyl)benzoyl]piperazin-1-yl]-N-[3-nitro-4-(2-phenylsulfanylethylamino)phenyl]sulfonylpyridazine-3-carboxamide |
| 2Q16 | 6-[4-[3-tert-Butyl-5-(5-hydroxypyridin-3-yl)benzoyl]piperazin-1-yl]-N-[3-nitro-4-(2-phenylsulfanylethylamino)phenyl]sulfonylpyridazine-3-carboxamide |
| 2Q17 | 6-[4-[3-(3-Hydroxyphenyl)-5-(trifluoromethyl)benzoyl]piperazin-1-yl]-N-[3- nitro-4-(2-phenylsulfanylethylamino)phenyl]sulfonylpyridazine-3-carboxamide |
| 2Q18 | 6-[4-[3-(5-Hydroxypyridin-3-yl)-5-(trifluoromethyl)benzoyl]piperazin-1-yl]-N-[3-nitro-4-(2-phenylsulfanylethylamino)phenyl]sulfonylpyridazine-3-carboxamide |

(continued)

| Mixture No. used | mixEI17-02R |
|---|---|
| Post-cleavage mixture No. | mixEJ11 |
| Compound No. in Table EJ11 | Compound name |
| 2Q19 | 6-[4-[3-[2-(3-Hydroxyphenyl)ethynyl]-5-(trifluoromethyl)benzoyl]piperazin-1-yl]-N-[3-nitro-4-(2-phenylsulfanylethylamino)phenyl]sulfonylpyridazine-3-carboxamide |
| 2Q20 | 6-[4-[3-[2-(5-Hydroxypyridin-3-yl)ethynyl]-5-(trifluoromethyl)benzoyl]piperazin-1-yl]-N-[3-nitro-4-(2-phenylsulfanylethylamino)phenyl]sulfonylpyridazine-3-carboxamide |
| 2Q21 | 6-[4-[3-[4-(3-Hydroxyphenyl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| 2Q22 | 6-[4-[3-[4-(5-Hydroxypyridin-3-yl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| 2Q23 | 4-[4-[4-(3-Hydroxyphenyl)thiophene-2-carbonyl]piperazin-1-yl]-N-[3-nitro-4-(2-phenylsulfanylethylamino)phenyl]sulfonylbenzamide |
| 2Q24 | 4-[4-[4-(5-Hydroxypyridin-3-yl)thiophene-2-carbonyl]piperazin-1-yl]-N-[3-nitro-4-(2-phenylsulfanylethylamino)phenyl]sulfonylbenzamide |
| 2Q25 | 4-[4-[4-(3-Hydroxyphenyl)-3-methylbenzoyl]piperazin-1-yl]-N-[3-nitro-4-(2-phenylsulfanylethylamino)phenyl]sulfonylbenzamide |
| 2Q26 | 4-[4-[4-(5-Hydroxypyridin-3-yl)-3-methylbenzoyl]piperazin-1-yl]-N-[3-nitro-4-(2-phenylsulfanylethylamino)phenyl]sulfonylbenzamide |
| 2Q27 | 4-[4-[3-Fluoro-4-(3-hydroxyphenyl)benzoyl]piperazin-1-yl]-N-[3-nitro-4-(2-phenylsulfanylethylamino)phenyl]sulfonylbenzamide |
| 2Q28 | 4-[4-[3-Fluoro-4-(5-hydroxypyridin-3-yl)benzoyl]piperazin-1-yl]-N-[3-nitro-4-(2-phenylsulfanylethylamino)phenyl]sulfonylbenzamide |
| 2Q29 | 4-[4-[2-[2-(3-Hydroxyphenyl)ethynyl]benzoyl]piperazin-1-yl]-N-[3-nitro-4-(2-phenylsulfanylethylamino)phenyl]sulfonylbenzamide |
| 2Q30 | 4-[4-[2-[2-(5-Hydroxypyridin-3-yl)ethynyl]benzoyl]piperazin-1-yl]-N-[3-nitro-4-(2-phenylsulfanylethylamino)phenyl]sulfonylbenzamide |
| 2Q31 | 4-[4-[5-[2-(3-Hydroxyphenyl)ethynyl]pyridine-3-carbonyl]piperazin-1-yl]-N-[3-nitro-4-(2-phenylsulfanylethylamino)phenyl]sulfonylbenzamide |
| 2Q32 | 4-[4-[5-[2-(5-Hydroxypyridin-3-yl)ethynyl]pyridine-3-carbonyl]piperazin-1-yl]-N-[3-nitro-4-(2-phenylsulfanylethylamino)phenyl]sulfonylbenzamide |
| 2Q33 | 4-[4-[3-[2-(3-Hydroxyphenyl)phenyl]propanoyl]piperazin-1-yl]-N-[3-nitro-4-(2-phenylsulfanylethylamino)phenyl]sulfonylbenzamide |
| 2Q34 | 4-[4-[3-[2-(5-Hydroxypyridin-3-yl)phenyl]propanoyl]piperazin-1-yl]-N-[3-nitro-4-(2-phenylsulfanylethylamino)phenyl]sulfonylbenzamide |
| 2Q35 | 4-[4-[4-(3-Hydroxyphenyl)naphthalene-1-carbonyl]piperazin-1-yl]-N-[3-nitro-4-(2-phenylsulfanylethylamino)phenyl]sulfonylbenzamide |
| 2Q36 | 4-[4-[4-(5-Hydroxypyridin-3-yl)naphthalene-1-carbonyl]piperazin-1-yl]-N-[3- nitro-4-(2-phenylsulfanylethylamino)phenyl]sulfonylbenzamide |
| 2Q37 | 4-[4-[3-tert-Butyl-5-(3-hydroxyphenyl)benzoyl]piperazin-1-yl]-N-[3-nitro-4-(2-phenylsulfanylethylamino)phenyl]sulfonylbenzamide |
| 2Q38 | 4-[4-[3-tert-Butyl-5-(5-hydroxypyridin-3-yl)benzoyl]piperazin-1-yl]-N-[3-nitro-4-(2-phenylsulfanylethylamino)phenyl]sulfonylbenzamide |

(continued)

| Mixture No. used | mixEI17-02R |
|---|---|
| Post-cleavage mixture No. | mixEJ11 |
| Compound No. in Table EJ11 | Compound name |
| 2Q39 | 4-[4-[3-(3-Hydroxyphenyl)-5-(trifluoromethyl)benzoyl]piperazin-1-yl]-N-[3- nitro-4-(2-phenylsulfanylethylamino)phenyl]sulfonylbenzamide |
| 2Q40 | 4-[4-[3-(5-Hydroxypyridin-3-yl)-5-(trifluoromethyl)benzoyl]piperazin-1-yl]-N-[3-nitro-4-(2-phenylsulfanylethylamino)phenyl]sulfonylbenzamide |
| 2Q41 | 4-[4-[3-[2-(3-Hydroxyphenyl)ethynyl]-5-(trifluoromethyl)benzoyl]piperazin-1-yl]-N-[3-nitro-4-(2-phenylsulfanylethylamino)phenyl]sulfonylbenzamide |
| 2Q42 | 4-[4-[3-[2-(5-Hydroxypyridin-3-yl)ethynyl]-5-(trifluoromethyl)benzoyl]piperazin-1-yl]-N-[3-nitro-4-(2-phenylsulfanylethylamino)phenyl]sulfonylbenzamide |
| 2Q43 | 4-[4-[3-[4-(3-Hydroxyphenyl)pyrazol-1-yl]-5-(trifluoromethyl)benzoyl]piperazin-1-yl]-N-[3-nitro-4-(2-phenylsulfanylethylamino)phenyl]sulfonylbenzamide |
| 2Q44 | 4-[4-[3-[4-(5-Hydroxypyridin-3-yl)pyrazol-1-yl]-5- (trifluoromethyl)benzoyl]piperazin-1-yl]-N-[3-nitro-4-(2-phenylsulfanylethylamino)phenyl]sulfonylbenzamide |
| 2Q45 | 4-[4-[[2-(3-Hydroxyphenyl)phenyl]methyl]piperazin-1-yl]-N-[3-nitro-4-(2-phenylsulfanylethylamino)phenyl]sulfonylbenzamide |
| 2Q46 | 4-[4-[[2-(5-Hydroxypyridin-3-yl)phenyl]methyl]piperazin-1-yl]-N-[3-nitro-4-(2-phenylsulfanylethylamino)phenyl]sulfonylbenzamide |
| 2Q47 | 4-[4-[[4-(3-Hydroxyphenyl)-3-methylphenyl]methyl]piperazin-1-yl]-N-[3-nitro-4-(2-phenylsulfanylethylamino)phenyl]sulfonylbenzamide |
| 2Q48 | 4-[4-[[4-(5-Hydroxypyridin-3-yl)-3-methylphenyl]methyl]piperazin-1-yl]-N-[3-nitro-4-(2-phenylsulfanylethylamino)phenyl]sulfonylbenzamide |
| 2Q49 | 4-[4-[[2-Ethyl-4-(3-hydroxyphenyl)phenyl]methyl]piperazin-1-yl]-N-[3-nitro-4-(2-phenylsulfanylethylamino)phenyl]sulfonylbenzamide |
| 2Q50 | 4-[4-[[2-Ethyl-4-(5-hydroxypyridin-3-yl)phenyl]methyl]piperazin-1-yl]-N-[3-nitro-4-(2-phenylsulfanylethylamino)phenyl]sulfonylbenzamide |
| 2Q51 | 4-[4-[[3-Fluoro-4-(3-hydroxyphenyl)phenyl]methyl]piperazin-1-yl]-N-[3-nitro-4-(2-phenylsulfanylethylamino)phenyl]sulfonylbenzamide |
| 2Q52 | 4-[4-[[3-Fluoro-4-(5-hydroxypyridin-3-yl)phenyl]methyl]piperazin-1-yl]-N-[3-nitro-4-(2-phenylsulfanylethylamino)phenyl]sulfonylbenzamide |
| 2Q53 | 4-[4-[[4-(3-Hydroxyphenyl)-2-methoxyphenyl]methyl]piperazin-1-yl]-N-[3-nitro-4-(2-phenylsulfanylethylamino)phenyl]sulfonylbenzamide |
| 2Q54 | 4-[4-[[4-(5-Hydroxypyridin-3-yl)-2-methoxyphenyl]methyl]piperazin-1-yl]-N-[3-nitro-4-(2-phenylsulfanylethylamino)phenyl]sulfonylbenzamide |
| 2Q55 | 4-[4-[[3-Ethoxy-5-(3-hydroxyphenyl)phenyl]methyl]piperazin-1-yl]-N-[3-nitro-4-(2-phenylsulfanylethylamino)phenyl]sulfonylbenzamide |
| 2Q56 | 4-[4-[[3-Ethoxy-5-(5-hydroxypyridin-3-yl)phenyl]methyl]piperazin-1-yl]-N-[3-nitro-4-(2-phenylsulfanylethylamino)phenyl]sulfonylbenzamide |
| 2Q57 | 4-[4-[[3-(3-Hydroxyphenyl)-5-propan-2-yloxyphenyl]methyl]piperazin-1-yl]-N-[3-nitro-4-(2-phenylsulfanylethylamino)phenyl]sulfonylbenzamide |
| 2Q58 | 4-[4-[[3-(5-Hydroxypyridin-3-yl)-5-propan-2-yloxyphenyl]methyl]piperazin-1-yl]-N-[3-nitro-4-(2-phenylsulfanylethylamino)phenyl]sulfonylbenzamide |

(continued)

| Mixture No. used | mixEI17-02R |
|---|---|
| Post-cleavage mixture No. | mixEJ11 |
| Compound No. in Table EJ11 | Compound name |
| 2Q59 | 4-[4-[[4-(3-Hydroxyphenyl)-2,6-dimethoxyphenyl]methyl]piperazin-1-yl]-N-[3-nitro-4-(2-phenylsulfanylethylamino)phenyl]sulfonylbenzamide |
| 2Q60 | 4-[4-[[4-(5-Hydroxypyridin-3-yl)-2,6-dimethoxyphenyl]methyl]piperazin-1-yl]-N-[3-nitro-4-(2-phenylsulfanylethylamino)phenyl]sulfonylbenzamide |
| 2Q61 | 4-[4-[[3-(3-Hydroxyphenyl)-5-(trifluoromethyl)phenyl]methyl]piperazin-1-yl]-N-[3-nitro-4-(2-phenylsulfanylethylamino)phenyl]sulfonylbenzamide |
| 2Q62 | 4-[4-[[3-(5-Hydroxypyridin-3-yl)-5-(trifluoromethyl)phenyl]methyl]piperazin-1-yl]-N-[3-nitro-4-(2-phenylsulfanylethylamino)phenyl]sulfonylbenzamide |
| 2Q63 | 4-[4-[[3-(3-Hydroxyphenyl)-5-(trifluoromethoxy)phenyl]methyl]piperazin-1-yl]-N-[3-nitro-4-(2-phenylsulfanylethylamino)phenyl]sulfonylbenzamide |
| 2Q64 | 4-[4-[[3-(5-Hydroxypyridin-3-yl)-5-(trifluoromethoxy)phenyl]methyl]piperazin-1-yl]-N-[3-nitro-4-(2-phenylsulfanylethylamino)phenyl]sulfonylbenzamide |
| 2Q65 | 4-[4-[[4-(3-Hydroxyphenyl)naphthalen-1-yl]methyl]piperazin-1-yl]-N-[3-nitro-4-(2-phenylsulfanylethylamino)phenyl]sulfonylbenzamide |
| 2Q66 | 4-[4-[[4-(5-Hydroxypyridin-3-yl)naphthalen-1-yl]methyl]piperazin-1-yl]-N-[3-nitro-4-(2-phenylsulfanylethylamino)phenyl]sulfonylbenzamide |
| 2Q67 | 4-[4-[[4-(3-Hydroxyphenyl)thiophen-2-yl]methyl]piperazin-1-yl]-N-[3-nitro-4-(2-phenylsulfanylethylamino)phenyl]sulfonylbenzamide |
| 2Q68 | 4-[4-[[4-(5-Hydroxypyridin-3-yl)thiophen-2-yl]methyl]piperazin-1-yl]-N-[3-nitro-4-(2-phenylsulfanylethylamino)phenyl]sulfonylbenzamide |
| 2Q69 | 4-[4-[[2-[2-(3-Hydroxyphenyl)ethynyl]phenyl]methyl]piperazin-1-yl]-N-[3-nitro-4-(2-phenylsulfanylethylamino)phenyl]sulfonylbenzamide |
| 2Q70 | 4-[4-[[2-[2-(5-Hydroxypyridin-3-yl)ethynyl]phenyl]methyl]piperazin-1-yl]-N-[3-nitro-4-(2-phenylsulfanylethylamino)phenyl]sulfonylbenzamide |
| 2Q71 | 6-[4-[[2-(3-Hydroxyphenyl)phenyl]methyl]piperazin-1-yl]-N-[3-nitro-4-(2-phenylsulfanylethylamino)phenyl]sulfonylpyridazine-3-carboxamide |
| 2Q72 | 6-[4-[[2-(5-Hydroxypyridin-3-yl)phenyl]methyl]piperazin-1-yl]-N-[3-nitro-4-(2-phenylsulfanylethylamino)phenyl]sulfonylpyridazine-3-carboxamide |
| 2Q73 | 6-[4-[[4-(3-Hydroxyphenyl)-3-methylphenyl]methyl]piperazin-1-yl]-N-[3-nitro-4-(2-phenylsulfanylethylamino)phenyl]sulfonylpyridazine-3-carboxamide |
| 2Q74 | 6-[4-[[4-(5-Hydroxypyridin-3-yl)-3-methylphenyl]methyl]piperazin-1-yl]-N-[3-nitro-4-(2-phenylsulfanylethylamino)phenyl]sulfonylpyridazine-3- carboxamide |
| 2Q75 | 6-[4-[[2-Ethyl-4-(3-hydroxyphenyl)phenyl]methyl]piperazin-1-yl]-N-[3-nitro-4-(2-phenylsulfanylethylamino)phenyl]sulfonylpyridazine-3-carboxamide |
| 2Q76 | 6-[4-[[2-Ethyl-4-(5-hydroxypyridin-3-yl)phenyl]methyl]piperazin-1-yl]-N-[3-nitro-4-(2-phenylsulfanylethylamino)phenyl]sulfonylpyridazine-3-carboxamide |
| 2Q77 | 6-[4-[[3-Fluoro-4-(3-hydroxyphenyl)phenyl]methyl]piperazin-1-yl]-N-[3-nitro-4-(2-phenylsulfanylethylamino)phenyl]sulfonylpyridazine-3-carboxamide |
| 2Q78 | 6-[4-[[3-Fluoro-4-(5-hydroxypyridin-3-yl)phenyl]methyl]piperazin-1-yl]-N-[3- nitro-4-(2-phenylsulfanylethylamino)phenyl]sulfonylpyridazine-3-carboxamide |

(continued)

| Mixture No. used | mixEI17-02R |
|---|---|
| Post-cleavage mixture No. | mixEJ11 |
| Compound No. in Table EJ11 | Compound name |
| 2Q79 | 6-[4-[[4-(3-Hydroxyphenyl)-2-methoxyphenyl]methyl]piperazin-1-yl]-N-[3-nitro-4-(2-phenylsulfanylethylamino)phenyl]sulfonylpyridazine-3-carboxamide |
| 2Q80 | 6-[4-[[4-(5-Hydroxypyridin-3-yl)-2-methoxyphenyl]methyl]piperazin-1-yl]-N-[3-nitro-4-(2-phenylsulfanylethylamino)phenyl]sulfonylpyridazine-3- carboxamide |
| 2Q81 | 6-[4-[[3-Ethoxy-5-(3-hydroxyphenyl)phenyl]methyl]piperazin-1-yl]-N-[3-nitro-4-(2-phenylsulfanylethylamino)phenyl]sulfonylpyridazine-3-carboxamide |
| 2Q82 | 6-[4-[[3-Ethoxy-5-(5-hydroxypyridin-3-yl)phenyl]methyl]piperazin-1-yl]-N-[3-nitro-4-(2-phenylsulfanylethylamino)phenyl]sulfonylpyridazine-3- carboxamide |
| 2Q83 | 6-[4-[[3-(3-Hydroxyphenyl)-5-propan-2-yloxyphenyl]methyl]piperazin-1-yl]-N-[3-nitro-4-(2-phenylsulfanylethylamino)phenyl]sulfonylpyridazine-3-carboxamide |
| 2Q84 | 6-[4-[[3-(5-Hydroxypyridin-3-yl)-5-propan-2-yloxyphenyl]methyl]piperazin-1-yl]-N-[3-nitro-4-(2-phenylsulfanylethylamino)phenyl]sulfonylpyridazine-3-carboxamide |
| 2Q85 | 6-[4-[[4-(3-Hydroxyphenyl)-2,6-dimethoxyphenyl]methyl]piperazin-1-yl]-N-[3-nitro-4-(2-phenylsulfanylethylamino)phenyl]sulfonylpyridazine-3- carboxamide |
| 2Q86 | 6-[4-[[4-(5-Hydroxypyridin-3-yl)-2,6-dimethoxyphenyl]methyl]piperazin-1- yl]-N-[3-nitro-4-(2-phenylsulfanylethylamino)phenyl]sulfonylpyridazine-3-carboxamide |
| 2Q87 | 6-[4-[[3-(3-Hydroxyphenyl)-5-(trifluoromethyl)phenyl]methyl]piperazin-1-yl]-N-[3-nitro-4-(2-phenylsulfanylethylamino)phenyl]sulfonylpyridazine-3-carboxamide |
| 2Q88 | 6-[4-[[3-(5-Hydroxypyridin-3-yl)-5-(trifluoromethyl)phenyl]methyl]piperazin-1-yl]-N-[3-nitro-4-(2-phenylsulfanylethylamino)phenyl]sulfonylpyridazine-3-carboxamide |
| 2Q89 | 6-[4-[[3-(3-Hydroxyphenyl)-5-(trifluoromethoxy)phenyl]methyl]piperazin-1-yl]-N-[3-nitro-4-(2-phenylsulfanylethylamino)phenyl]sulfonylpyridazine-3-carboxamide |
| 2Q90 | 6-[4-[[3-(5-Hydroxypyridin-3-yl)-5-(trifluoromethoxy)phenyl]methyl]piperazin-1-yl]-N-[3-nitro-4-(2-phenylsulfanylethylamino)phenyl]sulfonylpyridazine-3-carboxamide |
| 2Q91 | 6-[4-[[4-(3-Hydroxyphenyl)naphthalen-1-yl]methyl]piperazin-1-yl]-N-[3-nitro-4-(2-phenylsulfanylethylamino)phenyl]sulfonylpyridazme-3-carboxamide |
| 2Q92 | 6-[4-[[4-(5-Hydroxypyridin-3-yl)naphthalen-1-yl]methyl]piperazin-1-yl]-N-[3-nitro-4-(2-phenylsulfanylethylamino)phenyl]sulfonylpyridazine-3-carboxamide |
| 2Q93 | 6-[4-[[4-(3-Hydroxyphenyl)thiophen-2-yl]methyl]piperazin-1-yl]-N-[3-nitro-4-(2-phenylsulfanylethylamino)phenyl]sulfonylpyridazine-3-carboxamide |
| 2Q94 | 6-[4-[[4-(5-Hydroxypyridin-3-yl)thiophen-2-yl]methyl]piperazin-1-yl]-N-[3-nitro-4-(2-phenylsulfanylethylamino)phenyl]sulfonylpyridazine-3- carboxamide |
| 2Q95 | 6-[4-[[2-[2-(3-Hydroxyphenyl)ethynyl]phenyl]methyl]piperazin-1-yl]-N-[3-nitro-4-(2-phenylsulfanylethylamino)phenyl]sulfonylpyridazine-3- carboxamide |
| 2Q96 | 6-[4-[[2-[2-(5-Hydroxypyridin-3-yl)ethynyl]phenyl]methyl]piperazin-1-yl]-N-[3-nitro-4-(2-phenylsulfanylethylamino)phenyl]sulfonylpyridazine-3- carboxamide |
| 2Q97 | 4-[4-[1-[4-(3-Hydroxyphenyl)phenyl]ethyl]piperazin-1-yl]-N-[3-nitro-4-(2-phenylsulfanylethylamino)phenyl]sulfonylbenzamide |
| 2Q98 | 4-[4-[1-[4-(5-Hydroxypyridin-3-yl)phenyl]ethyl]piperazin-1-yl]-N-[3-nitro-4-(2-phenylsulfanylethylamino)phenyl]sulfonylbenzamide |

(continued)

| Mixture No. used | mixEI17-02R |
|---|---|
| Post-cleavage mixture No. | mixEJ11 |
| Compound No. in Table EJ11 | Compound name |
| 2Q99 | 4-[4-[1-[2-Fluoro-4-(3-hydroxyphenyl)phenyl]ethyl]piperazin-1-yl]-N-[3-nitro-4-(2-phenylsulfanylethylamino)phenyl]sulfonylbenzamide |
| 2Q100 | 4-[4-[1-[2-Fluoro-4-(5-hydroxypyridin-3-yl)phenyl]ethyl]piperazin-1-yl]-N-[3-nitro-4-(2-phenylsulfanylethylamino)phenyl]sulfonylbenzamide |

[Table 83]

[Table EIJ12]

| Mixture No. used | mixEI18-02R |
|---|---|
| Post-cleavage mixture No. | mixEJ 12 |
| Compound No. in Table EJ12 | Compound name |
| 2R01 | 6-[4-[4-(3-Hydroxyphenyl)thiophene-2-carbonyl]piperazin-1-yl]-N-[4-(2-phenylsulfanylethylamino)-3-(trifluoromethyl)phenyl]sulfonylpyridazine-3-carboxamide |
| 2R02 | 6-[4-[4-(5-Hydroxypyridin-3-yl)thiophene-2-carbonyl]piperazin-1-yl]-N-[4-(2-phenylsulfanylethylamino)-3-(trifluoromethyl)phenyl]sulfonylpyridazine-3-carboxamide |
| 2R03 | 6-[4-[4-(3-Hydroxyphenyl)-3-methylbenzoyl]piperazin-1-yl]-N-[4-(2-phenylsulfanylethylamino)-3-(trifluoromethyl)phenyl]sulfonylpyridazine-3-carboxamide |
| 2R04 | 6-[4-[4-(5-Hydroxypyridin-3-yl)-3-methylbenzoyl]piperazin-1-yl]-N-[4-(2-phenylsulfanylethylamino)-3-(trifluoromethyl)phenyl]sulfonylpyridazine-3-carboxamide |
| 2R05 | 6-[4-[3-Fluoro-4-(3-hydroxyphenyl)benzoyl]piperazin-1-yl]-N-[4-(2-phenylsulfanylethylamino)-3-(trifluoromethyl)phenyl]sulfonylpyridazine-3-carboxamide |
| 2R06 | 6-[4-[3-Fluoro-4-(5-hydroxypyridin-3-yl)benzoyl]piperazin-1-yl]-N-[4-(2-phenylsulfanylethylamino)-3-(trifluoromethyl)phenyl]sulfonylpyridazine-3-carboxamide |
| 2R07 | 6-[4-[2-[2-(3-Hydroxyphenyl)ethynyl]benzoyl]piperazin-1-yl]-N-[4-(2-phenylsulfanylethylamino)-3-(trifluoromethyl)phenyl]sulfonylpyridazine-3-carboxamide |
| 2R08 | 6-[4-[2-[2-(5-Hydroxypyridin-3-yl)ethynyl]benzoyl]piperazin-1-yl]-N-[4-(2-phenylsulfanylethylamino)-3-(trifluoromethyl)phenyl]sulfonylpyridazine-3-carboxamide |
| 2R09 | 6-[4-[5-[2-(3-Hydroxyphenyl)ethynyl]pyridine-3-carbonyl]piperazin-1-yl]-N-[4-(2-phenylsulfanylethylamino)-3-(trifluoromethyl)phenyl]sulfonylpyridazine-3-carboxamide |
| 2R10 | 6-[4-[5-[2-(5-Hydroxypyridin-3-yl)ethynyl]pyridine-3-carbonyl]piperazin-1-yl]-N-[4-(2-phenylsulfanylethylamino)-3-(trifluoromethyl)phenyl]sulfonylpyridazine-3-carboxamide |
| 2R11 | 6-[4-[3-[2-(3-Hydroxyphenyl)phenyl]propanoyl]piperazin-1-yl]-N-[4-(2-phenylsulfanylethylamino)-3-(trifluoromethyl)phenyl]sulfonylpyridazine-3-carboxamide |
| 2R12 | 6-[4-[3-[2-(5-Hydroxypyridin-3-yl)phenyl]propanoyl]piperazin-1-yl]-N-[4-(2-phenylsulfanylethylamino)-3-(trifluoromethyl)phenyl]sulfonylpyridazine-3-carboxamide |

(continued)

| Mixture No. used | mixEI18-02R |
|---|---|
| Post-cleavage mixture No. | mixEJ 12 |
| Compound No. in Table EJ12 | Compound name |
| 2R13 | 6-[4-[4-(3-Hydroxyphenyl)naphthalene-1-carbonyl]piperazin-1-yl]-N-[4-(2-phenylsulfanylethylamino)-3-(trifluoromethyl)phenyl]sulfonylpyridazine-3-carboxamide |
| 2R14 | 6-[4-[4-(5-Hydroxypyridin-3-yl)naphthalene-1-carbonyl]piperazin-1-yl]-N-[4-(2-phenylsulfanylethylamino)-3-(trifluoromethyl)phenyl]sulfonylpyridazine-3-carboxamide |
| 2R15 | 6-[4-[3-tert-Butyl-5-(3-hydroxyphenyl)benzoyl]piperazin-1-yl]-N-[4-(2-phenylsulfanylethylamino)-3-(trifluoromethyl)phenyl]sulfonylpyridazine-3-carboxamide |
| 2R16 | 6-[4-[3-tert-Butyl-5-(5-hydroxypyridin-3-yl)benzoyl]piperazin-1-yl]-N-[4-(2-phenylsulfanylethylamino)-3-(trifluoromethyl)phenyl]sulfonylpyridazine-3-carboxamide |
| 2R17 | 6-[4-[3-(3-Hydroxyphenyl)-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| 2R18 | 6-[4-[3-(5-Hydroxypyridin-3-yl)-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| 2R19 | 6-[4-[3-[2-(3-Hydroxyphenyl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| 2R20 | 6-[4-[3-[2-(5-Hydroxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| 2R21 | 6-[4-[3-[4-(3-Hydroxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| 2R22 | 6-[4-[3-[4-(5-Hydroxypyridin-3-yl)pyrazol-1-yl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| 2R23 | 4-[4-[4-(3-Hydroxyphenyl)thiophene-2-carbonyl]piperazin-1-yl]-N-[4-(2-phenylsulfanylethylamino)-3-(trifluoromethyl)phenyl]sulfonylbenzamide |
| 2R24 | 4-[4-[4-(5-Hydroxypyridin-3-yl)thiophene-2-carbonyl]piperazin-1-yl]-N-[4-(2-phenylsulfanylethylamino)-3-(trifluoromethyl)phenyl]sulfonylbenzamide |
| 2R25 | 4-[4-[4-(3-Hydroxyphenyl)-3-methylbenzoyl]piperazin-1-yl]-N-[4-(2-phenylsulfanylethylamino)-3-(trifluoromethyl)phenyl]sulfonylbenzamide |
| 2R26 | 4-[4-[4-(5-Hydroxypyridin-3-yl)-3-methylbenzoyl]piperazin-1-yl]-N-[4-(2-phenylsulfanylethylamino)-3-(trifluoromethyl)phenyl]sulfonylbenzamide |
| 2R27 | 4-[4-[3-Fluoro-4-(3-hydroxyphenyl)benzoyl]piperazin-1-yl]-N-[4-(2-phenylsulfanylethylamino)-3-(trifluoromethyl)phenyl]sulfonylbenzamide |
| 2R28 | 4-[4-[3-Fluoro-4-(5-hydroxypyridin-3-yl)benzoyl]piperazin-1-yl]-N-[4-(2-phenylsulfanylethylamino)-3-(trifluoromethyl)phenyl]sulfonylbenzamide |
| 2R29 | 4-[4-[2-[2-(3-Hydroxyphenyl)ethynyl]benzoyl]piperazin-1-yl]-N-[4-(2-phenylsulfanylethylamino)-3-(trifluoromethyl)phenyl]sulfonylbenzamide |
| 2R30 | 4-[4-[2-[2-(5-Hydroxypyridin-3-yl)ethynyl]benzoyl]piperazin-1-yl]-N-[4-(2-phenylsulfanylethylamino)-3-(trifluoromethyl)phenyl]sulfonylbenzamide |
| 2R31 | 4-[4-[5-[2-(3-Hydroxyphenyl)ethynyl]pyridine-3-carbonyl]piperazin-1-yl]-N-[4-(2-phenylsulfanylethylamino)-3-(trifluoromethyl)phenyl]sulfonylbenzamide |
| 2R32 | 4-[4-[5-[2-(5-Hydroxypyridin-3-yl)ethynyl]pyridine-3-carbonyl]piperazin-1-yl]-N-[4-(2-phenylsulfanylethylamino)-3-(trifluoromethyl)phenyl]sulfonylbenzamide |

(continued)

| Mixture No. used | mixEI18-02R |
|---|---|
| Post-cleavage mixture No. | mixEJ 12 |
| Compound No. in Table EJ12 | Compound name |
| 2R33 | 4-[4-[3-[2-(3-Hydroxyphenyl)phenyl]propanoyl]piperazin-1-yl]-N-[4-(2-phenylsulfanylethylamino)-3-(trifluoromethyl)phenyl]sulfonylbenzamide |
| 2R34 | 4-[4-[3-[2-(5-Hydroxypyridin-3-yl)phenyl]propanoyl]piperazin-1-yl]-N-[4-(2-phenylsulfanylethylamino)-3-(trifluoromethyl)phenyl]sulfonylbenzamide |
| 2R35 | 4-[4-[4-(3-Hydroxyphenyl)naphthalene-1-carbonyl]piperazin-1-yl]-N-[4-(2-phenylsulfanylethylamino)-3-(trifluoromethyl)phenyl]sulfonylbenzamide |
| 2R36 | 4-[4-[4-(5-Hydroxypyridin-3-yl)naphthalene-1-carbonyl]piperazin-1-yl]-N-[4-(2-phenylsulfanylethylamino)-3-(trifluoromethyl)phenyl]sulfonylbenzamide |
| 2R37 | 4-[4-[3-tert-Butyl-5-(3-hydroxyphenyl)benzoyl]piperazin-1-yl]-N-[4-(2-phenylsulfanylethylamino)-3-(trifluoromethyl)phenyl]sulfonylbenzamide |
| 2R38 | 4-[4-[3-tert-Butyl-5-(5-hydroxypyridin-3-yl)benzoyl]piperazin-1-yl]-N-[4-(2-phenylsulfanylethylamino)-3-(trifluoromethyl)phenyl]sulfonylbenzamide |
| 2R39 | 4-[4-[3-(3-Hydroxyphenyl)-5-(trifluoromethyl)benzoyl]piperazin-1-yl]-N-[4-(2-phenylsulfanylethylamino)-3-(trifluoromethyl)phenyl]sulfonylbenzamide |
| 2R40 | 4-[4-[3-(5-Hydroxypyridin-3-yl)-5-(trifluoromethyl)benzoyl]piperazin-1-yl]-N-[4-(2-phenylsulfanylethylamino)-3-(trifluoromethyl)phenyl]sulfonylbenzamide |
| 2R41 | 4-[4-[3-[2-(3-Hydroxyphenyl)ethynyl]-5-(trifluoromethyl)benzoyl]piperazin-1-yl]-N-[4-(2-phenylsulfanylethylamino)-3-(trifluoromethyl)phenyl]sulfonylbenzamide |
| 2R42 | 4-[4-[3-[2-(5-Hydroxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylbenzamide |
| 2R43 | 4-[4-[3-[4-(3-Hydroxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylbenzamide |
| 2R44 | 4-[4-[3-[4-(5-Hydroxypyridin-3-yl)pyrazol-1-yl]-5-(trifluoromethyl)benzoyl]piperazin-1-yl]-N-[4-(2-phenylsulfanylethylamino)-3-(trifluoromethyl)phenyl]sulfonylbenzamide |
| 2R45 | 4-[4-[[2-(3-Hydroxyphenyl)phenyl]methyl]piperazin-1-yl]-N-[4-(2-phenylsulfanylethylamino)-3-(trifluoromethyl)phenyl]sulfonylbenzamide |
| 2R46 | 4-[4-[[2-(5-Hydroxypyridin-3-yl)phenyl]methyl]piperazin-1-yl]-N-[4-(2-phenylsulfanylethylamino)-3-(trifluoromethyl)phenyl]sulfonylbenzamide |
| 2R47 | 4-[4-[[4-(3-Hydroxyphenyl)-3-methylphenyl]methyl]piperazin-1-yl]-N-[4-(2-phenylsulfanylethylamino)-3-(trifluoromethyl)phenyl]sulfonylbenzamide |
| 2R48 | 4-[4-[[4-(5-Hydroxypyridin-3-yl)-3-methylphenyl]methyl]piperazin-1-yl]-N-[4-(2-phenylsulfanylethylamino)-3-(trifluoromethyl)phenyl]sulfonylbenzamide |
| 2R49 | 4-[4-[[2-Ethyl-4-(3-hydroxyphenyl)phenyl]methyl]piperazin-1-yl]-N-[4-(2-phenylsulfanylethylamino)-3-(trifluoromethyl)phenyl]sulfonylbenzamide |
| 2R50 | 4-[4-[[2-Ethyl-4-(5-hydroxypyridin-3-yl)phenyl]methyl]piperazin-1-yl]-N-[4-(2-phenylsulfanylethylamino)-3-(trifluoromethyl)phenyl]sulfonylbenzamide |
| 2R51 | 4-[4-[[3-Fluoro-4-(3-hydroxyphenyl)phenyl]methyl]piperazin-1-yl]-N-[4-(2-phenylsulfanylethylamino)-3-(trifluoromethyl)phenyl]sulfonylbenzamide |
| 2R52 | 4-[4-[[3-Fluoro-4-(5-hydroxypyridin-3-yl)phenyl]methyl]piperazin-1-yl]-N-[4-(2-phenylsulfanylethylamino)-3-(trifluoromethyl)phenyl]sulfonylbenzamide |

(continued)

| Mixture No. used | mixEI18-02R |
|---|---|
| Post-cleavage mixture No. | mixEJ 12 |
| Compound No. in Table EJ12 | Compound name |
| 2R53 | 4-[4-[[4-(3-Hydroxyphenyl)-2-methoxyphenyl]methyl]piperazin-1-yl]-N-[4-(2-phenylsulfanylethylamino)-3-(trifluoromethyl)phenyl]sulfonylbenzamide |
| 2R54 | 4-[4-[[4-(5-Hydroxypyridin-3-yl)-2-methoxyphenyl]methyl]piperazin-1-yl]-N-[4-(2-phenylsulfanylethylamino)-3-(trifluoromethyl)phenyl]sulfonylbenzamide |
| 2R55 | 4-[4-[[3-Ethoxy-5-(3-hydroxyphenyl)phenyl]methyl]piperazin-1-yl]-N-[4-(2-phenylsulfanylethylamino)-3-(trifluoromethyl)phenyl]sulfonylbenzamide |
| 2R56 | 4-[4-[[3-Ethoxy-5-(5-hydroxypyridin-3-yl)phenyl]methyl]piperazin-1-yl]-N-[4-(2-phenylsulfanylethylamino)-3- (trifluoromethyl)phenyl]sulfonylbenzamide |
| 2R57 | 4-[4-[[3-(3-Hydroxyphenyl)-5-propan-2-yloxyphenyl]methyl]piperazin-1-yl]-N-[4-(2-phenylsulfanylethylamino)-3-(trifluoromethyl)phenyl]sulfonylbenzamide |
| 2R58 | 4-[4-[[3-(5-Hydroxypyridin-3-yl)-5-propan-2-yloxyphenyl]methyl]piperazin-1-yl]-N-[4-(2-phenylsulfanylethylamino)-3-(trifluoromethyl)phenyl]sulfonylbenzamide |
| 2R59 | 4-[4-[[4-(3-Hydroxyphenyl)-2,6-dimethoxyphenyl]methyl]piperazin-1-yl]-N-[4-(2-phenylsulfanylethylamino)-3-(trifluoromethyl)phenyl]sulfonylbenzamide |
| 2R60 | 4-[4-[[4-(5-Hydroxypyridin-3-yl)-2,6-dimethoxyphenyl]methyl]piperazin-1-yl]-N-[4-(2-phenylsulfanylethylamino)-3-(trifluoromethyl)phenyl]sulfonylbenzamide |
| 2R61 | 4-[4-[[3-(3-Hydroxyphenyl)-5-(trifluoromethyl)phenyl]methyl]piperazin-1-yl]-N-[4-(2-phenylsulfanylethylamino)-3-(trifluoromethyl)phenyl]sulfonylbenzamide |
| 2R62 | 4-[4-[[3-(5-Hydroxypyridin-3-yl)-5-(trifluoromethyl)phenyl]methyl]piperazin-1-yl]-N-[4-(2-phenylsulfanylethylamino)-3-(trifluoromethyl)phenyl]sulfonylbenzamide |
| 2R63 | 4-[4-[[3-(3-Hydroxyphenyl)-5-(trifluoromethoxy)phenyl]methyl]piperazin-1-yl]-N-[4-(2-phenylsulfanylethylamino)-3-(trifluoromethyl)phenyl]sulfonylbenzamide |
| 2R64 | 4-[4-[[3-(5-Hydroxypyridin-3-yl)-5-(trifluoromethoxy)phenyl]methyl]piperazin-1-yl]-N-[4-(2-phenylsulfanylethylamino)-3-(trifluoromethyl)phenyl]sulfonylbenzamide |
| 2R65 | 4-[4-[[4-(3-Hydroxyphenyl)naphthalen-1-yl]methyl]piperazin-1-yl]-N-[4-(2-phenylsulfanylethylamino)-3-(trifluoromethyl)phenyl]sulfonylbenzamide |
| 2R66 | 4-[4-[[4-(5-Hydroxypyridin-3-yl)naphthalen-1-yl]methyl]piperazin-1-yl]-N-[4-(2-phenylsulfanylethylamino)-3- (trifluoromethyl)phenyl]sulfonylbenzamide |
| 2R67 | 4-[4-[[4-(3-Hydroxyphenyl)thiophen-2-yl]methyl]piperazin-1-yl]-N-[4-(2-phenylsulfanylethylamino)-3-(trifluoromethyl)phenyl]sulfonylbenzamide |
| 2R68 | 4-[4-[[4-(5-Hydroxypyridin-3-yl)thiophen-2-yl]methyl]piperazin-1-yl]-N-[4-(2-phenylsulfanylethylamino)-3-(trifluoromethyl)phenyl]sulfonylbenzamide |
| 2R69 | 4-[4-[[2-[2-(3-Hydroxyphenyl)ethynyl]phenyl]methyl]piperazin-1-yl]-N-[4-(2-phenylsulfanylethylamino)-3-(trifluoromethyl)phenyl]sulfonylbenzamide |
| 2R70 | 4-[4-[[2-[2-(5-Hydroxypyridin-3-yl)ethynyl]phenyl]methyl]piperazin-1-yl]-N-[4-(2-phenylsulfanylethylamino)-3-(trifluoromethyl)phenyl]sulfonylbenzamide |
| 2R71 | 6-[4-[[2-(3-Hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| 2R72 | 6-[4-[[2-(5-Hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |

(continued)

| Mixture No. used | mixEI18-02R |
|---|---|
| Post-cleavage mixture No. | mixEJ 12 |
| Compound No. in Table EJ12 | Compound name |
| 2R73 | 6-[4-[[4-(3-Hydroxyphenyl)-3-methylphenyl]methyl]piperazin-1-yl]-N-[4-(2-phenylsulfanylethylamino)-3-(trifluoromethyl)phenyl]sulfonylpyridazine-3-carboxamide |
| 2R74 | 6-[4-[[4-(5-Hydroxypyridin-3-yl)-3-methylphenyl]methyl]piperazin-1-yl]-N-[4-(2-phenylsulfanylethylamino)-3- (trifluoromethyl)phenyl]sulfonylpyridazine-3-carboxamide |
| 2R75 | 6-[4-[[2-Ethyl-4-(3-hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| 2R76 | 6-[4-[[2-Ethyl-4-(5-hydroxypyridin-3-yl)phenyl]methyl]piperazin-1-yl]-N-[4-(2-phenylsulfanylethylamino)-3- (trifluoromethyl)phenyl]sulfonylpyridazine-3-carboxamide |
| 2R77 | 6-[4-[[3-Fluoro-4-(3-hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| 2R78 | 6-[4-[[3-Fluoro-4-(5-hydroxypyridin-3-yl)phenyl]methyl]piperazin-1-yl]-N-[4-(2-phenylsulfanylethylamino)-3- (trifluoromethyl)phenyl]sulfonylpyridazine-3-carboxamide |
| 2R79 | 6-[4-[[4-(3-Hydroxyphenyl)-2-methoxyphenyl]methyl]piperazin-1-yl]-N-[4-(2-phenylsulfanylethylamino)-3-(trifluoromethyl)phenyl]sulfonylpyridazine-3-carboxamide |
| 2R80 | 6-[4-[[4-(5-Hydroxypyridin-3-yl)-2-methoxyphenyl]methyl]piperazin-1-yl]-N-[4-(2-phenylsulfanylethylamino)-3-(trifluoromethyl)phenyl]sulfonylpyridazine-3-carboxamide |
| 2R81 | 6-[4-[[3-Ethoxy-5-(3-hydroxyphenyl)phenyl]methyl]piperazin-1-yl]-N-[4-(2-phenylsulfanylethylamino)-3-(trifluoromethyl)phenyl]sulfonylpyridazine-3-carboxamide |
| 2R82 | 6-[4-[[3-Ethoxy-5-(5-hydroxypyridin-3-yl)phenyl]methyl]piperazin-1-yl]-N-[4-(2-phenylsulfanylethylamino)-3-(trifluoromethyl)phenyl]sulfonylpyridazine-3-carboxamide |
| 2R83 | 6-[4-[[3-(3-Hydroxyphenyl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| 2R84 | 6-[4-[[3-(5-Hydroxypyridin-3-yl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| 2R85 | 6-[4-[[4-(3-Hydroxyphenyl)-2,6-dimethoxyphenyl]methyl]piperazin-1-yl]-N-[4-(2-phenylsulfanylethylamino)-3- (trifluoromethyl)phenyl]sulfonylpyridazine-3-carboxamide |
| 2R86 | 6-[4-[[4-(5-Hydroxypyridin-3-yl)-2,6-dimethoxyphenyl]methyl]piperazin-1- yl]-N-[4-(2-phenylsulfanylethylamino)-3-(trifluoromethyl)phenyl]sulfonylpyridazine-3-carboxamide |
| 2R87 | 6-[4-[[3-(3-Hydroxyphenyl)-5-(trifluoromethyl)phenyl]methyl]piperazin-1-yl]-N-[4-(2-phenylsulfanylethylamino)-3-(trifluoromethyl)phenyl]sulfonylpyridazine-3-carboxamide |
| 2R88 | 6-[4-[[3-(5-Hydroxypyridin-3-yl)-5-(trifluoromethyl)phenyl]methyl]piperazin-1-yl]-N-[4-(2-phenylsulfanylethylamino)-3-(trifluoromethyl)phenyl]sulfonylpyridazine-3-carboxamide |
| 2R89 | 6-[4-[[3-(3-Hydroxyphenyl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |
| 2R90 | 6-[4-[[3-(5-Hydroxypyridin-3-yl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |
| 2R91 | 6-[4-[[4-(3-Hydroxyphenyl)naphthalen-1-yl]methyl]piperazin-1-yl]-N-[4-(2-phenylsulfanylethylamino)-3-(trifluoromethyl)phenyl]sulfonylpyridazine-3-carboxamide |
| 2R92 | 6-[4-[[4-(5-Hydroxypyridin-3-yl)naphthalen-1-yl]methyl]piperazin-1-yl]-N-[4-(2-phenylsulfanylethylamino)-3- (trifluoromethyl)phenyl]sulfonylpyridazine-3-carboxamide |

(continued)

| Mixture No. used | mixEI18-02R |
|---|---|
| Post-cleavage mixture No. | mixEJ 12 |
| Compound No. in Table EJ12 | Compound name |
| 2R93 | 6-[4-[[4-(3-Hydroxyphenyl)thiophen-2-yl]methyl]piperazin-1-yl]-N-[4-(2-phenylsulfanylethylamino)-3-(trifluoromethyl)phenyl]sulfonylpyridazine-3-carboxamide |
| 2R94 | 6-[4-[[4-(5-Hydroxypyridin-3-yl)thiophen-2-yl]methyl]piperazin-1-yl]-N-[4-(2-phenylsulfanylethylamino)-3-(trifluoromethyl)phenyl]sulfonylpyridazine-3-carboxamide |
| 2R95 | 6-[4-[[2-[2-(3-Hydroxyphenyl)ethynyl]phenyl]methyl]piperazin-1-yl]-N-[4-(2-phenylsulfanylethylamino)-3-(trifluoromethyl)phenyl]sulfonylpyridazine-3-carboxamide |
| 2R96 | 6-[4-[[2-[2-(5-Hydroxypyridin-3-yl)ethynyl]phenyl]methyl]piperazin-1-yl]-N-[4-(2-phenylsulfanylethylamino)-3-(trifluoromethyl)phenyl]sulfonylpyridazine-3-carboxamide |
| 2R97 | 4-[4-[1-[4-(3-Hydroxyphenyl)phenyl]ethyl]piperazin-1-yl]-N-[4-(2-phenylsulfanylethylamino)-3-(trifluoromethyl)phenyl]sulfonylbenzamide |
| 2R98 | 4-[4-[1-[4-(5-Hydroxypyridin-3-yl)phenyl]ethyl]piperazin-1-yl]-N-[4-(2-phenylsulfanylethylamino)-3-(trifluoromethyl)phenyl]sulfonylbenzamide |
| 2R99 | 4-[4-[1-[2-Fluoro-4-(3-hydroxyphenyl)phenyl]ethyl]piperazin-1-yl]-N-[4-(2-phenylsulfanylethylamino)-3-(trifluoromethyl)phenyl]sulfonylbenzamide |
| 2R100 | 4-[4-[1-[2-Fluoro-4-(5-hydroxypyridin-3-yl)phenyl]ethyl]piperazin-1-yl]-N-[4-(2-phenylsulfanylethylamino)-3-(trifluoromethyl)phenyl]sulfonylbenzamide |

[Table EJ02] to [Table EJ12] describe the retention time (min) of the observed UV peak, the $(M+H)^+$ observed in that UV peak, the retention time of the MS peak in the MS chromatogram, and the number of the compound attributed to the UV peak and the MS peak. Since two compounds may be missing in Example 6-2-2 and two compounds may be missing in Example 6-2-3, 44 compounds may be missing among the 1100 compounds shown in [Table EU02] to [Table EIJ12].

[Table 84]

[Table EJ02]

| Mixture No. | mixEJ02 | | | | | | |
|---|---|---|---|---|---|---|---|
| m/z [M+H]+ | Retention time of MS peak (min) | Retention time of UV peak (min) | Attributed compound No. | m/z [M+H]+ | Retention time of MS peak (min) | Retention time of UV peak (min) | Attributed compound No. |
| 439.191 | 3.66 | 3.624 | 1M94 | 490.27 | 6.277 | 6.247 | 1M59, 1M83 |
| 437.201 | 3.76 | 3.716 | 1M68 | 454.249 | 6.28 | 6.247 | 1M69 |
| 451.18 | 3.88 | 3.847 | 1M24, 1M78 | 458.244 | 6.303 | 6.247 | 1M25, 1M49 |
| 445.26 | 3.917 | 3.872 | 1M48, 1M98 | 482.255 | 6.343 | 6.313 | 1M91 |
| 463.214 | 3.967 | 3.912 | 1M100, 1M28, 1M80 | 474.25 | 6.37 | 6.313 | 1M11, 1M55 |
| 449.235 | 3.98 | 3.912 | 1M52 | 457.235 | 6.433 | 6.393 | 1M96 |
| 433.235 | 4.083 | 4.045 | 1M72 | 481.26 | 6.463 | 6.393 | 1M66 |
| 461.23 | 4.107 | 4.045 | 1M04, 1M54, 1M76 | 480.265 | 6.473 | 6.435 | 1M65 |
| 431.244 | 4.17 | 4.133 | 1M46 | 490.27 | 6.513 | 6.473 | 1M59, 1M83 |
| 459.239 | 4.347 | 4.323 | 1M26, 1M50 | 500.291 | 6.67 | 6.633 | 1M37, 1M87 |
| 493.256 | 4.357 | 4.323 | 1M86 | 515.201 | 6.673 | 6.633 | 1M18, 1M64 |
| 483.25 | 4.463 | 4.433 | 1M92 | 488.291 | 6.68 | 6.633 | 1M57 |
| 477.261 | 4.487 | 4.433 | 1M82 | 498.236 | 6.75 | 6.708 | 1M61 |
| 491.265 | 4.55 | 4.509 | 1M60, 1M84 | 503.277 | 6.837 | 6.808 | 1M16 |
| 475.245 | 4.643 | 4.599 | 1M12, 1M56 | 516.222 | 6.837 | 6.808 | 1M89 |
| 491.265 | 4.807 | 4.777 | 1M60, 1M84 | 581.223 | 6.88 | 6.825 | 1M22 |
| 489.286 | 4.96 | 4.888 | 1M58 | 514.206 | 6.923 | 6.88 | 1M17, 1M63 |
| 501.286 | 5.09 | 5.051 | 1M38, 1M88 | 513.211 | 7.07 | 7.024 | 1M40 |
| 453.17 | 5.093 | 5.051 | 1M02 | 501.286 | 7.157 | 7.121 | 1M38, 1M88 |
| 461.23 | 5.127 | 5.051 | 1M04, 1M54, 1M76 | 579.233 | 7.223 | 7.184 | 1M44 |
| 461.23 | 5.147 | 5.109 | 1M04, 1M54, 1M76 | 445.26 | 7.293 | 7.229 | 1M48, 1M98 |
| 499.232 | 5.193 | 5.152 | 1M62 | 469.223 | 7.447 | 7.44 | 1M30, 1M31 |
| 455.244 | 5.23 | 5.189 | 1M70 | 471.214 | 7.477 | 7.44 | 1M08, 1M09 |
| 517.217 | 5.27 | 5.231 | 1M90 | 452.175 | 7.697 | 7.653 | 1M01 |
| 465.204 | 5.297 | 5.261 | 1M06 | 463.214 | 7.867 | 7.844 | 1M100, 1M28, 1M80 |
| 515.201 | 5.367 | 5.335 | 1M18, 1M64 | 464.209 | 7.88 | 7.844 | 1M05 |
| 438.196 | 5.403 | 5.368 | 1M93 | 470.219 | 7.887 | 7.844 | 1M07, 1M32 |
| 432.239 | 5.477 | 5.447 | 1M71 | 469.223 | 7.89 | 7.844 | 1M30, 1M31 |
| 451.18 | 5.5 | 5.447 | 1M24, 1M78 | 460.234 | 8.04 | 8.003 | 1M03, 1M53, 1M75 |
| 459.239 | 5.513 | 5.467 | 1M26, 1M50 | 450.185 | 8.157 | 8.127 | 1M23, 1M77 |
| 436.205 | 5.567 | 5.525 | 1M67 | 470.219 | 8.21 | 8.127 | 1M07, 1M32 |
| 450.185 | 5.623 | 5.587 | 1M23, 1M77 | 539.201 | 8.247 | 8.207 | 1M20 |
| 475.245 | 5.657 | 5.619 | 1M12, 1M56 | 462.219 | 8.283 | 8.251 | 1M27, 1M79, 1M99 |
| 430.249 | 5.66 | 5.619 | 1M45 | 463.214 | 8.303 | 8.251 | 1M100, 1M28, 1M80 |
| 462.219 | 5.73 | 5.668 | 1M27, 1M79, 1M99 | 458.244 | 8.447 | 8.407 | 1M25, 1M49 |
| 447.25 | 5.76 | 5.721 | 1M74 | 474.25 | 8.473 | 8.436 | 1M11, 1M55 |
| 446.255 | 5.76 | 5.721 | 1M73 | 468.228 | 8.583 | 8.52 | 1M29 |
| 497.23 | 5.763 | 5.721 | 1M14 | 496.234 | 8.6 | 8.567 | 1M13 |
| 448.239 | 5.783 | 5.733 | 1M51 | 537.211 | 8.617 | 8.567 | 1M42 |
| 472.209 | 5.843 | 5.801 | 1M10, 1M33 | 471.214 | 8.813 | 8.792 | 1M08, 1M09 |
| 444.265 | 5.923 | 5.905 | 1M47, 1M97 | 472.209 | 8.84 | 8.792 | 1M10, 1M33 |
| 460.234 | 5.933 | 5.905 | 1M03, 1M53, 1M75 | 494.244 | 8.93 | 8.891 | 1M35 |
| 444.265 | 5.943 | 5.905 | 1M47, 1M97 | 514.206 | 9.08 | 9.047 | 1M17, 1M63 |
| 462.219 | 5.967 | 5.905 | 1M27, 1M79, 1M99 | 512.216 | 9.413 | 9.368 | 1M39 |
| 473.255 | 6.01 | 5.972 | 1M34 | 580.228 | 9.49 | 9.457 | 1M21 |
| 492.261 | 6.06 | 6.023 | 1M85 | 502.281 | 9.71 | 9.676 | 1M15 |
| 495.239 | 6.113 | 6.076 | 1M36 | 578.237 | 9.81 | 9.764 | 1M43 |
| 460.234 | 6.16 | 6.076 | 1M03, 1M53, 1M75 | 538.206 | 9.863 | 9.824 | 1M19 |
| 456.239 | 6.187 | 6.149 | 1M95 | 500.291 | 9.99 | 9.956 | 1M37, 1M87 |
| 476.266 | 6.197 | 6.149 | 1M81 | 536.216 | 10.157 | 10.116 | 1M41 |

[Table 85]

[Table EJ03]

| Mixture No. | mixEJ03 | | | | | | |
|---|---|---|---|---|---|---|---|
| m/z [M+H]+ | Retention time of MS peak (min) | Retention time of UV peak (min) | Attributed compound No. | m/z [M+H]+ | Retention time of MS peak (min) | Retention time of UV peak (min) | Attributed compound No. |
| 479.147 | 3.777 | 3.73 | 1K94 | 498.2 | 6.57 | 6.54 | 1K25, 1K49 |
| 487.206 | 3.887 | 3.85 | 1K74 | 522.211 | 6.577 | 6.54 | 1K91 |
| 477.157 | 3.93 | 3.89 | 1K68 | 514.206 | 6.613 | 6.57 | 1K11, 1K55 |
| 491.136 | 4.01 | 3.98 | 1K24, 1K78 | 510.175 | 6.653 | 6.62 | 1K07, 1K32 |
| 503.17 | 4.063 | 4.03 | 1K100, 1K28, 1K80 | 530.226 | 6.717 | 6.69 | 1K59, 1K83 |
| 485.216 | 4.087 | 4.03 | 1K48, 1K98 | 520.221 | 6.737 | 6.69 | 1K65 |
| 473.191 | 4.23 | 4.2 | 1K72 | 528.247 | 6.907 | 6.87 | 1K57 |
| 501.186 | 4.297 | 4.26 | 1K04, 1K54, 1K76 | 540.247 | 6.907 | 6.87 | 1K37, 1K87 |
| 471.2 | 4.387 | 4.36 | 1K46 | 555.157 | 6.987 | 6.94 | 1K18, 1K64 |
| 501.186 | 4.42 | 4.36 | 1K04, 1K54, 1K76 | 538.192 | 7.02 | 6.98 | 1K61 |
| 533.212 | 4.49 | 4.46 | 1K86 | 556.178 | 7.077 | 7.07 | 1K89 |
| 523.206 | 4.633 | 4.6 | 1K92 | 484.221 | 7.087 | 7.07 | 1K47, 1K97 |
| 517.217 | 4.637 | 4.6 | 1K82 | 543.233 | 7.103 | 7.07 | 1K16 |
| 531.221 | 4.737 | 4.71 | 1K60, 1K84 | 557.173 | 7.107 | 7.07 | 1K90 |
| 521.216 | 4.817 | 4.77 | 1K66 | 554.162 | 7.187 | 7.15 | 1K17, 1K63 |
| 515.201 | 4.867 | 4.82 | 1K12, 1K56 | 553.167 | 7.43 | 7.4 | 1K40 |
| 531.221 | 4.99 | 4.95 | 1K60, 1K84 | 541.242 | 7.487 | 7.45 | 1K38, 1K88 |
| 529.242 | 5.2 | 5.16 | 1K58 | 619.189 | 7.533 | 7.5 | 1K44 |
| 541.242 | 5.3 | 5.26 | 1K38, 1K88 | 509.18 | 7.783 | 7.7 | 1K30, 1K31 |
| 493.126 | 5.347 | 5.3 | 1K02 | 511.17 | 7.81 | 7.77 | 1K08, 1K09 |
| 502.175 | 5.387 | 5.35 | 1K27, 1K79, 1K99 | 492.131 | 8.05 | 8.01 | 1K01 |
| 501.186 | 5.39 | 5.35 | 1K04, 1K54, 1K76 | 504.165 | 8.203 | 8.17 | 1K05 |
| 539.188 | 5.46 | 5.42 | 1K62 | 503.17 | 8.213 | 8.17 | 1K100, 1K28, 1K80 |
| 505.161 | 5.567 | 5.53 | 1K06 | 510.175 | 8.25 | 8.22 | 1K07, 1K32 |
| 555.157 | 5.647 | 5.6 | 1K18, 1K64 | 509.18 | 8.253 | 8.22 | 1K30, 1K31 |
| 472.195 | 5.67 | 5.64 | 1K71 | 511.17 | 8.27 | 8.22 | 1K08, 1K09 |
| 499.195 | 5.82 | 5.79 | 1K26, 1K50 | 499.195 | 8.36 | 8.33 | 1K26, 1K50 |
| 476.161 | 5.823 | 5.79 | 1K67 | 500.19 | 8.363 | 8.33 | 1K03, 1K53, 1K75 |
| 490.141 | 5.837 | 5.79 | 1K23, 1K77 | 579.157 | 8.533 | 8.51 | 1K20 |
| 491.136 | 5.847 | 5.79 | 1K24, 1K78 | 490.141 | 8.55 | 8.51 | 1K23, 1K77 |
| 470.205 | 5.903 | 5.88 | 1K45 | 502.175 | 8.667 | 8.63 | 1K27, 1K79, 1K99 |
| 515.201 | 5.91 | 5.88 | 1K12, 1K56 | 495.2 | 8.753 | 8.63 | 1K70 |
| 502.175 | 5.923 | 5.88 | 1K27, 1K79, 1K99 | 514.206 | 8.817 | 8.78 | 1K11, 1K55 |
| 486.211 | 5.96 | 5.92 | 1K73 | 498.2 | 8.823 | 8.78 | 1K25, 1K49 |
| 537.186 | 6.027 | 5.99 | 1K14 | 536.19 | 8.89 | 8.85 | 1K13 |
| 488.196 | 6.043 | 5.99 | 1K51 | 508.184 | 8.937 | 8.85 | 1K29 |
| 503.17 | 6.053 | 6.01 | 1K100, 1K28, 1K80 | 512.165 | 9.213 | 9.18 | 1K10, 1K33 |
| 489.191 | 6.053 | 6.01 | 1K52 | 512.165 | 9.24 | 9.18 | 1K10, 1K33 |
| 500.19 | 6.187 | 6.15 | 1K03, 1K53, 1K75 | 534.2 | 9.29 | 9.25 | 1K35 |
| 484.221 | 6.2 | 6.15 | 1K47, 1K97 | 554.162 | 9.37 | 9.32 | 1K17, 1K63 |
| 485.216 | 6.207 | 6.15 | 1K48, 1K98 | 620.184 | 9.733 | 9.7 | 1K21 |
| 532.217 | 6.247 | 6.21 | 1K85 | 621.179 | 9.733 | 9.7 | 1K22 |
| 513.211 | 6.327 | 6.29 | 1K34 | 552.172 | 9.74 | 9.7 | 1K39 |
| 497.191 | 6.367 | 6.34 | 1K96 | 542.237 | 9.96 | 9.92 | 1K15 |
| 500.19 | 6.373 | 6.34 | 1K03, 1K53, 1K75 | 578.162 | 10.08 | 10.07 | 1K19 |
| 496.195 | 6.38 | 6.34 | 1K95 | 618.193 | 10.11 | 10.07 | 1K43 |
| 516.222 | 6.4 | 6.34 | 1K81 | 540.247 | 10.313 | 10.28 | 1K37, 1K87 |
| 535.195 | 6.443 | 6.41 | 1K36 | 577.167 | 10.44 | 10.41 | 1K42 |
| 494.205 | 6.517 | 6.48 | 1K69 | 576.172 | 10.457 | 10.41 | 1K41 |
| 530.226 | 6.517 | 6.48 | 1K59, 1K83 | 478.152 | 14.887 | 13.56 | 1K93 |

[Table 86]

[Table EJ04]

| Mixture No. | mixEJ04 | | | | | | |
|---|---|---|---|---|---|---|---|
| m/z [M+H]+ | Retention time of MS peak (min) | Retention time of UV peak (min) | Attributed compound No. | m/z [M+H]+ | Retention time of MS peak (min) | Retention time of UV peak (min) | Attributed compound No. |
| 503.186 | 4.597 | 4.58 | 1O94 | 538.245 | 7.05 | 7.02 | 1O11, 1O55 |
| 501.195 | 4.62 | 4.58 | 1O68 | 536.204 | 7.063 | 7.02 | 1O10, 1O33 |
| 511.245 | 4.67 | 4.63 | 1O74 | 546.25 | 7.173 | 7.15 | 1O91 |
| 509.255 | 4.76 | 4.71 | 1O48, 1O98 | 544.259 | 7.187 | 7.15 | 1O65 |
| 527.209 | 4.82 | 4.78 | 1O100, 1O28, 1O80 | 547.245 | 7.197 | 7.15 | 1O92 |
| 515.175 | 4.847 | 4.81 | 1O24, 1O78 | 554.265 | 7.247 | 7.22 | 1O59, 1O83 |
| 525.224 | 4.927 | 4.88 | 1O04, 1O54, 1O76 | 552.286 | 7.33 | 7.29 | 1O57 |
| 495.239 | 5.05 | 5.01 | 1O46 | 534.214 | 7.35 | 7.29 | 1O07, 1O32 |
| 509.255 | 5.07 | 5.01 | 1O48, 1O98 | 562.231 | 7.45 | 7.41 | 1O61 |
| 523.234 | 5.187 | 5.15 | 1O26, 1O50 | 565.281 | 7.507 | 7.49 | 1O38, 1O88 |
| 557.251 | 5.193 | 5.15 | 1O86 | 564.286 | 7.527 | 7.49 | 1O37, 1O87 |
| 555.26 | 5.313 | 5.28 | 1O60, 1O84 | 578.201 | 7.61 | 7.56 | 1O17, 1O63 |
| 541.256 | 5.367 | 5.31 | 1O82 | 580.217 | 7.683 | 7.65 | 1O89 |
| 545.255 | 5.4 | 5.36 | 1O66 | 579.196 | 7.827 | 7.83 | 1O18, 1O64 |
| 539.24 | 5.44 | 5.4 | 1O12, 1O56 | 645.218 | 7.85 | 7.83 | 1O22 |
| 555.26 | 5.67 | 5.63 | 1O60, 1O84 | 567.271 | 7.877 | 7.83 | 1O16 |
| 553.281 | 5.75 | 5.71 | 1O58 | 565.281 | 8.053 | 8.03 | 1O38, 1O88 |
| 563.226 | 6.037 | 6.01 | 1O62 | 577.206 | 8.057 | 8.03 | 1O40 |
| 579.196 | 6.203 | 6.18 | 1O18, 1O64 | 643.228 | 8.077 | 8.03 | 1O44 |
| 581.212 | 6.213 | 6.18 | 1O90 | 515.175 | 8.43 | 8.28 | 1O24, 1O78 |
| 517.165 | 6.227 | 6.18 | 1O02 | 497.23 | 8.443 | 8.28 | 1O72 |
| 525.224 | 6.26 | 6.22 | 1O04, 1O54, 1O76 | 535.209 | 8.61 | 8.57 | 1O08, 1O09 |
| 502.191 | 6.333 | 6.3 | 1O93 | 535.209 | 8.637 | 8.57 | 1O08, 1O09 |
| 496.234 | 6.347 | 6.3 | 1O71 | 533.218 | 8.843 | 8.8 | 1O30, 1O31 |
| 500.2 | 6.37 | 6.3 | 1O67 | 516.17 | 8.893 | 8.86 | 1O01 |
| 494.244 | 6.427 | 6.39 | 1O45 | 529.199 | 9.033 | 9 | 1O06 |
| 523.234 | 6.503 | 6.46 | 1O26, 1O50 | 528.204 | 9.037 | 9 | 1O05 |
| 514.18 | 6.507 | 6.46 | 1O23, 1O77 | 514.18 | 9.153 | 9.11 | 1O23, 1O77 |
| 526.214 | 6.543 | 6.52 | 1O27, 1O79, 1O99 | 524.229 | 9.207 | 9.16 | 1O03, 1O53, 1O75 |
| 513.23 | 6.553 | 6.52 | 1O52 | 527.209 | 9.247 | 9.21 | 1O100, 1O28, 1O80 |
| 534.214 | 6.56 | 6.52 | 1O07, 1O32 | 526.214 | 9.247 | 9.21 | 1O27, 1O79, 1O99 |
| 508.259 | 6.56 | 6.52 | 1O47, 1O97 | 603.196 | 9.32 | 9.28 | 1O20 |
| 510.25 | 6.567 | 6.52 | 1O73 | 522.239 | 9.403 | 9.37 | 1O25, 1O49 |
| 512.234 | 6.59 | 6.52 | 1O51 | 601.206 | 9.513 | 9.48 | 1O42 |
| 524.229 | 6.667 | 6.64 | 1O03, 1O53, 1O75 | 533.218 | 9.527 | 9.48 | 1O30, 1O31 |
| 508.259 | 6.677 | 6.64 | 1O47, 1O97 | 532.223 | 9.537 | 9.48 | 1O29 |
| 539.24 | 6.703 | 6.64 | 1O12, 1O56 | 538.245 | 9.597 | 9.56 | 1O11, 1O55 |
| 526.214 | 6.713 | 6.64 | 1O27, 1O79, 1O99 | 560.229 | 9.7 | 9.67 | 1O13 |
| 527.209 | 6.783 | 6.75 | 1O100, 1O28, 1O80 | 536.204 | 9.767 | 9.73 | 1O10, 1O33 |
| 556.255 | 6.827 | 6.79 | 1O85 | 558.239 | 9.85 | 9.81 | 1O35 |
| 561.224 | 6.877 | 6.85 | 1O14 | 559.234 | 9.853 | 9.81 | 1O36 |
| 537.25 | 6.937 | 6.85 | 1O34 | 578.201 | 10.127 | 10.08 | 1O17, 1O63 |
| 554.265 | 6.957 | 6.94 | 1O59, 1O83 | 576.21 | 10.26 | 10.23 | 1O39 |
| 540.261 | 6.96 | 6.94 | 1O81 | 644.223 | 10.443 | 10.4 | 1O21 |
| 520.234 | 6.967 | 6.94 | 1O95 | 519.239 | 10.587 | 10.55 | 1O70 |
| 518.244 | 6.977 | 6.94 | 1O69 | 642.232 | 10.6 | 10.55 | 1O43 |
| 525.224 | 6.983 | 6.94 | 1O04, 1O54, 1O76 | 566.276 | 10.737 | 10.7 | 1O15 |
| 521.23 | 6.983 | 6.94 | 1O96 | 602.201 | 10.79 | 10.76 | 1O19 |
| 524.229 | 6.993 | 6.94 | 1O03, 1O53, 1O75 | 564.286 | 10.837 | 10.8 | 1O37, 1O87 |
| 522.239 | 7.02 | 6.94 | 1O25, 1O49 | 600.21 | 10.93 | 10.89 | 1O41 |

[Table 87]

[Table EJ05]

| Mixture No. | mixEJ04 | | | | | | | |
|---|---|---|---|---|---|---|---|---|
| m/z [M+H]+ | Retention time of MS peak (min) | Retention time of UV peak (min) | Attributed compound No. | | m/z [M+H]+ | Retention time of MS peak (min) | Retention time of UV peak (min) | Attributed compound No. |
| 551.338 | 5.62 | 5.62 | 1A48, 1A98 | | 581.323 | 8.607 | 8.607 | 1A12, 1A56 |
| 599.334 | 8.333 | 8.333 | 1A86 | | 586.343 | 8.623 | 8.623 | 1A65 |
| 597.344 | 8.37 | 8.37 | 1A60, 1A84 | | 568.297 | 11.383 | 11.383 | 1A27, 1A79, 1A99 |
| 596.348 | 8.363 | 8.363 | 1A59, 1A83 | | 570.287 | 11.327 | 11.327 | 1A05 |
| 598.339 | 8.32 | 8.32 | 1A85 | | 571.283 | 11.327 | 11.327 | 1A06 |
| 574.306 | 11.69 | 11.69 | 1A29 | | 568.297 | 11.357 | 11.357 | 1A27, 1A79, 1A99 |
| 576.297 | 11.71 | 11.71 | 1A07, 1A32 | | 645.28 | 11.567 | 11.567 | 1A20 |
| 563.313 | 7.57 | 7.57 | 1A96 | | 643.289 | 11.523 | 11.523 | 1A42 |
| 623.295 | 7.907 | 7.907 | 1A90 | | 565.317 | 11.533 | 11.533 | 1A26, 1A50 |
| 542.284 | 7.933 | 7.933 | 1A67 | | 564.322 | 11.55 | 11.55 | 1A25, 1A49 |
| 558.253 | 11.207 | 11.207 | 1A01 | | 589.329 | 7.063 | 7.063 | 1A92 |
| 566.313 | 8.593 | 8.593 | 1A03, 1A53, 1A75 | | 606.369 | 12.933 | 12.933 | 1A37, 1A87 |
| 562.318 | 8.54 | 8.54 | 1A95 | | 642.294 | 12.873 | 12.873 | 1A41 |
| 577.292 | 11.89 | 11.89 | 1A08, 1A09 | | 569.292 | 8.88 | 8.88 | 1A100, 1A28, 1A80 |
| 580.328 | 11.883 | 11.883 | 1A11, 1A55 | | 604.315 | 8.873 | 8.873 | 1A61 |
| 578.287 | 11.917 | 11.917 | 1A10, 1A33 | | 607.364 | 9.957 | 9.957 | 1A38, 1A88 |
| 581.323 | 11.907 | 11.907 | 1A12, 1A56 | | 603.308 | 8.943 | 8.943 | 1A14 |
| 578.287 | 11.89 | 11.89 | 1A10, 1A33 | | 619.289 | 10.1 | 10.1 | 1A40 |
| 583.339 | 6.98 | 6.98 | 1A82 | | 545.269 | 6.337 | 6.337 | 1A94 |
| 544.274 | 8.017 | 8.017 | 1A93 | | 543.279 | 6.357 | 6.357 | 1A68 |
| 550.343 | 8.117 | 8.117 | 1A47, 1A97 | | 687.301 | 9.807 | 9.807 | 1A22 |
| 553.329 | 8.123 | 8.123 | 1A74 | | 557.258 | 8.46 | 8.46 | 1A24, 1A78 |
| 557.258 | 8.117 | 8.117 | 1A24, 1A78 | | 565.317 | 8.453 | 8.453 | 1A26, 1A50 |
| 556.263 | 8.123 | 8.123 | 1A23, 1A77 | | 582.344 | 8.46 | 8.46 | 1A81 |
| 550.343 | 8.133 | 8.133 | 1A47, 1A97 | | 564.322 | 8.473 | 8.473 | 1A25, 1A49 |
| 566.313 | 8.127 | 8.127 | 1A03, 1A53, 1A75 | | 594.369 | 8.68 | 8.68 | 1A57 |
| 569.292 | 6.487 | 6.487 | 1A100, 1A28, 1A80 | | 588.333 | 8.823 | 8.823 | 1A91 |
| 552.333 | 6.43 | 6.43 | 1A73 | | 559.249 | 8.237 | 8.237 | 1A02 |
| 551.338 | 6.427 | 6.427 | 1A48, 1A98 | | 567.308 | 8.273 | 8.273 | 1A04, 1A54, 1A76 |
| 567.308 | 6.59 | 6.59 | 1A04, 1A54, 1A76 | | 609.355 | 9.917 | 9.917 | 1A16 |
| 569.292 | 8.073 | 8.073 | 1A100, 1A28, 1A80 | | 685.311 | 9.89 | 9.89 | 1A44 |
| 568.297 | 8.073 | 8.073 | 1A27, 1A79, 1A99 | | 577.292 | 10.817 | 10.817 | 1A08, 1A09 |
| 554.318 | 8.07 | 8.07 | 1A51 | | 620.284 | 9.01 | 9.01 | 1A17, 1A63 |
| 580.328 | 8.433 | 8.433 | 1A11, 1A55 | | 606.369 | 9.18 | 9.18 | 1A37, 1A87 |
| 560.327 | 8.407 | 8.407 | 1A69 | | 567.308 | 6.823 | 6.823 | 1A04, 1A54, 1A76 |
| 596.348 | 8.727 | 8.727 | 1A59, 1A83 | | 587.338 | 7.013 | 7.013 | 1A66 |
| 575.302 | 10.913 | 10.913 | 1A30, 1A31 | | 538.318 | 7.967 | 7.967 | 1A71 |
| 576.297 | 10.903 | 10.903 | 1A07, 1A32 | | 536.327 | 7.94 | 7.94 | 1A45 |
| 575.302 | 10.89 | 10.89 | 1A30, 1A31 | | 555.313 | 6.66 | 6.66 | 1A52 |
| 561.322 | 7.543 | 7.543 | 1A70 | | 595.364 | 7.26 | 7.26 | 1A58 |
| 608.36 | 13.08 | 13.08 | 1A15 | | 686.306 | 12.583 | 12.583 | 1A21 |
| 579.333 | 8.787 | 8.787 | 1A34 | | 620.284 | 12.363 | 12.363 | 1A17, 1A63 |
| 597.344 | 6.897 | 6.897 | 1A60, 1A84 | | 601.317 | 9.07 | 9.07 | 1A36 |
| 605.31 | 7.63 | 7.63 | 1A62 | | 602.313 | 12.03 | 12.03 | 1A13 |
| 539.313 | 6.747 | 6.747 | 1A72 | | 622.3 | 9.32 | 9.32 | 1A89 |
| 537.322 | 6.733 | 6.733 | 1A46 | | 556.263 | 11.283 | 11.283 | 1A23, 1A77 |
| 621.28 | 7.763 | 7.763 | 1A18, 1A64 | | 684.316 | 12.527 | 12.527 | 1A43 |
| 607.364 | 7.757 | 7.757 | 1A38, 1A88 | | 644.284 | 12.973 | 12.973 | 1A19 |
| 621.28 | 10.007 | 10.007 | 1A18, 1A64 | | 600.322 | 11.967 | 11.967 | 1A35 |
| 566.313 | 8.613 | 8.613 | 1A03, 1A53, 1A75 | | 618.294 | 12.29 | 12.29 | 1A39 |

[Table 88]

[Table EJ06]

| Mixture No. | mixEJ04 | | | | | | |
|---|---|---|---|---|---|---|---|
| m/z [M+H]+ | Retention time of MS peak (min) | Retention time of UV peak (min) | Attributed compound No. | m/z [M+H]+ | Retention time of MS peak (min) | Retention time of UV peak (min) | Attributed compound No. |
| 559.285 | 6.01 | 5.96 | 3A94 | 578.338 | 8.843 | 8.8 | 3A25, 3A49 |
| 567.344 | 6.063 | 6.01 | 3A74 | 579.333 | 8.853 | 8.8 | 3A26, 3A50 |
| 571.274 | 6.3 | 6.25 | 3A24, 3A78 | 600.358 | 9.013 | 8.99 | 3A65 |
| 553.329 | 6.4 | 6.37 | 3A72 | 608.385 | 9.02 | 8.99 | 3A57 |
| 613.35 | 6.5 | 6.44 | 3A86 | 579.333 | 9.04 | 8.99 | 3A26, 3A50 |
| 597.355 | 6.66 | 6.63 | 3A82 | 571.274 | 9.08 | 8.99 | 3A24, 3A78 |
| 603.344 | 6.677 | 6.63 | 3A92 | 618.33 | 9.293 | 9.25 | 3A61 |
| 557.294 | 6.713 | 6.67 | 3A68 | 701.317 | 9.303 | 9.25 | 3A22 |
| 565.354 | 6.767 | 6.73 | 3A48, 3A98 | 623.37 | 9.343 | 9.25 | 3A16 |
| 565.354 | 6.78 | 6.73 | 3A48, 3A98 | 593.349 | 9.38 | 9.34 | 3A34 |
| 581.323 | 6.933 | 6.89 | 3A04, 3A54, 3A76 | 635.295 | 9.407 | 9.34 | 3A18, 3A64 |
| 611.359 | 6.933 | 6.89 | 3A60, 3A84 | 634.3 | 9.43 | 9.39 | 3A17, 3A63 |
| 551.338 | 7.083 | 7.05 | 3A46 | 583.308 | 9.55 | 9.51 | 3A100, 3A28, 3A80 |
| 611.359 | 7.25 | 7.21 | 3A60, 3A84 | 583.308 | 9.573 | 9.51 | 3A100, 3A28, 3A80 |
| 595.339 | 7.34 | 7.3 | 3A12, 3A56 | 615.333 | 9.757 | 9.72 | 3A36 |
| 621.38 | 7.34 | 7.3 | 3A38, 3A88 | 590.313 | 10.13 | 10.09 | 3A07, 3A32 |
| 601.354 | 7.393 | 7.35 | 3A66 | 591.308 | 10.213 | 10.18 | 3A08, 3A09 |
| 637.311 | 7.473 | 7.44 | 3A90 | 591.308 | 10.237 | 10.18 | 3A08, 3A09 |
| 609.38 | 7.6 | 7.57 | 3A58 | 564.358 | 10.467 | 10.4 | 3A47, 3A97 |
| 558.29 | 7.613 | 7.57 | 3A93 | 572.269 | 10.553 | 10.57 | 3A01 |
| 552.333 | 7.627 | 7.57 | 3A71 | 621.38 | 10.707 | 10.68 | 3A38, 3A88 |
| 573.264 | 7.737 | 7.71 | 3A02 | 584.303 | 10.71 | 10.68 | 3A05 |
| 570.278 | 7.75 | 7.71 | 3A23, 3A77 | 633.305 | 10.847 | 10.82 | 3A40 |
| 581.323 | 7.76 | 7.71 | 3A04, 3A54, 3A76 | 580.328 | 10.883 | 10.82 | 3A03, 3A53, 3A75 |
| 582.313 | 7.773 | 7.71 | 3A27, 3A79, 3A99 | 659.295 | 10.93 | 10.88 | 3A20 |
| 566.349 | 7.8 | 7.71 | 3A73 | 590.313 | 11.147 | 11.09 | 3A07, 3A32 |
| 575.338 | 7.88 | 7.85 | 3A70 | 596.36 | 11.293 | 11.25 | 3A81 |
| 619.325 | 8.013 | 7.98 | 3A62 | 594.344 | 11.303 | 11.25 | 3A11, 3A55 |
| 612.354 | 8.023 | 7.98 | 3A85 | 616.328 | 11.393 | 11.35 | 3A13 |
| 595.339 | 8.14 | 8.11 | 3A12, 3A56 | 589.317 | 11.623 | 11.6 | 3A30, 3A31 |
| 635.295 | 8.147 | 8.11 | 3A18, 3A64 | 634.3 | 11.77 | 11.73 | 3A17, 3A63 |
| 585.298 | 8.157 | 8.11 | 3A06 | 700.322 | 12.037 | 11.99 | 3A21 |
| 580.328 | 8.197 | 8.11 | 3A03, 3A53, 3A75 | 589.317 | 12.06 | 11.99 | 3A30, 3A31 |
| 550.343 | 8.293 | 8.26 | 3A45 | 570.278 | 12.087 | 12.05 | 3A23, 3A77 |
| 556.299 | 8.297 | 8.26 | 3A67 | 582.313 | 12.153 | 12.11 | 3A27, 3A79, 3A99 |
| 577.329 | 8.32 | 8.26 | 3A96 | 582.313 | 12.167 | 12.11 | 3A27, 3A79, 3A99 |
| 576.333 | 8.323 | 8.26 | 3A95 | 583.308 | 12.183 | 12.11 | 3A100, 3A28, 3A80 |
| 602.349 | 8.363 | 8.33 | 3A91 | 657.305 | 12.347 | 12.34 | 3A42 |
| 610.364 | 8.4 | 8.33 | 3A59, 3A83 | 578.338 | 12.367 | 12.34 | 3A25, 3A49 |
| 617.323 | 8.413 | 8.37 | 3A14 | 658.3 | 12.393 | 12.34 | 3A19 |
| 569.329 | 8.417 | 8.37 | 3A52 | 622.375 | 12.45 | 12.34 | 3A15 |
| 568.333 | 8.42 | 8.37 | 3A51 | 588.322 | 12.557 | 12.52 | 3A29 |
| 581.323 | 8.45 | 8.42 | 3A04, 3A54, 3A76 | 592.303 | 12.703 | 12.67 | 3A10, 3A33 |
| 580.328 | 8.46 | 8.42 | 3A03, 3A53, 3A75 | 592.303 | 12.737 | 12.67 | 3A10, 3A33 |
| 564.358 | 8.473 | 8.42 | 3A47, 3A97 | 614.338 | 12.833 | 12.79 | 3A35 |
| 620.385 | 8.69 | 8.67 | 3A37, 3A87 | 632.309 | 13.12 | 13.08 | 3A39 |
| 610.364 | 8.72 | 8.67 | 3A59, 3A83 | 698.331 | 13.33 | 13.29 | 3A43 |
| 594.344 | 8.77 | 8.75 | 3A11, 3A55 | 699.327 | 13.337 | 13.29 | 3A44 |
| 574.343 | 8.79 | 8.75 | 3A69 | 656.309 | 13.693 | 13.66 | 3A41 |
| 636.316 | 8.833 | 8.8 | 3A89 | 620.385 | 13.84 | 13.79 | 3A37, 3A87 |

[Table 89]

[Table EJ07]

| Mixture No. | mixEJ07 | | | | | | |
|---|---|---|---|---|---|---|---|
| m/z [M+H]+ | Retention time of MS peak (min) | Retention time of UV peak (min) | Attributed compound No. | m/z [M+H]+ | Retention time of MS peak (min) | Retention time of UV peak (min) | Attributed compound No. |
| 475.122 | 2.943 | 2.9 | 2I94 | 526.201 | 5.99 | 5.96 | 2I59, 2I83 |
| 483.181 | 3.043 | 3 | 2I74 | 511.176 | 5.99 | 5.96 | 2I12, 2I56 |
| 481.19 | 3.423 | 3.36 | 2I48, 2I98 | 539.207 | 6.087 | 6.05 | 2I16 |
| 469.165 | 3.44 | 3.4 | 2I72 | 516.195 | 6.117 | 6.05 | 2I65 |
| 485.165 | 3.5 | 3.46 | 2I52 | 617.154 | 6.14 | 6.11 | 2I22 |
| 497.16 | 3.61 | 3.57 | 2I04, 2I54, 2I76 | 536.221 | 6.157 | 6.11 | 2I37, 2I87 |
| 467.175 | 3.743 | 3.7 | 2I46 | 524.221 | 6.327 | 6.31 | 2I57 |
| 529.186 | 3.743 | 3.7 | 2I86 | 552.152 | 6.35 | 6.31 | 2I89 |
| 513.191 | 3.817 | 3.79 | 2I82 | 534.167 | 6.42 | 6.39 | 2I61 |
| 495.17 | 3.937 | 3.9 | 2I26, 2I50 | 549.141 | 6.51 | 6.47 | 2I40 |
| 527.196 | 4.147 | 4.12 | 2I60, 2I84 | 551.132 | 6.6 | 6.57 | 2I18, 2I64 |
| 517.19 | 4.15 | 4.12 | 2I66 | 473.131 | 6.603 | 6.57 | 2I68 |
| 489.101 | 4.307 | 4.27 | 2I02 | 550.137 | 6.607 | 6.57 | 2I17, 2I63 |
| 497.16 | 4.353 | 4.32 | 2I04, 2I54, 2I76 | 487.11 | 6.66 | 6.63 | 2I24, 2I78 |
| 537.217 | 4.41 | 4.38 | 2I38, 2I88 | 537.217 | 6.667 | 6.63 | 2I38, 2I88 |
| 501.135 | 4.453 | 4.41 | 2I06 | 507.145 | 6.667 | 6.63 | 2I08, 2I09 |
| 525.217 | 4.53 | 4.5 | 2I58 | 615.163 | 6.743 | 6.7 | 2I44 |
| 553.148 | 4.61 | 4.58 | 2I90 | 488.106 | 6.83 | 6.79 | 2I01 |
| 535.162 | 4.757 | 4.69 | 2I62 | 500.14 | 7.007 | 6.97 | 2I05 |
| 468.17 | 4.847 | 4.81 | 2I71 | 496.165 | 7.16 | 7.13 | 2I03, 2I53, 2I75 |
| 474.126 | 4.92 | 4.92 | 2I93 | 497.16 | 7.167 | 7.13 | 2I04, 2I54, 2I76 |
| 533.16 | 4.95 | 4.92 | 2I14 | 496.165 | 7.187 | 7.13 | 2I03, 2I53, 2I75 |
| 511.176 | 4.96 | 4.92 | 2I12, 2I56 | 507.145 | 7.32 | 7.29 | 2I08, 2I09 |
| 495.17 | 4.97 | 4.92 | 2I26, 2I50 | 505.154 | 7.327 | 7.29 | 2I30, 2I31 |
| 486.115 | 4.983 | 4.92 | 2I23, 2I77 | 506.149 | 7.333 | 7.29 | 2I07, 2I32 |
| 499.145 | 5.093 | 5.09 | 2I100, 2I28, 2I80 | 505.154 | 7.377 | 7.29 | 2I30, 2I31 |
| 498.149 | 5.11 | 5.09 | 2I27, 2I79, 2I99 | 575.132 | 7.44 | 7.39 | 2I20 |
| 472.136 | 5.113 | 5.09 | 2I67 | 486.115 | 7.567 | 7.54 | 2I23, 2I77 |
| 482.186 | 5.14 | 5.09 | 2I73 | 487.11 | 7.57 | 7.54 | 2I24, 2I78 |
| 466.18 | 5.23 | 5.19 | 2I45 | 498.149 | 7.677 | 7.66 | 2I27, 2I79, 2I99 |
| 484.17 | 5.36 | 5.32 | 2I51 | 498.149 | 7.697 | 7.66 | 2I27, 2I79, 2I99 |
| 528.191 | 5.467 | 5.43 | 2I85 | 510.181 | 7.7 | 7.66 | 2I11, 2I55 |
| 480.195 | 5.517 | 5.49 | 2I47, 2I97 | 499.145 | 7.717 | 7.66 | 2I100, 2I28, 2I80 |
| 496.165 | 5.52 | 5.49 | 2I03, 2I53, 2I75 | 532.165 | 7.73 | 7.66 | 2I13 |
| 481.19 | 5.52 | 5.49 | 2I48, 2I98 | 494.174 | 7.843 | 7.81 | 2I25, 2I49 |
| 509.185 | 5.52 | 5.49 | 2I34 | 504.159 | 8 | 7.97 | 2I29 |
| 531.17 | 5.573 | 5.53 | 2I36 | 573.141 | 8.07 | 8.04 | 2I42 |
| 493.165 | 5.627 | 5.6 | 2I96 | 508.14 | 8.263 | 8.25 | 2I10, 2I33 |
| 492.17 | 5.64 | 5.6 | 2I95 | 499.145 | 8.273 | 8.25 | 2I100, 2I28, 2I80 |
| 512.196 | 5.643 | 5.6 | 2I81 | 550.137 | 8.277 | 8.25 | 2I17, 2I63 |
| 506.149 | 5.687 | 5.65 | 2I07, 2I32 | 508.14 | 8.283 | 8.25 | 2I10, 2I33 |
| 519.181 | 5.793 | 5.77 | 2I92 | 530.174 | 8.343 | 8.3 | 2I35 |
| 518.186 | 5.797 | 5.77 | 2I91 | 480.195 | 8.347 | 8.3 | 2I47, 2I97 |
| 551.132 | 5.823 | 5.77 | 2I18, 2I64 | 616.158 | 8.74 | 8.7 | 2I21 |
| 526.201 | 5.847 | 5.82 | 2I59, 2I83 | 548.146 | 8.86 | 8.82 | 2I39 |
| 527.196 | 5.863 | 5.82 | 2I60, 2I84 | 538.212 | 8.877 | 8.82 | 2I15 |
| 491.175 | 5.903 | 5.87 | 2I70 | 574.137 | 9.09 | 9.06 | 2I19 |
| 490.18 | 5.92 | 5.87 | 2I69 | 614.168 | 9.277 | 9.25 | 2I43 |
| 494.174 | 5.92 | 5.87 | 2I25, 2I49 | 536.221 | 9.43 | 9.39 | 2I37, 2I87 |
| 510.181 | 5.99 | 5.96 | 2I11, 2I55 | 572.146 | 9.627 | 9.59 | 2I41 |

[Table 90]

[Table EJ08]

| Mixture No. | mixEJ08 | | | | | | |
|---|---|---|---|---|---|---|---|
| m/z [M+H]+ | Retention time of MS peak (min) | Retention time of UV peak (min) | Attributed compound No. | m/z [M+H]+ | Retention time of MS peak (min) | Retention time of UV peak (min) | Attributed compound No. |
| 517.169 | 3.873 | 3.83 | 2L94 | 532.226 | 6.513 | 6.48 | 2L69 |
| 525.228 | 3.92 | 3.88 | 2L74 | 536.221 | 6.56 | 6.52 | 2L25, 2L49 |
| 515.178 | 4.04 | 4 | 2L68 | 552.228 | 6.587 | 6.52 | 2L11, 2L55 |
| 541.192 | 4.073 | 4.04 | 2L100, 2L28, 2L80 | 560.233 | 6.633 | 6.59 | 2L91 |
| 529.157 | 4.077 | 4.04 | 2L24, 2L78 | 548.196 | 6.643 | 6.59 | 2L07, 2L32 |
| 523.237 | 4.17 | 4.12 | 2L48, 2L98 | 568.248 | 6.723 | 6.69 | 2L59, 2L83 |
| 527.212 | 4.287 | 4.24 | 2L52 | 558.242 | 6.737 | 6.69 | 2L65 |
| 511.212 | 4.367 | 4.32 | 2L72 | 566.268 | 6.887 | 6.86 | 2L57 |
| 539.207 | 4.437 | 4.41 | 2L04, 2L54, 2L76 | 593.179 | 6.997 | 6.97 | 2L18, 2L64 |
| 571.233 | 4.47 | 4.41 | 2L86 | 578.268 | 7.01 | 6.97 | 2L37, 2L87 |
| 509.222 | 4.483 | 4.44 | 2L46 | 576.214 | 7.037 | 6.97 | 2L61 |
| 555.238 | 4.673 | 4.65 | 2L82 | 581.254 | 7.123 | 7.1 | 2L16 |
| 561.228 | 4.697 | 4.65 | 2L92 | 659.201 | 7.137 | 7.1 | 2L22 |
| 559.237 | 4.853 | 4.81 | 2L66 | 539.207 | 7.143 | 7.1 | 2L04, 2L54, 2L76 |
| 569.243 | 5 | 4.97 | 2L60, 2L84 | 594.199 | 7.18 | 7.15 | 2L89 |
| 567.264 | 5.213 | 5.18 | 2L58 | 592.184 | 7.207 | 7.15 | 2L17, 2L63 |
| 533.222 | 5.36 | 5.34 | 2L70 | 591.188 | 7.423 | 7.38 | 2L40 |
| 531.148 | 5.367 | 5.34 | 2L02 | 579.264 | 7.473 | 7.44 | 2L38, 2L88 |
| 579.264 | 5.38 | 5.34 | 2L38, 2L88 | 657.21 | 7.513 | 7.48 | 2L44 |
| 540.196 | 5.397 | 5.34 | 2L27, 2L79, 2L99 | 549.191 | 7.833 | 7.79 | 2L08, 2L09 |
| 539.207 | 5.417 | 5.34 | 2L04, 2L54, 2L76 | 530.153 | 8.07 | 8.03 | 2L01 |
| 577.209 | 5.493 | 5.47 | 2L62 | 547.201 | 8.213 | 8.19 | 2L30, 2L31 |
| 595.194 | 5.57 | 5.53 | 2L90 | 548.196 | 8.227 | 8.19 | 2L07, 2L32 |
| 543.182 | 5.597 | 5.53 | 2L06 | 542.187 | 8.227 | 8.19 | 2L05 |
| 516.173 | 5.673 | 5.63 | 2L93 | 546.206 | 8.243 | 8.19 | 2L29 |
| 593.179 | 5.683 | 5.63 | 2L18, 2L64 | 538.212 | 8.39 | 8.35 | 2L03, 2L53, 2L75 |
| 510.217 | 5.707 | 5.67 | 2L71 | 528.162 | 8.503 | 8.47 | 2L23, 2L77 |
| 537.217 | 5.823 | 5.79 | 2L26, 2L50 | 617.179 | 8.56 | 8.52 | 2L20 |
| 514.183 | 5.837 | 5.79 | 2L67 | 541.192 | 8.607 | 8.59 | 2L100, 2L28, 2L80 |
| 537.217 | 5.84 | 5.79 | 2L26, 2L50 | 540.196 | 8.627 | 8.59 | 2L27, 2L79, 2L99 |
| 528.162 | 5.863 | 5.83 | 2L23, 2L77 | 535.212 | 8.77 | 8.74 | 2L96 |
| 529.157 | 5.863 | 5.83 | 2L24, 2L78 | 536.221 | 8.78 | 8.74 | 2L25, 2L49 |
| 508.226 | 5.897 | 5.86 | 2L45 | 552.228 | 8.857 | 8.82 | 2L11, 2L55 |
| 540.196 | 5.913 | 5.86 | 2L27, 2L79, 2L99 | 553.223 | 8.867 | 8.82 | 2L12, 2L56 |
| 524.233 | 5.947 | 5.91 | 2L73 | 574.212 | 8.903 | 8.88 | 2L13 |
| 553.223 | 5.953 | 5.91 | 2L12, 2L56 | 547.201 | 8.933 | 8.88 | 2L30, 2L31 |
| 575.207 | 6.023 | 6 | 2L14 | 615.188 | 8.937 | 8.88 | 2L42 |
| 526.217 | 6.043 | 6 | 2L51 | 549.191 | 9.163 | 9.14 | 2L08, 2L09 |
| 541.192 | 6.06 | 6 | 2L100, 2L28, 2L80 | 550.187 | 9.183 | 9.14 | 2L10, 2L33 |
| 538.212 | 6.163 | 6.13 | 2L03, 2L53, 2L75 | 572.221 | 9.247 | 9.2 | 2L35 |
| 550.187 | 6.167 | 6.13 | 2L10, 2L33 | 592.184 | 9.397 | 9.36 | 2L17, 2L63 |
| 522.242 | 6.183 | 6.13 | 2L47, 2L97 | 522.242 | 9.68 | 9.67 | 2L47, 2L97 |
| 570.238 | 6.223 | 6.19 | 2L85 | 590.193 | 9.707 | 9.67 | 2L39 |
| 551.232 | 6.327 | 6.3 | 2L34 | 658.205 | 9.78 | 9.74 | 2L21 |
| 538.212 | 6.403 | 6.36 | 2L03, 2L53, 2L75 | 580.259 | 10.01 | 9.97 | 2L15 |
| 554.243 | 6.407 | 6.36 | 2L81 | 656.215 | 10.077 | 10.03 | 2L43 |
| 534.217 | 6.433 | 6.39 | 2L95 | 616.184 | 10.13 | 10.09 | 2L19 |
| 573.217 | 6.437 | 6.39 | 2L36 | 578.268 | 10.293 | 10.25 | 2L37, 2L87 |
| 568.248 | 6.467 | 6.39 | 2L59, 2L83 | 614.193 | 10.413 | 10.38 | 2L41 |
| 569.243 | 6.477 | 6.39 | 2L60, 2L84 | 523.237 | 12.043 | 12 | 2L48, 2L98 |

[Table 91]

[Table EJ09]

| Mixture No. | mixEJ09 | | | | | | | |
|---|---|---|---|---|---|---|---|---|
| m/z [M+H]+ | Retention time of MS peak (min) | Retention time of UV peak (min) | Attributed compound No. | | m/z [M+H]+ | Retention time of MS peak (min) | Retention time of UV peak (min) | Attributed compound No. |
| 557.125 | 4.003 | 3.95 | 2K94 | | 600.189 | 6.923 | 6.89 | 2K91 |
| 565.184 | 4.067 | 4.03 | 2K74 | | 591.188 | 6.933 | 6.89 | 2K34 |
| 581.148 | 4.223 | 4.2 | 2K100, 2K28, 2K80 | | 608.204 | 6.947 | 6.89 | 2K59, 2K83 |
| 555.134 | 4.487 | 4.45 | 2K68 | | 608.204 | 7.01 | 6.98 | 2K59, 2K83 |
| 551.168 | 4.523 | 4.49 | 2K72 | | 572.183 | 7.027 | 6.98 | 2K69 |
| 563.193 | 4.603 | 4.6 | 2K48, 2K98 | | 613.173 | 7.053 | 6.98 | 2K36 |
| 579.163 | 4.783 | 4.75 | 2K04, 2K54, 2K76 | | 576.177 | 7.067 | 7.04 | 2K25, 2K49 |
| 595.194 | 4.867 | 4.84 | 2K82 | | 592.184 | 7.08 | 7.04 | 2K11, 2K55 |
| 549.178 | 4.987 | 4.94 | 2K46 | | 598.198 | 7.25 | 7.21 | 2K65 |
| 577.173 | 5.11 | 5.07 | 2K26, 2K50 | | 633.135 | 7.277 | 7.21 | 2K18, 2K64 |
| 609.199 | 5.213 | 5.18 | 2K60, 2K84 | | 618.224 | 7.31 | 7.27 | 2K37, 2K87 |
| 609.199 | 5.237 | 5.18 | 2K60, 2K84 | | 588.152 | 7.337 | 7.27 | 2K07, 2K32 |
| 599.193 | 5.347 | 5.33 | 2K66 | | 621.21 | 7.35 | 7.32 | 2K16 |
| 593.179 | 5.37 | 5.33 | 2K12, 2K56 | | 699.157 | 7.353 | 7.32 | 2K22 |
| 571.104 | 5.56 | 5.52 | 2K02 | | 606.224 | 7.373 | 7.32 | 2K57 |
| 575.168 | 5.577 | 5.52 | 2K96 | | 588.152 | 7.373 | 7.32 | 2K07, 2K32 |
| 579.163 | 5.607 | 5.58 | 2K04, 2K54, 2K76 | | 634.155 | 7.48 | 7.44 | 2K89 |
| 619.22 | 5.63 | 5.58 | 2K38, 2K88 | | 616.17 | 7.557 | 7.52 | 2K61 |
| 607.22 | 5.703 | 5.67 | 2K58 | | 632.14 | 7.71 | 7.67 | 2K17, 2K63 |
| 635.151 | 5.813 | 5.78 | 2K90 | | 631.144 | 8.033 | 8.01 | 2K40 |
| 583.138 | 5.827 | 5.78 | 2K06 | | 619.22 | 8.037 | 8.01 | 2K38, 2K88 |
| 556.129 | 5.977 | 5.94 | 2K93 | | 697.166 | 8.05 | 8.01 | 2K44 |
| 573.178 | 6.01 | 5.97 | 2K70 | | 589.148 | 8.133 | 8.09 | 2K08, 2K09 |
| 550.173 | 6.017 | 5.97 | 2K71 | | 570.109 | 8.35 | 8.32 | 2K01 |
| 617.165 | 6.053 | 5.97 | 2K62 | | 582.143 | 8.507 | 8.47 | 2K05 |
| 568.118 | 6.173 | 6.15 | 2K23, 2K77 | | 578.168 | 8.647 | 8.61 | 2K03, 2K53, 2K75 |
| 593.179 | 6.177 | 6.15 | 2K12, 2K56 | | 579.163 | 8.66 | 8.61 | 2K04, 2K54, 2K76 |
| 580.152 | 6.197 | 6.15 | 2K27, 2K79, 2K99 | | 657.135 | 8.8 | 8.79 | 2K20 |
| 580.152 | 6.217 | 6.15 | 2K27, 2K79, 2K99 | | 587.157 | 8.823 | 8.79 | 2K30, 2K31 |
| 633.135 | 6.23 | 6.15 | 2K18, 2K64 | | 589.148 | 8.823 | 8.79 | 2K08, 2K09 |
| 564.189 | 6.25 | 6.22 | 2K73 | | 568.118 | 9.113 | 9.1 | 2K23, 2K77 |
| 615.163 | 6.273 | 6.22 | 2K14 | | 569.113 | 9.113 | 9.1 | 2K24, 2K78 |
| 554.139 | 6.39 | 6.36 | 2K67 | | 592.184 | 9.117 | 9.1 | 2K11, 2K55 |
| 577.173 | 6.44 | 6.41 | 2K26, 2K50 | | 614.168 | 9.143 | 9.1 | 2K13 |
| 548.183 | 6.443 | 6.41 | 2K45 | | 580.152 | 9.22 | 9.18 | 2K27, 2K79, 2K99 |
| 569.113 | 6.45 | 6.41 | 2K24, 2K78 | | 581.148 | 9.223 | 9.18 | 2K100, 2K28, 2K80 |
| 590.143 | 6.46 | 6.41 | 2K10, 2K33 | | 576.177 | 9.353 | 9.32 | 2K25, 2K49 |
| 610.194 | 6.5 | 6.46 | 2K85 | | 655.144 | 9.473 | 9.44 | 2K42 |
| 611.189 | 6.507 | 6.46 | 2K86 | | 586.162 | 9.52 | 9.44 | 2K29 |
| 567.168 | 6.573 | 6.55 | 2K52 | | 632.14 | 9.62 | 9.59 | 2K17, 2K63 |
| 566.173 | 6.587 | 6.55 | 2K51 | | 590.143 | 9.76 | 9.74 | 2K10, 2K33 |
| 563.193 | 6.663 | 6.66 | 2K48, 2K98 | | 612.177 | 9.777 | 9.74 | 2K35 |
| 578.168 | 6.683 | 6.66 | 2K03, 2K53, 2K75 | | 698.161 | 9.977 | 9.94 | 2K21 |
| 562.198 | 6.69 | 6.66 | 2K47, 2K97 | | 620.215 | 10.19 | 10.17 | 2K15 |
| 594.199 | 6.693 | 6.66 | 2K81 | | 630.149 | 10.213 | 10.17 | 2K39 |
| 578.168 | 6.707 | 6.66 | 2K03, 2K53, 2K75 | | 656.14 | 10.3 | 10.27 | 2K19 |
| 562.198 | 6.717 | 6.66 | 2K47, 2K97 | | 696.171 | 10.53 | 10.49 | 2K43 |
| 581.148 | 6.733 | 6.7 | 2K100, 2K28, 2K80 | | 618.224 | 10.76 | 10.72 | 2K37, 2K87 |
| 574.173 | 6.747 | 6.7 | 2K95 | | 654.149 | 10.847 | 10.82 | 2K41 |
| 601.184 | 6.91 | 6.89 | 2K92 | | 587.157 | 11.25 | 10.82 | 2K30, 2K31 |

[Table 92]

[Table EJ10]

| Mixture No. | mixEJ10 | | | | | | | |
|---|---|---|---|---|---|---|---|---|
| m/z [M+H]+ | Retention time of MS peak (min) | Retention time of UV peak (min) | Attributed compound No. | | m/z [M+H]+ | Retention time of MS peak (min) | Retention time of UV peak (min) | Attributed compound No. |
| 617.29 | 6.513 | 6.47 | 2A74 | | 621.22 | 8.75 | 8.68 | 2A24, 2A78 |
| 609.231 | 6.557 | 6.52 | 2A94 | | 660.31 | 8.787 | 8.76 | 2A59, 2A83 |
| 607.241 | 6.683 | 6.65 | 2A68 | | 658.331 | 8.853 | 8.82 | 2A57 |
| 615.3 | 6.7 | 6.65 | 2A48, 2A98 | | 650.305 | 8.893 | 8.86 | 2A65 |
| 621.22 | 6.843 | 6.8 | 2A24, 2A78 | | 652.295 | 8.967 | 8.94 | 2A91 |
| 632.259 | 6.983 | 6.96 | 2A27, 2A79, 2A99 | | 667.27 | 8.997 | 8.94 | 2A14 |
| 631.27 | 6.987 | 6.96 | 2A04, 2A54, 2A76 | | 643.295 | 9.073 | 9.03 | 2A34 |
| 603.275 | 6.997 | 6.96 | 2A72 | | 633.254 | 9.187 | 9.16 | 2A100, 2A28, 2A80 |
| 601.284 | 7.063 | 7.03 | 2A46 | | 668.276 | 9.197 | 9.16 | 2A61 |
| 647.301 | 7.103 | 7.07 | 2A82 | | 669.272 | 9.197 | 9.16 | 2A62 |
| 661.305 | 7.12 | 7.07 | 2A60, 2A84 | | 684.246 | 9.327 | 9.29 | 2A17, 2A63 |
| 651.3 | 7.34 | 7.29 | 2A66 | | 665.279 | 9.343 | 9.29 | 2A36 |
| 661.305 | 7.367 | 7.33 | 2A60, 2A84 | | 642.249 | 9.357 | 9.29 | 2A10, 2A33 |
| 659.326 | 7.513 | 7.48 | 2A58 | | 670.331 | 9.387 | 9.35 | 2A37, 2A87 |
| 671.326 | 7.993 | 7.95 | 2A38, 2A88 | | 686.262 | 9.517 | 9.48 | 2A89 |
| 685.241 | 8.087 | 8.07 | 2A18, 2A64 | | 640.259 | 9.717 | 9.64 | 2A07, 2A32 |
| 633.254 | 8.097 | 8.07 | 2A100, 2A28, 2A80 | | 751.263 | 9.753 | 9.71 | 2A22 |
| 602.28 | 8.097 | 8.07 | 2A71 | | 673.317 | 9.853 | 9.82 | 2A16 |
| 632.259 | 8.103 | 8.07 | 2A27, 2A79, 2A99 | | 685.241 | 9.977 | 9.95 | 2A18, 2A64 |
| 633.254 | 8.117 | 8.07 | 2A100, 2A28, 2A80 | | 749.273 | 10.073 | 10.05 | 2A44 |
| 687.257 | 8.12 | 8.07 | 2A90 | | 671.326 | 10.167 | 10.13 | 2A38, 2A88 |
| 600.289 | 8.173 | 8.15 | 2A45 | | 683.251 | 10.31 | 10.28 | 2A40 |
| 616.295 | 8.183 | 8.15 | 2A73 | | 641.254 | 10.717 | 10.68 | 2A08, 2A09 |
| 608.236 | 8.203 | 8.15 | 2A93 | | 641.254 | 10.743 | 10.68 | 2A08, 2A09 |
| 606.245 | 8.21 | 8.15 | 2A67 | | 639.264 | 11.013 | 10.98 | 2A30, 2A31 |
| 620.225 | 8.257 | 8.23 | 2A23, 2A77 | | 638.268 | 11.033 | 10.98 | 2A29 |
| 653.29 | 8.257 | 8.23 | 2A92 | | 622.215 | 11.06 | 10.98 | 2A01 |
| 630.274 | 8.287 | 8.23 | 2A03, 2A53, 2A75 | | 635.245 | 11.143 | 11.11 | 2A06 |
| 663.296 | 8.31 | 8.28 | 2A86 | | 634.249 | 11.153 | 11.11 | 2A05 |
| 618.28 | 8.313 | 8.28 | 2A51 | | 709.241 | 11.303 | 11.27 | 2A20 |
| 619.275 | 8.313 | 8.28 | 2A52 | | 630.274 | 11.313 | 11.27 | 2A03, 2A53, 2A75 |
| 662.301 | 8.317 | 8.28 | 2A85 | | 630.274 | 11.34 | 11.27 | 2A03, 2A53, 2A75 |
| 614.305 | 8.32 | 8.28 | 2A47, 2A97 | | 620.225 | 11.377 | 11.33 | 2A23, 2A77 |
| 615.3 | 8.323 | 8.28 | 2A48, 2A98 | | 632.259 | 11.44 | 11.4 | 2A27, 2A79, 2A99 |
| 614.305 | 8.343 | 8.28 | 2A47, 2A97 | | 640.259 | 11.46 | 11.4 | 2A07, 2A32 |
| 623.21 | 8.36 | 8.33 | 2A02 | | 707.251 | 11.597 | 11.57 | 2A42 |
| 631.27 | 8.37 | 8.33 | 2A04, 2A54, 2A76 | | 628.284 | 11.607 | 11.57 | 2A25, 2A49 |
| 625.284 | 8.373 | 8.33 | 2A70 | | 644.29 | 11.703 | 11.68 | 2A11, 2A55 |
| 660.31 | 8.52 | 8.5 | 2A59, 2A83 | | 666.274 | 11.74 | 11.68 | 2A13 |
| 646.306 | 8.537 | 8.5 | 2A81 | | 639.264 | 11.947 | 11.92 | 2A30, 2A31 |
| 644.29 | 8.627 | 8.59 | 2A11, 2A55 | | 642.249 | 11.953 | 11.92 | 2A10, 2A33 |
| 624.289 | 8.637 | 8.59 | 2A69 | | 664.284 | 11.987 | 11.95 | 2A35 |
| 626.28 | 8.687 | 8.68 | 2A95 | | 684.246 | 12.05 | 12.02 | 2A17, 2A63 |
| 631.27 | 8.707 | 8.68 | 2A04, 2A54, 2A76 | | 750.268 | 12.253 | 12.23 | 2A21 |
| 627.275 | 8.71 | 8.68 | 2A96 | | 682.256 | 12.297 | 12.26 | 2A39 |
| 628.284 | 8.71 | 8.68 | 2A25, 2A49 | | 748.278 | 12.513 | 12.47 | 2A43 |
| 629.279 | 8.717 | 8.68 | 2A26, 2A50 | | 708.246 | 12.59 | 12.55 | 2A19 |
| 645.285 | 8.727 | 8.68 | 2A12, 2A56 | | 672.321 | 12.663 | 12.62 | 2A15 |
| 629.279 | 8.733 | 8.68 | 2A26, 2A50 | | 706.256 | 12.82 | 12.79 | 2A41 |
| 645.285 | 8.743 | 8.68 | 2A12, 2A56 | | 670.331 | 12.883 | 12.85 | 2A37, 2A87 |

[Table 93]

[Table EJ11]

| Mixture No. | mixEJ11 | | | | | | |
|---|---|---|---|---|---|---|---|
| m/z [M+H]+ | Retention time of MS peak (min) | Retention time of UV peak (min) | Attributed compound No. | m/z [M+H]+ | Retention time of MS peak (min) | Retention time of UV peak (min) | Attributed compound No. |
| 763.2 | 5.617 | 5.59 | 2Q30, 2Q31 | 745.157 | 8.743 | 8.71 | 2Q24, 2Q78 |
| 741.227 | 6.79 | 6.75 | 2Q74 | 752.221 | 8.75 | 8.71 | 2Q25, 2Q49 |
| 757.191 | 6.883 | 6.83 | 2Q100, 2Q28, 2Q80 | 759.181 | 8.763 | 8.71 | 2Q06 |
| 731.177 | 6.943 | 6.89 | 2Q68 | 785.242 | 8.803 | 8.76 | 2Q60, 2Q84 |
| 739.237 | 6.953 | 6.89 | 2Q48, 2Q98 | 784.247 | 8.81 | 8.76 | 2Q59, 2Q83 |
| 739.237 | 6.97 | 6.89 | 2Q48, 2Q98 | 791.206 | 8.873 | 8.87 | 2Q14 |
| 755.206 | 7.063 | 7.05 | 2Q04, 2Q54, 2Q76 | 782.268 | 8.897 | 8.87 | 2Q57 |
| 745.157 | 7.087 | 7.05 | 2Q24, 2Q78 | 774.241 | 8.9 | 8.87 | 2Q65 |
| 787.233 | 7.09 | 7.05 | 2Q86 | 776.232 | 8.913 | 8.87 | 2Q91 |
| 755.206 | 7.167 | 7.13 | 2Q04, 2Q54, 2Q76 | 767.232 | 9.043 | 9 | 2Q34 |
| 756.196 | 7.17 | 7.13 | 2Q27, 2Q79, 2Q99 | 757.191 | 9.097 | 9.07 | 2Q100, 2Q28, 2Q8 |
| 744.161 | 7.203 | 7.13 | 2Q23, 2Q77 | 792.213 | 9.163 | 9.15 | 2Q61 |
| 743.212 | 7.22 | 7.19 | 2Q52 | 793.208 | 9.177 | 9.15 | 2Q62 |
| 727.212 | 7.223 | 7.19 | 2Q72 | 789.216 | 9.197 | 9.15 | 2Q36 |
| 753.216 | 7.287 | 7.26 | 2Q26, 2Q50 | 808.183 | 9.273 | 9.24 | 2Q17, 2Q63 |
| 785.242 | 7.31 | 7.26 | 2Q60, 2Q84 | 794.268 | 9.287 | 9.24 | 2Q37, 2Q87 |
| 725.221 | 7.32 | 7.26 | 2Q46 | 766.186 | 9.287 | 9.24 | 2Q10, 2Q33 |
| 777.227 | 7.41 | 7.37 | 2Q92 | 811.194 | 9.363 | 9.35 | 2Q90 |
| 769.222 | 7.447 | 7.41 | 2Q12, 2Q56 | 810.199 | 9.387 | 9.35 | 2Q89 |
| 775.237 | 7.503 | 7.48 | 2Q66 | 875.2 | 9.543 | 9.51 | 2Q22 |
| 783.263 | 7.65 | 7.62 | 2Q58 | 764.196 | 9.59 | 9.51 | 2Q07, 2Q32 |
| 751.212 | 7.907 | 7.87 | 2Q96 | 797.253 | 9.597 | 9.57 | 2Q16 |
| 749.221 | 7.98 | 7.94 | 2Q70 | 765.191 | 9.623 | 9.57 | 2Q08, 2Q09 |
| 795.263 | 8.057 | 8.02 | 2Q38, 2Q88 | 764.196 | 9.627 | 9.57 | 2Q07, 2Q32 |
| 809.178 | 8.167 | 8.13 | 2Q18, 2Q64 | 809.178 | 9.763 | 9.72 | 2Q18, 2Q64 |
| 726.216 | 8.203 | 8.17 | 2Q71 | 873.209 | 9.86 | 9.83 | 2Q44 |
| 756.196 | 8.203 | 8.17 | 2Q27, 2Q79, 2Q99 | 874.205 | 9.87 | 9.83 | 2Q21 |
| 757.191 | 8.21 | 8.17 | 2Q100, 2Q28, 2Q80 | 795.263 | 9.91 | 9.87 | 2Q38, 2Q88 |
| 732.173 | 8.26 | 8.17 | 2Q93 | 807.188 | 10.08 | 10.04 | 2Q40 |
| 740.232 | 8.27 | 8.25 | 2Q73 | 765.191 | 10.44 | 10.4 | 2Q08, 2Q09 |
| 724.226 | 8.287 | 8.25 | 2Q45 | 872.214 | 10.523 | 10.48 | 2Q43 |
| 730.182 | 8.293 | 8.25 | 2Q67 | 763.2 | 10.747 | 10.7 | 2Q30, 2Q31 |
| 744.161 | 8.33 | 8.29 | 2Q23, 2Q77 | 758.186 | 10.79 | 10.73 | 2Q05 |
| 746.152 | 8.337 | 8.29 | 2Q01 | 754.211 | 10.873 | 10.87 | 2Q03, 2Q53, 2Q75 |
| 754.211 | 8.363 | 8.29 | 2Q03, 2Q53, 2Q75 | 833.178 | 10.907 | 10.87 | 2Q20 |
| 747.147 | 8.367 | 8.34 | 2Q02 | 756.196 | 11.063 | 11.02 | 2Q27, 2Q79, 2Q99 |
| 786.237 | 8.383 | 8.34 | 2Q85 | 752.221 | 11.173 | 11.19 | 2Q25, 2Q49 |
| 754.211 | 8.383 | 8.34 | 2Q03, 2Q53, 2Q75 | 831.188 | 11.227 | 11.19 | 2Q42 |
| 742.216 | 8.407 | 8.34 | 2Q51 | 790.211 | 11.243 | 11.19 | 2Q13 |
| 738.241 | 8.423 | 8.34 | 2Q47, 2Q97 | 768.227 | 11.29 | 11.24 | 2Q11, 2Q55 |
| 738.241 | 8.44 | 8.34 | 2Q47, 2Q97 | 762.205 | 11.343 | 11.29 | 2Q29 |
| 771.238 | 8.563 | 8.54 | 2Q82 | 788.221 | 11.497 | 11.47 | 2Q35 |
| 770.243 | 8.577 | 8.54 | 2Q81 | 766.186 | 11.563 | 11.53 | 2Q10, 2Q33 |
| 784.247 | 8.583 | 8.54 | 2Q59, 2Q83 | 808.183 | 11.57 | 11.53 | 2Q17, 2Q63 |
| 768.227 | 8.673 | 8.66 | 2Q11, 2Q55 | 806.192 | 11.837 | 11.8 | 2Q39 |
| 755.206 | 8.683 | 8.66 | 2Q04, 2Q54, 2Q76 | 832.183 | 12.057 | 12.01 | 2Q19 |
| 748.226 | 8.687 | 8.66 | 2Q69 | 796.258 | 12.067 | 12.01 | 2Q15 |
| 750.216 | 8.693 | 8.66 | 2Q95 | 794.268 | 12.327 | 12.29 | 2Q37, 2Q87 |
| 753.216 | 8.697 | 8.66 | 2Q26, 2Q50 | 830.192 | 12.347 | 12.29 | 2Q41 |
| 769.222 | 8.707 | 8.66 | 2Q12, 2Q56 | 733.168 | 14.537 | 14.6 | 2Q94 |

[Table 94]

[Table EJ12]

| Mixture No. | mixEJ12 | | | | | | |
|---|---|---|---|---|---|---|---|
| m/z [M+H]+ | Retention time of MS peak (min) | Retention time of UV peak (min) | Attributed compound No. | m/z [M+H]+ | Retention time of MS peak (min) | Retention time of UV peak (min) | Attributed compound No. |
| 788.193 | 3.34 | 3.34 | 2R08, 2R09 | 807.249 | 9.27 | 9.23 | 2R59, 2R83 |
| 810.235 | 6.247 | 6.21 | 2R86 | 805.27 | 9.293 | 9.23 | 2R57 |
| 816.211 | 6.3 | 6.25 | 2R62 | 769.154 | 9.31 | 9.23 | 2R01 |
| 786.203 | 7.023 | 6.99 | 2R30, 2R31 | 768.159 | 9.33 | 9.29 | 2R24, 2R78 |
| 767.164 | 7.127 | 7.09 | 2R23, 2R77 | 792.224 | 9.33 | 9.29 | 2R12, 2R56 |
| 763.234 | 7.24 | 7.2 | 2R73 | 798.239 | 9.337 | 9.29 | 2R66 |
| 764.23 | 7.347 | 7.32 | 2R74 | 797.244 | 9.34 | 9.29 | 2R65 |
| 756.17 | 7.43 | 7.39 | 2R94 | 799.234 | 9.423 | 9.39 | 2R91 |
| 780.193 | 7.443 | 7.39 | 2R100, 2R28, 2R80 | 782.184 | 9.427 | 9.39 | 2R06 |
| 762.239 | 7.493 | 7.44 | 2R48, 2R98 | 814.209 | 9.527 | 9.48 | 2R14 |
| 754.18 | 7.497 | 7.44 | 2R68 | 790.234 | 9.593 | 9.57 | 2R34 |
| 778.209 | 7.74 | 7.71 | 2R04, 2R54, 2R76 | 815.215 | 9.607 | 9.57 | 2R61 |
| 766.214 | 7.763 | 7.71 | 2R52 | 780.193 | 9.703 | 9.67 | 2R100, 2R28, 2R80 |
| 808.244 | 7.803 | 7.78 | 2R60, 2R84 | 831.185 | 9.703 | 9.67 | 2R17, 2R63 |
| 776.218 | 7.807 | 7.78 | 2R26, 2R50 | 812.218 | 9.77 | 9.76 | 2R36 |
| 750.214 | 7.817 | 7.78 | 2R72 | 817.27 | 9.793 | 9.76 | 2R37, 2R87 |
| 794.24 | 7.827 | 7.78 | 2R82 | 833.201 | 9.903 | 9.86 | 2R89 |
| 800.23 | 7.997 | 7.96 | 2R92 | 898.202 | 10.133 | 10.1 | 2R22 |
| 806.265 | 8.137 | 8.1 | 2R58 | 787.198 | 10.197 | 10.17 | 2R07, 2R32 |
| 772.223 | 8.463 | 8.41 | 2R70 | 820.256 | 10.207 | 10.17 | 2R16 |
| 834.196 | 8.61 | 8.58 | 2R90 | 832.181 | 10.383 | 10.35 | 2R18, 2R64 |
| 818.265 | 8.613 | 8.58 | 2R38, 2R88 | 896.212 | 10.39 | 10.35 | 2R44 |
| 779.198 | 8.677 | 8.58 | 2R27, 2R79, 2R99 | 818.265 | 10.443 | 10.41 | 2R38, 2R88 |
| 779.198 | 8.703 | 8.7 | 2R27, 2R79, 2R99 | 830.19 | 10.637 | 10.6 | 2R40 |
| 749.219 | 8.717 | 8.7 | 2R71 | 895.217 | 10.913 | 10.78 | 2R43 |
| 747.228 | 8.74 | 8.7 | 2R45 | 789.188 | 11.027 | 10.99 | 2R10, 2R33 |
| 748.223 | 8.747 | 8.7 | 2R46 | 788.193 | 11.037 | 10.99 | 2R08, 2R09 |
| 753.185 | 8.763 | 8.7 | 2R67 | 786.203 | 11.257 | 11.22 | 2R30, 2R31 |
| 755.175 | 8.793 | 8.7 | 2R93 | 787.198 | 11.257 | 11.22 | 2R07, 2R32 |
| 777.214 | 8.803 | 8.7 | 2R03, 2R53, 2R75 | 781.188 | 11.36 | 11.32 | 2R05 |
| 778.209 | 8.823 | 8.81 | 2R04, 2R54, 2R76 | 856.181 | 11.487 | 11.45 | 2R20 |
| 761.244 | 8.83 | 8.81 | 2R47, 2R97 | 777.214 | 11.487 | 11.45 | 2R03, 2R53, 2R75 |
| 809.24 | 8.84 | 8.81 | 2R85 | 767.164 | 11.533 | 11.45 | 2R23, 2R77 |
| 761.244 | 8.847 | 8.81 | 2R47, 2R97 | 780.193 | 11.557 | 11.54 | 2R100, 2R28, 2R80 |
| 765.219 | 8.85 | 8.81 | 2R51 | 779.198 | 11.57 | 11.54 | 2R27, 2R79, 2R99 |
| 768.159 | 8.85 | 8.81 | 2R24, 2R78 | 775.223 | 11.687 | 11.61 | 2R25, 2R49 |
| 762.239 | 8.86 | 8.81 | 2R48, 2R98 | 854.19 | 11.72 | 11.67 | 2R42 |
| 807.249 | 8.98 | 8.97 | 2R59, 2R83 | 813.214 | 11.82 | 11.77 | 2R13 |
| 778.209 | 9.003 | 8.97 | 2R04, 2R54, 2R76 | 785.207 | 11.86 | 11.83 | 2R29 |
| 770.15 | 9.033 | 8.97 | 2R02 | 791.229 | 11.867 | 11.83 | 2R11, 2R55 |
| 793.245 | 9.05 | 9.01 | 2R81 | 811.223 | 11.983 | 11.97 | 2R35 |
| 775.223 | 9.057 | 9.01 | 2R25, 2R49 | 789.188 | 12.067 | 12.03 | 2R10, 2R33 |
| 792.224 | 9.09 | 9.01 | 2R12, 2R56 | 832.181 | 12.117 | 12.09 | 2R18, 2R64 |
| 791.229 | 9.1 | 9.07 | 2R11, 2R55 | 831.185 | 12.12 | 12.09 | 2R17, 2R63 |
| 771.228 | 9.107 | 9.07 | 2R69 | 897.207 | 12.297 | 12.27 | 2R21 |
| 774.214 | 9.183 | 9.15 | 2R96 | 829.195 | 12.317 | 12.27 | 2R39 |
| 773.219 | 9.19 | 9.15 | 2R95 | 855.185 | 12.57 | 12.54 | 2R19 |
| 777.214 | 9.193 | 9.15 | 2R03, 2R53, 2R75 | 819.26 | 12.607 | 12.54 | 2R15 |
| 808.244 | 9.263 | 9.23 | 2R60, 2R84 | 853.195 | 12.76 | 12.73 | 2R41 |
| 776.218 | 9.267 | 9.23 | 2R26, 2R50 | 817.27 | 12.79 | 12.73 | 2R37, 2R87 |

Example 6-5: Post-treatment after cleavage from solid phase and selective ethylation reaction of arenol moiety

After performing an operation of removing TFA on the mixture cleaved from the resin, selective ethylation was performed on the arenol moiety of the obtained compound to convert the functional group. It was performed by the method shown below using the mixtures mixEJ01 to mixEJ12 shown in [Table EIJ01] to [Table EIJ12] obtained in Example 6-4-1 and Example 6-4-2. As an example, an experimental operation is shown in which, using mixEJ-11, TFA was removed, ethylation was then performed, and mixAI-B2Q-01 was obtained.

Example 6-5-1: Synthesis of mixture mixAI-B2Q-01

[Formula 115]

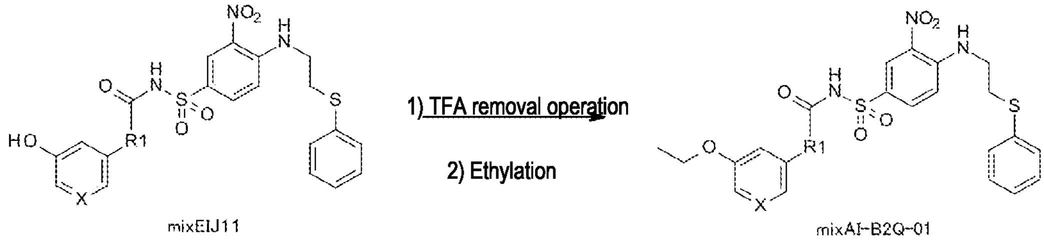


mixEIJ11 mixAI-B2Q-01

DMI (1 mL) and fluorobenzene (as an internal standard, 30 μL was added) were added to mixEJ11 (35.3 μmol assumed) and completely dissolved by vortex stirring. This solution was transferred to a syringe column packed with solid phase-supported morpholine (Merck, cat. No. 493813, morpholine, polymer-supported 200 to 400 mesh, extent of labeling: 2.75 to 3.25 mmol/g loading, 1% crosslinking, 300 mg), washed twice with MeCN (250 μL), and then allowed to stand for 10 minutes. Under pressure, the filtrate was recovered from the bottom of the column in a test tube. The solid phase-supported morpholine was washed three times with MeCN (1 mL), pressure filtered, and all filtrates were collected together. For the collected solution, the solvent was distilled off under reduced pressure in Genevac (40°C, after reducing pressure at 50 to 200 mbar until it was detected that the low boiling point solvent had been completely distilled off, 40°C, < 0.5 mbar for 16 hours). DMI (500 μL) was added to the residue, and the mixture was dissolved by stirring, then transferred to a glass vial, and washed twice with DMI (250 μL). For the obtained solution, the solvent was distilled off under reduced pressure in Genevac (40°C, pressure reduced at 50 to 200 mbar until it was detected that the low boiling point solvent had been completely distilled off) to obtain a concentrate to be used for the subsequent ethylation.

Under a nitrogen atmosphere, to the glass vial in which the obtained concentrate (31.9 μmol assumed) was placed were added DMI (550 μL), BTPP (151 μL), and $Et_3PO_4$ (151 μL), and the mixture was stirred at 80°C for 30 hours. After cooling to room temperature, the reaction solution was adsorbed on a carboxylic acid-supported silica gel column (ISOLUTE (R) CBA SPE column manufactured by Biotage AB, 1 g/6 mL, 0.7 meq/g, product number: 520-0100-C) and allowed to stand for 10 minutes. MeCN (1 mL) was added, pressurized, and the filtrate was recovered in a test tube. The carboxylic acid-supported silica gel was washed and pressure filtered twice with MeCN (1 mL) and filtrates were collected. After adding DMSO (1 mL), the solvent was distilled off under reduced pressure in Genevac (40°C, after reducing pressure at 50 to 200 mbar until it was detected that the low boiling point solvent had been completely distilled off, 40°C, < 0.5 mbar for 16 hours), and the title mixture mixAI-B2Q-01 (25.0 mg) shown in [Table AI-03-B2Q-01] was obtained as an oily substance.

Example 6-5-2: Performing TFA removal and ethylation to other 11 mixtures

The same operations as in Example 6-5-1 were performed for the mixtures mixEJ01 to mixEJ10 and mixEJ12 shown in [Table EJ01] to [Table EJ10] and [Table EJ12], and the mixtures shown in [Table AI-03-B4J-01], [Table AI-03-B1M-01], [Table AI-03-B1K-01], [Table AI-03-B1O-01], [Table AI-03-B1A-01], [Table AI-03-B3A-01], [Table AI-03-B2I-01], [Table AI-03-B2L-01], [Table AI-03-B2N-01], [Table AI-03-B2A-01], and [Table AI-03-B2R-01] were obtained.

The raw material mixture No. used, the target product mixture No. obtained from each, the compound No. that may be included in the target product mixture, and the weight of the obtained mixture are shown in [Table AI-03-01].

[Table 95]

[Table AI-03-01]

Table AI-03-01

| Raw material mixture No. | Target product mixture No. | Compound No. that may be included in target product mixture | Weight of obtained mixture (mg) |
|---|---|---|---|
| mixEJ01 | mixAI-B4J-01 | B4J001-01~B4J100-01 | 21.9 |
| mixEJ02 | mixAI-B1M-01 | B1M001-01~B1M100-01 | 22.2 |
| mixEJ03 | mixAI-B1K-01 | B1K001-01~B1K100-01 | 24.3 |
| mixEJ04 | mixAI-B1O-01 | B1O001-01~B1O100-01 | 22.3 |
| mixEJ05 | mixAI-B1A-01 | B1A001-01~B1A100-01 | 18.3 |
| mixEJ06 | mixAI-B3A-01 | B3A001-01~B3A100-01 | 27.5 |
| mixEJ07 | mixAI-B2I-01 | B2I001-01~B2I100-01 | 30.9 |
| mixEJ08 | mixAI-B2L-01 | B2L001-01~B2L100-01 | 28.1 |
| mixEJ09 | mixAI-B2N-01 | B2N001-01~B2N100-01 | 25.6 |
| mixEJ10 | mixAI-B2A-01 | B2A001-01~B2A100-01 | 23.3 |
| mixEJ11 | mixAI-B2Q-01 | B2Q001-01~B2Q100-01 | 25.0 |
| mixEJ12 | mixAI-B2R-01 | B2R001-01~B2R100-01 | 28.2 |

[Table 96]

[Table AI-03-B2Q-01]

| Mixture No. | mixAI-B2Q-01 |
|---|---|
| Compound No. that may be included | Compound name |
| B2Q001-01 | 6-[4-[4-(3-Ethoxyphenyl)thiophene-2-carbonyl]piperazin-1-yl]-N-[3-nitro-4-(2-phenylsulfanylethylamino)phenyl]sulfonylpyridazine-3-carboxamide |
| B2Q002-01 | 6-[4-[4-(5-Ethoxypyridin-3-yl)thiophene-2-carbonyl]piperazin-1-yl]-N-[3-nitro-4-(2-phenylsulfanylethylamino)phenyl]sulfonylpyridazine-3-carboxamide |
| B2Q003-01 | 6-[4-[4-(3-Ethoxyphenyl)-3-methylbenzoyl]piperazin-1-yl]-N-[3-nitro-4-(2-phenylsulfanylethylamino)phenyl]sulfonylpyridazine-3-carboxamide |
| B2Q004-01 | 6-[4-[4-(5-Ethoxypyridin-3-yl)-3-methylbenzoyl]piperazin-1-yl]-N-[3-nitro-4-(2-phenylsulfanylethylamino)phenyl]sulfonylpyridazine-3-carboxamide |
| B2Q005-01 | 6-[4-[4-(3-Ethoxyphenyl)-3-fluorobenzoyl]piperazin-1-yl]-N-[3-nitro-4-(2-phenylsulfanylethylamino)phenyl]sulfonylpyridazine-3-carboxamide |
| B2Q006-01 | 6-[4-[4-(5-Ethoxypyridin-3-yl)-3-fluorobenzoyl]piperazin-1-yl]-N-[3-nitro-4-(2-phenylsulfanylethylamino)phenyl]sulfonylpyridazine-3-carboxamide |
| B2Q007-01 | 6-[4-[2-[2-(3-Ethoxyphenyl)ethynyl]benzoyl]piperazin-1-yl]-N-[3-nitro-4-(2-phenylsulfanylethylamino)phenyl]sulfonylpyridazine-3-carboxamide |
| B2Q008-01 | 6-[4-[2-[2-(5-Ethoxypyridin-3-yl)ethynyl]benzoyl]piperazin-1-yl]-N-[3-nitro-4-(2-phenylsulfanylethylamino)phenyl]sulfonylpyridazine-3-carboxamide |
| B2Q009-01 | 6-[4-[5-[2-(3-Ethoxyphenyl)ethynyl]pyridine-3-carbonyl]piperazin-1-yl]-N-[3-nitro-4-(2-phenylsulfanylethylamino)phenyl]sulfonylpyridazine-3-carboxamide |
| B2Q010-01 | 6-[4-[5-[2-(5-Ethoxypyridin-3-yl)ethynyl]pyridine-3-carbonyl]piperazin-1-yl]-N-[3-nitro-4-(2-phenylsulfanylethylamino)phenyl]sulfonylpyridazine-3-carboxamide |
| B2Q011-01 | 6-[4-[3-[2-(3-Ethoxyphenyl)phenyl]propanoyl]piperazin-1-yl]-N-[3-nitro-4-(2-phenylsulfanylethylamino)phenyl]sulfonylpyridazine-3-carboxamide |
| B2Q012-01 | 6-[4-[3-[2-(5-Ethoxypyridin-3-yl)phenyl]propanoyl]piperazin-1-yl]-N-[3-nitro-4-(2-phenylsulfanylethylamino)phenyl]sulfonylpyridazine-3-carboxamide |
| B2Q013-01 | 6-[4-[4-(3-Ethoxyphenyl)naphthalene-1-carbonyl]piperazin-1-yl]-N-[3-nitro-4-(2-phenylsulfanylethylamino)phenyl]sulfonylpyridazine-3-carboxamide |

(continued)

| Mixture No. | mixAI-B2Q-01 |
|---|---|
| Compound No. that may be included | Compound name |
| B2Q014-01 | 6-[4-[4-(5-Ethoxypyridin-3-yl)naphthalene-1-carbonyl]piperazin-1-yl]-N-[3-nitro-4-(2-phenylsulfanylethylamino)phenyl]sulfonylpyridazine-3-carboxamide |
| B2Q015-01 | 6-[4-[3-tert-Butyl-5-(3-ethoxyphenyl)benzoyl]piperazin-1-yl]-N-[3-nitro-4-(2-phenylsulfanylethylamino)phenyl]sulfonylpyridazine-3-carboxamide |
| B2Q016-01 | 6-[4-[3-tert-Butyl-5-(5-ethoxypyridin-3-yl)benzoyl]piperazin-1-yl]-N-[3-nitro-4-(2-phenylsulfanylethylamino)phenyl]sulfonylpyridazine-3-carboxamide |
| B2Q017-01 | 6-[4-[3-(3-Ethoxyphenyl)-5-(trifluoromethyl)benzoyl]piperazin-1-yl]-N-[3-nitro-4-(2-phenylsulfanylethylamino)phenyl]sulfonylpyridazine-3-carboxamide |
| B2Q018-01 | 6-[4-[3-(5-Ethoxypyridin-3-yl)-5-(trifluoromethyl)benzoyl]piperazin-1-yl]-N-[3-nitro-4-(2-phenylsulfanylethylamino)phenyl]sulfonylpyridazine-3-carboxamide |
| B2Q019-01 | 6-[4-[3-[2-(3-Ethoxyphenyl)ethynyl]-5-(trifluoromethyl)benzoyl]piperazin-1-yl]-N-[3-nitro-4-(2-phenylsulfanylethylamino)phenyl]sulfonylpyridazine-3-carboxamide |
| B2Q020-01 | 6-[4-[3-[2-(5-Ethoxypyridin-3-yl)ethynyl]-5-(trifluoromethyl)benzoyl]piperazin-1-yl]-N-[3-nitro-4-(2-phenylsulfanylethylamino)phenyl]sulfonylpyridazine-3-carboxamide |
| B2Q021-01 | 6-[4-[3-[4-(3-Ethoxyphenyl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| B2Q022-01 | 6-[4-[3-[4-(5-Ethoxypyridin-3-yl)pyrazol-1-yl]-5-(trifluoromethyl)benzoyl]piperazin-1-yl]-N-[3-nitro-4-(2-phenylsulfanylethylamino)phenyl]sulfonylpyridazine-3-carboxamide |
| B2Q023-01 | 4-[4-[4-(3-Ethoxyphenyl)thiophene-2-carbonyl]piperazin-1-yl]-N-[3-nitro-4-(2-phenylsulfanylethylamino)phenyl]sulfonylbenzamide |
| B2Q024-01 | 4-[4-[4-(5-Ethoxypyridin-3-yl)thiophene-2-carbonyl]piperazin-1-yl]-N-[3-nitro-4-(2-phenylsulfanylethylamino)phenyl]sulfonylbenzamide |
| B2Q025-01 | 4-[4-[4-(3-Ethoxyphenyl)-3-methylbenzoyl]piperazin-1-yl]-N-[3-nitro-4-(2-phenylsulfanylethylamino)phenyl]sulfonylbenzamide |
| B2Q026-01 | 4-[4-[4-(5-Ethoxypyridin-3-yl)-3-methylbenzoyl]piperazin-1-yl]-N-[3-nitro-4-(2-phenylsulfanylethylamino)phenyl]sulfonylbenzamide |
| B2Q027-01 | 4-[4-[4-(3-Ethoxyphenyl)-3-fluorobenzoyl]piperazin-1-yl]-N-[3-nitro-4-(2-phenylsulfanylethylamino)phenyl]sulfonylbenzamide |
| B2Q028-01 | -[4-[4-(5-Etoxypyridin-3-yl)-3-fluorobenzoyl]piperazin-1-yl]-N-[3-nitro-4-(2-phenylsulfanylethylamino)phenyl]sulfonylbenzamide |
| B2Q029-01 | -[4-[2-[2-(3-Ethoxyphenyl)ethynyl]benzoyl]piperazin-1-yl]-N-[3-nitro-4-(2-phenylsulfanylethylamino)phenyl]sulfonylbenzamide |
| B2Q030-01 | -[4-[2-[2-(5-Ethoxypyridin-3-yl)ethynyl]benzoyl]piperazin-1-yl]-N-[3-nitro-4-(2-phenylsulfanylethylamino)phenyl]sulfonylbenzamide |
| B2Q031-01 | 4-[4-[5-[2-(3-Ethoxyphenyl)ethynyl]pyridine-3-carbonyl]piperazin-1-yl]-N-[3-nitro-4-(2-phenylsulfanylethylamino)phenyllsulfonylbenzamide |
| B2Q032-01 | 4-[4-[5-[2-(5-Ethoxypyridin-3-yl)ethynyl]pyridine-3-carbonyl]piperazin-1-yl]-N-[3-nitro-4-(2-phenylsulfanylethylamino)phenyl]sulfonylbenzamide |
| B2Q033-01 | 4-[4-[3-[2-(3-Ethoxyphenyl)phenyl]propanoyl]piperazin-1-yl]-N-[3-nitro-4-(2-phenylsulfanylethylamino)phenyl]sulfonylbenzamide |
| B2Q034-01 | 4-[4-[3-[2-(5-Ethoxypyridin-3-yl)phenyl]propanoyl]piperazin-1-yl]-N-[3-nitro-4-(2-phenylsulfanylethylamino)phenyl]sulfonylbenzamide |

(continued)

| Mixture No. | mixAI-B2Q-01 |
|---|---|
| Compound No. that may be included | Compound name |
| B2Q035-01 | 4-[4-[4-(3-Ethoxyphenyl)naphthalene-1-carbonyl]piperazin-1-yl]-N-[3-nitro-4-(2-phenylsulfanylethylamino)phenyl]sulfonylbenzamide |
| B2Q036-01 | 4-[4-[4-(5-Ethoxypyridin-3-yl)naphthalene-1-carbonyl]piperazin-1-yl]-N-[3-nitro-4-(2-phenylsulfanylethylamino)phenyl]sulfonylbenzamide |
| B2Q037-01 | 4-[4-[3-tert-Butyl-5-(3-ethoxyphenyl)benzoyl]piperazin-1-yl]-N-[3-nitro-4-(2-phenylsulfanylethylamino)phenyl]sulfonylbenzamide |
| B2Q038-01 | 4-[4-[3-tert-Butyl-5-(5-ethoxypyridin-3-yl)benzoyl]piperazin-1-yl]-N-[3-nitro-4-(2-phenylsulfanylethylamino)phenyl]sulfonylbenzamide |
| B2Q039-01 | 4-[4-[3-(3-Ethoxyphenyl)-5-(trifluoromethyl)benzoyl]piperazin-1-yl]-N-[3-nitro-4-(2-phenylsulfanylethylamino)phenyl]sulfonylbenzamide |
| B2Q040-01 | 4-[4-[3-(5-Ethoxypyridin-3-yl)-5-(trifluoromethyl)benzoyl]piperazin-1-yl]-N-[3-nitro-4-(2-phenylsulfanylethylamino)phenyl]sulfonylbenzamide |
| B2Q041-01 | 4-[4-[3-[2-(3-Ethoxyphenyl)ethynyl]-5-(trifluoromethyl)benzoyl]piperazin-1-yl]-N-[3-nitro-4-(2-phenylsulfanylethylamino)phenyl]sulfonylbenzamide |
| B2Q042-01 | 4-[4-[3-[2-(5-Ethoxypyridin-3-yl)ethynyl]-5-(trifluoromethyl)benzoyl]piperazin-1-yl]-N-[3-nitro-4-(2-phenylsulfanylethylamino)phenyl]sulfonylbenzamide |
| B2Q043-01 | 4-[4-[3-[4-(3-Ethoxyphenyl)pyrazol-1-yl]-5-(trifluoromethyl)benzoyl]piperazin-1-yl]-N-[3-nitro-4-(2-phenylsulfanylethylamino)phenyl]sulfonylbenzamide |
| B2Q044-01 | 4-[4-[3-[4-(5-Ethoxypyridin-3-yl)pyrazol-1-yl]-5-(trifluoromethyl)benzoyl]piperazin-1-yl]-N-[3-nitro-4-(2-phenylsulfanylethylamino)phenyl]sulfonylbenzamide |
| B2Q045-01 | 4-[4-[[2-(3-Ethoxyphenyl)phenyl]methyl]piperazin-1-yl]-N-[3-nitro-4-(2-phenylsulfanylethylamino)phenyl]sulfonylbenzamide |
| B2Q046-01 | 4-[4-[[2-(5-Ethoxypyridin-3-yl)phenyl]methyl]piperazin-1-yl]-N-[3-nitro-4-(2-phenylsulfanylethylamino)phenyl]sulfonylbenzamide |
| B2Q047-01 | 4-[4-[[4-(3-Ethoxyphenyl)-3-methylphenyl]methyl]piperazin-1-yl]-N-[3-nitro-4-(2-phenylsulfanylethylamino)phenyl]sulfonylbenzamide |
| B2Q048-01 | 4-[4-[[4-(5-Ethoxypyridin-3-yl)-3-methylphenyl]methyl]piperazin-1-yl]-N-[3-nitro-4-(2-phenylsulfanylethylamino)phenyl]sulfonylbenzamide |
| B2Q049-01 | 4-[4-[[4(-Ethoxyphenyl)-2-ethylphenyl]methyl]piperazin-1-yl]-N-[3-nitro-4-(2-phenylsulfanylethylamino)phenyl]sulfonylbenzamide |
| B2Q050-01 | 4-[4-[[4-(5-Ethoxypyridin-3-yl)-2-ethylphenyl]methyl]piperazin-1-yl]-N-[3-nitro-4-(2-phenylsulfanylethylamino)phenyl]sulfonylbenzamide |
| B2Q051-01 | 4-[4-[[4-(3-Ethoxyphenyl)-3-fluorophenyl]methyl]piperazin-1-yl]-N-[3-nitro-4-(2-phenylsulfanylethylamino)phenyl]sulfonylbenzamide |
| B2Q052-01 | 4-[4-[[4-(5-Ethoxypyridin-3-yl)-3-fluorophenyl]methyl]piperazin-1-yl]-N-[3-nitro-4-(2-phenylsulfanylethylamino)phenyl]sulfonylbenzamide |
| B2Q053-01 | 4-[4-[[4-(3-Ethoxyphenyl)-2-methoxyphenyl]methyl]piperazin-1-yl]-N-[3-nitro-4-(2-phenylsulfanylethylamino)phenyl]sulfonylbenzamide |
| B2Q054-01 | 4-[4-[[4-(5-Ethoxypyridin-3-yl)-2-methoxyphenyl]methyl]piperazin-1-yl]-N-[3-nitro-4-(2-phenylsulfanylethylamino)phenyl]sulfonylbenzamide |
| B2Q055-01 | 4-[4-[[3-Ethoxy-5-(3-ethoxyphenyl)phenyl]methyl]piperazin-1-yl]-N-[3-nitro-4-(2-phenylsulfanylethylamino)phenyl]sulfonylbenzamide |

(continued)

| Mixture No. | mixAI-B2Q-01 |
|---|---|
| Compound No. that may be included | Compound name |
| B2Q056-01 | 4-[4-[[3-Ethoxy-5-(5-ethoxypyridin-3-yl)phenyl]methyl]piperazin-1-yl]-N-[3-nitro-4-(2-phenylsulfanylethylamino)phenyl]sulfonylbenzamide |
| B2Q057-01 | 4-[4-[[3-(3-Ethoxyphenyl)-5-propan-2-yloxyphenyl]methyl]piperazin-1-yl]-N-[3-nitro-4-(2-phenylsulfanylethylamino)phenyl]sulfonylbenzamide |
| B2Q058-01 | 4-[4-[[3-(5-Ethoxypyridin-3-yl)-5-propan-2-yloxyphenyl]methyl]piperazin-1-yl]-N-[3-nitro-4-(2-phenylsulfanylethylamino)phenyl]sulfonylbenzamide |
| B2Q059-01 | 4-[4-[[4-(3-Ethoxyphenyl)-2,6-dimethoxyphenyl]methyl]piperazin-1-yl]-N-[3-nitro-4-(2-phenylsulfanylethylamino)phenyl]sulfonylbenzamide |
| B2Q060-01 | 4-[4-[[4-(5-Ethoxypyridin-3-yl)-2,6-dimethoxyphenyl]methyl]piperazin-1-yl]-N-[3-nitro-4-(2-phenylsulfanylethylamino)phenyl]sulfonylbenzamide |
| B2Q061-01 | 4-[4-[[3-(3-Ethoxyphenyl)-5-(trifluoromethyl)phenyl]methyl]piperazin-1-yl]-N-[3-nitro-4-(2-phenylsulfanylethylamino)phenyl]sulfonylbenzamide |
| B2Q062-01 | 4-[4-[[3-(5-Ethoxypyridin-3-yl)-5-(trifluoromethyl)phenyl]methyl]piperazin-1-yl]-N-[3-nitro-4-(2-phenylsulfanylethylamino)phenyl]sulfonylbenzamide |
| B2Q063-01 | 4-[4-[[3-(3-Ethoxyphenyl)-5-(trifluoromethoxy)phenyl]methyl]piperazin-1-yl]-N-[3-nitro-4-(2-phenylsulfanylethylamino)phenyl]sulfonylbenzamide |
| B2Q064-01 | 4-[4-[[3-(5-Ethoxypyridin-3-yl)-5-(trifluoromethoxy)phenyl] methyl]piperazin-1-yl]-N-[3-nitro-4-(2-phenylsulfanylethylamino)phenyl]sulfonylbenzamide |
| B2Q065-01 | 4-[4-[[4-(3-Ethoxyphenyl)naphthalen-1-yl]methyl]piperazin-1-yl]-N-[3-nitro-4-(2-phenylsulfanylethylamino)phenyl]sulfonylbenzamide |
| B2Q066-01 | 4-[4-[[4-(5-Ethoxypyridin-3-yl)naphthalen-1-yl]methyl]piperazin-1-yl]-N-[3-nitro-4-(2-phenylsulfanylethylamino)phenyl]sulfonylbenzamide |
| B2Q067-01 | 4-[4-[[4-(-Ethoxyphenyl)thiophen-2-yl]methyl]piperazin-1-yl]-N-[3-nitro-4-(2-phenylsulfanylethylamino)phenyl]sulfonylbenzamide |
| B2Q068-01 | 4-[4-[[4-(5-Ethoxypyridin-3-yl)thiophen-2-yl]methyl]piperazin-1-yl]-N-[3-nitro-4-(2-phenylsulfanylethylamino)phenyl]sulfonylbenzamide |
| B2Q069-01 | 4-[4-[[2-[2-(3-Ethoxyphenyl)ethynyl]phenyl]methyl]piperazin-1-yl]-N-[3-nitro-4-(2-phenylsulfanylethylamino)phenyl]sulfonylbenzamide |
| B2Q070-01 | 4-[4-[[2-[2-(5-Ethoxypyridin-3-yl)ethynyl]phenyl]methyl]piperazin-1-yl]-N-[3-nitro-4-(2-phenylsulfanylethylamino)phenyl]sulfonylbenzamide |
| B2Q071-01 | 6-[4-[[2-(3-Ethoxyphenyl)phenyl]methyl]piperazin-1-yl]-N-[3-nitro-4-(2-phenylsulfanylethylamino)phenyl] sulfonylpyridazine-3-carboxamide |
| B2Q072-01 | 6-[4-[[2-(5-Ethoxypyridin-3-yl)phenyl]methyl]piperazin-1-yl]-N-[3-nitro-4-(2-phenylsulfanylethylamino)phenyl]sulfonylpyridazine-3-carboxamide |
| B2Q073-01 | 6-[4-[[4-(3-Ethoxyphenyl)-3-methylphenyl]methyl]piperazin-1-yl]-N-[3-nitro-4-(2-phenylsulfanylethylamino)phenyl]sulfonylpyridazine-3-carboxamide |
| B2Q074-01 | 6-[4-[[4-(5-Ethoxypyridin-3-yl)-3-methylphenyl]methyl]piperazin-1-yl]-N-[3-nitro-4-(2-phenylsulfanylethylamino)phenyl]sulfonylpyridazine-3-carboxamide |
| B2Q075-01 | 6-[4-[[4-(-Ethoxyphenyl)-2-ethylphenyl]methyl]piperazin-1-yl]-N-[3-nitro-4-(2-phenylsulfanylethylamino)phenyl] sulfonylpyridazine-3-carboxamide |
| B2Q076-01 | 6-[4-[[4-(5-Ethoxypyridin-3-yl)-2-ethylphenyl]methyl]piperazin-1-yl]-N-[3-nitro-4-(2-phenylsulfanylethylamino)phenyl]sulfonylpyridazine-3-carboxamide |

(continued)

| Mixture No. | mixAI-B2Q-01 |
|---|---|
| Compound No. that may be included | Compound name |
| B2Q077-01 | 6-[4-[[4-(3-Ethoxyphenyl)-3-fluorophenyl]methyl]piperazin-1-yl]-N-[3-nitro-4-(2-phenylsulfanylethylamino)phenyl] sulfonylpyridazine-3-carboxamide |
| B2Q078-01 | 6-[4-[[4-(5-Ethoxypyridin-3-yl)-3-fluorophenyl]methyl]piperazin-1-yl]-N-[3-nitro-4-(2-phenylsulfanylethylamino)phenyl]sulfonylpyridazine-3-carboxamide |
| B2Q079-01 | 6-[4-[[4-(-Ethoxyphenyl)-2-methoxyphenyl]methyl]piperazin-1-yl]-N-[3-nitro-4-(2-phenylsulfanylethylamino)phenyl] sulfonylpyridazine-3-carboxamide |
| B2Q080-01 | 6-[4-[[4-(5-Ethoxypyridin-3-yl)-2-methoxyphenyl]methyl]piperazin-1-yl]-N-[3-nitro-4-(2-phenylsulfanylethylamino)phenyl]sulfonylpyridazine-3-carboxamide |
| B2Q081-01 | 6-[4-[[3-Ethoxy-5-(3-ethoxyphenyl)phenyl]methyl]piperazin-1-yl]-N-[3-nitro-4-(2-phenylsulfanylethylamino)phenyl] sulfonylpyridazine-3-carboxamide |
| B2Q082-01 | 6-[4-[[3-Ethoxy-5-(5-ethoxypyridin-3-yl)phenyl]methyl]piperazin-1-yl]-N-[3-nitro-4-(2-phenylsulfanylethylamino)phenyl]sulfonylpyridazine-3-carboxamide |
| B2Q083-01 | 6-[4-[[3-(3-Ethoxyphenyl)-5-propan-2-yloxyphenyl]methyl]piperazin-1-yl]-N-[3-nitro-4-(2-phenylsulfanylethylamino)phenyl] sulfonylpyridazine-3-carboxamide |
| B2Q084-01 | 6-[4-[[3-(5-Ethoxypyridin-3-yl)-5-propan-2-yloxyphenyl]methyl]piperazin-1-yl]-N-[3-nitro-4-(2-phenylsulfanylethylamino)phenyl]sulfonylpyridazine-3-carboxamide |
| B2Q085-01 | 6-[4-[[4-(3-Ethoxyphenyl)-2,6-dimethoxyphenyl]methyl]piperazin-1-yl]-N-[3-nitro-4-(2-phenylsulfanylethylamino)phenyl]sulfonylpyridazine-3-carboxamide |
| B2Q086-01 | 6-[4-[[4-(5-Ethoxypyridin-3-yl)-2,6-dimethoxyphenyl]methyl]piperazin-1-yl]-N-[3-nitro-4-(2-phenylsulfanylethylamino)phenyl]sulfonylpyridazine-3-carboxamide |
| B2Q087-01 | 6-[4-[[3-(3-Ethoxyphenyl)-5-(trifluoromethyl)phenyl]methyl]piperazin-1-yl]-N-[3-nitro-4-(2-phenylsulfanylethylamino)phenyl]sulfonylpyridazine-3-carboxamide |
| B2Q088-01 | 6-[4-[[3-(5-Ethoxypyridin-3-yl)-5-(trifluoromethyl)phenyl]methyl]piperazin-1-yl]-N-[3-nitro-4-(2-phenylsulfanylethylamino)phenyl] sulfonylpyridazine-3-carboxamide |
| B2Q089-01 | 6-[4-[[3-(3-Ethoxyphenyl)-5-(trifluoromethoxy)phenyl]methyl]piperazin-1-yl]-N-[3-nitro-4-(2-phenylsulfanylethylamino)phenyl]sulfonylpyridazine-3-carboxamide |
| B2Q090-01 | 6-[4-[[3-(5-Ethoxypyridin-3-yl)-5-(trifluoromethoxy)phenyl]methyl]piperazin-1-yl]-N-[3-nitro-4-(2-phenylsulfanylethylamino)phenyl]sulfonylpyridazine-3-carboxamide |
| B2Q091-01 | 6-[4-[[4-(3-Ethoxyphenyl)naphthalen-1-yl]methyl]piperazin-1-yl]-N-[3-nitro-4-(2-phenylsulfanylethylamino)phenyl]sulfonylpyridazine-3-carboxamide |
| B2Q092-01 | 6-[4-[[4-(5-Ethoxypyridin-3-yl)naphthalen-1-yl]methyl]piperazin-1-yl]-N-[3-nitro-4-(2-phenylsulfanylethylamino)phenyl]sulfonylpyridazine-3-carboxamide |
| B2Q093-01 | 6-[4-[[4-(-Ethoxyphenyl)thiophen-2-yl]methyl]piperazin-1-yl]-N-[3-nitro-4-(2-phenylsulfanylethylamino)phenyl]sulfonylpyridazine-3-carboxamide |
| B2Q094-01 | 6-[4-[[4-(5-Ethoxypyridin-3-yl)thiophen-2-yl]methyl]piperazin-1-yl]-N-[3-nitro-4-(2-phenylsulfanylethylamino)phenyl]sulfonylpyridazine-3-carboxamide |
| B2Q095-01 | 6-[4-[[2-[2-(3-Ethoxyphenyl)ethynyl]phenyl]methyl]piperazin-1-yl]-N-[3-nitro-4-(2-phenylsulfanylethylamino)phenyl]sulfonylpyridazine-3-carboxamide |
| B2Q096-01 | 6-[4-[[2-[2-(5-Ethoxypyridin-3-yl)ethynyl]phenyl]methyl]piperazin-1-yl]-N-[3-nitro-4-(2-phenylsulfanylethylamino)phenyl]sulfonylpyridazine-3-carboxamide |
| B2Q097-01 | 4-[4-[1-[4-(3-Ethoxyphenyl)phenyl]ethyl]piperazin-1-yl]-N-[3-nitro-4-(2-phenylsulfanylethylamino)phenyl]sulfonylbenzamide |

(continued)

| Mixture No. | mixAI-B2Q-01 |
|---|---|
| Compound No. that may be included | Compound name |
| B2Q098-01 | 4-[4-[1-[4-(5-Ethoxypyridin-3-yl)phenyl]ethyl]piperazin-1-yl]-N-[3-nitro-4-(2-phenylsulfanylethylamino)phenyl]sulfonylbenzamide |
| B2Q099-01 | 4-[4-[1-[4-(3-Ethoxyphenyl)-2-fluorophenyl]ethyl]piperazin-1-yl]-N-[3-nitro-4-(2-phenylsulfanylethylamino)phenyl]sulfonylbenzamide |
| B2Q 100-01 | 4-[4-[1-[4-(5-Ethoxypyridin-3-yl)-2-fluorophenyl]ethyl]piperazin-1-yl]-N-[3-nitro-4-(2-phenylsulfanylethylamino)phenyl]sulfonylbenzamide |

[Table 97]

[Table AI-03-B4J-01]

| Mixture No. | mixAI-B4J-01 |
|---|---|
| Compound No. that may be included | Compound name |
| B4J001-01 | 6-[4-[4-(3-Ethoxyphenyl)thiophene-2-carbonyl]piperazin-1-yl]-N,N-diethylpyridazine-3-carboxamide |
| B4J002-01 | 6-[4-[4-(5-Ethoxypyridin-3-yl)thiophene-2-carbonyl]piperazin-1-yl]-N,N-diethylpyridazine-3-carboxamide |
| B4J003-01 | 6-[4-[4-(3-Ethoxyphenyl)-3-methylbenzoyl]piperazin-1-yl]-N,N-diethylpyridazine-3-carboxamide |
| B4J004-01 | 6-[4-[4-(5-Ethoxypyridin-3-yl)-3-methylbenzoyl]piperazin-1-yl]-N,N-diethylpyridazine-3-carboxamide |
| B4J005-01 | 6-[4-[4-(3-Ethoxyphenyl)-3-fluorobenzoyl]piperazin-1-yl]-N,N-diethylpyridazine-3-carboxamide |
| B4J006-01 | 6-[4-[4-(5-Ethoxypyridin-3-yl)-3-fluorobenzoyl]piperazin-1-yl]-N,N-diethylpyridazine-3-carboxamide |
| B4J007-01 | 6-[4-[2-[2-(3-Ethoxyphenyl)ethynyl]benzoyl]piperazin-1-yl]-N,N-diethylpyridazine-3-carboxamide |
| B4J008-01 | 6-[4-[2-[2-(5-Ethoxypyridin-3-yl)ethynyl]benzoyl]piperazin-1-yl]-N,N-diethylpyridazine-3-carboxamide |
| B4J009-01 | 6-[4-[5-[2-(3-Ethoxyphenyl)ethynyl]pyridine-3-carbonyl]piperazin-1-yl]-N,N-diethylpyridazine-3-carboxamide |
| B4J010-01 | 6-[4-[5-[2-(5-Ethoxypyridin-3-yl)ethynyl]pyridine-3-carbonyl]piperazin-1-yl]-N,N-diethylpyridazine-3-carboxamide |
| B4J011-01 | 6-[4-[3-[2-(3-Ethoxyphenyl)phenyl]propanoyl]piperazin-1-yl]-N,N-diethylpyridazine-3-carboxamide |
| B4J012-01 | 6-[4-[3-[2-(5-Ethoxypyridin-3-yl)phenyl]propanoyl]piperazin-1-yl]-N,N-diethylpyridazine-3-carboxamide |
| B4J013-01 | 6-[4-[4-(3-Ethoxyphenyl)naphthalene-1-carbonyl]piperazin-1-yl]-N,N-diethylpyridazine-3-carboxamide |

(continued)

| Mixture No. | mixAI-B4J-01 |
|---|---|
| Compound No. that may be included | Compound name |
| B4J014-01 | 6-[4-[4-(5-Ethoxypyridin-3-yl)naphthalene-1-carbonyl]piperazin-1-yl]-N,N-diethylpyridazine-3-carboxamide |
| B4J015-01 | 6-[4-[3-tert-Butyl-5-(3-ethoxyphenyl)benzoyl]piperazin-1-yl]-N,N-diethylpyridazine-3-carboxamide |
| B4J016-01 | 6-[4-[3-tert-Butyl-5-(5-ethoxypyridin-3-yl)benzoyl]piperazin-1-yl]-N,N-diethylpyridazine-3-carboxamide |
| B4J017-01 | 6-[4-[3-(3-Ethoxyphenyl)-5-(trifluoromethyl)benzoyl]piperazin-1-yl]-N,N-diethylpyridazine-3-carboxamide |
| B4J018-01 | 6-[4-[3-(5-Ethoxypyridin-3-yl)-5-(trifluoromethyl)benzoyl]piperazin-1-yl]-N,N-diethylpyridazine-3-carboxamide |
| B4J019-01 | 6-[4-[3-[2-(3-Ethoxyphenyl)ethynyl]-5-(trifluoromethyl)benzoyl]piperazin-1-yl]-N,N-diethylpyridazine-3-carboxamide |
| B4J020-01 | 6-[4-[3-[2-(5-Ethoxypyridin-3-yl)ethynyl]-5-(trifluoromethyl)benzoyl]piperazin-1-yl]-N,N-diethylpyridazine-3-carboxamide |
| B4J021-01 | 6-[4-[3-[4-(3-Ethoxyphenyl)pyrazol-1-yl]-5-(trifluoromethyl)benzoyl]piperazin-1-yl]-N,N-diethylpyridazine-3-carboxamide |
| B4J022-01 | 6-[4-[3-[4-(5-Ethoxypyridin-3-yl)pyrazol-1-yl]-5-(trifluoromethyl)benzoyl]piperazin-1-yl]-N,N-diethylpyridazine-3-carboxamide |
| B4J023-01 | 4-[4-[4-(3-Ethoxyphenyl)thiophene-2-carbonyl]piperazin-1-yl]-N,N-diethylbenzamide |
| B4J024-01 | 4-[4-[4-(5-Ethoxypyridin-3-yl)thiophene-2-carbonyl]piperazin-1-yl]-N,N-diethylbenzamide |
| B4J025-01 | 4-[4-[4-(3-Ethoxyphenyl)-3-methylbenzoyl]piperazin-1-yl]-N,N-diethylbenzamide |
| B4J026-01 | 4-[4-[4-(5-Ethoxypyridin-3-yl)-3-methylbenzoyl]piperazin-1-yl]-N,N-diethylbenzamide |
| B4J027-01 | 4-[4-[4-(3-Ethoxyphenyl)-3-fluorobenzoyl]piperazin-1-yl]-N,N-diethylbenzamide |
| B4J028-01 | 4-[4-[4-(5-Ethoxypyridin-3-yl)-3-fluorobenzoyl]piperazin-1-yl]-N,N-diethylbenzamide |
| B4J029-01 | 4-[4-[2-[2-(3-Ethoxyphenyl)ethynyl]benzoyl]piperazin-1-yl]-N,N-diethylbenzamide |
| B4J030-01 | 4-[4-[2-[2-(5-Ethoxypyridin-3-yl)ethynyl]benzoyl]piperazin-1-yl]-N,N-diethylbenzamide |
| B4J031-01 | 4-[4-[5-[2-(3-Ethoxyphenyl)ethynyl]pyridine-3-carbonyl]piperazin-1-yl]-N,N-diethylbenzamide |
| B4J032-01 | 4-[4-[5-[2-(5-Ethoxypyridin-3-yl)ethynyl]pyridine-3-carbonyl]piperazin-1-yl]-N,N-diethylbenzamide |
| B4J033-01 | 4-[4-[3-[2-(3-Ethoxyphenyl)phenyl]propanoyl]piperazin-1-yl]-N,N-diethylbenzamide |
| B4J034-01 | 4-[4-3-[2-(5-Ethoxypyridin-3-yl)phenyl]propanoyl]piperazin-1-yl]-N,N-diethylbenzamide |
| B4J035-01 | 4-[4-[4-(3-Ethoxyphenyl)naphthalene-1-carbonyl]piperazin-1-yl]-N,N-diethylbenzamide |
| B4J036-01 | 4-[4-[4-(5-Ethoxypyridin-3-yl)naphthalene-1-carbonyl]piperazin-1-yl]-N,N-diethylbenzamide |
| B4J037-01 | 4-[4-[3-tert-Butyl-5-(3-ethoxyphenyl)benzoyl]piperazin-1-yl]-N,N-diethylbenzamide |
| B4J038-01 | 4-[4-[3-tert-Butyl-5-(5-ethoxypyridin-3-yl)benzoyl]piperazin-1-yl]-N,N-diethylbenzamide |
| B4J039-01 | 4-[4-[3-(3-Ethoxyphenyl)-5-(trifluoromethyl)benzoyl]piperazin-1-yl]-N,N-diethylbenzamide |
| B4J040-01 | 4-[4-[3-(5-Ethoxypyridin-3-yl)-5-(trifluoromethyl)benzoyl]piperazin-1-yl]-N,N-diethylbenzamide |
| B4J041-01 | 4-[4-[3-[2-(3-Ethoxyphenyl)ethynyl]-5-(trifluoromethyl)benzoyl]piperazin-1-yl]-N,N-diethylbenzamide |

(continued)

| Mixture No. | mixAI-B4J-01 |
|---|---|
| Compound No. that may be included | Compound name |
| B4J042-01 | 4-[4-[3-[2-(5-Ethoxypyridin-3-yl)ethynyl]-5-(trifluoromethyl)benzoyl]piperazin-1-yl]-N,N-diethylbenzamide |
| B4J043-01 | 4-[4-[3-[4-(3-Ethoxyphenyl)pyrazol-1-yl]-5-(trifluoromethyl)benzoyl]piperazin-1-yl]-N,N-diethylbenzamide |
| B4J044-01 | 4-[4-[3-[4-(5-Ethoxypyridin-3-yl)pyrazol-1-yl]-5-(trifluoromethyl)benzoyl]piperazin-1-yl]-N,N-diethylbenzamide |
| B4J045-01 | 4-[4-[[2-(3-Ethoxyphenyl)phenyllmethyl]piperazin-1-yl]-N,N-diethylbenzamide |
| B4J046-01 | 4-[4-[[2-(5-Ethoxypyridin-3-yl)phenyl]methyl]piperazin-1-yl]-N,N-diethylbenzamide |
| B4J047-01 | 4-[4-[4-(3-Ethoxyphenyl)-3-methylphenyl]methyl]piperazin-1-yl]-N,N-diethylbenzamide |
| B4J048-01 | 4-[4-[[4-(5-Ethoxypyridin-3-yl)-3-methylphenyl]methyl]piperazin-1-yl]-N,N-diethylbenzamide |
| B4J049-01 | 4-[4-[[4-(3-Ethoxyphenyl)-2-ethylphenyl]methyl]piperazin-1-yl]-N,N-diethylbenzamide |
| B4J050-01 | 4-[4-[4-(5-Ethoxypyridin-3-yl)-2-ethylphenyl]methyl]piperazin-1-yl]-N,N-diethylbenzamide |
| B4J051-01 | 4-[4-[[4-(3-Ethoxyphenyl)-3-fluorophenyl]methyl]piperazin-1-yl]-N,N-diethylbenzamide |
| B4J052-01 | 4-[4-[[4-(5-Ethoxypyridin-3-yl)-3-fluorophenyl]methyl]piperazin-1-yl]-N,N-diethylbenzamide |
| B4J053-01 | 4-[4-[[4-(3-Ethoxyphenyl)-2-methoxyphenyl]methyl]piperazin-1-yl]-N,N-diethylbenzamide |
| B4J054-01 | 4-[4-[4-(5-Ethoxypyridin-3-yl)-2-methoxyphenyl]methyl]piperazin-1-yl]-N,N-diethylbenzamide |
| B4J055-01 | 4-[4-[[3-Ethoxy-5-(3-ethoxyphenyl)phenyl]methyl]piperazin-1-yl]-N,N-diethylbenzamide |
| B4J056-01 | 4-[4-[[3-Ethoxy-5-(5-ethoxypyridin-3-yl)phenyl]methyl]piperazin-1-yl]-N,N-diethylbenzamide |
| B4J057-01 | 4-[4-[3-(3-Ethoxyphenyl)-5-propan-2-yloxyphenyl]methyl]piperazin-1-yl]-N,N-diethylbenzamide |
| B4J058-01 | 4-[4-[3-(5-Ethoxypyridin-3-yl)-5-propan-2-yloxyphenyl]methyl]piperazin-1-yl]-N,N-diethylbenzamide |
| B4J059-01 | 4-[4-[[4-(3-Ethoxyphenyl)-2,6-dimethoxyphenyl]methyl]piperazin-1-yl]-N,N-diethylbenzamide |
| B4J060-01 | 4-[4-[[4-(5-Ethoxypyridin-3-yl)-2,6-dimethoxyphenyl]methyl]piperazin-1-yl]-N,N-diethylbenzamide |
| B4J061-01 | 4-[4-[[3-(3-Ethoxyphenyl)-5-(trifluoromethyl)phenyl]methyl]piperazin-1-yl]-N,N-diethylbenzamide |
| B4J062-01 | 4-[4-[[3-(5-Ethoxypyridin-3-yl)-5-(trifluoromethyl)phenyl]methyl]piperazin-1-yl]-N,N-diethylbenzamide |
| B4J063-01 | 4-[4-[[3-(3-Ethoxyphenyl)-5-(trifluoromethoxy)phenyl]methyl]piperazin-1-yl]-N,N-diethylbenzamide |
| B4J064-01 | 4-[4-[[3-(5-Ethoxypyridin-3-yl)-5-(trifluoromethoxy)phenyl]methyl]piperazin-1-yl]-N,N-diethylbenzamide |
| B4J065-01 | 4-[4-[[4-(3-Ethoxyphenyl)naphthalen-1-yl]methyl]piperazin-1-yl]-N,N-diethylbenzamide |
| B4J066-01 | 4-[4-[[4-(5-Ethoxypyridin-3-yl)naphthalen-1-yl]methyl]piperazin-1-yl]-N,N-diethylbenzamide |
| B4J067-01 | 4-[4-[[4-(3-Ethoxyphenyl)thiophen-2-yl]methyl]piperazin-1-yl]-N,N-diethylbenzamide |
| B4J068-01 | 4-[4-[[4-(5-Ethoxypyridin-3-yl)thiophen-2-yl]methyl]piperazin-1-yl]-N,N-diethylbenzamide |
| B4J069-01 | 4-[4-[[2-[2-(3-Ethoxyphenyl)ethynyl]phenyl]methyl]piperazin-1-yl]-N,N-diethylbenzamide |

(continued)

| Mixture No. | mixAI-B4J-01 |
|---|---|
| Compound No. that may be included | Compound name |
| B4J070-01 | 4-[4-[[2-[2-(5-Ethoxypyridin-3-yl)ethynyl]phenyl]methyl]piperazin-1-yl]-N,N-diethylbenzamide |
| B4J071-01 | 6-[4-[[2-(3-Ethoxyphenyl)phenyl]methyl]piperazin-1-yl]-N,N-diethylpyridazine-3-carboxamide |
| B4J072-01 | 6-[4-[[2-(5-Ethoxypyridin-3-yl)phenyl]methyl]piperazin-1-yl]-N,N-diethylpyridazine-3-carboxamide |
| B4J073-01 | 6-[4-[[4-(3-Ethoxyphenyl)-3-methylphenyl]methyl]piperazin-1-yl]-N,N-diethylpyridazine-3-carboxamide |
| B4J074-01 | 6-[4-[[4-(5-Ethoxypyridin-3-yl)-3-methylphenyl]methyl]piperazin-1-yl]-N,N-diethylpyridazine-3-carboxamide |
| B4J075-01 | 6-[4-[[4-(3-Ethoxyphenyl)-2-ethylphenyl]methyl]piperazin-1-yl]-N,N-diethylpyridazine-3-carboxamide |
| B4J076-01 | 6-[4-[[4-(5-Ethoxypyridin-3-yl)-2-ethylphenyl]methyl]piperazin-1-yl]-N,N-diethylpyridazine-3-carboxamide |
| B4J077-01 | 6-[4-[[4-(3-Ethoxyphenyl)-3-fluorophenyl]methyl]piperazin-1-yl]-N,N-diethylpyridazine-3-carboxamide |
| B4J078-01 | 6-[4-[[4-(5-Ethoxypyridin-3-yl)-3-fluorophenyl]methyl]piperazin-1-yl]-N,N-diethylpyridazine-3-carboxamide |
| B4J079-01 | 6-[4-[[4-(3-Ethoxyphenyl)-2-methoxyphenyl]methyl]piperazin-1-yl]-N,N-diethylpyridazine-3-carboxamide |
| B4J080-01 | 6-[4-[[4-(5-Ethoxypyridin-3-yl)-2-methoxyphenyl]methyl]piperazin-1-yl]-N,N-diethylpyridazine-3-carboxamide |
| B4J081-01 | 6-[4-[[3-Ethoxy-5-(3-ethoxyphenyl)phenyl]methyl]piperazin-1-yl]-N,N-diethylpyridazine-3-carboxamide |
| B4J082-01 | 6-[4-[[3-Ethoxy-5-(5-ethoxypyridin-3-yl)phenyl]methyl]piperazin-1-yl]-N,N-diethylpyridazine-3-carboxamide |
| B4J083-01 | 6-[4-[[3-(3-Ethoxyphenyl)-5-propan-2-yloxyphenyl]methyl]piperazin-1-yl]-N,N-diethylpyridazine-3-carboxamide |
| B4J084-01 | 6-[4-[[3-(5-Ethoxypyridin-3-yl)-5-propan-2-yloxyphenyl]methyl]piperazin-1-yl]-N,N-diethylpyridazine-3-carboxamide |
| B4J085-01 | 6-[4-[[4-(3-Ethoxyphenyl)-2,6-dimethoxyphenyl]methyl]piperazin-1-yl]-N,N-diethylpyridazine-3-carboxamide |
| B4J086-01 | 6-[4-[[4-(5-Ethoxypyridin-3-yl)-2,6-dimethoxyphenyl]methyl]piperazin-1-yl]-N,N-diethylpyridazine-3-carboxamide |
| B4J087-01 | 6-[4-[[3-(3-Ethoxyphenyl)-5-(trifluoromethyl)phenyl]methyl]piperazin-1-yl]-N,N-diethylpyridazine-3-carboxamide |
| B4J088-01 | 6-[4-[[3-(5-Ethoxypyridin-3-yl)-5-(trifluoromethyl)phenyl]methyl]piperazin-1-yl]-N,N-diethylpyridazine-3-carboxamide |
| B4J089-01 | 6-[4-[[3-(3-Ethoxyphenyl)-5-(trifluoromethoxy)phenyl]methyl]piperazin-1-yl]-N,N-diethylpyridazine-3-carboxamide |
| B4J090-01 | 6-[4-[[3-(5-Ethoxypyridin-3-yl)-5-(trifluoromethoxy)phenyl]methyl]piperazin-1-yl]-N,N-diethylpyridazine-3-carboxamide |

(continued)

| Mixture No. | mixAI-B4J-01 |
|---|---|
| Compound No. that may be included | Compound name |
| B4J091-01 | 6-[4-[[4-(3-Ethoxyphenyl)naphthalen-1-yl]methyl]piperazin-1-yl]-N,N-diethylpyridazine-3-carboxamide |
| B4J092-01 | 6-[4-[[4-(5-Ethoxypyridin-3-yl)naphthalen-1-yl]methyl]piperazin-1-yl]-N,N-diethylpyridazine-3-carboxamide |
| B4J093-01 | 6-[4-[[4-(3-Ethoxyphenyl)thiophen-2-yl]methyl]piperazin-1-yl]-N,N-diethylpyridazine-3-carboxamide |
| B4J094-01 | 6-[4-[[4-(5-Ethoxypyridin-3-yl)thiophen-2-yl]methyl]piperazin-1-yl]-N,N-diethylpyridazine-3-carboxamide |
| B4J095-01 | 6-[4-[[2-[2-(3-Ethoxyphenyl)ethynyl]phenyl]methyl]piperazin-1-yl]-N,N-diethylpyridazine-3-carboxamide |
| B4J096-01 | 6-[4-[[2-[2-(5-Ethoxypyridin-3-yl)ethynyl]phenyl]methyl]piperazin-1-yl]-N,N-diethylpyridazine-3-carboxamide |
| B4J097-01 | 4-[4-[1-[4-(3-Ethoxyphenyl)phenyl]ethyl]piperazin-1-yl]-N,N-diethylbenzamide |
| B4J098-01 | 4-[4-[1-[4-(5-Ethoxypyridin-3-yl)phenyl]ethyl]piperazin-1-yl]-N,N-diethylbenzamide |
| B4J099-01 | 4-[4-[1-[4-(3-Ethoxyphenyl)-2-fluorophenyl]ethyl]piperazin-1-yl]-N,N-diethylbenzamide |
| B4J100-01 | 4-[4-[1-[4-(5-Ethoxypyridin-3-yl)-2-fluorophenyl]ethyl]piperazin-1-yl]-N,N-diethylbenzamide |

[Table 98]

[Table AI-03-B1M-01]

| Mixture No. | mixAI-B1M-01 |
|---|---|
| Compound No. that may be included | Compound name |
| B1M001-01 | 6-[4-[5-[2-(3-Ethoxyphenyl)ethynyl]pyridine-3-carbonyl]piperazin-1-yl]-N-propylpyridazine-3-carboxamide |
| B1M002-01 | 4-[4-[5-[2-(3-Ethoxyphenyl)ethynyl]pyridine-3-carbonyl]piperazin-1-yl]-N-propylbenzamide |
| B1M003-01 | 4-[4-[1-[4-(3-Ethoxyphenyl)phenyl]ethyl]piperazin-1-yl]-N-propylbenzamide |
| B1M004-01 | 4-[4-[1-[4-(5-Ethoxypyridin-3-yl)phenyl]ethyl]piperazin-1-yl]-N-propylbenzamide |
| B1M005-01 | 4-[4-[1-[4-(3-Ethoxyphenyl)-2-fluorophenyl]ethyl]piperazin-1-yl]-N-propylbenzamide |
| B1M006-01 | 4-[4-[1-[4-(5-Ethoxypyridin-3-yl)-2-fluorophenyl]ethyl]piperazin-1-yl]-N-propylbenzamide |
| B1M007-01 | 6-[4-[3-(5-Ethoxypyridin-3-yl)-5-(trifluoromethyl)benzoyl]piperazin-1-yl]-N-propylpyridazine-3-carboxamide |
| B1M008-01 | 4-[4-[3-(5-Ethoxypyridin-3-yl)-5-(trifluoromethyl)benzoyl]piperazin-1-yl]-N-propylbenzamide |
| B1M009-01 | 4-[4-[[3-Ethoxy-5-(3-ethoxyphenyl)phenyl]methyl]piperazin-1-yl]-N-propylbenzamide |
| B1M010-01 | 6-[4-[[3-Ethoxy-5-(3-ethoxyphenyl)phenyl]methyl]piperazin-1-yl]-N-propylpyridazine-3-carboxamide |
| B1M011-01 | 4-[4-[[3-Ethoxy-5-(5-ethoxypyridin-3-yl)phenyl]methyl]piperazin-1-yl]-N-propylbenzamide |

(continued)

| Mixture No. | mixAI-B1M-01 |
|---|---|
| Compound No. that may be included | Compound name |
| B1M012-01 | 6-[4-[[3-Ethoxy-5-(5-ethoxypyridin-3-yl)phenyl]methyl]piperazin-1-yl]-N-propylpyridazine-3-carboxamide |
| B1M013-01 | 6-[4-[3-[2-(3-Ethoxyphenyl)ethynyl]-5-(trifluoromethyl)benzoyl]piperazin-1-yl]-N-propylpyridazine-3-carboxamide |
| B1M014-01 | 4-[4-[3-[2-(3-Ethoxyphenyl)ethynyl]-5-(trifluoromethyl)benzoyl]piperazin-1-yl]-N-propylbenzamide |
| B1M015-01 | 4-[4-[[3-(3-Ethoxyphenyl)-5-propan-2-yloxyphenyl]methyl]piperazin-1-yl]-N-propylbenzamide |
| B1M016-01 | 6-[4-[[3-(3-Ethoxyphenyl)-5-propan-2-yloxyphenyl]methyl]piperazin-1-yl]-N-propylpyridazine-3-carboxamide |
| B1M017-01 | 4-[4-[[3-(5-Ethoxypyridin-3-yl)-5-propan-2-yloxyphenyl]methyl]piperazin-1-yl]-N-propylbenzamide |
| B1M018-01 | 6-[4-[3-[2-(5-Ethoxypyridin-3-yl)ethynyl]-5-(trifluoromethyl)benzoyl]piperazin-1-yl]-N-propylpyridazine-3-carboxamide |
| B1M019-01 | 4-[4-[3-[2-(5-Ethoxypyridin-3-yl)ethynyl]-5-(trifluoromethyl)benzoyl]piperazin-1-yl]-N-propylbenzamide |
| B1M020-01 | 4-[4-[[4-(3-Ethoxyphenyl)-2,6-dimethoxyphenyl]methyl]piperazin-1-yl]-N-propylbenzamide |
| B1M021-01 | 6-[4-[[4-(3-Ethoxyphenyl)-2,6-dimethoxyphenyl]methyl]piperazin-1-yl]-N-propylpyridazine-3-carboxamide |
| B1M022-01 | 4-[4-[[4-(5-Ethoxypyridin-3-yl)-2,6-dimethoxyphenyl]methyl]piperazin-1-yl]-N-propylbenzamide |
| B1M023-01 | 6-[4-[[4-(5-Ethoxypyridin-3-yl)-2,6-dimethoxyphenyl]methyl]piperazin-1-yl]-N-propylpyridazine-3-carboxamide |
| B1M024-01 | 6-[4-[3-[4-(3-Ethoxyphenyl)pyrazol-1-yl]-5-(trifluoromethyl)benzoyl]piperazin-1-yl]-N-propylpyridazine-3-carboxamide |
| B1M025-01 | 4-[4-[3-[4-(3-Ethoxyphenyl)pyrazol-1-yl]-5-(trifluoromethyl)benzoyl]piperazin-1-yl]-N-propylbenzamide |
| B1M026-01 | 4-[4-[[3-(3-Ethoxyphenyl)-5-(trifluoromethyl)phenyl]methyl]piperazin-1-yl]-N-propylbenzamide |
| B1M027-01 | 6-[4-[[3-(3-Ethoxyphenyl)-5-(trifluoromethyl)phenyl]methyl]piperazin-1-yl]-N-propylpyridazine-3-carboxamide |
| B1M028-01 | 4-[4-[[3-(5-Ethoxypyridin-3-yl)-5-(trifluoromethyl)phenyl]methyl]piperazin-1-yl]-N-propylbenzamide |
| B1M029-01 | 6-[4-[[3-(5-Ethoxypyridin-3-yl)-5-(trifluoromethyl)phenyl]methyl]piperazin-1-yl]-N-propylpyridazine-3-carboxamide |
| B1M030-01 | 6-[4-[3-[4-(5-Ethoxypyridin-3-yl)pyrazol-1-yl]-5-(trifluoromethyl)benzoyl]piperazin-1-yl]-N-propylpyridazine-3-carboxamide |
| B1M031-01 | 4-[4-[3-[4-(5-Ethoxypyridin-3-yl)pyrazol-1-yl]-5-(trifluoromethyl)benzoyl]piperazin-1-yl]-N-propylbenzamide |
| B1M032-01 | 4-[4-[[3-(3-Ethoxyphenyl)-5-(trifluoromethoxy)phenyl]methyl]piperazin-1-yl]-N-propylbenzamide |
| B1M033-01 | 6-[4-[[3-(3-Ethoxyphenyl)-5-(trifluoromethoxy)phenyl]methyl]piperazin-1-yl]-N-propylpyridazine-3-carboxamide |

(continued)

| Mixture No. | mixAI-B1M-01 |
|---|---|
| Compound No. that may be included | Compound name |
| B1M034-01 | 4-[4-[[3-(5-Ethoxypyridin-3-yl)-5-(trifluoromethoxy)phenyl]methyl]piperazin-1-yl]-N-propylbenzamide |
| B1M035-01 | 6-[4-[[3-(5-Ethoxypyridin-3-yl)-5-(trifluoromethoxy)phenyl]methyl]piperazin-1-yl]-N-propylpyridazine-3-carboxamide |
| B1M036-01 | 6-[4-[4-(3-Ethoxyphenyl)-3-methylbenzoyl]piperazin-1-yl]-N-propylpyridazine-3-carboxamide |
| B1M037-01 | 6-[4-[4-(5-Ethoxypyridin-3-yl)-3-methylbenzoyl]piperazin-1-yl]-N-propylpyridazine-3-carboxamide |
| B1M038-01 | 4-[4-[4-(3-Ethoxyphenyl)-3-methylbenzoyl]piperazin-1-yl]-N-propylbenzamide |
| B1M039-01 | 4-[4-[4-(5-Ethoxypyridin-3-yl)-3-methylbenzoyl]piperazin-1-yl]-N-propylbenzamide |
| B1M040-01 | 4-[4-[[2-(3-Ethoxyphenyl)phenyl]methyl]piperazin-1-yl]-N-propylbenzamide |
| B1M041-01 | 4-[4-[[2-(5-Ethoxypyridin-3-yl)phenyl]methyl]piperazin-1-yl]-N-propylbenzamide |
| B1M042-01 | 6-[4-[[2-(3-Ethoxyphenyl)phenyl]methyl]piperazin-1-yl]-N-propylpyridazine-3-carboxamide |
| B1M043-01 | 6-[4-[[2-(5-Ethoxypyridin-3-yl)phenyl]methyl]piperazin-1-yl]-N-propylpyridazine-3-carboxamide |
| B1M044-01 | 4-[4-[[4-(3-Ethoxyphenyl)naphthalen-1-yl]methyl]piperazin-1-yl]-N-propylbenzamide |
| B1M045-01 | 6-[4-[[4-(3-Ethoxyphenyl)naphthalen-1-yl]methyl]piperazin-1-yl]-N-propylpyridazine-3-carboxamide |
| B1M046-01 | 4-[4-[[4-(5-Ethoxypyridin-3-yl)naphthalen-1-yl]methyl]piperazin-1-yl]-N-propylbenzamide |
| B1M047-01 | 6-[4-[[4-(5-Ethoxypyridin-3-yl)naphthalen-1-yl]methyl]piperazin-1-yl]-N-propylpyridazine-3-carboxamide |
| B1M048-01 | 4-[4-[[4-(3-Ethoxyphenyl)thiophen-2-yl]methyl]piperazin-1-yl]-N-propylbenzamide |
| B1M049-01 | 6-[4-[[4-(3-Ethoxyphenyl)thiophen-2-yl]methyl]piperazin-1-yl]-N-propylpyridazine-3-carboxamide |
| B1M050-01 | 4-[4-[[4-(5-Ethoxypyridin-3-yl)thiophen-2-yl]methyl]piperazin-1-yl]-N-propylbenzamide |
| B1M051-01 | 6-[4-[[4-(5-Ethoxypyridin-3-yl)thiophen-2-yl]methyl]piperazin-1-yl]-N-propylpyridazine-3-carboxamide |
| B1M052-01 | 4-[4-[[2-[2-(3-Ethoxyphenyl)ethynyl]phenyl]methyl]piperazin-1-yl]-N-propylbenzamide |
| B1M053-01 | 6-[4-[[2-[2-(3-Ethoxyphenyl)ethynyl]phenyl]methyl]piperazin-1-yl]-N-propylpyridazine-3-carboxamide |
| B1M054-01 | 4-[4-[[2-[2-(5-Ethoxypyridin-3-yl)ethynyl]phenyl]methyl]piperazin-1-yl]-N-propylbenzamide |
| B1M055-01 | 6-[4-[[2-[2-(5-Ethoxypyridin-3-yl)ethynyl]phenyl]methyl]piperazin-1-yl]-N-propylpyridazine-3-carboxamide |
| B1M056-01 | 6-[4-[5-[2-(5-Ethoxypyridin-3-yl)ethynyl]pyridine-3-carbonyl]piperazin-1-yl]-N-propylpyridazine-3-carboxamide |
| B1M057-01 | 4-[4-[5-[2-(5-Ethoxypyridin-3-yl)ethynyl]pyridine-3-carbonyl]piperazin-1-yl]-N-propylbenzamide |
| B1M058-01 | 4-[4-[[4-(3-Ethoxyphenyl)-3-methylphenyl]methyl]piperazin-1-yl]-N-propylbenzamide |
| B1M059-01 | 6-[4-[[4-(3-Ethoxyphenyl)-3-methylphenyl]methyl]piperazin-1-yl]-N-propylpyridazine-3-carboxamide |

(continued)

| Mixture No. | mixAI-B1M-01 |
|---|---|
| Compound No. that may be included | Compound name |
| B1M060-01 | 6-[4-[3-tert-Butyl-5-(3-ethoxyphenyl)benzoyl]piperazin-1-yl]-N-propylpyridazine-3-carboxamide |
| B1M061-01 | 6-[4-[3-tert-Butyl-5-(5-ethoxypyridin-3-yl)benzoyl]piperazin-1-yl]-N-propylpyridazine-3-carboxamide |
| B1M062-01 | 4-[4-[3-tert-Butyl-5-(3-ethoxyphenyl)benzoyl]piperazin-1-yl]-N-propylbenzamide |
| B1M063-01 | 4-[4-[3-tert-Butyl-5-(5-ethoxypyridin-3-yl)benzoyl]piperazin-1-yl]-N-propylbenzamide |
| B1M064-01 | 4-[4-[[4-(5-Ethoxypyridin-3-yl)-3-methylphenyl]methyl]piperazin-1-yl]-N-propylbenzamide |
| B1M065-01 | 6-[4-[[4-(5-Ethoxypyridin-3-yl)-3-methylphenyl]methyl]piperazin-1-yl]-N-propylpyridazine-3-carboxamide |
| B1M066-01 | 6-[4-[[3-(5-Ethoxypyridin-3-yl)-5-propan-2-yloxyphenyl]methyl]piperazin-1-yl]-N-propylpyridazine-3-carboxamide |
| B1M067-01 | 6-[4-[3-[2-(3-Ethoxyphenyl)phenyl]propanoyl]piperazin-1-yl]-N-propylpyridazine-3-carboxamide |
| B1M068-01 | 4-[4-[3-[2-(3-Ethoxyphenyl)phenyl]propanoyl]piperazin-1-yl]-N-propylbenzamide |
| B1M069-01 | 4-[4-[[4-(3-Ethoxyphenyl)-2-ethylphenyl]methyl]piperazin-1-yl]-N-propylbenzamide |
| B1M070-01 | 6-[4-[[4-(3-Ethoxyphenyl)-2-ethylphenyl]methyl]piperazin-1-yl]-N-propylpyridazine-3-carboxamide |
| B1M071-01 | 6-[4-[4-(3-Ethoxyphenyl)naphthalene-1-carbonyl]piperazin-1-yl]-N-propylpyridazine-3-carboxamide |
| B1M072-01 | 6-[4-[4-(5-Ethoxypyridin-3-yl)naphthalene-1-carbonyl]piperazin-1-yl]-N-propylpyridazine-3-carboxamide |
| B1M073-01 | 4-[4-[4-(3-Ethoxyphenyl)naphthalene-1-carbonyl]piperazin-1-yl]-N-propylbenzamide |
| B1M074-01 | 4-[4-[4-(5-Ethoxypyridin-3-yl)naphthalene-1-carbonyl]piperazin-1-yl]-N-propylbenzamide |
| B1M075-01 | 4-[4-[[4-(5-Ethoxypyridin-3-yl)-2-ethylphenyl]methyl]piperazin-1-yl]-N-propylbenzamide |
| B1M076-01 | 6-[4-[[4-(5-Ethoxypyridin-3-yl)-2-ethylphenyl]methyl]piperazin-1-yl]-N-propylpyridazine-3-carboxamide |
| B1M077-01 | 6-[4-[3-[2-(5-Ethoxypyridin-3-yl)phenyl]propanoyl]piperazin-1-yl]-N-propylpyridazine-3-carboxamide |
| B1M078-01 | 4-[4-[3-[2-(5-Ethoxypyridin-3-yl)phenyl]propanoyl]piperazin-1-yl]-N-propylbenzamide |
| B1M079-01 | 4-[4-[[4-(3-Ethoxyphenyl)-3-fluorophenyl]methyl]piperazin-1-yl]-N-propylbenzamide |
| B1M080-01 | 6-[4-[[4-(3-Ethoxyphenyl)-3-fluorophenyl]methyl]piperazin-1-yl]-N-propylpyridazine-3-carboxamide |
| B1M081-01 | 6-[4-[4-(3-Ethoxyphenyl)thiophene-2-carbonyl]piperazin-1-yl]-N-propylpyridazine-3-carboxamide |
| B1M082-01 | 6-[4-[4-(5-Ethoxypyridin-3-yl)thiophene-2-carbonyl]piperazin-1-yl]-N-propylpyridazine-3-carboxamide |
| B1M083-01 | 4-[4-[4-(3-Ethoxyphenyl)thiophene-2-carbonyl]piperazin-1-yl]-N-propylbenzamide |
| B1M084-01 | 4-[4-[4-(5-Ethoxypyridin-3-yl)thiophene-2-carbonyl]piperazin-1-yl]-N-propylbenzamide |
| B1M085-01 | 4-[4-[[4-(5-Ethoxypyridin-3-yl)-3-fluorophenyl]methyl]piperazin-1-yl]-N-propylbenzamide |

(continued)

| Mixture No. | mixAI-B1M-01 |
|---|---|
| Compound No. that may be included | Compound name |
| B1M086-01 | 6-[4-[[4-(5-Ethoxypyridin-3-yl)-3-fluorophenyl]methyl]piperazin-1-yl]-N-propylpyridazine-3-carboxamide |
| B1M087-01 | 6-[4-[4-(3-Ethoxyphenyl)-3-fluorobenzoyl]piperazin-1-yl]-N-propylpyridazine-3-carboxamide |
| B1M088-01 | 6-[4-[4-(5-Ethoxypyridin-3-yl)-3-fluorobenzoyl]piperazin-1-yl]-N-propylpyridazine-3-carboxamide |
| B1M089-01 | 6-[4-[3-(3-Ethoxyphenyl)-5-(trifluoromethyl)benzoyl]piperazin-1-yl]-N-propylpyridazine-3-carboxamide |
| B1M090-01 | 4-[4-[4-(3-Ethoxyphenyl)-3-fluorobenzoyl]piperazin-1-yl]-N-propylbenzamide |
| B1M091-01 | 4-[4-[4-(5-Ethoxypyridin-3-yl)-3-fluorobenzoyl]piperazin-1-yl]-N-propylbenzamide |
| B1M092-01 | 4-[4-[3-(3-Ethoxyphenyl)-5-(trifluoromethyl)benzoyl]piperazin-1-yl]-N-propylbenzamide |
| B1M093-01 | 4-[4-[[4-(3-Ethoxyphenyl)-2-methoxyphenyl]methyl]piperazin-1-yl]-N-propylbenzamide |
| B1M094-01 | 6-[4-[[4-(3-Ethoxyphenyl)-2-methoxyphenyl]methyl]piperazin-1-yl]-N-propylpyridazine-3-carboxamide |
| B1M095-01 | 6-[4-[2-[2-(3-Ethoxyphenyl)ethynyl]benzoyl]piperazin-1-yl]-N-propylpyridazine-3-carboxamide |
| B1M096-01 | 6-[4-[2-[2-(5-Ethoxypyridin-3-yl)ethynyl]benzoyl]piperazin-1-yl]-N-propylpyridazine-3-carboxamide |
| B1M097-01 | 4-[4-[2-[2-(3-Ethoxyphenyl)ethynyl]benzoyl]piperazin-1-yl]-N-propylbenzamide |
| B1M098-01 | 4-[4-[2-[2-(5-Ethoxypyridin-3-yl)ethynyl]benzoyl]piperazin-1-yl]-N-propylbenzamide |
| B1M099-01 | 4-[4-[[4-(5-Ethoxypyridin-3-yl)-2-methoxyphenyl]methyl]piperazin-1-yl]-N-propylbenzamide |
| B1M100-01 | 6-[4-[[4-(5-Ethoxypyridin-3-yl)-2-methoxyphenyl]methyl]piperazin-1-yl]-N-propylpyridazine-3-carboxamide |

[Table 99]

[Table AI-03-B1K-01]

| Mixture No. | mixAI-B1K-01 |
|---|---|
| Compound No. that may be included | Compound name |
| B1K001-01 | 6-[4-[4-(3-Ethoxyphenyl)thiophene-2-carbonyl]piperazin-1-yl]-N-(2,2,2-trifluoroethyl)pyridazine-3-carboxamide |
| B1K002-01 | 6-[4-[4-(5-Ethoxypyridin-3-yl)thiophene-2-carbonyl]piperazin-1-yl]-N-(2,2,2-trifluoroethyl)pyridazine-3-carboxamide |
| B1K003-01 | 6-[4-[4-(3-Ethoxyphenyl)-3-methylbenzoyl]piperazin-1-yl]-N-(2,2,2-trifluoroethyl)pyridazine-3-carboxamide |
| B1K004-01 | 6-[4-[4-(5-Ethoxypyridin-3-yl)-3-methylbenzoyl]piperazin-1-yl]-N-(2,2,2-trifluoroethyl)pyridazine-3-carboxamide |
| B1K005-01 | 6-[4-[4-(3-Ethoxyphenyl)-3-fluorobenzoyl]piperazin-1-yl]-N-(2,2,2-trifluoroethyl)pyridazine-3-carboxamide |

(continued)

| Mixture No. | mixAI-B1K-01 |
|---|---|
| Compound No. that may be included | Compound name |
| B1K006-01 | 6-[4-[4-(5-Ethoxypyridin-3-yl)-3-fluorobenzoyl]piperazin-1-yl]-N-(2,2,2-trifluoroethyl)pyridazine-3-carboxamide |
| B1K007-01 | 6-[4-[2-[2-(3-Ethoxyphenyl)ethynyl]benzoyl]piperazin-1-yl]-N-(2,2,2-trifluoroethyl)pyridazine-3-carboxamide |
| B1K008-01 | 6-[4-[2-[2-(5-Ethoxypyridin-3-yl)ethynyl]benzoyl]piperazin-1-yl]-N-(2,2,2-trifluoroethyl)pyridazine-3-carboxamide |
| B1K009-01 | 6-[4-[5-[2-(3-Ethoxyphenyl)ethynyl]pyridine-3-carbonyl]piperazin-1-yl]-N-(2,2,2-trifluoroethyl)pyridazine-3-carboxamide |
| B1K010-01 | 6-[4-[5-[2-(5-Ethoxypyridin-3-yl)ethynyl]pyridine-3-carbonyl]piperazin-1-yl]-N-(2,2,2-trifluoroethyl)pyridazine-3-carboxamide |
| B1K011-01 | 6-[4-[3-[2-(3-Ethoxyphenyl)phenyl]propanoyl]piperazin-1-yl]-N-(2,2,2-trifluoroethyl)pyridazine-3-carboxamide |
| B1K012-01 | 6-[4-[3-[2-(5-Ethoxypyridin-3-yl)phenyl]propanoyl]piperazin-1-yl]-N-(2,2,2-trifluoroethyl)pyridazine-3-carboxamide |
| B1K013-01 | 6-[4-[4-(3-Ethoxyphenyl)naphthalene-1-carbonyl]piperazin-1-yl]-N-(2,2,2-trifluoroethyl)pyridazine-3-carboxamide |
| B1K014-01 | 6-[4-[4-(5-Ethoxypyridin-3-yl)naphthalene-1-carbonyl]piperazin-1-yl]-N-(2,2,2-trifluoroethyl)pyridazine-3-carboxamide |
| B1K015-01 | 6-[4-[3-tert-Butyl-5-(3-ethoxyphenyl)benzoyl]piperazin-1-yl]-N-(2,2,2-trifluoroethyl)pyridazine-3-carboxamide |
| B1K016-01 | 6-[4-[3-tert-Butyl-5-(5-ethoxypyridin-3-yl)benzoyl]piperazin-1-yl]-N-(2,2,2-trifluoroethyl)pyridazine-3-carboxamide |
| B1K017-01 | 6-[4-[3-(3-Ethoxyphenyl)-5-(trifluoromethyl)benzoyl]piperazin-1-yl]-N-(2,2,2-trifluoroethyl)pyridazine-3-carboxamide |
| B1K018-01 | 6-[4-[3-(5-Ethoxypyridin-3-yl)-5-(trifluoromethyl)benzoyl]piperazin-1-yl]-N-(2,2,2-trifluoroethyl)pyridazine-3-carboxamide |
| B1K019-01 | 6-[4-[3-[2-(3-Ethoxyphenyl)ethynyl]-5-(trifluoromethyl)benzoyl]piperazin-1-yl]-N-(2,2,2-trifluoroethyl)pyridazine-3-carboxamide |
| B1K020-01 | 6-[4-[3-[2-(5-Ethoxypyridin-3-yl)ethynyl]-5-(trifluoromethyl)benzoyl]piperazin-1-yl]-N-(2,2,2-trifluoroethyl)pyridazine-3-carboxamide |
| B1K021-01 | 6-[4-[3-[4-(3-Ethoxyphenyl)pyrazol-1-yl]-5-(trifluoromethyl)benzoyl]piperazin-1-yl]-N-(2,2,2-trifluoroethyl)pyridazine-3-carboxamide |
| B1K022-01 | 6-[4-[3-[4-(5-Ethoxypyridin-3-yl)pyrazol-1-yl]-5-(trifluoromethyl)benzoyl]piperazin-1-yl]-N-(2,2,2-trifluoroethyl)pyridazine-3-carboxamide |
| B1K023-01 | 4-[4-[4-(3-Ethoxyphenyl)thiophene-2-carbonyl]piperazin-1-yl]-N-(2,2,2-trifluoroethyl)benzamide |
| B1K024-01 | 4-[4-[4-(5-Ethoxypyridin-3-yl)thiophene-2-carbonyl]piperazin-1-yl]-N-(2,2,2-trifluoroethyl)benzamide |
| B1K025-01 | 4-[4-[4-(3-Ethoxyphenyl)-3-methylbenzoyl]piperazin-1-yl]-N-(2,2,2-trifluoroethyl)benzamide |
| B1K026-01 | 4-[4-[4-(5-Ethoxypyridin-3-yl)-3-methylbenzoyl]piperazin-1-yl]-N-(2,2,2-trifluoroethyl)benzamide |

(continued)

| Mixture No. | mixAI-B1K-01 |
|---|---|
| Compound No. that may be included | Compound name |
| B1K027-01 | 4-[4-[4-(3-Ethoxyphenyl)-3-fluorobenzoyl]piperazin-1-yl]-N-(2,2,2-trifluoroethyl)benzamide |
| B1K028-01 | 4-[4-[4-(5-Ethoxypyridin-3-yl)-3-fluorobenzoyl]piperazin-1-yl]-N-(2,2,2-trifluoroethyl)benzamide |
| B1K029-01 | 4-[4-[2-[2-(3-Ethoxyphenyl)ethynyl]benzoyl]piperazin-1-yl]-N-(2,2,2-trifluoroethyl)benzamide |
| B1K030-01 | 4-[4-[2-[2-(5-Ethoxypyridin-3-yl)ethynyl]benzoyl]piperazin-1-yl]-N-(2,2,2-trifluoroethyl)benzamide |
| B1K031-01 | 4-[4-[5-[2-(3-Ethoxyphenyl)ethynyl]pyridine-3-carbonyl]piperazin-1-yl]-N-(2,2,2-trifluoroethyl)benzamide |
| B1K032-01 | 4-[4-[5-[2-(5-Ethoxypyridin-3-yl)ethynyl]pyridine-3-carbonyl]piperazin-1-yl]-N-(2,2,2-trifluoroethyl)benzamide |
| B1K033-01 | 4-[4-[3-[2-(3-Ethoxyphenyl)phenyl]propanoyl]piperazin-1-yl]-N-(2,2,2-trifluoroethyl)benzamide |
| B1K034-01 | 4-[4-[3-[2-(5-Ethoxypyridin-3-yl)phenyl]propanoyl]piperazin-1-yl]-N-(2,2,2-trifluoroethyl)benzamide |
| B1K035-01 | 4-[4-[4-(3-Ethoxyphenyl)naphthalene-1-carbonyl]piperazin-1-yl]-N-(2,2,2-trifluoroethyl)benzamide |
| B1K036-01 | 4-[4-[4-(5-Ethoxypyridin-3-yl)naphthalene-1-carbonyl]piperazin-1-yl]-N-(2,2,2-trifluoroethyl)benzamide |
| B1K037-01 | 4-[4-[3-tert-Butyl-5-(3-ethoxyphenyl)benzoyl]piperazin-1-yl]-N-(2,2,2-trifluoroethyl)benzamide |
| B1K038-01 | 4-[4-[3-tert-Butyl-5-(5-ethoxypyridin-3-yl)benzoyl]piperazin-1-yl]-N-(2,2,2-trifluoroethyl)benzamide |
| B1K039-01 | 4-[4-[3-(3-Ethoxyphenyl)-5-(trifluoromethyl)benzoyl]piperazin-1-yl]-N-(2,2,2-trifluoroethyl)benzamide |
| B1K040-01 | 4-[4-[3-(5-Ethoxypyridin-3-yl)-5-(trifluoromethyl)benzoyl]piperazin-1-yl]-N-(2,2,2-trifluoroethyl)benzamide |
| B1K041-01 | 4-[4-[3-[2-(3-Ethoxyphenyl)ethynyl]-5-(trifluoromethyl)benzoyl]piperazin-1-yl]-N-(2,2,2-trifluoroethyl)benzamide |
| B1K042-01 | 4-[4-[3-[2-(5-Ethoxypyridin-3-yl)ethynyl]-5-(trifluoromethyl)benzoyl]piperazin-1-yl]-N-(2,2,2-trifluoroethyl)benzamide |
| B1K043-01 | 4-[4-[3-[4-(3-Ethoxyphenyl)pyrazol-1-yl]-5-(trifluoromethyl)benzoyl]piperazin-1-yl]-N-(2,2,2-trifluoroethyl)benzamide |
| B1K044-01 | 4-[4-[3-[4-(5-Ethoxypyridin-3-yl)pyrazol-1-yl]-5-(trifluoromethyl)benzoyl]piperazin-1-yl]-N-(2,2,2-trifluoroethyl)benzamide |
| B1K045-01 | 4-[4-[[2-(3-Ethoxyphenyl)phenyl]methyl]piperazin-1-yl]-N-(2,2,2-trifluoroethyl)benzamide |
| B1K046-01 | 4-[4-[[2-(5-Ethoxypyridin-3-yl)phenyl]methyl]piperazin-1-yl]-N-(2,2,2-trifluoroethyl)benzamide |
| B1K047-01 | 4-[4-[[4-(3-Ethoxyphenyl)-3-methylphenyl]methyl]piperazin-1-yl]-N-(2,2,2-trifluoroethyl)benzamide |

(continued)

| Mixture No. | mixAI-B1K-01 |
|---|---|
| Compound No. that may be included | Compound name |
| B1K048-01 | 4-[4-[[4-(5-Ethoxypyridin-3-yl)-3-methylphenyl]methyl]piperazin-1-yl]-N-(2,2,2-trifluoroethyl)benzamide |
| B1K049-01 | 4-[4-[[4-(3-Ethoxyphenyl)-2-ethylphenyl]methyl]piperazin-1-yl]-N-(2,2,2-trifluoroethyl)benzamide |
| B1K050-01 | 4-[4-[[4-(5-Ethoxypyridin-3-yl)-2-ethylphenyl]methyl]piperazin-1-yl]-N-(2,2,2-trifluoroethyl)benzamide |
| B1K051-01 | 4-[4-[[4-(3-Ethoxyphenyl)-3-fluorophenyl]methyl]piperazin-1-yl]-N-(2,2,2-trifluoroethyl)benzamide |
| B1K052-01 | 4-[4-[[4-(5-Ethoxypyridin-3-yl)-3-fluorophenyl]methyl]piperazin-1-yl]-N-(2,2,2-trifluoroethyl)benzamide |
| B1K053-01 | 4-[4-[[4-(3-Ethoxyphenyl)-2-methoxyphenyl]methyl]piperazin-1-yl]-N-(2,2,2-trifluoroethyl)benzamide |
| B1K054-01 | 4-[4-[[4-(5-Ethoxypyridin-3-yl)-2-methoxyphenyl]methyl]piperazin-1-yl]-N-(2,2,2-trifluoroethyl)benzamide |
| B1K055-01 | 4-[4-[[3-Ethoxy-5-(3-ethoxyphenyl)phenyl]methyl]piperazin-1-yl]-N-(2,2,2-trifluoroethyl)benzamide |
| B1K056-01 | 4-[4-[[3-Ethoxy-5-(5-ethoxypyridin-3-yl)phenyl]methyl]piperazin-1-yl]-N-(2,2,2-trifluoroethyl)benzamide |
| B1K057-01 | 4-[4-[[3-(3-Ethoxyphenyl)-5-propan-2-yloxyphenyl]methyl]piperazin-1-yl]-N-(2,2,2-trifluoroethyl)benzamide |
| B1K058-01 | 4-[4-[[3-(5-Ethoxypyridin-3-yl)-5-propan-2-yloxyphenyl]methyl]piperazin-1-yl]-N-(2,2,2-trifluoroethyl)benzamide |
| B1K059-01 | 4-[4-[[4-(3-Ethoxyphenyl)-2,6-dimethoxyphenyl]methyl]piperazin-1-yl]-N-(2,2,2-trifluoroethyl)benzamide |
| B1K060-01 | 4-[4-[[4-(5-Ethoxypyridin-3-yl)-2,6-dimethoxyphenyl]methyl]piperazin-1-yl]-N-(2,2,2-trifluoroethyl)benzamide |
| B1K061-01 | 4-[4-[[3-(3-Ethoxyphenyl)-5-(trifluoromethyl)phenyl]methyl]piperazin-1-yl]-N-(2,2,2-trifluoroethyl)benzamide |
| B1K062-01 | 4-[4-[[3-(5-Ethoxypyridin-3-yl)-5-(trifluoromethyl)phenyl]methyl]piperazin-1-yl]-N-(2,2,2-trifluoroethyl)benzamide |
| B1K063-01 | 4-[4-[[3-(3-Ethoxyphenyl)-5-(trifluoromethoxy)phenyl]methyl]piperazin-1-yl]-N-(2,2,2-trifluoroethyl)benzamide |
| B1K064-01 | 4-[4-[[3-(5-Ethoxypyridin-3-yl)-5-(trifluoromethoxy)phenyl]methyl]piperazin-1-yl]-N-(2,2,2-trifluoroethyl)benzamide |
| B1K065-01 | 4-[4-[[4-(3-Ethoxyphenyl)naphthalen-1-yl]methyl]piperazin-1-yl]-N-(2,2,2-trifluoroethyl)benzamide |
| B1K066-01 | 4-[4-[[4-(5-Ethoxypyridin-3-yl)naphthalen-1-yl]methyl]piperazin-1-yl]-N-(2,2,2-trifluoroethyl)benzamide |
| BIK067-01 | 4-[4-[[4-(3-Ethoxyphenyl)thiophen-2-yl]methyl]piperazin-1-yl]-N-(2,2,2-trifluoroethyl)benzamide |
| B1K068-01 | 4-[4-[[4-(5-Ethoxypyridin-3-yl)thiophen-2-yl]methyl]piperazin-1-yl]-N-(2,2,2-trifluoroethyl)benzamide |

(continued)

| Mixture No. | mixAI-B1K-01 |
|---|---|
| Compound No. that may be included | Compound name |
| B1K069-01 | 4-[4-[[2-[2-(3-Ethoxyphenyl)ethynyl]phenyl]methyl]piperazin-1-yl]-N-(2,2,2-trifluoroethyl)benzamide |
| B1K070-01 | 4-[4-[[2-[2-(5-Ethoxypyridin-3-yl)ethynyl]phenyl]methyl]piperazin-1-yl]-N-(2,2,2-trifluoroethyl)benzamide |
| B1K071-01 | 6-[4-[[2-(3-Ethoxyphenyl)phenyl]methyl]piperazin-1-yl]-N-(2,2,2-trifluoroethyl)pyridazine-3-carboxamide |
| B1K072-01 | 6-[4-[[2-(5-Ethoxypyridin-3-yl)phenyl]methyl]piperazin-1-yl]-N-(2,2,2-trifluoroethyl)pyridazine-3-carboxamide |
| BIK073-01 | 6-[4-[[4-(3-Ethoxyphenyl)-3-methylphenyl]methyl]piperazin-1-yl]-N-(2,2,2-trifluoroethyl)pyridazine-3-carboxamide |
| B1K074-01 | 6-[4-[[4-(5-Ethoxypyridin-3-yl)-3-methylphenyl]methyl]piperazin-1-yl]-N-(2,2,2-trifluoroethyl)pyridazine-3-carboxamide |
| B1K075-01 | 6-[4-[[4-(3-Ethoxyphenyl)-2-ethylphenyl]methyl]piperazin-1-yl]-N-(2,2,2-trifluoroethyl)pyridazine-3-carboxamide |
| B1K076-01 | 6-[4-[[4-(5-Ethoxypyridin-3-yl)-2-ethylphenyl]methyl]piperazin-1-yl]-N-(2,2,2-trifluoroethyl)pyridazine-3-carboxamide |
| B1K077-01 | 6-[4-[[4-(3-Ethoxyphenyl)-3-fluorophenyl]methyl]piperazin-1-yl]-N-(2,2,2-trifluoroethyl)pyridazine-3-carboxamide |
| B1K078-01 | 6-[4-[[4-(5-Ethoxypyridin-3-yl)-3-fluorophenyl]methyl]piperazin-1-yl]-N-(2,2,2-trifluoroethyl)pyridazine-3-carboxamide |
| B1K079-01 | 6-[4-[[4-(3-Ethoxyphenyl)-2-methoxyphenyl]methyl]piperazin-1-yl]-N-(2,2,2-trifluoroethyl)pyridazine-3-carboxamide |
| B1K080-01 | 6-[4-[[4-(5-Ethoxypyridin-3-yl)-2-methoxyphenyl]methyl]piperazin-1-yl]-N-(2,2,2-trifluoroethyl)pyridazine-3-carboxamide |
| B1K081-01 | 6-[4-[[3-Ethoxy-5-(3-ethoxyphenyl)phenyl]methyl]piperazin-1-yl]-N-(2,2,2-trifluoroethyl)pyridazine-3-carboxamide |
| B1K082-01 | 6-[4-[[3-Ethoxy-5-(5-ethoxypyridin-3-yl)phenyl]methyl]piperazin-1-yl]-N-(2,2,2-trifluoroethyl)pyridazine-3-carboxamide |
| B1K083-01 | 6-[4-[[3-(3-Ethoxyphenyl)-5-propan-2-yloxyphenyl]methyl]piperazin-1-yl]-N-(2,2,2-trifluoroethyl)pyridazine-3-carboxamide |
| B1K084-01 | 6-[4-[[3-(5-Ethoxypyridin-3-yl)-5-propan-2-yloxyphenyl]methyl]piperazin-1-yl]-N-(2,2,2-trifluoroethyl)pyridazine-3-carboxamide |
| B1K085-01 | 6-[4-[[4-(3-Ethoxyphenyl)-2,6-dimethoxyphenyl]methyl]piperazin-1-yl]-N-(2,2,2-trifluoroethyl)pyridazine-3-carboxamide |
| B1K086-01 | 6-[4-[[4-(5-Ethoxypyridin-3-yl)-2,6-dimethoxyphenyl]methyl]piperazin-1-yl]-N-(2,2,2-trifluoroethyl)pyridazine-3-carboxamide |
| B1K087-01 | 6-[4-[[3-(3-Ethoxyphenyl)-5-(trifluoromethyl)phenyl]methyl]piperazin-1-yl]-N-(2,2,2-trifluoroethyl)pyridazine-3-carboxamide |
| B1K088-01 | 6-[4-[[3-(5-Ethoxypyridin-3-yl)-5-(trifluoromethyl)phenyl]methyl]piperazin-1-yl]-N-(2,2,2-trifluoroethyl)pyridazine-3-carboxamide |
| B1K089-01 | 6-[4-[[3-(3-Ethoxyphenyl)-5-(trifluoromethoxy)phenyl]methyl]piperazin-1-yl]-N-(2,2,2-trifluoroethyl)pyridazine-3-carboxamide |

(continued)

| Mixture No. | mixAI-B1K-01 |
|---|---|
| Compound No. that may be included | Compound name |
| B1K090-01 | 6-[4-[[3-(5-Ethoxypyridin-3-yl)-5-(trifluoromethoxy)phenyl] methyl]piperazin-1-yl]-N-(2,2,2-trifluoroethyl)pyridazine-3-carboxamide |
| B1K091-01 | 6-[4-[[4-(3-Ethoxyphenyl)naphthalen-1-yl]methyl]piperazin-1-yl]-N-(2,2,2-trifluoroethyl)pyridazine-3-carboxamide |
| B1K092-01 | 6-[4-[[4-(5-Ethoxypyridin-3-yl)naphthalen-1-yl]methyl]piperazin-1-yl]-N-(2,2,2-trifluoroethyl)pyridazine-3-carboxamide |
| B1K093-01 | 6-[4-[[4-(3-Ethoxyphenyl)thiophen-2-yl]methyl]piperazin-1-yl]-N-(2,2,2-trifluoroethyl)pyridazine-3-carboxamide |
| B1K094-01 | 6-[4-[[4-(5-Ethoxypyridin-3-yl)thiophen-2-yl]methyl]piperazin-1-yl]-N-(2,2,2-trifluoroethyl)pyridazine-3-carboxamide |
| B1K095-01 | 6-[4-[[2-[2-(3-Ethoxyphenyl)ethynyl]phenyl]methyl]piperazin-1-yl]-N-(2,2,2-trifluoroethyl)pyridazine-3-carboxamide |
| B1K096-01 | 6-[4-[[2-[2-(5-Ethoxypyridin-3-yl)ethynyl]phenyl]methyl]piperazin-1-yl]-N-(2,2,2-trifluoroethyl)pyridazine-3-carboxamide |
| B1K097-01 | 4-[4-[1-[4-(3-Ethoxyphenyl)phenyl]ethyl]piperazin-1-yl]-N-(2,2,2-trifluoroethyl)benzamide |
| B1K098-01 | 4-[4-[1-[4-(5-Ethoxypyridin-3-yl)phenyl]ethyl]piperazin-1-yl]-N-(2,2,2-trifluoroethyl)benzamide |
| B1K099-01 | 4-[4-[1-[4-(3-Ethoxyphenyl)-2-fluorophenyl]ethyl]piperazin-1-yl]-N-(2,2,2-trifluoroethyl)benzamide |
| B1K100-01 | 4-[4-[1-[4-(5-Ethoxypyridin-3-yl)-2-fluorophenyl]ethyl]piperazin-1-yl]-N-(2,2,2-trifluoroethyl)benzamide |

[Table 100]

[Table AI-03-B1O-01]

| Mixture No. | mixAI-B 10-01 |
|---|---|
| Compound No. that may be included | Compound name |
| B1O001-01 | 6-[4-[4-(3-Ethoxyphenyl)thiophene-2-carbonyl]piperazin-1-yl]-N-(4-methoxyphenyl)pyridazine-3-carboxamide |
| B1O002-01 | 6-[4-[4-(5-Ethoxypyridin-3-yl)thiophene-2-carbonyl]piperazin-1-yl]-N-(4-methoxyphenyl)pyridazine-3-carboxamide |
| B1O003-01 | 6-[4-[4-(3-Ethoxyphenyl)-3-methylbenzoyl]piperazin-1-yl]-N-(4-methoxyphenyl)pyridazine-3-carboxamide |
| B1O004-01 | 6-[4-[4-(5-Ethoxypyridin-3-yl)-3-methylbenzoyl]piperazin-1-yl]-N-(4-methoxyphenyl)pyridazine-3-carboxamide |
| B1O005-01 | 6-[4-[4-(3-Ethoxyphenyl)-3-fluorobenzoyl]piperazin-1-yl]-N-(4-methoxyphenyl)pyridazine-3-carboxamide |
| B1O006-01 | 6-[4-[4-(5-Ethoxypyridin-3-yl)-3-fluorobenzoyl]piperazin-1-yl]-N-(4-methoxyphenyl)pyridazine-3-carboxamide |

(continued)

| Mixture No. | mixAI-B 10-01 |
|---|---|
| Compound No. that may be included | Compound name |
| B1O007-01 | 6-[4-[2-[2-(3-Ethoxyphenyl)ethynyl]benzoyl]piperazin-1-yl]-N-(4-methoxyphenyl)pyridazine-3-carboxamide |
| B1O008-01 | 6-[4-[2-[2-(5-Ethoxypyridin-3-yl)ethynyl]benzoyl]piperazin-1-yl]-N-(4-methoxyphenyl)pyridazine-3-carboxamide |
| B1O009-01 | 6-[4-[5-[2-(3-Ethoxyphenyl)ethynyl]pyridine-3-carbonyl]piperazin-1-yl]-N-(4-methoxyphenyl)pyridazine-3-carboxamide |
| B1O010-01 | 6-[4-[5-[2-(5-Ethoxypyridin-3-yl)ethynyl]pyridine-3-carbonyl]piperazin-1-yl]-N-(4-methoxyphenyl)pyridazine-3-carboxamide |
| B1O011-01 | 6-[4-[3-[2-(3-Ethoxyphenyl)phenyl]propanoyl]piperazin-1-yl]-N-(4-methoxyphenyl)pyridazine-3-carboxamide |
| B1O012-01 | 6-[4-[3-[2-(5-Ethoxypyridin-3-yl)phenyl]propanoyl]piperazin-1-yl]-N-(4-methoxyphenyl)pyridazine-3-carboxamide |
| B1O013-01 | 6-[4-[4-(3-Ethoxyphenyl)naphthalene-1-carbonyl]piperazin-1-yl]-N-(4-methoxyphenyl)pyridazine-3-carboxamide |
| B1O014-01 | 6-[4-[4-(5-Ethoxypyridin-3-yl)naphthalene-1-carbonyl]piperazin-1-yl]-N-(4-methoxyphenyl)pyridazine-3-carboxamide |
| B1O015-01 | 6-[4-[3-tert-Butyl-5-(3-ethoxyphenyl)benzoyl]piperazin-1-yl]-N-(4-methoxyphenyl)pyridazine-3-carboxamide |
| B1O016-01 | 6-[4-[3-tert-Butyl-5-(5-ethoxypyridin-3-yl)benzoyl]piperazin-1-yl]-N-(4-methoxyphenyl)pyridazine-3-carboxamide |
| B1O017-01 | 6-[4-[3-(3-Ethoxyphenyl)-5-(trifluoromethyl)benzoyl]piperazin-1-yl]-N-(4-methoxyphenyl)pyridazine-3-carboxamide |
| B1O018-01 | 6-[4-[3-(5-Ethoxypyridin-3-yl)-5-(trifluoromethyl)benzoyl]piperazin-1-yl]-N-(4-methoxyphenyl)pyridazine-3-carboxamide |
| B1O019-01 | 6-[4-[3-[2-(3-Ethoxyphenyl)ethynyl]-5-(trifluoromethyl)benzoyl]piperazin-1-yl]-N-(4-methoxyphenyl)pyridazine-3-carboxamide |
| B1O020-01 | 6-[4-[3-[2-(5-Ethoxypyridin-3-yl)ethynyl]-5-(trifluoromethyl)benzoyl]piperazin-1-yl]-N-(4-methoxyphenyl)pyridazine-3-carboxamide |
| B1O021-01 | 6-[4-[3-[4-(3-Ethoxyphenyl)pyrazol-1-yl]-5-(trifluoromethyl)benzoyl]piperazin-1-yl]-N-(4-methoxyphenyl)pyridazine-3-carboxamide |
| B1O022-01 | 6-[4-[3-[4-(5-Ethoxypyridin-3-yl)pyrazol-1-yl]-5-(trifluoromethyl)benzoyl]piperazin-1-yl]-N-(4-methoxyphenyl)pyridazine-3-carboxamide |
| B1O023-01 | 4-[4-[4-(3-Ethoxyphenyl)thiophene-2-carbonyl]piperazin-1-yl]-N-(4-methoxyphenyl)benzamide |
| B1O024-01 | 4-[4-[4-(5-Ethoxypyridin-3-yl)thiophene-2-carbonyl]piperazin-1 -yl]-N-(4-methoxyphenyl)benzamide |
| B1O025-01 | 4-[4-[4-(3-Ethoxyphenyl)-3-methylbenzoyl]piperazin-1-yl]-N-(4-methoxyphenyl)benzamide |
| B1O026-01 | 4-[4-[4-(5-Ethoxypyridin-3-yl)-3-methylbenzoyl]piperazin-1-yl]-N-(4-methoxyphenyl)benzamide |
| B1O027-01 | 4-[4-[4-(3-Ethoxyphenyl)-3-fluorobenzoyl]piperazin-1-yl]-N-(4-methoxyphenyl)benzamide |

(continued)

| Mixture No. | mixAI-B 10-01 |
|---|---|
| Compound No. that may be included | Compound name |
| B1O028-01 | 4-[4-[4-(5-Ethoxypyridin-3-yl)-3-fluorobenzoyl]piperazin-1-yl]-N-(4-methoxyphenyl)benzamide |
| B1O029-01 | 4-[4-[2-[2-(3-Ethoxyphenyl)ethynyl]benzoyl]piperazin-1-yl]-N-(4-methoxyphenyl)benzamide |
| B1O030-01 | 4-[4-[2-[2-(5-Ethoxypyridin-3-yl)ethynyl]benzoyl]piperazin-1-yl]-N-(4-methoxyphenyl)benzamide |
| B1O031-01 | 4-[4-[5-[2-(3-Ethoxyphenyl)ethynyl]pyridine-3-carbonyl]piperazin-1-yl]-N-(4-methoxyphenyl)benzamide |
| B1O032-01 | 4-[4-[5-[2-(5-Ethoxypyridin-3-yl)ethynyl]pyridine-3-carbonyl]piperazin-1-yl]-N-(4-methoxyphenyl)benzamide |
| B1O033-01 | 4-[4-[3-[2-(3-Ethoxyphenyl)phenyl]propanoyl]piperazin-1-yl]-N-(4-methoxyphenyl)benzamide |
| B1O034-01 | 4-[4-[3-[2-(5-Ethoxypyridin-3-yl)phenyl]propanoyl]piperazin-1-yl]-N-(4-methoxyphenyl)benzamide |
| B1O035-01 | 4-[4-[4-(3-Ethoxyphenyl)naphthalene-1-carbonyl]piperazin-1-yl]-N-(4-methoxyphenyl)benzamide |
| B1O036-01 | 4-[4-[4-(5-Ethoxypyridin-3-yl)naphthalene-1-carbonyl]piperazin-1-yl]-N-(4-methoxyphenyl)benzamide |
| B1O037-01 | 4-[4-[3-tert-Butyl-5-(3-ethoxyphenyl)benzoyl]piperazin-1-yl]-N-(4-methoxyphenyl)benzamide |
| B1O038-01 | 4-[4-[3-tert-Butyl-5-(5-ethoxypyridin-3-yl)benzoyl]piperazin-1-yl]-N-(4-methoxyphenyl)benzamide |
| B1O039-01 | 4-[4-[3-(3-Ethoxyphenyl)-5-(trifluoromethyl)benzoyl]piperazin-1-yl]-N-(4-methoxyphenyl)benzamide |
| B1O040-01 | 4-[4-[3-(5-Ethoxypyridin-3-yl)-5-(trifluoromethyl)benzoyl]piperazin-1-yl]-N-(4-methoxyphenyl)benzamide |
| B1O041-01 | 4-[4-[3-[2-(3-Ethoxyphenyl)ethynyl]-5-(trifluoromethyl)benzoyl]piperazin-1-yl]-N-(4-methoxyphenyl)benzamide |
| B1O042-01 | 4-[4-[3-[2-(5-Ethoxypyridin-3-yl)ethynyl]-5-(trifluoromethyl)benzoyl]piperazin-1-yl]-N-(4-methoxyphenyl)benzamide |
| B1O043-01 | 4-[4-[3-[4-(3-Ethoxyphenyl)pyrazol-1-yl]-5-(trifluoromethyl)benzoyl]piperazin-1-yl]-N-(4-methoxyphenyl)benzamide |
| B1O044-01 | 4-[4-[3-[4-(5-Ethoxypyridin-3-yl)pyrazol-1-yl]-5-(trifluoromethyl)benzoyl]piperazin-1-yl]-N-(4-methoxyphenyl)benzamide |
| B1O045-01 | 4-[4-[[2-(3-Ethoxyphenyl)phenyl]methyl]piperazin-1-yl]-N-(4-methoxyphenyl)benzamide |
| B1O046-01 | 4-[4-[[2-(5-Ethoxypyridin-3-yl)phenyl]methyl]piperazin-1-yl]-N-(4-methoxyphenyl)benzamide |
| B1O047-01 | 4-[4-[[4-(3-Ethoxyphenyl)-3-methylphenyl]methyl]piperazin-1-yl]-N-(4-methoxyphenyl)benzamide |
| B1O048-01 | 4-[4-[[4-(5 -Ethoxypyridin-3 -yl)-3 -methylphenyl]methyl]piperazin-1 -yl] -N-(4-methoxyphenyl)benzamide |

(continued)

| Mixture No. | mixAI-B 10-01 |
|---|---|
| Compound No. that may be included | Compound name |
| B1O049-01 | 4-[4-[[4-(3-Ethoxyphenyl)-2-ethylphenyl]methyl]piperazin-1-yl]-N-(4-methoxyphenyl)benzamide |
| B1O050-01 | 4-[4-[[4-(5-Ethoxypyridin-3-yl)-2-ethylphenyl]methyl]piperazin-1-yl]-N-(4-methoxyphenyl)benzamide |
| B1O051-01 | 4-[4-[[4-(3-Ethoxyphenyl)-3-fluorophenyl]methyl]piperazin-1-yl]-N-(4-methoxyphenyl)benzamide |
| B1O052-01 | 4-[4-[[4-(5-Ethoxypyridin-3-yl)-3-fluorophenyl]methyl]piperazin-1-yl]-N-(4-methoxyphenyl)benzamide |
| B1O053-01 | 4-[4-[[4-(3-Ethoxyphenyl)-2-methoxyphenyl]methyl]piperazin-1-yl]-N-(4-methoxyphenyl)benzamide |
| B1O054-01 | 4-[4-[[4-(5-Ethoxypyridin-3-yl)-2-methoxyphenyl]methyl]piperazin-1-yl]-N-(4-methoxyphenyl)benzamide |
| B1O055-01 | 4-[4-[[3-Ethoxy-5-(3-ethoxyphenyl)phenyl]methyl]piperazin-1-yl]-N-(4-methoxyphenyl)benzamide |
| B1O056-01 | 4-[4-[[3-Ethoxy-5-(5-ethoxypyridin-3-yl)phenyl]methyl]piperazin-1-yl]-N-(4-methoxyphenyl)benzamide |
| B1O057-01 | 4-[4-[[3-(3-Ethoxyphenyl)-5-propan-2-yloxyphenyl]methyl]piperazin-1-yl]-N-(4-methoxyphenyl)benzamide |
| B1O058-01 | 4-[4-[[3-(5-Ethoxypyridin-3-yl)-5-propan-2-yloxyphenyl]methyl]piperazin-1-yl]-N-(4-methoxyphenyl)benzamide |
| B1O059-01 | 4-[4-[[4-(3-Ethoxyphenyl)-2,6-dimethoxyphenyl]methyl]piperazin-1-yl]-N-(4-methoxyphenyl)benzamide |
| B1O060-01 | 4-[4-[[4-(5-Ethoxypyridin-3-yl)-2,6-dimethoxyphenyl]methyl]piperazin-1-yl]-N-(4-methoxyphenyl)benzamide |
| B1O061-01 | 4-[4-[[3-(3-Ethoxyphenyl)-5-(trifluoromethyl)phenyl]methyl]piperazin-1-yl]-N-(4-methoxyphenyl)benzamide |
| B1O062-01 | 4-[4-[[3-(5-Ethoxypyridin-3-yl)-5-(trifluoromethyl)phenyl]methyl]piperazin-1-yl]-N-(4-methoxyphenyl)benzamide |
| B1O063-01 | 4-[4-[[3-(3-Ethoxyphenyl)-5-(trifluoromethoxy)phenyl]methyl]piperazin-1-yl]-N-(4-methoxyphenyl)benzamide |
| B1O064-01 | 4-[4-[[3-(5-Ethoxypyridin-3-yl)-5-(trifluoromethoxy)phenyl]methyl]piperazin-1-yl]-N-(4-methoxyphenyl)benzamide |
| B1O065-01 | 4-[4-[[4-(3-Ethoxyphenyl)naphthalen-1-yl]methyl]piperazin-1-yl]-N-(4-methoxyphenyl)benzamide |
| B1O066-01 | 4-[4-[[4-(5-Ethoxypyridin-3-yl)naphthalen-1-yl]methyl]piperazin-1-yl]-N-(4-methoxyphenyl)benzamide |
| B1O067-01 | 4-[4-[[4-(3-Ethoxyphenyl)thiophen-2-yl]methyl]piperazin-1-yl]-N-(4-methoxyphenyl)benzamide |
| B1O068-01 | 4-[4-[[4-(5-Ethoxypyridin-3-yl)thiophen-2-yl]methyl]piperazin-1-yl]-N-(4-methoxyphenyl)benzamide |
| B1O069-01 | 4-[4-[[2-[2-(3-Ethoxyphenyl)ethynyl]phenyl]methyl]piperazin-1-yl]-N-(4-methoxyphenyl)benzamide |

(continued)

| Mixture No. | mixAI-B 10-01 |
|---|---|
| Compound No. that may be included | Compound name |
| B1O070-01 | 4-[4-[[2-[2-(5-Ethoxypyridin-3-yl)ethynyl]phenyl]methyl]piperazin-1-yl]-N-(4-methoxyphenyl)benzamide |
| B1O071-01 | 6-[4-[[2-(3-Ethoxyphenyl)phenyl]methyl]piperazin-1-yl]-N-(4-methoxyphenyl)pyridazine-3-carboxamide |
| B1O072-01 | 6-[4-[[2-(5-Ethoxypyridin-3-yl)phenyl]methyl]piperazin-1-yl]-N-(4-methoxyphenyl)pyridazine-3-carboxamide |
| B1O073-01 | 6-[4-[[4-(3-Ethoxyphenyl)-3-methylphenyl]methyl]piperazin-1-yl]-N-(4-methoxyphenyl)pyridazine-3-carboxamide |
| B1O074-01 | 6-[4-[[4-(5-Ethoxypyridin-3-yl)-3-methylphenyl]methyl]piperazin-1-yl]-N-(4-methoxyphenyl)pyridazine-3-carboxamide |
| B1O075-01 | 6-[4-[[4-(3-Ethoxyphenyl)-2-ethylphenyl]methyl]piperazin-1-yl]-N-(4-methoxyphenyl)pyridazine-3-carboxamide |
| B1O076-01 | 6-[4-[[4-(5-Ethoxypyridin-3-yl)-2-ethylphenyl]methyl]piperazin-1-yl]-N-(4-methoxyphenyl)pyridazine-3-carboxamide |
| B1O077-01 | 6-[4-[[4-(3-Ethoxyphenyl)-3-fluorophenyl]methyl]piperazin-1-yl]-N-(4-methoxyphenyl)pyridazine-3-carboxamide |
| B1O078-01 | 6-[4-[[4-(5-Ethoxypyridin-3-yl)-3-fluorophenyl]methyl]piperazin-1-yl]-N-(4-methoxyphenyl)pyridazine-3-carboxamide |
| B1O079-01 | 6-[4-[[4-(3-Ethoxyphenyl)-2-methoxyphenyl]methyl]piperazin-1-yl]-N-(4-methoxyphenyl)pyridazine-3-carboxamide |
| B1O080-01 | 6-[4-[[4-(5-Ethoxypyridin-3-yl)-2-methoxyphenyl]methyl]piperazin-1-yl]-N-(4-methoxyphenyl)pyridazine-3-carboxamide |
| B1O081-01 | 6-[4-[[3-Ethoxy-5-(3-ethoxyphenyl)phenyl]methyl]piperazin-1-yl]-N-(4-methoxyphenyl)pyridazine-3-carboxamide |
| B1O082-01 | 6-[4-[[3-Ethoxy-5-(5-ethoxypyridin-3-yl)phenyl]methyl]piperazin-1-yl]-N-(4-methoxyphenyl)pyridazine-3-carboxamide |
| B1O083-01 | 6-[4-[[3-(3-Ethoxyphenyl)-5-propan-2-yloxyphenyl]methyl]piperazin-1-yl]-N-(4-methoxyphenyl)pyridazine-3-carboxamide |
| B1O084-01 | 6-[4-[[3-(5-Ethoxypyridin-3-yl)-5-propan-2-yloxyphenyl]methyl]piperazin-1-yl]-N-(4-methoxyphenyl)pyridazine-3-carboxamide |
| B1O085-01 | 6-[4-[[4-(3-Ethoxyphenyl)-2,6-dimethoxyphenyl]methyl]piperazin-1-yl]-N-(4-methoxyphenyl)pyridazine-3-carboxamide |
| B1O086-01 | 6-[4-[[4-(5-Ethoxypyridin-3-yl)-2,6-dimethoxyphenyl]methyl]piperazin-1-yl]-N-(4-methoxyphenyl)pyridazine-3-carboxamide |
| B1O087-01 | 6-[4-[[3-(3-Ethoxyphenyl)-5-(trifluoromethyl)phenyl]methyl]piperazin-1-yl]-N-(4-methoxyphenyl)pyridazine-3-carboxamide |
| B1O088-01 | 6-[4-[[3-(5-Ethoxypyridin-3-yl)-5-(trifluoromethyl)phenyl]methyl]piperazin-1-yl]-N-(4-methoxyphenyl)pyridazine-3-carboxamide |
| B1O089-01 | 6-[4-[[3-(3-Ethoxyphenyl)-5-(trifluoromethoxy)phenyl]methyl]piperazin-1-yl]-N-(4-methoxyphenyl)pyridazine-3-carboxamide |
| B1O090-01 | 6-[4-[[3-(5-Ethoxypyridin-3-yl)-5-(trifluoromethoxy)phenyl]methyl]piperazin-1-yl]-N-(4-methoxyphenyl)pyridazine-3-carboxamide |

(continued)

| Mixture No. | mixAI-B 10-01 |
|---|---|
| Compound No. that may be included | Compound name |
| B1O091-01 | 6-[4-[[4-(3-Ethoxyphenyl)naphthalen-1-yl]methyl]piperazin-1-yl]-N-(4-methoxyphenyl)pyridazine-3-carboxamide |
| B1O092-01 | 6-[4-[[4-(5-Ethoxypyridin-3-yl)naphthalen-1-yl]methyl]piperazin-1-yl]-N-(4-methoxyphenyl)pyridazine-3-carboxamide |
| B1O093-01 | 6-[4-[[4-(3-Ethoxyphenyl)thiophen-2-yl]methyl]piperazin-1-yl]-N-(4-methoxyphenyl)pyridazine-3-carboxamide |
| B1O094-01 | 6-[4-[[4-(5-Ethoxypyridin-3-yl)thiophen-2-yl]methyl]piperazin-1-yl]-N-(4-methoxyphenyl)pyridazine-3-carboxamide |
| B1O095-01 | 6-[4-[[2-[2-(3-Ethoxyphenyl)ethynyl]phenyl]methyl]piperazin-1-yl]-N-(4-methoxyphenyl)pyridazine-3-carboxamide |
| B1O096-01 | 6-[4-[[2-[2-(5-Ethoxypyridin-3-yl)ethynyl]phenyl]methyl]piperazin-1-yl]-N-(4-methoxyphenyl)pyridazine-3-carboxamide |
| B1O097-01 | 4-[4-[1-[4-(3-Ethoxyphenyl)phenyl]ethyl]piperazin-1-yl]-N-(4-methoxyphenyl)benzamide |
| B1O098-01 | 4-[4-[1-[4-(5-Ethoxypyridin-3-yl)phenyl]ethyl]piperazin-1-yl]-N-(4-methoxyphenyl)benzamide |
| B1O099-01 | 4-[4-[1-[4-(3-Ethoxyphenyl)-2-fluorophenyl]ethyl]piperazin-1-yl]-N-(4-methoxyphenyl)benzamide |
| B1O100-01 | 4-[4-[1-[4-(5-Ethoxypyridin-3-yl)-2-fluorophenyl]ethyl]piperazin-1-yl]-N-(4-methoxyphenyl)benzamide |

[Table 101]

[Table AI-03-B1A-01]

| Mixture No. | mixAI-B1A-01 |
|---|---|
| Compound No. that may be included | Compound name |
| B1A001-01 | N-(1-Adamantylmethyl)-6-[4-[4-(3-ethoxyphenyl)thiophene-2-carbonyl]piperazin-1-yl]pyridazine-3-carboxamide |
| B1A002-01 | N-(1-Adamantylmethyl)-6-[4-[4-(5-ethoxypyridin-3-yl)thiophene-2-carbonyl]piperazin-1 -yl]pyridazine-3 -carboxamide |
| B1A003-01 | N-(1-Adamantylmethyl)-6-[4-[4-(3-ethoxyphenyl)-3-methylbenzoyl]piperazin-1-yl]pyridazine-3-carboxamide |
| B1A004-01 | N-(1-Adamantylmethyl)-6-[4-[4-(5-ethoxypyridin-3-yl)-3-methylbenzoyl]piperazin-1-yl]pyridazine-3-carboxamide |
| B1A005-01 | N-(1-Adamantylmethyl)-6-[4-[4-(3-ethoxyphenyl)-3-fluorobenzoyl]piperazin-1-yl]pyridazine-3-carboxamide |
| B1A006-01 | N-(1-Adamantylmethyl)-6-[4-[4-(5-ethoxypyridin-3-yl)-3-fluorobenzoyl]piperazin-1-yl]pyridazine-3-carboxamide |
| B1A007-01 | N-(1-Adamantylmethyl)-6-[4-[2-[2-(3-ethoxyphenyl)ethynyl]benzoyl]piperazin-1-yl]pyridazine-3-carboxamide |

(continued)

| Mixture No. | mixAI-B1A-01 |
|---|---|
| Compound No. that may be included | Compound name |
| B1A008-01 | N-(1-Adamantylmethyl)-6-[4-[2-[2-(5-ethoxypyridin-3-yl)ethynyl]benzoyl]piperazin-1-yl]pyridazine-3-carboxamide |
| B1A009-01 | N-(1-Adamantylmethyl)-6-[4-[5-[2-(3-ethoxyphenyl)ethynyl]pyridine-3-carbonyl]piperazin-1-yl]pyridazine-3-carboxamide |
| B1A010-01 | N-(1-Adamantylmethyl)-6-[4-[5-[2-(5-ethoxypyridin-3-yl)ethynyl]pyridine-3-carbonyl]piperazin-1-yl]pyridazine-3-carboxamide |
| B1A011-01 | N-(1-Adamantylmethyl)-6-[4-[3-[2-(3-ethoxyphenyl)phenyl]propanoyl]piperazin-1-yl]pyridazine-3-carboxamide |
| B1A012-01 | N-(1-Adamantylmethyl)-6-[4-[3-[2-(5-ethoxypyridin-3-yl)phenyl]propanoyl]piperazin-1-yl]pyridazine-3-carboxamide |
| B1A013-01 | N-(1-Adamantylmethyl)-6-[4-[4-(3-ethoxyphenyl)naphthalene-1-carbonyl]piperazin-1-yl]pyridazine-3-carboxamide |
| B1A014-01 | N-(1-Adamantylmethyl)-6-[4-[4-(5-ethoxypyridin-3-yl)naphthalene-1-carbonyl]piperazin-1-yl]pyridazine-3-carboxamide |
| B1A015-01 | N-(1-Adamantylmethyl)-6-[4-[3-tert-butyl-5-(3-ethoxyphenyl)benzoyl]piperazin-1-yl]pyridazine-3-carboxamide |
| B1A016-01 | N-(1-Adamantylmethyl)-6-[4-[3-tert-butyl-5-(5-ethoxypyridin-3-yl)benzoyl]piperazin-1-yl]pyridazine-3 -carboxamide |
| B1A017-01 | N-(1-Adamantylmethyl)-6-[4-[3-(3-ethoxyphenyl)-5-(trifluoromethyl)benzoyl]piperazin-1-yl]pyridazine-3-carboxamide |
| B1A018-01 | N-(1-Adamantylmethyl)-6-[4-[3-(5-ethoxypyridin-3-yl)-5-(trifluoromethyl)benzoyl]piperazin-1-yl]pyridazine-3-carboxamide |
| B1A019-01 | N-(1-Adamantylmethyl)-6-[4-[3-[2-(3-ethoxyphenyl)ethynyl]-5-(trifluoromethyl)benzoyl]piperazin-1-yl]pyridazine-3-carboxamide |
| B1A020-01 | N-(1-Adamantylmethyl)-6-[4-[3-[2-(5-ethoxypyridin-3-yl)ethynyl]-5-(trifluoromethyl)benzoyl]piperazin-1-yl]pyridazine-3-carboxamide |
| B1A021-01 | N-(1-Adamantylmethyl)-6-[4-[3-[4-(3-ethoxyphenyl)pyrazol-1-yl]-5-(trifluoromethyl)benzoyl]piperazin-1-yl]pyridazine-3-carboxamide |
| B1A022-01 | N-(1-Adamantylmethyl)-6-[4-[3-[4-(5-ethoxypyridin-3-yl)pyrazol-1-yl]-5-(trifluoromethyl)benzoyl]piperazin-1-yl]pyridazine-3-carboxamide |
| B1A023-01 | N-(1-Adamantylmethyl)-4-[4-[4-(3-ethoxyphenyl)thiophene-2-carbonyl]piperazin-1-yl]benzamide |
| B1A024-01 | N-(1-Adamantylmethyl)-4-[4-[4-(5-ethoxypyridin-3-yl)thiophene-2-carbonyl]piperazin-1-yl]benzamide |
| B1A025-01 | N-(1-Adamantylmethyl)-4-[4-[4-(3-ethoxyphenyl)-3-methylbenzoyl]piperazin-1-yl]benzamide |
| B1A026-01 | N-(1-Adamantylmethyl)-4-[4-[4-(5-ethoxypyridin-3-yl)-3-methylbenzoyl]piperazin-1-yl]benzamide |
| B1A027-01 | N-(1-Adamantylmethyl)-4-[4-[4-(3-ethoxyphenyl)-3-fluorobenzoyl]piperazin-1-yl]benzamide |
| B1A028-01 | N-(1-Adamantylmethyl)-4-[4-[4-(5-ethoxypyridin-3-yl)-3-fluorobenzoyl]piperazin-1-yl]benzamide |

(continued)

| Mixture No. | mixAI-B1A-01 |
|---|---|
| Compound No. that may be included | Compound name |
| B1A029-01 | N-(1-Adamantylmethyl)-4-[4-[2-[2-(3-ethoxyphenyl)ethynyl]benzoyl]piperazin-1-yl]benzamide |
| B1A030-01 | N-(1-Adamantylmethyl)-4-[4-[2-[2-(5-ethoxypyridin-3-yl)ethynyl]benzoyl]piperazin-1-yl]benzamide |
| B1A031-01 | N-(1-Adamantylmethyl)-4-[4-[5-[2-(3-ethoxyphenyl)ethynyl]pyridine-3-carbonyl]piperazin-1-yl]benzamide |
| B1A032-01 | N-(1-Adamantylmethyl)-4-[4-[5-[2-(5-ethoxypyridin-3-yl)ethynyl]pyridine-3-carbonyl]piperazin-1-yl]benzamide |
| B1A033-01 | N-(1-Adamantylmethyl)-4-[4-[3-[2-(3-ethoxyphenyl)phenyl]propanoyl]piperazin-1-yl]benzamide |
| B1A034-01 | N-(1-Adamantylmethyl)-4-[4-[3-[2-(5-ethoxypyridin-3-yl)phenyl]propanoyl]piperazin-1-yl]benzamide |
| B1A035-01 | N-(1-Adamantylmethyl)-4-[4-[4-(3-ethoxyphenyl)naphthalene-1-carbonyl]piperazin-1-yl]benzamide |
| B1A036-01 | N-(1-Adamantylmethyl)-4-[4-[4-(5-ethoxypyridin-3-yl)naphthalene-1-carbonyl]piperazin-1-yl]benzamide |
| B1A037-01 | N-(1-Adamantylmethyl)-4-[4-[3-tert-butyl-5-(3-ethoxyphenyl)benzoyl]piperazin-1-yl]benzamide |
| B1A038-01 | N-(1-Adamantylmethyl)-4-[4-[3-tert-butyl-5-(5-ethoxypyridin-3-yl)benzoyl]piperazin-1-yl]benzamide |
| B1A039-01 | N-(1-Adamantylmethyl)-4-[4-[3-(3-ethoxyphenyl)-5-(trifluoromethyl)benzoyl]piperazin-1-yl]benzamide |
| B1A040-01 | N-(1-Adamantylmethyl)-4-[4-[3-(5-ethoxypyridin-3-yl)-5-(trifluoromethyl)benzoyl]piperazin-1-yl]benzamide |
| B1A041-01 | N-(1-Adamantylmethyl)-4-[4-[3-[2-(3-ethoxyphenyl)ethynyl]-5-(trifluoromethyl)benzoyl]piperazin-1-yl]benzamide |
| B1A042-01 | N-(1-Adamantylmethyl)-4-[4-[3-[2-(5-ethoxypyridin-3-yl)ethynyl]-5-(trifluoromethyl)benzoyl]piperazin-1-yl]benzamide |
| B1A043-01 | N-(1-Adamantylmethyl)-4-[4-[3-[4-(3-ethoxyphenyl)pyrazol-1-yl]-5-(trifluoromethyl)benzoyl]piperazin-1-yl]benzamide |
| B1A044-01 | N-(1-Adamantylmethyl)-4-[4-[3-[4-(5-ethoxypyridin-3-yl)pyrazol-1-yl]-5-(trifluoromethyl)benzoyl]piperazin-1-yl]benzamide |
| B1A045-01 | N-(1-Adamantylmethyl)-4-[4-[[2-(3-ethoxyphenyl)phenyl]methyl]piperazin-1-yl]benzamide |
| B1A046-01 | N-(1-Adamantylmethyl)-4-[4-[[2-(5-ethoxypyridin-3-yl)phenyl]methyl]piperazin-1-yl]benzamide |
| B1A047-01 | N-(1-Adamantylmethyl)-4-[4-[[4-(3-ethoxyphenyl)-3-methylphenyl]methyl]piperazin-1-yl]benzamide |
| B1A048-01 | N-(1-Adamantylmethyl)-4-[4-[[4-(5-ethoxypyridin-3-yl)-3-methylphenyl]methyl]piperazin-1-yl]benzamide |
| B1A049-01 | N-(1-Adamantylmethyl)-4-[4-[[4-(3-ethoxyphenyl)-2-ethylphenyl]methyl]piperazin-1-yl]benzamide |

(continued)

| Mixture No. | mixAI-B1A-01 |
|---|---|
| Compound No. that may be included | Compound name |
| B1A050-01 | N-(1-Adamantylmethyl)-4-[4-[[4-(5-ethoxypyridin-3-yl)-2-ethylphenyl]methyl]piperazin-1-yl]benzamide |
| B1A051-01 | N-(1-Adamantylmethyl)-4-[4-[[4-(3-ethoxyphenyl)-3-fluorophenyl]methyl]piperazin-1-yl]benzamide |
| B1A052-01 | N-(1-Adamantylmethyl)-4-[4-[[4-(5-ethoxypyridin-3-yl)-3-fluorophenyl]methyl]piperazin-1-yl]benzamide |
| B1A053-01 | N-(1-Adamantylmethyl)-4-[4-[[4-(3-ethoxyphenyl)-2-methoxyphenyl]methyl]piperazin-1-yl]benzamide |
| B1A054-01 | N-(1-Adamantylmethyl)-4-[4-[[4-(5-ethoxypyridin-3-yl)-2-methoxyphenyl]methyl]piperazin-1-yl]benzamide |
| B1A055-01 | N-(1-Adamantylmethyl)-4-[4-[[3-ethoxy-5-(3-ethoxyphenyl)phenyl]methyl]piperazin-1-yl]benzamide |
| B1A056-01 | N-(1-Adamantylmethyl)-4-[4-[[3-ethoxy-5-(5-ethoxypyridin-3-yl)phenyl]methyl]piperazin-1-yl]benzamide |
| B1A057-01 | N-(1-Adamantylmethyl)-4-[4-[[3-(3-ethoxyphenyl)-5-propan-2-yloxyphenyl]methyl]piperazin-1-yl]benzamide |
| B1A058-01 | N-(1-Adamantylmethyl)-4-[4-[[3-(5-ethoxypyridin-3-yl)-5-propan-2-yloxyphenyl]methyl]piperazin-1-yl]benzamide |
| B1A059-01 | N-(1-Adamantylmethyl)-4-[4-[[4-(3-ethoxyphenyl)-2,6-dimethoxyphenyl]methyl]piperazin-1-yl]benzamide |
| B1A060-01 | N-(1-Adamantylmethyl)-4-[4-[[4-(5-ethoxypyridin-3-yl)-2,6-dimethoxyphenyl]-1-yl]benzamide |
| B1A061-01 | N-(1-Adamantylmethyl)-4-[4-[[3-(3-ethoxyphenyl)-5-(trifluoromethyl)phenyl]methyl]piperazin-1-yl]benzamide |
| B1A062-01 | N-(1-Adamantylmethyl)-4-[4-[[3-(5-ethoxypyridin-3-yl)-5-(trifluoromethyl)phenyl]methyl]piperazin-1-yl]benzamide |
| B1A063-01 | N-(1-Adamantylmethyl)-4-[4-[[3-(3-ethoxyphenyl)-5-(trifluoromethoxy)phenyl]methyl]piperazin-1-yl]benzamide |
| B1A064-01 | N-(1-Adamantylmethyl)-4-[4-[[3-(5-ethoxypyridin-3-yl)-5-(trifluoromethoxy)phenyl]methyl]piperazin-1-yl]benzamide |
| B1A065-01 | N-(1-Adamantylmethyl)-4-[4-[[4-(3-ethoxyphenyl)naphthalen-1-yl]methyl]piperazin-1-yl]benzamide |
| B1A066-01 | N-(1-Adamantylmethyl)-4-[4-[[4-(5-ethoxypyridin-3-yl)naphthalen-1-yl]methyl]piperazin-1-yl]benzamide |
| B1A067-01 | N-(1-Adamantylmethyl)-4-[4-[[4-(3-ethoxyphenyl)thiophen-2-yl]methyl]piperazin-1-yl]benzamide |
| B1A068-01 | N-(1-Adamantylmethyl)-4-[4-[[4-(5-ethoxypyridin-3-yl)thiophen-2-yl]methyl]piperazin-1-yl]benzamide |
| B1A069-01 | N-(1-Adamantylmethyl)-4-[4-[[2-[2-(3-ethoxyphenyl)ethynyl]phenyl]methyl]piperazin-1-yl]benzamide |
| B1A070-01 | N-(1-Adamantylmethyl)-4-[4-[[2-[2-(5-ethoxypyridin-3-yl)ethynyl]phenyl]methyl]piperazin-1-yl]benzamide |

(continued)

| Mixture No. | mixAI-B1A-01 |
|---|---|
| Compound No. that may be included | Compound name |
| B1A071-01 | N-(1-Adamantylmethyl)-6-[4-[[2-(3-ethoxyphenyl)phenyl]methyl]piperazin-1-yl]pyridazine-3-carboxamide |
| B1A072-01 | N-(1-Adamantylmethyl)-6-[4-[[2-(5-ethoxypyridin-3-yl)phenyl]methyl]piperazin-1-yl]pyridazine-3 -carboxamide |
| B1A073-01 | N-(1-Adamantylmethyl)-6-[4-[[4-(3-ethoxyphenyl)-3-methylphenyl]methyl]piperazin-1-yl]pyridazine-3-carboxamide |
| B1A074-01 | N-(1-Adamantylmethyl)-6-[4-[[4-(5-ethoxypyridin-3-yl)-3-methylphenyl]methyl]piperazin-1-yl]pyridazine-3-carboxamide |
| B1A075-01 | N-(1-Adamantylmethyl)-6-[4-[[4-(3-ethoxyphenyl)-2-ethylphenyl]methyl]piperazin-1-yl]pyridazine-3-carboxamide |
| B1A076-01 | N-(1-Adamantylmethyl)-6-[4-[[4-(5-ethoxypyridin-3-yl)-2-ethylphenyl]methyl]piperazin-1-yl]pyridazine-3-carboxamide |
| B1A077-01 | N-(1-Adamantylmethyl)-6-[4-[[4-(3-ethoxyphenyl)-3-fluorophenyl]methyl]piperazin-1-yl]pyridazine-3-carboxamide |
| B1A078-01 | N-(1-Adamantylmethyl)-6-[4-[[4-(5-ethoxypyridin-3-yl)-3-fluorophenyl]methyl]piperazin-1-yl]pyridazine-3-carboxamide |
| B1A079-01 | N-(1-Adamantylmethyl)-6-[4-[[4-(3-ethoxyphenyl)-2-methoxyphenyl]methyl]piperazin-1-yl]pyridazine-3-carboxamide |
| B1A080-01 | N-(1-Adamantylmethyl)-6-[4-[[4-(5-ethoxypyridin-3-yl)-2-methoxyphenyl]methyl]piperazin-1-yl]pyridazine-3-carboxamide |
| B1A081-01 | N-(1-Adamantylmethyl)-6-[4-[[3-ethoxy-5-(3-ethoxyphenyl)phenyl]methyl]piperazin-1-yl]pyridazine-3-carboxamide |
| B1A082-01 | N-(1-Adamantylmethyl)-6-[4-[[3-ethoxy-5-(5-ethoxypyridin-3-yl)phenyl]methyl]piperazin-1-yl]pyridazine-3-carboxamide |
| B1A083-01 | N-(1-Adamantylmethyl)-6-[4-[[3-(3-ethoxyphenyl)-5-propan-2-yloxyphenyl]methyl]piperazin-1-yl]pyridazine-3-carboxamide |
| B1A084-01 | N-(1-Adamantylmethyl)-6-[4-[[3-(5-ethoxypyridin-3-yl)-5-propan-2-yloxyphenyl]methyl]piperazin-1-yl]pyridazine-3-carboxamide |
| B1A085-01 | N-(1-Adamantylmethyl)-6-[4-[[4-(3-ethoxyphenyl)-2,6-dimethoxyphenyl]methyl]piperazin-1-yl]pyridazine-3-carboxamide |
| B1A086-01 | N-(1-Adamantylmethyl)-6-[4-[[4-(5-ethoxypyridin-3-yl)-2,6-dimethoxyphenyl]methyl]piperazin-1-yl]pyridazine-3-carboxamide |
| B1A087-01 | N-(1-Adamantylmethyl)-6-[4-[[3-(3-ethoxyphenyl)-5-(trifluoromethyl)phenyl]methyl]piperazin-1-yl]pyridazine-3-carboxamide |
| B1A088-01 | N-(1-Adamantylmethyl)-6-[4-[[3-(5-ethoxypyridin-3-yl)-5-(trifluoromethyl)phenyl]methyl]piperazin-1-yl]pyridazine-3-carboxamide |
| B1A089-01 | N-(1-Adamantylmethyl)-6-[4-[[3-(3-ethoxyphenyl)-5-(trifluoromethoxy)phenyl]methyl]piperazin-1-yl]pyridazine-3-carboxamide |
| B1A090-01 | N-(1-Adamantylmethyl)-6-[4-[[3-(5-ethoxypyridin-3-yl)-5-(trifluoromethoxy)phenyl]methyl]piperazin-1-yl]pyridazine-3-carboxamide |
| B1A091-01 | N-(1-Adamantylmethyl)-6-[4-[[4-(3-ethoxyphenyl)naphthalen-1-yl]methyl]piperazin-1-yl]pyridazine-3-carboxamide |

(continued)

| Mixture No. | mixAI-B1A-01 |
|---|---|
| Compound No. that may be included | Compound name |
| B1A092-01 | N-(1-Adamantylmethyl)-6-[4-[[4-(5-ethoxypyridin-3-yl)naphthalen-1-yl]methyl]piperazin-1-yl]pyridazine-3-carboxamide |
| B1A093-01 | N-(1-Adamantylmethyl)-6-[4-[[4-(3-ethoxyphenyl)thiophen-2-yl]methyl]piperazin-1-yl]pyridazine-3-carboxamide |
| B1A094-01 | N-(1-Adamantylmethyl)-6-[4-[[4-(5-ethoxypyridin-3-yl)thiophen-2-yl]methyl]piperazin-1-yl]pyridazine-3-carboxamide |
| B1A095-01 | N-(1-Adamantylmethyl)-6-[4-[[2-[2-(3-ethoxyphenyl)ethynyl]phenyl]methyl]piperazin-1-yl]pyridazine-3-carboxamide |
| B1A096-01 | N-(1-Adamantylmethyl)-6-[4-[[2-[2-(5-ethoxypyridin-3-yl)ethynyl]phenyl]methyl]piperazin-1-yl]pyridazine-3 -carboxamide |
| B1A097-01 | N-(1-Adamantylmethyl)-4-[4-[1-[4-(3-ethoxyphenyl)phenyl]ethyl]piperazin-1-yl]benzamide |
| B1A098-01 | N-(1-Adamantylmethyl)-4-[4-[1-[4-(5-ethoxypyridin-3-yl)phenyl]ethyl]piperazin-1-yl]benzamide |
| B1A099-01 | N-(1-Adamantylmethyl)-4-[4-[1-[4-(3-ethoxyphenyl)-2-fluorophenyl]ethyl]piperazin-1-yl]benzamide |
| B1A100-01 | N-(1-Adamantylmethyl)-4-[4-[1-[4-(5-ethoxypyridin-3-yl)-2-fluorophenyl]ethyl]piperazin-1-yl]benzamide |

[Table 102]

[Table AI-03-B3A-01]

| Mixture No. | mixAI-B3A-01 |
|---|---|
| Compound No. that may be included | Compound name |
| B3A001-01 | N-(1-Adamantylmethyl)-6-[4-[4-(3-ethoxyphenyl)thiophene-2-carbonyl]piperazin-1-yl]-N-methylpyridazine-3-carboxamide |
| B3A002-01 | N-(1-Adamantylmethyl)-6-[4-[4-(5-ethoxypyridin-3-yl)thiophene-2-carbonyl]piperazin-1-yl]-N-methylpyridazine-3-carboxamide |
| B3A003-01 | N-(1-Adamantylmethyl)-6-[4-[4-(3-ethoxyphenyl)-3 -methylbenzoyl]piperazin-1-yl]-N-methylpyridazine-3-carboxamide |
| B3A004-01 | N-(1-Adamantylmethyl)-6-[4-[4-(5-ethoxypyridin-3-yl)-3-methylbenzoyl]piperazin-1-yl]-N-methylpyridazine-3-carboxamide |
| B3A005-01 | N-(1-Adamantylmethyl)-6-[4-[4-(3-ethoxyphenyl)-3-fluorobenzoyl]piperazin-1-yl]-N-methylpyridazine-3-carboxamide |
| B3A006-01 | N-(1-Adamantylmethyl)-6-[4-[4-(5-ethoxypyridin-3-yl)-3-fluorobenzoyl]piperazin-1-yl]-N-methylpyridazine-3-carboxamide |
| B3A007-01 | N-(1-Adamantylmethyl)-6-[4-[2-[2-(3-ethoxyphenyl)ethynyl]benzoyl]piperazin-1-yl]-N-methylpyridazine-3-carboxamide |
| B3A008-01 | N-(1-Adamantylmethyl)-6-[4-[2-[2-(5-ethoxypyridin-3-yl)ethynyl]benzoyl]piperazin-1-yl]-N-methylpyridazine-3-carboxamide |

(continued)

| Mixture No. | mixAI-B3A-01 |
|---|---|
| Compound No. that may be included | Compound name |
| B3A009-01 | N-(1-Adamantylmethyl)-6-[4-[5-[2-(3-ethoxyphenyl)ethynyl]pyridine-3-carbonyl]piperazin-1-yl]-N-methylpyridazine-3-carboxamide |
| B3A010-01 | N-(1-Adamantylmethyl)-6-[4-[5-[2-(5-ethoxypyridin-3-yl)ethynyl]pyridine-3-carbonyl]piperazin-1-yl]-N-methylpyridazine-3-carboxamide |
| B3A011-01 | N-(1-Adamantylmethyl)-6-[4-[3-[2-(3-ethoxyphenyl)phenyl]propanoyl]piperazin-1-yl]-N-methylpyridazine-3-carboxamide |
| B3A012-01 | N-(1-Adamantylmethyl)-6-[4-[3-[2-(5-ethoxypyridin-3-yl)phenyl]propanoyl]piperazin-1-yl]-N-methylpyridazine-3-carboxamide |
| B3A013-01 | N-(1-Adamantylmethyl)-6-[4-[4-(3-ethoxyphenyl)naphthalene-1-carbonyl]piperazin-1-yl]-N-methylpyridazine-3-carboxamide |
| B3A014-01 | N-(1-Adamantylmethyl)-6-[4-[4-(5-ethoxypyridin-3-yl)naphthalene-1-carbonyl]piperazin-1-yl]-N-methylpyridazine-3-carboxamide |
| B3A015-01 | N-(1-Adamantylmethyl)-6-[4-[3-tert-butyl-5-(3-ethoxyphenyl)benzoyl]piperazin-1-yl]-N-methylpyridazine-3-carboxamide |
| B3A016-01 | N-(1-Adamantylmethyl)-6-[4-[3-tert-butyl-5-(5-ethoxypyridin-3-yl)benzoyl]piperazin-1-yl]-N-methylpyridazine-3-carboxamide |
| B3A017-01 | N-(1-Adamantylmethyl)-6-[4-[3-(3-ethoxyphenyl)-5-(trifluoromethyl)benzoyl]piperazin-1-yl]-N-methylpyridazine-3-carboxamide |
| B3A018-01 | N-(1-Adamantylmethyl)-6-[4-[3-(5-ethoxypyridin-3-yl)-5-(trifluoromethyl)benzoyl]piperazin-1-yl]-N-methylpyridazine-3-carboxamide |
| B3A019-01 | N-(1-Adamantylmethyl)-6-[4-[3-[2-(3-ethoxyphenyl)ethynyl]-5-(trifluoromethyl)benzoyl]piperazin-1-yl]-N-methylpyridazine-3-carboxamide |
| B3A020-01 | N-(1-Adamantylmethyl)-6-[4-[3-[2-(5-ethoxypyridin-3-yl)ethynyl]-5-(trifluoromethyl)benzoyl]piperazin-1-yl]-N-methylpyridazine-3-carboxamide |
| B3A021-01 | N-(1-Adamantylmethyl)-6-[4-[3-[4-(3-ethoxyphenyl)pyrazol-1-yl]-5-(trifluoromethyl)benzoyl]piperazin-1-yl]-N-methylpyridazme-3-carboxamide |
| B3A022-01 | N-(1-Adamantylmethyl)-6-[4-[3-[4-(5-ethoxypyridin-3-yl)pyrazol-1-yl]-5-(trifluoromethyl)benzoyl]piperazin-1-yl]-N-methylpyridazine-3-carboxamide |
| B3A023-01 | N-(1-Adamantylmethyl)-4-[4-[4-(3-ethoxyphenyl)thiophene-2-carbonyl]piperazin-1-yl]-N-methylbenzamide |
| B3A024-01 | N-(1-Adamantylmethyl)-4-[4-[4-(5-ethoxypyridin-3-yl)thiophene-2-carbonyl]piperazin-1-yl]-N-methylbenzamide |
| B3A025-01 | N-(1-Adamantylmethyl)-4-[4-[4-(3-ethoxyphenyl)-3-methylbenzoyl]piperazin-1-yl]-N-methylbenzamide |
| B3A026-01 | N-(1-Adamantylmethyl)-4-[4-[4-(5-ethoxypyridin-3-yl)-3-methylbenzoyl]piperazin-1-yl]-N-methylbenzamide |
| B3A027-01 | N-(1-Adamantylmethyl)-4-[4-[4-(3-ethoxyphenyl)-3-fluorobenzoyl]piperazin-1-yl]-N-methylbenzamide |
| B3A028-01 | N-(1-Adamantylmethyl)-4-[4-[4-(5-ethoxypyridin-3-yl)-3-fluorobenzoyl]piperazin-1-yl]-N-methylbenzamide |
| B3A029-01 | N-(1-Adamantylmethyl)-4-[4-[2-[2-(3-ethoxyphenyl)ethynyl]benzoyl]piperazin-1-yl]-N-methylbenzamide |

(continued)

| Mixture No. | mixAI-B3A-01 |
|---|---|
| Compound No. that may be included | Compound name |
| B3A030-01 | N-(1-Adamantylmethyl)-4-[4-[2-[2-(5-ethoxypyridin-3-yl)ethynyl]benzoyl]piperazin-1-yl] -N-methylbenzamide |
| B3A031-01 | N-(1-Adamantylmethyl)-4-[4-[5-[2-(3-ethoxyphenyl)ethynyl]pyridine-3-carbonyl]piperazin-1-yl]-N-methylbenzamide |
| B3A032-01 | N-(1-Adamantylmethyl)-4-[4-[5-[2-(5-ethoxypyridin-3-yl)ethynyl]pyridine-3-carbonyl]piperazin-1-yl]-N-methylbenzamide |
| B3A033-01 | N-(1-Adamantylmethyl)-4-[4-[3-[2-(3-ethoxyphenyl)phenyl]propanoyl]piperazin-1-yl]-N-methylbenzamide |
| B3A034-01 | N-(1-Adamantylmethyl)-4-[4-[3-[2-(5-ethoxypyridin-3-yl)phenyl]propanoyl]piperazin-1-yl]-N-methylbenzamide |
| B3A035-01 | N-(1-Adamantylmethyl)-4-[4-[4-(3-ethoxyphenyl)naphthalene-1-carbonyl]piperazin-1-yl]-N-methylbenzamide |
| B3A036-01 | N-(1-Adamantylmethyl)-4-[4-[4-(5-ethoxypyridin-3-yl)naphthalene-1-carbonyl]piperazin-1-yl]-N-methylbenzamide |
| B3A037-01 | N-(1-Adamantylmethyl)-4-[4-[3-tert-butyl-5-(3-ethoxyphenyl)benzoyl]piperazin-1-yl]-N-methylbenzamide |
| B3A038-01 | N-(1-Adamantylmethyl)-4-[4-[3-tert-butyl-5-(5-ethoxypyridin-3-yl)benzoyl]piperazin-1-yl]-N-methylbenzamide |
| B3A039-01 | N-(1-Adamantylmethyl)-4-[4-[3-(3-ethoxyphenyl)-5-(trifluoromethyl)benzoyl]piperazin-1-yl]-N-methylbenzamide |
| B3A040-01 | N-(1-Adamantylmethyl)-4-[4-[3-(5-ethoxypyridin-3-yl)-5-(trifluoromethyl)benzoyl]piperazin-1-yl]-N-methylbenzamide |
| B3A041-01 | N-(1-Adamantylmethyl)-4-[4-[3-[2-(3-ethoxyphenyl)ethynyl]-5-(trifluoromethyl)benzoyl]piperazin-1-yl]-N-methylbenzamide |
| B3A042-01 | N-(1-Adamantylmethyl)-4-[4-[3-[2-(5-ethoxypyridin-3-yl)ethynyl]-5-(trifluoromethyl)benzoyl]piperazin-1-yl]-N-methylbenzamide |
| B3A043-01 | N-(1-Adamantylmethyl)-4-[4-[3-[4-(3-ethoxyphenyl)pyrazol-1-yl]-5-(trifluoromethyl)benzoyl]piperazin-1-yl]-N-methylbenzamide |
| B3A044-01 | N-(1-Adamantylmethyl)-4-[4-[3-[4-(5-ethoxypyridin-3-yl)pyrazol-1-yl]-5-(trifluoromethyl)benzoyl]piperazin-1-yl]-N-methylbenzamide |
| B3A045-01 | N-(1-Adamantylmethyl)-4-[4-[[2-(3-ethoxyphenyl)phenyl]methyl]piperazin-1-yl]-N-methylbenzamide |
| B3A046-01 | N-(1-Adamantylmethyl)-4-[4-[[2-(5-ethoxypyridin-3-yl)phenyl]methyl]piperazin-1-yl]-N-methylbenzamide |
| B3A047-01 | N-(1-Adamantylmethyl)-4-[4-[[4-(3-ethoxyphenyl)-3-methylphenyl]methyl]piperazin-1-yl]-N-methylbenzamide |
| B3A048-01 | N-(1-Adamantylmethyl)-4-[4-[[4-(5-ethoxypyridin-3-yl)-3-methylphenyl]methyl]piperazin-1-yl]-N-methylbenzamide |
| B3A049-01 | N-(1-Adamantylmethyl)-4-[4-[[4-(3-ethoxyphenyl)-2-ethylphenyl]methyl]piperazin-1-yl]-N-methylbenzamide |
| B3A050-01 | N-(1-Adamantylmethyl)-4-[4-[[4-(5-ethoxypyridin-3-yl)-2-ethylphenyl]methyl]piperazin-1-yl]-N-methylbenzamide |

(continued)

| Mixture No. | mixAI-B3A-01 |
|---|---|
| Compound No. that may be included | Compound name |
| B3A051-01 | N-(1-Adamantylmethyl)-4-[4-[[4-(3-ethoxyphenyl)-3-fluorophenyl]methyl]piperazin-1-yl]-N-methylbenzamide |
| B3A052-01 | N-(1-Adamantylmethyl)-4-[4-[[4-(5-ethoxypyridin-3-yl)-3-fluorophenyl]methyl]piperazin-1-yl]-N-methylbenzamide |
| B3A053-01 | N-(1-Adamantylmethyl)-4-[4-[[4-(3-ethoxyphenyl)-2-methoxyphenyl]methyl]piperazin-1-yl]-N-methylbenzamide |
| B3A054-01 | N-(1-Adamantylmethyl)-4-[4-[[4-(5-ethoxypyridin-3-yl)-2-methoxyphenyl]methyl]piperazin-1-yl]-N-methylbenzamide |
| B3A055-01 | N-(1-Adamantylmethyl)-4-[4-[[3-ethoxy-5-(3-ethoxyphenyl)phenyl]methyl]piperazin-1-yl]-N-methylbenzamide |
| B3A056-01 | N-(1-Adamantylmethyl)-4-[4-[[3-ethoxy-5-(5-ethoxypyridin-3-yl)phenyl]methyl]piperazin-1-yl]-N-methylbenzamide |
| B3A057-01 | N-(1-Adamantylmethyl)-4-[4-[[3-(3-ethoxyphenyl)-5-propan-2-yloxyphenyl]methyl]piperazin-1-yl]-N-methylbenzamide |
| B3A058-01 | N-(1-Adamantylmethyl)-4-[4-[[3-(5-ethoxypyridin-3-yl)-5-propan-2-yloxyphenyl]methyl]piperazin-1-yl]-N-methylbenzamide |
| B3A059-01 | N-(1-Adamantylmethyl)-4-[4-[[4-(3-ethoxyphenyl)-2,6-dimethoxyphenyl]methyl]piperazin-1-yl]-N-methylbenzamide |
| B3A060-01 | N-(1-Adamantylmethyl)-4-[4-[[4-(5-ethoxypyridin-3-yl)-2,6-dimethoxyphenyl]methyl]piperazin-1-yl]-N-methylbenzamide |
| B3A061-01 | N-(1-Adamantylmethyl)-4-[4-[[3-(3-ethoxyphenyl)-5-(trifluoromethyl)phenyl]methyl]piperazin-1-yl]-N-methylbenzamide |
| B3A062-01 | N-(1-Adamantylmethyl)-4-[4-[[3-(5-ethoxypyridin-3-yl)-5-(trifluoromethyl)phenyl]methyl]piperazin-1-yl]-N-methylbenzamide |
| B3A063-01 | N-(1-Adamantylmethyl)-4-[4-[[3-(3-ethoxyphenyl)-5-(trifluoromethoxy)phenyl]methyl]piperazin-1-yl]-N-methylbenzamide |
| B3A064-01 | N-(1-Adamantylmethyl)-4-[4-[[3-(5-ethoxypyridin-3-yl)-5-(trifluoromethoxy)phenyl]methyl]piperazin-1-yl]-N-methylbenzamide |
| B3A065-01 | N-(1-Adamantylmethyl)-4-[4-[[4-(3-ethoxyphenyl)naphthalen-1-yl]methyl]piperazin-1-yl]-N-methylbenzamide |
| B3A066-01 | N-(1-Adamantylmethyl)-4-[4-[[4-(5-ethoxypyridin-3-yl)naphthalen-1-yl]methyl]piperazin-1-yl]-N-methylbenzamide |
| B3A067-01 | N-(1-Adamantylmethyl)-4-[4-[[4-(3-ethoxyphenyl)thiophen-2-yl]methyl]piperazin-1-yl]-N-methylbenzamide |
| B3A068-01 | N-(1-Adamantylmethyl)-4-[4-[[4-(5-ethoxypyridin-3-yl)thiophen-2-yl]methyl]piperazin-1-yl]-N-methylbenzamide |
| B3A069-01 | N-(1-Adamantylmethyl)-4-[4-[[2-[2-(3-ethoxyphenyl)ethynyl]phenyl]methyl]piperazin-1-yl]-N-methylbenzamide |
| B3A070-01 | N-(1-Adamantylmethyl)-4-[4-[[2-[2-(5-ethoxypyridin-3-yl)ethynyl]phenyl]methyl]piperazin-1-yl]-N-methylbenzamide |
| B3A071-01 | N-(1-Adamantylmethyl)-6-[4-[[2-(3-ethoxyphenyl)phenyl]methyl]piperazin-1-yl]-N-methylpyridazine-3-carboxamide |

(continued)

| Mixture No. | mixAI-B3A-01 |
|---|---|
| Compound No. that may be included | Compound name |
| B3A072-01 | N-(1-Adamantylmethyl)-6-[4-[[2-(5-ethoxypyridin-3-yl)phenyl]methyl]piperazin-1-yl]-N-methylpyridazine-3-carboxamide |
| B3A073-01 | N-(1-Adamantylmethyl)-6-[4-[[4-(3-ethoxyphenyl)-3-methylphenyl]methyl]piperazin-1-yl]-N-methylpyridazine-3-carboxamide |
| B3A074-01 | N-(1-Adamantylmethyl)-6-[4-[[4-(5-ethoxypyridin-3-yl)-3-methylphenyl]methyl]piperazin-1-yl]-N-methylpyridazine-3-carboxamide |
| B3A075-01 | N-(1-Adamantylmethyl)-6-[4-[[4-(3-ethoxyphenyl)-2-ethylphenyl]methyl]piperazin-1-yl]-N-methylpyridazine-3-carboxamide |
| B3A076-01 | N-(1-Adamantylmethyl)-6-[4-[[4-(5-ethoxypyridin-3-yl)-2-ethylphenyl]methyl]piperazin-1-yl]-N-methylpyridazine-3-carboxamide |
| B3A077-01 | N-(1-Adamantylmethyl)-6-[4-[[4-(3-ethoxyphenyl)-3-fluorophenyl]methyl]piperazin-1-yl]-N-methylpyridazine-3-carboxamide |
| B3A078-01 | N-(1-Adamantylmethyl)-6-[4-[[4-(5-ethoxypyridin-3-yl)-3-fluorophenyl]methyl]piperazin-1-yl]-N-methylpyridazine-3-carboxamide |
| B3A079-01 | N-(1-Adamantylmethyl)-6-[4-[[4-(3-ethoxyphenyl)-2-methoxyphenyl]methyl]piperazin-1-yl]-N-methylpyridazine-3-carboxamide |
| B3A080-01 | N-(1-Adamantylmethyl)-6-[4-[[4-(5-ethoxypyridin-3-yl)-2-methoxyphenyl]methyl]piperazin-1-yl]-N-methylpyridazine-3-carboxamide |
| B3A081-01 | N-(1-Adamantylmethyl)-6-[4-[[3-ethoxy-5-(3-ethoxyphenyl)phenyl-yl]-N-methylpyridazine-3-carboxamide |
| B3A082-01 | N-(1-Adamantylmethyl)-6-[4-[[3-ethoxy-5-(5-ethoxypyridin-3-yl)phenyl]methyl]piperazin-1-yl]-N-methylpyridazine-3-carboxamide |
| B3A083-01 | N-(1-Adamantylmethyl)-6-[4-[[3-(3-ethoxyphenyl)-5-propan-2-yloxyphenyl]methyl]piperazin-1-yl]-N-methylpyridazine-3-carboxamide |
| B3A084-01 | N-(1-Adamantylmethyl)-6-[4-[[3-(5-ethoxypyridin-3-yl)-5-propan-2-yloxyphenyl]methyl]piperazin-1-yl]-N-methylpyridazine-3-carboxamide |
| B3A085-01 | N-(1-Adamantylmethyl)-6-[4-[[4-(3-ethoxyphenyl)-2,6-dimethoxyphenyl]methyl]piperazin-1-yl]-N-methylpyridazine-3-carboxamide |
| B3A086-01 | N-(1-Adamantylmethyl)-6-[4-[[4-(5-ethoxypyridin-3-yl)-2,6-dimethoxyphenyl]methyl]piperazin-1-yl]-N-methylpyridazine-3-carboxamide |
| B3A087-01 | N-(1-Adamantylmethyl)-6-[4-[[3-(3-ethoxyphenyl)-5-(trifluoromethyl)phenyl]methyl]piperazin-1-yl]-N-methylpyridazine-3-carboxamide |
| B3A088-01 | N-(1-Adamantylmethyl)-6-[4-[[3-(5-ethoxypyridin-3-yl)-5-(trifluoromethyl)phenyl]methyl]piperazin-1-yl]-N-methylpyridazine-3 -carboxamide |
| B3A089-01 | N-(1-Adamantylmethyl)-6-[4-[[3-(3-ethoxyphenyl)-5-(trifluoromethoxy)phenyl]methyl]piperazin-1-yl]-N-methylpyridazine-3-carboxamide |
| B3A090-01 | N-(1-Adamantylmethyl)-6-[4-[[3-(5-ethoxypyridin-3-yl)-5-(trifluoromethoxy)phenyl]methyl]piperazin-1-yl]-N-methylpyridazine-3-carboxamide |
| B3A091-01 | N-(1-Adamantylmethyl)-6-[4-[[4-(3-ethoxyphenyl)naphthalen-1-yl]methyl]piperazin-1-yl]-N-methylpyridazine-3-carboxamide |
| B3A092-01 | N-(1-Adamantylmethyl)-6-[4-[[4-(5-ethoxypyridin-3-yl)naphthalen-1-yl]methyl]piperazin-1-yl]-N-methylpyridazine-3-carboxamide |

(continued)

| Mixture No. | mixAI-B3A-01 |
|---|---|
| Compound No. that may be included | Compound name |
| B3A093-01 | N-(1-Adamantylmethyl)-6-[4-[[4-(3-ethoxyphenyl)thiophen-2-yl]methyl]piperazin-1-yl]-N-methylpyridazine-3-carboxamide |
| B3A094-01 | N-(1-Adamatitylmethyl)-6-[4-[[4-(5-ethoxypyridin-3-yl)thiophen-2-yl]methyl]piperazin-1-yl]-N-methylpyridazine-3-carboxamide |
| B3A095-01 | N-(1-Adamantylmethyl)-6-[4-[[2-[2-(3-ethoxyphenyl)ethynyl]phenyl]methyl]piperazin-1-yl]-N-methylpyridazine-3-carboxamide |
| B3A096-01 | N-(1-Adamantylmethyl)-6-[4-[[2-[2-(5-ethoxypyridin-3-yl)ethynyl]phenyl]methyl]piperazin-1-yl]-N-methylpyridazine-3-carboxamide |
| B3A097-01 | N-(1-Adamantylmethyl)-4-[4-[1-[4-(3-ethoxyphenyl)phenyl]ethyl]piperazin-1-yl]-N-methylbenzamide |
| B3A098-01 | N-(1-Adamantylmethyl)-4-[4-[1-[4-(5-ethoxypyridin-3-yl)phenyl]ethyl]piperazin-1-yl]-N-methylbenzamide |
| B3A099-01 | N-(1-Adamantylmethyl)-4-[4-[1-[4-(3-ethoxyphenyl)-2-fluorophenyl]ethyl]piperazin-1-yl]-N-methylbenzamide |
| B3A100-01 | N-(1-Adamantylmethyl)-4-[4-[1-[4-(5-ethoxypyridin-3-yl)-2-fluorophenyl]ethyl]piperazin-1-yl]-N-methylbenzamide |

[Table 103]

[Table AI-03-B2I-01]

| Mixture No. | mixAI-B2I-01 |
|---|---|
| Compound No. that may be included | Compound name |
| B2I001-01 | 6-[4-[4-(3-Ethoxyphenyl)thiophene-2-carbonyl]piperazin-1-yl]-N-methylsulfonylpyridazine-3-carboxamide |
| B2I002-01 | 6-[4-[4-(5-Ethoxypyridin-3-yl)thiophene-2-carbonyl]piperazin-1-yl]-N-methylsulfonylpyridazine-3-carboxamide |
| B2I003-01 | 6-[4-[4-(3-Ethoxyphenyl)-3-methylbenzoyl]piperazin-1-yl]-N-methylsulfonylpyridazine-3-carboxamide |
| B2I004-01 | 6-[4-[4-(5-Ethoxypyridin-3-yl)-3-methylbenzoyl]piperazin-1-yl]-N-methylsulfonylpyridazine-3-carboxamide |
| B2I005-01 | 6-[4-[4-(3-Ethoxyphenyl)-3-fluorobenzoyl]piperazin-1-yl]-N-methylsulfonylpyridazine-3-carboxamide |
| B2I006-01 | 6-[4-[4-(5-Ethoxypyridin-3-yl)-3-fluorobenzoyl]piperazin-1-yl]-N-methylsulfonylpyridazine-3-carboxamide |
| B2I007-01 | 6-[4-[2-[2-(3-Ethoxyphenyl)ethynyl]benzoyl]piperazin-1-yl]-N-methylsulfonylpyridazine-3-carboxamide |
| B2I008-01 | 6-[4-[2-[2-(5-Ethoxypyridin-3-yl)ethynyl]benzoyl]piperazin-1-yl]-N-methylsulfonylpyridazine-3-carboxamide |
| B2I009-01 | 6-[4-[5-[2-(3-Ethoxyphenyl)ethynyl]pyridine-3-carbonyl]piperazin-1-yl]-N-methylsulfonylpyridazine-3-carboxamide |

(continued)

| Mixture No. | mixAI-B2I-01 |
|---|---|
| Compound No. that may be included | Compound name |
| B2I010-01 | 6-[4-[5-[2-(5-Ethoxypyridin-3-yl)ethynyl]pyridine-3-carbonyl]piperazin-1-yl]-N-methylsulfonylpyridazine-3-carboxamide |
| B2I01-1-01 | 6-[4-[3-[2-(3-Ethoxyphenyl)phenyl]propanoyl]piperazin-1-yl]-N-methylsulfonylpyridazine-3-carboxamide |
| B2I012-01 | 6-[4-[3-[2-(5-Ethoxypyridin-3-yl)phenyl]propanoyl]piperazin-1-yl]-N-methylsulfonylpyridazine-3-carboxamide |
| B2I013-01 | 6-[4-[4-(3-Ethoxyphenyl)naphthalene-1-carbonyl]piperazin-1-yl]-N-methylsulfonylpyridazine-3-carboxamide |
| B2I014-01 | 6-[4-[4-(5-Ethoxypyridin-3-yl)naphthalene-1-carbonyl]piperazin-1-yl]-N-methylsulfonylpyridazine-3-carboxamide |
| B2I015-01 | 6-[4-[3-tert-Butyl-5-(3-ethoxyphenyl)benzoyl]piperazin-1-yl]-N-methylsulfonylpyridazine-3-carboxamide |
| B2I016-01 | 6-[4-[3-tert-Butyl-5-(5-ethoxypyridin-3-yl)benzoyl]piperazin-1-yl]-N-methylsulfonylpyridazine-3-carboxamide |
| B2I017-01 | 6-[4-[3-(3-Ethoxyphenyl)-5-(trifluoromethyl)benzoyl]piperazin-1-yl]-N-methylsulfonylpyridazine-3-carboxamide |
| B2I018-01 | 6-[4-[3-(5-Ethoxypyridin-3-yl)-5-(trifluoromethyl)benzoyl]piperazin-1-yl]-N-methylsulfonylpyridazine-3-carboxamide |
| B2I019-01 | 6-[4-[3-[2-(3-Ethoxyphenyl)ethynyl]-5-(trifluoromethyl)benzoyl]piperazin-1-yl]-N-methylsulfonylpyridazine-3-carboxamide |
| B2I020-01 | 6-[4-[3-[2-(5-Ethoxypyridin-3-yl)ethynyl]-5-(trifluoromethyl)benzoyl]piperazin-1-yl]-N-methylsulfonylpyridazine-3-carboxamide |
| B2I021-01 | 6-[4-[3-[4-(3-Ethoxyphenyl)pyrazol-1-yl]-5-(trifluoromethyl)benzoyl]piperazin-1-yl]-N-methylsulfonylpyridazine-3-carboxamide |
| B2I022-01 | 6-[4-[3-[4-(5-Ethoxypyridin-3-yl)pyrazol-1-yl]-5-(trifluoromethyl)benzoyl]piperazin-1-yl]-N-methylsulfonylpyridazine-3 -carboxamide |
| B2I023-01 | 4-[4-[4-(3-Ethoxyphenyl)thiophene-2-carbonyl]piperazin-1-yl]-N-methylsulfonylbenzamide |
| B2I024-01 | 4-[4-[4-(5-Ethoxypyridin-3-yl)thiophene-2-carbonyl]piperazin-1-yl]-N-methylsulfonylbenzamide |
| B2I025-01 | 4-[4-[4-(3-Ethoxyphenyl)-3-methylbenzoyl]piperazin-1-yl]-N-methylsulfonylbenzamide |
| B2I026-01 | 4-[4-[4-(5-Ethoxypyridin-3-yl)-3-methylbenzoyl]piperazin-1-yl]-N-methylsulfonylbenzamide |
| B2I027-01 | 4-[4-[4-(3-Ethoxyphenyl)-3-fluorobenzoyl]piperazin-1-yl]-N-methylsulfonylbenzamide |
| B2I028-01 | 4-[4-[4-(5-Ethoxypyridin-3-yl)-3-fluorobenzoyl]piperazin-1-yl]-N-methylsulfonylbenzamide |
| B2I029-01 | 4-[4-[2-[2-(3-Ethoxyphenyl)ethynyl]benzoyl]piperazin-1-yl]-N-methylsulfonylbenzamide |
| B2I030-01 | 4-[4-[2-[2-(5-Ethoxypyridin-3-yl)ethynyl]benzoyl]piperazin-1-yl]-N-methylsulfonylbenzamide |
| B2I03-1-01 | 4-[4-[5-[2-(3-Ethoxyphenyl)ethynyl]pyridine-3-carbonyl]piperazin-1-yl]-N-methylsulfonylbenzamide |
| B2I032-01 | 4-[4-[5-[2-(5-Ethoxypyridin-3-yl)ethynyl]pyridine-3-carbonyl]piperazin-1-yl]-N-methylsulfonylbenzamide |

(continued)

| Mixture No. | mixAI-B2I-01 |
|---|---|
| Compound No. that may be included | Compound name |
| B2I033-01 | 4-[4-[3-[2-(3-Ethoxyphenyl)phenyl]propanoyl]piperazin-1-yl]-N-methylsulfonylbenzamide |
| B2I034-01 | 4-[4-[3-[2-(5-Ethoxypyridin-3-yl)phenyl]propanoyl]piperazin-1-yl]-N-methylsulfonylbenzamide |
| B2I035-01 | 4-[4-[4-(3-Ethoxyphenyl)naphthalene-1-carbonyl]piperazin-1-yl]-N-methylsulfonylbenzamide |
| B2I036-01 | 4-[4-[4-(5-Ethoxypyridin-3-yl)naphthalene-1-carbonyl]piperazin-1-yl]-N-methylsulfonylbenzamide |
| B2I037-01 | 4-[4-[3-tert-Butyl-5-(3-ethoxyphenyl)benzoyl]piperazin-1-yl]-N-methylsulfonylbenzamide |
| B2I038-01 | 4-[4-[3-tert-Butyl-5-(5-ethoxypyridin-3-yl)benzoyl]piperazin-1-yl]-N-methylsulfonylbenzamide |
| B2I039-01 | 4-[4-[3-(3-Ethoxyphenyl)-5-(trifluoromethyl)benzoyl]piperazin-1-yl]-N-methylsulfonylbenzamide |
| B2I040-01 | 4-[4-[3-(5-Ethoxypyridin-3-yl)-5-(trifluoromethyl)benzoyl]piperazin-1-yl]-N-methylsulfonylbenzamide |
| B2I041-01 | 4-[4-[3-[2-(3-Ethoxyphenyl)ethynyl]-5-(trifhioromethyl)benzoyl]piperazin-1-yl]-N-methylsulfonylbenzamide |
| B2I042-01 | 4-[4-[3-[2-(5-Ethoxypyridin-3-yl)ethynyl]-5-(trifluoromethyl)benzoyl]piperazin--1-yl]-N-methylsulfonylbenzamide |
| B21043-01 | 4-[4-[3-[4-(3-Ethoxyphenyl)pyrazol-1-yl]-5-(trifluoromethyl)benzoyl]piperazin-1-yl]-N-methylsulfonylbenzamide |
| B2I044-01 | 4-[4-[3-[4-(5-Ethoxypyridin-3-yl)pyrazol-1-yl]-5-(trifluoromethyl)benzoyl]piperazin-1-yl]-N-methylsulfonylbenzamide |
| B2I045-01 | 4-[4-[[2-(3-Ethoxyphenyl)phenyl]methyl]piperazin-1-yl]-N-methylsulfonylbenzamide |
| B2I046-01 | 4-[4-[[2-(5-Ethoxypyridin-3-yl)phenyl]methyl]piperazin-1-yl]-N-methylsulfonylbenzamide |
| B2I047-01 | 4-[4-[[4-(3-Ethoxyphenyl)-3-methylphenyl]methyl]piperazin-1-yl]-N-methylsulfonylbenzamide |
| B2I048-01 | 4-[4-[4-(5-Ehoxypyridin-3-yl)-3-methylphenyl]methyl]piperazin-1-yl]-N-methylsulfonylbenzamide |
| B2I049-01 | 4-[4-[[4-(3-Ethoxyphenyl)-2-ethylphenyl]methyl]piperazin-1-yl]-N-methylsulfonylbenzamide |
| B2I050-01 | 4-[4-[[4-(5-Ethoxypyridin-3-yl)-2-ethylphenyl]methyl]piperazin-1-yl]-N-methylsulfonylbenzamide |
| B2I051-01 | 4-[4-[[4-(3-Ethoxyphenyl)-3-fluorophenyl]methyl]piperazin-1-yl]-N-methylsulfonylbenzamide |
| B2I052-01 | 4-[4-[[4-(5-Ethoxypyridin-3-yl)-3-fluorophenyl]methyl]piperazin-1-yl]-N-methylsulfonylbenzamide |
| B2I053-01 | 4-[4-[[4-(3-Ethoxyphenyl)-2-methoxyphenyl]methyl]piperazin-1-yl]-N-methylsulfonylbenzamide |
| B2I054-01 | 4-[4-[[4-(5-Ethoxypyridin-3-yl)-2-methoxyphenyl]methyl]piperazin-1-yl]-N-methylsulfonylbenzamide |
| B2I055-01 | 4-[4-[[3-Ethoxy-5-(3-ethoxyphenyl)phenyl]methyl]piperazin-1-yl]-N-methylsulfonylbenzamide |

(continued)

| Mixture No. | mixAI-B2I-01 |
|---|---|
| Compound No. that may be included | Compound name |
| B2I056-01 | 4-[4-[[3-Ethoxy-5-(5-ethoxypyridin-3-yl)phenyl]methyl]piperazin-1-yl]-N-methylsulfonylbenzamide |
| B2I057-01 | 4-[4-[[3-(3-Ethoxyphenyl)-5-propan-2-yloxyphenyl]methyl]piperazin-1-yl]-N-methylsulfonylbenzamide |
| B2I058-01 | 4-[4-[[3-(5-Ethoxypyridin-3-yl)-5-propan-2-yloxyphenyl]methyl]piperazin-1-yl]-N-methylsulfonylbenzamide |
| B2I059-01 | 4-[4-[[4-(3-Ethoxyphenyl)-2,6-dimethoxyphenyl]methyl]piperazin-1-yl]-N-methylsulfonylbenzamide |
| B2I060-01 | 4-[4-[[4-(5-Ethoxypyridin-3-yl)-2,6-dimethoxyphenyl]methyl]piperazin-1-yl]-N-methylsulfonylbenzamide |
| B2I061-01 | 4-[4-[[3-(3-Ethoxyphenyl)-5-(trifluoromethyl)phenyl]methyl]piperazin-1-yl]-N-methylsulfonylbenzamide |
| B2I062-01 | 4-[4-[[3-(5-Ethoxypyridin-3-yl)-5-(trifluoromethyl)phenyl]methyl]piperazin-1-yl]-N-methylsulfonylbenzamide |
| B2I063-01 | 4-[4-[[3-(3-Ethoxyphenyl)-5-(trifluoromethoxy)phenyl]methyl]piperazin-1-yl]-N-methylsulfonylbenzamide |
| B2I064-01 | 4-[4-[[3-(5-Ethoxypyridin-3-yl)-5-(trifluoromethoxy)phenyl]methyl]piperazin-1-yl]-N-methylsulfonylbenzamide |
| B21065-01 | 4-[4-[[4-(3-Ethoxyphenyl)naphthalen-1-yl]methyl]piperazin-1-yl]-N-methylsulfonylbenzamide |
| B2I066-01 | 4-[4-[4-(5-Ehoxypyridin-3-yl)naphthalen-1-yl]methyl]piperazin-1-yl]-N-methylsulfonylbenzamide |
| B2I067-01 | 4-[4-[[4-(3-Ethoxyphenyl)thiophen-2-yl]methyl]piperazin-1-yl]-N-methylsulfonylbenzamide |
| B2I068-01 | 4-[4-[[4-(5-Ethoxypyridin-3-yl)thiophen-2-yl]methyl]piperazin-1-yl]-N-methylsulfonylbenzamide |
| B2I069-01 | 4-[4-[[2-[2-(3-Ethoxyphenyl)ethynyl]phenyl]methyl]piperazin-1-yl]-N-methylsulfonylbenzamide |
| B2I070-01 | 4-[4-[[2-[2-(5-Ethoxypyridin-3-yl)ethynyl]phenyl]methyl]piperazin-1-yl]-N-methylsulfonylbenzamide |
| B2I071-01 | 6-[4-[[2-(3-Ethoxyphenyl)phenyl]methyl]piperazin-1-yl]-N-methylsulfonylpyridazine-3-carboxamide |
| B2I072-01 | 6-[4-[[2-(5-Ethoxypyridin-3-yl)phenyl]methyl]piperazin-1-yl]-N-methylsulfonylpyridazine-3-carboxamide |
| B21073-01 | 6-[4-[[4-(3-Ethoxyphenyl)-3-methylphenyl]methyl]piperazin-1-yl]-N-methylsulfonylpyridazine-3-carboxamide |
| B2I074-01 | 6-[4-[[4-(5-Ethoxypyridin-3-yl)-3-methylphenyl]methyl]piperazin-1-yl]-N-methylsulfonylpyridazine-3-carboxamide |
| B21075-01 | 6-[4-[[4-(3-Ethoxyphenyl)-2-ethylphenyl]methyl]piperazin-1-yl]-N-methylsulfonylpyridazine-3-carboxamide |
| B2I076-01 | 6-[4-[[4-(5-Ethoxypyridin-3-yl)-2-ethylphenyl]methyl]piperazin-1-yl]-N-methylsulfonylpyridazine-3-carboxamide |

(continued)

| Mixture No. | mixAI-B2I-01 |
|---|---|
| Compound No. that may be included | Compound name |
| B2I077-01 | 6-[4-[[4-(3-Ethoxyphenyl)-3-fluorophenyl]methyl]piperazin-1-yl]-N-methylsulfonylpyridazine-3-carboxamide |
| B21078-01 | 6-[4-[[4-(5-Ethoxypyridin-3-yl)-3-fluorophenyl]methyl]piperazin-1-yl]-N-methylsulfonylpyridazine-3-carboxamide |
| B2I079-01 | 6-[4-[[4-(3-Ethoxyphenyl)-2-methoxyphenyl]methyl]piperazin-1-yl]-N-methylsulfonylpyridazine-3-carboxamide |
| B2I080-01 | 6-[4-[[4-(5-Ethoxypyridin-3-yl)-2-methoxyphenyl]methyl]piperazin-1-yl]-N-methylsulfonylpyridazine-3-carboxamide |
| B2I081-01 | 6-[4-[[3-Ethoxy-5-(3-ethoxyphenyl)phenyl]methyl]piperazin-1-yl]-N-methylsulfonylpyridazine-3-carboxamide |
| B2I082-01 | 6-[4-[[3-Ethoxy-5-(5-ethoxypyridin-3-yl)phenyl]methyl]piperazin-1-yl]-N-methylsulfonylpyridazine-3-carboxamide |
| B2I083-01 | 6-[4-[[3-(3-Ethoxyphenyl)-5-propan-2-yloxyphenyl]methyl]piperazin-1-yl]-N-methylsulfonylpyridazine-3-carboxamide |
| B2I084-01 | 6-[4-[[3-(5-Ethoxypyridin-3-yl)-5-propan-2-yloxyphenyl]methyl]piperazin-1-yl]-N-methylsulfonylpyridazine-3-carboxamide |
| B2I085-01 | 6-[4-[[4-(3-Ethoxyphenyl)-2,6-dimethoxyphenyl]methyl]piperazin-1-yl]-N-methylsulfonylpyridazine-3-carboxamide |
| B2I086-01 | 6-[4-[[4-(5-Ethoxypyridin-3-yl)-2,6-dimethoxyphenyl]methyl]piperazin-1-yl]-N-methylsulfonylpyridazine-3-carboxamide |
| B2I087-01 | 6-[4-[[3-(3-Ethoxyphenyl)-5-(trifluoromethyl)phenyl]methyl]piperazin-1-yl]-N-methylsulfonylpyridazine-3-carboxamide |
| B2I088-01 | 6-[4-[[3-(5-Ethoxypyridin-3-yl)-5-(trifluoromethyl)phenyl]methyl]piperazin-1-yl]-N-methylsulfonylpyridazine-3-carboxamide |
| B2I089-01 | 6-[4-[[3-(3-Ethoxyphenyl)-5-(trifluoromethoxy)phenyl]methyl]piperazin-1-yl]-N-methylsulfonylpyridazine-3-carboxamide |
| B2I090-01 | 6-[4-[[3-(5-Ethoxypyridin-3-yl)-5-(trifluoromethoxy)phenyl]methyl]piperazin-1-yl]-N-methylsulfonylpyridazine-3-carboxamide |
| B2I091-01 | 6-[4-[[4-(3-Ethoxyphenyl)naphthalen-1-yl]methyl]piperazin-1-yl]-N-methylsulfonylpyridazine-3-carboxamide |
| B2I092-01 | 6-[4-[[4-(5-Ethoxypyridin-3-yl)naphthalen-1-yl]methyl]piperazin-1-yl]-N-methylsulfonylpyridazine-3-carboxamide |
| B21093-01 | 6-[4-[[4-(3-Ethoxyphenyl)thiophen-2-yl]methyl]piperazin-1-yl]-N-methylsulfonylpyridazine-3-carboxamide |
| B2I094-01 | 6-[4-[[4-(5-Ethoxypyridin-3-yl)thiophen-2-yl]methyl]piperazin-1-yl]-N-methylsulfonylpyridazine-3-carboxamide |
| B21095-01 | 6-[4-[[2-[2-(3-Ethoxyphenyl)ethynyl]phenyl]methyl]piperazin-1-yl]-N-methylsulfonylpyridazine-3-carboxamide |
| B2I096-01 | 6-[4-[[2-[2-(5-Ethoxypyridin-3-yl)ethynyl]phenyl]methyl]piperazin-1-yl]-N-methylsulfonylpyridazine-3-carboxamide |
| B2I097-01 | 4-[4-[1-[4-(3-Ethoxyphenyl)phenyl]ethyl]piperazin-1-yl]-N-methylsulfonylbenzamide |
| B2I098-01 | 4-[4-[1-[4-(5-Ethoxypyridin-3-yl)phenyl]ethyl]piperazin-1-yl]-N-methylsulfonylbenzamide |

(continued)

| Mixture No. | mixAI-B2I-01 |
|---|---|
| Compound No. that may be included | Compound name |
| B2I099-01 | 4-[4-[1-[4-(3-Ethoxyphenyl)-2-fluorophenyl]ethyl]piperazin-1-yl]-N-methylsulfonylbenzamide |
| B2I100-01 | 4-[4-[1-[4-(5-Ethoxypyridin-3-yl)-2-fluorophenyl] ethyl]piperazin-1-yl]-N-methylsulfonylbenzamide |

[Table 104]

[Table AI-03-B2L-01]

| Mixture No. | mixAI-B2L-01 |
|---|---|
| Compound No. that may be included | Compound name |
| B2L001-01 | N-tert-Butylsulfonyl-6-[4-[4-(3-ethoxyphenyl)thiophene-2-carbonyl]piperazin-1- yl]pyridazine-3-carboxamide |
| B2L002-01 | N-tert-Butylsulfonyl-6-[4-[4-(5-ethoxypyridin-3-yl)thiophene-2-carbonyl]piperazin-1-yl]pyridazine-3 -carboxamide |
| B2L003-01 | N-tert-Butylsulfonyl-6-[4-[4-(3-ethoxyphenyl)-3-methylbenzoyl]piperazin-1-yl]pyridazine-3-carboxamide |
| B2L004-01 | N-tert-Butylsulfonyl-6-[4-[4-(5-ethoxypyridin-3-yl)-3-methylbenzoyl]piperazin-1-yl]pyridazine-3-carboxamide |
| B2L005-01 | N-tert-Butylsulfonyl-6-[4-[4-(3-ethoxyphenyl)-3-fluorobenzoyl]piperazin-1-yl]pyridazine-3-carboxamide |
| B2L006-01 | N-tert-Butylsulfonyl-6-[4-[4-(5-ethoxypyridin-3-yl)-3-fluorobenzoyl]piperazin-1-yl]pyridazine-3-carboxamide |
| B2L007-01 | N-tert-Butylsulfonyl-6-[4-[2-[2-(3-ethoxyphenyl)ethynyl]benzoyl]piperazin-1-yl]pyridazine-3-carboxamide |
| B2L008-01 | N-tert-Butylsulfonyl-6-[4-[2-[2-(5-ethoxypyridin-3-yl)ethynyl]benzoyl]piperazin-1-yl]pyridazine-3-carboxamide |
| B2L009-01 | N-tert-Butylsulfonyl-6-[4-[5-[2-(3-ethoxyphenyl)ethynyl]pyridine-3-carbonyl]piperazin-1-yl]pyridazine-3-carboxamide |
| B2L010-01 | N-tert-Butylsulfonyl-6-[4-[5-[2-(5-ethoxypyridin-3-yl)ethynyl]pyridine-3-carbonyl]piperazin-1-yl]pyridazine-3-carboxamide |
| B2L011-01 | N-tert-Butylsulfonyl-6-[4-[3-[2-(3-ethoxyphenyl)phenyl]propanoyl]piperazin-1-yl]pyridazine-3-carboxamide |
| B2L012-01 | N-tert-Butylsulfonyl-6-[4-[3-[2-(5-ethoxypyridin-3-yl)phenyl]propanoyl]piperazin-1-yl]pyridazine-3-carboxamide |
| B2L013-01 | N-tert-Butylsulfonyl-6-[4-[4-(3-ethoxyphenyl)naphthalene-1-carbonyl]piperazin-1-yl]pyridazine-3-carboxamide |
| B2L014-01 | N-tert-Butylsulfonyl-6-[4-[4-(5-ethoxypyridin-3-yl)naphthalene-1-carbonyl]piperazin-1-yl]pyridazine-3-carboxamide |

(continued)

| Mixture No. | mixAI-B2L-01 |
| --- | --- |
| Compound No. that may be included | Compound name |
| B2L015-01 | 6-[4-[3-tert-Butyl-5-(3-ethoxyphenyl)benzoyl]piperazin-1-yl]-N-tert-butylsulfonylpyridazine-3-carboxamide |
| B2L016-01 | 6-[4-[3-tert-Butyl-5-(5-ethoxypyridin-3-yl)benzoyl]piperazin-1-yl]-N-tert-butylsulfonylpyridazine-3-carboxamide |
| B2L017-01 | N-tert-Butylsulfonyl-6-[4-[3-(3-ethoxyphenyl)-5-(trifluoromethyl)benzoyl]piperazin-1-yl]pyridazine-3-carboxamide |
| B2L018-01 | N-tert-Butylsulfonyl-6-[4-[3-(5-ethoxypyridin-3-yl)-5-(trifluoromethyl)benzoyl]piperazin-1-yl]pyridazine-3-carboxamide |
| B2L019-01 | N-tert-Butylsulfonyl-6-[4-[3-[2-(3-ethoxyphenyl)ethynyl]-5-(trifluoromethyl)benzoyl]piperazin-1-yl]pyridazine-3-carboxamide |
| B2L020-01 | N-tert-Butylsulfonyl-6-[4-[3-[2-(5-ethoxypyridin-3-yl)ethynyl]-5-(trifluoromethyl)benzoyl]piperazin-1-yl]pyridazine-3-carboxamide |
| B2L021-01 | N-tert-Butylsulfonyl-6-[4-[3-[4-(3-ethoxyphenyl)pyrazol-1-yl]-5-(trifluoromethyl)benzoyl]piperazin-1-yl]pyridazine-3-carboxamide |
| B2L022-01 | N-tert-Butylsulfonyl-6-[4-[3-[4-(5-ethoxypyridin-3-yl)pyrazol-1-yl]-5-(trifluoromethyl)benzoyl]piperazin-1-yl]pyridazine-3-carboxamide |
| B2L023-01 | N-tert-Butylsulfonyl-4-[4-[4-(3-ethoxyphenyl)thiophene-2-carbonyl]piperazin-1-yl]benzamide |
| B2L024-01 | N-tert-Butylsulfonyl-4-[4-[4-(5-ethoxypyridin-3-yl)thiophene-2-carbonyl]piperazin-1-yl]benzamide |
| B2L025-01 | N-tert-Butylsulfonyl-4-[4-[4-(3-ethoxyphenyl)-3-methylbenzoyl]piperazin-1-yl]benzamide |
| B2L026-01 | N-tert-Butylsulfonyl-4-[4-[4-(5-ethoxypyridin-3-yl)-3-methylbenzoyl]piperazin-1-yl]benzamide |
| B2L027-01 | N-tert-Butylsulfonyl-4-[4-[4-(3-ethoxyphenyl)-3-fluorobenzoyl]piperazin-1-yl]benzamide |
| B2L028-01 | N-tert-Butylsulfonyl-4-[4-[4-(5-ethoxypyridin-3-yl)-3-fluorobenzoyl]piperazin-1-yl]benzamide |
| B2L029-01 | N-tert-Butylsulfonyl-4-[4-[2-[2-(3-ethoxyphenyl)ethynyl]benzoyl]piperazin-1-yl]benzamide |
| B2L030-01 | N-tert-Butylsulfonyl-4-[4-[2-[2-(5-ethoxypyridin-3-yl)ethynyl]benzoyl]piperazin-1-yl]benzamide |
| B2L031-01 | N-tert-Butylsulfonyl-4-[4-[5-[2-(3-ethoxyphenyl)ethynyl]pyridine-3-carbonyl]piperazin-1-yl]benzamide |
| B2L032-01 | N-tert-Butylsulfonyl-4-[4-[5-[2-(5-ethoxypyridin-3-yl)ethynyl]pyridine-3-carbonyl]piperazin-1-yl]benzamide |
| B2L033-01 | N-tert-Butylsulfonyl-4-[4-[3-[2-(3-ethoxyphenyl)phenyl]propanoyl]piperazin-1-yl]benzamide |
| B2L034-01 | N-tert-Butylsulfonyl-4-[4-[3-[2-(5-ethoxypyridin-3-yl)phenyl]propanoyl]piperazin-1-yl]benzamide |
| B2L035-01 | N-tert-Butylsulfonyl-4-[4-[4-(3-ethoxyphenyl)naphthalene-1-carbonyl]piperazin-1-yl]benzamide |
| B2L036-01 | N-tert-Butylsulfonyl-4-[4-[4-(5-ethoxypyridin-3-yl)naphthalene-1-carbonyl]piperazin-1-yl]benzamide |
| B2L037-01 | 4-[4-[3-tert-Butyl-5-(3-ethoxyphenyl)benzoyl]piperazin-1-yl]-N-tert-butylsulfonylbenzamide |
| B2L038-01 | 4-[4-[3-tert-Butyl-5-(5-ethoxypyridin-3-yl)benzoyl]piperazin-1-yl]-N-tert-butylsulfonylbenzamide |

(continued)

| Mixture No. | mixAI-B2L-01 |
|---|---|
| Compound No. that may be included | Compound name |
| B2L039-01 | N-tert-Butylsulfonyl-4-[4-[3-(3-ethoxyphenyl)-5-(trifluoromethyl)benzoyl]piperazin-1-yl]benzamide |
| B2L040-01 | N-tert-Butylsulfonyl-4-[4-[3-(5-ethoxypyridin-3-yl)-5-(trifluoromethyl)benzoyl]piperazin-1-yl]benzamide |
| B2L041-01 | N-tert-Butylsulfonyl-4-[4-[3-[2-(3-ethoxyphenyl)ethynyl]-5-(trifluoromethyl)benzoyl]piperazin-1-yl]benzamide |
| B2L042-01 | N-tert-Butylsulfonyl-4-[4-[3-[2-(5-ethoxypyridin-3-yl)ethynyl]-5-(trifluoromethyl)benzoyl]piperazin-1-yl]benzamide |
| B2L043-01 | N-tert-Butylsulfonyl-4-[4-[3-[4-(3-ethoxyphenyl)pyrazol-1-yl]-5-(trifluoromethyl)benzoyl]piperazin-1-yl]benzamide |
| B2L044-01 | N-tert-Butylsulfonyl-4-[4-[3-[4-(5-ethoxypyridin-3-yl)pyrazol-1-yl]-5-(trifluoromethyl)benzoyl]piperazin-1-yl]benzamide |
| B2L045-01 | N-tert-Butylsulfonyl-4-[4-[[2-(3-ethoxyphenyl)phenyl]methyl]piperazin-1-yl]benzamide |
| B2L046-01 | N-tert-Butylsulfonyl-4-[4-[[2-(5-ethoxypyridin-3-yl)phenyl]methyl]piperazin-1-yl]benzamide |
| B2L047-01 | N-tert-Butylsulfonyl-4-[4-[[4-(3-ethoxyphenyl)-3-methylphenyl]methyl]piperazin-1-yl]benzamide |
| B2L048-01 | N-tert-Butylsulfonyl-4-[4-[[4-(5-ethoxypyridin-3-yl)-3-methylphenyl]methyl]piperazin-1-yl]benzamide |
| B2L049-01 | N-tert-Butylsulfonyl-4-[4-[[4-(3-ethoxyphenyl)-2-ethylphenyl]methyl]piperazin-1-yl]benzamide |
| B2L050-01 | N-tert-Butylsulfonyl-4-[4-[[4-(5-ethoxypyridin-3-yl)-2-ethylphenyl]methyl]piperazin-1-yl]benzamide |
| B2L051-01 | N-tert-Butylsulfonyl-4-[4-[[4-(3-ethoxyphenyl)-3-fluorophenyl]methyl]piperazin-1-yl]benzamide |
| B2L052-01 | N-tert-Butylsulfonyl-4-[4-[[4-(5-ethoxypyridin-3-yl)-3-fluorophenyl]methyl]piperazin-1-yl]benzamide |
| B2L053-01 | N-tert-Butylsulfonyl-4-[4-[[4-(3-ethoxyphenyl)-2-methoxyphenyl]methyl]piperazin-1-yl]benzamide |
| B2L054-01 | N-tert-Butylsulfonyl-4-[4-[[4-(5-ethoxypyridin-3-yl)-2-methoxyphenyl]methyl]piperazin-1-yl]benzamide |
| B2L055-01 | N-tert-Butylsulfonyl-4-[4-[[3-ethoxy-5-(3-ethoxyphenyl)phenyl]methyl]piperazin-1-yl]benzamide |
| B2L056-01 | N-tert-Butylsulfonyl-4-[4-[[3-ethoxy-5-(5-ethoxypyridin-3-yl)phenyl]methyl]piperazin-1-yl]benzamide |
| B2L057-01 | N-tert-Butylsulfonyl-4-[4-[[3-(3-ethoxyphenyl)-5-propan-2-yloxyphenyl]methyl]piperazin-1-yl]benzamide |
| B2L058-01 | N-tert-Butylsulfonyl-4-[4-[[3-(5-ethoxypyridin-3-yl)-5-propan-2-yloxyphenyl]methyl]piperazin-1-yl]benzamide |
| B2L059-01 | N-tert-Butylsulfonyl-4-[4-[[4-(3-ethoxyphenyl)-2,6-dimethoxyphenyl]methyl]piperazin-1-yl]benzamide |
| B2L060-01 | N-tert-Butylsulfonyl-4-[4-[[4-(5-ethoxypyridin-3-yl)-2,6-dimethoxyphenyl]methyl]piperazin-1-yl]benzamide |

(continued)

| Mixture No. | mixAI-B2L-01 |
|---|---|
| Compound No. that may be included | Compound name |
| B2L061-01 | N-tert-Butylsulfonyl-4-[4-[[3-(3-ethoxyphenyl)-5-(trifluoromethyl)phenyl]methyl]piperazin-1-yl]benzamide |
| B2L062-01 | N-tert-Butylsulfonyl-4-[4-[[3-(5-ethoxypyridin-3-yl)-5-(trifluoromethyl)phenyl]methyl]piperazin-1-yl]benzamide |
| B2L063-01 | N-tert-Butylsulfonyl-4-[4-[[3-(3-ethoxyphenyl)-5-(trifluoromethoxy)phenyl]methyl]piperazin-1-yl]benzamide |
| B2L064-01 | N-tert-Butylsulfonyl-4-[4-[[3-(5-ethoxypyridin-3-yl)-5-(trifluoromethoxy)phenyl]methyl]piperazin-1-yl]benzamide |
| B2L065-01 | N-tert-Butylsulfonyl-4-[4-[[4-(3-ethoxyphenyl)naphthalen-1-yl]methyl]piperazin-1-yl]benzamide |
| B2L066-01 | N-tert-Butylsulfonyl-4-[4-[[4-(5-ethoxypyridin-3-yl)naphthalen-1-yl]methyl]piperazin-1-yl]benzamide |
| B2L067-01 | N-tert-Butylsulfonyl-4-[4-[[4-(3-ethoxyphenyl)thiophen-2-yl]methyl]piperazin-1- yl]benzamide |
| B2L068-01 | N-tert-Butylsulfonyl-4-[4-[[4-(5-ethoxypyridin-3-yl)thiophen-2-yl]methyl]piperazin-1-yl]benzamide |
| B2L069-01 | N-tert-Butylsulfonyl-4-[4-[[2-[2-(3-ethoxyphenyl)ethynyl]phenyl]methyl]piperazin-1-yl]benzamide |
| B2L070-01 | N-tert-Butylsulfonyl-4-[4-[[2-[2-(5-ethoxypyridin-3-yl)ethynyl]phenyl]methyl]piperazin-1-yl]benzamide |
| B2L071-01 | N-tert-Butylsulfonyl-6-[4-[[2-(3-ethoxyphenyl)phenyl]methyl]piperazin-1-yl]pyridazine-3-carboxamide |
| B2L072-01 | N-tert-Butylsulfonyl-6-[4-[[2-(5-ethoxypyridin-3-yl)phenyl]methyl]piperazin-1-yl]pyridazine-3-carboxamide |
| B2L073-01 | N-tert-Butylsulfonyl-6-[4-[[4-(3-ethoxyphenyl)-3-methylphenyl]methyl]piperazin-1-yl]pyridazine-3-carboxamide |
| B2L074-01 | N-tert-Butylsulfonyl-6-[4-[[4-(5-ethoxypyridin-3-yl)-3-methylphenyl]methyl]piperazin-1-yl]pyridazine-3-carboxamide |
| B2L075-01 | N-tert-Butylsulfonyl-6-[4-[[4-(3-ethoxyphenyl)-2-ethylphenyl]methyl]piperazin-1-yl]pyridazine-3-carboxamide |
| B2L076-01 | N-tert-Butylsulfonyl-6-[4-[[4-(5-ethoxypyridin-3-yl)-2-ethylphenyl]methyl]piperazin-1-yl]pyridazine-3-carboxamide |
| B2L077-01 | N-tert-Butylsulfonyl-6-[4-[[4-(3-ethoxyphenyl)-3-fluorophenyl]methyl]piperazin-1-yl]pyridazine-3-carboxamide |
| B2L078-01 | N-tert-Butylsulfonyl-6-[4-[[4-(5-ethoxypyridin-3-yl)-3-fluorophenyl]methyl]piperazin-1-yl]pyridazine-3-carboxamide |
| B2L079-01 | N-tert-Butylsulfonyl-6-[4-[[4-(3-ethoxyphenyl)-2-methoxyphenyl]methyl]piperazin-1-yl]pyridazine-3-carboxamide |
| B2L080-01 | N-tert-Butylsulfonyl-6-[4-[[4-(5-ethoxypyridin-3-yl)-2-methoxyphenyl]methyl]piperazin-1-yl]pyridazine-3-carboxamide |
| B2L081-01 | N-tert-Butylsulfonyl-6-[4-[[3-ethoxy-5-(3-ethoxyphenyl)phenyl]methyl]piperazin-1-yl]pyridazine-3-carboxamide |

(continued)

| Mixture No. | mixAI-B2L-01 |
|---|---|
| Compound No. that may be included | Compound name |
| B2L082-01 | N-tert-Butylsulfonyl-6-[4-[[3-ethoxy-5-(5-ethoxypyridin-3-yl)phenyl]methyl]piperazin-1-yl]pyridazine-3-carboxamide |
| B2L083-01 | N-tert-Butylsulfonyl-6-[4-[[3-(3-ethoxyphenyl)-5-propan-2-yloxyphenyl]methyl]piperazin-1-yl]pyridazine-3-carboxamide |
| B2L084-01 | N-tert-Butylsulfonyl-6-[4-[[3-(5-ethoxypyridin-3-yl)-5-propan-2-yloxyphenyl]methyl]piperazin-1-yl]pyridazine-3-carboxamide |
| B2L085-01 | N-tert-Butylsulfonyl-6-[4-[[4-(3-ethoxyphenyl)-2,6-dimethoxyphenyl]methyl]piperazin-1-yl]pyridazine-3-carboxamide |
| B2L086-01 | N-tert-Butylsulfonyl-6-[4-[[4-(5-ethoxypyridin-3-yl)-2,6-dimethoxyphenyl]methyl]piperazin-1-yl]pyridazine-3-carboxamide |
| B2L087-01 | N-tert-Butylsulfonyl-6-[4-[[3-(3-ethoxyphenyl)-5-(trifluoromethyl)phenyl]methyl]piperazin-1-yl]pyridazine-3-carboxamide |
| B2L088-01 | N-tert-Butylsulfonyl-6-[4-[[3-(5-ethoxypyridin-3-yl)-5-(trifluoromethyl)phenyl]methyl]piperazin-1-yl]pyridazine-3-carboxamide |
| B2L089-01 | N-tert-Butylsulfonyl-6-[4-[[3-(3-ethoxyphenyl)-5-(trifluoromethoxy)phenyl]methyl]piperazin-1-yl]pyridazine-3-carboxamide |
| B2L090-01 | N-tert-Butylsulfonyl-6-[4-[[3-(5-ethoxypyridin-3-yl)-5-(trifluoromethoxy)phenyl]methyl]piperazin-1-yl]pyridazine-3-carboxamide |
| B2L091-01 | N-tert-Butylsulfonyl-6-[4-[[4-(3-ethoxyphenyl)naphthalen-1-yl]methyl]piperazin-1-yl]pyridazine-3-carboxamide |
| B2L092-01 | N-tert-Butylsulfonyl-6-[4-[[4-(5-ethoxypyridin-3-yl)naphthalen-1-yl]methyl]piperazin-1-yl]pyridazine-3-carboxamide |
| B2L093-01 | N-tert-Butylsulfonyl-6-[4-[[4-(3-ethoxyphenyl)thiophen-2-yl]methyl]piperazin-1- yl]pyridazine-3-carboxamide |
| B2L094-01 | N-tert-Butylsulfonyl-6-[4-[[4-(5-ethoxypyridin-3-yl)thiophen-2-yl]methyl]piperazin-1-yl]pyridazine-3-carboxamide |
| B2L095-01 | N-tert-Butylsulfonyl-6-[4-[[2-[2-(3-ethoxyphenyl)ethynyl]phenyl]methyl]piperazin-1-yl]pyridazine-3-carboxamide |
| B2L096-01 | N-tert-Butylsulfonyl-6-[4-[[2-[2-(5-ethoxypyridin-3-yl)ethynyl]phenyl]methyl]piperazin-1-yl]pyridazine-3-carboxamide |
| B2L097-01 | N-tert-Butylsulfonyl-4-[4-[1-[4-(3-ethoxyphenyl)phenyl]ethyl]piperazin-1- yl]benzamide |
| B2L098-01 | N-tert-Butylsulfonyl-4-[4-[1-[4-(5-ethoxypyridin-3-yl)phenyl]ethyl]piperazin-1-yl]benzamide |
| B2L099-01 | N-tert-Butylsulfonyl-4-[4-[1-[4-(3-ethoxyphenyl)-2-fluorophenyl]ethyl]piperazin-1-yl]benzamide |
| B2L100-01 | N-tert-Butylsulfonyl-4-[4-[1-[4-(5-ethoxypyridin-3-yl)-2-fluorophenyl]ethyl]piperazin-1-yl]benzamide |

[Table 105]

[Table AI-03-B2N-01]

| Mixture No. | mixAI-B2N-01 |
|---|---|
| Compound No. that may be included | Compound name |
| B2N001-01 | 6-[4-[4-(3-Ethoxyphenyl)thiophene-2-carbonyl]piperazin-1-yl]-N-(3,3,3-trifluoropropylsulfonyl)pyridazine-3-carboxamide |
| B2N002-01 | 6-[4-[4-(5-Ethoxypyridin-3-yl)thiophene-2-carbonyl]piperazin-1-yl]-N-(3,3,3-trifluoropropylsulfonyl)pyridazine-3-carboxamide |
| B2N003-01 | 6-[4-[4-(3-Ethoxyphenyl)-3-methylbenzoyl]piperazin-1-yl]-N-(3,3,3-trifluoropropylsulfonyl)pyridazine-3-carboxamide |
| B2N004-01 | 6-[4-[4-(5-Ethoxypyridin-3-yl)-3-methylbenzoyl]piperazin-1-yl]-N-(3,3,3-trifluoropropylsulfonyl)pyridazine-3-carboxamide |
| B2N005-01 | 6-[4-[4-(3-Ethoxyphenyl)-3-fluorobenzoyl]piperazin-1-yl]-N-(3,3,3-trifluoropropylsulfonyl)pyridazine-3-carboxamide |
| B2N006-01 | 6-[4-[4-(5-Ethoxypyridin-3-yl)-3-fluorobenzoyl]piperazin-1-yl]-N-(3,3,3-trifluoropropylsulfonyl)pyridazine-3-carboxamide |
| B2N007-01 | 6-[4-[2-[2-(3-Ethoxyphenyl)ethynyl]benzoyl]piperazin-1-yl]-N-(3,3,3-trifluoropropylsulfonyl)pyridazine-3-carboxamide |
| B2N008-01 | 6-[4-[2-[2-(5-Ethoxypyridin-3-yl)ethynyl]benzoyl]piperazin-1-yl]-N-(3,3,3-trifluoropropylsulfonyl)pyridazine-3-carboxamide |
| B2N009-01 | 6-[4-[5-[2-(3-Ethoxyphenyl)ethynyl]pyridine-3-carbonyl]piperazin-1-yl]-N-(3,3,3-trifluoropropylsulfonyl)pyridazine-3-carboxamide |
| B2N010-01 | 6-[4-[5-[2-(5-Ethoxypyridin-3-yl)ethynyl]pyridine-3-carbonyl]piperazin-1-yl]-N-(3,3,3-trifluoropropylsulfonyl)pyridazine-3-carboxamide |
| B2N011-01 | 6-[4-[3-[2-(3-Ethoxyphenyl)phenyl]propanoyl]piperazin-1-yl]-N-(3,3,3-trifluoropropylsulfonyl)pyridazine-3-carboxamide |
| B2N012-01 | 6-[4-[3-[2-(5-Ethoxypyridin-3-yl)phenyl]propanoyl]piperazin-1-yl]-N-(3,3,3-trifluoropropylsulfonyl)pyridazine-3-carboxamide |
| B2N013-01 | 6-[4-[4-(3-Ethoxyphenyl)naphthalene-1-carbonyl]piperazin-1-yl]-N-(3,3,3-trifluoropropylsulfonyl)pyridazine-3-carboxamide |
| B2N014-01 | 6-[4-[4-(5-Ethoxypyridin-3-yl)naphthalene-1-carbonyl]piperazin-1-yl]-N-(3,3,3-trifluoropropylsulfonyl)pyridazine-3-carboxamide |
| B2N015-01 | 6-[4-[3-tert-Butyl-5-(3-ethoxyphenyl)benzoyl]piperazin-1-yl]-N-(3,3,3-trifluoropropylsulfonyl)pyridazine-3-carboxamide |
| B2N016-01 | 6-[4-[3-tert-Butyl-5-(5-ethoxypyridin-3-yl)benzoyl]piperazin-1-yl]-N-(3,3,3-trifluoropropylsulfonyl)pyridazine-3-carboxamide |
| B2N017-01 | 6-[4-[3-(3-Ethoxyphenyl)-5-(trifluoromethyl)benzoyl]piperazin-1-yl]-N-(3,3,3-trifluoropropylsulfonyl)pyridazine-3-carboxamide |
| B2N018-01 | 6-[4-[3-(5-Ethoxypyridin-3-yl)-5-(trifluoromethyl)benzoyl]piperazin-1-yl]-N-(3,3,3-trifluoropropylsulfonyl)pyridazine-3-carboxamide |
| B2N019-01 | 6-[4-[3-[2-(3-Ethoxyphenyl)ethynyl]-5-(trifluoromethyl)benzoyl]piperazin-1-yl]-N-(3,3,3-trifluoropropylsulfonyl)pyridazine-3-carboxamide |
| B2N020-01 | 6-[4-[3-[2-(5-Ethoxypyridin-3-yl)ethynyl]-5-(trifluoromethyl)benzoyl]piperazin-1-yl]-N-(3,3,3-trifluoropropylsulfonyl)pyridazine-3-carboxamide |
| B2N021-01 | 6-[4-[3-[4-(3 -Ethoxyphenyl)pyrazol-1-yl]-5-(trifluoromethyl)benzoyl]piperazin-1-yl]-N-(3,3,3-trifluoropropylsulfonyl)pyridazine-3-carboxamide |

(continued)

| Mixture No. | mixAI-B2N-01 |
|---|---|
| Compound No. that may be included | Compound name |
| B2N022-01 | 6-[4-[3-[4-(5-Ethoxypyridin-3-yl)pyrazol-1-yl]-5-(trifluoromethyl)benzoyl]piperazin-1 -yl] -N-(3,3,3-trifluoropropylsulfonyl)pyridazine-3-carboxamide |
| B2N023-01 | 4-[4-[4-(3-Ethoxyphenyl)thiophene-2-carbonyl]piperazin-1-yl]-N-(3,3,3-trifluoropropylsulfonyl)benzamide |
| B2N024-01 | 4-[4-[4-(5-Ethoxypyridin-3-yl)thiophene-2-carbonyl]piperazin-1-yl]-N-(3,3,3-trifluoropropylsulfonyl)benzamide |
| B2N025-01 | 4-[4-[4-(3-Ethoxyphenyl)-3-methylbenzoyl]piperazin-1-yl]-N-(3,3,3-trifluoropropylsulfonyl)benzamide |
| B2N026-01 | 4-[4-[4-(5-Ethoxypyridin-3-yl)-3-methylbenzoyl]piperazin-1-yl]-N-(3,3,3-trifluoropropylsulfonyl)benzamide |
| B2N027-01 | 4-[4-[4-(3-Ethoxyphenyl)-3-fluorobenzoyl]piperazin-1-yl]-N-(3,3,3-trifluoropropylsulfonyl)benzamide |
| B2N028-01 | 4-[4-[4-(5-Ethoxypyridin-3-yl)-3-fluorobenzoyl]piperazin-1-yl]-N-(3,3,3-trifluoropropylsulfonyl)benzamide |
| B2N029-01 | 4-[4-[2-[2-(3-Ethoxyphenyl)ethynyl]benzoyl]piperazin-1-yl]-N-(3,3,3-trifluoropropylsulfonyl)benzamide |
| B2N030-01 | 4-[4-[2-[2-(5-Ethoxypyridin-3-yl)ethynyl]benzoyl]piperazin-1-yl]-N-(3,3,3-trifluoropropylsulfonyl)benzamide |
| B2N031-01 | 4-[4-[5-[2-(3-Ethoxyphenyl)ethynyl]pyridine-3-carbonyl]piperazin-1-yl]-N-(3,3,3-trifluoropropylsulfonyl)benzamide |
| B2N032-01 | 4-[4-[5-[2-(5-Ethoxypyridin-3-yl)ethynyl]pyridine-3-carbonyl]piperazin-1-yl]-N-(3,3,3-trifluoropropylsulfonyl)benzamide |
| B2N033-01 | 4-[4-[3-[2-(3-Ethoxyphenyl)phenyl]propanoyl]piperazin-1-yl]-N-(3,3,3-trifluoropropylsulfonyl)benzamide |
| B2N034-01 | 4-[4-[3-[2-(5-Ethoxypyridin-3-yl)phenyl]propanoyl]piperazin-1-yl]-N-(3,3,3-trifluoropropylsulfonyl)benzamide |
| B2N035-01 | 4-[4-[4-(3-Ethoxyphenyl)naphthalene-1-carbonyl]piperazin-1-yl]-N-(3,3,3-trifluoropropylsulfonyl)benzamide |
| B2N036-01 | 4-[4-[4-(5-Ethoxypyridin-3-yl)naphthalene-1-carbonyl]piperazin-1-yl]-N-(3,3,3-trifluoropropylsulfonyl)benzamide |
| B2N037-01 | 4-[4-[3-tert-Butyl-5-(3-ethoxyphenyl)benzoyl]piperazin-1-yl]-N-(3,3,3-trifluoropropylsulfonyl)benzamide |
| B2N038-01 | 4-[4-[3-tert-Butyl-5-(5-ethoxypyridin-3-yl)benzoyl]piperazin-1-yl]-N-(3,3,3-trifluoropropylsulfonyl)benzamide |
| B2N039-01 | 4-[4-[3-(3-Ethoxyphenyl)-5-(trifluoromethyl)benzoyl]piperazin-1-yl]-N-(3,3,3-trifluoropropylsulfonyl)benzamide |
| B2N040-01 | 4-[4-[3-(5-Ethoxypyridin-3-yl)-5-(trifluoromethyl)benzoyl]piperazin-1-yl]-N-(3,3,3-trifluoropropylsulfonyl)benzamide |
| B2N041-01 | 4-[4-[3-[2-(3-Ethoxyphenyl)ethynyl]-5-(trifluoromethyl)benzoyl]piperazin-1-yl]-N-(3,3,3-trifluoropropylsulfonyl)benzamide |
| B2N042-01 | 4-[4-[3-[2-(5-Ethoxypyridin-3-yl)ethynyl]-5-(trifluoromethyl)benzoyl]piperazin-1-yl]-N-(3,3,3-trifluoropropylsulfonyl)benzamide |

(continued)

| Mixture No. | mixAI-B2N-01 |
|---|---|
| Compound No. that may be included | Compound name |
| B2N043-01 | 4-[4-[3-[4-(3-Ethoxyphenyl)pyrazol-1-yl]-5-(trifluoromethyl)benzoyl]piperazin-1-yl]-N-(3,3,3-trifluoropropylsulfonyl)benzamide |
| B2N044-01 | 4-[4-[3-[4-(5-Ethoxypyridin-3-yl)pyrazol-1-yl]-5-(trifluoromethyl)benzoyl]piperazin-1-yl]-N-(3,3,3-trifluoropropylsulfonyl)benzamide |
| B2N045-01 | 4-[4-[[2-(3-Ethoxyphenyl)phenyl]methyl]piperazin-1-yl]-N-(3,3,3-trifluoropropylsulfonyl)benzamide |
| B2N046-01 | 4-[4-[[2-(5-Ethoxypyridin-3-yl)phenyl]methyl]piperazin-1-yl]-N-(3,3,3-trifluoropropylsulfonyl)benzamide |
| B2N047-01 | 4-[4-[[4-(3-Ethoxyphenyl)-3-methylphenyl]methyl]piperazin-1-yl]-N-(3,3,3-trifluoropropylsulfonyl)benzamide |
| B2N048-01 | 4-[4-[[4-(5-Ethoxypyridin-3-yl)-3-methylphenyl]methyl]piperazin-1-yl]-N-(3,3,3-trifluoropropylsulfonyl)benzamide |
| B2N049-01 | 4-[4-[[4-(3-Ethoxyphenyl)-2-ethylphenyl]methyl]piperazin-1-yl]-N-(3,3,3-trifluoropropylsulfonyl)benzamide |
| B2N050-01 | 4-[4-[[4-(5-Ethoxypyridin-3-yl)-2-ethylphenyl]methyl]piperazin-1-yl]-N-(3,3,3-trifluoropropylsulfonyl)benzamide |
| B2N051-01 | 4-[4-[[4-(3-Ethoxyphenyl)-3-fluorophenyl]methyl]piperazin-1-yl]-N-(3,3,3-trifluoropropylsulfonyl)benzamide |
| B2N052-01 | 4-[4-[[4-(5-Ethoxypyridin-3-yl)-3-fluorophenyl]methyl]piperazin-1-yl]-N-(3,3,3-trifluoropropylsulfonyl)benzamide |
| B2N053-01 | 4-[4-[[4-(3-Ethoxyphenyl)-2-methoxyphenyl]methyl]piperazin-1-yl]-N-(3,3,3-trifluoropropylsulfonyl)benzamide |
| B2N054-01 | 4-[4-[[4-(5-Ethoxypyridin-3-yl)-2-methoxyphenyl]methyl]piperazin-1-yl]-N-(3,3,3-trifluoropropylsulfonyl)benzamide |
| B2N055-01 | 4-[4-[[3-Ethoxy-5-(3-ethoxyphenyl)phenyl]methyl]piperazin-1-yl]-N-(3,3,3-trifluoropropylsulfonyl)benzamide |
| B2N056-01 | 4-[4-[[3-Ethoxy-5-(5-ethoxypyridin-3-yl)phenyl]methyl]piperazin-1-yl]-N-(3,3,3-trifluoropropylsulfonyl)benzamide |
| B2N057-01 | 4-[4-[[3-(3-Ethoxyphenyl)-5-propan-2-yloxyphenyl]methyl]piperazin-1-yl]-N-(3,3,3-trifluoropropylsulfonyl)benzamide |
| B2N058-01 | 4-[4-[[3-(5-Ethoxypyridin-3-yl)-5-propan-2-yloxyphenyl]methyl]piperazin-1-yl]-N-(3,3,3-trifluoropropylsulfonyl)benzamide |
| B2N059-01 | 4-[4-[[4-(3-Ethoxyphenyl)-2,6-dimethoxyphenyl]methyl]piperazin-1-yl]-N-(3,3,3-trifluoropropylsulfonyl)benzamide |
| B2N060-01 | 4-[4-[[4-(5-Ethoxypyridin-3-yl)-2,6-dimethoxyphenyl]methyl]piperazin-1-yl]-N-(3,3,3-trifluoropropylsulfonyl)benzamide |
| B2N061-01 | 4-[4-[[3-(3-Ethoxyphenyl)-5-(trifluoromethyl)phenyl]methyl]piperazin-1-yl]-N-(3,3,3-trifluoropropylsulfonyl)benzamide |
| B2N062-01 | 4-[4-[[3-(5-Ethoxypyridin-3-yl)-5-(trifluoromethyl)phenyl]methyl]piperazin-1-yl]-N-(3,3,3-trifluoropropylsulfonyl)benzamide |
| B2N063-01 | 4-[4-[[3-(3-Ethoxyphenyl)-5-(trifluoromethoxy)phenyl]methyl]piperazin-1-yl]-N-(3,3,3-trifluoropropylsulfonyl)benzamide |

(continued)

| Mixture No. | mixAI-B2N-01 |
|---|---|
| Compound No. that may be included | Compound name |
| B2N064-01 | 4-[4-[[3-(5-Ethoxypyridin-3-yl)-5-(trifluoromethoxy)phenyl]methyl]piperazin-1-yl]-N-(3,3,3-trifluoropropylsulfonyl)benzamide |
| B2N065-01 | 4-[4-[[4-(3-Ethoxyphenyl)naphthalen-1-yl]methyl]piperazin-1-yl]-N-(3,3,3-trifluoropropylsulfonyl)benzamide |
| B2N066-01 | 4-[4-[[4-(5-Ethoxypyridin-3-yl)naphthalen-1-yl]methyl]piperazin-1-yl]-N-(3,3,3-trifluoropropylsulfonyl)benzamide |
| B2N067-01 | 4-[4-[[4-(3-Ethoxyphenyl)thiophen-2-yl]methyl]piperazin-1-yl]-N-(3,3,3-trifluoropropylsulfonyl)benzamide |
| B2N068-01 | 4-[4-[[4-(5 -Ethoxypyridin-3 -yl)thiophen-2-yl]methyl]piperazin-1-yl] -N-(3,3, 3-trifluoropropylsulfonyl)benzamide |
| B2N069-01 | 4-[4-[[2-[2-(3-Ethoxyphenyl)ethynyl]phenyl]methyl]piperazin-1-yl]-N-(3,3,3-trifluoropropylsulfonyl)benzamide |
| B2N070-01 | 4-[4-[[2-[2-(5-Ethoxypyridin-3-yl)ethynyl]phenyl]methyl]piperazin-1-yl]-N-(3,3,3-trifluoropropylsulfonyl)benzamide |
| B2N071-01 | 6-[4-[[2-(3-Ethoxyphenyl)phenyl]methyl]piperazin-1-yl]-N-(3,3,3-trifluoropropylsulfonyl)pyridazine-3-carboxamide |
| B2N072-01 | 6-[4-[[2-(5-Ethoxypyridin-3-yl)phenyl]methyl]piperazin-1-yl]-N-(3,3,3-trifluoropropylsulfonyl)pyridazine-3-carboxamide |
| B2N073-01 | 6-[4-[[4-(3-Ethoxyphenyl)-3-methylphenyl]methyl]piperazin-1-yl]-N-(3,3,3-trifluoropropylsulfonyl)pyridazine-3-carboxamide |
| B2N074-01 | 6-[4-[[4-(5-Ethoxypyridin-3-yl)-3-methylphenyl]methyl]piperazin-1-yl]-N-(3,3,3-trifluoropropylsulfonyl)pyridazine-3-carboxamide |
| B2N075-01 | 6-[4-[[4-(3-Ethoxyphenyl)-2-ethylphenyl]methyl]piperazin-1-yl]-N-(3,3,3-trifluoropropylsulfonyl)pyridazine-3-carboxamide |
| B2N076-01 | 6-[4-[[4-(5-Ethoxypyridin-3-yl)-2-ethylphenyl]methyl]piperazin-1-yl]-N-(3,3,3-trifluoropropylsulfonyl)pyridazine-3-carboxamide |
| B2N077-01 | 6-[4-[[4-(3-Ethoxyphenyl)-3-fluorophenyl]methyl]piperazin-1-yl]-N-(3,3,3-trifluoropropylsulfonyl)pyridazine-3-carboxamide |
| B2N078-01 | 6-[4-[[4-(5-Ethoxypyridin-3-yl)-3-fluorophenyl]methyl]piperazin-1-yl]-N-(3,3,3-trifluoropropylsulfonyl)pyridazine-3-carboxamide |
| B2N079-01 | 6-[4-[[4-(3-Ethoxyphenyl)-2-methoxyphenyl]methyl]piperazin-1-yl]-N-(3,3,3-trifluoropropylsulfonyl)pyridazine-3-carboxamide |
| B2N080-01 | 6-[4-[[4-(5 -Ethoxypyridin-3 -yl)-2-methoxyphenyl]methyl]piperazin-1 -yl]-N-(3,3,3-trifluoropropylsulfonyl)pyridazine-3-carboxamide |
| B2N081-01 | 6-[4-[[3-Ethoxy-5-(3-ethoxyphenyl)phenyl]methyl]piperazin-1-yl]-N-(3,3,3-trifluoropropylsulfonyl)pyridazine-3-carboxamide |
| B2N082-01 | 6-[4-[[3-Ethoxy-5-(5-ethoxypyridin-3-yl)phenyl]methyl]piperazin-1-yl]-N-(3,3,3-trifluoropropylsulfonyl)pyridazine-3-carboxamide |
| B2N083-01 | 6-[4-[[3-(3-Ethoxyphenyl)-5-propan-2-yloxyphenyl]methyl]piperazin-1-yl]-N-(3,3,3-trifluoropropylsulfonyl)pyridazine-3-carboxamide |
| B2N084-01 | 6-[4-[[3-(5-Ethoxypyridin-3-yl)-5-propan-2-yloxyphenyl]methyl]piperazin-1-yl]-N-(3,3,3-trifluoropropylsulfonyl)pyridazine-3-carboxamide |

(continued)

| Mixture No. | mixAI-B2N-01 |
|---|---|
| Compound No. that may be included | Compound name |
| B2N085-01 | 6-[4-[[4-(3-Ethoxyphenyl)-2,6-dimethoxyphenyl]methyl]piperazin-1-yl]-N-(3,3,3-trifluoropropylsulfonyl)pyridazine-3-carboxamide |
| B2N086-01 | 6-[4-[[4-(5-Ethoxypyridin-3-yl)-2,6-dimethoxyphenyl]methyl]piperazin-1-yl]-N-(3,3,3-trifluoropropylsulfonyl)pyridazine-3-carboxamide |
| B2N087-01 | 6-[4-[[3-(3-Ethoxyphenyl)-5-(trifluoromethyl)phenyl]methyl]piperazin-1-yl]-N-(3,3,3-trifluoropropylsulfonyl)pyridazine-3-carboxamide |
| B2N088-01 | 6-[4-[[3-(5-Ethoxypyridin-3-yl)-5-(trifluoromethyl)phenyl]methyl]piperazin-1-yl]-N-(3,3,3-trifluoropropylsulfonyl)pyridazine-3-carboxamide |
| B2N089-01 | 6-[4-[[3-(3-Ethoxyphenyl)-5-(trifluoromethoxy)phenyl]methyl]piperazin-1-yl]-N-(3,3,3-trifluoropropylsulfonyl)pyridazine-3-carboxamide |
| B2N090-01 | 6-[4-[[3-(5-Ethoxypyridin-3-yl)-5-(trifluoromethoxy)phenyl]methyl]piperazin-1-yl]-N-(3,3,3-trifluoropropylsulfonyl)pyridazine-3-carboxamide |
| B2N091-01 | 6-[4-[[4-(3-Ethoxyphenyl)naphthalen-1-yl]methyl]piperazin-1-yl]-N-(3,3,3-trifluoropropylsulfonyl)pyridazine-3-carboxamide |
| B2N092-01 | 6-[4-[[4-(5-Ethoxypyridin-3-yl)naphthalen-1-yl]methyl]piperazin-1-yl]-N-(3,3,3-trifluoropropylsulfonyl)pyridazine-3-carboxamide |
| B2N093-01 | 6-[4-[[4-(3-Ethoxyphenyl)thiophen-2-yl]methyl]piperazin-1-yl]-N-(3,3,3-trifluoropropylsulfonyl)pyridazine-3-carboxamide |
| B2N094-01 | 6-[4-[[4-(5 -Ethoxypyridin-3-yl)thiophen-2-yl]methyl]piperazin-1-yl] -N-(3,3,3-trifluoropropylsulfonyl)pyridazine-3-carboxamide |
| B2N095-01 | 6-[4-[[2-[2-(3-Ethoxyphenyl)ethynyl]phenyl]methyl]piperazin-1-yl]-N-(3,3,3-trifluoropropylsulfonyl)pyridazine-3-carboxamide |
| B2N096-01 | 6-[4-[[2-[2-(5-Ethoxypyridin-3-yl)ethynyl]phenyl]methyl]piperazin-1-yl]-N-(3,3,3-trifluoropropylsulfonyl)pyridazine-3-carboxamide |
| B2N097-01 | 4-[4-[1-[4-(3-Ethoxyphenyl)phenyl]ethyl]piperazin-1-yl]-N-(3,3,3-trifluoropropylsulfonyl)benzamide |
| B2N098-01 | 4-[4-[1-[4-(5-Ethoxypyridin-3-yl)phenyl]ethyl]piperazin-1-yl]-N-(3,3,3-trifluoropropylsulfonyl)benzamide |
| B2N099-01 | 4-[4-[1-[4-(3-Ethoxyphenyl)-2-fluorophenyl]ethyl]piperazin-1-yl]-N-(3,3,3-trifluoropropylsulfonyl)benzamide |
| B2N100-01 | 4-[4-[1-[4-(5-Ethoxypyridin-3-yl)-2-fluorophenyl]ethyl]piperazin-1-yl]-N-(3,3,3-trifluoropropylsulfonyl)benzamide |

[Table 106]

[Table AI-03-B2A-01]

| Mixture No. | mixAI-B2A-01 |
|---|---|
| Compound No. that may be included | Compound name |
| B2A001-01 | N-(1-Adamantylmethylsulfonyl)-6-[4-[4-(3-ethoxyphenyl)thiophene-2-carbonyl]piperazin-1-yl]pyridazine-3-carboxamide |

(continued)

| Mixture No. | mixAI-B2A-01 |
|---|---|
| Compound No. that may be included | Compound name |
| B2A002-01 | N-(1-Adamantylmethylsulfonyl)-6-[4-[4-(5-ethoxypyridin-3-yl)thiophene-2-carbonyl]piperazin-1-yl]pyridazine-3-carboxamide |
| B2A003-01 | N-(1-Adamantylmethylsulfonyl)-6-[4-[4-(3-ethoxyphenyl)-3-methylbenzoyl]piperazin-1-yl]pyridazine-3-carboxamide |
| B2A004-01 | N-(1-Adamantylmethylsulfonyl)-6-[4-[4-(5-ethoxypyridin-3-yl)-3-methylbenzoyl]piperazin-1-yl]pyridazine-3-carboxamide |
| B2A005-01 | N-(1-Adamantylmethylsulfonyl)-6-[4-[4-(3-ethoxyphenyl)-3-fluorobenzoyl]piperazin-1-yl]pyridazine-3-carboxamide |
| B2A006-01 | N-(1-Adamantylmethylsulfonyl)-6-[4-[4-(5-ethoxypyridin-3-yl)-3-fluorobenzoyl]piperazin-1-yl]pyridazine-3-carboxamide |
| B2A007-01 | N-(1-Adamantylmethylsulfonyl)-6-[4-[2-[2-(3-ethoxyphenyl)ethynyl]benzoyl]piperazin-1-yl]pyridazine-3-carboxamide |
| B2A008-01 | N-(1-Adamantylmethylsulfonyl)-6-[4-[2-[2-(5-ethoxypyridin-3-yl)ethynyl]benzoyl]piperazin-1-yl]pyridazine-3-carboxamide |
| B2A009-01 | N-(1-Adamantylmethylsulfonyl)-6-[4-[5-[2-(3-ethoxyphenyl)ethynyl]pyridine-3-carbonyl]piperazin-1-yl]pyridazine-3-carboxamide |
| B2A010-01 | N-(1-Adamantylmethylsulfonyl)-6-[4-[5-[2-(5-ethoxypyridin-3-yl)ethynyl]pyridine-3-carbonyl]piperazin-1-yl]pyridazine-3-carboxamide |
| B2A011-01 | N-(1-Adamantylmethylsulfonyl)-6-[4-[3-[2-(3-ethoxyphenyl)phenyl]propanoyl]piperazin-1-yl]pyridazine-3-carboxamide |
| B2A012-01 | N-(1-Adamantylmethylsulfonyl)-6-[4-[3-[2-(5-ethoxypyridin-3-yl)phenyl]propanoyl]piperazin-1-yl]pyridazine-3-carboxamide |
| B2A013-01 | N-(1-Adamantylmethylsulfonyl)-6-[4-[4-(3-ethoxyphenyl)naphthalene-1-carbonyl]piperazin-1-yl]pyridazine-3-carboxamide |
| B2A014-01 | N-(1-Adamantylmethylsulfonyl)-6-[4-[4-(5-ethoxypyridin-3-yl)naphthalene-1-carbonyl]piperazin-1-yl]pyridazine-3-carboxamide |
| B2A015-01 | N-(1-Adamantylmethylsulfonyl)-6-[4-[3-tert-butyl-5-(3-ethoxyphenyl)benzoyl]piperazin-1-yl]pyridazine-3-carboxamide |
| B2A016-01 | N-(1-Adamantylmethylsulfonyl)-6-[4-[3-tert-butyl-5-(5-ethoxypyridin-3-yl)benzoyl]piperazin-1-yl]pyridazine-3-carboxamide |
| B2A017-01 | N-(1-Adamantylmethylsulfonyl)-6-[4-[3-(3-ethoxyphenyl)-5-(trifluoromethyl)benzoyl]piperazin-1-yl]pyridazine-3-carboxamide |
| B2A018-01 | N-(1-Adamantylmethylsulfonyl)-6-[4-[3-(5-ethoxypyridin-3-yl)-5-(trifluoromethyl)benzoyl]piperazin-1-yl]pyridazine-3-carboxamide |
| B2A019-01 | N-(1-Adamantylmethylsulfonyl)-6-[4-[3-[2-(3-ethoxyphenyl)ethynyl]-5-(trifluoromethyl)benzoyl]piperazin-1-yl]pyridazine-3-carboxamide |
| B2A020-01 | N-(1-Adamantylmethylsulfonyl)-6-[4-[3-[2-(5-ethoxypyridin-3-yl)ethynyl]-5-(trifluoromethyl)benzoyl]piperazin-1-yl]pyridazine-3-carboxamide |
| B2A021-01 | N-(1-Adamantylmethylsulfonyl)-6-[4-[3-[4-(3-ethoxyphenyl)pyrazol-1-yl]-5-(trifluoromethyl)benzoyl]piperazin-1-yl]pyridazine-3-carboxamide |
| B2A022-01 | N-(1-Adamantylmethylsulfonyl)-6-[4-[3-[4-(5-ethoxypyridin-3-yl)pyrazol-1-yl]-5-(trifluoromethyl)benzoyl]piperazin-1-yl]pyridazine-3-carboxamide |

(continued)

| Mixture No. | mixAI-B2A-01 |
|---|---|
| Compound No. that may be included | Compound name |
| B2A023-01 | N-(1-Adamantylmethylsulfonyl)-4-[4-[4-(3-ethoxyphenyl)thiophene-2-carbonyl]piperazin-1-yl]benzamide |
| B2A024-01 | N-(1-Adamantylmethylsulfonyl)-4-[4-[4-(5-ethoxypyridin-3-yl)thiophene-2-carbonyl]piperazin-1-yl]benzamide |
| B2A025-01 | N-(1-Adamantylmethylsulfonyl)-4-[4-[4-(3-ethoxyphenyl)-3-methylbenzoyl]piperazin-1-yl]benzamide |
| B2A026-01 | N-(1-Adamantylmethylsulfonyl)-4-[4-[4-(5-ethoxypyridin-3-yl)-3-methylbenzoyl]piperazin-1-yl]benzamide |
| B2A027-01 | N-(1-Adamantylmethylsulfonyl)-4-[4-[4-(3-ethoxyphenyl)-3-fluorobenzoyl]piperazin-1-yl]benzamide |
| B2A028-01 | N-(1-Adamantylmethylsulfonyl)-4-[4-[4-(5-ethoxypyridin-3-yl)-3-fluorobenzoyl]piperazin-1-yl]benzamide |
| B2A029-01 | N-(1-Adamantylmethylsulfonyl)-4-[4-[2-[2-(3-ethoxyphenyl)ethynyl]benzoyl]piperazin-1-yl]benzamide |
| B2A030-01 | N-(1-Adamantylmethylsulfonyl)-4-[4-[2-[2-(5-ethoxypyridin-3-yl)ethynyl]benzoyl]piperazin-1-yl]benzamide |
| B2A031-01 | N-(1-Adamantylmethylsulfonyl)-4-[4-[5-[2-(3-ethoxyphenyl)ethynyl]pyridine-3-carbonyl]piperazin-1-yl]benzamide |
| B2A032-01 | N-(1-Adamantylmethylsulfonyl)-4-[4-[5-[2-(5-ethoxypyridin-3-yl)ethynyl]pyridine-3-carbonyl]piperazin-1-yl]benzamide |
| B2A033-01 | N-(1-Adamantylmethylsulfonyl)-4-[4-[3-[2-(3-ethoxyphenyl)phenyl]propanoyl]piperazin-1-yl]benzamide |
| B2A034-01 | N-(1-Adamantylmethylsulfonyl)-4-[4-[3-[2-(5-ethoxypyridin-3-yl)phenyl]propanoyl]piperazin-1-yl]benzamide |
| B2A035-01 | N-(1-Adamantylmethylsulfonyl)-4-[4-[4-(3-ethoxyphenyl)naphthalene-1-carbonyl]piperazin-1-yl]benzamide |
| B2A036-01 | N-(1-Adamantylmethylsulfonyl)-4-[4-[4-(5-ethoxypyridin-3-yl)naphthalene-1-carbonyl]piperazin-1-yl]benzamide |
| B2A037-01 | N-(1-Adamantylmethylsulfonyl)-4-[4-[3-tert-butyl-5-(3-ethoxyphenyl)benzoyl]piperazin-1-yl]benzamide |
| B2A038-01 | N-(1-Adamantylmethylsulfonyl)-4-[4-[3-tert-butyl-5-(5-ethoxypyridin-3-yl)benzoyl]piperazin-1-yl]benzamide |
| B2A039-01 | N-(1-Adamantylmethylsulfonyl)-4-[4-[3-(3-ethoxyphenyl)-5-(trifluoromethyl)benzoyl]piperazin-1-yl]benzamide |
| B2A040-01 | N-(1-Adamantylmethylsulfonyl)-4-[4-[3-(5-ethoxypyridin-3-yl)-5-(trifluoromethyl)benzoyl]piperazin-1-yl]benzamide |
| B2A041-01 | N-(1-Adamantylmethylsulfonyl)-4-[4-[3-[2-(3-ethoxyphenyl)ethynyl]-5-(trifluoromethyl)benzoyl]piperazin-1-yl]benzamide |
| B2A042-01 | N-(1-Adamantylmethylsulfonyl)-4-[4-[3-[2-(5-ethoxypyridin-3-yl)ethynyl]-5-(trifluoromethyl)benzoyl]piperazin-1-yl]benzamide |
| B2A043-01 | N-(1-Adamantylmethylsulfonyl)-4-[4-[3-[4-(3-ethoxyphenyl)pyrazol-1-yl]-5-(trifluoromethyl)benzoyl]piperazin-1-yl]benzamide |

(continued)

| Mixture No. | mixAI-B2A-01 |
|---|---|
| Compound No. that may be included | Compound name |
| B2A044-01 | N-(1-Adamantylmethylsulfonyl)-4-[4-[3-[4-(5-ethoxypyridin-3-yl)pyrazol-1-yl]-5-(trifluoromethyl)benzoyl]piperazin-1-yl]benzamide |
| B2A045-01 | N-(1-Adamantylmethylsulfonyl)-4-[4-[[2-(3-ethoxyphenyl)phenyl]methyl]piperazin-1-yl]benzamide |
| B2A046-01 | N-(1-Adamantylmethylsulfonyl)-4-[4-[[2-(5-ethoxypyridin-3-yl)phenyl]methyl]piperazin-1-yl]benzamide |
| B2A047-01 | N-(1-Adamantylmethylsulfonyl)-4-[4-[[4-(3-ethoxyphenyl)-3-methylphenyl]methyl]piperazin-1-yl]benzamide |
| B2A048-01 | N-(1-Adamantylmethylsulfonyl)-4-[4-[[4-(5-ethoxypyridin-3-yl)-3-methylphenyl]methyl]piperazin-1-yl]benzamide |
| B2A049-01 | N-(1-Adamantylmethylsulfonyl)-4-[4-[[4-(3-ethoxyphenyl)-2-ethylphenyl]methyl]piperazin-1-yl]benzamide |
| B2A050-01 | N-(1-Adamantylmethylsulfonyl)-4-[4-[[4-(5-ethoxypyridin-3-yl)-2-ethylphenyl]methyl]piperazin-1-yl]benzamide |
| B2A051-01 | N-(1-Adamantylmethylsulfonyl)-4-[4-[[4-(3-ethoxyphenyl)-3-fluorophenyl]methyl]piperazin-1-yl]benzamide |
| B2A052-01 | N-(1-Adamantylmethylsulfonyl)-4-[4-[[4-(5-ethoxypyridin-3-yl)-3-fluorophenyl]methyl]piperazin-1-yl]benzamide |
| B2A053-01 | N-(1-Adamantylmethylsulfonyl)-4-[4-[[4-(3-ethoxyphenyl)-2-methoxyphenyl]methyl]piperazin-1-yl]benzamide |
| B2A054-01 | N-(1-Adamantylmethylsulfonyl)-4-[4-[[4-(5-ethoxypyridin-3-yl)-2-methoxyphenyl]methyl]piperazin-1-yl]benzamide |
| B2A055-01 | N-(1-Adamantylmethylsulfonyl)-4-[4-[[3-ethoxy-5-(3-ethoxyphenyl)phenyl]methyl]piperazin-1-yl]benzamide |
| B2A056-01 | N-(1-Adamantylmethylsulfonyl)-4-[4-[[3-ethoxy-5-(5-ethoxypyridin-3-yl)phenyl]methyl]piperazin-1-yl]benzamide |
| B2A057-01 | N-(1-Adamantylmethylsulfonyl)-4-[4-[[3-(3-ethoxyphenyl)-5-propan-2-yloxyphenyl]methyl]piperazin-1-yl]benzamide |
| B2A058-01 | N-(1-Adamantylmethylsulfonyl)-4-[4-[[3-(5-ethoxypyridin-3-yl)-5-propan-2-yloxyphenyl]methyl]piperazin-1-yl]benzamide |
| B2A059-01 | N-(1-Adamantylmethylsulfonyl)-4-[4-[[4-(3-ethoxyphenyl)-2,6-dimethoxyphenyl]methyl]piperazin-1-yl]benzamide |
| B2A060-01 | N-(1-Adamantylmethylsulfonyl)-4-[4-[[4-(5-ethoxypyridin-3-yl)-2,6-dimethoxyphenyl]methyl]piperazin-1-yl]benzamide |
| B2A061-01 | N-(1-Adamantylmethylsulfonyl)-4-[4-[[3-(3-ethoxyphenyl)-5-(trifluoromethyl)phenyl]methyl]piperazin-1-yl]benzamide |
| B2A062-01 | N-(1-Adamantylmethylsulfonyl)-4-[4-[[3-(5-ethoxypyridin-3-yl)-5-(trifluoromethyl)phenyl]methyl]piperazin-1-yl]benzamide |
| B2A063-01 | N-(1-Adamantylmethylsulfonyl)-4-[4-[[3-(3-ethoxyphenyl)-5-(trifluoromethoxy)phenyl]methyl]piperazin-1-yl]benzamide |
| B2A064-01 | N-(1-Adamantylmethylsulfonyl)-4-[4-[[3-(5-ethoxypyridin-3-yl)-5-(trifluoromethoxy)phenyl]methyl]piperazin-1-yl]benzamide |

(continued)

| Mixture No. | mixAI-B2A-01 |
|---|---|
| Compound No. that may be included | Compound name |
| B2A065-01 | N-(1-Adamantylmethylsulfonyl)-4-[4-[[4-(3-ethoxyphenyl)naphthalen-1-yl]methyl]piperazin-1-yl]benzamide |
| B2A066-01 | N-(1-Adamantylmethylsulfonyl)-4-[4-[[4-(5-ethoxypyridin-3-yl)naphthalen-1-yl]methyl]piperazin-1-yl]benzamide |
| B2A067-01 | N-(1-Adamantylmethylsulfonyl)-4-[4-[[4-(3-ethoxyphenyl)thiophen-2-yl]methyl]piperazin-1-yl]benzamide |
| B2A068-01 | N-(1-Adamantylmethylsulfonyl)-4-[4-[[4-(5-ethoxypyridin-3-yl)thiophen-2-yl]methyl]piperazin-1-yl]benzamide |
| B2A069-01 | N-(1-Adamantylmethylsulfonyl)-4-[4-[[2-[2-(3-ethoxyphenyl)ethynyl]phenyl]methyl]piperazin-1-yl]benzamide |
| B2A070-01 | N-(1-Adamantylmethylsulfonyl)-4-[4-[[2-[2-(5-ethoxypyridin-3-yl)ethynyl]phenyl]methyl]piperazin-1-yl]benzamide |
| B2A071-01 | N-(1-Adamantylmethylsulfonyl)-6-[4-[[2-(3-ethoxyphenyl)phenyl]methyl]piperazin-1-yl]pyridazine-3-carboxamide |
| B2A072-01 | N-(1-Adamantylmethylsulfonyl)-6-[4-[[2-(5-ethoxypyridin-3-yl)phenyl]methyl]piperazin-1-yl]pyridazine-3-carboxamide |
| B2A073-01 | N-(1-Adamantylmethylsulfonyl)-6-[4-[[4-(3-ethoxyphenyl)-3-methylphenyl]methyl]piperazin-1-yl]pyridazine-3-carboxamide |
| B2A074-01 | N-(1-Adamantylmethylsulfonyl)-6-[4-[[4-(5-ethoxypyridin-3-yl)-3-methylphenyl]methyl]piperazin-1-yl]pyridazine-3-carboxamide |
| B2A075-01 | N-(1-Adamantylmethylsulfonyl)-6-[4-[[4-(3-ethoxyphenyl)-2-ethylphenyl]methyl]piperazin-1-yl]pyridazine-3-carboxamide |
| B2A076-01 | N-(1-Adamantylmethylsulfonyl)-6-[4-[[4-(5-ethoxypyridin-3-yl)-2-ethylphenyl]methyl]piperazin-1-yl]pyridazine-3 -carboxamide |
| B2A077-01 | N-(1-Adamantylmethylsulfonyl)-6-[4-[[4-(3-ethoxyphenyl)-3-fluorophenyl]methyl]piperazin-1-yl]pyridazine-3-carboxamide |
| B2A078-01 | N-(1-Adamantylmethylsulfonyl)-6-[4-[[4-(5-ethoxypyridin-3-yl)-3-fluorophenyl]methyl]piperazin-1-yl]pyridazine-3-carboxamide |
| B2A079-01 | N-(1-Adamantylmethylsulfonyl)-6-[4-[[4-(3-ethoxyphenyl)-2-methoxyphenyl]methyl]piperazin-1-yl]pyridazine-3-carboxamide |
| B2A080-01 | N-(1-Adamantylmethylsulfonyl)-6-[4-[[4-(5-ethoxypyridin-3-yl)-2-methoxyphenyl]methyl]piperazin-1-yl]pyridazine-3-carboxamide |
| B2A081-01 | N-(1-Adamantylmethylsulfonyl)-6-[4-[[3-ethoxy-5-(3-ethoxyphenyl)phenyl]methyl]piperazin-1-yl]pyridazine-3-carboxamide |
| B2A082-01 | N-(1-Adamantylmethylsulfonyl)-6-[4-[[3-ethoxy-5-(5-ethoxypyridin-3-yl)phenyl]methyl]piperazin-1-yl]pyridazine-3-carboxamide |
| B2A083-01 | N-(1-Adamantylmethylsulfonyl)-6-[4-[[3-(3-ethoxyphenyl)-5-propan-2-yloxyphenyl]methyl]piperazin-1-yl]pyridazine-3-carboxamide |
| B2A084-01 | N-(1-Adamantylmethylsulfonyl)-6-[4-[[3-(5-ethoxypyridin-3-yl)-5-propan-2-yloxyphenyl]methyl]piperazin-1-yl]pyridazine-3-carboxamide |
| B2A085-01 | N-(1-Adamantylmethylsulfonyl)-6-[4-[[4-(3-ethoxyphenyl)-2,6-dimethoxyphenyl]methyl]piperazin-1-yl]pyridazine-3-carboxamide |

(continued)

| Mixture No. | mixAI-B2A-01 |
|---|---|
| Compound No. that may be included | Compound name |
| B2A086-01 | N-(1-Adamantylmethylsulfonyl)-6-[4-[[4-(5-ethoxypyridin-3-yl)-2,6-dimethoxyphenyl]methyl]piperazin-1-yl]pyridazine-3-carboxamide |
| B2A087-01 | N-(1-Adamantylmethylsulfonyl)-6-[4-[[3-(3-ethoxyphenyl)-5-(trifluoromethyl)phenyl]methyl]piperazin-1-yl]pyridazine-3-carboxamide |
| B2A088-01 | N-(1-Adamantylmethylsulfonyl)-6-[4-[[3-(5-ethoxypyridin-3-yl)-5-(trifluoromethyl)phenyl]methyl]piperazin-1-yl]pyridazine-3-carboxamide |
| B2A089-01 | N-(1-Adamantylmethylsulfonyl)-6-[4-[[3-(3-ethoxyphenyl)-5-(trifluoromethoxy)phenyl]methyl]piperazin-1-yl]pyridazine-3-carboxamide |
| B2A090-01 | N-(1-Adamantylmethylsulfonyl)-6-[4-[[3-(5-ethoxypyridin-3-yl)-5-(trifluoromethoxy)phenyl]methyl]piperazin-1-yl]pyridazine-3 -carboxamide |
| B2A091-01 | N-(1-Adamantylmethylsulfonyl)-6-[4-[[4-(3-ethoxyphenyl)naphthalen-1-yl]methyl]piperazin-1-yl]pyridazine-3-carboxamide |
| B2A092-01 | N-(1-Adamantylmethylsulfonyl)-6-[4-[[4-(5-ethoxypyridin-3-yl)naphthalen-1-yl]methyl]piperazin-1-yl]pyridazine-3-carboxamide |
| B2A093-01 | N-(1-Adamantylmethylsulfonyl)-6-[4-[[4-(3-ethoxyphenyl)thiophen-2-yl]methyl]piperazin-1-yl]pyridazine-3-carboxamide |
| B2A094-01 | N-(1-Adamantylmethylsulfonyl)-6-[4-[[4-(5-ethoxypyridin-3-yl)thiophen-2-yl]methyl]piperazin-1-yl]pyridazine-3-carboxamide |
| B2A095-01 | N-(1-Adamantylmethylsulfonyl)-6-[4-[[2-[2-(3-ethoxyphenyl)ethynyl]phenyl]methyl]piperazin-1-yl]pyridazine-3-carboxamide |
| B2A096-01 | N-(1-Adamantylmethylsulfonyl)-6-[4-[[2-[2-(5-ethoxypyridin-3-yl)ethynyl]phenyl]methyl]piperazin-1-yl]pyridazine-3 -carboxamide |
| B2A097-01 | N-(1-Adamantylmethylsulfonyl)-4-[4-[1-[4-(3-ethoxyphenyl)phenyl]ethyl]piperazin-1-yl]benzamide |
| B2A098-01 | N-(1-Adamantylmethylsulfonyl)-4-[4-[1-[4-(5-ethoxypyridin-3-yl)phenyl] ethyl]piperazin-1-yl]benzamide |
| B2A099-01 | N-(1-Adamantylmethylsulfonyl)-4-[4-[1-[4-(3-ethoxyphenyl)-2-fluorophenyl]ethyl]piperazin-1-yl]benzamide |
| B2A100-01 | N-(1-Adamantylmethylsulfonyl)-4-[4-[1-[4-(5-ethoxypyridin-3-yl)-2-fluorophenyl]ethyl]piperazin-1-yl]benzamide |

[Table 107]

[Table AI-03-B2R-01]

| Mixture No. | mixAI-B2R-01 |
|---|---|
| Compound No. that may be included | Compound name |
| B2R001-01 | 6-[4-[4-(3-Ethoxyphenyl)thiophene-2-carbonyl]piperazin-1-yl]-N-[4-(2-phenylsulfanylethylamino)-3 -(trifluoromethyl)phenyl] sulfonylpyridazine-3 - carboxamide |
| B2R002-01 | 6-[4-[4-(5-Ethoxypyridin-3-yl)thiophene-2-carbonyl]piperazin-1-yl]-N-[4-(2-phenylsulfanylethylamino)-3-(trifluoromethyl)phenyl] sulfonylpyridazine-3- carboxamide |

(continued)

| Mixture No. | mixAI-B2R-01 |
|---|---|
| Compound No. that may be included | Compound name |
| B2R003-01 | 6-[4-[4-(3-Ethoxyphenyl)-3-methylbenzoyl]piperazin-1-yl]-N-[4-(2-phenylsulfanylethylamino)-3-(trifluoromethyl)phenyl]sulfonylpyridazine-3- carboxamide |
| B2R004-01 | 6-[4-[4-(5-Ethoxypyridin-3-yl)-3-methylbenzoyl]piperazin-1-yl]-N-[4-(2-phenylsulfanylethylamino)-3-(trifluoromethyl)phenyl]sulfonylpyridazine-3- carboxamide |
| B2R005-01 | 6-[4-[4-(3-Ethoxyphenyl)-3-fluorobenzoyl]piperazin-1-yl]-N-[4-(2-phenylsulfanylethylamino)-3-(trifluoromethyl)phenyl]sulfonylpyridazine-3- carboxamide |
| B2R006-01 | 6-[4-[4-(5-Ethoxypyridin-3-yl)-3-fluorobenzoyl]piperazin-1-yl]-N-[4-(2-phenylsulfanylethylamino)-3-(trifluoromethyl)phenyl]sulfonylpyridazine-3- carboxamide |
| B2R007-01 | 6-[4-[2-[2-(3-Ethoxyphenyl)ethynyl]benzoyl]piperazin-1-yl]-N-[4-(2-phenylsulfanylethylamino)-3-(trifluoromethyl)phenyl]sulfonylpyridazine-3- carboxamide |
| B2R008-01 | 6-[4-[2-[2-(5-Ethoxypyridin-3-yl)ethynyl]benzoyl]piperazin-1-yl]-N-[4-(2-phenylsulfanylethylamino)-3-(trifluoromethyl)phenyl]sulfonylpyridazine-3- carboxamide |
| B2R009-01 | 6-[4-[5-[2-(3-Ethoxyphenyl)ethynyl]pyridine-3-carbonyl]piperazin-1-yl]-N-[4-(2-phenylsulfanylethylamino)-3-(trifluoromethyl)phenyl]sulfonylpyridazine-3- carboxamide |
| B2R010-01 | 6-[4-[5-[2-(5-Ethoxypyridin-3-yl)ethynyl]pyridine-3-carbonyl]piperazin-1-yl]-N-[4-(2-phenylsulfanylethylamino)-3-(trifluoromethyl)phenyl]sulfonylpyridazine-3-carboxamide |
| B2R011-01 | 6-[4-[3-[2-(3-Ethoxyphenyl)phenyl]propanoyl]piperazin-1-yl]-N-[4-(2-phenylsulfanylethylamino)-3-(trifluoromethyl)phenyl]sulfonylpyridazine-3- carboxamide |
| B2R012-01 | 6-[4-[3-[2-(5-Ethoxypyridin-3-yl)phenyl]propanoyl]piperazin-1-yl]-N-[4-(2-phenylsulfanylethylamino)-3-(trifluoromethyl)phenyl]sulfonylpyridazine-3- carboxamide |
| B2R013-01 | 6-[4-[4-(3-Ethoxyphenyl)naphthalene-1-carbonyl]piperazin-1-yl]-N-[4-(2-phenylsulfanylethylamino)-3-(trifluoromethyl)phenyl]sulfonylpyridazine-3- carboxamide |
| B2R014-01 | 6-[4-[4-(5-Ethoxypyridin-3-yl)naphthalene-1-carbonyl]piperazin-1-yl]-N-[4-(2-phenylsulfanylethylamino)-3-(trifluoromethyl)phenyl]sulfonylpyridazine-3- carboxamide |
| B2R015-01 | 6-[4-[3-tert-Butyl-5-(3-ethoxyphenyl)benzoyl]piperazin-1-yl]-N-[4-(2-phenylsulfanylethylamino)-3-(trifluoromethyl)phenyl]sulfonylpyridazine-3- carboxamide |
| B2R016-01 | 6-[4-[3-tert-Butyl-5-(5-ethoxypyridin-3-yl)benzoyl]piperazin-1-yl]-N-[4-(2-phenylsulfanylethylamino)-3-(trifluoromethyl)phenyl]sulfonylpyridazine-3- carboxamide |
| B2R017-01 | 6-[4-[3-(3-Ethoxyphenyl)-5-(trifluoromethyl)benzoyl]piperazin-1-yl]-N-[4-(2-phenylsulfanylethylamino)-3-(trifluoromethyl)phenyl]sulfonylpyridazine-3- carboxamide |
| B2R018-01 | 6-[4-[3-(5-Ethoxypyridin-3-yl)-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| B2R019-01 | 6-[4-[3-[2-(3-Ethoxyphenyl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| B2R020-01 | 6-[4-[3-[2-(5-Ethoxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| B2R021-01 | 6-[4-[3-[4-(3-Ethoxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylpyridazine-3-carboxamide |
| B2R022-01 | 6-[4-[3-[4-(5-Ethoxypyridin-3-yl)pyrazol-1-yl]-5-(trifluoromethyl)benzoyl]piperazin-1-yl]-N-[4-(2-phenylsulfanylethylamino)-3- (trifluoromethyl)phenyl]sulfonylpyridazine-3-carboxamide |
| B2R023-01 | 4-[4-[4-(3-Ethoxyphenyl)thiophene-2-carbonyl]piperazin-1-yl]-N-[4-(2-phenylsulfanylethylamino)-3-(trifluoromethyl)phenyl]sulfonylbenzamide |

(continued)

| Mixture No. | mixAI-B2R-01 |
|---|---|
| Compound No. that may be included | Compound name |
| B2R024-01 | 4-[4-[4-(5-Ethoxypyridin-3-yl)thiophene-2-carbonyl]piperazin-1-yl]-N-[4-(2-phenylsulfanylethylamino)-3-(trifluoromethyl)phenyl]sulfonylbenzamide |
| B2R025-01 | 4-[4-[4-(3-Ethoxyphenyl)-3-methylbenzoyl]piperazin-1-yl]-N-[4-(2-phenylsulfanylethylamino)-3-(trifluoromethyl)phenyl]sulfonylbenzamide |
| B2R026-01 | 4-[4-[4-(5-Ethoxypyridin-3-yl)-3-methylbenzoyl]piperazin-1-yl]-N-[4-(2-phenylsulfanylethylamino)-3-(trifluoromethyl)phenyl]sulfonylbenzamide |
| B2R027-01 | 4-[4-[4-(3-Ethoxyphenyl)-3-fluorobenzoyl]piperazin-1-yl]-N-[4-(2-phenylsulfanylethylamino)-3-(trifluoromethyl)phenyl]sulfonylbenzamide |
| B2R028-01 | 4-[4-[4-(5-Ethoxypyridin-3-yl)-3-fluorobenzoyl]piperazin-1-yl]-N-[4-(2-phenylsulfanylethylamino)-3-(trifluoromethyl)phenyl]sulfonylbenzamide |
| B2R029-01 | 4-[4-[2-[2-(3-Ethoxyphenyl)ethynyl]benzoyl]piperazin-1-yl]-N-[4-(2-phenylsulfanylethylamino)-3-(trifluoromethyl)phenyl]sulfonylbenzamide |
| B2R030-01 | 4-[4-[2-[2-(5-Ethoxypyridin-3-yl)ethynyl]benzoyl]piperazin-1-yl]-N-[4-(2-phenylsulfanylethylamino)-3-(trifluoromethyl)phenyl]sulfonylbenzamide |
| B2R031-01 | 4-[4-[5-[2-(3-Ethoxyphenyl)ethynyl]pyridine-3-carbonyl]piperazin-1-yl]-N-[4-(2-phenylsulfanylethylamino)-3-(trifluoromethyl)phenyl]sulfonylbenzamide |
| B2R032-01 | 4-[4-[5-[2-(5-Ethoxypyridin-3-yl)ethynyl]pyridine-3-carbonyl]piperazin-1-yl]-N-[4-(2-phenylsulfanylethylamino)-3-(trifluoromethyl)phenyl]sulfonylbenzamide |
| B2R033-01 | 4-[4-[3-[2-(3-Ethoxyphenyl)phenyl]propanoyl]piperazin-1-yl]-N-[4-(2-phenylsulfanylethylamino)-3-(trifluoromethyl)phenyl]sulfonylbenzamide |
| B2R034-01 | 4-[4-[3-[2-(5-Ethoxypyridin-3-yl)phenyl]propanoyl]piperazin-1-yl]-N-[4-(2-phenylsulfanylethylamino)-3-(trifluoromethyl)phenyl]sulfonylbenzamide |
| B2R035-01 | 4-[4-[4-(3-Ethoxyphenyl)naphthalene-1-carbonyl]piperazin-1-yl]-N-[4-(2-phenylsulfanylethylamino)-3-(trifluoromethyl)phenyl]sulfonylbenzamide |
| B2R036-01 | 4-[4-[4-(5-Ethoxypyridin-3-yl)naphthalene-1-carbonyl]piperazin-1-yl]-N-[4-(2-phenylsulfanylethylamino)-3-(trifluoromethyl)phenyl]sulfonylbenzamide |
| B2R037-01 | 4-[4-[3-tert-Butyl-5-(3-ethoxyphenyl)benzoyl]piperazin-1-yl]-N-[4-(2-phenylsulfanylethylamino)-3-(trifluoromethyl)phenyl]sulfonylbenzamide |
| B2R038-01 | 4-[4-[3-tert-Butyl-5-(5-ethoxypyridin-3-yl)benzoyl]piperazin-1-yl]-N-[4-(2-phenylsulfanylethylamino)-3-(trifluoromethyl)phenyl]sulfonylbenzamide |
| B2R039-01 | 4-[4-[3-(3-Ethoxyphenyl)-5-(trifluoromethyl)benzoyl]piperazin-1-yl]-N-[4-(2-phenylsulfanylethylamino)-3-(trifluoromethyl)phenyl]sulfonylbenzamide |
| B2R040-01 | 4-[4-[3-(5-Ethoxypyridin-3-yl)-5-(trifluoromethyl)benzoyl]piperazin-1-yl]-N-[4-(2-phenylsulfanylethylamino)-3-(trifluoromethyl)phenyl]sulfonylbenzamide |
| B2R041-01 | 4-[4-[3-[2-(3-Ethoxyphenyl)ethynyl]-5-(trifluoromethyl)benzoyl]piperazin-1-yl]-N-[4-(2-phenylsulfanylethylamino)-3-(trifluoromethyl)phenyl]sulfonylbenzamide |
| B2R042-01 | 4-[4-[3-[2-(5-Ethoxypyridin-3-yl)ethynyl]-5-(trifluoromethyl)benzoyl]piperazin-1-yl]-N-[4-(2-phenylsulfanylethylamino)-3-(trifluoromethyl)phenyl]sulfonylbenzamide |
| B2R043-01 | 4-[4-[3-[4-(3-Ethoxyphenyl)pyrazol-1-yl]-5-(trifluoromethyl)benzoyl]piperazin-1-yl]-N-[4-(2-phenylsulfanylethylamino)-3-(trifluoromethyl)phenyl]sulfonylbenzamide |
| B2R044-01 | 4-[4-[3-[4-(5-Ethoxypyridin-3-yl)pyrazol-1-yl]-5-(trifluoromethyl)benzoyl]piperazin-1-yl]-N-[4-(2-phenylsulfanylethylamino)-3- (trifluoromethyl)phenyl]sulfonylbenzamide |

(continued)

| Mixture No. | mixAI-B2R-01 |
|---|---|
| Compound No. that may be included | Compound name |
| B2R045-01 | 4-[4-[[2-(3-Ethoxyphenyl)phenyl]methyl]piperazin-1-yl]-N-[4-(2-phenylsulfanylethylamino)-3-(trifluoromethyl)phenyl]sulfonylbenzamide |
| B2R046-01 | 4-[4-[[2-(5-Ethoxypyridin-3-yl)phenyl]methyl]piperazin-1-yl]-N-[4-(2-phenylsulfanylethylamino)-3-(trifluoromethyl)phenyl]sulfonylbenzamide |
| B2R047-01 | 4-[4-[[4-(3-Ethoxyphenyl)-3-methylphenyl]methyl]piperazin-1-yl]-N-[4-(2-phenylsulfanylethylamino)-3-(trifluoromethyl)phenyl]sulfonylbenzamide |
| B2R048-01 | 4-[4-[[4-(5-Ethoxypyridin-3-yl)-3-methylphenyl]methyl]piperazin-1-yl]-N-[4-(2-phenylsulfanylethylamino)-3-(trifluoromethyl)phenyl]sulfonylbenzamide |
| B2R049-01 | 4-[4-[[4-(3-Ethoxyphenyl)-2-ethylphenyl]methyl]piperazin-1-yl]-N-[4-(2-phenylsulfanylethylamino)-3-(trifluoromethyl)phenyl]sulfonylbenzamide |
| B2R050-01 | 4-[4-[[4-(5-Ethoxypyridin-3-yl)-2-ethylphenyl]methyl]piperazin-1-yl]-N-[4-(2-phenylsulfanylethylamino)-3-(trifluoromethyl)phenyl]sulfonylbenzamide |
| B2R051-01 | 4-[4-[[4-(3-Ethoxyphenyl)-3-fluorophenyl]methyl]piperazin-1-yl]-N-[4-(2-phenylsulfanylethylamino)-3-(trifluoromethyl)phenyl]sulfonylbenzamide |
| B2R052-01 | 4-[4-[[4-(5-Ethoxypyridin-3-yl)-3-fluorophenyl]methyl]piperazin-1-yl]-N-[4-(2-phenylsulfanylethylamino)-3-(trifluoromethyl)phenyl]sulfonylbenzamide |
| B2R053-01 | 4-[4-[[4-(3-Ethoxyphenyl)-2-methoxyphenyl]methyl]piperazin-1-yl]-N-[4-(2-phenylsulfanylethylamino)-3-(trifluoromethyl)phenyl]sulfonylbenzamide |
| B2R054-01 | 4-[4-[[4-(5-Ethoxypyridin-3-yl)-2-methoxyphenyl]methyl]piperazin-1-yl]-N-[4-(2-phenylsulfanylethylamino)-3-(trifluoromethyl)phenyl]sulfonylbenzamide |
| B2R055-01 | 4-[4-[[3-Ethoxy-5-(3-ethoxyphenyl)phenyl]methyl]piperazin-1-yl]-N-[4-(2-phenylsulfanylethylamino)-3-(trifluoromethyl)phenyl]sulfonylbenzamide |
| B2R056-01 | 4-[4-[[3-Ethoxy-5-(5-ethoxypyridin-3-yl)phenyl]methyl]piperazin-1-yl]-N-[4-(2-phenylsulfanylethylamino)-3-(trifluoromethyl)phenyl]sulfonylbenzamide |
| B2R057-01 | 4-[4-[[3-(3-Ethoxyphenyl)-5-propan-2-yloxyphenyl]methyl]piperazin-1-yl]-N-[4-(2-phenylsulfanylethylamino)-3-(trifluoromethyl)phenyl]sulfonylbenzamide |
| B2R058-01 | 4-[4-[[3-(5-Ethoxypyridin-3-yl)-5-propan-2-yloxyphenyl]methyl]piperazin-1-yl]-N-[4-(2-phenylsulfanylethylamino)-3-(trifluoromethyl)phenyl]sulfonylbenzamide |
| B2R059-01 | 4-[4-[[4-(3-Ethoxyphenyl)-2,6-dimethoxyphenyl]methyl]piperazin-1-yl]-N-[4-(2-phenylsulfanylethylamino)-3-(trifluoromethyl)phenyl]sulfonylbenzamide |
| B2R060-01 | 4-[4-[[4-(5-Ethoxypyridin-3-yl)-2,6-dimethoxyphenyl]methyl]piperazin-1-yl]-N-[4-(2-phenylsulfanylethylamino)-3-(trifluoromethyl)phenyl]sulfonylbenzamide |
| B2R061-01 | 4-[4-[[3-(3-Ethoxyphenyl)-5-(trifluoromethyl)phenyl]methyl]piperazin-1-yl]-N-[4-(2-phenylsulfanylethylamino)-3-(trifluoromethyl)phenyl]sulfonylbenzamide |
| B2R062-01 | 4-[4-[[3-(5-Ethoxypyridin-3-yl)-5-(trifluoromethyl)phenyl]methyl]piperazin-1-yl]-N-[4-(2-phenylsulfanylethylamino)-3-(trifluoromethyl)phenyl]sulfonylbenzamide |
| B2R063-01 | 4-[4-[[3-(3-Ethoxyphenyl)-5-(trifluoromethoxy)phenyl]methyl]piperazin-1-yl]-N-[4-(2-phenylsulfanylethylamino)-3-(trifluoromethyl)phenyl]sulfonylbenzamide |
| B2R064-01 | 4-[4-[[3-(5-Ethoxypyridin-3-yl)-5-(trifluoromethoxy)phenyl]methyl]piperazin-1-yl]-N-[4-(2-phenylsulfanylethylamino)-3-(trifluoromethyl)phenyl]sulfonylbenzamide |
| B2R065-01 | 4-[4-[[4-(3-Ethoxyphenyl)naphthalen-1-yl]methyl]piperazin-1-yl]-N-[4-(2-phenylsulfanylethylamino)-3-(trifluoromethyl)phenyl]sulfonylbenzamide |

(continued)

| Mixture No. | mixAI-B2R-01 |
|---|---|
| Compound No. that may be included | Compound name |
| B2R066-01 | 4-[4-[[4-(5-Ethoxypyridin-3-yl)naphthalen-1-yl]methyl]piperazin-1-yl]-N-[4-(2-phenylsulfanylethylamino)-3-(trifluoromethyl)phenyl]sulfonylbenzamide |
| B2R067-01 | 4-[4-[[4-(3-Ethoxyphenyl)thiophen-2-yl]methyl]piperazin-1-yl]-N-[4-(2-phenylsulfanylethylamino)-3-(trifluoromethyl)phenyl]sulfonylbenzamide |
| B2R068-01 | 4-[4-[[4-(5-Ethoxypyridin-3-yl)thiophen-2-yl]methyl]piperazin-1-yl]-N-[4-(2-phenylsulfanylethylamino)-3-(trifluoromethyl)phenyl]sulfonylbenzamide |
| B2R069-01 | 4-[4-[[2-[2-(3-Ethoxyphenyl)ethynyl]phenyl]methyl]piperazin-1-yl]-N-[4-(2-phenylsulfanylethylamino)-3-(trifluoromethyl)phenyl]sulfonylbenzamide |
| B2R070-01 | 4-[4-[[2-[2-(5-Ethoxypyridin-3-yl)ethynyl]phenyl]methyl]piperazin-1-yl]-N-[4-(2-phenylsulfanylethylamino)-3-(trifluoromethyl)phenyl]sulfonylbenzamide |
| B2R071-01 | 6-[4-[[2-(3-Ethoxyphenyl)phenyl]methyl]piperazin-1-yl]-N-[4-(2-phenylsulfanylethylamino)-3-(trifluoromethyl)phenyl]sulfonylpyridazine-3- carboxamide |
| B2R072-01 | 6-[4-[[2-(5-Ethoxypyridin-3-yl)phenyl]methyl]piperazin-1-yl]-N-[4-(2-phenylsulfanylethylamino)-3-(trifluoromethyl)phenyl]sulfonylpyridazine-3- carboxamide |
| B2R073-01 | 6-[4-[[4-(3-Ethoxyphenyl)-3-methylphenyl]methyl]piperazin-1-yl]-N-[4-(2-phenylsulfanylethylamino)-3-(trifluoromethyl)phenyl]sulfonylpyridazine-3- carboxamide |
| B2R074-01 | 6-[4-[[4-(5-Ethoxypyridin-3-yl)-3-methylphenyl]methyl]piperazin-1-yl]-N-[4-(2-phenylsulfanylethylamino)-3-(trifluoromethyl)phenyl]sulfonylpyridazine-3- carboxamide |
| B2R075-01 | 6-[4-[[4-(3-Ethoxyphenyl)-2-ethylphenyl]methyl]piperazin-1-yl]-N-[4-(2-phenylsulfanylethylamino)-3-(trifluoromethyl)phenyl]sulfonylpyridazine-3- carboxamide |
| B2R076-01 | 6-[4-[[4-(5-Ethoxypyridin-3-yl)-2-ethylphenyl]methyl]piperazin-1-yl]-N-[4-(2-phenylsulfanylethylamino)-3-(trifluoromethyl)phenyl]sulfonylpyridazine-3- carboxamide |
| B2R077-01 | 6-[4-[[4-(3-Ethoxyphenyl)-3-fluorophenyl]methyl]piperazin-1-yl]-N-[4-(2-phenylsulfanylethylamino)-3-(trifluoromethyl)phenyl]sulfonylpyridazine-3- carboxamide |
| B2R078-01 | 6-[4-[[4-(5-Ethoxypyridin-3-yl)-3-fluorophenyl]methyl]piperazin-1-yl]-N-[4-(2-phenylsulfanylethylamino)-3-(trifluoromethyl)phenyl]sulfonylpyridazine-3- carboxamide |
| B2R079-01 | 6-[4-[[4-(3-Ethoxyphenyl)-2-methoxyphenyl]methyl]piperazin-1-yl]-N-[4-(2-phenylsulfanylethylamino)-3-(trifluoromethyl)phenyl]sulfonylpyridazine-3- carboxamide |
| B2R080-01 | 6-[4-[[4-(5-Ethoxypyridin-3-yl)-2-methoxyphenyl]methyl]piperazin-1-yl]-N-[4-(2-phenylsulfanylethylamino)-3-(trifluoromethyl)phenyl]sulfonylpyridazine-3-carboxamide |
| B2R081-01 | 6-[4-[[3-Ethoxy-5-(3-ethoxyphenyl)phenyl]methyl]piperazin-1-yl]-N-[4-(2-phenylsulfanylethylamino)-3-(trifluoromethyl)phenyl]sulfonylpyridazine-3- carboxamide |
| B2R082-01 | 6-[4-[[3-Ethoxy-5-(5-ethoxypyridin-3-yl)phenyl]methyl]piperazin-1-yl]-N-[4-(2-phenylsulfanylethylamino)-3-(trifluoromethyl)phenyl]sulfonylpyridazine-3- carboxamide |
| B2R083-01 | 6-[4-[[3-(3-Ethoxyphenyl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| B2R084-01 | 6-[4-[[3-(5-Ethoxypyridin-3-yl)-5-propan-2-yloxyphenyl]methyl]piperazin-1-yl]-N-[4-(2-phenylsulfanylethylamino)-3-(trifluoromethyl)phenyl]sulfonylpyridazine-3-carboxamide |
| B2R085-01 | 6-[4-[[4-(3-Ethoxyphenyl)-2,6-dimethoxyphenyl]methyl]piperazin-1-yl]-N-[4-(2-phenylsulfanylethylamino)-3-(trifluoromethyl)phenyl]sulfonylpyridazine-3- carboxamide |
| B2R086-01 | 6-[4-[[4-(5-Ethoxypyridin-3-yl)-2,6-dimethoxyphenyl]methyl]piperazin-1-yl]-N-[4-(2-phenylsulfanylethylamino)-3-(trifluoromethyl)phenyl]sulfonylpyridazine-3-carboxamide |

(continued)

| Mixture No. | mixAI-B2R-01 |
|---|---|
| Compound No. that may be included | Compound name |
| B2R087-01 | 6-[4-[[3-(3-Ethoxyphenyl)-5-(trifluoromethyl)phenyl]methyl]piperazin-1-yl]-N-[4-(2-phenylsulfanylethylamino)-3-(trifluoromethyl)phenyl]sulfonylpyridazine-3-carboxamide |
| B2R088-01 | 6-[4-[[3-(5-Ethoxypyridin-3-yl)-5-(trifluoromethyl)phenyl]methyl]piperazin-1-yl]-N-[4-(2-phenylsulfanylethylamino)-3-(trifluoromethyl)phenyl]sulfonylpyridazine-3-carboxamide |
| B2R089-01 | 6-[4-[[3-(3-Ethoxyphenyl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |
| B2R090-01 | 6-[4-[[3-(5-Ethoxypyridin-3-yl)-5-(trifluoromethoxy)phenyl]methyl]piperazin-1-yl]-N-[4-(2-phenylsulfanylethylamino)-3-(trifluoromethyl)phenyl]sulfonylpyridazine-3-carboxamide |
| B2R091-01 | 6-[4-[[4-(3-Ethoxyphenyl)naphthalen-1-yl]methyl]piperazin-1-yl]-N-[4-(2-phenylsulfanylethylamino)-3-(trifluoromethyl)phenyl]sulfonylpyridazine-3- carboxamide |
| B2R092-01 | 6-[4-[[4-(5-Ethoxypyridin-3-yl)naphthalen-1-yl]methyl]piperazin-1-yl]-N-[4-(2-phenylsulfanylethylamino)-3-(trifluoromethyl)phenyl]sulfonylpyridazine-3- carboxamide |
| B2R093-01 | 6-[4-[[4-(3-Ethoxyphenyl)thiophen-2-yl]methyl]piperazin-1-yl]-N-[4-(2-phenylsulfanylethylamino)-3-(trifluoromethyl)phenyl]sulfonylpyridazine-3- carboxamide |
| B2R094-01 | 6-[4-[[4-(5-Ethoxypyridin-3-yl)thiophen-2-yl]methyl]piperazin-1-yl]-N-[4-(2-phenylsulfanylethylamino)-3-(trifluoromethyl)phenyl]sulfonylpyridazine-3- carboxamide |
| B2R095-01 | 6-[4-[[2-[2-(3-Ethoxyphenyl)ethynyl]phenyl]methyl]piperazin-1-yl]-N-[4-(2-phenylsulfanylethylamino)-3-(trifluoromethyl)phenyl]sulfonylpyridazine-3- carboxamide |
| B2R096-01 | 6-[4-[[2-[2-(5-Ethoxypyridin-3-yl)ethynyl]phenyl]methyl]piperazin-1-yl]-N-[4-(2-phenylsulfanylethylamino)-3-(trifluoromethyl)phenyl]sulfonylpyridazine-3- carboxamide |
| B2R097-01 | 4-[4-[1-[4-(3-Ethoxyphenyl)phenyl]ethyl]piperazin-1-yl]-N-[4-(2-phenylsulfanylethylamino)-3-(trifluoromethyl)phenyl]sulfonylbenzamide |
| B2R098-01 | 4-[4-[1-[4-(5-Ethoxypyridin-3-yl)phenyl]ethyl]piperazin-1-yl]-N-[4-(2-phenylsulfanylethylamino)-3-(trifluoromethyl)phenyl]sulfonylbenzamide |
| B2R099-01 | 4-[4-[1-[4-(3-Ethoxyphenyl)-2-fluorophenyl]ethyl]piperazin-1-yl]-N-[4-(2-phenylsulfanylethylamino)-3-(trifluoromethyl)phenyl]sulfonylbenzamide |
| B2R100-01 | 4-[4-[1-[4-(5-Ethoxypyridin-3-yl)-2-fluorophenyl]ethyl]piperazin-1-yl]-N-[4-(2-phenylsulfanylethylamino)-3-(trifluoromethyl)phenyl]sulfonylbenzamide |

Example 6-5-3: Preparation of mixture solution and analysis by LCMS

DMSO was added to each of the 12 mixtures obtained in Example 6-5-1 and Example 6-5-2, and 12 DMSO solutions of approximately 10 mM were prepared. 100 μL of the solution was subjected to a binding evaluation experiment to Bcl-2 by AS-MS shown in Example 7. Also, some of the 12 DMSO solutions of 10 mM were diluted with MeCN and analyzed by LCMS. The analysis results are shown in [Table AI-03-02].

[Table 108]

[Table AI-03-02]

| Mixture No. | mixAJ-B1A-01 | | |
|---|---|---|---|
| m/z | Retention time of MS peak (min) | Retention time of UV peak (min) | Attributed compound No. |
| 712.347 | 14.453 | 14.41 | B1A043-01 |
| 628.353 | 14.22 | 14.17 | B1A035-01 |
| 646.325 | 14.2 | 14.17 | B1A039-01 |
| 714.337 | 14.637 | 14.59 | B1A021-01 |
| 634.4 | 11.127 | 11.09 | B1A037-01, B1A087-01 |
| 584.294 | 9.7 | 9.65 | B1A023-01, B1A077-01 |
| 603.333 | 13.047 | 13.01 | B1A031-01 |
| 564.358 | 9.197 | 9.15 | B1A045-01 |
| 648.316 | 10.537 | 10.5 | B1A017-01, B1A063-01 |
| 632.346 | 10.39 | 10.36 | B1A061-01 |
| 605.323 | 13.127 | 13.08 | B1A009-01 |
| 637.386 | 12.733 | 12.71 | B1A016-01 |
| 606.319 | 13.793 | 13.75 | B1A100-01, B1A033-01 |
| 629.349 | 11.683 | 11.65 | B1A036-01 |
| 634.4 | 14.913 | 14.88 | B1A037-01, B1A087-01 |
| 670.325 | 14.91 | 14.88 | B1A041-01 |
| 672.316 | 15.117 | 15.08 | B1A019-01 |
| 647.32 | 12.327 | 12.31 | B1A040-01 |
| 713.342 | 12.353 | 12.31 | B1A044-01 |
| 649.311 | 12.4 | 12.37 | B1A018-01, B1A064-01 |
| 715.333 | 12.4 | 12.37 | B1A022-01 |
| 586.285 | 13.477 | 13.42 | B1A001-01 |
| 596.328 | 13.447 | 13.42 | B1A027-01, B1A079-01, B1A099-01 |
| 587.28 | 10.673 | 10.63 | B1A002-01 |
| 593.349 | 10.903 | 10.87 | B1A026-01, B1A050-01 |
| 585.289 | 10.78 | 10.75 | B1A024-01, B1A078-01 |
| 618.365 | 10.793 | 10.75 | B1A091-01 |
| 583.344 | 7.963 | 7.92 | B1A052-01 |
| 594.344 | 13.927 | 13.89 | B1A003-01, B1A053-01, B1A075-01 |
| 635.396 | 12.607 | 12.57 | B1A038-01, B1A088-01 |
| 590.349 | 10.177 | 10.13 | B1A095-01 |
| 614.374 | 10.177 | 10.13 | B1A065-01 |
| 608.36 | 14.02 | 13.98 | B1A011-01, B1A055-01 |
| 631.339 | 11.737 | 11.69 | B1A014-01 |
| 617.36 | 8.833 | 8.79 | B1A092-01 |
| 579.369 | 7.68 | 7.65 | B1A048-01, B1A098-01 |
| 573.301 | 7.75 | 7.71 | B1A094-01 |
| 597.324 | 7.733 | 7.71 | B1A100-01, B1A028-01, B1A080-01 |
| 611.37 | 8.313 | 8.26 | B1A082-01 |
| 630.344 | 14.43 | 14.36 | B1A013-01 |
| 648.316 | 14.397 | 14.36 | B1A017-01, B1A063-01 |
| 615.369 | 8.493 | 8.48 | B1A066-01 |
| 623.396 | 8.527 | 8.48 | B1A058-01 |
| 597.324 | 11.3 | 11.25 | B1A100-01, B1A028-01, B1A080-01 |
| 599.314 | 11.24 | 11.25 | B1A006-01 |
| 650.331 | 11.277 | 11.25 | B1A089-01 |
| 609.355 | 11.033 | 11 | B1A012-01, B1A056-01 |
| 588.358 | 9.77 | 9.73 | B1A069-01 |
| 595.339 | 10.857 | 10.81 | B1A004-01, B1A054-01, B1A076-01 |
| 635.396 | 9.397 | 9.36 | B1A038-01, B1A088-01 |
| 565.354 | 8.003 | 7.97 | B1A046-01 |
| 585.289 | 8.107 | 8.07 | B1A024-01, B1A078-01 |
| 625.375 | 8.093 | 8.07 | B1A060-01, B1A084-01 |
| 571.31 | 7.607 | 7.57 | B1A068-01 |
| 597.324 | 13.583 | 13.54 | B1A100-01, B1A028-01, B1A080-01 |
| 598.319 | 13.577 | 13.54 | B1A005-01 |
| 671.32 | 13.57 | 13.54 | B1A042-01 |
| 592.353 | 9.887 | 9.86 | B1A025-01, B1A049-01 |
| 622.4 | 9.9 | 9.86 | B1A057-01 |
| 607.364 | 11.08 | 11.04 | B1A034-01 |
| 609.355 | 8.247 | 8.21 | B1A012-01, B1A056-01 |
| 578.374 | 9.35 | 9.31 | B1A047-01, B1A097-01 |
| 578.374 | 9.383 | 9.31 | B1A047-01, B1A097-01 |
| 582.349 | 9.347 | 9.31 | B1A051-01 |
| 570.315 | 9.273 | 9.23 | B1A067-01 |
| 594.344 | 9.247 | 9.23 | B1A003-01, B1A053-01, B1A075-01 |
| 595.339 | 9.263 | 9.23 | B1A004-01, B1A054-01, B1A076-01 |
| 636.391 | 15.18 | 15.14 | B1A015-01 |
| 625.375 | 8.633 | 8.59 | B1A060-01, B1A084-01 |
| 595.339 | 8.41 | 8.38 | B1A004-01, B1A054-01, B1A076-01 |
| 567.344 | 8.203 | 8.17 | B1A072-01 |
| 593.349 | 8.197 | 8.17 | B1A026-01, B1A050-01 |
| 610.375 | 9.83 | 9.8 | B1A081-01 |
| 596.328 | 9.603 | 9.53 | B1A027-01, B1A079-01, B1A099-01 |
| 651.327 | 9.57 | 9.53 | B1A090-01 |
| 566.349 | 9.47 | 9.43 | B1A071-01 |
| 581.38 | 9.49 | 9.43 | B1A074-01 |
| 592.353 | 13.753 | 13.72 | B1A025-01, B1A049-01 |
| 604.328 | 13.763 | 13.72 | B1A032-01 |
| 606.319 | 13.773 | 13.72 | B1A100-01, B1A033-01 |
| 649.311 | 9.123 | 9.09 | B1A018-01, B1A064-01 |
| 594.344 | 10.353 | 10.31 | B1A003-01, B1A053-01, B1A075-01 |
| 580.365 | 9.5 | 9.46 | B1A073-01 |
| 624.38 | 9.513 | 9.46 | B1A059-01, B1A083-01 |
| 626.37 | 9.527 | 9.46 | B1A085-01 |
| 627.365 | 9.507 | 9.46 | B1A086-01 |
| 591.344 | 8.973 | 8.94 | B1A096-01 |
| 633.341 | 8.977 | 8.94 | B1A062-01 |
| 624.38 | 10.13 | 10.09 | B1A059-01, B1A083-01 |
| 589.354 | 8.737 | 8.7 | B1A070-01 |
| 584.294 | 13.37 | 13.34 | B1A023-01, B1A077-01 |
| 596.328 | 13.43 | 13.34 | B1A027-01, B1A079-01, B1A099-01 |
| 673.311 | 13.717 | 13.67 | B1A020-01 |
| 572.305 | 9.67 | 9.6 | B1A093-01 |
| 608.36 | 9.63 | 9.6 | B1A011-01, B1A055-01 |
| 579.369 | 10.067 | 9.98 | B1A048-01, B1A098-01 |

[Table 109]

| Mixture No. | mixA1-B1K-01 | | |
|---|---|---|---|
| m/z | Retention time of MS peak (min) | Retention time of UV peak (min) | Attributed compound No. |
| 539.201 | 9.893 | 9.86 | B1K009-01 |
| 580.203 | 11.68 | 11.64 | B1K039-01 |
| 646.225 | 12.083 | 12.05 | B1K043-01 |
| 582.193 | 11.327 | 11.29 | B1K017-01, B1K063-01 |
| 648.215 | 11.767 | 11.73 | B1K021-01 |
| 528.222 | 10.577 | 10.54 | B1K003-01, B1K053-01, B1K075-01 |
| 532.197 | 10.34 | 10.29 | B1K005-01 |
| 537.211 | 10.317 | 10.29 | B1K031-01 |
| 568.278 | 8.507 | 8.46 | B1K037-01, B1K087-01 |
| 542.237 | 10.673 | 10.63 | B1K011-01, B1K055-01 |
| 606.193 | 12.23 | 12.2 | B1K019-01 |
| 564.222 | 11.133 | 11.1 | B1K013-01 |
| 518.172 | 7.36 | 7.31 | B1K023-01, B1K077-01 |
| 540.197 | 11.047 | 10.98 | B1K100-01, B1K033-01 |
| 540.197 | 11.023 | 10.98 | B1K100-01, B1K033-01 |
| 563.226 | 8.68 | 8.64 | B1K036-01 |
| 584.209 | 8.673 | 8.64 | B1K089-01 |
| 550.242 | 8.22 | 8.18 | B1K091-01 |
| 556.278 | 8.233 | 8.18 | B1K057-01 |
| 562.231 | 11.513 | 11.47 | B1K035-01 |
| 545.248 | 5.987 | 5.96 | B1K082-01 |
| 500.227 | 7.09 | 7.05 | B1K071-01 |
| 583.189 | 7.09 | 7.05 | B1K018-01, B1K064-01 |
| 571.284 | 9.203 | 9.14 | B1K016-01 |
| 649.21 | 9.177 | 9.14 | B1K022-01 |
| 570.269 | 12.01 | 11.98 | B1K015-01 |
| 529.217 | 7.317 | 7.27 | B1K004-01, B1K054-01, B1K076-01 |
| 531.201 | 7.3 | 7.27 | B1K100-01, B1K028-01, B1K080-01 |
| 505.188 | 12.567 | 12.52 | B1K068-01 |
| 604.203 | 12.557 | 12.52 | B1K041-01 |
| 582.193 | 8.617 | 8.59 | B1K017-01, B1K063-01 |
| 583.189 | 9.07 | 9.03 | B1K018-01, B1K064-01 |
| 506.183 | 7.17 | 7.11 | B1K093-01 |
| 521.158 | 7.143 | 7.11 | B1K002-01 |
| 530.206 | 10.75 | 10.72 | B1K027-01, B1K079-01, B1K099-01 |
| 530.206 | 10.763 | 10.72 | B1K027-01, B1K079-01, B1K099-01 |
| 541.242 | 8.163 | 8.14 | B1K034-01 |
| 565.217 | 8.187 | 8.14 | B1K014-01 |
| 522.236 | 7.877 | 7.83 | B1K069-01 |
| 527.226 | 7.867 | 7.83 | B1K026-01, B1K050-01 |
| 519.167 | 7.74 | 7.71 | B1K024-01, B1K078-01 |
| 533.192 | 7.727 | 7.71 | B1K006-01 |
| 544.253 | 7.76 | 7.71 | B1K081-01 |
| 568.278 | 12.347 | 12.31 | B1K037-01, B1K087-01 |
| 529.217 | 9.63 | 9.59 | B1K004-01, B1K054-01, B1K076-01 |
| 569.273 | 9.657 | 9.59 | B1K038-01, B1K088-01 |
| 647.22 | 9.633 | 9.59 | B1K044-01 |
| 543.233 | 7.667 | 7.62 | B1K012-01, B1K056-01 |
| 585.204 | 7.027 | 6.99 | B1K090-01 |
| 501.222 | 5.597 | 5.56 | B1K072-01 |
| 551.238 | 6.157 | 6.13 | B1K092-01 |
| 581.198 | 9.533 | 9.5 | B1K040-01 |
| 523.222 | 7.543 | 7.51 | B1K003-01, B1K053-01, B1K075-01 |
| 499.232 | 5.757 | 5.71 | B1K046-01 |
| 513.247 | 7.407 | 7.36 | B1K048-01, B1K098-01 |
| 514.242 | 7.407 | 7.36 | B1K073-01 |
| 518.172 | 10.62 | 10.58 | B1K023-01, B1K077-01 |
| 529.217 | 5.847 | 5.8 | B1K004-01, B1K054-01, B1K076-01 |
| 567.219 | 6.93 | 6.89 | B1K062-01 |
| 566.224 | 8.453 | 8.42 | B1K061-01 |
| 569.273 | 8.48 | 8.42 | B1K038-01, B1K088-01 |
| 523.232 | 8.023 | 8.03 | B1K070-01 |
| 526.231 | 8.05 | 8.03 | B1K025-01, B1K049-01 |
| 557.273 | 8.077 | 8.03 | B1K058-01 |
| 558.257 | 8.077 | 8.03 | B1K059-01, B1K083-01 |
| 512.252 | 7.627 | 7.58 | B1K047-01, B1K097-01 |
| 512.252 | 7.6 | 7.56 | B1K047-01, B1K097-01 |
| 560.248 | 7.597 | 7.56 | B1K085-01 |
| 531.201 | 5.37 | 5.34 | B1K100-01, B1K028-01, B1K080-01 |
| 528.222 | 7.957 | 7.91 | B1K003-01, B1K053-01, B1K075-01 |
| 530.206 | 7.953 | 7.91 | B1K027-01, B1K079-01, B1K099-01 |
| 542.237 | 7.937 | 7.91 | B1K011-01, B1K055-01 |
| 524.227 | 7.817 | 7.78 | B1K095-01 |
| 525.222 | 7.8 | 7.78 | B1K096-01 |
| 558.257 | 7.853 | 7.78 | B1K059-01, B1K083-01 |
| 520.162 | 10.163 | 10.13 | B1K001-01 |
| 543.233 | 10.203 | 10.13 | B1K012-01, B1K056-01 |
| 549.247 | 6.297 | 6.28 | B1K066-01 |
| 559.253 | 6.32 | 6.28 | B1K060-01, B1K084-01 |
| 561.243 | 5.783 | 5.75 | B1K086-01 |
| 531.201 | 8.293 | 8.26 | B1K100-01, B1K028-01, B1K080-01 |
| 498.236 | 7.23 | 7.2 | B1K045-01 |
| 504.193 | 7.277 | 7.2 | B1K067-01 |
| 515.238 | 7.26 | 7.2 | B1K074-01 |
| 538.206 | 8.583 | 8.54 | B1K032-01 |
| 513.247 | 9.463 | 9.43 | B1K048-01, B1K098-01 |
| 507.178 | 5.017 | 4.98 | B1K094-01 |
| 516.227 | 7.493 | 7.46 | B1K051-01 |
| 517.222 | 7.503 | 7.46 | B1K052-01 |
| 519.167 | 7.487 | 7.46 | B1K024-01, B1K078-01 |
| 526.231 | 11 | 10.92 | B1K025-01, B1K049-01 |
| 527.226 | 10.983 | 10.92 | B1K026-01, B1K050-01 |
| 605.198 | 10.96 | 10.92 | B1K042-01 |
| 548.252 | 8.26 | 8.22 | B1K065-01 |
| 559.253 | 6.04 | 6.01 | B1K060-01, B1K084-01 |
| 607.189 | 10.563 | 10.51 | B1K020-01 |

[Table 110]

| Mixture No. | mixA1-B1M-01 | | |
|---|---|---|---|
| m/z | Retention time of MS peak (min) | Retention time of UV peak (min) | Attributed compound No. |
| 497.255 | 9.977 | 9.94 | B1M031-01 |
| 531.308 | 8.92 | 8.87 | B1M016-01 |
| 606.269 | 11.847 | 11.81 | B1M043-01 |
| 543.233 | 8.817 | 8.76 | B1M018-01, B1M064-01 |
| 542.237 | 11.143 | 11.11 | B1M017-01, B1M063-01 |
| 526.268 | 8.153 | 8.12 | B1M061-01 |
| 460.271 | 6.86 | 6.82 | B1M071-01 |
| 564.247 | 12.347 | 12.31 | B1M041-01 |
| 542.237 | 8.32 | 8.29 | B1M017-01, B1M063-01 |
| 544.253 | 8.397 | 8.36 | B1M089-01 |
| 484.271 | 7.593 | 7.55 | B1M095-01 |
| 458.28 | 6.973 | 6.94 | B1M045-01 |
| 489.261 | 6.97 | 6.94 | B1M004-01, B1M054-01, B1M076-01 |
| 524.266 | 10.953 | 10.91 | B1M013-01 |
| 490.25 | 10.427 | 10.39 | B1M027-01, B1M079-01, B1M099-01 |
| 502.281 | 10.41 | 10.39 | B1M011-01, B1M055-01 |
| 528.322 | 8.227 | 8.19 | B1M037-01, B1M087-01 |
| 489.261 | 5.403 | 5.34 | B1M004-01, B1M054-01, B1M076-01 |
| 529.317 | 9.28 | 9.23 | B1M038-01, B1M088-01 |
| 607.264 | 9.263 | 9.23 | B1M044-01 |
| 522.275 | 11.207 | 11.17 | B1M035-01 |
| 481.202 | 11.407 | 11.37 | B1M002-01 |
| 540.247 | 11.403 | 11.37 | B1M039-01 |
| 505.292 | 5.8 | 5.77 | B1M082-01 |
| 478.216 | 7.127 | 7.09 | B1M023-01, B1M077-01 |
| 490.25 | 7.127 | 7.09 | B1M027-01, B1M079-01, B1M099-01 |
| 467.222 | 4.867 | 4.83 | B1M094-01 |
| 545.248 | 6.75 | 6.71 | B1M090-01 |
| 499.245 | 9.61 | 9.58 | B1M009-01 |
| 529.317 | 9.653 | 9.58 | B1M038-01, B1M088-01 |
| 519.297 | 6.14 | 6.11 | B1M060-01, B1M084-01 |
| 509.291 | 6.03 | 6 | B1M066-01 |
| 488.266 | 10.337 | 10.3 | B1M003-01, B1M053-01, B1M075-01 |
| 567.233 | 10.327 | 10.3 | B1M020-01 |
| 486.275 | 7.797 | 7.75 | B1M025-01, B1M049-01 |
| 541.242 | 9.14 | 9.1 | B1M040-01 |
| 508.296 | 7.977 | 7.94 | B1M065-01 |
| 510.286 | 7.97 | 7.94 | B1M091-01 |
| 517.317 | 7.997 | 7.94 | B1M058-01 |
| 479.211 | 7.32 | 7.27 | B1M024-01, B1M078-01 |
| 488.266 | 7.333 | 7.27 | B1M003-01, B1M053-01, B1M075-01 |
| 503.277 | 7.31 | 7.27 | B1M012-01, B1M056-01 |
| 492.241 | 10.08 | 10.04 | B1M005-01 |
| 498.25 | 10.077 | 10.04 | B1M032-01 |
| 527.263 | 10.057 | 10.04 | B1M062-01 |
| 478.216 | 10.273 | 10.23 | B1M023-01, B1M077-01 |
| 491.245 | 7.86 | 7.82 | B1M100-01, B1M028-01, B1M080-01 |
| 518.301 | 7.883 | 7.82 | B1M059-01, B1M083-01 |
| 525.261 | 7.86 | 7.82 | B1M014-01 |
| 480.206 | 9.89 | 9.85 | B1M001-01 |
| 482.28 | 7.63 | 7.59 | B1M069-01 |
| 518.301 | 7.637 | 7.59 | B1M059-01, B1M083-01 |
| 608.259 | 11.623 | 11.59 | B1M021-01 |
| 609.254 | 11.637 | 11.59 | B1M022-01 |
| 491.245 | 5.197 | 5.17 | B1M100-01, B1M028-01, B1M080-01 |
| 459.275 | 5.477 | 5.43 | B1M046-01 |
| 477.266 | 5.457 | 5.43 | B1M052-01 |
| 487.27 | 5.753 | 5.71 | B1M026-01, B1M050-01 |
| 511.282 | 5.937 | 5.9 | B1M092-01 |
| 464.237 | 8.267 | 8.22 | B1M067-01 |
| 523.27 | 8.26 | 8.22 | B1M036-01 |
| 472.296 | 7.383 | 7.34 | B1M047-01, B1M097-01 |
| 493.236 | 7.377 | 7.34 | B1M006-01 |
| 543.233 | 8.763 | 8.72 | B1M018-01, B1M064-01 |
| 485.266 | 10.667 | 10.64 | B1M096-01 |
| 486.275 | 10.677 | 10.64 | B1M025-01, B1M049-01 |
| 500.24 | 10.683 | 10.64 | B1M100-01, B1M033-01 |
| 500.24 | 10.677 | 10.64 | B1M100-01, B1M032-01 |
| 565.242 | 10.65 | 10.64 | B1M042-01 |
| 473.291 | 5.26 | 5.22 | B1M048-01, B1M098-01 |
| 465.232 | 4.99 | 4.96 | B1M068-01 |
| 487.27 | 7.437 | 7.4 | B1M026-01, B1M050-01 |
| 490.25 | 7.463 | 7.4 | B1M027-01, B1M079-01, B1M099-01 |
| 519.297 | 7.5 | 7.4 | B1M060-01, B1M084-01 |
| 520.292 | 7.433 | 7.4 | B1M085-01 |
| 466.227 | 6.907 | 6.87 | B1M093-01 |
| 530.313 | 11.903 | 11.86 | B1M015-01 |
| 473.291 | 7.197 | 7.19 | B1M048-01, B1M098-01 |
| 474.286 | 7.203 | 7.19 | B1M073-01 |
| 475.282 | 7.227 | 7.19 | B1M074-01 |
| 476.271 | 7.217 | 7.19 | B1M051-01 |
| 488.266 | 7.723 | 7.7 | B1M003-01, B1M053-01, B1M075-01 |
| 501.286 | 7.737 | 7.7 | B1M034-01 |
| 502.281 | 7.713 | 7.7 | B1M011-01, B1M055-01 |
| 503.277 | 7.707 | 7.7 | B1M012-01, B1M056-01 |
| 489.261 | 5.622 | 5.58 | B1M004-01, B1M054-01, B1M076-01 |
| 491.245 | 5.633 | 5.58 | B1M100-01, B1M028-01, B1M080-01 |
| 521.287 | 5.61 | 5.58 | B1M086-01 |
| 483.275 | 6.223 | 6.15 | B1M070-01 |
| 461.266 | 5.363 | 5.29 | B1M072-01 |
| 472.296 | 5.343 | 5.29 | B1M047-01, B1M097-01 |
| 479.211 | 5.33 | 5.29 | B1M024-01, B1M078-01 |
| 504.297 | 7.573 | 7.53 | B1M081-01 |
| 516.322 | 8.013 | 7.97 | B1M057-01 |
| 528.322 | 12.107 | 12.07 | B1M037-01, B1M087-01 |
| 566.237 | 12.13 | 12.07 | B1M019-01 |

[Table 111]

| Mixture No. | mixA1-B2Q-01 | | |
|---|---|---|---|
| m/z | Retention time of MS peak (min) | Retention time of UV peak (min) | Attributed compound No. |
| 834.224 | 13.403 | 13.37 | B2Q039-01 |
| 792.227 | 11.153 | 11.11 | B2Q032-01 |
| 771.243 | 8.4 | 8.38 | B2Q052-01 |
| 793.222 | 12.13 | 12.1 | B2Q009-01 |
| 786.217 | 12.46 | 12.41 | B2Q005-01 |
| 791.232 | 12.44 | 12.41 | B2Q031-01 |
| 817.247 | 11.3 | 11.26 | B2Q038-01 |
| 776.257 | 9.887 | 9.84 | B2Q069-01 |
| 861.209 | 12.583 | 12.55 | B2Q020-01 |
| 816.252 | 13.337 | 13.31 | B2Q035-01 |
| 794.217 | 13.023 | 12.99 | B2Q100-01, B2Q033-01 |
| 900.246 | 13.653 | 13.61 | B2Q043-01 |
| 781.247 | 10.717 | 10.68 | B2Q026-01, B2Q050-01 |
| 836.214 | 10.717 | 10.68 | B2Q017-01, B2Q063-01 |
| 858.224 | 14.037 | 14 | B2Q041-01 |
| 836.214 | 13.157 | 13.11 | B2Q017-01, B2Q063-01 |
| 837.209 | 11.56 | 11.52 | B2Q018-01, B2Q064-01 |
| 903.231 | 11.55 | 11.52 | B2Q022-01 |
| 752.257 | 9.413 | 9.38 | B2Q045-01 |
| 782.243 | 9.4 | 9.38 | B2Q003-01, B2Q053-01, B2Q075-01 |
| 782.243 | 12.877 | 12.84 | B2Q003-01, B2Q053-01, B2Q075-01 |
| 775.179 | 10.22 | 10.18 | B2Q002-01 |
| 802.273 | 10.387 | 10.35 | B2Q065-01 |
| 761.198 | 8.06 | 8.02 | B2Q094-01 |
| 780.252 | 12.983 | 12.94 | B2Q025-01, B2Q049-01 |
| 811.294 | 8.833 | 8.79 | B2Q058-01 |
| 803.268 | 8.923 | 8.87 | B2Q066-01 |
| 835.219 | 11.88 | 11.84 | B2Q040-01 |
| 901.241 | 11.887 | 11.84 | B2Q044-01 |
| 902.236 | 11.863 | 11.84 | B2Q021-01 |
| 784.227 | 12.757 | 12.72 | B2Q027-01, B2Q079-01, B2Q099-01 |
| 796.258 | 12.783 | 12.72 | B2Q011-01, B2Q055-01 |
| 773.188 | 10.627 | 10.57 | B2Q024-01, B2Q078-01 |
| 787.213 | 10.65 | 10.57 | B2Q006-01 |
| 804.263 | 10.6 | 10.57 | B2Q091-01 |
| 820.244 | 10.597 | 10.57 | B2Q061-01 |
| 794.217 | 10.807 | 10.77 | B2Q100-01, B2Q033-01 |
| 818.243 | 13.083 | 13.03 | B2Q013-01 |
| 779.243 | 9.097 | 9.06 | B2Q096-01 |
| 777.252 | 9.04 | 9 | B2Q070-01 |
| 795.263 | 10.923 | 10.89 | B2Q034-01 |
| 819.238 | 10.94 | 10.89 | B2Q014-01 |
| 822.299 | 10.933 | 10.89 | B2Q037-01, B2Q087-01 |
| 824.289 | 10.927 | 10.89 | B2Q015-01 |
| 784.227 | 9.247 | 9.21 | B2Q027-01, B2Q079-01, B2Q099-01 |
| 754.248 | 9.463 | 9.43 | B2Q071-01 |
| 768.263 | 9.46 | 9.43 | B2Q073-01 |
| 823.294 | 9.5 | 9.43 | B2Q038-01, B2Q088-01 |
| 837.209 | 9.467 | 9.43 | B2Q018-01, B2Q064-01 |
| 753.252 | 8.463 | 8.44 | B2Q046-01 |
| 755.243 | 8.477 | 8.44 | B2Q072-01 |
| 785.222 | 8.51 | 8.44 | B2Q100-01, B2Q028-01, B2Q080-01 |
| 785.222 | 11.033 | 11.01 | B2Q100-01, B2Q028-01, B2Q080-01 |
| 838.23 | 11.057 | 11.01 | B2Q089-01 |
| 839.225 | 11.037 | 11.01 | B2Q090-01 |
| 797.253 | 10.543 | 10.51 | B2Q012-01, B2Q056-01 |
| 783.238 | 8.603 | 8.56 | B2Q004-01, B2Q054-01, B2Q076-01 |
| 797.253 | 8.587 | 8.56 | B2Q012-01, B2Q056-01 |
| 798.274 | 8.587 | 8.56 | B2Q081-01 |
| 778.248 | 10.047 | 10 | B2Q095-01 |
| 810.299 | 10.027 | 10 | B2Q057-01 |
| 813.273 | 10.03 | 10 | B2Q060-01, B2Q084-01 |
| 770.248 | 9.617 | 9.59 | B2Q051-01 |
| 812.278 | 9.607 | 9.59 | B2Q059-01, B2Q083-01 |
| 813.273 | 9.593 | 9.59 | B2Q060-01, B2Q084-01 |
| 814.269 | 9.613 | 9.59 | B2Q085-01 |
| 805.258 | 8.967 | 8.93 | B2Q092-01 |
| 821.24 | 9.35 | 9.31 | B2Q062-01 |
| 815.264 | 12.927 | 12.84 | B2Q086-01 |
| 859.219 | 12.883 | 12.84 | B2Q042-01 |
| 860.214 | 12.877 | 12.84 | B2Q019-01 |
| 822.299 | 12.05 | 12.01 | B2Q037-01, B2Q087-01 |
| 823.294 | 12.043 | 12.01 | B2Q038-01, B2Q088-01 |
| 825.285 | 12.037 | 12.01 | B2Q016-01 |
| 784.227 | 9.783 | 9.74 | B2Q027-01, B2Q079-01, B2Q099-01 |
| 796.258 | 9.783 | 9.74 | B2Q011-01, B2Q055-01 |
| 799.269 | 9.77 | 9.74 | B2Q082-01 |
| 767.268 | 6.52 | 7.74 | B2Q048-01, B2Q098-01 |
| 769.258 | 6.837 | 7.74 | B2Q074-01 |
| 772.193 | 9.693 | 9.66 | B2Q023-01, B2Q077-01 |
| 772.193 | 9.717 | 9.66 | B2Q023-01, B2Q077-01 |
| 774.193 | 9.697 | 9.66 | B2Q001-01 |
| 783.238 | 10.3 | 10.27 | B2Q004-01, B2Q054-01, B2Q076-01 |
| 785.222 | 10.313 | 10.27 | B2Q100-01, B2Q028-01, B2Q080-01 |
| 759.209 | 8.1 | 8.05 | B2Q068-01 |
| 760.204 | 8.113 | 8.05 | B2Q093-01 |
| 767.268 | 8.087 | 8.05 | B2Q048-01, B2Q098-01 |
| 783.238 | 8.143 | 8.05 | B2Q004-01, B2Q054-01, B2Q076-01 |
| 758.214 | 9.547 | 9.49 | B2Q067-01 |
| 766.273 | 9.537 | 9.49 | B2Q047-01, B2Q097-01 |
| 766.273 | 9.567 | 9.49 | B2Q047-01, B2Q097-01 |
| 812.278 | 9.59 | 9.49 | B2Q059-01, B2Q083-01 |
| 773.188 | 14.453 | 14.36 | B2Q024-01, B2Q078-01 |
| 780.252 | 10.097 | 10.06 | B2Q025-01, B2Q049-01 |
| 781.247 | 10.093 | 10.06 | B2Q026-01, B2Q050-01 |
| 782.243 | 10.113 | 10.06 | B2Q003-01, B2Q053-01, B2Q075-01 |

[Table 112]

| Mixture No. | mixAI--B1O--01 | | |
|---|---|---|---|
| m/z | Retention time of MS peak (min) | Retention time of UV peak (min) | Attributed compound No. |
| 606.232 | 12.077 | 12.03 | B1O017-01, B1O063-01 |
| 564.235 | 10.68 | 10.64 | B1O100-01, B1O033-01 |
| 574.281 | 8.87 | 8.83 | B1O091-01 |
| 524.266 | 7.723 | 7.68 | B1O071-01 |
| 528.232 | 7.74 | 7.68 | B1O067-01 |
| 604.242 | 12.157 | 12.12 | B1O039-01 |
| 561.25 | 10.857 | 10.81 | B1O031-01 |
| 673.249 | 9.983 | 9.94 | B1O022-01 |
| 606.232 | 9.003 | 8.97 | B1O017-01, B1O063-01 |
| 671.259 | 10.183 | 10.14 | B1O044-01 |
| 672.254 | 12.44 | 12.4 | B1O021-01 |
| 628.242 | 12.993 | 12.96 | B1O041-01 |
| 630.232 | 12.93 | 12.89 | B1O019-01 |
| 556.235 | 11.127 | 11.09 | B1O005-01 |
| 562.245 | 11.153 | 11.09 | B1O032-01 |
| 554.245 | 11.283 | 11.26 | B1O027-01, B1O079-01, B1O099-01 |
| 631.228 | 11.31 | 11.26 | B1O020-01 |
| 588.261 | 11.953 | 11.91 | B1O013-01 |
| 592.317 | 9.173 | 9.14 | B1O037-01, B1O087-01 |
| 593.312 | 7.54 | 7.5 | B1O038-01, B1O088-01 |
| 589.256 | 9.127 | 9.08 | B1O014-01 |
| 523.27 | 6.337 | 6.31 | B1O046-01 |
| 525.261 | 6.353 | 6.31 | B1O072-01 |
| 607.228 | 7.57 | 7.53 | B1O018-01, B1O064-01 |
| 557.231 | 8.653 | 8.62 | B1O006-01 |
| 572.291 | 8.653 | 8.62 | B1O065-01 |
| 607.228 | 9.917 | 9.88 | B1O018-01, B1O064-01 |
| 536.291 | 8.003 | 7.97 | B1O047-01, B1O097-01 |
| 575.277 | 6.947 | 6.91 | B1O092-01 |
| 583.291 | 7.013 | 6.98 | B1O060-01, B1O084-01 |
| 581.312 | 7.09 | 7.04 | B1O058-01 |
| 536.291 | 7.983 | 7.92 | B1O047-01, B1O097-01 |
| 538.281 | 7.947 | 7.92 | B1O073-01 |
| 542.211 | 7.957 | 7.92 | B1O023-01, B1O077-01 |
| 553.256 | 6.2 | 6.16 | B1O004-01, B1O054-01, B1O076-01 |
| 550.27 | 11.543 | 11.52 | B1O025-01, B1O049-01 |
| 564.235 | 11.56 | 11.52 | B1O100-01, B1O033-01 |
| 552.261 | 11.4 | 11.37 | B1O003-01, B1O053-01, B1O075-01 |
| 573.288 | 6.887 | 6.85 | B1O066-01 |
| 593.312 | 10.257 | 10.22 | B1O038-01, B1O088-01 |
| 595.303 | 10.09 | 10.07 | B1O016-01 |
| 605.237 | 10.12 | 10.07 | B1O040-01 |
| 591.258 | 7.413 | 7.37 | B1O062-01 |
| 587.265 | 9.33 | 9.3 | B1O036-01 |
| 608.248 | 9.347 | 9.3 | B1O089-01 |
| 609.243 | 9.33 | 9.3 | B1O090-01 |
| 544.201 | 10.997 | 10.95 | B1O001-01 |
| 566.276 | 11.487 | 11.44 | B1O011-01, B1O055-01 |
| 629.237 | 11.457 | 11.44 | B1O042-01 |
| 543.206 | 8.43 | 8.4 | B1O024-01, B1O078-01 |
| 548.266 | 8.413 | 8.4 | B1O095-01 |
| 549.261 | 8.44 | 8.4 | B1O096-01 |
| 550.27 | 8.447 | 8.4 | B1O025-01, B1O049-01 |
| 547.27 | 12.53 | 12.49 | B1O070-01 |
| 670.264 | 12.52 | 12.49 | B1O043-01 |
| 537.286 | 6.077 | 6.03 | B1O048-01, B1O098-01 |
| 555.24 | 6.067 | 6.03 | B1O100-01, B1O028-01, B1O080-01 |
| 539.277 | 5.973 | 5.93 | B1O074-01 |
| 567.271 | 6.757 | 6.72 | B1O012-01, B1O056-01 |
| 545.197 | 8.11 | 8.07 | B1O002-01 |
| 554.245 | 8.14 | 8.07 | B1O027-01, B1O079-01, B1O099-01 |
| 584.287 | 8.1 | 8.07 | B1O085-01 |
| 594.307 | 12.79 | 12.76 | B1O015-01 |
| 546.275 | 8.297 | 8.24 | B1O069-01 |
| 553.256 | 8.277 | 8.24 | B1O004-01, B1O054-01, B1O076-01 |
| 553.256 | 8.297 | 8.24 | B1O004-01, B1O054-01, B1O076-01 |
| 566.276 | 8.307 | 8.24 | B1O011-01, B1O055-01 |
| 568.292 | 8.27 | 8.24 | B1O081-01 |
| 569.287 | 8.297 | 8.24 | B1O082-01 |
| 530.222 | 7.843 | 7.78 | B1O093-01 |
| 554.245 | 7.82 | 7.78 | B1O027-01, B1O079-01, B1O099-01 |
| 551.265 | 8.56 | 8.54 | B1O026-01, B1O050-01 |
| 551.265 | 8.543 | 8.54 | B1O026-01, B1O050-01 |
| 552.261 | 8.61 | 8.54 | B1O003-01, B1O053-01, B1O075-01 |
| 567.271 | 8.537 | 8.54 | B1O012-01, B1O056-01 |
| 580.317 | 8.58 | 8.54 | B1O057-01 |
| 582.296 | 8.573 | 8.54 | B1O059-01, B1O083-01 |
| 583.291 | 8.583 | 8.54 | B1O060-01, B1O084-01 |
| 541.261 | 6.307 | 6.25 | B1O052-01 |
| 543.206 | 6.287 | 6.25 | B1O024-01, B1O078-01 |
| 592.317 | 12.837 | 12.8 | B1O037-01, B1O087-01 |
| 531.217 | 8.227 | 8.17 | B1O094-01 |
| 582.296 | 8.21 | 8.17 | B1O059-01, B1O083-01 |
| 565.281 | 8.8 | 8.77 | B1O034-01 |
| 590.263 | 8.85 | 8.77 | B1O061-01 |
| 537.286 | 7.943 | 7.87 | B1O048-01, B1O098-01 |
| 540.266 | 7.91 | 7.87 | B1O051-01 |
| 552.261 | 7.94 | 7.87 | B1O003-01, B1O053-01, B1O075-01 |
| 555.24 | 8.97 | 8.93 | B1O100-01, B1O028-01, B1O080-01 |
| 555.24 | 6.467 | 6.39 | B1O100-01, B1O028-01, B1O080-01 |
| 585.282 | 6.437 | 6.39 | B1O086-01 |
| 529.227 | 9.657 | 9.62 | B1O068-01 |
| 522.275 | 7.697 | 7.65 | B1O045-01 |
| 596.27 | 12.047 | 11.99 | B1O035-01 |
| 542.211 | 11.173 | 11.13 | B1O023-01, B1O077-01 |

[Table 113]

| Mixture No. | mixA1-B3A-01 | | |
|---|---|---|---|
| m/z | Retention time of MS peak (min) | Retention time of UV peak (min) | Attributed compound No. |
| 617.349 | 13.957 | 13.92 | B3A031-01 |
| 660.341 | 15.067 | 15.02 | B3A039-01 |
| 726.363 | 15.26 | 15.21 | B3A043-01 |
| 598.31 | 9.143 | 9.1 | B3A023-01, B3A077-01 |
| 729.348 | 11.76 | 11.72 | B3A022-01 |
| 684.341 | 15.713 | 15.67 | B3A041-01 |
| 728.353 | 14.123 | 14.09 | B3A021-01 |
| 612.334 | 12.937 | 12.9 | B3A005-01 |
| 643.364 | 12.69 | 12.65 | B3A036-01 |
| 644.36 | 13.813 | 13.79 | B3A013-01 |
| 662.331 | 13.833 | 13.79 | B3A017-01, B3A063-01 |
| 649.411 | 13.663 | 13.61 | B3A038-01, B3A088-01 |
| 607.364 | 11.853 | 11.82 | B3A026-01, B3A050-01 |
| 628.39 | 10.723 | 10.68 | B3A065-01 |
| 642.369 | 15.15 | 15.11 | B3A035-01 |
| 599.305 | 11.703 | 11.67 | B3A024-01, B3A078-01 |
| 646.361 | 10.953 | 10.92 | B3A061-01 |
| 606.369 | 14.703 | 14.68 | B3A025-01, B3A049-01 |
| 620.334 | 14.72 | 14.68 | B3A100-01, B3A033-01 |
| 601.296 | 9.933 | 9.9 | B3A002-01 |
| 609.355 | 10.133 | 10.08 | B3A004-01, B3A054-01, B3A076-01 |
| 647.357 | 9.447 | 9.4 | B3A062-01 |
| 610.344 | 14.367 | 14.33 | B3A027-01, B3A079-01, B3A099-01 |
| 611.339 | 14.387 | 14.33 | B3A100-01, B3A028-01, B3A080-01 |
| 685.336 | 14.47 | 14.44 | B3A042-01 |
| 596.365 | 9.787 | 9.75 | B3A051-01 |
| 687.327 | 13.103 | 13.06 | B3A020-01 |
| 650.406 | 14.62 | 14.58 | B3A015-01 |
| 686.331 | 14.613 | 14.58 | B3A019-01 |
| 608.36 | 13.263 | 13.25 | B3A003-01, B3A053-01, B3A075-01 |
| 609.355 | 13.267 | 13.25 | B3A004-01, B3A054-01, B3A076-01 |
| 661.336 | 13.25 | 13.25 | B3A040-01 |
| 727.358 | 13.297 | 13.25 | B3A044-01 |
| 618.344 | 12.27 | 12.23 | B3A032-01 |
| 619.339 | 12.493 | 12.45 | B3A009-01 |
| 620.334 | 12.48 | 12.45 | B3A100-01, B3A033-01 |
| 622.375 | 13.373 | 13.34 | B3A011-01, B3A055-01 |
| 600.3 | 12.803 | 12.78 | B3A001-01 |
| 640.386 | 9.193 | 9.16 | B3A085-01 |
| 638.395 | 9.867 | 9.83 | B3A059-01, B3A083-01 |
| 645.355 | 11.01 | 10.96 | B3A014-01 |
| 610.344 | 10.067 | 10.03 | B3A027-01, B3A079-01, B3A099-01 |
| 630.38 | 10.07 | 10.03 | B3A091-01 |
| 621.38 | 11.98 | 11.95 | B3A034-01 |
| 651.402 | 12.023 | 11.95 | B3A016-01 |
| 599.305 | 7.64 | 7.6 | B3A024-01, B3A078-01 |
| 648.416 | 15.803 | 15.76 | B3A037-01, B3A087-01 |
| 649.411 | 8.793 | 8.75 | B3A038-01, B3A088-01 |
| 606.369 | 10.38 | 10.33 | B3A025-01, B3A049-01 |
| 623.37 | 10.34 | 10.33 | B3A012-01, B3A056-01 |
| 648.416 | 10.37 | 10.33 | B3A037-01, B3A087-01 |
| 578.374 | 9.637 | 9.61 | B3A045-01 |
| 604.365 | 9.647 | 9.61 | B3A095-01 |
| 638.395 | 9.69 | 9.61 | B3A059-01, B3A083-01 |
| 611.339 | 12.213 | 12.18 | B3A100-01, B3A028-01, B3A080-01 |
| 609.355 | 8.12 | 8.08 | B3A004-01, B3A054-01, B3A076-01 |
| 631.376 | 8.237 | 8.19 | B3A092-01 |
| 639.39 | 8.23 | 8.19 | B3A060-01, B3A084-01 |
| 625.386 | 7.92 | 7.87 | B3A082-01 |
| 611.339 | 7.39 | 7.35 | B3A100-01, B3A028-01, B3A080-01 |
| 595.378 | 7.327 | 7.28 | B3A074-01 |
| 602.374 | 10.267 | 10.23 | B3A069-01 |
| 636.416 | 10.31 | 10.23 | B3A057-01 |
| 662.331 | 11.103 | 11.06 | B3A017-01, B3A063-01 |
| 663.327 | 11.113 | 11.06 | B3A018-01, B3A064-01 |
| 580.365 | 8.677 | 8.92 | B3A071-01 |
| 610.344 | 8.947 | 8.92 | B3A027-01, B3A079-01, B3A099-01 |
| 629.385 | 8.947 | 8.92 | B3A066-01 |
| 665.342 | 8.95 | 8.92 | B3A090-01 |
| 587.316 | 7.28 | 7.23 | B3A094-01 |
| 597.36 | 8.36 | 8.32 | B3A052-01 |
| 598.31 | 14.31 | 14.27 | B3A023-01, B3A077-01 |
| 593.385 | 8.05 | 8.01 | B3A048-01, B3A098-01 |
| 585.326 | 8.01 | 7.98 | B3A068-01 |
| 637.411 | 8.863 | 8.85 | B3A058-01 |
| 593.385 | 10.55 | 10.49 | B3A048-01, B3A098-01 |
| 613.33 | 10.557 | 10.49 | B3A006-01 |
| 664.347 | 10.52 | 10.49 | B3A089-01 |
| 608.36 | 9.597 | 9.56 | B3A003-01, B3A053-01, B3A075-01 |
| 663.327 | 9.597 | 9.56 | B3A018-01, B3A064-01 |
| 581.36 | 7.723 | 7.68 | B3A072-01 |
| 641.381 | 7.707 | 7.68 | B3A086-01 |
| 607.364 | 8.637 | 8.59 | B3A026-01, B3A050-01 |
| 623.37 | 8.603 | 8.59 | B3A012-01, B3A056-01 |
| 579.369 | 8.44 | 8.41 | B3A046-01 |
| 605.36 | 8.487 | 8.41 | B3A096-01 |
| 639.39 | 8.453 | 8.41 | B3A060-01, B3A084-01 |
| 584.331 | 9.73 | 9.69 | B3A067-01 |
| 592.39 | 9.74 | 9.69 | B3A047-01, B3A097-01 |
| 592.39 | 9.72 | 9.69 | B3A047-01, B3A097-01 |
| 608.36 | 9.72 | 9.69 | B3A003-01, B3A053-01, B3A075-01 |
| 622.375 | 10.023 | 9.99 | B3A011-01, B3A055-01 |
| 624.391 | 9.4 | 9.36 | B3A081-01 |
| 586.321 | 9.073 | 9.04 | B3A093-01 |
| 594.38 | 9.09 | 9.04 | B3A073-01 |
| 603.369 | 9.11 | 9.04 | B3A070-01 |

[Table 114]

| Mixture No. | mixAJ-B4J-01 | | |
|---|---|---|---|
| m/z | Retention time of MS peak (min) | Retention time of UV peak (min) | Attributed compound No. |
| 623.27 | 8.463 | 8.42 | B4J022-01 |
| 621.29 | 9.76 | 9.72 | B4J044-01 |
| 511.27 | 10.47 | 10.43 | B4J031-01 |
| 538.281 | 10.473 | 10.43 | B4J013-01 |
| 506.256 | 9.6 | 9.56 | B4J005-01 |
| 620.284 | 12.403 | 12.36 | B4J043-01 |
| 513.261 | 9.163 | 9.12 | B4J009-01 |
| 578.263 | 12.937 | 12.9 | B4J041-01 |
| 556.253 | 10.73 | 10.69 | B4J017-01, B4J063-01 |
| 495.217 | 6.33 | 6.29 | B4J002-01 |
| 556.253 | 8.57 | 8.52 | B4J017-01, B4J063-01 |
| 580.253 | 11.743 | 11.71 | B4J019-01 |
| 502.281 | 9.847 | 9.81 | B4J003-01, B4J053-01, B4J075-01 |
| 537.286 | 8.777 | 8.74 | B4J036-01 |
| 500.291 | 11.263 | 11.21 | B4J025-01, B4J049-01 |
| 514.256 | 11.25 | 11.21 | B4J100-01, B4J033-01 |
| 622.275 | 11.247 | 11.21 | B4J021-01 |
| 539.277 | 7.373 | 7.33 | B4J014-01 |
| 481.238 | 11.973 | 11.94 | B4J094-01 |
| 554.263 | 11.987 | 11.94 | B4J039-01 |
| 516.297 | 9.933 | 9.9 | B4J011-01, B4J055-01 |
| 487.307 | 5.563 | 5.52 | B4J048-01, B4J098-01 |
| 503.277 | 6.497 | 6.47 | B4J004-01, B4J054-01, B4J076-01 |
| 492.232 | 10.833 | 10.8 | B4J023-01, B4J077-01 |
| 475.282 | 8.12 | 8.13 | B4J072-01 |
| 522.312 | 8.223 | 8.18 | B4J065-01 |
| 530.338 | 8.21 | 8.18 | B4J057-01 |
| 501.286 | 6.027 | 5.95 | B4J026-01, B4J050-01 |
| 555.258 | 9.64 | 9.6 | B4J040-01 |
| 494.222 | 9.37 | 9.34 | B4J001-01 |
| 581.248 | 9.9 | 9.85 | B4J020-01 |
| 498.286 | 7.34 | 7.29 | B4J095-01 |
| 518.313 | 7.327 | 7.28 | B4J081-01 |
| 533.312 | 5.913 | 5.87 | B4J060-01, B4J084-01 |
| 532.317 | 7.847 | 7.8 | B4J059-01, B4J083-01 |
| 542.338 | 7.85 | 7.8 | B4J037-01, B4J087-01 |
| 492.232 | 6.84 | 6.8 | B4J023-01, B4J077-01 |
| 496.296 | 7.903 | 7.88 | B4J069-01 |
| 501.286 | 7.927 | 7.88 | B4J026-01, B4J050-01 |
| 516.297 | 7.913 | 7.88 | B4J011-01, B4J055-01 |
| 517.292 | 6.21 | 6.15 | B4J012-01, B4J056-01 |
| 543.333 | 6.183 | 6.15 | B4J038-01, B4J088-01 |
| 557.248 | 7.05 | 7.01 | B4J018-01, B4J064-01 |
| 488.302 | 6.973 | 6.93 | B4J073-01 |
| 512.266 | 8.627 | 8.58 | B4J032-01 |
| 557.248 | 8.28 | 8.24 | B4J018-01, B4J064-01 |
| 489.297 | 4.833 | 4.8 | B4J074-01 |
| 505.281 | 8.35 | 8.31 | B4J100-01, B4J028-01, B4J080-01 |
| 543.333 | 9.817 | 9.77 | B4J038-01, B4J088-01 |
| 579.258 | 11.187 | 11.15 | B4J042-01 |
| 503.277 | 10.963 | 10.94 | B4J004-01, B4J054-01, B4J076-01 |
| 504.266 | 10.973 | 10.94 | B4J027-01, B4J079-01, B4J099-01 |
| 505.281 | 10.953 | 10.94 | B4J100-01, B4J028-01, B4J080-01 |
| 499.282 | 6.097 | 6.05 | B4J096-01 |
| 533.312 | 6.083 | 6.05 | B4J060-01, B4J084-01 |
| 534.307 | 7.197 | 7.16 | B4J085-01 |
| 542.338 | 12.76 | 12.73 | B4J037-01, B4J087-01 |
| 474.286 | 6.553 | 6.53 | B4J071-01 |
| 480.243 | 6.553 | 6.53 | B4J093-01 |
| 531.333 | 6.573 | 6.53 | B4J058-01 |
| 490.286 | 7.45 | 7.42 | B4J051-01 |
| 519.308 | 7.467 | 7.42 | B4J082-01 |
| 504.266 | 6.903 | 6.85 | B4J027-01, B4J079-01, B4J099-01 |
| 507.251 | 6.897 | 6.85 | B4J006-01 |
| 517.292 | 6.887 | 6.85 | B4J012-01, B4J056-01 |
| 541.278 | 6.877 | 6.85 | B4J062-01 |
| 473.291 | 5.777 | 5.73 | B4J046-01 |
| 491.282 | 5.747 | 5.73 | B4J052-01 |
| 535.303 | 11.83 | 11.8 | B4J086-01 |
| 536.291 | 11.84 | 11.8 | B4J035-01 |
| 540.283 | 8.387 | 8.35 | B4J061-01 |
| 545.323 | 8.433 | 8.35 | B4J016-01 |
| 500.291 | 8.027 | 7.98 | B4J025-01, B4J049-01 |
| 558.269 | 8.01 | 7.98 | B4J089-01 |
| 559.264 | 8.023 | 7.98 | B4J090-01 |
| 502.281 | 7.41 | 7.37 | B4J003-01, B4J053-01, B4J075-01 |
| 472.296 | 7.247 | 7.2 | B4J045-01 |
| 478.252 | 7.247 | 7.2 | B4J067-01 |
| 515.302 | 7.25 | 7.2 | B4J034-01 |
| 479.248 | 5.323 | 5.3 | B4J068-01 |
| 503.277 | 5.34 | 5.3 | B4J004-01, B4J054-01, B4J076-01 |
| 525.297 | 5.643 | 5.6 | B4J092-01 |
| 486.312 | 7.597 | 7.57 | B4J047-01, B4J097-01 |
| 487.307 | 7.603 | 7.57 | B4J048-01, B4J098-01 |
| 524.302 | 7.62 | 7.57 | B4J091-01 |
| 532.317 | 7.637 | 7.57 | B4J059-01, B4J083-01 |
| 493.227 | 7.79 | 7.75 | B4J024-01, B4J078-01 |
| 497.291 | 7.74 | 7.75 | B4J070-01 |
| 504.266 | 7.72 | 7.75 | B4J027-01, B4J079-01, B4J099-01 |
| 514.256 | 7.76 | 7.75 | B4J100-01, B4J033-01 |
| 544.328 | 11.46 | 11.43 | B4J015-01 |
| 486.312 | 7.577 | 7.5 | B4J047-01, B4J097-01 |
| 502.281 | 7.537 | 7.5 | B4J003-01, B4J053-01, B4J075-01 |
| 493.227 | 5.027 | 4.95 | B4J024-01, B4J078-01 |
| 505.281 | 4.953 | 4.88 | B4J100-01, B4J028-01, B4J080-01 |
| 523.307 | 6.3 | 6.26 | B4J066-01 |

[Table 115]

| Mixture No. | mixAJ--B2A--01 | | |
|---|---|---|---|
| m/z | Retention time of MS peak (min) | Retention time of UV peak (min) | Attributed compound No. |
| 647.306 | 8.303 | 8.26 | B2A052-01 |
| 695.382 | 11.333 | 11.3 | B2A037-01, B2A087-01 |
| 662.281 | 13.107 | 13.06 | B2A005-01 |
| 669.285 | 12.713 | 12.68 | B2A009-01 |
| 776.309 | 14.293 | 14.25 | B2A043-01 |
| 778.299 | 14.103 | 14.06 | B2A021-01 |
| 694.306 | 13.833 | 13.8 | B2A013-01 |
| 712.278 | 13.853 | 13.8 | B2A017-01, B2A063-01 |
| 692.315 | 14.05 | 14.01 | B2A035-01 |
| 710.287 | 14.053 | 14.01 | B2A039-01 |
| 696.308 | 10.823 | 10.8 | B2A061-01 |
| 630.311 | 9.62 | 9.58 | B2A071-01 |
| 634.277 | 9.627 | 9.58 | B2A067-01 |
| 688.341 | 9.623 | 9.58 | B2A059-01, B2A083-01 |
| 650.247 | 13.01 | 12.96 | B2A001-01 |
| 667.295 | 12.997 | 12.96 | B2A031-01 |
| 638.267 | 9.873 | 9.81 | B2A093-01 |
| 648.256 | 9.847 | 9.81 | B2A023-01, B2A077-01 |
| 649.251 | 9.86 | 9.81 | B2A024-01, B2A078-01 |
| 689.337 | 8.733 | 8.69 | B2A060-01, B2A084-01 |
| 654.311 | 10.337 | 10.3 | B2A035-01 |
| 668.29 | 11.513 | 11.44 | B2A032-01 |
| 714.293 | 11.483 | 11.44 | B2A089-01 |
| 628.32 | 9.49 | 9.44 | B2A045-01 |
| 698.382 | 14.7 | 14.66 | B2A037-01, B2A087-01 |
| 734.287 | 14.697 | 14.66 | B2A041-01 |
| 670.281 | 13.703 | 13.66 | B2A100-01, B2A033-01 |
| 711.282 | 12.367 | 12.34 | B2A040-01 |
| 777.304 | 12.367 | 12.34 | B2A044-01 |
| 693.311 | 11.777 | 11.74 | B2A036-01 |
| 701.348 | 12.313 | 12.27 | B2A016-01 |
| 656.315 | 13.647 | 13.6 | B2A025-01, B2A049-01 |
| 700.353 | 14.517 | 14.48 | B2A015-01 |
| 736.278 | 14.52 | 14.48 | B2A019-01 |
| 652.32 | 10.057 | 10.02 | B2A069-01 |
| 713.273 | 12.06 | 12.02 | B2A018-01, B2A064-01 |
| 779.295 | 12.063 | 12.02 | B2A022-01 |
| 659.301 | 10.67 | 10.63 | B2A004-01, B2A054-01, B2A076-01 |
| 658.306 | 13.383 | 13.33 | B2A003-01, B2A053-01, B2A075-01 |
| 659.301 | 13.387 | 13.33 | B2A004-01, B2A054-01, B2A076-01 |
| 660.29 | 13.36 | 13.33 | B2A027-01, B2A079-01, B2A099-01 |
| 675.336 | 10.563 | 10.53 | B2A065-01 |
| 672.321 | 13.503 | 13.47 | B2A011-01, B2A055-01 |
| 651.242 | 10.53 | 10.49 | B2A002-01 |
| 659.301 | 8.603 | 8.57 | B2A004-01, B2A054-01, B2A076-01 |
| 686.362 | 10.103 | 10.07 | B2A057-01 |
| 631.306 | 8.45 | 8.41 | B2A072-01 |
| 675.332 | 8.43 | 8.41 | B2A082-01 |
| 735.282 | 13.473 | 13.43 | B2A042-01 |
| 673.317 | 10.9 | 10.86 | B2A012-01, B2A056-01 |
| 642.336 | 7.79 | 7.77 | B2A047-01, B2A097-01 |
| 661.285 | 7.807 | 7.77 | B2A100-01, B2A028-01, B2A080-01 |
| 637.263 | 10.957 | 10.93 | B2A094-01 |
| 649.251 | 10.957 | 10.93 | B2A024-01, B2A078-01 |
| 680.327 | 10.96 | 10.93 | B2A091-01 |
| 712.278 | 10.96 | 10.93 | B2A017-01, B2A063-01 |
| 699.357 | 12.62 | 12.59 | B2A038-01, B2A088-01 |
| 737.273 | 13.21 | 13.17 | B2A020-01 |
| 658.306 | 10.487 | 10.45 | B2A003-01, B2A053-01, B2A075-01 |
| 660.29 | 9.263 | 9.23 | B2A027-01, B2A079-01, B2A099-01 |
| 661.285 | 9.22 | 9.23 | B2A100-01, B2A028-01, B2A080-01 |
| 648.256 | 13.293 | 13.25 | B2A023-01, B2A077-01 |
| 635.272 | 7.977 | 7.94 | B2A068-01 |
| 643.331 | 7.957 | 7.94 | B2A048-01, B2A098-01 |
| 643.331 | 7.923 | 7.94 | B2A048-01, B2A098-01 |
| 679.331 | 8.89 | 8.86 | B2A066-01 |
| 661.285 | 11.427 | 11.39 | B2A100-01, B2A028-01, B2A080-01 |
| 695.301 | 11.42 | 11.39 | B2A014-01 |
| 681.322 | 9.05 | 9.01 | B2A092-01 |
| 658.306 | 9.397 | 9.36 | B2A003-01, B2A053-01, B2A075-01 |
| 697.303 | 9.383 | 9.36 | B2A062-01 |
| 657.311 | 11.077 | 11.04 | B2A026-01, B2A050-01 |
| 663.276 | 11.047 | 11.04 | B2A006-01 |
| 657.311 | 8.563 | 8.52 | B2A026-01, B2A050-01 |
| 642.336 | 9.55 | 9.5 | B2A047-01, B2A097-01 |
| 644.327 | 9.563 | 9.5 | B2A073-01 |
| 645.322 | 9.533 | 9.5 | B2A074-01 |
| 713.273 | 9.53 | 9.5 | B2A018-01, B2A064-01 |
| 671.326 | 11.263 | 11.23 | B2A034-01 |
| 655.306 | 9.19 | 9.16 | B2A096-01 |
| 656.315 | 10.217 | 10.16 | B2A025-01, B2A049-01 |
| 688.341 | 10.19 | 10.16 | B2A059-01, B2A083-01 |
| 689.337 | 10.203 | 10.16 | B2A060-01, B2A084-01 |
| 687.357 | 8.79 | 8.75 | B2A058-01 |
| 629.316 | 8.38 | 8.34 | B2A046-01 |
| 690.332 | 9.45 | 9.41 | B2A085-01 |
| 691.327 | 9.463 | 9.41 | B2A086-01 |
| 673.317 | 8.513 | 8.47 | B2A012-01, B2A056-01 |
| 670.281 | 11.157 | 11.12 | B2A100-01, B2A033-01 |
| 660.29 | 9.9 | 9.85 | B2A027-01, B2A079-01, B2A099-01 |
| 674.327 | 9.893 | 9.85 | B2A081-01 |
| 646.311 | 9.67 | 9.63 | B2A051-01 |
| 699.357 | 9.66 | 9.63 | B2A038-01, B2A088-01 |
| 653.316 | 9.09 | 9.05 | B2A070-01 |
| 672.321 | 9.84 | 9.77 | B2A011-01, B2A055-01 |
| 715.288 | 9.827 | 9.77 | B2A090-01 |

[Table 116]

| Mixture No. | mixA1-B21-01 | | |
|---|---|---|---|
| m/z | Retention time of MS peak (min) | Retention time of UV peak (min) | Attributed compound No. |
| 578.168 | 10.283 | 10.24 | B21017-01, B21063-01 |
| 643.194 | 8.707 | 8.67 | B21044-01 |
| 539.207 | 6.45 | 6.43 | B21012-01, B21056-01 |
| 494.211 | 6.56 | 6.52 | B21045-01 |
| 514.146 | 6.55 | 6.52 | B21023-01, B21077-01 |
| 510.217 | 6.663 | 6.63 | B21073-01 |
| 524.196 | 9.403 | 9.35 | B21003-01, B21053-01, B21075-01 |
| 533.185 | 9.383 | 9.35 | B21031-01 |
| 535.176 | 8.773 | 8.74 | B21009-01 |
| 578.168 | 8.08 | 8.04 | B21017-01, B21063-01 |
| 545.185 | 8.043 | 8 | B21022-01 |
| 495.206 | 9.223 | 9.14 | B21046-01 |
| 528.171 | 9.177 | 9.14 | B21005-01 |
| 582.198 | 7.9 | 7.87 | B21061-01 |
| 560.196 | 9.997 | 9.96 | B21013-01 |
| 500.167 | 10.833 | 10.78 | B21067-01 |
| 576.177 | 10.83 | 10.78 | B21039-01 |
| 644.19 | 10.82 | 10.78 | B21021-01 |
| 581.179 | 6.163 | 6.13 | B21090-01 |
| 496.201 | 6.243 | 6.21 | B21071-01 |
| 563.193 | 6.23 | 6.21 | B21062-01 |
| 539.207 | 9.527 | 9.49 | B21012-01, B21056-01 |
| 497.197 | 7.76 | 7.73 | B21072-01 |
| 564.253 | 7.767 | 7.73 | B21037-01, B21087-01 |
| 558.206 | 10.583 | 10.54 | B21035-01 |
| 547.212 | 11.293 | 11.25 | B21092-01 |
| 602.168 | 11.263 | 11.25 | B21019-01 |
| 642.199 | 11.297 | 11.25 | B21043-01 |
| 567.238 | 7.99 | 7.92 | B21016-01 |
| 580.184 | 7.947 | 7.92 | B21089-01 |
| 559.201 | 7.623 | 7.58 | B21036-01 |
| 512.201 | 6.897 | 6.86 | B21051-01 |
| 556.222 | 6.907 | 6.86 | B21085-01 |
| 557.218 | 6.91 | 6.86 | B21086-01 |
| 577.173 | 8.553 | 8.52 | B21040-01 |
| 516.137 | 8.963 | 8.92 | B21001-01 |
| 523.201 | 11.767 | 11.73 | B21026-01, B21050-01 |
| 600.177 | 11.773 | 11.73 | B21041-01 |
| 523.201 | 6.803 | 6.75 | B21026-01, B21050-01 |
| 553.248 | 5.95 | 5.92 | B21058-01 |
| 565.249 | 5.967 | 5.92 | B21038-01, B21088-01 |
| 534.181 | 7.583 | 7.53 | B21032-01 |
| 565.248 | 8.673 | 8.59 | B21038-01, B21088-01 |
| 520.201 | 7.073 | 7.04 | B21095-01 |
| 540.228 | 7.083 | 7.04 | B21081-01 |
| 508.226 | 7.033 | 6.99 | B21047-01, B21097-01 |
| 524.196 | 6.97 | 6.93 | B21003-01, B21053-01, B21075-01 |
| 502.158 | 7.243 | 7.24 | B21093-01 |
| 515.142 | 7.257 | 7.24 | B21024-01, B21078-01 |
| 518.211 | 7.277 | 7.24 | B21069-01 |
| 554.232 | 7.283 | 7.24 | B21059-01, B21083-01 |
| 538.212 | 7.393 | 7.35 | B21011-01, B21055-01 |
| 541.223 | 5.193 | 5.16 | B21082-01 |
| 526.181 | 10.94 | 10.92 | B21027-01, B21079-01, B21099-01 |
| 566.243 | 10.95 | 10.92 | B21015-01 |
| 515.142 | 7.233 | 7.17 | B21024-01, B21078-01 |
| 524.196 | 7.207 | 7.17 | B21003-01, B21053-01, B21075-01 |
| 509.222 | 4.293 | 4.3 | B21048-01, B21098-01 |
| 564.253 | 11.473 | 11.44 | B21037-01, B21087-01 |
| 522.206 | 10.05 | 10.03 | B21025-01, B21049-01 |
| 527.176 | 10.047 | 10.03 | B21100-01, B21028-01, B21080-01 |
| 536.171 | 10.053 | 10.03 | B21100-01, B21033-01 |
| 536.171 | 10.077 | 10.03 | B21100-01, B21033-01 |
| 601.173 | 10.073 | 10.03 | B21042-01 |
| 525.191 | 9.813 | 9.79 | B21004-01, B21054-01, B21076-01 |
| 525.191 | 9.84 | 9.79 | B21004-01, B21054-01, B21076-01 |
| 526.181 | 9.83 | 9.79 | B21027-01, B21079-01, B21099-01 |
| 526.181 | 9.853 | 9.79 | B21027-01, B21079-01, B21099-01 |
| 527.176 | 9.803 | 9.79 | B21100-01, B21028-01, B21080-01 |
| 579.163 | 8.417 | 8.34 | B21018-01, B21064-01 |
| 513.197 | 9.477 | 9.44 | B21052-01 |
| 538.212 | 9.51 | 9.44 | B21011-01, B21055-01 |
| 603.163 | 9.48 | 9.44 | B21020-01 |
| 554.232 | 7.417 | 7.38 | B21059-01, B21083-01 |
| 555.227 | 7.427 | 7.38 | B21060-01, B21084-01 |
| 501.162 | 4.503 | 4.47 | B21068-01 |
| 579.163 | 7.847 | 7.81 | B21018-01, B21064-01 |
| 544.228 | 7.683 | 7.66 | B21065-01 |
| 545.222 | 7.7 | 7.66 | B21066-01 |
| 552.253 | 7.707 | 7.66 | B21057-01 |
| 514.146 | 9.65 | 9.61 | B21023-01, B21077-01 |
| 529.166 | 9.647 | 9.61 | B21006-01 |
| 503.153 | 8.893 | 8.86 | B21094-01 |
| 517.132 | 7.47 | 7.44 | B21002-01 |
| 519.206 | 7.49 | 7.44 | B21070-01 |
| 521.197 | 7.473 | 7.44 | B21096-01 |
| 522.206 | 7.493 | 7.44 | B21025-01, B21049-01 |
| 546.217 | 7.473 | 7.44 | B21091-01 |
| 527.176 | 7.197 | 7.13 | B21100-01, B21028-01, B21080-01 |
| 537.217 | 7.167 | 7.13 | B21034-01 |
| 509.222 | 4.033 | 4.1 | B21048-01, B21098-01 |
| 511.212 | 10.663 | 10.62 | B21074-01 |
| 555.227 | 5.387 | 5.35 | B21060-01, B21084-01 |
| 581.191 | 6.867 | 6.82 | B21014-01 |
| 525.191 | 5.987 | 5.95 | B21004-01, B21054-01, B21076-01 |

[Table 117]

| Mixture No. | mixA1-B2L-01 | | |
|---|---|---|---|
| m/z | Retention time of MS peak (min) | Retention time of UV peak (min) | Attributed compound No. |
| 577.223 | 9.913 | 9.87 | B2L009-01 |
| 570.218 | 10.347 | 10.31 | B2L005-01 |
| 622.231 | 8.887 | 8.85 | B2L089-01 |
| 686.237 | 11.807 | 11.77 | B2L021-01 |
| 606.3 | 8.713 | 8.68 | B2L037-01, B2L087-01 |
| 607.295 | 6.94 | 6.91 | B2L038-01, B2L088-01 |
| 562.248 | 7.94 | 7.9 | B2L095-01 |
| 575.232 | 10.247 | 10.21 | B2L031-01 |
| 642.224 | 12.527 | 12.49 | B2L041-01 |
| 687.232 | 9.203 | 9.16 | B2L022-01 |
| 588.264 | 8.39 | 8.35 | B2L091-01 |
| 618.224 | 11.63 | 11.59 | B2L039-01 |
| 538.248 | 7.163 | 7.13 | B2L071-01 |
| 559.179 | 7.153 | 7.13 | B2L002-01 |
| 623.226 | 7.15 | 7.13 | B2L090-01 |
| 602.243 | 11.173 | 11.13 | B2L013-01 |
| 589.259 | 6.277 | 6.24 | B2L092-01 |
| 568.228 | 10.697 | 10.67 | B2L027-01, B2L079-01, B2L099-01 |
| 580.259 | 10.717 | 10.67 | B2L011-01, B2L055-01 |
| 595.295 | 11.37 | 11.33 | B2L058-01 |
| 620.215 | 11.373 | 11.33 | B2L017-01, B2L063-01 |
| 609.285 | 9.23 | 9.19 | B2L016-01 |
| 551.269 | 7.413 | 7.4 | B2L048-01, B2L098-01 |
| 552.264 | 7.42 | 7.4 | B2L073-01 |
| 556.193 | 7.437 | 7.4 | B2L023-01, B2L077-01 |
| 608.29 | 12.07 | 12.01 | B2L015-01 |
| 684.246 | 12.047 | 12.01 | B2L043-01 |
| 606.3 | 12.317 | 12.24 | B2L037-01, B2L087-01 |
| 644.215 | 12.287 | 12.24 | B2L019-01 |
| 607.295 | 9.607 | 9.54 | B2L038-01, B2L088-01 |
| 635.241 | 9.563 | 9.54 | B2L044-01 |
| 539.244 | 5.697 | 5.66 | B2L072-01 |
| 600.253 | 11.463 | 11.42 | B2L035-01 |
| 563.244 | 6.75 | 6.69 | B2L096-01 |
| 621.21 | 9.093 | 9.05 | B2L018-01, B2L064-01 |
| 553.259 | 5.27 | 5.25 | B2L074-01 |
| 557.189 | 5.603 | 5.56 | B2L024-01, B2L078-01 |
| 603.238 | 8.187 | 8.15 | B2L014-01 |
| 579.264 | 8.127 | 8.09 | B2L034-01 |
| 596.279 | 8.12 | 8.09 | B2L059-01, B2L083-01 |
| 571.213 | 7.733 | 7.7 | B2L006-01 |
| 556.193 | 10.547 | 10.56 | B2L023-01, B2L077-01 |
| 566.243 | 10.603 | 10.56 | B2L003-01, B2L053-01, B2L075-01 |
| 645.21 | 10.587 | 10.56 | B2L020-01 |
| 536.258 | 7.24 | 7.2 | B2L045-01 |
| 544.205 | 7.27 | 7.2 | B2L093-01 |
| 537.253 | 5.807 | 5.75 | B2L046-01 |
| 601.248 | 8.613 | 8.58 | B2L036-01 |
| 564.253 | 10.953 | 10.92 | B2L025-01, B2L049-01 |
| 578.218 | 10.963 | 10.92 | B2L100-01, B2L033-01 |
| 578.218 | 10.973 | 10.92 | B2L100-01, B2L033-01 |
| 581.254 | 6.23 | 6.13 | B2L012-01, B2L056-01 |
| 542.214 | 7.297 | 7.27 | B2L067-01 |
| 567.238 | 7.31 | 7.27 | B2L004-01, B2L054-01, B2L076-01 |
| 569.223 | 7.303 | 7.27 | B2L100-01, B2L028-01, B2L080-01 |
| 569.223 | 6.233 | 6.18 | B2L100-01, B2L028-01, B2L080-01 |
| 594.3 | 8.22 | 8.18 | B2L057-01 |
| 557.189 | 7.697 | 7.65 | B2L024-01, B2L078-01 |
| 581.254 | 7.693 | 7.65 | B2L012-01, B2L056-01 |
| 567.238 | 5.913 | 5.88 | B2L004-01, B2L054-01, B2L076-01 |
| 545.2 | 5.15 | 5.12 | B2L094-01 |
| 576.228 | 8.517 | 8.48 | B2L032-01 |
| 604.245 | 8.52 | 8.48 | B2L061-01 |
| 586.273 | 8.293 | 8.26 | B2L065-01 |
| 583.27 | 6.03 | 6.01 | B2L082-01 |
| 597.274 | 6.047 | 6.01 | B2L060-01, B2L084-01 |
| 619.22 | 9.473 | 9.43 | B2L040-01 |
| 564.253 | 8.067 | 8.03 | B2L025-01, B2L049-01 |
| 567.238 | 8.06 | 8.03 | B2L004-01, B2L054-01, B2L076-01 |
| 568.228 | 8.073 | 8.03 | B2L027-01, B2L079-01, B2L099-01 |
| 569.223 | 5.383 | 5.34 | B2L100-01, B2L028-01, B2L080-01 |
| 587.269 | 6.36 | 6.36 | B2L066-01 |
| 597.274 | 6.39 | 6.36 | B2L060-01, B2L084-01 |
| 543.209 | 10.21 | 10.14 | B2L068-01 |
| 558.184 | 10.19 | 10.14 | B2L001-01 |
| 565.248 | 7.817 | 7.77 | B2L026-01, B2L050-01 |
| 566.243 | 7.817 | 7.77 | B2L003-01, B2L053-01, B2L075-01 |
| 582.274 | 7.8 | 7.77 | B2L081-01 |
| 596.279 | 7.81 | 7.77 | B2L059-01, B2L083-01 |
| 605.24 | 8.7 | 8.64 | B2L062-01 |
| 620.215 | 8.68 | 8.64 | B2L017-01, B2L063-01 |
| 560.258 | 7.89 | 7.85 | B2L069-01 |
| 580.259 | 7.923 | 7.85 | B2L011-01, B2L055-01 |
| 550.273 | 7.58 | 7.55 | B2L047-01, B2L097-01 |
| 598.269 | 7.597 | 7.55 | B2L085-01 |
| 599.265 | 7.597 | 7.55 | B2L086-01 |
| 554.248 | 7.493 | 7.47 | B2L051-01 |
| 555.244 | 7.503 | 7.47 | B2L052-01 |
| 566.243 | 7.51 | 7.47 | B2L003-01, B2L053-01, B2L075-01 |
| 568.228 | 7.513 | 7.47 | B2L027-01, B2L079-01, B2L099-01 |
| 561.253 | 6.817 | 6.77 | B2L070-01 |
| 565.248 | 10.933 | 10.86 | B2L026-01, B2L050-01 |
| 643.22 | 10.9 | 10.86 | B2L042-01 |
| 621.21 | 7.127 | 7.05 | B2L018-01, B2L064-01 |
| 550.273 | 11.953 | 11.91 | B2L047-01, B2L097-01 |
| 551.269 | 11.96 | 11.91 | B2L048-01, B2L098-01 |

[Table 118]

| Mixture No. | mixAJ-B2N-01 | | |
|---|---|---|---|
| m/z | Retention time of MS peak (min) | Retention time of UV peak (min) | Attributed compound No. |
| 576.214 | 7.78 | 7.73 | B2N045-01 |
| 724.202 | 12.373 | 12.34 | B2N043-01 |
| 634.256 | 8.673 | 8.64 | B2N057-01 |
| 682.18 | 12.813 | 12.78 | B2N041-01 |
| 658.18 | 12.01 | 11.97 | B2N039-01 |
| 607.194 | 6.177 | 6.14 | B2N004-01, B2N054-01, B2N076-01 |
| 608.184 | 11.17 | 11.13 | B2N027-01, B2N079-01, B2N099-01 |
| 609.179 | 6.48 | 6.45 | B2N100-01, B2N028-01, B2N080-01 |
| 644.201 | 9.057 | 9.01 | B2N061-01 |
| 591.225 | 6.07 | 6.02 | B2N048-01, B2N098-01 |
| 635.251 | 7.127 | 7.09 | B2N058-01 |
| 593.215 | 8.077 | 8.03 | B2N074-01 |
| 617.179 | 10.133 | 10.09 | B2N009-01 |
| 725.198 | 10.12 | 10.09 | B2N044-01 |
| 597.145 | 8.41 | 8.35 | B2N024-01, B2N078-01 |
| 600.214 | 8.383 | 8.35 | B2N069-01 |
| 620.215 | 8.39 | 8.35 | B2N011-01, B2N055-01 |
| 636.235 | 8.413 | 8.35 | B2N059-01, B2N083-01 |
| 641.204 | 9.27 | 9.23 | B2N038-01 |
| 621.21 | 8.01 | 7.95 | B2N012-01, B2N056-01 |
| 605.204 | 8.53 | 8.49 | B2N026-01, B2N050-01 |
| 604.209 | 8.573 | 8.54 | B2N025-01, B2N049-01 |
| 604.209 | 11.407 | 11.36 | B2N025-01, B2N049-01 |
| 627.225 | 6.977 | 6.93 | B2N066-01 |
| 619.22 | 8.813 | 8.78 | B2N034-01 |
| 626.229 | 8.823 | 8.78 | B2N065-01 |
| 606.199 | 10.757 | 10.72 | B2N003-01, B2N053-01, B2N075-01 |
| 615.188 | 10.76 | 10.72 | B2N031-01 |
| 685.166 | 10.753 | 10.72 | B2N020-01 |
| 660.171 | 11.477 | 11.44 | B2N017-01, B2N063-01 |
| 601.209 | 7.33 | 7.29 | B2N070-01 |
| 578.204 | 7.487 | 7.45 | B2N071-01 |
| 577.209 | 6.38 | 6.31 | B2N046-01 |
| 595.2 | 6.35 | 6.31 | B2N052-01 |
| 645.196 | 7.54 | 7.49 | B2N062-01 |
| 621.21 | 6.79 | 6.75 | B2N012-01, B2N056-01 |
| 683.176 | 11.347 | 11.31 | B2N042-01 |
| 590.229 | 8.06 | 7.99 | B2N047-01, B2N097-01 |
| 594.204 | 8.023 | 7.99 | B2N051-01 |
| 611.169 | 8.047 | 7.99 | B2N006-01 |
| 609.179 | 8.933 | 8.89 | B2N100-01, B2N028-01, B2N080-01 |
| 585.156 | 7.603 | 7.57 | B2N094-01 |
| 607.194 | 7.61 | 7.57 | B2N004-01, B2N054-01, B2N076-01 |
| 639.221 | 11.867 | 11.85 | B2N086-01 |
| 726.193 | 11.883 | 11.85 | B2N021-01 |
| 727.188 | 11.89 | 11.85 | B2N022-01 |
| 598.14 | 10.373 | 10.33 | B2N001-01 |
| 602.204 | 8.233 | 8.21 | B2N095-01 |
| 603.2 | 8.247 | 8.21 | B2N096-01 |
| 608.184 | 8.227 | 8.21 | B2N027-01, B2N079-01, B2N099-01 |
| 636.235 | 8.243 | 8.21 | B2N059-01, B2N083-01 |
| 629.215 | 6.537 | 6.51 | B2N092-01 |
| 637.23 | 6.533 | 6.51 | B2N060-01, B2N084-01 |
| 620.215 | 10.873 | 10.83 | B2N011-01, B2N055-01 |
| 646.256 | 12.843 | 12.81 | B2N037-01, B2N087-01 |
| 661.166 | 9.36 | 9.32 | B2N018-01, B2N064-01 |
| 583.166 | 5.883 | 5.85 | B2N068-01 |
| 597.145 | 5.897 | 5.85 | B2N024-01, B2N078-01 |
| 582.17 | 7.853 | 7.82 | B2N067-01 |
| 638.225 | 7.87 | 7.82 | B2N085-01 |
| 599.135 | 7.19 | 7.17 | B2N002-01 |
| 642.199 | 11.28 | 11.25 | B2N013-01 |
| 618.174 | 11.467 | 11.4 | B2N100-01, B2N033-01 |
| 618.174 | 11.437 | 11.4 | B2N100-01, B2N033-01 |
| 579.2 | 6 | 5.96 | B2N072-01 |
| 646.256 | 9.023 | 8.97 | B2N037-01, B2N087-01 |
| 647.251 | 9.037 | 8.97 | B2N038-01, B2N088-01 |
| 592.22 | 7.73 | 7.69 | B2N073-01 |
| 596.15 | 7.76 | 7.69 | B2N023-01, B2N077-01 |
| 661.166 | 7.717 | 7.69 | B2N018-01, B2N064-01 |
| 647.251 | 10.18 | 10.15 | B2N038-01, B2N088-01 |
| 648.246 | 10.193 | 10.15 | B2N015-01 |
| 591.225 | 11.047 | 11 | B2N048-01, B2N098-01 |
| 596.15 | 11.043 | 11 | B2N023-01, B2N077-01 |
| 637.23 | 10.947 | 10.89 | B2N060-01, B2N084-01 |
| 623.228 | 6.313 | 6.26 | B2N082-01 |
| 590.229 | 7.95 | 7.92 | B2N047-01, B2N097-01 |
| 605.204 | 7.963 | 7.92 | B2N026-01, B2N050-01 |
| 606.199 | 7.977 | 7.92 | B2N003-01, B2N053-01, B2N075-01 |
| 607.194 | 7.957 | 7.92 | B2N004-01, B2N054-01, B2N076-01 |
| 643.194 | 8.46 | 8.42 | B2N014-01 |
| 628.22 | 8.667 | 8.6 | B2N091-01 |
| 622.231 | 8.097 | 8.06 | B2N081-01 |
| 606.199 | 8.35 | 8.28 | B2N003-01, B2N053-01, B2N075-01 |
| 584.161 | 7.59 | 7.53 | B2N093-01 |
| 608.184 | 7.597 | 7.53 | B2N027-01, B2N079-01, B2N099-01 |
| 609.179 | 7.593 | 7.53 | B2N100-01, B2N028-01, B2N080-01 |
| 640.209 | 11.863 | 11.78 | B2N035-01 |
| 649.241 | 9.477 | 9.43 | B2N016-01 |
| 659.176 | 10.053 | 10.01 | B2N040-01 |
| 660.171 | 9.193 | 9.15 | B2N017-01, B2N063-01 |
| 684.171 | 12.33 | 12.28 | B2N019-01 |
| 610.174 | 10.527 | 10.48 | B2N005-01 |
| 616.184 | 9.16 | 9.13 | B2N032-01 |
| 662.187 | 9.183 | 9.12 | B2N029-01 |
| 663.182 | 9.177 | 9.13 | B2N090-01 |

[Table 119]

| Mixture No. | mixAI−B2R−01 | | |
|---|---|---|---|
| m/z | Retention time of MS peak (min) | Retention time of UV peak (min) | Attributed compound No. |
| 814.234 | 12.897 | 12.87 | B2R031-01 |
| 857.228 | 13.81 | 13.76 | B2R039-01 |
| 775.259 | 9.847 | 9.81 | B2R045-01 |
| 816.224 | 12.68 | 12.65 | B2R009-01 |
| 784.202 | 8.677 | 8.64 | B2R094-01 |
| 923.248 | 14.02 | 13.98 | B2R043-01 |
| 841.245 | 9.233 | 9.2 | B2R013-01 |
| 817.22 | 13.497 | 13.44 | B2R100-01, B2R033-01 |
| 803.254 | 13.403 | 13.38 | B2R025-01, B2R049-01 |
| 881.226 | 14.387 | 14.35 | B2R041-01 |
| 859.217 | 13.63 | 13.59 | B2R017-01, B2R063-01 |
| 840.25 | 11.85 | 11.81 | B2R036-01 |
| 839.254 | 13.77 | 13.73 | B2R035-01 |
| 884.212 | 13.103 | 13.09 | B2R020-01 |
| 846.297 | 12.55 | 12.52 | B2R038-01, B2R088-01 |
| 800.255 | 9.48 | 9.43 | B2R070-01 |
| 776.255 | 8.947 | 8.89 | B2R046-01 |
| 858.221 | 12.367 | 12.33 | B2R040-01 |
| 924.243 | 12.377 | 12.33 | B2R044-01 |
| 860.212 | 12.14 | 12.1 | B2R018-01, B2R064-01 |
| 926.234 | 12.137 | 12.1 | B2R022-01 |
| 925.238 | 13.863 | 13.82 | B2R021-01 |
| 825.275 | 10.807 | 10.77 | B2R065-01 |
| 848.287 | 12.313 | 12.28 | B2R016-01 |
| 827.266 | 11.157 | 11.1 | B2R091-01 |
| 828.261 | 11.14 | 11.1 | B2R092-01 |
| 859.217 | 11.133 | 11.1 | B2R017-01, B2R063-01 |
| 819.26 | 10.157 | 10.12 | B2R011-01, B2R055-01 |
| 838.266 | 8.807 | 8.77 | B2R086-01 |
| 807.229 | 9.667 | 9.62 | B2R027-01, B2R079-01, B2R099-01 |
| 808.224 | 9.67 | 9.62 | B2R100-01, B2R028-01, B2R080-01 |
| 799.259 | 10.277 | 10.24 | B2R069-01 |
| 782.211 | 8.62 | 8.51 | B2R068-01 |
| 790.27 | 8.557 | 8.51 | B2R048-01, B2R098-01 |
| 809.22 | 8.537 | 8.51 | B2R005-01 |
| 843.247 | 11.033 | 10.99 | B2R061-01 |
| 844.242 | 11.057 | 10.99 | B2R062-01 |
| 805.245 | 9.773 | 9.74 | B2R003-01, B2R053-01, B2R075-01 |
| 837.271 | 9.813 | 9.74 | B2R085-01 |
| 808.224 | 11.577 | 11.54 | B2R100-01, B2R028-01, B2R080-01 |
| 842.24 | 11.58 | 11.54 | B2R014-01 |
| 861.232 | 11.58 | 11.54 | B2R089-01 |
| 805.245 | 13.207 | 13.16 | B2R003-01, B2R053-01, B2R075-01 |
| 806.24 | 13.2 | 13.16 | B2R004-01, B2R054-01, B2R076-01 |
| 807.229 | 13.193 | 13.16 | B2R027-01, B2R079-01, B2R099-01 |
| 817.22 | 11.417 | 11.38 | B2R100-01, B2R033-01 |
| 805.245 | 10.737 | 10.7 | B2R003-01, B2R053-01, B2R075-01 |
| 798.181 | 10.89 | 10.85 | B2R002-01 |
| 783.206 | 10.217 | 10.17 | B2R093-01 |
| 795.195 | 10.203 | 10.17 | B2R023-01, B2R077-01 |
| 821.276 | 10.2 | 10.17 | B2R081-01 |
| 862.227 | 10.197 | 10.17 | B2R090-01 |
| 847.292 | 14.183 | 14.19 | B2R015-01 |
| 883.217 | 14.23 | 14.18 | B2R019-01 |
| 804.25 | 9.097 | 9.05 | B2R026-01, B2R050-01 |
| 819.26 | 13.303 | 13.27 | B2R011-01, B2R055-01 |
| 882.221 | 13.307 | 13.27 | B2R042-01 |
| 815.229 | 11.683 | 11.64 | B2R032-01 |
| 801.25 | 10.537 | 10.46 | B2R095-01 |
| 802.245 | 10.537 | 10.46 | B2R096-01 |
| 803.254 | 10.493 | 10.46 | B2R025-01, B2R049-01 |
| 835.281 | 10.467 | 10.46 | B2R059-01, B2R083-01 |
| 777.25 | 9.98 | 9.95 | B2R071-01 |
| 781.216 | 9.997 | 9.95 | B2R067-01 |
| 793.25 | 10.023 | 9.95 | B2R051-01 |
| 835.281 | 9.96 | 9.95 | B2R059-01, B2R083-01 |
| 804.25 | 11.29 | 11.25 | B2R026-01, B2R050-01 |
| 806.24 | 11.283 | 11.25 | B2R004-01, B2R054-01, B2R076-01 |
| 810.215 | 11.307 | 11.25 | B2R006-01 |
| 790.27 | 8.323 | 8.28 | B2R048-01, B2R098-01 |
| 791.266 | 8.32 | 8.28 | B2R073-01 |
| 818.265 | 11.473 | 11.43 | B2R034-01 |
| 845.301 | 11.48 | 11.43 | B2R037-01, B2R087-01 |
| 845.301 | 11.463 | 11.43 | B2R037-01, B2R087-01 |
| 846.297 | 11.457 | 11.43 | B2R038-01, B2R088-01 |
| 797.186 | 8.407 | 8.4 | B2R001-01 |
| 808.224 | 8.453 | 8.4 | B2R100-01, B2R028-01, B2R080-01 |
| 778.245 | 9.05 | 8.98 | B2R072-01 |
| 820.256 | 9.02 | 8.98 | B2R012-01, B2R056-01 |
| 822.271 | 8.997 | 8.98 | B2R082-01 |
| 789.275 | 9.907 | 9.87 | B2R047-01, B2R097-01 |
| 789.275 | 9.933 | 9.87 | B2R047-01, B2R097-01 |
| 792.261 | 9.91 | 9.87 | B2R074-01 |
| 836.276 | 9.94 | 9.87 | B2R060-01, B2R084-01 |
| 860.212 | 9.903 | 9.87 | B2R018-01, B2R064-01 |
| 806.24 | 10.893 | 10.84 | B2R004-01, B2R054-01, B2R076-01 |
| 807.229 | 10.987 | 10.94 | B2R027-01, B2R079-01, B2R099-01 |
| 796.19 | 11.21 | 11.16 | B2R024-01, B2R078-01 |
| 796.19 | 11.193 | 11.16 | B2R024-01, B2R078-01 |
| 820.256 | 11.197 | 11.16 | B2R012-01, B2R056-01 |
| 826.27 | 9.387 | 9.35 | B2R066-01 |
| 836.276 | 9.393 | 9.35 | B2R060-01, B2R084-01 |
| 833.301 | 10.393 | 10.35 | B2R057-01 |
| 834.297 | 10.393 | 10.35 | B2R058-01 |
| 794.245 | 8.89 | 8.84 | B2R052-01 |
| 795.195 | 8.887 | 8.84 | B2R023-01, B2R077-01 |

[Table AI-03-02] shows the retention times of the UV and MS peaks to which each compound was attributed, and the value of m/z $(M+H)^+$ detected during that time period. Since two compounds in Example 6-2-2 and two compounds in Example 6-2-3 may be missing, 48 compounds derived from those four compounds in total may also be missing. Therefore, as shown in "Table AI-03-02", the m/z $(M+H)^+$ peaks corresponding to 1152 compounds among the 1200

compounds set as library compounds were confirmed.

From the above results, it was shown that, under the reaction conditions shown in the present Example, by carrying out highly selective ethylation of arenol having various reactive functional groups and by performing post-treatment that does not require column purification, it is possible to construct a compound library of high quality and diversity in functional groups, in which the number of compounds is greater than 1000 and 95% or more of them can be detected by the retention times and mass-to-charge ratios (m/z) of LC-MS.

Example 6-6: Production example 2 of compound library having diversity in functional groups using O-selective ethylation method for arenol

In Examples 6-1 to 6-5, one example of application to synthesis of a compound library having diversity in functional groups was shown, in which highly selective ethylation to arenol in 12 mixtures with 100 mixed compounds was carried out and post-treatment that does not require purification operation with a column was performed. Example 6-6 shows an example of constructing a library by performing a highly selective ethylation reaction to arenol for a mixture containing 1000 compounds.

The "diversity of core blocks" and "diversity of linkers" included in the library performed in Example 6-6 are illustrated in Figure 2, and the compounds that may be included in a mixture 2-1-m1E01-1 separately prepared as a raw material are shown in [Table 6-6-2].

The notation method in the table will be described. In [Table 6-6-2], each compound that may be included in the mixture 2-1-m1E01-1 is shown by ID, and the combination of core blocks and linkers in the structure of the mixture 2-1-m1E01-1 is shown by symbol or chemical formula. The correspondence between symbols and specific structures is illustrated in Figure 2. In [Table 6-6-2], "" is represented by a chemical formula.

[Formula 116]

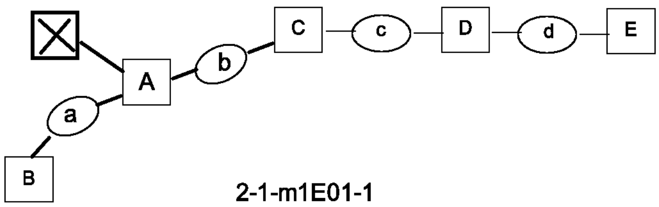


2-1-m1E01-1

For example, if the ID is 2-1-m1E01-1-0181, the following structural formula is shown in [Table 6-6-1].

[Formula 117]

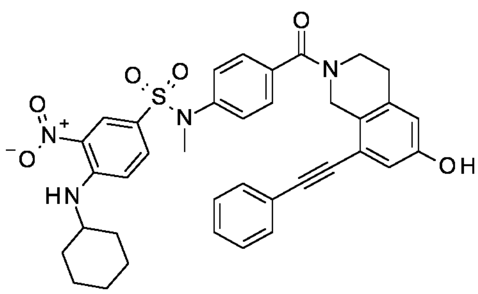

2-1-m1E01-1-0181

[Table 120]

[Table 6-6-1] Compound structure in ID notation (2-1-m1E01-1-0181)

| ID | | a | B | b | C | c | D | d | E |
|---|---|---|---|---|---|---|---|---|---|
| 2-1-m1E01-1-0181 | OH | a2 | B11 | b2 | C20 | c3 | D02 | d1 | E01 |

[Table 121]

[Table 6-6-2] Compounds that may be included in mixture 2-1-m1E01-1

| ID | Exact Mass | | a | B | b | C | c | D | d | E |
|---|---|---|---|---|---|---|---|---|---|---|
| 2-1-m1E01-1-0001 | 586.258 | OH | a2 | B11 | b1 | C08 | c1 | D01 | d1 | E01 |
| 2-1-m1E01-1-0002 | 587.253 | OH | a2 | B12 | b1 | C08 | c1 | D01 | d1 | E01 |
| 2-1-m1E01-1-0003 | 587.253 | OH | a2 | B11 | b1 | C11 | c1 | D01 | d1 | E01 |
| 2-1-m1E01-1-0004 | 588.249 | OH | a2 | B11 | b1 | C10 | c1 | D01 | d1 | E01 |
| 2-1-m1E01-1-0005 | 588.249 | OH | a2 | B12 | b1 | C11 | c1 | D01 | d1 | E01 |
| 2-1-m1E01-1-0006 | 589.244 | OH | a2 | B12 | b1 | C10 | c1 | D01 | d1 | E01 |
| 2-1-m1E01-1-0007 | 593.210 | OH | a2 | B11 | b1 | C12 | c1 | D01 | d1 | E01 |
| 2-1-m1E01-1-0008 | 594.205 | OH | a2 | B12 | b1 | C12 | c1 | D01 | d1 | E01 |
| 2-1-m1E01-1-0009 | 600.274 | OH | a2 | B13 | b1 | C08 | c1 | D01 | d1 | E01 |
| 2-1-m1E01-1-0010 | 601.269 | OH | a2 | B13 | b1 | C11 | c1 | D01 | d1 | E01 |
| 2-1-m1E01-1-0011 | 602.264 | OH | a2 | B13 | b1 | C10 | c1 | D01 | d1 | E01 |
| 2-1-m1E01-1-0012 | 604.232 | OH | a2 | B11 | b2 | C01 | c1 | D01 | d1 | E01 |
| 2-1-m1E01-1-0013 | 604.249 | OH | a2 | B14 | b1 | C08 | c1 | D01 | d1 | E01 |
| 2-1-m1E01-1-0014 | 605.227 | OH | a2 | B12 | b2 | C01 | c1 | D01 | d1 | E01 |
| 2-1-m1E01-1-0015 | 605.244 | OH | a2 | B14 | b1 | C11 | c1 | D01 | d1 | E01 |
| 2-1-m1E01-1-0016 | 605.264 | OH | a1 | B01 | b1 | C08 | c1 | D01 | d1 | E01 |
| 2-1-m1E01-1-0017 | 606.239 | OH | a2 | B14 | b1 | C10 | c1 | D01 | d1 | E01 |
| 2-1-m1E01-1-0018 | 606.259 | OH | a1 | B02 | b1 | C08 | c1 | D01 | d1 | E01 |
| 2-1-m1E01-1-0019 | 606.259 | OH | a1 | B01 | b1 | C11 | c1 | D01 | d1 | E01 |
| 2-1-m1E01-1-0020 | 607.225 | OH | a2 | B13 | b1 | C12 | c1 | D01 | d1 | E01 |
| 2-1-m1E01-1-0021 | 607.254 | OH | a1 | B01 | b1 | C10 | c1 | D01 | d1 | E01 |
| 2-1-m1E01-1-0022 | 607.254 | OH | a1 | B02 | b1 | C11 | c1 | D01 | d1 | E01 |
| 2-1-m1E01-1-0023 | 608.250 | OH | a1 | B02 | b1 | C10 | c1 | D01 | d1 | E01 |
| 2-1-m1E01-1-0024 | 611.200 | OH | a2 | B14 | b1 | C12 | c1 | D01 | d1 | E01 |
| 2-1-m1E01-1-0025 | 612.215 | OH | a1 | B01 | b1 | C12 | c1 | D01 | d1 | E01 |
| 2-1-m1E01-1-0026 | 613.211 | OH | a1 | B02 | b1 | C12 | c1 | D01 | d1 | E01 |
| 2-1-m1E01-1-0027 | 614.253 | OH | a2 | B11 | b2 | C02 | c1 | D01 | d1 | E01 |
| 2-1-m1E01-1-0028 | 614.289 | OH | a2 | B15 | b1 | C08 | c1 | D01 | d1 | E01 |
| 2-1-m1E01-1-0029 | 615.248 | OH | a2 | B12 | b2 | C02 | c1 | D01 | d1 | E01 |
| 2-1-m1E01-1-0030 | 615.248 | OH | a2 | B11 | b2 | C03 | c1 | D01 | d1 | E01 |
| 2-1-m1E01-1-0031 | 615.285 | OH | a2 | B15 | b1 | C11 | c1 | D01 | d1 | E01 |
| 2-1-m1E01-1-0032 | 616.243 | OH | a2 | B12 | b2 | C03 | c1 | D01 | d1 | E01 |
| 2-1-m1E01-1-0033 | 616.269 | OH | a2 | B16 | b1 | C08 | c1 | D01 | d1 | E01 |
| 2-1-m1E01-1-0034 | 616.280 | OH | a2 | B15 | b1 | C10 | c1 | D01 | d1 | E01 |
| 2-1-m1E01-1-0035 | 617.264 | OH | a2 | B16 | b1 | C11 | c1 | D01 | d1 | E01 |

(continued)

| ID | Exact Mass | | a | B | b | C | c | D | d | E |
|---|---|---|---|---|---|---|---|---|---|---|
| 2-1-m1E01-1-0036 | 618.248 | OH | a2 | B13 | b2 | C01 | c1 | D01 | d1 | E01 |
| 2-1-m1E01-1-0037 | 618.259 | OH | a2 | B16 | b1 | C10 | c1 | D01 | d1 | E01 |
| 2-1-m1E01-1-0038 | 619.279 | OH | a1 | B03 | b1 | C08 | c1 | D01 | d1 | E01 |
| 2-1-m1E01-1-0039 | 620.209 | OH | a2 | B11 | b2 | C06 | c1 | D01 | d1 | E01 |
| 2-1-m1E01-1-0040 | 620.275 | OH | a1 | B03 | b1 | C11 | c1 | D01 | d1 | E01 |
| 2-1-m1E01-1-0041 | 621.205 | OH | a2 | B12 | b2 | C06 | c1 | D01 | d1 | E01 |
| 2-1-m1E01-1-0042 | 621.241 | OH | a2 | B15 | b1 | C12 | c1 | D01 | d1 | E01 |
| 2-1-m1E01-1-0043 | 621.270 | OH | a1 | B03 | b1 | C10 | c1 | D01 | d1 | E01 |
| 2-1-m1E01-1-0044 | 622.223 | OH | a2 | B14 | b2 | C01 | c1 | D01 | d1 | E01 |
| 2-1-m1E01-1-0045 | 622.239 | OH | a2 | B17 | b1 | C08 | c1 | D01 | d1 | E01 |
| 2-1-m1E01-1-0046 | 623.220 | OH | a2 | B16 | b1 | C12 | c1 | D01 | d1 | E01 |
| 2-1-m1E01-1-0047 | 623.234 | OH | a2 | B17 | b1 | C11 | c1 | D01 | d1 | E01 |
| 2-1-m1E01-1-0048 | 623.238 | OH | a1 | B01 | b2 | C01 | c1 | D01 | d1 | E01 |
| 2-1-m1E01-1-0049 | 623.254 | OH | a1 | B04 | b1 | C08 | c1 | D01 | d1 | E01 |
| 2-1-m1E01-1-0050 | 624.230 | OH | a2 | B17 | b1 | C10 | c1 | D01 | d1 | E01 |
| 2-1-m1E01-1-0051 | 624.233 | OH | a1 | B02 | b2 | C01 | c1 | D01 | d1 | E01 |
| 2-1-m1E01-1-0052 | 624.250 | OH | a1 | B04 | b1 | C11 | c1 | D01 | d1 | E01 |
| 2-1-m1E01-1-0053 | 625.245 | OH | a1 | B04 | b1 | C10 | c1 | D01 | d1 | E01 |
| 2-1-m1E01-1-0054 | 626.231 | OH | a1 | B03 | b1 | C12 | c1 | D01 | d1 | E01 |
| 2-1-m1E01-1-0055 | 628.269 | OH | a2 | B13 | b2 | C02 | c1 | D01 | d1 | E01 |
| 2-1-m1E01-1-0056 | 629.191 | OH | a2 | B17 | b1 | C12 | c1 | D01 | d1 | E01 |
| 2-1-m1E01-1-0057 | 629.264 | OH | a2 | B13 | b2 | C03 | c1 | D01 | d1 | E01 |
| 2-1-m1E01-1-0058 | 629.264 | OH | a2 | B11 | b2 | C05 | c1 | D01 | d1 | E01 |
| 2-1-m1E01-1-0059 | 630.206 | OH | a1 | B04 | b1 | C12 | c1 | D01 | d1 | E01 |
| 2-1-m1E01-1-0060 | 630.259 | OH | a2 | B12 | b2 | C05 | c1 | D01 | d1 | E01 |
| 2-1-m1E01-1-0061 | 632.243 | OH | a2 | B14 | b2 | C02 | c1 | D01 | d1 | E01 |
| 2-1-m1E01-1-0062 | 632.243 | OH | a2 | B11 | b2 | C04 | c1 | D01 | d1 | E01 |
| 2-1-m1E01-1-0063 | 632.263 | OH | a2 | B15 | b2 | C01 | c1 | D01 | d1 | E01 |
| 2-1-m1E01-1-0064 | 633.239 | OH | a2 | B12 | b2 | C04 | c1 | D01 | d1 | E01 |
| 2-1-m1E01-1-0065 | 633.239 | OH | a2 | B14 | b2 | C03 | c1 | D01 | d1 | E01 |
| 2-1-m1E01-1-0066 | 633.259 | OH | a1 | B01 | b2 | C02 | c1 | D01 | d1 | E01 |
| 2-1-m1E01-1-0067 | 633.295 | OH | a1 | B05 | b1 | C08 | c1 | D01 | d1 | E01 |
| 2-1-m1E01-1-0068 | 634.225 | OH | a2 | B13 | b2 | C06 | c1 | D01 | d1 | E01 |
| 2-1-m1E01-1-0069 | 634.243 | OH | a2 | B16 | b2 | C01 | c1 | D01 | d1 | E01 |
| 2-1-m1E01-1-0070 | 634.254 | OH | a1 | B02 | b2 | C02 | c1 | D01 | d1 | E01 |
| 2-1-m1E01-1-0071 | 634.254 | OH | a1 | B01 | b2 | C03 | c1 | D01 | d1 | E01 |
| 2-1-m1E01-1-0072 | 634.290 | OH | a1 | B05 | b1 | C11 | c1 | D01 | d1 | E01 |
| 2-1-m1E01-1-0073 | 635.249 | OH | a1 | B02 | b2 | C03 | c1 | D01 | d1 | E01 |

(continued)

| ID | Exact Mass | | a | B | b | C | c | D | d | E |
|---|---|---|---|---|---|---|---|---|---|---|
| 2-1-m1E01-1-0074 | 635.274 | OH | a1 | B06 | b1 | C08 | c1 | D01 | d1 | E01 |
| 2-1-m1E01-1-0075 | 635.286 | OH | a1 | B05 | b1 | C10 | c1 | D01 | d1 | E01 |
| 2-1-m1E01-1-0076 | 636.270 | OH | a1 | B06 | b1 | C11 | c1 | D01 | d1 | E01 |
| 2-1-m1E01-1-0077 | 636.274 | OH | a2 | B18 | b1 | C08 | c1 | D01 | d1 | E01 |
| 2-1-m1E01-1-0078 | 637.254 | OH | a1 | B03 | b2 | C01 | c1 | D01 | d1 | E01 |
| 2-1-m1E01-1-0079 | 637.265 | OH | a1 | B06 | b1 | C10 | c1 | D01 | d1 | E01 |
| 2-1-m1E01-1-0080 | 637.269 | OH | a2 | B18 | b1 | C11 | c1 | D01 | d1 | E01 |
| 2-1-m1E01-1-0081 | 638.200 | OH | a2 | B14 | b2 | C06 | c1 | D01 | d1 | E01 |
| 2-1-m1E01-1-0082 | 638.264 | OH | a2 | B18 | b1 | C10 | c1 | D01 | d1 | E01 |
| 2-1-m1E01-1-0083 | 638.264 | OH | a2 | B11 | b1 | C13 | c1 | D01 | d1 | E01 |
| 2-1-m1E01-1-0084 | 639.215 | OH | a1 | B01 | b2 | C06 | c1 | D01 | d1 | E01 |
| 2-1-m1E01-1-0085 | 639.259 | OH | a2 | B12 | b1 | C13 | c1 | D01 | d1 | E01 |
| 2-1-m1E01-1-0086 | 640.199 | OH | a2 | B11 | b3 | C15 | c1 | D01 | d1 | E01 |
| 2-1-m1E01-1-0087 | 640.210 | OH | a1 | B02 | b2 | C06 | c1 | D01 | d1 | E01 |
| 2-1-m1E01-1-0088 | 640.213 | OH | a2 | B17 | b2 | C01 | c1 | D01 | d1 | E01 |
| 2-1-m1E01-1-0089 | 640.247 | OH | a1 | B05 | b1 | C12 | c1 | D01 | d1 | E01 |
| 2-1-m1E01-1-0090 | 641.194 | OH | a2 | B12 | b3 | C15 | c1 | D01 | d1 | E01 |
| 2-1-m1E01-1-0091 | 641.229 | OH | a1 | B04 | b2 | C01 | c1 | D01 | d1 | E01 |
| 2-1-m1E01-1-0092 | 641.245 | OH | a1 | B07 | b1 | C08 | c1 | D01 | d1 | E01 |
| 2-1-m1E01-1-0093 | 642.226 | OH | a1 | B06 | b1 | C12 | c1 | D01 | d1 | E01 |
| 2-1-m1E01-1-0094 | 642.230 | OH | a2 | B11 | b1 | C14 | c1 | D01 | d1 | E01 |
| 2-1-m1E01-1-0095 | 642.240 | OH | a1 | B07 | b1 | C11 | c1 | D01 | d1 | E01 |
| 2-1-m1E01-1-0096 | 642.251 | OH | a2 | B11 | b2 | C33 | c3 | D02 | d1 | E01 |
| 2-1-m1E01-1-0097 | 642.284 | OH | a2 | B15 | b2 | C02 | c1 | D01 | d1 | E01 |
| 2-1-m1E01-1-0098 | 643.225 | OH | a2 | B18 | b1 | C12 | c1 | D01 | d1 | E01 |
| 2-1-m1E01-1-0099 | 643.225 | OH | a2 | B12 | b1 | C14 | c1 | D01 | d1 | E01 |
| 2-1-m1E01-1-0100 | 643.235 | OH | a1 | B07 | b1 | C10 | c1 | D01 | d1 | E01 |
| 2-1-m1E01-1-0101 | 643.246 | OH | a2 | B12 | b2 | C33 | c3 | D02 | d1 | E01 |
| 2-1-m1E01-1-0102 | 643.279 | OH | a2 | B15 | b2 | C03 | c1 | D01 | d1 | E01 |
| 2-1-m1E01-1-0103 | 643.279 | OH | a2 | B13 | b2 | C05 | c1 | D01 | d1 | E01 |
| 2-1-m1E01-1-0104 | 644.263 | OH | a2 | B16 | b2 | C02 | c1 | D01 | d1 | E01 |
| 2-1-m1E01-1-0105 | 645.259 | OH | a2 | B16 | b2 | C03 | c1 | D01 | d1 | E01 |
| 2-1-m1E01-1-0106 | 646.259 | OH | a2 | B13 | b2 | C04 | c1 | D01 | d1 | E01 |
| 2-1-m1E01-1-0107 | 646.316 | OH | a2 | B11 | b2 | C07 | c1 | D01 | d1 | E01 |
| 2-1-m1E01-1-0108 | 647.254 | OH | a2 | B14 | b2 | C05 | c1 | D01 | d1 | E01 |
| 2-1-m1E01-1-0109 | 647.274 | OH | a1 | B03 | b2 | C02 | c1 | D01 | d1 | E01 |
| 2-1-m1E01-1-0110 | 647.311 | OH | a2 | B12 | b2 | C07 | c1 | D01 | d1 | E01 |
| 2-1-m1E01-1-0111 | 648.197 | OH | a1 | B07 | b1 | C12 | c1 | D01 | d1 | E01 |

(continued)

| ID | Exact Mass | | a | B | b | C | c | D | d | E |
|---|---|---|---|---|---|---|---|---|---|---|
| 2-1-m1E01-1-0112 | 648.241 | OH | a2 | B15 | b2 | C06 | c1 | D01 | d1 | E01 |
| 2-1-m1E01-1-0113 | 648.270 | OH | a1 | B03 | b2 | C03 | c1 | D01 | d1 | E01 |
| 2-1-m1E01-1-0114 | 648.270 | OH | a1 | B01 | b2 | C05 | c1 | D01 | d1 | E01 |
| 2-1-m1E01-1-0115 | 649.265 | OH | a1 | B02 | b2 | C05 | c1 | D01 | d1 | E01 |
| 2-1-m1E01-1-0116 | 650.220 | OH | a2 | B16 | b2 | C06 | c1 | D01 | d1 | E01 |
| 2-1-m1E01-1-0117 | 650.220 | OH | a2 | B11 | b3 | C08 | c1 | D01 | d1 | E01 |
| 2-1-m1E01-1-0118 | 650.220 | OH | a2 | B11 | b1 | C08 | c2 | D02 | d1 | E01 |
| 2-1-m1E01-1-0119 | 650.234 | OH | a2 | B17 | b2 | C02 | c1 | D01 | d1 | E01 |
| 2-1-m1E01-1-0120 | 650.234 | OH | a2 | B14 | b2 | C04 | c1 | D01 | d1 | E01 |
| 2-1-m1E01-1-0121 | 651.215 | OH | a2 | B12 | b3 | C08 | c1 | D01 | d1 | E01 |
| 2-1-m1E01-1-0122 | 651.215 | OH | a2 | B12 | b1 | C08 | c2 | D02 | d1 | E01 |
| 2-1-m1E01-1-0123 | 651.215 | OH | a2 | B11 | b1 | C11 | c2 | D02 | d1 | E01 |
| 2-1-m1E01-1-0124 | 651.229 | OH | a2 | B17 | b2 | C03 | c1 | D01 | d1 | E01 |
| 2-1-m1E01-1-0125 | 651.249 | OH | a1 | B04 | b2 | C02 | c1 | D01 | d1 | E01 |
| 2-1-m1E01-1-0126 | 651.249 | OH | a1 | B01 | b2 | C04 | c1 | D01 | d1 | E01 |
| 2-1-m1E01-1-0127 | 651.269 | OH | a1 | B05 | b2 | C01 | c1 | D01 | d1 | E01 |
| 2-1-m1E01-1-0128 | 652.210 | OH | a2 | B11 | b1 | C10 | c2 | D02 | d1 | E01 |
| 2-1-m1E01-1-0129 | 652.210 | OH | a2 | B12 | b1 | C11 | c2 | D02 | d1 | E01 |
| 2-1-m1E01-1-0130 | 652.245 | OH | a1 | B02 | b2 | C04 | c1 | D01 | d1 | E01 |
| 2-1-m1E01-1-0131 | 652.245 | OH | a1 | B04 | b2 | C03 | c1 | D01 | d1 | E01 |
| 2-1-m1E01-1-0132 | 652.280 | OH | a2 | B13 | b1 | C13 | c1 | D01 | d1 | E01 |
| 2-1-m1E01-1-0133 | 653.206 | OH | a2 | B12 | b1 | C10 | c2 | D02 | d1 | E01 |
| 2-1-m1E01-1-0134 | 653.231 | OH | a1 | B03 | b2 | C06 | c1 | D01 | d1 | E01 |
| 2-1-m1E01-1-0135 | 653.249 | OH | a1 | B06 | b2 | C01 | c1 | D01 | d1 | E01 |
| 2-1-m1E01-1-0136 | 654.215 | OH | a2 | B13 | b3 | C15 | c1 | D01 | d1 | E01 |
| 2-1-m1E01-1-0137 | 654.245 | OH | a2 | B19 | b1 | C08 | c1 | D01 | d1 | E01 |
| 2-1-m1E01-1-0138 | 654.245 | OH | a2 | B11 | b1 | C09 | c1 | D01 | d1 | E01 |
| 2-1-m1E01-1-0139 | 654.248 | OH | a2 | B18 | b2 | C01 | c1 | D01 | d1 | E01 |
| 2-1-m1E01-1-0140 | 655.241 | OH | a2 | B12 | b1 | C09 | c1 | D01 | d1 | E01 |
| 2-1-m1E01-1-0141 | 655.241 | OH | a2 | B19 | b1 | C11 | c1 | D01 | d1 | E01 |
| 2-1-m1E01-1-0142 | 656.176 | OH | a2 | B11 | b3 | C06 | c1 | D01 | d1 | E01 |
| 2-1-m1E01-1-0143 | 656.190 | OH | a2 | B17 | b2 | C06 | c1 | D01 | d1 | E01 |
| 2-1-m1E01-1-0144 | 656.236 | OH | a2 | B19 | b1 | C10 | c1 | D01 | d1 | E01 |
| 2-1-m1E01-1-0145 | 656.246 | OH | a2 | B13 | b1 | C14 | c1 | D01 | d1 | E01 |
| 2-1-m1E01-1-0146 | 656.255 | OH | a2 | B14 | b1 | C13 | c1 | D01 | d1 | E01 |
| 2-1-m1E01-1-0147 | 656.267 | OH | a2 | B13 | b2 | C33 | c3 | D02 | d1 | E01 |
| 2-1-m1E01-1-0148 | 657.172 | OH | a2 | B12 | b3 | C06 | c1 | D01 | d1 | E01 |
| 2-1-m1E01-1-0149 | 657.172 | OH | a2 | B11 | b1 | C12 | c2 | D02 | d1 | E01 |

(continued)

| ID | Exact Mass | | a | B | b | C | c | D | d | E |
|---|---|---|---|---|---|---|---|---|---|---|
| 2-1-m1E01-1-0150 | 657.206 | OH | a1 | B04 | b2 | C06 | c1 | D01 | d1 | E01 |
| 2-1-m1E01-1-0151 | 657.270 | OH | a1 | B01 | b1 | C13 | c1 | D01 | d1 | E01 |
| 2-1-m1E01-1-0152 | 657.295 | OH | a2 | B15 | b2 | C05 | c1 | D01 | d1 | E01 |
| 2-1-m1E01-1-0153 | 658.167 | OH | a2 | B12 | b1 | C12 | c2 | D02 | d1 | E01 |
| 2-1-m1E01-1-0154 | 658.190 | OH | a2 | B14 | b3 | C15 | c1 | D01 | d1 | E01 |
| 2-1-m1E01-1-0155 | 658.265 | OH | a1 | B02 | b1 | C13 | c1 | D01 | d1 | E01 |
| 2-1-m1E01-1-0156 | 659.205 | OH | a1 | B01 | b3 | C15 | c1 | D01 | d1 | E01 |
| 2-1-m1E01-1-0157 | 659.219 | OH | a1 | B07 | b2 | C01 | c1 | D01 | d1 | E01 |
| 2-1-m1E01-1-0158 | 659.274 | OH | a2 | B16 | b2 | C05 | c1 | D01 | d1 | E01 |
| 2-1-m1E01-1-0159 | 660.200 | OH | a1 | B02 | b3 | C15 | c1 | D01 | d1 | E01 |
| 2-1-m1E01-1-0160 | 660.221 | OH | a2 | B14 | b1 | C14 | c1 | D01 | d1 | E01 |
| 2-1-m1E01-1-0161 | 660.242 | OH | a2 | B14 | b2 | C33 | c3 | D02 | d1 | E01 |
| 2-1-m1E01-1-0162 | 660.275 | OH | a2 | B15 | b2 | C04 | c1 | D01 | d1 | E01 |
| 2-1-m1E01-1-0163 | 660.331 | OH | a2 | B13 | b2 | C07 | c1 | D01 | d1 | E01 |
| 2-1-m1E01-1-0164 | 661.197 | OH | a2 | B19 | b1 | C12 | c1 | D01 | d1 | E01 |
| 2-1-m1E01-1-0165 | 661.236 | OH | a1 | B01 | b1 | C14 | c1 | D01 | d1 | E01 |
| 2-1-m1E01-1-0166 | 661.257 | OH | a1 | B01 | b2 | C33 | c3 | D02 | d1 | E01 |
| 2-1-m1E01-1-0167 | 661.290 | OH | a1 | B05 | b2 | C02 | c1 | D01 | d1 | E01 |
| 2-1-m1E01-1-0168 | 662.231 | OH | a1 | B08 | b1 | C08 | c1 | D01 | d1 | E01 |
| 2-1-m1E01-1-0169 | 662.231 | OH | a1 | B02 | b1 | C14 | c1 | D01 | d1 | E01 |
| 2-1-m1E01-1-0170 | 662.252 | OH | a1 | B02 | b2 | C33 | c3 | D02 | d1 | E01 |
| 2-1-m1E01-1-0171 | 662.254 | OH | a2 | B16 | b2 | C04 | c1 | D01 | d1 | E01 |
| 2-1-m1E01-1-0172 | 662.256 | OH | a2 | B11 | b1 | C30 | c3 | D02 | d1 | E01 |
| 2-1-m1E01-1-0173 | 662.285 | OH | a1 | B05 | b2 | C03 | c1 | D01 | d1 | E01 |
| 2-1-m1E01-1-0174 | 662.285 | OH | a1 | B03 | b2 | C05 | c1 | D01 | d1 | E01 |
| 2-1-m1E01-1-0175 | 663.226 | OH | a1 | B08 | b1 | C11 | c1 | D01 | d1 | E01 |
| 2-1-m1E01-1-0176 | 663.252 | OH | a2 | B12 | b1 | C30 | c3 | D02 | d1 | E01 |
| 2-1-m1E01-1-0177 | 663.269 | OH | a1 | B06 | b2 | C02 | c1 | D01 | d1 | E01 |
| 2-1-m1E01-1-0178 | 664.222 | OH | a1 | B08 | b1 | C10 | c1 | D01 | d1 | E01 |
| 2-1-m1E01-1-0179 | 664.236 | OH | a2 | B13 | b3 | C08 | c1 | D01 | d1 | E01 |
| 2-1-m1E01-1-0180 | 664.236 | OH | a2 | B13 | b1 | C08 | c2 | D02 | d1 | E01 |
| 2-1-m1E01-1-0181 | 664.236 | OH | a2 | B11 | b2 | C20 | c3 | D02 | d1 | E01 |
| 2-1-m1E01-1-0182 | 664.265 | OH | a1 | B06 | b2 | C03 | c1 | D01 | d1 | E01 |
| 2-1-m1E01-1-0183 | 664.269 | OH | a2 | B18 | b2 | C02 | c1 | D01 | d1 | E01 |
| 2-1-m1E01-1-0184 | 664.306 | OH | a2 | B14 | b2 | C07 | c1 | D01 | d1 | E01 |
| 2-1-m1E01-1-0185 | 665.231 | OH | a2 | B13 | b1 | C11 | c2 | D02 | d1 | E01 |
| 2-1-m1E01-1-0186 | 665.231 | OH | a2 | B12 | b2 | C20 | c3 | D02 | d1 | E01 |
| 2-1-m1E01-1-0187 | 665.231 | OH | a2 | B11 | b2 | C28 | c3 | D02 | d1 | E01 |

(continued)

| ID | Exact Mass | | a | B | b | C | c | D | d | E |
|---|---|---|---|---|---|---|---|---|---|---|
| 2-1-m1E01-1-0188 | 665.245 | OH | a2 | B17 | b2 | C05 | c1 | D01 | d1 | E01 |
| 2-1-m1E01-1-0189 | 665.264 | OH | a2 | B18 | b2 | C03 | c1 | D01 | d1 | E01 |
| 2-1-m1E01-1-0190 | 665.265 | OH | a1 | B03 | b2 | C04 | c1 | D01 | d1 | E01 |
| 2-1-m1E01-1-0191 | 665.321 | OH | a1 | B01 | b2 | C07 | c1 | D01 | d1 | E01 |
| 2-1-m1E01-1-0192 | 666.226 | OH | a2 | B13 | b1 | C10 | c2 | D02 | d1 | E01 |
| 2-1-m1E01-1-0193 | 666.226 | OH | a2 | B12 | b2 | C28 | c3 | D02 | d1 | E01 |
| 2-1-m1E01-1-0194 | 666.260 | OH | a1 | B04 | b2 | C05 | c1 | D01 | d1 | E01 |
| 2-1-m1E01-1-0195 | 666.295 | OH | a2 | B15 | b1 | C13 | c1 | D01 | d1 | E01 |
| 2-1-m1E01-1-0196 | 666.317 | OH | a1 | B02 | b2 | C07 | c1 | D01 | d1 | E01 |
| 2-1-m1E01-1-0197 | 667.246 | OH | a1 | B05 | b2 | C06 | c1 | D01 | d1 | E01 |
| 2-1-m1E01-1-0198 | 668.194 | OH | a2 | B11 | b2 | C01 | c2 | D02 | d1 | E01 |
| 2-1-m1E01-1-0199 | 668.210 | OH | a2 | B14 | b3 | C08 | c1 | D01 | d1 | E01 |
| 2-1-m1E01-1-0200 | 668.210 | OH | a2 | B14 | b1 | C08 | c2 | D02 | d1 | E01 |
| 2-1-m1E01-1-0201 | 668.225 | OH | a2 | B17 | b2 | C04 | c1 | D01 | d1 | E01 |
| 2-1-m1E01-1-0202 | 668.230 | OH | a2 | B15 | b3 | C15 | c1 | D01 | d1 | E01 |
| 2-1-m1E01-1-0203 | 668.261 | OH | a2 | B13 | b1 | C09 | c1 | D01 | d1 | E01 |
| 2-1-m1E01-1-0204 | 668.275 | OH | a2 | B16 | b1 | C13 | c1 | D01 | d1 | E01 |
| 2-1-m1E01-1-0205 | 669.183 | OH | a1 | B08 | b1 | C12 | c1 | D01 | d1 | E01 |
| 2-1-m1E01-1-0206 | 669.189 | OH | a2 | B12 | b2 | C01 | c2 | D02 | d1 | E01 |
| 2-1-m1E01-1-0207 | 669.206 | OH | a2 | B14 | b1 | C11 | c2 | D02 | d1 | E01 |
| 2-1-m1E01-1-0208 | 669.226 | OH | a1 | B06 | b2 | C06 | c1 | D01 | d1 | E01 |
| 2-1-m1E01-1-0209 | 669.226 | OH | a1 | B01 | b3 | C08 | c1 | D01 | d1 | E01 |
| 2-1-m1E01-1-0210 | 669.226 | OH | a1 | B01 | b1 | C08 | c2 | D02 | d1 | E01 |
| 2-1-m1E01-1-0211 | 669.240 | OH | a1 | B07 | b2 | C02 | c1 | D01 | d1 | E01 |
| 2-1-m1E01-1-0212 | 669.240 | OH | a1 | B04 | b2 | C04 | c1 | D01 | d1 | E01 |
| 2-1-m1E01-1-0213 | 670.192 | OH | a2 | B13 | b3 | C06 | c1 | D01 | d1 | E01 |
| 2-1-m1E01-1-0214 | 670.201 | OH | a2 | B14 | b1 | C10 | c2 | D02 | d1 | E01 |
| 2-1-m1E01-1-0215 | 670.210 | OH | a2 | B16 | b3 | C15 | c1 | D01 | d1 | E01 |
| 2-1-m1E01-1-0216 | 670.221 | OH | a1 | B02 | b3 | C08 | c1 | D01 | d1 | E01 |
| 2-1-m1E01-1-0217 | 670.221 | OH | a1 | B02 | b1 | C08 | c2 | D02 | d1 | E01 |
| 2-1-m1E01-1-0218 | 670.221 | OH | a1 | B01 | b1 | C11 | c2 | D02 | d1 | E01 |
| 2-1-m1E01-1-0219 | 670.225 | OH | a2 | B18 | b2 | C06 | c1 | D01 | d1 | E01 |
| 2-1-m1E01-1-0220 | 670.235 | OH | a1 | B07 | b2 | C03 | c1 | D01 | d1 | E01 |
| 2-1-m1E01-1-0221 | 670.240 | OH | a2 | B20 | b1 | C08 | c1 | D01 | d1 | E01 |
| 2-1-m1E01-1-0222 | 670.261 | OH | a2 | B15 | b1 | C14 | c1 | D01 | d1 | E01 |
| 2-1-m1E01-1-0223 | 670.283 | OH | a2 | B15 | b2 | C33 | c3 | D02 | d1 | E01 |
| 2-1-m1E01-1-0224 | 671.187 | OH | a2 | B13 | b1 | C12 | c2 | D02 | d1 | E01 |
| 2-1-m1E01-1-0225 | 671.216 | OH | a1 | B01 | b1 | C10 | c2 | D02 | d1 | E01 |

(continued)

| ID | Exact Mass | | a | B | b | C | c | D | d | E |
|---|---|---|---|---|---|---|---|---|---|---|
| 2-1-m1E01-1-0226 | 671.216 | OH | a1 | B02 | b1 | C11 | c2 | D02 | d1 | E01 |
| 2-1-m1E01-1-0227 | 671.236 | OH | a2 | B20 | b1 | C11 | c1 | D01 | d1 | E01 |
| 2-1-m1E01-1-0228 | 671.286 | OH | a1 | B03 | b1 | C13 | c1 | D01 | d1 | E01 |
| 2-1-m1E01-1-0229 | 672.211 | OH | a1 | B02 | b1 | C10 | c2 | D02 | d1 | E01 |
| 2-1-m1E01-1-0230 | 672.220 | OH | a2 | B19 | b2 | C01 | c1 | D01 | d1 | E01 |
| 2-1-m1E01-1-0231 | 672.231 | OH | a2 | B20 | b1 | C10 | c1 | D01 | d1 | E01 |
| 2-1-m1E01-1-0232 | 672.236 | OH | a2 | B14 | b1 | C09 | c1 | D01 | d1 | E01 |
| 2-1-m1E01-1-0233 | 672.241 | OH | a2 | B16 | b1 | C14 | c1 | D01 | d1 | E01 |
| 2-1-m1E01-1-0234 | 672.262 | OH | a2 | B16 | b2 | C33 | c3 | D02 | d1 | E01 |
| 2-1-m1E01-1-0235 | 673.221 | OH | a1 | B03 | b3 | C15 | c1 | D01 | d1 | E01 |
| 2-1-m1E01-1-0236 | 673.251 | OH | a1 | B09 | b1 | C08 | c1 | D01 | d1 | E01 |
| 2-1-m1E01-1-0237 | 673.251 | OH | a1 | B01 | b1 | C09 | c1 | D01 | d1 | E01 |
| 2-1-m1E01-1-0238 | 674.167 | OH | a2 | B14 | b3 | C06 | c1 | D01 | d1 | E01 |
| 2-1-m1E01-1-0239 | 674.245 | OH | a2 | B17 | b1 | C13 | c1 | D01 | d1 | E01 |
| 2-1-m1E01-1-0240 | 674.246 | OH | a1 | B02 | b1 | C09 | c1 | D01 | d1 | E01 |
| 2-1-m1E01-1-0241 | 674.246 | OH | a1 | B09 | b1 | C11 | c1 | D01 | d1 | E01 |
| 2-1-m1E01-1-0242 | 674.347 | OH | a2 | B15 | b2 | C07 | c1 | D01 | d1 | E01 |
| 2-1-m1E01-1-0243 | 675.162 | OH | a2 | B14 | b1 | C12 | c2 | D02 | d1 | E01 |
| 2-1-m1E01-1-0244 | 675.182 | OH | a1 | B01 | b3 | C06 | c1 | D01 | d1 | E01 |
| 2-1-m1E01-1-0245 | 675.196 | OH | a1 | B07 | b2 | C06 | c1 | D01 | d1 | E01 |
| 2-1-m1E01-1-0246 | 675.242 | OH | a1 | B09 | b1 | C10 | c1 | D01 | d1 | E01 |
| 2-1-m1E01-1-0247 | 675.252 | OH | a1 | B03 | b1 | C14 | c1 | D01 | d1 | E01 |
| 2-1-m1E01-1-0248 | 675.261 | OH | a1 | B04 | b1 | C13 | c1 | D01 | d1 | E01 |
| 2-1-m1E01-1-0249 | 675.273 | OH | a1 | B03 | b2 | C33 | c3 | D02 | d1 | E01 |
| 2-1-m1E01-1-0250 | 676.177 | OH | a1 | B02 | b3 | C06 | c1 | D01 | d1 | E01 |
| 2-1-m1E01-1-0251 | 676.177 | OH | a1 | B01 | b1 | C12 | c2 | D02 | d1 | E01 |
| 2-1-m1E01-1-0252 | 676.180 | OH | a2 | B17 | b3 | C15 | c1 | D01 | d1 | E01 |
| 2-1-m1E01-1-0253 | 676.272 | OH | a2 | B13 | b1 | C30 | c3 | D02 | d1 | E01 |
| 2-1-m1E01-1-0254 | 676.272 | OH | a2 | B11 | b1 | C32 | c3 | D02 | d1 | E01 |
| 2-1-m1E01-1-0255 | 676.301 | OH | a1 | B05 | b2 | C05 | c1 | D01 | d1 | E01 |
| 2-1-m1E01-1-0256 | 676.326 | OH | a2 | B16 | b2 | C07 | c1 | D01 | d1 | E01 |
| 2-1-m1E01-1-0257 | 677.173 | OH | a1 | B02 | b1 | C12 | c2 | D02 | d1 | E01 |
| 2-1-m1E01-1-0258 | 677.192 | OH | a2 | B20 | b1 | C12 | c1 | D01 | d1 | E01 |
| 2-1-m1E01-1-0259 | 677.196 | OH | a1 | B04 | b3 | C15 | c1 | D01 | d1 | E01 |
| 2-1-m1E01-1-0260 | 677.267 | OH | a2 | B12 | b1 | C32 | c3 | D02 | d1 | E01 |
| 2-1-m1E01-1-0261 | 678.211 | OH | a2 | B17 | b1 | C14 | c1 | D01 | d1 | E01 |
| 2-1-m1E01-1-0262 | 678.215 | OH | a2 | B11 | b2 | C02 | c2 | D02 | d1 | E01 |
| 2-1-m1E01-1-0263 | 678.232 | OH | a2 | B17 | b2 | C33 | c3 | D02 | d1 | E01 |

(continued)

| ID | Exact Mass | | a | B | b | C | c | D | d | E |
|---|---|---|---|---|---|---|---|---|---|---|
| 2-1-m1E01-1-0264 | 678.251 | OH | a2 | B15 | b3 | C08 | c1 | D01 | d1 | E01 |
| 2-1-m1E01-1-0265 | 678.251 | OH | a2 | B15 | b1 | C08 | c2 | D02 | d1 | E01 |
| 2-1-m1E01-1-0266 | 678.251 | OH | a2 | B13 | b2 | C20 | c3 | D02 | d1 | E01 |
| 2-1-m1E01-1-0267 | 678.251 | OH | a2 | B11 | b2 | C22 | c3 | D02 | d1 | E01 |
| 2-1-m1E01-1-0268 | 678.280 | OH | a1 | B06 | b2 | C05 | c1 | D01 | d1 | E01 |
| 2-1-m1E01-1-0269 | 679.210 | OH | a2 | B12 | b2 | C02 | c2 | D02 | d1 | E01 |
| 2-1-m1E01-1-0270 | 679.210 | OH | a2 | B11 | b2 | C03 | c2 | D02 | d1 | E01 |
| 2-1-m1E01-1-0271 | 679.226 | OH | a1 | B04 | b1 | C14 | c1 | D01 | d1 | E01 |
| 2-1-m1E01-1-0272 | 679.246 | OH | a2 | B15 | b1 | C11 | c2 | D02 | d1 | E01 |
| 2-1-m1E01-1-0273 | 679.246 | OH | a2 | B12 | b2 | C22 | c3 | D02 | d1 | E01 |
| 2-1-m1E01-1-0274 | 679.246 | OH | a2 | B13 | b2 | C28 | c3 | D02 | d1 | E01 |
| 2-1-m1E01-1-0275 | 679.248 | OH | a1 | B04 | b2 | C33 | c3 | D02 | d1 | E01 |
| 2-1-m1E01-1-0276 | 679.279 | OH | a2 | B18 | b2 | C05 | c1 | D01 | d1 | E01 |
| 2-1-m1E01-1-0277 | 679.281 | OH | a1 | B05 | b2 | C04 | c1 | D01 | d1 | E01 |
| 2-1-m1E01-1-0278 | 679.337 | OH | a1 | B03 | b2 | C07 | c1 | D01 | d1 | E01 |
| 2-1-m1E01-1-0279 | 680.203 | OH | a1 | B09 | b1 | C12 | c1 | D01 | d1 | E01 |
| 2-1-m1E01-1-0280 | 680.205 | OH | a1 | B08 | b2 | C01 | c1 | D01 | d1 | E01 |
| 2-1-m1E01-1-0281 | 680.205 | OH | a2 | B12 | b2 | C03 | c2 | D02 | d1 | E01 |
| 2-1-m1E01-1-0282 | 680.230 | OH | a2 | B16 | b3 | C08 | c1 | D01 | d1 | E01 |
| 2-1-m1E01-1-0283 | 680.230 | OH | a2 | B16 | b1 | C08 | c2 | D02 | d1 | E01 |
| 2-1-m1E01-1-0284 | 680.242 | OH | a2 | B15 | b1 | C10 | c2 | D02 | d1 | E01 |
| 2-1-m1E01-1-0285 | 680.247 | OH | a2 | B14 | b1 | C30 | c3 | D02 | d1 | E01 |
| 2-1-m1E01-1-0286 | 681.226 | OH | a2 | B16 | b1 | C11 | c2 | D02 | d1 | E01 |
| 2-1-m1E01-1-0287 | 681.260 | OH | a1 | B06 | b2 | C04 | c1 | D01 | d1 | E01 |
| 2-1-m1E01-1-0288 | 681.262 | OH | a1 | B01 | b1 | C30 | c3 | D02 | d1 | E01 |
| 2-1-m1E01-1-0289 | 682.210 | OH | a2 | B13 | b2 | C01 | c2 | D02 | d1 | E01 |
| 2-1-m1E01-1-0290 | 682.221 | OH | a2 | B16 | b1 | C10 | c2 | D02 | d1 | E01 |
| 2-1-m1E01-1-0291 | 682.226 | OH | a2 | B14 | b2 | C20 | c3 | D02 | d1 | E01 |
| 2-1-m1E01-1-0292 | 682.226 | OH | a2 | B11 | b2 | C24 | c3 | D02 | d1 | E01 |
| 2-1-m1E01-1-0293 | 682.240 | OH | a2 | B19 | b2 | C02 | c1 | D01 | d1 | E01 |
| 2-1-m1E01-1-0294 | 682.257 | OH | a1 | B02 | b1 | C30 | c3 | D02 | d1 | E01 |
| 2-1-m1E01-1-0295 | 682.259 | OH | a2 | B18 | b2 | C04 | c1 | D01 | d1 | E01 |
| 2-1-m1E01-1-0296 | 682.277 | OH | a2 | B15 | b1 | C09 | c1 | D01 | d1 | E01 |
| 2-1-m1E01-1-0297 | 682.297 | OH | a2 | B17 | b2 | C07 | c1 | D01 | d1 | E01 |
| 2-1-m1E01-1-0298 | 683.221 | OH | a2 | B12 | b2 | C24 | c3 | D02 | d1 | E01 |
| 2-1-m1E01-1-0299 | 683.221 | OH | a2 | B14 | b2 | C28 | c3 | D02 | d1 | E01 |
| 2-1-m1E01-1-0300 | 683.236 | OH | a2 | B19 | b2 | C03 | c1 | D01 | d1 | E01 |
| 2-1-m1E01-1-0301 | 683.241 | OH | a1 | B03 | b3 | C08 | c1 | D01 | d1 | E01 |

(continued)

| ID | Exact Mass | | a | B | b | C | c | D | d | E |
|---|---|---|---|---|---|---|---|---|---|---|
| 2-1-m1E01-1-0302 | 683.241 | OH | a1 | B03 | b1 | C08 | c2 | D02 | d1 | E01 |
| 2-1-m1E01-1-0303 | 683.241 | OH | a1 | B01 | b2 | C20 | c3 | D02 | d1 | E01 |
| 2-1-m1E01-1-0304 | 683.312 | OH | a1 | B04 | b2 | C07 | c1 | D01 | d1 | E01 |
| 2-1-m1E01-1-0305 | 684.171 | OH | a2 | B11 | b2 | C06 | c2 | D02 | d1 | E01 |
| 2-1-m1E01-1-0306 | 684.208 | OH | a2 | B15 | b3 | C06 | c1 | D01 | d1 | E01 |
| 2-1-m1E01-1-0307 | 684.237 | OH | a1 | B03 | b1 | C11 | c2 | D02 | d1 | E01 |
| 2-1-m1E01-1-0308 | 684.237 | OH | a1 | B02 | b2 | C20 | c3 | D02 | d1 | E01 |
| 2-1-m1E01-1-0309 | 684.237 | OH | a1 | B01 | b2 | C28 | c3 | D02 | d1 | E01 |
| 2-1-m1E01-1-0310 | 684.251 | OH | a1 | B07 | b2 | C05 | c1 | D01 | d1 | E01 |
| 2-1-m1E01-1-0311 | 684.256 | OH | a2 | B16 | b1 | C09 | c1 | D01 | d1 | E01 |
| 2-1-m1E01-1-0312 | 685.166 | OH | a2 | B12 | b2 | C06 | c2 | D02 | d1 | E01 |
| 2-1-m1E01-1-0313 | 685.203 | OH | a2 | B15 | b1 | C12 | c2 | D02 | d1 | E01 |
| 2-1-m1E01-1-0314 | 685.232 | OH | a1 | B03 | b1 | C10 | c2 | D02 | d1 | E01 |
| 2-1-m1E01-1-0315 | 685.232 | OH | a1 | B02 | b2 | C28 | c3 | D02 | d1 | E01 |
| 2-1-m1E01-1-0316 | 685.301 | OH | a1 | B05 | b1 | C13 | c1 | D01 | d1 | E01 |
| 2-1-m1E01-1-0317 | 686.185 | OH | a2 | B14 | b2 | C01 | c2 | D02 | d1 | E01 |
| 2-1-m1E01-1-0318 | 686.187 | OH | a2 | B16 | b3 | C06 | c1 | D01 | d1 | E01 |
| 2-1-m1E01-1-0319 | 686.201 | OH | a2 | B17 | b3 | C08 | c1 | D01 | d1 | E01 |
| 2-1-m1E01-1-0320 | 686.201 | OH | a2 | B17 | b1 | C08 | c2 | D02 | d1 | E01 |
| 2-1-m1E01-1-0321 | 687.182 | OH | a2 | B16 | b1 | C12 | c2 | D02 | d1 | E01 |
| 2-1-m1E01-1-0322 | 687.196 | OH | a2 | B17 | b1 | C11 | c2 | D02 | d1 | E01 |
| 2-1-m1E01-1-0323 | 687.200 | OH | a1 | B01 | b2 | C01 | c2 | D02 | d1 | E01 |
| 2-1-m1E01-1-0324 | 687.216 | OH | a1 | B04 | b3 | C08 | c1 | D01 | d1 | E01 |
| 2-1-m1E01-1-0325 | 687.216 | OH | a1 | B04 | b1 | C08 | c2 | D02 | d1 | E01 |
| 2-1-m1E01-1-0326 | 687.230 | OH | a1 | B07 | b2 | C04 | c1 | D01 | d1 | E01 |
| 2-1-m1E01-1-0327 | 687.236 | OH | a1 | B05 | b3 | C15 | c1 | D01 | d1 | E01 |
| 2-1-m1E01-1-0328 | 687.267 | OH | a1 | B03 | b1 | C09 | c1 | D01 | d1 | E01 |
| 2-1-m1E01-1-0329 | 687.281 | OH | a1 | B06 | b1 | C13 | c1 | D01 | d1 | E01 |
| 2-1-m1E01-1-0330 | 688.192 | OH | a2 | B17 | b1 | C10 | c2 | D02 | d1 | E01 |
| 2-1-m1E01-1-0331 | 688.195 | OH | a1 | B02 | b2 | C01 | c2 | D02 | d1 | E01 |
| 2-1-m1E01-1-0332 | 688.197 | OH | a2 | B19 | b2 | C06 | c1 | D01 | d1 | E01 |
| 2-1-m1E01-1-0333 | 688.212 | OH | a1 | B04 | b1 | C11 | c2 | D02 | d1 | E01 |
| 2-1-m1E01-1-0334 | 688.214 | OH | a2 | B20 | b2 | C01 | c1 | D01 | d1 | E01 |
| 2-1-m1E01-1-0335 | 688.280 | OH | a2 | B18 | b1 | C13 | c1 | D01 | d1 | E01 |
| 2-1-m1E01-1-0336 | 689.198 | OH | a1 | B03 | b3 | C06 | c1 | D01 | d1 | E01 |
| 2-1-m1E01-1-0337 | 689.207 | OH | a1 | B04 | b1 | C10 | c2 | D02 | d1 | E01 |
| 2-1-m1E01-1-0338 | 689.216 | OH | a1 | B06 | b3 | C15 | c1 | D01 | d1 | E01 |
| 2-1-m1E01-1-0339 | 689.246 | OH | a1 | B10 | b1 | C08 | c1 | D01 | d1 | E01 |

(continued)

| ID | Exact Mass | | a | B | b | C | c | D | d | E |
|---|---|---|---|---|---|---|---|---|---|---|
| 2-1-m1E01-1-0340 | 689.267 | OH | a1 | B05 | b1 | C14 | c1 | D01 | d1 | E01 |
| 2-1-m1E01-1-0341 | 689.288 | OH | a1 | B05 | b2 | C33 | c3 | D02 | d1 | E01 |
| 2-1-m1E01-1-0342 | 690.193 | OH | a1 | B03 | b1 | C12 | c2 | D02 | d1 | E01 |
| 2-1-m1E01-1-0343 | 690.215 | OH | a2 | B18 | b3 | C15 | c1 | D01 | d1 | E01 |
| 2-1-m1E01-1-0344 | 690.226 | OH | a1 | B08 | b2 | C02 | c1 | D01 | d1 | E01 |
| 2-1-m1E01-1-0345 | 690.227 | OH | a2 | B17 | b1 | C09 | c1 | D01 | d1 | E01 |
| 2-1-m1E01-1-0346 | 690.241 | OH | a1 | B10 | b1 | C11 | c1 | D01 | d1 | E01 |
| 2-1-m1E01-1-0347 | 690.288 | OH | a2 | B15 | b1 | C30 | c3 | D02 | d1 | E01 |
| 2-1-m1E01-1-0348 | 690.288 | OH | a2 | B11 | b1 | C29 | c3 | D02 | d1 | E01 |
| 2-1-m1E01-1-0349 | 690.288 | OH | a2 | B13 | b1 | C32 | c3 | D02 | d1 | E01 |
| 2-1-m1E01-1-0350 | 691.221 | OH | a1 | B08 | b2 | C03 | c1 | D01 | d1 | E01 |
| 2-1-m1E01-1-0351 | 691.225 | OH | a1 | B09 | b2 | C01 | c1 | D01 | d1 | E01 |
| 2-1-m1E01-1-0352 | 691.237 | OH | a1 | B10 | b1 | C10 | c1 | D01 | d1 | E01 |
| 2-1-m1E01-1-0353 | 691.242 | OH | a1 | B04 | b1 | C09 | c1 | D01 | d1 | E01 |
| 2-1-m1E01-1-0354 | 691.246 | OH | a1 | B06 | b1 | C14 | c1 | D01 | d1 | E01 |
| 2-1-m1E01-1-0355 | 691.268 | OH | a1 | B06 | b2 | C33 | c3 | D02 | d1 | E01 |
| 2-1-m1E01-1-0356 | 691.283 | OH | a2 | B12 | b1 | C29 | c3 | D02 | d1 | E01 |
| 2-1-m1E01-1-0357 | 692.157 | OH | a2 | B17 | b3 | C06 | c1 | D01 | d1 | E01 |
| 2-1-m1E01-1-0358 | 692.230 | OH | a2 | B13 | b2 | C02 | c2 | D02 | d1 | E01 |
| 2-1-m1E01-1-0359 | 692.246 | OH | a2 | B18 | b1 | C14 | c1 | D01 | d1 | E01 |
| 2-1-m1E01-1-0360 | 692.267 | OH | a2 | B15 | b2 | C20 | c3 | D02 | d1 | E01 |
| 2-1-m1E01-1-0361 | 692.267 | OH | a2 | B13 | b2 | C22 | c3 | D02 | d1 | E01 |
| 2-1-m1E01-1-0362 | 692.267 | OH | a2 | B16 | b1 | C30 | c3 | D02 | d1 | E01 |
| 2-1-m1E01-1-0363 | 692.267 | OH | a2 | B18 | b2 | C33 | c3 | D02 | d1 | E01 |
| 2-1-m1E01-1-0364 | 693.153 | OH | a2 | B17 | b1 | C12 | c2 | D02 | d1 | E01 |
| 2-1-m1E01-1-0365 | 693.173 | OH | a1 | B04 | b3 | C06 | c1 | D01 | d1 | E01 |
| 2-1-m1E01-1-0366 | 693.226 | OH | a2 | B13 | b2 | C03 | c2 | D02 | d1 | E01 |
| 2-1-m1E01-1-0367 | 693.226 | OH | a2 | B11 | b2 | C05 | c2 | D02 | d1 | E01 |
| 2-1-m1E01-1-0368 | 693.251 | OH | a1 | B07 | b1 | C13 | c1 | D01 | d1 | E01 |
| 2-1-m1E01-1-0369 | 693.262 | OH | a2 | B15 | b2 | C28 | c3 | D02 | d1 | E01 |
| 2-1-m1E01-1-0370 | 693.353 | OH | a1 | B05 | b2 | C07 | c1 | D01 | d1 | E01 |
| 2-1-m1E01-1-0371 | 694.168 | OH | a1 | B04 | b1 | C12 | c2 | D02 | d1 | E01 |
| 2-1-m1E01-1-0372 | 694.221 | OH | a2 | B12 | b2 | C05 | c2 | D02 | d1 | E01 |
| 2-1-m1E01-1-0373 | 694.246 | OH | a2 | B16 | b2 | C20 | c3 | D02 | d1 | E01 |
| 2-1-m1E01-1-0374 | 694.246 | OH | a2 | B11 | b2 | C26 | c3 | D02 | d1 | E01 |
| 2-1-m1E01-1-0375 | 694.263 | OH | a2 | B14 | b1 | C32 | c3 | D02 | d1 | E01 |
| 2-1-m1E01-1-0376 | 695.186 | OH | a1 | B07 | b3 | C15 | c1 | D01 | d1 | E01 |
| 2-1-m1E01-1-0377 | 695.241 | OH | a2 | B12 | b2 | C26 | c3 | D02 | d1 | E01 |

(continued)

| ID | Exact Mass | | a | B | b | C | c | D | d | E |
|---|---|---|---|---|---|---|---|---|---|---|
| 2-1-m1E01-1-0378 | 695.241 | OH | a2 | B16 | b2 | C28 | c3 | D02 | d1 | E01 |
| 2-1-m1E01-1-0379 | 695.278 | OH | a1 | B03 | b1 | C30 | c3 | D02 | d1 | E01 |
| 2-1-m1E01-1-0380 | 695.278 | OH | a1 | B01 | b1 | C32 | c3 | D02 | d1 | E01 |
| 2-1-m1E01-1-0381 | 695.332 | OH | a1 | B06 | b2 | C07 | c1 | D01 | d1 | E01 |
| 2-1-m1E01-1-0382 | 696.182 | OH | a1 | B08 | b2 | C06 | c1 | D01 | d1 | E01 |
| 2-1-m1E01-1-0383 | 696.198 | OH | a1 | B10 | b1 | C12 | c1 | D01 | d1 | E01 |
| 2-1-m1E01-1-0384 | 696.205 | OH | a2 | B14 | b2 | C02 | c2 | D02 | d1 | E01 |
| 2-1-m1E01-1-0385 | 696.205 | OH | a2 | B11 | b2 | C04 | c2 | D02 | d1 | E01 |
| 2-1-m1E01-1-0386 | 696.225 | OH | a2 | B15 | b2 | C01 | c2 | D02 | d1 | E01 |
| 2-1-m1E01-1-0387 | 696.242 | OH | a2 | B14 | b2 | C22 | c3 | D02 | d1 | E01 |
| 2-1-m1E01-1-0388 | 696.242 | OH | a2 | B13 | b2 | C24 | c3 | D02 | d1 | E01 |
| 2-1-m1E01-1-0389 | 696.273 | OH | a1 | B02 | b1 | C32 | c3 | D02 | d1 | E01 |
| 2-1-m1E01-1-0390 | 696.331 | OH | a2 | B18 | b2 | C07 | c1 | D01 | d1 | E01 |
| 2-1-m1E01-1-0391 | 697.201 | OH | a2 | B12 | b2 | C04 | c2 | D02 | d1 | E01 |
| 2-1-m1E01-1-0392 | 697.201 | OH | a2 | B14 | b2 | C03 | c2 | D02 | d1 | E01 |
| 2-1-m1E01-1-0393 | 697.217 | OH | a1 | B07 | b1 | C14 | c1 | D01 | d1 | E01 |
| 2-1-m1E01-1-0394 | 697.221 | OH | a1 | B01 | b2 | C02 | c2 | D02 | d1 | E01 |
| 2-1-m1E01-1-0395 | 697.238 | OH | a1 | B07 | b2 | C33 | c3 | D02 | d1 | E01 |
| 2-1-m1E01-1-0396 | 697.251 | OH | a2 | B19 | b2 | C05 | c1 | D01 | d1 | E01 |
| 2-1-m1E01-1-0397 | 697.257 | OH | a1 | B05 | b3 | C08 | c1 | D01 | d1 | E01 |
| 2-1-m1E01-1-0398 | 697.257 | OH | a1 | B05 | b1 | C08 | c2 | D02 | d1 | E01 |
| 2-1-m1E01-1-0399 | 697.257 | OH | a1 | B03 | b2 | C20 | c3 | D02 | d1 | E01 |
| 2-1-m1E01-1-0400 | 697.257 | OH | a1 | B01 | b2 | C22 | c3 | D02 | d1 | E01 |
| 2-1-m1E01-1-0401 | 698.187 | OH | a2 | B13 | b2 | C06 | c2 | D02 | d1 | E01 |
| 2-1-m1E01-1-0402 | 698.205 | OH | a2 | B16 | b2 | C01 | c2 | D02 | d1 | E01 |
| 2-1-m1E01-1-0403 | 698.216 | OH | a1 | B02 | b2 | C02 | c2 | D02 | d1 | E01 |
| 2-1-m1E01-1-0404 | 698.216 | OH | a1 | B01 | b2 | C03 | c2 | D02 | d1 | E01 |
| 2-1-m1E01-1-0405 | 698.235 | OH | a2 | B20 | b2 | C02 | c1 | D01 | d1 | E01 |
| 2-1-m1E01-1-0406 | 698.237 | OH | a2 | B17 | b1 | C30 | c3 | D02 | d1 | E01 |
| 2-1-m1E01-1-0407 | 698.252 | OH | a1 | B05 | b1 | C11 | c2 | D02 | d1 | E01 |
| 2-1-m1E01-1-0408 | 698.252 | OH | a1 | B02 | b2 | C22 | c3 | D02 | d1 | E01 |
| 2-1-m1E01-1-0409 | 698.252 | OH | a1 | B03 | b2 | C28 | c3 | D02 | d1 | E01 |
| 2-1-m1E01-1-0410 | 699.211 | OH | a1 | B02 | b2 | C03 | c2 | D02 | d1 | E01 |
| 2-1-m1E01-1-0411 | 699.230 | OH | a2 | B20 | b2 | C03 | c1 | D01 | d1 | E01 |
| 2-1-m1E01-1-0412 | 699.236 | OH | a1 | B06 | b3 | C08 | c1 | D01 | d1 | E01 |
| 2-1-m1E01-1-0413 | 699.236 | OH | a1 | B06 | b1 | C08 | c2 | D02 | d1 | E01 |
| 2-1-m1E01-1-0414 | 699.248 | OH | a1 | B05 | b1 | C10 | c2 | D02 | d1 | E01 |
| 2-1-m1E01-1-0415 | 699.253 | OH | a1 | B04 | b1 | C30 | c3 | D02 | d1 | E01 |

(continued)

| ID | Exact Mass | | a | B | b | C | c | D | d | E |
|---|---|---|---|---|---|---|---|---|---|---|
| 2-1-m1E01-1-0416 | 700.217 | OH | a2 | B17 | b2 | C20 | c3 | D02 | d1 | E01 |
| 2-1-m1E01-1-0417 | 700.217 | OH | a2 | B14 | b2 | C24 | c3 | D02 | d1 | E01 |
| 2-1-m1E01-1-0418 | 700.231 | OH | a2 | B19 | b2 | C04 | c1 | D01 | d1 | E01 |
| 2-1-m1E01-1-0419 | 700.232 | OH | a1 | B06 | b1 | C11 | c2 | D02 | d1 | E01 |
| 2-1-m1E01-1-0420 | 700.236 | OH | a2 | B18 | b3 | C08 | c1 | D01 | d1 | E01 |
| 2-1-m1E01-1-0421 | 700.236 | OH | a2 | B18 | b1 | C08 | c2 | D02 | d1 | E01 |
| 2-1-m1E01-1-0422 | 701.212 | OH | a2 | B17 | b2 | C28 | c3 | D02 | d1 | E01 |
| 2-1-m1E01-1-0423 | 701.216 | OH | a1 | B03 | b2 | C01 | c2 | D02 | d1 | E01 |
| 2-1-m1E01-1-0424 | 701.227 | OH | a1 | B06 | b1 | C10 | c2 | D02 | d1 | E01 |
| 2-1-m1E01-1-0425 | 701.231 | OH | a2 | B18 | b1 | C11 | c2 | D02 | d1 | E01 |
| 2-1-m1E01-1-0426 | 701.232 | OH | a1 | B04 | b2 | C20 | c3 | D02 | d1 | E01 |
| 2-1-m1E01-1-0427 | 701.232 | OH | a1 | B01 | b2 | C24 | c3 | D02 | d1 | E01 |
| 2-1-m1E01-1-0428 | 701.246 | OH | a1 | B09 | b2 | C02 | c1 | D01 | d1 | E01 |
| 2-1-m1E01-1-0429 | 701.283 | OH | a1 | B05 | b1 | C09 | c1 | D01 | d1 | E01 |
| 2-1-m1E01-1-0430 | 701.302 | OH | a1 | B07 | b2 | C07 | c1 | D01 | d1 | E01 |
| 2-1-m1E01-1-0431 | 702.162 | OH | a2 | B14 | b2 | C06 | c2 | D02 | d1 | E01 |
| 2-1-m1E01-1-0432 | 702.226 | OH | a2 | B18 | b1 | C10 | c2 | D02 | d1 | E01 |
| 2-1-m1E01-1-0433 | 702.226 | OH | a2 | B11 | b1 | C13 | c2 | D02 | d1 | E01 |
| 2-1-m1E01-1-0434 | 702.227 | OH | a1 | B02 | b2 | C24 | c3 | D02 | d1 | E01 |
| 2-1-m1E01-1-0435 | 702.227 | OH | a1 | B04 | b2 | C28 | c3 | D02 | d1 | E01 |
| 2-1-m1E01-1-0436 | 702.241 | OH | a1 | B09 | b2 | C03 | c1 | D01 | d1 | E01 |
| 2-1-m1E01-1-0437 | 703.177 | OH | a1 | B01 | b2 | C06 | c2 | D02 | d1 | E01 |
| 2-1-m1E01-1-0438 | 703.213 | OH | a1 | B05 | b3 | C06 | c1 | D01 | d1 | E01 |
| 2-1-m1E01-1-0439 | 703.221 | OH | a2 | B12 | b1 | C13 | c2 | D02 | d1 | E01 |
| 2-1-m1E01-1-0440 | 703.262 | OH | a1 | B06 | b1 | C09 | c1 | D01 | d1 | E01 |
| 2-1-m1E01-1-0441 | 704.161 | OH | a2 | B11 | b3 | C15 | c2 | D02 | d1 | E01 |
| 2-1-m1E01-1-0442 | 704.172 | OH | a1 | B02 | b2 | C06 | c2 | D02 | d1 | E01 |
| 2-1-m1E01-1-0443 | 704.175 | OH | a2 | B17 | b2 | C01 | c2 | D02 | d1 | E01 |
| 2-1-m1E01-1-0444 | 704.192 | OH | a2 | B20 | b2 | C06 | c1 | D01 | d1 | E01 |
| 2-1-m1E01-1-0445 | 704.209 | OH | a1 | B05 | b1 | C12 | c2 | D02 | d1 | E01 |
| 2-1-m1E01-1-0446 | 704.230 | OH | a2 | B11 | b3 | C16 | c1 | D01 | d1 | E01 |
| 2-1-m1E01-1-0447 | 704.261 | OH | a2 | B18 | b1 | C09 | c1 | D01 | d1 | E01 |
| 2-1-m1E01-1-0448 | 704.303 | OH | a2 | B13 | b1 | C29 | c3 | D02 | d1 | E01 |
| 2-1-m1E01-1-0449 | 704.303 | OH | a2 | B15 | b1 | C32 | c3 | D02 | d1 | E01 |
| 2-1-m1E01-1-0450 | 705.156 | OH | a2 | B12 | b3 | C15 | c2 | D02 | d1 | E01 |
| 2-1-m1E01-1-0451 | 705.190 | OH | a1 | B04 | b2 | C01 | c2 | D02 | d1 | E01 |
| 2-1-m1E01-1-0452 | 705.193 | OH | a1 | B06 | b3 | C06 | c1 | D01 | d1 | E01 |
| 2-1-m1E01-1-0453 | 705.207 | OH | a1 | B07 | b3 | C08 | c1 | D01 | d1 | E01 |

(continued)

| ID | Exact Mass | | a | B | b | C | c | D | d | E |
|---|---|---|---|---|---|---|---|---|---|---|
| 2-1-m1E01-1-0454 | 705.207 | OH | a1 | B07 | b1 | C08 | c2 | D02 | d1 | E01 |
| 2-1-m1E01-1-0455 | 705.226 | OH | a2 | B12 | b3 | C16 | c1 | D01 | d1 | E01 |
| 2-1-m1E01-1-0456 | 705.237 | OH | a1 | B08 | b2 | C05 | c1 | D01 | d1 | E01 |
| 2-1-m1E01-1-0457 | 706.188 | OH | a1 | B06 | b1 | C12 | c2 | D02 | d1 | E01 |
| 2-1-m1E01-1-0458 | 706.192 | OH | a2 | B18 | b3 | C06 | c1 | D01 | d1 | E01 |
| 2-1-m1E01-1-0459 | 706.192 | OH | a2 | B11 | b3 | C17 | c1 | D01 | d1 | E01 |
| 2-1-m1E01-1-0460 | 706.192 | OH | a2 | B11 | b1 | C14 | c2 | D02 | d1 | E01 |
| 2-1-m1E01-1-0461 | 706.202 | OH | a1 | B07 | b1 | C11 | c2 | D02 | d1 | E01 |
| 2-1-m1E01-1-0462 | 706.246 | OH | a2 | B15 | b2 | C02 | c2 | D02 | d1 | E01 |
| 2-1-m1E01-1-0463 | 706.252 | OH | a2 | B19 | b1 | C13 | c1 | D01 | d1 | E01 |
| 2-1-m1E01-1-0464 | 706.283 | OH | a2 | B15 | b2 | C22 | c3 | D02 | d1 | E01 |
| 2-1-m1E01-1-0465 | 706.283 | OH | a2 | B16 | b1 | C32 | c3 | D02 | d1 | E01 |
| 2-1-m1E01-1-0466 | 707.187 | OH | a2 | B12 | b3 | C17 | c1 | D01 | d1 | E01 |
| 2-1-m1E01-1-0467 | 707.187 | OH | a2 | B18 | b1 | C12 | c2 | D02 | d1 | E01 |
| 2-1-m1E01-1-0468 | 707.187 | OH | a2 | B12 | b1 | C14 | c2 | D02 | d1 | E01 |
| 2-1-m1E01-1-0469 | 707.197 | OH | a1 | B07 | b1 | C10 | c2 | D02 | d1 | E01 |
| 2-1-m1E01-1-0470 | 707.203 | OH | a1 | B09 | b2 | C06 | c1 | D01 | d1 | E01 |
| 2-1-m1E01-1-0471 | 707.220 | OH | a1 | B10 | b2 | C01 | c1 | D01 | d1 | E01 |
| 2-1-m1E01-1-0472 | 707.241 | OH | a2 | B15 | b2 | C03 | c2 | D02 | d1 | E01 |
| 2-1-m1E01-1-0473 | 707.241 | OH | a2 | B13 | b2 | C05 | c2 | D02 | d1 | E01 |
| 2-1-m1E01-1-0474 | 708.187 | OH | a2 | B19 | b3 | C15 | c1 | D01 | d1 | E01 |
| 2-1-m1E01-1-0475 | 708.217 | OH | a1 | B08 | b2 | C04 | c1 | D01 | d1 | E01 |
| 2-1-m1E01-1-0476 | 708.225 | OH | a2 | B16 | b2 | C02 | c2 | D02 | d1 | E01 |
| 2-1-m1E01-1-0477 | 708.262 | OH | a2 | B16 | b2 | C22 | c3 | D02 | d1 | E01 |
| 2-1-m1E01-1-0478 | 708.262 | OH | a2 | B13 | b2 | C26 | c3 | D02 | d1 | E01 |
| 2-1-m1E01-1-0479 | 708.278 | OH | a2 | B14 | b1 | C29 | c3 | D02 | d1 | E01 |
| 2-1-m1E01-1-0480 | 709.221 | OH | a2 | B16 | b2 | C03 | c2 | D02 | d1 | E01 |
| 2-1-m1E01-1-0481 | 709.232 | OH | a1 | B07 | b1 | C09 | c1 | D01 | d1 | E01 |
| 2-1-m1E01-1-0482 | 709.293 | OH | a1 | B05 | b1 | C30 | c3 | D02 | d1 | E01 |
| 2-1-m1E01-1-0483 | 709.293 | OH | a1 | B01 | b1 | C29 | c3 | D02 | d1 | E01 |
| 2-1-m1E01-1-0484 | 709.293 | OH | a1 | B03 | b1 | C32 | c3 | D02 | d1 | E01 |
| 2-1-m1E01-1-0485 | 710.217 | OH | a2 | B19 | b1 | C14 | c1 | D01 | d1 | E01 |
| 2-1-m1E01-1-0486 | 710.221 | OH | a2 | B13 | b2 | C04 | c2 | D02 | d1 | E01 |
| 2-1-m1E01-1-0487 | 710.239 | OH | a2 | B19 | b2 | C33 | c3 | D02 | d1 | E01 |
| 2-1-m1E01-1-0488 | 710.257 | OH | a2 | B15 | b2 | C24 | c3 | D02 | d1 | E01 |
| 2-1-m1E01-1-0489 | 710.277 | OH | a2 | B11 | b2 | C07 | c2 | D02 | d1 | E01 |
| 2-1-m1E01-1-0490 | 710.289 | OH | a1 | B02 | b1 | C29 | c3 | D02 | d1 | E01 |
| 2-1-m1E01-1-0491 | 711.163 | OH | a1 | B07 | b3 | C06 | c1 | D01 | d1 | E01 |

(continued)

| ID | Exact Mass | | a | B | b | C | c | D | d | E |
|---|---|---|---|---|---|---|---|---|---|---|
| 2-1-m1E01-1-0492 | 711.216 | OH | a2 | B14 | b2 | C05 | c2 | D02 | d1 | E01 |
| 2-1-m1E01-1-0493 | 711.236 | OH | a1 | B03 | b2 | C02 | c2 | D02 | d1 | E01 |
| 2-1-m1E01-1-0494 | 711.273 | OH | a2 | B12 | b2 | C07 | c2 | D02 | d1 | E01 |
| 2-1-m1E01-1-0495 | 711.273 | OH | a1 | B05 | b2 | C20 | c3 | D02 | d1 | E01 |
| 2-1-m1E01-1-0496 | 711.273 | OH | a1 | B03 | b2 | C22 | c3 | D02 | d1 | E01 |
| 2-1-m1E01-1-0497 | 711.273 | OH | a1 | B06 | b1 | C30 | c3 | D02 | d1 | E01 |
| 2-1-m1E01-1-0498 | 712.159 | OH | a1 | B07 | b1 | C12 | c2 | D02 | d1 | E01 |
| 2-1-m1E01-1-0499 | 712.203 | OH | a2 | B15 | b2 | C06 | c2 | D02 | d1 | E01 |
| 2-1-m1E01-1-0500 | 712.232 | OH | a1 | B03 | b2 | C03 | c2 | D02 | d1 | E01 |
| 2-1-m1E01-1-0501 | 712.232 | OH | a1 | B01 | b2 | C05 | c2 | D02 | d1 | E01 |
| 2-1-m1E01-1-0502 | 712.237 | OH | a2 | B16 | b2 | C24 | c3 | D02 | d1 | E01 |
| 2-1-m1E01-1-0503 | 712.237 | OH | a2 | B14 | b2 | C26 | c3 | D02 | d1 | E01 |
| 2-1-m1E01-1-0504 | 712.253 | OH | a2 | B17 | b1 | C32 | c3 | D02 | d1 | E01 |
| 2-1-m1E01-1-0505 | 712.268 | OH | a1 | B05 | b2 | C28 | c3 | D02 | d1 | E01 |
| 2-1-m1E01-1-0506 | 712.272 | OH | a2 | B18 | b1 | C30 | c3 | D02 | d1 | E01 |
| 2-1-m1E01-1-0507 | 713.227 | OH | a1 | B02 | b2 | C05 | c2 | D02 | d1 | E01 |
| 2-1-m1E01-1-0508 | 713.246 | OH | a2 | B20 | b2 | C05 | c1 | D01 | d1 | E01 |
| 2-1-m1E01-1-0509 | 713.252 | OH | a1 | B06 | b2 | C20 | c3 | D02 | d1 | E01 |
| 2-1-m1E01-1-0510 | 713.252 | OH | a1 | B01 | b2 | C26 | c3 | D02 | d1 | E01 |
| 2-1-m1E01-1-0511 | 713.268 | OH | a1 | B04 | b1 | C32 | c3 | D02 | d1 | E01 |
| 2-1-m1E01-1-0512 | 714.182 | OH | a2 | B16 | b2 | C06 | c2 | D02 | d1 | E01 |
| 2-1-m1E01-1-0513 | 714.182 | OH | a2 | B11 | b3 | C08 | c2 | D02 | d1 | E01 |
| 2-1-m1E01-1-0514 | 714.196 | OH | a2 | B17 | b2 | C02 | c2 | D02 | d1 | E01 |
| 2-1-m1E01-1-0515 | 714.196 | OH | a2 | B14 | b2 | C04 | c2 | D02 | d1 | E01 |
| 2-1-m1E01-1-0516 | 714.232 | OH | a2 | B17 | b2 | C22 | c3 | D02 | d1 | E01 |
| 2-1-m1E01-1-0517 | 714.237 | OH | a1 | B08 | b1 | C13 | c1 | D01 | d1 | E01 |
| 2-1-m1E01-1-0518 | 714.247 | OH | a1 | B02 | b2 | C26 | c3 | D02 | d1 | E01 |
| 2-1-m1E01-1-0519 | 714.247 | OH | a1 | B06 | b2 | C28 | c3 | D02 | d1 | E01 |
| 2-1-m1E01-1-0520 | 714.251 | OH | a2 | B18 | b2 | C20 | c3 | D02 | d1 | E01 |
| 2-1-m1E01-1-0521 | 714.303 | OH | a2 | B19 | b2 | C07 | c1 | D01 | d1 | E01 |
| 2-1-m1E01-1-0522 | 715.177 | OH | a2 | B12 | b3 | C08 | c2 | D02 | d1 | E01 |
| 2-1-m1E01-1-0523 | 715.191 | OH | a2 | B17 | b2 | C03 | c2 | D02 | d1 | E01 |
| 2-1-m1E01-1-0524 | 715.211 | OH | a1 | B04 | b2 | C02 | c2 | D02 | d1 | E01 |
| 2-1-m1E01-1-0525 | 715.211 | OH | a1 | B01 | b2 | C04 | c2 | D02 | d1 | E01 |
| 2-1-m1E01-1-0526 | 715.231 | OH | a1 | B05 | b2 | C01 | c2 | D02 | d1 | E01 |
| 2-1-m1E01-1-0527 | 715.246 | OH | a2 | B18 | b2 | C28 | c3 | D02 | d1 | E01 |
| 2-1-m1E01-1-0528 | 715.248 | OH | a1 | B04 | b2 | C22 | c3 | D02 | d1 | E01 |
| 2-1-m1E01-1-0529 | 715.248 | OH | a1 | B03 | b2 | C24 | c3 | D02 | d1 | E01 |

(continued)

| ID | Exact Mass | | a | B | b | C | c | D | d | E |
|---|---|---|---|---|---|---|---|---|---|---|
| 2-1-m1E01-1-0530 | 716.172 | OH | a1 | B08 | b3 | C15 | c1 | D01 | d1 | E01 |
| 2-1-m1E01-1-0531 | 716.206 | OH | a1 | B02 | b2 | C04 | c2 | D02 | d1 | E01 |
| 2-1-m1E01-1-0532 | 716.206 | OH | a1 | B04 | b2 | C03 | c2 | D02 | d1 | E01 |
| 2-1-m1E01-1-0533 | 716.226 | OH | a2 | B20 | b2 | C04 | c1 | D01 | d1 | E01 |
| 2-1-m1E01-1-0534 | 716.242 | OH | a2 | B13 | b1 | C13 | c2 | D02 | d1 | E01 |
| 2-1-m1E01-1-0535 | 716.257 | OH | a1 | B09 | b2 | C05 | c1 | D01 | d1 | E01 |
| 2-1-m1E01-1-0536 | 717.193 | OH | a1 | B03 | b2 | C06 | c2 | D02 | d1 | E01 |
| 2-1-m1E01-1-0537 | 717.210 | OH | a1 | B06 | b2 | C01 | c2 | D02 | d1 | E01 |
| 2-1-m1E01-1-0538 | 717.241 | OH | a1 | B10 | b2 | C02 | c1 | D01 | d1 | E01 |
| 2-1-m1E01-1-0539 | 717.243 | OH | a1 | B07 | b1 | C30 | c3 | D02 | d1 | E01 |
| 2-1-m1E01-1-0540 | 718.177 | OH | a2 | B13 | b3 | C15 | c2 | D02 | d1 | E01 |
| 2-1-m1E01-1-0541 | 718.203 | OH | a1 | B08 | b1 | C14 | c1 | D01 | d1 | E01 |
| 2-1-m1E01-1-0542 | 718.207 | OH | a2 | B19 | b3 | C08 | c1 | D01 | d1 | E01 |
| 2-1-m1E01-1-0543 | 718.207 | OH | a2 | B19 | b1 | C08 | c2 | D02 | d1 | E01 |
| 2-1-m1E01-1-0544 | 718.207 | OH | a2 | B11 | b1 | C09 | c2 | D02 | d1 | E01 |
| 2-1-m1E01-1-0545 | 718.207 | OH | a2 | B17 | b2 | C24 | c3 | D02 | d1 | E01 |
| 2-1-m1E01-1-0546 | 718.210 | OH | a2 | B18 | b2 | C01 | c2 | D02 | d1 | E01 |
| 2-1-m1E01-1-0547 | 718.224 | OH | a1 | B08 | b2 | C33 | c3 | D02 | d1 | E01 |
| 2-1-m1E01-1-0548 | 718.236 | OH | a1 | B10 | b2 | C03 | c1 | D01 | d1 | E01 |
| 2-1-m1E01-1-0549 | 718.246 | OH | a2 | B13 | b3 | C16 | c1 | D01 | d1 | E01 |
| 2-1-m1E01-1-0550 | 718.283 | OH | a2 | B11 | b1 | C31 | c3 | D02 | d1 | E01 |
| 2-1-m1E01-1-0551 | 718.319 | OH | a2 | B15 | b1 | C29 | c3 | D02 | d1 | E01 |
| 2-1-m1E01-1-0552 | 719.203 | OH | a2 | B12 | b1 | C09 | c2 | D02 | d1 | E01 |
| 2-1-m1E01-1-0553 | 719.203 | OH | a2 | B19 | b1 | C11 | c2 | D02 | d1 | E01 |
| 2-1-m1E01-1-0554 | 719.223 | OH | a1 | B07 | b2 | C20 | c3 | D02 | d1 | E01 |
| 2-1-m1E01-1-0555 | 719.223 | OH | a1 | B04 | b2 | C24 | c3 | D02 | d1 | E01 |
| 2-1-m1E01-1-0556 | 719.237 | OH | a1 | B09 | b2 | C04 | c1 | D01 | d1 | E01 |
| 2-1-m1E01-1-0557 | 719.278 | OH | a2 | B12 | b1 | C31 | c3 | D02 | d1 | E01 |
| 2-1-m1E01-1-0558 | 720.138 | OH | a2 | B11 | b3 | C06 | c2 | D02 | d1 | E01 |
| 2-1-m1E01-1-0559 | 720.152 | OH | a2 | B17 | b2 | C06 | c2 | D02 | d1 | E01 |
| 2-1-m1E01-1-0560 | 720.198 | OH | a2 | B19 | b1 | C10 | c2 | D02 | d1 | E01 |
| 2-1-m1E01-1-0561 | 720.208 | OH | a2 | B13 | b3 | C17 | c1 | D01 | d1 | E01 |
| 2-1-m1E01-1-0562 | 720.208 | OH | a2 | B13 | b1 | C14 | c2 | D02 | d1 | E01 |
| 2-1-m1E01-1-0563 | 720.217 | OH | a2 | B14 | b1 | C13 | c2 | D02 | d1 | E01 |
| 2-1-m1E01-1-0564 | 720.218 | OH | a1 | B07 | b2 | C28 | c3 | D02 | d1 | E01 |
| 2-1-m1E01-1-0565 | 720.298 | OH | a2 | B16 | b1 | C29 | c3 | D02 | d1 | E01 |
| 2-1-m1E01-1-0566 | 721.133 | OH | a2 | B12 | b3 | C06 | c2 | D02 | d1 | E01 |
| 2-1-m1E01-1-0567 | 721.168 | OH | a1 | B04 | b2 | C06 | c2 | D02 | d1 | E01 |

(continued)

| ID | Exact Mass | | a | B | b | C | c | D | d | E |
|---|---|---|---|---|---|---|---|---|---|---|
| 2-1-m1E01-1-0568 | 721.203 | OH | a2 | B11 | b3 | C18 | c1 | D01 | d1 | E01 |
| 2-1-m1E01-1-0569 | 721.232 | OH | a1 | B01 | b1 | C13 | c2 | D02 | d1 | E01 |
| 2-1-m1E01-1-0570 | 721.257 | OH | a2 | B15 | b2 | C05 | c2 | D02 | d1 | E01 |
| 2-1-m1E01-1-0571 | 722.152 | OH | a2 | B14 | b3 | C15 | c2 | D02 | d1 | E01 |
| 2-1-m1E01-1-0572 | 722.198 | OH | a2 | B12 | b3 | C18 | c1 | D01 | d1 | E01 |
| 2-1-m1E01-1-0573 | 722.221 | OH | a2 | B14 | b3 | C16 | c1 | D01 | d1 | E01 |
| 2-1-m1E01-1-0574 | 722.227 | OH | a1 | B02 | b1 | C13 | c2 | D02 | d1 | E01 |
| 2-1-m1E01-1-0575 | 722.233 | OH | a2 | B19 | b1 | C09 | c1 | D01 | d1 | E01 |
| 2-1-m1E01-1-0576 | 722.246 | OH | a2 | B20 | b1 | C13 | c1 | D01 | d1 | E01 |
| 2-1-m1E01-1-0577 | 722.277 | OH | a2 | B15 | b2 | C26 | c3 | D02 | d1 | E01 |
| 2-1-m1E01-1-0578 | 722.289 | OH | a1 | B08 | b2 | C07 | c1 | D01 | d1 | E01 |
| 2-1-m1E01-1-0579 | 723.167 | OH | a1 | B01 | b3 | C15 | c2 | D02 | d1 | E01 |
| 2-1-m1E01-1-0580 | 723.181 | OH | a1 | B07 | b2 | C01 | c2 | D02 | d1 | E01 |
| 2-1-m1E01-1-0581 | 723.197 | OH | a1 | B10 | b2 | C06 | c1 | D01 | d1 | E01 |
| 2-1-m1E01-1-0582 | 723.236 | OH | a1 | B01 | b3 | C16 | c1 | D01 | d1 | E01 |
| 2-1-m1E01-1-0583 | 723.236 | OH | a2 | B16 | b2 | C05 | c2 | D02 | d1 | E01 |
| 2-1-m1E01-1-0584 | 723.309 | OH | a1 | B03 | b1 | C29 | c3 | D02 | d1 | E01 |
| 2-1-m1E01-1-0585 | 723.309 | OH | a1 | B05 | b1 | C32 | c3 | D02 | d1 | E01 |
| 2-1-m1E01-1-0586 | 724.162 | OH | a1 | B02 | b3 | C15 | c2 | D02 | d1 | E01 |
| 2-1-m1E01-1-0587 | 724.164 | OH | a2 | B19 | b3 | C06 | c1 | D01 | d1 | E01 |
| 2-1-m1E01-1-0588 | 724.181 | OH | a2 | B20 | b3 | C15 | c1 | D01 | d1 | E01 |
| 2-1-m1E01-1-0589 | 724.183 | OH | a2 | B14 | b3 | C17 | c1 | D01 | d1 | E01 |
| 2-1-m1E01-1-0590 | 724.183 | OH | a2 | B14 | b1 | C14 | c2 | D02 | d1 | E01 |
| 2-1-m1E01-1-0591 | 724.232 | OH | a1 | B02 | b3 | C16 | c1 | D01 | d1 | E01 |
| 2-1-m1E01-1-0592 | 724.237 | OH | a2 | B15 | b2 | C04 | c2 | D02 | d1 | E01 |
| 2-1-m1E01-1-0593 | 724.257 | OH | a2 | B16 | b2 | C26 | c3 | D02 | d1 | E01 |
| 2-1-m1E01-1-0594 | 724.293 | OH | a2 | B13 | b2 | C07 | c2 | D02 | d1 | E01 |
| 2-1-m1E01-1-0595 | 725.159 | OH | a2 | B19 | b1 | C12 | c2 | D02 | d1 | E01 |
| 2-1-m1E01-1-0596 | 725.198 | OH | a1 | B01 | b3 | C17 | c1 | D01 | d1 | E01 |
| 2-1-m1E01-1-0597 | 725.198 | OH | a1 | B01 | b1 | C14 | c2 | D02 | d1 | E01 |
| 2-1-m1E01-1-0598 | 725.252 | OH | a1 | B05 | b2 | C02 | c2 | D02 | d1 | E01 |
| 2-1-m1E01-1-0599 | 725.257 | OH | a1 | B09 | b1 | C13 | c1 | D01 | d1 | E01 |
| 2-1-m1E01-1-0600 | 725.288 | OH | a1 | B05 | b2 | C22 | c3 | D02 | d1 | E01 |
| 2-1-m1E01-1-0601 | 725.288 | OH | a1 | B06 | b1 | C32 | c3 | D02 | d1 | E01 |
| 2-1-m1E01-1-0602 | 726.193 | OH | a1 | B02 | b3 | C17 | c1 | D01 | d1 | E01 |
| 2-1-m1E01-1-0603 | 726.193 | OH | a1 | B08 | b3 | C08 | c1 | D01 | d1 | E01 |
| 2-1-m1E01-1-0604 | 726.193 | OH | a1 | B08 | b1 | C08 | c2 | D02 | d1 | E01 |
| 2-1-m1E01-1-0605 | 726.193 | OH | a1 | B02 | b1 | C14 | c2 | D02 | d1 | E01 |

(continued)

| ID | Exact Mass | | a | B | b | C | c | D | d | E |
|---|---|---|---|---|---|---|---|---|---|---|
| 2-1-m1E01-1-0606 | 726.212 | OH | a2 | B20 | b1 | C14 | c1 | D01 | d1 | E01 |
| 2-1-m1E01-1-0607 | 726.216 | OH | a2 | B16 | b2 | C04 | c2 | D02 | d1 | E01 |
| 2-1-m1E01-1-0608 | 726.234 | OH | a2 | B20 | b2 | C33 | c3 | D02 | d1 | E01 |
| 2-1-m1E01-1-0609 | 726.247 | OH | a1 | B05 | b2 | C03 | c2 | D02 | d1 | E01 |
| 2-1-m1E01-1-0610 | 726.247 | OH | a1 | B03 | b2 | C05 | c2 | D02 | d1 | E01 |
| 2-1-m1E01-1-0611 | 726.269 | OH | a2 | B17 | b1 | C29 | c3 | D02 | d1 | E01 |
| 2-1-m1E01-1-0612 | 726.288 | OH | a2 | B18 | b1 | C32 | c3 | D02 | d1 | E01 |
| 2-1-m1E01-1-0613 | 727.188 | OH | a1 | B08 | b1 | C11 | c2 | D02 | d1 | E01 |
| 2-1-m1E01-1-0614 | 727.192 | OH | a1 | B09 | b3 | C15 | c1 | D01 | d1 | E01 |
| 2-1-m1E01-1-0615 | 727.231 | OH | a1 | B06 | b2 | C02 | c2 | D02 | d1 | E01 |
| 2-1-m1E01-1-0616 | 727.268 | OH | a1 | B06 | b2 | C22 | c3 | D02 | d1 | E01 |
| 2-1-m1E01-1-0617 | 727.268 | OH | a1 | B03 | b2 | C26 | c3 | D02 | d1 | E01 |
| 2-1-m1E01-1-0618 | 727.284 | OH | a1 | B04 | b1 | C29 | c3 | D02 | d1 | E01 |
| 2-1-m1E01-1-0619 | 728.184 | OH | a1 | B08 | b1 | C10 | c2 | D02 | d1 | E01 |
| 2-1-m1E01-1-0620 | 728.197 | OH | a2 | B13 | b3 | C08 | c2 | D02 | d1 | E01 |
| 2-1-m1E01-1-0621 | 728.226 | OH | a1 | B06 | b2 | C03 | c2 | D02 | d1 | E01 |
| 2-1-m1E01-1-0622 | 728.230 | OH | a2 | B18 | b2 | C02 | c2 | D02 | d1 | E01 |
| 2-1-m1E01-1-0623 | 728.267 | OH | a2 | B18 | b2 | C22 | c3 | D02 | d1 | E01 |
| 2-1-m1E01-1-0624 | 728.268 | OH | a2 | B14 | b2 | C07 | c2 | D02 | d1 | E01 |
| 2-1-m1E01-1-0625 | 729.207 | OH | a2 | B17 | b2 | C05 | c2 | D02 | d1 | E01 |
| 2-1-m1E01-1-0626 | 729.223 | OH | a1 | B09 | b1 | C14 | c1 | D01 | d1 | E01 |
| 2-1-m1E01-1-0627 | 729.226 | OH | a2 | B18 | b2 | C03 | c2 | D02 | d1 | E01 |
| 2-1-m1E01-1-0628 | 729.227 | OH | a1 | B03 | b2 | C04 | c2 | D02 | d1 | E01 |
| 2-1-m1E01-1-0629 | 729.244 | OH | a1 | B09 | b2 | C33 | c3 | D02 | d1 | E01 |
| 2-1-m1E01-1-0630 | 729.263 | OH | a1 | B05 | b2 | C24 | c3 | D02 | d1 | E01 |
| 2-1-m1E01-1-0631 | 729.283 | OH | a1 | B01 | b2 | C07 | c2 | D02 | d1 | E01 |
| 2-1-m1E01-1-0632 | 730.219 | OH | a1 | B08 | b1 | C09 | c1 | D01 | d1 | E01 |
| 2-1-m1E01-1-0633 | 730.222 | OH | a1 | B04 | b2 | C05 | c2 | D02 | d1 | E01 |
| 2-1-m1E01-1-0634 | 730.227 | OH | a2 | B17 | b2 | C26 | c3 | D02 | d1 | E01 |
| 2-1-m1E01-1-0635 | 730.244 | OH | a2 | B19 | b1 | C30 | c3 | D02 | d1 | E01 |
| 2-1-m1E01-1-0636 | 730.257 | OH | a2 | B15 | b1 | C13 | c2 | D02 | d1 | E01 |
| 2-1-m1E01-1-0637 | 730.278 | OH | a1 | B02 | b2 | C07 | c2 | D02 | d1 | E01 |
| 2-1-m1E01-1-0638 | 730.298 | OH | a2 | B20 | b2 | C07 | c1 | D01 | d1 | E01 |
| 2-1-m1E01-1-0639 | 731.208 | OH | a1 | B05 | b2 | C06 | c2 | D02 | d1 | E01 |
| 2-1-m1E01-1-0640 | 731.243 | OH | a1 | B06 | b2 | C24 | c3 | D02 | d1 | E01 |
| 2-1-m1E01-1-0641 | 731.243 | OH | a1 | B04 | b2 | C26 | c3 | D02 | d1 | E01 |
| 2-1-m1E01-1-0642 | 731.259 | OH | a1 | B07 | b1 | C32 | c3 | D02 | d1 | E01 |
| 2-1-m1E01-1-0643 | 732.149 | OH | a1 | B08 | b3 | C06 | c1 | D01 | d1 | E01 |

(continued)

| ID | Exact Mass | | a | B | b | C | c | D | d | E |
|---|---|---|---|---|---|---|---|---|---|---|
| 2-1-m1E01-1-0644 | 732.172 | OH | a2 | B14 | b3 | C08 | c2 | D02 | d1 | E01 |
| 2-1-m1E01-1-0645 | 732.187 | OH | a2 | B17 | b2 | C04 | c2 | D02 | d1 | E01 |
| 2-1-m1E01-1-0646 | 732.192 | OH | a2 | B15 | b3 | C15 | c2 | D02 | d1 | E01 |
| 2-1-m1E01-1-0647 | 732.223 | OH | a2 | B13 | b1 | C09 | c2 | D02 | d1 | E01 |
| 2-1-m1E01-1-0648 | 732.223 | OH | a2 | B19 | b2 | C20 | c3 | D02 | d1 | E01 |
| 2-1-m1E01-1-0649 | 732.237 | OH | a2 | B16 | b1 | C13 | c2 | D02 | d1 | E01 |
| 2-1-m1E01-1-0650 | 732.242 | OH | a2 | B18 | b2 | C24 | c3 | D02 | d1 | E01 |
| 2-1-m1E01-1-0651 | 732.252 | OH | a1 | B10 | b2 | C05 | c1 | D01 | d1 | E01 |
| 2-1-m1E01-1-0652 | 732.262 | OH | a2 | B15 | b3 | C16 | c1 | D01 | d1 | E01 |
| 2-1-m1E01-1-0653 | 732.298 | OH | a2 | B13 | b1 | C31 | c3 | D02 | d1 | E01 |
| 2-1-m1E01-1-0654 | 733.145 | OH | a1 | B08 | b1 | C12 | c2 | D02 | d1 | E01 |
| 2-1-m1E01-1-0655 | 733.188 | OH | a1 | B06 | b2 | C06 | c2 | D02 | d1 | E01 |
| 2-1-m1E01-1-0656 | 733.188 | OH | a1 | B01 | b3 | C08 | c2 | D02 | d1 | E01 |
| 2-1-m1E01-1-0657 | 733.202 | OH | a1 | B07 | b2 | C02 | c2 | D02 | d1 | E01 |
| 2-1-m1E01-1-0658 | 733.202 | OH | a1 | B04 | b2 | C04 | c2 | D02 | d1 | E01 |
| 2-1-m1E01-1-0659 | 733.218 | OH | a2 | B19 | b2 | C28 | c3 | D02 | d1 | E01 |
| 2-1-m1E01-1-0660 | 733.238 | OH | a1 | B07 | b2 | C22 | c3 | D02 | d1 | E01 |
| 2-1-m1E01-1-0661 | 733.309 | OH | a1 | B09 | b2 | C07 | c1 | D01 | d1 | E01 |
| 2-1-m1E01-1-0662 | 734.154 | OH | a2 | B13 | b3 | C06 | c2 | D02 | d1 | E01 |
| 2-1-m1E01-1-0663 | 734.172 | OH | a2 | B16 | b3 | C15 | c2 | D02 | d1 | E01 |
| 2-1-m1E01-1-0664 | 734.183 | OH | a1 | B02 | b3 | C08 | c2 | D02 | d1 | E01 |
| 2-1-m1E01-1-0665 | 734.187 | OH | a2 | B18 | b2 | C06 | c2 | D02 | d1 | E01 |
| 2-1-m1E01-1-0666 | 734.197 | OH | a1 | B07 | b2 | C03 | c2 | D02 | d1 | E01 |
| 2-1-m1E01-1-0667 | 734.202 | OH | a2 | B20 | b3 | C08 | c1 | D01 | d1 | E01 |
| 2-1-m1E01-1-0668 | 734.202 | OH | a2 | B20 | b1 | C08 | c2 | D02 | d1 | E01 |
| 2-1-m1E01-1-0669 | 734.223 | OH | a2 | B15 | b3 | C17 | c1 | D01 | d1 | E01 |
| 2-1-m1E01-1-0670 | 734.223 | OH | a2 | B15 | b1 | C14 | c2 | D02 | d1 | E01 |
| 2-1-m1E01-1-0671 | 734.241 | OH | a2 | B16 | b3 | C16 | c1 | D01 | d1 | E01 |
| 2-1-m1E01-1-0672 | 735.197 | OH | a2 | B20 | b1 | C11 | c2 | D02 | d1 | E01 |
| 2-1-m1E01-1-0673 | 735.219 | OH | a2 | B13 | b3 | C18 | c1 | D01 | d1 | E01 |
| 2-1-m1E01-1-0674 | 735.232 | OH | a1 | B10 | b2 | C04 | c1 | D01 | d1 | E01 |
| 2-1-m1E01-1-0675 | 735.248 | OH | a1 | B03 | b1 | C13 | c2 | D02 | d1 | E01 |
| 2-1-m1E01-1-0676 | 736.181 | OH | a2 | B19 | b2 | C01 | c2 | D02 | d1 | E01 |
| 2-1-m1E01-1-0677 | 736.193 | OH | a2 | B20 | b1 | C10 | c2 | D02 | d1 | E01 |
| 2-1-m1E01-1-0678 | 736.198 | OH | a2 | B14 | b1 | C09 | c2 | D02 | d1 | E01 |
| 2-1-m1E01-1-0679 | 736.203 | OH | a2 | B16 | b3 | C17 | c1 | D01 | d1 | E01 |
| 2-1-m1E01-1-0680 | 736.203 | OH | a2 | B16 | b1 | C14 | c2 | D02 | d1 | E01 |
| 2-1-m1E01-1-0681 | 736.273 | OH | a2 | B14 | b1 | C31 | c3 | D02 | d1 | E01 |

(continued)

| ID | Exact Mass | | a | B | b | C | c | D | d | E |
|---|---|---|---|---|---|---|---|---|---|---|
| 2-1-m1E01-1-0682 | 737.183 | OH | a1 | B03 | b3 | C15 | c2 | D02 | d1 | E01 |
| 2-1-m1E01-1-0683 | 737.213 | OH | a1 | B09 | b3 | C08 | c1 | D01 | d1 | E01 |
| 2-1-m1E01-1-0684 | 737.213 | OH | a1 | B09 | b1 | C08 | c2 | D02 | d1 | E01 |
| 2-1-m1E01-1-0685 | 737.213 | OH | a1 | B01 | b1 | C09 | c2 | D02 | d1 | E01 |
| 2-1-m1E01-1-0686 | 737.213 | OH | a1 | B07 | b2 | C24 | c3 | D02 | d1 | E01 |
| 2-1-m1E01-1-0687 | 737.252 | OH | a1 | B03 | b3 | C16 | c1 | D01 | d1 | E01 |
| 2-1-m1E01-1-0688 | 737.288 | OH | a1 | B01 | b1 | C31 | c3 | D02 | d1 | E01 |
| 2-1-m1E01-1-0689 | 737.325 | OH | a1 | B05 | b1 | C29 | c3 | D02 | d1 | E01 |
| 2-1-m1E01-1-0690 | 738.129 | OH | a2 | B14 | b3 | C06 | c2 | D02 | d1 | E01 |
| 2-1-m1E01-1-0691 | 738.207 | OH | a2 | B17 | b1 | C13 | c2 | D02 | d1 | E01 |
| 2-1-m1E01-1-0692 | 738.208 | OH | a1 | B02 | b1 | C09 | c2 | D02 | d1 | E01 |
| 2-1-m1E01-1-0693 | 738.208 | OH | a1 | B09 | b1 | C11 | c2 | D02 | d1 | E01 |
| 2-1-m1E01-1-0694 | 738.228 | OH | a2 | B20 | b1 | C09 | c1 | D01 | d1 | E01 |
| 2-1-m1E01-1-0695 | 738.229 | OH | a1 | B08 | b1 | C30 | c3 | D02 | d1 | E01 |
| 2-1-m1E01-1-0696 | 738.284 | OH | a1 | B02 | b1 | C31 | c3 | D02 | d1 | E01 |
| 2-1-m1E01-1-0697 | 738.309 | OH | a2 | B15 | b2 | C07 | c2 | D02 | d1 | E01 |
| 2-1-m1E01-1-0698 | 739.144 | OH | a1 | B01 | b3 | C06 | c2 | D02 | d1 | E01 |
| 2-1-m1E01-1-0699 | 739.158 | OH | a1 | B07 | b2 | C06 | c2 | D02 | d1 | E01 |
| 2-1-m1E01-1-0700 | 739.193 | OH | a2 | B14 | b3 | C18 | c1 | D01 | d1 | E01 |
| 2-1-m1E01-1-0701 | 739.204 | OH | a1 | B09 | b1 | C10 | c2 | D02 | d1 | E01 |
| 2-1-m1E01-1-0702 | 739.213 | OH | a1 | B03 | b3 | C17 | c1 | D01 | d1 | E01 |
| 2-1-m1E01-1-0703 | 739.213 | OH | a1 | B03 | b1 | C14 | c2 | D02 | d1 | E01 |
| 2-1-m1E01-1-0704 | 739.222 | OH | a1 | B04 | b1 | C13 | c2 | D02 | d1 | E01 |
| 2-1-m1E01-1-0705 | 739.304 | OH | a1 | B06 | b1 | C29 | c3 | D02 | d1 | E01 |
| 2-1-m1E01-1-0706 | 740.139 | OH | a1 | B02 | b3 | C06 | c2 | D02 | d1 | E01 |
| 2-1-m1E01-1-0707 | 740.142 | OH | a2 | B17 | b3 | C15 | c2 | D02 | d1 | E01 |
| 2-1-m1E01-1-0708 | 740.159 | OH | a2 | B20 | b3 | C06 | c1 | D01 | d1 | E01 |
| 2-1-m1E01-1-0709 | 740.209 | OH | a1 | B01 | b3 | C18 | c1 | D01 | d1 | E01 |
| 2-1-m1E01-1-0710 | 740.209 | OH | a1 | B08 | b2 | C20 | c3 | D02 | d1 | E01 |
| 2-1-m1E01-1-0711 | 740.212 | OH | a2 | B17 | b3 | C16 | c1 | D01 | d1 | E01 |
| 2-1-m1E01-1-0712 | 740.263 | OH | a1 | B05 | b2 | C05 | c2 | D02 | d1 | E01 |
| 2-1-m1E01-1-0713 | 740.288 | OH | a2 | B16 | b2 | C07 | c2 | D02 | d1 | E01 |
| 2-1-m1E01-1-0714 | 740.303 | OH | a2 | B18 | b1 | C29 | c3 | D02 | d1 | E01 |
| 2-1-m1E01-1-0715 | 741.154 | OH | a2 | B20 | b1 | C12 | c2 | D02 | d1 | E01 |
| 2-1-m1E01-1-0716 | 741.157 | OH | a1 | B04 | b3 | C15 | c2 | D02 | d1 | E01 |
| 2-1-m1E01-1-0717 | 741.204 | OH | a1 | B02 | b3 | C18 | c1 | D01 | d1 | E01 |
| 2-1-m1E01-1-0718 | 741.204 | OH | a1 | B08 | b2 | C28 | c3 | D02 | d1 | E01 |
| 2-1-m1E01-1-0719 | 741.227 | OH | a1 | B04 | b3 | C16 | c1 | D01 | d1 | E01 |

(continued)

| ID | Exact Mass | | a | B | b | C | c | D | d | E |
|---|---|---|---|---|---|---|---|---|---|---|
| 2-1-m1E01-1-0720 | 741.239 | OH | a1 | B09 | b1 | C09 | c1 | D01 | d1 | E01 |
| 2-1-m1E01-1-0721 | 741.252 | OH | a1 | B10 | b1 | C13 | c1 | D01 | d1 | E01 |
| 2-1-m1E01-1-0722 | 741.283 | OH | a1 | B05 | b2 | C26 | c3 | D02 | d1 | E01 |
| 2-1-m1E01-1-0723 | 742.173 | OH | a2 | B17 | b3 | C17 | c1 | D01 | d1 | E01 |
| 2-1-m1E01-1-0724 | 742.173 | OH | a2 | B17 | b1 | C14 | c2 | D02 | d1 | E01 |
| 2-1-m1E01-1-0725 | 742.213 | OH | a2 | B15 | b3 | C08 | c2 | D02 | d1 | E01 |
| 2-1-m1E01-1-0726 | 742.242 | OH | a1 | B06 | b2 | C05 | c2 | D02 | d1 | E01 |
| 2-1-m1E01-1-0727 | 743.170 | OH | a1 | B09 | b3 | C06 | c1 | D01 | d1 | E01 |
| 2-1-m1E01-1-0728 | 743.187 | OH | a1 | B10 | b3 | C15 | c1 | D01 | d1 | E01 |
| 2-1-m1E01-1-0729 | 743.188 | OH | a1 | B04 | b3 | C17 | c1 | D01 | d1 | E01 |
| 2-1-m1E01-1-0730 | 743.188 | OH | a1 | B04 | b1 | C14 | c2 | D02 | d1 | E01 |
| 2-1-m1E01-1-0731 | 743.241 | OH | a2 | B18 | b2 | C05 | c2 | D02 | d1 | E01 |
| 2-1-m1E01-1-0732 | 743.243 | OH | a1 | B05 | b2 | C04 | c2 | D02 | d1 | E01 |
| 2-1-m1E01-1-0733 | 743.262 | OH | a1 | B06 | b2 | C26 | c3 | D02 | d1 | E01 |
| 2-1-m1E01-1-0734 | 743.299 | OH | a1 | B03 | b2 | C07 | c2 | D02 | d1 | E01 |
| 2-1-m1E01-1-0735 | 744.165 | OH | a1 | B09 | b1 | C12 | c2 | D02 | d1 | E01 |
| 2-1-m1E01-1-0736 | 744.167 | OH | a1 | B08 | b2 | C01 | c2 | D02 | d1 | E01 |
| 2-1-m1E01-1-0737 | 744.192 | OH | a2 | B16 | b3 | C08 | c2 | D02 | d1 | E01 |
| 2-1-m1E01-1-0738 | 744.259 | OH | a2 | B19 | b1 | C32 | c3 | D02 | d1 | E01 |
| 2-1-m1E01-1-0739 | 744.262 | OH | a2 | B18 | b2 | C26 | c3 | D02 | d1 | E01 |
| 2-1-m1E01-1-0740 | 745.218 | OH | a1 | B10 | b1 | C14 | c1 | D01 | d1 | E01 |
| 2-1-m1E01-1-0741 | 745.222 | OH | a1 | B06 | b2 | C04 | c2 | D02 | d1 | E01 |
| 2-1-m1E01-1-0742 | 745.239 | OH | a1 | B10 | b2 | C33 | c3 | D02 | d1 | E01 |
| 2-1-m1E01-1-0743 | 745.275 | OH | a1 | B07 | b1 | C29 | c3 | D02 | d1 | E01 |
| 2-1-m1E01-1-0744 | 746.202 | OH | a2 | B19 | b2 | C02 | c2 | D02 | d1 | E01 |
| 2-1-m1E01-1-0745 | 746.221 | OH | a2 | B18 | b2 | C04 | c2 | D02 | d1 | E01 |
| 2-1-m1E01-1-0746 | 746.239 | OH | a2 | B15 | b1 | C09 | c2 | D02 | d1 | E01 |
| 2-1-m1E01-1-0747 | 746.239 | OH | a2 | B19 | b2 | C22 | c3 | D02 | d1 | E01 |
| 2-1-m1E01-1-0748 | 746.239 | OH | a2 | B20 | b1 | C30 | c3 | D02 | d1 | E01 |
| 2-1-m1E01-1-0749 | 746.259 | OH | a2 | B17 | b2 | C07 | c2 | D02 | d1 | E01 |
| 2-1-m1E01-1-0750 | 746.314 | OH | a2 | B15 | b1 | C31 | c3 | D02 | d1 | E01 |
| 2-1-m1E01-1-0751 | 747.197 | OH | a2 | B19 | b2 | C03 | c2 | D02 | d1 | E01 |
| 2-1-m1E01-1-0752 | 747.203 | OH | a1 | B03 | b3 | C08 | c2 | D02 | d1 | E01 |
| 2-1-m1E01-1-0753 | 747.274 | OH | a1 | B04 | b2 | C07 | c2 | D02 | d1 | E01 |
| 2-1-m1E01-1-0754 | 748.170 | OH | a2 | B15 | b3 | C06 | c2 | D02 | d1 | E01 |
| 2-1-m1E01-1-0755 | 748.213 | OH | a1 | B07 | b2 | C05 | c2 | D02 | d1 | E01 |
| 2-1-m1E01-1-0756 | 748.218 | OH | a2 | B16 | b1 | C09 | c2 | D02 | d1 | E01 |
| 2-1-m1E01-1-0757 | 748.218 | OH | a2 | B20 | b2 | C20 | c3 | D02 | d1 | E01 |

(continued)

| ID | Exact Mass | | a | B | b | C | c | D | d | E |
|---|---|---|---|---|---|---|---|---|---|---|
| 2-1-m1E01-1-0758 | 748.293 | OH | a2 | B16 | b1 | C31 | c3 | D02 | d1 | E01 |
| 2-1-m1E01-1-0759 | 749.213 | OH | a2 | B20 | b2 | C28 | c3 | D02 | d1 | E01 |
| 2-1-m1E01-1-0760 | 749.233 | OH | a1 | B07 | b2 | C26 | c3 | D02 | d1 | E01 |
| 2-1-m1E01-1-0761 | 749.234 | OH | a2 | B15 | b3 | C18 | c1 | D01 | d1 | E01 |
| 2-1-m1E01-1-0762 | 749.249 | OH | a1 | B09 | b1 | C30 | c3 | D02 | d1 | E01 |
| 2-1-m1E01-1-0763 | 749.263 | OH | a1 | B05 | b1 | C13 | c2 | D02 | d1 | E01 |
| 2-1-m1E01-1-0764 | 749.304 | OH | a1 | B10 | b2 | C07 | c1 | D01 | d1 | E01 |
| 2-1-m1E01-1-0765 | 750.149 | OH | a2 | B16 | b3 | C06 | c2 | D02 | d1 | E01 |
| 2-1-m1E01-1-0766 | 750.163 | OH | a2 | B17 | b3 | C08 | c2 | D02 | d1 | E01 |
| 2-1-m1E01-1-0767 | 750.214 | OH | a2 | B19 | b2 | C24 | c3 | D02 | d1 | E01 |
| 2-1-m1E01-1-0768 | 751.178 | OH | a1 | B04 | b3 | C08 | c2 | D02 | d1 | E01 |
| 2-1-m1E01-1-0769 | 751.192 | OH | a1 | B07 | b2 | C04 | c2 | D02 | d1 | E01 |
| 2-1-m1E01-1-0770 | 751.198 | OH | a1 | B05 | b3 | C15 | c2 | D02 | d1 | E01 |
| 2-1-m1E01-1-0771 | 751.213 | OH | a2 | B16 | b3 | C18 | c1 | D01 | d1 | E01 |
| 2-1-m1E01-1-0772 | 751.229 | OH | a1 | B03 | b1 | C09 | c2 | D02 | d1 | E01 |
| 2-1-m1E01-1-0773 | 751.229 | OH | a1 | B09 | b2 | C20 | c3 | D02 | d1 | E01 |
| 2-1-m1E01-1-0774 | 751.242 | OH | a1 | B06 | b1 | C13 | c2 | D02 | d1 | E01 |
| 2-1-m1E01-1-0775 | 751.268 | OH | a1 | B05 | b3 | C16 | c1 | D01 | d1 | E01 |
| 2-1-m1E01-1-0776 | 751.304 | OH | a1 | B03 | b1 | C31 | c3 | D02 | d1 | E01 |
| 2-1-m1E01-1-0777 | 752.159 | OH | a2 | B19 | b2 | C06 | c2 | D02 | d1 | E01 |
| 2-1-m1E01-1-0778 | 752.176 | OH | a2 | B20 | b2 | C01 | c2 | D02 | d1 | E01 |
| 2-1-m1E01-1-0779 | 752.224 | OH | a1 | B09 | b2 | C28 | c3 | D02 | d1 | E01 |
| 2-1-m1E01-1-0780 | 752.242 | OH | a2 | B18 | b1 | C13 | c2 | D02 | d1 | E01 |
| 2-1-m1E01-1-0781 | 752.245 | OH | a1 | B08 | b1 | C32 | c3 | D02 | d1 | E01 |
| 2-1-m1E01-1-0782 | 753.160 | OH | a1 | B03 | b3 | C06 | c2 | D02 | d1 | E01 |
| 2-1-m1E01-1-0783 | 753.177 | OH | a1 | B06 | b3 | C15 | c2 | D02 | d1 | E01 |
| 2-1-m1E01-1-0784 | 753.208 | OH | a1 | B10 | b3 | C08 | c1 | D01 | d1 | E01 |
| 2-1-m1E01-1-0785 | 753.208 | OH | a1 | B10 | b1 | C08 | c2 | D02 | d1 | E01 |
| 2-1-m1E01-1-0786 | 753.229 | OH | a1 | B05 | b3 | C17 | c1 | D01 | d1 | E01 |
| 2-1-m1E01-1-0787 | 753.229 | OH | a1 | B05 | b1 | C14 | c2 | D02 | d1 | E01 |
| 2-1-m1E01-1-0788 | 753.247 | OH | a1 | B06 | b3 | C16 | c1 | D01 | d1 | E01 |
| 2-1-m1E01-1-0789 | 754.177 | OH | a2 | B18 | b3 | C15 | c2 | D02 | d1 | E01 |
| 2-1-m1E01-1-0790 | 754.188 | OH | a1 | B08 | b2 | C02 | c2 | D02 | d1 | E01 |
| 2-1-m1E01-1-0791 | 754.188 | OH | a2 | B17 | b1 | C09 | c2 | D02 | d1 | E01 |
| 2-1-m1E01-1-0792 | 754.203 | OH | a1 | B10 | b1 | C11 | c2 | D02 | d1 | E01 |
| 2-1-m1E01-1-0793 | 754.224 | OH | a1 | B03 | b3 | C18 | c1 | D01 | d1 | E01 |
| 2-1-m1E01-1-0794 | 754.224 | OH | a1 | B08 | b2 | C22 | c3 | D02 | d1 | E01 |
| 2-1-m1E01-1-0795 | 754.246 | OH | a2 | B18 | b3 | C16 | c1 | D01 | d1 | E01 |

(continued)

| ID | Exact Mass | | a | B | b | C | c | D | d | E |
|---|---|---|---|---|---|---|---|---|---|---|
| 2-1-m1E01-1-0796 | 754.264 | OH | a2 | B17 | b1 | C31 | c3 | D02 | d1 | E01 |
| 2-1-m1E01-1-0797 | 755.183 | OH | a1 | B08 | b2 | C03 | c2 | D02 | d1 | E01 |
| 2-1-m1E01-1-0798 | 755.187 | OH | a1 | B09 | b2 | C01 | c2 | D02 | d1 | E01 |
| 2-1-m1E01-1-0799 | 755.199 | OH | a1 | B10 | b1 | C10 | c2 | D02 | d1 | E01 |
| 2-1-m1E01-1-0800 | 755.204 | OH | a1 | B04 | b1 | C09 | c2 | D02 | d1 | E01 |
| 2-1-m1E01-1-0801 | 755.208 | OH | a1 | B06 | b3 | C17 | c1 | D01 | d1 | E01 |
| 2-1-m1E01-1-0802 | 755.208 | OH | a1 | B06 | b1 | C14 | c2 | D02 | d1 | E01 |
| 2-1-m1E01-1-0803 | 755.279 | OH | a1 | B04 | b1 | C31 | c3 | D02 | d1 | E01 |
| 2-1-m1E01-1-0804 | 756.119 | OH | a2 | B17 | b3 | C06 | c2 | D02 | d1 | E01 |
| 2-1-m1E01-1-0805 | 756.208 | OH | a2 | B18 | b3 | C17 | c1 | D01 | d1 | E01 |
| 2-1-m1E01-1-0806 | 756.208 | OH | a2 | B18 | b1 | C14 | c2 | D02 | d1 | E01 |
| 2-1-m1E01-1-0807 | 757.135 | OH | a1 | B04 | b3 | C06 | c2 | D02 | d1 | E01 |
| 2-1-m1E01-1-0808 | 757.184 | OH | a2 | B17 | b3 | C18 | c1 | D01 | d1 | E01 |
| 2-1-m1E01-1-0809 | 757.213 | OH | a1 | B07 | b1 | C13 | c2 | D02 | d1 | E01 |
| 2-1-m1E01-1-0810 | 757.234 | OH | a1 | B10 | b1 | C09 | c1 | D01 | d1 | E01 |
| 2-1-m1E01-1-0811 | 757.315 | OH | a1 | B05 | b2 | C07 | c2 | D02 | d1 | E01 |
| 2-1-m1E01-1-0812 | 758.199 | OH | a1 | B04 | b3 | C18 | c1 | D01 | d1 | E01 |
| 2-1-m1E01-1-0813 | 758.199 | OH | a1 | B08 | b2 | C24 | c3 | D02 | d1 | E01 |
| 2-1-m1E01-1-0814 | 758.275 | OH | a2 | B19 | b1 | C29 | c3 | D02 | d1 | E01 |
| 2-1-m1E01-1-0815 | 759.148 | OH | a1 | B07 | b3 | C15 | c2 | D02 | d1 | E01 |
| 2-1-m1E01-1-0816 | 759.164 | OH | a1 | B10 | b3 | C06 | c1 | D01 | d1 | E01 |
| 2-1-m1E01-1-0817 | 759.217 | OH | a1 | B07 | b3 | C16 | c1 | D01 | d1 | E01 |
| 2-1-m1E01-1-0818 | 759.294 | OH | a1 | B06 | b2 | C07 | c2 | D02 | d1 | E01 |
| 2-1-m1E01-1-0819 | 760.144 | OH | a1 | B08 | b2 | C06 | c2 | D02 | d1 | E01 |
| 2-1-m1E01-1-0820 | 760.160 | OH | a1 | B10 | b1 | C12 | c2 | D02 | d1 | E01 |
| 2-1-m1E01-1-0821 | 760.254 | OH | a2 | B20 | b1 | C32 | c3 | D02 | d1 | E01 |
| 2-1-m1E01-1-0822 | 760.293 | OH | a2 | B18 | b2 | C07 | c2 | D02 | d1 | E01 |
| 2-1-m1E01-1-0823 | 761.179 | OH | a1 | B07 | b3 | C17 | c1 | D01 | d1 | E01 |
| 2-1-m1E01-1-0824 | 761.179 | OH | a1 | B07 | b1 | C14 | c2 | D02 | d1 | E01 |
| 2-1-m1E01-1-0825 | 761.213 | OH | a2 | B19 | b2 | C05 | c2 | D02 | d1 | E01 |
| 2-1-m1E01-1-0826 | 761.219 | OH | a1 | B05 | b3 | C08 | c2 | D02 | d1 | E01 |
| 2-1-m1E01-1-0827 | 762.197 | OH | a2 | B20 | b2 | C02 | c2 | D02 | d1 | E01 |
| 2-1-m1E01-1-0828 | 762.234 | OH | a2 | B20 | b2 | C22 | c3 | D02 | d1 | E01 |
| 2-1-m1E01-1-0829 | 762.234 | OH | a2 | B19 | b2 | C26 | c3 | D02 | d1 | E01 |
| 2-1-m1E01-1-0830 | 763.192 | OH | a2 | B20 | b2 | C03 | c2 | D02 | d1 | E01 |
| 2-1-m1E01-1-0831 | 763.198 | OH | a1 | B06 | b3 | C08 | c2 | D02 | d1 | E01 |
| 2-1-m1E01-1-0832 | 763.265 | OH | a1 | B09 | b1 | C32 | c3 | D02 | d1 | E01 |
| 2-1-m1E01-1-0833 | 764.193 | OH | a2 | B19 | b2 | C04 | c2 | D02 | d1 | E01 |

(continued)

| ID | Exact Mass | | a | B | b | C | c | D | d | E |
|---|---|---|---|---|---|---|---|---|---|---|
| 2-1-m1E01-1-0834 | 764.197 | OH | a2 | B18 | b3 | C08 | c2 | D02 | d1 | E01 |
| 2-1-m1E01-1-0835 | 765.208 | OH | a1 | B09 | b2 | C02 | c2 | D02 | d1 | E01 |
| 2-1-m1E01-1-0836 | 765.244 | OH | a1 | B05 | b1 | C09 | c2 | D02 | d1 | E01 |
| 2-1-m1E01-1-0837 | 765.244 | OH | a1 | B09 | b2 | C22 | c3 | D02 | d1 | E01 |
| 2-1-m1E01-1-0838 | 765.244 | OH | a1 | B10 | b1 | C30 | c3 | D02 | d1 | E01 |
| 2-1-m1E01-1-0839 | 765.264 | OH | a1 | B07 | b2 | C07 | c2 | D02 | d1 | E01 |
| 2-1-m1E01-1-0840 | 765.320 | OH | a1 | B05 | b1 | C31 | c3 | D02 | d1 | E01 |
| 2-1-m1E01-1-0841 | 766.203 | OH | a1 | B09 | b2 | C03 | c2 | D02 | d1 | E01 |
| 2-1-m1E01-1-0842 | 766.208 | OH | a2 | B20 | b2 | C24 | c3 | D02 | d1 | E01 |
| 2-1-m1E01-1-0843 | 766.261 | OH | a1 | B08 | b1 | C29 | c3 | D02 | d1 | E01 |
| 2-1-m1E01-1-0844 | 767.175 | OH | a1 | B05 | b3 | C06 | c2 | D02 | d1 | E01 |
| 2-1-m1E01-1-0845 | 767.224 | OH | a1 | B06 | b1 | C09 | c2 | D02 | d1 | E01 |
| 2-1-m1E01-1-0846 | 767.224 | OH | a1 | B10 | b2 | C20 | c3 | D02 | d1 | E01 |
| 2-1-m1E01-1-0847 | 767.299 | OH | a1 | B06 | b1 | C31 | c3 | D02 | d1 | E01 |
| 2-1-m1E01-1-0848 | 768.154 | OH | a2 | B20 | b2 | C06 | c2 | D02 | d1 | E01 |
| 2-1-m1E01-1-0849 | 768.192 | OH | a2 | B11 | b3 | C16 | c2 | D02 | d1 | E01 |
| 2-1-m1E01-1-0850 | 768.219 | OH | a1 | B10 | b2 | C28 | c3 | D02 | d1 | E01 |
| 2-1-m1E01-1-0851 | 768.223 | OH | a2 | B18 | b1 | C09 | c2 | D02 | d1 | E01 |
| 2-1-m1E01-1-0852 | 768.240 | OH | a1 | B05 | b3 | C18 | c1 | D01 | d1 | E01 |
| 2-1-m1E01-1-0853 | 768.298 | OH | a2 | B18 | b1 | C31 | c3 | D02 | d1 | E01 |
| 2-1-m1E01-1-0854 | 769.155 | OH | a1 | B06 | b3 | C06 | c2 | D02 | d1 | E01 |
| 2-1-m1E01-1-0855 | 769.169 | OH | a1 | B07 | b3 | C08 | c2 | D02 | d1 | E01 |
| 2-1-m1E01-1-0856 | 769.188 | OH | a2 | B12 | b3 | C16 | c2 | D02 | d1 | E01 |
| 2-1-m1E01-1-0857 | 769.199 | OH | a1 | B08 | b2 | C05 | c2 | D02 | d1 | E01 |
| 2-1-m1E01-1-0858 | 769.219 | OH | a1 | B09 | b2 | C24 | c3 | D02 | d1 | E01 |
| 2-1-m1E01-1-0859 | 770.154 | OH | a2 | B18 | b3 | C06 | c2 | D02 | d1 | E01 |
| 2-1-m1E01-1-0860 | 770.154 | OH | a2 | B11 | b3 | C17 | c2 | D02 | d1 | E01 |
| 2-1-m1E01-1-0861 | 770.213 | OH | a2 | B19 | b1 | C13 | c2 | D02 | d1 | E01 |
| 2-1-m1E01-1-0862 | 770.219 | OH | a1 | B06 | b3 | C18 | c1 | D01 | d1 | E01 |
| 2-1-m1E01-1-0863 | 770.219 | OH | a1 | B08 | b2 | C26 | c3 | D02 | d1 | E01 |
| 2-1-m1E01-1-0864 | 771.149 | OH | a2 | B12 | b3 | C17 | c2 | D02 | d1 | E01 |
| 2-1-m1E01-1-0865 | 771.164 | OH | a1 | B09 | b2 | C06 | c2 | D02 | d1 | E01 |
| 2-1-m1E01-1-0866 | 771.182 | OH | a1 | B10 | b2 | C01 | c2 | D02 | d1 | E01 |
| 2-1-m1E01-1-0867 | 771.219 | OH | a2 | B18 | b3 | C18 | c1 | D01 | d1 | E01 |
| 2-1-m1E01-1-0868 | 772.148 | OH | a2 | B19 | b3 | C15 | c2 | D02 | d1 | E01 |
| 2-1-m1E01-1-0869 | 772.179 | OH | a1 | B08 | b2 | C04 | c2 | D02 | d1 | E01 |
| 2-1-m1E01-1-0870 | 772.218 | OH | a2 | B19 | b3 | C16 | c1 | D01 | d1 | E01 |
| 2-1-m1E01-1-0871 | 773.194 | OH | a1 | B07 | b1 | C09 | c2 | D02 | d1 | E01 |

(continued)

| ID | Exact Mass | | a | B | b | C | c | D | d | E |
|---|---|---|---|---|---|---|---|---|---|---|
| 2-1-m1E01-1-0872 | 773.269 | OH | a1 | B07 | b1 | C31 | c3 | D02 | d1 | E01 |
| 2-1-m1E01-1-0873 | 774.179 | OH | a2 | B19 | b3 | C17 | c1 | D01 | d1 | E01 |
| 2-1-m1E01-1-0874 | 774.179 | OH | a2 | B19 | b1 | C14 | c2 | D02 | d1 | E01 |
| 2-1-m1E01-1-0875 | 774.270 | OH | a2 | B20 | b1 | C29 | c3 | D02 | d1 | E01 |
| 2-1-m1E01-1-0876 | 775.125 | OH | a1 | B07 | b3 | C06 | c2 | D02 | d1 | E01 |
| 2-1-m1E01-1-0877 | 776.190 | OH | a1 | B07 | b3 | C18 | c1 | D01 | d1 | E01 |
| 2-1-m1E01-1-0878 | 777.208 | OH | a2 | B20 | b2 | C05 | c2 | D02 | d1 | E01 |
| 2-1-m1E01-1-0879 | 777.281 | OH | a1 | B09 | b1 | C29 | c3 | D02 | d1 | E01 |
| 2-1-m1E01-1-0880 | 778.199 | OH | a1 | B08 | b1 | C13 | c2 | D02 | d1 | E01 |
| 2-1-m1E01-1-0881 | 778.228 | OH | a2 | B20 | b2 | C26 | c3 | D02 | d1 | E01 |
| 2-1-m1E01-1-0882 | 778.265 | OH | a2 | B19 | b2 | C07 | c2 | D02 | d1 | E01 |
| 2-1-m1E01-1-0883 | 779.260 | OH | a1 | B10 | b1 | C32 | c3 | D02 | d1 | E01 |
| 2-1-m1E01-1-0884 | 780.134 | OH | a1 | B08 | b3 | C15 | c2 | D02 | d1 | E01 |
| 2-1-m1E01-1-0885 | 780.188 | OH | a2 | B20 | b2 | C04 | c2 | D02 | d1 | E01 |
| 2-1-m1E01-1-0886 | 780.204 | OH | a1 | B08 | b3 | C16 | c1 | D01 | d1 | E01 |
| 2-1-m1E01-1-0887 | 780.219 | OH | a1 | B09 | b2 | C05 | c2 | D02 | d1 | E01 |
| 2-1-m1E01-1-0888 | 781.203 | OH | a1 | B10 | b2 | C02 | c2 | D02 | d1 | E01 |
| 2-1-m1E01-1-0889 | 781.239 | OH | a1 | B10 | b2 | C22 | c3 | D02 | d1 | E01 |
| 2-1-m1E01-1-0890 | 781.239 | OH | a1 | B09 | b2 | C26 | c3 | D02 | d1 | E01 |
| 2-1-m1E01-1-0891 | 782.165 | OH | a1 | B08 | b3 | C17 | c1 | D01 | d1 | E01 |
| 2-1-m1E01-1-0892 | 782.165 | OH | a1 | B08 | b1 | C14 | c2 | D02 | d1 | E01 |
| 2-1-m1E01-1-0893 | 782.169 | OH | a2 | B19 | b3 | C08 | c2 | D02 | d1 | E01 |
| 2-1-m1E01-1-0894 | 782.198 | OH | a1 | B10 | b2 | C03 | c2 | D02 | d1 | E01 |
| 2-1-m1E01-1-0895 | 782.208 | OH | a2 | B13 | b3 | C16 | c2 | D02 | d1 | E01 |
| 2-1-m1E01-1-0896 | 783.199 | OH | a1 | B09 | b2 | C04 | c2 | D02 | d1 | E01 |
| 2-1-m1E01-1-0897 | 784.170 | OH | a2 | B13 | b3 | C17 | c2 | D02 | d1 | E01 |
| 2-1-m1E01-1-0898 | 785.165 | OH | a2 | B11 | b3 | C18 | c2 | D02 | d1 | E01 |
| 2-1-m1E01-1-0899 | 785.214 | OH | a1 | B10 | b2 | C24 | c3 | D02 | d1 | E01 |
| 2-1-m1E01-1-0900 | 786.160 | OH | a2 | B12 | b3 | C18 | c2 | D02 | d1 | E01 |
| 2-1-m1E01-1-0901 | 786.183 | OH | a2 | B14 | b3 | C16 | c2 | D02 | d1 | E01 |
| 2-1-m1E01-1-0902 | 786.195 | OH | a2 | B19 | b1 | C09 | c2 | D02 | d1 | E01 |
| 2-1-m1E01-1-0903 | 786.208 | OH | a2 | B20 | b1 | C13 | c2 | D02 | d1 | E01 |
| 2-1-m1E01-1-0904 | 786.251 | OH | a1 | B08 | b2 | C07 | c2 | D02 | d1 | E01 |
| 2-1-m1E01-1-0905 | 786.270 | OH | a2 | B19 | b1 | C31 | c3 | D02 | d1 | E01 |
| 2-1-m1E01-1-0906 | 787.159 | OH | a1 | B10 | b2 | C06 | c2 | D02 | d1 | E01 |
| 2-1-m1E01-1-0907 | 787.198 | OH | a1 | B01 | b3 | C16 | c2 | D02 | d1 | E01 |
| 2-1-m1E01-1-0908 | 788.126 | OH | a2 | B19 | b3 | C06 | c2 | D02 | d1 | E01 |
| 2-1-m1E01-1-0909 | 788.143 | OH | a2 | B20 | b3 | C15 | c2 | D02 | d1 | E01 |

(continued)

| ID | Exact Mass | | a | B | b | C | c | D | d | E |
|---|---|---|---|---|---|---|---|---|---|---|
| 2-1-m1E01-1-0910 | 788.144 | OH | a2 | B14 | b3 | C17 | c2 | D02 | d1 | E01 |
| 2-1-m1E01-1-0911 | 788.193 | OH | a1 | B02 | b3 | C16 | c2 | D02 | d1 | E01 |
| 2-1-m1E01-1-0912 | 788.213 | OH | a2 | B20 | b3 | C16 | c1 | D01 | d1 | E01 |
| 2-1-m1E01-1-0913 | 789.160 | OH | a1 | B01 | b3 | C17 | c2 | D02 | d1 | E01 |
| 2-1-m1E01-1-0914 | 789.190 | OH | a2 | B19 | b3 | C18 | c1 | D01 | d1 | E01 |
| 2-1-m1E01-1-0915 | 789.219 | OH | a1 | B09 | b1 | C13 | c2 | D02 | d1 | E01 |
| 2-1-m1E01-1-0916 | 790.155 | OH | a1 | B02 | b3 | C17 | c2 | D02 | d1 | E01 |
| 2-1-m1E01-1-0917 | 790.155 | OH | a1 | B08 | b3 | C08 | c2 | D02 | d1 | E01 |
| 2-1-m1E01-1-0918 | 790.174 | OH | a2 | B20 | b3 | C17 | c1 | D01 | d1 | E01 |
| 2-1-m1E01-1-0919 | 790.174 | OH | a2 | B20 | b1 | C14 | c2 | D02 | d1 | E01 |
| 2-1-m1E01-1-0920 | 791.154 | OH | a1 | B09 | b3 | C15 | c2 | D02 | d1 | E01 |
| 2-1-m1E01-1-0921 | 791.224 | OH | a1 | B09 | b3 | C16 | c1 | D01 | d1 | E01 |
| 2-1-m1E01-1-0922 | 793.185 | OH | a1 | B09 | b3 | C17 | c1 | D01 | d1 | E01 |
| 2-1-m1E01-1-0923 | 793.185 | OH | a1 | B09 | b1 | C14 | c2 | D02 | d1 | E01 |
| 2-1-m1E01-1-0924 | 793.276 | OH | a1 | B10 | b1 | C29 | c3 | D02 | d1 | E01 |
| 2-1-m1E01-1-0925 | 794.180 | OH | a1 | B08 | b1 | C09 | c2 | D02 | d1 | E01 |
| 2-1-m1E01-1-0926 | 794.256 | OH | a1 | B08 | b1 | C31 | c3 | D02 | d1 | E01 |
| 2-1-m1E01-1-0927 | 794.260 | OH | a2 | B20 | b2 | C07 | c2 | D02 | d1 | E01 |
| 2-1-m1E01-1-0928 | 796.111 | OH | a1 | B08 | b3 | C06 | c2 | D02 | d1 | E01 |
| 2-1-m1E01-1-0929 | 796.214 | OH | a1 | B10 | b2 | C05 | c2 | D02 | d1 | E01 |
| 2-1-m1E01-1-0930 | 796.224 | OH | a2 | B15 | b3 | C16 | c2 | D02 | d1 | E01 |
| 2-1-m1E01-1-0931 | 797.176 | OH | a1 | B08 | b3 | C18 | c1 | D01 | d1 | E01 |
| 2-1-m1E01-1-0932 | 797.234 | OH | a1 | B10 | b2 | C26 | c3 | D02 | d1 | E01 |
| 2-1-m1E01-1-0933 | 797.271 | OH | a1 | B09 | b2 | C07 | c2 | D02 | d1 | E01 |
| 2-1-m1E01-1-0934 | 798.164 | OH | a2 | B20 | b3 | C08 | c2 | D02 | d1 | E01 |
| 2-1-m1E01-1-0935 | 798.185 | OH | a2 | B15 | b3 | C17 | c2 | D02 | d1 | E01 |
| 2-1-m1E01-1-0936 | 798.203 | OH | a2 | B16 | b3 | C16 | c2 | D02 | d1 | E01 |
| 2-1-m1E01-1-0937 | 799.180 | OH | a2 | B13 | b3 | C18 | c2 | D02 | d1 | E01 |
| 2-1-m1E01-1-0938 | 799.194 | OH | a1 | B10 | b2 | C04 | c2 | D02 | d1 | E01 |
| 2-1-m1E01-1-0939 | 800.164 | OH | a2 | B16 | b3 | C17 | c2 | D02 | d1 | E01 |
| 2-1-m1E01-1-0940 | 801.175 | OH | a1 | B09 | b3 | C08 | c2 | D02 | d1 | E01 |
| 2-1-m1E01-1-0941 | 801.214 | OH | a1 | B03 | b3 | C16 | c2 | D02 | d1 | E01 |
| 2-1-m1E01-1-0942 | 802.190 | OH | a2 | B20 | b1 | C09 | c2 | D02 | d1 | E01 |
| 2-1-m1E01-1-0943 | 802.265 | OH | a2 | B20 | b1 | C31 | c3 | D02 | d1 | E01 |
| 2-1-m1E01-1-0944 | 803.155 | OH | a2 | B14 | b3 | C18 | c2 | D02 | d1 | E01 |
| 2-1-m1E01-1-0945 | 803.175 | OH | a1 | B03 | b3 | C17 | c2 | D02 | d1 | E01 |
| 2-1-m1E01-1-0946 | 804.121 | OH | a2 | B20 | b3 | C06 | c2 | D02 | d1 | E01 |
| 2-1-m1E01-1-0947 | 804.171 | OH | a1 | B01 | b3 | C18 | c2 | D02 | d1 | E01 |

(continued)

| ID | Exact Mass | | a | B | b | C | c | D | d | E |
|---|---|---|---|---|---|---|---|---|---|---|
| 2-1-m1E01-1-0948 | 804.174 | OH | a2 | B17 | b3 | C16 | c2 | D02 | d1 | E01 |
| 2-1-m1E01-1-0949 | 805.166 | OH | a1 | B02 | b3 | C18 | c2 | D02 | d1 | E01 |
| 2-1-m1E01-1-0950 | 805.185 | OH | a2 | B20 | b3 | C18 | c1 | D01 | d1 | E01 |
| 2-1-m1E01-1-0951 | 805.189 | OH | a1 | B04 | b3 | C16 | c2 | D02 | d1 | E01 |
| 2-1-m1E01-1-0952 | 805.200 | OH | a1 | B09 | b1 | C09 | c2 | D02 | d1 | E01 |
| 2-1-m1E01-1-0953 | 805.214 | OH | a1 | B10 | b1 | C13 | c2 | D02 | d1 | E01 |
| 2-1-m1E01-1-0954 | 805.276 | OH | a1 | B09 | b1 | C31 | c3 | D02 | d1 | E01 |
| 2-1-m1E01-1-0955 | 806.135 | OH | a2 | B17 | b3 | C17 | c2 | D02 | d1 | E01 |
| 2-1-m1E01-1-0956 | 807.131 | OH | a1 | B09 | b3 | C06 | c2 | D02 | d1 | E01 |
| 2-1-m1E01-1-0957 | 807.149 | OH | a1 | B10 | b3 | C15 | c2 | D02 | d1 | E01 |
| 2-1-m1E01-1-0958 | 807.150 | OH | a1 | B04 | b3 | C17 | c2 | D02 | d1 | E01 |
| 2-1-m1E01-1-0959 | 807.219 | OH | a1 | B10 | b3 | C16 | c1 | D01 | d1 | E01 |
| 2-1-m1E01-1-0960 | 808.196 | OH | a1 | B09 | b3 | C18 | c1 | D01 | d1 | E01 |
| 2-1-m1E01-1-0961 | 809.180 | OH | a1 | B10 | b3 | C17 | c1 | D01 | d1 | E01 |
| 2-1-m1E01-1-0962 | 809.180 | OH | a1 | B10 | b1 | C14 | c2 | D02 | d1 | E01 |
| 2-1-m1E01-1-0963 | 813.196 | OH | a2 | B15 | b3 | C18 | c2 | D02 | d1 | E01 |
| 2-1-m1E01-1-0964 | 813.266 | OH | a1 | B10 | b2 | C07 | c2 | D02 | d1 | E01 |
| 2-1-m1E01-1-0965 | 815.175 | OH | a2 | B16 | b3 | C18 | c2 | D02 | d1 | E01 |
| 2-1-m1E01-1-0966 | 815.229 | OH | a1 | B05 | b3 | C16 | c2 | D02 | d1 | E01 |
| 2-1-m1E01-1-0967 | 817.170 | OH | a1 | B10 | b3 | C08 | c2 | D02 | d1 | E01 |
| 2-1-m1E01-1-0968 | 817.191 | OH | a1 | B05 | b3 | C17 | c2 | D02 | d1 | E01 |
| 2-1-m1E01-1-0969 | 817.209 | OH | a1 | B06 | b3 | C16 | c2 | D02 | d1 | E01 |
| 2-1-m1E01-1-0970 | 818.186 | OH | a1 | B03 | b3 | C18 | c2 | D02 | d1 | E01 |
| 2-1-m1E01-1-0971 | 818.208 | OH | a2 | B18 | b3 | C16 | c2 | D02 | d1 | E01 |
| 2-1-m1E01-1-0972 | 819.170 | OH | a1 | B06 | b3 | C17 | c2 | D02 | d1 | E01 |
| 2-1-m1E01-1-0973 | 820.170 | OH | a2 | B18 | b3 | C17 | c2 | D02 | d1 | E01 |
| 2-1-m1E01-1-0974 | 821.146 | OH | a2 | B17 | b3 | C18 | c2 | D02 | d1 | E01 |
| 2-1-m1E01-1-0975 | 821.195 | OH | a1 | B10 | b1 | C09 | c2 | D02 | d1 | E01 |
| 2-1-m1E01-1-0976 | 821.271 | OH | a1 | B10 | b1 | C31 | c3 | D02 | d1 | E01 |
| 2-1-m1E01-1-0977 | 822.161 | OH | a1 | B04 | b3 | C18 | c2 | D02 | d1 | E01 |
| 2-1-m1E01-1-0978 | 823.126 | OH | a1 | B10 | b3 | C06 | c2 | D02 | d1 | E01 |
| 2-1-m1E01-1-0979 | 823.179 | OH | a1 | B07 | b3 | C16 | c2 | D02 | d1 | E01 |
| 2-1-m1E01-1-0980 | 824.191 | OH | a1 | B10 | b3 | C18 | c1 | D01 | d1 | E01 |
| 2-1-m1E01-1-0981 | 825.141 | OH | a1 | B07 | b3 | C17 | c2 | D02 | d1 | E01 |
| 2-1-m1E01-1-0982 | 832.202 | OH | a1 | B05 | b3 | C18 | c2 | D02 | d1 | E01 |
| 2-1-m1E01-1-0983 | 834.181 | OH | a1 | B06 | b3 | C18 | c2 | D02 | d1 | E01 |
| 2-1-m1E01-1-0984 | 835.180 | OH | a2 | B18 | b3 | C18 | c2 | D02 | d1 | E01 |
| 2-1-m1E01-1-0985 | 836.180 | OH | a2 | B19 | b3 | C16 | c2 | D02 | d1 | E01 |

(continued)

| ID | Exact Mass | | a | B | b | C | c | D | d | E |
|---|---|---|---|---|---|---|---|---|---|---|
| 2-1-m1E01-1-0986 | 838.141 | OH | a2 | B19 | b3 | C17 | c2 | D02 | d1 | E01 |
| 2-1-m1E01-1-0987 | 840.152 | OH | a1 | B07 | b3 | C18 | c2 | D02 | d1 | E01 |
| 2-1-m1E01-1-0988 | 844.166 | OH | a1 | B08 | b3 | C16 | c2 | D02 | d1 | E01 |
| 2-1-m1E01-1-0989 | 846.127 | OH | a1 | B08 | b3 | C17 | c2 | D02 | d1 | E01 |
| 2-1-m1E01-1-0990 | 852.175 | OH | a2 | B20 | b3 | C16 | c2 | D02 | d1 | E01 |
| 2-1-m1E01-1-0991 | 853.152 | OH | a2 | B19 | b3 | C18 | c2 | D02 | d1 | E01 |
| 2-1-m1E01-1-0992 | 854.136 | OH | a2 | B20 | b3 | C17 | c2 | D02 | d1 | E01 |
| 2-1-m1E01-1-0993 | 855.186 | OH | a1 | B09 | b3 | C16 | c2 | D02 | d1 | E01 |
| 2-1-m1E01-1-0994 | 857.147 | OH | a1 | B09 | b3 | C17 | c2 | D02 | d1 | E01 |
| 2-1-m1E01-1-0995 | 861.138 | OH | a1 | B08 | b3 | C18 | c2 | D02 | d1 | E01 |
| 2-1-m1E01-1-0996 | 869.147 | OH | a2 | B20 | b3 | C18 | c2 | D02 | d1 | E01 |
| 2-1-m1E01-1-0997 | 871.180 | OH | a1 | B10 | b3 | C16 | c2 | D02 | d1 | E01 |
| 2-1-m1E01-1-0998 | 872.158 | OH | a1 | B09 | b3 | C18 | c2 | D02 | d1 | E01 |
| 2-1-m1E01-1-0999 | 873.142 | OH | a1 | B10 | b3 | C17 | c2 | D02 | d1 | E01 |
| 2-1-m1E01-1-1000 | 888.153 | OH | a1 | B10 | b3 | C18 | c2 | D02 | d1 | E01 |

The reaction formula for the case where the ethylation reaction is carried out using a mixture 2-1-m1E01-1 separately prepared is shown below as an example.

[Formula 118]

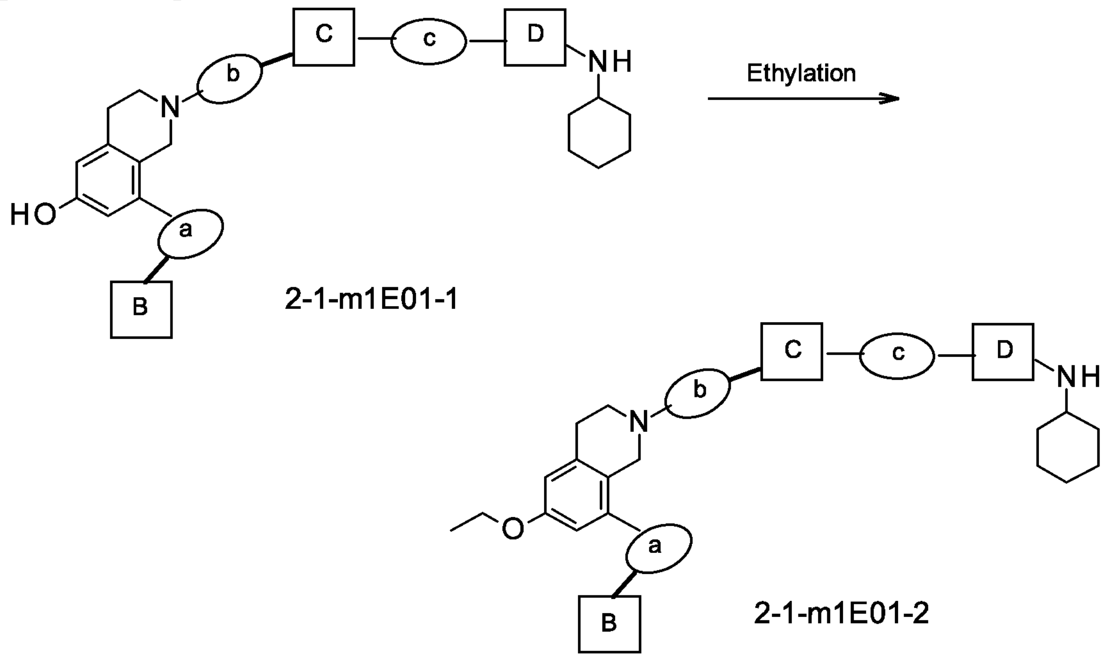


2-1-m1E01-1

2-1-m1E01-2

Using the mixture 2-1-m1E01-1 separately prepared, a mixture 2-1-m1E01-2 was synthesized as follows.

To the mixture 2-1-m1E01-1 (9.6 mg, 13.5 μmol) placed in a 0.5 to 2 mL microwave container were added DMF (154 μL), EtOH (38.6 μL), BTPP (41.3 μL, 0.135 mmol), and $Et_3PO_4$ (34.4 μL, 0.203 mmol), and the mixture was shaken at 70°C for 27 hours. The reaction solution was transferred to a 6 mL column equipped with a filter packed with ISOLUTE (R) CBA (0.7 mmol/g, 189 mg) using MeCN (0.2 mL) twice. After 10 minutes, the mixture was washed five

times with MeCN (0.5 mL), and the combined filtrate was distilled off under reduced pressure (using a Genevac reduced pressure concentrator) to afford the mixture 2-1-m1E01-2. Details of the mixture 2-1-m1E01-2 are shown in [Table 6-6-4].

The notation method in [Table 6-6-4] will be described. In [Table 6-6-4], each compound that may be included in the mixture 2-1-m1E01-2 is shown by ID, and the combination of core blocks and linkers in the structure of the mixture 2-1-m1E01-2 is shown by symbol or chemical formula. The correspondence between symbols and specific structures is illustrated in Figure 2. In [Table 6-6-4], "☒" is represented by a chemical formula.

[Formula 119]

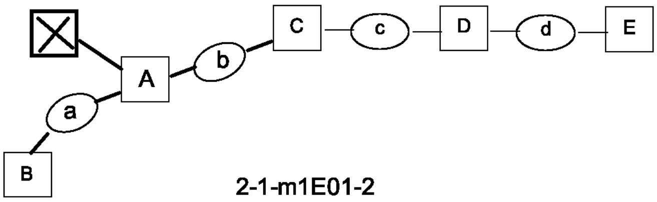

2-1-m1E01-2

For example, if the ID is 2-1-m1E01-2-0001, the following structural formula is shown in [Table 6-6-3].

[Formula 120]

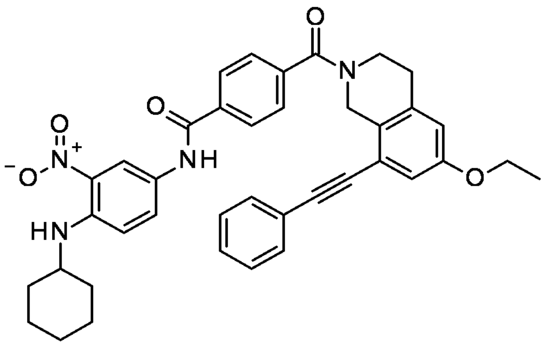

2-1-m1E01-2-0001

[Table 122]

[Table 6-6-3] Compound structure in ID notation

| ID | | a | B | b | C | c | D | d | E |
|---|---|---|---|---|---|---|---|---|---|
| 2-1-m1E01-2-0001 | OEt | a2 | B11 | b1 | C08 | c1 | D01 | d1 | E01 |

Compounds that may be included in the mixture 2-1-m1E01-2 are shown in [Table 6-6-4] .

[Table 123]

[Table 6-6-4] Compounds that may be included in mixture 2-1-m1E01-2

| ID | Exact Mass | ☒ | a | B | b | C | c | D | d | E |
|---|---|---|---|---|---|---|---|---|---|---|
| 2-1-m1E01-2-0001 | 614.289 | OEt | a2 | B11 | b1 | C08 | c1 | D01 | d1 | E01 |

(continued)

| ID | Exact Mass | ☒ | a | B | b | C | c | D | d | E |
|---|---|---|---|---|---|---|---|---|---|---|
| 2-1-m1E01-2-0002 | 615.285 | OEt | a2 | B12 | b1 | C08 | c1 | D01 | d1 | E01 |
| 2-1-m1E01-2-0003 | 615.285 | OEt | a2 | B11 | b1 | C11 | c1 | D01 | d1 | E01 |
| 2-1-m1E01-2-0004 | 616.280 | OEt | a2 | B11 | b1 | C10 | c1 | D01 | d1 | E01 |
| 2-1-m1E01-2-0005 | 616.280 | OEt | a2 | B12 | b1 | C11 | c1 | D01 | d1 | E01 |
| 2-1-m1E01-2-0006 | 617.275 | OEt | a2 | B12 | b1 | C10 | c1 | D01 | d1 | E01 |
| 2-1-m1E01-2-0007 | 621.241 | OEt | a2 | B11 | b1 | C12 | c1 | D01 | d1 | E01 |
| 2-1-m1E01-2-0008 | 622.236 | OEt | a2 | B12 | b1 | C12 | c1 | D01 | d1 | E01 |
| 2-1-m1E01-2-0009 | 628.305 | OEt | a2 | B13 | b1 | C08 | c1 | D01 | d1 | E01 |
| 2-1-m1E01-2-0010 | 629.300 | OEt | a2 | B13 | b1 | C11 | c1 | D01 | d1 | E01 |
| 2-1-m1E01-2-0011 | 630.295 | OEt | a2 | B13 | b1 | C10 | c1 | D01 | d1 | E01 |
| 2-1-m1E01-2-0012 | 632.263 | OEt | a2 | B11 | b2 | C01 | c1 | D01 | d1 | E01 |
| 2-1-m1E01-2-0013 | 632.280 | OEt | a2 | B14 | b1 | C08 | c1 | D01 | d1 | E01 |
| 2-1-m1E01-2-0014 | 633.259 | OEt | a2 | B12 | b2 | C01 | c1 | D01 | d1 | E01 |
| 2-1-m1E01-2-0015 | 633.275 | OEt | a2 | B14 | b1 | C11 | c1 | D01 | d1 | E01 |
| 2-1-m1E01-2-0016 | 633.295 | OEt | a1 | B01 | b1 | C08 | c1 | D01 | d1 | E01 |
| 2-1-m1E01-2-0017 | 634.270 | OEt | a2 | B14 | b1 | C10 | c1 | D01 | d1 | E01 |
| 2-1-m1E01-2-0018 | 634.290 | OEt | a1 | B02 | b1 | C08 | c1 | D01 | d1 | E01 |
| 2-1-m1E01-2-0019 | 634.290 | OEt | a1 | B01 | b1 | C11 | c1 | D01 | d1 | E01 |
| 2-1-m1E01-2-0020 | 635.257 | OEt | a2 | B13 | b1 | C12 | c1 | D01 | d1 | E01 |
| 2-1-m1E01-2-0021 | 635.286 | OEt | a1 | B01 | b1 | C10 | c1 | D01 | d1 | E01 |
| 2-1-m1E01-2-0022 | 635.286 | OEt | a1 | B02 | b1 | C11 | c1 | D01 | d1 | E01 |
| 2-1-m1E01-2-0023 | 636.281 | OEt | a1 | B02 | b1 | C10 | c1 | D01 | d1 | E01 |
| 2-1-m1E01-2-0024 | 639.232 | OEt | a2 | B14 | b1 | C12 | c1 | D01 | d1 | E01 |
| 2-1-m1E01-2-0025 | 640.247 | OEt | a1 | B01 | b1 | C12 | c1 | D01 | d1 | E01 |
| 2-1-m1E01-2-0026 | 641.242 | OEt | a1 | B02 | b1 | C12 | c1 | D01 | d1 | E01 |
| 2-1-m1E01-2-0027 | 642.284 | OEt | a2 | B11 | b2 | C02 | c1 | D01 | d1 | E01 |
| 2-1-m1E01-2-0028 | 642.321 | OEt | a2 | B15 | b1 | C08 | c1 | D01 | d1 | E01 |
| 2-1-m1E01-2-0029 | 643.279 | OEt | a2 | B12 | b2 | C02 | c1 | D01 | d1 | E01 |
| 2-1-m1E01-2-0030 | 643.279 | OEt | a2 | B11 | b2 | C03 | c1 | D01 | d1 | E01 |
| 2-1-m1E01-2-0031 | 643.316 | OEt | a2 | B15 | b1 | C11 | c1 | D01 | d1 | E01 |
| 2-1-m1E01-2-0032 | 644.275 | OEt | a2 | B12 | b2 | C03 | c1 | D01 | d1 | E01 |
| 2-1-m1E01-2-0033 | 644.300 | OEt | a2 | B16 | b1 | C08 | c1 | D01 | d1 | E01 |
| 2-1-m1E01-2-0034 | 644.311 | OEt | a2 | B15 | b1 | C10 | c1 | D01 | d1 | E01 |
| 2-1-m1E01-2-0035 | 645.295 | OEt | a2 | B16 | b1 | C11 | c1 | D01 | d1 | E01 |
| 2-1-m1E01-2-0036 | 646.279 | OEt | a2 | B13 | b2 | C01 | c1 | D01 | d1 | E01 |
| 2-1-m1E01-2-0037 | 646.290 | OEt | a2 | B16 | b1 | C10 | c1 | D01 | d1 | E01 |
| 2-1-m1E01-2-0038 | 647.311 | OEt | a1 | B03 | b1 | C08 | c1 | D01 | d1 | E01 |

(continued)

| ID | Exact Mass | ⊠ | a | B | b | C | c | D | d | E |
|---|---|---|---|---|---|---|---|---|---|---|
| 2-1-m1E01-2-0039 | 648.241 | OEt | a2 | B11 | b2 | C06 | c1 | D01 | d1 | E01 |
| 2-1-m1E01-2-0040 | 648.306 | OEt | a1 | B03 | b1 | C11 | c1 | D01 | d1 | E01 |
| 2-1-m1E01-2-0041 | 649.236 | OEt | a2 | B12 | b2 | C06 | c1 | D01 | d1 | E01 |
| 2-1-m1E01-2-0042 | 649.272 | OEt | a2 | B15 | b1 | C12 | c1 | D01 | d1 | E01 |
| 2-1-m1E01-2-0043 | 649.301 | OEt | a1 | B03 | b1 | C10 | c1 | D01 | d1 | E01 |
| 2-1-m1E01-2-0044 | 650.254 | OEt | a2 | B14 | b2 | C01 | c1 | D01 | d1 | E01 |
| 2-1-m1E01-2-0045 | 650.270 | OEt | a2 | B17 | b1 | C08 | c1 | D01 | d1 | E01 |
| 2-1-m1E01-2-0046 | 651.252 | OEt | a2 | B16 | b1 | C12 | c1 | D01 | d1 | E01 |
| 2-1-m1E01-2-0047 | 651.266 | OEt | a2 | B17 | b1 | C11 | c1 | D01 | d1 | E01 |
| 2-1-m1E01-2-0048 | 651.269 | OEt | a1 | B01 | b2 | C01 | c1 | D01 | d1 | E01 |
| 2-1-m1E01-2-0049 | 651.286 | OEt | a1 | B04 | b1 | C08 | c1 | D01 | d1 | E01 |
| 2-1-m1E01-2-0050 | 652.261 | OEt | a2 | B17 | b1 | C10 | c1 | D01 | d1 | E01 |
| 2-1-m1E01-2-0051 | 652.265 | OEt | a1 | B02 | b2 | C01 | c1 | D01 | d1 | E01 |
| 2-1-m1E01-2-0052 | 652.281 | OEt | a1 | B04 | b1 | C11 | c1 | D01 | d1 | E01 |
| 2-1-m1E01-2-0053 | 653.276 | OEt | a1 | B04 | b1 | C10 | c1 | D01 | d1 | E01 |
| 2-1-m1E01-2-0054 | 654.262 | OEt | a1 | B03 | b1 | C12 | c1 | D01 | d1 | E01 |
| 2-1-m1E01-2-0055 | 656.300 | OEt | a2 | B13 | b2 | C02 | c1 | D01 | d1 | E01 |
| 2-1-m1E01-2-0056 | 657.222 | OEt | a2 | B17 | b1 | C12 | c1 | D01 | d1 | E01 |
| 2-1-m1E01-2-0057 | 657.295 | OEt | a2 | B13 | b2 | C03 | c1 | D01 | d1 | E01 |
| 2-1-m1E01-2-0058 | 657.295 | OEt | a2 | B11 | b2 | C05 | c1 | D01 | d1 | E01 |
| 2-1-m1E01-2-0059 | 658.237 | OEt | a1 | B04 | b1 | C12 | c1 | D01 | d1 | E01 |
| 2-1-m1E01-2-0060 | 658.290 | OEt | a2 | B12 | b2 | C05 | c1 | D01 | d1 | E01 |
| 2-1-m1E01-2-0061 | 660.275 | OEt | a2 | B14 | b2 | C02 | c1 | D01 | d1 | E01 |
| 2-1-m1E01-2-0062 | 660.275 | OEt | a2 | B11 | b2 | C04 | c1 | D01 | d1 | E01 |
| 2-1-m1E01-2-0063 | 660.295 | OEt | a2 | B15 | b2 | C01 | c1 | D01 | d1 | E01 |
| 2-1-m1E01-2-0064 | 661.270 | OEt | a2 | B12 | b2 | C04 | c1 | D01 | d1 | E01 |
| 2-1-m1E01-2-0065 | 661.270 | OEt | a2 | B14 | b2 | C03 | c1 | D01 | d1 | E01 |
| 2-1-m1E01-2-0066 | 661.290 | OEt | a1 | B01 | b2 | C02 | c1 | D01 | d1 | E01 |
| 2-1-m1E01-2-0067 | 661.326 | OEt | a1 | B05 | b1 | C08 | c1 | D01 | d1 | E01 |
| 2-1-m1E01-2-0068 | 662.256 | OEt | a2 | B13 | b2 | C06 | c1 | D01 | d1 | E01 |
| 2-1-m1E01-2-0069 | 662.274 | OEt | a2 | B16 | b2 | C01 | c1 | D01 | d1 | E01 |
| 2-1-m1E01-2-0070 | 662.285 | OEt | a1 | B02 | b2 | C02 | c1 | D01 | d1 | E01 |
| 2-1-m1E01-2-0071 | 662.285 | OEt | a1 | B01 | b2 | C03 | c1 | D01 | d1 | E01 |
| 2-1-m1E01-2-0072 | 662.322 | OEt | a1 | B05 | b1 | C11 | c1 | D01 | d1 | E01 |
| 2-1-m1E01-2-0073 | 663.281 | OEt | a1 | B02 | b2 | C03 | c1 | D01 | d1 | E01 |
| 2-1-m1E01-2-0074 | 663.306 | OEt | a1 | B06 | b1 | C08 | c1 | D01 | d1 | E01 |
| 2-1-m1E01-2-0075 | 663.317 | OEt | a1 | B05 | b1 | C10 | c1 | D01 | d1 | E01 |

(continued)

| ID | Exact Mass | ⊠ | a | B | b | C | c | D | d | E |
|---|---|---|---|---|---|---|---|---|---|---|
| 2-1-m1E01-2-0076 | 664.301 | OEt | a1 | B06 | b1 | C11 | c1 | D01 | d1 | E01 |
| 2-1-m1E01-2-0077 | 664.305 | OEt | a2 | B18 | b1 | C08 | c1 | D01 | d1 | E01 |
| 2-1-m1E01-2-0078 | 665.285 | OEt | a1 | B03 | b2 | C01 | c1 | D01 | d1 | E01 |
| 2-1-m1E01-2-0079 | 665.296 | OEt | a1 | B06 | b1 | C10 | c1 | D01 | d1 | E01 |
| 2-1-m1E01-2-0080 | 665.300 | OEt | a2 | B18 | b1 | C11 | c1 | D01 | d1 | E01 |
| 2-1-m1E01-2-0081 | 666.231 | OEt | a2 | B14 | b2 | C06 | c1 | D01 | d1 | E01 |
| 2-1-m1E01-2-0082 | 666.295 | OEt | a2 | B18 | b1 | C10 | c1 | D01 | d1 | E01 |
| 2-1-m1E01-2-0083 | 666.295 | OEt | a2 | B11 | b1 | C13 | c1 | D01 | d1 | E01 |
| 2-1-m1E01-2-0084 | 667.246 | OEt | a1 | B01 | b2 | C06 | c1 | D01 | d1 | E01 |
| 2-1-m1E01-2-0085 | 667.291 | OEt | a2 | B12 | b1 | C13 | c1 | D01 | d1 | E01 |
| 2-1-m1E01-2-0086 | 668.230 | OEt | a2 | B11 | b3 | C15 | c1 | D01 | d1 | E01 |
| 2-1-m1E01-2-0087 | 668.242 | OEt | a1 | B02 | b2 | C06 | c1 | D01 | d1 | E01 |
| 2-1-m1E01-2-0088 | 668.245 | OEt | a2 | B17 | b2 | C01 | c1 | D01 | d1 | E01 |
| 2-1-m1E01-2-0089 | 668.278 | OEt | a1 | B05 | b1 | C12 | c1 | D01 | d1 | E01 |
| 2-1-m1E01-2-0090 | 669.226 | OEt | a2 | B12 | b3 | C15 | c1 | D01 | d1 | E01 |
| 2-1-m1E01-2-0091 | 669.260 | OEt | a1 | B04 | b2 | C01 | c1 | D01 | d1 | E01 |
| 2-1-m1E01-2-0092 | 669.276 | OEt | a1 | B07 | b1 | C08 | c1 | D01 | d1 | E01 |
| 2-1-m1E01-2-0093 | 670.257 | OEt | a1 | B06 | b1 | C12 | c1 | D01 | d1 | E01 |
| 2-1-m1E01-2-0094 | 670.261 | OEt | a2 | B11 | b1 | C14 | c1 | D01 | d1 | E01 |
| 2-1-m1E01-2-0095 | 670.272 | OEt | a1 | B07 | b1 | C11 | c1 | D01 | d1 | E01 |
| 2-1-m1E01-2-0096 | 670.283 | OEt | a2 | B11 | b2 | C33 | c3 | D02 | d1 | E01 |
| 2-1-m1E01-2-0097 | 670.316 | OEt | a2 | B15 | b2 | C02 | c1 | D01 | d1 | E01 |
| 2-1-m1E01-2-0098 | 671.257 | OEt | a2 | B18 | b1 | C12 | c1 | D01 | d1 | E01 |
| 2-1-m1E01-2-0099 | 671.257 | OEt | a2 | B12 | b1 | C14 | c1 | D01 | d1 | E01 |
| 2-1-m1E01-2-0100 | 671.267 | OEt | a1 | B07 | b1 | C10 | c1 | D01 | d1 | E01 |
| 2-1-m1E01-2-0101 | 671.278 | OEt | a2 | B12 | b2 | C33 | c3 | D02 | d1 | E01 |
| 2-1-m1E01-2-0102 | 671.311 | OEt | a2 | B15 | b2 | C03 | c1 | D01 | d1 | E01 |
| 2-1-m1E01-2-0103 | 671.311 | OEt | a2 | B13 | b2 | C05 | c1 | D01 | d1 | E01 |
| 2-1-m1E01-2-0104 | 672.295 | OEt | a2 | B16 | b2 | C02 | c1 | D01 | d1 | E01 |
| 2-1-m1E01-2-0105 | 673.290 | OEt | a2 | B16 | b2 | C03 | c1 | D01 | d1 | E01 |
| 2-1-m1E01-2-0106 | 674.290 | OEt | a2 | B13 | b2 | C04 | c1 | D01 | d1 | E01 |
| 2-1-m1E01-2-0107 | 674.347 | OEt | a2 | B11 | b2 | C07 | c1 | D01 | d1 | E01 |
| 2-1-m1E01-2-0108 | 675.286 | OEt | a2 | B14 | b2 | C05 | c1 | D01 | d1 | E01 |
| 2-1-m1E01-2-0109 | 675.306 | OEt | a1 | B03 | b2 | C02 | c1 | D01 | d1 | E01 |
| 2-1-m1E01-2-0110 | 675.342 | OEt | a2 | B12 | b2 | C07 | c1 | D01 | d1 | E01 |
| 2-1-m1E01-2-0111 | 676.228 | OEt | a1 | B07 | b1 | C12 | c1 | D01 | d1 | E01 |
| 2-1-m1E01-2-0112 | 676.272 | OEt | a2 | B15 | b2 | C06 | c1 | D01 | d1 | E01 |

(continued)

| ID | Exact Mass | ☒ | a | B | b | C | c | D | d | E |
|---|---|---|---|---|---|---|---|---|---|---|
| 2-1-m1E01-2-0113 | 676.301 | OEt | a1 | B03 | b2 | C03 | c1 | D01 | d1 | E01 |
| 2-1-m1E01-2-0114 | 676.301 | OEt | a1 | B01 | b2 | C05 | c1 | D01 | d1 | E01 |
| 2-1-m1E01-2-0115 | 677.296 | OEt | a1 | B02 | b2 | C05 | c1 | D01 | d1 | E01 |
| 2-1-m1E01-2-0116 | 678.251 | OEt | a2 | B16 | b2 | C06 | c1 | D01 | d1 | E01 |
| 2-1-m1E01-2-0117 | 678.251 | OEt | a2 | B11 | b3 | C08 | c1 | D01 | d1 | E01 |
| 2-1-m1E01-2-0118 | 678.251 | OEt | a2 | B11 | b1 | C08 | c2 | D02 | d1 | E01 |
| 2-1-m1E01-2-0119 | 678.265 | OEt | a2 | B17 | b2 | C02 | c1 | D01 | d1 | E01 |
| 2-1-m1E01-2-0120 | 678.265 | OEt | a2 | B14 | b2 | C04 | c1 | D01 | d1 | E01 |
| 2-1-m1E01-2-0121 | 679.246 | OEt | a2 | B12 | b3 | C08 | c1 | D01 | d1 | E01 |
| 2-1-m1E01-2-0122 | 679.246 | OEt | a2 | B12 | b1 | C08 | c2 | D02 | d1 | E01 |
| 2-1-m1E01-2-0123 | 679.246 | OEt | a2 | B11 | b1 | C11 | c2 | D02 | d1 | E01 |
| 2-1-m1E01-2-0124 | 679.261 | OEt | a2 | B17 | b2 | C03 | c1 | D01 | d1 | E01 |
| 2-1-m1E01-2-0125 | 679.281 | OEt | a1 | B04 | b2 | C02 | c1 | D01 | d1 | E01 |
| 2-1-m1E01-2-0126 | 679.281 | OEt | a1 | B01 | b2 | C04 | c1 | D01 | d1 | E01 |
| 2-1-m1E01-2-0127 | 679.301 | OEt | a1 | B05 | b2 | C01 | c1 | D01 | d1 | E01 |
| 2-1-m1E01-2-0128 | 680.242 | OEt | a2 | B11 | b1 | C10 | c2 | D02 | d1 | E01 |
| 2-1-m1E01-2-0129 | 680.242 | OEt | a2 | B12 | b1 | C11 | c2 | D02 | d1 | E01 |
| 2-1-m1E01-2-0130 | 680.276 | OEt | a1 | B02 | b2 | C04 | c1 | D01 | d1 | E01 |
| 2-1-m1E01-2-0131 | 680.276 | OEt | a1 | B04 | b2 | C03 | c1 | D01 | d1 | E01 |
| 2-1-m1E01-2-0132 | 680.311 | OEt | a2 | B13 | b1 | C13 | c1 | D01 | d1 | E01 |
| 2-1-m1E01-2-0133 | 681.237 | OEt | a2 | B12 | b1 | C10 | c2 | D02 | d1 | E01 |
| 2-1-m1E01-2-0134 | 681.262 | OEt | a1 | B03 | b2 | C06 | c1 | D01 | d1 | E01 |
| 2-1-m1E01-2-0135 | 681.280 | OEt | a1 | B06 | b2 | C01 | c1 | D01 | d1 | E01 |
| 2-1-m1E01-2-0136 | 682.246 | OEt | a2 | B13 | b3 | C15 | c1 | D01 | d1 | E01 |
| 2-1-m1E01-2-0137 | 682.277 | OEt | a2 | B19 | b1 | C08 | c1 | D01 | d1 | E01 |
| 2-1-m1E01-2-0138 | 682.277 | OEt | a2 | B11 | b1 | C09 | c1 | D01 | d1 | E01 |
| 2-1-m1E01-2-0139 | 682.279 | OEt | a2 | B18 | b2 | C01 | c1 | D01 | d1 | E01 |
| 2-1-m1E01-2-0140 | 683.272 | OEt | a2 | B12 | b1 | C09 | c1 | D01 | d1 | E01 |
| 2-1-m1E01-2-0141 | 683.272 | OEt | a2 | B19 | b1 | C11 | c1 | D01 | d1 | E01 |
| 2-1-m1E01-2-0142 | 684.208 | OEt | a2 | B11 | b3 | C06 | c1 | D01 | d1 | E01 |
| 2-1-m1E01-2-0143 | 684.222 | OEt | a2 | B17 | b2 | C06 | c1 | D01 | d1 | E01 |
| 2-1-m1E01-2-0144 | 684.267 | OEt | a2 | B19 | b1 | C10 | c1 | D01 | d1 | E01 |
| 2-1-m1E01-2-0145 | 684.277 | OEt | a2 | B13 | b1 | C14 | c1 | D01 | d1 | E01 |
| 2-1-m1E01-2-0146 | 684.286 | OEt | a2 | B14 | b1 | C13 | c1 | D01 | d1 | E01 |
| 2-1-m1E01-2-0147 | 684.298 | OEt | a2 | B13 | b2 | C33 | c3 | D02 | d1 | E01 |
| 2-1-m1E01-2-0148 | 685.203 | OEt | a2 | B12 | b3 | C06 | c1 | D01 | d1 | E01 |
| 2-1-m1E01-2-0149 | 685.203 | OEt | a2 | B11 | b1 | C12 | c2 | D02 | d1 | E01 |

(continued)

| ID | Exact Mass | ⊠ | a | B | b | C | c | D | d | E |
|---|---|---|---|---|---|---|---|---|---|---|
| 2-1-m1E01-2-0150 | 685.237 | OEt | a1 | B04 | b2 | C06 | c1 | D01 | d1 | E01 |
| 2-1-m1E01-2-0151 | 685.301 | OEt | a1 | B01 | b1 | C13 | c1 | D01 | d1 | E01 |
| 2-1-m1E01-2-0152 | 685.326 | OEt | a2 | B15 | b2 | C05 | c1 | D01 | d1 | E01 |
| 2-1-m1E01-2-0153 | 686.198 | OEt | a2 | B12 | b1 | C12 | c2 | D02 | d1 | E01 |
| 2-1-m1E01-2-0154 | 686.221 | OEt | a2 | B14 | b3 | C15 | c1 | D01 | d1 | E01 |
| 2-1-m1E01-2-0155 | 686.297 | OEt | a1 | B02 | b1 | C13 | c1 | D01 | d1 | E01 |
| 2-1-m1E01-2-0156 | 687.236 | OEt | a1 | B01 | b3 | C15 | c1 | D01 | d1 | E01 |
| 2-1-m1E01-2-0157 | 687.250 | OEt | a1 | B07 | b2 | C01 | c1 | D01 | d1 | E01 |
| 2-1-m1E01-2-0158 | 687.306 | OEt | a2 | B16 | b2 | C05 | c1 | D01 | d1 | E01 |
| 2-1-m1E01-2-0159 | 688.232 | OEt | a1 | B02 | b3 | C15 | c1 | D01 | d1 | E01 |
| 2-1-m1E01-2-0160 | 688.252 | OEt | a2 | B14 | b1 | C14 | c1 | D01 | d1 | E01 |
| 2-1-m1E01-2-0161 | 688.273 | OEt | a2 | B14 | b2 | C33 | c3 | D02 | d1 | E01 |
| 2-1-m1E01-2-0162 | 688.306 | OEt | a2 | B15 | b2 | C04 | c1 | D01 | d1 | E01 |
| 2-1-m1E01-2-0163 | 688.362 | OEt | a2 | B13 | b2 | C07 | c1 | D01 | d1 | E01 |
| 2-1-m1E01-2-0164 | 689.228 | OEt | a2 | B19 | b1 | C12 | c1 | D01 | d1 | E01 |
| 2-1-m1E01-2-0165 | 689.267 | OEt | a1 | B01 | b1 | C14 | c1 | D01 | d1 | E01 |
| 2-1-m1E01-2-0166 | 689.288 | OEt | a1 | B01 | b2 | C33 | c3 | D02 | d1 | E01 |
| 2-1-m1E01-2-0167 | 689.321 | OEt | a1 | B05 | b2 | C02 | c1 | D01 | d1 | E01 |
| 2-1-m1E01-2-0168 | 690.262 | OEt | a1 | B08 | b1 | C08 | c1 | D01 | d1 | E01 |
| 2-1-m1E01-2-0169 | 690.262 | OEt | a1 | B02 | b1 | C14 | c1 | D01 | d1 | E01 |
| 2-1-m1E01-2-0170 | 690.284 | OEt | a1 | B02 | b2 | C33 | c3 | D02 | d1 | E01 |
| 2-1-m1E01-2-0171 | 690.285 | OEt | a2 | B16 | b2 | C04 | c1 | D01 | d1 | E01 |
| 2-1-m1E01-2-0172 | 690.288 | OEt | a2 | B11 | b1 | C30 | c3 | D02 | d1 | E01 |
| 2-1-m1E01-2-0173 | 690.317 | OEt | a1 | B05 | b2 | C03 | c1 | D01 | d1 | E01 |
| 2-1-m1E01-2-0174 | 690.317 | OEt | a1 | B03 | b2 | C05 | c1 | D01 | d1 | E01 |
| 2-1-m1E01-2-0175 | 691.258 | OEt | a1 | B08 | b1 | C11 | c1 | D01 | d1 | E01 |
| 2-1-m1E01-2-0176 | 691.283 | OEt | a2 | B12 | b1 | C30 | c3 | D02 | d1 | E01 |
| 2-1-m1E01-2-0177 | 691.301 | OEt | a1 | B06 | b2 | C02 | c1 | D01 | d1 | E01 |
| 2-1-m1E01-2-0178 | 692.253 | OEt | a1 | B08 | b1 | C10 | c1 | D01 | d1 | E01 |
| 2-1-m1E01-2-0179 | 692.267 | OEt | a2 | B13 | b3 | C08 | c1 | D01 | d1 | E01 |
| 2-1-m1E01-2-0180 | 692.267 | OEt | a2 | B13 | b1 | C08 | c2 | D02 | d1 | E01 |
| 2-1-m1E01-2-0181 | 692.267 | OEt | a2 | B11 | b2 | C20 | c3 | D02 | d1 | E01 |
| 2-1-m1E01-2-0182 | 692.296 | OEt | a1 | B06 | b2 | C03 | c1 | D01 | d1 | E01 |
| 2-1-m1E01-2-0183 | 692.300 | OEt | a2 | B18 | b2 | C02 | c1 | D01 | d1 | E01 |
| 2-1-m1E01-2-0184 | 692.337 | OEt | a2 | B14 | b2 | C07 | c1 | D01 | d1 | E01 |
| 2-1-m1E01-2-0185 | 693.262 | OEt | a2 | B13 | b1 | C11 | c2 | D02 | d1 | E01 |
| 2-1-m1E01-2-0186 | 693.262 | OEt | a2 | B12 | b2 | C20 | c3 | D02 | d1 | E01 |

(continued)

| ID | Exact Mass | ⊠ | a | B | b | C | c | D | d | E |
|---|---|---|---|---|---|---|---|---|---|---|
| 2-1-m1E01-2-0187 | 693.262 | OEt | a2 | B11 | b2 | C28 | c3 | D02 | d1 | E01 |
| 2-1-m1E01-2-0188 | 693.276 | OEt | a2 | B17 | b2 | C05 | c1 | D01 | d1 | E01 |
| 2-1-m1E01-2-0189 | 693.295 | OEt | a2 | B18 | b2 | C03 | c1 | D01 | d1 | E01 |
| 2-1-m1E01-2-0190 | 693.296 | OEt | a1 | B03 | b2 | C04 | c1 | D01 | d1 | E01 |
| 2-1-m1E01-2-0191 | 693.353 | OEt | a1 | B01 | b2 | C07 | c1 | D01 | d1 | E01 |
| 2-1-m1E01-2-0192 | 694.257 | OEt | a2 | B13 | b1 | C10 | c2 | D02 | d1 | E01 |
| 2-1-m1E01-2-0193 | 694.257 | OEt | a2 | B12 | b2 | C28 | c3 | D02 | d1 | E01 |
| 2-1-m1E01-2-0194 | 694.292 | OEt | a1 | B04 | b2 | C05 | c1 | D01 | d1 | E01 |
| 2-1-m1E01-2-0195 | 694.327 | OEt | a2 | B15 | b1 | C13 | c1 | D01 | d1 | E01 |
| 2-1-m1E01-2-0196 | 694.348 | OEt | a1 | B02 | b2 | C07 | c1 | D01 | d1 | E01 |
| 2-1-m1E01-2-0197 | 695.278 | OEt | a1 | B05 | b2 | C06 | c1 | D01 | d1 | E01 |
| 2-1-m1E01-2-0198 | 696.225 | OEt | a2 | B11 | b2 | C01 | c2 | D02 | d1 | E01 |
| 2-1-m1E01-2-0199 | 696.242 | OEt | a2 | B14 | b3 | C08 | c1 | D01 | d1 | E01 |
| 2-1-m1E01-2-0200 | 696.242 | OEt | a2 | B14 | b1 | C08 | c2 | D02 | d1 | E01 |
| 2-1-m1E01-2-0201 | 696.256 | OEt | a2 | B17 | b2 | C04 | c1 | D01 | d1 | E01 |
| 2-1-m1E01-2-0202 | 696.262 | OEt | a2 | B15 | b3 | C15 | c1 | D01 | d1 | E01 |
| 2-1-m1E01-2-0203 | 696.292 | OEt | a2 | B13 | b1 | C09 | c1 | D01 | d1 | E01 |
| 2-1-m1E01-2-0204 | 696.306 | OEt | a2 | B16 | b1 | C13 | c1 | D01 | d1 | E01 |
| 2-1-m1E01-2-0205 | 697.214 | OEt | a1 | B08 | b1 | C12 | c1 | D01 | d1 | E01 |
| 2-1-m1E01-2-0206 | 697.221 | OEt | a2 | B12 | b2 | C01 | c2 | D02 | d1 | E01 |
| 2-1-m1E01-2-0207 | 697.237 | OEt | a2 | B14 | b1 | C11 | c2 | D02 | d1 | E01 |
| 2-1-m 1 E01-2-0208 | 697.257 | OEt | a1 | B06 | b2 | C06 | c1 | D01 | d1 | E01 |
| 2-1-m1E01-2-0209 | 697.257 | OEt | a1 | B01 | b3 | C08 | c1 | D01 | d1 | E01 |
| 2-1-m1E01-2-0210 | 697.257 | OEt | a1 | B01 | b1 | C08 | c2 | D02 | d1 | E01 |
| 2-1-m1E01-2-0211 | 697.271 | OEt | a1 | B07 | b2 | C02 | c1 | D01 | d1 | E01 |
| 2-1-m1E01-2-0212 | 697.271 | OEt | a1 | B04 | b2 | C04 | c1 | D01 | d1 | E01 |
| 2-1-m1E01-2-0213 | 698.223 | OEt | a2 | B13 | b3 | C06 | c1 | D01 | d1 | E01 |
| 2-1-m1E01-2-0214 | 698.232 | OEt | a2 | B14 | b1 | C10 | c2 | D02 | d1 | E01 |
| 2-1-m1E01-2-0215 | 698.241 | OEt | a2 | B16 | b3 | C15 | c1 | D01 | d1 | E01 |
| 2-1-m1E01-2-0216 | 698.252 | OEt | a1 | B02 | b3 | C08 | c1 | D01 | d1 | E01 |
| 2-1-m1E01-2-0217 | 698.252 | OEt | a1 | B02 | b1 | C08 | c2 | D02 | d1 | E01 |
| 2-1-m1E01-2-0218 | 698.252 | OEt | a1 | B01 | b1 | C11 | c2 | D02 | d1 | E01 |
| 2-1-m1E01-2-0219 | 698.256 | OEt | a2 | B18 | b2 | C06 | c1 | D01 | d1 | E01 |
| 2-1-m1E01-2-0220 | 698.266 | OEt | a1 | B07 | b2 | C03 | c1 | D01 | d1 | E01 |
| 2-1-m1E01-2-0221 | 698.272 | OEt | a2 | B20 | b1 | C08 | c1 | D01 | d1 | E01 |
| 2-1-m1E01-2-0222 | 698.293 | OEt | a2 | B15 | b1 | C14 | c1 | D01 | d1 | E01 |
| 2-1-m1E01-2-0223 | 698.314 | OEt | a2 | B15 | b2 | C33 | c3 | D02 | d1 | E01 |

(continued)

| ID | Exact Mass | ☒ | a | B | b | C | c | D | d | E |
|---|---|---|---|---|---|---|---|---|---|---|
| 2-1-m1E01-2-0224 | 699.219 | OEt | a2 | B13 | b1 | C12 | c2 | D02 | d1 | E01 |
| 2-1-m1E01-2-0225 | 699.248 | OEt | a1 | B01 | b1 | C10 | c2 | D02 | d1 | E01 |
| 2-1-m1E01-2-0226 | 699.248 | OEt | a1 | B02 | b1 | C11 | c2 | D02 | d1 | E01 |
| 2-1-m1E01-2-0227 | 699.267 | OEt | a2 | B20 | b1 | C11 | c1 | D01 | d1 | E01 |
| 2-1-m1E01-2-0228 | 699.317 | OEt | a1 | B03 | b1 | C13 | c1 | D01 | d1 | E01 |
| 2-1-m1E01-2-0229 | 700.243 | OEt | a1 | B02 | b1 | C10 | c2 | D02 | d1 | E01 |
| 2-1-m1E01-2-0230 | 700.251 | OEt | a2 | B19 | b2 | C01 | c1 | D01 | d1 | E01 |
| 2-1-m1E01-2-0231 | 700.262 | OEt | a2 | B20 | b1 | C10 | c1 | D01 | d1 | E01 |
| 2-1-m1E01-2-0232 | 700.267 | OEt | a2 | B14 | b1 | C09 | c1 | D01 | d1 | E01 |
| 2-1-m1E01-2-0233 | 700.272 | OEt | a2 | B16 | b1 | C14 | c1 | D01 | d1 | E01 |
| 2-1-m1E01-2-0234 | 700.293 | OEt | a2 | B16 | b2 | C33 | c3 | D02 | d1 | E01 |
| 2-1-m1E01-2-0235 | 701.252 | OEt | a1 | B03 | b3 | C15 | c1 | D01 | d1 | E01 |
| 2-1-m1E01-2-0236 | 701.283 | OEt | a1 | B09 | b1 | C08 | c1 | D01 | d1 | E01 |
| 2-1-m1E01-2-0237 | 701.283 | OEt | a1 | B01 | b1 | C09 | c1 | D01 | d1 | E01 |
| 2-1-m1E01-2-0238 | 702.198 | OEt | a2 | B14 | b3 | C06 | c1 | D01 | d1 | E01 |
| 2-1-m1E01-2-0239 | 702.277 | OEt | a2 | B17 | b1 | C13 | c1 | D01 | d1 | E01 |
| 2-1-m1E01-2-0240 | 702.278 | OEt | a1 | B02 | b1 | C09 | c1 | D01 | d1 | E01 |
| 2-1-m1E01-2-0241 | 702.278 | OEt | a1 | B09 | b1 | C11 | c1 | D01 | d1 | E01 |
| 2-1-m1E01-2-0242 | 702.378 | OEt | a2 | B15 | b2 | C07 | c1 | D01 | d1 | E01 |
| 2-1-m1E01-2-0243 | 703.193 | OEt | a2 | B14 | b1 | C12 | c2 | D02 | d1 | E01 |
| 2-1-m1E01-2-0244 | 703.213 | OEt | a1 | B01 | b3 | C06 | c1 | D01 | d1 | E01 |
| 2-1-m1E01-2-0245 | 703.228 | OEt | a1 | B07 | b2 | C06 | c1 | D01 | d1 | E01 |
| 2-1-m1E01-2-0246 | 703.273 | OEt | a1 | B09 | b1 | C10 | c1 | D01 | d1 | E01 |
| 2-1-m1E01-2-0247 | 703.283 | OEt | a1 | B03 | b1 | C14 | c1 | D01 | d1 | E01 |
| 2-1-m1E01-2-0248 | 703.292 | OEt | a1 | B04 | b1 | C13 | c1 | D01 | d1 | E01 |
| 2-1-m1E01-2-0249 | 703.304 | OEt | a1 | B03 | b2 | C33 | c3 | D02 | d1 | E01 |
| 2-1-m1E01-2-0250 | 704.209 | OEt | a1 | B02 | b3 | C06 | c1 | D01 | d1 | E01 |
| 2-1-m1E01-2-0251 | 704.209 | OEt | a1 | B01 | b1 | C12 | c2 | D02 | d1 | E01 |
| 2-1-m1E01-2-0252 | 704.212 | OEt | a2 | B17 | b3 | C15 | c1 | D01 | d1 | E01 |
| 2-1-m1E01-2-0253 | 704.303 | OEt | a2 | B13 | b1 | C30 | c3 | D02 | d1 | E01 |
| 2-1-m1E01-2-0254 | 704.303 | OEt | a2 | B11 | b1 | C32 | c3 | D02 | d1 | E01 |
| 2-1-m1E01-2-0255 | 704.332 | OEt | a1 | B05 | b2 | C05 | c1 | D01 | d1 | E01 |
| 2-1-m1E01-2-0256 | 704.357 | OEt | a2 | B16 | b2 | C07 | c1 | D01 | d1 | E01 |
| 2-1-m1E01-2-0257 | 705.204 | OEt | a1 | B02 | b1 | C12 | c2 | D02 | d1 | E01 |
| 2-1-m1E01-2-0258 | 705.223 | OEt | a2 | B20 | b1 | C12 | c1 | D01 | d1 | E01 |
| 2-1-m1E01-2-0259 | 705.227 | OEt | a1 | B04 | b3 | C15 | c1 | D01 | d1 | E01 |
| 2-1-m1E01-2-0260 | 705.298 | OEt | a2 | B12 | b1 | C32 | c3 | D02 | d1 | E01 |

(continued)

| ID | Exact Mass | ☒ | a | B | b | C | c | D | d | E |
|---|---|---|---|---|---|---|---|---|---|---|
| 2-1-m1E01-2-0261 | 706.243 | OEt | a2 | B17 | b1 | C14 | c1 | D01 | d1 | E01 |
| 2-1-m1E01-2-0262 | 706.246 | OEt | a2 | B11 | b2 | C02 | c2 | D02 | d1 | E01 |
| 2-1-m1E01-2-0263 | 706.264 | OEt | a2 | B17 | b2 | C33 | c3 | D02 | d1 | E01 |
| 2-1-m1E01-2-0264 | 706.283 | OEt | a2 | B15 | b3 | C08 | c1 | D01 | d1 | E01 |
| 2-1-m1E01-2-0265 | 706.283 | OEt | a2 | B15 | b1 | C08 | c2 | D02 | d1 | E01 |
| 2-1-m1E01-2-0266 | 706.283 | OEt | a2 | B13 | b2 | C20 | c3 | D02 | d1 | E01 |
| 2-1-m1E01-2-0267 | 706.283 | OEt | a2 | B11 | b2 | C22 | c3 | D02 | d1 | E01 |
| 2-1-m1E01-2-0268 | 706.311 | OEt | a1 | B06 | b2 | C05 | c1 | D01 | d1 | E01 |
| 2-1-m1E01-2-0269 | 707.241 | OEt | a2 | B12 | b2 | C02 | c2 | D02 | d1 | E01 |
| 2-1-m1E01-2-0270 | 707.241 | OEt | a2 | B11 | b2 | C03 | c2 | D02 | d1 | E01 |
| 2-1-m1E01-2-0271 | 707.258 | OEt | a1 | B04 | b1 | C14 | c1 | D01 | d1 | E01 |
| 2-1-m1E01-2-0272 | 707.278 | OEt | a2 | B15 | b1 | C11 | c2 | D02 | d1 | E01 |
| 2-1-m1E01-2-0273 | 707.278 | OEt | a2 | B12 | b2 | C22 | c3 | D02 | d1 | E01 |
| 2-1-m1E01-2-0274 | 707.278 | OEt | a2 | B13 | b2 | C28 | c3 | D02 | d1 | E01 |
| 2-1-m1E01-2-0275 | 707.279 | OEt | a1 | B04 | b2 | C33 | c3 | D02 | d1 | E01 |
| 2-1-m1E01-2-0276 | 707.311 | OEt | a2 | B18 | b2 | C05 | c1 | D01 | d1 | E01 |
| 2-1-m1E01-2-0277 | 707.312 | OEt | a1 | B05 | b2 | C04 | c1 | D01 | d1 | E01 |
| 2-1-m1E01-2-0278 | 707.368 | OEt | a1 | B03 | b2 | C07 | c1 | D01 | d1 | E01 |
| 2-1-m1E01-2-0279 | 708.234 | OEt | a1 | B09 | b1 | C12 | c1 | D01 | d1 | E01 |
| 2-1-m1E01-2-0280 | 708.237 | OEt | a1 | B08 | b2 | C01 | c1 | D01 | d1 | E01 |
| 2-1-m1E01-2-0281 | 708.237 | OEt | a2 | B12 | b2 | C03 | c2 | D02 | d1 | E01 |
| 2-1-m1E01-2-0282 | 708.262 | OEt | a2 | B16 | b3 | C08 | c1 | D01 | d1 | E01 |
| 2-1-m1E01-2-0283 | 708.262 | OEt | a2 | B16 | b1 | C08 | c2 | D02 | d1 | E01 |
| 2-1-m1E01-2-0284 | 708.273 | OEt | a2 | B15 | b1 | C10 | c2 | D02 | d1 | E01 |
| 2-1-m1E01-2-0285 | 708.278 | OEt | a2 | B14 | b1 | C30 | c3 | D02 | d1 | E01 |
| 2-1-m1E01-2-0286 | 709.257 | OEt | a2 | B16 | b1 | C11 | c2 | D02 | d1 | E01 |
| 2-1-m1E01-2-0287 | 709.291 | OEt | a1 | B06 | b2 | C04 | c1 | D01 | d1 | E01 |
| 2-1-m1E01-2-0288 | 709.293 | OEt | a1 | B01 | b1 | C30 | c3 | D02 | d1 | E01 |
| 2-1-m1E01-2-0289 | 710.241 | OEt | a2 | B13 | b2 | C01 | c2 | D02 | d1 | E01 |
| 2-1-m1E01-2-0290 | 710.252 | OEt | a2 | B16 | b1 | C10 | c2 | D02 | d1 | E01 |
| 2-1-m1E01-2-0291 | 710.257 | OEt | a2 | B14 | b2 | C20 | c3 | D02 | d1 | E01 |
| 2-1-m1E01-2-0292 | 710.257 | OEt | a2 | B11 | b2 | C24 | c3 | D02 | d1 | E01 |
| 2-1-m1E01-2-0293 | 710.272 | OEt | a2 | B19 | b2 | C02 | c1 | D01 | d1 | E01 |
| 2-1-m1E01-2-0294 | 710.289 | OEt | a1 | B02 | b1 | C30 | c3 | D02 | d1 | E01 |
| 2-1-m1E01-2-0295 | 710.290 | OEt | a2 | B18 | b2 | C04 | c1 | D01 | d1 | E01 |
| 2-1-m1E01-2-0296 | 710.308 | OEt | a2 | B15 | b1 | C09 | c1 | D01 | d1 | E01 |
| 2-1-m1E01-2-0297 | 710.328 | OEt | a2 | B17 | b2 | C07 | c1 | D01 | d1 | E01 |

(continued)

| ID | Exact Mass | ☒ | a | B | b | C | c | D | d | E |
|---|---|---|---|---|---|---|---|---|---|---|
| 2-1-m1E01-2-0298 | 711.253 | OEt | a2 | B12 | b2 | C24 | c3 | D02 | d1 | E01 |
| 2-1-m1E01-2-0299 | 711.253 | OEt | a2 | B14 | b2 | C28 | c3 | D02 | d1 | E01 |
| 2-1-m1E01-2-0300 | 711.267 | OEt | a2 | B19 | b2 | C03 | c1 | D01 | d1 | E01 |
| 2-1-m1E01-2-0301 | 711.273 | OEt | a1 | B03 | b3 | C08 | c1 | D01 | d1 | E01 |
| 2-1-m1E01-2-0302 | 711.273 | OEt | a1 | B03 | b1 | C08 | c2 | D02 | d1 | E01 |
| 2-1-m1E01-2-0303 | 711.273 | OEt | a1 | B01 | b2 | C20 | c3 | D02 | d1 | E01 |
| 2-1-m1E01-2-0304 | 711.343 | OEt | a1 | B04 | b2 | C07 | c1 | D01 | d1 | E01 |
| 2-1-m1E01-2-0305 | 712.203 | OEt | a2 | B11 | b2 | C06 | c2 | D02 | d1 | E01 |
| 2-1-m1E01-2-0306 | 712.239 | OEt | a2 | B15 | b3 | C06 | c1 | D01 | d1 | E01 |
| 2-1-m1E01-2-0307 | 712.268 | OEt | a1 | B03 | b1 | C11 | c2 | D02 | d1 | E01 |
| 2-1-m1E01-2-0308 | 712.268 | OEt | a1 | B02 | b2 | C20 | c3 | D02 | d1 | E01 |
| 2-1-m1E01-2-0309 | 712.268 | OEt | a1 | B01 | b2 | C28 | c3 | D02 | d1 | E01 |
| 2-1-m1E01-2-0310 | 712.282 | OEt | a1 | B07 | b2 | C05 | c1 | D01 | d1 | E01 |
| 2-1-m1E01-2-0311 | 712.287 | OEt | a2 | B16 | b1 | C09 | c1 | D01 | d1 | E01 |
| 2-1-m1E01-2-0312 | 713.198 | OEt | a2 | B12 | b2 | C06 | c2 | D02 | d1 | E01 |
| 2-1-m1E01-2-0313 | 713.234 | OEt | a2 | B15 | b1 | C12 | c2 | D02 | d1 | E01 |
| 2-1-m1E01-2-0314 | 713.263 | OEt | a1 | B03 | b1 | C10 | c2 | D02 | d1 | E01 |
| 2-1-m1E01-2-0315 | 713.263 | OEt | a1 | B02 | b2 | C28 | c3 | D02 | d1 | E01 |
| 2-1-m1E01-2-0316 | 713.333 | OEt | a1 | B05 | b1 | C13 | c1 | D01 | d1 | E01 |
| 2-1-m1E01-2-0317 | 714.216 | OEt | a2 | B14 | b2 | C01 | c2 | D02 | d1 | E01 |
| 2-1-m1E01-2-0318 | 714.218 | OEt | a2 | B16 | b3 | C06 | c1 | D01 | d1 | E01 |
| 2-1-m1E01-2-0319 | 714.232 | OEt | a2 | B17 | b3 | C08 | c1 | D01 | d1 | E01 |
| 2-1-m1E01-2-0320 | 714.232 | OEt | a2 | B17 | b1 | C08 | c2 | D02 | d1 | E01 |
| 2-1-m1E01-2-0321 | 715.213 | OEt | a2 | B16 | b1 | C12 | c2 | D02 | d1 | E01 |
| 2-1-m1E01-2-0322 | 715.228 | OEt | a2 | B17 | b1 | C11 | c2 | D02 | d1 | E01 |
| 2-1-m1E01-2-0323 | 715.231 | OEt | a1 | B01 | b2 | C01 | c2 | D02 | d1 | E01 |
| 2-1-m1E01-2-0324 | 715.248 | OEt | a1 | B04 | b3 | C08 | c1 | D01 | d1 | E01 |
| 2-1-m1E01-2-0325 | 715.248 | OEt | a1 | B04 | b1 | C08 | c2 | D02 | d1 | E01 |
| 2-1-m1E01-2-0326 | 715.262 | OEt | a1 | B07 | b2 | C04 | c1 | D01 | d1 | E01 |
| 2-1-m1E01-2-0327 | 715.268 | OEt | a1 | B05 | b3 | C15 | c1 | D01 | d1 | E01 |
| 2-1-m1E01-2-0328 | 715.298 | OEt | a1 | B03 | b1 | C09 | c1 | D01 | d1 | E01 |
| 2-1-m1E01-2-0329 | 715.312 | OEt | a1 | B06 | b1 | C13 | c1 | D01 | d1 | E01 |
| 2-1-m1E01-2-0330 | 716.223 | OEt | a2 | B17 | b1 | C10 | c2 | D02 | d1 | E01 |
| 2-1-m1E01-2-0331 | 716.226 | OEt | a1 | B02 | b2 | C01 | c2 | D02 | d1 | E01 |
| 2-1-m1E01-2-0332 | 716.228 | OEt | a2 | B19 | b2 | C06 | c1 | D01 | d1 | E01 |
| 2-1-m1E01-2-0333 | 716.243 | OEt | a1 | B04 | b1 | C11 | c2 | D02 | d1 | E01 |
| 2-1-m1E01-2-0334 | 716.246 | OEt | a2 | B20 | b2 | C01 | c1 | D01 | d1 | E01 |

(continued)

| ID | Exact Mass | ☒ | a | B | b | C | c | D | d | E |
|---|---|---|---|---|---|---|---|---|---|---|
| 2-1-m1E01-2-0335 | 716.311 | OEt | a2 | B18 | b1 | C13 | c1 | D01 | d1 | E01 |
| 2-1-m1E01-2-0336 | 717.229 | OEt | a1 | B03 | b3 | C06 | c1 | D01 | d1 | E01 |
| 2-1-m1E01-2-0337 | 717.238 | OEt | a1 | B04 | b1 | C10 | c2 | D02 | d1 | E01 |
| 2-1-m1E01-2-0338 | 717.247 | OEt | a1 | B06 | b3 | C15 | c1 | D01 | d1 | E01 |
| 2-1-m1E01-2-0339 | 717.277 | OEt | a1 | B10 | b1 | C08 | c1 | D01 | d1 | E01 |
| 2-1-m1E01-2-0340 | 717.298 | OEt | a1 | B05 | b1 | C14 | c1 | D01 | d1 | E01 |
| 2-1-m1E01-2-0341 | 717.320 | OEt | a1 | B05 | b2 | C33 | c3 | D02 | d1 | E01 |
| 2-1-m1E01-2-0342 | 718.224 | OEt | a1 | B03 | b1 | C12 | c2 | D02 | d1 | E01 |
| 2-1-m1E01-2-0343 | 718.246 | OEt | a2 | B18 | b3 | C15 | c1 | D01 | d1 | E01 |
| 2-1-m1E01-2-0344 | 718.257 | OEt | a1 | B08 | b2 | C02 | c1 | D01 | d1 | E01 |
| 2-1-m1E01-2-0345 | 718.258 | OEt | a2 | B17 | b1 | C09 | c1 | D01 | d1 | E01 |
| 2-1-m1E01-2-0346 | 718.273 | OEt | a1 | B10 | b1 | C11 | c1 | D01 | d1 | E01 |
| 2-1-m1E01-2-0347 | 718.319 | OEt | a2 | B15 | b1 | C30 | c3 | D02 | d1 | E01 |
| 2-1-m1E01-2-0348 | 718.319 | OEt | a2 | B11 | b1 | C29 | c3 | D02 | d1 | E01 |
| 2-1-m1E01-2-0349 | 718.319 | OEt | a2 | B13 | b1 | C32 | c3 | D02 | d1 | E01 |
| 2-1-m1E01-2-0350 | 719.253 | OEt | a1 | B08 | b2 | C03 | c1 | D01 | d1 | E01 |
| 2-1-m1E01-2-0351 | 719.257 | OEt | a1 | B09 | b2 | C01 | c1 | D01 | d1 | E01 |
| 2-1-m1E01-2-0352 | 719.268 | OEt | a1 | B10 | b1 | C10 | c1 | D01 | d1 | E01 |
| 2-1-m1E01-2-0353 | 719.273 | OEt | a1 | B04 | b1 | C09 | c1 | D01 | d1 | E01 |
| 2-1-m1E01-2-0354 | 719.278 | OEt | a1 | B06 | b1 | C14 | c1 | D01 | d1 | E01 |
| 2-1-m1E01-2-0355 | 719.299 | OEt | a1 | B06 | b2 | C33 | c3 | D02 | d1 | E01 |
| 2-1-m1E01-2-0356 | 719.314 | OEt | a2 | B12 | b1 | C29 | c3 | D02 | d1 | E01 |
| 2-1-m1E01-2-0357 | 720.189 | OEt | a2 | B17 | b3 | C06 | c1 | D01 | d1 | E01 |
| 2-1-m1E01-2-0358 | 720.262 | OEt | a2 | B13 | b2 | C02 | c2 | D02 | d1 | E01 |
| 2-1-m1E01-2-0359 | 720.277 | OEt | a2 | B18 | b1 | C14 | c1 | D01 | d1 | E01 |
| 2-1-m1E01-2-0360 | 720.298 | OEt | a2 | B15 | b2 | C20 | c3 | D02 | d1 | E01 |
| 2-1-m1E01-2-0361 | 720.298 | OEt | a2 | B13 | b2 | C22 | c3 | D02 | d1 | E01 |
| 2-1-m1E01-2-0362 | 720.298 | OEt | a2 | B16 | b1 | C30 | c3 | D02 | d1 | E01 |
| 2-1-m1E01-2-0363 | 720.298 | OEt | a2 | B18 | b2 | C33 | c3 | D02 | d1 | E01 |
| 2-1-m1E01-2-0364 | 721.184 | OEt | a2 | B17 | b1 | C12 | c2 | D02 | d1 | E01 |
| 2-1-m1E01-2-0365 | 721.204 | OEt | a1 | B04 | b3 | C06 | c1 | D01 | d1 | E01 |
| 2-1-m1E01-2-0366 | 721.257 | OEt | a2 | B13 | b2 | C03 | c2 | D02 | d1 | E01 |
| 2-1-m1E01-2-0367 | 721.257 | OEt | a2 | B11 | b2 | C05 | c2 | D02 | d1 | E01 |
| 2-1-m1E01-2-0368 | 721.282 | OEt | a1 | B07 | b1 | C13 | c1 | D01 | d1 | E01 |
| 2-1-m1E01-2-0369 | 721.293 | OEt | a2 | B15 | b2 | C28 | c3 | D02 | d1 | E01 |
| 2-1-m1E01-2-0370 | 721.384 | OEt | a1 | B05 | b2 | C07 | c1 | D01 | d1 | E01 |
| 2-1-m1E01-2-0371 | 722.199 | OEt | a1 | B04 | b1 | C12 | c2 | D02 | d1 | E01 |

(continued)

| ID | Exact Mass | ☒ | a | B | b | C | c | D | d | E |
|---|---|---|---|---|---|---|---|---|---|---|
| 2-1-m1E01-2-0372 | 722.252 | OEt | a2 | B12 | b2 | C05 | c2 | D02 | d1 | E01 |
| 2-1-m1E01-2-0373 | 722.277 | OEt | a2 | B16 | b2 | C20 | c3 | D02 | d1 | E01 |
| 2-1-m1E01-2-0374 | 722.277 | OEt | a2 | B11 | b2 | C26 | c3 | D02 | d1 | E01 |
| 2-1-m1E01-2-0375 | 722.294 | OEt | a2 | B14 | b1 | C32 | c3 | D02 | d1 | E01 |
| 2-1-m1E01-2-0376 | 723.217 | OEt | a1 | B07 | b3 | C15 | c1 | D01 | d1 | E01 |
| 2-1-m1E01-2-0377 | 723.273 | OEt | a2 | B12 | b2 | C26 | c3 | D02 | d1 | E01 |
| 2-1-m1E01-2-0378 | 723.273 | OEt | a2 | B16 | b2 | C28 | c3 | D02 | d1 | E01 |
| 2-1-m1E01-2-0379 | 723.309 | OEt | a1 | B03 | b1 | C30 | c3 | D02 | d1 | E01 |
| 2-1-m1E01-2-0380 | 723.309 | OEt | a1 | B01 | b1 | C32 | c3 | D02 | d1 | E01 |
| 2-1-m1E01-2-0381 | 723.363 | OEt | a1 | B06 | b2 | C07 | c1 | D01 | d1 | E01 |
| 2-1-m1E01-2-0382 | 724.214 | OEt | a1 | B08 | b2 | C06 | c1 | D01 | d1 | E01 |
| 2-1-m1E01-2-0383 | 724.229 | OEt | a1 | B10 | b1 | C12 | c1 | D01 | d1 | E01 |
| 2-1-m1E01-2-0384 | 724.237 | OEt | a2 | B14 | b2 | C02 | c2 | D02 | d1 | E01 |
| 2-1-m1E01-2-0385 | 724.237 | OEt | a2 | B11 | b2 | C04 | c2 | D02 | d1 | E01 |
| 2-1-m1E01-2-0386 | 724.257 | OEt | a2 | B15 | b2 | C01 | c2 | D02 | d1 | E01 |
| 2-1-m1E01-2-0387 | 724.273 | OEt | a2 | B14 | b2 | C22 | c3 | D02 | d1 | E01 |
| 2-1-m1E01-2-0388 | 724.273 | OEt | a2 | B13 | b2 | C24 | c3 | D02 | d1 | E01 |
| 2-1-m1E01-2-0389 | 724.304 | OEt | a1 | B02 | b1 | C32 | c3 | D02 | d1 | E01 |
| 2-1-m1E01-2-0390 | 724.362 | OEt | a2 | B18 | b2 | C07 | c1 | D01 | d1 | E01 |
| 2-1-m1E01-2-0391 | 725.232 | OEt | a2 | B12 | b2 | C04 | c2 | D02 | d1 | E01 |
| 2-1-m1E01-2-0392 | 725.232 | OEt | a2 | B14 | b2 | C03 | c2 | D02 | d1 | E01 |
| 2-1-m1E01-2-0393 | 725.248 | OEt | a1 | B07 | b1 | C14 | c1 | D01 | d1 | E01 |
| 2-1-m1E01-2-0394 | 725.252 | OEt | a1 | B01 | b2 | C02 | c2 | D02 | d1 | E01 |
| 2-1-m1E01-2-0395 | 725.269 | OEt | a1 | B07 | b2 | C33 | c3 | D02 | d1 | E01 |
| 2-1-m1E01-2-0396 | 725.283 | OEt | a2 | B19 | b2 | C05 | c1 | D01 | d1 | E01 |
| 2-1-m1E01-2-0397 | 725.288 | OEt | a1 | B05 | b3 | C08 | c1 | D01 | d1 | E01 |
| 2-1-m1E01-2-0398 | 725.288 | OEt | a1 | B05 | b1 | C08 | c2 | D02 | d1 | E01 |
| 2-1-m1E01-2-0399 | 725.288 | OEt | a1 | B03 | b2 | C20 | c3 | D02 | d1 | E01 |
| 2-1-m1E01-2-0400 | 725.288 | OEt | a1 | B01 | b2 | C22 | c3 | D02 | d1 | E01 |
| 2-1-m1E01-2-0401 | 726.218 | OEt | a2 | B13 | b2 | C06 | c2 | D02 | d1 | E01 |
| 2-1-m1E01-2-0402 | 726.236 | OEt | a2 | B16 | b2 | C01 | c2 | D02 | d1 | E01 |
| 2-1-m1E01-2-0403 | 726.247 | OEt | a1 | B02 | b2 | C02 | c2 | D02 | d1 | E01 |
| 2-1-m1E01-2-0404 | 726.247 | OEt | a1 | B01 | b2 | C03 | c2 | D02 | d1 | E01 |
| 2-1-m1E01-2-0405 | 726.267 | OEt | a2 | B20 | b2 | C02 | c1 | D01 | d1 | E01 |
| 2-1-m1E01-2-0406 | 726.269 | OEt | a2 | B17 | b1 | C30 | c3 | D02 | d1 | E01 |
| 2-1-m1E01-2-0407 | 726.284 | OEt | a1 | B05 | b1 | C11 | c2 | D02 | d1 | E01 |
| 2-1-m1E01-2-0408 | 726.284 | OEt | a1 | B02 | b2 | C22 | c3 | D02 | d1 | E01 |

(continued)

| ID | Exact Mass | ⊠ | a | B | b | C | c | D | d | E |
|---|---|---|---|---|---|---|---|---|---|---|
| 2-1-m1E01-2-0409 | 726.284 | OEt | a1 | B03 | b2 | C28 | c3 | D02 | d1 | E01 |
| 2-1-m1E01-2-0410 | 727.242 | OEt | a1 | B02 | b2 | C03 | c2 | D02 | d1 | E01 |
| 2-1-m1E01-2-0411 | 727.262 | OEt | a2 | B20 | b2 | C03 | c1 | D01 | d1 | E01 |
| 2-1-m1E01-2-0412 | 727.268 | OEt | a1 | B06 | b3 | C08 | c1 | D01 | d1 | E01 |
| 2-1-m1E01-2-0413 | 727.268 | OEt | a1 | B06 | b1 | C08 | c2 | D02 | d1 | E01 |
| 2-1-m1E01-2-0414 | 727.279 | OEt | a1 | B05 | b1 | C10 | c2 | D02 | d1 | E01 |
| 2-1-m1E01-2-0415 | 727.284 | OEt | a1 | B04 | b1 | C30 | c3 | D02 | d1 | E01 |
| 2-1-m1E01-2-0416 | 728.248 | OEt | a2 | B17 | b2 | C20 | c3 | D02 | d1 | E01 |
| 2-1-m1E01-2-0417 | 728.248 | OEt | a2 | B14 | b2 | C24 | c3 | D02 | d1 | E01 |
| 2-1-m1E01-2-0418 | 728.262 | OEt | a2 | B19 | b2 | C04 | c1 | D01 | d1 | E01 |
| 2-1-m1E01-2-0419 | 728.263 | OEt | a1 | B06 | b1 | C11 | c2 | D02 | d1 | E01 |
| 2-1-m1E01-2-0420 | 728.267 | OEt | a2 | B18 | b3 | C08 | c1 | D01 | d1 | E01 |
| 2-1-m1E01-2-0421 | 728.267 | OEt | a2 | B18 | b1 | C08 | c2 | D02 | d1 | E01 |
| 2-1-m1E01-2-0422 | 729.243 | OEt | a2 | B17 | b2 | C28 | c3 | D02 | d1 | E01 |
| 2-1-m1E01-2-0423 | 729.247 | OEt | a1 | B03 | b2 | C01 | c2 | D02 | d1 | E01 |
| 2-1-m1E01-2-0424 | 729.258 | OEt | a1 | B06 | b1 | C10 | c2 | D02 | d1 | E01 |
| 2-1-m1E01-2-0425 | 729.262 | OEt | a2 | B18 | b1 | C11 | c2 | D02 | d1 | E01 |
| 2-1-m1E01-2-0426 | 729.263 | OEt | a1 | B04 | b2 | C20 | c3 | D02 | d1 | E01 |
| 2-1-m1E01-2-0427 | 729.263 | OEt | a1 | B01 | b2 | C24 | c3 | D02 | d1 | E01 |
| 2-1-m1E01-2-0428 | 729.277 | OEt | a1 | B09 | b2 | C02 | c1 | D01 | d1 | E01 |
| 2-1-m1E01-2-0429 | 729.314 | OEt | a1 | B05 | b1 | C09 | c1 | D01 | d1 | E01 |
| 2-1-m1E01-2-0430 | 729.334 | OEt | a1 | B07 | b2 | C07 | c1 | D01 | d1 | E01 |
| 2-1-m1E01-2-0431 | 730.193 | OEt | a2 | B14 | b2 | C06 | c2 | D02 | d1 | E01 |
| 2-1-m1E01-2-0432 | 730.257 | OEt | a2 | B18 | b1 | C10 | c2 | D02 | d1 | E01 |
| 2-1-m1E01-2-0433 | 730.257 | OEt | a2 | B11 | b1 | C13 | c2 | D02 | d1 | E01 |
| 2-1-m1E01-2-0434 | 730.258 | OEt | a1 | B02 | b2 | C24 | c3 | D02 | d1 | E01 |
| 2-1-m1E01-2-0435 | 730.258 | OEt | a1 | B04 | b2 | C28 | c3 | D02 | d1 | E01 |
| 2-1-m1E01-2-0436 | 730.273 | OEt | a1 | B09 | b2 | C03 | c1 | D01 | d1 | E01 |
| 2-1-m1E01-2-0437 | 731.208 | OEt | a1 | B01 | b2 | C06 | c2 | D02 | d1 | E01 |
| 2-1-m1E01-2-0438 | 731.245 | OEt | a1 | B05 | b3 | C06 | c1 | D01 | d1 | E01 |
| 2-1-m1E01-2-0439 | 731.253 | OEt | a2 | B12 | b1 | C13 | c2 | D02 | d1 | E01 |
| 2-1-m1E01-2-0440 | 731.293 | OEt | a1 | B06 | b1 | C09 | c1 | D01 | d1 | E01 |
| 2-1-m1E01-2-0441 | 732.192 | OEt | a2 | B11 | b3 | C15 | c2 | D02 | d1 | E01 |
| 2-1-m1E01-2-0442 | 732.204 | OEt | a1 | B02 | b2 | C06 | c2 | D02 | d1 | E01 |
| 2-1-m1E01-2-0443 | 732.207 | OEt | a2 | B17 | b2 | C01 | c2 | D02 | d1 | E01 |
| 2-1-m1E01-2-0444 | 732.223 | OEt | a2 | B20 | b2 | C06 | c1 | D01 | d1 | E01 |
| 2-1-m1E01-2-0445 | 732.240 | OEt | a1 | B05 | b1 | C12 | c2 | D02 | d1 | E01 |

(continued)

| ID | Exact Mass | ⊠ | a | B | b | C | c | D | d | E |
|---|---|---|---|---|---|---|---|---|---|---|
| 2-1-m1E01-2-0446 | 732.262 | OEt | a2 | B11 | b3 | C16 | c1 | D01 | d1 | E01 |
| 2-1-m1E01-2-0447 | 732.292 | OEt | a2 | B18 | b1 | C09 | c1 | D01 | d1 | E01 |
| 2-1-m1E01-2-0448 | 732.335 | OEt | a2 | B13 | b1 | C29 | c3 | D02 | d1 | E01 |
| 2-1-m1E01-2-0449 | 732.335 | OEt | a2 | B15 | b1 | C32 | c3 | D02 | d1 | E01 |
| 2-1-m1E01-2-0450 | 733.188 | OEt | a2 | B12 | b3 | C15 | c2 | D02 | d1 | E01 |
| 2-1-m1E01-2-0451 | 733.222 | OEt | a1 | B04 | b2 | C01 | c2 | D02 | d1 | E01 |
| 2-1-m1E01-2-0452 | 733.224 | OEt | a1 | B06 | b3 | C06 | c1 | D01 | d1 | E01 |
| 2-1-m1E01-2-0453 | 733.238 | OEt | a1 | B07 | b3 | C08 | c1 | D01 | d1 | E01 |
| 2-1-m1E01-2-0454 | 733.238 | OEt | a1 | B07 | b1 | C08 | c2 | D02 | d1 | E01 |
| 2-1-m1E01-2-0455 | 733.257 | OEt | a2 | B12 | b3 | C16 | c1 | D01 | d1 | E01 |
| 2-1-m1E01-2-0456 | 733.268 | OEt | a1 | B08 | b2 | C05 | c1 | D01 | d1 | E01 |
| 2-1-m1E01-2-0457 | 734.219 | OEt | a1 | B06 | b1 | C12 | c2 | D02 | d1 | E01 |
| 2-1-m1E01-2-0458 | 734.223 | OEt | a2 | B18 | b3 | C06 | c1 | D01 | d1 | E01 |
| 2-1-m1E01-2-0459 | 734.223 | OEt | a2 | B11 | b3 | C17 | c1 | D01 | d1 | E01 |
| 2-1-m1E01-2-0460 | 734.223 | OEt | a2 | B11 | b1 | C14 | c2 | D02 | d1 | E01 |
| 2-1-m1E01-2-0461 | 734.233 | OEt | a1 | B07 | b1 | C11 | c2 | D02 | d1 | E01 |
| 2-1-m1E01-2-0462 | 734.277 | OEt | a2 | B15 | b2 | C02 | c2 | D02 | d1 | E01 |
| 2-1-m1E01-2-0463 | 734.283 | OEt | a2 | B19 | b1 | C13 | c1 | D01 | d1 | E01 |
| 2-1-m1E01-2-0464 | 734.314 | OEt | a2 | B15 | b2 | C22 | c3 | D02 | d1 | E01 |
| 2-1-m1E01-2-0465 | 734.314 | OEt | a2 | B16 | b1 | C32 | c3 | D02 | d1 | E01 |
| 2-1-m1E01-2-0466 | 735.219 | OEt | a2 | B12 | b3 | C17 | c1 | D01 | d1 | E01 |
| 2-1-m1E01-2-0467 | 735.219 | OEt | a2 | B18 | b1 | C12 | c2 | D02 | d1 | E01 |
| 2-1-m1E01-2-0468 | 735.219 | OEt | a2 | B12 | b1 | C14 | c2 | D02 | d1 | E01 |
| 2-1-m1E01-2-0469 | 735.229 | OEt | a1 | B07 | b1 | C10 | c2 | D02 | d1 | E01 |
| 2-1-m1E01-2-0470 | 735.234 | OEt | a1 | B09 | b2 | C06 | c1 | D01 | d1 | E01 |
| 2-1-m1E01-2-0471 | 735.252 | OEt | a1 | B10 | b2 | C01 | c1 | D01 | d1 | E01 |
| 2-1-m1E01-2-0472 | 735.273 | OEt | a2 | B15 | b2 | C03 | c2 | D02 | d1 | E01 |
| 2-1-m1E01-2-0473 | 735.273 | OEt | a2 | B13 | b2 | C05 | c2 | D02 | d1 | E01 |
| 2-1-m1E01-2-0474 | 736.218 | OEt | a2 | B19 | b3 | C15 | c1 | D01 | d1 | E01 |
| 2-1-m1E01-2-0475 | 736.248 | OEt | a1 | B08 | b2 | C04 | c1 | D01 | d1 | E01 |
| 2-1-m1E01-2-0476 | 736.257 | OEt | a2 | B16 | b2 | C02 | c2 | D02 | d1 | E01 |
| 2-1-m1E01-2-0477 | 736.293 | OEt | a2 | B16 | b2 | C22 | c3 | D02 | d1 | E01 |
| 2-1-m1E01-2-0478 | 736.293 | OEt | a2 | B13 | b2 | C26 | c3 | D02 | d1 | E01 |
| 2-1-m1E01-2-0479 | 736.309 | OEt | a2 | B14 | b1 | C29 | c3 | D02 | d1 | E01 |
| 2-1-m1E01-2-0480 | 737.252 | OEt | a2 | B16 | b2 | C03 | c2 | D02 | d1 | E01 |
| 2-1-m1E01-2-0481 | 737.264 | OEt | a1 | B07 | b1 | C09 | c1 | D01 | d1 | E01 |
| 2-1-m1E01-2-0482 | 737.325 | OEt | a1 | B05 | b1 | C30 | c3 | D02 | d1 | E01 |

(continued)

| ID | Exact Mass | ☒ | a | B | b | C | c | D | d | E |
|---|---|---|---|---|---|---|---|---|---|---|
| 2-1-m1E01-2-0483 | 737.325 | OEt | a1 | B01 | b1 | C29 | c3 | D02 | d1 | E01 |
| 2-1-m1E01-2-0484 | 737.325 | OEt | a1 | B03 | b1 | C32 | c3 | D02 | d1 | E01 |
| 2-1-m1E01-2-0485 | 738.249 | OEt | a2 | B19 | b1 | C14 | c1 | D01 | d1 | E01 |
| 2-1-m1E01-2-0486 | 738.252 | OEt | a2 | B13 | b2 | C04 | c2 | D02 | d1 | E01 |
| 2-1-m1E01-2-0487 | 738.270 | OEt | a2 | B19 | b2 | C33 | c3 | D02 | d1 | E01 |
| 2-1-m1E01-2-0488 | 738.289 | OEt | a2 | B15 | b2 | C24 | c3 | D02 | d1 | E01 |
| 2-1-m1E01-2-0489 | 738.309 | OEt | a2 | B11 | b2 | C07 | c2 | D02 | d1 | E01 |
| 2-1-m1E01-2-0490 | 738.320 | OEt | a1 | B02 | b1 | C29 | c3 | D02 | d1 | E01 |
| 2-1-m1E01-2-0491 | 739.195 | OEt | a1 | B07 | b3 | C06 | c1 | D01 | d1 | E01 |
| 2-1-m1E01-2-0492 | 739.248 | OEt | a2 | B14 | b2 | C05 | c2 | D02 | d1 | E01 |
| 2-1-m1E01-2-0493 | 739.268 | OEt | a1 | B03 | b2 | C02 | c2 | D02 | d1 | E01 |
| 2-1-m1E01-2-0494 | 739.304 | OEt | a2 | B12 | b2 | C07 | c2 | D02 | d1 | E01 |
| 2-1-m1E01-2-0495 | 739.304 | OEt | a1 | B05 | b2 | C20 | c3 | D02 | d1 | E01 |
| 2-1-m1E01-2-0496 | 739.304 | OEt | a1 | B03 | b2 | C22 | c3 | D02 | d1 | E01 |
| 2-1-m1E01-2-0497 | 739.304 | OEt | a1 | B06 | b1 | C30 | c3 | D02 | d1 | E01 |
| 2-1-m1E01-2-0498 | 740.190 | OEt | a1 | B07 | b1 | C12 | c2 | D02 | d1 | E01 |
| 2-1-m1E01-2-0499 | 740.234 | OEt | a2 | B15 | b2 | C06 | c2 | D02 | d1 | E01 |
| 2-1-m1E01-2-0500 | 740.263 | OEt | a1 | B03 | b2 | C03 | c2 | D02 | d1 | E01 |
| 2-1-m1E01-2-0501 | 740.263 | OEt | a1 | B01 | b2 | C05 | c2 | D02 | d1 | E01 |
| 2-1-m1E01-2-0502 | 740.268 | OEt | a2 | B16 | b2 | C24 | c3 | D02 | d1 | E01 |
| 2-1-m1E01-2-0503 | 740.268 | OEt | a2 | B14 | b2 | C26 | c3 | D02 | d1 | E01 |
| 2-1-m1E01-2-0504 | 740.284 | OEt | a2 | B17 | b1 | C32 | c3 | D02 | d1 | E01 |
| 2-1-m1E01-2-0505 | 740.299 | OEt | a1 | B05 | b2 | C28 | c3 | D02 | d1 | E01 |
| 2-1-m1E01-2-0506 | 740.303 | OEt | a2 | B18 | b1 | C30 | c3 | D02 | d1 | E01 |
| 2-1-m1E01-2-0507 | 741.258 | OEt | a1 | B02 | b2 | C05 | c2 | D02 | d1 | E01 |
| 2-1-m1E01-2-0508 | 741.277 | OEt | a2 | B20 | b2 | C05 | c1 | D01 | d1 | E01 |
| 2-1-m1E01-2-0509 | 741.283 | OEt | a1 | B06 | b2 | C20 | c3 | D02 | d1 | E01 |
| 2-1-m1E01-2-0510 | 741.283 | OEt | a1 | B01 | b2 | C26 | c3 | D02 | d1 | E01 |
| 2-1-m1E01-2-0511 | 741.300 | OEt | a1 | B04 | b1 | C32 | c3 | D02 | d1 | E01 |
| 2-1-m1E01-2-0512 | 742.213 | OEt | a2 | B16 | b2 | C06 | c2 | D02 | d1 | E01 |
| 2-1-m1E01-2-0513 | 742.213 | OEt | a2 | B11 | b3 | C08 | c2 | D02 | d1 | E01 |
| 2-1-m1E01-2-0514 | 742.227 | OEt | a2 | B17 | b2 | C02 | c2 | D02 | d1 | E01 |
| 2-1-m1E01-2-0515 | 742.227 | OEt | a2 | B14 | b2 | C04 | c2 | D02 | d1 | E01 |
| 2-1-m1E01-2-0516 | 742.264 | OEt | a2 | B17 | b2 | C22 | c3 | D02 | d1 | E01 |
| 2-1-m1E01-2-0517 | 742.269 | OEt | a1 | B08 | b1 | C13 | c1 | D01 | d1 | E01 |
| 2-1-m1E01-2-0518 | 742.278 | OEt | a1 | B02 | b2 | C26 | c3 | D02 | d1 | E01 |
| 2-1-m1E01-2-0519 | 742.278 | OEt | a1 | B06 | b2 | C28 | c3 | D02 | d1 | E01 |

(continued)

| ID | Exact Mass | ⊠ | a | B | b | C | c | D | d | E |
|---|---|---|---|---|---|---|---|---|---|---|
| 2-1-m1E01-2-0520 | 742.283 | OEt | a2 | B18 | b2 | C20 | c3 | D02 | d1 | E01 |
| 2-1-m1E01-2-0521 | 742.334 | OEt | a2 | B19 | b2 | C07 | c1 | D01 | d1 | E01 |
| 2-1-m1E01-2-0522 | 743.208 | OEt | a2 | B12 | b3 | C08 | c2 | D02 | d1 | E01 |
| 2-1-m1E01-2-0523 | 743.223 | OEt | a2 | B17 | b2 | C03 | c2 | D02 | d1 | E01 |
| 2-1-m1E01-2-0524 | 743.243 | OEt | a1 | B04 | b2 | C02 | c2 | D02 | d1 | E01 |
| 2-1-m1E01-2-0525 | 743.243 | OEt | a1 | B01 | b2 | C04 | c2 | D02 | d1 | E01 |
| 2-1-m1E01-2-0526 | 743.262 | OEt | a1 | B05 | b2 | C01 | c2 | D02 | d1 | E01 |
| 2-1-m1E01-2-0527 | 743.278 | OEt | a2 | B18 | b2 | C28 | c3 | D02 | d1 | E01 |
| 2-1-m1E01-2-0528 | 743.279 | OEt | a1 | B04 | b2 | C22 | c3 | D02 | d1 | E01 |
| 2-1-m1E01-2-0529 | 743.279 | OEt | a1 | B03 | b2 | C24 | c3 | D02 | d1 | E01 |
| 2-1-m1E01-2-0530 | 744.204 | OEt | a1 | B08 | b3 | C15 | c1 | D01 | d1 | E01 |
| 2-1-m1E01-2-0531 | 744.238 | OEt | a1 | B02 | b2 | C04 | c2 | D02 | d1 | E01 |
| 2-1-m1E01-2-0532 | 744.238 | OEt | a1 | B04 | b2 | C03 | c2 | D02 | d1 | E01 |
| 2-1-m1E01-2-0533 | 744.257 | OEt | a2 | B20 | b2 | C04 | c1 | D01 | d1 | E01 |
| 2-1-m1E01-2-0534 | 744.273 | OEt | a2 | B13 | b1 | C13 | c2 | D02 | d1 | E01 |
| 2-1-m1E01-2-0535 | 744.288 | OEt | a1 | B09 | b2 | C05 | c1 | D01 | d1 | E01 |
| 2-1-m1E01-2-0536 | 745.224 | OEt | a1 | B03 | b2 | C06 | c2 | D02 | d1 | E01 |
| 2-1-m1E01-2-0537 | 745.242 | OEt | a1 | B06 | b2 | C01 | c2 | D02 | d1 | E01 |
| 2-1-m1E01-2-0538 | 745.272 | OEt | a1 | B10 | b2 | C02 | c1 | D01 | d1 | E01 |
| 2-1-m1E01-2-0539 | 745.275 | OEt | a1 | B07 | b1 | C30 | c3 | D02 | d1 | E01 |
| 2-1-m1E01-2-0540 | 746.208 | OEt | a2 | B13 | b3 | C15 | c2 | D02 | d1 | E01 |
| 2-1-m1E01-2-0541 | 746.235 | OEt | a1 | B08 | b1 | C14 | c1 | D01 | d1 | E01 |
| 2-1-m1E01-2-0542 | 746.239 | OEt | a2 | B19 | b3 | C08 | c1 | D01 | d1 | E01 |
| 2-1-m1E01-2-0543 | 746.239 | OEt | a2 | B19 | b1 | C08 | c2 | D02 | d1 | E01 |
| 2-1-m1E01-2-0544 | 746.239 | OEt | a2 | B11 | b1 | C09 | c2 | D02 | d1 | E01 |
| 2-1-m1E01-2-0545 | 746.239 | OEt | a2 | B17 | b2 | C24 | c3 | D02 | d1 | E01 |
| 2-1-m1E01-2-0546 | 746.241 | OEt | a2 | B18 | b2 | C01 | c2 | D02 | d1 | E01 |
| 2-1-m1E01-2-0547 | 746.256 | OEt | a1 | B08 | b2 | C33 | c3 | D02 | d1 | E01 |
| 2-1-m1E01-2-0548 | 746.268 | OEt | a1 | B10 | b2 | C03 | c1 | D01 | d1 | E01 |
| 2-1-m1E01-2-0549 | 746.277 | OEt | a2 | B13 | b3 | C16 | c1 | D01 | d1 | E01 |
| 2-1-m1E01-2-0550 | 746.314 | OEt | a2 | B11 | b1 | C31 | c3 | D02 | d1 | E01 |
| 2-1-m1E01-2-0551 | 746.350 | OEt | a2 | B15 | b1 | C29 | c3 | D02 | d1 | E01 |
| 2-1-m1E01-2-0552 | 747.234 | OEt | a2 | B12 | b1 | C09 | c2 | D02 | d1 | E01 |
| 2-1-m1E01-2-0553 | 747.234 | OEt | a2 | B19 | b1 | C11 | c2 | D02 | d1 | E01 |
| 2-1-m1E01-2-0554 | 747.254 | OEt | a1 | B07 | b2 | C20 | c3 | D02 | d1 | E01 |
| 2-1-m1E01-2-0555 | 747.254 | OEt | a1 | B04 | b2 | C24 | c3 | D02 | d1 | E01 |
| 2-1-m1E01-2-0556 | 747.268 | OEt | a1 | B09 | b2 | C04 | c1 | D01 | d1 | E01 |

(continued)

| ID | Exact Mass | ⊠ | a | B | b | C | c | D | d | E |
|---|---|---|---|---|---|---|---|---|---|---|
| 2-1-m1E01-2-0557 | 747.309 | OEt | a2 | B12 | b1 | C31 | c3 | D02 | d1 | E01 |
| 2-1-m1E01-2-0558 | 748.170 | OEt | a2 | B11 | b3 | C06 | c2 | D02 | d1 | E01 |
| 2-1-m1E01-2-0559 | 748.184 | OEt | a2 | B17 | b2 | C06 | c2 | D02 | d1 | E01 |
| 2-1-m1E01-2-0560 | 748.229 | OEt | a2 | B19 | b1 | C10 | c2 | D02 | d1 | E01 |
| 2-1-m1E01-2-0561 | 748.239 | OEt | a2 | B13 | b3 | C17 | cl | D01 | d1 | E01 |
| 2-1-m1E01-2-0562 | 748.239 | OEt | a2 | B13 | b1 | C14 | c2 | D02 | d1 | E01 |
| 2-1-m1E01-2-0563 | 748.248 | OEt | a2 | B14 | b1 | C13 | c2 | D02 | d1 | E01 |
| 2-1-m1E01-2-0564 | 748.249 | OEt | a1 | B07 | b2 | C28 | c3 | D02 | d1 | E01 |
| 2-1-m1E01-2-0565 | 748.329 | OEt | a2 | B16 | b1 | C29 | c3 | D02 | d1 | E01 |
| 2-1-m1E01-2-0566 | 749.165 | OEt | a2 | B12 | b3 | C06 | c2 | D02 | d1 | E01 |
| 2-1-m1E01-2-0567 | 749.199 | OEt | a1 | B04 | b2 | C06 | c2 | D02 | d1 | E01 |
| 2-1-m1E01-2-0568 | 749.234 | OEt | a2 | B11 | b3 | C18 | c1 | D01 | d1 | E01 |
| 2-1-m1E01-2-0569 | 749.263 | OEt | a1 | B01 | b1 | C13 | c2 | D02 | d1 | E01 |
| 2-1-m1E01-2-0570 | 749.288 | OEt | a2 | B15 | b2 | C05 | c2 | D02 | d1 | E01 |
| 2-1-m1E01-2-0571 | 750.183 | OEt | a2 | B14 | b3 | C15 | c2 | D02 | d1 | E01 |
| 2-1-m1E01-2-0572 | 750.229 | OEt | a2 | B12 | b3 | C18 | c1 | D01 | d1 | E01 |
| 2-1-m1E01-2-0573 | 750.252 | OEt | a2 | B14 | b3 | C16 | c1 | D01 | d1 | E01 |
| 2-1-m1E01-2-0574 | 750.258 | OEt | a1 | B02 | b1 | C13 | c2 | D02 | d1 | E01 |
| 2-1-m1E01-2-0575 | 750.264 | OEt | a2 | B19 | b1 | C09 | c1 | D01 | d1 | E01 |
| 2-1-m1E01-2-0576 | 750.278 | OEt | a2 | B20 | b1 | C13 | c1 | D01 | d1 | E01 |
| 2-1-m1E01-2-0577 | 750.309 | OEt | a2 | B15 | b2 | C26 | c3 | D02 | d1 | E01 |
| 2-1-m1E01-2-0578 | 750.320 | OEt | a1 | B08 | b2 | C07 | c1 | D01 | d1 | E01 |
| 2-1-m1E01-2-0579 | 751.198 | OEt | a1 | B01 | b3 | C15 | c2 | D02 | d1 | E01 |
| 2-1-m1E01-2-0580 | 751.212 | OEt | a1 | B07 | b2 | C01 | c2 | D02 | d1 | E01 |
| 2-1-m1E01-2-0581 | 751.229 | OEt | a1 | B10 | b2 | C06 | c1 | D01 | d1 | E01 |
| 2-1-m1E01-2-0582 | 751.268 | OEt | a1 | B01 | b3 | C16 | c1 | D01 | d1 | E01 |
| 2-1-m1E01-2-0583 | 751.268 | OEt | a2 | B16 | b2 | C05 | c2 | D02 | d1 | E01 |
| 2-1-m1E01-2-0584 | 751.340 | OEt | a1 | B03 | b1 | C29 | c3 | D02 | d1 | E01 |
| 2-1-m1E01-2-0585 | 751.340 | OEt | a1 | B05 | b1 | C32 | c3 | D02 | d1 | E01 |
| 2-1-m1E01-2-0586 | 752.193 | OEt | a1 | B02 | b3 | C15 | c2 | D02 | d1 | E01 |
| 2-1-m1E01-2-0587 | 752.195 | OEt | a2 | B19 | b3 | C06 | c1 | D01 | d1 | E01 |
| 2-1-m1E01-2-0588 | 752.213 | OEt | a2 | B20 | b3 | C15 | c1 | D01 | d1 | E01 |
| 2-1-m1E01-2-0589 | 752.214 | OEt | a2 | B14 | b3 | C17 | c1 | D01 | d1 | E01 |
| 2-1-m1E01-2-0590 | 752.214 | OEt | a2 | B14 | b1 | C14 | c2 | D02 | d1 | E01 |
| 2-1-m1E01-2-0591 | 752.263 | OEt | a1 | B02 | b3 | C16 | c1 | D01 | d1 | E01 |
| 2-1-m1E01-2-0592 | 752.268 | OEt | a2 | B15 | b2 | C04 | c2 | D02 | d1 | E01 |
| 2-1-m1E01-2-0593 | 752.288 | OEt | a2 | B16 | b2 | C26 | c3 | D02 | d1 | E01 |

(continued)

| ID | Exact Mass | ⊠ | a | B | b | C | c | D | d | E |
|---|---|---|---|---|---|---|---|---|---|---|
| 2-1-m1E01-2-0594 | 752.324 | OEt | a2 | B13 | b2 | C07 | c2 | D02 | d1 | E01 |
| 2-1-m1E01-2-0595 | 753.190 | OEt | a2 | B19 | b1 | C12 | c2 | D02 | d1 | E01 |
| 2-1-m1E01-2-0596 | 753.229 | OEt | a1 | B01 | b3 | C17 | c1 | D01 | d1 | E01 |
| 2-1-m1E01-2-0597 | 753.229 | OEt | a1 | B01 | b1 | C14 | c2 | D02 | d1 | E01 |
| 2-1-m1E01-2-0598 | 753.283 | OEt | a1 | B05 | b2 | C02 | c2 | D02 | d1 | E01 |
| 2-1-m1E01-2-0599 | 753.289 | OEt | a1 | B09 | b1 | C13 | c1 | D01 | d1 | E01 |
| 2-1-m1E01-2-0600 | 753.320 | OEt | a1 | B05 | b2 | C22 | c3 | D02 | d1 | E01 |
| 2-1-m1E01-2-0601 | 753.320 | OEt | a1 | B06 | b1 | C32 | c3 | D02 | d1 | E01 |
| 2-1-m1E01-2-0602 | 754.224 | OEt | a1 | B02 | b3 | C17 | c1 | D01 | d1 | E01 |
| 2-1-m1E01-2-0603 | 754.224 | OEt | a1 | B08 | b3 | C08 | c1 | D01 | d1 | E01 |
| 2-1-m1E01-2-0604 | 754.224 | OEt | a1 | B08 | b1 | C08 | c2 | D02 | d1 | E01 |
| 2-1-m1E01-2-0605 | 754.224 | OEt | a1 | B02 | b1 | C14 | c2 | D02 | d1 | E01 |
| 2-1-m1E01-2-0606 | 754.244 | OEt | a2 | B20 | b1 | C14 | c1 | D01 | d1 | E01 |
| 2-1-m1E01-2-0607 | 754.247 | OEt | a2 | B16 | b2 | C04 | c2 | D02 | d1 | E01 |
| 2-1-m1E01-2-0608 | 754.265 | OEt | a2 | B20 | b2 | C33 | c3 | D02 | d1 | E01 |
| 2-1-m1E01-2-0609 | 754.278 | OEt | a1 | B05 | b2 | C03 | c2 | D02 | d1 | E01 |
| 2-1-m1E01-2-0610 | 754.278 | OEt | a1 | B03 | b2 | C05 | c2 | D02 | d1 | E01 |
| 2-1-m1E01-2-0611 | 754.300 | OEt | a2 | B17 | b1 | C29 | c3 | D02 | d1 | E01 |
| 2-1-m1E01-2-0612 | 754.319 | OEt | a2 | B18 | b1 | C32 | c3 | D02 | d1 | E01 |
| 2-1-m1E01-2-0613 | 755.220 | OEt | a1 | B08 | b1 | C11 | c2 | D02 | d1 | E01 |
| 2-1-m1E01-2-0614 | 755.224 | OEt | a1 | B09 | b3 | C15 | c1 | D01 | d1 | E01 |
| 2-1-m1E01-2-0615 | 755.262 | OEt | a1 | B06 | b2 | C02 | c2 | D02 | d1 | E01 |
| 2-1-m1E01-2-0616 | 755.299 | OEt | a1 | B06 | b2 | C22 | c3 | D02 | d1 | E01 |
| 2-1-m1E01-2-0617 | 755.299 | OEt | a1 | B03 | b2 | C26 | c3 | D02 | d1 | E01 |
| 2-1-m1E01-2-0618 | 755.315 | OEt | a1 | B04 | b1 | C29 | c3 | D02 | d1 | E01 |
| 2-1-m1E01-2-0619 | 756.215 | OEt | a1 | B08 | b1 | C10 | c2 | D02 | d1 | E01 |
| 2-1-m1E01-2-0620 | 756.229 | OEt | a2 | B13 | b3 | C08 | c2 | D02 | d1 | E01 |
| 2-1-m1E01-2-0621 | 756.258 | OEt | a1 | B06 | b2 | C03 | c2 | D02 | d1 | E01 |
| 2-1-m1E01-2-0622 | 756.262 | OEt | a2 | B18 | b2 | C02 | c2 | D02 | d1 | E01 |
| 2-1-m1E01-2-0623 | 756.298 | OEt | a2 | B18 | b2 | C22 | c3 | D02 | d1 | E01 |
| 2-1-m1E01-2-0624 | 756.299 | OEt | a2 | B14 | b2 | C07 | c2 | D02 | d1 | E01 |
| 2-1-m1E01-2-0625 | 757.238 | OEt | a2 | B17 | b2 | C05 | c2 | D02 | d1 | E01 |
| 2-1-m1E01-2-0626 | 757.255 | OEt | a1 | B09 | b1 | C14 | c1 | D01 | d1 | E01 |
| 2-1-m1E01-2-0627 | 757.257 | OEt | a2 | B18 | b2 | C03 | c2 | D02 | d1 | E01 |
| 2-1-m1E01-2-0628 | 757.258 | OEt | a1 | B03 | b2 | C04 | c2 | D02 | d1 | E01 |
| 2-1-m1E01-2-0629 | 757.276 | OEt | a1 | B09 | b2 | C33 | c3 | D02 | d1 | E01 |
| 2-1-m1E01-2-0630 | 757.295 | OEt | a1 | B05 | b2 | C24 | c3 | D02 | d1 | E01 |

(continued)

| ID | Exact Mass | ⊠ | a | B | b | C | c | D | d | E |
|---|---|---|---|---|---|---|---|---|---|---|
| 2-1-m1E01-2-0631 | 757.315 | OEt | a1 | B01 | b2 | C07 | c2 | D02 | d1 | E01 |
| 2-1-m1E01-2-0632 | 758.250 | OEt | a1 | B08 | b1 | C09 | c1 | D01 | d1 | E01 |
| 2-1-m1E01-2-0633 | 758.253 | OEt | a1 | B04 | b2 | C05 | c2 | D02 | d1 | E01 |
| 2-1-m1E01-2-0634 | 758.259 | OEt | a2 | B17 | b2 | C26 | c3 | D02 | d1 | E01 |
| 2-1-m1E01-2-0635 | 758.275 | OEt | a2 | B19 | b1 | C30 | c3 | D02 | d1 | E01 |
| 2-1-m1E01-2-0636 | 758.289 | OEt | a2 | B15 | b1 | C13 | c2 | D02 | d1 | E01 |
| 2-1-m1E01-2-0637 | 758.310 | OEt | a1 | B02 | b2 | C07 | c2 | D02 | d1 | E01 |
| 2-1-m1E01-2-0638 | 758.329 | OEt | a2 | B20 | b2 | C07 | c1 | D01 | d1 | E01 |
| 2-1-m1E01-2-0639 | 759.240 | OEt | a1 | B05 | b2 | C06 | c2 | D02 | d1 | E01 |
| 2-1-m1E01-2-0640 | 759.274 | OEt | a1 | B06 | b2 | C24 | c3 | D02 | d1 | E01 |
| 2-1-m1E01-2-0641 | 759.274 | OEt | a1 | B04 | b2 | C26 | c3 | D02 | d1 | E01 |
| 2-1-m1E01-2-0642 | 759.290 | OEt | a1 | B07 | b1 | C32 | c3 | D02 | d1 | E01 |
| 2-1-m1E01-2-0643 | 760.181 | OEt | a1 | B08 | b3 | C06 | c1 | D01 | d1 | E01 |
| 2-1-m1E01-2-0644 | 760.204 | OEt | a2 | B14 | b3 | C08 | c2 | D02 | d1 | E01 |
| 2-1-m1E01-2-0645 | 760.218 | OEt | a2 | B17 | b2 | C04 | c2 | D02 | d1 | E01 |
| 2-1-m1E01-2-0646 | 760.224 | OEt | a2 | B15 | b3 | C15 | c2 | D02 | d1 | E01 |
| 2-1-m1E01-2-0647 | 760.254 | OEt | a2 | B13 | b1 | C09 | c2 | D02 | d1 | E01 |
| 2-1-m1E01-2-0648 | 760.254 | OEt | a2 | B19 | b2 | C20 | c3 | D02 | d1 | E01 |
| 2-1-m1E01-2-0649 | 760.268 | OEt | a2 | B16 | b1 | C13 | c2 | D02 | d1 | E01 |
| 2-1-m1E01-2-0650 | 760.273 | OEt | a2 | B18 | b2 | C24 | c3 | D02 | d1 | E01 |
| 2-1-m1E01-2-0651 | 760.283 | OEt | a1 | B10 | b2 | C05 | c1 | D01 | d1 | E01 |
| 2-1-m1E01-2-0652 | 760.293 | OEt | a2 | B15 | b3 | C16 | c1 | D01 | d1 | E01 |
| 2-1-m1E01-2-0653 | 760.329 | OEt | a2 | B13 | b1 | C31 | c3 | D02 | d1 | E01 |
| 2-1-m1E01-2-0654 | 761.176 | OEt | a1 | B08 | b1 | C12 | c2 | D02 | d1 | E01 |
| 2-1-m1E01-2-0655 | 761.219 | OEt | a1 | B06 | b2 | C06 | c2 | D02 | d1 | E01 |
| 2-1-m1E01-2-0656 | 761.219 | OEt | a1 | B01 | b3 | C08 | c2 | D02 | d1 | E01 |
| 2-1-m1E01-2-0657 | 761.233 | OEt | a1 | B07 | b2 | C02 | c2 | D02 | d1 | E01 |
| 2-1-m1E01-2-0658 | 761.233 | OEt | a1 | B04 | b2 | C04 | c2 | D02 | d1 | E01 |
| 2-1-m1E01-2-0659 | 761.249 | OEt | a2 | B19 | b2 | C28 | c3 | D02 | d1 | E01 |
| 2-1-m1E01-2-0660 | 761.269 | OEt | a1 | B07 | b2 | C22 | c3 | D02 | d1 | E01 |
| 2-1-m1E01-2-0661 | 761.340 | OEt | a1 | B09 | b2 | C07 | c1 | D01 | d1 | E01 |
| 2-1-m1E01-2-0662 | 762.185 | OEt | a2 | B13 | b3 | C06 | c2 | D02 | d1 | E01 |
| 2-1-m1E01-2-0663 | 762.203 | OEt | a2 | B16 | b3 | C15 | c2 | D02 | d1 | E01 |
| 2-1-m1E01-2-0664 | 762.214 | OEt | a1 | B02 | b3 | C08 | c2 | D02 | d1 | E01 |
| 2-1-m1E01-2-0665 | 762.218 | OEt | a2 | B18 | b2 | C06 | c2 | D02 | d1 | E01 |
| 2-1-m1E01-2-0666 | 762.228 | OEt | a1 | B07 | b2 | C03 | c2 | D02 | d1 | E01 |
| 2-1-m1E01-2-0667 | 762.234 | OEt | a2 | B20 | b3 | C08 | c1 | D01 | d1 | E01 |

(continued)

| ID | Exact Mass | ☒ | a | B | b | C | c | D | d | E |
|---|---|---|---|---|---|---|---|---|---|---|
| 2-1-m1E01-2-0668 | 762.234 | OEt | a2 | B20 | b1 | C08 | c2 | D02 | d1 | E01 |
| 2-1-m1E01-2-0669 | 762.255 | OEt | a2 | B15 | b3 | C17 | c1 | D01 | d1 | E01 |
| 2-1-m1E01-2-0670 | 762.255 | OEt | a2 | B15 | b1 | C14 | c2 | D02 | d1 | E01 |
| 2-1-m1E01-2-0671 | 762.272 | OEt | a2 | B16 | b3 | C16 | c1 | D01 | d1 | E01 |
| 2-1-m1E01-2-0672 | 763.229 | OEt | a2 | B20 | b1 | C11 | c2 | D02 | d1 | E01 |
| 2-1-m1E01-2-0673 | 763.250 | OEt | a2 | B13 | b3 | C18 | c1 | D01 | d1 | E01 |
| 2-1-m1E01-2-0674 | 763.263 | OEt | a1 | B10 | b2 | C04 | c1 | D01 | d1 | E01 |
| 2-1-m1E01-2-0675 | 763.279 | OEt | a1 | B03 | b1 | C13 | c2 | D02 | d1 | E01 |
| 2-1-m1E01-2-0676 | 764.213 | OEt | a2 | B19 | b2 | C01 | c2 | D02 | d1 | E01 |
| 2-1-m1E01-2-0677 | 764.224 | OEt | a2 | B20 | b1 | C10 | c2 | D02 | d1 | E01 |
| 2-1-m1E01-2-0678 | 764.229 | OEt | a2 | B14 | b1 | C09 | c2 | D02 | d1 | E01 |
| 2-1-m1E01-2-0679 | 764.234 | OEt | a2 | B16 | b3 | C17 | c1 | D01 | d1 | E01 |
| 2-1-m1E01-2-0680 | 764.234 | OEt | a2 | B16 | b1 | C14 | c2 | D02 | d1 | E01 |
| 2-1-m1E01-2-0681 | 764.304 | OEt | a2 | B14 | b1 | C31 | c3 | D02 | d1 | E01 |
| 2-1-m1E01-2-0682 | 765.214 | OEt | a1 | B03 | b3 | C15 | c2 | D02 | d1 | E01 |
| 2-1-m1E01-2-0683 | 765.244 | OEt | a1 | B09 | b3 | C08 | c1 | D01 | d1 | E01 |
| 2-1-m1E01-2-0684 | 765.244 | OEt | a1 | B09 | b1 | C08 | c2 | D02 | d1 | E01 |
| 2-1-m1E01-2-0685 | 765.244 | OEt | a1 | B01 | b1 | C09 | c2 | D02 | d1 | E01 |
| 2-1-m1E01-2-0686 | 765.244 | OEt | a1 | B07 | b2 | C24 | c3 | D02 | d1 | E01 |
| 2-1-m1E01-2-0687 | 765.283 | OEt | a1 | B03 | b3 | C16 | c1 | D01 | d1 | E01 |
| 2-1-m1E01-2-0688 | 765.320 | OEt | a1 | B01 | b1 | C31 | c3 | D02 | d1 | E01 |
| 2-1-m1E01-2-0689 | 765.356 | OEt | a1 | B05 | b1 | C29 | c3 | D02 | d1 | E01 |
| 2-1-m1E01-2-0690 | 766.160 | OEt | a2 | B14 | b3 | C06 | c2 | D02 | d1 | E01 |
| 2-1-m1E01-2-0691 | 766.239 | OEt | a2 | B17 | b1 | C13 | c2 | D02 | d1 | E01 |
| 2-1-m1E01-2-0692 | 766.240 | OEt | a1 | B02 | b1 | C09 | c2 | D02 | d1 | E01 |
| 2-1-m1E01-2-0693 | 766.240 | OEt | a1 | B09 | b1 | C11 | c2 | D02 | d1 | E01 |
| 2-1-m1E01-2-0694 | 766.259 | OEt | a2 | B20 | b1 | C09 | c1 | D01 | d1 | E01 |
| 2-1-m1E01-2-0695 | 766.261 | OEt | a1 | B08 | b1 | C30 | c3 | D02 | d1 | E01 |
| 2-1-m1E01-2-0696 | 766.315 | OEt | a1 | B02 | b1 | C31 | c3 | D02 | d1 | E01 |
| 2-1-m1E01-2-0697 | 766.340 | OEt | a2 | B15 | b2 | C07 | c2 | D02 | d1 | E01 |
| 2-1-m1E01-2-0698 | 767.175 | OEt | a1 | B01 | b3 | C06 | c2 | D02 | d1 | E01 |
| 2-1-m1E01-2-0699 | 767.190 | OEt | a1 | B07 | b2 | C06 | c2 | D02 | d1 | E01 |
| 2-1-m1E01-2-0700 | 767.225 | OEt | a2 | B14 | b3 | C18 | c1 | D01 | d1 | E01 |
| 2-1-m1E01-2-0701 | 767.235 | OEt | a1 | B09 | b1 | C10 | c2 | D02 | d1 | E01 |
| 2-1-m1E01-2-0702 | 767.245 | OEt | a1 | B03 | b3 | C17 | c1 | D01 | d1 | E01 |
| 2-1-m1E01-2-0703 | 767.245 | OEt | a1 | B03 | b1 | C14 | c2 | D02 | d1 | E01 |
| 2-1-m1E01-2-0704 | 767.254 | OEt | a1 | B04 | b1 | C13 | c2 | D02 | d1 | E01 |

(continued)

| ID | Exact Mass | ⊠ | a | B | b | C | c | D | d | E |
|---|---|---|---|---|---|---|---|---|---|---|
| 2-1-m1E01-2-0705 | 767.335 | OEt | a1 | B06 | b1 | C29 | c3 | D02 | d1 | E01 |
| 2-1-m1E01-2-0706 | 768.171 | OEt | a1 | B02 | b3 | C06 | c2 | D02 | d1 | E01 |
| 2-1-m1E01-2-0707 | 768.174 | OEt | a2 | B17 | b3 | C15 | c2 | D02 | d1 | E01 |
| 2-1-m1E01-2-0708 | 768.190 | OEt | a2 | B20 | b3 | C06 | c1 | D01 | d1 | E01 |
| 2-1-m1E01-2-0709 | 768.240 | OEt | a1 | B01 | b3 | C18 | c1 | D01 | d1 | E01 |
| 2-1-m1E01-2-0710 | 768.240 | OEt | a1 | B08 | b2 | C20 | c3 | D02 | d1 | E01 |
| 2-1-m1E01-2-0711 | 768.243 | OEt | a2 | B17 | b3 | C16 | c1 | D01 | d1 | E01 |
| 2-1-m1E01-2-0712 | 768.294 | OEt | a1 | B05 | b2 | C05 | c2 | D02 | d1 | E01 |
| 2-1-m1E01-2-0713 | 768.319 | OEt | a2 | B16 | b2 | C07 | c2 | D02 | d1 | E01 |
| 2-1-m1E01-2-0714 | 768.335 | OEt | a2 | B18 | b1 | C29 | c3 | D02 | d1 | E01 |
| 2-1-m1E01-2-0715 | 769.185 | OEt | a2 | B20 | b1 | C12 | c2 | D02 | d1 | E01 |
| 2-1-m1E01-2-0716 | 769.189 | OEt | a1 | B04 | b3 | C15 | c2 | D02 | d1 | E01 |
| 2-1-m1E01-2-0717 | 769.235 | OEt | a1 | B02 | b3 | C18 | c1 | D01 | d1 | E01 |
| 2-1-m1E01-2-0718 | 769.235 | OEt | a1 | B08 | b2 | C28 | c3 | D02 | d1 | E01 |
| 2-1-m1E01-2-0719 | 769.258 | OEt | a1 | B04 | b3 | C16 | c1 | D01 | d1 | E01 |
| 2-1-m1E01-2-0720 | 769.270 | OEt | a1 | B09 | b1 | C09 | c1 | D01 | d1 | E01 |
| 2-1-m1E01-2-0721 | 769.284 | OEt | a1 | B10 | b1 | C13 | c1 | D01 | d1 | E01 |
| 2-1-m1E01-2-0722 | 769.315 | OEt | a1 | B05 | b2 | C26 | c3 | D02 | d1 | E01 |
| 2-1-m1E01-2-0723 | 770.204 | OEt | a2 | B17 | b3 | C17 | c1 | D01 | d1 | E01 |
| 2-1-m1E01-2-0724 | 770.204 | OEt | a2 | B17 | b1 | C14 | c2 | D02 | d1 | E01 |
| 2-1-m1E01-2-0725 | 770.244 | OEt | a2 | B15 | b3 | C08 | c2 | D02 | d1 | E01 |
| 2-1-m1E01-2-0726 | 770.273 | OEt | a1 | B06 | b2 | C05 | c2 | D02 | d1 | E01 |
| 2-1-m1E01-2-0727 | 771.201 | OEt | a1 | B09 | b3 | C06 | c1 | D01 | d1 | E01 |
| 2-1-m1E01-2-0728 | 771.219 | OEt | a1 | B10 | b3 | C15 | c1 | D01 | d1 | E01 |
| 2-1-m1E01-2-0729 | 771.220 | OEt | a1 | B04 | b3 | C17 | c1 | D01 | d1 | E01 |
| 2-1-m1E01-2-0730 | 771.220 | OEt | a1 | B04 | b1 | C14 | c2 | D02 | d1 | E01 |
| 2-1-m1E01-2-0731 | 771.273 | OEt | a2 | B18 | b2 | C05 | c2 | D02 | d1 | E01 |
| 2-1-m1E01-2-0732 | 771.274 | OEt | a1 | B05 | b2 | C04 | c2 | D02 | d1 | E01 |
| 2-1-m1E01-2-0733 | 771.294 | OEt | a1 | B06 | b2 | C26 | c3 | D02 | d1 | E01 |
| 2-1-m1E01-2-0734 | 771.330 | OEt | a1 | B03 | b2 | C07 | c2 | D02 | d1 | E01 |
| 2-1-m1E01-2-0735 | 772.196 | OEt | a1 | B09 | b1 | C12 | c2 | D02 | d1 | E01 |
| 2-1-m1E01-2-0736 | 772.199 | OEt | a1 | B08 | b2 | C01 | c2 | D02 | d1 | E01 |
| 2-1-m1E01-2-0737 | 772.224 | OEt | a2 | B16 | b3 | C08 | c2 | D02 | d1 | E01 |
| 2-1-m1E01-2-0738 | 772.291 | OEt | a2 | B19 | b1 | C32 | c3 | D02 | d1 | E01 |
| 2-1-m1E01-2-0739 | 772.293 | OEt | a2 | B18 | b2 | C26 | c3 | D02 | d1 | E01 |
| 2-1-m1E01-2-0740 | 773.249 | OEt | a1 | B10 | b1 | C14 | c1 | D01 | d1 | E01 |
| 2-1-m1E01-2-0741 | 773.253 | OEt | a1 | B06 | b2 | C04 | c2 | D02 | d1 | E01 |

(continued)

| ID | Exact Mass | ☒ | a | B | b | C | c | D | d | E |
|---|---|---|---|---|---|---|---|---|---|---|
| 2-1-m1E01-2-0742 | 773.271 | OEt | a1 | B10 | b2 | C33 | c3 | D02 | d1 | E01 |
| 2-1-m1E01-2-0743 | 773.306 | OEt | a1 | B07 | b1 | C29 | c3 | D02 | d1 | E01 |
| 2-1-m1E01-2-0744 | 774.234 | OEt | a2 | B19 | b2 | C02 | c2 | D02 | d1 | E01 |
| 2-1-m1E01-2-0745 | 774.252 | OEt | a2 | B18 | b2 | C04 | c2 | D02 | d1 | E01 |
| 2-1-m1E01-2-0746 | 774.270 | OEt | a2 | B15 | b1 | C09 | c2 | D02 | d1 | E01 |
| 2-1-m1E01-2-0747 | 774.270 | OEt | a2 | B19 | b2 | C22 | c3 | D02 | d1 | E01 |
| 2-1-m1E01-2-0748 | 774.270 | OEt | a2 | B20 | b1 | C30 | c3 | D02 | d1 | E01 |
| 2-1-m1E01-2-0749 | 774.290 | OEt | a2 | B17 | b2 | C07 | c2 | D02 | d1 | E01 |
| 2-1-m1E01-2-0750 | 774.345 | OEt | a2 | B15 | b1 | C31 | c3 | D02 | d1 | E01 |
| 2-1-m1E01-2-0751 | 775.229 | OEt | a2 | B19 | b2 | C03 | c2 | D02 | d1 | E01 |
| 2-1-m1E01-2-0752 | 775.235 | OEt | a1 | B03 | b3 | C08 | c2 | D02 | d1 | E01 |
| 2-1-m1E01-2-0753 | 775.305 | OEt | a1 | B04 | b2 | C07 | c2 | D02 | d1 | E01 |
| 2-1-m1E01-2-0754 | 776.201 | OEt | a2 | B15 | b3 | C06 | c2 | D02 | d1 | E01 |
| 2-1-m1E01-2-0755 | 776.244 | OEt | a1 | B07 | b2 | C05 | c2 | D02 | d1 | E01 |
| 2-1-m1E01-2-0756 | 776.249 | OEt | a2 | B16 | b1 | C09 | c2 | D02 | d1 | E01 |
| 2-1-m1E01-2-0757 | 776.249 | OEt | a2 | B20 | b2 | C20 | c3 | D02 | d1 | E01 |
| 2-1-m1E01-2-0758 | 776.324 | OEt | a2 | B16 | b1 | C31 | c3 | D02 | d1 | E01 |
| 2-1-m1E01-2-0759 | 777.244 | OEt | a2 | B20 | b2 | C28 | c3 | D02 | d1 | E01 |
| 2-1-m1E01-2-0760 | 777.264 | OEt | a1 | B07 | b2 | C26 | c3 | D02 | d1 | E01 |
| 2-1-m1E01-2-0761 | 777.265 | OEt | a2 | B15 | b3 | C18 | c1 | D01 | d1 | E01 |
| 2-1-m1E01-2-0762 | 777.281 | OEt | a1 | B09 | b1 | C30 | c3 | D02 | d1 | E01 |
| 2-1-m1E01-2-0763 | 777.294 | OEt | a1 | B05 | b1 | C13 | c2 | D02 | d1 | E01 |
| 2-1-m1E01-2-0764 | 777.335 | OEt | a1 | B10 | b2 | C07 | c1 | D01 | d1 | E01 |
| 2-1-m1E01-2-0765 | 778.180 | OEt | a2 | B16 | b3 | C06 | c2 | D02 | d1 | E01 |
| 2-1-m1E01-2-0766 | 778.194 | OEt | a2 | B17 | b3 | C08 | c2 | D02 | d1 | E01 |
| 2-1-m1E01-2-0767 | 778.245 | OEt | a2 | B19 | b2 | C24 | c3 | D02 | d1 | E01 |
| 2-1-m1E01-2-0768 | 779.209 | OEt | a1 | B04 | b3 | C08 | c2 | D02 | d1 | E01 |
| 2-1-m1E01-2-0769 | 779.224 | OEt | a1 | B07 | b2 | C04 | c2 | D02 | d1 | E01 |
| 2-1-m1E01-2-0770 | 779.229 | OEt | a1 | B05 | b3 | C15 | c2 | D02 | d1 | E01 |
| 2-1-m1E01-2-0771 | 779.245 | OEt | a2 | B16 | b3 | C18 | c1 | D01 | d1 | E01 |
| 2-1-m1E01-2-0772 | 779.260 | OEt | a1 | B03 | b1 | C09 | c2 | D02 | d1 | E01 |
| 2-1-m1E01-2-0773 | 779.260 | OEt | a1 | B09 | b2 | C20 | c3 | D02 | d1 | E01 |
| 2-1-m1E01-2-0774 | 779.274 | OEt | a1 | B06 | b1 | C13 | c2 | D02 | d1 | E01 |
| 2-1-m1E01-2-0775 | 779.299 | OEt | a1 | B05 | b3 | C16 | c1 | D01 | d1 | E01 |
| 2-1-m1E01-2-0776 | 779.335 | OEt | a1 | B03 | b1 | C31 | c3 | D02 | d1 | E01 |
| 2-1-m1E01-2-0777 | 780.190 | OEt | a2 | B19 | b2 | C06 | c2 | D02 | d1 | E01 |
| 2-1-m1E01-2-0778 | 780.208 | OEt | a2 | B20 | b2 | C01 | c2 | D02 | d1 | E01 |

(continued)

| ID | Exact Mass | ⊠ | a | B | b | C | c | D | d | E |
|---|---|---|---|---|---|---|---|---|---|---|
| 2-1-m1E01-2-0779 | 780.255 | OEt | a1 | B09 | b2 | C28 | c3 | D02 | d1 | E01 |
| 2-1-m1E01-2-0780 | 780.273 | OEt | a2 | B18 | b1 | C13 | c2 | D02 | d1 | E01 |
| 2-1-m1E01-2-0781 | 780.276 | OEt | a1 | B08 | b1 | C32 | c3 | D02 | d1 | E01 |
| 2-1-m1E01-2-0782 | 781.191 | OEt | a1 | B03 | b3 | C06 | c2 | D02 | d1 | E01 |
| 2-1-m1E01-2-0783 | 781.209 | OEt | a1 | B06 | b3 | C15 | c2 | D02 | d1 | E01 |
| 2-1-m1E01-2-0784 | 781.239 | OEt | a1 | B10 | b3 | C08 | c1 | D01 | d1 | E01 |
| 2-1-m1E01-2-0785 | 781.239 | OEt | a1 | B10 | b1 | C08 | c2 | D02 | d1 | E01 |
| 2-1-m1E01-2-0786 | 781.260 | OEt | a1 | B05 | b3 | C17 | c1 | D01 | d1 | E01 |
| 2-1-m1E01-2-0787 | 781.260 | OEt | a1 | B05 | b1 | C14 | c2 | D02 | d1 | E01 |
| 2-1-m1E01-2-0788 | 781.278 | OEt | a1 | B06 | b3 | C16 | c1 | D01 | d1 | E01 |
| 2-1-m1E01-2-0789 | 782.208 | OEt | a2 | B18 | b3 | C15 | c2 | D02 | d1 | E01 |
| 2-1-m1E01-2-0790 | 782.219 | OEt | a1 | B08 | b2 | C02 | c2 | D02 | d1 | E01 |
| 2-1-m1E01-2-0791 | 782.220 | OEt | a2 | B17 | b1 | C09 | c2 | D02 | d1 | E01 |
| 2-1-m1E01-2-0792 | 782.235 | OEt | a1 | B10 | b1 | C11 | c2 | D02 | d1 | E01 |
| 2-1-m1E01-2-0793 | 782.256 | OEt | a1 | B03 | b3 | C18 | c1 | D01 | d1 | E01 |
| 2-1-m1E01-2-0794 | 782.256 | OEt | a1 | B08 | b2 | C22 | c3 | D02 | d1 | E01 |
| 2-1-m1E01-2-0795 | 782.277 | OEt | a2 | B18 | b3 | C16 | c1 | D01 | d1 | E01 |
| 2-1-m1E01-2-0796 | 782.295 | OEt | a2 | B17 | b1 | C31 | c3 | D02 | d1 | E01 |
| 2-1-m1E01-2-0797 | 783.215 | OEt | a1 | B08 | b2 | C03 | c2 | D02 | d1 | E01 |
| 2-1-m1E01-2-0798 | 783.219 | OEt | a1 | B09 | b2 | C01 | c2 | D02 | d1 | E01 |
| 2-1-m1E01-2-0799 | 783.230 | OEt | a1 | B10 | b1 | C10 | c2 | D02 | d1 | E01 |
| 2-1-m1E01-2-0800 | 783.235 | OEt | a1 | B04 | b1 | C09 | c2 | D02 | d1 | E01 |
| 2-1-m1E01-2-0801 | 783.240 | OEt | a1 | B06 | b3 | C17 | c1 | D01 | d1 | E01 |
| 2-1-m1E01-2-0802 | 783.240 | OEt | a1 | B06 | b1 | C14 | c2 | D02 | d1 | E01 |
| 2-1-m1E01-2-0803 | 783.310 | OEt | a1 | B04 | b1 | C31 | c3 | D02 | d1 | E01 |
| 2-1-m1E01-2-0804 | 784.151 | OEt | a2 | B17 | b3 | C06 | c2 | D02 | d1 | E01 |
| 2-1-m1E01-2-0805 | 784.239 | OEt | a2 | B18 | b3 | C17 | c1 | D01 | d1 | E01 |
| 2-1-m1E01-2-0806 | 784.239 | OEt | a2 | B18 | b1 | C14 | c2 | D02 | d1 | E01 |
| 2-1-m1E01-2-0807 | 785.166 | OEt | a1 | B04 | b3 | C06 | c2 | D02 | d1 | E01 |
| 2-1-m1E01-2-0808 | 785.215 | OEt | a2 | B17 | b3 | C18 | c1 | D01 | d1 | E01 |
| 2-1-m1E01-2-0809 | 785.244 | OEt | a1 | B07 | b1 | C13 | c2 | D02 | d1 | E01 |
| 2-1-m1E01-2-0810 | 785.265 | OEt | a1 | B10 | b1 | C09 | c1 | D01 | d1 | E01 |
| 2-1-m1E01-2-0811 | 785.346 | OEt | a1 | B05 | b2 | C07 | c2 | D02 | d1 | E01 |
| 2-1-m1E01-2-0812 | 786.231 | OEt | a1 | B04 | b3 | C18 | c1 | D01 | d1 | E01 |
| 2-1-m1E01-2-0813 | 786.231 | OEt | a1 | B08 | b2 | C24 | c3 | D02 | d1 | E01 |
| 2-1-m1E01-2-0814 | 786.306 | OEt | a2 | B19 | b1 | C29 | c3 | D02 | d1 | E01 |
| 2-1-m1E01-2-0815 | 787.179 | OEt | a1 | B07 | b3 | C15 | c2 | D02 | d1 | E01 |

(continued)

| ID | Exact Mass | ☒ | a | B | b | C | c | D | d | E |
|---|---|---|---|---|---|---|---|---|---|---|
| 2-1-m1E01-2-0816 | 787.196 | OEt | a1 | B10 | b3 | C06 | c1 | D01 | d1 | E01 |
| 2-1-m1E01-2-0817 | 787.249 | OEt | a1 | B07 | b3 | C16 | c1 | D01 | d1 | E01 |
| 2-1-m1E01-2-0818 | 787.325 | OEt | a1 | B06 | b2 | C07 | c2 | D02 | d1 | E01 |
| 2-1-m1E01-2-0819 | 788.176 | OEt | a1 | B08 | b2 | C06 | c2 | D02 | d1 | E01 |
| 2-1-m1E01-2-0820 | 788.191 | OEt | a1 | B10 | b1 | C12 | c2 | D02 | d1 | E01 |
| 2-1-m1E01-2-0821 | 788.286 | OEt | a2 | B20 | b1 | C32 | c3 | D02 | d1 | E01 |
| 2-1-m1E01-2-0822 | 788.324 | OEt | a2 | B18 | b2 | C07 | c2 | D02 | d1 | E01 |
| 2-1-m1E01-2-0823 | 789.210 | OEt | a1 | B07 | b3 | C17 | c1 | D01 | d1 | E01 |
| 2-1-m1E01-2-0824 | 789.210 | OEt | a1 | B07 | b1 | C14 | c2 | D02 | d1 | E01 |
| 2-1-m1E01-2-0825 | 789.244 | OEt | a2 | B19 | b2 | C05 | c2 | D02 | d1 | E01 |
| 2-1-m1E01-2-0826 | 789.250 | OEt | a1 | B05 | b3 | C08 | c2 | D02 | d1 | E01 |
| 2-1-m1E01-2-0827 | 790.228 | OEt | a2 | B20 | b2 | C02 | c2 | D02 | d1 | E01 |
| 2-1-m1E01-2-0828 | 790.265 | OEt | a2 | B20 | b2 | C22 | c3 | D02 | d1 | E01 |
| 2-1-m1E01-2-0829 | 790.265 | OEt | a2 | B19 | b2 | C26 | c3 | D02 | d1 | E01 |
| 2-1-m1E01-2-0830 | 791.224 | OEt | a2 | B20 | b2 | C03 | c2 | D02 | d1 | E01 |
| 2-1-m1E01-2-0831 | 791.229 | OEt | a1 | B06 | b3 | C08 | c2 | D02 | d1 | E01 |
| 2-1-m1E01-2-0832 | 791.296 | OEt | a1 | B09 | b1 | C32 | c3 | D02 | d1 | E01 |
| 2-1-m1E01-2-0833 | 792.224 | OEt | a2 | B19 | b2 | C04 | c2 | D02 | d1 | E01 |
| 2-1-m1E01-2-0834 | 792.229 | OEt | a2 | B18 | b3 | C08 | c2 | D02 | d1 | E01 |
| 2-1-m1E01-2-0835 | 793.239 | OEt | a1 | B09 | b2 | C02 | c2 | D02 | d1 | E01 |
| 2-1-m1E01-2-0836 | 793.276 | OEt | a1 | B05 | b1 | C09 | c2 | D02 | d1 | E01 |
| 2-1-m1E01-2-0837 | 793.276 | OEt | a1 | B09 | b2 | C22 | c3 | D02 | d1 | E01 |
| 2-1-m1E01-2-0838 | 793.276 | OEt | a1 | B10 | b1 | C30 | c3 | D02 | d1 | E01 |
| 2-1-m1E01-2-0839 | 793.296 | OEt | a1 | B07 | b2 | C07 | c2 | D02 | d1 | E01 |
| 2-1-m1E01-2-0840 | 793.351 | OEt | a1 | B05 | b1 | C31 | c3 | D02 | d1 | E01 |
| 2-1-m1E01-2-0841 | 794.235 | OEt | a1 | B09 | b2 | C03 | c2 | D02 | d1 | E01 |
| 2-1-m1E01-2-0842 | 794.240 | OEt | a2 | B20 | b2 | C24 | c3 | D02 | d1 | E01 |
| 2-1-m1E01-2-0843 | 794.292 | OEt | a1 | B08 | b1 | C29 | c3 | D02 | d1 | E01 |
| 2-1-m1E01-2-0844 | 795.207 | OEt | a1 | B05 | b3 | C06 | c2 | D02 | d1 | E01 |
| 2-1-m1E01-2-0845 | 795.255 | OEt | a1 | B06 | b1 | C09 | c2 | D02 | d1 | E01 |
| 2-1-m1E01-2-0846 | 795.255 | OEt | a1 | B10 | b2 | C20 | c3 | D02 | d1 | E01 |
| 2-1-m1E01-2-0847 | 795.330 | OEt | a1 | B06 | b1 | C31 | c3 | D02 | d1 | E01 |
| 2-1-m1E01-2-0848 | 796.185 | OEt | a2 | B20 | b2 | C06 | c2 | D02 | d1 | E01 |
| 2-1-m1E01-2-0849 | 796.224 | OEt | a2 | B11 | b3 | C16 | c2 | D02 | d1 | E01 |
| 2-1-m1E01-2-0850 | 796.250 | OEt | a1 | B10 | b2 | C28 | c3 | D02 | d1 | E01 |
| 2-1-m1E01-2-0851 | 796.254 | OEt | a2 | B18 | b1 | C09 | c2 | D02 | d1 | E01 |
| 2-1-m1E01-2-0852 | 796.271 | OEt | a1 | B05 | b3 | C18 | c1 | D01 | d1 | E01 |

(continued)

| ID | Exact Mass | ☒ | a | B | b | C | c | D | d | E |
|---|---|---|---|---|---|---|---|---|---|---|
| 2-1-m1E01-2-0853 | 796.329 | OEt | a2 | B18 | b1 | C31 | c3 | D02 | d1 | E01 |
| 2-1-m1E01-2-0854 | 797.186 | OEt | a1 | B06 | b3 | C06 | c2 | D02 | d1 | E01 |
| 2-1-m1E01-2-0855 | 797.200 | OEt | a1 | B07 | b3 | C08 | c2 | D02 | d1 | E01 |
| 2-1-m1E01-2-0856 | 797.219 | OEt | a2 | B12 | b3 | C16 | c2 | D02 | d1 | E01 |
| 2-1-m1E01-2-0857 | 797.230 | OEt | a1 | B08 | b2 | C05 | c2 | D02 | d1 | E01 |
| 2-1-m1E01-2-0858 | 797.251 | OEt | a1 | B09 | b2 | C24 | c3 | D02 | d1 | E01 |
| 2-1-m1E01-2-0859 | 798.185 | OEt | a2 | B18 | b3 | C06 | c2 | D02 | d1 | E01 |
| 2-1-m1E01-2-0860 | 798.185 | OEt | a2 | B11 | b3 | C17 | c2 | D02 | d1 | E01 |
| 2-1-m1E01-2-0861 | 798.245 | OEt | a2 | B19 | b1 | C13 | c2 | D02 | d1 | E01 |
| 2-1-m1E01-2-0862 | 798.251 | OEt | a1 | B06 | b3 | C18 | c1 | D01 | d1 | E01 |
| 2-1-m1E01-2-0863 | 798.251 | OEt | a1 | B08 | b2 | C26 | c3 | D02 | d1 | E01 |
| 2-1-m1E01-2-0864 | 799.180 | OEt | a2 | B12 | b3 | C17 | c2 | D02 | d1 | E01 |
| 2-1-m1E01-2-0865 | 799.196 | OEt | a1 | B09 | b2 | C06 | c2 | D02 | d1 | E01 |
| 2-1-m1E01-2-0866 | 799.213 | OEt | a1 | B10 | b2 | C01 | c2 | D02 | d1 | E01 |
| 2-1-m1E01-2-0867 | 799.250 | OEt | a2 | B18 | b3 | C18 | c1 | D01 | d1 | E01 |
| 2-1-m1E01-2-0868 | 800.180 | OEt | a2 | B19 | b3 | C15 | c2 | D02 | d1 | E01 |
| 2-1-m1E01-2-0869 | 800.210 | OEt | a1 | B08 | b2 | C04 | c2 | D02 | d1 | E01 |
| 2-1-m1E01-2-0870 | 800.249 | OEt | a2 | B19 | b3 | C16 | c1 | D01 | d1 | E01 |
| 2-1-m1E01-2-0871 | 801.226 | OEt | a1 | B07 | b1 | C09 | c2 | D02 | d1 | E01 |
| 2-1-m1E01-2-0872 | 801.301 | OEt | a1 | B07 | b1 | C31 | c3 | D02 | d1 | E01 |
| 2-1-m1E01-2-0873 | 802.211 | OEt | a2 | B19 | b3 | C17 | c1 | D01 | d1 | E01 |
| 2-1-m1E01-2-0874 | 802.211 | OEt | a2 | B19 | b1 | C14 | c2 | D02 | d1 | E01 |
| 2-1-m1E01-2-0875 | 802.301 | OEt | a2 | B20 | b1 | C29 | c3 | D02 | d1 | E01 |
| 2-1-m1E01-2-0876 | 803.156 | OEt | a1 | B07 | b3 | C06 | c2 | D02 | d1 | E01 |
| 2-1-m1E01-2-0877 | 804.221 | OEt | a1 | B07 | b3 | C18 | c1 | D01 | d1 | E01 |
| 2-1-m1E01-2-0878 | 805.239 | OEt | a2 | B20 | b2 | C05 | c2 | D02 | d1 | E01 |
| 2-1-m1E01-2-0879 | 805.312 | OEt | a1 | B09 | b1 | C29 | c3 | D02 | d1 | E01 |
| 2-1-m1E01-2-0880 | 806.230 | OEt | a1 | B08 | b1 | C13 | c2 | D02 | d1 | E01 |
| 2-1-m1E01-2-0881 | 806.260 | OEt | a2 | B20 | b2 | C26 | c3 | D02 | d1 | E01 |
| 2-1-m1E01-2-0882 | 806.296 | OEt | a2 | B19 | b2 | C07 | c2 | D02 | d1 | E01 |
| 2-1-m1E01-2-0883 | 807.291 | OEt | a1 | B10 | b1 | C32 | c3 | D02 | d1 | E01 |
| 2-1-m1E01-2-0884 | 808.166 | OEt | a1 | B08 | b3 | C15 | c2 | D02 | d1 | E01 |
| 2-1-m1E01-2-0885 | 808.219 | OEt | a2 | B20 | b2 | C04 | c2 | D02 | d1 | E01 |
| 2-1-m1E01-2-0886 | 808.235 | OEt | a1 | B08 | b3 | C16 | c1 | D01 | d1 | E01 |
| 2-1-m1E01-2-0887 | 808.250 | OEt | a1 | B09 | b2 | C05 | c2 | D02 | d1 | E01 |
| 2-1-m1E01-2-0888 | 809.234 | OEt | a1 | B10 | b2 | C02 | c2 | D02 | d1 | E01 |
| 2-1-m1E01-2-0889 | 809.271 | OEt | a1 | B10 | b2 | C22 | c3 | D02 | d1 | E01 |

(continued)

| ID | Exact Mass | ☒ | a | B | b | C | c | D | d | E |
|---|---|---|---|---|---|---|---|---|---|---|
| 2-1-m1E01-2-0890 | 809.271 | OEt | a1 | B09 | b2 | C26 | c3 | D02 | d1 | E01 |
| 2-1-m1E01-2-0891 | 810.196 | OEt | a1 | B08 | b3 | C17 | c1 | D01 | d1 | E01 |
| 2-1-m1E01-2-0892 | 810.196 | OEt | a1 | B08 | b1 | C14 | c2 | D02 | d1 | E01 |
| 2-1-m1E01-2-0893 | 810.200 | OEt | a2 | B19 | b3 | C08 | c2 | D02 | d1 | E01 |
| 2-1-m1E01-2-0894 | 810.229 | OEt | a1 | B10 | b2 | C03 | c2 | D02 | d1 | E01 |
| 2-1-m1E01-2-0895 | 810.239 | OEt | a2 | B13 | b3 | C16 | c2 | D02 | d1 | E01 |
| 2-1-m1E01-2-0896 | 811.230 | OEt | a1 | B09 | b2 | C04 | c2 | D02 | d1 | E01 |
| 2-1-m1E01-2-0897 | 812.201 | OEt | a2 | B13 | b3 | C17 | c2 | D02 | d1 | E01 |
| 2-1-m1E01-2-0898 | 813.196 | OEt | a2 | B11 | b3 | C18 | c2 | D02 | d1 | E01 |
| 2-1-m1E01-2-0899 | 813.246 | OEt | a1 | B10 | b2 | C24 | c3 | D02 | d1 | E01 |
| 2-1-m1E01-2-0900 | 814.191 | OEt | a2 | B12 | b3 | C18 | c2 | D02 | d1 | E01 |
| 2-1-m1E01-2-0901 | 814.214 | OEt | a2 | B14 | b3 | C16 | c2 | D02 | d1 | E01 |
| 2-1-m1E01-2-0902 | 814.226 | OEt | a2 | B19 | b1 | C09 | c2 | D02 | d1 | E01 |
| 2-1-m1E01-2-0903 | 814.240 | OEt | a2 | B20 | b1 | C13 | c2 | D02 | d1 | E01 |
| 2-1-m1E01-2-0904 | 814.282 | OEt | a1 | B08 | b2 | C07 | c2 | D02 | d1 | E01 |
| 2-1-m1E01-2-0905 | 814.301 | OEt | a2 | B19 | b1 | C31 | c3 | D02 | d1 | E01 |
| 2-1-m1E01-2-0906 | 815.191 | OEt | a1 | B10 | b2 | C06 | c2 | D02 | d1 | E01 |
| 2-1-m1E01-2-0907 | 815.229 | OEt | a1 | B01 | b3 | C16 | c2 | D02 | d1 | E01 |
| 2-1-m1E01-2-0908 | 816.157 | OEt | a2 | B19 | b3 | C06 | c2 | D02 | d1 | E01 |
| 2-1-m1E01-2-0909 | 816.175 | OEt | a2 | B20 | b3 | C15 | c2 | D02 | d1 | E01 |
| 2-1-m1E01-2-0910 | 816.176 | OEt | a2 | B14 | b3 | C17 | c2 | D02 | d1 | E01 |
| 2-1-m1E01-2-0911 | 816.225 | OEt | a1 | B02 | b3 | C16 | c2 | D02 | d1 | E01 |
| 2-1-m1E01-2-0912 | 816.244 | OEt | a2 | B20 | b3 | C16 | c1 | D01 | d1 | E01 |
| 2-1-m1E01-2-0913 | 817.191 | OEt | a1 | B01 | b3 | C17 | c2 | D02 | d1 | E01 |
| 2-1-m1E01-2-0914 | 817.222 | OEt | a2 | B19 | b3 | C18 | c1 | D01 | d1 | E01 |
| 2-1-m1E01-2-0915 | 817.251 | OEt | a1 | B09 | b1 | C13 | c2 | D02 | d1 | E01 |
| 2-1-m1E01-2-0916 | 818.186 | OEt | a1 | B02 | b3 | C17 | c2 | D02 | d1 | E01 |
| 2-1-m1E01-2-0917 | 818.186 | OEt | a1 | B08 | b3 | C08 | c2 | D02 | d1 | E01 |
| 2-1-m1E01-2-0918 | 818.206 | OEt | a2 | B20 | b3 | C17 | c1 | D01 | d1 | E01 |
| 2-1-m1E01-2-0919 | 818.206 | OEt | a2 | B20 | b1 | C14 | c2 | D02 | d1 | E01 |
| 2-1-m1E01-2-0920 | 819.186 | OEt | a1 | B09 | b3 | C15 | c2 | D02 | d1 | E01 |
| 2-1-m1E01-2-0921 | 819.255 | OEt | a1 | B09 | b3 | C16 | c1 | D01 | d1 | E01 |
| 2-1-m1E01-2-0922 | 821.216 | OEt | a1 | B09 | b3 | C17 | c1 | D01 | d1 | E01 |
| 2-1-m1E01-2-0923 | 821.216 | OEt | a1 | B09 | b1 | C14 | c2 | D02 | d1 | E01 |
| 2-1-m1E01-2-0924 | 821.307 | OEt | a1 | B10 | b1 | C29 | c3 | D02 | d1 | E01 |
| 2-1-m1E01-2-0925 | 822.212 | OEt | a1 | B08 | b1 | C09 | c2 | D02 | d1 | E01 |
| 2-1-m1E01-2-0926 | 822.287 | OEt | a1 | B08 | b1 | C31 | c3 | D02 | d1 | E01 |

(continued)

| ID | Exact Mass | ⊠ | a | B | b | C | c | D | d | E |
|---|---|---|---|---|---|---|---|---|---|---|
| 2-1-m1E01-2-0927 | 822.291 | OEt | a2 | B20 | b2 | C07 | c2 | D02 | d1 | E01 |
| 2-1-m1E01-2-0928 | 824.143 | OEt | a1 | B08 | b3 | C06 | c2 | D02 | d1 | E01 |
| 2-1-m1E01-2-0929 | 824.245 | OEt | a1 | B10 | b2 | C05 | c2 | D02 | d1 | E01 |
| 2-1-m1E01-2-0930 | 824.255 | OEt | a2 | B15 | b3 | C16 | c2 | D02 | d1 | E01 |
| 2-1-m1E01-2-0931 | 825.207 | OEt | a1 | B08 | b3 | C18 | c1 | D01 | d1 | E01 |
| 2-1-m1E01-2-0932 | 825.266 | OEt | a1 | B10 | b2 | C26 | c3 | D02 | d1 | E01 |
| 2-1-m1E01-2-0933 | 825.302 | OEt | a1 | B09 | b2 | C07 | c2 | D02 | d1 | E01 |
| 2-1-m1E01-2-0934 | 826.195 | OEt | a2 | B20 | b3 | C08 | c2 | D02 | d1 | E01 |
| 2-1-m1E01-2-0935 | 826.216 | OEt | a2 | B15 | b3 | C17 | c2 | D02 | d1 | E01 |
| 2-1-m1E01-2-0936 | 826.234 | OEt | a2 | B16 | b3 | C16 | c2 | D02 | d1 | E01 |
| 2-1-m1E01-2-0937 | 827.212 | OEt | a2 | B13 | b3 | C18 | c2 | D02 | d1 | E01 |
| 2-1-m1E01-2-0938 | 827.225 | OEt | a1 | B10 | b2 | C04 | c2 | D02 | d1 | E01 |
| 2-1-m1E01-2-0939 | 828.196 | OEt | a2 | B16 | b3 | C17 | c2 | D02 | d1 | E01 |
| 2-1-m1E01-2-0940 | 829.206 | OEt | a1 | B09 | b3 | C08 | c2 | D02 | d1 | E01 |
| 2-1-m1E01-2-0941 | 829.245 | OEt | a1 | B03 | b3 | C16 | c2 | D02 | d1 | E01 |
| 2-1-m1E01-2-0942 | 830.221 | OEt | a2 | B20 | b1 | C09 | c2 | D02 | d1 | E01 |
| 2-1-m1E01-2-0943 | 830.296 | OEt | a2 | B20 | b1 | C31 | c3 | D02 | d1 | E01 |
| 2-1-m1E01-2-0944 | 831.187 | OEt | a2 | B14 | b3 | C18 | c2 | D02 | d1 | E01 |
| 2-1-m1E01-2-0945 | 831.207 | OEt | a1 | B03 | b3 | C17 | c2 | D02 | d1 | E01 |
| 2-1-m1E01-2-0946 | 832.152 | OEt | a2 | B20 | b3 | C06 | c2 | D02 | d1 | E01 |
| 2-1-m1E01-2-0947 | 832.202 | OEt | a1 | B01 | b3 | C18 | c2 | D02 | d1 | E01 |
| 2-1-m1E01-2-0948 | 832.205 | OEt | a2 | B17 | b3 | C16 | c2 | D02 | d1 | E01 |
| 2-1-m1E01-2-0949 | 833.197 | OEt | a1 | B02 | b3 | C18 | c2 | D02 | d1 | E01 |
| 2-1-m1E01-2-0950 | 833.216 | OEt | a2 | B20 | b3 | C18 | c1 | D01 | d1 | E01 |
| 2-1-m1E01-2-0951 | 833.220 | OEt | a1 | B04 | b3 | C16 | c2 | D02 | d1 | E01 |
| 2-1-m1E01-2-0952 | 833.232 | OEt | a1 | B09 | b1 | C09 | c2 | D02 | d1 | E01 |
| 2-1-m1E01-2-0953 | 833.245 | OEt | a1 | B10 | b1 | C13 | c2 | D02 | d1 | E01 |
| 2-1-m1E01-2-0954 | 833.307 | OEt | a1 | B09 | b1 | C31 | c3 | D02 | d1 | E01 |
| 2-1-m1E01-2-0955 | 834.166 | OEt | a2 | B17 | b3 | C17 | c2 | D02 | d1 | E01 |
| 2-1-m1E01-2-0956 | 835.163 | OEt | a1 | B09 | b3 | C06 | c2 | D02 | d1 | E01 |
| 2-1-m1E01-2-0957 | 835.180 | OEt | a1 | B10 | b3 | C15 | c2 | D02 | d1 | E01 |
| 2-1-m1E01-2-0958 | 835.182 | OEt | a1 | B04 | b3 | C17 | c2 | D02 | d1 | E01 |
| 2-1-m1E01-2-0959 | 835.250 | OEt | a1 | B10 | b3 | C16 | c1 | D01 | d1 | E01 |
| 2-1-m1E01-2-0960 | 836.227 | OEt | a1 | B09 | b3 | C18 | c1 | D01 | d1 | E01 |
| 2-1-m1E01-2-0961 | 837.211 | OEt | a1 | B10 | b3 | C17 | c1 | D01 | d1 | E01 |
| 2-1-m1E01-2-0962 | 837.211 | OEt | a1 | B10 | b1 | C14 | c2 | D02 | d1 | E01 |
| 2-1-m1E01-2-0963 | 841.227 | OEt | a2 | B15 | b3 | C18 | c2 | D02 | d1 | E01 |

(continued)

| ID | Exact Mass | ☒ | a | B | b | C | c | D | d | E |
|---|---|---|---|---|---|---|---|---|---|---|
| 2-1-m1E01-2-0964 | 841.297 | OEt | a1 | B10 | b2 | C07 | c2 | D02 | d1 | E01 |
| 2-1-m1E01-2-0965 | 843.207 | OEt | a2 | B16 | b3 | C18 | c2 | D02 | d1 | E01 |
| 2-1-m1E01-2-0966 | 843.261 | OEt | a1 | B05 | b3 | C16 | c2 | D02 | d1 | E01 |
| 2-1-m1E01-2-0967 | 845.201 | OEt | a1 | B10 | b3 | C08 | c2 | D02 | d1 | E01 |
| 2-1-m1E01-2-0968 | 845.222 | OEt | a1 | B05 | b3 | C17 | c2 | D02 | d1 | E01 |
| 2-1-m1E01-2-0969 | 845.240 | OEt | a1 | B06 | b3 | C16 | c2 | D02 | d1 | E01 |
| 2-1-m1E01-2-0970 | 846.218 | OEt | a1 | B03 | b3 | C18 | c2 | D02 | d1 | E01 |
| 2-1-m1E01-2-0971 | 846.239 | OEt | a2 | B18 | b3 | C16 | c2 | D02 | d1 | E01 |
| 2-1-m1E01-2-0972 | 847.202 | OEt | a1 | B06 | b3 | C17 | c2 | D02 | d1 | E01 |
| 2-1-m1E01-2-0973 | 848.201 | OEt | a2 | B18 | b3 | C17 | c2 | D02 | d1 | E01 |
| 2-1-m1E01-2-0974 | 849.177 | OEt | a2 | B17 | b3 | C18 | c2 | D02 | d1 | E01 |
| 2-1-m1E01-2-0975 | 849.227 | OEt | a1 | B10 | b1 | C09 | c2 | D02 | d1 | E01 |
| 2-1-m1E01-2-0976 | 849.302 | OEt | a1 | B10 | b1 | C31 | c3 | D02 | d1 | E01 |
| 2-1-m1E01-2-0977 | 850.192 | OEt | a1 | B04 | b3 | C18 | c2 | D02 | d1 | E01 |
| 2-1-m1E01-2-0978 | 851.158 | OEt | a1 | B10 | b3 | C06 | c2 | D02 | d1 | E01 |
| 2-1-m1E01-2-0979 | 851.211 | OEt | a1 | B07 | b3 | C16 | c2 | D02 | d1 | E01 |
| 2-1-m1E01-2-0980 | 852.222 | OEt | a1 | B10 | b3 | C18 | c1 | D01 | d1 | E01 |
| 2-1-m1E01-2-0981 | 853.172 | OEt | a1 | B07 | b3 | C17 | c2 | D02 | d1 | E01 |
| 2-1-m1E01-2-0982 | 860.233 | OEt | a1 | B05 | b3 | C18 | c2 | D02 | d1 | E01 |
| 2-1-m1E01-2-0983 | 862.212 | OEt | a1 | B06 | b3 | C18 | c2 | D02 | d1 | E01 |
| 2-1-m1E01-2-0984 | 863.212 | OEt | a2 | B18 | b3 | C18 | c2 | D02 | d1 | E01 |
| 2-1-m1E01-2-0985 | 864.211 | OEt | a2 | B19 | b3 | C16 | c2 | D02 | d1 | E01 |
| 2-1-m1E01-2-0986 | 866.173 | OEt | a2 | B19 | b3 | C17 | c2 | D02 | d1 | E01 |
| 2-1-m1E01-2-0987 | 868.183 | OEt | a1 | B07 | b3 | C18 | c2 | D02 | d1 | E01 |
| 2-1-m1E01-2-0988 | 872.197 | OEt | a1 | B08 | b3 | C16 | c2 | D02 | d1 | E01 |
| 2-1-m1E01-2-0989 | 874.158 | OEt | a1 | B08 | b3 | C17 | c2 | D02 | d1 | E01 |
| 2-1-m1E01-2-0990 | 880.206 | OEt | a2 | B20 | b3 | C16 | c2 | D02 | d1 | E01 |
| 2-1-m1E01-2-0991 | 881.183 | OEt | a2 | B19 | b3 | C18 | c2 | D02 | d1 | E01 |
| 2-1-m1E01-2-0992 | 882.167 | OEt | a2 | B20 | b3 | C17 | c2 | D02 | d1 | E01 |
| 2-1-m1E01-2-0993 | 883.217 | OEt | a1 | B09 | b3 | C16 | c2 | D02 | d1 | E01 |
| 2-1-m1E01-2-0994 | 885.178 | OEt | a1 | B09 | b3 | C17 | c2 | D02 | d1 | E01 |
| 2-1-m1E01-2-0995 | 889.169 | OEt | a1 | B08 | b3 | C18 | c2 | D02 | d1 | E01 |
| 2-1-m1E01-2-0996 | 897.178 | OEt | a2 | B20 | b3 | C18 | c2 | D02 | d1 | E01 |
| 2-1-m1E01-2-0997 | 899.212 | OEt | a1 | B10 | b3 | C16 | c2 | D02 | d1 | E01 |
| 2-1-m1E01-2-0998 | 900.189 | OEt | a1 | B09 | b3 | C18 | c2 | D02 | d1 | E01 |
| 2-1-m1E01-2-0999 | 901.173 | OEt | a1 | B10 | b3 | C17 | c2 | D02 | d1 | E01 |
| 2-1-m1E01-2-1000 | 916.184 | OEt | a1 | B10 | b3 | C18 | c2 | D02 | d1 | E01 |

Example 6-7: Analysis of mixture

The mixture synthesized in Example 6-6 was analyzed by measuring the retention time and performing mass spectrometry under the analysis conditions shown below, using Compound Discoverer 3.2 (Thermo Fisher Scientific).

[Table 124]

| | |
|---|---|
| Reversed phase LC/MS conditions | |
| Vanquish UHPLC (Thermo Fisher Scientific) | |
| Oven (°C) | 35 |
| Column | Ascentis Express C18<br>2.1 mm I.D. × 150 mm, 2.7 μm |
| Autosampler (°C) | 20 |
| Mobile phase | A) 0.1% FA $H_2O$<br>B) 0.1% FA MeCN |
| Gradient | A/B = 95/5 → 0/100 (18 min) → 0/100 (20 min) |
| Flow rate | 0.5 mL/min |
| Orbitrap Fusion Lumos Tribrid mass spectrometer (Thermo Fisher Scientific) | |
| Polarity | Positive/Negative mode |
| Data Type | profile |

The analysis results for each mixture are shown in [Table 6-7-1].

[Table 125]

[Table 6-7-1] Compounds that may be included in mixture 2-1-m1E01-2

| Type of ion | m/z $[M+H]^+$ or $[M-H]^-$ | Retention time of MS peak (min) | Attributed compound No. Described omitting 2-1-m1E01-2- |
|---|---|---|---|
| M+H | 615.296 | 17.252 | 0001 |
| M+H | 616.2912 | 15.766 | 0002,0003 |
| M+H | 616.2921 | 16.748 | 0002,0003 |
| M+H | 617.2864 | 14.961 | 0004,0005 |
| M+H | 617.2868 | 18.132 | 0004,0005 |
| M+H | 618.2826 | 16.819 | 0006 |
| M+H | 622.2482 | 17.910 | 0007 |
| M+H | 623.2421 | 16.535 | 0008 |
| M+H | 629.3113 | 17.654 | 0009 |
| M+H | 630.3073 | 12.138 | 0010 |
| M+H | 631.3014 | 10.986 | 0011 |
| M+H | 633.2696 | 16.026 | 0012 |
| M+H | 633.2874 | 17.173 | 0013 |
| M+H | 634.2657 | 14.144 | 0014 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 634.2827 | 16.799 | 0015 |
| M+H | 634.3017 | 14.716 | 0016 |
| M+H | 635.2768 | 10.625 | 0017 |
| M+H | 635.2972 | 13.588 | 0018,0019 |
| M+H | 635.2966 | 11.635 | 0018,0019 |
| M+H | 636.2633 | 18.390 | 0020 |
| M+H | 636.2922 | 10.398 | 0021,0022 |
| M+H | 636.2939 | 15.822 | 0021,0022 |
| M+H | 637.2877 | 13.021 | 0023 |
| M+H | 640.2389 | 17.848 | 0024 |
| M+H | 641.2543 | 15.133 | 0025 |
| M+H | 642.2482 | 12.183 | 0026 |
| M+H | 643.2916 | 15.790 | 0027 |
| M+H | 643.3284 | 17.979 | 0028 |
| M+H | 644.2869 | 14.099 | 0029,0030 |
| M+H | 644.2863 | 10.186 | 0029,0030 |
| M+H | 644.3225 | 12.769 | 0031 |
| M+H | 645.2822 | 14.271 | 0032 |
| M+H | 645.3079 | 17.164 | 0033 |
| M+H | 645.3181 | 18.758 | 0034 |
| M+H | 646.3018 | 16.677 | 0035 |
| M+H | 647.2863 | 16.484 | 0036 |
| M+H | 647.2980 | 17.961 | 0037 |
| M+H | 648.3182 | 15.069 | 0038 |
| M+H | 649.2470 | 16.224 | 0039 |
| M+H | 649.3133 | 14.027 | 0040 |
| M+H | 650.2432 | 14.670 | 0041 |
| M+H | 650.2805 | 17.141 | 0042 |
| M+H | 650.3084 | 14.535 | 0043 |
| M+H | 651.2604 | 15.993 | 0044 |
| M+H | 651.2754 | 13.334 | 0045 |
| M+H | 652.2584 | 17.776 | 0046 |
| M+H | 652.2725 | 17.059 | 0047,0048 |
| M-H | 650.2586 | 13.796 | 0047,0048 |
| M+H | 652.2942 | 11.170 | 0049 |
| M+H | 653.2672 | 18.198 | 0050,0051 |
| M+H | 653.2714 | 10.600 | 0050,0051 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 653.2870 | 12.907 | 0052 |
| M+H | 654.2846 | 15.820 | 0053 |
| M+H | 655.2689 | 15.546 | 0054 |
| M+H | 657.3065 | 16.333 | 0055 |
| M+H | 658.2298 | 18.013 | 0056 |
| M+H | 658.3021 | 15.741 | 0057,0058 |
| M+H | 658.3018 | 10.667 | 0057,0058 |
| M+H | 659.2446 | 15.226 | 0059 |
| M+H | 659.2972 | 14.033 | 0060 |
| M+H | 661.2812 | 15.924 | 0061,0062 |
| M-H | 659.2634 | 15.849 | 0061,0062 |
| M+H | 661.3017 | 16.974 | 0063 |
| M+H | 662.2762 | 10.328 | 0064,0065 |
| M+H | 662.2770 | 16.194 | 0064,0065 |
| M+H | 662.2968 | 13.437 | 0066 |
| M+H | 662.3338 | 15.595 | 0067 |
| M+H | 663.2626 | 16.704 | 0068 |
| M+H | 663.2819 | 15.859 | 0069 |
| M+H | 663.2916 | 10.306 | 0070,0071 |
| M+H | 663.2923 | 13.796 | 0070,0071 |
| M+H | 663.3294 | 14.603 | 0072 |
| M+H | 664.2873 | 10.637 | 0073 |
| M+H | 664.3120 | 14.447 | 0074 |
| M+H | 664.3239 | 12.308 | 0075 |
| M+H | 665.3080 | 13.566 | 0076,0077 |
| M+H | 665.3110 | 17.998 | 0076,0077 |
| M+H | 666.2917 | 14.217 | 0078 |
| M+H | 666.3020 | 15.686 | 0079 |
| M+H | 666.3076 | 17.731 | 0079,0080 |
| M+H | 667.2388 | 14.082 | 0081 |
| M+H | 667.3013 | 16.569 | 0082,0083 |
| M+H | 667.3013 | 16.594 | 0082,0083 |
| M+H | 668.2530 | 13.582 | 0084 |
| M+H | 668.2970 | 14.635 | 0085 |
| M+H | 669.2384 | 13.926 | 0086 |
| M+H | 669.2485 | 14.546 | 0087,0088 |
| M+H | 669.2487 | 10.568 | 0087,0088 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 669.2851 | 15.997 | 0089 |
| M+H | 670.2323 | 14.607 | 0090 |
| M+H | 670.2656 | 13.856 | 0091 |
| M+H | 670.2842 | 15.407 | 0092 |
| M+H | 671.2639 | 15.095 | 0093,0094 |
| M+H | 671.2684 | 17.988 | 0093,0094 |
| M+H | 671.2786 | 14.526 | 0095 |
| M+H | 671.2910 | 15.790 | 0096 |
| M+H | 671.3243 | 16.814 | 0097 |
| M+H | 672.2627 | 12.732 | 0098,0099 |
| M+H | 672.2628 | 16.856 | 0098,0099 |
| M+H | 672.2729 | 16.489 | 0100 |
| M+H | 672.2849 | 14.072 | 0101 |
| M+H | 672.3176 | 11.310 | 0102,0103 |
| M+H | 672.3175 | 13.583 | 0102,0103 |
| M+H | 673.3029 | 15.751 | 0104 |
| M+H | 674.2962 | 15.999 | 0105 |
| M+H | 675.2965 | 14.262 | 0106 |
| M+H | 675.3538 | 16.332 | 0107 |
| M+H | 676.2940 | 15.826 | 0108 |
| M-H | 674.2946 | 13.847 | 0109 |
| M+H | 676.3491 | 14.582 | 0110 |
| M+H | 677.2358 | 15.817 | 0111 |
| M+H | 677.2772 | 16.134 | 0112 |
| M+H | 677.3075 | 14.248 | 0113,0114 |
| M+H | 677.3075 | 13.062 | 0113,0114 |
| M+H | 678.3022 | 9.947 | 0115 |
| M+H | 679.2574 | 15.081 | 0116,0117,0118 |
| M+H | 679.2573 | 15.165 | 0116,0117,0118 |
| M+H | 679.2596 | 16.594 | 0116,0117,0118 |
| M+H | 679.2701 | 16.073 | 0119,0120 |
| M+H | 679.2731 | 2.379 | 0119,0120 |
| M+H | 680.2530 | 14.950 | 0121,0122,0123 |
| M+H | 680.2530 | 15.078 | 0121,0122,0123 |
| M+H | 680.2530 | 13.453 | 0121,0122,0123 |
| M+H | 680.2675 | 16.368 | 0124 |
| M+H | 680.2869 | 13.492 | 0125,0126 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M-H | 678.2699 | 13.449 | 0125,0126 |
| M+H | 680.3065 | 14.758 | 0127 |
| M+H | 681.2487 | 14.493 | 0128,0129 |
| M+H | 681.2481 | 13.177 | 0128,0129 |
| M+H | 681.2833 | 13.846 | 0130,0131 |
| M+H | 681.2822 | 13.901 | 0130,0131 |
| M+H | 681.3201 | 14.149 | 0132 |
| M+H | 682.2435 | 15.823 | 0133 |
| M-H | 680.2510 | 13.985 | 0134 |
| M+H | 682.2901 | 11.290 | 0135 |
| M+H | 683.2554 | 14.695 | 0136 |
| M+H | 683.2838 | 17.684 | 0137,0138,0139 |
| M+H | 683.2836 | 17.160 | 0137,0138,0139 |
| M+H | 683.2847 | 16.868 | 0137,0138,0139 |
| M+H | 684.2792 | 15.811 | 0140,0141 |
| M+H | 684.2795 | 15.767 | 0140,0141 |
| M+H | 685.2129 | 16.678 | 0142 |
| M+H | 685.2271 | 16.444 | 0143 |
| M+H | 685.2738 | 8.939 | 0144 |
| M+H | 685.2839 | 15.221 | 0145 |
| M+H | 685.2918 | 16.595 | 0146 |
| M+H | 685.3038 | 9.451 | 0147 |
| M+H | 686.2109 | 17.173 | 0148,0149 |
| M+H | 686.2109 | 17.107 | 0148,0149 |
| M+H | 686.2434 | 13.604 | 0150 |
| M-H | 684.2905 | 13.503 | 0151 |
| M+H | 686.3343 | 16.646 | 0152 |
| M+H | 687.2043 | 15.619 | 0153 |
| M+H | 687.2293 | 16.187 | 0154 |
| M+H | 687.3029 | 10.301 | 0155 |
| M+H | 688.2431 | 14.248 | 0156 |
| M+H | 688.2569 | 14.352 | 0157 |
| M+H | 688.3120 | 15.403 | 0158 |
| M+H | 689.2379 | 11.167 | 0159 |
| M+H | 689.2601 | 14.356 | 0160 |
| M+H | 689.2813 | 15.715 | 0161 |
| M+H | 689.3134 | 17.270 | 0162 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 689.3712 | 16.897 | 0163 |
| M+H | 690.2344 | 18.258 | 0164 |
| M+H | 690.2734 | 15.547 | 0165 |
| M-H | 688.2785 | 13.425 | 0166 |
| M+H | 690.3284 | 14.340 | 0167 |
| M+H | 691.2687 | 12.811 | 0168,0169 |
| M+H | 691.2681 | 15.483 | 0168,0169 |
| M+H | 691.2904 | 10.453 | 0170,0171,0172 |
| M-H | 689.2794 | 13.125 | 0170,0171,0172 |
| M+H | 691.2948 | 17.722 | 0170,0171,0172 |
| M+H | 691.3237 | 13.408 | 0173,0174 |
| M+H | 691.3234 | 12.201 | 0173,0174 |
| M+H | 692.2646 | 14.927 | 0175 |
| M+H | 692.2908 | 16.486 | 0176 |
| M+H | 692.3085 | 13.956 | 0177 |
| M+H | 693.2600 | 16.515 | 0178 |
| M+H | 693.2743 | 17.054 | 0179,0180,0181 |
| M+H | 693.2733 | 15.497 | 0179,0180,0181 |
| M+H | 693.2743 | 17.102 | 0179,0180,0181 |
| M+H | 693.3040 | 13.798 | 0182,0183 |
| M+H | 693.3059 | 16.228 | 0182,0183 |
| M+H | 693.3435 | 16.356 | 0184 |
| M+H | 694.2689 | 13.800 | 0185,0186,0187 |
| M+H | 694.2700 | 16.750 | 0185,0186,0187 |
| M+H | 694.2706 | 15.463 | 0185,0186,0187 |
| M+H | 694.2823 | 15.936 | 0188 |
| M+H | 694.3036 | 13.571 | 0189,0190 |
| M+H | 694.3031 | 17.047 | 0189,0190 |
| M+H | 694.3589 | 13.823 | 0191 |
| M+H | 695.2660 | 13.996 | 0192,0193 |
| M+H | 695.2630 | 17.693 | 0192,0193 |
| M+H | 695.2985 | 13.067 | 0194 |
| M+H | 695.3333 | 17.611 | 0195 |
| M-H | 693.3377 | 10.705 | 0196 |
| M+H | 696.2845 | 14.428 | 0197 |
| M+H | 697.2343 | 13.973 | 0198 |
| M+H | 697.2486 | 15.229 | 0199,0200 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 697.2476 | 16.563 | 0199,0200 |
| M+H | 697.2627 | 17.796 | 0201 |
| M+H | 697.2706 | 15.025 | 0202 |
| M+H | 697.2985 | 17.528 | 0203 |
| M+H | 697.3126 | 16.663 | 0204 |
| M+H | 698.2205 | 16.015 | 0205 |
| M+H | 698.2275 | 13.881 | 0206 |
| M+H | 698.2435 | 13.373 | 0207 |
| M+H | 698.2638 | 12.925 | 0208,0209,0210 |
| M+H | 698.2634 | 14.362 | 0208,0209,0210 |
| M+H | 698.2653 | 14.127 | 0208,0209,0210 |
| M+H | 698.2781 | 14.027 | 0211,0212 |
| M+H | 698.2781 | 14.069 | 0211,0212 |
| M-H | 697.2124 | 17.068 | 0213 |
| M+H | 699.2424 | 14.531 | 0214 |
| M-H | 697.2318 | 16.580 | 0215 |
| M+H | 699.2587 | 12.608 | 0216,0217,0218,0219 |
| M+H | 699.2588 | 11.244 | 0216,0217,0218,0219 |
| M+H | 699.2587 | 9.851 | 0216,0217,0218,0219 |
| M+H | 699.2669 | 13.021 | 0219 |
| M+H | 699.2733 | 14.418 | 0220 |
| M+H | 699.2783 | 17.769 | 0220,0221 |
| M+H | 699.3013 | 10.955 | 0222 |
| M+H | 699.3213 | 16.740 | 0223 |
| M+H | 700.2244 | 17.595 | 0224 |
| M+H | 700.2543 | 10.169 | 0225,0226 |
| M+H | 700.2543 | 9.464 | 0225,0226 |
| M+H | 700.2734 | 13.050 | 0227 |
| M+H | 700.3243 | 13.946 | 0228 |
| M+H | 701.2531 | 10.868 | 0229 |
| M-H | 699.2390 | 16.637 | 0229,0230 |
| M+H | 701.2687 | 12.129 | 0231 |
| M+H | 701.2749 | 17.155 | 0232,0233 |
| M+H | 701.2785 | 17.912 | 0232,0233 |
| M+H | 701.2996 | 15.737 | 0234 |
| M+H | 702.2612 | 14.657 | 0235 |
| M+H | 702.2888 | 14.967 | 0236,0237 |

(continued)

| Type of ion | m/z [M+H]+ or [M-H]- | Retention time of MS peak (min) | Attributed compound No. Described omitting 2-1-m1E01-2- |
|---|---|---|---|
| M+H | 702.2897 | 15.613 | 0236,0237 |
| M+H | 701.1874 | 16.714 | 0238 |
| M+H | 703.2843 | 12.256 | 0239,0240,0241 |
| M+H | 703.2849 | 14.850 | 0239,0240,0241 |
| M+H | 703.2844 | 14.883 | 0239,0240,0241 |
| M+H | 703.3860 | 17.262 | 0242 |
| M+H | 704.1995 | 17.107 | 0243 |
| M+H | 704.2211 | 14.523 | 0244 |
| M+H | 704.2346 | 14.264 | 0245 |
| M+H | 704.2786 | 16.543 | 0246 |
| M+H | 704.2886 | 15.991 | 0247 |
| M+H | 704.2981 | 13.701 | 0248 |
| M+H | 704.3115 | 13.846 | 0249 |
| M-H | 703.1988 | 11.622 | 0250,0251,0252 |
| M+H | 705.2171 | 14.625 | 0250,0251,0252 |
| M+H | 705.2155 | 11.715 | 0250,0251,0252 |
| M+H | 705.3087 | 16.694 | 0253,0254 |
| M+H | 705.3087 | 16.784 | 0253,0254 |
| M+H | 705.3397 | 13.554 | 0255 |
| M+H | 705.3663 | 16.330 | 0256 |
| M+H | 706.2104 | 10.956 | 0257 |
| M+H | 706.2299 | 18.390 | 0258,0259 |
| M+H | 706.2354 | 13.995 | 0258,0259 |
| M+H | 706.3045 | 16.547 | 0260 |
| M+H | 707.2516 | 15.395 | 0261,0262 |
| M+H | 707.2516 | 15.294 | 0261,0262 |
| M+H | 707.2741 | 17.594 | 0263 |
| M+H | 707.2911 | 17.449 | 0264,0265,0266,0267 |
| M+H | 707.2881 | 16.055 | 0264,0265,0266,0267 |
| M+H | 707.2897 | 17.413 | 0264,0265,0266,0267 |
| M+H | 707.2879 | 16.206 | 0264,0265,0266,0267 |
| M+H | 707.3182 | 11.515 | 0268 |
| M+H | 708.2480 | 13.674 | 0269,0270 |
| M-H | 706.2298 | 13.629 | 0269,0270 |
| M+H | 708.2653 | 18.019 | 0271 |
| M+H | 708.2852 | 13.494 | 0272,0273,0274,0275 |
| M+H | 708.2852 | 13.537 | 0272,0273,0274,0275 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 708.2859 | 17.166 | 0272,0273,0274,0275 |
| M+H | 708.2873 | 17.218 | 0272,0273,0274,0275 |
| M+H | 708.3194 | 13.928 | 0276,0277 |
| M+H | 708.3178 | 16.569 | 0276,0277 |
| M+H | 708.3748 | 14.255 | 0278 |
| M+H | 709.2434 | 13.528 | 0279,0280,0281 |
| M+H | 709.2432 | 13.569 | 0279,0280,0281 |
| M-H | 707.2259 | 14.241 | 0279,0280,0281 |
| M+H | 709.2676 | 15.627 | 0282,0283 |
| M+H | 709.2687 | 14.726 | 0282,0283 |
| M+H | 709.2805 | 17.954 | 0284,0285 |
| M+H | 709.2828 | 13.904 | 0284,0285 |
| M+H | 710.2636 | 12.816 | 0286 |
| M+H | 710.3007 | 15.085 | 0287,0288 |
| M+H | 710.3022 | 14.517 | 0287,0288 |
| M+H | 711.2483 | 16.326 | 0289 |
| M-H | 709.2454 | 15.742 | 0290,0291,0292 |
| M+H | 711.2646 | 15.742 | 0291,0292 |
| M-H | 709.2508 | 12.898 | 0291,0292 |
| M+H | 711.2766 | 16.534 | 0293 |
| M+H | 711.2934 | 12.273 | 0294,0295 |
| M+H | 711.2990 | 18.595 | 0294,0295 |
| M+H | 711.3161 | 17.849 | 0296 |
| M+H | 711.3362 | 16.640 | 0297 |
| M+H | 712.2599 | 14.412 | 0298,0299 |
| M+H | 712.2599 | 14.325 | 0298,0299 |
| M+H | 712.2738 | 11.572 | 0300 |
| M+H | 712.2792 | 13.192 | 0301,0302,0303 |
| M+H | 712.2792 | 13.352 | 0301,0302,0303 |
| M+H | 712.2792 | 14.747 | 0301,0302,0303 |
| M+H | 712.3493 | 13.884 | 0304 |
| M+H | 713.2098 | 16.385 | 0305 |
| M+H | 713.2474 | 17.450 | 0306 |
| M+H | 713.2754 | 13.032 | 0307,0308,0309 |
| M+H | 713.2745 | 10.103 | 0307,0308,0309 |
| M+H | 713.2754 | 13.996 | 0307,0308,0309 |
| M+H | 713.2878 | 13.784 | 0310 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 713.2943 | 17.098 | 0310,0311 |
| M+H | 714.2041 | 14.526 | 0312 |
| M+H | 714.2429 | 17.907 | 0313 |
| M+H | 714.2722 | 15.223 | 0314,0315 |
| M-H | 712.2583 | 13.969 | 0314,0315 |
| M+H | 714.3410 | 14.507 | 0316 |
| M+H | 715.2215 | 15.885 | 0317,0318 |
| M-H | 713.2116 | 12.964 | 0317,0318 |
| M+H | 715.2381 | 15.275 | 0319,0320 |
| M+H | 715.2381 | 15.497 | 0319,0320 |
| M+H | 716.2211 | 17.039 | 0321 |
| M+H | 716.2343 | 15.350 | 0322,0323 |
| M-H | 714.2205 | 13.541 | 0322,0323 |
| M+H | 716.2538 | 12.974 | 0324,0325 |
| M+H | 716.2537 | 13.237 | 0324,0325 |
| M+H | 716.2684 | 8.983 | 0326 |
| M+H | 716.2740 | 15.130 | 0326,0327 |
| M-H | 714.2864 | 15.382 | 0328 |
| M+H | 716.3175 | 13.502 | 0329 |
| M+H | 717.2300 | 17.430 | 0330,0331 |
| M+H | 717.2330 | 10.061 | 0330,0331,0332 |
| M+H | 717.2378 | 16.858 | 0331,0332 |
| M+H | 717.2490 | 13.557 | 0333,0334 |
| M+H | 717.2500 | 10.113 | 0333,0334 |
| M+H | 717.3182 | 17.724 | 0335 |
| M+H | 718.2356 | 14.959 | 0336 |
| M+H | 718.2480 | 12.040 | 0337 |
| M+H | 718.2521 | 13.501 | 0338 |
| M+H | 718.2837 | 15.700 | 0339 |
| M+H | 718.3066 | 16.343 | 0340 |
| M+H | 718.3261 | 14.398 | 0341 |
| M+H | 719.2317 | 8.899 | 0342 |
| M+H | 719.2517 | 10.127 | 0343 |
| M+H | 719.2660 | 14.040 | 0344,0345 |
| M-H | 717.2462 | 14.063 | 0344,0345 |
| M+H | 719.2804 | 14.865 | 0346 |
| M+H | 719.3294 | 13.604 | 0347,0348,0349 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 719.3256 | 18.075 | 0347,0348,0349 |
| M+H | 719.3256 | 18.127 | 0347,0348,0349 |
| M+H | 720.2588 | 14.346 | 0350 |
| M+H | 720.2630 | 14.736 | 0350,0351 |
| M+H | 720.2733 | 16.649 | 0352 |
| M+H | 720.2793 | 14.956 | 0352,0353 |
| M+H | 720.2840 | 10.845 | 0353,0354 |
| M+H | 720.3050 | 13.477 | 0355 |
| M+H | 720.3205 | 11.236 | 0356 |
| M-H | 719.1770 | 16.865 | 0357 |
| M-H | 719.2514 | 15.916 | 0358 |
| M+H | 721.2808 | 18.693 | 0359 |
| M+H | 721.3045 | 17.653 | 0360,0361,0362,0363 |
| M+H | 721.3036 | 16.492 | 0360,0361,0362,0363 |
| M+H | 721.3039 | 16.569 | 0360,0361,0362,0363 |
| M+H | 721.3048 | 16.695 | 0360,0361,0362,0363 |
| M+H | 722.1923 | 17.233 | 0364 |
| M+H | 722.2112 | 14.633 | 0365 |
| M+H | 722.2631 | 15.443 | 0366,0367 |
| M+H | 722.2627 | 15.371 | 0366,0367 |
| M+H | 722.2902 | 14.358 | 0368 |
| M+H | 722.3000 | 13.524 | 0369 |
| M+H | 722.3900 | 14.747 | 0370 |
| M+H | 723.2097 | 13.742 | 0371 |
| M+H | 723.2605 | 15.347 | 0372 |
| M+H | 723.2853 | 15.260 | 0373,0374 |
| M-H | 721.2659 | 15.276 | 0373,0374 |
| M+H | 723.3009 | 17.688 | 0375 |
| M+H | 724.2233 | 14.834 | 0376 |
| M+H | 724.2829 | 13.863 | 0377,0378 |
| M+H | 724.2797 | 13.903 | 0377,0378 |
| M+H | 724.3132 | 14.323 | 0379,0380 |
| M+H | 724.3141 | 14.270 | 0379,0380 |
| M+H | 724.3702 | 13.802 | 0381 |
| M+H | 725.2208 | 14.278 | 0382 |
| M+H | 725.2352 | 16.106 | 0383 |
| M+H | 725.2420 | 15.453 | 0384,0385 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 725.2419 | 15.349 | 0384,0385 |
| M+H | 725.2646 | 14.003 | 0386 |
| M+H | 725.2802 | 16.128 | 0387,0388 |
| M-H | 723.2628 | 16.223 | 0387,0388 |
| M+H | 725.3143 | 18.853 | 0389 |
| M+H | 725.3686 | 17.330 | 0390 |
| M+H | 726.2389 | 14.366 | 0391,0392 |
| M+H | 726.2380 | 14.411 | 0391,0392 |
| M+H | 726.2588 | 13.165 | 0393,0394 |
| M-H | 724.2419 | 13.209 | 0393,0394 |
| M+H | 726.2758 | 14.652 | 0395 |
| M+H | 726.2877 | 16.453 | 0396 |
| M+H | 726.2950 | 13.565 | 0397,0398,0399,0400 |
| M+H | 726.2953 | 15.212 | 0397,0398,0399,0400 |
| M+H | 726.2948 | 13.834 | 0397,0398,0399,0400 |
| M+H | 726.2945 | 13.880 | 0396,0397,0398,0399,0400 |
| M+H | 727.2255 | 16.932 | 0401 |
| M+H | 727.2434 | 15.737 | 0402 |
| M+H | 727.2540 | 10.096 | 0403,0404 |
| M-H | 725.2375 | 13.420 | 0403,0404 |
| M+H | 727.2734 | 16.672 | 0405,0406 |
| M+H | 727.2761 | 17.909 | 0405,0406 |
| M+H | 727.2904 | 14.418 | 0407,0408,0409 |
| M+H | 727.2901 | 13.125 | 0407,0408,0409 |
| M+H | 727.2913 | 14.451 | 0407,0408,0409 |
| M+H | 728.2488 | 10.359 | 0410 |
| M+H | 728.2685 | 11.665 | 0411 |
| M+H | 728.2741 | 12.900 | 0412,0413 |
| M+H | 728.2732 | 12.954 | 0411,0412,0413 |
| M+H | 728.2853 | 12.188 | 0414 |
| M+H | 728.2935 | 13.526 | 0415 |
| M+H | 729.2557 | 15.829 | 0416,0417 |
| M+H | 729.2581 | 11.289 | 0416,0417 |
| M+H | 729.2700 | 9.768 | 0418,0419,0420,0421 |
| M+H | 729.2690 | 13.441 | 0418,0419 |
| M-H | 727.2549 | 16.170 | 0418,0419,0420,0421 |
| M-H | 727.2549 | 16.105 | 0418,0419,0420,0421 |

(continued)

| Type of ion | m/z [M+H]+ or [M-H]- | Retention time of MS peak (min) | Attributed compound No. Described omitting 2-1-m1E01-2- |
|---|---|---|---|
| M-H | 728.2362 | 13.935 | 0422,0423 |
| M-H | 728.2366 | 13.971 | 0422,0423 |
| M-H | 728.2524 | 13.644 | 0424,0425,0426,0427 |
| M+H | 730.2704 | 13.613 | 0424,0425,0426,0427 |
| M+H | 730.2700 | 13.708 | 0424,0425,0426,0427 |
| M+H | 730.2703 | 13.269 | 0424,0425,0426,0427 |
| M-H | 728.2659 | 14.445 | 0428 |
| M+H | 730.3214 | 15.185 | 0429 |
| M+H | 730.3416 | 14.510 | 0430 |
| M+H | 731.2004 | 16.421 | 0431 |
| M+H | 731.2647 | 16.133 | 0432,0433,0434,0435 |
| M+H | 731.2645 | 10.859 | 0432,0433,0434,0435 |
| M+H | 731.2671 | 13.380 | 0432,0433,0434,0435 |
| M+H | 731.2631 | 6.320 | 0432,0433,0434,0435 |
| M+H | 731.2786 | 14.803 | 0436 |
| M+H | 732.2147 | 13.681 | 0437 |
| M+H | 732.2504 | 15.396 | 0438 |
| M+H | 732.2591 | 13.978 | 0439 |
| M+H | 732.2985 | 14.968 | 0440 |
| M+H | 733.1993 | 11.693 | 0441 |
| M-H | 731.1942 | 16.055 | 0442,0443 |
| M+H | 733.2103 | 10.189 | 0442,0443 |
| M+H | 733.2305 | 17.026 | 0444 |
| M+H | 733.2472 | 13.325 | 0445 |
| M+H | 733.2678 | 14.196 | 0446 |
| M+H | 733.3007 | 9.881 | 0447 |
| M+H | 733.3402 | 18.362 | 0448,0449 |
| M+H | 733.3415 | 18.427 | 0448,0449 |
| M+H | 734.1941 | 14.441 | 0450 |
| M+H | 734.2293 | 13.610 | 0451,0452 |
| M-H | 732.2123 | 13.650 | 0451,0452 |
| M-H | 732.2290 | 13.330 | 0453,0454 |
| M+H | 734.2454 | 13.271 | 0453,0454 |
| M+H | 734.2632 | 16.112 | 0455 |
| M+H | 734.2748 | 13.716 | 0456 |
| M-H | 733.2118 | 17.524 | 0457,0458,0459,0460 |
| M-H | 733.2185 | 11.388 | 0458,0459,0460 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 735.2296 | 17.475 | 0457,0458,0459,0460 |
| M+H | 735.2313 | 17.142 | 0457,0458,0459,0460 |
| M+H | 735.2408 | 12.623 | 0461 |
| M+H | 735.2849 | 16.408 | 0462 |
| M+H | 735.2908 | 17.223 | 0463 |
| M+H | 735.3204 | 17.604 | 0464,0465 |
| M+H | 735.3204 | 17.678 | 0464,0465 |
| M+H | 736.2244 | 16.201 | 0466,0467,0468 |
| M+H | 736.2243 | 16.143 | 0466,0467,0468 |
| M+H | 736.2257 | 16.347 | 0466,0467,0468 |
| M+H | 736.2346 | 15.355 | 0469 |
| M+H | 736.2397 | 14.557 | 0469,0470 |
| M+H | 736.2576 | 14.797 | 0471 |
| M+H | 736.2782 | 13.662 | 0472,0473 |
| M+H | 736.2786 | 15.997 | 0472,0473 |
| M-H | 735.2092 | 16.392 | 0474 |
| M+H | 737.2553 | 11.128 | 0475 |
| M+H | 737.2644 | 15.365 | 0476 |
| M+H | 737.3003 | 15.795 | 0477,0478 |
| M+H | 737.3003 | 15.733 | 0477,0478 |
| M+H | 737.3170 | 17.997 | 0479 |
| M+H | 738.2605 | 15.261 | 0480 |
| M+H | 738.2694 | 15.550 | 0481 |
| M+H | 738.3312 | 10.684 | 0482,0483,0484 |
| M+H | 738.3316 | 15.542 | 0482,0483,0484 |
| M+H | 738.3316 | 15.493 | 0482,0483,0484 |
| M+H | 739.2541 | 18.354 | 0485 |
| M-H | 737.2414 | 16.003 | 0485,0486 |
| M+H | 739.2766 | 16.481 | 0487 |
| M+H | 739.2964 | 16.793 | 0488 |
| M-H | 737.2974 | 15.726 | 0489 |
| M+H | 739.3244 | 12.897 | 0490 |
| M+H | 740.2006 | 15.136 | 0491 |
| M+H | 740.2550 | 15.520 | 0492 |
| M-H | 738.2565 | 13.670 | 0493 |
| M+H | 740.3115 | 14.031 | 0494,0495,0496,0497 |
| M+H | 740.3114 | 14.119 | 0494,0495,0496,0497 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 740.3104 | 14.905 | 0494,0495,0496,0497 |
| M+H | 740.3121 | 13.952 | 0494,0495,0496,0497 |
| M+H | 741.1997 | 15.198 | 0498 |
| M+H | 741.2407 | 17.352 | 0499 |
| M+H | 741.2687 | 12.877 | 0500,0501 |
| M+H | 741.2691 | 13.810 | 0500,0501 |
| M+H | 741.2752 | 10.341 | 0502,0503 |
| M-H | 739.2593 | 13.762 | 0500,0501,0502,0503 |
| M+H | 741.2914 | 17.909 | 0504 |
| M+H | 741.3062 | 8.210 | 0505,0506 |
| M+H | 739.2960 | 14.325 | 0506 |
| M+H | 742.2646 | 9.904 | 0507 |
| M+H | 742.2827 | 16.586 | 0508 |
| M+H | 742.2901 | 13.341 | 0509,0510 |
| M+H | 742.2897 | 13.387 | 0509,0510 |
| M+H | 742.3076 | 15.245 | 0511 |
| M-H | 741.2016 | 16.465 | 0512,0513 |
| M-H | 741.2018 | 16.270 | 0512,0513 |
| M+H | 743.2332 | 15.480 | 0514,0515 |
| M+H | 743.2333 | 15.726 | 0514,0515 |
| M+H | 743.2734 | 13.744 | 0516,0517 |
| M+H | 743.2791 | 11.274 | 0517 |
| M-H | 741.2699 | 16.506 | 0518,0519,0520 |
| M+H | 743.2833 | 2.364 | 0518,0519 |
| M-H | 741.2756 | 13.351 | 0520 |
| M+H | 743.3398 | 16.915 | 0521 |
| M+H | 744.2153 | 14.886 | 0522 |
| M-H | 742.2119 | 15.598 | 0523 |
| M+H | 744.2487 | 13.119 | 0524,0525 |
| M+H | 744.2485 | 13.314 | 0524,0525 |
| M-H | 742.2514 | 14.502 | 0526 |
| M+H | 744.2858 | 14.177 | 0527,0528,0529 |
| M+H | 744.2864 | 14.365 | 0527,0528,0529 |
| M+H | 744.2839 | 12.853 | 0527,0528,0529 |
| M+H | 745.2106 | 14.680 | 0530 |
| M+H | 745.2439 | 10.327 | 0531,0532 |
| M+H | 745.2437 | 13.486 | 0531,0532 |

(continued)

| Type of ion | m/z [M+H]+ or [M-H]- | Retention time of MS peak (min) | Attributed compound No. Described omitting 2-1-m1E01-2- |
|---|---|---|---|
| M+H | 745.2651 | 12.033 | 0533 |
| M+H | 745.2791 | 11.648 | 0534 |
| M+H | 745.2951 | 14.045 | 0535 |
| M+H | 746.2319 | 14.179 | 0536 |
| M+H | 746.2509 | 15.034 | 0537 |
| M+H | 746.2808 | 14.467 | 0538,0539 |
| M+H | 746.2808 | 14.509 | 0538,0539 |
| M-H | 745.1961 | 16.589 | 0540 |
| M+H | 747.2445 | 17.065 | 0541,0542,0543,0544,0545,0546 |
| M-H | 745.2282 | 16.785 | 0541,0542,0543,0544,0545,0546 |
| M-H | 745.2266 | 16.912 | 0541,0542,0543,0544,0545 |
| M+H | 747.2438 | 15.942 | 0541,0542,0543,0544,0545,0546 |
| M+H | 747.2444 | 17.158 | 0541,0542,0543,0544,0545,0546 |
| M+H | 747.2452 | 15.897 | 0541,0542,0543,0544,0545,0546 |
| M+H | 747.2633 | 14.097 | 0547 |
| M+H | 747.2749 | 14.908 | 0548 |
| M+H | 747.2811 | 11.480 | 0549 |
| M+H | 747.3246 | 14.547 | 0550 |
| M+H | 747.3560 | 16.702 | 0551 |
| M+H | 748.2406 | 15.266 | 0552,0553 |
| M+H | 748.2398 | 15.301 | 0552,0553 |
| M+H | 748.2598 | 13.870 | 0554,0555 |
| M+H | 748.2602 | 13.814 | 0554,0555 |
| M-H | 746.2574 | 12.369 | 0556 |
| M+H | 748.3175 | 16.355 | 0557 |
| M+H | 749.1755 | 12.361 | 0558 |
| M+H | 749.1883 | 16.526 | 0559 |
| M-H | 747.2182 | 17.744 | 0560 |
| M+H | 749.2460 | 17.843 | 0561,0562 |
| M+H | 749.2443 | 15.249 | 0561,0562 |
| M+H | 749.2548 | 16.030 | 0563,0564 |
| M+H | 749.2548 | 15.977 | 0563,0564 |
| M+H | 749.3365 | 17.956 | 0565 |
| M+H | 750.1691 | 16.944 | 0566 |
| M+H | 750.2044 | 13.814 | 0567 |
| M+H | 750.2422 | 14.751 | 0568 |
| M+H | 750.2674 | 12.696 | 0569 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 750.2961 | 16.475 | 0570 |
| M+H | 751.1926 | 11.832 | 0571 |
| M+H | 751.2359 | 15.481 | 0572 |
| M+H | 751.2590 | 17.404 | 0573 |
| M+H | 751.2650 | 9.544 | 0574 |
| M+H | 751.2700 | 17.535 | 0574,0575 |
| M+H | 751.2838 | 17.330 | 0576 |
| M+H | 751.3167 | 16.568 | 0577 |
| M+H | 751.3265 | 14.552 | 0578 |
| M-H | 750.1895 | 14.195 | 0579 |
| M+H | 752.2208 | 12.527 | 0580 |
| M-H | 750.2169 | 14.616 | 0581 |
| M+H | 752.2730 | 15.399 | 0582,0583 |
| M-H | 750.2560 | 15.344 | 0582,0583 |
| M+H | 752.3440 | 14.742 | 0584,0585 |
| M+H | 752.3467 | 11.172 | 0584,0585 |
| M-H | 751.1820 | 17.214 | 0586,0587 |
| M+H | 753.1993 | 10.920 | 0586,0587 |
| M+H | 753.2206 | 17.478 | 0588,0589,0590 |
| M+H | 753.2206 | 17.415 | 0588,0589,0590 |
| M+H | 753.2199 | 17.057 | 0588,0589,0590 |
| M-H | 751.2515 | 12.710 | 0591 |
| M-H | 751.2567 | 16.381 | 0591,0592 |
| M+H | 753.2945 | 13.316 | 0593 |
| M+H | 753.3309 | 14.545 | 0594 |
| M+H | 754.1977 | 17.577 | 0595 |
| M+H | 754.2349 | 15.132 | 0596,0597 |
| M+H | 754.2351 | 14.969 | 0596,0597 |
| M+H | 754.2913 | 14.171 | 0598,0599 |
| M+H | 754.2957 | 14.672 | 0599 |
| M+H | 754.3287 | 13.455 | 0600,0601 |
| M+H | 754.3266 | 15.220 | 0600,0601 |
| M+H | 755.2314 | 12.654 | 0602,0603,0604,0605 |
| M+H | 755.2309 | 13.598 | 0602,0603,0604,0605 |
| M+H | 755.2314 | 12.127 | 0602,0603,0604,0605 |
| M+H | 755.2322 | 14.896 | 0602,0603,0604,0605 |
| M+H | 755.2522 | 15.450 | 0606,0607 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 755.2523 | 15.388 | 0606,0607 |
| M+H | 755.2684 | 13.027 | 0608 |
| M+H | 755.2851 | 13.343 | 0609,0610 |
| M+H | 755.2850 | 12.008 | 0609,0610 |
| M+H | 755.3048 | 17.306 | 0611 |
| M+H | 755.3248 | 18.391 | 0612 |
| M+H | 756.2259 | 14.466 | 0613 |
| M+H | 756.2300 | 12.207 | 0613,0614 |
| M-H | 754.2531 | 13.000 | 0615 |
| M+H | 756.3056 | 13.904 | 0616,0617 |
| M+H | 756.3059 | 13.963 | 0616,0617 |
| M+H | 756.3234 | 15.556 | 0618 |
| M+H | 757.2211 | 15.505 | 0619 |
| M+H | 757.2339 | 12.130 | 0620 |
| M+H | 757.2615 | 10.939 | 0621 |
| M+H | 757.2698 | 12.987 | 0621,0622 |
| M+H | 757.3087 | 13.847 | 0623,0624 |
| M+H | 757.3043 | 15.717 | 0623,0624 |
| M-H | 756.2261 | 15.709 | 0625 |
| M+H | 758.2645 | 12.944 | 0626,0627,0628 |
| M+H | 758.2652 | 10.394 | 0626,0627,0628 |
| M+H | 758.2640 | 13.566 | 0626,0627,0628 |
| M+H | 758.2837 | 14.505 | 0629 |
| M+H | 758.3015 | 13.195 | 0630 |
| M+H | 758.3207 | 13.326 | 0631 |
| M+H | 759.2565 | 15.596 | 0632,0633 |
| M+H | 759.2595 | 12.967 | 0632,0633 |
| M-H | 757.2495 | 13.649 | 0633,0634 |
| M+H | 759.2817 | 18.104 | 0635 |
| M+H | 759.2959 | 17.264 | 0636 |
| M+H | 759.3163 | 10.478 | 0637 |
| M+H | 759.3368 | 17.068 | 0638 |
| M-H | 758.2283 | 14.724 | 0639 |
| M-H | 758.2686 | 15.660 | 0640,0641 |
| M+H | 760.2794 | 13.733 | 0640,0641 |
| M+H | 760.2946 | 11.583 | 0642 |
| M-H | 759.1688 | 15.118 | 0643 |

(continued)

| Type of ion | m/z [M+H]+ or [M-H]- | Retention time of MS peak (min) | Attributed compound No. Described omitting 2-1-m1E01-2- |
|---|---|---|---|
| M+H | 761.2116 | 16.536 | 0644 |
| M+H | 761.2231 | 15.691 | 0645 |
| M+H | 761.2346 | 13.503 | 0646 |
| M+H | 761.2627 | 16.265 | 0647,0648 |
| M+H | 761.2596 | 17.152 | 0647,0648 |
| M+H | 761.2775 | 15.145 | 0649,0650 |
| M+H | 761.2779 | 15.097 | 0649,0650 |
| M+H | 761.2911 | 14.124 | 0651 |
| M+H | 761.2991 | 14.574 | 0652 |
| M+H | 761.3366 | 17.910 | 0653 |
| M+H | 762.1838 | 15.260 | 0654 |
| M-H | 760.2085 | 14.222 | 0655,0656 |
| M-H | 760.2089 | 14.282 | 0655,0656 |
| M+H | 762.2387 | 13.196 | 0657,0658 |
| M-H | 760.2214 | 13.868 | 0657,0658 |
| M+H | 762.2565 | 11.893 | 0659 |
| M+H | 762.2741 | 14.576 | 0660 |
| M+H | 762.3461 | 14.800 | 0661 |
| M+H | 763.1910 | 12.939 | 0662 |
| M+H | 763.2206 | 11.101 | 0664,0665 |
| M-H | 761.2060 | 17.305 | 0664,0665 |
| M-H | 761.2189 | 17.017 | 0666 |
| M+H | 763.2412 | 17.010 | 0667,0668 |
| M+H | 763.2412 | 17.222 | 0667,0668 |
| M+H | 763.2614 | 18.133 | 0669,0670 |
| M+H | 763.2614 | 18.206 | 0669,0670 |
| M+H | 763.2764 | 14.295 | 0671 |
| M+H | 764.2351 | 15.924 | 0672 |
| M+H | 764.2547 | 13.508 | 0673 |
| M+H | 764.2689 | 12.750 | 0674 |
| M+H | 764.2852 | 13.411 | 0675 |
| M-H | 763.2016 | 16.540 | 0676 |
| M+H | 765.2309 | 17.771 | 0677 |
| M-H | 763.2175 | 15.955 | 0677,0678 |
| M+H | 765.2406 | 17.415 | 0678,0679,0680 |
| M+H | 765.2406 | 17.374 | 0678,0679,0680 |
| M+H | 765.3115 | 17.540 | 0681 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M-H | 764.2027 | 14.588 | 0682 |
| M+H | 766.2512 | 14.485 | 0683,0684,0685,0686 |
| M+H | 766.2521 | 13.933 | 0683,0684,0685,0686 |
| M+H | 766.2509 | 15.065 | 0683,0684,0685,0686 |
| M+H | 766.2521 | 13.975 | 0683,0684,0685,0686 |
| M+H | 766.2901 | 15.717 | 0687 |
| M+H | 766.3270 | 15.191 | 0688 |
| M+H | 766.3651 | 16.296 | 0689 |
| M+H | 767.1699 | 10.958 | 0690 |
| M+H | 767.2462 | 11.813 | 0691,0692,0693 |
| M+H | 767.2470 | 14.580 | 0691,0692,0693 |
| M+H | 767.2470 | 14.525 | 0691,0692,0693 |
| M+H | 767.2653 | 17.611 | 0694,0695 |
| M+H | 767.2654 | 17.658 | 0694,0695 |
| M+H | 767.3212 | 12.662 | 0696 |
| M+H | 767.3482 | 16.695 | 0697 |
| M+H | 768.1952 | 14.453 | 0699 |
| M+H | 768.2297 | 16.870 | 0700 |
| M+H | 768.2403 | 15.687 | 0701 |
| M+H | 768.2506 | 15.456 | 0702,0703 |
| M+H | 768.2507 | 15.502 | 0702,0703 |
| M+H | 768.2604 | 13.058 | 0704 |
| M+H | 768.3438 | 14.048 | 0705 |
| M+H | 769.1768 | 11.480 | 0706,0707 |
| M-H | 767.1614 | 16.345 | 0706,0707 |
| M-H | 767.1782 | 17.332 | 0708 |
| M-H | 767.2282 | 13.836 | 0709,0710 |
| M-H | 767.2281 | 14.912 | 0709,0710 |
| M+H | 769.2504 | 17.583 | 0709,0710,0711 |
| M+H | 769.3008 | 13.788 | 0712 |
| M-H | 767.3089 | 15.709 | 0713 |
| M+H | 769.3387 | 12.569 | 0714 |
| M-H | 768.1769 | 14.243 | 0715,0716 |
| M-H | 768.1773 | 14.326 | 0715,0716 |
| M-H | 768.2254 | 12.101 | 0717,0718 |
| M+H | 770.2417 | 14.216 | 0717,0718 |
| M+H | 770.2661 | 15.396 | 0719 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 770.2783 | 15.736 | 0720 |
| M+H | 770.2895 | 14.730 | 0721 |
| M+H | 770.3214 | 14.299 | 0722 |
| M+H | 771.2083 | 13.960 | 0723,0724 |
| M+H | 771.2094 | 17.284 | 0723,0724 |
| M+H | 771.2552 | 12.939 | 0725 |
| M+H | 771.2798 | 12.958 | 0726 |
| M+H | 772.2091 | 15.375 | 0727 |
| M+H | 772.2256 | 15.149 | 0728,0729,0730 |
| M-H | 770.2087 | 13.731 | 0728,0729,0730 |
| M-H | 770.2087 | 13.705 | 0728,0729,0730 |
| M+H | 772.2819 | 14.057 | 0731,0732 |
| M+H | 772.2819 | 13.999 | 0731,0732 |
| M-H | 770.2819 | 8.168 | 0733 |
| M+H | 772.3361 | 13.756 | 0734 |
| M+H | 773.2043 | 14.199 | 0735,0736 |
| M+H | 773.2025 | 15.481 | 0735,0736 |
| M-H | 771.2174 | 11.904 | 0737 |
| M+H | 773.2972 | 18.076 | 0738,0739 |
| M+H | 773.2972 | 18.141 | 0738,0739 |
| M+H | 774.2564 | 16.415 | 0740,0741 |
| M+H | 774.2583 | 13.204 | 0740,0741 |
| M+H | 774.2785 | 14.498 | 0742 |
| M+H | 774.3100 | 15.191 | 0743 |
| M+H | 775.2399 | 16.119 | 0744 |
| M-H | 773.2478 | 14.325 | 0745 |
| M+H | 775.2767 | 17.470 | 0746,0747,0748 |
| M+H | 775.2767 | 17.409 | 0746,0747,0748 |
| M+H | 775.2764 | 18.202 | 0746,0747,0748 |
| M+H | 775.2934 | 15.586 | 0749 |
| M+H | 775.3510 | 18.265 | 0750 |
| M-H | 774.2175 | 15.972 | 0751 |
| M-H | 774.2239 | 14.689 | 0751,0752 |
| M+H | 776.3112 | 13.414 | 0753 |
| M-H | 775.1889 | 18.527 | 0754 |
| M+H | 777.2501 | 13.610 | 0755 |
| M+H | 777.2556 | 15.467 | 0755,0756,0757 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 777.2538 | 16.578 | 0755,0756,0757 |
| M+H | 777.3303 | 17.492 | 0758 |
| M+H | 778.2500 | 12.212 | 0759 |
| M+H | 778.2739 | 17.612 | 0760,0761 |
| M+H | 778.2703 | 13.103 | 0760,0761 |
| M+H | 778.2873 | 18.127 | 0762 |
| M+H | 778.3006 | 11.472 | 0763 |
| M+H | 778.3417 | 14.890 | 0764 |
| M+H | 779.2013 | 16.736 | 0766 |
| M-H | 777.2326 | 13.021 | 0767 |
| M-H | 778.1974 | 14.373 | 0768 |
| M+H | 780.2309 | 13.765 | 0769 |
| M-H | 778.2180 | 15.077 | 0769,0770 |
| M+H | 780.2514 | 14.040 | 0771 |
| M+H | 780.2659 | 14.745 | 0772,0773 |
| M+H | 780.2659 | 14.799 | 0772,0773 |
| M+H | 780.2802 | 12.967 | 0774 |
| M+H | 780.3071 | 16.064 | 0775 |
| M+H | 780.3408 | 15.546 | 0776 |
| M+H | 781.1981 | 17.137 | 0777 |
| M-H | 779.1955 | 16.691 | 0778 |
| M+H | 781.2656 | 18.580 | 0779 |
| M+H | 781.2822 | 15.961 | 0780,0781 |
| M+H | 781.2810 | 11.326 | 0780,0781 |
| M-H | 780.1791 | 16.531 | 0782 |
| M-H | 780.2036 | 10.063 | 0783 |
| M+H | 782.2463 | 15.274 | 0784,0785 |
| M+H | 782.2473 | 13.985 | 0784,0785 |
| M+H | 782.2663 | 15.732 | 0786,0787 |
| M+H | 782.2659 | 15.795 | 0786,0787 |
| M+H | 782.2860 | 15.362 | 0788 |
| M-H | 781.2031 | 16.851 | 0789 |
| M-H | 781.2102 | 13.738 | 0790,0791 |
| M-H | 781.2102 | 13.768 | 0790,0791 |
| M+H | 783.2406 | 14.626 | 0792 |
| M+H | 783.2600 | 15.092 | 0793,0794 |
| M+H | 783.2614 | 14.013 | 0793,0794 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 783.2843 | 18.073 | 0795 |
| M+H | 783.3019 | 17.731 | 0796 |
| M-H | 782.2073 | 14.579 | 0797,0798 |
| M-H | 782.2073 | 14.627 | 0797,0798 |
| M+H | 784.2409 | 14.182 | 0799,0800 |
| M+H | 784.2430 | 14.450 | 0800,0801,0802 |
| M-H | 782.2337 | 14.030 | 0801,0802 |
| M+H | 784.2451 | 14.267 | 0800,0801,0802 |
| M+H | 784.3165 | 15.245 | 0803 |
| M+H | 785.2443 | 18.194 | 0805,0806 |
| M-H | 783.2271 | 17.875 | 0805,0806 |
| M-H | 784.1536 | 16.231 | 0807 |
| M+H | 786.2233 | 17.042 | 0808 |
| M+H | 786.2510 | 13.861 | 0809 |
| M+H | 786.2724 | 15.793 | 0810 |
| M+H | 786.3520 | 14.243 | 0811 |
| M+H | 787.2365 | 14.932 | 0812,0813 |
| M+H | 787.2370 | 14.886 | 0812,0813 |
| M+H | 787.3109 | 18.399 | 0814 |
| M-H | 786.1678 | 14.909 | 0815 |
| M+H | 788.2024 | 15.486 | 0816 |
| M+H | 788.2553 | 15.882 | 0817 |
| M+H | 788.3305 | 13.358 | 0818 |
| M+H | 789.1837 | 14.553 | 0819 |
| M+H | 789.2016 | 15.550 | 0820 |
| M+H | 789.2920 | 18.131 | 0821 |
| M-H | 787.3133 | 16.707 | 0822 |
| M+H | 790.2173 | 15.628 | 0823,0824 |
| M+H | 790.2173 | 15.671 | 0823,0824 |
| M+H | 790.2496 | 16.231 | 0825 |
| M-H | 788.2395 | 15.083 | 0825,0826 |
| M+H | 791.2380 | 16.305 | 0827 |
| M+H | 791.2730 | 17.048 | 0828,0829 |
| M+H | 791.2728 | 18.064 | 0828,0829 |
| M+H | 792.2286 | 16.119 | 0830 |
| M-H | 790.2187 | 14.277 | 0830,0831 |
| M+H | 792.3008 | 15.006 | 0832 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 793.2303 | 14.468 | 0833 |
| M-H | 791.2199 | 9.963 | 0833,0834 |
| M-H | 792.2284 | 14.251 | 0835 |
| M+H | 794.2824 | 13.752 | 0836,0837,0838 |
| M+H | 794.2821 | 15.198 | 0836,0837,0838 |
| M+H | 794.2827 | 15.285 | 0836,0837,0838 |
| M+H | 794.3037 | 14.019 | 0839 |
| M+H | 794.3568 | 15.981 | 0840 |
| M-H | 793.2241 | 14.376 | 0841 |
| M+H | 795.2468 | 14.253 | 0841,0842 |
| M+H | 795.2960 | 11.823 | 0843 |
| M+H | 796.2145 | 17.260 | 0844 |
| M+H | 796.2617 | 14.213 | 0845,0846 |
| M+H | 796.2617 | 14.263 | 0845,0846 |
| M+H | 796.3350 | 15.180 | 0847 |
| M+H | 797.1931 | 17.272 | 0848 |
| M+H | 797.2291 | 16.795 | 0849 |
| M-H | 795.2412 | 17.509 | 0850,0851 |
| M+H | 797.2610 | 17.424 | 0850,0851 |
| M+H | 797.2773 | 15.362 | 0852 |
| M+H | 797.3355 | 18.245 | 0853 |
| M-H | 796.1755 | 17.162 | 0854 |
| M-H | 796.1881 | 14.866 | 0855 |
| M+H | 798.2257 | 15.286 | 0856 |
| M+H | 798.2371 | 13.648 | 0857 |
| M+H | 798.2577 | 14.357 | 0858 |
| M+H | 799.1943 | 17.989 | 0859,0860 |
| M-H | 797.1779 | 15.980 | 0859,0860 |
| M+H | 799.2523 | 16.847 | 0861 |
| M+H | 799.2571 | 14.399 | 0862,0863 |
| M+H | 799.2567 | 14.848 | 0861,0862,0863 |
| M-H | 798.1692 | 16.682 | 0864 |
| M-H | 798.1855 | 14.926 | 0865 |
| M-H | 798.2013 | 14.704 | 0866 |
| M+H | 800.2564 | 17.674 | 0867 |
| M-H | 799.1727 | 17.791 | 0868 |
| M+H | 801.2160 | 13.854 | 0869 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M+H | 801.2539 | 15.248 | 0870 |
| M+H | 802.2328 | 13.526 | 0871 |
| M-H | 800.2903 | 14.629 | 0872 |
| M+H | 803.2180 | 17.842 | 0873,0874 |
| M+H | 803.2180 | 17.908 | 0873,0874 |
| M+H | 803.3068 | 18.391 | 0875 |
| M+H | 804.1641 | 14.591 | 0876 |
| M-H | 803.2088 | 15.449 | 0877 |
| M+H | 806.2439 | 16.263 | 0878 |
| M+H | 806.3193 | 16.106 | 0879 |
| M+H | 807.2373 | 13.949 | 0880 |
| M+H | 807.2680 | 16.532 | 0881 |
| M+H | 807.3032 | 16.388 | 0882 |
| M+H | 808.2968 | 16.042 | 0883 |
| M+H | 809.1720 | 14.741 | 0884 |
| M+H | 809.2256 | 13.202 | 0885 |
| M+H | 809.2408 | 15.897 | 0886 |
| M+H | 809.2566 | 13.999 | 0887 |
| M+H | 810.2416 | 14.334 | 0888 |
| M+H | 810.2765 | 14.918 | 0889,0890 |
| M+H | 810.2757 | 13.791 | 0889,0890 |
| M+H | 811.2028 | 15.672 | 0891,0892,0893 |
| M-H | 809.1879 | 17.069 | 0891,0892,0893 |
| M+H | 811.2355 | 14.430 | 0894 |
| M+H | 811.2441 | 14.264 | 0895 |
| M+H | 812.2381 | 14.174 | 0896 |
| M-H | 812.1905 | 18.086 | 0898 |
| M-H | 812.2369 | 15.522 | 0899 |
| M-H | 813.1842 | 11.388 | 0900 |
| M+H | 815.2220 | 16.789 | 0901 |
| M+H | 815.2320 | 14.825 | 0902 |
| M+H | 815.2444 | 17.030 | 0903 |
| M+H | 815.2898 | 14.033 | 0904 |
| M+H | 815.3085 | 17.910 | 0905 |
| M-H | 814.1800 | 14.884 | 0906 |
| M+H | 816.2344 | 14.574 | 0907 |
| M-H | 815.1449 | 18.556 | 0908 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M-H | 815.1631 | 17.928 | 0909,0910 |
| M+H | 817.1818 | 12.938 | 0909,0910 |
| M+H | 817.2316 | 11.741 | 0911 |
| M+H | 817.2514 | 17.826 | 0912 |
| M+H | 818.1967 | 15.553 | 0913 |
| M-H | 816.2095 | 17.287 | 0914 |
| M+H | 818.2566 | 14.217 | 0915 |
| M+H | 819.1926 | 12.219 | 0916,0917 |
| M+H | 819.1932 | 14.866 | 0916,0917 |
| M+H | 819.2128 | 17.907 | 0918,0919 |
| M-H | 817.1941 | 17.633 | 0918,0919 |
| M+H | 820.1922 | 15.292 | 0920 |
| M+H | 820.2600 | 16.113 | 0921 |
| M+H | 822.2237 | 15.853 | 0922,0923 |
| M+H | 822.2237 | 15.902 | 0922,0923 |
| M+H | 822.3129 | 12.100 | 0924 |
| M+H | 823.2185 | 15.045 | 0925 |
| M+H | 823.2935 | 15.902 | 0926,0927 |
| M-H | 821.2800 | 16.468 | 0926,0927 |
| M-H | 823.1303 | 16.851 | 0928 |
| M+H | 825.2520 | 14.072 | 0929 |
| M+H | 825.2600 | 12.075 | 0930 |
| M+H | 826.2134 | 15.425 | 0931 |
| M+H | 826.2767 | 10.428 | 0932 |
| M+H | 826.3091 | 14.327 | 0933 |
| M+H | 827.2024 | 17.103 | 0934 |
| M-H | 825.2058 | 12.497 | 0935 |
| M+H | 827.2441 | 10.795 | 0936 |
| M-H | 826.2068 | 18.212 | 0937 |
| M+H | 828.2300 | 14.242 | 0938 |
| M+H | 829.2041 | 17.907 | 0939 |
| M-H | 828.1945 | 15.205 | 0940 |
| M+H | 830.2517 | 14.976 | 0941 |
| M-H | 829.2101 | 17.264 | 0942 |
| M+H | 831.3038 | 17.956 | 0943 |
| M-H | 830.1793 | 14.422 | 0944 |
| M-H | 830.1943 | 15.956 | 0945 |

(continued)

| Type of ion | m/z $[M+H]^+$ or $[M-H]^-$ | Retention time of MS peak (min) | Attributed compound No. Described omitting 2-1-m1E01-2- |
|---|---|---|---|
| M-H | 831.1913 | 16.968 | 0947 |
| M+H | 833.2083 | 9.790 | 0947,0948 |
| M+H | 834.2039 | 12.195 | 0949 |
| M+H | 834.2274 | 14.605 | 0950,0951 |
| M-H | 832.2097 | 14.668 | 0950,0951 |
| M-H | 832.2251 | 12.090 | 0952 |
| M+H | 834.2519 | 14.275 | 0953 |
| M+H | 834.3144 | 15.984 | 0954 |
| M-H | 833.1565 | 12.228 | 0955 |
| M-H | 834.1530 | 14.879 | 0956 |
| M-H | 834.1680 | 15.316 | 0957 |
| M+H | 836.1885 | 15.733 | 0957,0958 |
| M+H | 836.2561 | 16.138 | 0959 |
| M+H | 837.2324 | 15.633 | 0960 |
| M+H | 838.2156 | 15.817 | 0961,0962 |
| M+H | 838.2158 | 12.870 | 0961,0962 |
| M-H | 840.2155 | 13.268 | 0963 |
| M+H | 842.3032 | 14.393 | 0964 |
| M-H | 842.1947 | 14.445 | 0965 |
| M-H | 842.2489 | 15.381 | 0966 |
| M-H | 844.1926 | 13.276 | 0967 |
| M+H | 846.2262 | 16.539 | 0968 |
| M+H | 846.2488 | 14.604 | 0969 |
| M+H | 847.2240 | 10.015 | 0970 |
| M-H | 845.2291 | 15.305 | 0971 |
| M-H | 846.1922 | 18.550 | 0972 |
| M-H | 847.1959 | 14.830 | 0973 |
| M-H | 848.1668 | 14.979 | 0974 |
| M+H | 850.2319 | 15.302 | 0975 |
| M+H | 850.3056 | 16.045 | 0976 |
| M+H | 852.1639 | 17.447 | 0978 |
| M+H | 852.2180 | 15.176 | 0979 |
| M+H | 853.2274 | 15.737 | 0980 |
| M+H | 854.1781 | 16.302 | 0981 |
| M-H | 859.2250 | 10.481 | 0982 |
| M+H | 863.2161 | 15.436 | 0983 |
| M-H | 862.2001 | 15.938 | 0984 |

(continued)

| Type of ion | m/z [M+H]$^+$ or [M-H]$^-$ | Retention time of MS peak (min) | Attributed compound No. Described omitting 2-1-m1E01-2- |
|---|---|---|---|
| M+H | 865.2189 | 17.273 | 0985 |
| M-H | 865.1611 | 19.710 | 0986 |
| M+H | 869.1892 | 18.089 | 0987 |
| M+H | 873.2025 | 15.158 | 0988 |
| M-H | 873.1458 | 16.398 | 0989 |
| M+H | 881.2129 | 17.314 | 0990 |
| M-H | 880.1714 | 15.221 | 0991 |
| M-H | 882.2044 | 15.477 | 0993 |
| M-H | 884.1676 | 15.524 | 0994 |
| M+H | 890.1761 | 18.189 | 0995 |
| M-H | 898.1998 | 15.541 | 0997 |
| M+H | 901.1952 | 18.349 | 0998 |
| M-H | 900.1606 | 16.582 | 0999 |
| M-H | 915.1751 | 14.882 | 1000 |

Example 6-8: Production example 3 of compound library having diversity in functional groups using O-selective ethylation method for arenol

Example 6-8 shows an example of constructing a library by performing an ethylation reaction to a mixture containing 500 compounds. The "diversity of core blocks" and "diversity of linkers" included in the library performed in Example 6-8 are illustrated in Figure 3, and a mixture 2-2-d1E01-1 containing arenol separately prepared as a raw material is shown in [Table 6-8-2],

The notation method in the table will be described. In [Table 6-8-2], each compound that may be included in the mixture 2-2-d1E01-1 is shown by ID, and the combination of core blocks and linkers in the structure of the mixture 2-2-d1E01-1 is shown by symbol or chemical formula. The correspondence between symbols and specific structures is illustrated in Figure 3. In [Table 6-8-2], "M" is represented by a chemical formula.

[Formula 121]

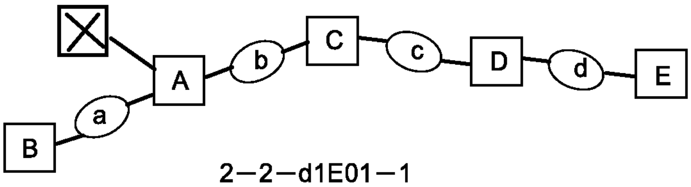


2—2—d1E01—1

For example, if the ID is 2-2-d1E01-1-0001, the following structural formula is shown in [Table 6-8-1].

[Formula 122]

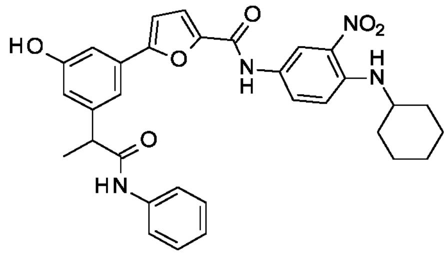

2-2-d1E01-1-0001

[Table 126]

[Table 6-8-1] Notation example of compound in ID notation

| ID | ☒ | A | a | B | b | C | c | D | d | E |
|---|---|---|---|---|---|---|---|---|---|---|
| 2-2-d1E01-1-0001 | OH | A01 | a1 | B01 | b1 | C01 | c1 | D01 | d1 | E01 |

[Table 127]

[Table 6-8-2] Compounds that may be included in mixture 2-2-d1E01-1

| ID | Exact Mass | ☒ | A | a | B | b | C | c | D | d | E |
|---|---|---|---|---|---|---|---|---|---|---|---|
| 2-2-d1E01-1-0001 | 568.232 | OH | A01 | a1 | B01 | b1 | C01 | c1 | D01 | d1 | E01 |
| 2-2-d1E01-1-0002 | 572.300 | OH | A01 | a1 | B01 | b4 | C12 | c1 | D01 | d1 | E01 |
| 2-2-d1E01-1-0003 | 574.279 | OH | A01 | a1 | B01 | b4 | C13 | c1 | D01 | d1 | E01 |
| 2-2-d1E01-1-0004 | 578.253 | OH | A01 | a1 | B01 | b1 | C02 | c1 | D01 | d1 | E01 |
| 2-2-d1E01-1-0005 | 578.253 | OH | A01 | a1 | B01 | b1 | C03 | c1 | D01 | d1 | E01 |
| 2-2-d1E01-1-0006 | 579.248 | OH | A01 | a1 | B01 | b1 | C04 | c1 | D01 | d1 | E01 |
| 2-2-d1E01-1-0007 | 582.248 | OH | A01 | a1 | B02 | b1 | C01 | c1 | D01 | d1 | E01 |
| 2-2-d1E01-1-0008 | 584.209 | OH | A01 | a1 | B01 | b1 | C05 | c1 | D01 | d1 | E01 |
| 2-2-d1E01-1-0009 | 585.222 | OH | A01 | a1 | B01 | b3 | C08 | c1 | D01 | d1 | E01 |
| 2-2-d1E01-1-0010 | 586.223 | OH | A01 | a1 | B03 | b1 | C01 | c1 | D01 | d1 | E01 |
| 2-2-d1E01-1-0011 | 586.316 | OH | A01 | a1 | B02 | b4 | C12 | c1 | D01 | d1 | E01 |
| 2-2-d1E01-1-0012 | 588.295 | OH | A01 | a1 | B02 | b4 | C13 | c1 | D01 | d1 | E01 |
| 2-2-d1E01-1-0013 | 590.290 | OH | A01 | a1 | B03 | b4 | C12 | c1 | D01 | d1 | E01 |
| 2-2-d1E01-1-0014 | 592.269 | OH | A01 | a1 | B02 | b1 | C02 | c1 | D01 | d1 | E01 |
| 2-2-d1E01-1-0015 | 592.269 | OH | A01 | a1 | B02 | b1 | C03 | c1 | D01 | d1 | E01 |
| 2-2-d1E01-1-0016 | 592.270 | OH | A01 | a1 | B03 | b4 | C13 | c1 | D01 | d1 | E01 |
| 2-2-d1E01-1-0017 | 593.264 | OH | A01 | a1 | B02 | b1 | C04 | c1 | D01 | d1 | E01 |
| 2-2-d1E01-1-0018 | 594.248 | OH | A02 | a1 | B01 | b1 | C01 | c1 | D01 | d1 | E01 |
| 2-2-d1E01-1-0019 | 596.238 | OH | A01 | a1 | B01 | b3 | C09 | c1 | D01 | d1 | E01 |

(continued)

| ID | Exact Mass | ☒ | A | a | B | b | C | c | D | d | E |
|---|---|---|---|---|---|---|---|---|---|---|---|
| 2-2-d1E01-1-0020 | 596.243 | OH | A01 | a1 | B03 | b1 | C02 | c1 | D01 | d1 | E01 |
| 2-2-d1E01-1-0021 | 596.243 | OH | A01 | a1 | B03 | b1 | C03 | c1 | D01 | d1 | E01 |
| 2-2-d1E01-1-0022 | 597.239 | OH | A01 | a1 | B03 | b1 | C04 | c1 | D01 | d1 | E01 |
| 2-2-d1E01-1-0023 | 598.225 | OH | A01 | a1 | B02 | b1 | C05 | c1 | D01 | d1 | E01 |
| 2-2-d1E01-1-0024 | 598.254 | OH | A01 | a1 | B01 | b1 | C14 | c1 | D01 | d1 | E01 |
| 2-2-d1E01-1-0025 | 598.316 | OH | A02 | a1 | B01 | b4 | C12 | c1 | D01 | d1 | E01 |
| 2-2-d1E01-1-0026 | 599.238 | OH | A01 | a1 | B02 | b3 | C08 | c1 | D01 | d1 | E01 |
| 2-2-d1E01-1-0027 | 600.295 | OH | A02 | a1 | B01 | b4 | C13 | c1 | D01 | d1 | E01 |
| 2-2-d1E01-1-0028 | 601.200 | OH | A01 | a1 | B01 | b3 | C10 | c1 | D01 | d1 | E01 |
| 2-2-d1E01-1-0029 | 602.200 | OH | A01 | a1 | B03 | b1 | C05 | c1 | D01 | d1 | E01 |
| 2-2-d1E01-1-0030 | 602.253 | OH | A01 | a1 | B01 | b2 | C02 | c1 | D01 | d1 | E01 |
| 2-2-d1E01-1-0031 | 602.253 | OH | A01 | a1 | B01 | b2 | C03 | c1 | D01 | d1 | E01 |
| 2-2-d1E01-1-0032 | 603.213 | OH | A01 | a1 | B03 | b3 | C08 | c1 | D01 | d1 | E01 |
| 2-2-d1E01-1-0033 | 603.248 | OH | A01 | a1 | B01 | b2 | C04 | c1 | D01 | d1 | E01 |
| 2-2-d1E01-1-0034 | 603.248 | OH | A01 | a1 | B01 | b2 | C06 | c1 | D01 | d1 | E01 |
| 2-2-d1E01-1-0035 | 603.248 | OH | A01 | a1 | B01 | b2 | C07 | c1 | D01 | d1 | E01 |
| 2-2-d1E01-1-0036 | 604.269 | OH | A02 | a1 | B01 | b1 | C02 | c1 | D01 | d1 | E01 |
| 2-2-d1E01-1-0037 | 604.269 | OH | A02 | a1 | B01 | b1 | C03 | c1 | D01 | d1 | E01 |
| 2-2-d1E01-1-0038 | 605.264 | OH | A02 | a1 | B01 | b1 | C04 | c1 | D01 | d1 | E01 |
| 2-2-d1E01-1-0039 | 607.279 | OH | A01 | a1 | B01 | b1 | C15 | c1 | D01 | d1 | E01 |
| 2-2-d1E01-1-0040 | 608.263 | OH | A02 | a1 | B02 | b1 | C01 | c1 | D01 | d1 | E01 |
| 2-2-d1E01-1-0041 | 608.275 | OH | A01 | a1 | B01 | b1 | C16 | c1 | D01 | d1 | E01 |
| 2-2-d1E01-1-0042 | 609.270 | OH | A01 | a1 | B01 | b1 | C17 | c1 | D01 | d1 | E01 |
| 2-2-d1E01-1-0043 | 610.225 | OH | A02 | a1 | B01 | b1 | C05 | c1 | D01 | d1 | E01 |
| 2-2-d1E01-1-0044 | 610.254 | OH | A01 | a1 | B02 | b3 | C09 | c1 | D01 | d1 | E01 |
| 2-2-d1E01-1-0045 | 610.279 | OH | A01 | a1 | B01 | b4 | C01 | c1 | D01 | d1 | E01 |
| 2-2-d1E01-1-0046 | 611.238 | OH | A02 | a1 | B01 | b3 | C08 | c1 | D01 | d1 | E01 |
| 2-2-d1E01-1-0047 | 612.238 | OH | A02 | a1 | B03 | b1 | C01 | c1 | D01 | d1 | E01 |
| 2-2-d1E01-1-0048 | 612.270 | OH | A01 | a1 | B02 | b1 | C14 | c1 | D01 | d1 | E01 |
| 2-2-d1E01-1-0049 | 612.331 | OH | A02 | a1 | B02 | b4 | C12 | c1 | D01 | d1 | E01 |
| 2-2-d1E01-1-0050 | 614.229 | OH | A01 | a1 | B03 | b3 | C09 | c1 | D01 | d1 | E01 |
| 2-2-d1E01-1-0051 | 614.231 | OH | A01 | a1 | B01 | b1 | C18 | c1 | D01 | d1 | E01 |
| 2-2-d1E01-1-0052 | 614.310 | OH | A02 | a1 | B02 | b4 | C13 | c1 | D01 | d1 | E01 |
| 2-2-d1E01-1-0053 | 615.215 | OH | A01 | a1 | B02 | b3 | C10 | c1 | D01 | d1 | E01 |
| 2-2-d1E01-1-0054 | 616.245 | OH | A01 | a1 | B03 | b1 | C14 | c1 | D01 | d1 | E01 |
| 2-2-d1E01-1-0055 | 616.269 | OH | A01 | a1 | B02 | b2 | C02 | c1 | D01 | d1 | E01 |
| 2-2-d1E01-1-0056 | 616.269 | OH | A01 | a1 | B02 | b2 | C03 | c1 | D01 | d1 | E01 |

(continued)

| ID | Exact Mass | ⊠ | A | a | B | b | C | c | D | d | E |
|---|---|---|---|---|---|---|---|---|---|---|---|
| 2-2-d1E01-1-0057 | 616.306 | OH | A02 | a1 | B03 | b4 | C12 | c1 | D01 | d1 | E01 |
| 2-2-d1E01-1-0058 | 617.264 | OH | A01 | a1 | B02 | b2 | C04 | c1 | D01 | d1 | E01 |
| 2-2-d1E01-1-0059 | 617.264 | OH | A01 | a1 | B02 | b2 | C06 | c1 | D01 | d1 | E01 |
| 2-2-d1E01-1-0060 | 617.264 | OH | A01 | a1 | B02 | b2 | C07 | c1 | D01 | d1 | E01 |
| 2-2-d1E01-1-0061 | 618.284 | OH | A02 | a1 | B02 | b1 | C02 | c1 | D01 | d1 | E01 |
| 2-2-d1E01-1-0062 | 618.284 | OH | A02 | a1 | B02 | b1 | C03 | c1 | D01 | d1 | E01 |
| 2-2-d1E01-1-0063 | 618.285 | OH | A02 | a1 | B03 | b4 | C13 | c1 | D01 | d1 | E01 |
| 2-2-d1E01-1-0064 | 619.190 | OH | A01 | a1 | B03 | b3 | C10 | c1 | D01 | d1 | E01 |
| 2-2-d1E01-1-0065 | 619.279 | OH | A02 | a1 | B02 | b1 | C04 | c1 | D01 | d1 | E01 |
| 2-2-d1E01-1-0066 | 620.243 | OH | A01 | a1 | B03 | b2 | C02 | c1 | D01 | d1 | E01 |
| 2-2-d1E01-1-0067 | 620.243 | OH | A01 | a1 | B03 | b2 | C03 | c1 | D01 | d1 | E01 |
| 2-2-d1E01-1-0068 | 620.300 | OH | A01 | a1 | B01 | b4 | C02 | c1 | D01 | d1 | E01 |
| 2-2-d1E01-1-0069 | 621.239 | OH | A01 | a1 | B03 | b2 | C04 | c1 | D01 | d1 | E01 |
| 2-2-d1E01-1-0070 | 621.239 | OH | A01 | a1 | B03 | b2 | C06 | c1 | D01 | d1 | E01 |
| 2-2-d1E01-1-0071 | 621.239 | OH | A01 | a1 | B03 | b2 | C07 | c1 | D01 | d1 | E01 |
| 2-2-d1E01-1-0072 | 621.295 | OH | A01 | a1 | B02 | b1 | C15 | c1 | D01 | d1 | E01 |
| 2-2-d1E01-1-0073 | 622.254 | OH | A02 | a1 | B01 | b3 | C09 | c1 | D01 | d1 | E01 |
| 2-2-d1E01-1-0074 | 622.259 | OH | A02 | a1 | B03 | b1 | C02 | c1 | D01 | d1 | E01 |
| 2-2-d1E01-1-0075 | 622.259 | OH | A02 | a1 | B03 | b1 | C03 | c1 | D01 | d1 | E01 |
| 2-2-d1E01-1-0076 | 622.290 | OH | A01 | a1 | B02 | b1 | C16 | c1 | D01 | d1 | E01 |
| 2-2-d1E01-1-0077 | 623.254 | OH | A02 | a1 | B03 | b1 | C04 | c1 | D01 | d1 | E01 |
| 2-2-d1E01-1-0078 | 623.286 | OH | A01 | a1 | B02 | b1 | C17 | c1 | D01 | d1 | E01 |
| 2-2-d1E01-1-0079 | 624.241 | OH | A02 | a1 | B02 | b1 | C05 | c1 | D01 | d1 | E01 |
| 2-2-d1E01-1-0080 | 624.270 | OH | A02 | a1 | B01 | b1 | C14 | c1 | D01 | d1 | E01 |
| 2-2-d1E01-1-0081 | 624.295 | OH | A01 | a1 | B02 | b4 | C01 | c1 | D01 | d1 | E01 |
| 2-2-d1E01-1-0082 | 625.200 | OH | A01 | a1 | B04 | b1 | C01 | c1 | D01 | d1 | E01 |
| 2-2-d1E01-1-0083 | 625.254 | OH | A02 | a1 | B02 | b3 | C08 | c1 | D01 | d1 | E01 |
| 2-2-d1E01-1-0084 | 625.270 | OH | A01 | a1 | B03 | b1 | C15 | c1 | D01 | d1 | E01 |
| 2-2-d1E01-1-0085 | 626.256 | OH | A01 | a1 | B01 | b4 | C05 | c1 | D01 | d1 | E01 |
| 2-2-d1E01-1-0086 | 626.265 | OH | A01 | a1 | B03 | b1 | C16 | c1 | D01 | d1 | E01 |
| 2-2-d1E01-1-0087 | 627.215 | OH | A02 | a1 | B01 | b3 | C10 | c1 | D01 | d1 | E01 |
| 2-2-d1E01-1-0088 | 627.261 | OH | A01 | a1 | B03 | b1 | C17 | c1 | D01 | d1 | E01 |
| 2-2-d1E01-1-0089 | 628.216 | OH | A02 | a1 | B03 | b1 | C05 | c1 | D01 | d1 | E01 |
| 2-2-d1E01-1-0090 | 628.247 | OH | A01 | a1 | B02 | b1 | C18 | c1 | D01 | d1 | E01 |
| 2-2-d1E01-1-0091 | 628.269 | OH | A02 | a1 | B01 | b2 | C02 | c1 | D01 | d1 | E01 |
| 2-2-d1E01-1-0092 | 628.269 | OH | A02 | a1 | B01 | b2 | C03 | c1 | D01 | d1 | E01 |
| 2-2-d1E01-1-0093 | 628.270 | OH | A01 | a1 | B03 | b4 | C01 | c1 | D01 | d1 | E01 |

(continued)

| ID | Exact Mass | ⊠ | A | a | B | b | C | c | D | d | E |
|---|---|---|---|---|---|---|---|---|---|---|---|
| 2-2-d1E01-1-0094 | 629.229 | OH | A02 | a1 | B03 | b3 | C08 | c1 | D01 | d1 | E01 |
| 2-2-d1E01-1-0095 | 629.264 | OH | A02 | a1 | B01 | b2 | C04 | c1 | D01 | d1 | E01 |
| 2-2-d1E01-1-0096 | 629.264 | OH | A02 | a1 | B01 | b2 | C06 | c1 | D01 | d1 | E01 |
| 2-2-d1E01-1-0097 | 629.264 | OH | A02 | a1 | B01 | b2 | C07 | c1 | D01 | d1 | E01 |
| 2-2-d1E01-1-0098 | 629.267 | OH | A01 | a1 | B04 | b4 | C12 | c1 | D01 | d1 | E01 |
| 2-2-d1E01-1-0099 | 631.246 | OH | A01 | a1 | B04 | b4 | C13 | c1 | D01 | d1 | E01 |
| 2-2-d1E01-1-0100 | 632.194 | OH | A01 | a1 | B01 | b1 | C01 | c2 | D02 | d1 | E01 |
| 2-2-d1E01-1-0101 | 632.222 | OH | A01 | a1 | B03 | b1 | C18 | c1 | D01 | d1 | E01 |
| 2-2-d1E01-1-0102 | 633.295 | OH | A02 | a1 | B01 | b1 | C15 | c1 | D01 | d1 | E01 |
| 2-2-d1E01-1-0103 | 634.243 | OH | A01 | a1 | B01 | b3 | Cll | c1 | D01 | d1 | E01 |
| 2-2-d1E01-1-0104 | 634.290 | OH | A02 | a1 | B01 | b1 | C16 | c1 | D01 | d1 | E01 |
| 2-2-d1E01-1-0105 | 634.316 | OH | A01 | a1 | B02 | b4 | C02 | c1 | D01 | d1 | E01 |
| 2-2-d1E01-1-0106 | 635.220 | OH | A01 | a1 | B04 | b1 | C02 | c1 | D01 | d1 | E01 |
| 2-2-d1E01-1-0107 | 635.220 | OH | A01 | a1 | B04 | b1 | C03 | c1 | D01 | d1 | E01 |
| 2-2-d1E01-1-0108 | 635.286 | OH | A02 | a1 | B01 | b1 | C17 | c1 | D01 | d1 | E01 |
| 2-2-d1E01-1-0109 | 636.215 | OH | A01 | a1 | B04 | b1 | C04 | c1 | D01 | d1 | E01 |
| 2-2-d1E01-1-0110 | 636.220 | OH | A01 | a1 | B05 | b1 | C01 | c1 | D01 | d1 | E01 |
| 2-2-d1E01-1-0111 | 636.262 | OH | A01 | a1 | B01 | b4 | C12 | c2 | D02 | d1 | E01 |
| 2-2-d1E01-1-0112 | 636.270 | OH | A02 | a1 | B02 | b3 | C09 | c1 | D01 | d1 | E01 |
| 2-2-d1E01-1-0113 | 636.295 | OH | A02 | a1 | B01 | b4 | C01 | c1 | D01 | d1 | E01 |
| 2-2-d1E01-1-0114 | 638.241 | OH | A01 | a1 | B01 | b4 | C13 | c2 | D02 | d1 | E01 |
| 2-2-d1E01-1-0115 | 638.285 | OH | A02 | a1 | B02 | b1 | C14 | c1 | D01 | d1 | E01 |
| 2-2-d1E01-1-0116 | 638.290 | OH | A01 | a1 | B03 | b4 | C02 | c1 | D01 | d1 | E01 |
| 2-2-d1E01-1-0117 | 640.245 | OH | A02 | a1 | B03 | b3 | C09 | c1 | D01 | d1 | E01 |
| 2-2-d1E01-1-0118 | 640.247 | OH | A02 | a1 | B01 | b1 | C18 | c1 | D01 | d1 | E01 |
| 2-2-d1E01-1-0119 | 640.272 | OH | A01 | a1 | B02 | b4 | C05 | c1 | D01 | d1 | E01 |
| 2-2-d1E01-1-0120 | 640.287 | OH | A01 | a1 | B05 | b4 | C12 | c1 | D01 | d1 | E01 |
| 2-2-d1E01-1-0121 | 641.177 | OH | A01 | a1 | B04 | b1 | C05 | c1 | D01 | d1 | E01 |
| 2-2-d1E01-1-0122 | 641.231 | OH | A02 | a1 | B02 | b3 | C10 | c1 | D01 | d1 | E01 |
| 2-2-d1E01-1-0123 | 642.190 | OH | A01 | a1 | B04 | b3 | C08 | c1 | D01 | d1 | E01 |
| 2-2-d1E01-1-0124 | 642.215 | OH | A01 | a1 | B01 | b1 | C02 | c2 | D02 | d1 | E01 |
| 2-2-d1E01-1-0125 | 642.215 | OH | A01 | a1 | B01 | b1 | C03 | c2 | D02 | d1 | E01 |
| 2-2-d1E01-1-0126 | 642.260 | OH | A02 | a1 | B03 | b1 | C14 | c1 | D01 | d1 | E01 |
| 2-2-d1E01-1-0127 | 642.267 | OH | A01 | a1 | B05 | b4 | C13 | c1 | D01 | d1 | E01 |
| 2-2-d1E01-1-0128 | 642.284 | OH | A02 | a1 | B02 | b2 | C02 | c1 | D01 | d1 | E01 |
| 2-2-d1E01-1-0129 | 642.284 | OH | A02 | a1 | B02 | b2 | C03 | c1 | D01 | d1 | E01 |
| 2-2-d1E01-1-0130 | 643.210 | OH | A01 | a1 | B01 | b1 | C04 | c2 | D02 | d1 | E01 |

(continued)

| ID | Exact Mass | ☒ | A | a | B | b | C | c | D | d | E |
|---|---|---|---|---|---|---|---|---|---|---|---|
| 2-2-d1E01-1-0131 | 643.279 | OH | A02 | a1 | B02 | b2 | C04 | c1 | D01 | d1 | E01 |
| 2-2-d1E01-1-0132 | 643.279 | OH | A02 | a1 | B02 | b2 | C06 | c1 | D01 | d1 | E01 |
| 2-2-d1E01-1-0133 | 643.279 | OH | A02 | a1 | B02 | b2 | C07 | c1 | D01 | d1 | E01 |
| 2-2-d1E01-1-0134 | 644.247 | OH | A01 | a1 | B03 | b4 | C05 | c1 | D01 | d1 | E01 |
| 2-2-d1E01-1-0135 | 645.206 | OH | A02 | a1 | B03 | b3 | C10 | c1 | D01 | d1 | E01 |
| 2-2-d1E01-1-0136 | 646.210 | OH | A01 | a1 | B02 | b1 | C01 | c2 | D02 | d1 | E01 |
| 2-2-d1E01-1-0137 | 646.240 | OH | A01 | a1 | B05 | b1 | C02 | c1 | D01 | d1 | E01 |
| 2-2-d1E01-1-0138 | 646.240 | OH | A01 | a1 | B05 | b1 | C03 | c1 | D01 | d1 | E01 |
| 2-2-d1E01-1-0139 | 646.259 | OH | A02 | a1 | B03 | b2 | C02 | c1 | D01 | d1 | E01 |
| 2-2-d1E01-1-0140 | 646.259 | OH | A02 | a1 | B03 | b2 | C03 | c1 | D01 | d1 | E01 |
| 2-2-d1E01-1-0141 | 646.316 | OH | A02 | a1 | B01 | b4 | C02 | c1 | D01 | d1 | E01 |
| 2-2-d1E01-1-0142 | 647.236 | OH | A01 | a1 | B05 | b1 | C04 | c1 | D01 | d1 | E01 |
| 2-2-d1E01-1-0143 | 647.254 | OH | A02 | a1 | B03 | b2 | C04 | c1 | D01 | d1 | E01 |
| 2-2-d1E01-1-0144 | 647.254 | OH | A02 | a1 | B03 | b2 | C06 | c1 | D01 | d1 | E01 |
| 2-2-d1E01-1-0145 | 647.254 | OH | A02 | a1 | B03 | b2 | C07 | c1 | D01 | d1 | E01 |
| 2-2-d1E01-1-0146 | 647.311 | OH | A02 | a1 | B02 | b1 | C15 | c1 | D01 | d1 | E01 |
| 2-2-d1E01-1-0147 | 648.171 | OH | A01 | a1 | B01 | b1 | C05 | c2 | D02 | d1 | E01 |
| 2-2-d1E01-1-0148 | 648.258 | OH | A01 | a1 | B02 | b3 | C11 | c1 | D01 | d1 | E01 |
| 2-2-d1E01-1-0149 | 648.306 | OH | A02 | a1 | B02 | b1 | C16 | c1 | D01 | d1 | E01 |
| 2-2-d1E01-1-0150 | 649.184 | OH | A01 | a1 | B01 | b3 | C08 | c2 | D02 | d1 | E01 |
| 2-2-d1E01-1-0151 | 649.301 | OH | A02 | a1 | B02 | b1 | C17 | c1 | D01 | d1 | E01 |
| 2-2-d1E01-1-0152 | 650.185 | OH | A01 | a1 | B03 | b1 | C01 | c2 | D02 | d1 | E01 |
| 2-2-d1E01-1-0153 | 650.277 | OH | A01 | a1 | B02 | b4 | C12 | c2 | D02 | d1 | E01 |
| 2-2-d1E01-1-0154 | 650.310 | OH | A02 | a1 | B02 | b4 | C01 | c1 | D01 | d1 | E01 |
| 2-2-d1E01-1-0155 | 651.215 | OH | A02 | a1 | B04 | b1 | C01 | c1 | D01 | d1 | E01 |
| 2-2-d1E01-1-0156 | 651.286 | OH | A02 | a1 | B03 | b1 | C15 | c1 | D01 | d1 | E01 |
| 2-2-d1E01-1-0157 | 652.197 | OH | A01 | a1 | B05 | b1 | C05 | c1 | D01 | d1 | E01 |
| 2-2-d1E01-1-0158 | 652.233 | OH | A01 | a1 | B03 | b3 | C11 | c1 | D01 | d1 | E01 |
| 2-2-d1E01-1-0159 | 652.257 | OH | A01 | a1 | B02 | b4 | C13 | c2 | D02 | d1 | E01 |
| 2-2-d1E01-1-0160 | 652.272 | OH | A02 | a1 | B01 | b4 | C05 | c1 | D01 | d1 | E01 |
| 2-2-d1E01-1-0161 | 652.281 | OH | A02 | a1 | B03 | b1 | C16 | c1 | D01 | d1 | E01 |
| 2-2-d1E01-1-0162 | 653.206 | OH | A01 | a1 | B04 | b3 | C09 | c1 | D01 | d1 | E01 |
| 2-2-d1E01-1-0163 | 653.210 | OH | A01 | a1 | B05 | b3 | C08 | c1 | D01 | d1 | E01 |
| 2-2-d1E01-1-0164 | 653.276 | OH | A02 | a1 | B03 | b1 | C17 | c1 | D01 | d1 | E01 |
| 2-2-d1E01-1-0165 | 654.252 | OH | A01 | a1 | B03 | b4 | C12 | c2 | D02 | d1 | E01 |
| 2-2-d1E01-1-0166 | 654.262 | OH | A02 | a1 | B02 | b1 | C18 | c1 | D01 | d1 | E01 |
| 2-2-d1E01-1-0167 | 654.285 | OH | A02 | a1 | B03 | b4 | C01 | c1 | D01 | d1 | E01 |

(continued)

| ID | Exact Mass | ☒ | A | a | B | b | C | c | D | d | E |
|---|---|---|---|---|---|---|---|---|---|---|---|
| 2-2-d1E01-1-0168 | 655.221 | OH | A01 | a1 | B04 | b1 | C14 | c1 | D01 | d1 | E01 |
| 2-2-d1E01-1-0169 | 655.283 | OH | A02 | a1 | B04 | b4 | C12 | c1 | D01 | d1 | E01 |
| 2-2-d1E01-1-0170 | 656.230 | OH | A01 | a1 | B02 | b1 | C02 | c2 | D02 | d1 | E01 |
| 2-2-d1E01-1-0171 | 656.230 | OH | A01 | a1 | B02 | b1 | C03 | c2 | D02 | d1 | E01 |
| 2-2-d1E01-1-0172 | 656.232 | OH | A01 | a1 | B03 | b4 | C13 | c2 | D02 | d1 | E01 |
| 2-2-d1E01-1-0173 | 657.226 | OH | A01 | a1 | B02 | b1 | C04 | c2 | D02 | d1 | E01 |
| 2-2-d1E01-1-0174 | 657.262 | OH | A02 | a1 | B04 | b4 | C13 | c1 | D01 | d1 | E01 |
| 2-2-d1E01-1-0175 | 658.167 | OH | A01 | a1 | B04 | b3 | C10 | c1 | D01 | d1 | E01 |
| 2-2-d1E01-1-0176 | 658.210 | OH | A02 | a1 | B01 | b1 | C01 | c2 | D02 | d1 | E01 |
| 2-2-d1E01-1-0177 | 658.237 | OH | A02 | a1 | B03 | b1 | C18 | c1 | D01 | d1 | E01 |
| 2-2-d1E01-1-0178 | 659.220 | OH | A01 | a1 | B04 | b2 | C02 | c1 | D01 | d1 | E01 |
| 2-2-d1E01-1-0179 | 659.220 | OH | A01 | a1 | B04 | b2 | C03 | c1 | D01 | d1 | E01 |
| 2-2-d1E01-1-0180 | 660.200 | OH | A01 | a1 | B01 | b3 | C09 | c2 | D02 | d1 | E01 |
| 2-2-d1E01-1-0181 | 660.205 | OH | A01 | a1 | B03 | b1 | C02 | c2 | D02 | d1 | E01 |
| 2-2-d1E01-1-0182 | 660.205 | OH | A01 | a1 | B03 | b1 | C03 | c2 | D02 | d1 | E01 |
| 2-2-d1E01-1-0183 | 660.215 | OH | A01 | a1 | B04 | b2 | C04 | c1 | D01 | d1 | E01 |
| 2-2-d1E01-1-0184 | 660.215 | OH | A01 | a1 | B04 | b2 | C06 | c1 | D01 | d1 | E01 |
| 2-2-d1E01-1-0185 | 660.215 | OH | A01 | a1 | B04 | b2 | C07 | c1 | D01 | d1 | E01 |
| 2-2-d1E01-1-0186 | 660.258 | OH | A02 | a1 | B01 | b3 | C11 | c1 | D01 | d1 | E01 |
| 2-2-d1E01-1-0187 | 660.331 | OH | A02 | a1 | B02 | b4 | C02 | c1 | D01 | d1 | E01 |
| 2-2-d1E01-1-0188 | 661.201 | OH | A01 | a1 | B03 | b1 | C04 | c2 | D02 | d1 | E01 |
| 2-2-d1E01-1-0189 | 661.236 | OH | A02 | a1 | B04 | b1 | C02 | c1 | D01 | d1 | E01 |
| 2-2-d1E01-1-0190 | 661.236 | OH | A02 | a1 | B04 | b1 | C03 | c1 | D01 | d1 | E01 |
| 2-2-d1E01-1-0191 | 662.187 | OH | A01 | a1 | B02 | b1 | C05 | c2 | D02 | d1 | E01 |
| 2-2-d1E01-1-0192 | 662.216 | OH | A01 | a1 | B01 | b1 | C14 | c2 | D02 | d1 | E01 |
| 2-2-d1E01-1-0193 | 662.231 | OH | A02 | a1 | B04 | b1 | C04 | c1 | D01 | d1 | E01 |
| 2-2-d1E01-1-0194 | 662.235 | OH | A02 | a1 | B05 | b1 | C01 | c1 | D01 | d1 | E01 |
| 2-2-d1E01-1-0195 | 662.277 | OH | A02 | a1 | B01 | b4 | C12 | c2 | D02 | d1 | E01 |
| 2-2-d1E01-1-0196 | 662.322 | OH | A01 | a1 | B01 | b1 | C19 | c1 | D01 | d1 | E01 |
| 2-2-d1E01-1-0197 | 663.200 | OH | A01 | a1 | B02 | b3 | C08 | c2 | D02 | d1 | E01 |
| 2-2-d1E01-1-0198 | 664.226 | OH | A01 | a1 | B05 | b3 | C09 | c1 | D01 | d1 | E01 |
| 2-2-d1E01-1-0199 | 664.247 | OH | A01 | a1 | B04 | b1 | C15 | c1 | D01 | d1 | E01 |
| 2-2-d1E01-1-0200 | 664.257 | OH | A02 | a1 | B01 | b4 | C13 | c2 | D02 | d1 | E01 |
| 2-2-d1E01-1-0201 | 664.306 | OH | A02 | a1 | B03 | b4 | C02 | c1 | D01 | d1 | E01 |
| 2-2-d1E01-1-0202 | 665.161 | OH | A01 | a1 | B01 | b3 | C10 | c2 | D02 | d1 | E01 |
| 2-2-d1E01-1-0203 | 665.242 | OH | A01 | a1 | B04 | b1 | C16 | c1 | D01 | d1 | E01 |
| 2-2-d1E01-1-0204 | 666.162 | OH | A01 | a1 | B03 | b1 | C05 | c2 | D02 | d1 | E01 |

(continued)

| ID | Exact Mass | ☒ | A | a | B | b | C | c | D | d | E |
|---|---|---|---|---|---|---|---|---|---|---|---|
| 2-2-d1E01-1-0205 | 666.215 | OH | A01 | a1 | B01 | b2 | C02 | c2 | D02 | d1 | E01 |
| 2-2-d1E01-1-0206 | 666.215 | OH | A01 | a1 | B01 | b2 | C03 | c2 | D02 | d1 | E01 |
| 2-2-d1E01-1-0207 | 666.237 | OH | A01 | a1 | B04 | b1 | C17 | c1 | D01 | d1 | E01 |
| 2-2-d1E01-1-0208 | 666.241 | OH | A01 | a1 | B05 | b1 | C14 | c1 | D01 | d1 | E01 |
| 2-2-d1E01-1-0209 | 666.288 | OH | A02 | a1 | B02 | b4 | C05 | c1 | D01 | d1 | E01 |
| 2-2-d1E01-1-0210 | 666.303 | OH | A02 | a1 | B05 | b4 | C12 | c1 | D01 | d1 | E01 |
| 2-2-d1E01-1-0211 | 667.175 | OH | A01 | a1 | B03 | b3 | C08 | c2 | D02 | d1 | E01 |
| 2-2-d1E01-1-0212 | 667.192 | OH | A02 | a1 | B04 | b1 | C05 | c1 | D01 | d1 | E01 |
| 2-2-d1E01-1-0213 | 667.210 | OH | A01 | a1 | B01 | b2 | C04 | c2 | D02 | d1 | E01 |
| 2-2-d1E01-1-0214 | 667.210 | OH | A01 | a1 | B01 | b2 | C06 | c2 | D02 | d1 | E01 |
| 2-2-d1E01-1-0215 | 667.210 | OH | A01 | a1 | B01 | b2 | C07 | c2 | D02 | d1 | E01 |
| 2-2-d1E01-1-0216 | 667.246 | OH | A01 | a1 | B04 | b4 | C01 | c1 | D01 | d1 | E01 |
| 2-2-d1E01-1-0217 | 668.205 | OH | A02 | a1 | B04 | b3 | C08 | c1 | D01 | d1 | E01 |
| 2-2-d1E01-1-0218 | 668.230 | OH | A02 | a1 | B01 | b1 | C02 | c2 | D02 | d1 | E01 |
| 2-2-d1E01-1-0219 | 668.230 | OH | A02 | a1 | B01 | b1 | C03 | c2 | D02 | d1 | E01 |
| 2-2-d1E01-1-0220 | 668.282 | OH | A02 | a1 | B05 | b4 | C13 | c1 | D01 | d1 | E01 |
| 2-2-d1E01-1-0221 | 669.187 | OH | A01 | a1 | B05 | b3 | C10 | c1 | D01 | d1 | E01 |
| 2-2-d1E01-1-0222 | 669.226 | OH | A02 | a1 | B01 | b1 | C04 | c2 | D02 | d1 | E01 |
| 2-2-d1E01-1-0223 | 670.240 | OH | A01 | a1 | B05 | b2 | C02 | c1 | D01 | d1 | E01 |
| 2-2-d1E01-1-0224 | 670.240 | OH | A01 | a1 | B05 | b2 | C03 | c1 | D01 | d1 | E01 |
| 2-2-d1E01-1-0225 | 670.263 | OH | A02 | a1 | B03 | b4 | C05 | c1 | D01 | d1 | E01 |
| 2-2-d1E01-1-0226 | 671.198 | OH | A01 | a1 | B04 | b1 | C18 | c1 | D01 | d1 | E01 |
| 2-2-d1E01-1-0227 | 671.236 | OH | A01 | a1 | B05 | b2 | C04 | c1 | D01 | d1 | E01 |
| 2-2-d1E01-1-0228 | 671.236 | OH | A01 | a1 | B05 | b2 | C06 | c1 | D01 | d1 | E01 |
| 2-2-d1E01-1-0229 | 671.236 | OH | A01 | a1 | B05 | b2 | C07 | c1 | D01 | d1 | E01 |
| 2-2-d1E01-1-0230 | 671.241 | OH | A01 | a1 | B01 | b1 | C15 | c2 | D02 | d1 | E01 |
| 2-2-d1E01-1-0231 | 672.225 | OH | A02 | a1 | B02 | b1 | C01 | c2 | D02 | d1 | E01 |
| 2-2-d1E01-1-0232 | 672.237 | OH | A01 | a1 | B01 | b1 | C16 | c2 | D02 | d1 | E01 |
| 2-2-d1E01-1-0233 | 672.256 | OH | A02 | a1 | B05 | b1 | C02 | c1 | D01 | d1 | E01 |
| 2-2-d1E01-1-0234 | 672.256 | OH | A02 | a1 | B05 | b1 | C03 | c1 | D01 | d1 | E01 |
| 2-2-d1E01-1-0235 | 673.232 | OH | A01 | a1 | B01 | b1 | C17 | c2 | D02 | d1 | E01 |
| 2-2-d1E01-1-0236 | 673.251 | OH | A02 | a1 | B05 | b1 | C04 | c1 | D01 | d1 | E01 |
| 2-2-d1E01-1-0237 | 674.187 | OH | A02 | a1 | B01 | b1 | C05 | c2 | D02 | d1 | E01 |
| 2-2-d1E01-1-0238 | 674.216 | OH | A01 | a1 | B02 | b3 | C09 | c2 | D02 | d1 | E01 |
| 2-2-d1E01-1-0239 | 674.241 | OH | A01 | a1 | B01 | b4 | C01 | c2 | D02 | d1 | E01 |
| 2-2-d1E01-1-0240 | 674.274 | OH | A02 | a1 | B02 | b3 | C11 | c1 | D01 | d1 | E01 |
| 2-2-d1E01-1-0241 | 675.200 | OH | A02 | a1 | B01 | b3 | C08 | c2 | D02 | d1 | E01 |

(continued)

| ID | Exact Mass | ☒ | A | a | B | b | C | c | D | d | E |
|---|---|---|---|---|---|---|---|---|---|---|---|
| 2-2-d1E01-1-0242 | 675.267 | OH | A01 | a1 | B05 | b1 | C15 | c1 | D01 | d1 | E01 |
| 2-2-d1E01-1-0243 | 676.200 | OH | A02 | a1 | B03 | b1 | C01 | c2 | D02 | d1 | E01 |
| 2-2-d1E01-1-0244 | 676.232 | OH | A01 | a1 | B02 | b1 | C14 | c2 | D02 | d1 | E01 |
| 2-2-d1E01-1-0245 | 676.262 | OH | A01 | a1 | B05 | b1 | C16 | c1 | D01 | d1 | E01 |
| 2-2-d1E01-1-0246 | 676.293 | OH | A02 | a1 | B02 | b4 | C12 | c2 | D02 | d1 | E01 |
| 2-2-d1E01-1-0247 | 676.337 | OH | A01 | a1 | B02 | b1 | C19 | c1 | D01 | d1 | E01 |
| 2-2-d1E01-1-0248 | 677.257 | OH | A01 | a1 | B05 | b1 | C17 | c1 | D01 | d1 | E01 |
| 2-2-d1E01-1-0249 | 677.267 | OH | A01 | a1 | B04 | b4 | C02 | c1 | D01 | d1 | E01 |
| 2-2-d1E01-1-0250 | 678.191 | OH | A01 | a1 | B03 | b3 | C09 | c2 | D02 | d1 | E01 |
| 2-2-d1E01-1-0251 | 678.193 | OH | A01 | a1 | B01 | b1 | C18 | c2 | D02 | d1 | E01 |
| 2-2-d1E01-1-0252 | 678.212 | OH | A02 | a1 | B05 | b1 | C05 | c1 | D01 | d1 | E01 |
| 2-2-d1E01-1-0253 | 678.249 | OH | A02 | a1 | B03 | b3 | C11 | c1 | D01 | d1 | E01 |
| 2-2-d1E01-1-0254 | 678.267 | OH | A01 | a1 | B05 | b4 | C01 | c1 | D01 | d1 | E01 |
| 2-2-d1E01-1-0255 | 678.272 | OH | A02 | a1 | B02 | b4 | C13 | c2 | D02 | d1 | E01 |
| 2-2-d1E01-1-0256 | 679.177 | OH | A01 | a1 | B02 | b3 | C10 | c2 | D02 | d1 | E01 |
| 2-2-d1E01-1-0257 | 679.221 | OH | A02 | a1 | B04 | b3 | C09 | c1 | D01 | d1 | E01 |
| 2-2-d1E01-1-0258 | 679.225 | OH | A02 | a1 | B05 | b3 | C08 | c1 | D01 | d1 | E01 |
| 2-2-d1E01-1-0259 | 680.206 | OH | A01 | a1 | B03 | b1 | C14 | c2 | D02 | d1 | E01 |
| 2-2-d1E01-1-0260 | 680.230 | OH | A01 | a1 | B02 | b2 | C02 | c2 | D02 | d1 | E01 |
| 2-2-d1E01-1-0261 | 680.230 | OH | A01 | a1 | B02 | b2 | C03 | c2 | D02 | d1 | E01 |
| 2-2-d1E01-1-0262 | 680.268 | OH | A02 | a1 | B03 | b4 | C12 | c2 | D02 | d1 | E01 |
| 2-2-d1E01-1-0263 | 680.312 | OH | A01 | a1 | B03 | b1 | C19 | c1 | D01 | d1 | E01 |
| 2-2-d1E01-1-0264 | 681.226 | OH | A01 | a1 | B02 | b2 | C04 | c2 | D02 | d1 | E01 |
| 2-2-d1E01-1-0265 | 681.226 | OH | A01 | a1 | B02 | b2 | C06 | c2 | D02 | d1 | E01 |
| 2-2-d1E01-1-0266 | 681.226 | OH | A01 | a1 | B02 | b2 | C07 | c2 | D02 | d1 | E01 |
| 2-2-d1E01-1-0267 | 681.237 | OH | A02 | a1 | B04 | b1 | C14 | c1 | D01 | d1 | E01 |
| 2-2-d1E01-1-0268 | 682.219 | OH | A01 | a1 | B05 | b1 | C18 | c1 | D01 | d1 | E01 |
| 2-2-d1E01-1-0269 | 682.246 | OH | A02 | a1 | B02 | b1 | C02 | c2 | D02 | d1 | E01 |
| 2-2-d1E01-1-0270 | 682.246 | OH | A02 | a1 | B02 | b1 | C03 | c2 | D02 | d1 | E01 |
| 2-2-d1E01-1-0271 | 682.247 | OH | A02 | a1 | B03 | b4 | C13 | c2 | D02 | d1 | E01 |
| 2-2-d1E01-1-0272 | 683.152 | OH | A01 | a1 | B03 | b3 | C10 | c2 | D02 | d1 | E01 |
| 2-2-d1E01-1-0273 | 683.224 | OH | A01 | a1 | B04 | b4 | C05 | c1 | D01 | d1 | E01 |
| 2-2-d1E01-1-0274 | 683.241 | OH | A02 | a1 | B02 | b1 | C04 | c2 | D02 | d1 | E01 |
| 2-2-d1E01-1-0275 | 684.182 | OH | A02 | a1 | B04 | b3 | C10 | c1 | D01 | d1 | E01 |
| 2-2-d1E01-1-0276 | 684.205 | OH | A01 | a1 | B03 | b2 | C02 | c2 | D02 | d1 | E01 |
| 2-2-d1E01-1-0277 | 684.205 | OH | A01 | a1 | B03 | b2 | C03 | c2 | D02 | d1 | E01 |
| 2-2-d1E01-1-0278 | 684.262 | OH | A01 | a1 | B01 | b4 | C02 | c2 | D02 | d1 | E01 |

(continued)

| ID | Exact Mass | ☒ | A | a | B | b | C | c | D | d | E |
|---|---|---|---|---|---|---|---|---|---|---|---|
| 2-2-d1E01-1-0279 | 685.201 | OH | A01 | a1 | B03 | b2 | C04 | c2 | D02 | d1 | E01 |
| 2-2-d1E01-1-0280 | 685.201 | OH | A01 | a1 | B03 | b2 | C06 | c2 | D02 | d1 | E01 |
| 2-2-d1E01-1-0281 | 685.201 | OH | A01 | a1 | B03 | b2 | C07 | c2 | D02 | d1 | E01 |
| 2-2-d1E01-1-0282 | 685.236 | OH | A02 | a1 | B04 | b2 | C02 | c1 | D01 | d1 | E01 |
| 2-2-d1E01-1-0283 | 685.236 | OH | A02 | a1 | B04 | b2 | C03 | c1 | D01 | d1 | E01 |
| 2-2-d1E01-1-0284 | 685.257 | OH | A01 | a1 | B02 | b1 | C15 | c2 | D02 | d1 | E01 |
| 2-2-d1E01-1-0285 | 686.216 | OH | A02 | a1 | B01 | b3 | C09 | c2 | D02 | d1 | E01 |
| 2-2-d1E01-1-0286 | 686.221 | OH | A02 | a1 | B03 | b1 | C02 | c2 | D02 | d1 | E01 |
| 2-2-d1E01-1-0287 | 686.221 | OH | A02 | a1 | B03 | b1 | C03 | c2 | D02 | d1 | E01 |
| 2-2-d1E01-1-0288 | 686.231 | OH | A02 | a1 | B04 | b2 | C04 | c1 | D01 | d1 | E01 |
| 2-2-d1E01-1-0289 | 686.231 | OH | A02 | a1 | B04 | b2 | C06 | c1 | D01 | d1 | E01 |
| 2-2-d1E01-1-0290 | 686.231 | OH | A02 | a1 | B04 | b2 | C07 | c1 | D01 | d1 | E01 |
| 2-2-d1E01-1-0291 | 686.252 | OH | A01 | a1 | B02 | b1 | C16 | c2 | D02 | d1 | E01 |
| 2-2-d1E01-1-0292 | 687.216 | OH | A02 | a1 | B03 | b1 | C04 | c2 | D02 | d1 | E01 |
| 2-2-d1E01-1-0293 | 687.248 | OH | A01 | a1 | B02 | b1 | C17 | c2 | D02 | d1 | E01 |
| 2-2-d1E01-1-0294 | 688.203 | OH | A02 | a1 | B02 | b1 | C05 | c2 | D02 | d1 | E01 |
| 2-2-d1E01-1-0295 | 688.232 | OH | A02 | a1 | B01 | b1 | C14 | c2 | D02 | d1 | E01 |
| 2-2-d1E01-1-0296 | 688.257 | OH | A01 | a1 | B02 | b4 | C01 | c2 | D02 | d1 | E01 |
| 2-2-d1E01-1-0297 | 688.287 | OH | A01 | a1 | B05 | b4 | C02 | c1 | D01 | d1 | E01 |
| 2-2-d1E01-1-0298 | 688.337 | OH | A02 | a1 | B01 | b1 | C19 | c1 | D01 | d1 | E01 |
| 2-2-d1E01-1-0299 | 689.161 | OH | A01 | a1 | B04 | b1 | C01 | c2 | D02 | d1 | E01 |
| 2-2-d1E01-1-0300 | 689.216 | OH | A02 | a1 | B02 | b3 | C08 | c2 | D02 | d1 | E01 |
| 2-2-d1E01-1-0301 | 689.232 | OH | A01 | a1 | B03 | b1 | C15 | c2 | D02 | d1 | E01 |
| 2-2-d1E01-1-0302 | 690.218 | OH | A01 | a1 | B01 | b4 | C05 | c2 | D02 | d1 | E01 |
| 2-2-d1E01-1-0303 | 690.227 | OH | A01 | a1 | B03 | b1 | C16 | c2 | D02 | d1 | E01 |
| 2-2-d1E01-1-0304 | 690.241 | OH | A02 | a1 | B05 | b3 | C09 | c1 | D01 | d1 | E01 |
| 2-2-d1E01-1-0305 | 690.262 | OH | A02 | a1 | B04 | b1 | C15 | c1 | D01 | d1 | E01 |
| 2-2-d1E01-1-0306 | 691.177 | OH | A02 | a1 | B01 | b3 | C10 | c2 | D02 | d1 | E01 |
| 2-2-d1E01-1-0307 | 691.210 | OH | A01 | a1 | B04 | b3 | C11 | c1 | D01 | d1 | E01 |
| 2-2-d1E01-1-0308 | 691.222 | OH | A01 | a1 | B03 | b1 | C17 | c2 | D02 | d1 | E01 |
| 2-2-d1E01-1-0309 | 691.258 | OH | A02 | a1 | B04 | b1 | C16 | c1 | D01 | d1 | E01 |
| 2-2-d1E01-1-0310 | 692.177 | OH | A02 | a1 | B03 | b1 | C05 | c2 | D02 | d1 | E01 |
| 2-2-d1E01-1-0311 | 692.209 | OH | A01 | a1 | B02 | b1 | C18 | c2 | D02 | d1 | E01 |
| 2-2-d1E01-1-0312 | 692.230 | OH | A02 | a1 | B01 | b2 | C02 | c2 | D02 | d1 | E01 |
| 2-2-d1E01-1-0313 | 692.230 | OH | A02 | a1 | B01 | b2 | C03 | c2 | D02 | d1 | E01 |
| 2-2-d1E01-1-0314 | 692.232 | OH | A01 | a1 | B03 | b4 | C01 | c2 | D02 | d1 | E01 |
| 2-2-d1E01-1-0315 | 692.253 | OH | A02 | a1 | B04 | b1 | C17 | c1 | D01 | d1 | E01 |

(continued)

| ID | Exact Mass | ☒ | A | a | B | b | C | c | D | d | E |
|---|---|---|---|---|---|---|---|---|---|---|---|
| 2-2-d1E01-1-0316 | 692.257 | OH | A02 | a1 | B05 | b1 | C14 | c1 | D01 | d1 | E01 |
| 2-2-d1E01-1-0317 | 693.190 | OH | A02 | a1 | B03 | b3 | C08 | c2 | D02 | d1 | E01 |
| 2-2-d1E01-1-0318 | 693.226 | OH | A02 | a1 | B01 | b2 | C04 | c2 | D02 | d1 | E01 |
| 2-2-d1E01-1-0319 | 693.226 | OH | A02 | a1 | B01 | b2 | C06 | c2 | D02 | d1 | E01 |
| 2-2-d1E01-1-0320 | 693.226 | OH | A02 | a1 | B01 | b2 | C07 | c2 | D02 | d1 | E01 |
| 2-2-d1E01-1-0321 | 693.229 | OH | A01 | a1 | B04 | b4 | C12 | c2 | D02 | d1 | E01 |
| 2-2-d1E01-1-0322 | 693.262 | OH | A02 | a1 | B04 | b4 | C01 | c1 | D01 | d1 | E01 |
| 2-2-d1E01-1-0323 | 694.244 | OH | A01 | a1 | B05 | b4 | C05 | c1 | D01 | d1 | E01 |
| 2-2-d1E01-1-0324 | 695.203 | OH | A02 | a1 | B05 | b3 | C10 | c1 | D01 | d1 | E01 |
| 2-2-d1E01-1-0325 | 695.208 | OH | A01 | a1 | B04 | b4 | C13 | c2 | D02 | d1 | E01 |
| 2-2-d1E01-1-0326 | 696.184 | OH | A01 | a1 | B03 | b1 | C18 | c2 | D02 | d1 | E01 |
| 2-2-d1E01-1-0327 | 696.256 | OH | A02 | a1 | B05 | b2 | C02 | c1 | D01 | d1 | E01 |
| 2-2-d1E01-1-0328 | 696.256 | OH | A02 | a1 | B05 | b2 | C03 | c1 | D01 | d1 | E01 |
| 2-2-d1E01-1-0329 | 697.214 | OH | A02 | a1 | B04 | b1 | C18 | c1 | D01 | d1 | E01 |
| 2-2-d1E01-1-0330 | 697.251 | OH | A02 | a1 | B05 | b2 | C04 | c1 | D01 | d1 | E01 |
| 2-2-d1E01-1-0331 | 697.251 | OH | A02 | a1 | B05 | b2 | C06 | c1 | D01 | d1 | E01 |
| 2-2-d1E01-1-0332 | 697.251 | OH | A02 | a1 | B05 | b2 | C07 | c1 | D01 | d1 | E01 |
| 2-2-d1E01-1-0333 | 697.257 | OH | A02 | a1 | B01 | b1 | C15 | c2 | D02 | d1 | E01 |
| 2-2-d1E01-1-0334 | 698.205 | OH | A01 | a1 | B01 | b3 | C11 | c2 | D02 | d1 | E01 |
| 2-2-d1E01-1-0335 | 698.252 | OH | A02 | a1 | B01 | b1 | C16 | c2 | D02 | d1 | E01 |
| 2-2-d1E01-1-0336 | 698.277 | OH | A01 | a1 | B02 | b4 | C02 | c2 | D02 | d1 | E01 |
| 2-2-d1E01-1-0337 | 699.182 | OH | A01 | a1 | B04 | b1 | C02 | c2 | D02 | d1 | E01 |
| 2-2-d1E01-1-0338 | 699.182 | OH | A01 | a1 | B04 | b1 | C03 | c2 | D02 | d1 | E01 |
| 2-2-d1E01-1-0339 | 699.248 | OH | A02 | a1 | B01 | b1 | C17 | c2 | D02 | d1 | E01 |
| 2-2-d1E01-1-0340 | 700.177 | OH | A01 | a1 | B04 | b1 | C04 | c2 | D02 | d1 | E01 |
| 2-2-d1E01-1-0341 | 700.181 | OH | A01 | a1 | B05 | b1 | C01 | c2 | D02 | d1 | E01 |
| 2-2-d1E01-1-0342 | 700.232 | OH | A02 | a1 | B02 | b3 | C09 | c2 | D02 | d1 | E01 |
| 2-2-d1E01-1-0343 | 700.257 | OH | A02 | a1 | B01 | b4 | C01 | c2 | D02 | d1 | E01 |
| 2-2-d1E01-1-0344 | 701.283 | OH | A02 | a1 | B05 | b1 | C15 | c1 | D01 | d1 | E01 |
| 2-2-d1E01-1-0345 | 702.230 | OH | A01 | a1 | B05 | b3 | C11 | c1 | D01 | d1 | E01 |
| 2-2-d1E01-1-0346 | 702.247 | OH | A02 | a1 | B02 | b1 | C14 | c2 | D02 | d1 | E01 |
| 2-2-d1E01-1-0347 | 702.252 | OH | A01 | a1 | B03 | b4 | C02 | c2 | D02 | d1 | E01 |
| 2-2-d1E01-1-0348 | 702.278 | OH | A02 | a1 | B05 | b1 | C16 | c1 | D01 | d1 | E01 |
| 2-2-d1E01-1-0349 | 702.353 | OH | A02 | a1 | B02 | b1 | C19 | c1 | D01 | d1 | E01 |
| 2-2-d1E01-1-0350 | 703.273 | OH | A02 | a1 | B05 | b1 | C17 | c1 | D01 | d1 | E01 |
| 2-2-d1E01-1-0351 | 703.283 | OH | A02 | a1 | B04 | b4 | C02 | c1 | D01 | d1 | E01 |
| 2-2-d1E01-1-0352 | 704.206 | OH | A02 | a1 | B03 | b3 | C09 | c2 | D02 | d1 | E01 |

(continued)

| ID | Exact Mass | ☒ | A | a | B | b | C | c | D | d | E |
|---|---|---|---|---|---|---|---|---|---|---|---|
| 2-2-d1E01-1-0353 | 704.209 | OH | A02 | a1 | B01 | b1 | C18 | c2 | D02 | d1 | E01 |
| 2-2-d1E01-1-0354 | 704.234 | OH | A01 | a1 | B02 | b4 | C05 | c2 | D02 | d1 | E01 |
| 2-2-d1E01-1-0355 | 704.249 | OH | A01 | a1 | B05 | b4 | C12 | c2 | D02 | d1 | E01 |
| 2-2-d1E01-1-0356 | 704.282 | OH | A02 | a1 | B05 | b4 | C01 | c1 | D01 | d1 | E01 |
| 2-2-d1E01-1-0357 | 705.139 | OH | A01 | a1 | B04 | b1 | C05 | c2 | D02 | d1 | E01 |
| 2-2-d1E01-1-0358 | 705.193 | OH | A02 | a1 | B02 | b3 | C10 | c2 | D02 | d1 | E01 |
| 2-2-d1E01-1-0359 | 706.152 | OH | A01 | a1 | B04 | b3 | C08 | c2 | D02 | d1 | E01 |
| 2-2-d1E01-1-0360 | 706.222 | OH | A02 | a1 | B03 | b1 | C14 | c2 | D02 | d1 | E01 |
| 2-2-d1E01-1-0361 | 706.228 | OH | A01 | a1 | B05 | b4 | C13 | c2 | D02 | d1 | E01 |
| 2-2-d1E01-1-0362 | 706.246 | OH | A02 | a1 | B02 | b2 | C02 | c2 | D02 | d1 | E01 |
| 2-2-d1E01-1-0363 | 706.246 | OH | A02 | a1 | B02 | b2 | C03 | c2 | D02 | d1 | E01 |
| 2-2-d1E01-1-0364 | 706.328 | OH | A02 | a1 | B03 | b1 | C19 | c1 | D01 | d1 | E01 |
| 2-2-d1E01-1-0365 | 707.241 | OH | A02 | a1 | B02 | b2 | C04 | c2 | D02 | d1 | E01 |
| 2-2-d1E01-1-0366 | 707.241 | OH | A02 | a1 | B02 | b2 | C06 | c2 | D02 | d1 | E01 |
| 2-2-d1E01-1-0367 | 707.241 | OH | A02 | a1 | B02 | b2 | C07 | c2 | D02 | d1 | E01 |
| 2-2-d1E01-1-0368 | 708.209 | OH | A01 | a1 | B03 | b4 | C05 | c2 | D02 | d1 | E01 |
| 2-2-d1E01-1-0369 | 708.234 | OH | A02 | a1 | B05 | b1 | C18 | c1 | D01 | d1 | E01 |
| 2-2-d1E01-1-0370 | 709.168 | OH | A02 | a1 | B03 | b3 | C10 | c2 | D02 | d1 | E01 |
| 2-2-d1E01-1-0371 | 709.239 | OH | A02 | a1 | B04 | b4 | C05 | c1 | D01 | d1 | E01 |
| 2-2-d1E01-1-0372 | 710.202 | OH | A01 | a1 | B05 | b1 | C02 | c2 | D02 | d1 | E01 |
| 2-2-d1E01-1-0373 | 710.202 | OH | A01 | a1 | B05 | b1 | C03 | c2 | D02 | d1 | E01 |
| 2-2-d1E01-1-0374 | 710.221 | OH | A02 | a1 | B03 | b2 | C02 | c2 | D02 | d1 | E01 |
| 2-2-d1E01-1-0375 | 710.221 | OH | A02 | a1 | B03 | b2 | C03 | c2 | D02 | d1 | E01 |
| 2-2-d1E01-1-0376 | 710.277 | OH | A02 | a1 | B01 | b4 | C02 | c2 | D02 | d1 | E01 |
| 2-2-d1E01-1-0377 | 711.197 | OH | A01 | a1 | B05 | b1 | C04 | c2 | D02 | d1 | E01 |
| 2-2-d1E01-1-0378 | 711.216 | OH | A02 | a1 | B03 | b2 | C04 | c2 | D02 | d1 | E01 |
| 2-2-d1E01-1-0379 | 711.216 | OH | A02 | a1 | B03 | b2 | C06 | c2 | D02 | d1 | E01 |
| 2-2-d1E01-1-0380 | 711.216 | OH | A02 | a1 | B03 | b2 | C07 | c2 | D02 | d1 | E01 |
| 2-2-d1E01-1-0381 | 711.273 | OH | A02 | a1 | B02 | b1 | C15 | c2 | D02 | d1 | E01 |
| 2-2-d1E01-1-0382 | 712.220 | OH | A01 | a1 | B02 | b3 | C11 | c2 | D02 | d1 | E01 |
| 2-2-d1E01-1-0383 | 712.268 | OH | A02 | a1 | B02 | b1 | C16 | c2 | D02 | d1 | E01 |
| 2-2-d1E01-1-0384 | 713.263 | OH | A02 | a1 | B02 | b1 | C17 | c2 | D02 | d1 | E01 |
| 2-2-d1E01-1-0385 | 714.272 | OH | A02 | a1 | B02 | b4 | C01 | c2 | D02 | d1 | E01 |
| 2-2-d1E01-1-0386 | 714.303 | OH | A02 | a1 | B05 | b4 | C02 | c1 | D01 | d1 | E01 |
| 2-2-d1E01-1-0387 | 715.177 | OH | A02 | a1 | B04 | b1 | C01 | c2 | D02 | d1 | E01 |
| 2-2-d1E01-1-0388 | 715.248 | OH | A02 | a1 | B03 | b1 | C15 | c2 | D02 | d1 | E01 |
| 2-2-d1E01-1-0389 | 716.159 | OH | A01 | a1 | B05 | b1 | C05 | c2 | D02 | d1 | E01 |

(continued)

| ID | Exact Mass | ☒ | A | a | B | b | C | c | D | d | E |
|---|---|---|---|---|---|---|---|---|---|---|---|
| 2-2-d1E01-1-0390 | 716.195 | OH | A01 | a1 | B03 | b3 | C11 | c2 | D02 | d1 | E01 |
| 2-2-d1E01-1-0391 | 716.234 | OH | A02 | a1 | B01 | b4 | C05 | c2 | D02 | d1 | E01 |
| 2-2-d1E01-1-0392 | 716.243 | OH | A02 | a1 | B03 | b1 | C16 | c2 | D02 | d1 | E01 |
| 2-2-d1E01-1-0393 | 717.168 | OH | A01 | a1 | B04 | b3 | C09 | c2 | D02 | d1 | E01 |
| 2-2-d1E01-1-0394 | 717.172 | OH | A01 | a1 | B05 | b3 | C08 | c2 | D02 | d1 | E01 |
| 2-2-d1E01-1-0395 | 717.226 | OH | A02 | a1 | B04 | b3 | C11 | c1 | D01 | d1 | E01 |
| 2-2-d1E01-1-0396 | 717.238 | OH | A02 | a1 | B03 | b1 | C17 | c2 | D02 | d1 | E01 |
| 2-2-d1E01-1-0397 | 718.224 | OH | A02 | a1 | B02 | b1 | C18 | c2 | D02 | d1 | E01 |
| 2-2-d1E01-1-0398 | 718.247 | OH | A02 | a1 | B03 | b4 | C01 | c2 | D02 | d1 | E01 |
| 2-2-d1E01-1-0399 | 719.183 | OH | A01 | a1 | B04 | b1 | C14 | c2 | D02 | d1 | E01 |
| 2-2-d1E01-1-0400 | 719.245 | OH | A02 | a1 | B04 | b4 | C12 | c2 | D02 | d1 | E01 |
| 2-2-d1E01-1-0401 | 719.289 | OH | A01 | a1 | B04 | b1 | C19 | c1 | D01 | d1 | E01 |
| 2-2-d1E01-1-0402 | 720.259 | OH | A02 | a1 | B05 | b4 | C05 | c1 | D01 | d1 | E01 |
| 2-2-d1E01-1-0403 | 721.224 | OH | A02 | a1 | B04 | b4 | C13 | c2 | D02 | d1 | E01 |
| 2-2-d1E01-1-0404 | 722.129 | OH | A01 | a1 | B04 | b3 | C10 | c2 | D02 | d1 | E01 |
| 2-2-d1E01-1-0405 | 722.199 | OH | A02 | a1 | B03 | b1 | C18 | c2 | D02 | d1 | E01 |
| 2-2-d1E01-1-0406 | 723.182 | OH | A01 | a1 | B04 | b2 | C02 | c2 | D02 | d1 | E01 |
| 2-2-d1E01-1-0407 | 723.182 | OH | A01 | a1 | B04 | b2 | C03 | c2 | D02 | d1 | E01 |
| 2-2-d1E01-1-0408 | 724.177 | OH | A01 | a1 | B04 | b2 | C04 | c2 | D02 | d1 | E01 |
| 2-2-d1E01-1-0409 | 724.177 | OH | A01 | a1 | B04 | b2 | C06 | c2 | D02 | d1 | E01 |
| 2-2-d1E01-1-0410 | 724.177 | OH | A01 | a1 | B04 | b2 | C07 | c2 | D02 | d1 | E01 |
| 2-2-d1E01-1-0411 | 724.220 | OH | A02 | a1 | B01 | b3 | C11 | c2 | D02 | d1 | E01 |
| 2-2-d1E01-1-0412 | 724.293 | OH | A02 | a1 | B02 | b4 | C02 | c2 | D02 | d1 | E01 |
| 2-2-d1E01-1-0413 | 725.198 | OH | A02 | a1 | B04 | b1 | C02 | c2 | D02 | d1 | E01 |
| 2-2-d1E01-1-0414 | 725.198 | OH | A02 | a1 | B04 | b1 | C03 | c2 | D02 | d1 | E01 |
| 2-2-d1E01-1-0415 | 726.193 | OH | A02 | a1 | B04 | b1 | C04 | c2 | D02 | d1 | E01 |
| 2-2-d1E01-1-0416 | 726.197 | OH | A02 | a1 | B05 | b1 | C01 | c2 | D02 | d1 | E01 |
| 2-2-d1E01-1-0417 | 726.284 | OH | A01 | a1 | B01 | b1 | C19 | c2 | D02 | d1 | E01 |
| 2-2-d1E01-1-0418 | 728.188 | OH | A01 | a1 | B05 | b3 | C09 | c2 | D02 | d1 | E01 |
| 2-2-d1E01-1-0419 | 728.209 | OH | A01 | a1 | B04 | b1 | C15 | c2 | D02 | d1 | E01 |
| 2-2-d1E01-1-0420 | 728.246 | OH | A02 | a1 | B05 | b3 | C11 | c1 | D01 | d1 | E01 |
| 2-2-d1E01-1-0421 | 728.268 | OH | A02 | a1 | B03 | b4 | C02 | c2 | D02 | d1 | E01 |
| 2-2-d1E01-1-0422 | 729.204 | OH | A01 | a1 | B04 | b1 | C16 | c2 | D02 | d1 | E01 |
| 2-2-d1E01-1-0423 | 730.199 | OH | A01 | a1 | B04 | b1 | C17 | c2 | D02 | d1 | E01 |
| 2-2-d1E01-1-0424 | 730.203 | OH | A01 | a1 | B05 | b1 | C14 | c2 | D02 | d1 | E01 |
| 2-2-d1E01-1-0425 | 730.249 | OH | A02 | a1 | B02 | b4 | C05 | c2 | D02 | d1 | E01 |
| 2-2-d1E01-1-0426 | 730.265 | OH | A02 | a1 | B05 | b4 | C12 | c2 | D02 | d1 | E01 |

(continued)

| ID | Exact Mass | ☒ | A | a | B | b | C | c | D | d | E |
|---|---|---|---|---|---|---|---|---|---|---|---|
| 2-2-d1E01-1-0427 | 730.309 | OH | A01 | a1 | B05 | b1 | C19 | c1 | D01 | d1 | E01 |
| 2-2-d1E01-1-0428 | 731.154 | OH | A02 | a1 | B04 | b1 | C05 | c2 | D02 | d1 | E01 |
| 2-2-d1E01-1-0429 | 731.208 | OH | A01 | a1 | B04 | b4 | C01 | c2 | D02 | d1 | E01 |
| 2-2-d1E01-1-0430 | 732.167 | OH | A02 | a1 | B04 | b3 | C08 | c2 | D02 | d1 | E01 |
| 2-2-d1E01-1-0431 | 732.244 | OH | A02 | a1 | B05 | b4 | C13 | c2 | D02 | d1 | E01 |
| 2-2-d1E01-1-0432 | 733.149 | OH | A01 | a1 | B05 | b3 | C10 | c2 | D02 | d1 | E01 |
| 2-2-d1E01-1-0433 | 734.202 | OH | A01 | a1 | B05 | b2 | C02 | c2 | D02 | d1 | E01 |
| 2-2-d1E01-1-0434 | 734.202 | OH | A01 | a1 | B05 | b2 | C03 | c2 | D02 | d1 | E01 |
| 2-2-d1E01-1-0435 | 734.224 | OH | A02 | a1 | B03 | b4 | C05 | c2 | D02 | d1 | E01 |
| 2-2-d1E01-1-0436 | 735.160 | OH | A01 | a1 | B04 | b1 | C18 | c2 | D02 | d1 | E01 |
| 2-2-d1E01-1-0437 | 735.197 | OH | A01 | a1 | B05 | b2 | C04 | c2 | D02 | d1 | E01 |
| 2-2-d1E01-1-0438 | 735.197 | OH | A01 | a1 | B05 | b2 | C06 | c2 | D02 | d1 | E01 |
| 2-2-d1E01-1-0439 | 735.197 | OH | A01 | a1 | B05 | b2 | C07 | c2 | D02 | d1 | E01 |
| 2-2-d1E01-1-0440 | 736.218 | OH | A02 | a1 | B05 | b1 | C02 | c2 | D02 | d1 | E01 |
| 2-2-d1E01-1-0441 | 736.218 | OH | A02 | a1 | B05 | b1 | C03 | c2 | D02 | d1 | E01 |
| 2-2-d1E01-1-0442 | 737.213 | OH | A02 | a1 | B05 | b1 | C04 | c2 | D02 | d1 | E01 |
| 2-2-d1E01-1-0443 | 738.236 | OH | A02 | a1 | B02 | b3 | C11 | c2 | D02 | d1 | E01 |
| 2-2-d1E01-1-0444 | 739.229 | OH | A01 | a1 | B05 | b1 | C15 | c2 | D02 | d1 | E01 |
| 2-2-d1E01-1-0445 | 740.224 | OH | A01 | a1 | B05 | b1 | C16 | c2 | D02 | d1 | E01 |
| 2-2-d1E01-1-0446 | 740.299 | OH | A01 | a1 | B02 | b1 | C19 | c2 | D02 | d1 | E01 |
| 2-2-d1E01-1-0447 | 741.219 | OH | A01 | a1 | B05 | b1 | C17 | c2 | D02 | d1 | E01 |
| 2-2-d1E01-1-0448 | 741.229 | OH | A01 | a1 | B04 | b4 | C02 | c2 | D02 | d1 | E01 |
| 2-2-d1E01-1-0449 | 742.174 | OH | A02 | a1 | B05 | b1 | C05 | c2 | D02 | d1 | E01 |
| 2-2-d1E01-1-0450 | 742.211 | OH | A02 | a1 | B03 | b3 | C11 | c2 | D02 | d1 | E01 |
| 2-2-d1E01-1-0451 | 742.228 | OH | A01 | a1 | B05 | b4 | C01 | c2 | D02 | d1 | E01 |
| 2-2-d1E01-1-0452 | 743.183 | OH | A02 | a1 | B04 | b3 | C09 | c2 | D02 | d1 | E01 |
| 2-2-d1E01-1-0453 | 743.187 | OH | A02 | a1 | B05 | b3 | C08 | c2 | D02 | d1 | E01 |
| 2-2-d1E01-1-0454 | 744.274 | OH | A01 | a1 | B03 | b1 | C19 | c2 | D02 | d1 | E01 |
| 2-2-d1E01-1-0455 | 745.199 | OH | A02 | a1 | B04 | b1 | C14 | c2 | D02 | d1 | E01 |
| 2-2-d1E01-1-0456 | 745.305 | OH | A02 | a1 | B04 | b1 | C19 | c1 | D01 | d1 | E01 |
| 2-2-d1E01-1-0457 | 746.180 | OH | A01 | a1 | B05 | b1 | C18 | c2 | D02 | d1 | E01 |
| 2-2-d1E01-1-0458 | 747.186 | OH | A01 | a1 | B04 | b4 | C05 | c2 | D02 | d1 | E01 |
| 2-2-d1E01-1-0459 | 748.144 | OH | A02 | a1 | B04 | b3 | C10 | c2 | D02 | d1 | E01 |
| 2-2-d1E01-1-0460 | 749.198 | OH | A02 | a1 | B04 | b2 | C02 | c2 | D02 | d1 | E01 |
| 2-2-d1E01-1-0461 | 749.198 | OH | A02 | a1 | B04 | b2 | C03 | c2 | D02 | d1 | E01 |
| 2-2-d1E01-1-0462 | 750.193 | OH | A02 | a1 | B04 | b2 | C04 | c2 | D02 | d1 | E01 |
| 2-2-d1E01-1-0463 | 750.193 | OH | A02 | a1 | B04 | b2 | C06 | c2 | D02 | d1 | E01 |

(continued)

| ID | Exact Mass | ☒ | A | a | B | b | C | c | D | d | E |
|---|---|---|---|---|---|---|---|---|---|---|---|
| 2-2-d1E01-1-0464 | 750.193 | OH | A02 | a1 | B04 | b2 | C07 | c2 | D02 | d1 | E01 |
| 2-2-d1E01-1-0465 | 752.249 | OH | A01 | a1 | B05 | b4 | C02 | c2 | D02 | d1 | E01 |
| 2-2-d1E01-1-0466 | 752.299 | OH | A02 | a1 | B01 | b1 | C19 | c2 | D02 | d1 | E01 |
| 2-2-d1E01-1-0467 | 754.203 | OH | A02 | a1 | B05 | b3 | C09 | c2 | D02 | d1 | E01 |
| 2-2-d1E01-1-0468 | 754.224 | OH | A02 | a1 | B04 | b1 | C15 | c2 | D02 | d1 | E01 |
| 2-2-d1E01-1-0469 | 755.172 | OH | A01 | a1 | B04 | b3 | C11 | c2 | D02 | d1 | E01 |
| 2-2-d1E01-1-0470 | 755.220 | OH | A02 | a1 | B04 | b1 | C16 | c2 | D02 | d1 | E01 |
| 2-2-d1E01-1-0471 | 756.215 | OH | A02 | a1 | B04 | b1 | C17 | c2 | D02 | d1 | E01 |
| 2-2-d1E01-1-0472 | 756.219 | OH | A02 | a1 | B05 | b1 | C14 | c2 | D02 | d1 | E01 |
| 2-2-d1E01-1-0473 | 756.325 | OH | A02 | a1 | B05 | b1 | C19 | c1 | D01 | d1 | E01 |
| 2-2-d1E01-1-0474 | 757.224 | OH | A02 | a1 | B04 | b4 | C01 | c2 | D02 | d1 | E01 |
| 2-2-d1E01-1-0475 | 758.206 | OH | A01 | a1 | B05 | b4 | C05 | c2 | D02 | d1 | E01 |
| 2-2-d1E01-1-0476 | 759.164 | OH | A02 | a1 | B05 | b3 | C10 | c2 | D02 | d1 | E01 |
| 2-2-d1E01-1-0477 | 760.218 | OH | A02 | a1 | B05 | b2 | C02 | c2 | D02 | d1 | E01 |
| 2-2-d1E01-1-0478 | 760.218 | OH | A02 | a1 | B05 | b2 | C03 | c2 | D02 | d1 | E01 |
| 2-2-d1E01-1-0479 | 761.176 | OH | A02 | a1 | B04 | b1 | C18 | c2 | D02 | d1 | E01 |
| 2-2-d1E01-1-0480 | 761.213 | OH | A02 | a1 | B05 | b2 | C04 | c2 | D02 | d1 | E01 |
| 2-2-d1E01-1-0481 | 761.213 | OH | A02 | a1 | B05 | b2 | C06 | c2 | D02 | d1 | E01 |
| 2-2-d1E01-1-0482 | 761.213 | OH | A02 | a1 | B05 | b2 | C07 | c2 | D02 | d1 | E01 |
| 2-2-d1E01-1-0483 | 765.244 | OH | A02 | a1 | B05 | b1 | C15 | c2 | D02 | d1 | E01 |
| 2-2-d1E01-1-0484 | 766.192 | OH | A01 | a1 | B05 | b3 | C11 | c2 | D02 | d1 | E01 |
| 2-2-d1E01-1-0485 | 766.240 | OH | A02 | a1 | B05 | b1 | C16 | c2 | D02 | d1 | E01 |
| 2-2-d1E01-1-0486 | 766.315 | OH | A02 | a1 | B02 | b1 | C19 | c2 | D02 | d1 | E01 |
| 2-2-d1E01-1-0487 | 767.235 | OH | A02 | a1 | B05 | b1 | C17 | c2 | D02 | d1 | E01 |
| 2-2-d1E01-1-0488 | 767.245 | OH | A02 | a1 | B04 | b4 | C02 | c2 | D02 | d1 | E01 |
| 2-2-d1E01-1-0489 | 768.244 | OH | A02 | a1 | B05 | b4 | C01 | c2 | D02 | d1 | E01 |
| 2-2-d1E01-1-0490 | 770.290 | OH | A02 | a1 | B03 | b1 | C19 | c2 | D02 | d1 | E01 |
| 2-2-d1E01-1-0491 | 772.196 | OH | A02 | a1 | B05 | b1 | C18 | c2 | D02 | d1 | E01 |
| 2-2-d1E01-1-0492 | 773.201 | OH | A02 | a1 | B04 | b4 | C05 | c2 | D02 | d1 | E01 |
| 2-2-d1E01-1-0493 | 778.265 | OH | A02 | a1 | B05 | b4 | C02 | c2 | D02 | d1 | E01 |
| 2-2-d1E01-1-0494 | 781.188 | OH | A02 | a1 | B04 | b3 | C11 | c2 | D02 | d1 | E01 |
| 2-2-d1E01-1-0495 | 783.251 | OH | A01 | a1 | B04 | b1 | C19 | c2 | D02 | d1 | E01 |
| 2-2-d1E01-1-0496 | 784.221 | OH | A02 | a1 | B05 | b4 | C05 | c2 | D02 | d1 | E01 |
| 2-2-d1E01-1-0497 | 792.208 | OH | A02 | a1 | B05 | b3 | C11 | c2 | D02 | d1 | E01 |
| 2-2-d1E01-1-0498 | 794.271 | OH | A01 | a1 | B05 | b1 | C19 | c2 | D02 | d1 | E01 |
| 2-2-d1E01-1-0499 | 809.267 | OH | A02 | a1 | B04 | b1 | C19 | c2 | D02 | d1 | E01 |
| 2-2-d1E01-1-0500 | 820.287 | OH | A02 | a1 | B05 | b1 | C19 | c2 | D02 | d1 | E01 |

The reaction formula for the case where the ethylation reaction is carried out using a mixture 2-2-dlE01-l separately prepared is shown below as an example.

[Formula 123]

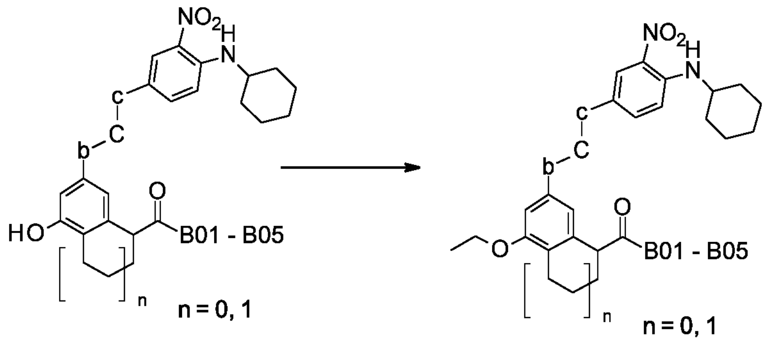

To the mixture 2-2-d1E01-1 (8.75 μmol) placed in a 0.5 to 2 mL microwave container were added DMF (100 μL), EtOH (25.0 μL), BTPP (26.8 μL, 0.088 mmol), and $Et_3PO_4$ (22.3 μL, 0.131 mmol), and the mixture was shaken at 70°C for 27 hours. The reaction solution was transferred to ISOLUTE (R) CBA (200 mg, 0.7 mmol/g) using MeCN (0.2 mL) twice. After 10 minutes, the mixture was washed three times with MeCN (0.5 mL), and the combined filtrate was received in a 15 mm × 75 mm test tube. The solution was distilled off under reduced pressure (using a Genevac reduced pressure concentrator) to afford the mixture 2-2-dlE01-2. Details of the mixture 2-2-dlE01-2 are shown in [Table 6-8-4].

The notation method in the table will be described. In [Table 6-8-4], each compound that may be included in the mixture 2-2-d1E01-2 is shown by ID, and the combination of core blocks and linkers in the structure of the mixture 2-2-dlE01-2 is shown by symbol or chemical formula. The correspondence between symbols and specific structures is illustrated in Figure 3. In [Table 6-8-4], "☒" is represented by a chemical formula.

[Formula 124]

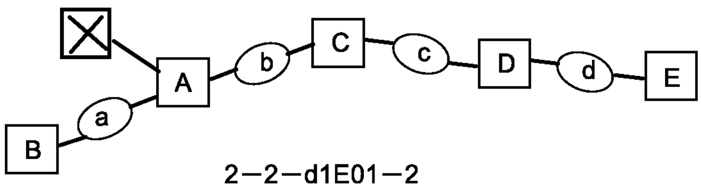


2—2—d1E01—2

For example, if the ID is 2-2-d1E01-2-0001, the following structural formula is shown in [Table 6-8-3].

[Formula 125]

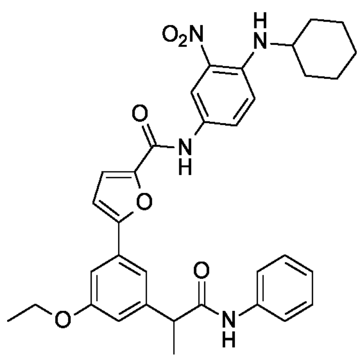

2-2-d1E01-2-0001

[Table 128]

[Table 6-8-3] Notation example of compound structure in ID notation

| ID | Exact Mass | ☒ | A | a | B | b | C | c | D | d | E |
|---|---|---|---|---|---|---|---|---|---|---|---|
| 2-2-d1E01-2-0001 | 596.263 | OEt | A01 | a1 | B01 | b1 | C01 | c1 | D01 | d1 | E01 |

Compounds that may be included in the mixture 2-2-dlE01-2 are shown in [Table 6-8-4].

[Table 129]

[Table 6-8-4] Compounds that may be included in mixture 2-2-dlE01-2

| ID | Exact Mass | ☒ | A | a | B | b | c | c | D | d | E |
|---|---|---|---|---|---|---|---|---|---|---|---|
| 2-2-d1E01-2-0001 | 596.263 | OEt | A01 | a1 | B01 | b1 | C01 | c1 | D01 | d1 | E01 |
| 2-2-d1E01-2-0002 | 600.331 | OEt | A01 | a1 | B01 | b4 | C12 | c1 | D01 | d1 | E01 |
| 2-2-d1E01-2-0003 | 602.310 | OEt | A01 | a1 | B01 | b4 | C13 | c1 | D01 | d1 | E01 |
| 2-2-d1E01-2-0004 | 606.284 | OEt | A01 | a1 | B01 | b1 | C02 | c1 | D01 | d1 | E01 |
| 2-2-d1E01-2-0005 | 606.284 | OEt | A01 | a1 | B01 | b1 | C03 | c1 | D01 | d1 | E01 |
| 2-2-d1E01-2-0006 | 607.279 | OEt | A01 | a1 | B01 | b1 | C04 | c1 | D01 | d1 | E01 |
| 2-2-d1E01-2-0007 | 610.279 | OEt | A01 | a1 | B02 | b1 | C01 | c1 | D01 | d1 | E01 |
| 2-2-d1E01-2-0008 | 612.241 | OEt | A01 | a1 | B01 | b1 | C05 | c1 | D01 | d1 | E01 |
| 2-2-d1E01-2-0009 | 613.254 | OEt | A01 | a1 | B01 | b3 | C08 | c1 | D01 | d1 | E01 |
| 2-2-d1E01-2-0010 | 614.254 | OEt | A01 | a1 | B03 | b1 | C01 | c1 | D01 | d1 | E01 |
| 2-2-d1E01-2-0011 | 614.347 | OEt | A01 | a1 | B02 | b4 | C12 | c1 | D01 | d1 | E01 |
| 2-2-d1E01-2-0012 | 616.326 | OEt | A01 | a1 | B02 | b4 | C13 | c1 | D01 | d1 | E01 |
| 2-2-d1E01-2-0013 | 618.322 | OEt | A01 | a1 | B03 | b4 | C12 | c1 | D01 | d1 | E01 |
| 2-2-d1E01-2-0014 | 620.300 | OEt | A01 | a1 | B02 | b1 | C02 | c1 | D01 | d1 | E01 |
| 2-2-d1E01-2-0015 | 620.300 | OEt | A01 | a1 | B02 | b1 | C03 | c1 | D01 | d1 | E01 |
| 2-2-d1E01-2-0016 | 620.301 | OEt | A01 | a1 | B03 | b4 | C13 | c1 | D01 | d1 | E01 |
| 2-2-d1E01-2-0017 | 621.295 | OEt | A01 | a1 | B02 | b1 | C04 | c1 | D01 | d1 | E01 |
| 2-2-d1E01-2-0018 | 622.279 | OEt | A02 | a1 | B01 | b1 | C01 | c1 | D01 | d1 | E01 |
| 2-2-d1E01-2-0019 | 624.270 | OEt | A01 | a1 | B01 | b3 | C09 | c1 | D01 | d1 | E01 |
| 2-2-d1E01-2-0020 | 624.275 | OEt | A01 | a1 | B03 | b1 | C02 | c1 | D01 | d1 | E01 |
| 2-2-d1E01-2-0021 | 624.275 | OEt | A01 | a1 | B03 | b1 | C03 | c1 | D01 | d1 | E01 |
| 2-2-d1E01-2-0022 | 625.270 | OEt | A01 | a1 | B03 | b1 | C04 | c1 | D01 | d1 | E01 |
| 2-2-d1E01-2-0023 | 626.256 | OEt | A01 | a1 | B02 | b1 | C05 | c1 | D01 | d1 | E01 |
| 2-2-d1E01-2-0024 | 626.285 | OEt | A01 | a1 | B01 | b1 | C14 | c1 | D01 | d1 | E01 |
| 2-2-d1E01-2-0025 | 626.347 | OEt | A02 | a1 | B01 | b4 | C12 | c1 | D01 | d1 | E01 |
| 2-2-d1E01-2-0026 | 627.269 | OEt | A01 | a1 | B02 | b3 | C08 | c1 | D01 | d1 | E01 |

(continued)

| ID | Exact Mass | ☒ | A | a | B | b | c | c | D | d | E |
|---|---|---|---|---|---|---|---|---|---|---|---|
| 2-2-d1E01-2-0027 | 628.326 | OEt | A02 | a1 | B01 | b4 | C13 | c1 | D01 | d1 | E01 |
| 2-2-d1E01-2-0028 | 629.231 | OEt | A01 | a1 | B01 | b3 | C10 | c1 | D01 | d1 | E01 |
| 2-2-d1E01-2-0029 | 630.231 | OEt | A01 | a1 | B03 | b1 | C05 | c1 | D01 | d1 | E01 |
| 2-2-d1E01-2-0030 | 630.284 | OEt | A01 | a1 | B01 | b2 | C02 | c1 | D01 | d1 | E01 |
| 2-2-d1E01-2-0031 | 630.284 | OEt | A01 | a1 | B01 | b2 | C03 | c1 | D01 | d1 | E01 |
| 2-2-d1E01-2-0032 | 631.244 | OEt | A01 | a1 | B03 | b3 | C08 | c1 | D01 | d1 | E01 |
| 2-2-d1E01-2-0033 | 631.279 | OEt | A01 | a1 | B01 | b2 | C04 | c1 | D01 | d1 | E01 |
| 2-2-d1E01-2-0034 | 631.279 | OEt | A01 | a1 | B01 | b2 | C06 | c1 | D01 | d1 | E01 |
| 2-2-d1E01-2-0035 | 631.279 | OEt | A01 | a1 | B01 | b2 | C07 | c1 | D01 | d1 | E01 |
| 2-2-d1E01-2-0036 | 632.300 | OEt | A02 | a1 | B01 | b1 | C02 | c1 | D01 | d1 | E01 |
| 2-2-d1E01-2-0037 | 632.300 | OEt | A02 | a1 | B01 | b1 | C03 | c1 | D01 | d1 | E01 |
| 2-2-d1E01-2-0038 | 633.295 | OEt | A02 | a1 | B01 | b1 | C04 | c1 | D01 | d1 | E01 |
| 2-2-d1E01-2-0039 | 635.311 | OEt | A01 | a1 | B01 | b1 | C15 | c1 | D01 | d1 | E01 |
| 2-2-d1E01-2-0040 | 636.295 | OEt | A02 | a1 | B02 | b1 | C01 | c1 | D01 | d1 | E01 |
| 2-2-d1E01-2-0041 | 636.306 | OEt | A01 | a1 | B01 | b1 | C16 | c1 | D01 | d1 | E01 |
| 2-2-d1E01-2-0042 | 637.301 | OEt | A01 | a1 | B01 | b1 | C17 | c1 | D01 | d1 | E01 |
| 2-2-d1E01-2-0043 | 638.256 | OEt | A02 | a1 | B01 | b1 | C05 | c1 | D01 | d1 | E01 |
| 2-2-d1E01-2-0044 | 638.285 | OEt | A01 | a1 | B02 | b3 | C09 | c1 | D01 | d1 | E01 |
| 2-2-d1E01-2-0045 | 638.310 | OEt | A01 | a1 | B01 | b4 | C01 | c1 | D01 | d1 | E01 |
| 2-2-d1E01-2-0046 | 639.269 | OEt | A02 | a1 | B01 | b3 | C08 | c1 | D01 | d1 | E01 |
| 2-2-d1E01-2-0047 | 640.270 | OEt | A02 | a1 | B03 | b1 | C01 | c1 | D01 | d1 | E01 |
| 2-2-d1E01-2-0048 | 640.301 | OEt | A01 | a1 | B02 | b1 | C14 | c1 | D01 | d1 | E01 |
| 2-2-d1E01-2-0049 | 640.362 | OEt | A02 | a1 | B02 | b4 | C12 | c1 | D01 | d1 | E01 |
| 2-2-d1E01-2-0050 | 642.260 | OEt | A01 | a1 | B03 | b3 | C09 | c1 | D01 | d1 | E01 |
| 2-2-d1E01-2-0051 | 642.262 | OEt | A01 | a1 | B01 | b1 | C18 | c1 | D01 | d1 | E01 |
| 2-2-d1E01-2-0052 | 642.342 | OEt | A02 | a1 | B02 | b4 | C13 | c1 | D01 | d1 | E01 |
| 2-2-d1E01-2-0053 | 643.246 | OEt | A01 | a1 | B02 | b3 | C10 | c1 | D01 | d1 | E01 |
| 2-2-d1E01-2-0054 | 644.276 | OEt | A01 | a1 | B03 | b1 | C14 | c1 | D01 | d1 | E01 |
| 2-2-d1E01-2-0055 | 644.300 | OEt | A01 | a1 | B02 | b2 | C02 | c1 | D01 | d1 | E01 |
| 2-2-d1E01-2-0056 | 644.300 | OEt | A01 | a1 | B02 | b2 | C03 | c1 | D01 | d1 | E01 |
| 2-2-d1E01-2-0057 | 644.337 | OEt | A02 | a1 | B03 | b4 | C12 | c1 | D01 | d1 | E01 |
| 2-2-d1E01-2-0058 | 645.295 | OEt | A01 | a1 | B02 | b2 | C04 | c1 | D01 | d1 | E01 |
| 2-2-d1E01-2-0059 | 645.295 | OEt | A01 | a1 | B02 | b2 | C06 | c1 | D01 | d1 | E01 |
| 2-2-d1E01-2-0060 | 645.295 | OEt | A01 | a1 | B02 | b2 | C07 | c1 | D01 | d1 | E01 |
| 2-2-d1E01-2-0061 | 646.316 | OEt | A02 | a1 | B02 | b1 | C02 | c1 | D01 | d1 | E01 |
| 2-2-d1E01-2-0062 | 646.316 | OEt | A02 | a1 | B02 | b1 | C03 | c1 | D01 | d1 | E01 |
| 2-2-d1E01-2-0063 | 646.317 | OEt | A02 | a1 | B03 | b4 | C13 | c1 | D01 | d1 | E01 |
| 2-2-d1E01-2-0064 | 647.221 | OEt | A01 | a1 | B03 | b3 | C10 | c1 | D01 | d1 | E01 |

(continued)

| ID | Exact Mass | ⊠ | A | a | B | b | c | c | D | d | E |
|---|---|---|---|---|---|---|---|---|---|---|---|
| 2-2-d1E01-2-0065 | 647.311 | OEt | A02 | a1 | B02 | b1 | C04 | c1 | D01 | d1 | E01 |
| 2-2-d1E01-2-0066 | 648.275 | OEt | A01 | a1 | B03 | b2 | C02 | c1 | D01 | d1 | E01 |
| 2-2-d1E01-2-0067 | 648.275 | OEt | A01 | a1 | B03 | b2 | C03 | c1 | D01 | d1 | E01 |
| 2-2-d1E01-2-0068 | 648.331 | OEt | A01 | a1 | B01 | b4 | C02 | c1 | D01 | d1 | E01 |
| 2-2-d1E01-2-0069 | 649.270 | OEt | A01 | a1 | B03 | b2 | C04 | c1 | D01 | d1 | E01 |
| 2-2-d1E01-2-0070 | 649.270 | OEt | A01 | a1 | B03 | b2 | C06 | c1 | D01 | d1 | E01 |
| 2-2-d1E01-2-0071 | 649.270 | OEt | A01 | a1 | B03 | b2 | C07 | c1 | D01 | d1 | E01 |
| 2-2-d1E01-2-0072 | 649.326 | OEt | A01 | a1 | B02 | b1 | C15 | c1 | D01 | d1 | E01 |
| 2-2-d1E01-2-0073 | 650.285 | OEt | A02 | a1 | B01 | b3 | C09 | c1 | D01 | d1 | E01 |
| 2-2-d1E01-2-0074 | 650.290 | OEt | A02 | a1 | B03 | b1 | C02 | c1 | D01 | d1 | E01 |
| 2-2-d1E01-2-0075 | 650.290 | OEt | A02 | a1 | B03 | b1 | C03 | c1 | D01 | d1 | E01 |
| 2-2-d1E01-2-0076 | 650.322 | OEt | A01 | a1 | B02 | b1 | C16 | c1 | D01 | d1 | E01 |
| 2-2-d1E01-2-0077 | 651.286 | OEt | A02 | a1 | B03 | b1 | C04 | c1 | D01 | d1 | E01 |
| 2-2-d1E01-2-0078 | 651.317 | OEt | A01 | a1 | B02 | b1 | C17 | c1 | D01 | d1 | E01 |
| 2-2-d1E01-2-0079 | 652.272 | OEt | A02 | a1 | B02 | b1 | C05 | c1 | D01 | d1 | E01 |
| 2-2-d1E01-2-0080 | 652.301 | OEt | A02 | a1 | B01 | b1 | C14 | c1 | D01 | d1 | E01 |
| 2-2-d1E01-2-0081 | 652.326 | OEt | A01 | a1 | B02 | b4 | C01 | c1 | D01 | d1 | E01 |
| 2-2-d1E01-2-0082 | 653.231 | OEt | A01 | a1 | B04 | b1 | C01 | c1 | D01 | d1 | E01 |
| 2-2-d1E01-2-0083 | 653.285 | OEt | A02 | a1 | B02 | b3 | C08 | c1 | D01 | d1 | E01 |
| 2-2-d1E01-2-0084 | 653.301 | OEt | A01 | a1 | B03 | b1 | C15 | c1 | D01 | d1 | E01 |
| 2-2-d1E01-2-0085 | 654.288 | OEt | A01 | a1 | B01 | b4 | C05 | c1 | D01 | d1 | E01 |
| 2-2-dIE01-2-0086 | 654.297 | OEt | A01 | a1 | B03 | b1 | C16 | c1 | D01 | d1 | E01 |
| 2-2-d1E01-2-0087 | 655.246 | OEt | A02 | a1 | B01 | b3 | C10 | c1 | D01 | d1 | E01 |
| 2-2-d1E01-2-0088 | 655.292 | OEt | A01 | a1 | B03 | b1 | C17 | c1 | D01 | d1 | E01 |
| 2-2-d1E01-2-0089 | 656.247 | OEt | A02 | a1 | B03 | b1 | C05 | c1 | D01 | d1 | E01 |
| 2-2-d1E01-2-0090 | 656.278 | OEt | A01 | a1 | B02 | b1 | C18 | c1 | D01 | d1 | E01 |
| 2-2-d1E01-2-0091 | 656.300 | OEt | A02 | a1 | B01 | b2 | C02 | c1 | D01 | d1 | E01 |
| 2-2-d1E01-2-0092 | 656.300 | OEt | A02 | a1 | B01 | b2 | C03 | c1 | D01 | d1 | E01 |
| 2-2-d1E01-2-0093 | 656.301 | OEt | A01 | a1 | B03 | b4 | C01 | c1 | D01 | d1 | E01 |
| 2-2-d1E01-2-0094 | 657.260 | OEt | A02 | a1 | B03 | b3 | C08 | c1 | D01 | d1 | E01 |
| 2-2-d1E01-2-0095 | 657.295 | OEt | A02 | a1 | B01 | b2 | C04 | c1 | D01 | d1 | E01 |
| 2-2-d1E01-2-0096 | 657.295 | OEt | A02 | a1 | B01 | b2 | C06 | c1 | D01 | d1 | E01 |
| 2-2-d1E01-2-0097 | 657.295 | OEt | A02 | a1 | B01 | b2 | C07 | c1 | D01 | d1 | E01 |
| 2-2-d1E01-2-0098 | 657.298 | OEt | A01 | a1 | B04 | b4 | C12 | c1 | D01 | d1 | E01 |
| 2-2-d1E01-2-0099 | 659.278 | OEt | A01 | a1 | B04 | b4 | C13 | c1 | D01 | d1 | E01 |
| 2-2-d1E01-2-0100 | 660.225 | OEt | A01 | a1 | B01 | b1 | C01 | c2 | D02 | d1 | E01 |
| 2-2-d1E01-2-0101 | 660.253 | OEt | A01 | a1 | B03 | b1 | C18 | c1 | D01 | d1 | E01 |
| 2-2-d1E01-2-0102 | 661.326 | OEt | A02 | a1 | B01 | b1 | C15 | c1 | D01 | d1 | E01 |

(continued)

| ID | Exact Mass | ⊠ | A | a | B | b | c | c | D | d | E |
|---|---|---|---|---|---|---|---|---|---|---|---|
| 2-2-d1E01-2-0103 | 662.274 | OEt | A01 | a1 | B01 | b3 | C11 | c1 | D01 | d1 | E01 |
| 2-2-d1E01-2-0104 | 662.322 | OEt | A02 | a1 | B01 | b1 | C16 | c1 | D01 | d1 | E01 |
| 2-2-d1E01-2-0105 | 662.347 | OEt | A01 | a1 | B02 | b4 | C02 | c1 | D01 | d1 | E01 |
| 2-2-d1E01-2-0106 | 663.252 | OEt | A01 | a1 | B04 | b1 | C02 | c1 | D01 | d1 | E01 |
| 2-2-d1E01-2-0107 | 663.252 | OEt | A01 | a1 | B04 | b1 | C03 | c1 | D01 | d1 | E01 |
| 2-2-d1E01-2-0108 | 663.317 | OEt | A02 | a1 | B01 | b1 | C17 | c1 | D01 | d1 | E01 |
| 2-2-d1E01-2-0109 | 664.247 | OEt | A01 | a1 | B04 | b1 | C04 | c1 | D01 | d1 | E01 |
| 2-2-d1E01-2-0110 | 664.251 | OEt | A01 | a1 | B05 | b1 | C01 | c1 | D01 | d1 | E01 |
| 2-2-d1E01-2-0111 | 664.293 | OEt | A01 | a1 | B01 | b4 | C12 | c2 | D02 | d1 | E01 |
| 2-2-d1E01-2-0112 | 664.301 | OEt | A02 | a1 | B02 | b3 | C09 | c1 | D01 | d1 | E01 |
| 2-2-d1E01-2-0113 | 664.326 | OEt | A02 | a1 | B01 | b4 | C01 | c1 | D01 | d1 | E01 |
| 2-2-d1E01-2-0114 | 666.272 | OEt | A01 | a1 | B01 | b4 | C13 | c2 | D02 | d1 | E01 |
| 2-2-d1E01-2-0115 | 666.317 | OEt | A02 | a1 | B02 | b1 | C14 | c1 | D01 | d1 | E01 |
| 2-2-d1E01-2-0116 | 666.322 | OEt | A01 | a1 | B03 | b4 | C02 | c1 | D01 | d1 | E01 |
| 2-2-d1E01-2-0117 | 668.276 | OEt | A02 | a1 | B03 | b3 | C09 | c1 | D01 | d1 | E01 |
| 2-2-d1E01-2-0118 | 668.278 | OEt | A02 | a1 | B01 | b1 | C18 | c1 | D01 | d1 | E01 |
| 2-2-d1E01-2-0119 | 668.303 | OEt | A01 | a1 | B02 | b4 | C05 | c1 | D01 | d1 | E01 |
| 2-2-d1E01-2-0120 | 668.319 | OEt | A01 | a1 | B05 | b4 | C12 | c1 | D01 | d1 | E01 |
| 2-2-d1E01-2-0121 | 669.208 | OEt | A01 | a1 | B04 | b1 | C05 | c1 | D01 | d1 | E01 |
| 2-2-d1E01-2-0122 | 669.262 | OEt | A02 | a1 | B02 | b3 | C10 | c1 | D01 | d1 | E01 |
| 2-2-d1E01-2-0123 | 670.221 | OEt | A01 | a1 | B04 | b3 | C08 | c1 | D01 | d1 | E01 |
| 2-2-d1E01-2-0124 | 670.246 | OEt | A01 | a1 | B01 | b1 | C02 | c2 | D02 | d1 | E01 |
| 2-2-d1E01-2-0125 | 670.246 | OEt | A01 | a1 | B01 | b1 | C03 | c2 | D02 | d1 | E01 |
| 2-2-d1E01-2-0126 | 670.292 | OEt | A02 | a1 | B03 | b1 | C14 | c1 | D01 | d1 | E01 |
| 2-2-d1E01-2-0127 | 670.298 | OEt | A01 | a1 | B05 | b4 | C13 | c1 | D01 | d1 | E01 |
| 2-2-d1E01-2-0128 | 670.316 | OEt | A02 | a1 | B02 | b2 | C02 | c1 | D01 | d1 | E01 |
| 2-2-d1E01-2-0129 | 670.316 | OEt | A02 | a1 | B02 | b2 | C03 | c1 | D01 | d1 | E01 |
| 2-2-d1E01-2-0130 | 671.241 | OEt | A01 | a1 | B01 | b1 | C04 | c2 | D02 | d1 | E01 |
| 2-2-d1E01-2-0131 | 671.311 | OEt | A02 | a1 | B02 | b2 | C04 | c1 | D01 | d1 | E01 |
| 2-2-d1E01-2-0132 | 671.311 | OEt | A02 | a1 | B02 | b2 | C06 | c1 | D01 | d1 | E01 |
| 2-2-d1E01-2-0133 | 671.311 | OEt | A02 | a1 | B02 | b2 | C07 | c1 | D01 | d1 | E01 |
| 2-2-d1E01-2-0134 | 672.278 | OEt | A01 | a1 | B03 | b4 | C05 | c1 | D01 | d1 | E01 |
| 2-2-d1E01-2-0135 | 673.237 | OEt | A02 | a1 | B03 | b3 | C10 | c1 | D01 | d1 | E01 |
| 2-2-d1E01-2-0136 | 674.241 | OEt | A01 | a1 | B02 | b1 | C01 | c2 | D02 | d1 | E01 |
| 2-2-d1E01-2-0137 | 674.272 | OEt | A01 | a1 | B05 | b1 | C02 | c1 | D01 | d1 | E01 |
| 2-2-d1E01-2-0138 | 674.272 | OEt | A01 | a1 | B05 | b1 | C03 | c1 | D01 | d1 | E01 |
| 2-2-d1E01-2-0139 | 674.290 | OEt | A02 | a1 | B03 | b2 | C02 | c1 | D01 | d1 | E01 |
| 2-2-d1E01-2-0140 | 674.290 | OEt | A02 | a1 | B03 | b2 | C03 | c1 | D01 | d1 | E01 |

(continued)

| ID | Exact Mass | ⊠ | A | a | B | b | c | c | D | d | E |
|---|---|---|---|---|---|---|---|---|---|---|---|
| 2-2-d1E01-2-0141 | 674.347 | OEt | A02 | a1 | B01 | b4 | C02 | c1 | D01 | d1 | E01 |
| 2-2-d1E01-2-0142 | 675.267 | OEt | A01 | a1 | B05 | b1 | C04 | c1 | D01 | d1 | E01 |
| 2-2-d1E01-2-0143 | 675.286 | OEt | A02 | a1 | B03 | b2 | C04 | c1 | D01 | d1 | E01 |
| 2-2-d1E01-2-0144 | 675.286 | OEt | A02 | a1 | B03 | b2 | C06 | c1 | D01 | d1 | E01 |
| 2-2-d1E01-2-0145 | 675.286 | OEt | A02 | a1 | B03 | b2 | C07 | c1 | D01 | d1 | E01 |
| 2-2-d1E01-2-0146 | 675.342 | OEt | A02 | a1 | B02 | b1 | C15 | c1 | D01 | d1 | E01 |
| 2-2-d1E01-2-0147 | 676.203 | OEt | A01 | a1 | B01 | b1 | C05 | c2 | D02 | d1 | E01 |
| 2-2-d1E01-2-0148 | 676.290 | OEt | A01 | a1 | B02 | b3 | C11 | c1 | D01 | d1 | E01 |
| 2-2-d1E01-2-0149 | 676.337 | OEt | A02 | a1 | B02 | b1 | C16 | c1 | D01 | d1 | E01 |
| 2-2-d1E01-2-0150 | 677.216 | OEt | A01 | a1 | B01 | b3 | C08 | c2 | D02 | d1 | E01 |
| 2-2-d1E01-2-0151 | 677.333 | OEt | A02 | a1 | B02 | b1 | C17 | c1 | D01 | d1 | E01 |
| 2-2-d1E01-2-0152 | 678.216 | OEt | A01 | a1 | B03 | b1 | C01 | c2 | D02 | d1 | E01 |
| 2-2-d1E01-2-0153 | 678.309 | OEt | A01 | a1 | B02 | b4 | C12 | c2 | D02 | d1 | E01 |
| 2-2-d1E01-2-0154 | 678.342 | OEt | A02 | a1 | B02 | b4 | C01 | c1 | D01 | d1 | E01 |
| 2-2-d1E01-2-0155 | 679.246 | OEt | A02 | a1 | B04 | b1 | C01 | c1 | D01 | d1 | E01 |
| 2-2-d1E01-2-0156 | 679.317 | OEt | A02 | a1 | B03 | b1 | C15 | c1 | D01 | d1 | E01 |
| 2-2-d1E01-2-0157 | 680.228 | OEt | A01 | a1 | B05 | b1 | C05 | c1 | D01 | d1 | E01 |
| 2-2-d1E01-2-0158 | 680.265 | OEt | A01 | a1 | B03 | b3 | C11 | c1 | D01 | d1 | E01 |
| 2-2-d1E01-2-0159 | 680.288 | OEt | A01 | a1 | B02 | b4 | C13 | c2 | D02 | d1 | E01 |
| 2-2-d1E01-2-0160 | 680.303 | OEt | A02 | a1 | B01 | b4 | C05 | c1 | D01 | d1 | E01 |
| 2-2-d1E01-2-0161 | 680.312 | OEt | A02 | a1 | B03 | b1 | C16 | c1 | D01 | d1 | E01 |
| 2-2-d1E01-2-0162 | 681.237 | OEt | A01 | a1 | B04 | b3 | C09 | c1 | D01 | d1 | E01 |
| 2-2-d1E01-2-0163 | 681.241 | OEt | A01 | a1 | B05 | b3 | C08 | c1 | D01 | d1 | E01 |
| 2-2-d1E01-2-0164 | 681.307 | OEt | A02 | a1 | B03 | b1 | C17 | c1 | D01 | d1 | E01 |
| 2-2-d1E01-2-0165 | 682.284 | OEt | A01 | a1 | B03 | b4 | C12 | c2 | D02 | d1 | E01 |
| 2-2-d1E01-2-0166 | 682.294 | OEt | A02 | a1 | B02 | b1 | C18 | c1 | D01 | d1 | E01 |
| 2-2-d1E01-2-0167 | 682.317 | OEt | A02 | a1 | B03 | b4 | C01 | c1 | D01 | d1 | E01 |
| 2-2-d1E01-2-0168 | 683.253 | OEt | A01 | a1 | B04 | b1 | C14 | c1 | D01 | d1 | E01 |
| 2-2-d1E01-2-0169 | 683.314 | OEt | A02 | a1 | B04 | b4 | C12 | c1 | D01 | d1 | E01 |
| 2-2-d1E01-2-0170 | 684.262 | OEt | A01 | a1 | B02 | b1 | C02 | c2 | D02 | d1 | E01 |
| 2-2-d1E01-2-0171 | 684.262 | OEt | A01 | a1 | B02 | b1 | C03 | c2 | D02 | d1 | E01 |
| 2-2-d1E01-2-0172 | 684.263 | OEt | A01 | a1 | B03 | b4 | C13 | c2 | D02 | d1 | E01 |
| 2-2-d1E01-2-0173 | 685.257 | OEt | A01 | a1 | B02 | b1 | C04 | c2 | D02 | d1 | E01 |
| 2-2-d1E01-2-0174 | 685.293 | OEt | A02 | a1 | B04 | b4 | C13 | c1 | D01 | d1 | E01 |
| 2-2-d1E01-2-0175 | 686.198 | OEt | A01 | a1 | B04 | b3 | C10 | c1 | D01 | d1 | E01 |
| 2-2-d1E01-2-0176 | 686.241 | OEt | A02 | a1 | B01 | b1 | C01 | c2 | D02 | d1 | E01 |
| 2-2-d1E01-2-0177 | 686.269 | OEt | A02 | a1 | B03 | b1 | C18 | c1 | D01 | d1 | E01 |
| 2-2-d1E01-2-0178 | 687.252 | OEt | A01 | a1 | B04 | b2 | C02 | c1 | D01 | d1 | E01 |

(continued)

| ID | Exact Mass | ☒ | A | a | B | b | c | c | D | d | E |
|---|---|---|---|---|---|---|---|---|---|---|---|
| 2-2-d1E01-2-0179 | 687.252 | OEt | A01 | a1 | B04 | b2 | C03 | c1 | D01 | d1 | E01 |
| 2-2-d1E01-2-0180 | 688.232 | OEt | A01 | a1 | B01 | b3 | C09 | c2 | D02 | d1 | E01 |
| 2-2-d1E01-2-0181 | 688.237 | OEt | A01 | a1 | B03 | b1 | C02 | c2 | D02 | d1 | E01 |
| 2-2-d1E01-2-0182 | 688.237 | OEt | A01 | a1 | B03 | b1 | C03 | c2 | D02 | d1 | E01 |
| 2-2-d1E01-2-0183 | 688.247 | OEt | A01 | a1 | B04 | b2 | C04 | c1 | D01 | d1 | E01 |
| 2-2-d1E01-2-0184 | 688.247 | OEt | A01 | a1 | B04 | b2 | C06 | c1 | D01 | d1 | E01 |
| 2-2-d1E01-2-0185 | 688.247 | OEt | A01 | a1 | B04 | b2 | C07 | c1 | D01 | d1 | E01 |
| 2-2-d1E01-2-0186 | 688.290 | OEt | A02 | a1 | B01 | b3 | C11 | c1 | D01 | d1 | E01 |
| 2-2-d1E01-2-0187 | 688.362 | OEt | A02 | a1 | B02 | b4 | C02 | c1 | D01 | d1 | E01 |
| 2-2-d1E01-2-0188 | 689.232 | OEt | A01 | a1 | B03 | b1 | C04 | c2 | D02 | d1 | E01 |
| 2-2-d1E01-2-0189 | 689.267 | OEt | A02 | a1 | B04 | b1 | C02 | c1 | D01 | d1 | E01 |
| 2-2-d1E01-2-0190 | 689.267 | OEt | A02 | a1 | B04 | b1 | C03 | c1 | D01 | d1 | E01 |
| 2-2-d1E01-2-0191 | 690.218 | OEt | A01 | a1 | B02 | b1 | C05 | c2 | D02 | d1 | E01 |
| 2-2-d1E01-2-0192 | 690.247 | OEt | A01 | a1 | B01 | b1 | C14 | c2 | D02 | d1 | E01 |
| 2-2-d1E01-2-0193 | 690.262 | OEt | A02 | a1 | B04 | b1 | C04 | c1 | D01 | d1 | E01 |
| 2-2-d1E01-2-0194 | 690.267 | OEt | A02 | a1 | B05 | b1 | C01 | c1 | D01 | d1 | E01 |
| 2-2-d1E01-2-0195 | 690.309 | OEt | A02 | a1 | B01 | b4 | C12 | c2 | D02 | d1 | E01 |
| 2-2-d1E01-2-0196 | 690.353 | OEt | A01 | a1 | B01 | b1 | C19 | c1 | D01 | d1 | E01 |
| 2-2-d1E01-2-0197 | 691.231 | OEt | A01 | a1 | B02 | b3 | C08 | c2 | D02 | d1 | E01 |
| 2-2-d1E01-2-0198 | 692.257 | OEt | A01 | a1 | B05 | b3 | C09 | c1 | D01 | d1 | E01 |
| 2-2-d1E01-2-0199 | 692.278 | OEt | A01 | a1 | B04 | b1 | C15 | c1 | D01 | d1 | E01 |
| 2-2-d1E01-2-0200 | 692.288 | OEt | A02 | a1 | B01 | b4 | C13 | c2 | D02 | d1 | E01 |
| 2-2-d1E01-2-0201 | 692.337 | OEt | A02 | a1 | B03 | b4 | C02 | c1 | D01 | d1 | E01 |
| 2-2-d1E01-2-0202 | 693.193 | OEt | A01 | a1 | B01 | b3 | C10 | c2 | D02 | d1 | E01 |
| 2-2-d1E01-2-0203 | 693.273 | OEt | A01 | a1 | B04 | b1 | C16 | c1 | D01 | d1 | E01 |
| 2-2-d1E01-2-0204 | 694.193 | OEt | A01 | a1 | B03 | b1 | C05 | c2 | D02 | d1 | E01 |
| 2-2-d1E01-2-0205 | 694.246 | OEt | A01 | a1 | B01 | b2 | C02 | c2 | D02 | d1 | E01 |
| 2-2-d1E01-2-0206 | 694.246 | OEt | A01 | a1 | B01 | b2 | C03 | c2 | D02 | d1 | E01 |
| 2-2-d1E01-2-0207 | 694.269 | OEt | A01 | a1 | B04 | b1 | C17 | c1 | D01 | d1 | E01 |
| 2-2-d1E01-2-0208 | 694.273 | OEt | A01 | a1 | B05 | b1 | C14 | c1 | D01 | d1 | E01 |
| 2-2-d1E01-2-0209 | 694.319 | OEt | A02 | a1 | B02 | b4 | C05 | c1 | D01 | d1 | E01 |
| 2-2-d1E01-2-0210 | 694.334 | OEt | A02 | a1 | B05 | b4 | C12 | c1 | D01 | d1 | E01 |
| 2-2-d1E01-2-0211 | 695.206 | OEt | A01 | a1 | B03 | b3 | C08 | c2 | D02 | d1 | E01 |
| 2-2-d1E01-2-0212 | 695.224 | OEt | A02 | a1 | B04 | b1 | C05 | c1 | D01 | d1 | E01 |
| 2-2-d1E01-2-0213 | 695.241 | OEt | A01 | a1 | B01 | b2 | C04 | c2 | D02 | d1 | E01 |
| 2-2-d1E01-2-0214 | 695.241 | OEt | A01 | a1 | B01 | b2 | C06 | c2 | D02 | d1 | E01 |
| 2-2-d1E01-2-0215 | 695.241 | OEt | A01 | a1 | B01 | b2 | C07 | c2 | D02 | d1 | E01 |
| 2-2-d1E01-2-0216 | 695.278 | OEt | A01 | a1 | B04 | b4 | C01 | c1 | D01 | d1 | E01 |

(continued)

| ID | Exact Mass | ☒ | A | a | B | b | c | c | D | d | E |
|---|---|---|---|---|---|---|---|---|---|---|---|
| 2-2-d1E01-2-0217 | 696.237 | OEt | A02 | a1 | B04 | b3 | C08 | c1 | D01 | d1 | E01 |
| 2-2-d1E01-2-0218 | 696.262 | OEt | A02 | a1 | B01 | b1 | C02 | c2 | D02 | d1 | E01 |
| 2-2-d1E01-2-0219 | 696.262 | OEt | A02 | a1 | B01 | b1 | C03 | c2 | D02 | d1 | E01 |
| 2-2-d1E01-2-0220 | 696.313 | OEt | A02 | a1 | B05 | b4 | C13 | c1 | D01 | \d1 | E01 |
| 2-2-d1E01-2-0221 | 697.218 | OEt | A01 | a1 | B05 | b3 | C10 | c1 | D01 | d1 | E01 |
| 2-2-d1E01-2-0222 | 697.257 | OEt | A02 | a1 | B01 | b1 | C04 | c2 | D02 | d1 | E01 |
| 2-2-d1E01-2-0223 | 698.272 | OEt | A01 | a1 | B05 | b2 | C02 | c1 | D01 | d1 | E01 |
| 2-2-d1E01-2-0224 | 698.272 | OEt | A01 | a1 | B05 | b2 | C03 | c1 | D01 | d1 | E01 |
| 2-2-d1E01-2-0225 | 698.294 | OEt | A02 | a1 | B03 | b4 | C05 | c1 | D01 | d1 | E01 |
| 2-2-d1E01-2-0226 | 699.230 | OEt | A01 | a1 | B04 | b1 | C18 | c1 | D01 | d1 | E01 |
| 2-2-d1E01-2-0227 | 699.267 | OEt | A01 | a1 | B05 | b2 | C04 | c1 | D01 | d1 | E01 |
| 2-2-d1E01-2-0228 | 699.267 | OEt | A01 | a1 | B05 | b2 | C06 | c1 | D01 | d1 | E01 |
| 2-2-d1E01-2-0229 | 699.267 | OEt | A01 | a1 | B05 | b2 | C07 | c1 | D01 | d1 | E01 |
| 2-2-d1E01-2-0230 | 699.273 | OEt | A01 | a1 | B01 | b1 | C15 | c2 | D02 | d1 | E01 |
| 2-2-d1E01-2-0231 | 700.257 | OEt | A02 | a1 | B02 | b1 | C01 | c2 | D02 | d1 | E01 |
| 2-2-d1E01-2-0232 | 700.268 | OEt | A01 | a1 | B01 | b1 | C16 | c2 | D02 | d1 | E01 |
| 2-2-d1E01-2-0233 | 700.287 | OEt | A02 | a1 | B05 | b1 | C02 | c1 | D01 | d1 | E01 |
| 2-2-d1E01-2-0234 | 700.287 | OEt | A02 | a1 | B05 | b1 | C03 | c1 | D01 | d1 | E01 |
| 2-2-d1E01-2-0235 | 701.263 | OEt | A01 | a1 | B01 | b1 | C17 | c2 | D02 | d1 | E01 |
| 2-2-d1E01-2-0236 | 701.283 | OEt | A02 | a1 | B05 | b1 | C04 | c1 | D01 | d1 | E01 |
| 2-2-d1E01-2-0237 | 702.218 | OEt | A02 | a1 | B01 | b1 | C05 | c2 | D02 | d1 | E01 |
| 2-2-d1E01-2-0238 | 702.247 | OEt | A01 | a1 | B02 | b3 | C09 | c2 | D02 | d1 | E01 |
| 2-2-d1E01-2-0239 | 702.272 | OEt | A01 | a1 | B01 | b4 | C01 | c2 | D02 | d1 | E01 |
| 2-2-d1E01-2-0240 | 702.305 | OEt | A02 | a1 | B02 | b3 | C11 | c1 | D01 | d1 | E01 |
| 2-2-d1E01-2-0241 | 703.231 | OEt | A02 | a1 | B01 | b3 | C08 | c2 | D02 | d1 | E01 |
| 2-2-d1E01-2-0242 | 703.298 | OEt | A01 | a1 | B05 | b1 | C15 | c1 | D01 | d1 | E01 |
| 2-2-d1E01-2-0243 | 704.232 | OEt | A02 | a1 | B03 | b1 | C01 | c2 | D02 | d1 | E01 |
| 2-2-d1E01-2-0244 | 704.263 | OEt | A01 | a1 | B02 | b1 | C14 | c2 | D02 | d1 | E01 |
| 2-2-d1E01-2-0245 | 704.293 | OEt | A01 | a1 | B05 | b1 | C16 | c1 | D01 | d1 | E01 |
| 2-2-d1E01-2-0246 | 704.324 | OEt | A02 | a1 | B02 | b4 | C12 | c2 | D02 | d1 | E01 |
| 2-2-d1E01-2-0247 | 704.369 | OEt | A01 | a1 | B02 | b1 | C19 | c1 | D01 | d1 | E01 |
| 2-2-d1E01-2-0248 | 705.289 | OEt | A01 | a1 | B05 | b1 | C17 | c1 | D01 | d1 | E01 |
| 2-2-d1E01-2-0249 | 705.298 | OEt | A01 | a1 | B04 | b4 | C02 | c1 | D01 | d1 | E01 |
| 2-2-d1E01-2-0250 | 706.222 | OEt | A01 | a1 | B03 | b3 | C09 | c2 | D02 | d1 | E01 |
| 2-2-d1E01-2-0251 | 706.224 | OEt | A01 | a1 | B01 | b1 | C18 | c2 | D02 | d1 | E01 |
| 2-2-d1E01-2-0252 | 706.244 | OEt | A02 | a1 | B05 | b1 | C05 | c1 | D01 | d1 | E01 |
| 2-2-d1E01-2-0253 | 706.280 | OEt | A02 | a1 | B03 | b3 | C11 | c1 | D01 | d1 | E01 |
| 2-2-d1E01-2-0254 | 706.298 | OEt | A01 | a1 | B05 | b4 | C01 | c1 | D01 | d1 | E01 |

(continued)

| ID | Exact Mass | ☒ | A | a | B | b | c | c | D | d | E |
|---|---|---|---|---|---|---|---|---|---|---|---|
| 2-2-d1E01-2-0255 | 706.304 | OEt | A02 | a1 | B02 | b4 | C13 | c2 | D02 | d1 | E01 |
| 2-2-d1E01-2-0256 | 707.208 | OEt | A01 | a1 | B02 | b3 | C10 | c2 | D02 | d1 | E01 |
| 2-2-d1E01-2-0257 | 707.253 | OEt | A02 | a1 | B04 | b3 | C09 | c1 | D01 | d1 | E01 |
| 2-2-d1E01-2-0258 | 707.257 | OEt | A02 | a1 | B05 | b3 | C08 | c1 | D01 | d1 | E01 |
| 2-2-d1E01-2-0259 | 708.238 | OEt | A01 | a1 | B03 | b1 | C14 | c2 | D02 | d1 | E01 |
| 2-2-d1E01-2-0260 | 708.262 | OEt | A01 | a1 | B02 | b2 | C02 | c2 | D02 | d1 | E01 |
| 2-2-d1E01-2-0261 | 708.262 | OEt | A01 | a1 | B02 | b2 | C03 | c2 | D02 | d1 | E01 |
| 2-2-d1E01-2-0262 | 708.299 | OEt | A02 | a1 | B03 | b4 | C12 | c2 | D02 | d1 | E01 |
| 2-2-d1E01-2-0263 | 708.344 | OEt | A01 | a1 | B03 | b1 | C19 | c1 | D01 | d1 | E01 |
| 2-2-d1E01-2-0264 | 709.257 | OEt | A01 | a1 | B02 | b2 | C04 | c2 | D02 | d1 | E01 |
| 2-2-d1E01-2-0265 | 709.257 | OEt | A01 | a1 | B02 | b2 | C06 | c2 | D02 | d1 | E01 |
| 2-2-d1E01-2-0266 | 709.257 | OEt | A01 | a1 | B02 | b2 | C07 | c2 | D02 | d1 | E01 |
| 2-2-d1E01-2-0267 | 709.268 | OEt | A02 | a1 | B04 | b1 | C14 | c1 | D01 | d1 | E01 |
| 2-2-d1E01-2-0268 | 710.250 | OEt | A01 | a1 | B05 | b1 | C18 | c1 | D01 | d1 | E01 |
| 2-2-d1E01-2-0269 | 710.277 | OEt | A02 | a1 | B02 | b1 | C02 | c2 | D02 | d1 | E01 |
| 2-2-d1E01-2-0270 | 710.277 | OEt | A02 | a1 | B02 | b1 | C03 | c2 | D02 | d1 | E01 |
| 2-2-d1E01-2-0271 | 710.279 | OEt | A02 | a1 | B03 | b4 | C13 | c2 | D02 | d1 | E01 |
| 2-2-d1E01-2-0272 | 711.183 | OEt | A01 | a1 | B03 | b3 | C10 | c2 | D02 | d1 | E01 |
| 2-2-d1E01-2-0273 | 711.255 | OEt | A01 | a1 | B04 | b4 | C05 | c1 | D01 | d1 | E01 |
| 2-2-d1E01-2-0274 | 711.273 | OEt | A02 | a1 | B02 | b1 | C04 | c2 | D02 | d1 | E01 |
| 2-2-d1E01-2-0275 | 712.214 | OEt | A02 | a1 | B04 | b3 | C10 | c1 | D01 | d1 | E01 |
| 2-2-d1E01-2-0276 | 712.237 | OEt | A01 | a1 | B03 | b2 | C02 | c2 | D02 | d1 | E01 |
| 2-2-d1E01-2-0277 | 712.237 | OEt | A01 | a1 | B03 | b2 | C03 | c2 | D02 | d1 | E01 |
| 2-2-d1E01-2-0278 | 712.293 | OEt | A01 | a1 | B01 | b4 | C02 | c2 | D02 | d1 | E01 |
| 2-2-d1E01-2-0279 | 713.232 | OEt | A01 | a1 | B03 | b2 | C04 | c2 | D02 | d1 | E01 |
| 2-2-d1E01-2-0280 | 713.232 | OEt | A01 | a1 | B03 | b2 | C06 | c2 | D02 | d1 | E01 |
| 2-2-d1E01-2-0281 | 713.232 | OEt | A01 | a1 | B03 | b2 | C07 | c2 | D02 | d1 | E01 |
| 2-2-d1E01-2-0282 | 713.267 | OEt | A02 | a1 | B04 | b2 | C02 | c1 | D01 | d1 | E01 |
| 2-2-d1E01-2-0283 | 713.267 | OEt | A02 | a1 | B04 | b2 | C03 | c1 | D01 | d1 | E01 |
| 2-2-d1E01-2-0284 | 713.288 | OEt | A01 | a1 | B02 | b1 | C15 | c2 | D02 | d1 | E01 |
| 2-2-d1E01-2-0285 | 714.247 | OEt | A02 | a1 | B01 | b3 | C09 | c2 | D02 | d1 | E01 |
| 2-2-d1E01-2-0286 | 714.252 | OEt | A02 | a1 | B03 | b1 | C02 | c2 | D02 | d1 | E01 |
| 2-2-d1E01-2-0287 | 714.252 | OEt | A02 | a1 | B03 | b1 | C03 | c2 | D02 | d1 | E01 |
| 2-2-d1E01-2-0288 | 714.262 | OEt | A02 | a1 | B04 | b2 | C04 | c1 | D01 | d1 | E01 |
| 2-2-d1E01-2-0289 | 714.262 | OEt | A02 | a1 | B04 | b2 | C06 | c1 | D01 | d1 | E01 |
| 2-2-d1E01-2-0290 | 714.262 | OEt | A02 | a1 | B04 | b2 | C07 | c1 | D01 | d1 | E01 |
| 2-2-d1E01-2-0291 | 714.284 | OEt | A01 | a1 | B02 | b1 | C16 | c2 | D02 | d1 | E01 |
| 2-2-d1E01-2-0292 | 715.248 | OEt | A02 | a1 | B03 | b1 | C04 | c2 | D02 | d1 | E01 |

(continued)

| ID | Exact Mass | ☒ | A | a | B | b | c | c | D | d | E |
|---|---|---|---|---|---|---|---|---|---|---|---|
| 2-2-d1E01-2-0293 | 715.279 | OEt | A01 | a1 | B02 | b1 | C17 | c2 | D02 | d1 | E01 |
| 2-2-d1E01-2-0294 | 716.234 | OEt | A02 | a1 | B02 | b1 | C05 | c2 | D02 | d1 | E01 |
| 2-2-d1E01-2-0295 | 716.263 | OEt | A02 | a1 | B01 | b1 | C14 | c2 | D02 | d1 | E01 |
| 2-2-d1E01-2-0296 | 716.288 | OEt | A01 | a1 | B02 | b4 | C01 | c2 | D02 | d1 | E01 |
| 2-2-d1E01-2-0297 | 716.319 | OEt | A01 | a1 | B05 | b4 | C02 | c1 | D01 | d1 | E01 |
| 2-2-d1E01-2-0298 | 716.369 | OEt | A02 | a1 | B01 | b1 | C19 | c1 | D01 | d1 | E01 |
| 2-2-d1E01-2-0299 | 717.193 | OEt | A01 | a1 | B04 | b1 | C01 | c2 | D02 | d1 | E01 |
| 2-2-d1E01-2-0300 | 717.247 | OEt | A02 | a1 | B02 | b3 | C08 | c2 | D02 | d1 | E01 |
| 2-2-d1E01-2-0301 | 717.263 | OEt | A01 | a1 | B03 | b1 | C15 | c2 | D02 | d1 | E01 |
| 2-2-d1E01-2-0302 | 718.249 | OEt | A01 | a1 | B01 | b4 | C05 | c2 | D02 | d1 | E01 |
| 2-2-d1E01-2-0303 | 718.258 | OEt | A01 | a1 | B03 | b1 | C16 | c2 | D02 | d1 | E01 |
| 2-2-d1E01-2-0304 | 718.273 | OEt | A02 | a1 | B05 | b3 | C09 | c1 | D01 | d1 | E01 |
| 2-2-d1E01-2-0305 | 718.294 | OEt | A02 | a1 | B04 | b1 | C15 | c1 | D01 | d1 | E01 |
| 2-2-d1E01-2-0306 | 719.208 | OEt | A02 | a1 | B01 | b3 | C10 | c2 | D02 | d1 | E01 |
| 2-2-d1E01-2-0307 | 719.241 | OEt | A01 | a1 | B04 | b3 | C11 | c1 | D01 | d1 | E01 |
| 2-2-d1E01-2-0308 | 719.254 | OEt | A01 | a1 | B03 | b1 | C17 | c2 | D02 | d1 | E01 |
| 2-2-d1E01-2-0309 | 719.289 | OEt | A02 | a1 | B04 | b1 | C16 | c1 | D01 | d1 | E01 |
| 2-2-d1E01-2-0310 | 720.209 | OEt | A02 | a1 | B03 | b1 | C05 | c2 | D02 | d1 | E01 |
| 2-2-d1E01-2-0311 | 720.240 | OEt | A01 | a1 | B02 | b1 | C18 | c2 | D02 | d1 | E01 |
| 2-2-d1E01-2-0312 | 720.262 | OEt | A02 | a1 | B01 | b2 | C02 | c2 | D02 | d1 | E01 |
| 2-2-d1E01-2-0313 | 720.262 | OEt | A02 | a1 | B01 | b2 | C03 | c2 | D02 | d1 | E01 |
| 2-2-d1E01-2-0314 | 720.263 | OEt | A01 | a1 | B03 | b4 | C01 | c2 | D02 | d1 | E01 |
| 2-2-d1E01-2-0315 | 720.284 | OEt | A02 | a1 | B04 | b1 | C17 | c1 | D01 | d1 | E01 |
| 2-2-d1E01-2-0316 | 720.288 | OEt | A02 | a1 | B05 | b1 | C14 | c1 | D01 | d1 | E01 |
| 2-2-d1E01-2-0317 | 721.222 | OEt | A02 | a1 | B03 | b3 | C08 | c2 | D02 | d1 | E01 |
| 2-2-d1E01-2-0318 | 721.257 | OEt | A02 | a1 | B01 | b2 | C04 | c2 | D02 | d1 | E01 |
| 2-2-d1E01-2-0319 | 721.257 | OEt | A02 | a1 | B01 | b2 | C06 | c2 | D02 | d1 | E01 |
| 2-2-d1E01-2-0320 | 721.257 | OEt | A02 | a1 | B01 | b2 | C07 | c2 | D02 | d1 | E01 |
| 2-2-d1E01-2-0321 | 721.260 | OEt | A01 | a1 | B04 | b4 | C12 | c2 | D02 | d1 | E01 |
| 2-2-d1E01-2-0322 | 721.293 | OEt | A02 | a1 | B04 | b4 | C01 | c1 | D01 | d1 | E01 |
| 2-2-d1E01-2-0323 | 722.275 | OEt | A01 | a1 | B05 | b4 | C05 | c1 | D01 | d1 | E01 |
| 2-2-d1E01-2-0324 | 723.234 | OEt | A02 | a1 | B05 | b3 | C10 | c1 | D01 | d1 | E01 |
| 2-2-d1E01-2-0325 | 723.240 | OEt | A01 | a1 | B04 | b4 | C13 | c2 | D02 | d1 | E01 |
| 2-2-d1E01-2-0326 | 724.215 | OEt | A01 | a1 | B03 | b1 | C18 | c2 | D02 | d1 | E01 |
| 2-2-d1E01-2-0327 | 724.287 | OEt | A02 | a1 | B05 | b2 | C02 | c1 | D01 | d1 | E01 |
| 2-2-d1E01-2-0328 | 724.287 | OEt | A02 | a1 | B05 | b2 | C03 | c1 | D01 | d1 | E01 |
| 2-2-d1E01-2-0329 | 725.245 | OEt | A02 | a1 | B04 | b1 | C18 | c1 | D01 | d1 | E01 |
| 2-2-d1E01-2-0330 | 725.283 | OEt | A02 | a1 | B05 | b2 | C04 | c1 | D01 | d1 | E01 |

(continued)

| ID | Exact Mass | ⊠ | A | a | B | b | c | c | D | d | E |
|---|---|---|---|---|---|---|---|---|---|---|---|
| 2-2-d1E01-2-0331 | 725.283 | OEt | A02 | a1 | B05 | b2 | C06 | c1 | D01 | d1 | E01 |
| 2-2-d1E01-2-0332 | 725.283 | OEt | A02 | a1 | B05 | b2 | C07 | c1 | D01 | d1 | E01 |
| 2-2-d1E01-2-0333 | 725.288 | OEt | A02 | a1 | B01 | b1 | C15 | c2 | D02 | d1 | E01 |
| 2-2-d1E01-2-0334 | 726.236 | OEt | A01 | a1 | B01 | b3 | C11 | c2 | D02 | d1 | E01 |
| 2-2-d1E01-2-0335 | 726.284 | OEt | A02 | a1 | B01 | b1 | C16 | c2 | D02 | d1 | E01 |
| 2-2-d1E01-2-0336 | 726.309 | OEt | A01 | a1 | B02 | b4 | C02 | c2 | D02 | d1 | E01 |
| 2-2-d1E01-2-0337 | 727.213 | OEt | A01 | a1 | B04 | b1 | C02 | c2 | D02 | d1 | E01 |
| 2-2-d1E01-2-0338 | 727.213 | OEt | A01 | a1 | B04 | b1 | C03 | c2 | D02 | d1 | E01 |
| 2-2-d1E01-2-0339 | 727.279 | OEt | A02 | a1 | B01 | b1 | C17 | c2 | D02 | d1 | E01 |
| 2-2-d1E01-2-0340 | 728.209 | OEt | A01 | a1 | B04 | b1 | C04 | c2 | D02 | d1 | E01 |
| 2-2-d1E01-2-0341 | 728.213 | OEt | A01 | a1 | B05 | b1 | C01 | c2 | D02 | d1 | E01 |
| 2-2-d1E01-2-0342 | 728.263 | OEt | A02 | a1 | B02 | b3 | C09 | c2 | D02 | d1 | E01 |
| 2-2-d1E01-2-0343 | 728.288 | OEt | A02 | a1 | B01 | b4 | C01 | c2 | D02 | d1 | E01 |
| 2-2-d1E01-2-0344 | 729.314 | OEt | A02 | a1 | B05 | b1 | C15 | c1 | D01 | d1 | E01 |
| 2-2-d1E01-2-0345 | 730.261 | OEt | A01 | a1 | B05 | b3 | C11 | c1 | D01 | d1 | E01 |
| 2-2-d1E01-2-0346 | 730.278 | OEt | A02 | a1 | B02 | b1 | C14 | c2 | D02 | d1 | E01 |
| 2-2-d1E01-2-0347 | 730.284 | OEt | A01 | a1 | B03 | b4 | C02 | c2 | D02 | d1 | E01 |
| 2-2-d1E01-2-0348 | 730.309 | OEt | A02 | a1 | B05 | b1 | C16 | c1 | D01 | d1 | E01 |
| 2-2-d1E01-2-0349 | 730.384 | OEt | A02 | a1 | B02 | b1 | C19 | c1 | D01 | d1 | E01 |
| 2-2-d1E01-2-0350 | 731.304 | OEt | A02 | a1 | B05 | b1 | C17 | c1 | D01 | d1 | E01 |
| 2-2-d1E01-2-0351 | 731.314 | OEt | A02 | a1 | B04 | b4 | C02 | c1 | D01 | d1 | E01 |
| 2-2-d1E01-2-0352 | 732.238 | OEt | A02 | a1 | B03 | b3 | C09 | c2 | D02 | d1 | E01 |
| 2-2-d1E01-2-0353 | 732.240 | OEt | A02 | a1 | B01 | b1 | C18 | c2 | D02 | d1 | E01 |
| 2-2-d1E01-2-0354 | 732.265 | OEt | A01 | a1 | B02 | b4 | C05 | c2 | D02 | d1 | E01 |
| 2-2-d1E01-2-0355 | 732.280 | OEt | A01 | a1 | B05 | b4 | C12 | c2 | D02 | d1 | E01 |
| 2-2-d1E01-2-0356 | 732.313 | OEt | A02 | a1 | B05 | b4 | C01 | c1 | D01 | d1 | E01 |
| 2-2-d1E01-2-0357 | 733.170 | OEt | A01 | a1 | B04 | b1 | C05 | c2 | D02 | d1 | E01 |
| 2-2-d1E01-2-0358 | 733.224 | OEt | A02 | a1 | B02 | b3 | C10 | c2 | D02 | d1 | E01 |
| 2-2-d1E01-2-0359 | 734.183 | OEt | A01 | a1 | B04 | b3 | C08 | c2 | D02 | d1 | E01 |
| 2-2-d1E01-2-0360 | 734.253 | OEt | A02 | a1 | B03 | b1 | C14 | c2 | D02 | d1 | E01 |
| 2-2-d1E01-2-0361 | 734.260 | OEt | A01 | a1 | B05 | b4 | C13 | c2 | D02 | d1 | E01 |
| 2-2-d1E01-2-0362 | 734.277 | OEt | A02 | a1 | B02 | b2 | C02 | c2 | D02 | d1 | E01 |
| 2-2-d1E01-2-0363 | 734.277 | OEt | A02 | a1 | B02 | b2 | C03 | c2 | D02 | d1 | E01 |
| 2-2-d1E01-2-0364 | 734.359 | OEt | A02 | a1 | B03 | b1 | C19 | c1 | D01 | d1 | E01 |
| 2-2-d1E01-2-0365 | 735.273 | OEt | A02 | a1 | B02 | b2 | C04 | c2 | D02 | d1 | E01 |
| 2-2-d1E01-2-0366 | 735.273 | OEt | A02 | a1 | B02 | b2 | C06 | c2 | D02 | d1 | E01 |
| 2-2-d1E01-2-0367 | 735.273 | OEt | A02 | a1 | B02 | b2 | C07 | c2 | D02 | d1 | E01 |
| 2-2-d1E01-2-0368 | 736.240 | OEt | A01 | a1 | B03 | b4 | C05 | c2 | D02 | d1 | E01 |

(continued)

| ID | Exact Mass | ☒ | A | a | B | b | c | c | D | d | E |
|---|---|---|---|---|---|---|---|---|---|---|---|
| 2-2-d1E01-2-0369 | 736.265 | OEt | A02 | a1 | B05 | b1 | C18 | c1 | D01 | d1 | E01 |
| 2-2-d1E01-2-0370 | 737.199 | OEt | A02 | a1 | B03 | b3 | C10 | c2 | D02 | d1 | E01 |
| 2-2-d1E01-2-0371 | 737.271 | OEt | A02 | a1 | B04 | b4 | C05 | c1 | D01 | d1 | E01 |
| 2-2-d1E01-2-0372 | 738.234 | OEt | A01 | a1 | B05 | b1 | C02 | c2 | D02 | d1 | E01 |
| 2-2-d1E01-2-0373 | 738.234 | OEt | A01 | a1 | B05 | b1 | C03 | c2 | D02 | d1 | E01 |
| 2-2-d1E01-2-0374 | 738.252 | OEt | A02 | a1 | B03 | b2 | C02 | c2 | D02 | d1 | E01 |
| 2-2-d1E01-2-0375 | 738.252 | OEt | A02 | a1 | B03 | b2 | C03 | c2 | D02 | d1 | E01 |
| 2-2-d1E01-2-0376 | 738.309 | OEt | A02 | a1 | B01 | b4 | C02 | c2 | D02 | d1 | E01 |
| 2-2-d1E01-2-0377 | 739.229 | OEt | A01 | a1 | B05 | b1 | C04 | c2 | D02 | d1 | E01 |
| 2-2-d1E01-2-0378 | 739.248 | OEt | A02 | a1 | B03 | b2 | C04 | c2 | D02 | d1 | E01 |
| 2-2-d1E01-2-0379 | 739.248 | OEt | A02 | a1 | B03 | b2 | C06 | c2 | D02 | d1 | E01 |
| 2-2-d1E01-2-0380 | 739.248 | OEt | A02 | a1 | B03 | b2 | C07 | c2 | D02 | d1 | E01 |
| 2-2-d1E01-2-0381 | 739.304 | OEt | A02 | a1 | B02 | b1 | C15 | c2 | D02 | d1 | E01 |
| 2-2-d1E01-2-0382 | 740.252 | OEt | A01 | a1 | B02 | b3 | C11 | c2 | D02 | d1 | E01 |
| 2-2-d1E01-2-0383 | 740.299 | OEt | A02 | a1 | B02 | b1 | C16 | c2 | D02 | d1 | E01 |
| 2-2-d1E01-2-0384 | 741.294 | OEt | A02 | a1 | B02 | b1 | C17 | c2 | D02 | d1 | E01 |
| 2-2-d1E01-2-0385 | 742.304 | OEt | A02 | a1 | B02 | b4 | C01 | c2 | D02 | d1 | E01 |
| 2-2-d1E01-2-0386 | 742.334 | OEt | A02 | a1 | B05 | b4 | C02 | c1 | D01 | d1 | E01 |
| 2-2-d1E01-2-0387 | 743.208 | OEt | A02 | a1 | B04 | b1 | C01 | c2 | D02 | d1 | E01 |
| 2-2-d1E01-2-0388 | 743.279 | OEt | A02 | a1 | B03 | b1 | C15 | c2 | D02 | d1 | E01 |
| 2-2-d1E01-2-0389 | 744.190 | OEt | A01 | a1 | B05 | b1 | C05 | c2 | D02 | d1 | E01 |
| 2-2-d1E01-2-0390 | 744.227 | OEt | A01 | a1 | B03 | b3 | C11 | c2 | D02 | d1 | E01 |
| 2-2-d1E01-2-0391 | 744.265 | OEt | A02 | a1 | B01 | b4 | C05 | c2 | D02 | d1 | E01 |
| 2-2-d1E01-2-0392 | 744.274 | OEt | A02 | a1 | B03 | b1 | C16 | c2 | D02 | d1 | E01 |
| 2-2-d1E01-2-0393 | 745.199 | OEt | A01 | a1 | B04 | b3 | C09 | c2 | D02 | d1 | E01 |
| 2-2-d1E01-2-0394 | 745.203 | OEt | A01 | a1 | B05 | b3 | C08 | c2 | D02 | d1 | E01 |
| 2-2-d1E01-2-0395 | 745.257 | OEt | A02 | a1 | B04 | b3 | C11 | c1 | D01 | d1 | E01 |
| 2-2-d1E01-2-0396 | 745.269 | OEt | A02 | a1 | B03 | b1 | C17 | c2 | D02 | d1 | E01 |
| 2-2-d1E01-2-0397 | 746.256 | OEt | A02 | a1 | B02 | b1 | C18 | c2 | D02 | d1 | E01 |
| 2-2-d1E01-2-0398 | 746.279 | OEt | A02 | a1 | B03 | b4 | C01 | c2 | D02 | d1 | E01 |
| 2-2-d1E01-2-0399 | 747.215 | OEt | A01 | a1 | B04 | b1 | C14 | c2 | D02 | d1 | E01 |
| 2-2-d1E01-2-0400 | 747.276 | OEt | A02 | a1 | B04 | b4 | C12 | c2 | D02 | d1 | E01 |
| 2-2-d1E01-2-0401 | 747.320 | OEt | A01 | a1 | B04 | b1 | C19 | c1 | D01 | d1 | E01 |
| 2-2-d1E01-2-0402 | 748.291 | OEt | A02 | a1 | B05 | b4 | C05 | c1 | D01 | d1 | E01 |
| 2-2-d1E01-2-0403 | 749.255 | OEt | A02 | a1 | B04 | b4 | C13 | c2 | D02 | d1 | E01 |
| 2-2-d1E01-2-0404 | 750.160 | OEt | A01 | a1 | B04 | b3 | C10 | c2 | D02 | d1 | E01 |
| 2-2-d1E01-2-0405 | 750.231 | OEt | A02 | a1 | B03 | b1 | C18 | c2 | D02 | d1 | E01 |
| 2-2-d1E01-2-0406 | 751.213 | OEt | A01 | a1 | B04 | b2 | C02 | c2 | D02 | d1 | E01 |

(continued)

| ID | Exact Mass | ☒ | A | a | B | b | c | c | D | d | E |
|---|---|---|---|---|---|---|---|---|---|---|---|
| 2-2-d1E01-2-0407 | 751.213 | OEt | A01 | a1 | B04 | b2 | C03 | c2 | D02 | d1 | E01 |
| 2-2-d1E01-2-0408 | 752.209 | OEt | A01 | a1 | B04 | b2 | C04 | c2 | D02 | d1 | E01 |
| 2-2-d1E01-2-0409 | 752.209 | OEt | A01 | a1 | B04 | b2 | C06 | c2 | D02 | d1 | E01 |
| 2-2-d1E01-2-0410 | 752.209 | OEt | A01 | a1 | B04 | b2 | C07 | c2 | D02 | d1 | E01 |
| 2-2-d1E01-2-0411 | 752.252 | OEt | A02 | a1 | B01 | b3 | C11 | c2 | D02 | d1 | E01 |
| 2-2-d1E01-2-0412 | 752.324 | OEt | A02 | a1 | B02 | b4 | C02 | c2 | D02 | d1 | E01 |
| 2-2-d1E01-2-0413 | 753.229 | OEt | A02 | a1 | B04 | b1 | C02 | c2 | D02 | d1 | E01 |
| 2-2-d1E01-2-0414 | 753.229 | OEt | A02 | a1 | B04 | b1 | C03 | c2 | D02 | d1 | E01 |
| 2-2-d1E01-2-0415 | 754.224 | OEt | A02 | a1 | B04 | b1 | C04 | c2 | D02 | d1 | E01 |
| 2-2-d1E01-2-0416 | 754.228 | OEt | A02 | a1 | B05 | b1 | C01 | c2 | D02 | d1 | E01 |
| 2-2-d1E01-2-0417 | 754.315 | OEt | A01 | a1 | B01 | b1 | C19 | c2 | D02 | d1 | E01 |
| 2-2-d1E01-2-0418 | 756.219 | OEt | A01 | a1 | B05 | b3 | C09 | c2 | D02 | d1 | E01 |
| 2-2-d1E01-2-0419 | 756.240 | OEt | A01 | a1 | B04 | b1 | C15 | c2 | D02 | d1 | E01 |
| 2-2-d1E01-2-0420 | 756.277 | OEt | A02 | a1 | B05 | b3 | C11 | c1 | D01 | d1 | E01 |
| 2-2-d1E01-2-0421 | 756.299 | OEt | A02 | a1 | B03 | b4 | C02 | c2 | D02 | d1 | E01 |
| 2-2-d1E01-2-0422 | 757.235 | OEt | A01 | a1 | B04 | b1 | C16 | c2 | D02 | d1 | E01 |
| 2-2-d1E01-2-0423 | 758.230 | OEt | A01 | a1 | B04 | b1 | C17 | c2 | D02 | d1 | E01 |
| 2-2-d1E01-2-0424 | 758.235 | OEt | A01 | a1 | B05 | b1 | C14 | c2 | D02 | d1 | E01 |
| 2-2-d1E01-2-0425 | 758.281 | OEt | A02 | a1 | B02 | b4 | C05 | c2 | D02 | d1 | E01 |
| 2-2-d1E01-2-0426 | 758.296 | OEt | A02 | a1 | B05 | b4 | C12 | c2 | D02 | d1 | E01 |
| 2-2-d1E01-2-0427 | 758.340 | OEt | A01 | a1 | B05 | b1 | C19 | c1 | D01 | d1 | E01 |
| 2-2-d1E01-2-0428 | 759.186 | OEt | A02 | a1 | B04 | b1 | C05 | c2 | D02 | d1 | E01 |
| 2-2-d1E01-2-0429 | 759.240 | OEt | A01 | a1 | B04 | b4 | C01 | c2 | D02 | d1 | E01 |
| 2-2-d1E01-2-0430 | 760.199 | OEt | A02 | a1 | B04 | b3 | C08 | c2 | D02 | d1 | E01 |
| 2-2-d1E01-2-0431 | 760.275 | OEt | A02 | a1 | B05 | b4 | C13 | c2 | D02 | d1 | E01 |
| 2-2-d1E01-2-0432 | 761.180 | OEt | A01 | a1 | B05 | b3 | C10 | c2 | D02 | d1 | E01 |
| 2-2-d1E01-2-0433 | 762.234 | OEt | A01 | a1 | B05 | b2 | C02 | c2 | D02 | d1 | E01 |
| 2-2-d1E01-2-0434 | 762.234 | OEt | A01 | a1 | B05 | b2 | C03 | c2 | D02 | d1 | E01 |
| 2-2-d1E01-2-0435 | 762.256 | OEt | A02 | a1 | B03 | b4 | C05 | c2 | D02 | d1 | E01 |
| 2-2-d1E01-2-0436 | 763.192 | OEt | A01 | a1 | B04 | b1 | C18 | c2 | D02 | d1 | E01 |
| 2-2-d1E01-2-0437 | 763.229 | OEt | A01 | a1 | B05 | b2 | C04 | c2 | D02 | d1 | E01 |
| 2-2-d1E01-2-0438 | 763.229 | OEt | A01 | a1 | B05 | b2 | C06 | c2 | D02 | d1 | E01 |
| 2-2-d1E01-2-0439 | 763.229 | OEt | A01 | a1 | B05 | b2 | C07 | c2 | D02 | d1 | E01 |
| 2-2-d1E01-2-0440 | 764.249 | OEt | A02 | a1 | B05 | b1 | C02 | c2 | D02 | d1 | E01 |
| 2-2-d1E01-2-0441 | 764.249 | OEt | A02 | a1 | B05 | b1 | C03 | c2 | D02 | d1 | E01 |
| 2-2-d1E01-2-0442 | 765.244 | OEt | A02 | a1 | B05 | b1 | C04 | c2 | D02 | d1 | E01 |
| 2-2-d1E01-2-0443 | 766.267 | OEt | A02 | a1 | B02 | b3 | C11 | c2 | D02 | d1 | E01 |
| 2-2-d1E01-2-0444 | 767.260 | OEt | A01 | a1 | B05 | b1 | C15 | c2 | D02 | d1 | E01 |

(continued)

| ID | Exact Mass | ☒ | A | a | B | b | c | c | D | d | E |
|---|---|---|---|---|---|---|---|---|---|---|---|
| 2-2-d1E01-2-0445 | 768.255 | OEt | A01 | a1 | B05 | b1 | C16 | c2 | D02 | d1 | E01 |
| 2-2-d1E01-2-0446 | 768.331 | OEt | A01 | a1 | B02 | b1 | C19 | c2 | D02 | d1 | E01 |
| 2-2-d1E01-2-0447 | 769.251 | OEt | A01 | a1 | B05 | b1 | C17 | c2 | D02 | d1 | E01 |
| 2-2-d1E01-2-0448 | 769.260 | OEt | A01 | a1 | B04 | b4 | C02 | c2 | D02 | d1 | E01 |
| 2-2-d1E01-2-0449 | 770.206 | OEt | A02 | a1 | B05 | b1 | C05 | c2 | D02 | d1 | E01 |
| 2-2-d1E01-2-0450 | 770.242 | OEt | A02 | a1 | B03 | b3 | C11 | c2 | D02 | d1 | E01 |
| 2-2-d1E01-2-0451 | 770.260 | OEt | A01 | a1 | B05 | b4 | C01 | c2 | D02 | d1 | E01 |
| 2-2-d1E01-2-0452 | 771.215 | OEt | A02 | a1 | B04 | b3 | C09 | c2 | D02 | d1 | E01 |
| 2-2-d1E01-2-0453 | 771.219 | OEt | A02 | a1 | B05 | b3 | C08 | c2 | D02 | d1 | E01 |
| 2-2-d1E01-2-0454 | 772.305 | OEt | A01 | a1 | B03 | b1 | C19 | c2 | D02 | d1 | E01 |
| 2-2-d1E01-2-0455 | 773.230 | OEt | A02 | a1 | B04 | b1 | C14 | c2 | D02 | d1 | E01 |
| 2-2-d1E01-2-0456 | 773.336 | OEt | A02 | a1 | B04 | b1 | C19 | c1 | D01 | d1 | E01 |
| 2-2-d1E01-2-0457 | 774.212 | OEt | A01 | a1 | B05 | b1 | C18 | c2 | D02 | d1 | E01 |
| 2-2-d1E01-2-0458 | 775.217 | OEt | A01 | a1 | B04 | b4 | C05 | c2 | D02 | d1 | E01 |
| 2-2-d1E01-2-0459 | 776.176 | OEt | A02 | a1 | B04 | b3 | C10 | c2 | D02 | d1 | E01 |
| 2-2-d1E01-2-0460 | 777.229 | OEt | A02 | a1 | B04 | b2 | C02 | c2 | D02 | d1 | E01 |
| 2-2-d1E01-2-0461 | 777.229 | OEt | A02 | a1 | B04 | b2 | C03 | c2 | D02 | d1 | E01 |
| 2-2-d1E01-2-0462 | 778.224 | OEt | A02 | a1 | B04 | b2 | C04 | c2 | D02 | d1 | E01 |
| 2-2-d1E01-2-0463 | 778.224 | OEt | A02 | a1 | B04 | b2 | C06 | c2 | D02 | d1 | E01 |
| 2-2-d1E01-2-0464 | 778.224 | OEt | A02 | a1 | B04 | b2 | C07 | c2 | D02 | d1 | E01 |
| 2-2-d1E01-2-0465 | 780.280 | OEt | A01 | a1 | B05 | b4 | C02 | c2 | D02 | d1 | E01 |
| 2-2-d1E01-2-0466 | 780.331 | OEt | A02 | a1 | B01 | b1 | C19 | c2 | D02 | d1 | E01 |
| 2-2-d1E01-2-0467 | 782.235 | OEt | A02 | a1 | B05 | b3 | C09 | c2 | D02 | d1 | E01 |
| 2-2-d1E01-2-0468 | 782.256 | OEt | A02 | a1 | B04 | b1 | C15 | c2 | D02 | d1 | E01 |
| 2-2-d1E01-2-0469 | 783.203 | OEt | A01 | a1 | B04 | b3 | C11 | c2 | D02 | d1 | E01 |
| 2-2-d1E01-2-0470 | 783.251 | OEt | A02 | a1 | B04 | b1 | C16 | c2 | D02 | d1 | E01 |
| 2-2-d1E01-2-0471 | 784.246 | OEt | A02 | a1 | B04 | b1 | C17 | c2 | D02 | d1 | E01 |
| 2-2-d1E01-2-0472 | 784.250 | OEt | A02 | a1 | B05 | b1 | C14 | c2 | D02 | d1 | E01 |
| 2-2-d1E01-2-0473 | 784.356 | OEt | A02 | a1 | B05 | b1 | C19 | c1 | D01 | d1 | E01 |
| 2-2-d1E01-2-0474 | 785.255 | OEt | A02 | a1 | B04 | b4 | C01 | c2 | D02 | d1 | E01 |
| 2-2-d1E01-2-0475 | 786.237 | OEt | A01 | a1 | B05 | b4 | C05 | c2 | D02 | d1 | E01 |
| 2-2-d1E01-2-0476 | 787.196 | OEt | A02 | a1 | B05 | b3 | C10 | c2 | D02 | d1 | E01 |
| 2-2-d1E01-2-0477 | 788.249 | OEt | A02 | a1 | B05 | b2 | C02 | c2 | D02 | d1 | E01 |
| 2-2-d1E01-2-0478 | 788.249 | OEt | A02 | a1 | B05 | b2 | C03 | c2 | D02 | d1 | E01 |
| 2-2-d1E01-2-0479 | 789.207 | OEt | A02 | a1 | B04 | b1 | C18 | c2 | D02 | d1 | E01 |
| 2-2-d1E01-2-0480 | 789.244 | OEt | A02 | a1 | B05 | b2 | C04 | c2 | D02 | d1 | E01 |
| 2-2-d1E01-2-0481 | 789.244 | OEt | A02 | a1 | B05 | b2 | C06 | c2 | D02 | d1 | E01 |
| 2-2-d1E01-2-0482 | 789.244 | OEt | A02 | a1 | B05 | b2 | C07 | c2 | D02 | d1 | E01 |

(continued)

| ID | Exact Mass | ⊠ | A | a | B | b | c | c | D | d | E |
|---|---|---|---|---|---|---|---|---|---|---|---|
| 2-2-d1E01-2-0483 | 793.276 | OEt | A02 | a1 | B05 | b1 | C15 | c2 | D02 | d1 | E01 |
| 2-2-d1E01-2-0484 | 794.223 | OEt | A01 | a1 | B05 | b3 | C11 | c2 | D02 | d1 | E01 |
| 2-2-d1E01-2-0485 | 794.271 | OEt | A02 | a1 | B05 | b1 | C16 | c2 | D02 | d1 | E01 |
| 2-2-d1E01-2-0486 | 794.346 | OEt | A02 | a1 | B02 | b1 | C19 | c2 | D02 | d1 | E01 |
| 2-2-d1E01-2-0487 | 795.266 | OEt | A02 | a1 | B05 | b1 | C17 | c2 | D02 | d1 | E01 |
| 2-2-d1E01-2-0488 | 795.276 | OEt | A02 | a1 | B04 | b4 | C02 | c2 | D02 | d1 | E01 |
| 2-2-d1E01-2-0489 | 796.275 | OEt | A02 | a1 | B05 | b4 | C01 | c2 | D02 | d1 | E01 |
| 2-2-d1E01-2-0490 | 798.321 | OEt | A02 | a1 | B03 | b1 | C19 | c2 | D02 | d1 | E01 |
| 2-2-d1E01-2-0491 | 800.227 | OEt | A02 | a1 | B05 | b1 | C18 | c2 | D02 | d1 | E01 |
| 2-2-d1E01-2-0492 | 801.232 | OEt | A02 | a1 | B04 | b4 | C05 | c2 | D02 | d1 | E01 |
| 2-2-d1E01-2-0493 | 806.296 | OEt | A02 | a1 | B05 | b4 | C02 | c2 | D02 | d1 | E01 |
| 2-2-d1E01-2-0494 | 809.219 | OEt | A02 | a1 | B04 | b3 | C11 | c2 | D02 | d1 | E01 |
| 2-2-d1E01-2-0495 | 811.282 | OEt | A01 | a1 | B04 | b1 | C19 | c2 | D02 | d1 | E01 |
| 2-2-d1E01-2-0496 | 812.253 | OEt | A02 | a1 | B05 | b4 | C05 | c2 | D02 | d1 | E01 |
| 2-2-d1E01-2-0497 | 820.239 | OEt | A02 | a1 | B05 | b3 | C11 | c2 | D02 | d1 | E01 |
| 2-2-d1E01-2-0498 | 822.302 | OEt | A01 | a1 | B05 | b1 | C19 | c2 | D02 | d1 | E01 |
| 2-2-d1E01-2-0499 | 837.298 | OEt | A02 | a1 | B04 | b1 | C19 | c2 | D02 | d1 | E01 |
| 2-2-d1E01-2-0500 | 848.318 | OEt | A02 | a1 | B05 | b1 | C19 | c2 | D02 | d1 | E01 |

Example 6-9: Analysis of mixture

The mixture synthesized in Example 6-8 was analyzed by measuring the retention time and performing mass spectrometry under the analysis conditions shown below, using Compound Discoverer 3.2 (Thermo Fisher Scientific).

[Table 130]

| Reversed phase LC/MS conditions | |
|---|---|
| Vanquish UHPLC (Thermo Fisher Scientific) | |
| Oven (°C) | 35 |
| Column | Ascentis Express C18<br>2.1 mm I.D. × 150 mm, 2.7 μm |
| Autosampler (°C) | 20 |
| Mobile phase | A) 0.1% FA $H_2O$<br>B) 0.1% FA MeCN |
| Gradient | A/B = 95/5 -> 0/100 (18 min) -> 0/100 (20 min) |
| Flow rate | 0.5 mL/min |
| Orbitrap Fusion Lumos Tribrid mass spectrometer (Thermo Fisher Scientific) | |
| Polarity | Positive/Negative mode |
| Data Type | profile |

The analysis results for each mixture are shown in [Table 6-9-1]. The table shows the m/z and retention time of the observed MS peak, as well as the number of the compound that can be attributed to each MS peak.

[Table 131]

[Table 6-9-1] Compounds that may be included in mixture 2-2-d1E01-2

| Type of ion | m/z $[M+H]^+$ or $[M-H]^-$ | Retention time of MS peak (min) | Attributed compound No. (denoted omitting 2-2-d1E01-2) |
|---|---|---|---|
| M+H | 597.2700 | 14.696 | 0001 |
| M+H | 601.3383 | 14.482 | 0002 |
| M+H | 603.3171 | 14.204 | 0003 |
| M+H | 607.2905 | 15.122 | 0004,0005 |
| M+H | 607.2905 | 14.919 | 0004,0005 |
| M+H | 608.2866 | 13.889 | 0006 |
| M+H | 611.2861 | 15.147 | 0007 |
| M+H | 613.2482 | 15.043 | 0008 |
| M+H | 614.2617 | 12.974 | 0009 |
| M+H | 615.2623 | 6.987 | 0010 |
| M+H | 615.3532 | 14.889 | 0011 |
| M+H | 617.3328 | 14.608 | 0012 |
| M+H | 619.3288 | 14.504 | 0013 |
| M+H | 621.3088 | 14.296 | 0014,0015,0016 |
| M+H | 621.3067 | 12.096 | 0014,0015,0016 |
| M+H | 621.3088 | 14.243 | 0014,0015,0016 |
| M+H | 622.3028 | 14.428 | 0017 |
| M+H | 623.2859 | 15.272 | 0018 |
| M-H | 623.2639 | 14.887 | 0019,0020,0021 |
| M+H | 625.2816 | 15.016 | 0019,0020,0021 |
| M+H | 625.2814 | 14.960 | 0019,0020,0021 |
| M+H | 626.2767 | 13.968 | 0022 |
| M+H | 627.2627 | 15.423 | 0023 |
| M+H | 627.2915 | 14.537 | 0024 |
| M+H | 627.3539 | 15.297 | 0025 |
| M+H | 628.2769 | 14.293 | 0026 |
| M+H | 629.3326 | 14.949 | 0027 |
| M+H | 630.2375 | 15.081 | 0028 |
| M+H | 631.2380 | 15.134 | 0029 |
| M+H | 631.2908 | 15.614 | 0030,0031 |
| M+H | 631.2908 | 15.539 | 0030,0031 |
| M+H | 632.2531 | 12.889 | 0032 |
| M+H | 632.2860 | 14.807 | 0033,0034,0035 |
| M+H | 632.2867 | 15.907 | 0033,0034,0035 |
| M+H | 632.2869 | 16.259 | 0033,0034,0035 |

(continued)

| Type of ion | m/z [M+H]+ or [M-H]- | Retention time of MS peak (min) | Attributed compound No. (denoted omitting 2-2-d1E01-2) |
|---|---|---|---|
| M+H | 633.3050 | 15.475 | 0036,0037 |
| M+H | 633.3047 | 15.351 | 0036,0037 |
| M+H | 634.3025 | 14.455 | 0038 |
| M+H | 636.3179 | 13.740 | 0039 |
| M+H | 637.3019 | 14.694 | 0040 |
| M+H | 637.3126 | 15.729 | 0041 |
| M+H | 638.3083 | 14.688 | 0042 |
| M+H | 639.2632 | 9.250 | 0043 |
| M-H | 637.2761 | 14.198 | 0044 |
| M+H | 639.3184 | 15.051 | 0045 |
| M+H | 640.2791 | 8.682 | 0046 |
| M+H | 641.2769 | 15.285 | 0047 |
| M-H | 639.2912 | 14.958 | 0048 |
| M+H | 641.3694 | 15.778 | 0049 |
| M+H | 643.2702 | 15.072 | 0050,0051 |
| M+H | 643.2702 | 15.134 | 0050,0051 |
| M+H | 643.3494 | 15.327 | 0052 |
| M+H | 644.2537 | 15.412 | 0053 |
| M+H | 645.2824 | 14.576 | 0054 |
| M+H | 645.3068 | 12.028 | 0055,0056 |
| M+H | 645.3064 | 15.933 | 0055,0056 |
| M+H | 645.3437 | 13.261 | 0057 |
| M+H | 646.3015 | 16.241 | 0058,0059,0060 |
| M+H | 646.3016 | 16.181 | 0058,0059,0060 |
| M+H | 646.3018 | 10.947 | 0058,0059,0060 |
| M+H | 647.3231 | 15.003 | 0061,0062,0063 |
| M+H | 647.3228 | 14.900 | 0061,0062,0063 |
| M+H | 647.3224 | 12.930 | 0061,0062,0063 |
| M+H | 648.2294 | 15.058 | 0064 |
| M+H | 648.3180 | 12.665 | 0065 |
| M+H | 649.2832 | 15.579 | 0066,0067 |
| M+H | 649.2819 | 11.785 | 0066,0067 |
| M+H | 649.3388 | 15.645 | 0068 |
| M+H | 650.2763 | 14.860 | 0069,0070,0071 |
| M+H | 650.2765 | 15.940 | 0069,0070,0071 |
| M+H | 650.2766 | 16.250 | 0069,0070,0071 |
| M+H | 650.3334 | 14.163 | 0072 |

(continued)

| Type of ion | m/z [M+H]+ or [M-H]- | Retention time of MS peak (min) | Attributed compound No. (denoted omitting 2-2-d1E01-2) |
|---|---|---|---|
| M-H | 649.2795 | 15.455 | 0073,0074,0075 |
| M+H | 651.2961 | 15.404 | 0073,0074,0075 |
| M+H | 651.2962 | 15.363 | 0073,0074,0075 |
| M+H | 651.3290 | 16.039 | 0076 |
| M+H | 652.2933 | 14.579 | 0077 |
| M+H | 652.3244 | 15.070 | 0078 |
| M+H | 653.2792 | 10.757 | 0079 |
| M+H | 653.3093 | 15.101 | 0080 |
| M+H | 653.3326 | 15.480 | 0081 |
| M+H | 654.2384 | 15.324 | 0082 |
| M-H | 652.2744 | 13.889 | 0083 |
| M+H | 654.3060 | 14.751 | 0084 |
| M+H | 655.2942 | 15.238 | 0085 |
| M+H | 655.3032 | 15.710 | 0086 |
| M+H | 656.2560 | 12.823 | 0087 |
| M+H | 656.2988 | 14.704 | 0088 |
| M+H | 657.2539 | 15.572 | 0089 |
| M+H | 657.2854 | 15.486 | 0090 |
| M+H | 657.3065 | 16.078 | 0091,0092,0093 |
| M+H | 657.3064 | 15.895 | 0091,0092,0093 |
| M+H | 657.3062 | 15.939 | 0091,0092,0093 |
| M+H | 658.2699 | 13.281 | 0094 |
| M+H | 658.3030 | 15.335 | 0095,0096,0097,0098 |
| M+H | 658.3051 | 14.256 | 0095,0096,0097,0098 |
| M+H | 658.3016 | 16.313 | 0095,0096,0097,0098 |
| M+H | 658.3066 | 15.104 | 0095,0096,0097,0098 |
| M+H | 660.2847 | 14.853 | 0099 |
| M-H | 659.2151 | 14.191 | 0100 |
| M+H | 661.2592 | 15.175 | 0101 |
| M+H | 662.3324 | 16.234 | 0102 |
| M+H | 663.2809 | 15.337 | 0103 |
| M+H | 663.3287 | 12.907 | 0104 |
| M+H | 663.3537 | 15.934 | 0105 |
| M+H | 664.2590 | 15.542 | 0106,0107 |
| M+H | 664.2584 | 15.402 | 0106,0107 |
| M+H | 664.3226 | 15.155 | 0108 |
| M+H | 665.2532 | 14.524 | 0109,0110 |

(continued)

| Type of ion | m/z [M+H]+ or [M-H]- | Retention time of MS peak (min) | Attributed compound No. (denoted omitting 2-2-d1E01-2) |
|---|---|---|---|
| M+H | 665.2612 | 15.651 | 0110 |
| M+H | 665.2999 | 14.028 | 0111 |
| M+H | 665.3079 | 11.568 | 0112 |
| M+H | 665.3321 | 15.756 | 0113 |
| M+H | 667.2789 | 13.746 | 0114 |
| M+H | 667.3238 | 15.460 | 0115 |
| M+H | 667.3290 | 15.686 | 0116 |
| M+H | 669.2821 | 11.183 | 0117,0118 |
| M+H | 669.2885 | 13.655 | 0118 |
| M+H | 669.3091 | 15.739 | 0119 |
| M-H | 667.3071 | 15.463 | 0120 |
| M+H | 670.2146 | 15.630 | 0121 |
| M+H | 670.2711 | 12.694 | 0122 |
| M+H | 671.2284 | 12.538 | 0123 |
| M+H | 671.2531 | 14.488 | 0124,0125 |
| M+H | 671.2522 | 14.279 | 0124,0125 |
| M+H | 671.3001 | 15.109 | 0126 |
| M+H | 671.3035 | 15.238 | 0126,0127 |
| M+H | 671.3218 | 16.387 | 0128,0129 |
| M+H | 671.3216 | 16.247 | 0128,0129 |
| M-H | 670.2322 | 13.571 | 0130 |
| M+H | 672.3181 | 15.682 | 0131,0132,0133 |
| M+H | 672.3179 | 16.914 | 0131,0132,0133 |
| M+H | 672.3175 | 16.691 | 0131,0132,0133 |
| M+H | 673.2842 | 15.482 | 0134 |
| M+H | 674.2440 | 15.552 | 0135 |
| M+H | 675.2477 | 14.586 | 0136 |
| M+H | 675.2789 | 15.772 | 0137,0138 |
| M+H | 675.2789 | 15.811 | 0137,0138 |
| M+H | 675.2966 | 16.078 | 0139,0140 |
| M+H | 675.2966 | 15.904 | 0139,0140 |
| M+H | 675.3543 | 16.185 | 0141 |
| M+H | 676.2744 | 15.052 | 0142 |
| M+H | 676.2923 | 15.347 | 0143,0144,0145 |
| M+H | 676.2920 | 16.367 | 0143,0144,0145 |
| M+H | 676.2920 | 16.644 | 0143,0144,0145 |
| M+H | 676.3493 | 13.106 | 0146 |

(continued)

| Type of ion | m/z [M+H]+ or [M-H]- | Retention time of MS peak (min) | Attributed compound No. (denoted omitting 2-2-d1E01-2) |
|---|---|---|---|
| M+H | 677.2086 | 14.513 | 0147 |
| M+H | 677.2965 | 15.678 | 0148 |
| M+H | 677.3478 | 9.909 | 0149 |
| M+H | 678.2221 | 13.975 | 0150 |
| M+H | 678.3387 | 15.361 | 0151 |
| M+H | 679.2236 | 14.310 | 0152 |
| M+H | 679.3150 | 14.404 | 0153 |
| M+H | 679.3488 | 16.081 | 0154 |
| M+H | 680.2535 | 15.948 | 0155 |
| M+H | 680.3219 | 8.435 | 0156 |
| M+H | 681.2343 | 15.911 | 0157 |
| M+H | 681.2700 | 15.406 | 0158 |
| M+H | 681.2944 | 14.111 | 0159 |
| M+H | 681.3110 | 16.047 | 0160 |
| M+H | 681.3208 | 16.272 | 0161 |
| M+H | 682.2436 | 14.958 | 0162,0163 |
| M+H | 682.2471 | 11.698 | 0162,0163 |
| M+H | 682.3142 | 15.112 | 0164 |
| M-H | 681.2722 | 11.935 | 0165 |
| M+H | 683.2999 | 9.765 | 0166 |
| M+H | 683.3224 | 15.803 | 0167 |
| M+H | 684.2598 | 15.116 | 0168 |
| M-H | 682.3025 | 15.958 | 0169 |
| M+H | 685.2700 | 13.853 | 0170,0171,0172 |
| M+H | 685.2689 | 14.638 | 0170,0171,0172 |
| M+H | 685.2699 | 13.808 | 0170,0171,0172 |
| M+H | 686.2637 | 14.054 | 0173 |
| M+H | 686.2999 | 14.704 | 0174 |
| M-H | 685.1864 | 15.538 | 0175 |
| M-H | 685.2309 | 13.716 | 0176 |
| M+H | 687.2749 | 15.625 | 0177 |
| M+H | 688.2579 | 16.053 | 0178,0179 |
| M+H | 688.2575 | 16.154 | 0178,0179 |
| M+H | 689.2409 | 13.919 | 0180,0181,0182 |
| M+H | 689.2429 | 14.344 | 0180,0181,0182 |
| M+H | 689.2432 | 14.503 | 0180,0181,0182 |
| M+H | 689.2534 | 15.387 | 0183,0184,0185 |

(continued)

| Type of ion | m/z [M+H]+ or [M-H]- | Retention time of MS peak (min) | Attributed compound No. (denoted omitting 2-2-d1E01-2) |
|---|---|---|---|
| M+H | 689.2544 | 16.398 | 0183,0184,0185 |
| M+H | 689.2546 | 16.431 | 0183,0184,0185 |
| M+H | 689.2943 | 13.377 | 0186 |
| M+H | 689.3677 | 16.559 | 0187 |
| M+H | 690.2391 | 13.728 | 0188 |
| M+H | 690.2752 | 15.893 | 0189,0190 |
| M+H | 690.2755 | 15.972 | 0189,0190 |
| M+H | 691.2248 | 14.868 | 0191 |
| M+H | 691.2539 | 13.847 | 0192 |
| M+H | 691.2686 | 15.128 | 0193 |
| M-H | 689.2561 | 16.054 | 0193,0194 |
| M+H | 691.3149 | 14.892 | 0195 |
| M+H | 691.3597 | 14.749 | 0196 |
| M+H | 692.2393 | 14.420 | 0197 |
| M+H | 693.2641 | 14.062 | 0198 |
| M+H | 693.2839 | 15.780 | 0199 |
| M+H | 693.2937 | 14.578 | 0200 |
| M+H | 693.3420 | 15.743 | 0201 |
| M+H | 694.1984 | 14.328 | 0202 |
| M+H | 694.2799 | 16.231 | 0203 |
| M+H | 695.1989 | 14.625 | 0204 |
| M+H | 695.2519 | 14.981 | 0205,0206 |
| M+H | 695.2518 | 14.926 | 0205,0206 |
| M+H | 695.2759 | 15.337 | 0207,0208 |
| M+H | 695.2786 | 15.481 | 0207,0208 |
| M-H | 693.3088 | 14.896 | 0209 |
| M+H | 695.3424 | 16.141 | 0210 |
| M+H | 696.2156 | 12.557 | 0211 |
| M+H | 696.2310 | 16.112 | 0212 |
| M+H | 696.2493 | 15.089 | 0213,0214,0215 |
| M+H | 696.2474 | 15.166 | 0213,0214,0215 |
| M-H | 694.2321 | 15.038 | 0213,0214,0215 |
| M+H | 696.2854 | 14.913 | 0216 |
| M+H | 697.2457 | 15.654 | 0217 |
| M+H | 697.2725 | 5.181 | 0218,0219 |
| M-H | 695.2539 | 12.461 | 0218,0219 |
| M+H | 697.3196 | 15.865 | 0220 |

(continued)

| Type of ion | m/z $[M+H]^+$ or $[M-H]^-$ | Retention time of MS peak (min) | Attributed compound No. (denoted omitting 2-2-d1E01-2) |
|---|---|---|---|
| M+H | 698.2246 | 15.891 | 0221 |
| M+H | 698.2637 | 12.413 | 0222 |
| M+H | 699.2769 | 16.374 | 0223,0224 |
| M+H | 699.2758 | 13.030 | 0223,0224 |
| M+H | 699.3023 | 16.038 | 0225 |
| M+H | 700.2372 | 15.658 | 0226 |
| M+H | 700.2730 | 15.730 | 0227,0228,0229 |
| M+H | 700.2727 | 16.669 | 0227,0228,0229 |
| M+H | 700.2724 | 16.620 | 0227,0228,0229 |
| M+H | 700.2789 | 14.266 | 0227,0228,0229,0230 |
| M+H | 701.2637 | 15.166 | 0231 |
| M+H | 701.2745 | 15.051 | 0232 |
| M+H | 701.2929 | 16.231 | 0233,0234 |
| M+H | 701.2928 | 16.280 | 0233,0234 |
| M+H | 702.2704 | 13.795 | 0235 |
| M+H | 702.2886 | 15.433 | 0236 |
| M+H | 703.2235 | 14.063 | 0237 |
| M-H | 701.2362 | 14.398 | 0238 |
| M+H | 703.2793 | 14.448 | 0239 |
| M+H | 703.3118 | 16.165 | 0240 |
| M+H | 704.2366 | 14.606 | 0241 |
| M+H | 704.3046 | 14.926 | 0242 |
| M+H | 705.2379 | 14.804 | 0243 |
| M+H | 705.2697 | 14.218 | 0244 |
| M+H | 705.2996 | 16.506 | 0245 |
| M-H | 703.3129 | 15.277 | 0246 |
| M+H | 705.3754 | 15.121 | 0247 |
| M+H | 706.2959 | 15.598 | 0248 |
| M+H | 706.3063 | 16.173 | 0249 |
| M+H | 707.2322 | 14.452 | 0250,0251 |
| M+H | 707.2319 | 14.508 | 0250,0251 |
| M+H | 707.2520 | 13.875 | 0252 |
| M+H | 707.2881 | 15.809 | 0253 |
| M+H | 707.3038 | 15.953 | 0254 |
| M-H | 705.2944 | 14.965 | 0254,0255 |
| M+H | 708.2143 | 14.707 | 0256 |
| M+H | 708.2591 | 15.778 | 0257,0258 |

(continued)

| Type of ion | m/z $[M+H]^+$ or $[M-H]^-$ | Retention time of MS peak (min) | Attributed compound No. (denoted omitting 2-2-d1E01-2) |
|---|---|---|---|
| M+H | 708.2606 | 15.857 | 0257,0258 |
| M+H | 709.2441 | 13.937 | 0259 |
| M+H | 709.2694 | 15.348 | 0260,0261 |
| M+H | 709.2691 | 15.246 | 0260,0261 |
| M+H | 709.3085 | 14.748 | 0262 |
| M+H | 709.3497 | 14.787 | 0263 |
| M+H | 710.2637 | 15.481 | 0264,0265,0266 |
| M+H | 710.2665 | 15.431 | 0264,0265,0266 |
| M+H | 710.2647 | 13.700 | 0264,0265,0266 |
| M+H | 710.2752 | 15.677 | 0267 |
| M+H | 711.2562 | 15.986 | 0268 |
| M+H | 711.2840 | 14.603 | 0269,0270,0271 |
| M+H | 711.2844 | 14.518 | 0269,0270,0271 |
| M-H | 709.2669 | 14.548 | 0269,0270,0271 |
| M-H | 710.1717 | 14.399 | 0272 |
| M+H | 712.2617 | 15.261 | 0273 |
| M+H | 712.2798 | 14.674 | 0274 |
| M+H | 713.2196 | 16.047 | 0275 |
| M+H | 713.2433 | 15.014 | 0276,0277 |
| M+H | 713.2435 | 15.153 | 0276,0277 |
| M+H | 713.2988 | 15.066 | 0278 |
| M+H | 714.2385 | 15.203 | 0279,0280,0281 |
| M-H | 712.2210 | 15.134 | 0279,0280,0281 |
| M+H | 714.2409 | 12.704 | 0279,0280,0281 |
| M+H | 714.2731 | 16.447 | 0282,0283 |
| M+H | 714.2727 | 16.603 | 0282,0283 |
| M+H | 714.2978 | 13.061 | 0284 |
| M-H | 713.2410 | 14.812 | 0285,0286,0287 |
| M+H | 715.2626 | 13.342 | 0286,0287 |
| M+H | 715.2589 | 14.890 | 0285,0286,0287 |
| M+H | 715.2688 | 15.932 | 0288,0289,0290 |
| M+H | 715.2708 | 16.887 | 0288,0289,0290 |
| M+H | 715.2700 | 17.146 | 0288,0289,0290 |
| M+H | 715.2897 | 15.426 | 0291 |
| M+H | 716.2548 | 14.298 | 0292 |
| M+H | 716.2858 | 14.182 | 0293 |
| M+H | 717.2704 | 14.547 | 0295 |

(continued)

| Type of ion | m/z [M+H]+ or [M-H]- | Retention time of MS peak (min) | Attributed compound No. (denoted omitting 2-2-d1E01-2) |
|---|---|---|---|
| M+H | 717.2943 | 14.853 | 0296 |
| M+H | 717.3243 | 16.392 | 0297 |
| M+H | 717.3742 | 15.211 | 0298 |
| M+H | 718.2005 | 14.775 | 0299 |
| M+H | 718.2547 | 14.934 | 0300 |
| M+H | 718.2731 | 14.594 | 0301 |
| M+H | 719.2564 | 14.813 | 0302 |
| M+H | 719.2662 | 15.123 | 0303 |
| M+H | 719.2816 | 12.000 | 0304 |
| M+H | 719.3001 | 15.940 | 0305 |
| M+H | 720.2122 | 14.882 | 0306 |
| M+H | 720.2518 | 13.301 | 0307 |
| M+H | 720.2608 | 13.843 | 0308 |
| M+H | 720.2963 | 16.834 | 0309 |
| M+H | 721.2140 | 15.169 | 0310 |
| M+H | 721.2463 | 14.882 | 0311 |
| M-H | 719.2510 | 14.566 | 0312,0313,0314 |
| M+H | 721.2697 | 15.587 | 0312,0313,0314 |
| M+H | 721.2697 | 15.532 | 0312,0313,0314 |
| M+H | 721.2913 | 15.838 | 0315,0316 |
| M+H | 721.2929 | 15.893 | 0315,0316 |
| M-H | 720.2103 | 14.686 | 0317 |
| M+H | 722.2631 | 15.756 | 0318,0319,0320,0321 |
| M+H | 722.2669 | 13.721 | 0318,0319,0320,0321 |
| M-H | 720.2467 | 15.631 | 0318,0319,0320 |
| M-H | 720.2508 | 13.815 | 0318,0319,0320,0321 |
| M+H | 722.2992 | 16.317 | 0322 |
| M+H | 723.2815 | 16.199 | 0323 |
| M+H | 724.2387 | 16.305 | 0324 |
| M+H | 724.2471 | 14.392 | 0325 |
| M+H | 725.2211 | 14.578 | 0326 |
| M+H | 725.2955 | 16.695 | 0327,0328 |
| M+H | 725.2958 | 16.581 | 0327,0328 |
| M+H | 726.2520 | 16.158 | 0329 |
| M+H | 726.2903 | 17.016 | 0330,0331,0332 |
| M+H | 726.2903 | 16.972 | 0330,0331,0332 |
| M+H | 726.2903 | 16.934 | 0330,0331,0332 |

(continued)

| Type of ion | m/z $[M+H]^+$ or $[M-H]^-$ | Retention time of MS peak (min) | Attributed compound No. (denoted omitting 2-2-d1E01-2) |
|---|---|---|---|
| M+H | 726.2940 | 12.059 | 0330,0331,0332,0333 |
| M-H | 725.2258 | 14.954 | 0334 |
| M+H | 727.2903 | 12.454 | 0335 |
| M+H | 727.3138 | 11.980 | 0336 |
| M+H | 728.2205 | 14.848 | 0337,0338 |
| M+H | 728.2207 | 15.005 | 0337,0338 |
| M+H | 728.2862 | 12.117 | 0339 |
| M+H | 729.2151 | 14.346 | 0340,0341 |
| M+H | 729.2192 | 15.189 | 0340,0341 |
| M+H | 729.2687 | 12.745 | 0342 |
| M-H | 727.2771 | 15.127 | 0343 |
| M+H | 730.3212 | 15.568 | 0344 |
| M+H | 731.2691 | 16.181 | 0345 |
| M+H | 731.2845 | 14.894 | 0346 |
| M+H | 731.2898 | 15.085 | 0346,0347 |
| M+H | 731.3160 | 16.894 | 0348 |
| M+H | 731.3908 | 15.617 | 0349 |
| M+H | 732.3112 | 16.048 | 0350 |
| M+H | 732.3211 | 16.756 | 0351 |
| M+H | 733.2442 | 12.441 | 0352,0353 |
| M-H | 731.2352 | 13.634 | 0353 |
| M+H | 733.2703 | 15.165 | 0354 |
| M-H | 731.2705 | 14.969 | 0355 |
| M+H | 733.3203 | 16.456 | 0356 |
| M+H | 734.1764 | 15.150 | 0357 |
| M+H | 734.2307 | 6.846 | 0358 |
| M+H | 735.1901 | 14.625 | 0359 |
| M-H | 733.2482 | 14.775 | 0360,0361 |
| M-H | 733.2482 | 14.709 | 0360,0361 |
| M+H | 735.2835 | 15.936 | 0362,0363 |
| M+H | 735.2845 | 15.878 | 0362,0363 |
| M+H | 735.3650 | 15.263 | 0364 |
| M+H | 736.2789 | 16.108 | 0365,0366,0367 |
| M+H | 736.2790 | 16.173 | 0365,0366,0367 |
| M+H | 736.2785 | 15.980 | 0365,0366,0367 |
| M+H | 737.2462 | 14.908 | 0368 |
| M+H | 737.2719 | 16.369 | 0369 |

(continued)

| Type of ion | m/z $[M+H]^+$ or $[M-H]^-$ | Retention time of MS peak (min) | Attributed compound No. (denoted omitting 2-2-d1E01-2) |
|---|---|---|---|
| M-H | 736.1887 | 14.918 | 0370 |
| M+H | 738.2766 | 16.576 | 0371 |
| M+H | 739.2393 | 15.398 | 0372,0373 |
| M+H | 739.2407 | 15.336 | 0372,0373 |
| M+H | 739.2604 | 15.597 | 0374,0375 |
| M+H | 739.2604 | 15.511 | 0374,0375 |
| M-H | 737.2974 | 15.716 | 0376 |
| M+H | 740.2356 | 14.904 | 0377 |
| M+H | 740.2531 | 15.819 | 0378,0379,0380 |
| M+H | 740.2537 | 15.781 | 0378,0379,0380 |
| M+H | 740.2537 | 15.671 | 0378,0379,0380 |
| M+H | 740.3100 | 12.806 | 0381 |
| M+H | 741.2568 | 15.288 | 0382 |
| M+H | 741.3096 | 9.676 | 0383 |
| M+H | 742.3002 | 14.779 | 0384 |
| M+H | 743.3116 | 15.527 | 0385 |
| M-H | 741.3232 | 16.856 | 0386 |
| M+H | 744.2160 | 15.309 | 0387 |
| M+H | 744.2853 | 12.163 | 0388 |
| M+H | 745.1982 | 15.543 | 0389 |
| M+H | 745.2339 | 15.023 | 0390 |
| M+H | 745.2703 | 14.506 | 0391 |
| M+H | 745.2809 | 15.569 | 0392 |
| M+H | 746.2049 | 14.573 | 0393 |
| M-H | 744.1920 | 15.052 | 0393,0394 |
| M+H | 746.2646 | 16.389 | 0395 |
| M+H | 746.2754 | 12.248 | 0396 |
| M+H | 747.2625 | 9.439 | 0397 |
| M+H | 747.2836 | 15.210 | 0398 |
| M+H | 748.2211 | 14.478 | 0399 |
| M+H | 748.2840 | 15.497 | 0400 |
| M+H | 748.3258 | 15.401 | 0401 |
| M+H | 749.2987 | 16.751 | 0402 |
| M+H | 750.2619 | 15.160 | 0403 |
| M+H | 751.1658 | 14.867 | 0404 |
| M+H | 751.2370 | 15.002 | 0405 |
| M+H | 752.2206 | 15.544 | 0406,0407 |

(continued)

| Type of ion | m/z [M+H]+ or [M-H]- | Retention time of MS peak (min) | Attributed compound No. (denoted omitting 2-2-d1E01-2) |
|---|---|---|---|
| M+H | 752.2206 | 15.636 | 0406,0407 |
| M+H | 753.2157 | 15.755 | 0408,0409,0410 |
| M+H | 753.2147 | 15.684 | 0408,0409,0410 |
| M+H | 753.2151 | 15.986 | 0408,0409,0410 |
| M-H | 751.2414 | 13.907 | 0411 |
| M+H | 753.3296 | 16.027 | 0412 |
| M+H | 754.2363 | 15.412 | 0413,0414 |
| M+H | 754.2326 | 14.937 | 0413,0414 |
| M+H | 755.2312 | 14.911 | 0415,0416 |
| M+H | 755.2321 | 15.191 | 0415,0416 |
| M+H | 755.3214 | 14.186 | 0417 |
| M-H | 755.2071 | 14.990 | 0418 |
| M+H | 757.2458 | 14.884 | 0419 |
| M+H | 757.2832 | 16.458 | 0420 |
| M-H | 755.2882 | 15.716 | 0421 |
| M+H | 758.2430 | 15.587 | 0422 |
| M+H | 759.2373 | 14.515 | 0423,0424 |
| M+H | 759.2415 | 12.819 | 0423,0424 |
| M+H | 759.2869 | 15.865 | 0425 |
| M+H | 759.3062 | 14.828 | 0426 |
| M+H | 759.3477 | 15.654 | 0427 |
| M+H | 760.1927 | 15.691 | 0428 |
| M+H | 760.2460 | 14.238 | 0429 |
| M+H | 761.2066 | 15.112 | 0430 |
| M-H | 759.2638 | 15.440 | 0431 |
| M-H | 760.1697 | 15.288 | 0432 |
| M+H | 763.2394 | 15.922 | 0433,0434 |
| M+H | 763.2386 | 15.828 | 0433,0434 |
| M+H | 763.2633 | 15.550 | 0435 |
| M+H | 764.1980 | 15.051 | 0436 |
| M+H | 764.2380 | 16.243 | 0437,0438,0439 |
| M+H | 764.2354 | 16.176 | 0437,0438,0439 |
| M-H | 762.2194 | 15.943 | 0437,0438,0439 |
| M+H | 765.2536 | 15.816 | 0440,0441 |
| M+H | 765.2537 | 15.682 | 0440,0441 |
| M+H | 766.2520 | 15.404 | 0442 |
| M+H | 767.2719 | 15.780 | 0443 |

(continued)

| Type of ion | m/z [M+H]+ or [M-H]- | Retention time of MS peak (min) | Attributed compound No. (denoted omitting 2-2-d1E01-2) |
|---|---|---|---|
| M+H | 768.2659 | 15.162 | 0444 |
| M+H | 769.2614 | 15.866 | 0445 |
| M+H | 769.3378 | 14.525 | 0446 |
| M+H | 770.2595 | 14.886 | 0447 |
| M+H | 770.2678 | 15.601 | 0448 |
| M+H | 771.2112 | 15.992 | 0449 |
| M+H | 771.2525 | 15.556 | 0450 |
| M+H | 771.2674 | 15.347 | 0451 |
| M+H | 772.2210 | 15.190 | 0452,0453 |
| M+H | 772.2260 | 15.349 | 0452,0453 |
| M+H | 773.3114 | 14.271 | 0454 |
| M+H | 774.2370 | 15.074 | 0455 |
| M+H | 774.3417 | 15.892 | 0456 |
| M+H | 775.2194 | 15.392 | 0457 |
| M+H | 776.2236 | 14.575 | 0458 |
| M+H | 777.1824 | 15.363 | 0459 |
| M+H | 778.2366 | 16.114 | 0460,0461 |
| M+H | 778.2366 | 15.987 | 0460,0461 |
| M+H | 779.2302 | 16.395 | 0462,0463,0464 |
| M+H | 779.2316 | 16.251 | 0462,0463,0464 |
| M+H | 779.2294 | 16.318 | 0462,0463,0464 |
| M-H | 779.2695 | 15.859 | 0465 |
| M+H | 781.3363 | 17.162 | 0466 |
| M-H | 781.2268 | 11.623 | 0467 |
| M+H | 783.2632 | 15.427 | 0468 |
| M+H | 784.2102 | 15.552 | 0469 |
| M+H | 784.2603 | 16.192 | 0470 |
| M+H | 785.2568 | 15.351 | 0471,0472 |
| M+H | 785.2561 | 15.321 | 0471,0472 |
| M+H | 785.3619 | 16.075 | 0473 |
| M+H | 786.2610 | 15.756 | 0474 |
| M-H | 785.2274 | 15.237 | 0475 |
| M-H | 786.1838 | 15.730 | 0476 |
| M-H | 787.2369 | 16.313 | 0477,0478 |
| M-H | 787.2369 | 16.178 | 0477,0478 |
| M+H | 790.2144 | 15.617 | 0479 |
| M+H | 790.2533 | 12.709 | 0480,0481,0482 |

(continued)

| Type of ion | m/z [M+H]+ or [M-H]- | Retention time of MS peak (min) | Attributed compound No. (denoted omitting 2-2-d1E01-2) |
|---|---|---|---|
| M+H | 790.2533 | 12.658 | 0480,0481,0482 |
| M+H | 790.2500 | 16.711 | 0480,0481,0482 |
| M+H | 794.2830 | 13.227 | 0483 |
| M+H | 795.2302 | 15.869 | 0484 |
| M+H | 795.2762 | 16.377 | 0485 |
| M+H | 795.3538 | 15.107 | 0486 |
| M+H | 796.2764 | 15.337 | 0487 |
| M+H | 796.2826 | 16.237 | 0488 |
| M+H | 797.2821 | 15.929 | 0489 |
| M+H | 799.3282 | 14.758 | 0490 |
| M-H | 799.2175 | 11.809 | 0491 |
| M+H | 802.2412 | 16.026 | 0492 |
| M-H | 805.2846 | 16.379 | 0493 |
| M+H | 810.2230 | 12.284 | 0494 |
| M+H | 812.2878 | 14.822 | 0495 |
| M-H | 811.2443 | 15.464 | 0496 |
| M+H | 821.2457 | 16.239 | 0497 |
| M+H | 823.3086 | 15.090 | 0498 |
| M+H | 838.3043 | 15.376 | 0499 |
| M+H | 849.3260 | 15.594 | 0500 |

From the above results, it was shown that, under the reaction conditions shown in the present Example, by carrying out highly selective ethylation of arenol having various reactive functional groups and by performing post-treatment that does not require operations such as column, it is possible to construct a compound library in which 500 compounds or 1000 compounds having diversity in functional groups are mixed.

Note that, as previously mentioned (Examples 6-5-1 and 6-5-2), for the purpose of illustrating the actual practicality of a compound group ethylated by the present approach of the present invention as a mixture library, as well as demonstrating that the desired compound group is obtained, binding evaluation of the synthesized mixture to Bcl-2 using AS-MS (Example 7), individual synthesis of Bcl-2 binding compounds identified by AS-MS (Example 8), and binding evaluation of the individually synthesized compounds to Bcl-2 by surface plasmon resonance (SPR) (Example 9) were performed.

Example 7: Binding evaluation of synthesized mixture library compounds (mixture libraries prepared in Example 6-5-1 and Example 6-5-2, in which the presence of 1152 compounds is confirmed) to Bcl-2 using AS-MS

For one well of MICROPLATE, 96-well, PP, V-BOTTOM (Greiner bio-one, 651201), DMSO solutions of 12 mixtures (prepared to approximately 0.58 mM based on calculations from the average molecular weight of each mixture), in each of which 100 compounds are mixed, were each dispensed, and dispensed into a total of 12 wells, where the 12 mixtures are mixAI-B4J-01, mixAI-B1M-01, mixAI-B1K-01, mixAI-B1O-01, mixAI-B1A-01, mixAI-B3A-01, mixAI-B2I-01, mixAI-B2L-01, mixAI-B2N-01, mixAI-B2A-01, mixAI-B2Q-01, and mixAI-B2R-01 listed in [Table AI-03-B4J-01], [Table AI-03-B1M-01], [Table AI-03-B1K-01], [Table AI-03-B1O-01], [Table AI-03-B1A-01], [Table AI-03-B3A-01], [Table AI-03-B2I-01], [Table AI-03-B2L-01], [Table AI-03-B2N-01], [Table AI-03-B2A-01], [Table AI-03-B2Q-01], and [AI-03-B2R-01] shown in Example 6. Using the LABCYTE ECHO (R) 555 Acoustic Dispensing System (BECKMAN COULTER), 70 nL was collected from each of the 12 wells into one arbitrary well to prepare a sample solution with 1200 compounds in the

mixed state (the presence of 1152 compounds had already been identified when prepared in Example 6-5-3). (In the present specification, the mixture collected in this one well is referred to as one library.) Furthermore, a Bcl-2 protein (Novus, NBP2-34889, Recombinant Human Bcl-2 Protein, Lot E60145051) (19.2 μL) that had been prepared in advance to be 20.9 μM using a Tris-HCl buffer (25 mM Tris-HCl pH 7.4, 150 mM NaCl, 0.025% Tween-20, 1mM DTT) was added to the well in which 1200 compounds (the presence of 1152 compounds had already been identified when prepared in Example 6-5-3) were dispensed (final compound concentration 20 nm, final Bcl-2 concentration 20 μM), and the mixture was incubated in the dark at 23°C for 1 hour. Next, using the 96-well MacroSpin column, P-2 Material (Harvard, 74-5655) that had been swollen, conditioning of the 96-well MacroSpin column was performed three times by centrifugation with 200 μL of an assay buffer (25 mM Tris-HCl pH 7.4, 150 mM NaCl, 0.025% Tween-20, 1 mM DTT), 2000 XG, 4°C, 2 min. Subsequently, 20 μL of the sample after incubation was added to the 96-well MacroSpin column, P-2 Material, that had been conditioned, and SEC treatment was performed by centrifugation at 2000 XG for 2 minutes. To the liquid passed from the SEC, 32 μL of a 1:1 mixed solution of acetonitrile and methanol (LC/MS grade, Fujifilm Wako Pure Chemical Corporation) was added, the mixture was centrifuged at 1800XG, 4°C, 5 min, and the supernatant was used as analysis sample ASMS (A), while a sample that had been prepared by the same operations at 1200 compounds/well and to which only the Tris-HCl buffer had been added without the Bcl-2 protein was incubated under the same conditions, no 96-well MacroSpin column treatment was performed, the same organic solvent was added, and the supernatant solution obtained by centrifugation was used as STD (B). Next, a sample that had been prepared by the same operations at 1200 compounds/well and to which only the Tris-HCl buffer had been added without the Bcl-2 protein was incubated under the same conditions, the same 96-well MacroSpin column treatment was performed, the same organic solvent was added, and the supernatant solution obtained by centrifugation was used as BK (C), and measured by reversed phase LC/MS.

Then, analysis of the 1200 compounds was carried out for samples A, B, and C with TraceFinder 5.1 (Thermo Fisher Scientific) and CHANCE Software (in-house), and the ASMS Ratio (%) was calculated according to the following calculation formula.

$$\text{Calculation formula: ASMS Ratio (\%)} = (A - C)/B \times 100$$

[Table 132]

| Reversed phase LC/MS conditions | |
|---|---|
| Vanquish UHPLC(Thermo Fisher Scientific) | |
| Oven (°C) | 40 |
| Column | Column packed with chemically bonded porous spherical silica gel with surface modified with ODS octadecylsilyl groups as stationary phase |
| Autosampler (°C) | 4 |
| Orbitrap Fusion Lumos Tribrid mass spectrometer (Thermo Fisher Scientific) | |
| Polarity | Positive mode |
| Data Type | profile |

The results are shown in [Table AJ-01].

[Table 133]

[Table AJ-01]

| Compound No. | Molecular formula | m/z (expected) | ASMS ratio(%) | |
|---|---|---|---|---|
| | | | n=1 | n=2 |
| B1A001-01 | C33H39N5O3S | 586.2846 | 17.6 | -0.7 |
| B1A002-01 | C32H38N6O3S | 587.2799 | 0.0 | 4.9 |
| B1A003-01 | C36H43N5O3 | 594.3439 | 7.5 | 5.8 |
| B1A004-01 | C35H42N6O3 | 595.3391 | -6.2 | 2.6 |
| B1A005-01 | C35H40FN5O3 | 598.3188 | 7.0 | -10.7 |
| B1A006-01 | C34H39FN6O3 | 599.314 | 2.4 | 9.1 |
| B1A007-01 | C37H41N5O3 | 604.3282 | 0.0 | na |
| B1A008-01 | C36H40N6O3 | 605.3235 | 3.0 | 0.0 |
| B1A009-01 | C36H40N6O3 | 605.3235 | 3.0 | 0.0 |
| B1A010-01 | C35H39N7O3 | 606.3187 | na | 0.0 |
| B1A011-01 | C37H45N5O3 | 608.3595 | 11.2 | -13.2 |
| B1A012-01 | C36H44N6O3 | 609.3548 | 0.0 | 0.0 |
| B1A013-01 | C39H43N5O3 | 630.3439 | -1.7 | 2.4 |
| B1A014-01 | C38H42N6O3 | 631.3391 | 8.1 | 0.0 |
| B1A015-01 | C39H49N5O3 | 636.3908 | -0.9 | -20.6 |
| B1A016-01 | C38H48N6O3 | 637.3861 | 12.1 | -4.6 |
| B1A017-01 | C36H40F3N5O3 | 648.3156 | 5.4 | 5.4 |
| B1A018-01 | C35H39F3N6O3 | 649.3109 | -6.5 | 9.9 |
| B1A019-01 | C38H40F3N5O3 | 672.3156 | -3.9 | -7.5 |
| B1A020-01 | C37H39F3N6O3 | 673.3109 | -4.3 | -10.5 |
| B1A021-01 | C39H42F3N7O3 | 714.3374 | -1.3 | -3.7 |
| B1A022-01 | C38H41F3N8O3 | 715.3327 | 8.0 | 7.7 |
| B1A023-01 | C35H41N3O3S | 584.2941 | 4.0 | 0.8 |
| B1A024-01 | C34H40N4O3S | 585.2894 | 4.1 | 1.2 |
| B1A025-01 | C38H45N3O3 | 592.3534 | 7.4 | -16.1 |
| B1A026-01 | C37H44N4O3 | 593.3486 | 3.0 | -2.9 |
| B1A027-01 | C37H42FN3O3 | 596.3283 | 6.7 | -7.8 |
| B1A028-01 | C36H41FN4O3 | 597.3236 | -1.6 | -4.7 |
| B1A029-01 | C39H43N3O3 | 602.3377 | -22.3 | -13.0 |
| B1A030-01 | C38H42N4O3 | 603.333 | 6.9 | -1.8 |
| B1A031-01 | C38H42N4O3 | 603.333 | 6.9 | -1.8 |
| B1A032-01 | C37H41N5O3 | 604.3282 | 0.0 | -9.1 |
| B1A033-01 | C39H47N3O3 | 606.369 | 3.3 | -13.3 |
| B1A034-01 | C38H46N4O3 | 607.3643 | 5.2 | -3.2 |
| B1A035-01 | C41H45N3O3 | 628.3534 | 14.9 | -7.0 |
| B1A036-01 | C40H44N4O3 | 629.3486 | 0.4 | 3.1 |
| B1A037-01 | C41H51N3O3 | 634.4003 | 3.3 | -5.0 |
| B1A038-01 | C40H50N4O3 | 635.3956 | 6.5 | -6.4 |
| B1A039-01 | C38H42F3N3O3 | 646.3251 | 6.3 | -8.4 |
| B1A040-01 | C37H41F3N4O3 | 647.3204 | 10.4 | 8.4 |
| B1A041-01 | C40H42F3N3O3 | 670.3251 | -2.4 | -4.0 |
| B1A042-01 | C39H41F3N4O3 | 671.3204 | 12.5 | -8.5 |
| B1A043-01 | C41H44F3N5O3 | 712.3469 | 3.8 | -2.9 |
| B1A044-01 | C40H43F3N6O3 | 713.3422 | 2.6 | -1.9 |
| B1A045-01 | C37H45N3O2 | 564.3585 | 1.1 | -2.2 |
| B1A046-01 | C36H44N4O2 | 565.3537 | 0.0 | 0.0 |
| B1A047-01 | C38H47N3O2 | 578.3741 | 3.2 | 2.5 |
| B1A048-01 | C37H46N4O2 | 579.3694 | 1.5 | 3.6 |
| B1A049-01 | C39H49N3O2 | 592.3898 | 4.1 | -9.1 |
| B1A050-01 | C38H48N4O2 | 593.385 | 0.6 | 3.0 |
| B1A051-01 | C37H44FN3O2 | 582.349 | -5.4 | -4.2 |
| B1A052-01 | C36H43FN4O2 | 583.3443 | 4.2 | -6.2 |
| B1A053-01 | C38H47N3O3 | 594.369 | 13.2 | 4.8 |
| B1A054-01 | C37H46N4O3 | 595.3643 | 0.0 | 0.0 |
| B1A055-01 | C39H49N3O3 | 608.3847 | -0.1 | -14.4 |
| B1A056-01 | C38H48N4O3 | 609.3799 | 9.7 | 4.0 |
| B1A057-01 | C40H51N3O3 | 622.4003 | 0.0 | 0.0 |
| B1A058-01 | C39H50N4O3 | 623.3956 | na | 0.0 |
| B1A059-01 | C39H49N3O4 | 624.3796 | 7.7 | -5.5 |
| B1A060-01 | C38H48N4O4 | 625.3748 | 0.0 | na |
| B1A061-01 | C38H44F3N3O2 | 632.3458 | 5.3 | 1.1 |
| B1A062-01 | C37H43F3N4O2 | 633.3411 | -0.3 | -9.7 |
| B1A063-01 | C38H44F3N3O3 | 648.3408 | -1.0 | -11.0 |
| B1A064-01 | C37H43F3N4O3 | 649.336 | 5.2 | -1.6 |
| B1A065-01 | C41H47N3O2 | 614.3741 | -32.6 | -10.4 |
| B1A066-01 | C40H46N4O2 | 615.3694 | 8.2 | 7.7 |
| B1A067-01 | C35H43N3O2S | 570.3149 | 3.6 | -7.8 |
| B1A068-01 | C34H42N4O2S | 571.3101 | 12.2 | 3.3 |
| B1A069-01 | C39H45N3O2 | 588.3585 | 8.3 | 0.8 |
| B1A070-01 | C38H44N4O2 | 589.3537 | 0.0 | 0.0 |

| Compound No. | Molecular formula | m/z (expected) | ASMS ratio(%) | |
|---|---|---|---|---|
| | | | n=1 | n=2 |
| B1A071-01 | C35H43N5O2 | 566.349 | -0.3 | -3.3 |
| B1A072-01 | C34H42N6O2 | 567.3442 | 3.9 | -0.2 |
| B1A073-01 | C36H45N5O2 | 580.3646 | 3.2 | -4.7 |
| B1A074-01 | C35H44N6O2 | 581.3599 | 3.2 | -0.1 |
| B1A075-01 | C37H47N5O2 | 594.3803 | 31.3 | 28.6 |
| B1A076-01 | C36H46N6O2 | 595.3755 | na | na |
| B1A077-01 | C35H42FN5O2 | 584.3395 | 6.6 | -1.3 |
| B1A078-01 | C34H41FN6O2 | 585.3348 | 4.7 | 1.2 |
| B1A079-01 | C36H45N5O3 | 596.3595 | 3.9 | -3.9 |
| B1A080-01 | C35H44N6O3 | 597.3548 | 3.4 | 0.0 |
| B1A081-01 | C37H47N5O3 | 610.3752 | -10.9 | -5.5 |
| B1A082-01 | C36H46N6O3 | 611.3704 | 3.0 | 0.2 |
| B1A083-01 | C38H49N5O3 | 624.3908 | -0.3 | 0.5 |
| B1A084-01 | C37H48N6O3 | 625.3861 | 2.1 | -2.7 |
| B1A085-01 | C37H47N5O4 | 626.3701 | 10.6 | 10.8 |
| B1A086-01 | C36H46N6O4 | 627.3653 | 0.0 | -14.8 |
| B1A087-01 | C36H42F3N5O2 | 634.3363 | na | na |
| B1A088-01 | C35H41F3N6O2 | 635.3316 | 18.7 | -7.1 |
| B1A089-01 | C36H42F3N5O3 | 650.3313 | 0.0 | na |
| B1A090-01 | C35H41F3N6O3 | 651.3265 | 3.3 | -1.0 |
| B1A091-01 | C39H45N5O2 | 616.3646 | 0.0 | 15.4 |
| B1A092-01 | C38H44N6O2 | 617.3599 | 2.6 | -2.4 |
| B1A093-01 | C33H41N5O2S | 572.3054 | -5.3 | 0.0 |
| B1A094-01 | C32H40N6O2S | 573.3006 | 6.2 | -0.7 |
| B1A095-01 | C37H43N5O2 | 590.349 | 0.3 | -17.1 |
| B1A096-01 | C36H42N6O2 | 591.3442 | 0.0 | 7.4 |
| B1A097-01 | C38H47N3O2 | 578.3741 | 3.2 | 2.5 |
| B1A098-01 | C37H46N4O2 | 579.3694 | na | na |
| B1A099-01 | C38H46FN3O2 | 596.3647 | 0.9 | -0.9 |
| B1A100-01 | C37H45FN4O2 | 597.3599 | 6.4 | 2.3 |
| B1K001-01 | C24H24F3N5O3S | 520.1625 | 0.0 | 0.0 |
| B1K002-01 | C23H23F3N6O3S | 521.1577 | 0.0 | 0.0 |
| B1K003-01 | C27H28F3N5O3 | 528.2217 | 0.1 | -0.2 |
| B1K004-01 | C26H27F3N6O3 | 529.217 | 0.0 | 0.6 |
| B1K005-01 | C26H25F4N5O3 | 532.1966 | -0.1 | 0.6 |
| B1K006-01 | C25H24F4N6O3 | 533.1919 | -0.6 | 0.0 |
| B1K007-01 | C28H26F3N5O3 | 538.2061 | 0.0 | 1.3 |
| B1K008-01 | C27H25F3N6O3 | 539.2013 | 0.0 | 0.0 |
| B1K009-01 | C27H25F3N6O3 | 539.2013 | na | na |
| B1K010-01 | C26H24F3N7O3 | 540.1966 | 0.0 | 0.0 |
| B1K011-01 | C28H30F3N5O3 | 542.2374 | -0.1 | 0.3 |
| B1K012-01 | C27H29F3N6O3 | 543.2326 | 0.0 | -0.4 |
| B1K013-01 | C30H28F3N5O3 | 564.2217 | 0.0 | 1.4 |
| B1K014-01 | C29H27F3N6O3 | 565.217 | -0.3 | 0.0 |
| B1K015-01 | C30H34F3N5O3 | 570.2687 | 2.9 | -1.8 |
| B1K016-01 | C29H33F3N6O3 | 571.2639 | 0.0 | 0.0 |
| B1K017-01 | C27H25F6N5O3 | 582.1934 | 0.0 | 0.0 |
| B1K018-01 | C26H24F6N6O3 | 583.1887 | -0.6 | 0.5 |
| B1K019-01 | C29H25F6N5O3 | 606.1934 | 0.0 | 0.0 |
| B1K020-01 | C28H24F6N6O3 | 607.1887 | 2.9 | -3.8 |
| B1K021-01 | C30H27F6N7O3 | 648.2152 | -0.5 | -1.9 |
| B1K022-01 | C29H26F6N8O3 | 649.2105 | 1.4 | 0.1 |
| B1K023-01 | C26H26F3N3O3S | 518.172 | 4.3 | 1.3 |
| B1K024-01 | C25H25F3N4O3S | 519.1672 | 0.0 | 0.0 |
| B1K025-01 | C29H30F3N3O3 | 526.2312 | 0.3 | -0.8 |
| B1K026-01 | C28H29F3N4O3 | 527.2265 | 4.2 | 0.2 |
| B1K027-01 | C28H27F4N3O3 | 530.2061 | -0.8 | -3.6 |
| B1K028-01 | C27H26F4N4O3 | 531.2014 | 0.0 | 0.0 |
| B1K029-01 | C30H28F3N3O3 | 536.2156 | na | na |
| B1K030-01 | C29H27F3N4O3 | 537.2108 | 0.0 | 0.9 |
| B1K031-01 | C29H27F3N4O3 | 537.2108 | 0.0 | 0.0 |
| B1K032-01 | C28H26F3N5O3 | 538.2061 | na | na |
| B1K033-01 | C30H32F3N3O3 | 540.2469 | 2.3 | -0.6 |
| B1K034-01 | C29H31F3N4O3 | 541.2421 | -0.3 | 0.0 |
| B1K035-01 | C32H30F3N3O3 | 562.2312 | 4.8 | 4.5 |
| B1K036-01 | C31H29F3N4O3 | 563.2265 | 1.1 | -0.4 |
| B1K037-01 | C32H36F3N3O3 | 568.2782 | 0.8 | 1.9 |
| B1K038-01 | C31H35F3N4O3 | 569.2734 | 0.6 | -1.8 |
| B1K039-01 | C29H27F6N3O3 | 580.2029 | 3.5 | 3.9 |
| B1K040-01 | C28H26F6N4O3 | 581.1982 | 0.0 | 0.0 |

na = not available

[Table 134]

| Compound No. | Molecular formula | m/z (expected) | ASMS ratio(%) n=1 | ASMS ratio(%) n=2 |
|---|---|---|---|---|
| B1K041-01 | C31H27F6N3O3 | 604.2029 | 0.0 | 0.0 |
| B1K042-01 | C30H26F6N4O3 | 605.1982 | 5.0 | -0.1 |
| B1K043-01 | C32H29F6N5O3 | 646.2247 | 3.8 | 1.9 |
| B1K044-01 | C31H28F6N6O3 | 647.22 | 2.6 | 1.0 |
| B1K045-01 | C28H30F3N3O2 | 498.2363 | 1.5 | -0.1 |
| B1K046-01 | C27H29F3N4O2 | 499.2315 | -0.1 | 0.5 |
| B1K047-01 | C29H32F3N3O2 | 512.2519 | 3.3 | 0.5 |
| B1K048-01 | C28H31F3N4O2 | 513.2472 | 0.1 | 1.2 |
| B1K049-01 | C30H34F3N3O2 | 526.2676 | -3.3 | 1.5 |
| B1K050-01 | C29H33F3N4O2 | 527.2628 | 0.0 | 0.0 |
| B1K051-01 | C28H29F4N3O2 | 516.2269 | 2.8 | 2.0 |
| B1K052-01 | C27H28F4N4O2 | 517.2221 | -3.3 | 0.5 |
| B1K053-01 | C29H32F3N3O3 | 528.2469 | -0.5 | -3.3 |
| B1K054-01 | C28H31F3N4O3 | 529.2421 | 0.0 | 0.0 |
| B1K055-01 | C30H34F3N3O3 | 542.2625 | -0.8 | 0.0 |
| B1K056-01 | C29H33F3N4O3 | 543.2578 | 3.3 | 0.2 |
| B1K057-01 | C31H36F3N3O3 | 556.2782 | 1.0 | -2.6 |
| B1K058-01 | C30H35F3N4O3 | 557.2734 | 1.3 | 0.1 |
| B1K059-01 | C30H34F3N3O4 | 558.2574 | 3.0 | -1.9 |
| B1K060-01 | C29H33F3N4O4 | 559.2527 | 0.0 | 0.0 |
| B1K061-01 | C29H29F6N3O2 | 566.2237 | 2.8 | -1.3 |
| B1K062-01 | C28H28F6N4O2 | 567.2189 | 1.2 | -0.3 |
| B1K063-01 | C29H29F6N3O3 | 582.2186 | 3.9 | 0.4 |
| B1K064-01 | C28H28F6N4O3 | 583.2138 | 1.4 | 0.7 |
| B1K065-01 | C32H32F3N3O2 | 548.2519 | 5.9 | -5.6 |
| B1K066-01 | C31H31F3N4O2 | 549.2472 | 1.8 | -0.7 |
| B1K067-01 | C26H28F3N3O2S | 504.1927 | 5.1 | 1.8 |
| B1K068-01 | C25H27F3N4O2S | 505.188 | 2.6 | -1.4 |
| B1K069-01 | C30H30F3N3O2 | 522.2363 | 3.5 | -1.4 |
| B1K070-01 | C29H29F3N4O2 | 523.2315 | 0.0 | 0.0 |
| B1K071-01 | C26H28F3N5O2 | 500.2268 | 1.6 | -0.9 |
| B1K072-01 | C25H27F3N6O2 | 501.222 | -0.6 | 1.7 |
| B1K073-01 | C27H30F3N5O2 | 514.2424 | 3.1 | -1.9 |
| B1K074-01 | C26H29F3N6O2 | 515.2377 | 0.0 | 0.0 |
| B1K075-01 | C28H32F3N5O2 | 528.2581 | 1.4 | -3.8 |
| B1K076-01 | C27H31F3N6O2 | 529.2533 | na | 0.0 |
| B1K077-01 | C26H27F4N5O2 | 518.2174 | 2.4 | -0.2 |
| B1K078-01 | C25H26F4N6O2 | 519.2126 | 0.8 | -0.2 |
| B1K079-01 | C27H30F3N5O3 | 530.2374 | 2.2 | -1.3 |
| B1K080-01 | C26H29F3N6O3 | 531.2326 | 0.0 | 0.0 |
| B1K081-01 | C28H32F3N5O3 | 544.253 | 2.3 | -0.4 |
| B1K082-01 | C27H31F3N6O3 | 545.2483 | 0.0 | 0.0 |
| B1K083-01 | C29H34F3N5O3 | 558.2687 | 0.0 | 0.0 |
| B1K084-01 | C28H33F3N6O3 | 559.2639 | 0.0 | 0.0 |
| B1K085-01 | C28H32F3N5O4 | 560.2479 | 4.2 | -0.2 |
| B1K086-01 | C27H31F3N6O4 | 561.2432 | na | 0.0 |
| B1K087-01 | C27H27F6N5O2 | 568.2142 | 1.8 | -0.1 |
| B1K088-01 | C26H26F6N6O2 | 569.2094 | 2.2 | -0.2 |
| B1K089-01 | C27H27F6N5O3 | 584.2091 | 2.0 | 1.0 |
| B1K090-01 | C26H26F6N6O3 | 585.2043 | -0.6 | -1.9 |
| B1K091-01 | C30H30F3N5O2 | 550.2424 | 3.6 | -4.2 |
| B1K092-01 | C29H29F3N6O2 | 551.2377 | 4.1 | -0.7 |
| B1K093-01 | C24H26F3N5O2S | 506.1832 | 2.7 | -0.4 |
| B1K094-01 | C23H25F3N6O2S | 507.1785 | 1.4 | -0.1 |
| B1K095-01 | C28H28F3N5O2 | 524.2268 | 3.5 | -0.6 |
| B1K096-01 | C27H27F3N6O2 | 525.222 | 0.0 | 0.0 |
| B1K097-01 | C29H32F3N3O2 | 512.2519 | -2.1 | -3.1 |
| B1K098-01 | C28H31F3N4O2 | 513.2472 | -1.1 | -2.4 |
| B1K099-01 | C29H31F4N3O2 | 530.2425 | 0.0 | 0.0 |
| B1K100-01 | C28H30F4N4O2 | 531.2378 | 0.0 | 0.0 |
| B1M001-01 | C25H29N5O3S | 480.2064 | 0.0 | 0.0 |
| B1M002-01 | C24H28N6O3S | 481.2016 | -0.7 | -1.5 |
| B1M003-01 | C28H33N5O3 | 488.2656 | -1.9 | 2.0 |
| B1M004-01 | C27H32N6O3 | 489.2609 | 0.0 | 0.0 |
| B1M005-01 | C27H30FN5O3 | 492.2405 | 0.0 | 1.4 |
| B1M006-01 | C26H29FN6O3 | 493.2358 | 0.0 | 0.0 |
| B1M007-01 | C29H31N5O3 | 498.25 | 0.3 | 0.3 |
| B1M008-01 | C28H30N6O3 | 499.2452 | na | 0.0 |
| B1M009-01 | C28H30N6O3 | 499.2452 | na | 0.0 |
| B1M010-01 | C27H29N7O3 | 500.2405 | 0.0 | 0.0 |

| Compound No. | Molecular formula | m/z (expected) | ASMS ratio(%) n=1 | ASMS ratio(%) n=2 |
|---|---|---|---|---|
| B1M011-01 | C29H35N5O3 | 502.2813 | 18.6 | 7.5 |
| B1M012-01 | C28H34N6O3 | 503.2765 | -3.5 | -2.3 |
| B1M013-01 | C31H33N5O3 | 524.2656 | 2.9 | -1.1 |
| B1M014-01 | C30H32N6O3 | 525.2609 | 4.1 | 0.4 |
| B1M015-01 | C31H39N5O3 | 530.3126 | -2.4 | -3.0 |
| B1M016-01 | C30H38N6O3 | 531.3078 | 0.0 | 0.0 |
| B1M017-01 | C28H30F3N5O3 | 542.2374 | -0.1 | 0.3 |
| B1M018-01 | C27H29F3N6O3 | 543.2326 | 0.0 | 0.0 |
| B1M019-01 | C30H30F3N5O3 | 566.2374 | 1.5 | 0.0 |
| B1M020-01 | C29H29F3N6O3 | 567.2326 | 0.0 | 0.0 |
| B1M021-01 | C31H32F3N7O3 | 608.2592 | 0.0 | 0.0 |
| B1M022-01 | C30H31F3N8O3 | 609.2544 | -13.6 | 6.7 |
| B1M023-01 | C27H31N3O3S | 478.2159 | 1.4 | -3.0 |
| B1M024-01 | C26H30N4O3S | 479.2111 | 0.0 | 0.0 |
| B1M025-01 | C30H35N3O3 | 486.2751 | 3.6 | -1.2 |
| B1M026-01 | C29H34N4O3 | 487.2704 | 0.9 | 0.9 |
| B1M027-01 | C29H32FN3O3 | 490.2501 | 1.0 | 0.4 |
| B1M028-01 | C28H31FN4O3 | 491.2453 | 0.0 | 0.0 |
| B1M029-01 | C31H33N3O3 | 496.2595 | 3.0 | -0.6 |
| B1M030-01 | C30H32N4O3 | 497.2547 | 0.0 | -5.9 |
| B1M031-01 | C30H32N4O3 | 497.2547 | 0.0 | -2.4 |
| B1M032-01 | C29H31N5O3 | 498.25 | 0.3 | 0.3 |
| B1M033-01 | C31H37N3O3 | 500.2908 | 0.3 | 0.0 |
| B1M034-01 | C30H36N4O3 | 501.286 | -0.3 | -0.2 |
| B1M035-01 | C33H35N3O3 | 522.2751 | 2.9 | -1.7 |
| B1M036-01 | C32H34N4O3 | 523.2704 | 6.7 | -1.2 |
| B1M037-01 | C33H41N3O3 | 528.3221 | -1.5 | 2.3 |
| B1M038-01 | C32H40N4O3 | 529.3173 | 0.0 | 1.7 |
| B1M039-01 | C30H32F3N3O3 | 540.2469 | 2.3 | -0.6 |
| B1M040-01 | C29H31F3N4O3 | 541.2421 | -0.3 | 0.0 |
| B1M041-01 | C32H32F3N3O3 | 564.2469 | 0.7 | -3.5 |
| B1M042-01 | C31H31F3N4O3 | 565.2421 | 0.0 | 0.0 |
| B1M043-01 | C33H34F3N5O3 | 606.2687 | 2.2 | -3.1 |
| B1M044-01 | C32H33F3N6O3 | 607.2639 | 1.0 | 1.1 |
| B1M045-01 | C29H35N3O2 | 458.2802 | 2.5 | -2.3 |
| B1M046-01 | C28H34N4O2 | 459.2755 | 0.7 | -1.2 |
| B1M047-01 | C30H37N3O2 | 472.2959 | 1.1 | 0.3 |
| B1M048-01 | C29H36N4O2 | 473.2911 | 0.9 | 0.3 |
| B1M049-01 | C31H39N3O2 | 486.3115 | -0.9 | -0.4 |
| B1M050-01 | C30H38N4O2 | 487.3068 | -2.0 | -0.2 |
| B1M051-01 | C29H34FN3O2 | 476.2708 | 0.7 | 1.9 |
| B1M052-01 | C28H33FN4O2 | 477.266 | 27.6 | 20.2 |
| B1M053-01 | C30H37N3O3 | 488.2908 | -3.0 | -3.1 |
| B1M054-01 | C29H36N4O3 | 489.286 | 0.9 | 0.0 |
| B1M055-01 | C31H39N3O3 | 502.3064 | 3.9 | -1.2 |
| B1M056-01 | C30H38N4O3 | 503.3017 | 0.5 | 0.0 |
| B1M057-01 | C32H41N3O3 | 516.3221 | -0.7 | 1.4 |
| B1M058-01 | C31H40N4O3 | 517.3173 | -1.4 | -1.5 |
| B1M059-01 | C31H39N3O4 | 518.3013 | -5.5 | -7.2 |
| B1M060-01 | C30H38N4O4 | 519.2966 | 0.0 | 0.0 |
| B1M061-01 | C30H34F3N3O2 | 526.2676 | -3.3 | -13.1 |
| B1M062-01 | C29H33F3N4O2 | 527.2628 | 1.6 | -1.7 |
| B1M063-01 | C30H34F3N3O3 | 542.2625 | 0.4 | -1.1 |
| B1M064-01 | C29H33F3N4O3 | 543.2578 | 5.4 | -4.3 |
| B1M065-01 | C33H37N3O2 | 508.2959 | 0.2 | -6.4 |
| B1M066-01 | C32H36N4O2 | 509.2911 | 0.0 | 4.4 |
| B1M067-01 | C27H33N3O2S | 464.2366 | -1.6 | -87.4 |
| B1M068-01 | C26H32N4O2S | 465.2319 | 1.1 | 0.0 |
| B1M069-01 | C31H35N3O2 | 482.2802 | -0.2 | -4.4 |
| B1M070-01 | C30H34N4O2 | 483.2755 | 0.0 | 0.0 |
| B1M071-01 | C27H33N5O2 | 460.2707 | 34.0 | 24.4 |
| B1M072-01 | C26H32N6O2 | 461.266 | 0.0 | 1.9 |
| B1M073-01 | C28H35N5O2 | 474.2864 | 0.4 | -0.5 |
| B1M074-01 | C27H34N6O2 | 475.2816 | -1.6 | 0.9 |
| B1M075-01 | C29H37N5O2 | 488.302 | 77.3 | 46.1 |
| B1M076-01 | C28H36N6O2 | 489.2973 | 0.5 | 1.5 |
| B1M077-01 | C27H32FN5O2 | 478.2613 | -0.7 | 3.1 |
| B1M078-01 | C26H31FN6O2 | 479.2565 | 0.0 | 0.0 |
| B1M079-01 | C28H35N5O3 | 490.2813 | 8.1 | 0.0 |
| B1M080-01 | C27H34N6O3 | 491.2765 | 0.0 | 0.0 |

na = not available

[Table 135]

| Compound No. | Molecular formula | m/z (expected) | ASMS ratio(%) | |
|---|---|---|---|---|
| | | | n=1 | n=2 |
| B1M081-01 | C29H37N5O3 | 504.2969 | 0.0 | 0.0 |
| B1M082-01 | C28H36N6O3 | 505.2922 | 0.0 | 0.0 |
| B1M083-01 | C30H39N5O3 | 518.3126 | 0.0 | -0.3 |
| B1M084-01 | C29H38N6O3 | 519.3078 | 0.0 | 0.0 |
| B1M085-01 | C29H37N5O4 | 520.2918 | 0.0 | 0.0 |
| B1M086-01 | C28H36N6O4 | 521.2871 | 0.0 | 0.0 |
| B1M087-01 | C28H32F3N5O2 | 528.2581 | 3.8 | 3.2 |
| B1M088-01 | C27H31F3N6O2 | 529.2533 | 0.0 | 0.0 |
| B1M089-01 | C28H32F3N5O3 | 544.253 | 3.9 | 0.2 |
| B1M090-01 | C27H31F3N6O3 | 545.2483 | 0.0 | 0.0 |
| B1M091-01 | C31H35N5O2 | 510.2864 | 0.0 | 0.0 |
| B1M092-01 | C30H34N6O2 | 511.2816 | na | 0.0 |
| B1M093-01 | C25H31N5O2S | 466.2271 | 0.0 | 0.0 |
| B1M094-01 | C24H30N6O2S | 467.2224 | -1.5 | -2.1 |
| B1M095-01 | C29H33N5O2 | 484.2707 | 0.0 | 0.0 |
| B1M096-01 | C28H32N6O2 | 485.266 | na | 0.0 |
| B1M097-01 | C30H37N3O2 | 472.2959 | 1.1 | 0.3 |
| B1M098-01 | C29H36N4O2 | 473.2911 | 0.9 | 0.3 |
| B1M099-01 | C30H36FN3O2 | 490.2864 | -0.7 | -0.3 |
| B1M100-01 | C29H35FN4O2 | 491.2817 | 0.5 | -1.5 |
| B10001-01 | C29H29N5O4S | 544.2013 | -0.1 | -3.1 |
| B10002-01 | C28H28N6O4S | 545.1966 | 2.2 | 2.6 |
| B10003-01 | C32H33N5O4 | 552.2605 | 0.0 | 0.0 |
| B10004-01 | C31H32N6O4 | 553.2558 | 2.8 | 0.4 |
| B10005-01 | C31H30FN5O4 | 556.2355 | 3.6 | -2.5 |
| B10006-01 | C30H29FN6O4 | 557.2307 | 4.7 | 0.8 |
| B10007-01 | C33H31N5O4 | 562.2449 | 4.5 | 0.9 |
| B10008-01 | C32H30N6O4 | 563.2401 | 1.1 | 1.3 |
| B10009-01 | C32H30N6O4 | 563.2401 | 1.1 | 1.3 |
| B10010-01 | C31H29N7O4 | 564.2354 | 0.0 | 0.0 |
| B10011-01 | C33H35N5O4 | 566.2762 | 0.0 | 4.3 |
| B10012-01 | C32H34N6O4 | 567.2714 | 0.8 | -0.9 |
| B10013-01 | C35H33N5O4 | 588.2605 | 2.2 | -4.0 |
| B10014-01 | C34H32N6O4 | 589.2558 | 2.5 | 0.2 |
| B10015-01 | C35H39N5O4 | 594.3075 | 6.0 | -2.7 |
| B10016-01 | C34H38N6O4 | 595.3027 | 6.6 | 0.7 |
| B10017-01 | C32H30F3N5O4 | 606.2323 | 2.5 | 0.0 |
| B10018-01 | C31H29F3N6O4 | 607.2275 | 1.0 | 3.2 |
| B10019-01 | C34H30F3N5O4 | 630.2323 | 0.6 | 0.2 |
| B10020-01 | C33H29F3N6O4 | 631.2275 | 8.6 | -0.5 |
| B10021-01 | C35H32F3N7O4 | 672.2541 | 2.9 | -1.2 |
| B10022-01 | C34H31F3N8O4 | 673.2493 | 5.6 | -1.2 |
| B10023-01 | C31H31N3O4S | 542.2108 | 12.4 | -0.3 |
| B10024-01 | C30H30N4O4S | 543.2061 | 3.6 | 2.3 |
| B10025-01 | C34H35N3O4 | 550.27 | -0.4 | -4.5 |
| B10026-01 | C33H34N4O4 | 551.2653 | 1.7 | -1.5 |
| B10027-01 | C33H32FN3O4 | 554.245 | 3.8 | 2.1 |
| B10028-01 | C32H31FN4O4 | 555.2402 | 4.9 | 0.4 |
| B10029-01 | C35H33N3O4 | 560.2544 | 0.0 | 0.0 |
| B10030-01 | C34H32N4O4 | 561.2496 | 1.8 | -2.0 |
| B10031-01 | C34H32N4O4 | 561.2496 | -1.3 | -4.1 |
| B10032-01 | C33H31N5O4 | 562.2449 | 4.5 | 0.9 |
| B10033-01 | C35H37N3O4 | 564.2857 | 1.9 | 4.2 |
| B10034-01 | C34H36N4O4 | 565.2809 | 0.1 | 0.2 |
| B10035-01 | C37H35N3O4 | 586.27 | 4.3 | -3.7 |
| B10036-01 | C36H34N4O4 | 587.2653 | 4.8 | 0.9 |
| B10037-01 | C37H41N3O4 | 592.317 | 4.7 | -1.7 |
| B10038-01 | C36H40N4O4 | 593.3122 | 2.8 | -0.7 |
| B10039-01 | C34H32F3N3O4 | 604.2418 | 5.1 | 0.2 |
| B10040-01 | C33H31F3N4O4 | 605.237 | 1.8 | 0.3 |
| B10041-01 | C36H32F3N3O4 | 628.2418 | 10.4 | -2.8 |
| B10042-01 | C35H31F3N4O4 | 629.237 | 2.8 | 2.1 |
| B10043-01 | C37H34F3N5O4 | 670.2636 | 2.2 | -5.0 |
| B10044-01 | C36H33F3N6O4 | 671.2588 | 5.8 | 0.4 |
| B10045-01 | C33H35N3O3 | 522.2751 | 0.0 | 0.0 |
| B10046-01 | C32H34N4O3 | 523.2704 | 0.6 | 1.3 |
| B10047-01 | C34H37N3O3 | 536.2908 | 4.1 | 1.5 |
| B10048-01 | C33H36N4O3 | 537.286 | -0.3 | -1.6 |
| B10049-01 | C35H39N3O3 | 550.3064 | 6.1 | 0.0 |
| B10050-01 | C34H38N4O3 | 551.3017 | 10.3 | -5.8 |
| B10051-01 | C33H34FN3O3 | 540.2657 | 3.9 | -1.9 |
| B10052-01 | C32H33FN4O3 | 541.261 | 1.6 | 0.4 |
| B10053-01 | C34H37N3O4 | 552.2857 | 2.2 | -1.0 |
| B10054-01 | C33H36N4O4 | 553.2809 | 2.6 | 3.2 |
| B10055-01 | C35H39N3O4 | 566.3013 | 3.0 | -4.2 |
| B10056-01 | C34H38N4O4 | 567.2966 | 6.7 | -0.9 |
| B10057-01 | C36H41N3O4 | 580.317 | 4.1 | -2.2 |
| B10058-01 | C35H40N4O4 | 581.3122 | -3.5 | 4.4 |
| B10059-01 | C35H39N3O5 | 582.2963 | 2.0 | -1.9 |
| B10060-01 | C34H38N4O5 | 583.2915 | 0.6 | -1.8 |
| B10061-01 | C34H34F3N3O3 | 590.2625 | 1.9 | -1.5 |
| B10062-01 | C33H33F3N4O3 | 591.2578 | 1.3 | 4.6 |
| B10063-01 | C34H34F3N3O4 | 606.2574 | 3.5 | -4.1 |
| B10064-01 | C33H33F3N4O4 | 607.2527 | 0.7 | -4.0 |
| B10065-01 | C37H37N3O3 | 572.2908 | 7.5 | 0.8 |
| B10066-01 | C36H36N4O3 | 573.286 | 12.1 | 2.7 |
| B10067-01 | C31H33N3O3S | 528.2315 | 0.0 | 0.0 |
| B10068-01 | C30H32N4O3S | 529.2268 | 1.9 | -1.1 |
| B10069-01 | C35H35N3O3 | 546.2751 | 6.2 | -2.4 |
| B10070-01 | C34H34N4O3 | 547.2704 | 8.8 | 2.3 |
| B10071-01 | C31H33N5O3 | 524.2656 | 2.9 | -1.1 |
| B10072-01 | C30H32N6O3 | 525.2609 | 4.1 | 0.4 |
| B10073-01 | C32H35N5O3 | 538.2813 | 2.0 | 3.7 |
| B10074-01 | C31H34N6O3 | 539.2765 | 3.9 | -0.9 |
| B10075-01 | C33H37N5O3 | 552.2969 | 9.3 | 0.8 |
| B10076-01 | C32H36N6O3 | 553.2922 | 4.4 | 1.1 |
| B10077-01 | C31H32FN5O3 | 542.2562 | 4.1 | 0.4 |
| B10078-01 | C30H31FN6O3 | 543.2514 | 4.8 | 1.8 |
| B10079-01 | C32H35N5O4 | 554.2762 | 4.5 | 1.3 |
| B10080-01 | C31H34N6O4 | 555.2714 | 1.4 | 3.4 |
| B10081-01 | C33H37N5O4 | 568.2918 | 3.2 | -2.2 |
| B10082-01 | C32H36N6O4 | 569.2871 | 1.5 | 2.6 |
| B10083-01 | C34H39N5O4 | 582.3075 | 7.2 | 1.0 |
| B10084-01 | C33H38N6O4 | 583.3027 | 6.8 | 2.0 |
| B10085-01 | C33H37N5O5 | 584.2868 | -1.1 | -3.1 |
| B10086-01 | C32H36N6O5 | 585.282 | na | -0.4 |
| B10087-01 | C32H32F3N5O3 | 592.253 | 2.2 | -0.5 |
| B10088-01 | C31H31F3N6O3 | 593.2483 | 3.1 | 1.4 |
| B10089-01 | C32H32F3N5O4 | 608.2479 | 3.8 | -5.3 |
| B10090-01 | C31H31F3N6O4 | 609.2432 | 3.3 | -2.0 |
| B10091-01 | C35H35N5O3 | 574.2813 | 4.5 | -2.2 |
| B10092-01 | C34H34N6O3 | 575.2765 | 6.3 | -1.4 |
| B10093-01 | C29H31N5O3S | 530.222 | 0.0 | 0.6 |
| B10094-01 | C28H30N6O3S | 531.2173 | 2.2 | -0.7 |
| B10095-01 | C33H33N5O3 | 548.2656 | 2.7 | 0.0 |
| B10096-01 | C32H32N6O3 | 549.2609 | 0.0 | 3.5 |
| B10097-01 | C34H37N3O3 | 536.2908 | 4.2 | 2.2 |
| B10098-01 | C33H36N4O3 | 537.286 | -42.7 | -0.2 |
| B10099-01 | C34H36FN3O3 | 554.2814 | 4.7 | 1.7 |
| B10100-01 | C33H35FN4O3 | 555.2766 | -19.7 | 11.1 |
| B3A001-01 | C34H41N5O3S | 600.3003 | 0.0 | -8.4 |
| B3A002-01 | C33H40N6O3S | 601.2955 | 1.9 | -1.2 |
| B3A003-01 | C37H45N5O3 | 608.3595 | 6.5 | 4.9 |
| B3A004-01 | C36H44N6O3 | 609.3548 | 3.5 | -0.5 |
| B3A005-01 | C36H42FN5O3 | 612.3344 | -1.5 | -4.8 |
| B3A006-01 | C35H41FN6O3 | 613.3297 | 0.0 | 0.0 |
| B3A007-01 | C38H43N5O3 | 618.3439 | 9.1 | -5.1 |
| B3A008-01 | C37H42N6O3 | 619.3391 | 0.6 | -0.3 |
| B3A009-01 | C37H42N6O3 | 619.3391 | 0.6 | -0.3 |
| B3A010-01 | C36H41N7O3 | 620.3344 | 0.0 | 0.0 |
| B3A011-01 | C38H47N5O3 | 622.3752 | 4.5 | 2.0 |
| B3A012-01 | C37H46N6O3 | 623.3704 | 1.4 | -2.7 |
| B3A013-01 | C40H45N5O3 | 644.3595 | 10.2 | -20.0 |
| B3A014-01 | C39H44N6O3 | 645.3548 | 0.6 | 0.8 |
| B3A015-01 | C40H51N5O3 | 650.4065 | 8.9 | 3.0 |
| B3A016-01 | C39H50N6O3 | 651.4017 | 2.8 | 0.1 |
| B3A017-01 | C37H42F3N5O3 | 662.3313 | 6.9 | -6.3 |
| B3A018-01 | C36H41F3N6O3 | 663.3265 | 5.0 | 1.8 |
| B3A019-01 | C39H42F3N5O3 | 686.3313 | 2.6 | -2.3 |
| B3A020-01 | C38H41F3N6O3 | 687.3265 | 7.5 | -3.2 |

na = not available

[Table 136]

| Compound No. | Molecular formula | m/z (expected) | ASMS ratio(%) | |
|---|---|---|---|---|
| | | | n=1 | n=2 |
| B3A021-01 | C40H44F3N7O3 | 728.3531 | 12.5 | -8.4 |
| B3A022-01 | C39H43F3N8O3 | 729.3483 | 4.9 | 7.2 |
| B3A023-01 | C36H43N3O3S | 598.3098 | 2.4 | 1.7 |
| B3A024-01 | C35H42N4O3S | 599.305 | 1.6 | -0.4 |
| B3A025-01 | C39H47N3O3 | 606.369 | 15.7 | -19.6 |
| B3A026-01 | C38H46N4O3 | 607.3643 | 4.4 | 0.6 |
| B3A027-01 | C38H44FN3O3 | 610.344 | -0.7 | -1.6 |
| B3A028-01 | C37H43FN4O3 | 611.3392 | 9.8 | 1.6 |
| B3A029-01 | C40H45N3O3 | 616.3534 | 0.0 | 0.0 |
| B3A030-01 | C39H44N4O3 | 617.3486 | 8.4 | -15.2 |
| B3A031-01 | C39H44N4O3 | 617.3486 | 8.4 | -15.2 |
| B3A032-01 | C38H43N5O3 | 618.3439 | 9.1 | -5.1 |
| B3A033-01 | C40H49N3O3 | 620.3847 | 1.1 | -0.6 |
| B3A034-01 | C39H48N4O3 | 621.3799 | 1.3 | -0.6 |
| B3A035-01 | C42H47N3O3 | 642.369 | 9.7 | 4.4 |
| B3A036-01 | C41H46N4O3 | 643.3643 | 2.8 | -3.3 |
| B3A037-01 | C42H53N3O3 | 648.416 | 0.0 | 0.0 |
| B3A038-01 | C41H52N4O3 | 649.4112 | 2.5 | -2.9 |
| B3A039-01 | C39H44F3N3O3 | 660.3408 | 3.8 | 0.0 |
| B3A040-01 | C38H43F3N4O3 | 661.336 | 3.5 | -6.7 |
| B3A041-01 | C41H44F3N3O3 | 684.3408 | na | 425.9 |
| B3A042-01 | C40H43F3N4O3 | 685.336 | -0.2 | -1.2 |
| B3A043-01 | C42H46F3N5O3 | 726.3626 | 0.5 | -0.3 |
| B3A044-01 | C41H45F3N6O3 | 727.3578 | 12.3 | -12.4 |
| B3A045-01 | C38H47N3O2 | 578.3741 | 3.2 | 2.5 |
| B3A046-01 | C37H46N4O2 | 579.3694 | 1.5 | 3.6 |
| B3A047-01 | C39H49N3O2 | 592.3898 | 1.8 | -5.9 |
| B3A048-01 | C38H48N4O2 | 593.385 | 7.3 | -8.8 |
| B3A049-01 | C40H51N3O2 | 606.4054 | na | na |
| B3A050-01 | C39H50N4O2 | 607.4007 | 6.1 | -4.3 |
| B3A051-01 | C38H46FN3O2 | 596.3647 | 21.3 | -4.2 |
| B3A052-01 | C37H45FN4O2 | 597.3599 | na | na |
| B3A053-01 | C39H49N3O3 | 608.3847 | 12.5 | 9.9 |
| B3A054-01 | C38H48N4O3 | 609.3799 | 0.0 | 0.0 |
| B3A055-01 | C40H51N3O3 | 622.4003 | 10.8 | -12.7 |
| B3A056-01 | C39H50N4O3 | 623.3956 | na | 0.0 |
| B3A057-01 | C41H53N3O3 | 636.416 | 6.3 | 2.5 |
| B3A058-01 | C40H52N4O3 | 637.4112 | 5.9 | -7.6 |
| B3A059-01 | C40H51N3O4 | 638.3952 | -1.9 | -8.0 |
| B3A060-01 | C39H50N4O4 | 639.3905 | 0.9 | 0.0 |
| B3A061-01 | C39H46F3N3O2 | 646.3615 | na | 421.3 |
| B3A062-01 | C38H45F3N4O2 | 647.3567 | 2.2 | 2.8 |
| B3A063-01 | C39H46F3N3O3 | 662.3564 | na | 390.0 |
| B3A064-01 | C38H45F3N4O3 | 663.3517 | -7.5 | -5.5 |
| B3A065-01 | C42H49N3O2 | 628.3898 | 0.0 | 0.0 |
| B3A066-01 | C41H48N4O2 | 629.385 | 6.3 | -2.9 |
| B3A067-01 | C36H45N3O2S | 584.3305 | -7.3 | 4.0 |
| B3A068-01 | C35H44N4O2S | 585.3258 | 1.4 | -3.6 |
| B3A069-01 | C40H47N3O2 | 602.3741 | na | 12872.1 |
| B3A070-01 | C39H46N4O2 | 603.3694 | 7.7 | -8.1 |
| B3A071-01 | C36H45N5O2 | 580.3646 | 3.2 | -4.7 |
| B3A072-01 | C35H44N6O2 | 581.3599 | 11.6 | 3.1 |
| B3A073-01 | C37H47N5O2 | 594.3803 | 31.3 | 28.6 |
| B3A074-01 | C36H46N6O2 | 595.3755 | 10.7 | -17.2 |
| B3A075-01 | C38H49N5O2 | 608.3959 | -5.8 | -16.0 |
| B3A076-01 | C37H48N6O2 | 609.3912 | 6.5 | -0.9 |
| B3A077-01 | C36H44FN5O2 | 598.3552 | 4.1 | -2.8 |
| B3A078-01 | C35H43FN6O2 | 599.3504 | 4.5 | -1.5 |
| B3A079-01 | C37H47N5O3 | 610.3752 | -5.8 | 5.4 |
| B3A080-01 | C36H46N6O3 | 611.3704 | 1.7 | -1.8 |
| B3A081-01 | C38H49N5O3 | 624.3908 | 13.4 | -14.2 |
| B3A082-01 | C37H48N6O3 | 625.3861 | -0.9 | 4.4 |
| B3A083-01 | C39H51N5O3 | 638.4065 | 3.9 | -18.6 |
| B3A084-01 | C38H50N6O3 | 639.4017 | -17.6 | -7.4 |
| B3A085-01 | C38H49N5O4 | 640.3857 | 5.3 | -7.1 |
| B3A086-01 | C37H48N6O4 | 641.381 | -2.7 | -1.9 |
| B3A087-01 | C37H44F3N5O2 | 648.352 | 0.4 | 1.4 |
| B3A088-01 | C36H43F3N6O2 | 649.3472 | 2.5 | 2.4 |
| B3A089-01 | C37H44F3N5O3 | 664.3469 | -5.5 | -7.2 |
| B3A090-01 | C36H43F3N6O3 | 665.3422 | 5.8 | -0.2 |
| B3A091-01 | C40H47N5O2 | 630.3803 | -1.0 | -16.5 |
| B3A092-01 | C39H46N6O2 | 631.3755 | 1.1 | -1.9 |
| B3A093-01 | C34H43N5O2S | 586.321 | -3.8 | 0.0 |
| B3A094-01 | C33H42N6O2S | 587.3163 | 3.7 | 0.5 |
| B3A095-01 | C38H45N5O2 | 604.3646 | -4.9 | -3.5 |
| B3A096-01 | C37H44N6O2 | 605.3599 | 7.7 | 2.4 |
| B3A097-01 | C39H49N3O2 | 592.3898 | 1.8 | -5.9 |
| B3A098-01 | C38H48N4O2 | 593.385 | 0.6 | 3.0 |
| B3A099-01 | C39H48FN3O2 | 610.3803 | -2.6 | 25.2 |
| B3A100-01 | C38H47FN4O2 | 611.3756 | -39.8 | 0.0 |
| B4J001-01 | C26H31N5O3S | 494.222 | 0.0 | 0.0 |
| B4J002-01 | C25H30N6O3S | 495.2173 | -2.7 | 2.1 |
| B4J003-01 | C29H35N5O3 | 502.2813 | 0.0 | 0.0 |
| B4J004-01 | C28H34N6O3 | 503.2765 | -2.2 | 0.4 |
| B4J005-01 | C28H32FN5O3 | 506.2562 | -0.4 | 2.1 |
| B4J006-01 | C27H31FN6O3 | 507.2514 | 0.0 | 0.0 |
| B4J007-01 | C30H33N5O3 | 512.2656 | -1.0 | 1.5 |
| B4J008-01 | C29H32N6O3 | 513.2609 | 0.0 | 0.0 |
| B4J009-01 | C29H32N6O3 | 513.2609 | 0.0 | 0.0 |
| B4J010-01 | C28H31N7O3 | 514.2561 | 0.0 | 2.3 |
| B4J011-01 | C30H37N5O3 | 516.2969 | -0.1 | -0.9 |
| B4J012-01 | C29H36N6O3 | 517.2922 | -1.1 | -0.8 |
| B4J013-01 | C32H35N5O3 | 538.2813 | 2.0 | 3.7 |
| B4J014-01 | C31H34N6O3 | 539.2765 | 0.0 | -0.8 |
| B4J015-01 | C32H41N5O3 | 544.3282 | 0.0 | 0.0 |
| B4J016-01 | C31H40N6O3 | 545.3235 | 0.0 | 0.9 |
| B4J017-01 | C29H32F3N5O3 | 556.253 | -0.3 | -0.5 |
| B4J018-01 | C28H31F3N6O3 | 557.2483 | -0.5 | -1.7 |
| B4J019-01 | C31H32F3N5O3 | 580.253 | -0.5 | -2.1 |
| B4J020-01 | C30H31F3N6O3 | 581.2483 | 0.0 | 0.0 |
| B4J021-01 | C32H34F3N7O3 | 622.2748 | 0.0 | 0.0 |
| B4J022-01 | C31H33F3N8O3 | 623.2701 | 0.2 | -0.7 |
| B4J023-01 | C28H33N3O3S | 492.2315 | 3.5 | -3.4 |
| B4J024-01 | C27H32N4O3S | 493.2268 | -0.6 | 6.8 |
| B4J025-01 | C31H37N3O3 | 500.2908 | 3.6 | -0.3 |
| B4J026-01 | C30H36N4O3 | 501.286 | -3.6 | -5.9 |
| B4J027-01 | C30H34FN3O3 | 504.2657 | -0.5 | 0.2 |
| B4J028-01 | C29H33FN4O3 | 505.261 | -1.0 | 0.9 |
| B4J029-01 | C32H35N3O3 | 510.2751 | 0.0 | 0.0 |
| B4J030-01 | C31H34N4O3 | 511.2704 | 0.1 | 0.8 |
| B4J031-01 | C31H34N4O3 | 511.2704 | 0.1 | 0.8 |
| B4J032-01 | C30H33N5O3 | 512.2656 | -7.2 | 1.5 |
| B4J033-01 | C32H39N3O3 | 514.3064 | 0.0 | -0.9 |
| B4J034-01 | C31H38N4O3 | 515.3017 | -0.7 | 0.9 |
| B4J035-01 | C34H37N3O3 | 536.2908 | 4.1 | 1.5 |
| B4J036-01 | C33H36N4O3 | 537.286 | -0.3 | -1.6 |
| B4J037-01 | C34H43N3O3 | 542.3377 | 2.7 | -1.5 |
| B4J038-01 | C33H42N4O3 | 543.333 | 0.8 | 0.0 |
| B4J039-01 | C31H34F3N3O3 | 554.2625 | 2.2 | 0.1 |
| B4J040-01 | C30H33F3N4O3 | 555.2578 | -0.5 | -1.2 |
| B4J041-01 | C33H34F3N3O3 | 578.2625 | 2.8 | 1.7 |
| B4J042-01 | C32H33F3N4O3 | 579.2578 | 1.0 | 0.0 |
| B4J043-01 | C34H36F3N5O3 | 620.2843 | 3.8 | 3.2 |
| B4J044-01 | C33H35F3N6O3 | 621.2796 | 1.8 | 0.3 |
| B4J045-01 | C30H37N3O2 | 472.2959 | 1.1 | 0.3 |
| B4J046-01 | C29H36N4O2 | 473.2911 | 0.0 | 0.0 |
| B4J047-01 | C31H39N3O2 | 486.3115 | -6.6 | 0.0 |
| B4J048-01 | C30H38N4O2 | 487.3068 | 1.2 | -2.1 |
| B4J049-01 | C32H41N3O2 | 500.3272 | 7.1 | -1.4 |
| B4J050-01 | C31H40N4O2 | 501.3224 | 2.1 | 0.6 |
| B4J051-01 | C30H36FN3O2 | 490.2864 | -9.6 | -10.2 |
| B4J052-01 | C29H35FN4O2 | 491.2817 | 0.5 | -1.5 |
| B4J053-01 | C31H39N3O3 | 502.3064 | -0.1 | -6.0 |
| B4J054-01 | C30H38N4O3 | 503.3017 | 0.0 | -1.1 |
| B4J055-01 | C32H41N3O3 | 516.3221 | -0.7 | 1.4 |
| B4J056-01 | C31H40N4O3 | 517.3173 | -1.4 | -1.5 |
| B4J057-01 | C33H43N3O3 | 530.3377 | 5.3 | -0.1 |
| B4J058-01 | C32H42N4O3 | 531.333 | 2.2 | 0.5 |
| B4J059-01 | C32H41N3O4 | 532.317 | -0.1 | -6.0 |
| B4J060-01 | C31H40N4O4 | 533.3122 | 0.0 | 0.0 |

na = not available

[Table 137]

| Compound No. | Molecular formula | m/z (expected) | ASMS ratio(%) | |
|---|---|---|---|---|
| | | | n=1 | n=2 |
| B4J061-01 | C31H36F3N3O2 | 540.2832 | 1.6 | -1.8 |
| B4J062-01 | C30H35F3N4O2 | 541.2785 | 0.0 | 0.0 |
| B4J063-01 | C31H36F3N3O3 | 556.2782 | 4.5 | 0.1 |
| B4J064-01 | C30H35F3N4O3 | 557.2734 | 1.6 | 0.0 |
| B4J065-01 | C34H39N3O2 | 522.3115 | 0.0 | 0.0 |
| B4J066-01 | C33H38N4O2 | 523.3068 | 0.0 | -1.5 |
| B4J067-01 | C28H35N3O2S | 478.2523 | -11.0 | -5.5 |
| B4J068-01 | C27H34N4O2S | 479.2475 | 0.7 | 0.0 |
| B4J069-01 | C32H37N3O2 | 496.2959 | 1.8 | -0.8 |
| B4J070-01 | C31H36N4O2 | 497.2911 | 0.0 | 0.0 |
| B4J071-01 | C28H35N5O2 | 474.2864 | 0.4 | -0.5 |
| B4J072-01 | C27H34N6O2 | 475.2816 | -1.6 | 0.9 |
| B4J073-01 | C29H37N5O2 | 488.302 | 2.1 | 0.0 |
| B4J074-01 | C28H36N6O2 | 489.2973 | 0.0 | 0.0 |
| B4J075-01 | C30H39N5O2 | 502.3177 | 0.0 | 0.0 |
| B4J076-01 | C29H38N6O2 | 503.3129 | 0.0 | 0.0 |
| B4J077-01 | C28H34FN5O2 | 492.2769 | 1.2 | -5.5 |
| B4J078-01 | C27H33FN6O2 | 493.2722 | -0.5 | 0.9 |
| B4J079-01 | C29H37N5O3 | 504.2969 | 0.0 | 0.0 |
| B4J080-01 | C28H36N6O3 | 505.2922 | 0.0 | 0.0 |
| B4J081-01 | C30H39N5O3 | 518.3126 | 0.0 | 0.0 |
| B4J082-01 | C29H38N6O3 | 519.3078 | 0.0 | 0.0 |
| B4J083-01 | C31H41N5O3 | 532.3282 | 0.0 | 0.0 |
| B4J084-01 | C30H40N6O3 | 533.3235 | 0.0 | -0.6 |
| B4J085-01 | C30H39N5O4 | 534.3075 | 0.0 | 2.8 |
| B4J086-01 | C29H38N6O4 | 535.3027 | 0.0 | 0.0 |
| B4J087-01 | C29H34F3N5O2 | 542.2737 | 0.9 | 0.3 |
| B4J088-01 | C28H33F3N6O2 | 543.269 | 0.0 | 0.0 |
| B4J089-01 | C29H34F3N5O3 | 558.2687 | 3.9 | 0.4 |
| B4J090-01 | C28H33F3N6O3 | 559.2639 | 1.4 | -1.5 |
| B4J091-01 | C32H37N5O2 | 524.302 | 3.7 | 1.5 |
| B4J092-01 | C31H36N6O2 | 525.2973 | -1.4 | 0.3 |
| B4J093-01 | C26H33N5O2S | 480.2428 | -2.1 | 0.6 |
| B4J094-01 | C25H32N6O2S | 481.238 | -1.3 | 0.1 |
| B4J095-01 | C30H35N5O2 | 498.2864 | 1.5 | 0.0 |
| B4J096-01 | C29H34N6O2 | 499.2816 | na | 0.0 |
| B4J097-01 | C31H39N3O2 | 486.3115 | -0.9 | -0.4 |
| B4J098-01 | C30H38N4O2 | 487.3068 | -2.0 | -0.2 |
| B4J099-01 | C31H38FN3O2 | 504.3021 | na | 0.0 |
| B4J100-01 | C30H37FN4O2 | 505.2973 | 0.0 | 0.0 |
| B2Q001-01 | C36H35N7O7S3 | 774.1833 | 0.0 | 0.0 |
| B2Q002-01 | C35H34N8O7S3 | 775.1785 | 24.5 | 17.2 |
| B2Q003-01 | C39H39N7O7S2 | 782.2425 | 8.2 | 6.9 |
| B2Q004-01 | C38H38N8O7S2 | 783.2378 | 10.7 | 8.7 |
| B2Q005-01 | C38H36FN7O7S2 | 786.2174 | 12.5 | 15.3 |
| B2Q006-01 | C37H35FN8O7S2 | 787.2127 | 9.5 | 2.2 |
| B2Q007-01 | C40H37N7O7S2 | 792.2269 | 4.2 | -8.3 |
| B2Q008-01 | C39H36N8O7S2 | 793.2221 | 16.5 | 0.5 |
| B2Q009-01 | C39H36N8O7S2 | 793.2221 | 16.5 | 0.5 |
| B2Q010-01 | C38H35N9O7S2 | 794.2174 | 13.3 | 8.9 |
| B2Q011-01 | C40H41N7O7S2 | 796.2582 | 25.0 | -1.1 |
| B2Q012-01 | C39H40N8O7S2 | 797.2534 | 21.4 | 22.5 |
| B2Q013-01 | C42H39N7O7S2 | 818.2425 | 0.0 | 0.0 |
| B2Q014-01 | C41H38N8O7S2 | 819.2378 | 9.3 | 14.5 |
| B2Q015-01 | C42H45N7O7S2 | 824.2895 | 17.1 | 13.2 |
| B2Q016-01 | C41H44N8O7S2 | 825.2847 | 10.6 | 6.5 |
| B2Q017-01 | C39H36F3N7O7S2 | 836.2143 | 12.8 | 0.2 |
| B2Q018-01 | C38H35F3N8O7S2 | 837.2095 | na | 0.0 |
| B2Q019-01 | C41H36F3N7O7S2 | 860.2143 | 0.7 | 14.1 |
| B2Q020-01 | C40H35F3N8O7S2 | 861.2095 | 6.1 | 5.6 |
| B2Q021-01 | C42H38F3N9O7S2 | 902.2361 | 0.0 | 0.0 |
| B2Q022-01 | C41H37F3N10O7S | 903.2313 | 13.8 | 23.6 |
| B2Q023-01 | C38H37N5O7S3 | 772.1928 | 0.0 | 0.0 |
| B2Q024-01 | C37H36N6O7S3 | 773.188 | 4.5 | 7.5 |
| B2Q025-01 | C41H41N5O7S2 | 780.252 | -5.7 | 0.0 |
| B2Q026-01 | C40H40N6O7S2 | 781.2473 | 4.4 | 0.4 |
| B2Q027-01 | C40H38FN5O7S2 | 784.2269 | 0.2 | -2.5 |
| B2Q028-01 | C39H37FN6O7S2 | 785.2222 | 0.0 | -3.7 |
| B2Q029-01 | C42H39N5O7S2 | 790.2364 | na | na |
| B2Q030-01 | C41H38N6O7S2 | 791.2316 | 6.6 | -4.1 |

| Compound No. | Molecular formula | m/z (expected) | ASMS ratio(%) | |
|---|---|---|---|---|
| | | | n=1 | n=2 |
| B2Q031-01 | C41H38N6O7S2 | 791.2316 | 6.6 | -4.1 |
| B2Q032-01 | C40H37N7O7S2 | 792.2269 | -0.2 | -374.2 |
| B2Q033-01 | C42H43N5O7S2 | 794.2677 | 4.2 | -2.3 |
| B2Q034-01 | C41H42N6O7S2 | 795.2629 | 0.0 | 0.0 |
| B2Q035-01 | C44H41N5O7S2 | 816.252 | 9.9 | -13.7 |
| B2Q036-01 | C43H40N6O7S2 | 817.2473 | 0.0 | -6.0 |
| B2Q037-01 | C44H47N5O7S2 | 822.299 | 10.9 | -1.5 |
| B2Q038-01 | C43H46N6O7S2 | 823.2942 | 3.3 | 0.4 |
| B2Q039-01 | C41H38F3N5O7S2 | 834.2238 | 7.2 | 4.6 |
| B2Q040-01 | C40H37F3N6O7S2 | 835.219 | 3.9 | 1.5 |
| B2Q041-01 | C43H38F3N5O7S2 | 858.2238 | 2.6 | 0.2 |
| B2Q042-01 | C42H37F3N6O7S2 | 859.219 | 26.1 | 19.5 |
| B2Q043-01 | C44H40F3N7O7S2 | 900.2456 | 12.4 | -13.4 |
| B2Q044-01 | C43H39F3N8O7S2 | 901.2408 | 11.0 | -1.3 |
| B2Q045-01 | C40H41N5O6S2 | 752.2571 | 49.9 | 49.9 |
| B2Q046-01 | C39H40N6O6S2 | 753.2524 | 20.3 | 20.5 |
| B2Q047-01 | C41H43N5O6S2 | 766.2728 | 142.5 | 326.4 |
| B2Q048-01 | C40H42N6O6S2 | 767.268 | 61.6 | 90.8 |
| B2Q049-01 | C42H45N5O6S2 | 780.2884 | -2.4 | -4.8 |
| B2Q050-01 | C41H44N6O6S2 | 781.2837 | 0.0 | 0.0 |
| B2Q051-01 | C40H40FN5O6S2 | 770.2477 | 22.6 | 2.1 |
| B2Q052-01 | C39H39FN6O6S2 | 771.2429 | 22.8 | 13.9 |
| B2Q053-01 | C41H43N5O7S2 | 782.2677 | 22.4 | 15.5 |
| B2Q054-01 | C40H42N6O7S2 | 783.2629 | 44.1 | 65.0 |
| B2Q055-01 | C42H45N5O7S2 | 796.2833 | 9.0 | -8.3 |
| B2Q056-01 | C41H44N6O7S2 | 797.2786 | 7.3 | 2.8 |
| B2Q057-01 | C43H47N5O7S2 | 810.299 | 17.5 | 4.4 |
| B2Q058-01 | C42H46N6O7S2 | 811.2942 | 3.7 | 3.7 |
| B2Q059-01 | C42H45N5O8S2 | 812.2782 | 9.8 | 7.0 |
| B2Q060-01 | C41H44N6O8S2 | 813.2735 | 0.0 | 0.0 |
| B2Q061-01 | C41H40F3N5O6S2 | 820.2445 | 15.6 | -17.9 |
| B2Q062-01 | C40H39F3N6O6S2 | 821.2397 | 9.4 | -3.3 |
| B2Q063-01 | C41H40F3N5O7S2 | 836.2394 | 13.3 | -25.5 |
| B2Q064-01 | C40H39F3N6O7S2 | 837.2347 | 8.6 | -1.6 |
| B2Q065-01 | C44H43N5O6S2 | 802.2728 | 30.6 | -1.6 |
| B2Q066-01 | C43H42N6O6S2 | 803.268 | 40.7 | 36.9 |
| B2Q067-01 | C38H39N5O6S3 | 758.2135 | na | na |
| B2Q068-01 | C37H38N6O6S3 | 759.2088 | na | na |
| B2Q069-01 | C42H41N5O6S2 | 776.2571 | 8.8 | 11.0 |
| B2Q070-01 | C41H40N6O6S2 | 777.2524 | 14.3 | -9.0 |
| B2Q071-01 | C38H39N7O6S2 | 754.2476 | 25.0 | 11.2 |
| B2Q072-01 | C37H38N8O6S2 | 755.2429 | 10.2 | 11.4 |
| B2Q073-01 | C39H41N7O6S2 | 768.2633 | 0.0 | 0.0 |
| B2Q074-01 | C38H40N8O6S2 | 769.2585 | 18.8 | 15.4 |
| B2Q075-01 | C40H43N7O6S2 | 782.2789 | 0.0 | 0.0 |
| B2Q076-01 | C39H42N8O6S2 | 783.2742 | 0.0 | na |
| B2Q077-01 | C38H38FN7O6S2 | 772.2382 | 14.7 | 17.2 |
| B2Q078-01 | C37H37FN8O6S2 | 773.2334 | 15.6 | 12.1 |
| B2Q079-01 | C39H41N7O7S2 | 784.2582 | 17.6 | 0.0 |
| B2Q080-01 | C38H40N8O7S2 | 785.2534 | 12.6 | 3.6 |
| B2Q081-01 | C40H43N7O7S2 | 798.2738 | -4.8 | 0.6 |
| B2Q082-01 | C39H42N8O7S2 | 799.2691 | 21.3 | 8.5 |
| B2Q083-01 | C41H45N7O7S2 | 812.2895 | na | 0.0 |
| B2Q084-01 | C40H44N8O7S2 | 813.2847 | 12.6 | -4.9 |
| B2Q085-01 | C40H43N7O8S2 | 814.2687 | 14.9 | 0.7 |
| B2Q086-01 | C39H42N8O8S2 | 815.264 | -39.4 | 0.0 |
| B2Q087-01 | C39H38F3N7O6S2 | 822.235 | 17.6 | -2.1 |
| B2Q088-01 | C38H37F3N8O6S2 | 823.2302 | 13.8 | 9.1 |
| B2Q089-01 | C39H38F3N7O7S2 | 838.2299 | 25.0 | -15.0 |
| B2Q090-01 | C38H37F3N8O7S2 | 839.2252 | 21.8 | 13.2 |
| B2Q091-01 | C42H41N7O6S2 | 804.2633 | 17.2 | -5.0 |
| B2Q092-01 | C41H40N8O6S2 | 805.2585 | 10.4 | 4.8 |
| B2Q093-01 | C36H37N7O6S3 | 760.204 | na | na |
| B2Q094-01 | C35H36N8O6S3 | 761.1993 | 7.2 | 2.5 |
| B2Q095-01 | C40H39N7O6S2 | 778.2476 | 19.9 | 11.9 |
| B2Q096-01 | C39H38N8O6S2 | 779.2429 | na | na |
| B2Q097-01 | C41H43N5O6S2 | 766.2728 | 142.5 | 326.4 |
| B2Q098-01 | C40H42N6O6S2 | 767.268 | 61.6 | 90.8 |
| B2Q099-01 | C41H42FN5O6S2 | 784.2633 | na | 0.0 |
| B2Q100-01 | C40H41FN6O6S2 | 785.2586 | 0.0 | 10.7 |

na = not available

[Table 138]

| Compound No. | Molecular formula | m/z (expected) | ASMS ratio(%) | |
|---|---|---|---|---|
| | | | n=1 | n=2 |
| B2A001-01 | C33H39N5O5S2 | 650.2465 | 0.0 | 0.0 |
| B2A002-01 | C32H38N6O5S2 | 651.2418 | 0.0 | 0.0 |
| B2A003-01 | C36H43N5O5S | 658.3058 | 21.0 | 11.0 |
| B2A004-01 | C35H42N6O5S | 659.301 | 0.0 | 0.0 |
| B2A005-01 | C35H40FN5O5S | 662.2807 | 4.7 | 12.4 |
| B2A006-01 | C34H39FN6O5S | 663.2759 | 0.0 | 0.0 |
| B2A007-01 | C37H41N5O5S | 668.2901 | na | 0.0 |
| B2A008-01 | C36H40N6O5S | 669.2854 | 3.1 | 7.2 |
| B2A009-01 | C36H40N6O5S | 669.2854 | 3.1 | 7.2 |
| B2A010-01 | C35H39N7O5S | 670.2806 | 0.0 | 0.0 |
| B2A011-01 | C37H45N5O5S | 672.3214 | 9.0 | 0.3 |
| B2A012-01 | C36H44N6O5S | 673.3167 | 1.5 | 1.9 |
| B2A013-01 | C39H43N5O5S | 694.3058 | 11.6 | 17.8 |
| B2A014-01 | C38H42N6O5S | 695.301 | 7.7 | 2.2 |
| B2A015-01 | C39H49N5O5S | 700.3527 | 5.9 | 9.7 |
| B2A016-01 | C38H48N6O5S | 701.348 | 0.0 | 2.5 |
| B2A017-01 | C36H40F3N5O5S | 712.2775 | 18.6 | 7.5 |
| B2A018-01 | C35H39F3N6O5S | 713.2728 | 5.1 | 0.0 |
| B2A019-01 | C38H40F3N5O5S | 736.2775 | 7.4 | 5.0 |
| B2A020-01 | C37H39F3N6O5S | 737.2728 | 3.4 | 8.9 |
| B2A021-01 | C39H42F3N7O5S | 778.2993 | 14.4 | 9.4 |
| B2A022-01 | C38H41F3N8O5S | 779.2946 | 27.0 | 3.7 |
| B2A023-01 | C35H41N3O5S2 | 648.256 | 0.0 | 0.0 |
| B2A024-01 | C34H40N4O5S2 | 649.2513 | -1.9 | 0.0 |
| B2A025-01 | C38H45N3O5S | 656.3153 | 4.6 | -2.7 |
| B2A026-01 | C37H44N4O5S | 657.3105 | 0.8 | -2.1 |
| B2A027-01 | C37H42FN3O5S | 660.2902 | 5.5 | -3.9 |
| B2A028-01 | C36H41FN4O5S | 661.2855 | -1.0 | 1.2 |
| B2A029-01 | C39H43N3O5S | 666.2996 | na | na |
| B2A030-01 | C38H42N4O5S | 667.2949 | 0.0 | 0.3 |
| B2A031-01 | C38H42N4O5S | 667.2949 | 0.0 | 0.3 |
| B2A032-01 | C37H41N5O5S | 668.2901 | 0.0 | 0.0 |
| B2A033-01 | C39H47N3O5S | 670.3309 | 1.7 | -4.5 |
| B2A034-01 | C38H46N4O5S | 671.3262 | 1.6 | 0.3 |
| B2A035-01 | C41H45N3O5S | 692.3153 | 6.2 | -9.8 |
| B2A036-01 | C40H44N4O5S | 693.3105 | 2.0 | -0.4 |
| B2A037-01 | C41H51N3O5S | 698.3622 | 3.0 | 0.0 |
| B2A038-01 | C40H50N4O5S | 699.3575 | 1.8 | -3.5 |
| B2A039-01 | C38H42F3N3O5S | 710.287 | 7.4 | -10.4 |
| B2A040-01 | C37H41F3N4O5S | 711.2823 | 0.0 | 0.0 |
| B2A041-01 | C40H42F3N3O5S | 734.287 | 0.0 | -3.7 |
| B2A042-01 | C39H41F3N4O5S | 735.2823 | 10.1 | -0.9 |
| B2A043-01 | C41H44F3N5O5S | 776.3088 | 14.0 | 4.4 |
| B2A044-01 | C40H43F3N6O5S | 777.3041 | 0.7 | -0.2 |
| B2A045-01 | C37H45N3O4S | 628.3204 | 6.5 | -2.9 |
| B2A046-01 | C36H44N4O4S | 629.3156 | -2.7 | -22.1 |
| B2A047-01 | C38H47N3O4S | 642.336 | 9.7 | 0.4 |
| B2A048-01 | C37H46N4O4S | 643.3313 | 18.2 | 2.1 |
| B2A049-01 | C39H49N3O4S | 656.3517 | -3.0 | 6.2 |
| B2A050-01 | C38H48N4O4S | 657.3469 | -1.1 | 1.6 |
| B2A051-01 | C37H44FN3O4S | 646.3109 | -0.3 | 3.6 |
| B2A052-01 | C36H43FN4O4S | 647.3062 | 0.0 | 0.0 |
| B2A053-01 | C38H47N3O5S | 658.3309 | 0.0 | 0.0 |
| B2A054-01 | C37H46N4O5S | 659.3262 | 17.5 | -0.9 |
| B2A055-01 | C39H49N3O5S | 672.3466 | 5.8 | -7.3 |
| B2A056-01 | C38H48N4O5S | 673.3418 | -3.9 | 0.2 |
| B2A057-01 | C40H51N3O5S | 686.3622 | -4.8 | -5.3 |
| B2A058-01 | C39H50N4O5S | 687.3575 | 4.8 | -10.6 |
| B2A059-01 | C39H49N3O6S | 688.3415 | 0.0 | -42.2 |
| B2A060-01 | C38H48N4O6S | 689.3367 | 0.0 | 0.0 |
| B2A061-01 | C38H44F3N3O4S | 696.3077 | 1.7 | -5.6 |
| B2A062-01 | C37H43F3N4O4S | 697.303 | -5.2 | 3.6 |
| B2A063-01 | C38H44F3N3O5S | 712.3027 | 0.5 | 3.3 |
| B2A064-01 | C37H43F3N4O5S | 713.2979 | 0.0 | -0.3 |
| B2A065-01 | C41H47N3O4S | 678.336 | 10.5 | -2.9 |
| B2A066-01 | C40H46N4O4S | 679.3313 | 3.2 | 0.0 |
| B2A067-01 | C35H43N3O4S2 | 634.2768 | 0.0 | 0.0 |
| B2A068-01 | C34H42N4O4S2 | 635.272 | 0.0 | 0.0 |
| B2A069-01 | C39H45N3O4S | 652.3204 | 4.6 | -4.6 |
| B2A070-01 | C38H44N4O4S | 653.3156 | na | 0.0 |
| B2A071-01 | C35H43N5O4S | 630.3109 | 16.1 | 5.6 |
| B2A072-01 | C34H42N6O4S | 631.3061 | 12.2 | 14.7 |
| B2A073-01 | C36H45N5O4S | 644.3265 | 16.2 | 13.5 |
| B2A074-01 | C35H44N6O4S | 645.3218 | 9.2 | 1.9 |
| B2A075-01 | C37H47N5O4S | 658.3422 | 4.6 | 4.4 |
| B2A076-01 | C36H46N6O4S | 659.3374 | 23.4 | 11.4 |
| B2A077-01 | C35H42FN5O4S | 648.3014 | 14.3 | 21.9 |
| B2A078-01 | C34H41FN6O4S | 649.2967 | 15.2 | 9.3 |
| B2A079-01 | C36H45N5O5S | 660.3214 | na | na |
| B2A080-01 | C35H44N6O5S | 661.3167 | 0.0 | 0.0 |
| B2A081-01 | C37H47N5O5S | 674.3371 | 18.6 | 16.7 |
| B2A082-01 | C36H46N6O5S | 675.3323 | na | na |
| B2A083-01 | C38H49N5O5S | 688.3527 | 10.3 | 0.0 |
| B2A084-01 | C37H48N6O5S | 689.348 | 13.3 | 11.9 |
| B2A085-01 | C37H47N5O6S | 690.332 | 0.0 | 0.0 |
| B2A086-01 | C36H46N6O6S | 691.3272 | 0.0 | 0.0 |
| B2A087-01 | C36H42F3N5O4S | 698.2982 | 7.2 | 12.7 |
| B2A088-01 | C35H41F3N6O4S | 699.2935 | 12.0 | 14.0 |
| B2A089-01 | C36H42F3N5O5S | 714.2932 | 10.5 | 0.5 |
| B2A090-01 | C35H41F3N6O5S | 715.2884 | 22.0 | 4.7 |
| B2A091-01 | C39H45N5O4S | 680.3265 | -2.6 | 3.8 |
| B2A092-01 | C38H44N6O4S | 681.3218 | 20.8 | 16.6 |
| B2A093-01 | C33H41N5O4S2 | 636.2673 | 13.0 | 0.0 |
| B2A094-01 | C32H40N6O4S2 | 637.2625 | 0.0 | 0.0 |
| B2A095-01 | C37H43N5O4S | 654.3109 | 1.6 | 13.8 |
| B2A096-01 | C36H42N6O4S | 655.3061 | na | na |
| B2A097-01 | C38H47N3O4S | 642.336 | 9.7 | 0.4 |
| B2A098-01 | C37H46N4O4S | 643.3313 | 18.2 | 2.1 |
| B2A099-01 | C38H46FN3O4S | 660.3266 | 0.0 | 0.0 |
| B2A100-01 | C37H45FN4O4S | 661.3218 | 0.0 | 0.0 |
| B2I001-01 | C23H25N5O5S2 | 516.137 | 0.0 | na |
| B2I002-01 | C22H24N6O5S2 | 517.1322 | 0.0 | 0.0 |
| B2I003-01 | C26H29N5O5S | 524.1962 | 0.0 | 0.0 |
| B2I004-01 | C25H28N6O5S | 525.1915 | 0.9 | -0.3 |
| B2I005-01 | C25H26FN5O5S | 528.1711 | 0.0 | 0.0 |
| B2I006-01 | C24H25FN6O5S | 529.1664 | 0.0 | 0.0 |
| B2I007-01 | C27H27N5O5S | 534.1806 | -1.6 | -1.6 |
| B2I008-01 | C26H26N6O5S | 535.1758 | 0.0 | 0.0 |
| B2I009-01 | C26H26N6O5S | 535.1758 | 0.0 | 0.0 |
| B2I010-01 | C25H25N7O5S | 536.1711 | na | na |
| B2I011-01 | C27H31N5O5S | 538.2119 | 0.0 | 0.0 |
| B2I012-01 | C26H30N6O5S | 539.2071 | -4.0 | -0.2 |
| B2I013-01 | C29H29N5O5S | 560.1962 | 0.0 | 0.0 |
| B2I014-01 | C28H28N6O5S | 561.1915 | 1.0 | 2.3 |
| B2I015-01 | C29H35N5O5S | 566.2432 | 0.0 | 0.0 |
| B2I016-01 | C28H34N6O5S | 567.2384 | 0.0 | 0.0 |
| B2I017-01 | C26H26F3N5O5S | 578.168 | 0.0 | 0.0 |
| B2I018-01 | C25H25F3N6O5S | 579.1632 | 0.0 | 0.0 |
| B2I019-01 | C28H26F3N5O5S | 602.168 | 0.0 | na |
| B2I020-01 | C27H25F3N6O5S | 603.1632 | 0.0 | 0.0 |
| B2I021-01 | C29H28F3N7O5S | 644.1898 | 0.0 | 0.0 |
| B2I022-01 | C28H27F3N8O5S | 645.185 | 0.0 | 0.0 |
| B2I023-01 | C25H27N3O5S2 | 514.1465 | 0.0 | 0.0 |
| B2I024-01 | C24H26N4O5S2 | 515.1417 | 0.0 | 0.0 |
| B2I025-01 | C28H31N3O5S | 522.2057 | 0.0 | 0.0 |
| B2I026-01 | C27H30N4O5S | 523.201 | -2.6 | -0.2 |
| B2I027-01 | C27H28FN3O5S | 526.1807 | 0.0 | 0.0 |
| B2I028-01 | C26H27FN4O5S | 527.1759 | 0.0 | 0.0 |
| B2I029-01 | C29H29N3O5S | 532.1901 | 0.0 | 0.0 |
| B2I030-01 | C28H28N4O5S | 533.1853 | 0.0 | 0.0 |
| B2I031-01 | C28H28N4O5S | 533.1853 | 0.0 | 0.0 |
| B2I032-01 | C27H27N5O5S | 534.1806 | -1.6 | -1.6 |
| B2I033-01 | C29H33N3O5S | 536.2214 | 0.0 | 0.0 |
| B2I034-01 | C28H32N4O5S | 537.2166 | 0.0 | 1.6 |
| B2I035-01 | C31H31N3O5S | 558.2057 | 0.0 | 1.6 |
| B2I036-01 | C30H30N4O5S | 559.201 | -0.7 | 1.5 |
| B2I037-01 | C31H37N3O5S | 564.2527 | 0.0 | 3.3 |
| B2I038-01 | C30H36N4O5S | 565.2479 | 0.0 | 0.0 |
| B2I039-01 | C28H28F3N3O5S | 576.1775 | 0.0 | 0.0 |
| B2I040-01 | C27H27F3N4O5S | 577.1727 | 0.0 | -2.7 |

na = not available

[Table 139]

| Compound No. | Molecular formula | m/z (expected) | ASMS ratio(%) | |
|---|---|---|---|---|
| | | | n=1 | n=2 |
| B2I041-01 | C30H28F3N3O5S | 600.1775 | 0.0 | 0.0 |
| B2I042-01 | C29H27F3N4O5S | 601.1727 | 0.0 | 0.0 |
| B2I043-01 | C31H30F3N5O5S | 642.1993 | 0.0 | 0.0 |
| B2I044-01 | C30H29F3N6O5S | 643.1945 | 0.0 | 0.0 |
| B2I045-01 | C27H31N3O4S | 494.2108 | -0.3 | 0.2 |
| B2I046-01 | C26H30N4O4S | 495.2061 | -0.6 | 1.5 |
| B2I047-01 | C28H33N3O4S | 508.2265 | 0.8 | 1.4 |
| B2I048-01 | C27H32N4O4S | 509.2217 | 0.0 | 0.0 |
| B2I049-01 | C29H35N3O4S | 522.2421 | 0.0 | 0.0 |
| B2I050-01 | C28H34N4O4S | 523.2374 | 0.0 | -1.5 |
| B2I051-01 | C27H30FN3O4S | 512.2014 | 0.0 | 0.0 |
| B2I052-01 | C26H29FN4O4S | 513.1966 | 0.0 | 0.0 |
| B2I053-01 | C28H33N3O5S | 524.2214 | 0.0 | 0.0 |
| B2I054-01 | C27H32N4O5S | 525.2166 | 0.0 | 0.0 |
| B2I055-01 | C29H35N3O5S | 538.237 | 0.0 | 0.0 |
| B2I056-01 | C28H34N4O5S | 539.2323 | 0.0 | 0.0 |
| B2I057-01 | C30H37N3O5S | 552.2527 | 0.0 | 0.0 |
| B2I058-01 | C29H36N4O5S | 553.2479 | 0.0 | 0.0 |
| B2I059-01 | C29H35N3O6S | 554.2319 | 0.0 | 0.0 |
| B2I060-01 | C28H34N4O6S | 555.2272 | 0.0 | 0.0 |
| B2I061-01 | C28H30F3N3O4S | 562.1982 | -0.8 | 0.5 |
| B2I062-01 | C27H29F3N4O4S | 563.1934 | 0.0 | 0.0 |
| B2I063-01 | C28H30F3N3O5S | 578.1931 | 0.9 | 0.0 |
| B2I064-01 | C27H29F3N4O5S | 579.1884 | 0.0 | 0.0 |
| B2I065-01 | C31H33N3O4S | 544.2265 | 0.0 | 0.0 |
| B2I066-01 | C30H32N4O4S | 545.2217 | 0.0 | 0.0 |
| B2I067-01 | C25H29N3O4S2 | 500.1672 | 0.0 | 0.0 |
| B2I068-01 | C24H28N4O4S2 | 501.1625 | 0.0 | 0.0 |
| B2I069-01 | C29H31N3O4S | 518.2108 | 0.0 | -2.2 |
| B2I070-01 | C28H30N4O4S | 519.2061 | na | 0.0 |
| B2I071-01 | C25H29N5O4S | 496.2013 | -1.2 | -1.9 |
| B2I072-01 | C24H28N6O4S | 497.1966 | 0.8 | 1.3 |
| B2I073-01 | C26H31N5O4S | 510.217 | 0.0 | 0.0 |
| B2I074-01 | C25H30N6O4S | 511.2122 | 0.0 | -8.4 |
| B2I075-01 | C27H33N5O4S | 524.2326 | 0.0 | 0.0 |
| B2I076-01 | C26H32N6O4S | 525.2279 | 0.2 | -3.2 |
| B2I077-01 | C25H28FN5O4S | 514.1919 | 0.0 | 0.0 |
| B2I078-01 | C24H27FN6O4S | 515.1871 | 0.0 | 0.0 |
| B2I079-01 | C26H31N5O5S | 526.2119 | 0.0 | 0.0 |
| B2I080-01 | C25H30N6O5S | 527.2071 | 0.0 | na |
| B2I081-01 | C27H33N5O5S | 540.2275 | 0.0 | 0.0 |
| B2I082-01 | C26H32N6O5S | 541.2228 | 0.0 | 1.9 |
| B2I083-01 | C28H35N5O5S | 554.2432 | 0.0 | 0.0 |
| B2I084-01 | C27H34N6O5S | 555.2384 | -0.9 | -1.5 |
| B2I085-01 | C27H33N5O6S | 556.2224 | 0.0 | 0.0 |
| B2I086-01 | C26H32N6O6S | 557.2177 | 0.0 | 0.0 |
| B2I087-01 | C26H28F3N5O4S | 564.1887 | 1.3 | -0.9 |
| B2I088-01 | C25H27F3N6O4S | 565.1839 | 0.0 | 0.0 |
| B2I089-01 | C26H28F3N5O5S | 580.1836 | 1.4 | 0.0 |
| B2I090-01 | C25H27F3N6O5S | 581.1789 | 0.0 | 0.0 |
| B2I091-01 | C29H31N5O4S | 546.217 | na | na |
| B2I092-01 | C28H30N6O4S | 547.2122 | 2.6 | 0.3 |
| B2I093-01 | C23H27N5O4S2 | 502.1577 | na | na |
| B2I094-01 | C22H26N6O4S2 | 503.153 | 0.0 | 0.0 |
| B2I095-01 | C27H29N5O4S | 520.2013 | 0.0 | 0.0 |
| B2I096-01 | C26H28N6O4S | 521.1966 | 0.0 | 0.0 |
| B2I097-01 | C28H33N3O4S | 508.2265 | 0.8 | 1.4 |
| B2I098-01 | C27H32N4O4S | 509.2217 | 0.0 | 0.0 |
| B2I099-01 | C28H32FN3O4S | 526.217 | na | 0.0 |
| B2I100-01 | C27H31FN4O4S | 527.2123 | 0.0 | 0.0 |
| B2L001-01 | C26H31N5O5S2 | 558.1839 | -67.5 | 0.0 |
| B2L002-01 | C25H30N6O5S2 | 559.1792 | 0.0 | 0.0 |
| B2L003-01 | C29H35N5O5S | 566.2432 | 0.0 | 0.0 |
| B2L004-01 | C28H34N6O5S | 567.2384 | 5.5 | 9.3 |
| B2L005-01 | C28H32FN5O5S | 570.2181 | 0.0 | 0.0 |
| B2L006-01 | C27H31FN6O5S | 571.2133 | 0.0 | 0.0 |
| B2L007-01 | C30H33N5O5S | 576.2275 | 0.0 | -1.0 |
| B2L008-01 | C29H32N6O5S | 577.2228 | 0.0 | 0.0 |
| B2L009-01 | C29H32N6O5S | 577.2228 | 0.0 | 0.0 |
| B2L010-01 | C28H31N7O5S | 578.218 | 0.0 | 0.0 |

| Compound No. | Molecular formula | m/z (expected) | ASMS ratio(%) | |
|---|---|---|---|---|
| | | | n=1 | n=2 |
| B2L011-01 | C30H37N5O5S | 580.2588 | 0.0 | 0.0 |
| B2L012-01 | C29H36N6O5S | 581.2541 | 4.3 | 7.3 |
| B2L013-01 | C32H35N5O5S | 602.2432 | 0.0 | 0.0 |
| B2L014-01 | C31H34N6O5S | 603.2384 | 0.3 | 0.5 |
| B2L015-01 | C32H41N5O5S | 608.2901 | 0.0 | 0.0 |
| B2L016-01 | C31H40N6O5S | 609.2854 | 0.0 | -5.4 |
| B2L017-01 | C29H32F3N5O5S | 620.2149 | 0.0 | 0.0 |
| B2L018-01 | C28H31F3N6O5S | 621.2102 | -1.9 | -2.5 |
| B2L019-01 | C31H32F3N5O5S | 644.2149 | na | na |
| B2L020-01 | C30H31F3N6O5S | 645.2102 | 0.0 | 0.0 |
| B2L021-01 | C32H34F3N7O5S | 686.2367 | 0.0 | -14.9 |
| B2L022-01 | C31H33F3N8O5S | 687.232 | 0.0 | 0.0 |
| B2L023-01 | C28H33N3O5S2 | 556.1934 | 0.0 | 0.0 |
| B2L024-01 | C27H32N4O5S2 | 557.1887 | 0.0 | 0.0 |
| B2L025-01 | C31H37N3O5S | 564.2527 | 0.0 | 0.0 |
| B2L026-01 | C30H36N4O5S | 565.2479 | 0.0 | 0.0 |
| B2L027-01 | C30H34FN3O5S | 568.2276 | 0.0 | 0.0 |
| B2L028-01 | C29H33FN4O5S | 569.2229 | 0.0 | 0.0 |
| B2L029-01 | C32H35N3O5S | 574.237 | 0.0 | na |
| B2L030-01 | C31H34N4O5S | 575.2323 | 0.0 | 0.0 |
| B2L031-01 | C31H34N4O5S | 575.2323 | 0.0 | 0.0 |
| B2L032-01 | C30H33N5O5S | 576.2275 | 0.0 | -1.0 |
| B2L033-01 | C32H39N3O5S | 578.2683 | 0.0 | 0.0 |
| B2L034-01 | C31H38N4O5S | 579.2636 | 1.6 | 3.5 |
| B2L035-01 | C34H37N3O5S | 600.2527 | 0.0 | na |
| B2L036-01 | C33H36N4O5S | 601.2479 | 0.0 | 0.0 |
| B2L037-01 | C34H43N3O5S | 606.2996 | 0.0 | 0.0 |
| B2L038-01 | C33H42N4O5S | 607.2949 | 0.0 | 0.0 |
| B2L039-01 | C31H34F3N3O5S | 618.2244 | 0.0 | 0.0 |
| B2L040-01 | C30H33F3N4O5S | 619.2197 | -0.2 | 1.0 |
| B2L041-01 | C33H34F3N3O5S | 642.2244 | 0.0 | na |
| B2L042-01 | C32H33F3N4O5S | 643.2197 | 0.0 | 0.0 |
| B2L043-01 | C34H36F3N5O5S | 684.2462 | 0.0 | 0.0 |
| B2L044-01 | C33H35F3N6O5S | 685.2415 | 0.0 | 0.0 |
| B2L045-01 | C30H37N3O4S | 536.2578 | 0.0 | 0.0 |
| B2L046-01 | C29H36N4O4S | 537.253 | 0.0 | 0.0 |
| B2L047-01 | C31H39N3O4S | 550.2734 | 0.0 | 0.0 |
| B2L048-01 | C30H38N4O4S | 551.2687 | 3.9 | -5.0 |
| B2L049-01 | C32H41N3O4S | 564.2891 | na | 0.0 |
| B2L050-01 | C31H40N4O4S | 565.2843 | 0.0 | 0.0 |
| B2L051-01 | C30H36FN3O4S | 554.2483 | 0.0 | 0.0 |
| B2L052-01 | C29H35FN4O4S | 555.2436 | 2.7 | 1.0 |
| B2L053-01 | C31H39N3O5S | 566.2683 | 0.0 | 0.0 |
| B2L054-01 | C30H38N4O5S | 567.2636 | 0.0 | 0.0 |
| B2L055-01 | C32H41N3O5S | 580.284 | 0.0 | 0.0 |
| B2L056-01 | C31H40N4O5S | 581.2792 | 0.0 | 0.0 |
| B2L057-01 | C33H43N3O5S | 594.2996 | 0.0 | 0.0 |
| B2L058-01 | C32H42N4O5S | 595.2949 | 0.0 | 0.0 |
| B2L059-01 | C32H41N3O6S | 596.2789 | 0.0 | 0.0 |
| B2L060-01 | C31H40N4O6S | 597.2741 | na | 0.0 |
| B2L061-01 | C31H36F3N3O4S | 604.2451 | 4.6 | -11.1 |
| B2L062-01 | C30H35F3N4O4S | 605.2404 | 0.0 | 0.0 |
| B2L063-01 | C31H36F3N3O5S | 620.2401 | 0.0 | 0.0 |
| B2L064-01 | C30H35F3N4O5S | 621.2353 | 0.0 | 0.0 |
| B2L065-01 | C34H39N3O4S | 586.2734 | na | na |
| B2L066-01 | C33H38N4O4S | 587.2687 | 0.0 | 0.0 |
| B2L067-01 | C28H35N3O4S2 | 542.2142 | 0.0 | 0.0 |
| B2L068-01 | C27H34N4O4S2 | 543.2094 | 0.0 | 0.0 |
| B2L069-01 | C32H37N3O4S | 560.2578 | 0.0 | 0.0 |
| B2L070-01 | C31H36N4O4S | 561.253 | 4.9 | -4.2 |
| B2L071-01 | C28H35N5O4S | 538.2483 | 1.2 | 0.0 |
| B2L072-01 | C27H34N6O4S | 539.2435 | -10.7 | 8.3 |
| B2L073-01 | C29H37N5O4S | 552.2639 | 0.0 | 0.0 |
| B2L074-01 | C28H36N6O4S | 553.2592 | -1.0 | 1.4 |
| B2L075-01 | C30H39N5O4S | 566.2796 | 0.0 | 33.9 |
| B2L076-01 | C29H38N6O4S | 567.2748 | 1.8 | -0.3 |
| B2L077-01 | C28H34FN5O4S | 556.2388 | 0.0 | 0.0 |
| B2L078-01 | C27H33FN6O4S | 557.2341 | 1.1 | -0.7 |
| B2L079-01 | C29H37N5O5S | 568.2588 | 0.0 | 0.0 |
| B2L080-01 | C28H36N6O5S | 569.2541 | 0.0 | 0.0 |

na = not available

[Table 140]

| Compound No. | Molecular formula | m/z (expected) | ASMS ratio(%) | |
|---|---|---|---|---|
| | | | n=1 | n=2 |
| B2L081-01 | C30H39N5O5S | 582.2745 | 0.0 | 0.0 |
| B2L082-01 | C29H38N6O5S | 583.2697 | 2.6 | -1.2 |
| B2L083-01 | C31H41N5O5S | 596.2901 | 0.0 | 0.0 |
| B2L084-01 | C30H40N6O5S | 597.2854 | -3.0 | -1.9 |
| B2L085-01 | C30H39N5O6S | 598.2694 | 0.0 | 0.0 |
| B2L086-01 | C29H38N6O6S | 599.2646 | na | 0.0 |
| B2L087-01 | C29H34F3N5O4S | 606.2356 | 1.9 | -24.6 |
| B2L088-01 | C28H33F3N6O4S | 607.2309 | 0.0 | 2.8 |
| B2L089-01 | C29H34F3N5O5S | 622.2306 | 0.0 | 0.0 |
| B2L090-01 | C28H33F3N6O5S | 623.2258 | 0.0 | 0.0 |
| B2L091-01 | C32H37N5O4S | 588.2639 | 0.0 | 0.0 |
| B2L092-01 | C31H36N6O4S | 589.2592 | 5.5 | 7.8 |
| B2L093-01 | C26H33N5O4S2 | 544.2047 | na | 0.0 |
| B2L094-01 | C25H32N6O4S2 | 545.1999 | 0.2 | 1.8 |
| B2L095-01 | C30H35N5O4S | 562.2483 | 0.0 | 0.0 |
| B2L096-01 | C29H34N6O4S | 563.2435 | 0.0 | 0.0 |
| B2L097-01 | C31H39N3O4S | 550.2734 | 0.0 | 0.0 |
| B2L098-01 | C30H38N4O4S | 551.2687 | 3.9 | -5.0 |
| B2L099-01 | C31H38FN3O4S | 568.264 | na | na |
| B2L100-01 | C30H37FN4O4S | 569.2592 | 0.0 | 0.0 |
| B2N001-01 | C25H26F3N5O5S2 | 598.14 | na | 0.0 |
| B2N002-01 | C24H25F3N6O5S2 | 599.1353 | 0.0 | 0.0 |
| B2N003-01 | C28H30F3N5O5S | 606.1993 | 0.0 | 0.0 |
| B2N004-01 | C27H29F3N6O5S | 607.1945 | 1.2 | -2.0 |
| B2N005-01 | C27H27F4N5O5S | 610.1742 | na | na |
| B2N006-01 | C26H26F4N6O5S | 611.1694 | na | na |
| B2N007-01 | C29H28F3N5O5S | 616.1836 | 0.0 | 0.0 |
| B2N008-01 | C28H27F3N6O5S | 617.1789 | 0.0 | -0.4 |
| B2N009-01 | C28H27F3N6O5S | 617.1789 | 0.0 | -0.4 |
| B2N010-01 | C27H26F3N7O5S | 618.1741 | 0.0 | 0.0 |
| B2N011-01 | C29H32F3N5O5S | 620.2149 | 0.0 | 0.0 |
| B2N012-01 | C28H31F3N6O5S | 621.2102 | 0.0 | 0.0 |
| B2N013-01 | C31H30F3N5O5S | 642.1993 | 0.0 | 0.0 |
| B2N014-01 | C30H29F3N6O5S | 643.1945 | 0.0 | 0.0 |
| B2N015-01 | C31H36F3N5O5S | 648.2462 | na | na |
| B2N016-01 | C30H35F3N6O5S | 649.2415 | 0.0 | 0.0 |
| B2N017-01 | C28H27F6N5O5S | 660.171 | na | na |
| B2N018-01 | C27H26F6N6O5S | 661.1662 | 0.0 | 1.0 |
| B2N019-01 | C30H27F6N5O5S | 684.171 | na | na |
| B2N020-01 | C29H26F6N6O5S | 685.1662 | 0.0 | 0.0 |
| B2N021-01 | C31H29F6N7O5S | 726.1928 | na | 0.0 |
| B2N022-01 | C30H28F6N8O5S | 727.188 | 0.0 | 0.0 |
| B2N023-01 | C27H28F3N3O5S2 | 596.1495 | 0.0 | 0.0 |
| B2N024-01 | C26H27F3N4O5S2 | 597.1448 | 0.0 | 0.0 |
| B2N025-01 | C30H32F3N3O5S | 604.2088 | 0.0 | 0.0 |
| B2N026-01 | C29H31F3N4O5S | 605.204 | -1.5 | -0.7 |
| B2N027-01 | C29H29F4N3O5S | 608.1837 | na | na |
| B2N028-01 | C28H28F4N4O5S | 609.1789 | -0.7 | -0.9 |
| B2N029-01 | C31H30F3N3O5S | 614.1931 | na | na |
| B2N030-01 | C30H29F3N4O5S | 615.1884 | 0.0 | 0.0 |
| B2N031-01 | C30H29F3N4O5S | 615.1884 | 0.0 | 0.0 |
| B2N032-01 | C29H28F3N5O5S | 616.1836 | -0.4 | -1.0 |
| B2N033-01 | C31H34F3N3O5S | 618.2244 | 0.0 | 0.0 |
| B2N034-01 | C30H33F3N4O5S | 619.2197 | -0.2 | 1.0 |
| B2N035-01 | C33H32F3N3O5S | 640.2088 | 0.0 | 0.0 |
| B2N036-01 | C32H31F3N4O5S | 641.204 | 0.0 | 0.4 |
| B2N037-01 | C33H38F3N3O5S | 646.2557 | 0.0 | 0.0 |
| B2N038-01 | C32H37F3N4O5S | 647.251 | 0.0 | 0.0 |
| B2N039-01 | C30H29F6N3O5S | 658.1805 | na | na |
| B2N040-01 | C29H28F6N4O5S | 659.1757 | 0.0 | 0.0 |
| B2N041-01 | C32H29F6N3O5S | 682.1805 | na | na |
| B2N042-01 | C31H28F6N4O5S | 683.1757 | 0.0 | 0.0 |
| B2N043-01 | C33H31F6N5O5S | 724.2023 | -1.4 | 0.1 |
| B2N044-01 | C32H30F6N6O5S | 725.1975 | 0.2 | 1.4 |
| B2N045-01 | C29H32F3N3O4S | 576.2138 | 0.0 | 0.1 |
| B2N046-01 | C28H31F3N4O4S | 577.2091 | 0.0 | -1.2 |
| B2N047-01 | C30H34F3N3O4S | 590.2295 | 0.0 | 0.0 |
| B2N048-01 | C29H33F3N4O4S | 591.2247 | 0.0 | -0.7 |
| B2N049-01 | C31H36F3N3O4S | 604.2451 | 0.0 | 0.0 |
| B2N050-01 | C30H35F3N4O4S | 605.2404 | 0.0 | na |

| Compound No. | Molecular formula | m/z (expected) | ASMS ratio(%) | |
|---|---|---|---|---|
| | | | n=1 | n=2 |
| B2N051-01 | C29H31F4N3O4S | 594.2044 | 0.0 | 0.0 |
| B2N052-01 | C28H30F4N4O4S | 595.1997 | 0.0 | -0.5 |
| B2N053-01 | C30H34F3N3O5S | 606.2244 | 0.0 | 1.4 |
| B2N054-01 | C29H33F3N4O5S | 607.2197 | 0.0 | 0.0 |
| B2N055-01 | C31H36F3N3O5S | 620.2401 | 0.0 | 0.0 |
| B2N056-01 | C30H35F3N4O5S | 621.2353 | 0.0 | 0.0 |
| B2N057-01 | C32H38F3N3O5S | 634.2557 | 0.0 | -1.8 |
| B2N058-01 | C31H37F3N4O5S | 635.251 | 0.0 | 0.0 |
| B2N059-01 | C31H36F3N3O6S | 636.235 | 0.0 | 0.0 |
| B2N060-01 | C30H35F3N4O6S | 637.2302 | 0.0 | 0.0 |
| B2N061-01 | C30H31F6N3O4S | 644.2012 | 0.0 | 0.0 |
| B2N062-01 | C29H30F6N4O4S | 645.1965 | 0.0 | 0.0 |
| B2N063-01 | C30H31F6N3O5S | 660.1961 | -0.3 | -3.8 |
| B2N064-01 | C29H30F6N4O5S | 661.1914 | 0.0 | 0.0 |
| B2N065-01 | C33H34F3N3O4S | 626.2295 | 0.0 | 0.0 |
| B2N066-01 | C32H33F3N4O4S | 627.2247 | 0.0 | 0.0 |
| B2N067-01 | C27H30F3N3O4S2 | 582.1703 | 0.0 | 0.0 |
| B2N068-01 | C26H29F3N4O4S2 | 583.1655 | 0.0 | 0.5 |
| B2N069-01 | C31H32F3N3O4S | 600.2138 | -1.8 | -1.5 |
| B2N070-01 | C30H31F3N4O4S | 601.2091 | 0.0 | 0.0 |
| B2N071-01 | C27H30F3N5O4S | 578.2043 | 0.0 | 0.0 |
| B2N072-01 | C26H29F3N6O4S | 579.1996 | 0.0 | 0.1 |
| B2N073-01 | C28H32F3N5O4S | 592.22 | 0.0 | 0.0 |
| B2N074-01 | C27H31F3N6O4S | 593.2152 | 0.0 | 0.0 |
| B2N075-01 | C29H34F3N5O4S | 606.2356 | na | na |
| B2N076-01 | C28H33F3N6O4S | 607.2309 | 0.0 | 0.0 |
| B2N077-01 | C27H29F4N5O4S | 596.1949 | 0.0 | 0.0 |
| B2N078-01 | C26H28F4N6O4S | 597.1902 | 0.0 | 0.0 |
| B2N079-01 | C28H32F3N5O5S | 608.2149 | 0.0 | 0.0 |
| B2N080-01 | C27H31F3N6O5S | 609.2102 | 0.0 | 0.0 |
| B2N081-01 | C29H34F3N5O5S | 622.2306 | 0.0 | 0.0 |
| B2N082-01 | C28H33F3N6O5S | 623.2258 | 2.5 | 2.8 |
| B2N083-01 | C30H36F3N5O5S | 636.2462 | 0.0 | 0.0 |
| B2N084-01 | C29H35F3N6O5S | 637.2415 | 0.0 | 0.0 |
| B2N085-01 | C29H34F3N5O6S | 638.2255 | 0.0 | 0.0 |
| B2N086-01 | C28H33F3N6O6S | 639.2207 | 0.0 | 0.0 |
| B2N087-01 | C28H29F6N5O4S | 646.1917 | 0.0 | 0.0 |
| B2N088-01 | C27H28F6N6O4S | 647.187 | 0.0 | 0.0 |
| B2N089-01 | C28H29F6N5O5S | 662.1866 | na | 0.0 |
| B2N090-01 | C27H28F6N6O5S | 663.1819 | 0.0 | 0.0 |
| B2N091-01 | C31H32F3N5O4S | 628.22 | 0.0 | 0.0 |
| B2N092-01 | C30H31F3N6O4S | 629.2152 | 0.0 | 0.0 |
| B2N093-01 | C25H28F3N5O4S2 | 584.1608 | 0.0 | na |
| B2N094-01 | C24H27F3N6O4S2 | 585.156 | 0.0 | 0.0 |
| B2N095-01 | C29H30F3N5O4S | 602.2043 | 0.0 | 0.0 |
| B2N096-01 | C28H29F3N6O4S | 603.1996 | na | na |
| B2N097-01 | C30H34F3N3O4S | 590.2295 | 0.0 | 0.0 |
| B2N098-01 | C29H33F3N4O4S | 591.2247 | 0.0 | -0.7 |
| B2N099-01 | C30H33F4N3O4S | 608.2201 | 0.0 | 0.0 |
| B2N100-01 | C29H32F4N4O4S | 609.2153 | 0.0 | 0.0 |
| B2R001-01 | C37H35F3N6O5S3 | 797.1856 | na | na |
| B2R002-01 | C36H34F3N7O5S3 | 798.1808 | 0.0 | 0.0 |
| B2R003-01 | C40H39F3N6O5S2 | 805.2448 | 21.6 | 0.0 |
| B2R004-01 | C39H38F3N7O5S2 | 806.2401 | 0.0 | 0.0 |
| B2R005-01 | C39H36F4N6O5S2 | 809.2198 | 44.0 | 39.5 |
| B2R006-01 | C38H35F4N7O5S2 | 810.215 | na | na |
| B2R007-01 | C41H37F3N6O5S2 | 815.2292 | 0.0 | na |
| B2R008-01 | C40H36F3N7O5S2 | 816.2244 | 0.0 | 19.5 |
| B2R009-01 | C40H36F3N7O5S2 | 816.2244 | 0.0 | 19.5 |
| B2R010-01 | C39H35F3N8O5S2 | 817.2197 | 0.0 | 0.0 |
| B2R011-01 | C41H41F3N6O5S2 | 819.2605 | 17.7 | -1.3 |
| B2R012-01 | C40H40F3N7O5S2 | 820.2557 | 15.8 | 13.5 |
| B2R013-01 | C43H39F3N6O5S2 | 841.2448 | 7.5 | 7.7 |
| B2R014-01 | C42H38F3N7O5S2 | 842.2401 | 18.6 | 15.1 |
| B2R015-01 | C43H45F3N6O5S2 | 847.2918 | 9.7 | 0.0 |
| B2R016-01 | C42H44F3N7O5S2 | 848.287 | 9.1 | 2.7 |
| B2R017-01 | C40H36F6N6O5S2 | 859.2166 | 32.9 | 27.2 |
| B2R018-01 | C39H35F6N7O5S2 | 860.2118 | 5.1 | 7.8 |
| B2R019-01 | C42H36F6N6O5S2 | 883.2166 | 5.2 | 14.0 |
| B2R020-01 | C41H35F6N7O5S2 | 884.2118 | 10.8 | -17.7 |

na = not available

[Table 141]

| Compound No. | Molecular formula | m/z (expected) | ASMS ratio(%) | |
|---|---|---|---|---|
| | | | n=1 | n=2 |
| B2R021-01 | C43H38F6N8O5S2 | 925.2384 | 13.0 | 0.0 |
| B2R022-01 | C42H37F6N9O5S2 | 926.2336 | 14.5 | 15.8 |
| B2R023-01 | C39H37F3N4O5S3 | 795.1951 | 0.0 | -15.5 |
| B2R024-01 | C38H36F3N5O5S3 | 796.1903 | 0.0 | -1.2 |
| B2R025-01 | C42H41F3N4O5S2 | 803.2543 | -1.0 | -2.4 |
| B2R026-01 | C41H40F3N5O5S2 | 804.2496 | 1.7 | -4.6 |
| B2R027-01 | C41H38F4N4O5S2 | 807.2293 | -3.7 | 1.2 |
| B2R028-01 | C40H37F4N5O5S2 | 808.2245 | 4.1 | -0.5 |
| B2R029-01 | C43H39F3N4O5S2 | 813.2387 | na | 0.0 |
| B2R030-01 | C42H38F3N5O5S2 | 814.2339 | 0.0 | 4.8 |
| B2R031-01 | C42H38F3N5O5S2 | 814.2339 | 0.0 | 4.8 |
| B2R032-01 | C41H37F3N6O5S2 | 815.2292 | 6.4 | 0.0 |
| B2R033-01 | C43H43F3N4O5S2 | 817.27 | 3.6 | -6.6 |
| B2R034-01 | C42H42F3N5O5S2 | 818.2652 | 0.1 | -1.3 |
| B2R035-01 | C45H41F3N4O5S2 | 839.2543 | 8.3 | 0.9 |
| B2R036-01 | C44H40F3N5O5S2 | 840.2496 | 3.2 | -0.8 |
| B2R037-01 | C45H47F3N4O5S2 | 845.3013 | 6.3 | -7.0 |
| B2R038-01 | C44H46F3N5O5S2 | 846.2965 | 7.1 | -6.0 |
| B2R039-01 | C42H38F6N4O5S2 | 857.2261 | 9.7 | -4.8 |
| B2R040-01 | C41H37F6N5O5S2 | 858.2213 | 2.6 | 0.2 |
| B2R041-01 | C44H38F6N4O5S2 | 881.2261 | 18.0 | 0.0 |
| B2R042-01 | C43H37F6N5O5S2 | 882.2213 | 3.4 | 6.0 |
| B2R043-01 | C45H40F6N6O5S2 | 923.2479 | 3.4 | -9.0 |
| B2R044-01 | C44H39F6N7O5S2 | 924.2431 | -3.7 | -4.1 |
| B2R045-01 | C41H41F3N4O4S2 | 775.2594 | 4.8 | -3.4 |
| B2R046-01 | C40H40F3N5O4S2 | 776.2547 | -0.2 | 0.0 |
| B2R047-01 | C42H43F3N4O4S2 | 789.2751 | 6.6 | -7.8 |
| B2R048-01 | C41H42F3N5O4S2 | 790.2703 | 9.2 | 7.5 |
| B2R049-01 | C43H45F3N4O4S2 | 803.2907 | 7.5 | -5.5 |
| B2R050-01 | C42H44F3N5O4S2 | 804.286 | 0.0 | 0.0 |
| B2R051-01 | C41H40F4N4O4S2 | 793.25 | 12.7 | -1.8 |
| B2R052-01 | C40H39F4N5O4S2 | 794.2452 | 0.0 | -7.4 |
| B2R053-01 | C42H43F3N4O5S2 | 805.27 | -15.0 | 0.0 |
| B2R054-01 | C41H42F3N5O5S2 | 806.2652 | 3.3 | -0.8 |
| B2R055-01 | C43H45F3N4O5S2 | 819.2856 | -3.3 | 6.3 |
| B2R056-01 | C42H44F3N5O5S2 | 820.2809 | na | -63.9 |
| B2R057-01 | C44H47F3N4O5S2 | 833.3013 | 5.7 | -5.1 |
| B2R058-01 | C43H46F3N5O5S2 | 834.2965 | 13.0 | 4.8 |
| B2R059-01 | C43H45F3N4O6S2 | 835.2805 | 0.0 | 0.0 |
| B2R060-01 | C42H44F3N5O6S2 | 836.2758 | 3.9 | 0.0 |

| Compound No. | Molecular formula | m/z (expected) | ASMS ratio(%) | |
|---|---|---|---|---|
| | | | n=1 | n=2 |
| B2R061-01 | C42H40F6N4O4S2 | 843.2468 | 9.1 | -6.0 |
| B2R062-01 | C41H39F6N5O4S2 | 844.242 | 0.0 | 0.0 |
| B2R063-01 | C42H40F6N4O5S2 | 859.2417 | 8.2 | 4.6 |
| B2R064-01 | C41H39F6N5O5S2 | 860.237 | 4.2 | -7.4 |
| B2R065-01 | C45H43F3N4O4S2 | 825.2751 | 3.8 | 0.3 |
| B2R066-01 | C44H42F3N5O4S2 | 826.2703 | 19.8 | -10.7 |
| B2R067-01 | C39H39F3N4O4S3 | 781.2158 | 8.0 | 0.0 |
| B2R068-01 | C38H38F3N5O4S3 | 782.2111 | 0.0 | 0.0 |
| B2R069-01 | C43H41F3N4O4S2 | 799.2594 | 12.3 | -18.5 |
| B2R070-01 | C42H40F3N5O4S2 | 800.2547 | na | na |
| B2R071-01 | C39H39F3N6O4S2 | 777.2499 | 14.3 | -9.0 |
| B2R072-01 | C38H38F3N7O4S2 | 778.2452 | 21.4 | 11.4 |
| B2R073-01 | C40H41F3N6O4S2 | 791.2656 | 24.0 | 0.0 |
| B2R074-01 | C39H40F3N7O4S2 | 792.2608 | 22.4 | 25.4 |
| B2R075-01 | C41H43F3N6O4S2 | 805.2812 | 8.6 | 0.0 |
| B2R076-01 | C40H42F3N7O4S2 | 806.2765 | 3.9 | 19.7 |
| B2R077-01 | C39H38F4N6O4S2 | 795.2405 | 0.0 | 0.0 |
| B2R078-01 | C38H37F4N7O4S2 | 796.2357 | 15.8 | 11.7 |
| B2R079-01 | C40H41F3N6O5S2 | 807.2605 | na | na |
| B2R080-01 | C39H40F3N7O5S2 | 808.2557 | 16.7 | 21.2 |
| B2R081-01 | C41H43F3N6O5S2 | 821.2761 | 21.1 | -8.8 |
| B2R082-01 | C40H42F3N7O5S2 | 822.2714 | 0.0 | 0.0 |
| B2R083-01 | C42H45F3N6O5S2 | 835.2918 | 31.9 | -11.1 |
| B2R084-01 | C41H44F3N7O5S2 | 836.287 | na | na |
| B2R085-01 | C41H43F3N6O6S2 | 837.271 | na | na |
| B2R086-01 | C40H42F3N7O6S2 | 838.2663 | 0.0 | 0.0 |
| B2R087-01 | C40H38F6N6O4S2 | 845.2373 | 3.8 | 7.1 |
| B2R088-01 | C39H37F6N7O4S2 | 846.2325 | 35.3 | 13.1 |
| B2R089-01 | C40H38F6N6O5S2 | 861.2322 | 3.4 | -9.2 |
| B2R090-01 | C39H37F6N7O5S2 | 862.2275 | 22.4 | 5.3 |
| B2R091-01 | C43H41F3N6O4S2 | 827.2656 | -3.5 | 1.8 |
| B2R092-01 | C42H40F3N7O4S2 | 828.2608 | 16.0 | -1.3 |
| B2R093-01 | C37H37F3N6O4S3 | 783.2063 | 56.5 | 0.0 |
| B2R094-01 | C36H36F3N7O4S3 | 784.2016 | 18.8 | 11.9 |
| B2R095-01 | C41H39F3N6O4S2 | 801.2499 | 42.2 | 5.5 |
| B2R096-01 | C40H38F3N7O4S2 | 802.2452 | na | na |
| B2R097-01 | C42H43F3N4O4S2 | 789.2751 | 6.6 | -7.8 |
| B2R098-01 | C41H42F3N5O4S2 | 790.2703 | 9.2 | 7.5 |
| B2R099-01 | C42H42F4N4O4S2 | 807.2656 | -12.6 | -7.5 |
| B2R100-01 | C41H41F4N5O4S2 | 808.2609 | 0.6 | 10.1 |

na = not available

In addition to two compounds B2Q045-01 (49.9%, 49.9%) and B2Q046-01 (20.3%, 20.5%), which were included as positive controls, binding to Bcl-2 was detected for multiple compounds. Among those compounds, 11 compounds, B2Q047-01 (142.5%, 326.4%), B2Q097-01 (142.5%, 326.4%), B2Q048-01 (61.6%, 90.8%), B2Q098-01 (61.6%, 90.8%), B2Q053-01 (22.4%, 15.5%), B2Q054-01 (44.1%, 65.0%), B2Q066-01 (40.7%, 36.9%), B1A075-01 (31.3%, 28.6%), B3A073-01 (31.3%, 28.6%), B2R074-01 (22.4%, 25.4%), and B2Q012-01 (21.4%, 22.5%), were synthesized separately and individually (described in Example 8), and a binding confirmation experiment by SPR was performed in Example 9.

Example 8: Individual compound synthesis of Bcl-2 binding compounds identified by AS-MS experiment

Example 8-1-1: Synthesis of 4-[4-[[4-(3-ethoxyphenyl)-3-methylphenyl]methyl]piperazin-1-yl]-N-[3-nitro-4-(2-phenylsulfanylethylamino)phenyl]sulfonylbenzamide [AG016 (B2Q047-01)]

[Formula 126]

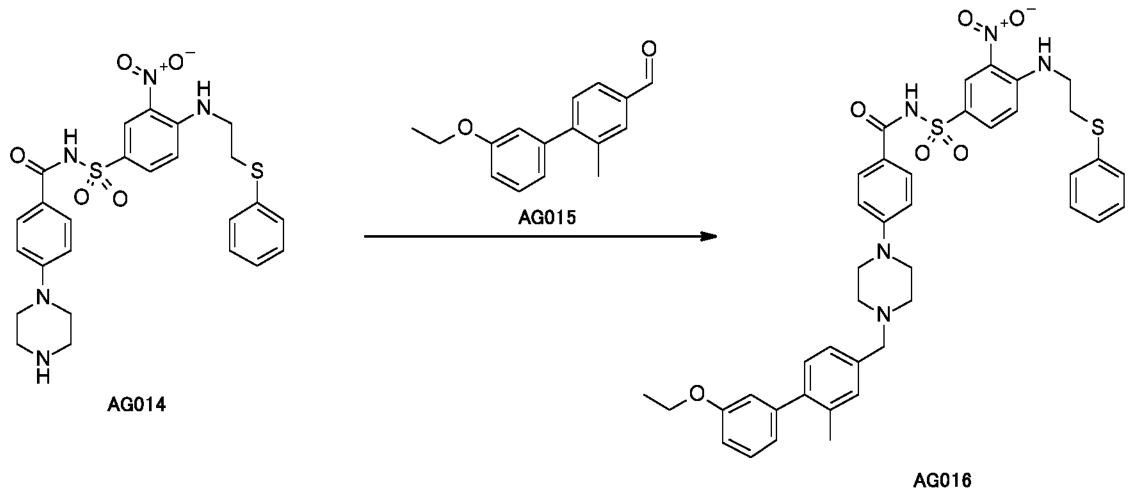


AG014

AG016

A solution of N-[3-nitro-4-(2-phenylsulfanylethylamino)phenyl]sulfonyl-4-piperazin-1-ylbenzamide (AG014, CAS: 406233-75-8) (150.00 mg, 0.277 mmol), 4-(3-ethoxyphenyl)-3-methylbenzaldehyde (AG015, CAS: 1506205-91-9) (79.86 mg, 0.332 mmol), and acetic acid (0.20 mL, 3.490 mmol) in DMSO/ethanol = 1/1 (3.00 mL) was stirred at room temperature for 2 hours. Sodium cyanoborohydride (34.81 mg, 0.554 mmol) was added, and the mixture was stirred at room temperature for 2 hours. After concentrating the mixture, the obtained residue was purified by reversed phase column chromatography (C18, 5 to 75% acetonitrile/10 mM aqueous ammonium bicarbonate solution). The obtained crude product was purified by preparative HPLC (Xselect CSH OBD column, 49 to 59% acetonitrile/10 mM aqueous ammonium bicarbonate solution) to afford the title compound AG016 (50 mg, 23.1%) as a yellow solid.

$^1$H-NMR (300 MHz, $CDCl_3$) $\delta$ 8.88 (s, 1H), 8.67 (s, 1H), 8.15 (d, J = 9.0 Hz, 1H), 7.69 (d, J = 8.4 Hz, 2H), 7.45-7.30 (m, 5H), 7.24 (d, J = 12.9 Hz, 4H), 6.92-6.80 (m, 5H), 4.08 (q, J = 7.2 Hz, 2H), 3.66-3.56 (m, 4H), 3.38-3.20 (m, 4H), 3.21 (s, 2H), 2.71 (s, 4H), 2.29 (s, 3H), 1.45 (t, J = 6.9 Hz, 3H).

LCMS (ESI): m/z 766 $[M+H]^+$.

AG016, whose synthetic method is illustrated in Example 8-1-1, may also be denoted as compound B2Q047-01 in the present specification.

Example 8-2-1: Synthesis of 4-[4-[[4-(5-ethoxypyridin-3-yl)-3-methylphenyl]methyl]piperazin-1-yl]-N-[3-nitro-4-(2-phenylsulfanylethylamino)phenyl] sulfonylbenzamide [AG018 (B2Q048-01)]

[Formula 127]

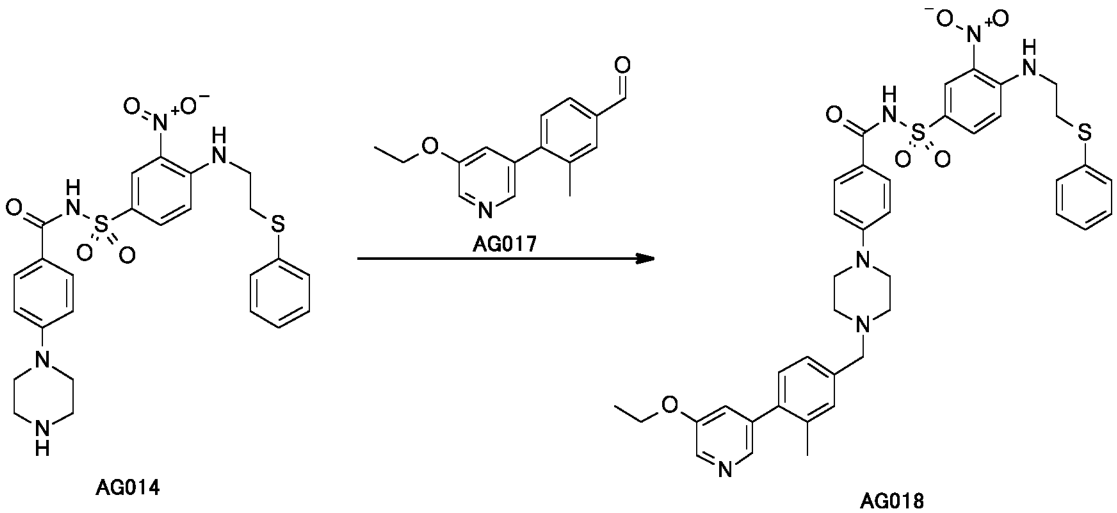


AG014

AG018

A solution of N-[3-nitro-4-(2-phenylsulfanylethylamino)phenyl]sulfonyl-4-piperazin-1-ylbenzamide (AG014,

CAS: 406233-75-8) (100.00 mg, 0.185 mmol), 4-(5-ethoxypyridin-3-yl)-3-methylbenzaldehyde (AG017, CAS: 1545014-81-0) (89.10 mg, 0.370 mmol), and acetic acid (0.05 mL, 0.873 mmol) in DMSO/ethanol (1 mL/1 mL) was stirred at room temperature for 1 hour. Sodium cyanoborohydride (23.20 mg, 0.370 mmol) was added, and the mixture was stirred at room temperature for 16 hours. After concentrating the mixture, the obtained residue was purified by silica gel column chromatography (0 to 100% ethyl acetate/petroleum ether). The obtained crude product was purified by preparative HPLC (XBridge Shield RP18 OBD column, 42 to 61% acetonitrile/10 mM ammonium bicarbonate + 0.05% 5M aqueous ammonia solution) to afford the title compound AG018 (64.1 mg, 43.87%) as a yellow solid.

$^1$H-NMR (400 MHz, DMSO-$d_6$) δ 8.68 (s, 1H), 8.55 (d, J = 2.0 Hz, 1H), 8.28 (d, J = 2.8 Hz, 1H), 8.14 (d, J = 2.0 Hz, 1H), 7.91-7.85 (m, 1H), 7.75 (d, J = 8.8 Hz, 2H), 7.42-7.34 (m, 3H), 7.28-7.25 (m, 5H), 7.19-7.09 (m, 1H), 7.11 (d, J = 9.6 Hz, 1H), 6.90 (d, J = 8.8 Hz, 2H), 4.16 (q, J = 8.0 Hz, 2H), 3.68-3.61 (m, 4H), 3.33-3.25 (m, 6H), 2.70-2.51 (m, 4H), 2.26 (s, 3H), 1.32 (t, J = 8.0 Hz, 3H).

LCMS (ESI): m/z 767 $[M+H]^+$.

AG018, whose synthetic method is illustrated in Example 8-2-1, may also be denoted as compound B2Q048-01 in the present specification.

Example 8-3-1: Synthesis of 4-(5-ethoxypyridin-3-yl)-2-methoxybenzaldehyde (AG021)

[Formula 128]

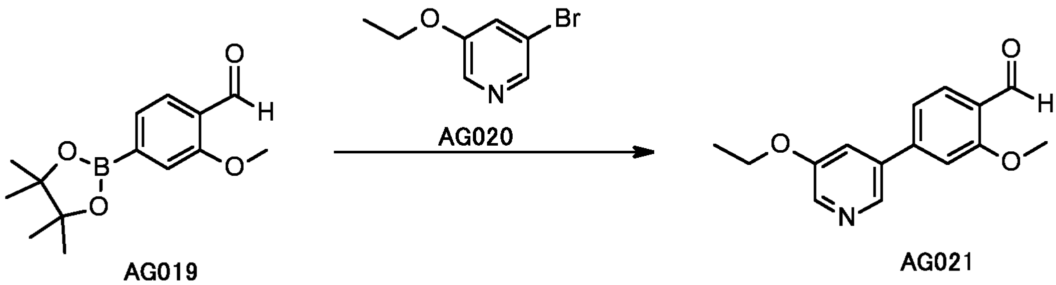


AG019

AG021

Under a nitrogen atmosphere, to a solution of 2-methoxy-4-(4,4,5,5-tetramethyl-1,3,2-dioxaborolan-2-yl)benzaldehyde (AG019, CAS: 956431-01-9) (300 mg, 1.145 mmol) in 1,4-dioxane/water (6 mL/0.6 mL) were added 3-bromo-5-ethoxypyridine (AG020, CAS: 17117-17-8) (277.5 mg, 1.374 mmol), potassium acetate (224.7 mg, 2.290 mmol), and Pd(dppf)$Cl_2$ (83.8 mg, 0.115 mmol), and the mixture was stirred at 90°C for 3 hours. After filtering the mixture and concentrating the filtrate, the obtained residue was purified by silica gel column chromatography (0 to 20% ethyl acetate/petroleum ether) to afford the title compound AG021 (250 mg, 80.7%) as a white solid.

LCMS (ESI): m/z 258 $[M+H]^+$.

Retention time: 0.845 min (analysis condition PH-TFA-13).

Example 8-3-2: Synthesis of 4-[4-[[4-(5-ethoxypyridin-3-yl)-2-methoxyphenyl]methyl]piperazin-1-yl]-N-[3-nitro-4-(2-phenylsulfanylethylamino)phenyl]sulfonylbenzamide [AG022 (B2Q054-01)]

[Formula 129]

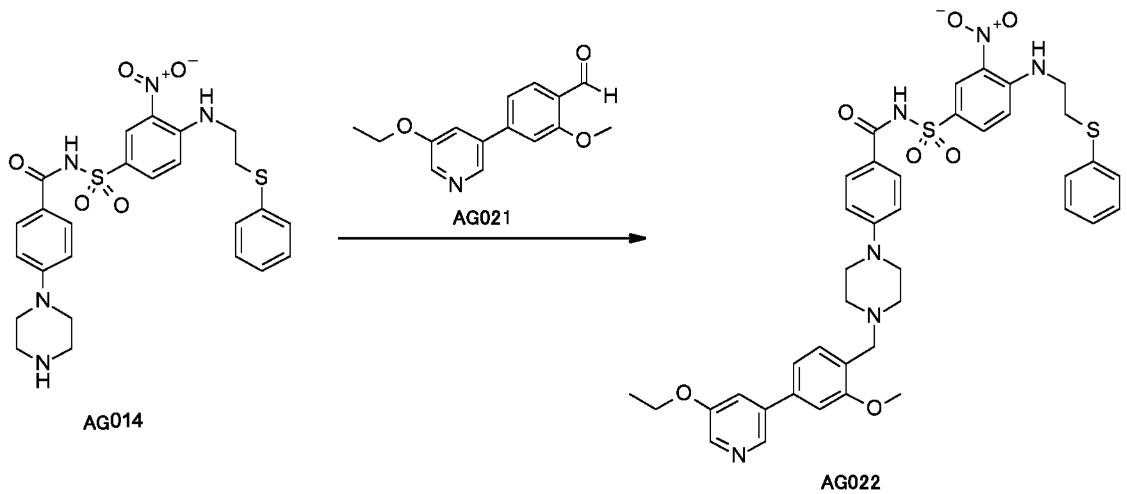

AG014 AG021 AG022

A solution of N-[3-nitro-4-(2-phenylsulfanylethylamino)phenyl]sulfonyl-4-piperazin-1-ylbenzamide (AG014, CAS: 406233-75-8) (150 mg, 0.277 mmol), 4-(5-ethoxypyridin-3-yl)-2-methoxybenzaldehyde (AG021) (106.9 mg, 0.416 mmol), and acetic acid (0.20 mL, 3.490 mmol) in DMSO/ethanol (3 mL/3 mL) was stirred at room temperature for 1 hour. Sodium cyanoborohydride (34.81 mg, 0.554 mmol) was added, and the mixture was stirred at room temperature for 16 hours. After concentrating the mixture, the obtained residue was purified by reversed phase column chromatography (C18, 0 to 80% acetonitrile/water) to afford the title compound AG022 (30 mg, 13.5%) as a white solid.

$^1$H-NMR (300 MHz, DMSO-$d_6$) $\delta$ 11.25 (s, 1H), 8.69 (t, J = 6.0 Hz, 1H), 8.59-8.52 (m, 2H), 8.28 (d, J = 2.7 Hz, 1H), 7.91-7.87 (m, 1H), 7.74 (d, J = 8.7 Hz, 2H), 7.64-7.63 (m, 1H), 7.49-7.46 (m, 1H), 7.39-7.31 (m, 4H), 7.30-7.24 (m, 2H), 7.20-7.09 (m, 2H), 6.91 (d, J = 8.7 Hz, 2H), 4.20 (q, J = 6.9 Hz, 2H), 3.92 (s, 3H), 3.79-3.74 (m, 2H), 3.67-3.60 (m, 2H), 3.39-3.23 (m, 6H), 2.72-2.68 (m, 4H), 1.37 (t, J = 6.9 Hz, 3H).

LCMS (ESI): m/z 783 $[M+H]^+$.

AG022, whose synthetic method is illustrated in Example 8-3-2, may also be denoted as compound B2Q054-01 in the present specification.

Example 8-4-1: Synthesis of 4-(5-ethoxypyridin-3-yl)naphthalene-1-carbaldehyde (AG024)

[Formula 130]

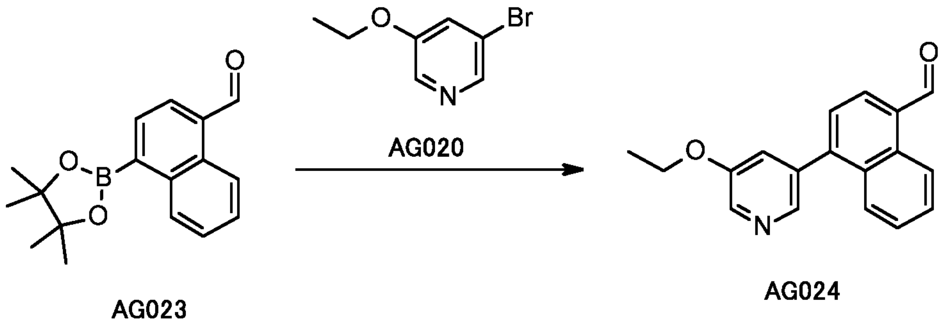

AG020 AG023 AG024

Under a nitrogen atmosphere, a solution of 4-(4,4,5,5-tetramethyl-1,3,2-dioxaborolan-2-yl)naphthalene-1-carbaldehyde (AG023, CAS: 1008361-71-4) (300 mg, 1.063 mmol), 3-bromo-5-ethoxypyridine (AG020, CAS: 17117-17-8) (179 mg, 0.886 mmol), Pd(dppf)$Cl_2$·DCM (72.2 mg, 0.089 mmol), and potassium acetate (173.89 mg, 1.772 mmol) in 1,4-dioxane/water (6 mL/0.60 mL) was stirred at 90°C for 2 hours. After filtering the mixture and concentrating the filtrate, the obtained residue was purified by silica gel column chromatography (0 to 40% ethyl acetate/petroleum ether) to afford the title compound AG024 (240 mg, 91%) as a pale yellow liquid.

LCMS (ESI): m/z 278 $[M+H]^+$.

Retention time: 0.769 min (analysis condition PH-FA-05).

Example 8-4-2: Synthesis of 4-[4-[[4-(5-ethoxypyridin-3-yl)naphthalen-1-yl]methyl]piperazin-1-yl]-N-[3-nitro-4-(2-phenylsulfanylethylamino)phenyl]sulfonylbenzamide [AG025 (B2Q066-01)]

[Formula 131]

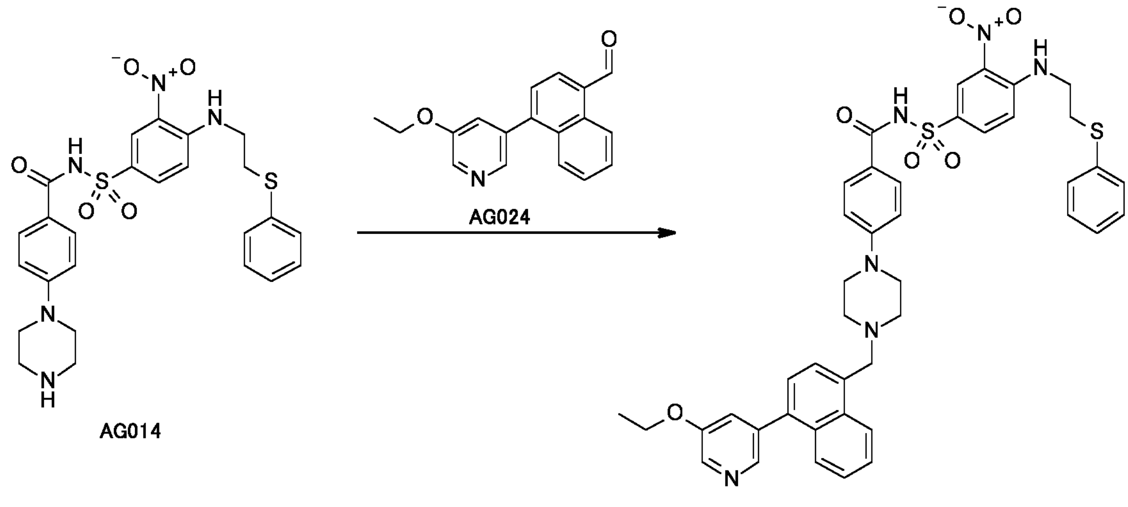


AG014

AG025

A solution of N-[3-nitro-4-(2-phenylsulfanylethylamino)phenyl]sulfonyl-4-piperazin-1-ylbenzamide (AG014, CAS: 406233-75-8) (180 mg, 0.332 mmol), 4-(5-ethoxypyridin-3-yl)naphthalene-1-carbaldehyde (AG024) (184 mg, 0.664 mmol), and acetic acid (0.05 mL, 0.873 mmol) in DMSO/ethanol (2 mL/2 mL) was stirred at room temperature for 1 hour. Sodium cyanoborohydride (41.77 mg, 0.664 mmol) was added, and the mixture was stirred at room temperature for 16 hours. After concentrating the mixture, the obtained residue was purified by silica gel column chromatography (0 to 100% ethyl acetate/petroleum ether, followed by 0 to 10% methanol/dichloromethane). The obtained crude product was purified by preparative HPLC (XBridge Prep OBD C18 column, 41 to 71% acetonitrile/10 mM aqueous ammonium bicarbonate solution) to afford the title compound AG025 (49.1 mg, 17.9%) as a yellow solid.

$^1$H-NMR (300 MHz, DMSO-$d_6$) $\delta$ 8.73 (s, 1H), 8.58 (d, J = 2.3 Hz, 1H), 8.43 (d, J = 8.4 Hz, 1H), 8.38 (d, J = 2.8 Hz, 1H), 8.24 (d, J = 1.7 Hz, 1H), 7.89 (dd, J = 9.2, 2.3 Hz, 1H), 7.76 (dd, J = 12.7, 8.9 Hz, 3H), 7.66-7.49 (m, 3H), 7.50-7.42 (m, 2H), 7.41-7.33 (m, 2H), 7.27 (t, J = 7.6 Hz, 2H), 7.20-7.11 (m, 2H), 6.92 (d, J = 8.7 Hz, 2H), 4.18 (q, J = 7.0 Hz, 2H), 4.07-4.00 (m, 2H), 3.68-3.60 (m, 2H), 3.32-3.21 (m, 6H), 2.68-2.60 (m, 4H), 1.37 (t, J = 6.9 Hz, 3H).

LCMS (ESI): m/z 803 $[M+H]^+$.

AG025, whose synthetic method is illustrated in Example 8-4-2, may also be denoted as compound B2Q066-01 in the present specification.

Example 8-5-1: Synthesis of 4-[4-[[2-(3-ethoxyphenyl)phenyl]methyl]piperazin-1-yl]-N-[3-nitro-4-(2-phenylsulfanvlethylamino)phenyllsulfonylbenzamide [AG005 (B2Q045-01)]

[Formula 132]

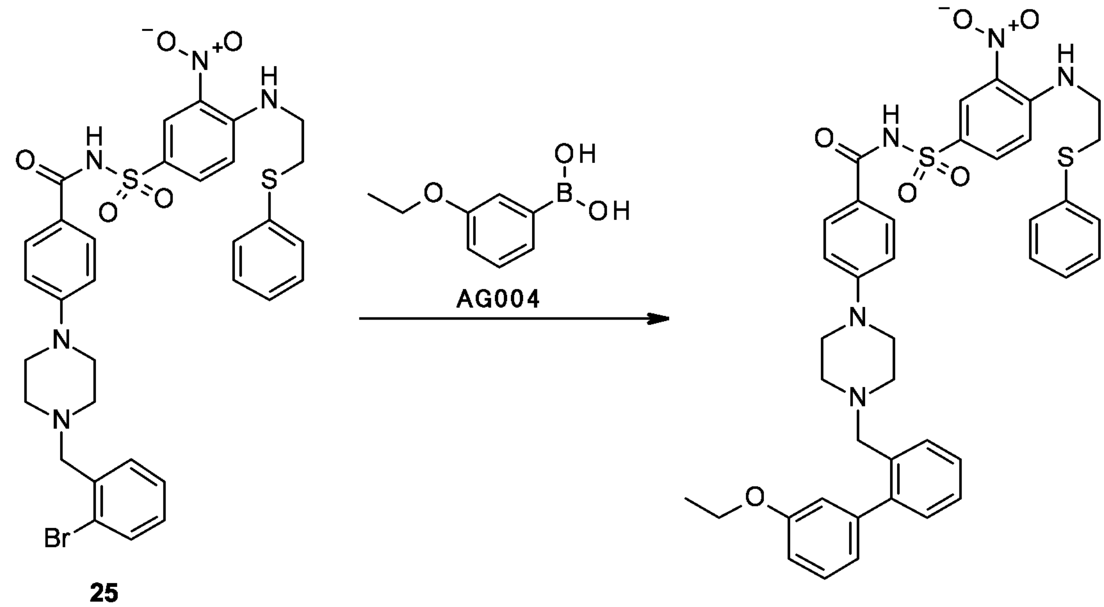


25

AG005

Under a nitrogen atmosphere, a solution of 4-[4-[(2-bromophenyl)methyl]piperazin-1-yl]-N-[3-nitro-4-(2-phenylsulfanylethylamino)phenyl]sulfonylbenzamide (compound 25) (250 mg, 0.352 mmol), (3-ethoxyphenyl)boronic acid (AG004, CAS: 90555-66-1) (116.8 mg, 0.704 mmol), $Pd(PPh_3)_2Cl_2$ (24.7 mg, 0.035 mmol), and sodium carbonate (48.5 mg, 0.457 mmol) in dimethoxyethane/ethanol/water = 1/1/1 (5 mL) was stirred at 80°C for 3 hours. The mixture was filtered, the filtrate was diluted with ethyl acetate, and water was added. The organic layer was extracted and dried over sodium sulfate. After concentration, the obtained residue was purified by reversed phase column chromatography (C18, 5 to 60% acetonitrile/water). The obtained crude product was purified by preparative HPLC (YMC-Actus Triart C18, 50 to 80% acetonitrile/10 mM aqueous ammonium bicarbonate solution) to afford the title compound AG005 (45 mg, 0.060 mmol, 16.9%) as a yellow solid.

$^1$H-NMR (300 MHz, DMSO-$d_6$) δ 11.93 (s, 1H), 8.72 (s, 1H), 8.55 (d, J = 2.4 Hz, 1H), 7.92-7.83 (m, 1H), 7.71 (d, J = 8.7 Hz, 2H), 7.52 (d, J = 7.2 Hz, 1H), 7.46-7.16 (m, 8H), 7.21-7.06 (m, 2H), 7.05-6.69 (m, 5H), 4.03 (q, J = 6.9 Hz, 2H), 3.63 (d, J = 6.6 Hz, 2H), 3.45 (s, 2H), 3.26 (t, J = 6.6 Hz, 6H), 2.41 (s, 4H), 1.30 (t, J = 6.9 Hz, 3H).

LCMS (ESI): m/z 752 $[M+H]^+$.

AG005, whose synthetic method is illustrated in Example 8-5-1, may also be denoted as compound B2Q045-01 in the present specification.

Example 8-6-1: Synthesis of 4-[4-[[2-(5-ethoxypyridin-3-yl)phenyl]methyl]piperazin-1-yl]-N-[3-nitro-4-(2-phenylsulfanylethylamino)phenyl] sulfonylbenzamide [AG007 (B2Q046-01)]

[Formula 133]

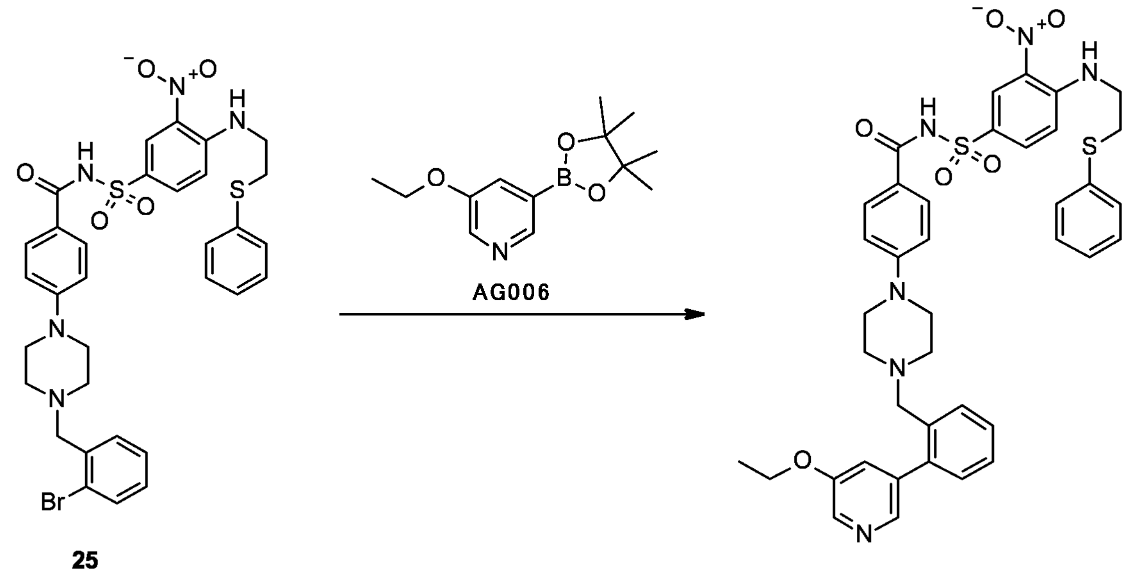


25

AG007

Under a nitrogen atmosphere, a solution of 4-[4-[(2-bromophenyl)methyl]piperazin-1-yl]-N-[3-nitro-4-(2-phenylsulfanylethylamino)phenyl]sulfonylbenzamide (compound 25) (350 mg, 0.492 mmol), 3-ethoxy-5-(4,4,5,5- tetramethyl-1,3,2-dioxaborolan-2-yl)pyridine (AG006, CAS: 1171892-40-2) (245.4 mg, 0.985 mmol), $Pd(PPh_3)_2Cl_2$ (34.6 mg, 0.049 mmol), and sodium carbonate (68.5 mg, 0.640 mmol) in dimethoxyethane/ethanol/water = 1/1/1 (6 mL) was stirred at 80°C for 3 hours. The mixture was filtered, the filtrate was diluted with ethyl acetate, and water was added. The organic layer was extracted and dried over sodium sulfate. After concentration, the obtained residue was purified by reversed phase column chromatography (C18, 5 to 80% acetonitrile/water). The obtained crude product was purified by preparative HPLC (XBridge Shield RP18 OBD column, 37 to 52% acetonitrile/10 mM aqueous ammonium bicarbonate solution) to afford the title compound AG007 (28.4 mg, 0.038 mmol, 7.6%) as a yellow solid.

$^1$H-NMR (300 MHz, DMSO-$d_6$) $\delta$ 11.97 (s, 1H), 8.68 (s, 1H), 8.53 (d, J = 2.1 Hz, 1H), 8.25 (d, J = 2.7 Hz, 1H), 8.16 (d, J = 1.8 Hz, 1H), 7.90-7.83 (m, 1H), 7.71 (d, J = 9.0 Hz, 2H), 7.59-7.46 (m, 2H), 7.46 -7.17 (m, 7H), 7.17-6.95 (m, 2H), 6.85 (d, J = 8.7 Hz, 2H), 4.11 (q, J = 6.9 Hz, 2H), 3.68-3.56 (m, 2H), 3.42 (s, 2H), 3.23 (s, 5H), 2.39 (s, 4H), 1.32 (t, J = 6.9 Hz, 3H).

LRMS (ESI): m/z 753 $[M+H]^+$.

AG007, whose synthetic method is illustrated in Example 8-6-1, may also be denoted as compound B2Q046-01 in the present specification.

Example 8-7-1: Synthesis of 4-(2-phenylsulfanylethylamino)-3-(trifluoromethyl)benzenesulfonamide [AG010 (BB42)]

[Formula 134]

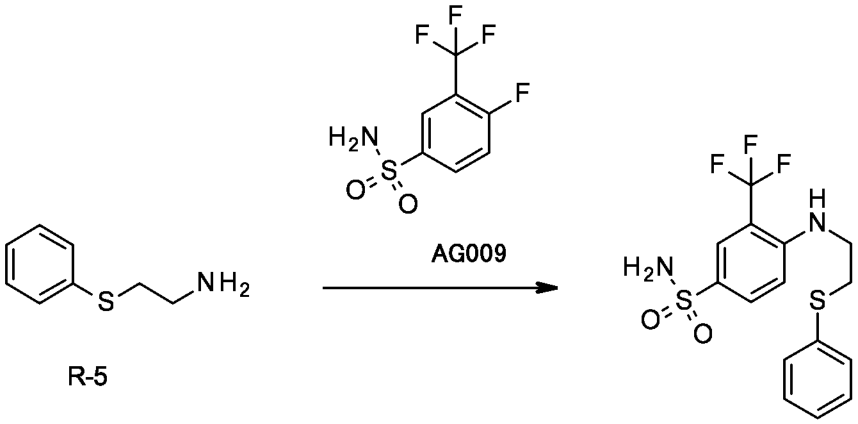


R-5

AG010

To a solution of 2-phenylsulfanylethanamine (R-5, CAS: 2014-75-7) (3.00 g, 19.48 mmol) and 4-fluoro-3-(trifluoromethyl)benzenesulfonamide (AG009, CAS: 1008304-87-7) (4.76 g, 19.48 mmol) in DMSO (30 mL) was added DIPEA (5.06 g, 38.96 mmol), and the mixture was stirred at 70°C for 16 hours. The mixture was purified by reversed phase column chromatography (C18, 5 to 80% acetonitrile/water) to afford the title compound AG010 (3.8 g, 10.1 mmol, 50.5%).

$^1$H-NMR (400 MHz, DMSO-$d_6$) $\delta$ 7.82 (d, J = 2.0 Hz, 1H), 7.79-7.73 (m, 1H), 7.45-7.38 (m, 2H), 7.36-7.30 (m, 2H), 7.26-7.19 (m, 1H), 7.15 (s, 2H), 6.89 (d, J = 8.0 Hz, 1H), 6.37 (t, J = 4.0 Hz, 1H), 3.52-3.44 (m, 2H), 3.32-3.14 (m, 2H).

LCMS (ESI): m/z 377 $[M+H]^+$.

AG010, whose synthetic method is illustrated in Example 8-7-1, may also be denoted as compound BB42 in the present Example.

Example 8-7-2: Synthesis of tert-butyl 4-[6-[[4-(2-phenylsulfanylethylamino)-3-(trifluoromethyl)phenyl]sulfonylcarbamoyl]pyridazin-3-yl]piperazine-1-carboxylate (AG027)

[Formula 135]

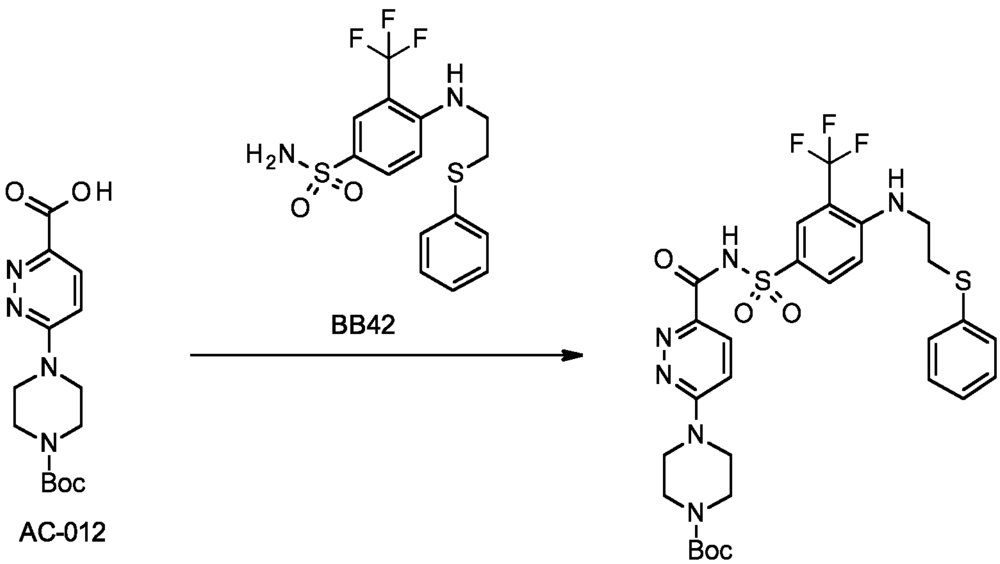


AC-012

AG027

To a solution of 6-[4-[(2-methylpropan-2-yl)oxycarbonyl]piperazin-1-yl]pyridazine-3-carboxylic acid (AC-012, CAS: 1690948-00-5) (400 mg, 1.297 mmol), 4-(2-phenylsulfanylethylamino)-3-(trifluoromethyl)benzenesulfonamide

(BB42) (488 mg, 1.297 mmol), EDCI (497 mg, 2.594 mmol), and DMAP (158 mg, 1.297 mmol) in dichloromethane (5 mL) was added DIPEA (671 mg, 5.188 mmol), and the mixture was stirred at room temperature for 16 hours. Water was added to the mixture, and the organic layer was extracted three times with ethyl acetate and dried over sodium sulfate. After concentration, the obtained residue was purified by silica gel column chromatography (0 to 100% ethyl acetate/petroleum ether, followed by 0 to 10% methanol/dichloromethane) to afford the title compound AG027 (400 mg, 43.9%) as a pale yellow solid.

LCMS (ESI): m/z 667 $[M+H]^+$.

Retention time: 1.185 min (analysis condition PH-FA-06).

Example 8-7-3: Synthesis of N-[4-(2-phenylsulfanylethylamino)-3-(trifluoromethyl)phenyl]sulfonyl-6-piperazin-1-ylpyridazine-3-carboxamide (AG028)

[Formula 136]

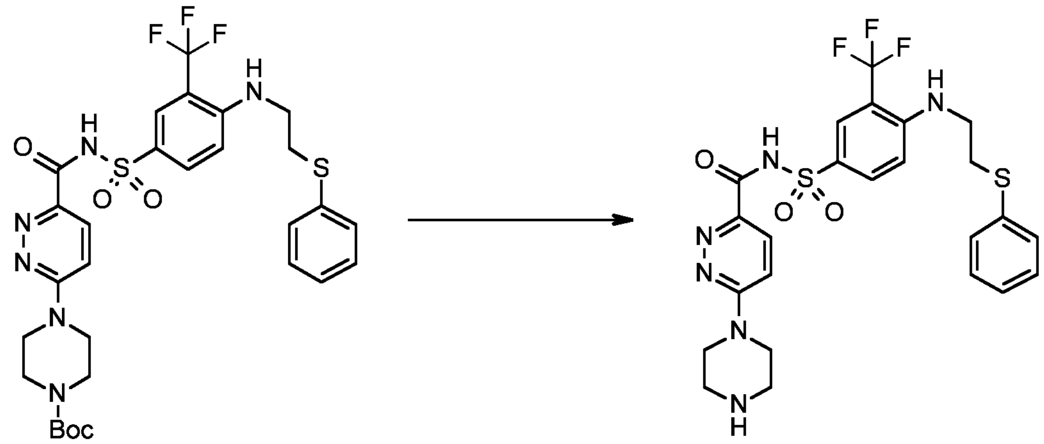

AG027 AG028

To a solution of tert-butyl 4-[6-[[4-(2-phenylsulfanylethylamino)-3-(trifluoromethyl)phenyl]sulfonylcarbamoyl]pyridazin-3-yl]piperazine-1-carboxylate (AG027) (200.00 mg, 0.300 mmol) in dichloromethane (4 mL) was added a solution of HCl in 1,4-dioxane (4M, 2 mL), and the mixture was stirred at room temperature for 16 hours. The mixture was concentrated and then dissolved in water, and an aqueous sodium bicarbonate solution was added to achieve a pH of 8. The resulting solid was filtered, then washed with water, and dried at 50°C for 16 hours to afford the title compound AG028 (150 mg) as a pale brown solid.

LCMS (ESI): m/z 567 $[M+H]^+$.

Retention time: 0.883 min (analysis condition PH-FA-06).

Example 8-7-4: Synthesis of 6-[4-[4-(3-ethoxyphenyl)-3-fluorobenzoyl]piperazin-1-yl]-N-[4-(2-phenylsulfanylethylamino)-3-(trifluoromethyl)phenyl]sulfonylpyridazine- 3-carboxamide [AG030 (B2R005-01)]

[Formula 137]

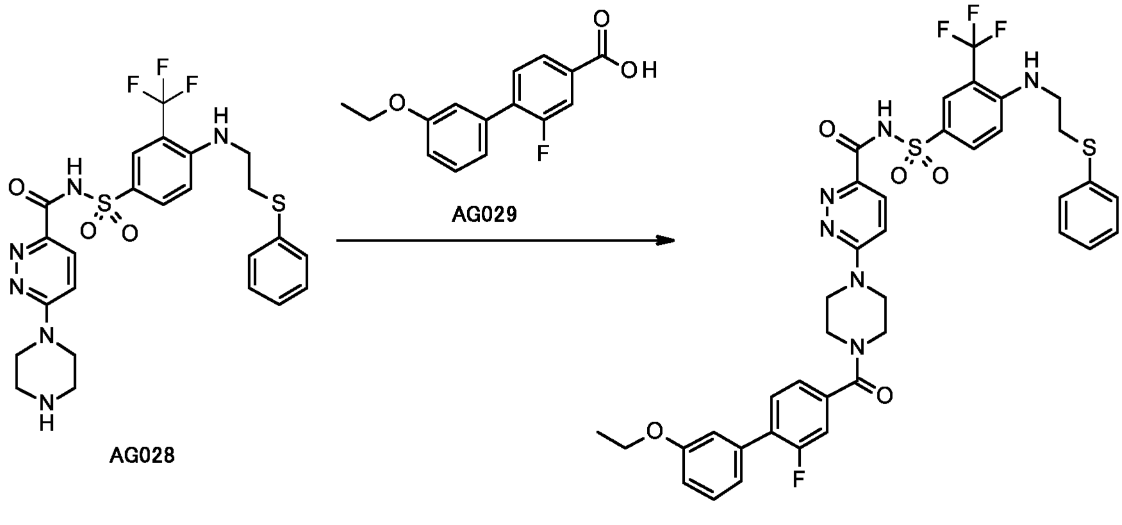


AG028

AG030

To a solution of N-[4-(2-phenylsulfanylethylamino)-3-(trifluoromethyl)phenyl]sulfonyl-6-piperazin-1-ylpyridazine-3-carboxamide (AG028) (70 mg, 0.124 mmol), 4-(3-ethoxyphenyl)-3-fluorobenzoic acid (AG029, CAS: 1261964-16-2) (32.2 mg, 0.124 mmol), EDCI (47.4 mg, 0.248 mmol), and DMAP (15.1 mg, 0.124 mmol) in dichloromethane (2 mL) was added DIPEA (63.9 mg, 0.496 mmol), and the mixture was stirred at room temperature for 16 hours. After concentrating the mixture, the obtained residue was purified by reversed phase column chromatography (C18, 0 to 50% acetonitrile/10 mM aqueous ammonium bicarbonate solution). The obtained crude product was purified by preparative HPLC (XBridge Prep OBD C18 column, 36 to 66% acetonitrile/10 mM aqueous ammonium bicarbonate solution) to afford the title compound AG030 (20 mg, 19.6%) as a white solid.

$^1$H-NMR (300 MHz, DMSO-$d_6$) $\delta$ 7.97 (s, 1H), 7.85 (t, J = 12.6 Hz, 2H), 7.64 (t, J = 8.1 Hz, 1H), 7.56-7.20 (m, 8H), 7.24-7.04 (m, 3H), 7.00 (d, J = 8.1 Hz, 1H), 6.86 (d, J = 9.0 Hz, 1H), 6.45 (s, 1H), 4.09 (q, J = 7.2 Hz, 2H), 3.85-3.73 (m, 5H), 3.59-3.45 (m, 5H), 3.20-3.13 (m, 2H), 1.35 (t, J = 6.9 Hz, 3H).

LCMS (ESI): m/z 809 $[M+H]^+$.

AG030, whose synthetic method is illustrated in Example 8-7-4, may also be denoted as compound B2R005-01 in the present specification.

Example 8-8-1: Synthesis of 6-[4-[3-(3-ethoxyphenyl)-5-(tnfluoromethyl)benzoyl]piperazin-1-yl]-N-[4-(2-phenylsulfanylethylamino)-3-(trifluoromethyl)phenyl]sulfonylpyridazine-3-carboxamide [AG032 (B2R017-01)]

[Formula 138]

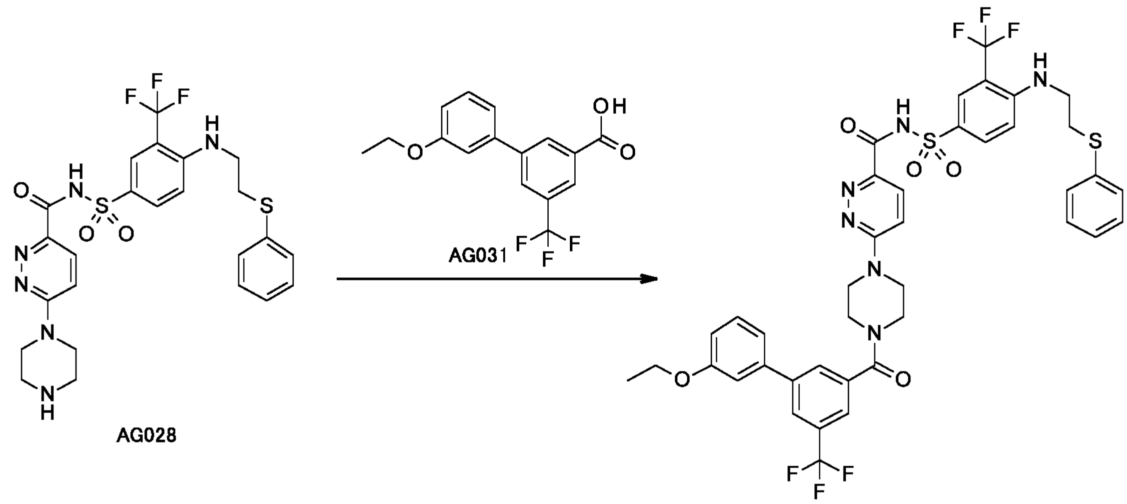


AG028

AG032

To a solution of N-[4-(2-phenylsulfanylethylamino)-3-(trifluoromethyl)phenyl]sulfonyl-6-piperazin-1-ylpyridazine-3-carboxamide (AG028) (70 mg, 0.124 mmol), 3-(3-ethoxyphenyl)-5-(trifluoromethyl)benzoic acid (AG031, CAS: 1261907-60-1) (69.00 mg, 0.223 mmol), EDCI (47.37 mg, 0.248 mmol), and DMAP (15.09 mg, 0.124 mmol) in dichloromethane (2 mL) was added DIPEA (63.87 mg, 0.496 mmol), and the mixture was stirred at room temperature for 16 hours. After concentrating the mixture, the obtained residue was purified by reversed phase column chromatography (C18, 0 to 45% acetonitrile/10 mM aqueous ammonium bicarbonate solution). The obtained crude product was purified by preparative HPLC (Xselect CSH OBD column, 68 to 83% acetonitrile/0.1% aqueous formic acid solution) to afford the title compound AG032 (30 mg, 28.2%) as a white solid.

$^1$H-NMR (300 MHz, DMSO-$d_6$) $\delta$ 12.22 (s, 1H), 8.12 (d, J = 11.7 Hz, 1H), 8.06 (s, 1H), 8.00 (d, J = 2.1 Hz, 1H), 7.94-7.87 (m, 1H), 7.82 (d, J = 9.6 Hz, 2H), 7.50-7.27 (m, 8H), 7.24-7.17 (m, 1H), 7.05-6.98 (m, 1H), 6.93 (d, J = 9.0 Hz, 1H), 6.64 (s, 1H), 4.14 (q, J = 6.9 Hz, 2H), 3.96-3.76 (m, 6H), 3.59-3.43 (m, 4H), 3.22-3.13 (m, 2H), 1.36 (t, J = 6.9 Hz, 3H).

LCMS (ESI): m/z 859 $[M+H]^+$.

AG032, whose synthetic method is illustrated in Example 8-8-1, may also be denoted as compound B2R017-01 in the present Example.

<u>Example 8-9-1: Synthesis of 4-[4-[(2-bromophenyl)methyl]piperazin-1-yl]-N-[4-(2-phenylsulfanylethylamino)-3-(trifluoromethyl)phenyl]sulfonylbenzamide (AGO 11)</u>

[Formula 139]

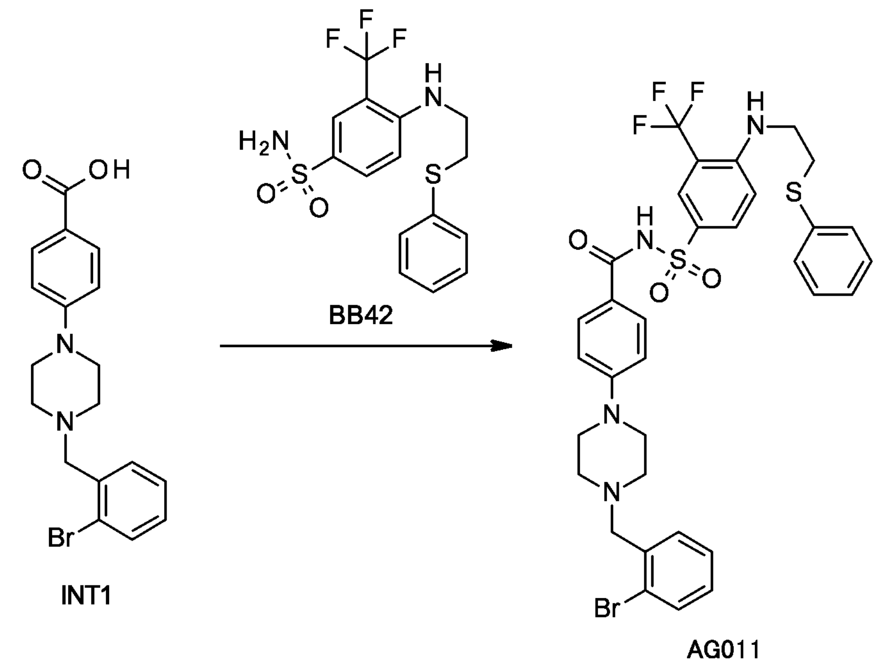

To a solution of 4-(2-phenylsulfanylethylamino)-3-(trifluoromethyl)benzenesulfonamide (BB42) (0.80 g, 2.125 mmol), 4-[4-[(2-bromophenyl)methyl]piperazin-1-yl]benzoic acid (INT1) (1.20 g, 3.188 mmol), EDCI (0.81 g, 4.251 mmol), and DMAP (0.26 g, 2.125 mmol) in dichloromethane (16 mL) was added DIPEA (1.10 g, 8.501 mmol), and the mixture was stirred at room temperature for 16 hours. An aqueous ammonium chloride solution was added to the mixture. The organic layer was extracted three times with dichloromethane and dried over sodium sulfate. After concentration, the obtained residue was purified by silica gel column chromatography (0 to 100% ethyl acetate/petroleum ether) to afford the title compound AG011 (1.3 g, 1.77 mmol, 83.4%) as a yellow solid.

LCMS (ESI): m/z 733 $[M+H]^+$.

Retention time: 1.319 min (analysis condition PH-FA05-2).

Example 8-9-2: Synthesis of 4-[4-[[2-(3-ethoxyphenyl)phenyl]methyl]piperazin-1-y]-N-[4-(2-phenylsulfanylethylamino)-3-(trifluoromethyl)phenyl]sulfonylbenzamide [AG012 (B2R045-01)]

[Formula 140]

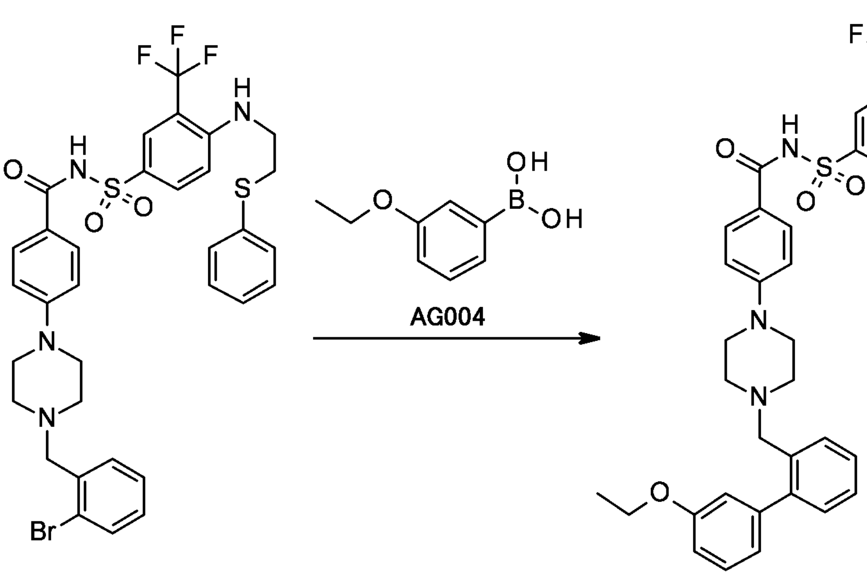


AG011

AG012

Under a nitrogen atmosphere, a solution of 4-[4-[(2-bromophenyl)methyl]piperazin-1-yl]-N-[4-(2-phenylsulfanylethylamino)-3-(trifluoromethyl)phenyl]sulfonylbenzamide (AG011) (165 mg, 0.225 mmol), (3-ethoxyphenyl)boronic acid (AG004, CAS: 90555-66-1) (74.7 mg, 0.450 mmol), $Pd(PPh_3)_2Cl_2$ (15.8 mg, 0.022 mmol), and sodium carbonate (31.0 mg, 0.292 mmol) in dimethoxyethane/ethanol/water = 1/1/1 (4 mL) was stirred at 80°C for 3 hours. The mixture was filtered, the filtrate was diluted with ethyl acetate, and water was added. The organic layer was extracted and dried over sodium sulfate. After concentration, the obtained residue was purified by reversed phase column chromatography (C18, 5 to 90% acetonitrile/water). The obtained crude product was purified by preparative HPLC (YMC-Actus Triart C18, 53 to 83% acetonitrile/10 mM aqueous ammonium bicarbonate solution) to afford the title compound AG012 (19.7 mg, 0.025 mmol, 11.3%) as a white solid.

$^1$H-NMR (400 MHz, DMSO-$d_6$) $\delta$ 11.84 (s, 1H), 7.94 (d, J = 2.0 Hz, 1H), 7.89-7.84 (m, 1H), 7.72 (d, J = 8.0 Hz, 2H), 7.57-7.50 (m, 1H), 7.45-7.25 (m, 7H), 7.26-7.17 (m, 2H), 7.05-6.81 (m, 6H), 6.60 (s, 1H), 4.04 (q, J = 8 Hz, 2H), 3.57-3.42 (m, 4H), 3.26 (t, J = 4 Hz, 4H), 3.22-3.12 (m, 2H), 2.42 (d, J = 5.7 Hz, 4H), 1.32 (t, J = 8 Hz, 3H).

LCMS (ESI): m/z 775 $[M+H]^+$.

AG012, whose synthetic method is illustrated in Example 8-9-2, may also be denoted as compound B2R045-01 in the present specification.

Example 8-10-1: 4-[4-[[2-(5-Ethoxypyridin-3-yl)phenyl]methyl]piperazin-1-yl]-N-[4-(2-phenylsulfanylethylamino)-3-(trifluoromethyl)phenyl]sulfonylbenzamide [AG013 (B2R046-01)]

[Formula 141]

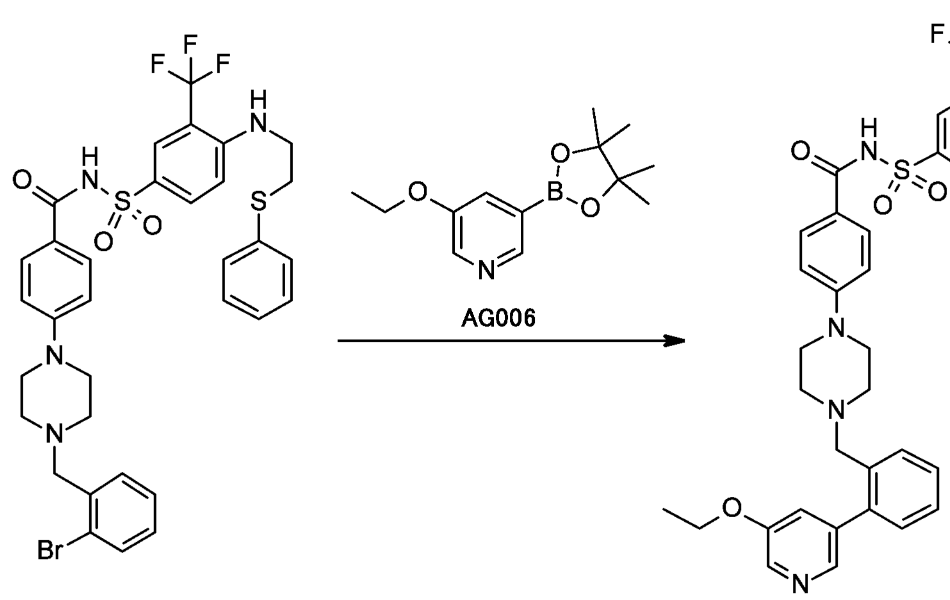


AG011

AG013

Under a nitrogen atmosphere, a solution of 4-[4-[(2-bromophenyl)methyl]piperazin-1-yl]-N-[4-(2-phenylsulfanylethylamino)-3-(trifluoromethyl)phenyl]sulfonylbenzamide (AG011) (250 mg, 0.341 mmol), 3-ethoxy-5-(4,4,5,5-tetramethyl-1,3,2-dioxaborolan-2-yl)pyridine (AG006, CAS: 1171892-40-2) (169.8 mg, 0.682 mmol), $Pd(PPh_3)_2Cl_2$ (23.9 mg, 0.034 mmol), and sodium carbonate (47.0 mg, 0.443 mmol) in dimethoxyethane/ethanol/water = 1/1/1 (5 mL) was stirred at 80°C for 16 hours. The mixture was filtered, the filtrate was diluted with ethyl acetate, and water was added. The organic layer was extracted and dried over sodium sulfate. After distilling off the solvent, the obtained residue was purified by reversed phase column chromatography (C18, 5 to 80% acetonitrile/water). The obtained crude product was purified by preparative HPLC (XBridge Prep OBD C18 column, 45 to 75% acetonitrile/10 mM aqueous ammonium bicarbonate solution) to afford the title compound AG013 (34.2 mg, 0.044 mmol, 12.8%) as a gray solid.

$^1$H-NMR (300 MHz, DMSO-$d_6$) $\delta$ 11.98 (s, 1H), 8.25 (d, J = 2.7 Hz, 1H), 8.16 (d, J = 1.8 Hz, 1H), 7.89 (d, J = 2.1 Hz, 1H), 7.80 (d, J = 9.3 Hz, 1H), 7.70 (d, J = 8.7 Hz, 2H), 7.55-7.47 (m, 2H), 7.48-7.23 (m, 7H), 7.22-7.15 (m, 1H), 6.83 (d, J = 8.6 Hz, 3H), 6.37 (s, 1H), 4.12 (q, J = 6.9 Hz, 2H), 3.40 (s, 4H), 3.17 (s, 4H), 3.13 (d, J = 8.4 Hz, 2H), 2.38 (s, 4H), 1.32 (t, J = 7.2 Hz, 3H).

LCMS (ESI): m/z 776 $[M+H]^+$.

AG013, whose synthetic method is illustrated in Example 8-10-1, may also be denoted as compound B2R046-01 in the present specification.

Example 8-11-1: Synthesis of tert-butyl 4-[6-(1-adamantylmethylcarbamoyl)pyridazin-3-yl]piperazine-1-carboxylate (AG034)

[Formula 142]

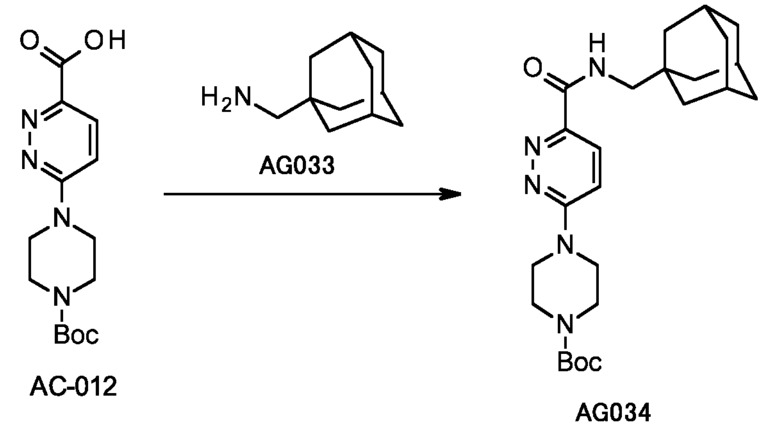

AG033

AC-012

AG034

To a solution of 6-[4-[(2-methylpropan-2-yl)oxycarbonyl]piperazin-1-yl]pyridazine-3-carboxylic acid (AC-012, CAS: 1690948-00-5) (200 mg, 0.649 mmol), 1-adamantylmethanamine (AG033, CAS: 17768-41-1) (160.8 mg, 0.973 mmol), EDCI (248.7 mg, 1.297 mmol), and DMAP (79.24 mg, 0.649 mmol) in dichloromethane (4 mL) was added DIPEA (335.3 mg, 2.596 mmol), and the mixture was stirred at room temperature for 16 hours. An aqueous ammonium chloride solution was added to the mixture. The organic layer was extracted three times with dichloromethane and dried over sodium sulfate. After concentration, the obtained residue was purified by silica gel column chromatography (0 to 100% ethyl acetate/petroleum ether, followed by 0 to 10% methanol/dichloromethane) to afford the title compound AG034 (180 mg, 60.9%) as a white solid.

LCMS (ESI): m/z 456 $[M+H]^+$.

Retention time: 1.238 min (analysis condition PH-TFA-13).

<u>Example 8-11-2: Synthesis of N-(1-adamantylmethyl)-6-piperazin-1-ylpyridazine-3-carboxamide (AG035)</u>

[Formula 143]

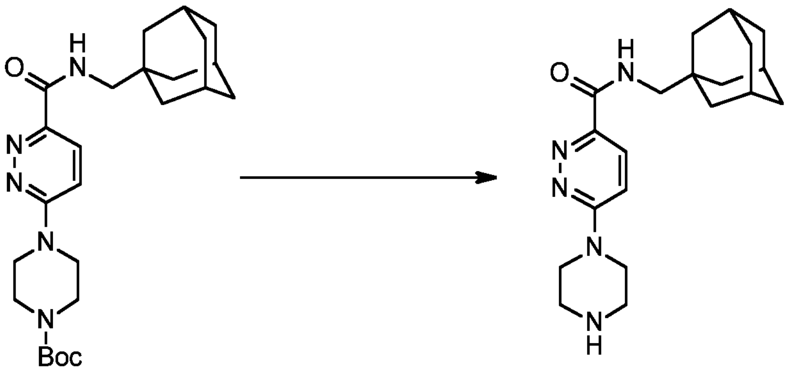

AG034

AG035

To a solution of tert-butyl 4-[6-(1-adamantylmethylcarbamoyl)pyridazin-3-yl]piperazine-1-carboxylate (AG034) (180 mg, 0.395 mmol) in dichloromethane (2 mL) was added a solution of HCl in 1,4-dioxane (4M, 5 mL), and the mixture was stirred at room temperature for 1 hour. The mixture was concentrated and then dissolved in water, and an aqueous sodium bicarbonate solution was added to achieve a pH of 8 to 9. The resulting solid was filtered, then washed three times with water, and dried at 50°C for 16 hours to afford the title compound AG035 (100 mg, 85%) as a gray solid.

LCMS (ESI): m/z 356 $[M+H]^+$.

Retention time: 0.917 min (analysis condition PH-TFA-13).

Example 8-11-3: Synthesis of 4-(3-ethoxyphenyl)-2-ethylbenzaldehyde (AG037)

[Formula 144]

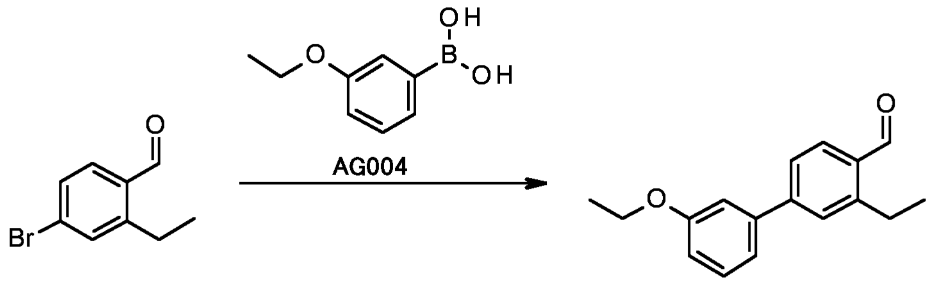


AG036

AG037

A solution of 4-bromo-2-ethylbenzaldehyde (AG036, CAS: 1114808-89-7) (1.00 g, 4.693 mmol), (3-ethoxyphenyl)boronic acid (AG004, CAS: 90555-66-1) (1.01 g, 6.085 mmol), $Pd(PPh_3)_2Cl_2$ (329.42 mg, 0.469 mmol), and sodium carbonate (646.65 mg, 6.101 mmol) in dimethoxyethane/ethanol/water = 1/1/1 (20 mL) was stirred at 80°C for 3 hours. An aqueous ammonium chloride solution was added to the mixture, and the organic layer was extracted three times with ethyl acetate and dried over sodium sulfate. After concentration, the obtained residue was purified by preparative TLC (20% ethyl acetate/petroleum ether) to afford the title compound AG037 (900 mg, 73.9%) as a pale yellow liquid.

LCMS (ESI): m/z 255 $[M+H]^+$.

Retention time: 1.184 min (analysis condition PH-FA-06).

Example 8-11-4: Synthesis of N-(1-adamantylmethyl)-6-[4-[[4-(3-ethoxyphenyl)-2-ethylphenyl]methyl]piperazin-1-yl]pyridazine-3-carboxamide [AG038 (B1A075-01)]

[Formula 145]

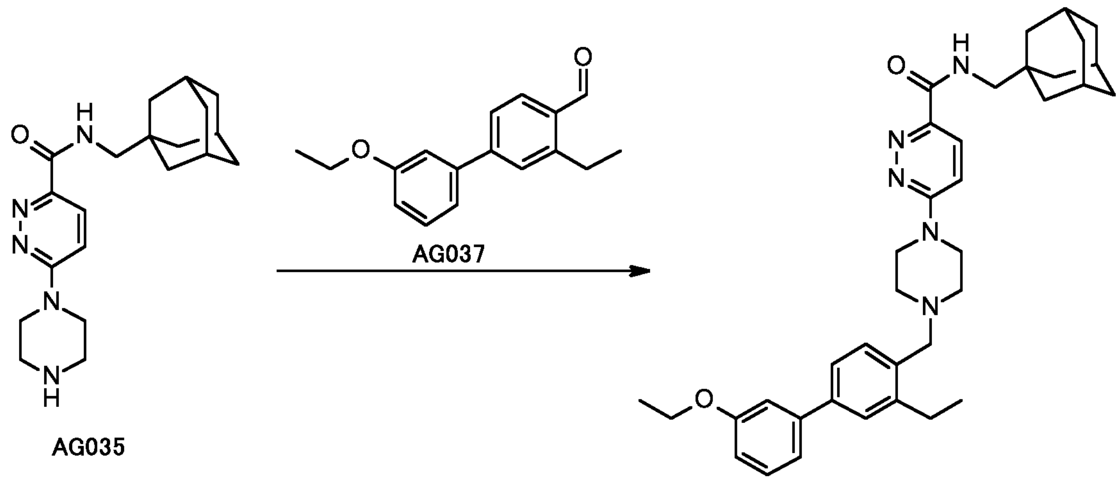


AG035

AG038

A solution of N-(1-adamantylmethyl)-6-piperazin-1-ylpyridazine-3-carboxamide (AG035) (150 mg, 0.422 mmol), 4-(3-ethoxyphenyl)-2-ethylbenzaldehyde (AG037) (128.8 mg, 0.506 mmol), and acetic acid (0.2 mL) in DMSO/ethanol (3 mL/3 mL) was stirred at room temperature for 1 hour. Sodium cyanoborohydride (53.03 mg, 0.844 mmol) was added, and the mixture was stirred at room temperature for 16 hours. After concentrating the mixture, the obtained residue was

purified by reversed phase column chromatography (C18, 0 to 80% acetonitrile/water) to afford the title compound AG038 (38.2 mg, 14.9%) as a white solid.

$^1$H-NMR (300 MHz, DMSO-$d_6$) δ 8.52 (t, J = 6.6 Hz, 1H), 7.83 (d, J = 9.6 Hz, 1H), 7.50-7.43 (m, 2H), 7.38-7.31 (m, 3H), 7.23-7.15 (m, 2H), 6.92-6.89 (m, 1H), 4.09 (q, J = 6.9 Hz, 2H), 3.72-3.67 (m, 4H), 3.55 (s, 2H), 3.32-3.28 (m, 1H), 3.02 (d, J = 6.6 Hz, 2H), 2.82-2.75 (m, 2H), 2.56-2.50 (m, 3H), 1.92 (s, 3H), 1.68-1.55 (m, 6H), 1.50-1.46 (m, 6H), 1.35 (t, J = 6.9 Hz, 3H), 1.24 (t, J = 7.8 Hz, 3H).

LCMS (ESI): m/z 594 $[M+H]^+$.

AG038, whose synthetic method is illustrated in Example 8-11-4, may also be denoted as compound B1A075-01 in the present specification.

Example 8-12-1: tert-Butyl 4-[6-[1-adamantylmethyl(methyl)carbamoyl]pyridazin-3-yl]piperazine-1 -carboxylate (AG040)

[Formula 146]

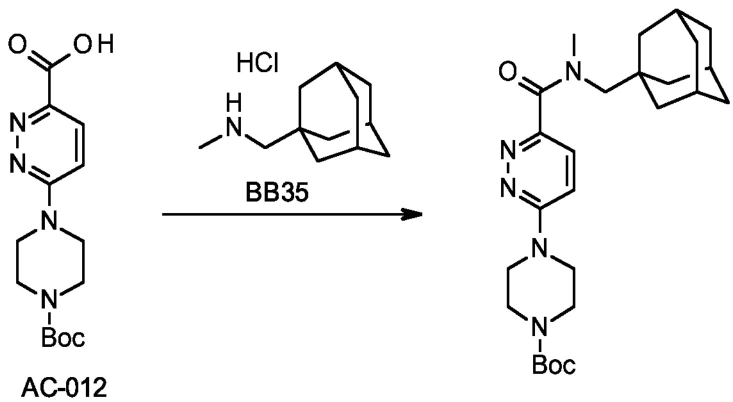


AC-012

AG040

To a solution of 6-[4-[(2-methylpropan-2-yl)oxycarbonyl]piperazin-1-yl]pyridazine-3-carboxylic acid (AC-012, CAS: 1690948-00-5) (150 mg, 0.486 mmol), 1-(1-adamantyl)-N-methylmethanamine hydrochloride (BB35, CAS: 1773-99-5) (174.5 mg, 0.973 mmol), EDCI (186.52 mg, 0.973 mmol), and DMAP (59.4 mg, 0.486 mmol) in dichloromethane (6 mL) was added DIPEA (251.5 mg, 1.946 mmol), and the mixture was stirred at room temperature for 16 hours. An aqueous ammonium chloride solution was added to the mixture. The organic layer was extracted three times with dichloromethane and dried over sodium sulfate. After concentration, the obtained residue was purified by silica gel column chromatography (0 to 100% ethyl acetate/petroleum ether, followed by 0 to 10% methanol/dichloromethane) to afford the title compound AG040 (150 mg, 63%) as a white solid.

LCMS (ESI): m/z 470 $[M+H]^+$.

Retention time: 1.155 min (analysis condition PH-TFA-13).

Example 8-12-2: Synthesis of N-(1-adamantylmethyl)-N-methyl-6-piperazin-1-ylpyridazine-3-carboxamide (AG041)

[Formula 147]

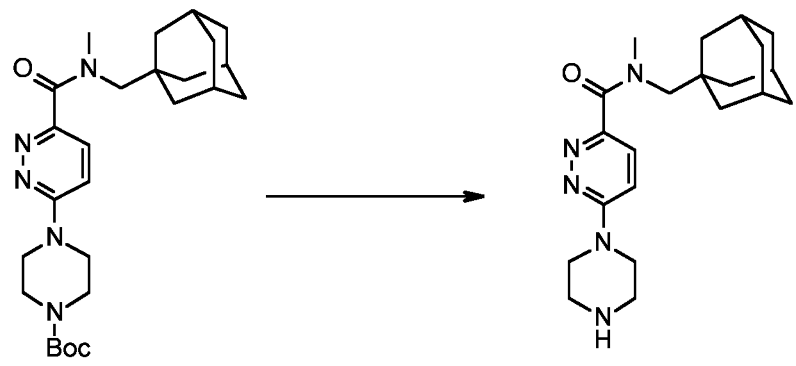

AG040 AG041

To a solution of tert-butyl 4-[6-[1-adamantylmethyl(methyl)carbamoyl]pyridazin-3-yl]piperazine-1-carboxylate (AG040) (150 mg, 0.319 mmol) in dichloromethane (3 mL) was added a solution of HCl in 1,4-dioxane (4M, 6 mL), and the mixture was stirred at room temperature for 1 hour. The mixture was concentrated and then dissolved in water, and an aqueous sodium bicarbonate solution was added to achieve a pH of 8 to 9. The resulting solid was filtered, then washed three times with water, and dried at 50°C for 16 hours to afford the title compound AG041 (100 mg) as a yellow solid.

LCMS (ESI): m/z 370 $[M+H]^+$.

Retention time: 0.882 min (analysis condition PH-TFA-13).

Example 8-12-3: Synthesis of N-(1-adamantylmethyl)-6-[4-[[4-(3-ethoxyphenyl)-3-methylphenyl]methyl]piperazin-1-yl]-N-methylpyridazine-3-carboxamide [AG042 (B3A073-01)]

[Formula 148]

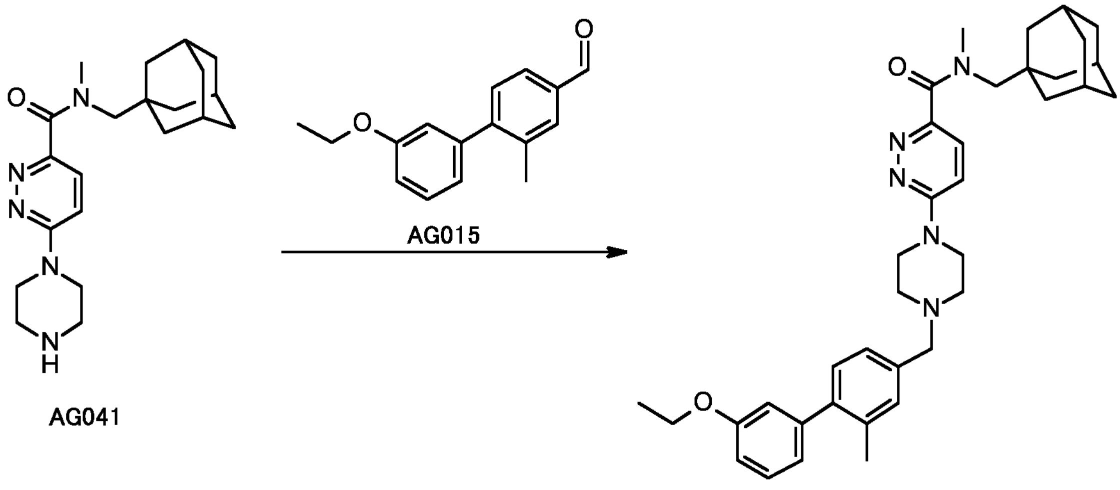


AG041

AG042

A solution ofN-(1-adamantylmethyl)-N-methyl-6-piperazin-1-ylpyridazine-3-carboxamide (AG041) (180 mg, 0.487 mmol), 4-(3-ethoxyphenyl)-3-methylbenzaldehyde (AG015, CAS: 1506205-91-9) (175.6 mg, 0.731 mmol), and acetic acid (0.6 mL) in DMSO/ethanol (3 mL/3 mL) was stirred at room temperature for 1 hour. Sodium cyanoborohydride (61.2 mg, 0.974 mmol) was added, and the mixture was stirred at room temperature for 16 hours. After concentrating the mixture, the obtained residue was purified by reversed phase column chromatography (C18, 0 to 80% acetonitrile/water) to afford the title compound AG042 (70 mg, 23.7%) as a white solid.

$^1$H-NMR (300 MHz, DMSO-$d_6$) δ 7.56-7.51 (m, 1H), 7.35-7.29 (m, 2H), 7.25-7.23 (m, 1H), 7.21-7.16 (m, 2H), 6.92-6.83 (m, 3H), 4.05 (q, J = 6.9 Hz, 2H), 3.70-3.65 (m, 4H), 3.53 (s, 3H), 3.33 (s, 2H), 3.11-3.06 (m, 3H), 2.53-2.50 (m, 2H), 2.31 (s, 3H), 1.97-1.91 (m, 2H), 1.84 (s, 1H), 1.70-1.45 (m, 11H), 1.33 (t, J = 6.9 Hz, 3H), 1.27-1.23 (m, 2H).

LCMS (ESI): m/z 594 $[M+H]^+$.

AG042, whose synthetic method is illustrated in Example 8-12-3, may also be denoted as compound B3A073-01 in the present specification.

Example 8-13-1: Synthesis of 4-(3-ethoxyphenyl)-2-methoxybenzaldehyde (AG044)

[Formula 149]

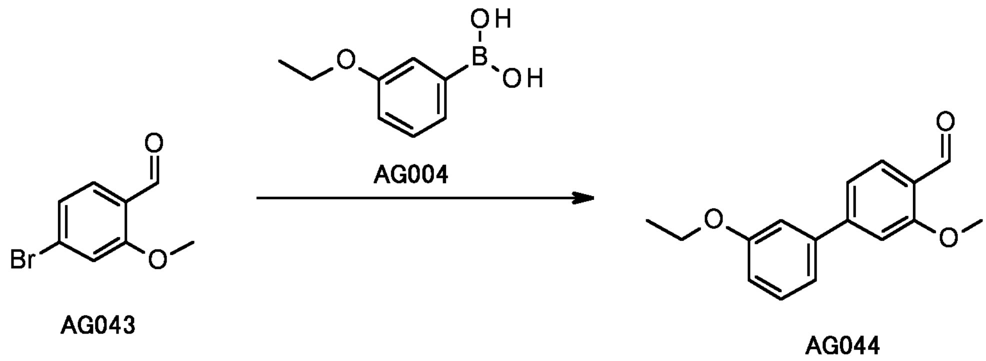

Under a nitrogen atmosphere, a solution of 4-bromo-2-methoxybenzaldehyde (AG043, CAS: 43192-33-2) (200 mg, 0.930 mmol), (3-ethoxyphenyl)boronic acid (AG004, CAS: 90555-66-1) (308.7 mg, 1.860 mmol), $Pd(PPh_3)_2Cl_2$ (65.28 mg, 0.093 mmol), and sodium carbonate (128.14 mg, 1.209 mmol) in dimethoxyethane/ethanol/water = 1/1/1 (6 mL) was stirred at 80°C for 3 hours. The mixture was filtered and the filtrate was concentrated. The obtained residue was purified by silica gel column chromatography (0 to 20% ethyl acetate/petroleum ether) to afford the title compound AG044 (214.0 mg, 54.0%) as a yellow liquid.

LCMS (ESI): m/z 257 $[M+H]^+$.

Retention time: 0.839 min (analysis condition PH-FA-05).

Example 8-13-2: Synthesis of 4-[4-[[4-(3-ethoxyphenyl)-2-methoxyphenyl]methyl]piperazin-1-yl]-N-[3-nitro-4-(2-phenylsulfanylethylamino)phenyl]sulfonylbenzamide [AG045 (B2Q053-01)]

[Formula 150]

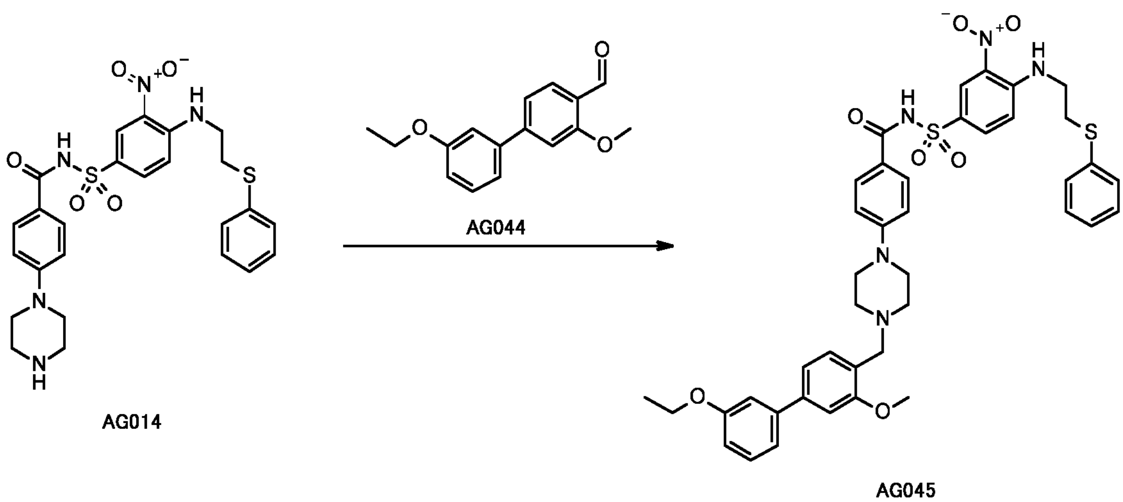

A solution of N-[3-nitro-4-(2-phenylsulfanylethylamino)phenyl]sulfonyl-4-piperazin-1-ylbenzamide (AG014, CAS: 406233-75-8) (100.00 mg, 0.185 mmol), 4-(3-ethoxyphenyl)-2-methoxybenzaldehyde (AG044) (94.64 mg, 0.369 mmol), and acetic acid (0.05 mL) in DMSO/ethanol (2 mL/2 mL) was stirred at room temperature for 1 hour. Sodium

cyanoborohydride (23.2 mg, 0.369 mmol) was added, and the mixture was stirred at room temperature for 16 hours. After concentrating the mixture, the obtained residue was purified by silica gel column chromatography (0 to 95% ethyl acetate/petroleum ether). The obtained crude product was purified by reversed phase column chromatography (C18, 10 to 70% acetonitrile/water) to afford the title compound AG045 (37 mg, 25.4%) as a yellow solid.

$^1$H-NMR (300 MHz, DMSO-$d_6$) δ 8.68 (s, 1H), 8.55 (d, J = 2.1 Hz, 1H), 7.89 (d, J = 9.6 Hz, 1H), 7.76 (d, J = 8.7 Hz, 2H), 7.49-7.33 (m, 4H), 7.33-7.14 (m, 7H), 7.11 (d, J = 9.3 Hz, 1H), 6.93-6.87 (m, 3H), 4.11 (q, J = 6.9 Hz, 2H), 3.91 (s, 3H), 3.81-3.78 (m, 2H), 3.77-3.68 (m, 2H), 3.68-3.58 (m, 2H), 3.30-3.25 (m, 4H), 2.78-2.69 (m, 4H), 1.36 (t, J = 6.9 Hz, 3H).

LCMS (ESI): m/z 782 [M+H]$^+$.

AG045, whose synthetic method is illustrated in Example 8-13-3, may also be denoted as compound B2Q053-01 in the present specification.

Example 8-14-1: Synthesis of methyl 3-[2-(5-ethoxypyridin-3-yl)phenyl]propanoate (AG047)

[Formula 151]

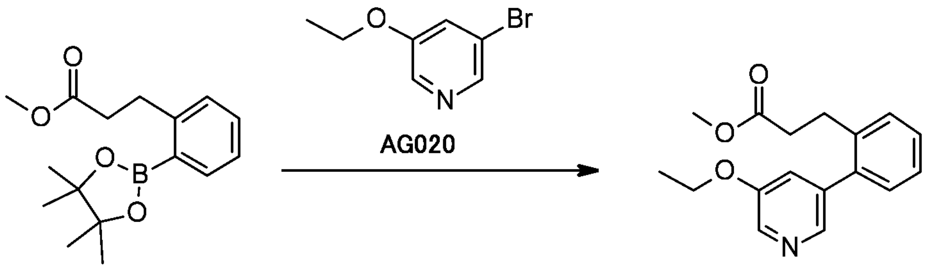


AG046 AG047

To a solution of methyl 3-[2-(4,4,5,5-tetramethyl-1,3,2-dioxaborolan-2-yl)phenyl]propanoate (AG046, CAS: 1282660-71-2) (250 mg, 0.862 mmol) in 1,4-dioxane/water (6 mL/0.6 mL) were added 3-bromo-5-ethoxypyridine (AG020, CAS: 17117-17-8) (191.5 mg, 0.948 mmol), potassium acetate (169.1 mg, 1.724 mmol), and Pd(dppf)$Cl_2$ (63.0 mg, 0.086 mmol), and the mixture was stirred at 90°C for 3 hours. After filtering the mixture and concentrating the filtrate, the obtained residue was purified by silica gel column chromatography (0 to 20% ethyl acetate/petroleum ether) to afford the title compound AG047 (200 mg, 77.2%) as a white solid.

LCMS (ESI): m/z 286 [M+H]$^+$.

Retention time: 0.877 min (analysis condition PH-FA-02).

Example 8-14-2: Synthesis of 3-[2-(5-ethoxypyridin-3-yl)phenyl]propanoic acid (AG048)

[Formula 152]

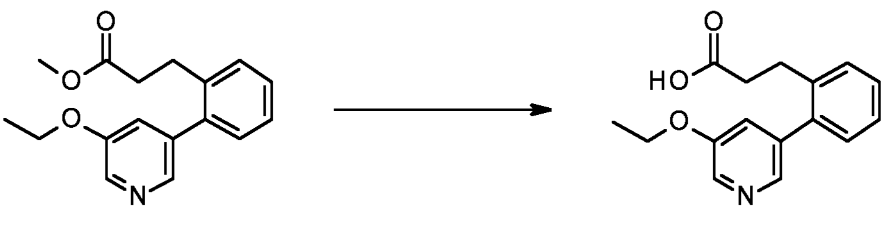

AG047 AG048

To a solution of methyl 3-[2-(5-ethoxypyridin-3-yl)phenyl]propanoate (AG047) (330 mg, 1.157 mmol) in methanol/water (30 mL/3 mL) was added lithium hydroxide (276.9 mg, 11.570 mmol), and the mixture was stirred at 35°C for 16 hours. The mixture was concentrated and then dissolved in water, and citric acid was added to achieve a pH of

5 to 6. The resulting solid was filtered, then washed three times with water, and dried at 50°C for 16 hours to afford the title compound AG048 (200 mg) as a gray solid.

LCMS (ESI): m/z 272 $[M+H]^+$.

Retention time: 0.765 min (analysis condition PH-FA-07).

Example 8-14-3: Synthesis of tert-butyl 4-[6-[[3-nitro-4-(2-phenylsulfanylethylamino)phenyl]sulfonylcarbamoyl]pyridazin-3-yl]piperazine-1-carboxylate (AG049)

[Formula 153]

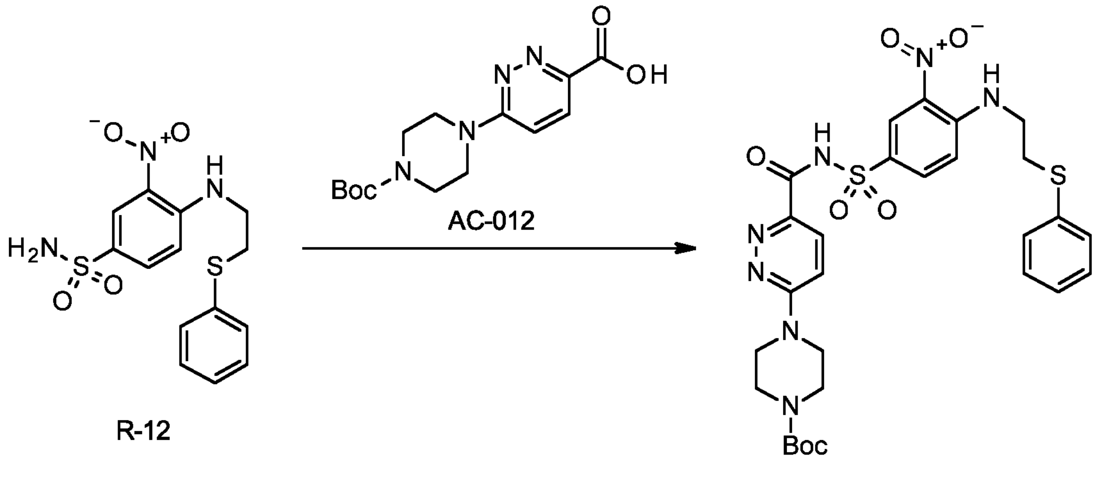


R-12

AG049

To a solution of 6-[4-[(2-methylpropan-2-yl)oxycarbonyl]piperazin-1-yl]pyridazine-3-carboxylic acid (AC-012, CAS: 1690948-00-5) (200 mg, 0.649 mmol), 3-nitro-4-(2-phenylsulfanylethylamino)benzenesulfonamide (R-12, CAS: 406233-13-4) (344.0 mg, 0.974 mmol), EDCI (249.3 mg, 1.298 mmol), and DMAP (79.2 mg, 0.64 mmol) in dichloromethane (4 mL) was added DIPEA (334.9 mg, 2.596 mmol), and the mixture was stirred at room temperature for 16 hours. An aqueous ammonium chloride solution was added to the mixture. The organic layer was extracted three times with dichloromethane and dried over sodium sulfate. After concentration, the obtained residue was purified by silica gel column chromatography (0 to 100% ethyl acetate/petroleum ether, followed by 0 to 10% methanol/dichloromethane) to afford the title compound AG049 (240 mg, 57.6%) as a white solid.

LCMS (ESI): m/z 644 $[M+H]^+$.

Retention time: 1.211 min (analysis condition PH-TFA-13).

Example 8-14-4: Synthesis of N-[3-nitro-4-(2-phenylsulfanylethylamino)phenyl] sulfonyl-6-piperazin-1 -ylpyridazine-3 -carboxamide (AG050)

[Formula 154]

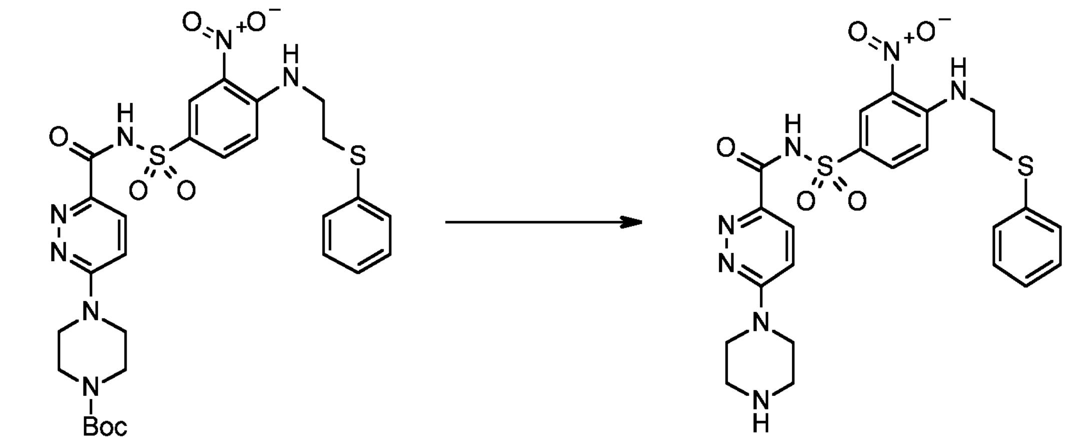

AG049 AG050

To a solution of tert-butyl 4-[6-[[3-nitro-4-(2-phenylsulfanylethylamino)phenyl]sulfonylcarbamoyl]pyridazin-3-yl]piperazine-1-carboxylate (AG049) (50.0 mg, 0.078 mmol) in dichloromethane (2 mL) was added a solution of HCl in 1,4-dioxane (4M, 4 mL), and the mixture was stirred at room temperature for 1 hour. The mixture was concentrated and then dissolved in water, and an aqueous sodium bicarbonate solution was added to achieve a pH of 8 to 9. The resulting solid was filtered, then washed three times with water, and dried at 50°C for 16 hours to afford the title compound AG050 (50 mg) as a gray solid.

LCMS (ESI): m/z 544 $[M+H]^+$.

Retention time: 0.960 min (analysis condition PH-FA-02).

Example 8-14-5: Synthesis of 6-[4-[3-[2-(5-ethoxypyridin-3-yl)phenyl]propanoyl]piperazin-1-yl]-N-[3-nitro-4-(2-phenylsulfanylethylamino)phenyl]sulfonylpyridazine-3-carboxamide [AG051 (B2Q 012-01)1

[Formula 155]

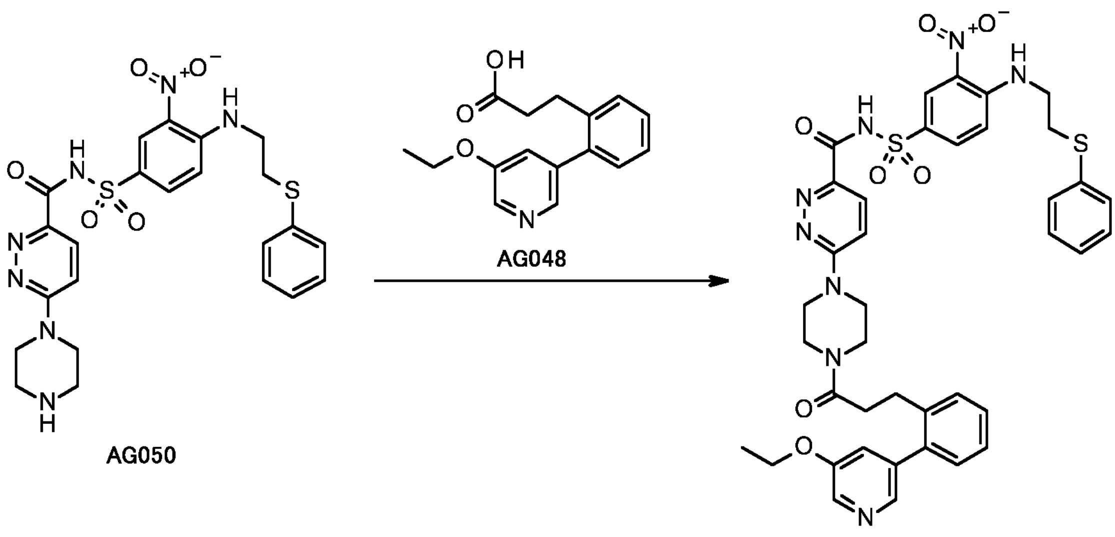

AG050 AG048 AG051

To a solution of N-[3-nitro-4-(2-phenylsulfanylethylamino)phenyl]sulfonyl-6-piperazin-1-ylpyridazine-3-carboxamide (AG050) (100 mg, 0.184 mmol), 3-[2-(5-ethoxypyridin-3-yl)phenyl]propanoic acid (AG048) (74.9 mg, 0.276 mmol), EDCI (70.5 mg, 0.368 mmol), and DMAP (22.5 mg, 0.184 mmol) in dichloromethane (2 mL) was added DIPEA (95.1 mg, 0.736 mmol), and the mixture was stirred at room temperature for 16 hours. An aqueous ammonium chloride solution

was added to the mixture. The organic layer was extracted three times with dichloromethane and dried over sodium sulfate. After concentration, the obtained residue was purified by silica gel column chromatography (0 to 100% ethyl acetate/petroleum ether, followed by 0 to 10% methanol/dichloromethane) to afford the title compound AG051 (40 mg, 26.7%) as a white solid.

$^1$H-NMR (300 MHz, DMSO-$d_6$) δ 8.54-8.48 (m, 1H), 8.29-8.24 (m, 1H), 8.13-8.07 (m, 1H), 7.89-7.76 (m, 1H), 7.45-7.26 (m, 7H), 7.22-7.12 (m, 2H), 7.03-7.92 (m, 3H), 4.18-4.08 (m, 2H), 3.63-3.51 (m, 5H), 3.45-3.38 (m, 2H), 3.28-3.22 (m, 2H), 2.81-2.71 (m, 2H), 2.59-2.50 (m, 6H), 1.35-1.25 (m, 3H).

LCMS (ESI): m/z 797 $[M+H]^+$.

AG051, whose synthetic method is illustrated in Example 8-14-5, may also be denoted as compound B2Q012-01 in the present specification.

Example 8-15-1: Synthesis of 6-[4-[[4-(5-ethoxypyridin-3-yl)-3-methylphenyl]methyl]piperazin-1-yl]-N-[4-(2-phenylsulfanylethylamino)-3-(trifluoromethyl)phenyl1sulfonylpyridazine-3-carboxamide [AG052 (B2R074-01)]

[Formula 156]

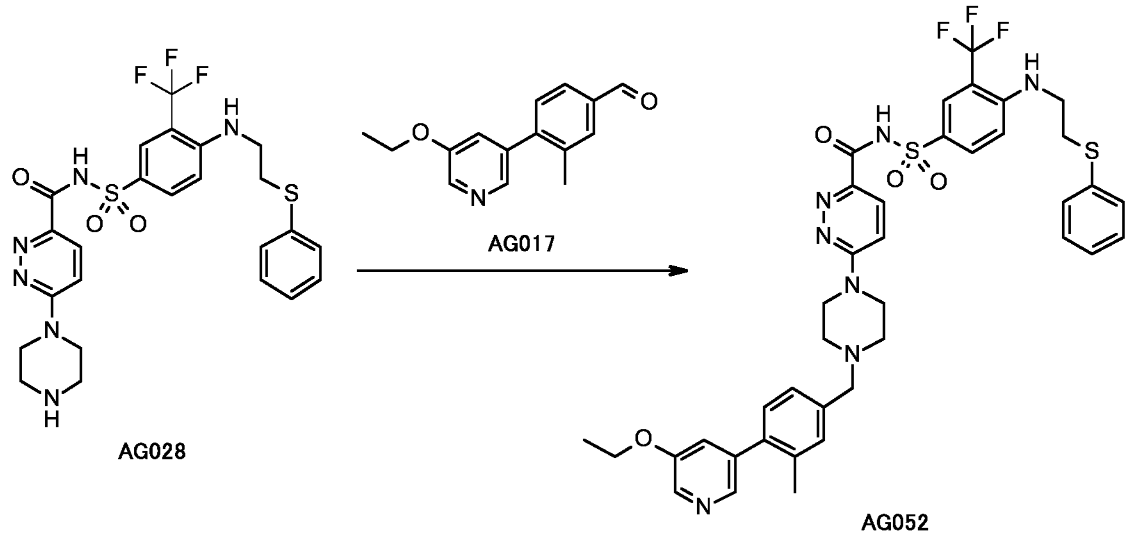


AG028

AG052

A solution of N-[4-(2-phenylsulfanylethylamino)-3-(trifluoromethyl)phenyl]sulfonyl-6-piperazin-1-ylpyridazine-3-carboxamide (AG028) (310.00 mg, 0.547 mmol), 4-(5-ethoxypyridin-3-yl)-3-methylbenzaldehyde (AG017, CAS: 1545014-81-0) (264.02 mg, 1.094 mmol), and acetic acid (0.05 mL) in DMSO/ethanol (3 mL/3 mL) was stirred at room temperature for 1 hour. Sodium cyanoborohydride (68.76 mg, 1.094 mmol) was added, and the mixture was stirred at room temperature for 16 hours. The mixture was purified by reversed phase column chromatography (C18, 5 to 70% acetonitrile/water). The obtained crude product was purified by preparative HPLC (XBridge Prep OBD C18 column, 34 to 64% acetonitrile/10 mM aqueous ammonium bicarbonate solution) to afford the title compound AG052 (37.7 mg, 8.6%) as a gray solid.

$^1$H-NMR (300 MHz, DMSO-$d_6$) δ 8.26 (d, J = 2.7 Hz, 1H), 8.12 (d, J = 1.8 Hz, 1H), 7.99-7.96 (m, 1H), 7.90-7.85 (m, 1H), 7.80-7.49 (m, 1H), 7.41-7.36 (m, 2H), 7.35-7.26 (m, 5H), 7.25-7.17 (m, 3H), 6.87 (d, J = 9.0 Hz, 1H), 6.55-6.49 (m, 1H), 4.15 (q, J = 6.9 Hz, 2H), 3.78-3.71 (m, 4H), 3.58 (s, 2H), 3.51-3.43 (m, 3H), 3.19-3.12 (m, 2H), 2.59-2.53 (m, 4H), 2.13 (s, 3H), 1.35 (t, J = 6.9 Hz, 3H).

LCMS (ESI): m/z 792 $[M+H]^+$.

AG052, whose synthetic method is illustrated in Example 8-15-1, may also be denoted as compound B2R074-01 in the present specification.

Example 8-16-1: Synthesis of solid phase-supported ArBr (compound D043-02R)

[Formula 157]

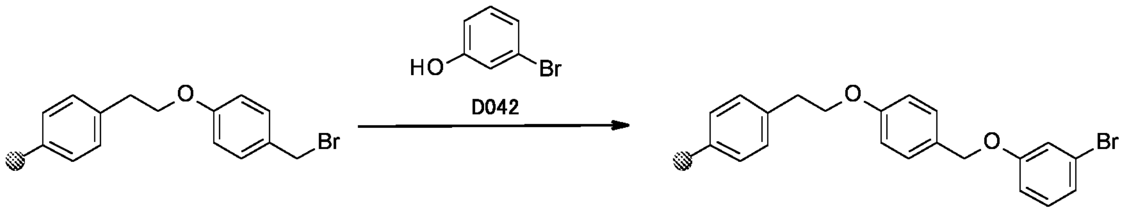


D041-02R D043-02R

Under a nitrogen atmosphere, to a 150 mL glass vial were added a modified Wang resin (compound D041-02R, 53447, Sigma-Aldrich, brominated, 100 to 200 mesh, loading rate: 1.25 mmol/g, copolymer (styrene-1% DVB), 5.00 g, 6.25 mmol) and NMP (50 mL), and the mixture was shaken at room temperature for 1 hour. Under a nitrogen atmosphere, 3-bromophenol (compound D042, CAS: 591-20-8, 2.16 g, 12.5 mmol) and tBuOK (1.33 g, 11.9 mmol) were added, and the mixture was shaken at room temperature for 20 hours. The reaction solution was transferred to a syringe equipped with a filter using NMP (100 mL), and the resin was washed twice with NMP (100 mL). The resin was further washed repeatedly three times with NMP/water (1/1, 100 mL), three times with water (100 mL), three times with NMP (100 mL), three times with methanol (100 mL), three times with DCM (100 mL), and three times with heptane (100 mL), and the obtained resin was dried under reduced pressure overnight to afford the title compound D043-02R (4.95 g).

Example 8-16-2: The loading rate of the solid phase-supported ArBr (compound D043-02R) was determined by the following method.

[Formula 158]

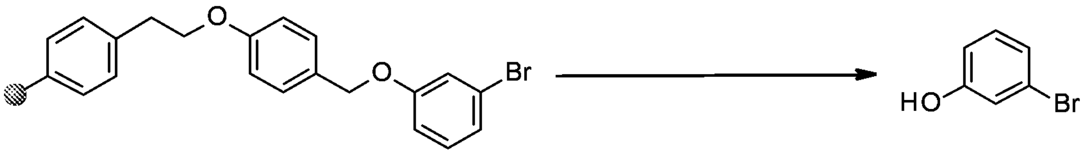

D043-02R D042

Under a nitrogen atmosphere, in a 3 mL glass vial, compound D043-02R (22.6 mg) was immersed in 30% TFA/DCM (0.08M, 1 mL) containing trimethylbenzene for 15 minutes. The cleavage solution was filtered and the resin was washed with NMP (500 μL). Based on the UV area of the obtained 3-bromophenol (D042), the concentration of 3-bromophenol (D042) was quantified using a calibration curve to determine the loading rate of 3-bromophenol (D042) on the resin. As a result, the loading rate of D043-02R was calculated to be 0.532 mmol/g.

Compound D042

Retention time: 0.893 min (analysis condition SMD-FA05-1, 290 nm).

Maximum absorption wavelength: 276 nm.

Example 8-16-3: Synthesis of solid phase-supported ArBr (compound D045-02R)

[Formula 159]

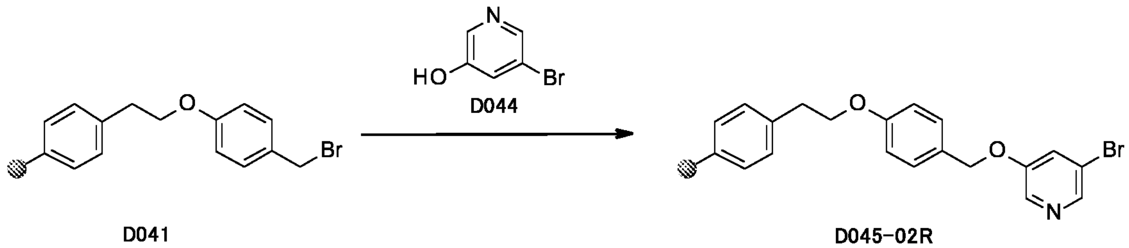


D041 D045-02R

Using 5-bromopyridin-3-ol (compound D044, CAS: 74115-13-2, 2.18 g, 12.5 mmol), a solid phase-supported ArBr (compound D045-02R) (5.01 g) was synthesized by the same method as for compound D043-02R in Example 8-12-1 and obtained.

Example 8-16-4: The loading rate of the solid phase-supported ArBr (compound D045-02R) was determined by the following method.

[Formula 160]

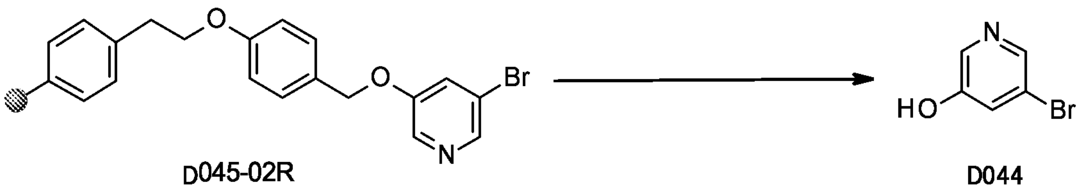

D045-02R D044

Using compound D045-02R (22.6 mg), the loading rate of D044 was calculated by the same method as in Example 8-12-2 (compound D042). As a result, the loading rate of D045-02R was calculated to be 0.735 mmol/g.

Compound D044

LCMS: m/z 174 $[M+H]^+$.

Retention time: 0.573 min (analysis condition SMD-FA05-1, 290 nm).

Example 8-16-5: Synthesis of solid phase-supported ketone (compound D047-02R)

[Formula 161]

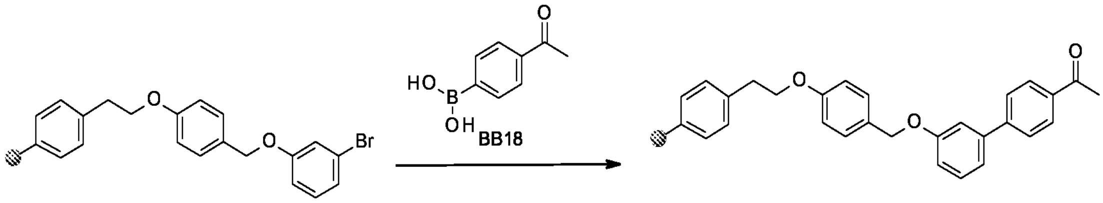


D043-02R D047-02R

Under a nitrogen atmosphere, to a 4 mL glass vial were added a solid phase-supported ArBr (compound D043-02R) (100 mg, loading rate 0.532 mmol/g, 0.0532 mmol) and NMP (1.9 mL), and the mixture was shaken at room temperature for 1 hour. Under a nitrogen atmosphere, (4-acetylphenyl)boronic acid (BB18, CAS: 149104-90-5) (52.3 mg, 0.319 mmol), an aqueous $K_3PO_4$ solution (1.7M, 188 μL, 0.319 mmol), and a solution of $P(Cy)_3Pd$ G3 in NMP

(0.04M, 266 μL, 10.6 μmol) were added, and the mixture was shaken at 90°C for 2 hours. The suspension of the reaction solution and the resins was transferred to a syringe equipped with a filter using NMP (2 mL), washed repeatedly twice with NMP (2 mL), three times with a solution (2 mL) of NMP containing NAC (0.05M):$H_2O$ (1:1), three times with water (2 mL), three times with NMP (2 mL), three times with MeOH (2 mL), three times with DCM (2 mL), and three times with heptane (2 mL), and the resin was then dried under reduced pressure to obtain the title compound D047-02R (107.6 mg). Part of the resin, compound D047-02R, was transferred onto a micropipette tip equipped with a filter and immersed in a 10% TFA/DCM solution (50 μL) containing 0.08M pentamethylbenzene for 5 minutes. The cleavage solution was filtered and the solid phase was washed with NMP (50 μL). The obtained filtrate was diluted with MeCN (500 μL) to prepare a LC sample, and by carrying out LC-MS analysis, production of D047 was confirmed.

[Formula 162]

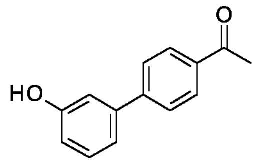

D047

Compound D047

LCMS: m/z 213 $[M+H]^+$.

Retention time: 0.927 min (analysis condition SMD-FA05-1, 290 nm).

Example 8-16-6: Synthesis of solid phase-supported ketone (compound D048-02R)

[Formula 163]

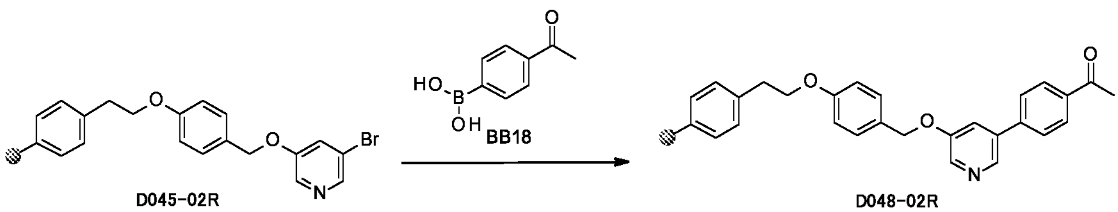


Using a solid phase-supported ArBr (compound D045-02R, loading rate:
0.735 mmol/g, 100 mg), the title compound D048-02R (108.3 mg) was synthesized by the same method as in Example 8-16-5 (compound D047-02R) and obtained. The same cleavage operation as in Example 8-16-5 was carried out and production of D048 was confirmed.

[Formula 164]

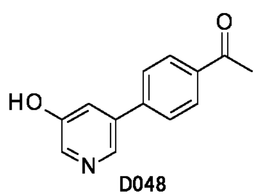

D048

Compound D048

LCMS: m/z 214 $[M+H]^+$.

Retention time: 0.534 min (analysis condition SMD-FA05-1, 290 nm).

Example 8-16-7: Synthesis of solid phase-supported allyl ester (compound D050-02R)

[Formula 165]

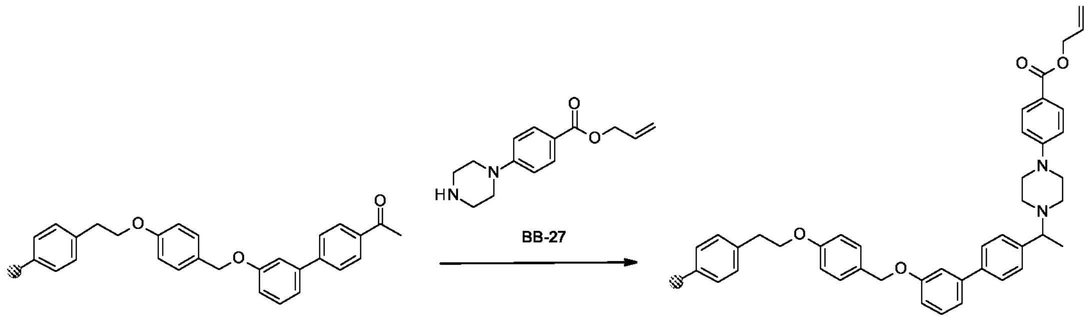


D047-02R

D050-02R

Under a nitrogen atmosphere, to a 3 mL glass vial were added a solid phase-supported ketone (compound D047-02R, 100 mg, loading rate 0.532 mmol/g, 0.0532 mmol) and anisole (3.75 mL), and the mixture was shaken at room temperature for 1 hour. Under a nitrogen atmosphere, allyl 4-piperazin-1-ylbenzoate (BB-27, 157 mg, 0.638 mmol) and $Ti(OtBu)_4$ (0.411 mL, 1.06 mmol) were added, and the mixture was shaken at 140°C for 19 hours. After cooling the reaction solution to room temperature, sodium triacetoxyborohydride (338 mg, 1.60 mmol) was added under a nitrogen atmosphere, and the mixture was shaken at 140°C for 17 hours. The reaction container was cooled to room temperature and MeOH (2 mL) was added to the reaction solution. The obtained suspension of reaction solution and resin was transferred to a syringe equipped with a filter using MeOH (2 mL), washed repeatedly three times with NMP (2 mL), three times with a 0.05M Rochelle salt solution (NMP:water = 1:1, 2 mL), three times with water (2 mL), three times with NMP (2 mL), three times with a semisaturated $NaHCO_3$ solution (2 mL), three times with water (2 mL), three times with NMP (2 mL), three times with MeOH (2 mL), three times with DCM (2 mL), and three times with heptane (2 mL), and the resin was then dried under reduced pressure to obtain the title compound D050-02R (103.2 mg). The same cleavage operation as in Example 8-16-5 was carried out and production of D050 was confirmed.

[Formula 166]

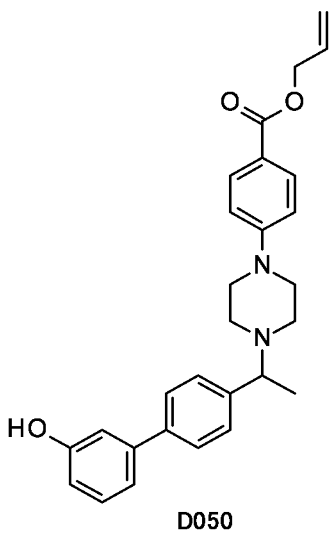

D050

Compound D050

LCMS: m/z 443 $[M+H]^+$.

Retention time: 0.797 min (analysis condition SMD-FA05-1, 290 nm).

Example 8-16-8: Synthesis of solid phase-supported allyl ester (compound D051-02R)

[Formula 167]

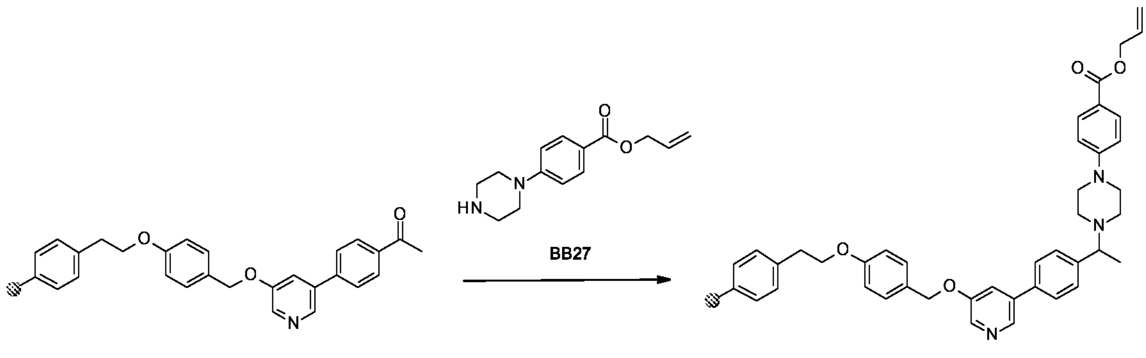


D048-02R

D051-02R

Using a solid phase-supported ketone (compound D048-02R, loading rate: 0.735 mmol/g, 100 mg), the title compound D051-02R (105.8 mg) was synthesized by the same method as in Example 8-16-7 (compound D050-02R) and obtained. The same cleavage operation as in Example 8-16-5 was carried out and production of D051 was confirmed.

[Formula 168]

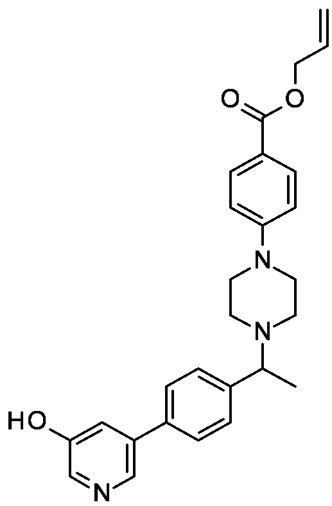

D051

Compound D051

LCMS: m/z 444 $[M+H]^+$.

Retention time: 0.610 min (analysis condition SMD-FA05-1, 290 nm).

Example 8-16-9: Synthesis of solid phase-supported carboxylic acid (compound D052-02R)

[Formula 169]

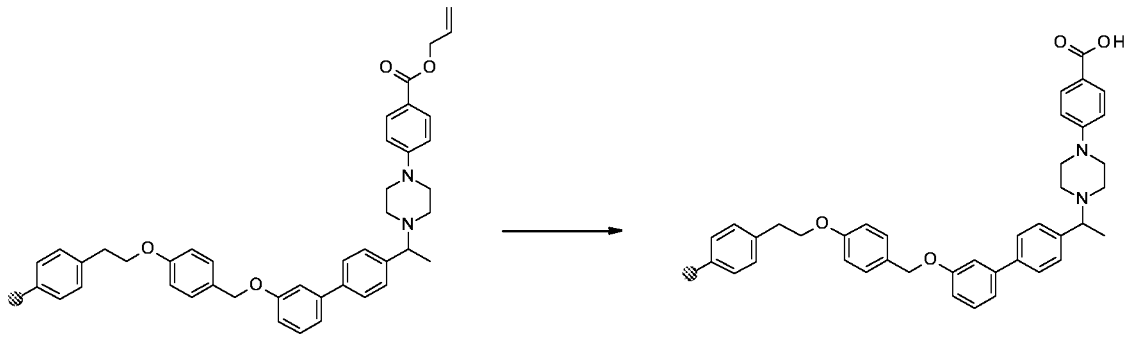

D050-02R D052-02R

Under a nitrogen atmosphere, to a 3 mL glass vial were added a solid phase-supported allyl ester (compound D050-02R, 99.4 mg, loading rate 0.532 mmol/g, 0.0532 mmol), NMP (1.2 mL), and MeOH (0.30 mL), and the mixture was shaken at room temperature for 1 hour. An aqueous sodium hydroxide solution (2M, 250 μL, 0.660 mmol) was added and the mixture was shaken at 80°C for 7 hours. The obtained suspension of reaction solution and resin was transferred to a syringe equipped with a filter using NMP (2 mL), washed repeatedly three times with NMP (2 mL), three times with water (2 mL), three times with NMP (2 mL), three times with a NMP solution containing HOAt (0.2M, 2 mL), three times with NMP (2 mL), three times with MeOH (2 mL), three times with DCM (2 mL), and three times with heptane (2 mL), and the resin was then dried under reduced pressure to obtain the title compound D052-02R (82.6 mg). The same cleavage operation as in Example 8-16-5 was carried out and production of D052 was confirmed.

[Formula 170]

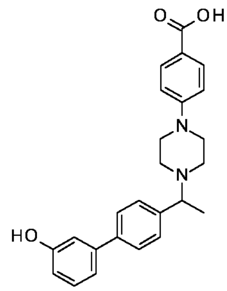

D052

Compound D052

LCMS: m/z 403 $[M+H]^+$.

Retention time: 0.649 min (analysis condition SMD-FA05-1, 290 nm).

Example 8-16-10: Synthesis of solid phase-supported carboxylic acid (compound D053-02R)

[Formula 171]

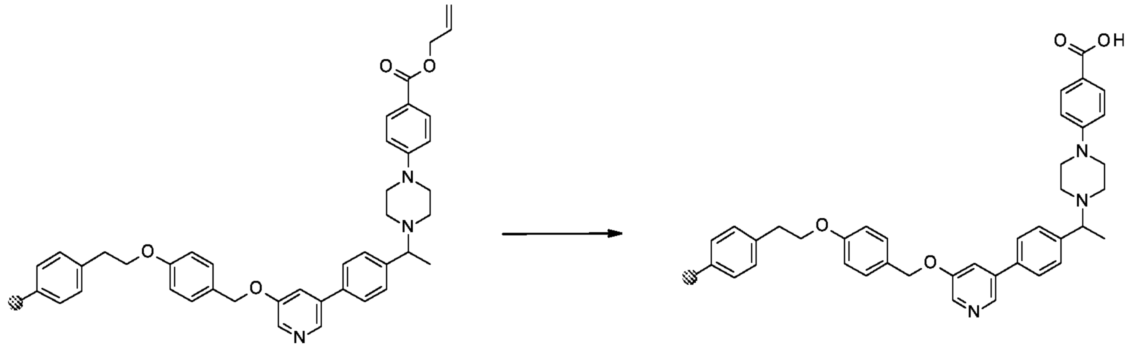

D051-02R D053-02R

Under a nitrogen atmosphere, to a 3 mL glass vial were added a solid phase-supported allyl ester (compound D051-02R, 100.2 mg, loading rate 0.736 mmol/g, 0.0737 mmol), NMP (1.2 mL), and MeOH (0.30 mL), and the mixture was shaken at room temperature for 1 hour. An aqueous sodium hydroxide solution (2M, 250 μL, 0.660 mmol) was added and the mixture was shaken at room temperature for 7 hours. Under a nitrogen atmosphere, to the reaction solution was added an aqueous sodium hydroxide solution (2M, 250 μL, 0.660 mmol), and the mixture was shaken at room temperature for 11 hours. The obtained suspension of reaction solution and resin was transferred to a syringe equipped with a filter using NMP (2 mL), washed repeatedly three times with NMP (2 mL), three times with water (2 mL), three times with NMP (2 mL), three times with a NMP solution containing HOAt (0.2M, 2 mL), three times with NMP (2 mL), three times with MeOH (2 mL), three times with DCM (2 mL), and three times with heptane (2 mL), and the resin was then dried under reduced pressure to obtain the title compound D053-02R (90.1 mg). The same cleavage operation as in Example 8-16-5 was carried out and production of D053 was confirmed.

[Formula 172]

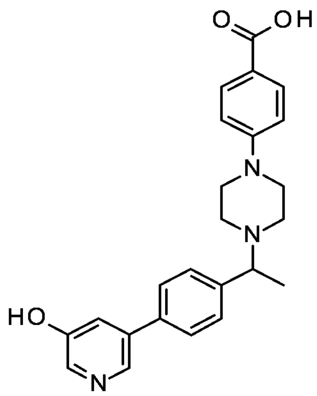

D053

Compound D053

LCMS: m/z 404 $[M+H]^+$.

Retention time: 0.457 min (analysis condition SMD-FA05-1, 290 nm.)

Example 8-16-11: Synthesis of compound D055-02R

[Formula 173]

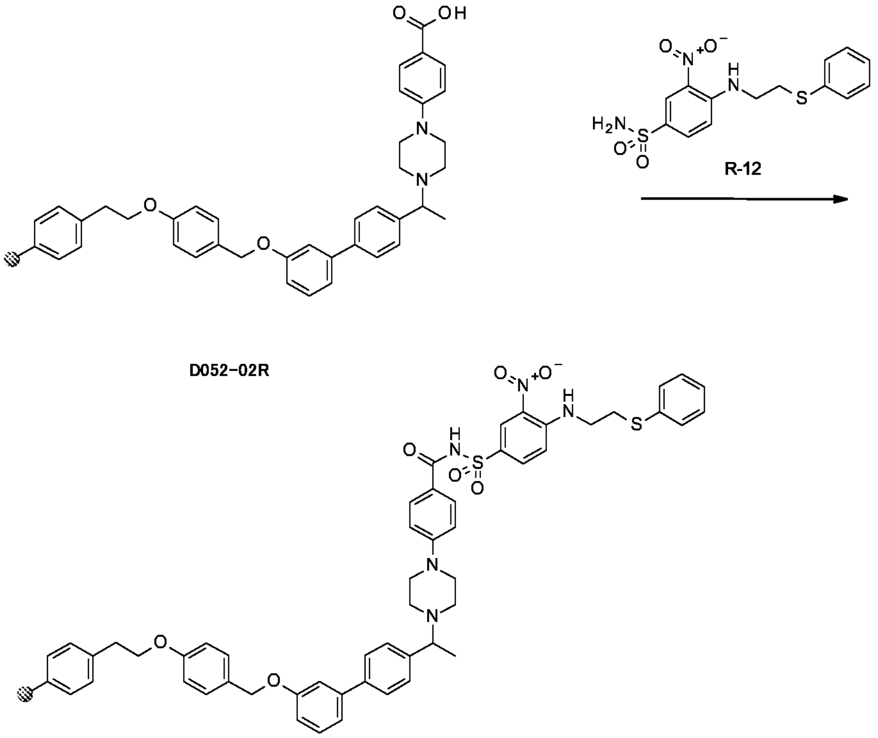

D052-02R

R-12

D055-02R

Under a nitrogen atmosphere, to a 3 mL glass vial were added a solid phase-supported carboxylic acid (compound D052-02R, 81.5 mg, loading rate 0.532 mmol/g, 0.0434 mmol) and NMP (1.8 mL), and the mixture was shaken at room temperature for 1 hour. HATU (99.0 mg, 0.260 mmol) and 2,6-lutidine (30.3 μL, 0.260 mmol) were added at room temperature, and the mixture was shaken at 40°C for 3 hours. 3-Nitro-4-(2-phenylsulfanylethylamino)benzenesulfonamide (R-12, 107 mg, 0.304 mmol) and P1-tBu (165 μL, 0.650 mmol) were added at room temperature, and the mixture was shaken at 40°C for 3 hours. The obtained suspension of reaction solution and resin was transferred to a syringe equipped with a filter using NMP (2 mL), washed repeatedly twice with NMP (2 mL), three times with NMP containing HOAt (0.2M, 2 mL), three times with NMP (2 mL), three times with MeOH (2 mL), three times with DCM (2 mL), and three times with heptane (2 mL), and dried under reduced pressure to obtain the title compound D055-02R (82.4 mg). The same cleavage operation as in Example 8-16-5 was carried out and production of D055 was confirmed.

[Formula 174]

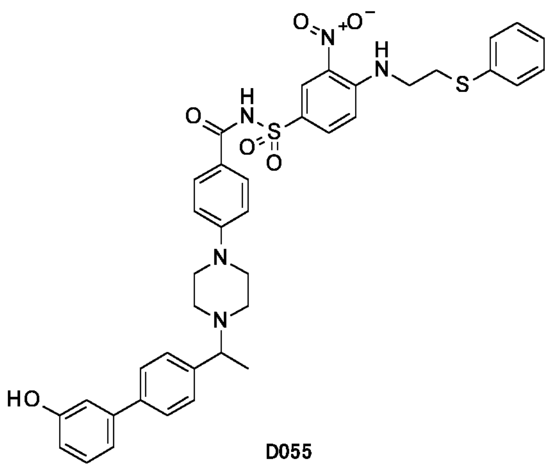

D055

Compound D055

LCMS: m/z 738 $[M+H]^+$.

Retention time: 0.932 min (analysis condition SMD-FA05-1, 290 nm).

Example 8-16-12: Synthesis of compound D056-02R

[Formula 175]

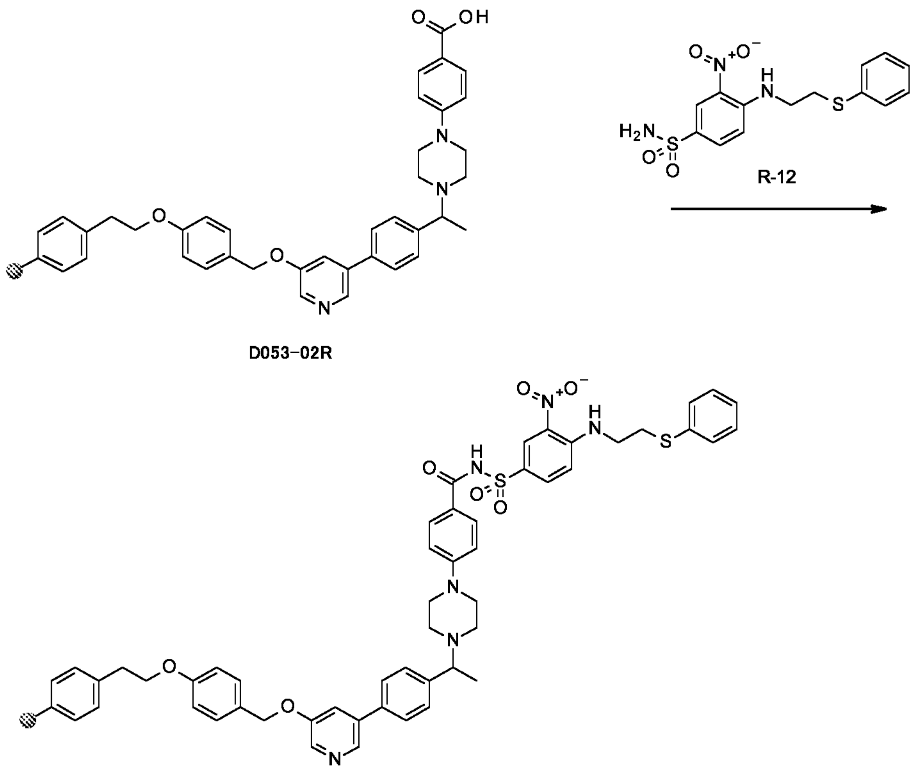


D056-02R

Using a solid phase-supported carboxylic acid (compound D053-02R, loading rate: 0.735 mmol/g, 67.1 mg), the title compound D056-02R (73.4 mg) was synthesized by the same method as in Example 8-16-12 (compound D055-02R) and obtained. The same cleavage operation as in Example 8-16-5 was carried out and production of D056 was confirmed.

[Formula 176]

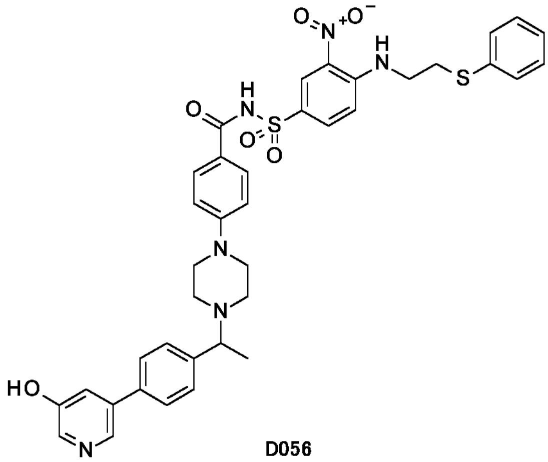

D056

Compound D056

LCMS: m/z 739 $[M+H]^+$.

Retention time: 1.866 min (analysis condition SMD-TFA05-long, 290 nm).

Example 8-16-13: Synthesis of 4-[4-[1-[4-(3-hydroxyphenyl)phenyl]ethyl]piperazin-1-yl]-N-[3-nitro-4-(2-phenylsulfanylethylamino)phenyl]sulfonylbenzamide (compound D055)

[Formula 177]

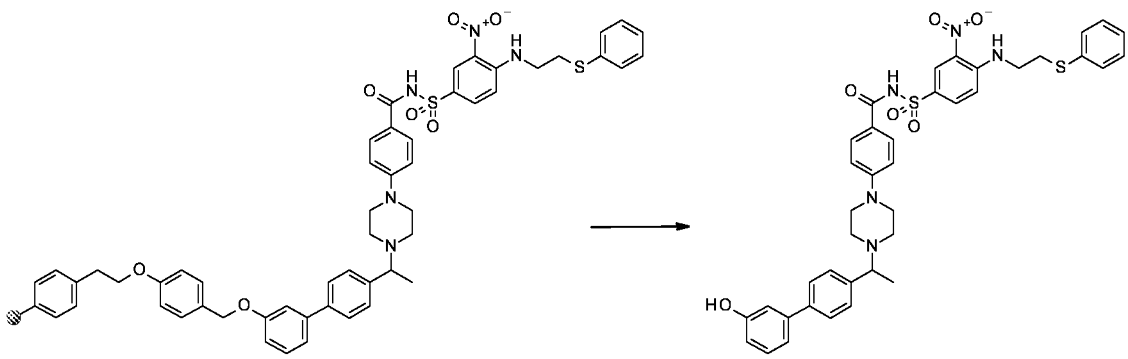

D055-02R D055

Under a nitrogen atmosphere, to a 3 mL glass vial were added compound D055-02R (81.2 mg, loading rate 0.532 mmol/g, 0.0432 mmol) and DCM containing 1,2,3-trimethylbenzene (0.23M, 1.14 mL), and the mixture was shaken at room temperature for 1 hour. TFA (487 μL, 6.32 mmol) and 1-bromo-4-iodobenzene (D057, 12.2 mg, 0.043 mmol, added as an internal standard) were added, and the mixture was shaken at room temperature for 3 hours. The obtained suspension of reaction solution and resin was transferred to a syringe equipped with a filter using DCM (0.8 mL) and filtered. The resin was washed with DCM (0.8 mL), to the obtained filtrate were added DMSO (0.8 mL) and $H_2O$ (0.2 mL), and the mixture was concentrated under reduced pressure. The obtained residue was purified by reversed phase column chromatography (C18, 0 to 100% 0.1% TFA solution in acetonitrile/0.1% aqueous TFA solution) to afford the title compound D055 (15.1 mg, 0.020 mmol, 47.4%) as a yellow solid.

Example 8-16-14: Synthesis of 4-[4-[1-[4-(5-hydroxypyridin-3-yl)phenyl]ethyl] piperazin-1-yl]-N-[3-nitro-4-(2-phenylsulfanylethylamino)phenyl]sulfonylbenzamide (compound D056)

[Formula 178]

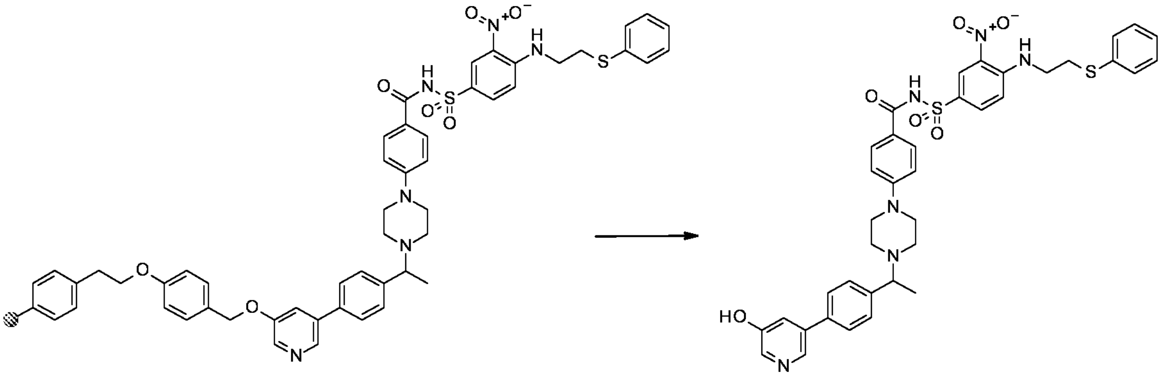

D056-02R

D056

Under a nitrogen atmosphere, to a 3 mL glass vial were added compound D056-02R (85.9 mg, loading rate 0.735 mmol/g, 0.0631 mmol) and DCM containing 1,2,3-trimethylbenzene (0.23M, 1.20 mL), and the mixture was shaken at room temperature for 1 hour. TFA (515 μL, 6.69 mmol) and 1-fluoro-4-(4-fluorophenyl)benzene (D058, 12.0 mg, 0.063 mmol, added as an internal standard) were added, and the mixture was shaken at room temperature for 3 hours. The obtained suspension of reaction solution and resin was transferred to a syringe equipped with a filter using DCM (0.8 mL) and filtered. The resin was washed with DCM (0.8 mL). DMSO (0.8 mL) and water (0.2 mL) were added to the filtrate, and the mixture was concentrated under reduced pressure. The obtained residue was purified by reversed phase column chromatography (C18, 0 to 100% 0.1% TFA solution in acetonitrile/0.1% aqueous TFA solution) to afford the title compound D056 (13.4 mg, 0.0181 mmol, 28.7%) as a yellow solid.

<u>Example 8-16-15: Synthesis of 4-[4-[1-[4-(3-ethoxyphenyl)phenyl]ethyl]piperazin-1-y1]-N-[3-nitro-4-(2-phenylsulfanylethylamino)phenyl]sulfonylbenzamide [D059 (B2Q097-</u>01)]

[Formula 179]

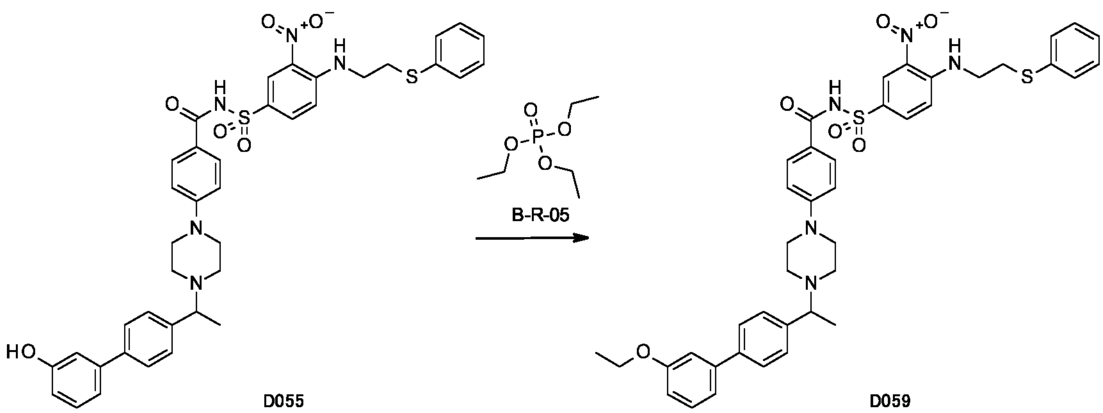


D055

D059

Under a nitrogen atmosphere, to a 1 mL screw cap vial were added 4-[4-[1-[4-(3-hydroxyphenyl)phenyl] ethyl]piperazin-1-yl] -N-[3 -nitro-4-(2-phenylsulfanylethylamino)phenyl]sulfonylbenzamide (D055, 15.1 mg, 0.020 mmol) and DMI (0.29 mL). To that solution were added BTPP (94.0 μL, 0.307 mmol) and triethyl phosphate (B-R-05, 52.3 μL, 307 mmol), and the mixture was stirred at 80°C for 30 hours. To the obtained residue was added formic acid (17.8 μL, 0.409 mmol), and the mixture was purified by reversed phase column chromatography (C18, 0 to 80% 0.1% formic acid solution in acetonitrile/0.1% aqueous formic acid solution) to afford the title compound D059 (2.0 mg, 2.6 μmol, 13%) as a yellow solid.

[Formula 180]

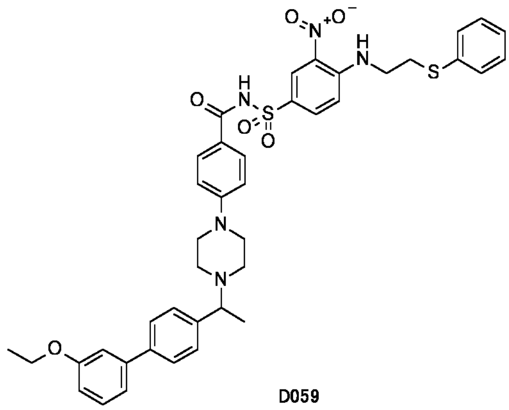

D059

Compound D059

$^1$H-NMR (400 MHz, $CD_2Cl_2$) $\delta$ 8.81 (d, J = 2.0 Hz, 1H), 8.63 (t, J = 5.2 Hz, 1H), 8.06 (dd, J = 9.2, 2.0 Hz, 1H), 7.64 (d, J = 8.8 Hz, 2H), 7.56 (d, J = 8.0 Hz, 2H), 7.41-7.39 (m, 4H), 7.35-7.22 (m, 4H), 7.16 (d, J = 7.6 Hz, 1H), 7.11 (t, J = 2.0 Hz, 1H), 6.88-6.81 (m, 4H), 4.08 (q, J = 7.2 Hz, 2H), 3.59 (q, J = 6.4 Hz, 2H), 3.50-3.45 (m, 1H), 3.32-3.31 (m, 2H), 3.23 (t, J = 6.4 Hz, 2H), 3.09-3.04 (m, 2H), 2.68-2.64 (m, 2H), 2.58-2.52 (m, 2H), 1.44-1.40 (m, 6H).

LCMS: m/z 766 $[M+H]^+$.

Retention time: 1.044 min (analysis condition SMD-FA05-1, 290 nm).

D059, whose synthetic method is illustrated in Example 8-16-15, may also be denoted as compound B2Q097-01 in the present specification.

Example 8-16-16: Synthesis of 4-[4-[1-[4-(5-ethoxypyridin-3-yl)phenyl]ethyl]piperazin-1-yl]-N-[3-nitro-4-(2-phenylsulfanylethylamino)phenyl]sulfonylbenzamide [D060 (B2Q098-01)]

[Formula 181]

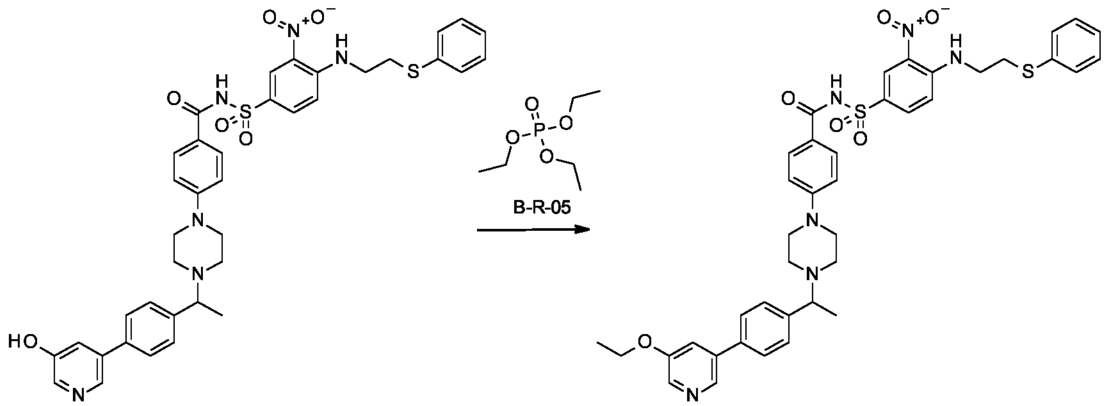


D056 D060

Under a nitrogen atmosphere, to a 1 mL screw cap vial were added 4-[4-[1-[4-(5-hydroxypyridin-3-yl)phenyl]ethyl]piperazin-1-yl]-N-[3-nitro-4-(2-phenylsulfanylethylamino)phenyl]sulfonylbenzamide (D056, 13.4 mg, 0.018 mmol) and DMI (0.26 mL). To that solution were added BTPP (83.0 μL, 0.272 mmol) and triethyl phosphate (B-R-05, 46.4 μL, 272 mmol), and the mixture was stirred at 80°C for 22 hours. To the obtained residue was added ammonium bicarbonate (28.7 mg, 0.363 mmol), and the mixture was purified by reversed phase column chromatography (C18, 0 to 60% acetonitrile/10 mM aqueous ammonium bicarbonate solution) to afford the title compound D060 (6.0 mg, 7.8 μmol, 43%) as a yellow solid.

[Formula 182]

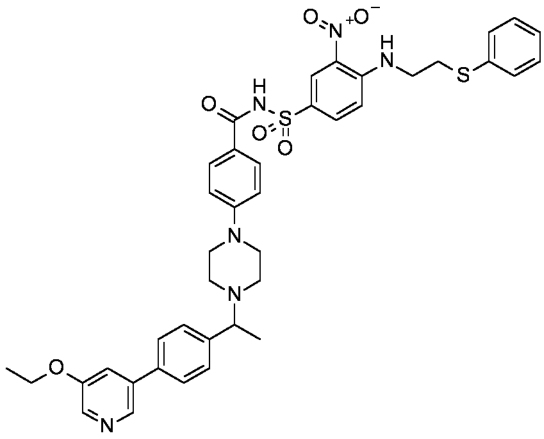

D060

Compound D060

$^1$H-NMR (400 MHz, $CD_2Cl_2$) $\delta$ 8.69 (d, J = 2.0 Hz, 1H), 8.42 (d, J = 1.6 Hz, 1H), 8.36 (t, J = 5.6 Hz, 1H), 8.23 (d, J = 2.8 Hz, 1H), 8.03 (dd, J = 8.8, 2.0 Hz, 1H), 7.89 (d, J = 8.8 Hz, 2H), 7.56 (d, J = 8.4 Hz, 2H), 7.47-7.37 (m, 5H), 7.33-7.21 (m, 3H), 6.76 (d, J = 9.2 Hz, 2H), 6.76 (d, J = 9.2 Hz, 1H), 4.15 (q, J = 7.2 Hz, 2H), 3.53 (q, J = 6.8 Hz, 2H), 3.45 (q, J = 6.8 Hz, 1H), 3.26-3.18 (m, 2H), 3.13-3.09 (m, 2H), 2.68-2.63 (m, 2H), 2.56-2.51 (m, 2H), 1.81-1.78 (m, 2H), 1.45 (t, J = 6.8 Hz, 3H), 1.40 (d, J = 6.8 Hz, 3H).

LCMS: m/z 767 $[M+H]^+$.

Retention time: 2.020 min (analysis condition SMD-TFA05-long, 290 nm).

D060, whose synthetic method is illustrated in Example 8-16-16, may also be denoted as compound B2Q098-01 in the present specification.

Example 9: Binding evaluation of compounds to Bcl-2 by surface plasmon resonance (SPR)

In order to analyze the binding affinity of the compounds shown in [Table AK-01] and synthesized in Example 8 to Bcl-2, the surface plasmon resonance method was used. Biacore T200 (GE Healthcare) was used as the apparatus, and HBS (10 mM HEPES-NaOH, 150 mM NaCl, pH 7.4) to which 1 mM DTT, 3 mM EDTA, 0.01% Tween 20, and 4% DMSO had been added was used as the running buffer. The human Bcl-2 protein (Novus Biologicals) was biotinylated using EZ-Link Sulfo-NHS-LC-LC-biotin (Thermofisher scientific) and immobilized on the surface of Sensor Chip SA (GE Healthcare). Thereafter, compound solutions of multiple concentrations and the running buffer (blank) were added to the Bcl-2 immobilized surface to obtain the binding response of the compounds. The measurement was carried out at 15°C.

The obtained sensorgrams were processed by Biacore T200 Evalution Software to determine the equilibrium dissociation constant $K_D$ for each compound to Bcl-2 as shown in [Table AK-02] and [Table AK-03] below.

[Table 142]

[Table AK-01]: Structural formula of compounds of compounds used in the present Example

| Table AK-01 | | |
|---|---|---|
| Compund No. in Example 9 | Compound No. in Example 8 | Structure |
| B2Q045-01 | AG005 | 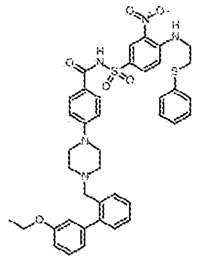 |
| B2Q046-01 | AG007 | 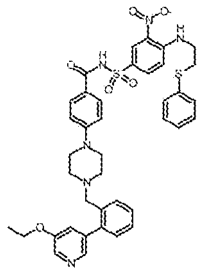 |
| B2R045-01 | AG012 | 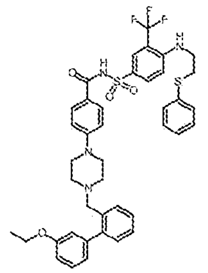 |
| B2R046-01 | AG013 | 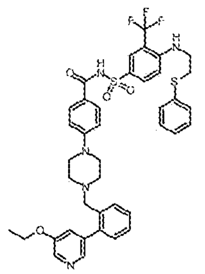 |
| B2Q047-01 | AG016 | 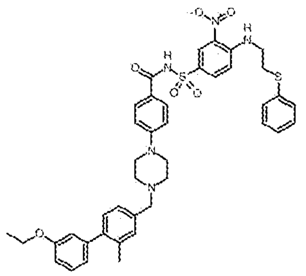 |

[Table 143]

| Compound No. in Example 9 | Compound No. in Example 8 | Structure |
|---|---|---|
| B2Q097-01 | D059 | 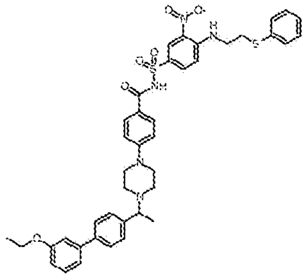 |
| B2Q048-01 | AG018 | 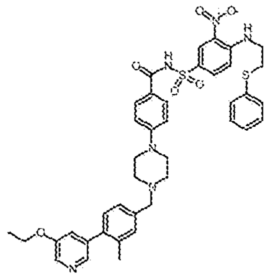 |
| B2Q098-01 | D060 | 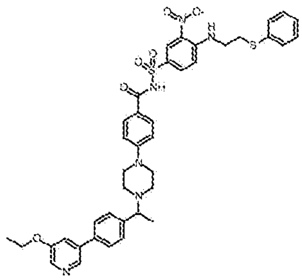 |
| B2Q053-01 | AG045 | 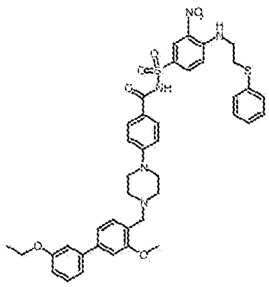 |
| B2Q054-01 | AG022 | 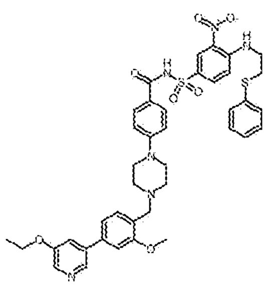 |

[Table 144]

| Compound No. in Example 9 | Compound No. in Example 8 | Structure |
|---|---|---|
| B2Q066-01 | AG025 | 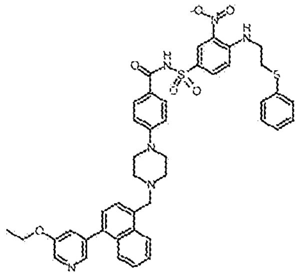 |
| B1A075-01 | AG038 | 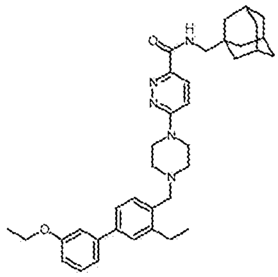 |
| B3A073-01 | AG042 | 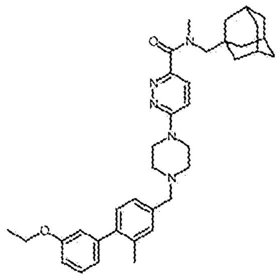 |
| B2R074-01 | AG052 | 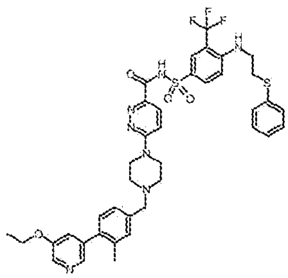 |
| B2Q012-01 | AG051 | 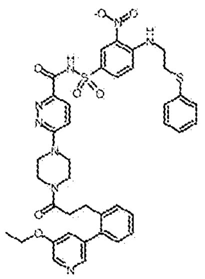 |

[Table 145]

[Table AK-02]: Equilibrium dissociation constant $K_D$ of compounds synthesized before performing library synthesis

| Compound | $K_D$ [μmol/L] |
|---|---|
| B2Q045-01 | 1.2 |
| B2Q046-01 | 8.1 |
| B2R045-01 | Bound |
| B2R046-01 | Bound |

[Table 146] [Table AK-03]: Equilibrium dissociation constant $K_D$ of compounds found by AS-MS evaluation of synthesized library

| Compound | Compositional formula | $K_D$ [μmol/L] |
|---|---|---|
| B2Q047-01 | $C_{41}H_{43}N_5O_6S_2$ | 0.70 |
| B2Q097-01 | $C_{41}H_{43}N_5O_6S_2$ | Binding not observed |
| B2Q048-01 | $C_{40}H_{42}N_6O_6S_2$ | 0.48 |
| B2Q098-01 | $C_{40}H_{42}N_6O_6S_2$ | Binding not observed |
| B2Q053-01 | $C_{41}H_{43}N_5$O7S2 | Binding not observed |
| B2Q054-01 | $C_{40}H_{42}N_6O_7S_2$ | 1.27 |
| B2Q066-01 | $C_{43}H_{42}N_6O_6S_2$ | 0.95 |
| B1A075-01 | $C_{37}H_{47}N_5O_2$ | Binding not observed |
| B3A073-01 | $C_{37}H_{47}N_5O_2$ | Binding not observed |
| B2R074-01 | $C_{39}H_{40}F_3N_7O_4S_2$ | Binding not observed |
| B2Q012-01 | $C_{39}H_{40}N_8O_7S_2$ | Bound |

As shown in [Table AK-02] and [Table AK-03], compounds with smaller equilibrium dissociation constants $K_D$ and higher binding to Bcl-2 (B2Q047-01 and B2Q048-01) were found compared to compound B2Q045-01, whose binding ability to Bcl-2 had been previously evaluated.

As mentioned above, in Example 7, compounds having binding ability to Bcl-2 were found by AS-MS from the mixture libraries in Examples 6-5-1 and 6-5-2, in which arenol had been ethylated using the approach of the present invention. Based on those results, the binding ability of the individually synthesized compounds in Example 8 to Bcl-2 was confirmed by SPR. In other words, it is meant that ethylation by the present invention in Example 6 gave the desired compound group in a highly functional group-selective manner. That is, it was demonstrated that the present invention can provide a practical method by which the desired acidic functional group can be alkylated in a highly functional group-selective manner in the presence of multiple substrates. It was also shown that the present invention can be applied to preparation of mixture libraries, which can be used for pharmaceutical hit discovery.

Example 10: Investigation on O-selective ethylation of carboxylic acid to substrates having multiple reactive functional groups in molecule

Example 10-1: O-Selective ethylation of carboxylic acid using 3-[2-[[4-[4-[[3-nitro-4-(2-phenylsulfanylethylamino)phenyl]sulfonylcarbamoyl]phenyl]piperazin-1 - yl]methyl]phenyl]benzoic acid (B-082) and 5-[2-[[4-[4-[[3-nitro-4-(2-phenylsulfanylethylamino)phenyl] sulfonylcarbamoyl]phenyl]piperazin-1-yllmethyllphenyllpyridine-3-carboxylic acid (B-084) as substrates having aniline NH group

[Formula 183]

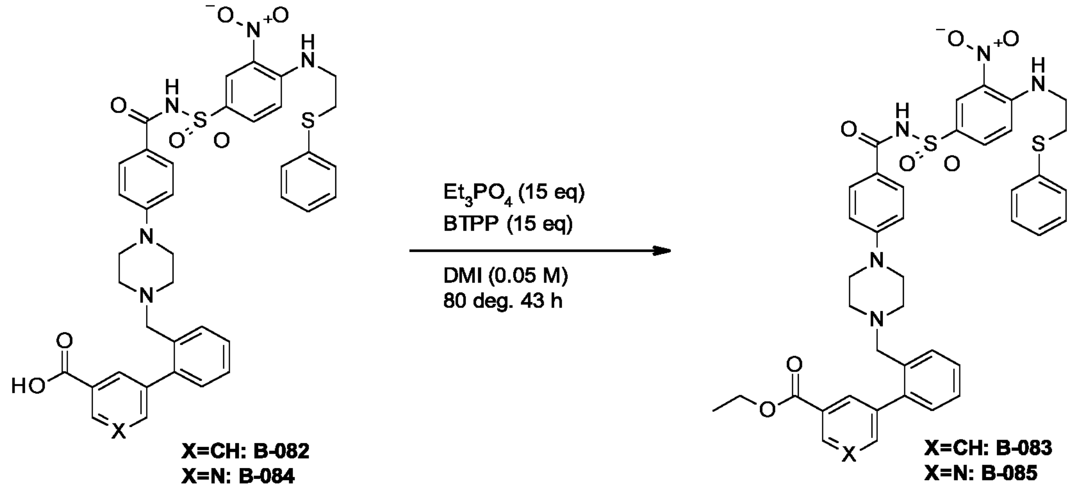


X=CH: B-082
X=N: B-084

X=CH: B-083
X=N: B-085

In a glove bag that had been purged with nitrogen, to a 0.6 mL screw cap vial were added 3-[2-[[4-[4-[[3-nitro-4-(2-phenylsulfanylethylamino)phenyl] sulfonylcarbamoyl]phenyl]piperazin-1-yl]methyl]phenyl]benzoic acid (B-082) (7.5 mg, 10.0 μmol, 1.0 eq, Table 10-1, Run 1) and DMI (188.0 μL). To the obtained solution was added BTPP (45.9 μL, 150.0 μmol, 15.0 eq), and after mixing for 15 to 20 seconds, triethyl phosphate (25.6 μL, 150.0 μmol, 15.0 eq) was added. The obtained reaction mixture was stirred at 80°C for 43 hours at a number of rotations of 800 rpm.

Also, in Table 10-1, Run 2, using 5-[2-[[4-[4-[[3-nitro-4-(2-phenylsulfanylethylamino)phenyl] sulfonylcarbamoyl]phenyl]piperazin-1-yl]methyl]phenyl]pyridine-3-carboxylic acid (B-084) (7.5 mg, 10.0 μmol, 1.0 eq, Table 10-1, Run 2), the reaction was performed as in Run 1 and the reaction progress was measured.

Reaction tracking

During the process, at 1.5, 26, and 43 hours after the start of the reaction, 5 μL of the reaction solution was sampled in a glove bag and diluted with $CH_3CN$ (1000 μL) to prepare a LC sample. The reaction progress was measured by carrying out LC-MS analysis. The results were as shown in Table 10-1, Run 1 to Run 2.

Ethyl 3-[2-[[4-[4-[[3-nitro-4-(2-phenylsulfanylethylamino)phenyl] sulfonylcarbamoyl]phenyl]piperazin-1-yl]methyl]phenyl]benzoate: Compound B-083.

LCMS (ESI, m/z): 780.2 $[M+H]^+$.

Retention time: 1.085 min (analysis condition SMD-FA05-1).

(The retention time of the raw material $ArCO_2H$ is 0.940 min (analysis condition SMD-FA05-1).

Ethyl 5-[2-[[4-[4-[[3-nitro-4-(2-phenylsulfanylethylamino)phenyl] sulfonylcarbamoyl]phenyl]piperazin-1-yl]methyl]phenyl]pyridine-3-carboxylate: Compound B-085.

LCMS (ESI, m/z): 781.2 $[M+H]^+$.

Retention time: 1.040 min (analysis condition SMD-FA05-1).

(The retention time of the raw material $ArCO_2H$ is 0.900 min (analysis condition SMD-FA05-1).

[Table 147]

[Table 10-1]

| Run | Substrate | X | Time (h) | SM (CO2H) (area%) | C02Et (area%) | Byproducts (area%) (not identified) |
|---|---|---|---|---|---|---|
| 1 | B-082 | CH | 1.5 | 68.9 | 29.3 | 1.8 |
| | | | 26 | ND | 97.1 | 2.9 |
| | | | 43 | ND | 97.0 | 3.0 |
| 2 | B-084 | N | 1.5 | 79.7 | 19.0 | 1.3 |
| | | | 26 | 1.1 | 97.1 | 1.8 |

(continued)

| Run | Substrate | X | Time (h) | SM (CO2H) (area%) | C02Et (area%) | Byproducts (area%) (not identified) |
|---|---|---|---|---|---|---|
| | | | 43 | 0.3 | 97.9 | 1.8 |

As shown in Table 10-1 above, it was confirmed that the reaction conditions set in Example 3-6-3 can carry out O-selective ethylation to carboxylic acid having various functional groups. Specifically, O-selective ethylation conditions for carboxylic acid in the coexistence of functional groups such as a tertiary amine, an acylsulfonamide, an aniline, and a pyridine ring were found. Note that the present approach of the present invention is a widely applicable approach, not limited to the functional groups exemplified in the present Example.

Example 10-2: Confirmation of further functional group selectivity under reaction conditions set in Example 3-6-3

In Example 10-1, it was confirmed that the reaction conditions set in Example 3-6-3 allow for selective O-ethylation of carboxylic acid even when many functional groups coexist. Under the present reaction conditions, confirmation of further functional group selectivity was carried out. The method of confirmation was to add a compound having a functional group to the ethylation reaction of a carboxylic acid model substrate (quinoline-3-carboxylic acid (B-086) (pKa[a] = 1.15) or [1,1'-biphenyl]-3-carboxylic acid (B-088) (pKa[a] = 4.19)) and to investigate whether the ethylation of the compound having a functional group proceeds. In other words, if the carboxylic acid of the carboxylic acid model substrate (B-086 or B-088) is selectively ethylated compared to the added compound having a functional group, then the functional group selectivity will be confirmed.
[a]: ADMET predictor (version 9.5) (all parameters that can be changed were used as default values)

Example 10-2-1: Confirmation of ethylation reaction in the presence of various NH groups using quinoline-3-carboxylic acid (B-086) as substrate

The possibility of N-ethylation of NH groups in the case where various NH groups are present in the reaction was investigated. In the following, the method of identifying the applicable range of various NH functional groups is illustrated as an example.

[Formula 184]

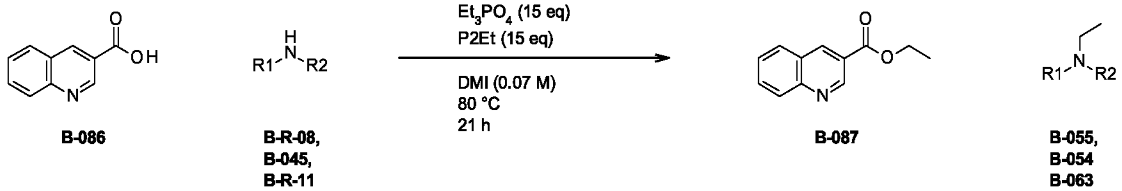


B-086 B-R-08, B-045, B-R-11 B-087 B-055, B-054 B-063

In a glove bag that had been purged with nitrogen, to a 1.5 mL screw cap vial were added quinoline-3-carboxylic acid (B-086) (6.1 mg, 35.0 μmol, 1.0 eq), diphenylamine (B-R-08) (5.9 mg, 35.0 μmol, 1.0 eq), and DMI (0.5 mL). To this solution was added BTPP (161.0 μL, 525.0 μmol, 15.0 eq), and after confirming the mixing, triethyl phosphate (B-R-05) (89.0 μL, 525.0 μmol, 15.0 eq) was added. The obtained reaction mixture was stirred at 80°C at a number of rotations of 800 rpm.
Also, instead of diphenylamine (B-R-08), the reaction was performed in the same manner, using tert-butyl 4-[6-(2,2,2-trifluoroethylcarbamoyl)pyridazin-3-yl]piperazine-1-carboxylate (B-045) (6.8 mg, 18.0 μmol, 0.5 eq, Run 2) and N-butylaniline (B-R-11) (14.0 μL, 88.0 μmol, 2.5 eq, Run 3).

Reaction tracking

At 21 hours after the start of the reaction, 5 μL of the reaction solution was sampled in a glove bag and diluted with $CH_3CN$ (1000 μL) to prepare a LC sample. The reaction progress was measured by carrying out LC-MS analysis. The results were as shown in Table 10-2-1.
Ethyl quinoline-3-carboxylate: Compound B-087.
LCMS (ESI, m/z): 202.1 $[M+H]^+$.
Retention time: 0.939 min (analysis condition SMD-FA05-1).
(The retention time of the raw material ArCOaH is 0.521 min (analysis condition SMD-FA05-1).

The results of Example 10-2-1 are shown in Table 10-2-1. Note that the conversion rate (%) (B-087/B-086) shown in Table 10-2-1 was calculated as (B-087, area%)/[(B-086, area%) + (B-087, area%)] × 100. Also, the conversion rate (%) (additive) was calculated as (ethylated product of additive, area%)/[(ethylated product of additive, area%) + (additive, area%)] × 100. The sample when the additive was ethylated was synthesized as shown in Example 1-3 and confirmed with reference to the retention time and detected MS in Table 1-3.

[Table 148]

[Table 10-2-1]

| Run | Additive | | Additive (equivalent) | Conversion rate (%) B-087 / B-086 | Conversion rate (%) Additive |
|---|---|---|---|---|---|
| 1 | B-R-08 | 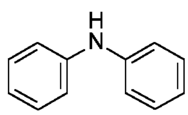 | 1.0 | 97.6 | < 0.1 |
| 2 | B-045 | 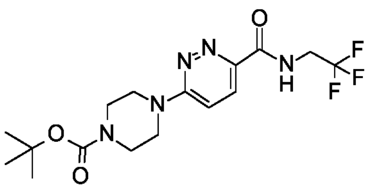 | 0.5 | 97.7 | < 0.1 |
| 3 | B-R-11 | 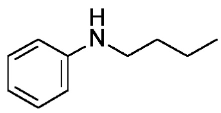 | 2.5 | 95.5 | < 0.1 |

As shown in Table 10-2-1, it was confirmed that ethylation of quinoline-3-carboxylic acid (B-086) proceeded under the present conditions, but the reaction conversion rates of the added diarylamine entity (B-R-08), amide entity (B-045), and secondary aniline (B-R-11) were 0.1% or less. From these results, it is shown that the present approach of the present invention allows for O-selective alkylation of carboxylic acid even when various amine NH groups are present in the reaction system, not limited to the presence of those exemplified in the present Example.

<u>Example 10-2-2: Confirmation of ethylation reaction in the presence of various NH groups using [1,1'-biphenyl]-3-carboxylic acid (B-088) as substrate</u>

Using a carboxylic acid with pKa[a] = 4.19, [1,1'-biphenyl]-3-carboxylic acid (B-088), as a substrate, the possibility of N-ethylation of NH groups in the case where various NH groups are present in the reaction was investigated. In the following, the method of identifying the applicable range of various NH functional groups using [1,1'-biphenyl]-3-carboxylic acid as a substrate is illustrated as an example.

[Formula 185]

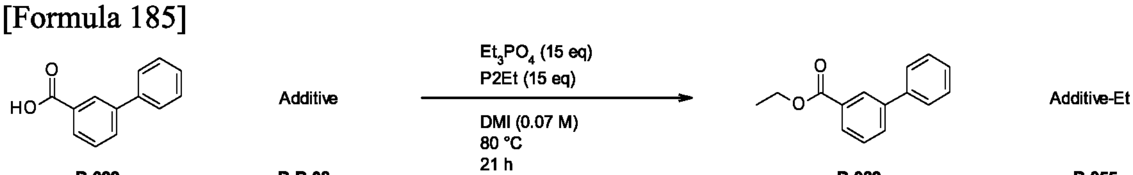


**B-088** **B-R-08, B-045, B-R-11** **B-089** **B-055, B-054 B-063**

In a glove bag that had been purged with nitrogen, to a 1.5 mL screw cap vial were added [1,1'-biphenyl]-3-carboxylic acid (B-088) (6.9 mg, 35.0 μmol, 1.0 eq), diphenylamine (B-R-08) (5.9 mg, 35.0 μmol, 1.0 eq), and DMI (0.5 mL). To this solution was added BTPP (161.0 μL, 525.0 μmol, 15.0 eq), and after confirming the mixing, triethyl phosphate

(B-R-05) (89.0 μL, 525.0 μmol, 15.0 eq) was added. The obtained reaction mixture was stirred at 80°C at a number of rotations of 800 rpm.

Also, instead of diphenylamine (B-R-08), the reaction was performed in the same manner, using tert-butyl 4-[6-(2,2,2-trifluoroethylcarbamoyl)pyridazin-3-yl]piperazine-1-carboxylate (B-045) (6.8 mg, 18.0 μmol, 0.5 eq, Run 2) and N-butylaniline (B-R-11) (14.0 μL, 88.0 μmol, 2.5 eq, Run 3).

Reaction tracking

At 21 hours after the start of the reaction, 5 μL of the reaction solution was sampled in a glove bag and diluted with $CH_3CN$ (1000 μL) to prepare a LC sample. The reaction progress was measured by carrying out LC-MS analysis. The results were as shown in Table 10-2-1.

Ethyl 3-phenylbenzoate: Compound B-089.

Retention time: 1.356 min (analysis condition SMD-FA05-1).

(The retention time of the raw material ArCOaH is 1.016 min (analysis condition SMD-FA05-1).

The results of Example 10-2-2 are shown in Table 10-2-2. Note that the conversion rate (%) (B-089/B-088) shown in Table 10-2-2 was calculated as (B-089, area%)/[(B-089, area%) + (B-0088, area%)] × 100. Also, the conversion rate (%) (additive) was calculated as (ethylated product of additive, area%)/[(ethylated product of additive, area%) + (additive, area%)] × 100. The sample when the additive was ethylated was synthesized as shown in Example 1-3 and confirmed with reference to the retention time and detected MS in Table 1-3.

[Table 149]

[Table 10-2-2]

| Run | Additive | | Additive (equivalent) | Conversion rate (%) B-089 / B-088 | Conversion rate (%) Additive |
|---|---|---|---|---|---|
| 1 | B-R-08 | 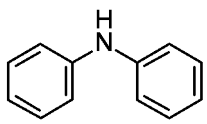 | 1.0 | 98.4 | < 0.1 |
| 2 | B-045 | 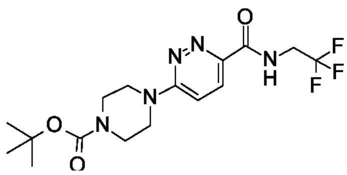 | 0.5 | 98.7 | < 0.1 |
| 3 | B-R-11 | 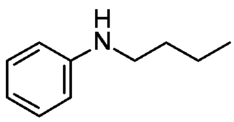 | 2.5 | 95.3 | < 0.1 |

As shown in Table 10-2-2, it was confirmed that ethylation of [1,1'-biphenyl]-3-carboxylic acid (B-088) proceeded under the present conditions, but the reaction conversion rates of the added diarylamine entity (B-R-08), amide entity (B-045), and secondary aniline (B-R-11) were 0.1 % or less. From these results, it is shown that the present approach of the present invention allows for O-selective alkylation of carboxylic acid even when various amine NH groups are present in the reaction system, not limited to the presence of those exemplified in the present Example.

As in the results of functional group selectivity in the O-ethylation reaction of arenol confirmed in Example 3-8, the alkylation progress of various NH functional groups (a diarylamine, an amide, a secondary aniline, a primary aniline, and a tertiary amine) was 5% or less under the present conditions, and it was confirmed that selective alkylation of carboxylic acid is possible even when an amine functional group is present, not limited to the amine functional groups exemplified in the present Example.

Example 10-3: Synthesis method in which alkylation of both arenol and carboxylic acid is performed in system where arenol and carboxylic acid having various functional groups are present, and then only alkylated product of carboxylic acid is hydrolyzed to regenerate carboxylic acid (alkylation as carboxylic acid protection/hydrolysis as deprotection)

[Formula 186]

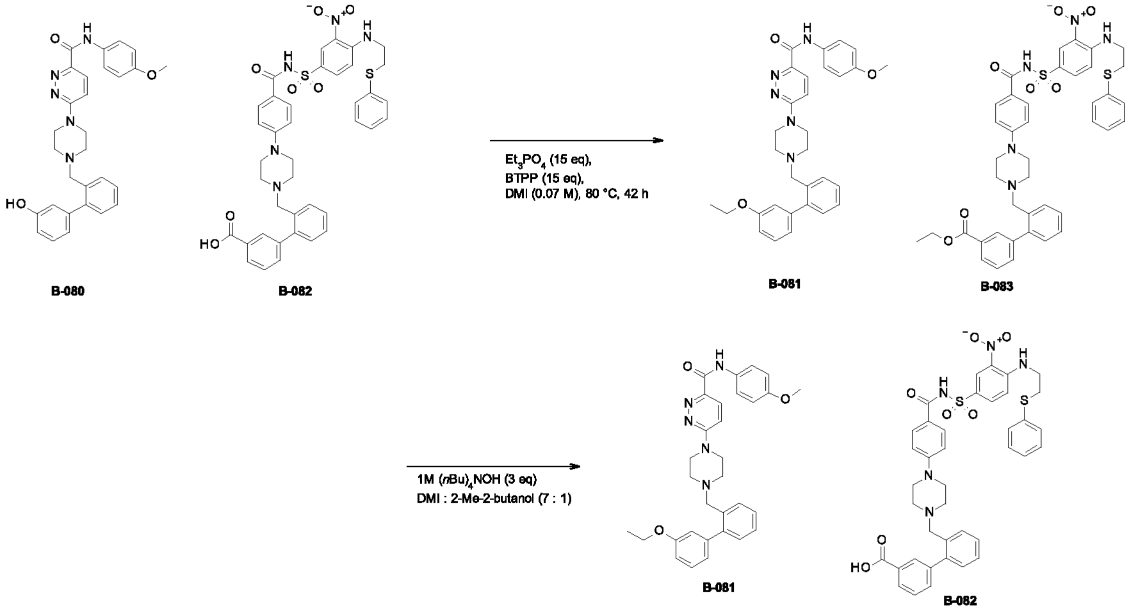


B-080 B-082 B-081 B-083 B-081 B-082

First step: Alkylation

In a glove bag that had been purged with nitrogen, to a 0.6 mL screw cap vial were added 6-[4-[[2-(3-hydroxyphenyl)phenyl]methyl]piperazin-1-yl]-N-(4-methoxyphenyl)pyridazine-3-carboxamide (B-080) (3.5 mg, 7.0 μmol, 1.0 eq), 3-[2-[[4-[4-[[3-nitro-4-(2-phenylsulfanylethylamino)phenyl]sulfonylcarbamoyl]phenyl]piperazin-1-yl]methyl]phenyl]benzoic acid (B-082) (5.3 mg, 7.0 μmol, 1.0 eq), and DMI (100.0 μL). To the obtained solution was added BTPP (32.1 μL, 105.0 μmol, 15.0 eq), and after mixing for 15 to 20 seconds, triethyl phosphate (17.9 μL, 105.0 μmol, 15.0 eq) was added. The obtained reaction mixture was stirred at 80°C for 42 hours at a number of rotations of 800 rpm.

Reaction tracking

During the process, at 21 and 42 hours after the start of the reaction, 5 μL of the reaction solution was sampled in a glove bag and diluted with $CH_3CN$ (1000 μL) to prepare a LC sample. The reaction progress was measured by carrying out LC-MS analysis. The results were as shown in Table 10-3-1, Run 1.

6-[4-[[2-(3-Ethoxyphenyl)phenyl]methyl]piperazin-1-yl]-N-(4-methoxyphenyl)pyridazine-3-carboxamide: Compound B-081.

LCMS (ESI, m/z): 524.3 $[M+H]^+$.

Retention time: 0.901 min (analysis condition SMD-FA05-1).

(The retention time of the raw material ArOH is 0.751 min (analysis condition SMD-FA05-1).

Ethyl 3-[2-[[4-[4-[[3-nitro-4-(2-phenylsulfanylethylamino)phenyl] sulfonylcarbamoyl]phenyl]piperazin-1-yl]methyl]phenyl]benzoate: Compound B-083.

LCMS (ESI, m/z): 780.2 $[M+H]^+$.

Retention time: 1.095 min (analysis condition SMD-FA05-1).

(The retention time of the raw material $ArCO_2H$ is 0.957 min (analysis condition SMD-FA05-1).

Second step: Hydrolysis

In a glove bag that had been purged with nitrogen, to the reaction mixed solution in a 0.6 mL screw cap vial obtained in the first step alkylation were added tetrabutylammonium hydroxide (0.1M, 21.0 μL, 21.0 μmol, 3.0 eq) and 2-methyl 2-butanol (28.5 μL). The obtained reaction mixture was stirred at 25°C for 19 hours.

Reaction tracking

During the process, at 19 hours after the start of the reaction, 5 μL of the reaction solution was sampled in a glove bag and diluted with $CH_3CN$ (1000 μL) to prepare a LC sample. The reaction progress was measured by carrying out LC-MS analysis. The results were as shown in Table 10-3-1, Run 2.

6-[4-[[2-(3-Ethoxyphenyl)phenyl]methyl]piperazin-1-yl]-N-(4-methoxyphenyl)pyridazine-3-carboxamide: Compound B-081.

LCMS (ESI, m/z): 524.3 $[M+H]^+$.

Retention time: 0.901 min (analysis condition SMD-FA05-1).

3-[2-[[4-[4-[[3-Nitro-4-(2-phenylsulfanylethylamino)phenyl] sulfonylcarbamoyl]phenyl]piperazin-1-yl]methyl]phenyl]benzoic acid (B-082).

LCMS (ESI, m/z): 752.1 $[M+H]^+$.

Retention time: 0.952 min (analysis condition SMD-FA05-1).

(The retention time of the raw material $ArCO_2Et$ is 1.095 min (analysis condition SMD-FA05-1).

[Table 150]

[Table 10-3]

| Run | Time (h) | ArOH (B-080) (area%) | ArOEt (B081) (area%) | ArCO2H (B-082) (area%) | ArCO2Et (B-083) (area%) | Byproducts (area%) (not identified) |
|---|---|---|---|---|---|---|
| 1 | 0 | 38.3 | | 59.7 | | 2.0 |
| | 21 | 2.5 | 41.1 | 0.7 | 52.1 | 3.6 |
| | 42 | ND | 46.6 | 0.3 | 50.1 | 3.0 |
| 2 | 19 | 0.3 | 43.8 | 53.3 | ND | 2.6 |

As shown in Table 10-3 above, it was confirmed that the reaction conditions set in Examples 3-6-3 and 10-1 can carry out O-selective ethylation of arenol and O-selective ethylation of carboxylic acid to a mixture of arenol and carboxylic acid having various functional groups. Specifically, O-selective ethylation conditions for arenol and carboxylic acid in the coexistence of functional groups such as a tertiary amine, an acylsulfonamide, an aniline, and a pyridine ring were found. In addition, it was also found that the alkylated product of carboxylic acid can be converted to carboxylic acid by selective hydrolysis in the coexistence of the O-alkyl form of arenol or other functional groups. This indicates that the present method can be utilized as a protective group for carboxylic acid. Note that the approach of the present invention is a widely applicable approach, not limited to the structures exemplified in the present Example.

## Claims

**1.** A method for producing a compound having an alkylated acidic functional group, the method comprising

reacting, in a mixture containing two or more compounds having an acidic functional group as substrates, the compounds with an alkylating agent selected from compounds represented by formula A, formula B, or formula C in the presence of a base to alkylate the acidic functional group:

[Formula 1]

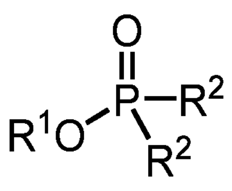

(A)

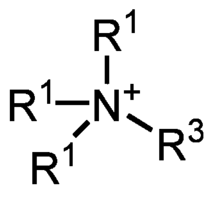

(B)

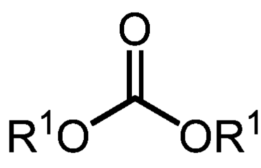

(C)

wherein $R^1$ is independently selected from a $C_{1-6}$ alkyl optionally substituted with one or more substituents selected from X;
X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;
$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and
$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

wherein the base is a base selected from the group consisting of an organic base whose conjugate acid has a pKa of 23 to 34 in acetonitril and an inorganic base whose conjugate acid has a pKa of 9 to 20 in water.

**2.** The method according to claim 1, comprising removing an impurity after the alkylation.

**3.** The method according to claim 1 or 2, wherein the base has a solubility in dimethylformamide (DMF) of 120 mg/mL or more at 25°C.

**4.** The method according to any one of claims 1 to 3, wherein the base is selected from the organic base.

**5.** The method according to any one of claims 1 to 4, wherein the base is selected from a phosphazene.

**6.** A method for producing a compound having an alkoxy-substituted aromatic ring, the method comprising

reacting a compound containing a hydroxy group as a substituent on an aromatic ring with an alkylating agent selected from compounds represented by formula A, formula B, or formula C in the presence of a base to alkylate the hydroxy group as a substituent on an aromatic ring:

[Formula 2]

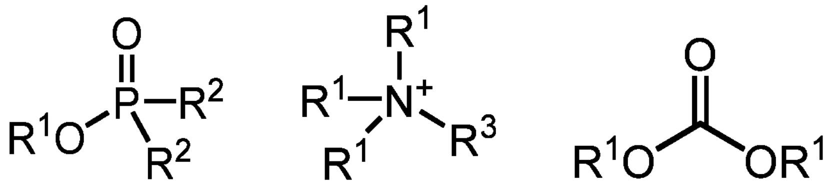

(A) (B) (C)

wherein $R^1$ is independently selected from a $C_{1-6}$ alkyl optionally substituted with one or more substituents selected from X;
X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;
$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and
$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

wherein the base is selected from a phosphazene.

**7.** A method for producing an ester compound, the method comprising

reacting a compound containing a carboxyl group with an alkylating agent selected from compounds represented by formula A, formula B, or formula C in the presence of a base to alkylate the carboxyl group:

[Formula 2]

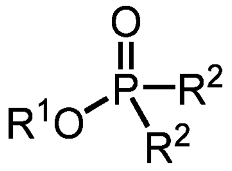 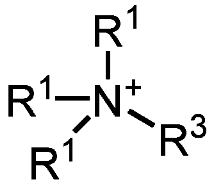 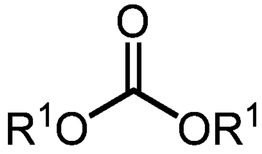

(A) (B) (C)

wherein $R^1$ is independently selected from a $C_{1-6}$ alkyl optionally substituted with one or more substituents selected from X;
X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;
$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and
$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

wherein the base is selected from a phosphazene.

**8.** The method according to any one of claims 1 to 7, wherein the alkylating agent is selected from the group consisting of alkylating agents represented by formulas A and B according to claim 1.

**9.** The method according to any one of claims 1 to 8, wherein the alkylating agent is selected from the group consisting of trimethyl phosphate, triethyl phosphate, and triallyl phosphate.

**10.** The method according to any one of claims 1 to 9, wherein the base is at least one selected from the group consisting of 1-ethyl-2,2,4,4,4-pentakis(dimethylamino)-2λ5,4λ5-catenadi(phosphazene) (P2Et), 1-tert-butyl-2,2,4,4,4-pentakis(dimethylamino)-2λ5,4kλ5-catenadi(phosphazene) (P2tBu), tert-butylimino-tris(dimethylamino)phosphorane (P1tBu), 2-tert-butylimino-2-diethylamino-1,3-dimethylperhydro-1,3,2-diazaphospholine (BEMP), and tert-butylimino-tri(pyrrolidino)phosphorane (BTPP).

**11.** The method according to any one of claims 1 to 10, wherein the acidic functional group is a carboxyl group or a hydroxy group as a substituent on an aromatic ring.

**12.** The method according to any one of claims 1 to 11, wherein the substrate contains one or more compounds containing a phenolic hydroxy group as the acidic functional group and a nitrogen atom that can be alkylated, and in the compound having a nitrogen atom in which alkylation proceeds most among the substrate, a production ratio of products by alkylation of the phenolic hydroxy group (alkylation A) and alkylation of the nitrogen atom (alkylation C), (alkylation A/alkylation C), is 1.5 or more.

**13.** The method according to any one of claims 1 to 11, wherein the substrate contains one or more compounds containing a carboxyl group as the acidic functional group and a nitrogen atom that can be alkylated, and in the compound having a nitrogen atom in which alkylation proceeds most among the substrate, a production ratio of products by alkylation of the carboxyl group (alkylation D) and alkylation of the nitrogen atom (alkylation E), (alkylation D/alkylation E), is 1.5 or more.

**14.** The method according to any one of claims 1 to 13, wherein alkylation C or alkylation D is alkylation of a nitrogen atom contained in a nitrogen-containing structure selected from a diarylamine, an alkylarylamine, an aniline, a tertiary amine, an amide, a sulfonamide, an acylsulfonamide, and a pyridine.

**15.** A method for producing a compound constituting a compound library, the method comprising producing a compound alkylated by the method according to any one of claims 1 to 14.

**16.** The method according to any one of claims 1 to 15, wherein a mixture containing 10 or more compounds is produced.

**17.** An alkylating agent containing a compound represented by formula A, formula B, or formula C:

[Formula 3]

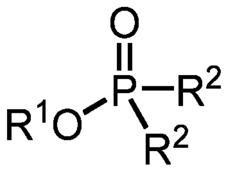

(A)

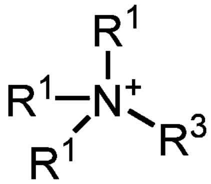

(B)

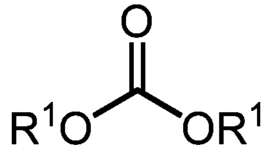

(C)

wherein $R^1$ is independently selected from a $C_{1-6}$ alkyl optionally substituted with one or more substituents selected from X;
X is independently selected from a $C_{3-6}$ cycloalkyl, a $C_{2-7}$ alkenyl, a $C_{2-7}$ alkynyl, and a $C_{6-10}$ aryl, where the aryl is optionally substituted with one or more substituents independently selected from a halogen atom, a $C_{1-4}$ alkyl, or a $C_{1-4}$ alkoxy;
$R^2$ is independently selected from $-OR^1$ and an aryl, where the aryl is optionally substituted with one or more substituents independently selected from a halogen atom, a $C_{1-4}$ alkyl, and a $C_{1-4}$ alkoxy; and
$R^3$ is independently selected from a $C_{1-6}$ alkyl optionally substituted with one or more substituents selected from X, and an aryl, where the aryl is optionally substituted with one or more substituents independently selected from a halogen atom, a $C_{1-4}$ alkyl, and a $C_{1-4}$ alkoxy,

for use in a method for producing an alkylated compound, wherein the method is the method according to any one of claims 1 to 14.

# Figure 1

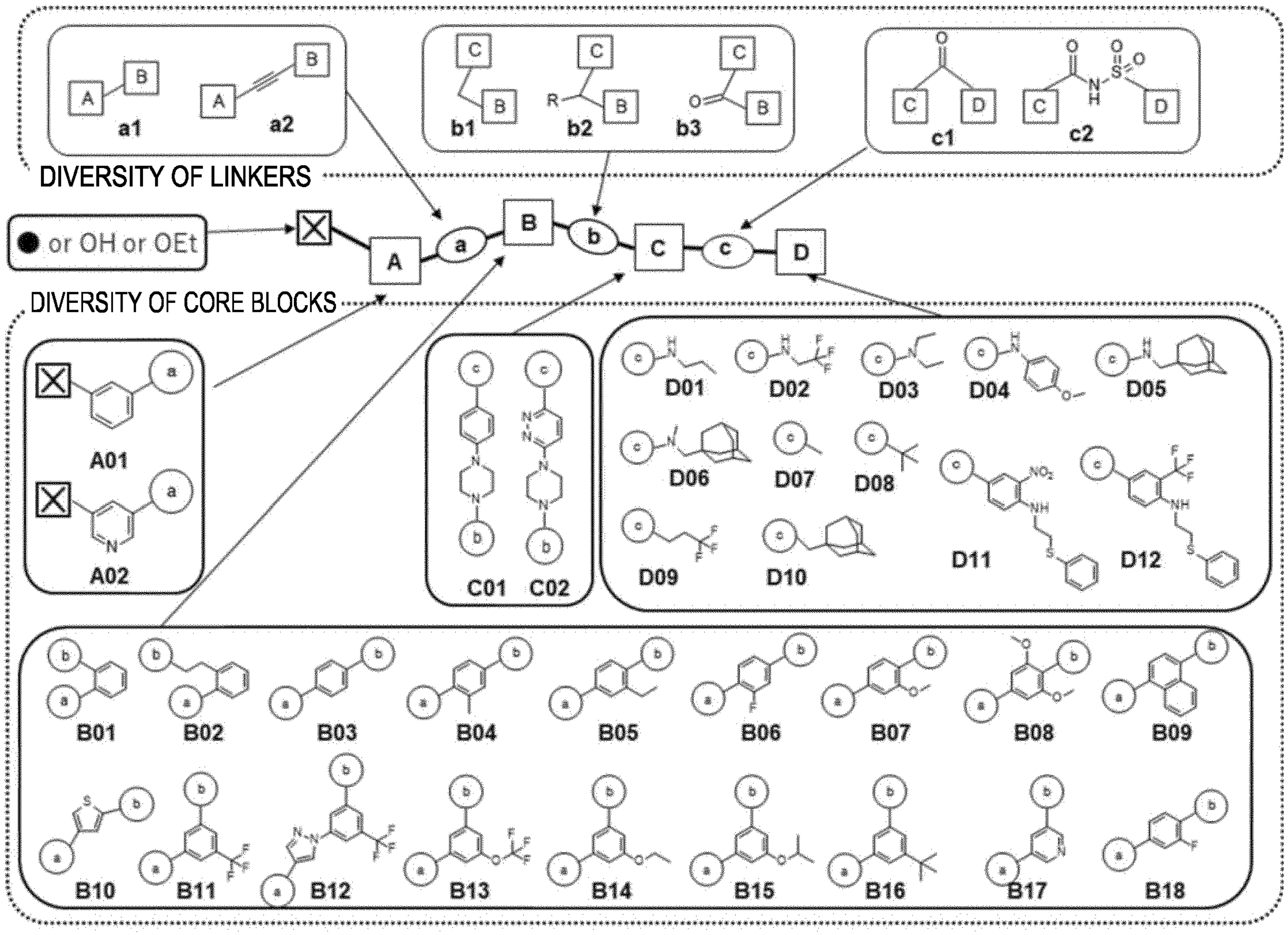


IN FIGURE 1, ● REPRESENTS 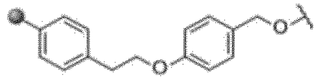 .

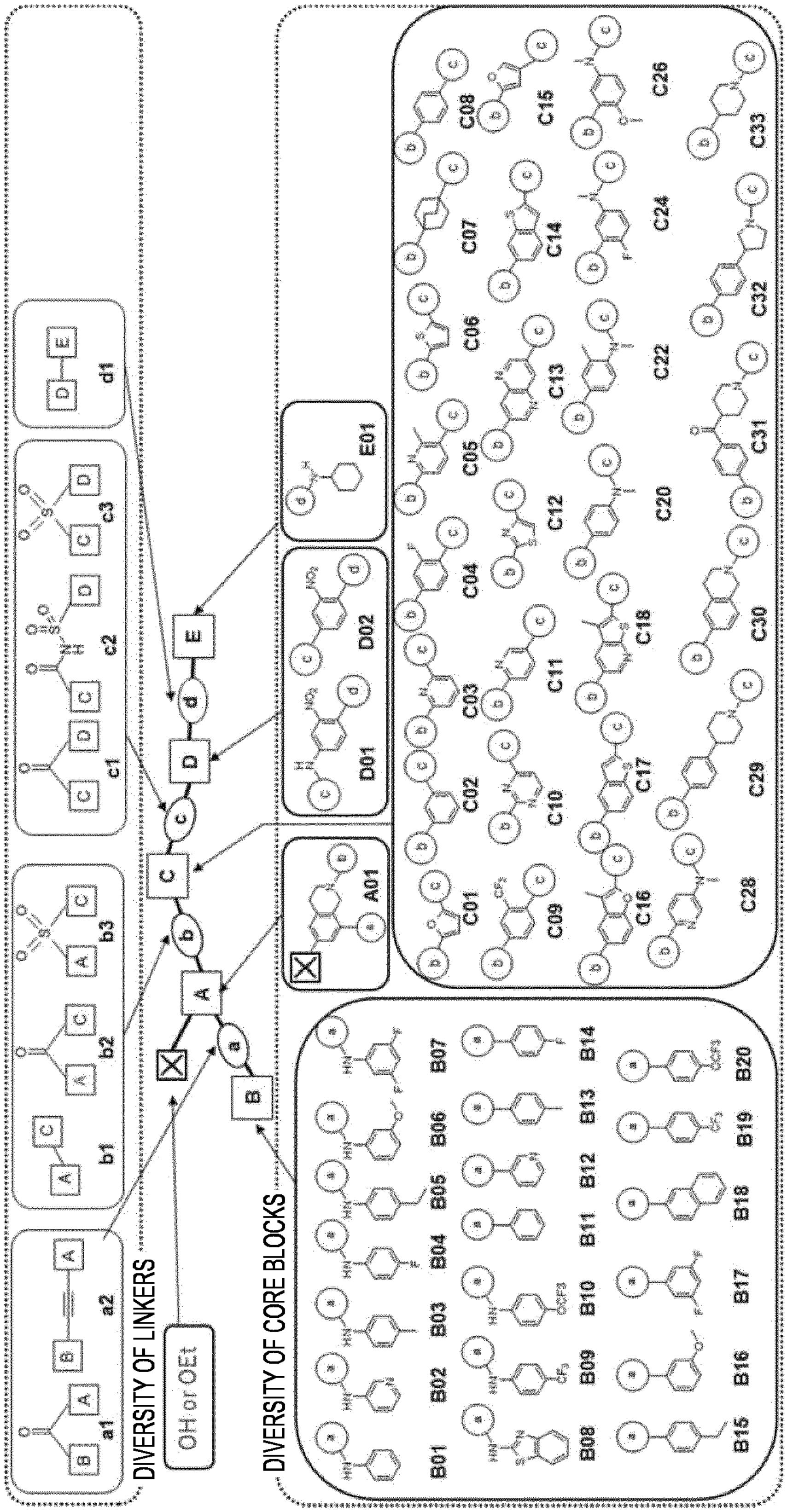
DIVERSITY OF LINKERS
OH or OEt
DIVERSITY OF CORE BLOCKS
a1
a2
b1
b2
b3
c1
c2
c3
d1
A01
D01
D02
E01
B01
B02
B03
B04
B05
B06
B07
B08
B09
B10
B11
B12
B13
B14
B15
B16
B17
B18
B19
B20
C01
C02
C03
C04
C05
C06
C07
C08
C09
C10
C11
C12
C13
C14
C15
C16
C17
C18
C20
C22
C24
C26
C28
C29
C30
C31
C32
C33

Figure 2

Figure 3

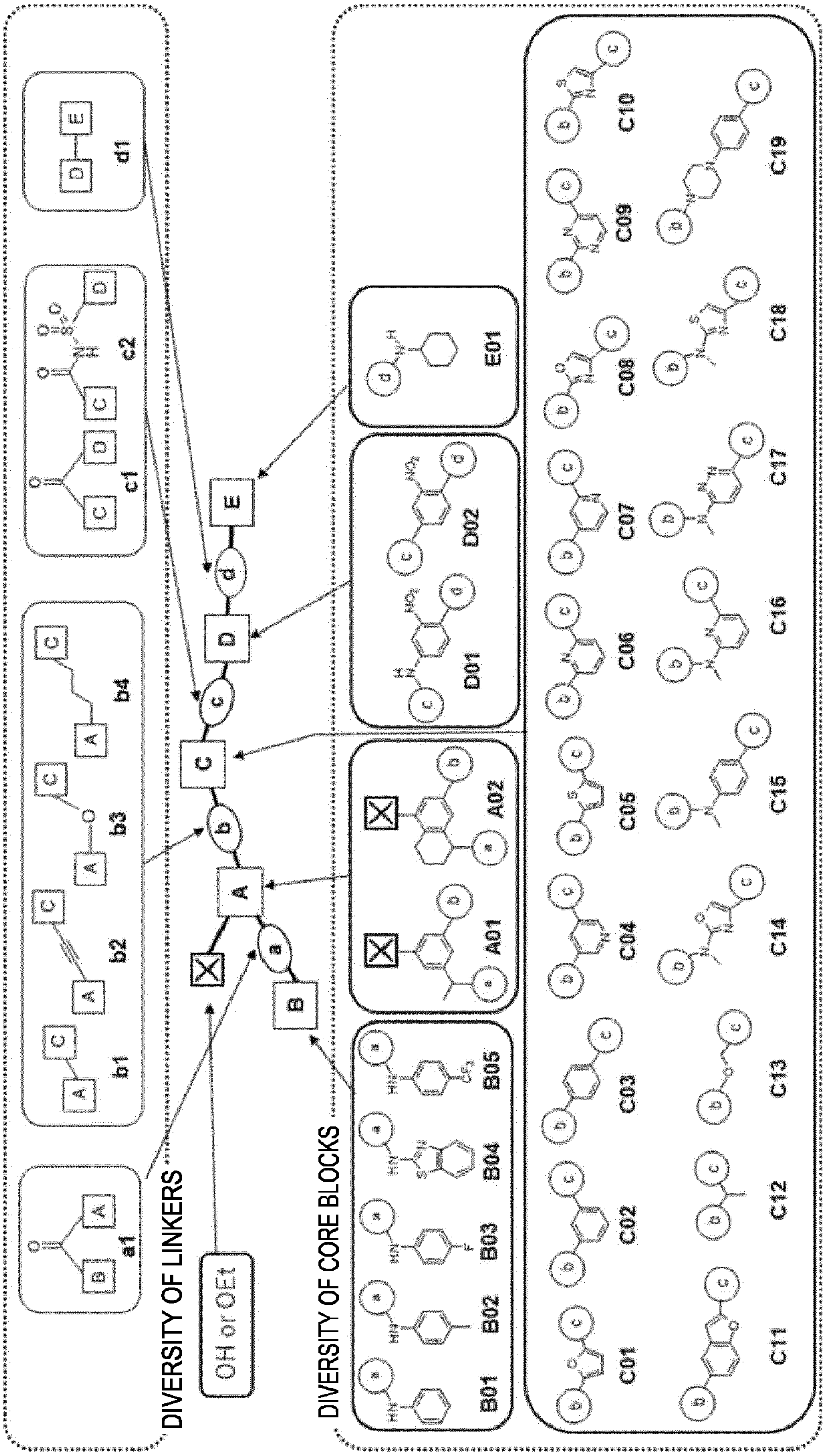
DIVERSITY OF LINKERS
OH or OEt
DIVERSITY OF CORE BLOCKS
a1
b1
b2
b3
b4
c1
c2
d1
B01
B02
B03
B04
B05
A01
A02
D01
D02
E01
C01
C02
C03
C04
C05
C06
C07
C08
C09
C10
C11
C12
C13
C14
C15
C16
C17
C18
C19

## INTERNATIONAL SEARCH REPORT

International application No.
**PCT/JP2022/003468**

### A. CLASSIFICATION OF SUBJECT MATTER

***C07C 41/16***(2006.01)i; ***C07C 41/58***(2006.01)i; ***C07C 43/23***(2006.01)i; ***C07C 209/68***(2006.01)i; ***C07C 211/27***(2006.01)i; ***C07C 211/55***(2006.01)i; ***C07C 211/56***(2006.01)i; ***C07C 211/63***(2006.01)i; ***C07D 237/24***(2006.01)i; ***C07D 401/12***(2006.01)i; ***C07D 401/14***(2006.01)i; ***C40B 40/04***(2006.01)i; ***C07D 213/65***(2006.01)i; ***C07D 213/74***(2006.01)i; ***C07D 213/82***(2006.01)i; ***C07D 295/155***(2006.01)i; ***C07D 295/192***(2006.01)i; ***C07D 295/205***(2006.01)i; ***C07D 239/42***(2006.01)i; ***C07F 9/09***(2006.01)i

FI: C07C41/16; C07C41/58; C07C43/23 A; C07C209/68; C07C211/55; C07C211/56; C07C211/27; C07F9/09 Z; C07C211/63; C07D213/65; C07D401/14; C07D401/12; C07D237/24; C07D213/82; C07D295/155; C07D295/192; C07D295/205; C07D213/74; C07D239/42; C40B40/04

According to International Patent Classification (IPC) or to both national classification and IPC

### B. FIELDS SEARCHED

Minimum documentation searched (classification system followed by classification symbols)

C07C41/16; C07C41/58; C07C43/23; C07C209/68; C07C211/27; C07C211/55; C07C211/56; C07C211/63; C07D237/24; C07D401/12; C07D401/14; C40B40/04; C07D213/65; C07D213/74; C07D213/82; C07D295/155; C07D295/192; C07D295/205; C07D239/42; C07F9/09

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN); CASREACT (STN)

### C. DOCUMENTS CONSIDERED TO BE RELEVANT

| Category* | Citation of document, with indication, where appropriate, of the relevant passages | Relevant to claim No. |
|---|---|---|
| A | HONG, X. et al. Monofluoromethyl-Substituted Sulfonium Ylides: Preparation, Structure-Reactivity Study and Substrate Scope. Chinese Journal of Chemistry. 2020, vol. 38, no. 11, pp. 1317-1331<br>abstract, schemes 6-7 | 1-17 |
| A | WO 2006/003902 A1 (MITSUI CHEMICALS, INC) 12 January 2006 (2006-01-12)<br>claims, paragraphs [0106]-[0109], example 45 | 1-17 |
| A | REGUEIRO-REN, A. et al. Novel semi-synthetic nocathiacin antibiotics: synthesis and antibacterial activity of bis- and mono-O-alkylated derivatives. Bioorganic & Medicinal Chemistry Letters. 2004, vol. 14, no. 1, pp. 171-175<br>abstract, table 1 | 1-17 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 March 2022** | **22 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/003468**

| C. DOCUMENTS CONSIDERED TO BE RELEVANT | | |
|---|---|---|
| Category* | Citation of document, with indication, where appropriate, of the relevant passages | Relevant to claim No. |
| A | JP 2005-536492 A (BRISTOL-MYERS SQUIBB COMPANY) 02 December 2005 (2005-12-02) paragraphs [0035]-[0038], examples 1, 18, 22 | 1-17 |
| A | REKHA, V. V. et al. A Simple, Efficient, Green, Cost Effective and Chemoselective Process for the Esterification of Carboxylic Acids. Organic Process Research & Development. 2009, vol. 13, no. 4, pp. 769-773 abstract, tables 1-4 | 1-17 |
| A | KIM, In-Hae et al. Journal of Medicinal Chemistry. 2011, 54, 1752-1761 abstract, scheme 2 | 1-17 |
| A | JP 2002-308812 A (SNPE) 23 October 2002 (2002-10-23) claims, examples | 1-17 |
| A | KONDO, Y. et al. 有機超強塩基を用いる有機合成反応, 有機合成化学協会誌, 2005, vol. 63, no. 5, 453-463 fig. 1-2, table 1, (Organic Synthesis Using Organic Superbase. Journal of Synthetic Organic Chemistry, Japan.) | 1-17 |

Form PCT/ISA/210 (second sheet) (January 2015)

**INTERNATIONAL SEARCH REPORT**
**Information on patent family members**

International application No.
**PCT/JP2022/003468**

| Patent document cited in search report | Publication date (day/month/year) | Patent family member(s) | Publication date (day/month/year) |
|---|---|---|---|
| WO 2006/003902 A1 | 12 January 2006 | US 2008/0318767 A1<br>claims, examples, paragraphs [0104]-[0110]<br>US 2011/0288240 A1<br>EP 1775301 A1<br>CN 1976940 A<br>KR 10-2007-0034615 A<br>BR PI0512937 A<br>RU 2007103824 A<br>TW 200609242 A<br>MY 145327 A | |
| JP 2005-536492 A | 02 December 2005 | US 2004/0018963 A1<br>paragraphs [0095]-[0098], examples 1, 18, 22<br>WO 2004/004646 A2<br>EP 1531854 A4<br>CA 2491074 A<br>BR 312550 A<br>CN 1678340 A<br>AU 2003247783 A<br>MX PA05000140 A | |
| JP 2002-308812 A | 23 October 2002 | US 2002/0193641 A1<br>claims, examples<br>EP 1234811 A1<br>FR 2821352 A<br>HU 200689 A<br>AT 280748 T<br>ES 2230451 T<br>HU 200689 D0 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4453017 A
- JP 2006213692 A


### Non-patent literature cited in the description


- *J. Am. Chem. Soc.,* 2007, vol. 129, 15830
- *Bioorg. Med. Chem. Lett.,* 2004, vol. 14, 3013
- *J. Med. Chem.,* 2011, vol. 54, 1752
- *J. Am. Chem. Soc.,* 1990, vol. 112, 297
- *J. Chem. Soc. PERKIN TRANS. I,* 1989, vol. 807
- *J. Org. Chem.,* 1998, vol. 63, 7868-7874
- Thermal Measurements and Equilibrium. Experimental Chemistry Lecture. Chemical Society of Japan, vol. 5, 460
- *Eur. J. Org. Chem.,* 2019, 6735-6748
- *Org. Biomol. Chem.,* 2017, vol. 15, 4081-4085
- *Chem. Eur. J.,* 25 December 2020
- *J. Org. Chem.,* 1984, vol. 49, 1122-1125
- *CHEMICAL ABSTRACTS,* 98015-45-3
- *CHEMICAL ABSTRACTS,* 98327-87-8
- *CHEMICAL ABSTRACTS,* 161118-67-8
- *CHEMICAL ABSTRACTS,* 13400-13-0
- *CHEMICAL ABSTRACTS,* 110-87-2
- *CHEMICAL ABSTRACTS,* 12012-95-2
- *CHEMICAL ABSTRACTS,* 13965-03-2
- *CHEMICAL ABSTRACTS,* 52522-40-4
- *CHEMICAL ABSTRACTS,* 95464-05-4
- *CHEMICAL ABSTRACTS,* 72287-26-4
- *CHEMICAL ABSTRACTS,* 165535-45-5
- *CHEMICAL ABSTRACTS,* 17061-62-0
- *CHEMICAL ABSTRACTS,* 81675-81-2
- *CHEMICAL ABSTRACTS,* 111324-03-9
- *CHEMICAL ABSTRACTS,* 13716-12-6
- *CHEMICAL ABSTRACTS,* 3087-39-6
- *CHEMICAL ABSTRACTS,* 373-68-2
- *CHEMICAL ABSTRACTS,* 1621274-19-8
- *CHEMICAL ABSTRACTS,* 1599466-81-5
- *CHEMICAL ABSTRACTS,* 162046-66-4
- *CHEMICAL ABSTRACTS,* 5096-73-1
- *CHEMICAL ABSTRACTS,* 57260-71-6
- *CHEMICAL ABSTRACTS,* 80518-57-6
- *Chem. Eur. J.,* 2021, vol. 27, 4216-4229
- *Expert Opinion on Drug Discovery,* 2008, vol. 3 (9), 1123-1143
- *J. Med. Chem.,* 2008, vol. 51, 6902-6915
- *CHEMICAL ABSTRACTS,* 406233-75-8
- *CHEMICAL ABSTRACTS,* 1506205-91-9
- *CHEMICAL ABSTRACTS,* 1545014-81-0
- *CHEMICAL ABSTRACTS,* 956431-01-9
- *CHEMICAL ABSTRACTS,* 17117-17-8
- *CHEMICAL ABSTRACTS,* 1008361-71-4
- *CHEMICAL ABSTRACTS,* 90555-66-1
- *CHEMICAL ABSTRACTS,* 1171892-40-2
- *CHEMICAL ABSTRACTS,* 2014-75-7
- *CHEMICAL ABSTRACTS,* 1008304-87-7
- *CHEMICAL ABSTRACTS,* 1690948-00-5
- *CHEMICAL ABSTRACTS,* 1261964-16-2
- *CHEMICAL ABSTRACTS,* 1261907-60-1
- *CHEMICAL ABSTRACTS,* 17768-41-1
- *CHEMICAL ABSTRACTS,* 1114808-89-7
- *CHEMICAL ABSTRACTS,* 1773-99-5
- *CHEMICAL ABSTRACTS,* 43192-33-2
- *CHEMICAL ABSTRACTS,* 1282660-71-2
- *CHEMICAL ABSTRACTS,* 406233-13-4
- *CHEMICAL ABSTRACTS,* 591-20-8
- *CHEMICAL ABSTRACTS,* 74115-13-2
- *CHEMICAL ABSTRACTS,* 149104-90-5